# EUROPEAN PATENT APPLICATION

(11) **EP 4 242 199 A1**
(43) Date of publication of application: **13.09.2023**
(21) Application number: 22160484.6
(22) Date of filing: 07.03.2022
(51) Int. Cl.: C07C 271/60, A01N 43/653, A01N 43/88, A01P 3/00, C07D 249/12, C07D 273/01

(54) **USE OF STROBILURIN TYPE COMPOUNDS FOR COMBATING PHYTOPATHOGENIC FUNGI CONTAINING AN AMINO ACID SUBSTITUTION F129L IN THE MITOCHONDRIAL CYTOCHROME B PROTEIN CONFERRING RESISTANCE TO QO INHIBITORS XII**

(71) Applicant: BASF SE, 67056 Ludwigshafen am Rhein (DE)
(72) Inventor: DEY, Chandan, 400705 Navi Mumbai (IN); DAS, Saikat, 400705 Navi Mumbai (IN); DIETZ, Jochen, 67056 Ludwigshafen am Rhein (DE); KHANNA, Smriti, 400705 Navi Mumbai (IN); LUDWIG, Lydia, 67117 Limburgerhof (DE); FEHR, Marcus, 67117 Limburgerhof (DE); KOCH, Andreas, 67117 Limburgerhof (DE); SIEPE, Isabella, 67117 Limburgerhof (DE); LE VEZOUET, Ronan, 67056 Ludwigshafen am Rhein (DE); CRAIG, Ian Robert, 67056 Ludwigshafen am Rhein (DE); RATH, Rakesh, 400705 Navi Mumbai (IN); GRAMMENOS, Wassilios, 67056 Ludwigshafen am Rhein (DE); TEGGE, Vanessa, 67117 Limburgerhof (DE)
(74) Representative: BASF IP Association

(57) **Abstract**

The present invention relates to the use of strobilurin type compounds of formula I and the N-oxides and the salts thereof for combating phytopathogenic fungi containing an amino acid substitution F129L in the mitochondrial cytochrome b protein (also referred to as F129L mutation in the mitochondrial cytochrome b gene) conferring resistance to Qo inhibitors, and to methods for combating such fungi. The invention also relates to novel compounds, processes for preparing these compounds, to compositions comprising at least one such compound, and to seeds coated with at least one such compound.

## Description

The present invention relates novel strobilurin type compounds of formula I and the N-oxides and the salts thereof and their use for combating phytopathogenic fungi containing an amino acid substitution F129L in the mitochondrial cytochrome b protein (also referred to as F129L mutation in the mitochondrial cytochrome b gene) conferring resistance to Qo inhibitors (Qol), and to methods for combating such fungi. The invention also relates to novel compounds, processes for preparing these compounds, to compositions comprising at least one such compound, to plant health applications, and to seeds coated with at least one such compound. The present invention also relates to a method for controlling soybean rust fungi (*Phakopsora pachyrhizi*) with the amino acid substitution F129L in the mitochondrial cytochrome b protein.

"Qo inhibitor," as used herein, includes any substance that is capable of diminishing and/or inhibiting respiration by binding to a ubihydroquinone oxidation center of a cytochrome bc₁ complex in mitochondria. The oxidation center is typically located on the outer side of the inner mitochondrial membrane. Many of these compounds are also known as strobilurin-type or strobilurin analogue compounds.

The mutation F129L in the mitochondrial cytochrome b (CYTB) gene shall mean any substitution of nucleotides of codon 129 encoding "F" (phenylalanine; e.g. TTT or TTC) that leads to a codon encoding "L" (leucine; e.g. TTA, TTG, TTG, CTT, CTC, CTA or CTG), for example the substitution of the first nucleotide of codon 129 'T' to 'C' (TTT to CTT), in the CYTB (cytochrome b) gene resulting in a single amino acid substitution in the position 129 from F to L in the cytochrome b protein. Such F129L mutation is known to confer resistance to Qo inhibitors.

Qol fungicides, often referred to as strobilurin-type fungicides, are conventionally used to control a number of fungal pathogens in crops. Qo inhibitors typically work by inhibiting respiration by binding to a ubihydroquinone oxidation center of a cytochrome bc₁ complex (electron transport complex III) in mitochondria. Said oxidation center is located on the outer side of the inner mitochondrial membrane. A prime example of the use of Qols includes the use of, for example, strobilurins on wheat for the control of *Septoria tritici* (also known as *Mycosphaerella graminicola),* which is the cause of wheat leaf blotch. Unfortunately, widespread use of such Qols has resulted in the selection of mutant pathogens which are resistant to such Qols. Resistance to Qols has been detected in several phytopathogenic fungi such as *Blumeria graminis, Mycosphaerella fijiensis, Pseudoperonspora cubensis* or *Venturia inaequalis.* The major part of resistance to Qols in agricultural uses has been attributed to pathogens containing a single amino acid residue substitution G143A in the cytochrome b gene for their cytochrome bc₁ complex, the target protein of Qols which have been found to be controlled by specific Qols (WO 2013/092224). Despite several commercial Qol fungicides have also been widely used in soybean rust control, the single amino acid residue substitution G143A in the cytochrome b protein conferring resistance to Qol fungicides was not observed.

Instead soybean rust acquired a different genetic mutation in the cytochrome b gene causing a single amino acid substitution F129L which also confers resistance against Qol fungicides. The efficacy of Qol fungicides used against soybean rust conventionally, i.e. pyraclostrobin, azoxystrobin, picoxystrobin, orysastrobin, dimoxystrobin and metominostrobin, has decreased to a level with practical problems for agricultural practice.

Although it seems that trifloxystrobin was less affected by the F129L amino acid substitution to the same degree as other Qol fungicides such as azoxystrobin and pyraclostrobin, trifloxystrobin was never as efficacious on a fungal population bearing the F129L Qol resistance mutation as on a sensitive population (Crop Protection 27, (2008) 427-435).

Thus, new methods are desirable for controlling pathogen induced diseases in crops comprising plants subjected to pathogens containing a F129L mutation in the mitochondrial cytochrome b gene conferring resistance to Qo inhibitors. Furthermore, in many cases, in particular at low application rates, the fungicidal activity of the known fungicidal strobilurin compounds is unsatisfactory, especially in case that a high proportion of the fungal pathogens contain a mutation in the mitochondrial cytochrome b gene conferring resistance to Qo inhibitors. Besides there is an ongoing need for new fungicidally active compounds which are more effective, less toxic and/or environmentally safer. Based on this, it was also an object of the present invention to provide compounds having improved activity and/or a broader activity spectrum against phytopathogenic fungi and/or even further reduced toxicity against non target organisms such as vertebrates and invertebrates.

Certain strobilurin type compounds have been described in WO 1997/05103 and EP 463488. However, it is not mentioned that these compounds inhibit fungal pathogens containing a F129L substitution in the mitochondrial cytochrome b protein conferring resistance to Qo inhibitors.

The compounds according to the present invention differ from those described in the abovementioned publications that the methyl oxime side chain contains an alkyne linker group as defined herein.

Therefore, the invention provides novel compounds of formula I 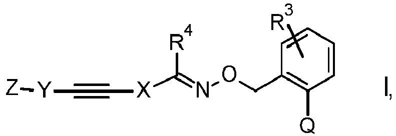 wherein
- Q: is a group selected from the groups Q1, Q2, Q3 and Q4, wherein * represents the point of attachment to the phenyl ring

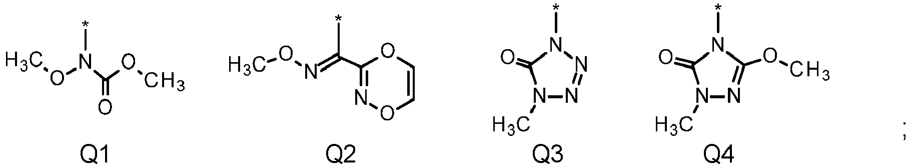

- R³: is selected from hydrogen, halogen, CN, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, -O-C₁-C₄-alkyl, -O-C₁-C₄-haloalkyl, -O-C₃-C₆-cycloalkyl, -C₁-C₂-alkyl-C₃-C₆-cycloalkyl, phenyl, 3- to 6-membered heterocycloalkyl and 5- or 6-membered heteroaryl, wherein said heterocycloalkyl and heteroaryl besides carbon atoms contain 1, 2 or 3 heteroatoms selected from N, O and S provided that such heterocycle cannot contain 2 contiguous atoms selected from O and S, wherein said phenyl, heterocycloalkyl and heteroaryl are bound directly or via an oxygen atom or via a C₁-C₂-alkylene linker, and wherein said phenyl and heteroaryl are unsubstituted or substituted by 1, 2 or 3 identical or different substituents selected from halogen, CN, NH₂, NO₂, C₁-C₄-alkyl, C₁-C₄-haloalkyl, -O-C₁-C₄-alkyl and -O-C₁-C₄-haloalkyl;
- R⁴: is selected from hydrogen, C₁-C₆-alkyl, C₂-C₄-alkenyl, C₂-C₄-alkynyl, C₁-C₆-haloalkyl, C₂-C₄-haloalkenyl, C₂-C₄-haloalkynyl, -(C₁-C₂-alkyl)-O-(C₁-C₂-alkyl), -(C₁-C₂-alkyl)-O-(C₁-C₂-haloalkyl) and -C₁-C₄-alkyl-C₃-C₆-cycloalkyl;
- X, Y: , independently of each other, are a direct bond or the divalent group -CL¹L²-;
- L¹, L²,: independently of each other, are selected from hydrogen, C₁-C₃-alkyl, C₁-C₃-haloalkyl, C₂-C₃-haloalkenyl, C₂-C₃-haloalkynyl, -(C₁-C₂-alkyl)-O-(C₁-C₂-alkyl), -(C₁-C₂-alkyl)-O-(C₁-C₂-haloalkyl), cyclopropyl and -C₁-C₂-alkyl-cyclopropyl; or L¹ and L², together with the interjacent carbon atom, form a cyclopropyl; wherein the cyclic moieties of L¹ and L², independently of each other, are unsubstituted or carry 1 or 2 identical or different groups R^{L}:

- R^{L}: is selected from halogen, CN, NO₂, C₁-C₄-alkyl, C₁-C₄-haloalkyl, -O-C₁-C₄-alkyl and -O-C₁-C₄-haloalkyl;

- Z: is selected from hydrogen, C₁-C₆-alkyl, C₂-C₆-alkenyl, -C(=O)-C₁-C₄-alkyl, C₁-C₄-alkyl-O-C₁-C₄-alkyl and SiR^{S1}R^{S2}R^{S3},

- R^{S1}, R^{S2}, R^{S3},: independently of each other, are selected from hydrogen, halogen, C₁-C₄-alkyl, -O-C₁-C₄-alkyl,phenyl and phenoxy, wherein phenyl and phenoxy are unsubstituted or carry 1, 2 or 3 identical or different groups R^{Sa}:
- R^{Sa}: is selected from halogen, C₁-C₄-alkyl and -O-C₁-C₄-alkyl; and wherein the abovementioned non-cyclic groups Z are unsubstituted or carry 1, 2, 3 or up to the maximum number of identical or different groups R^{na}:
- R^{na}: is selected from halogen, CN, hydroxy, C₁-C₄-alkyl and -O-C₁-C₄-alkyl; or

- Z: is selected from C₃-C₆-cycloalkyl, phenyl, 3- to 6-membered heterocycloalkyl, 3- to 6-membered heterocycloalkenyl and 5- or 6-membered heteroaryl, wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S, and wherein the abovementioned cyclic groups Z are unsubstituted or carry 1, 2, 3 or up to the maximum number of identical or different groups R^{a}:
- R^{a}: is selected from halogen, CN, hydroxy, NR^{A}R^{B}, C₁-C₄-alkyl, C₂-C₄-alkenyl, C₂-C₄-al-kynyl, -O-C₁-C₄-alkyl,-C(=N-O-C₁-C₄-alkyl)-C₁-C₄-alkyl, -C(=O)-C₁-C₄-alkyl, -C(=O)-O-C₁-C₄-alkyl, -C(=O)-NH-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl, wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S, wherein said phenyl, heterocycloalkyl, heterocycloalkenyl and heteroaryl are bound directly or via an oxygen atom or via a C₁-C₂-alkylene linker, and/or
- 2 R^{a}: substituents bound to neighboring ring atoms, together with the two interjacent ring atoms, form a saturated, partially unsaturated or aromatic 5- to 6-membered fused carbo- or heterocycle, wherein the heterocycle includes beside carbon atoms 1 or 2 heteroatoms independently selected from N, O and S as ring member atoms, provided that such heterocycle cannot contain 2 contiguous atoms selected from O and S; and wherein the aliphatic and cyclic moieties of R^{a} are unsubstituted or carry 1, 2, 3, 4 or up to the maximum number of identical or different groups R^{b}:
- R^{b}: is selected from halogen, CN, hydroxy, NO₂, C₁-C₄-alkyl, C₁-C₄-haloalkyl, -O-C₁-C₄-alkyl,and -O-C₁-C₄-haloalkyl;
- R^{A}, R^{B}: independently of each other, are selected from hydrogen, C₁-C₄-alkyl and C₁-C₄-haloalkyl;
and in form of stereoisomers and tautomers thereof, and the N-oxides and the agriculturally acceptable salts thereof.

Although the present invention will be described with respect to particular embodiments, this description is not to be construed in a limiting sense.

Before describing in detail exemplary embodiments of the present invention, definitions important for understanding the present invention are given. As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising of".

Unless otherwise indicated, the following definitions are set forth to illustrate and define the meaning and scope of the various terms used to describe the invention herein and the appended claims. These definitions should not be interpreted in the literal sense as they are not intended to be general definitions and are relevant only for this application.

The term "compounds I" refers to compounds of formula I. Likewise, this terminology applies to all sub-formulae, e. g. "compounds I.A" refers to compounds of formula I.A or "compounds IV" refers to compounds of formula IV, etc..

The term "independently" when used in the context of selection of substituents for a variable, it means that where more than one substituent is selected from a number of possible substituents, those substituents may be the same or different.

The organic moieties or groups mentioned in the above definitions of the variables are collective terms for individual listings of the individual group members. The term "Cᵥ-C_{w}" indicates the number of carbon atom possible in each case.

The term "halogen" refers to fluorine, chlorine, bromine and iodine.

The term "C₁-C₄-alkyl" refers to a straight-chained or branched saturated hydrocarbon group having 1 to 4 carbon atoms, for example, methyl (CH₃), ethyl (C₂H₅), propyl, 1-methylethyl (isopropyl), butyl, 1-methylpropyl, 2-methylpropyl, 1,1-dimethylethyl.

The term "C₂-C₄-alkenyl" refers to a straight-chain or branched unsaturated hydrocarbon radical having 2 to 4 carbon atoms and a double bond in any position such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₄-alkynyl" refers to a straight-chain or branched unsaturated hydrocarbon radical having 2 to 4 carbon atoms and containing at least one triple bond such as ethynyl, prop-1-ynyl, prop-2-ynyl, but-1-ynyl, but-2-ynyl, but-3-ynyl, 1-methyl-prop-2-ynyl.

The term "C₁-C₄-haloalkyl" refers to a straight-chained or branched alkyl group having 1 to 4 carbon atoms wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "-O-C₁-C₄-alkyl" refers to a straight-chain or branched alkyl group having 1 to 4 carbon atoms which is bonded via an oxygen, at any position in the alkyl group, e.g. OCH₃, OCH₂CH₃, O(CH₂)₂CH₃, 1-methylethoxy, O(CH₂)₃CH₃, 1-methyl¬propoxy, 2-methylpropoxy or 1,1-dimethylethoxy.

The term "C₃-C₆-cycloalkyl" refers to monocyclic saturated hydrocarbon radicals having 3 to 6 carbon ring members, such as cyclopropyl (C₃H₅), cyclobutyl, cyclopentyl or cyclohexyl. The term "C₃-C₆-cycloalkenyl " refers to monocyclic saturated hydrocarbon radicals having 3 to 6 carbon ring members and one or more double bonds.

The term "3- to 6-membered heterocycloalkyl" refers to 3- to 6-membered monocyclic saturated ring system having besides carbon atoms one or more heteroatoms, such as O, N, S as ring members. The term "C₃-C₆-membered heterocycloalkenyl" refers to 3- to 6-membered monocyclic ring system having besides carbon atoms one or more heteroatoms, such as O, N and S as ring members, and one or more double bonds.

The term "-C₁-C₄-alkyl-C₃-C₆-cycloalkyl" refers to alkyl having 1 to 4 carbon atoms (as defined above), wherein one hydrogen atom of the alkyl radical is replaced by a cycloalkyl radical having 3 to 6 carbon atoms.

The term "phenyl" refers to C₆H₅.

The term "5- or 6-membered heteroaryl" which contains 1, 2, 3 or 4 heteroatoms from the group consisting of O, N and S, is to be understood as meaning aromatic heterocycles having 5 or 6 ring atoms. Examples include:
- 5-membered heteroaryl which in addition to carbon atoms, e.g. contain 1, 2 or 3 N atoms and/or one sulfur and/or one oxygen atom: for example 2-thienyl, 3-thienyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl and 1,3,4-triazol-2-yl;
- 6-membered heteroaryl which, in addition to carbon atoms, e.g. contain 1, 2, 3 or 4 N atoms as ring members, e.g. 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

The term "C₁-C₂-alkylene linker" means a divalent alkyl group such as -CH₂- or -CH₂-CH₂-that is bound at one end to the core structure of formula I and at the other end to the particular substituent.

The term "2 R^{a} substituents bound to neighboring ring atoms, together with the two interjacent ring atoms, form a saturated, partially unsaturated or aromatic 5- to 6-membered fused carbo- or heterocycle" is to be understood that said fused cycle forms together with any cyclic group Z a condensed or fused biyclic ring system, wherein two rings share two adjacent atoms. In other words, the rings share one covalent bond, i.e. the so-called bridgehead atoms are directly connected. The bridgehead atoms are neighboring carbon or nitrogen ring atoms, preferably two neighboring carbon atoms or a carbon ring atome directly connected to a nitrogen ring atom. Such bicyclic ring system may be unsubstituted or carry 1, 2, 3 or up to the maximum number of identical or different groups R^{a}. Such bicyclic rings systems include 6-6, 6-5, 5-5 and 5-6 bicyclic ring systems, wherein the first number indiacates the number of ring atoms of the cycle attached to Y and the second number indicates the number of ring atoms of the fused cycle. Examples include:
- 6-6 fused bicyclic ring systems which contain no heteroatoms from the group consisting of O, N and S as ring atoms, e.g.: tetraline (1,2,3,4-tetrahydronaphthaline, e.g. tetralin-1-yl, tetralin-2-yl or tetralin-6-yl), dihydronaphthalene (e.g. 1,2-dihydronaphthalene, 1,4-dihydronaphthalene), naphthalene;
- 6-5 or 5-6 fused bicyclic ring systems which contain no heteroatoms from the group consisting of O, N and S as ring atoms: benzofuran (e.g. indan-1-yl, indan-5-yl), 1H-indene (e.g. 1*H*-inden-2-yl, 1*H*-inden-3-yl, 1*H*-inden-5-yl, 1*H*-inden-7-yl);
- 6-7 fused bicyclic ring systems which contain no heteroatoms from the group consisting of O, N and S as ring atoms: e.g. tetrahydro-5*H*-benzo[7]annulene;
- 6-6 fused bicyclic ring systems which contain 1 or 2 heteroatoms from the group consisting of O, N and S as ring atoms, e.g.: 2,3-dihydro-1,4-benzodioine (e.g. 2,3-di-hydro-1,4-benzodioxin-6-yl), chromane (e.g. chroman-3-yl, chroman-6-yl or chroman-7-yl);
- 6-5 or 5-6 fused bicyclic ring systems which contain 1 or 2 heteroatoms from the group consisting of O, N and S as ring atoms: 2,3-dihydrobenzofuran (e.g. 2,3-dihydrobenzofuran-4-yl), 1,3-benzodioxole (e.g. 1,3-benzodioxol-4-yl or 1,3-benzodioxol-5-yl), benzofuran (e.g. benzofuran-3-yl), benzothiophene (e.g. benzothiophen-3-yl), 1H-indole (e.g. 1H-indol-3-yl or 1-methylindol-3-yl).

As used herein, the "compounds", in particular "compounds I" include all the stereoisomeric and tautomeric forms and mixtures thereof in all ratios, prodrugs, isotopic forms, their agriculturally acceptable salts, N-oxides and S-oxides thereof.

The term "stereoisomer" is a general term used for all isomers of individual compounds that differ only in the orientation of their atoms in space. The term stereoisomer includes mirror image isomers (enantiomers), mixtures of mirror image isomers (racemates, racemic mixtures), geometric (cis/trans or E/Z) isomers, and isomers of compounds with more than one chiral center that are not mirror images of one another (diastereoisomers). The term tautomer" refers to the coexistence of two (or more) compounds that differ from each other only in the position of one (or more) mobile atoms and in electron distribution, for example, keto-enol tautomers. The term "agriculturally acceptable salts" as used herein, includes salts of the active compounds which are prepared with acids or bases, depending on the particular substituents found on the compounds described herein. "N-oxide" refers to the oxide of the nitrogen atom of a nitrogen-containing heteroaryl or heterocycle. N-oxide can be formed in the presence of an oxidizing agent for example peroxide such as m-chloro-perbenzoic acid or hydrogen peroxide. N-oxide refers to an amine oxide, also known as amine-N-oxide, and is a chemical compound that contains N→O bond.

In respect of the variables, the embodiments of the intermediates correspond to the embodiments of the compounds I.

Preference is given to those compounds I and where applicable also to compounds of all sub-formulae provided herein, e. g. formulae I.1 and 1.2, and to the intermediates such as compounds II, III, IV and V, wherein the substituents and variables (such as n, X, Y, Z, R¹, R², R³, R⁴, R^{a}, and R^{b}) have independently of each other or more preferably in combination (any possible combination of 2 or more substituents as defined herein) the following meanings:
Preference is also given to the uses, methods, mixtures and compositions, wherein the definitions (such as phytopathogenic fungi, treatments, crops, compounds II, further active ingredients, solvents, solid carriers) have independently of each other or more preferably in combination the following meanings and even more preferably in combination (any possible combination of 2 or more definitions as provided herein) with the preferred meanings of compounds I herein:
One embodiment of the invention relates to preferred compounds I, wherein Q is Q1. A further embodiment relates to compounds I, wherein Q is Q2. A further embodiment relates to compounds I, wherein Q is Q3. A further embodiment relates to compounds I, wherein Q is Q4.

According to a further embodiment, R³ is selected from hydrogen, halogen, CN, C₁-C₄-alkyl, C₂-C₄-alkenyl, C₁-C₄-haloalkyl, C₂-C₄-haloalkenyl, C₃-C₆-cycloalkyl, -O-C₁-C₄-alkyl,-O-C₁-C₄-haloalkyl, -C₁-C₂-alkyl-C₃-C₆-cycloalkyl and 3- to 6-membered heterocycloalkyl; more preferably from is selected from hydrogen, halogen, CN, C₁-C₄-alkyl, C₂-C₄-alkenyl, C₂-C₄-alkynyl, C₁-C₄-haloalkyl, C₃-C₄-cycloalkyl, -O-C₁-C₄-alkyl, -O-C₁-C₄-haloalkyl and 3- to 4-membered heterocycloalkyl, wherein said heterocycloalkyl besides carbon atoms contain 1 or 2 heteroatoms selected from N, O and S provided that such heterocycle cannot contain 2 contiguous atoms selected from O and S, and wherein said heterocycloalkyl is bound directly or via an oxygen atom or via a C₁-C₂-alkylene linker; even more preferably from hydrogen, C₁-C₂-alkyl, C₂-alkenyl, C₁-C₂-haloalkyl, -O-C₁-C₂-alkyl, O-C₁-C₂-haloalkyl, C₃-C₄-cycloalkyl, -C₁-C₂-alkyl-C₃-C₄-cycloalkyl, and 3- to 4-membered heterocycloalkyl; further more preferably form hydrogen, C₁-C₂-alkyl, C₁-C₂-haloalkyl, C₃-C₄-cycloalkyl, -O-C₁-C₂-alkyl and -O-C₁-C₂-haloalkyl; particularly preferred from hydrogen, halogen, C₁-C₂-alkyl, and C₁-C₂-haloalkyl, in particular hydrogen or methyl.

Preferably, R³ is in ortho position to the methyl oxime side chain of the molecule, which compounds are of formula I.A:

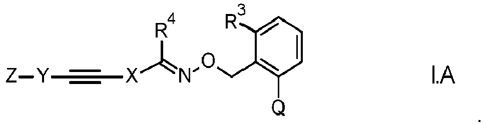

More preferably, Q is Q1 in formula I.A which compounds are of formula I.AQ1:

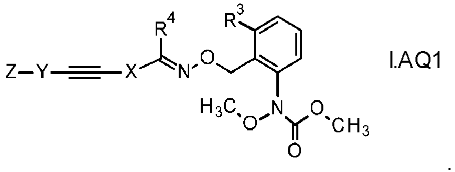

In another preferred embodiment, Q is Q2 in formula I.A which compounds are of formula I.AQ2:

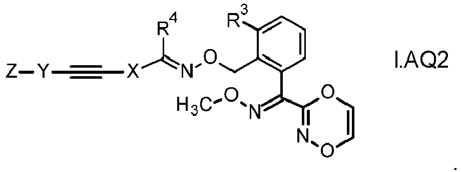

In another preferred embodiment, Q is Q3 in formula I.A which compounds are of formula I.AQ3:

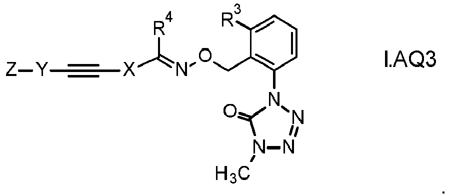

In another preferred embodiment, Q is Q4 in formula I.A which compounds are of formula I.AQ4: 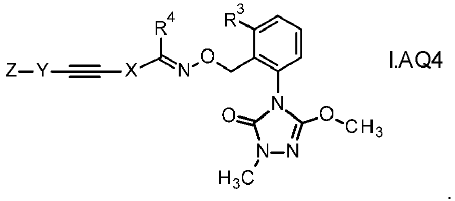 According to a further embodiment, R⁴ is selected from is selected from hydrogen, C₁-C₄-alkyl, C₂-C₄-alkenyl, C₁-C₄-haloalkyl, C₂-C₄-haloalkenyl, -(C₁-C₂-alkyl)-O-(C₁-C₂-alkyl) and -CH₂-cyclopropyl; more preferably from hydrogen, C₁-C₄-alkyl and C₁-C₄-haloalkyl, even more preferably from methyl and C₁-haloalkyl; in particular methyl.

According to a further embodiment, X is a direct bond.

According to a further embodiment, Y is a direct bond. More preferably, X and Y are both direct bonds.

According to a further embodiment, at least one of X and Y is a divalent group -CL¹L²-.

According to a further embodiment, L¹ and L², independently of each other, are selected from hydrogen, C₁-C₃-alkyl, C₁-C₃-haloalkyl, cyclopropyl and -CH₂-cyclopropyl; or, L¹ and L², together with the interjacent carbon atom, form a cyclopropyl; more preferably L¹ and L², independently of each other, are selected from hydrogen and C₁-C₃-alkyl; or, L¹ and L², together with the interjacent carbon atom, form a cyclopropyl; in particular L¹ and L² are both hydrogen.

According to a further embodiment, Y is CH₂, more preferably X is a direct bond and Y is CH₂.

According ot a further embodiment, Z is selected from is selected from hydrogen, C₁-C₆-alkyl, C₂-C₆-alkenyl, -C(=O)-C₁-C₄-alkyl, C₁-C₄-alkyl-O-C₁-C₄-alkyl and SiR^{S1}R^{S2}R^{S3}, wherein R^{S1}, R^{S2}, R^{S3}, independently of each other, are selected from hydrogen, halogen, C₁-C₄-alkyl, -O-C₁-C₄-alkyl,phenyl and phenoxy, wherein phenyl and phenoxy are unsubstituted or carry 1, 2 or 3 identical or different groups R^{Sa}, whererein R^{Sa} is selected from halogen, C₁-C₄-alkyl and -O-C₁-C₄-alkyl,and wherein Z is unsubstituted or carries 1, 2, 3 or up to the maximum number of identical or different groups R^{na}, wherein R^{na} is selected from halogen, CN, hydroxy, C₁-C₄-alkyl and -O-C₁-C₄-alkyl.

According ot a further embodiment, Z is selected from is selected from hydrogen, C₁-C₄-alkyl, -C(=O)-C₁-C₄-alkyl and SiR^{S1}R^{S2}R^{S3}, wherein R^{S1}, R^{S2}, R^{S3}, independently of each other, are selected from hydrogen, C₁-C₄-alkyl and phenyl, wherein phenyl is unsubstituted or carry 1, 2 or 3 identical or different groups R^{Sa}, whererein R^{Sa} is selected from halogen, C₁-C₄-alkyl and -O-C₁-C₄-alkyl,and wherein Z is unsubstituted or carries 1, 2, 3 or up to the maximum number of identical or different groups R^{na}, wherein R^{na} is selected from halogen and hydroxy.

According ot a further embodiment, Z is selected from is selected from hydrogen, C₁-C₄-alkyl and SiR^{S1}R^{S2}R^{S3}, wherein R^{S1}, R^{S2}, R^{S3}, independently of each other, are selected from hydrogen, C₁-C₂-alkyl and phenyl, and wherein Z is unsubstituted or carries 1, 2 or 3 identical or different groups R^{na}, wherein R^{na} is selected from halogen and hydroxy.

According ot a further embodiment, Z is selected from is selected from hydrogen, CH₃, CHF₂, CF₃, CH₂CH₃, C(CH₃)₃, CH(CH₃)OH and Si(CH3)3, Si(CH₂CH₃)₃ and Si(C₆H₅)₃.

According to a further embodiment, Z is selected from C₃-C₆-cycloalkyl, phenyl and 5- or 6-membered heteroaryl, wherein said heteroaryl besides carbon atoms contain 1, 2 or 3 heteroatoms selected from N, O and S provided that such heteroaryl cannot contain 2 contiguous atoms selected from O and S, and wherein Z is unsubstituted or carries 1, 2 or 3 identical or different groups R^{a} as defined herein; more preferably Z is unsubstituted or carries 1 or 2 identical or different groups R^{a} as defined herein; in particular Z is unsubstituted or carries 1 group R^{a} as defined herein.

According to a further embodiment, Z is selected from C₃-C₆-cycloalkyl, phenyl and 5- or 6-membered heteroaryl, wherein said heteroaryl besides carbon atoms contain 1, 2 or 3 heteroatoms selected from N, O and S provided that such heteroaryl cannot contain 2 contiguous atoms selected from O and S, and wherein Z carries 1, 2 or 3 identical or different groups R^{a} as defined herein; more preferably Z carries 1 or 2 identical or different groups R^{a} as defined herein; in particular Z carries 1 group R^{a} as defined herein.

According to a further embodiment, Z is selected from cyclopropyl and phenyl, and wherein Z is unsubstituted or carries 1, 2 or 3 identical or different groups R^{a} as defined herein; more preferably Z is unsubstituted or carries 1 or 2 identical or different groups R^{a} as defined herein; in particular Z is unsubstituted or carries 1 group R^{a} as defined herein.

According to a further embodiment, X is a direct bond and Y is -CH₂- and Z is selected from cyclopropyl and phenyl, and wherein Z is unsubstituted or carries 1, 2 or 3 identical or different groups R^{a} as defined herein; more preferably Z is unsubstituted or carries 1 or 2 identical or different groups R^{a} as defined herein; in particular Z is unsubstituted or carries 1 group R^{a} as defined herein.

According to a further embodiment, X are both direct bonds and Z is selected from cyclopropyl and phenyl, and wherein Z is unsubstituted or carries 1, 2 or 3 identical or different groups R^{a} as defined herein; more preferably Z is unsubstituted or carries 1 or 2 identical or different groups R^{a} as defined herein; in particular Z is unsubstituted or carries 1 group R^{a} as defined herein.

According to a further embodiment, Z is a cyclic group as defined herein and 2 R^{a} substituents bound to neighboring carbon ring atoms, together with the two interjacent carbon ring atoms, form a saturated, partially unsaturated or aromatic 5- to 6-membered fused carbo- or heterocycle, wherein the heterocycle includes beside carbon atoms 1 or 2 heteroatoms independently selected from N, O and S as ring member atoms, provided that such heterocycle cannot contain 2 contiguous atoms selected from O and S; and wherein Z is unsubstituted or carries 1, 2 or 3 further identical or different groups R^{a} and wherein the R^{a} are unsubstituted or carry 1, 2, 3, 4 or up to the maximum number of identical or different groups R^{b} being selected from halogen, CN, NO₂, C₁-C₄-alkyl, C₁-C₄-haloalkyl, -O-C₁-C₄-alkyl,and -O-C₁-C₄-haloalkyl. Said bicyclic ring system is preferably formed by a carbocycle Z selected from cyclopentyl, cyclohexyl or phenyl together with a saturated, partially unsaturated or aromatic 5- to 6-membered fused carbocycle and may be selected from 6-6, 6-5 and 5-6 bicyclic ring systems wherein said bicyclic ring system contains only carbon ring atoms, more preferably said bicyclic ring system is selected from indane, 1H-indene, tetraline (1,2,3,4-tetrahydronaphthaline), dihydronaphthalene, naphthalene and tetrahydro-5H-benzo[7]annulene. According to another preferred embodiment, said bicyclic ring system is formed by a heterocycle Z selected from 5- to 6-membered heterocycloalkyl, 5- to 6-membered heterocycloalkenyl and 5- or 6-membered heteroaryl together with a saturated, partially unsaturated or aromatic 5- to 6-membered fused carobo cycle and may be selected from 6-6, 6-5 and 5-6 bicyclic ring systems, wherein said bicyclic ring system contains besides carbon ring atoms 1 or 2 heteroatoms selected from N, O and S provided that such heterocycle cannot contain 2 contiguous atoms selected from O and S, even more preferably said bicyclic ring system is selected from 2,3-dihydrobenzofuran, 1,3-benzodioxole, benzofuran, benzothiophene, 1H-indole, and chromane.

According to the abovementioned embodiments, ,R^{a} is preferably selected from halogen, CN, C₁-C₄-alkyl, C₂-C₄-alkenyl, C₂-C₄-alkynyl, -O-C₁-C₄-alkyl,-C(=N-O-C₁-C₄-alkyl)-C₁-C₄-alkyl, -C(=O)-C₁-C₄-alkyl,-O-CH₂-C(=N-O-C₁-C₄-alkyl)-C₁-C₄-alkyl, C₃-C₄-cycloalkyl, -C₁-C₂-alkyl-C₃-C₄-cycloalkyl, -O-C₃-C₄-cycloalkyl, phenyl, 3- to 5-membered heterocycloalkyl, 3- to 5-membered heterocycloalkenyl and 5- or 6-membered heteroaryl, wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S, wherein said phenyl, heterocycloalkyl, heterocycloalkenyl and heteroaryl are bound directly or via an oxygen atom or via a C₁-C₂-alkylene linker.

Preferably, R^{a} is selected from halogen, CN, C₁-C₄-alkyl, C₂-C₄-alkenyl, C₂-C₄-alkynyl, -O-C₁-C₄-alkyl,-C(=O)-C₁-C₂-alkyl, -C(=N-O-C₁-C₄-alkyl)-C₁-C₄-alkyl, C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S provided that such heterocycle cannot contain 2 contiguous atoms selected from O and S, wherein said phenyl, heterocycloalkyl and heteroaryl are bound directly or via an oxygen atom or via a methylene linker.

More preferably, R^{a} is selected from halogen, CN, C₁-C₃-alkyl, -O-C₁-C₃-alkyl, -C(=N-O-CH₃)-CH₃, C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S provided that such heterocycle cannot contain 2 contiguous atoms selected from O and S, wherein said phenyl, heterocycloalkyl and heteroaryl are bound directly or via an oxygen atom or via a methylene linker.

In particular, R^{a} is selected from halogen, CN, C₁-C₂-alkyl, -O-C₁-C₂-alkyl, ethenyl, ethynyl and -C(=N-O-CH₃)-CH₃.

According to a further embodiment, R^{a} is selected from halogen, C₁-C₂-alkyl, -O-C₁-C₂-alkyl, wherein the aliphatic moieties are unsubstituted or carry 1, 2 or 3 identical or different groups R^{b} selected from halogen.

According to the abovementioned embodiments for R^{a}, the abovementioned heterocycloalkyl is more preferably a 4-membered heterocycloalkyl, wherein said heterocycloalkyl besides carbon atoms contains 1 heteroatom selected from N, O and S, preferably N.

According to the abovementioned embodiments for R^{a}, the abovementioned heteroaryl is more preferably a 5-membered heteroaryl, wherein said heteroaryl besides carbon atoms contains 1 or 2 heteroatoms selected from N, O and S provided that such heteroaryl cannot contain 2 contiguous atoms selected from O and S, preferably the heteroatoms are selected from N and O.

According to the abovementioned embodiments for R^{a}, the aliphatic and cyclic moieties of R^{a} are unsubstituted or carry 1, 2, 3, 4 or up to the maximum number of identical or different groups R^{b} selected from halogen, CN, NO₂, C₁-C₄-alkyl, C₁-C₄-haloalkyl, -O-C₁-C₄-alkyl and -O-C₁-C₄-haloalkyl; more preferably from halogen; even more preferably only the cyclic moieties of R^{a} are unsubstituted or carry 1, 2, 3, 4 or up to the maximum number of identical or different groups R^{b} selected from halogen, CN, NO₂, C₁-C₄-alkyl, C₁-C₄-haloalkyl, -O-C₁-C₄-alkyl and -O-C₁-C₄-haloalkyl; even more preferably only the phenyl moiety of R^{a} is unsubstituted or carries 1, 2, 3, 4 or 5 identical or different groups R^{b} selected from halogen, CN, C₁-C₄-alkyl, C₁-C₄-haloalkyl, -O-C₁-C₄-alkyl and -O-C₁-C₄-haloalkyl; in particular said phenyl is unsubstituted or carries 1, 2 or 3 identical or different groups R^{b} selected from halogen, CN, C₁-C₂-alkyl, C₁-C₂-haloalkyl, -O-C₁-C₂-alkyl and -O-C₁-C₂-haloalkyl.

According to a further embodiment, X and Y are both direct bonds, and Z is phenyl which is unsubstituted or carries 1, 2 or 3 identical or different substituents R^{a}, wherein R^{a} is selected from halogen, C₁-C₂-alkyl and -O-C₁-C₂-alkyl, wherein the aliphatic moieties of R^{a} are unsubstituted or carry 1, 2 or 3 identical or different groups R^{b} selected from halogen.

According to a further preferred embodiment, the present invention relates to compounds of formula I wherein:
- Q: is selected from Q1, Q2, Q3 and Q4 as defined herein,
- R³: is hydrogen, halogen or C₁-C₄-alkyl, wherein R³ is in ortho position to the methyl oxime side chain;
- R⁴: is selected from C₁-C₆-alkyl;
- X: is a direct bond;
- Y: ist a direct bond or a divalent group -CH₂-;
- Z: is selected from hydrogen, C₁-C₄-alkyl and SiR^{S1}R^{S2}R^{S3}, wherein R^{S1}, R^{S2}, R^{S3}, independently of each other, are selected from hydrogen, C₁-C₂-alkyl and phenyl, and wherein the abovementioned non-cyclic groups Z are unsubstituted or carry 1, 2 or 3 identical or different groups R^{na}, wherein R^{na} is selected from halogen and hydroxy; or
- Z: is selected from C₃-C₆-cycloalkyl, phenyl, 5- or 6-membered heterocycloalkyl, 5- or 6-membered heterocycloalkenyl and 5- or 6-membered heteroaryl, wherein said heteroaryl besides carbon atoms contains 1, 2 or 3 heteroatoms selected from N, O and S provided that such heteroaryl cannot contain 2 contiguous atoms selected from O and S, and wherein Z is unsubstituted or carries 1, 2, 3 or up to the maximum number of identical or different groups R^{a}:

- R^{a}: is selected from halogen, CN, hydroxy, C₁-C₄-haloalkyl, C₁-C₄-alkyl, -O-C₁-C₄-alkyl, -O-C₁-C₄-haloalkyl, -C(=N-O-C₁-C₄-alkyl)-C₁-C₄-alkyl, -C(=O)-C₁-C₄-alkyl, C₃-C₄-cycloalkyl, -C₁-C₂-alkyl-C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S provided that such heterocycle cannot contain 2 contiguous atoms selected from O and S, wherein said phenyl, heterocycloalkyl and heteroaryl are bound directly or via an oxygen atom or via a methylene linker, and wherein the abovementioned cyclic moieties of R^{a} are unsubstituted or carry 1, 2 or 3 identical or different groups R^{b} selected from halogen, CN, C₁-C₂-alkyl, C₁-C₂-halo-alkyl, -O-C₁-C₂-alkyl and -O-C₁-C₂-haloalkyl; and/or 2 R^{a} substituents bound to neighboring carbon ring atoms, together with the two interjacent carbon ring atoms, form a partially unsaturated or aromatic 5- to 6-membered fused carbo- or heterocycle, wherein the heterocycle includes beside carbon atoms 1 or 2 heteroatoms independently selected from N, O and S as ring member atoms, provided that such heterocycle cannot contain 2 contiguous atoms selected from O and S; and wherein the aliphatic and cyclic moieties of R^{a} are unsubstituted or carry 1, 2, 3, 4 or up to the maximum number of identical or different groups R^{b}: R^{b} is selected from halogen, hydroxy, C₁-C₄-alkyl, C₁-C₄-haloalkyl, -O-C₁-C₄-alkyl,and -O-C₁-C₄-haloalkyl;
and in form of stereoisomers and tautomers thereof, and the N-oxides and the agriculturally acceptable salts thereof.

Particularly preferred embodiments of the invention relate to compounds I, wherein X and Y are direct bonds. According to a further embodiment, R³ is in ortho position to the methyl oxime side chain of the molecule, X and Y are direct bonds, which compounds are of formula I.A1:

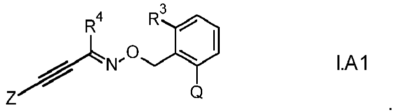

According to a further embodiment, R³ is in ortho position to the methyl oxime side chain of the molecule, X is a direct bond and Y is -CH₂-, which compounds are of formula I.A2:

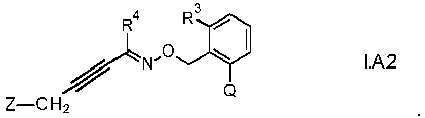

According to a further embodiment, R³ is H, X and Y are direct bonds, which compounds are of formula I.B1:

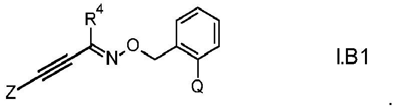

According to a further embodiment, R³ is H, X is a direct bond and Y is -CH₂-, which compounds are of formula I.B2:

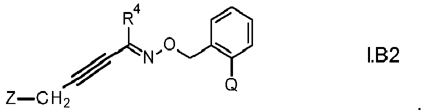

Preferably, R³ of compounds I is one of the following radicals 3-1 to 3-9:

| **No.** | **R³** |
|---|---|
| 3-1 | CH₃ |
| 3-2 | OCH₃ |
| 3-3 | CHF₂ |

| **No.** | **R³** |
|---|---|
| 3-4 | C₃H₅ |
| 3-5 | CH=CH₂ |
| 3-6 | CH₂CH=C(CH₃)₂ |

| **No.** | **R³** |
|---|---|
| 3-7 | CF₃ |
| 3-8 | C(=NOCH₃)CH₃ |
| 3-9 | H |

Even more preferably R³ is H, CH₃, OCH₃, CF₃, CHF₂ or C₃H₅, in particular H or CH₃.

Particularly preferred embodiments of the invention relate to compounds I, wherein the R⁴ is one of the following radicals 4-1 to 4-10:

| **No.** | **R⁴** |
|---|---|
| 4-1 | CH₃ |
| 4-2 | C₂H₅ |
| 4-3 | CH₂OCH₃ |
| 4-4 | CH₂CF₃ |

| **No.** | **R⁴** |
|---|---|
| 4-5 | CH₂C₆H₅ |
| 4-6 | CHF₂ |
| 4-7 | CH₂C₃H₅ |
| 4-8 | CH₂-C(=NOCH₃)CH₃ |

| **No.** | **R⁴** |
|---|---|
| 4-9 | C≡CH |
| 4-10 | C≡CCH₃ |

Particularly preferred embodiments of the invention relate to compounds I, wherein the R^{a} is selected of one of the following radicals a-1 to a-17:

| **No.** | **R^{a}** |
|---|---|
| a-1 | F |
| a-2 | Cl |
| a-3 | Br |
| a-4 | CH₃ |
| a-5 | CHF₂ |
| a-6 | CF₃ |
| a-7 | OCH₃ |
| a-8 | OCHF₂ |
| a-9 | OCF₃ |
| a-10 | C₂H₅ |
| a-11 | CH₂CF₃ |
| a-12 | CH=CH₂ |
| a-13 | C₆H₅ |
| a-14 | C≡CH |
| a-15 | C≡CCH₃ |
| a-16 | C₃H₅ |
| a-17 | C(=NOCH₃)CH₃ |

Preferably, Z in compounds I is one of the following radicals Z-1 to Z-342:

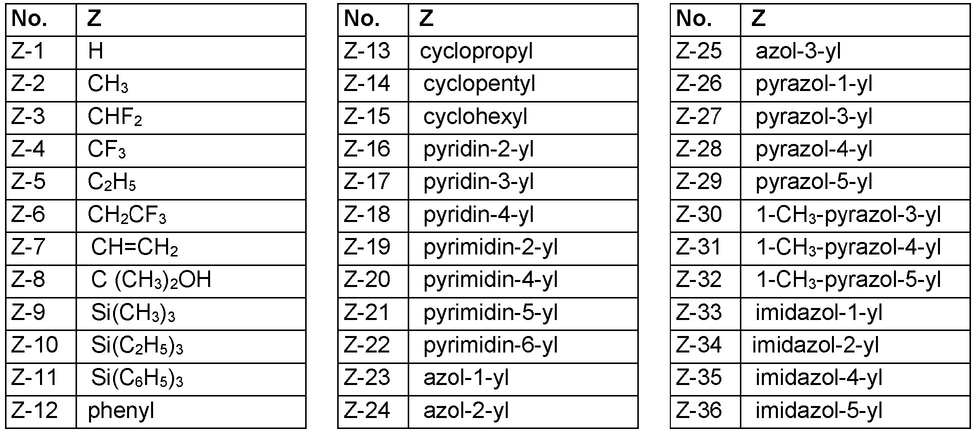

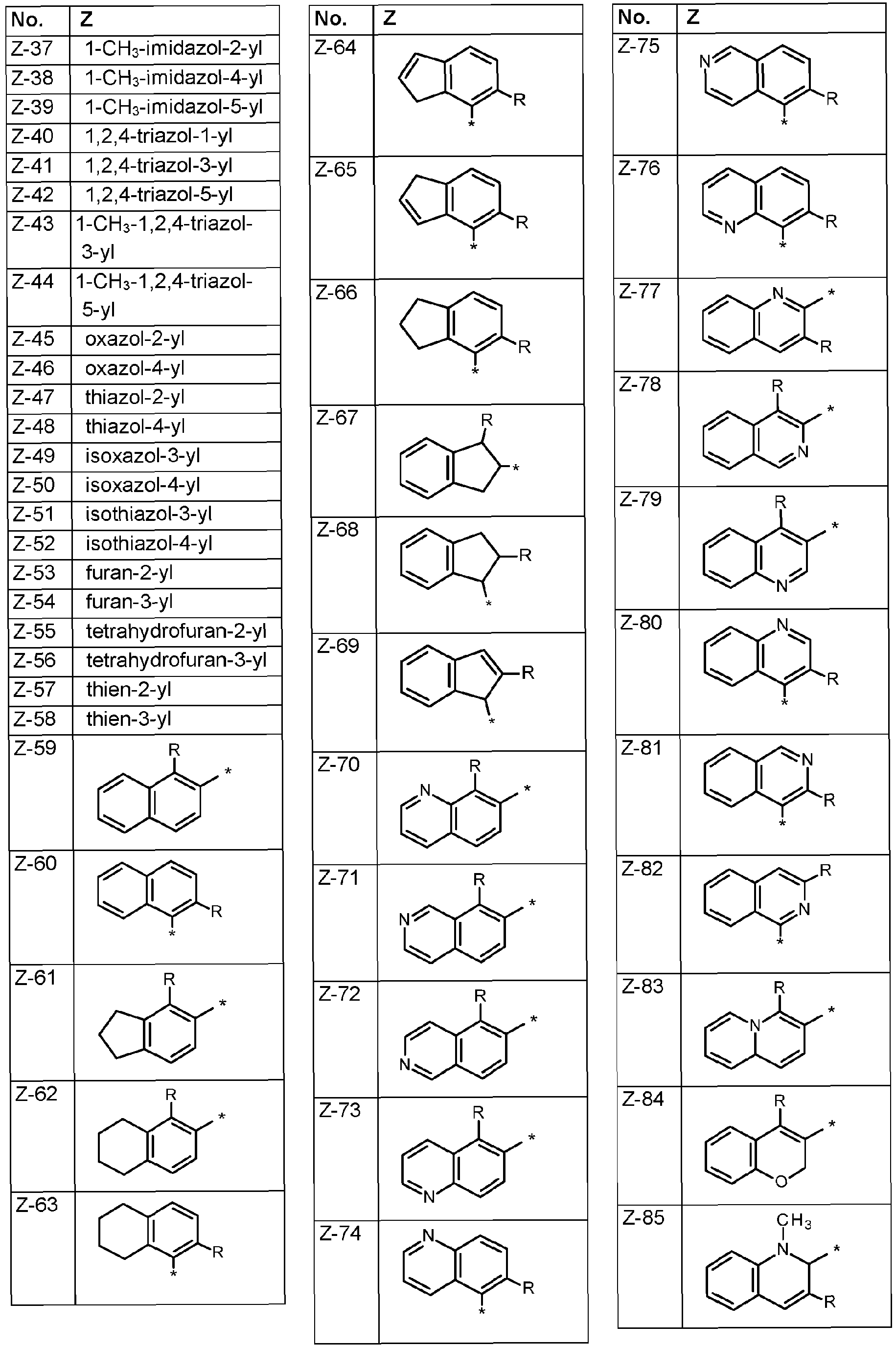

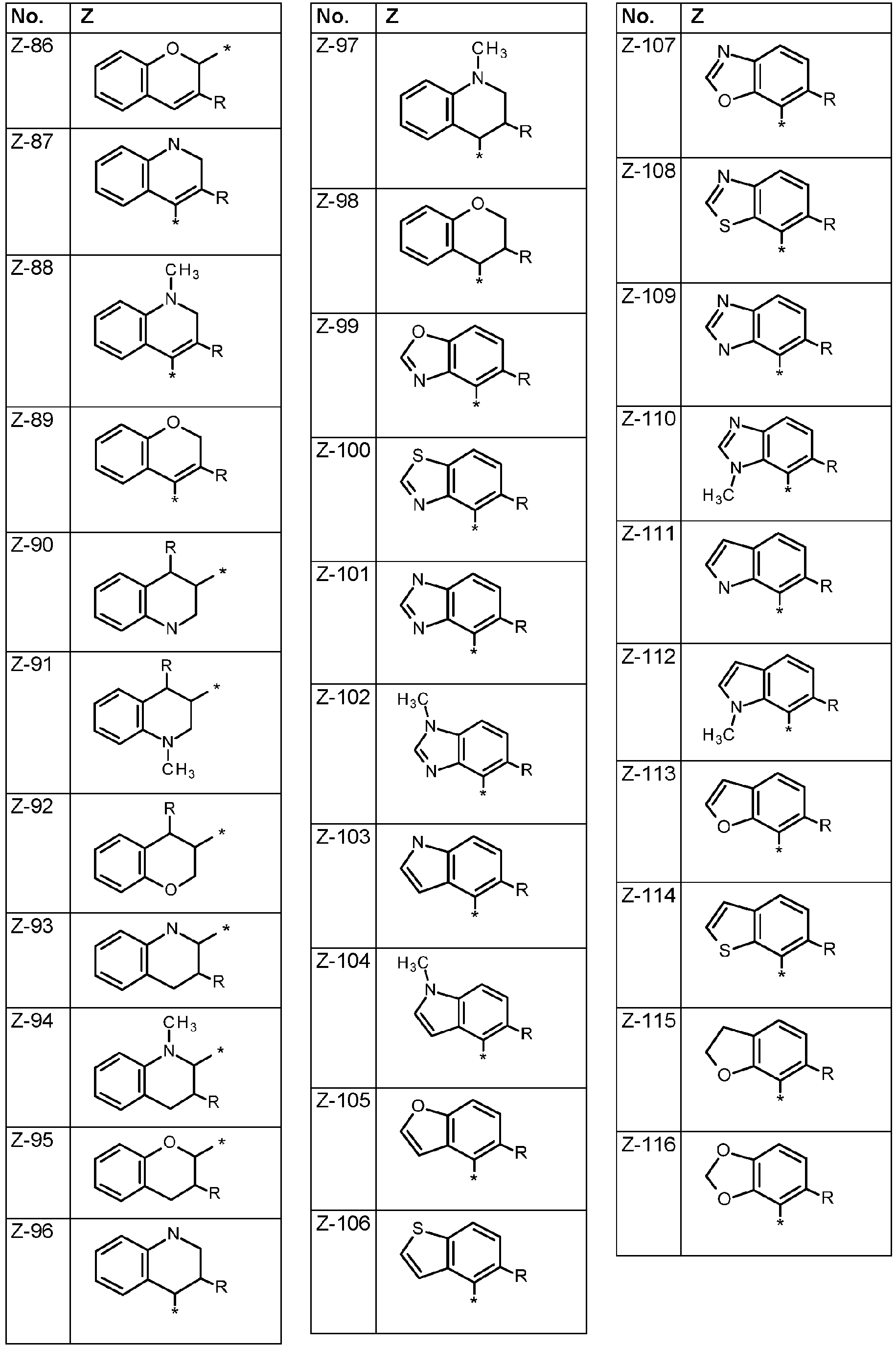

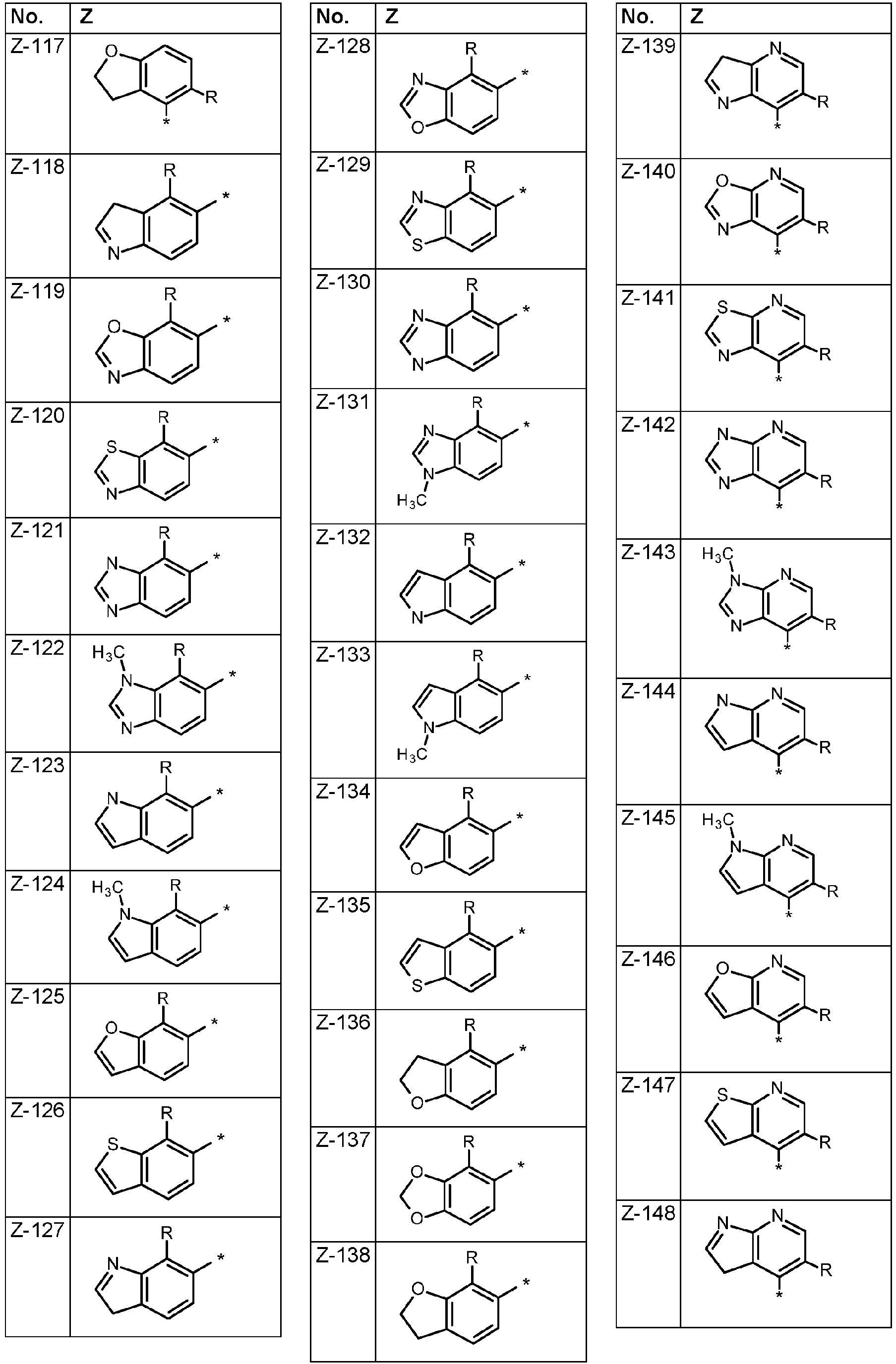

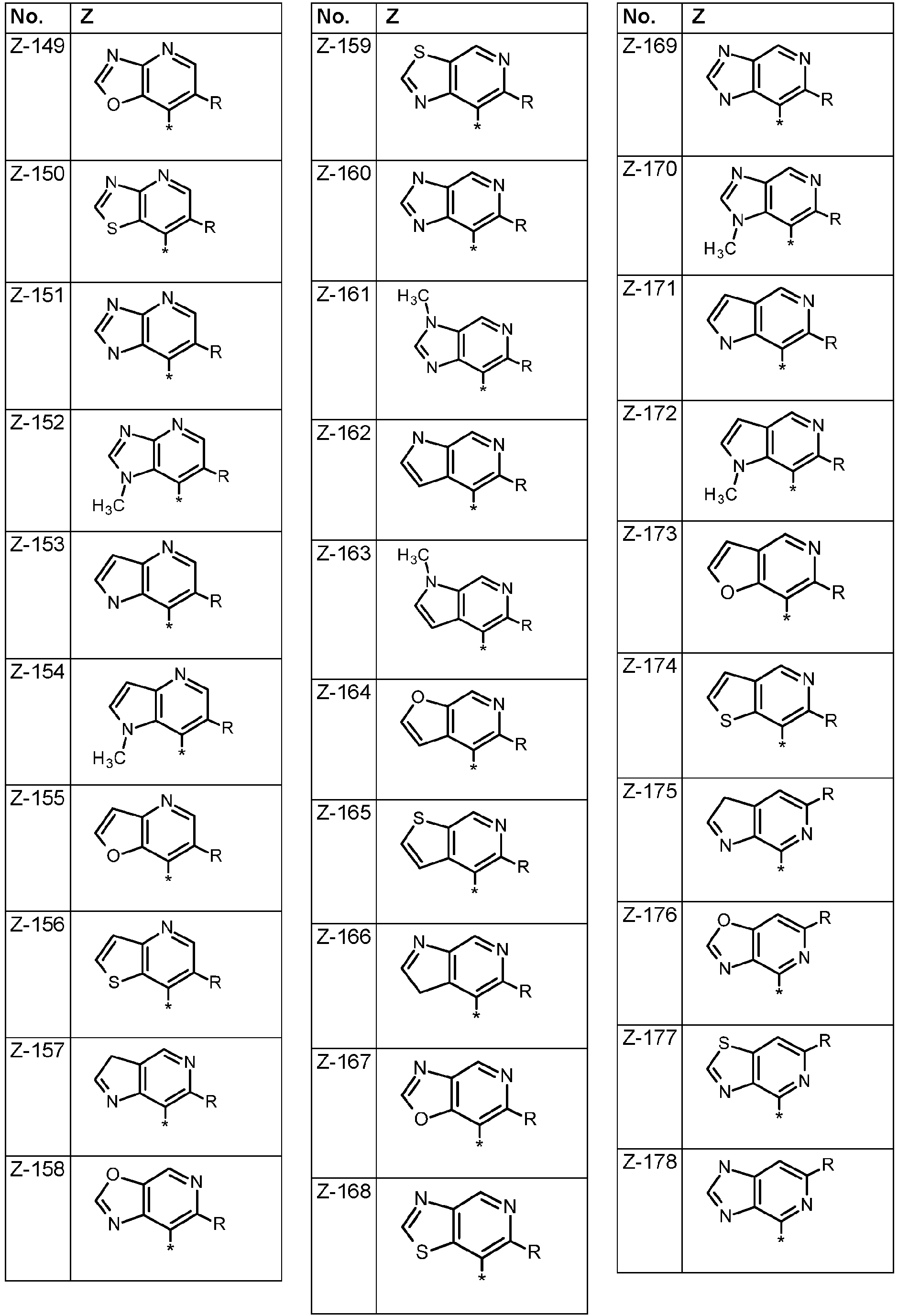

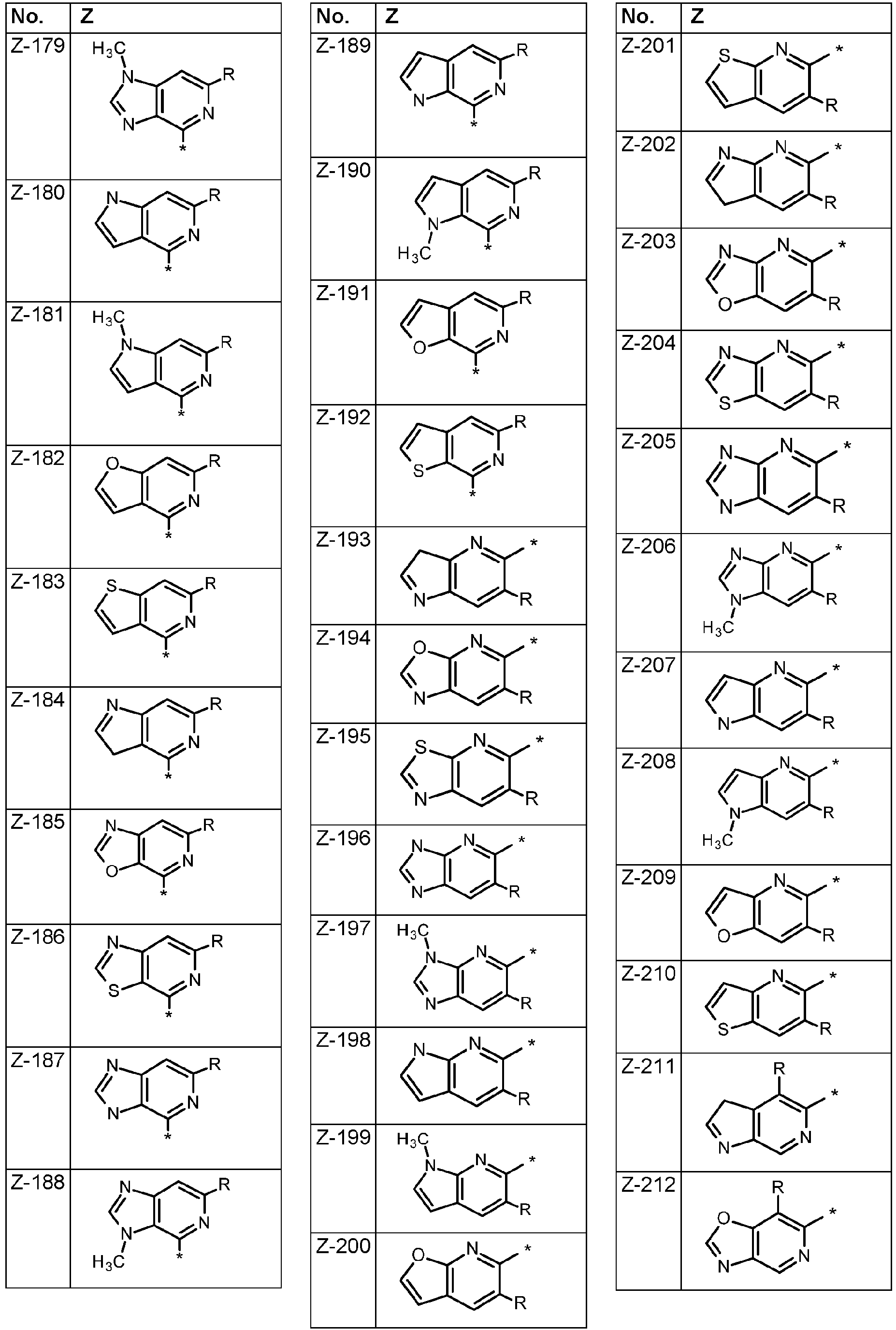

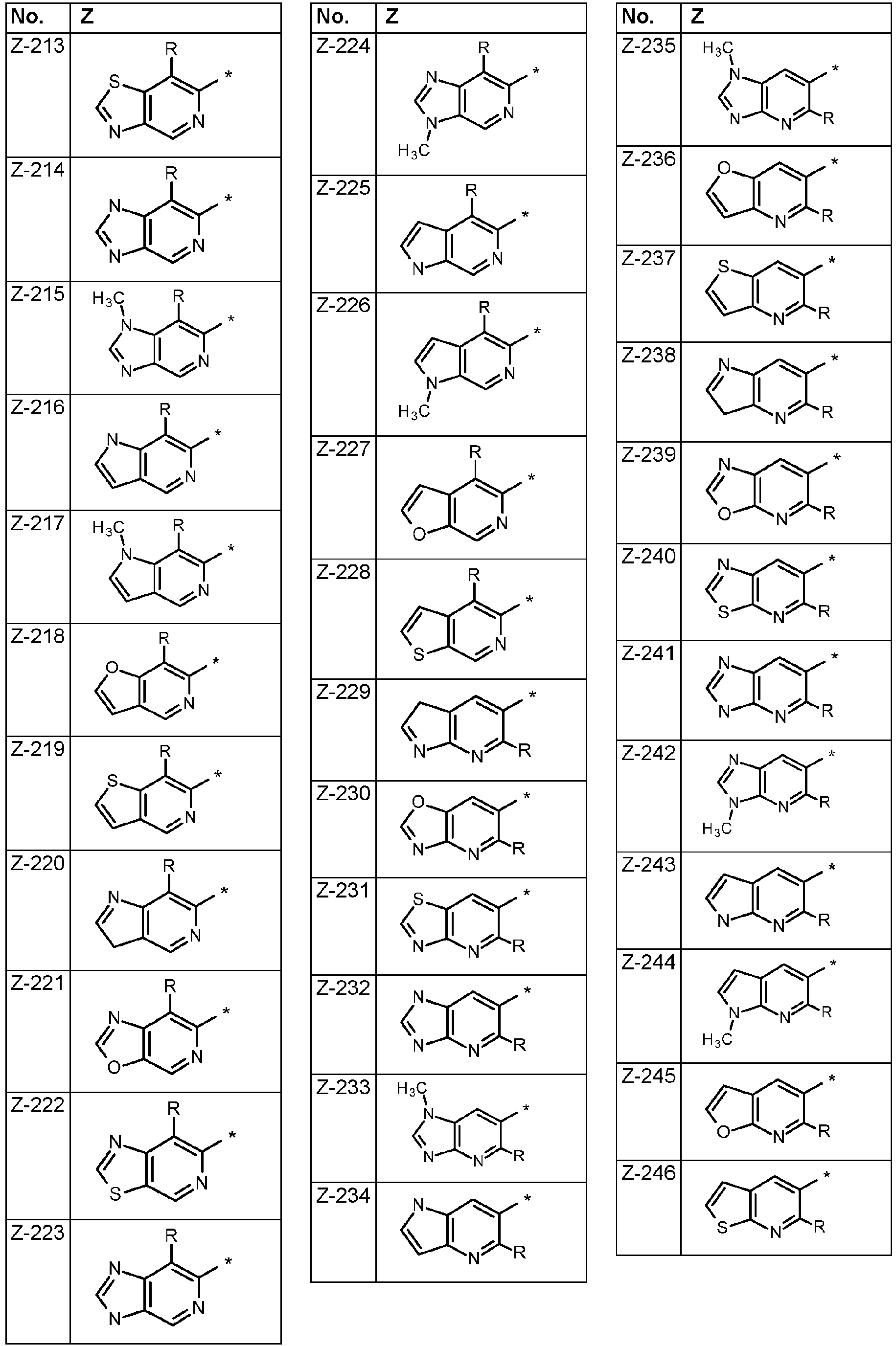

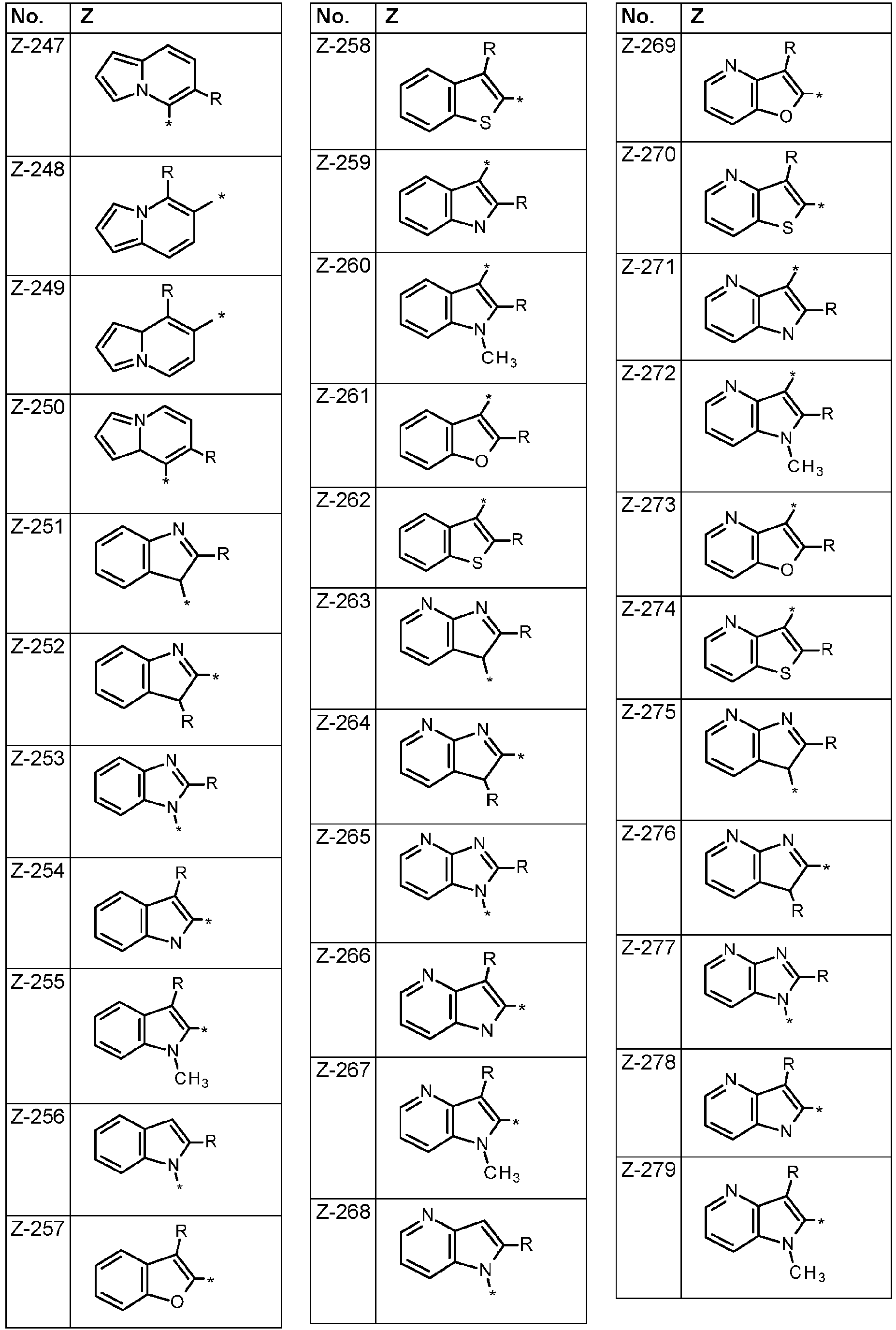

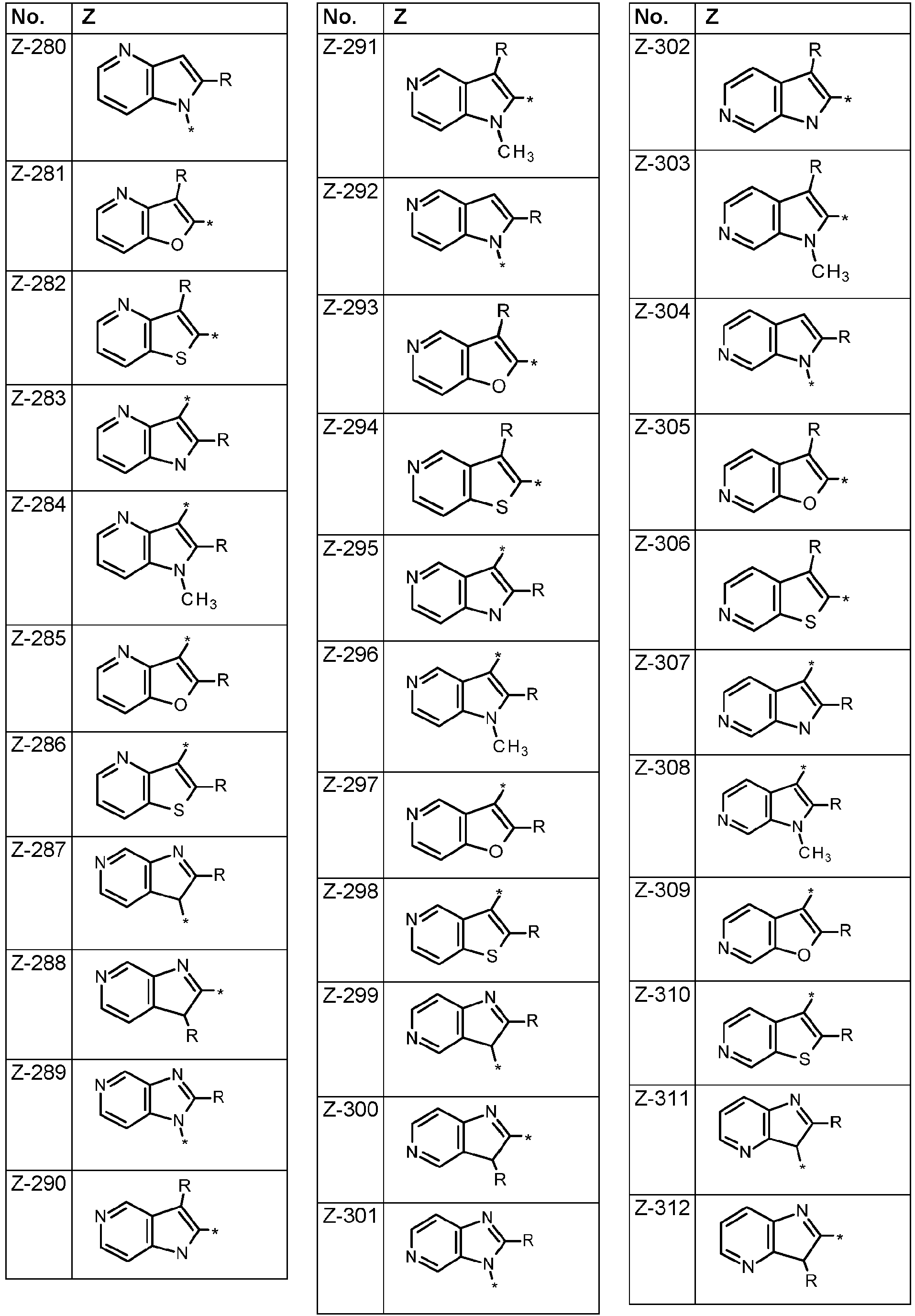

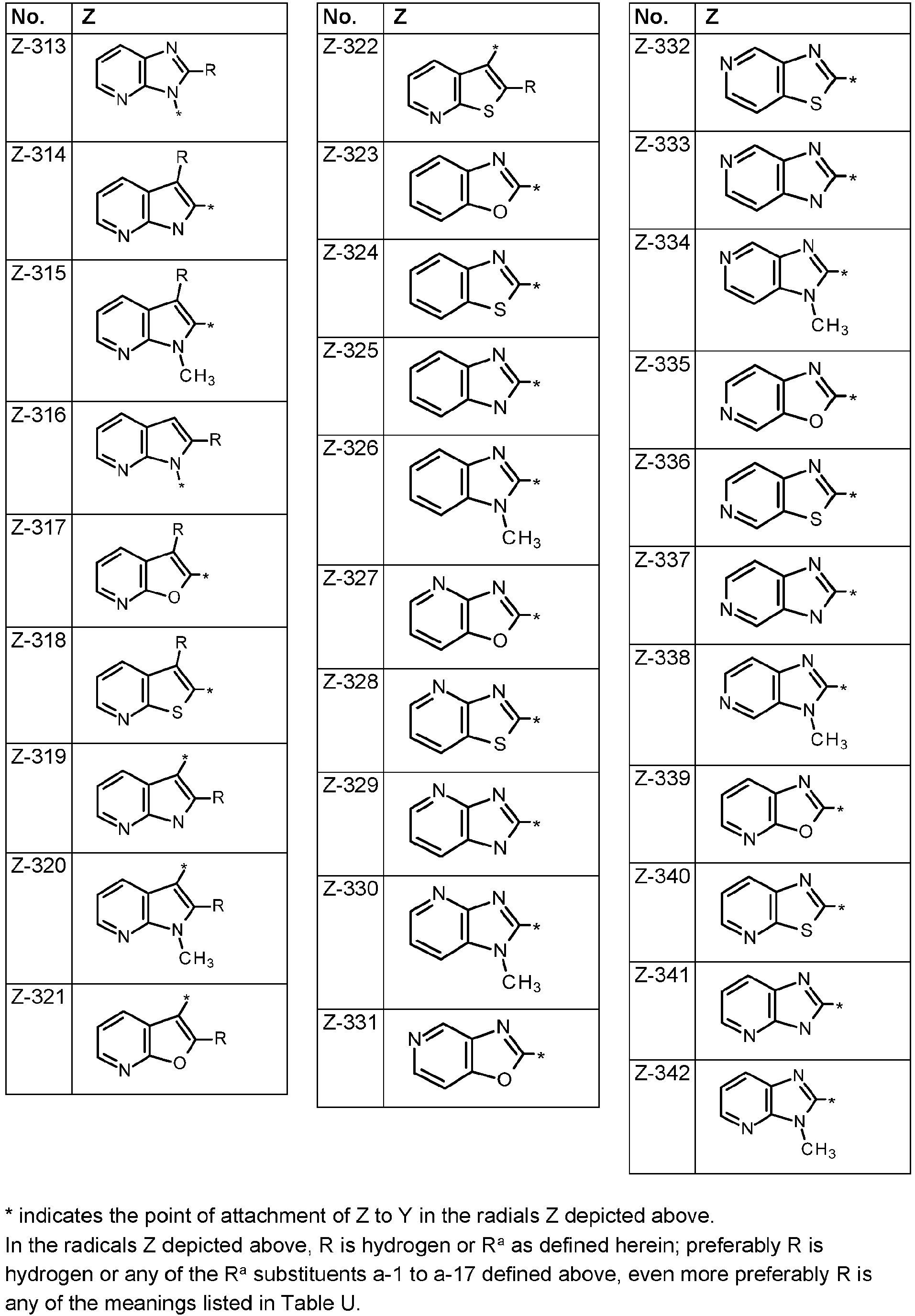

In an embodiment, compounds I are of formula I.A1 wherein R³ is CH₃, Q is Q1, and R^{a} and R⁴ are as per any row of Table Z below, which compounds are named I.A1-Q1-Z-1 to I.A1-Q1-Z-110.

In another embodiment, compounds I are of formula I.A1 wherein R³ is CH₃, Q is Q2, and R^{a} and R⁴ are as per any row of Table Z below, which compounds are named I.A1-Q2-Z-1 to I.A1-Q2-Z-110.

In another embodiment, compounds I are of formula I.A1 wherein R³ is CH₃, Q is Q3, and R^{a} and R⁴ are as per any row of Table Z below, which compounds are named I.A1-Q3-Z-1 to I.A1-Q3-Z-110.

In another embodiment, compounds I are of formula I.A1 wherein R³ is CH₃, Q is Q4, and R^{a} and R⁴ are as per any row of Table Z below, which compounds are named I.A1-Q4-Z-1 to I.A1-Q4-Z-110.

In another embodiment, compounds I are of formula I.A2 wherein R³ is CH₃, Q is Q1, and R^{a} and R⁴ are as per any row of Table Z below, which compounds are named I.A2-Q1-Z-1 to I.A2-Q1-Z-110.

In another embodiment, compounds I are of formula I.A2 wherein R³ is CH₃, Q is Q2, and R^{a} and R⁴ are as per any row of Table Z below, which compounds are named I.A2-Q2-Z-1 to I.A2-Q2-Z-110.

In another embodiment, compounds I are of formula I.A2 wherein R³ is CH₃, Q is Q3, and R^{a} and R⁴ are as per any row of Table Z below, which compounds are named I.A2-Q3-Z-1 to I.A2-Q3-Z-110.

In another embodiment, compounds I are of formula I.A2 wherein R³ is CH₃, Q is Q4, and R^{a} and R⁴ are as per any row of Table Z below, which compounds are named I.A2-Q4-Z-1 to I.A2-Q4-Z-110.

In another embodiment, compounds I are of formula I.B1 wherein Q is Q1, and R^{a} and R⁴ are as per any row of Table Z below, which compounds are named I.B1-Q1-Z-1 to I.B1-Q1-Z-110.

In another embodiment, compounds I are of formula I.B1 wherein Q is Q2, and R^{a} and R⁴ are as per any row of Table Z below, which compounds are named I.B1-Q2-Z-1 to I.B1-Q2-Z-110.

In another embodiment, compounds I are of formula I.B1 wherein Q is Q3, and R^{a} and R⁴ are as per any row of Table Z below, which compounds are named I.B1-Q3-Z-1 to I.B1-Q3-Z-110.

In another embodiment, compounds I are of formula I.B1 wherein Q is Q4, and R^{a} and R⁴ are as per any row of Table Z below, which compounds are named I.B1-Q4-Z-1 to I.B1-Q4-Z-110.

In another embodiment, compounds I are of formula I.B2 wherein Q is Q1, and R^{a} and R⁴ are as per any row of Table Z below, which compounds are named I.B2-Q1-Z-1 to I.B2-Q1-Z-110.

In another embodiment, compounds I are of formula I.B2 wherein Q is Q2, and R^{a} and R⁴ are as per any row of Table Z below, which compounds are named I.B2-Q2-Z-1 to I.B2-Q2-Z-110.

In another embodiment, compounds I are of formula I.B2 wherein Q is Q3, and R^{a} and R⁴ are as per any row of Table Z below, which compounds are named I.B2-Q3-Z-1 to I.B2-Q3-Z-110.

In another embodiment, compounds I are of formula I.B2 wherein Q is Q4, and R^{a} and R⁴ are as per any row of Table Z below, which compounds are named I.B2-Q4-Z-1 to I.B2-Q4-Z-110.

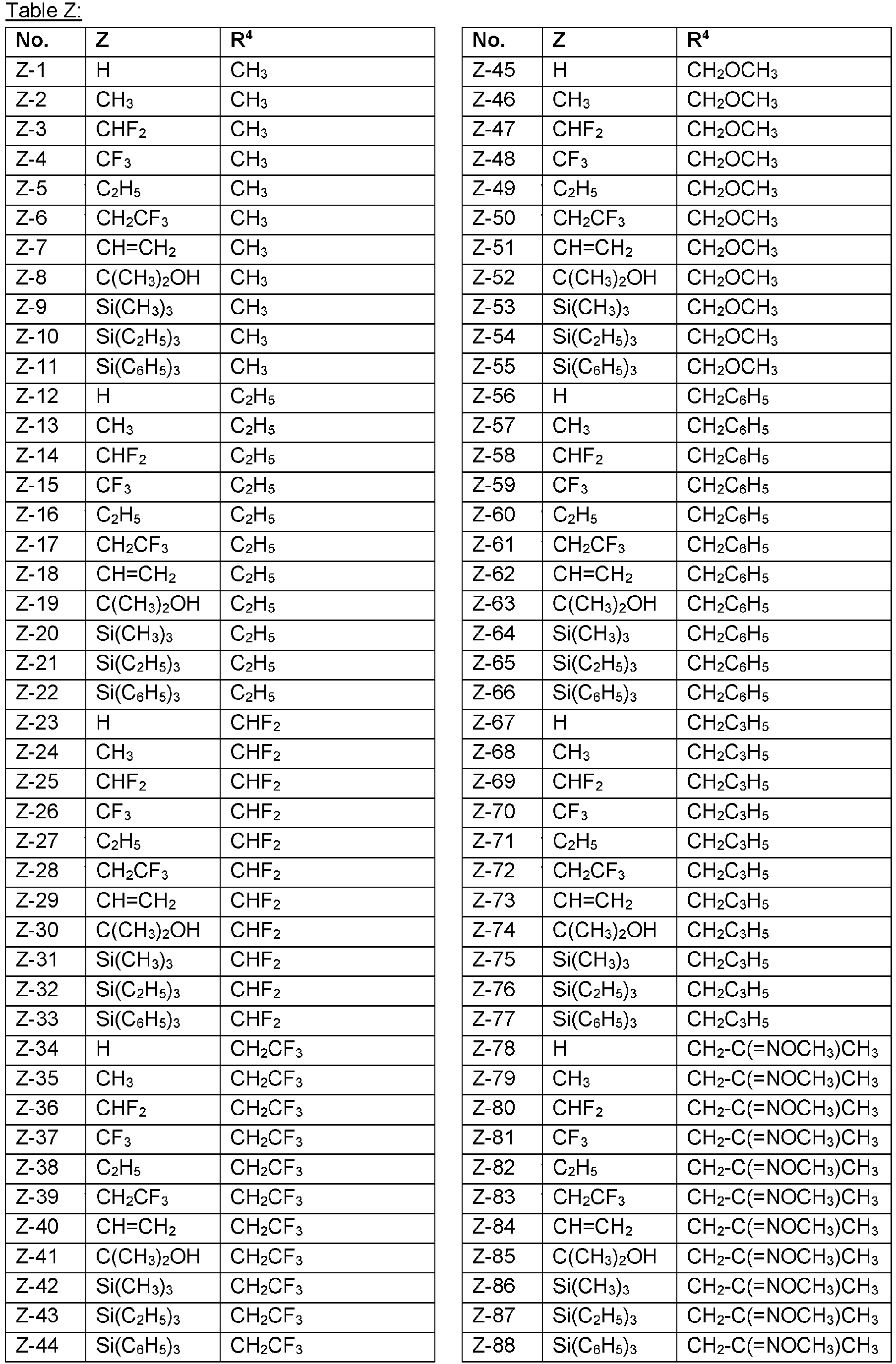

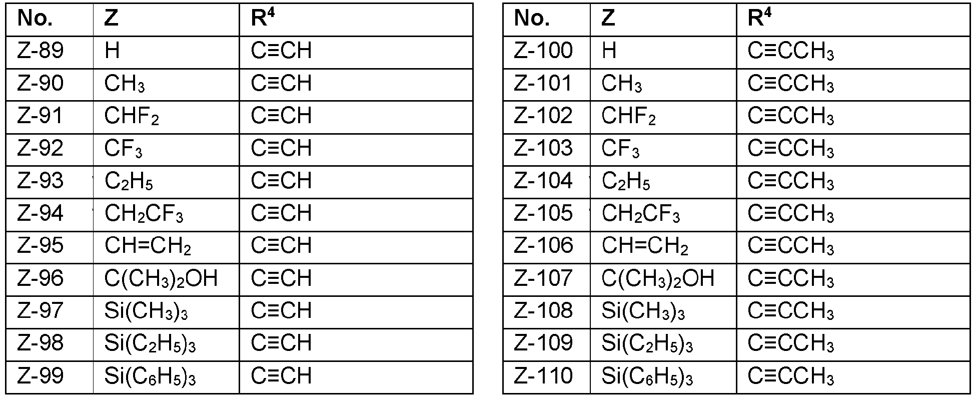

Particularly preferred embodiments of the invention relate to compounds I, wherein X and Y are direct bonds and Z is phenyl. Even more preferably, X and Y are direct bonds, Z is phenyl, R³ is in ortho position to the methyl oxime side chain, wherein the phenyl Z is unsubstituted or substituted by 1, 2 or 3 groups R^{a} as defined herein, which compounds are of formula I.A1.1:

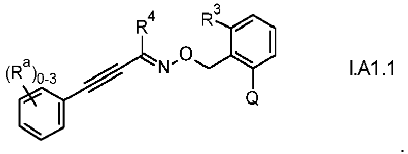

According to a further embodiment, X is a direct bond and Y is -CH₂-, Z is phenyl, R³ is in ortho position to the methyl oxime side chain, wherein the phenyl Z is unsubstituted or substituted by 1, 2 or 3 groups R^{a} as defined herein, which compounds are of formula I.A2.1:

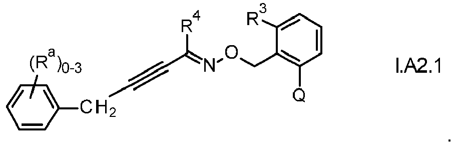

According ot a further embodiment, X and Y are direct bonds, R³ is H, Z is phenyl, wherein the phenyl Z is unsubstituted or substituted by 1, 2 or 3 groups R^{a} as defined herein, which compounds are of formula I.B1.1:

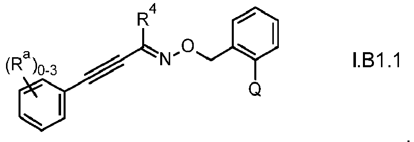

According to a further embodiment, R³ is H, X is a direct bond and Y is -CH₂-, Z is phenyl, wherein the phenyl Z is unsubstituted or substituted by 1, 2 or 3 groups R^{a} as defined herein, which compounds are of formula I.B2.1:

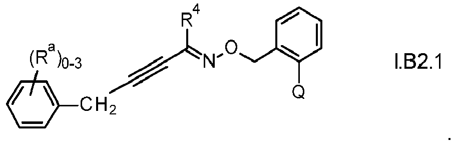

In an embodiment, compounds I are of formula I.A1.1 wherein R³ is CH₃ and Q is Q1, and R^{a} and R⁴ are as per any row of Table A below, which compounds are named I.A1.1-Q1-A-1 to I.A1.1-Q1-A-552.

In another embodiment, compounds I are of formula I.A1.1 wherein R³ is CH₃ and Q is Q2, and R^{a} and R⁴ are as per any row of Table A below, which compounds are named I.A1.1-Q2-A-1 to I.A1.1-Q2-A-552.

In another embodiment, compounds I are of formula I.A1.1 wherein R³ is CH₃ and Q is Q3, and R^{a} and R⁴ are as per any row of Table A below, which compounds are named I.A1.1-Q3-A-1 to I.A1.1-Q3-A-552.

In annother embodiment, compounds I are of formula I.A1.1 wherein R³ is CH₃ and Q is Q4, and R^{a} and R⁴ are as per any row of Table A below, which compounds are named I.A1.1-Q4-A-1 to I.A1.1-Q4-A-552.

In another embodiment, compounds I are of formula I.A2.1 wherein R³ is CH₃ and Q is Q1, and R^{a} and R⁴ are as per any row of Table A below, which compounds are named I.A1.1-Q1-A-1 to I.A2.1-Q1-A-552.

In another embodiment, compounds I are of formula I.A2.1 wherein R³ is CH₃ and Q is Q2, and R^{a} and R⁴ are as per any row of Table A below, which compounds are named I.A2.1-Q2-A-1 to I.A2.1-Q2-A-552.

In another embodiment, compounds I are of formula I.A2.1 wherein R³ is CH₃ and Q is Q3, and R^{a} and R⁴ are as per any row of Table A below, which compounds are named I.A2.1-Q3-A-1 to I.A1.1-Q3-A-552.

In another embodiment, compounds I are of formula I.A2.1 wherein R³ is CH₃ and Q is Q4, and R^{a} and R⁴ are as per any row of Table A below, which compounds are named I.A2.1-Q4-A-1 to I.A1.1-Q4-A-552.

In another embodiment, compounds I are of formula I.B1.1 wherein Q is Q1, and R^{a} and R⁴ are as per any row of Table A below, which compounds are named I.B1.1-Q1-A-1 to I.B1.1-Q1-A-552.

In another embodiment, compounds I are of formula I.B1.1 wherein Q is Q2, and R^{a} and R⁴ are as per any row of Table A below, which compounds are named I.B 1.1-Q2-A-1 to I.B 1.1-Q2-A-552.

In another embodiment, compounds I are of formula I.B1.1 wherein Q is Q3, and R^{a} and R⁴ are as per any row of Table A below, which compounds are named I.B 1.1-Q3-A-1 to I.B 1.1-Q3-A-552.

In annother embodiment, compounds I are of formula I.B1.1 wherein Q is Q4, and R^{a} and R⁴ are as per any row of Table A below, which compounds are named I.B 1.1-Q4-A-1 to I.B1.1-Q4-A-552.

In another embodiment, compounds I are of formula I.B2.1 wherein Q is Q1, and R^{a} and R⁴ are as per any row of Table A below, which compounds are named I.B1.1-Q1-A-1 to I.B2.1-Q1-A-552.

In another embodiment, compounds I are of formula I.B2.1 wherein Q is Q2, and R^{a} and R⁴ are as per any row of Table A below, which compounds are named I.B2.1-Q2-A-1 to I.B2.1-Q2-A-552.

In another embodiment, compounds I are of formula I.B2.1 wherein Q is Q3, and R^{a} and R⁴ are as per any row of Table A below, which compounds are named I.B2.1-Q3-A-1 to I.B1.1-Q3-A-552.

In another embodiment, compounds I are of formula I.B2.1 wherein Q is Q4, and R^{a} and R⁴ are as per any row of Table A below, which compounds are named I.B2.1-Q4-A-1 to I.B1.1-Q4-A-552.

In another embodiment, compounds I are of formula I.A1 wherein Z is cyclohexyl (Z-15), R³ is CH₃ and Q is Q1, and R^{a} and R⁴ are as per any row of Table A below, which compounds are named I.A1-Z15-Q1-A-1 to I.A1-Z15-Q1-A-552.

In another embodiment, compounds I are of formula I.A1 wherein Z is cyclohexyl (Z-15), R³ is CH₃ and Q is Q2, and R^{a} and R⁴ are as per any row of Table A below, which compounds are named I.A1-Z15-Q2-A-1 to I.A1-Z15-Q2-A-552.

In another embodiment, compounds I are of formula I.A1 wherein Z is cyclohexyl (Z-15), R³ is CH₃ and Q is Q3, and R^{a} and R⁴ are as per any row of Table A below, which compounds are named I.A1-Z15-Q3-A-1 to I.A1-Z15-Q3-A-552.

In another embodiment, compounds I are of formula I.A1 wherein Z is cyclohexyl (Z-15), R³ is CH₃ and Q is Q4, and R^{a} and R⁴ are as per any row of Table A below, which compounds are named I.A1-Z15-Q4-A-1 to I.A1-Z15-Q4-A-552.

In another embodiment, compounds I are of formula I.A2 wherein Z is cyclohexyl (Z-15), R³ is CH₃ and Q is Q1, and R^{a} and R⁴ are as per any row of Table A below, which compounds are named I.A2-Z15-Q1-A-1 to I.A2-Z15-Q1-A-552.

In another embodiment, compounds I are of formula I.A2 wherein Z is cyclohexyl (Z-15), R³ is CH₃ and Q is Q2, and R^{a} and R⁴ are as per any row of Table A below, which compounds are named I.A2-Z15-Q2-A-1 to I.A2-Z15-Q2-A-552.

In another embodiment, compounds I are of formula I.A2 wherein Z is cyclohexyl (Z-15), R³ is CH₃ and Q is Q3, and R^{a} and R⁴ are as per any row of Table A below, which compounds are named I.A2-Z15-Q3-A-1 to I.A2-Z15-Q3-A-552.

In another embodiment, compounds I are of formula I.A2 wherein Z is cyclohexyl (Z-15), R³ is CH₃ and Q is Q4, and R^{a} and R⁴ are as per any row of Table A below, which compounds are named I.A2-Z15-Q4-A-1 to I.A2-Z15-Q4-A-552.

In another embodiment, compounds I are of formula I.B1 wherein Z is cyclohexyl (Z-15) and Q is Q1, and R^{a} and R⁴ are as per any row of Table A below, which compounds are named I.B1-Z15-Q1-A-1 to I.B1-Z15-Q1-A-552.

In another embodiment, compounds I are of formula I.B1 wherein Z is cyclohexyl (Z-15) and Q is Q2, and R^{a} and R⁴ are as per any row of Table A below, which compounds are named I.B1-Z15-Q2-A-1 to I.B1-Z15-Q2-A-552.

In another embodiment, compounds I are of formula I.B1 wherein Z is cyclohexyl (Z-15) and Q is Q3, and R^{a} and R⁴ are as per any row of Table A below, which compounds are named I.B1-Z15-Q3-A-1 to I.B1-Z15-Q3-A-552.

In another embodiment, compounds I are of formula I.B1 wherein Z is cyclohexyl (Z-15) and Q is Q4, and R^{a} and R⁴ are as per any row of Table A below, which compounds are named I.B1-Z15-Q4-A-1 to I.B1-Z15-Q4-A-552.

In another embodiment, compounds I are of formula I.B2 wherein Z is cyclohexyl (Z-15) and Q is Q1, and R^{a} and R⁴ are as per any row of Table A below, which compounds are named I.B2-Z15-Q1-A-1 to I.B2-Z15-Q1-A-552.

In another embodiment, compounds I are of formula I.B2 wherein Z is cyclohexyl (Z-15) and Q is Q2, and R^{a} and R⁴ are as per any row of Table A below, which compounds are named I.B2-Z15-Q2-A-1 to I.B2-Z15-Q2-A-552.

In another embodiment, compounds I are of formula I.B2 wherein Z is cyclohexyl (Z-15) and Q is Q3, and R^{a} and R⁴ are as per any row of Table A below, which compounds are named I.B2-Z15-Q3-A-1 to I.B2-Z15-Q3-A-552.

In another embodiment, compounds I are of formula I.B2 wherein Z is cyclohexyl (Z-15) and Q is Q4, and R^{a} and R⁴ are as per any row of Table A below, which compounds are named I.B2-Z15-Q4-A-1 to I.B2-Z15-Q4-A-552.

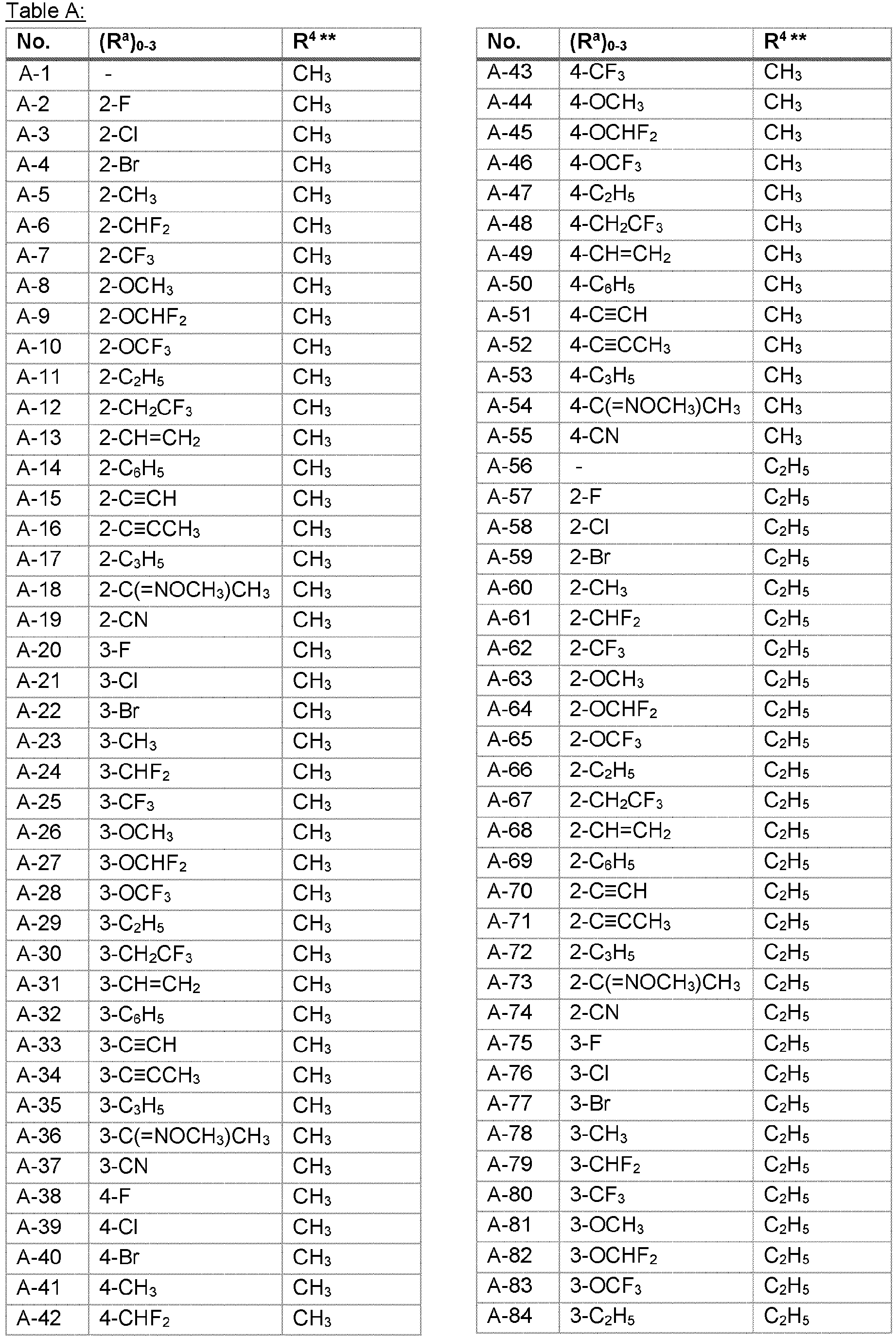

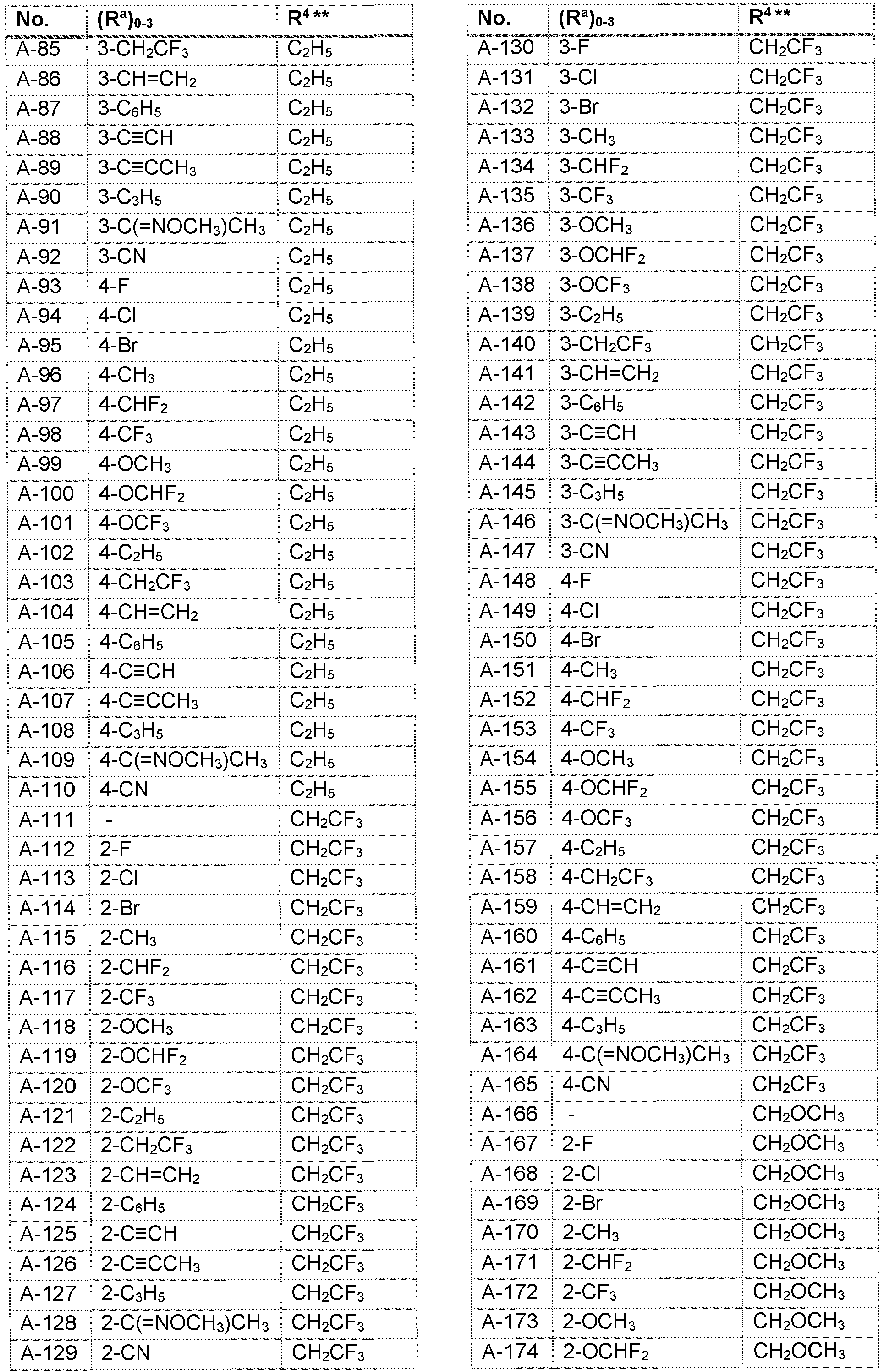

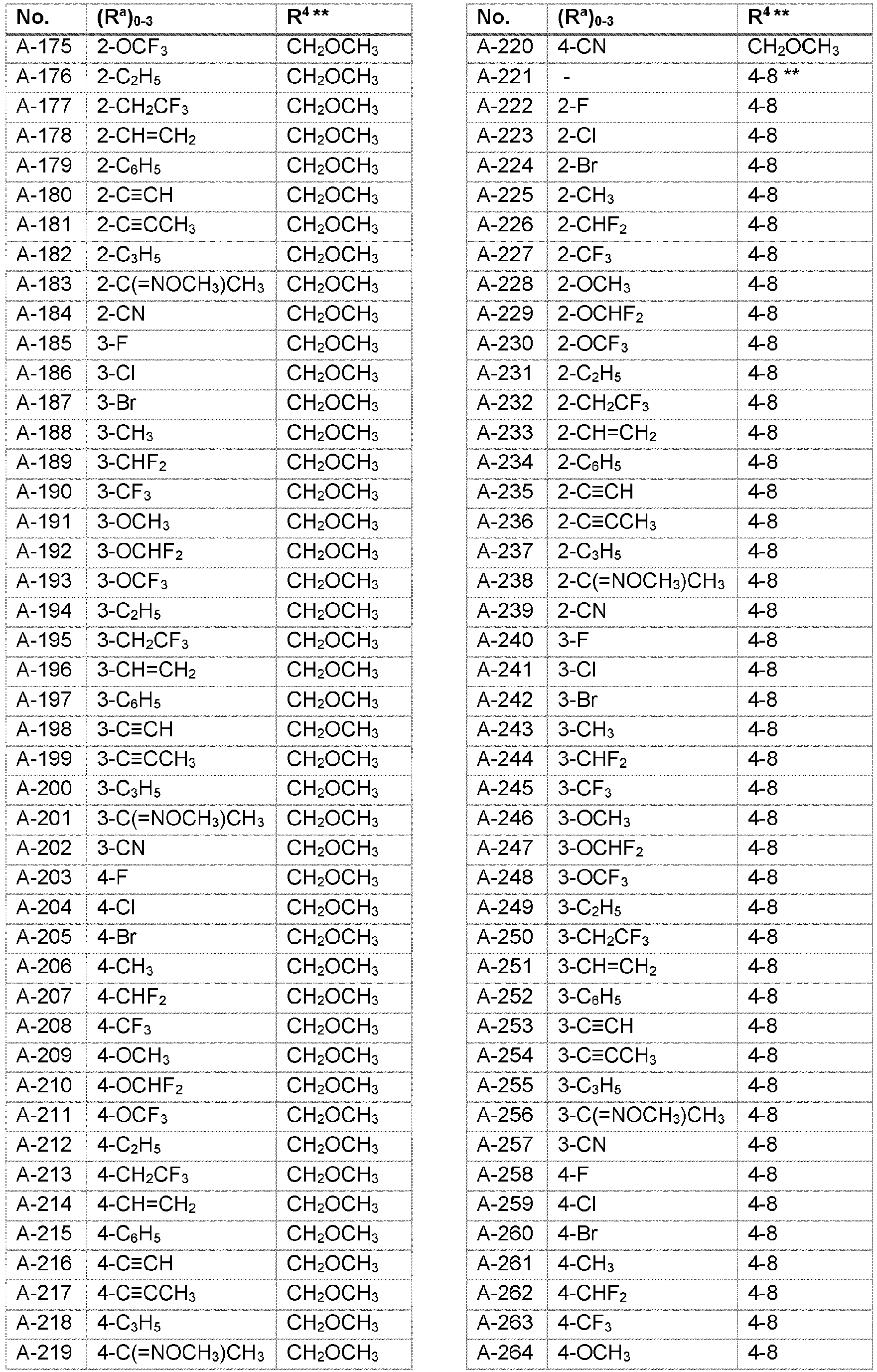

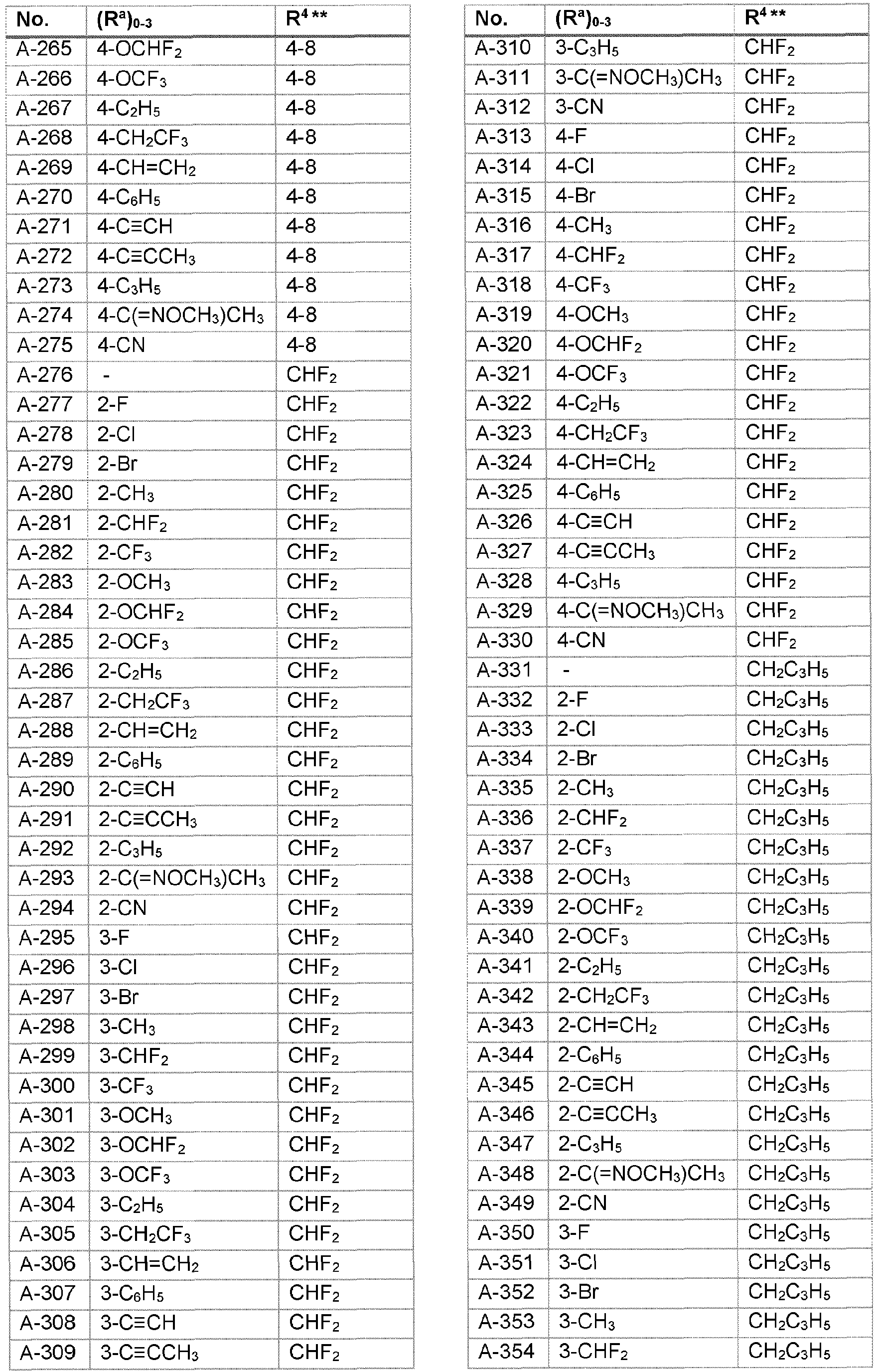

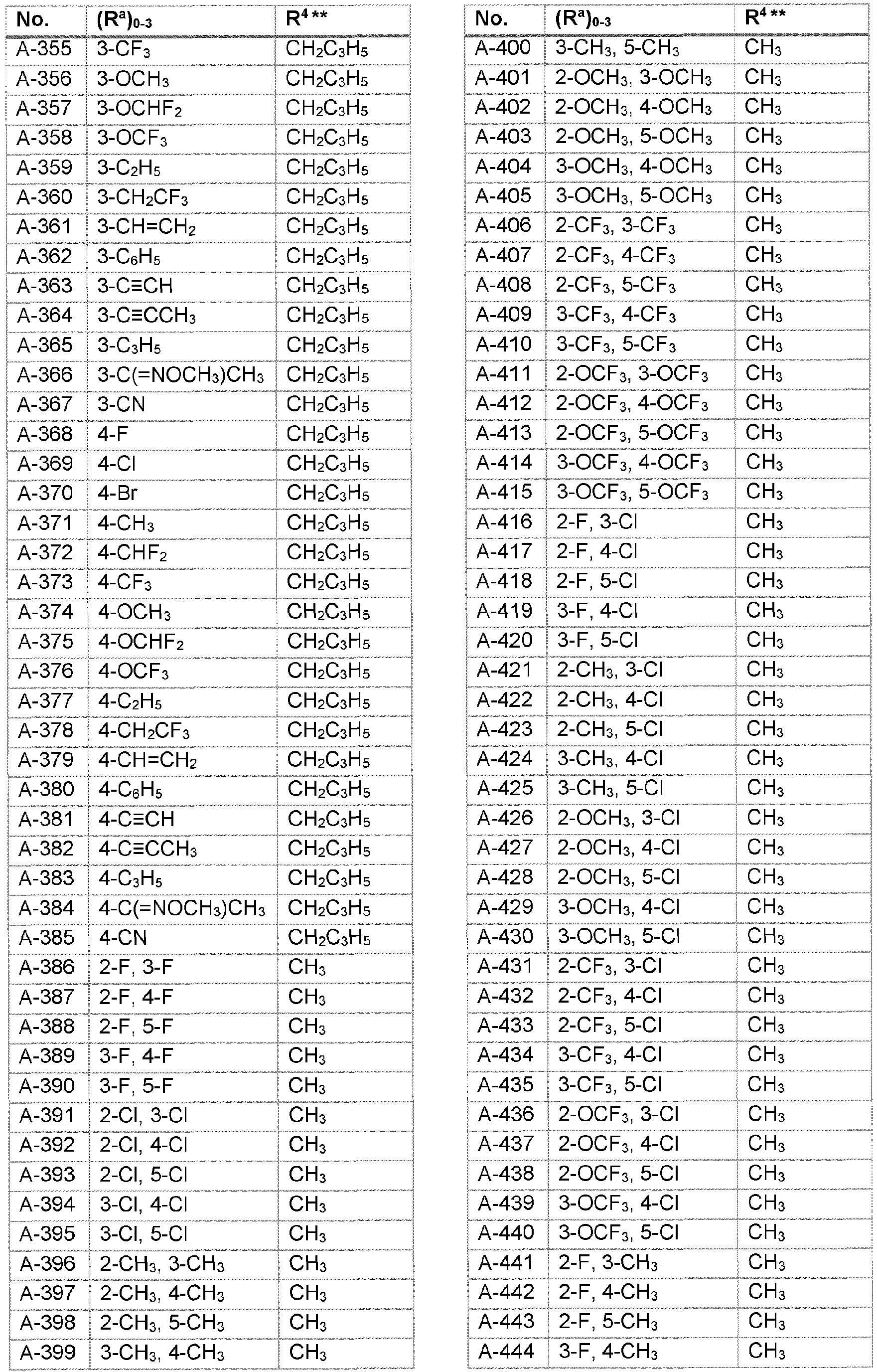

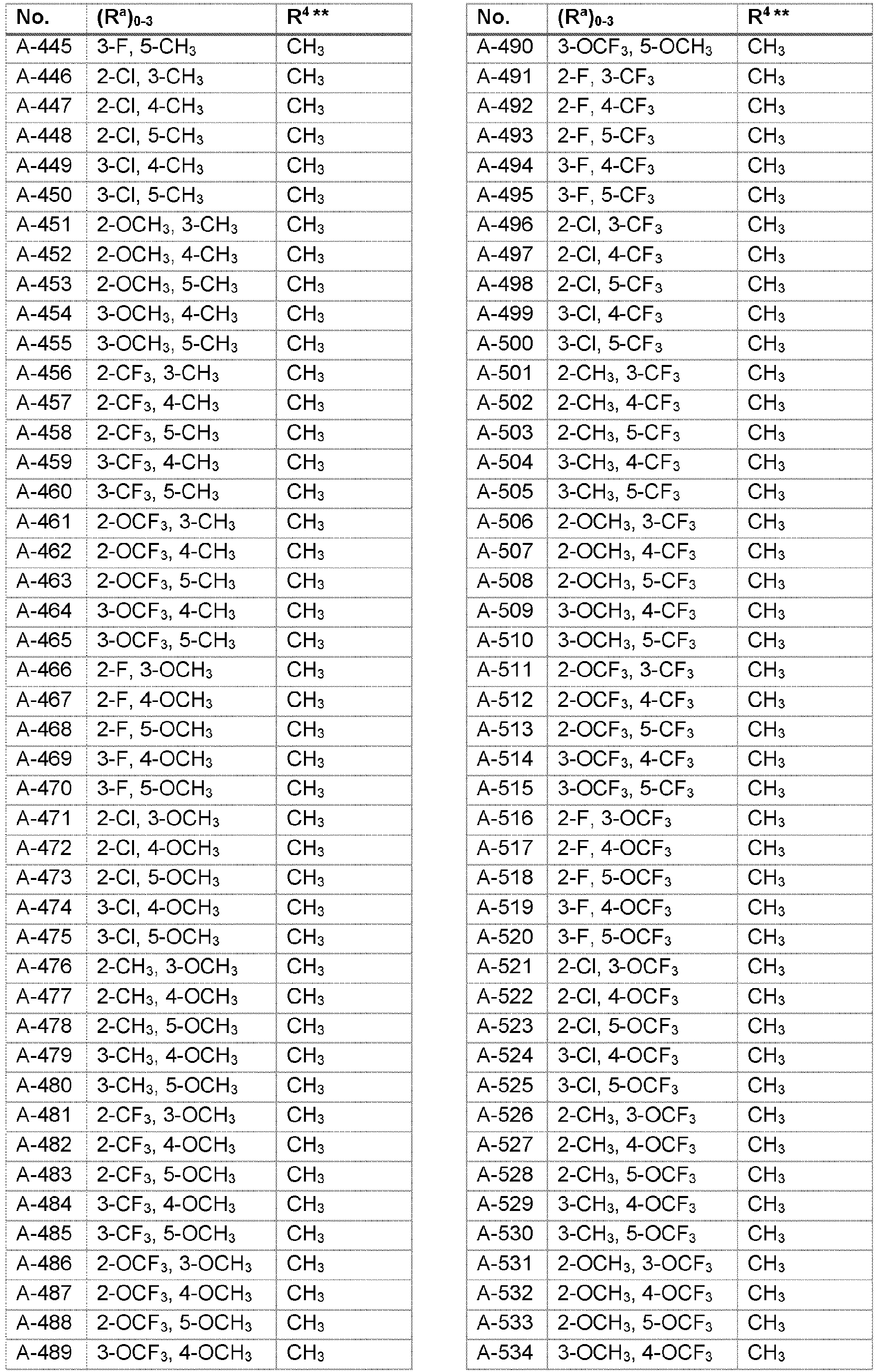

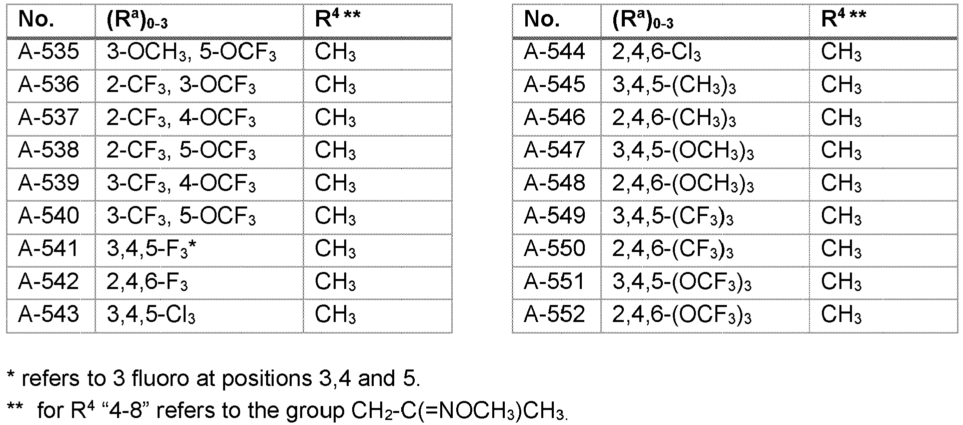

In another embodiment, compounds I are of formula I.A1 wherein Z is cyclopropyl (Z-13), R³ is CH₃ and Q is Q1, and R^{a} and R⁴ are as per any row of Table B below, which compounds are named I.A1-Z13-Q1-B-1 to I.A1-Z13-Q1-B-42.

In another embodiment, compounds I are of formula I.A1 wherein Z is cyclopropyl (Z-13), R³ is CH₃ and Q is Q2, and R^{a} and R⁴ are as per any row of Table B below, which compounds are named I.A1-Z13-Q2-B-1 to I.A1-Z13-Q2-B-42.

In another embodiment, compounds I are of formula I.A1 wherein Z is cyclopropyl (Z-13), R³ is CH₃ and Q is Q3, and R^{a} and R⁴ are as per any row of Table B below, which compounds are named I.A1-Z13-Q3-B-1 to I.A1-Z13-Q3-B-42.

In another embodiment, compounds I are of formula I.A1 wherein Z is cyclopropyl (Z-13), R³ is CH₃ and Q is Q4, and R^{a} and R⁴ are as per any row of Table B below, which compounds are named I.A1-Z13-Q4-B-1 to I.A1-Z13-Q4-B-42.

In another embodiment, compounds I are of formula I.A2 wherein Z is cyclopropyl (Z-13), R³ is CH₃ and Q is Q1, and R^{a} and R⁴ are as per any row of Table B below, which compounds are named I.A2-Z13-Q1-B-1 to I.A2-Z13-Q1-B-42.

In another embodiment, compounds I are of formula I.A2 wherein Z is cyclopropyl (Z-13), R³ is CH₃ and Q is Q2, and R^{a} and R⁴ are as per any row of Table B below, which compounds are named I.A2-Z13-Q2-B-1 to I.A2-Z13-Q2-B-42.

In another embodiment, compounds I are of formula I.A2 wherein Z is cyclopropyl (Z-13), R³ is CH₃ and Q is Q3, and R^{a} and R⁴ are as per any row of Table B below, which compounds are named I.A2-Z13-Q3-B-1 to I.A2-Z13-Q3-B-42.

In another embodiment, compounds I are of formula I.A2 wherein Z is cyclopropyl (Z-13), R³ is CH₃ and Q is Q4, and R^{a} and R⁴ are as per any row of Table B below, which compounds are named I.A2-Z13-Q4-B-1 to I.A2-Z13-Q4-B-42.

In another embodiment, compounds I are of formula I.B1 wherein Z is cyclopropyl (Z-13) and Q is Q1, and R^{a} and R⁴ are as per any row of Table B below, which compounds are named I.B1-Z13-Q1-B-1 to I.B1-Z13-Q1-B-42.

In another embodiment, compounds I are of formula I.B1 wherein Z is cyclopropyl (Z-13) and Q is Q2, and R^{a} and R⁴ are as per any row of Table B below, which compounds are named I.B1-Z13-Q2-B-1 to I.B1-Z13-Q2-B-42.

In another embodiment, compounds I are of formula I.B1 wherein Z is cyclopropyl (Z-13) and Q is Q3, and R^{a} and R⁴ are as per any row of Table B below, which compounds are named I.B1-Z13-Q3-B-1 to I.B1-Z13-Q3-B-42.

In another embodiment, compounds I are of formula I.B1 wherein Z is cyclopropyl (Z-13) and Q is Q4, and R^{a} and R⁴ are as per any row of Table B below, which compounds are named I.B1-Z13-Q4-B-1 to I.B1-Z13-Q4-B-42.

In another embodiment, compounds I are of formula I.B2 wherein Z is cyclopropyl (Z-13) and Q is Q1, and R^{a} and R⁴ are as per any row of Table B below, which compounds are named I.B2-Z13-Q1-B-1 to I.B2-Z13-Q1-B-42.

In another embodiment, compounds I are of formula I.B2 wherein Z is cyclopropyl (Z-13) and Q is Q2, and R^{a} and R⁴ are as per any row of Table B below, which compounds are named I.B2-Z13-Q2-B-1 to I.B2-Z13-Q2-B-42.

In another embodiment, compounds I are of formula I.B2 wherein Z is cyclopropyl (Z-13) and Q is Q3, and R^{a} and R⁴ are as per any row of Table B below, which compounds are named I.B2-Z13-Q3-B-1 to I.B2-Z13-Q3-B-42.

In another embodiment, compounds I are of formula I.B2 wherein Z is cyclopropyl (Z-13) and Q is Q4, and R^{a} and R⁴ are as per any row of Table B below, which compounds are named I.B2-Z13-Q4-B-1 to I.B2-Z13-Q4-B-42.

**Table B:**

| **No.** | **(R^{a})₀₋₁** | **R⁴** |
|---|---|---|
| B-1 | - | CH₃ |
| B-2 | 2-F | CH₃ |
| B-3 | 2-CI | CH₃ |
| B-4 | 2-CH₃ | CH₃ |
| B-5 | 2-CHF₂ | CH₃ |
| B-6 | 2-CF₃ | CH₃ |
| B-7 | 2-OCH₃ | CH₃ |
| B-8 | - | C₂H₅ |
| B-9 | 2-F | C₂H₅ |
| B-10 | 2-CI | C₂H₅ |
| B-11 | 2-CH₃ | C₂H₅ |
| B-12 | 2-CHF₂ | C₂H₅ |
| B-13 | 2-CF₃ | C₂H₅ |
| B-14 | 2-OCH₃ | C₂H₅ |
| B-15 | - | CHF₂ |
| B-16 | 2-F | CHF₂ |
| B-17 | 2-CI | CHF₂ |
| B-18 | 2-CH₃ | CHF₂ |
| B-19 | 2-CHF₂ | CHF₂ |
| B-20 | 2-CF₃ | CHF₂ |
| B-21 | 2-OCH₃ | CHF₂ |
| B-22 | - | CH₂CF₃ |
| B-23 | 2-F | CH₂CF₃ |
| B-24 | 2-CI | CH₂CF₃ |
| B-25 | 2-CH₃ | CH₂CF₃ |
| B-26 | 2-CHF₂ | CH₂CF₃ |
| B-27 | 2-CF₃ | CH₂CF₃ |
| B-28 | 2-OCH₃ | CH₂CF₃ |
| B-29 | - | CH₂C₃H₅ |
| B-30 | 2-F | CH₂C₃H₅ |
| B-31 | 2-CI | CH₂C₃H₅ |
| B-32 | 2-CH₃ | CH₂C₃H₅ |
| B-33 | 2-CHF₂ | CH₂C₃H₅ |
| B-34 | 2-CF₃ | CH₂C₃H₅ |
| B-35 | 2-OCH₃ | CH₂C₃H₅ |
| B-36 | - | CH₂OCH₃ |
| B-37 | 2-F | CH₂OCH₃ |
| B-38 | 2-CI | CH₂OCH₃ |
| B-39 | 2-CH₃ | CH₂OCH₃ |
| B-40 | 2-CHF₂ | CH₂OCH₃ |
| B-41 | 2-CF₃ | CH₂OCH₃ |
| B-42 | 2-OCH₃ | CH₂OCH₃ |

In another embodiment, compounds I are of formula I.A1 wherein Z is cyclopentyl (Z-14), R³ is CH₃ and Q is Q1, and R^{a} and R⁴ are as per any row of Table C below, which compounds are named I.A1-Z14-Q1-C-1 to I.A1-Z14-Q1-C-78.

In another embodiment, compounds I are of formula I.A1 wherein Z is cyclopentyl (Z-14), R³ is CH₃ and Q is Q2, and R^{a} and R⁴ are as per any row of Table C below, which compounds are named I.A1-Z14-Q2-C-1 to I.A1-Z14-Q2-C-78.

In another embodiment, compounds I are of formula I.A1 wherein Z is cyclopentyl (Z-14), R³ is CH₃ and Q is Q3, and R^{a} and R⁴ are as per any row of Table C below, which compounds are named I.A1-Z14-Q3-C-1 to I.A1-Z14-Q3-C-78.

In another embodiment, compounds I are of formula I.A1 wherein Z is cyclopentyl (Z-14), R³ is CH₃ and Q is Q4, and R^{a} and R⁴ are as per any row of Table C below, which compounds are named I.A1-Z14-Q4-C-1 to I.A1-Z14-Q4-C-78.

In another embodiment, compounds I are of formula I.A2 wherein Z is cyclopentyl (Z-14), R³ is CH₃ and Q is Q1, and R^{a} and R⁴ are as per any row of Table C below, which compounds are named I.A2-Z14-Q1-C-1 to I.A2-Z14-Q1-C-78.

In another embodiment, compounds I are of formula I.A2 wherein Z is cyclopentyl (Z-14), R³ is CH₃ and Q is Q2, and R^{a} and R⁴ are as per any row of Table C below, which compounds are named I.A2-Z14-Q2-C-1 to I.A2-Z14-Q2-C-78.

In another embodiment, compounds I are of formula I.A2 wherein Z is cyclopentyl (Z-14), R³ is CH₃ and Q is Q3, and R^{a} and R⁴ are as per any row of Table C below, which compounds are named I.A2-Z14-Q3-C-1 to I.A2-Z14-Q3-C-78.

In another embodiment, compounds I are of formula I.A2 wherein Z is cyclopentyl (Z-14), R³ is CH₃ and Q is Q4, and R^{a} and R⁴ are as per any row of Table C below, which compounds are named I.A2-Z14-Q4-C-1 to I.A2-Z14-Q4-C-78.

In another embodiment, compounds I are of formula I.B1 wherein Z is cyclopentyl (Z-14) and Q is Q1, and R^{a} and R⁴ are as per any row of Table C below, which compounds are named I.B1-Z14-Q1-C-1 to I.B1-Z14-Q1-C-78.

In another embodiment, compounds I are of formula I.B1 wherein Z is cyclopentyl (Z-14) and Q is Q2, and R^{a} and R⁴ are as per any row of Table C below, which compounds are named I.B1-Z14-Q2-C-1 to I.B1-Z14-Q2-C-78.

In another embodiment, compounds I are of formula I.B1 wherein Z is cyclopentyl (Z-14) and Q is Q3, and R^{a} and R⁴ are as per any row of Table C below, which compounds are named I.B1-Z14-Q3-C-1 to I.B1-Z14-Q3-C-78.

In another embodiment, compounds I are of formula I.B1 wherein Z is cyclopentyl (Z-14) and Q is Q4, and R^{a} and R⁴ are as per any row of Table C below, which compounds are named I.B1-Z14-Q4-C-1 to I.B1-Z14-Q4-C-78.

In another embodiment, compounds I are of formula I.B2 wherein Z is cyclopentyl (Z-14) and Q is Q1, and R^{a} and R⁴ are as per any row of Table C below, which compounds are named I.B2-Z14-Q1-C-1 to I.B2-Z14-Q1-C-78.

In another embodiment, compounds I are of formula I.B2 wherein Z is cyclopentyl (Z-14) and Q is Q2, and R^{a} and R⁴ are as per any row of Table C below, which compounds are named I.B2-Z14-Q2-C-1 to I.B2-Z14-Q2-C-78.

In another embodiment, compounds I are of formula I.B2 wherein Z is cyclopentyl (Z-14) and Q is Q3, and R^{a} and R⁴ are as per any row of Table C below, which compounds are named I.B2-Z14-Q3-C-1 to I.B2-Z14-Q3-C-78.

In another embodiment, compounds I are of formula I.B2 wherein Z is cyclopentyl (Z-14) and Q is Q4, and R^{a} and R⁴ are as per any row of Table C below, which compounds are named I.B2-Z14-Q4-C-1 to I.B2-Z14-Q4-C-78.

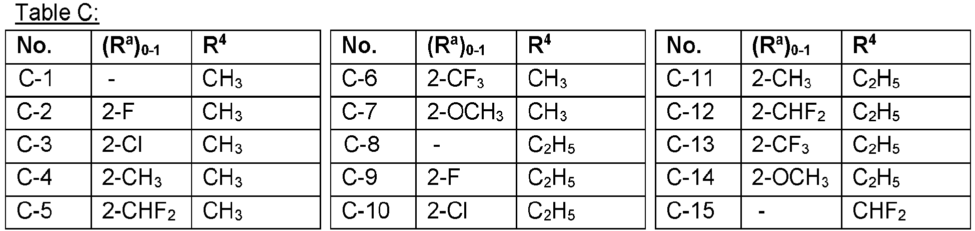

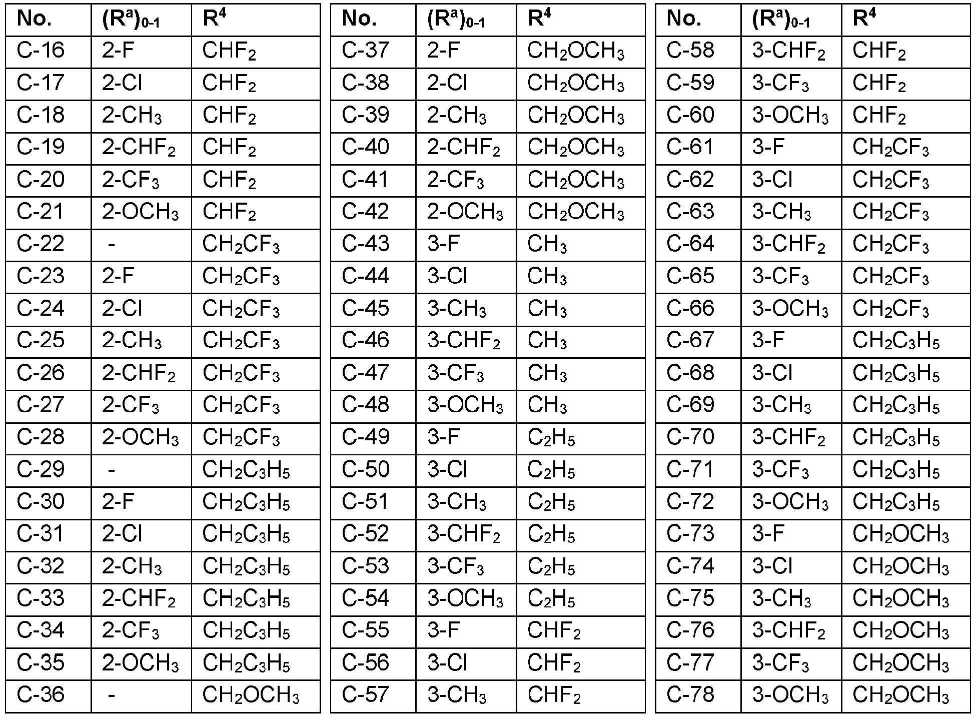

In another embodiment, compounds I are of formula I.A1 wherein Z is 1,2,4-triazol-5-yl (Z-42), R³ is CH₃ and Q is Q1, and R^{a} and R⁴ are as per any row of Table D below, which compounds are named I.A1-Z42-Q1-D-1 to I.A1-Z42-Q1-D-78.

In another embodiment, compounds I are of formula I.A1 wherein Z is 1,2,4-triazol-5-yl (Z-42), R³ is CH₃ and Q is Q2, and R^{a} and R⁴ are as per any row of Table D below, which compounds are named I.A1-Z42-Q2-D-1 to I.A1-Z42-Q2-D-78.

In another embodiment, compounds I are of formula I.A1 wherein Z is 1,2,4-triazol-5-yl (Z-42), R³ is CH₃ and Q is Q3, and R^{a} and R⁴ are as per any row of Table D below, which compounds are named I.A1-Z42-Q3-D-1 to I.A1-Z42-Q3-D-78.

In another embodiment, compounds I are of formula I.A1 wherein Z is 1,2,4-triazol-5-yl (Z-42), R³ is CH₃ and Q is Q4, and R^{a} and R⁴ are as per any row of Table D below, which compounds are named I.A1-Z42-Q4-D-1 to I.A1-Z42-Q4-D-78.

In another embodiment, compounds I are of formula I.A2 wherein Z is 1,2,4-triazol-5-yl (Z-42), R³ is CH₃ and Q is Q1, and R^{a} and R⁴ are as per any row of Table D below, which compounds are named I.A2-Z42-Q1-D-1 to I.A2-Z42-Q1-D-78.

In another embodiment, compounds I are of formula I.A2 wherein Z is 1,2,4-triazol-5-yl (Z-42), R³ is CH₃ and Q is Q2, and R^{a} and R⁴ are as per any row of Table D below, which compounds are named I.A2-Z42-Q2-D-1 to I.A2-Z42-Q2-D-78.

In another embodiment, compounds I are of formula I.A2 wherein Z is 1,2,4-triazol-5-yl (Z-42), R³ is CH₃ and Q is Q3, and R^{a} and R⁴ are as per any row of Table D below, which compounds are named I.A2-Z42-Q3-D-1 to I.A2-Z42-Q3-D-78.

In another embodiment, compounds I are of formula I.A2 wherein Z is 1,2,4-triazol-5-yl (Z-42), R³ is CH₃ and Q is Q4, and R^{a} and R⁴ are as per any row of Table D below, which compounds are named I.A2-Z42-Q4-D-1 to I.A2-Z42-Q4-D-78.

In another embodiment, compounds I are of formula I.A1 wherein Z is 1-CH₃-1,2,4-triazol-5-yl (Z-44), R³ is CH₃ and Q is Q1, and R^{a} and R⁴ are as per any row of Table D below, which compounds are named I.A1-Z44-Q1-D-1 to I.A1-Z44-Q1-D-78.

In another embodiment, compounds I are of formula I.A1 wherein Z is 1-CH₃-1,2,4-triazol-5-yl (Z-44), R³ is CH₃ and Q is Q2, and R^{a} and R⁴ are as per any row of Table D below, which compounds are named I.A1-Z44-Q2-D-1 to I.A1-Z44-Q2-D-78.

In another embodiment, compounds I are of formula I.A1 wherein Z is 1-CH₃-1,2,4-triazol-5-yl (Z-44), R³ is CH₃ and Q is Q3, and R^{a} and R⁴ are as per any row of Table D below, which compounds are named I.A1-Z44-Q3-D-1 to I.A1-Z44-Q3-D-78.

In another embodiment, compounds I are of formula I.A1 wherein Z is 1-CH₃-1,2,4-triazol-5-yl (Z-44), R³ is CH₃ and Q is Q4, and R^{a} and R⁴ are as per any row of Table D below, which compounds are named I.A1-Z44-Q4-D-1 to I.A1-Z44-Q4-D-78.

In another embodiment, compounds I are of formula I.A2 wherein Z is 1-CH₃-1,2,4-triazol-5-yl (Z-44), R³ is CH₃ and Q is Q1, and R^{a} and R⁴ are as per any row of Table D below, which compounds are named I.A2-Z44-Q1-D-1 to I.A2-Z44-Q1-D-78.

In another embodiment, compounds I are of formula I.A2 wherein Z is 1-CH₃-1,2,4-triazol-5-yl (Z-44), R³ is CH₃ and Q is Q2, and R^{a} and R⁴ are as per any row of Table D below, which compounds are named I.A2-Z44-Q2-D-1 to I.A2-Z44-Q2-D-78.

In another embodiment, compounds I are of formula I.A2 wherein Z is 1-CH₃-1,2,4-triazol-5-yl (Z-44), R³ is CH₃ and Q is Q3, and R^{a} and R⁴ are as per any row of Table D below, which compounds are named I.A2-Z44-Q3-D-1 to I.A2-Z44-Q3-D-78.

In another embodiment, compounds I are of formula I.A2 wherein Z is 1-CH₃-1,2,4-triazol-5-yl (Z-44), R³ is CH₃ and Q is Q4, and R^{a} and R⁴ are as per any row of Table D below, which compounds are named I.A2-Z44-Q4-D-1 to I.A2-Z44-Q4-D-78.

In another embodiment, compounds I are of formula I.B1 wherein Z is 1,2,4-triazol-5-yl (Z-42) and Q is Q1, and R^{a} and R⁴ are as per any row of Table D below, which compounds are named I.B1-Z42-Q1-D-1 to I.B1-Z42-Q1-D-78.

In another embodiment, compounds I are of formula I.B1 wherein Z is 1,2,4-triazol-5-yl (Z-42) and Q is Q2, and R^{a} and R⁴ are as per any row of Table D below, which compounds are named I.B1-Z42-Q2-D-1 to I.B1-Z42-Q2-D-78.

In another embodiment, compounds I are of formula I.B1 wherein Z is 1,2,4-triazol-5-yl (Z-42) and Q is Q3, and R^{a} and R⁴ are as per any row of Table D below, which compounds are named I.B1-Z42-Q3-D-1 to I.B1-Z42-Q3-D-78.

In another embodiment, compounds I are of formula I.B1 wherein Z is 1,2,4-triazol-5-yl (Z-42) and Q is Q4, and R^{a} and R⁴ are as per any row of Table D below, which compounds are named I.B1-Z42-Q4-D-1 to I.B1-Z42-Q4-D-78.

In another embodiment, compounds I are of formula I.B2 wherein Z is 1,2,4-triazol-5-yl (Z-42) and Q is Q1, and R^{a} and R⁴ are as per any row of Table D below, which compounds are named I.B2-Z42-Q1-D-1 to I.B2-Z42-Q1-D-78.

In another embodiment, compounds I are of formula I.B2 wherein Z is 1,2,4-triazol-5-yl (Z-42) and Q is Q2, and R^{a} and R⁴ are as per any row of Table D below, which compounds are named I.B2-Z42-Q2-D-1 to I.B2-Z42-Q2-D-78.

In another embodiment, compounds I are of formula I.B2 wherein Z is 1,2,4-triazol-5-yl (Z-42) and Q is Q3, and R^{a} and R⁴ are as per any row of Table D below, which compounds are named I.B2-Z42-Q3-D-1 to I.B2-Z42-Q3-D-78.

In another embodiment, compounds I are of formula I.B2 wherein Z is 1,2,4-triazol-5-yl (Z-42) and Q is Q4, and R^{a} and R⁴ are as per any row of Table D below, which compounds are named I.B2-Z42-Q4-D-1 to I.B2-Z42-Q4-D-78.

In another embodiment, compounds I are of formula I.B1 wherein Z is 1-CH₃-1,2,4-triazol-5-yl (Z-44) and Q is Q1, and R^{a} and R⁴ are as per any row of Table D below, which compounds are named I.B1-Z44-Q1-D-1 to I.B1-Z44-Q1-D-78.

In another embodiment, compounds I are of formula I.B1 wherein Z is 1-CH₃-1,2,4-triazol-5-yl (Z-44) and Q is Q2, and R^{a} and R⁴ are as per any row of Table D below, which compounds are named I.B1-Z44-Q2-D-1 to I.B1-Z44-Q2-D-78.

In another embodiment, compounds I are of formula I.B1 wherein Z is 1-CH₃-1,2,4-triazol-5-yl (Z-44) and Q is Q3, and R^{a} and R⁴ are as per any row of Table D below, which compounds are named I.B1-Z44-Q3-D-1 to I.B1-Z44-Q3-D-78.

In another embodiment, compounds I are of formula I.B1 wherein Z is 1-CH₃-1,2,4-triazol-5-yl (Z-44) and Q is Q4, and R^{a} and R⁴ are as per any row of Table D below, which compounds are named I.B1-Z44-Q4-D-1 to I.B1-Z44-Q4-D-78.

In another embodiment, compounds I are of formula I.A2 wherein Z is 1-CH₃-1,2,4-triazol-5-yl (Z-44) and Q is Q1, and R^{a} and R⁴ are as per any row of Table D below, which compounds are named I.A2-Z44-Q1-D-1 to I.A2-Z44-Q1-D-78.

In another embodiment, compounds I are of formula I.A2 wherein Z is 1-CH₃-1,2,4-triazol-5-yl (Z-44) and Q is Q2, and R^{a} and R⁴ are as per any row of Table D below, which compounds are named I.A2-Z44-Q2-D-1 to I.A2-Z44-Q2-D-78.

In another embodiment, compounds I are of formula I.A2 wherein Z is 1-CH₃-1,2,4-triazol-5-yl (Z-44) and Q is Q3, and R^{a} and R⁴ are as per any row of Table D below, which compounds are named I.A2-Z44-Q3-D-1 to I.A2-Z44-Q3-D-78.

In another embodiment, compounds I are of formula I.A2 wherein Z is 1-CH₃-1,2,4-triazol-5-yl (Z-44) and Q is Q4, and R^{a} and R⁴ are as per any row of Table D below, which compounds are named I.A2-Z44-Q4-D-1 to I.A2-Z44-Q4-D-78.

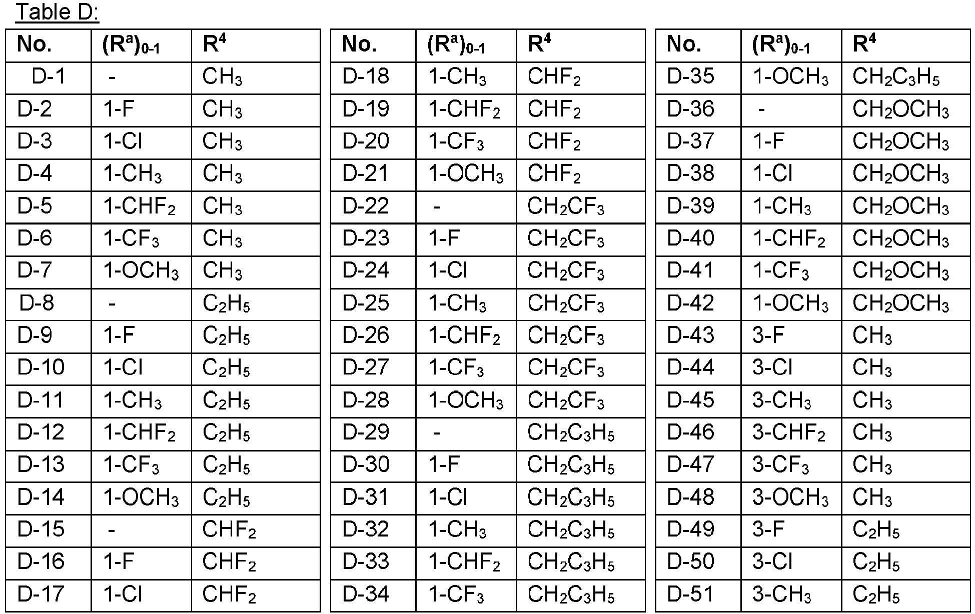

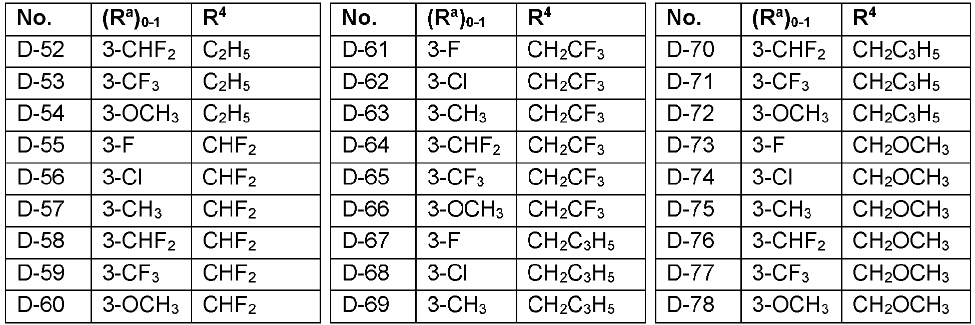

In another embodiment, compounds I are of formula I.A1 wherein Z is 1,2,4-triazol-3-yl (Z-41), R³ is CH₃ and Q is Q1, and R^{a} and R⁴ are as per any row of Table E below, which compounds are named I.A1-Z41-Q1-E-1 to I.A1-Z41-Q1-E-78.

In another embodiment, compounds I are of formula I.A1 wherein Z is 1,2,4-triazol-3-yl (Z-41), R³ is CH₃ and Q is Q2, and R^{a} and R⁴ are as per any row of Table E below, which compounds are named I.A1-Z41-Q2-E-1 to I.A1-Z41-Q2-E-78.

In another embodiment, compounds I are of formula I.A1 wherein Z is 1,2,4-triazol-3-yl (Z-41), R³ is CH₃ and Q is Q3, and R^{a} and R⁴ are as per any row of Table E below, which compounds are named I.A1-Z41-Q3-E-1 to I.A1-Z41-Q3-E-78.

In another embodiment, compounds I are of formula I.A1 wherein Z is 1,2,4-triazol-3-yl (Z-41), R³ is CH₃ and Q is Q4, and R^{a} and R⁴ are as per any row of Table E below, which compounds are named I.A1-Z41-Q4-E-1 to I.A1-Z41-Q4-E-78.

In another embodiment, compounds I are of formula I.A2 wherein Z is 1,2,4-triazol-3-yl (Z-41), R³ is CH₃ and Q is Q1, and R^{a} and R⁴ are as per any row of Table E below, which compounds are named I.A2-Z41-Q1-E-1 to I.A2-Z41-Q1-E-78.

In another embodiment, compounds I are of formula I.A2 wherein Z is 1,2,4-triazol-3-yl (Z-41), R³ is CH₃ and Q is Q2, and R^{a} and R⁴ are as per any row of Table E below, which compounds are named I.A2-Z41-Q2-E-1 to I.A2-Z41-Q2-E-78.

In another embodiment, compounds I are of formula I.A2 wherein Z is 1,2,4-triazol-3-yl (Z-41), R³ is CH₃ and Q is Q3, and R^{a} and R⁴ are as per any row of Table E below, which compounds are named I.A2-Z41-Q3-E-1 to I.A2-Z41-Q3-E-78.

In another embodiment, compounds I are of formula I.A2 wherein Z is 1,2,4-triazol-3-yl (Z-41), R³ is CH₃ and Q is Q4, and R^{a} and R⁴ are as per any row of Table E below, which compounds are named I.A2-Z41-Q4-E-1 to I.A2-Z41-Q4-E-78.

In another embodiment, compounds I are of formula I.A1 wherein Z is 1-CH₃-1,2,4-triazol-3-yl (Z-43), R³ is CH₃ and Q is Q1, and R^{a} and R⁴ are as per any row of Table E below, which compounds are named I.A1-Z43-Q1-E-1 to I.A1-Z43-Q1-E-78.

In another embodiment, compounds I are of formula I.A1 wherein Z is 1-CH₃-1,2,4-triazol-3-yl (Z-43), R³ is CH₃ and Q is Q2, and R^{a} and R⁴ are as per any row of Table E below, which compounds are named I.A1-Z43-Q2-E-1 to I.A1-Z43-Q2-E-78.

In another embodiment, compounds I are of formula I.A1 wherein Z is 1-CH₃-1,2,4-triazol-3-yl (Z-43), R³ is CH₃ and Q is Q3, and R^{a} and R⁴ are as per any row of Table E below, which compounds are named I.A1-Z43-Q3-E-1 to I.A1-Z43-Q3-E-78.

In another embodiment, compounds I are of formula I.A1 wherein Z is 1-CH₃-1,2,4-triazol-3-yl (Z-43), R³ is CH₃ and Q is Q4, and R^{a} and R⁴ are as per any row of Table E below, which compounds are named I.A1-Z43-Q4-E-1 to I.A1-Z43-Q4-E-78.

In another embodiment, compounds I are of formula I.A2 wherein Z is 1-CH₃-1,2,4-triazol-3-yl (Z-43), R³ is CH₃ and Q is Q1, and R^{a} and R⁴ are as per any row of Table E below, which compounds are named I.A2-Z43-Q1-E-1 to I.A2-Z43-Q1-E-78.

In another embodiment, compounds I are of formula I.A2 wherein Z is 1-CH₃-1,2,4-triazol-3-yl (Z-43), R³ is CH₃ and Q is Q2, and R^{a} and R⁴ are as per any row of Table E below, which compounds are named I.A2-Z43-Q2-E-1 to I.A2-Z43-Q2-E-78.

In another embodiment, compounds I are of formula I.A2 wherein Z is 1-CH₃-1,2,4-triazol-3-yl (Z-43), R³ is CH₃ and Q is Q3, and R^{a} and R⁴ are as per any row of Table E below, which compounds are named I.A2-Z43-Q3-E-1 to I.A2-Z43-Q3-E-78.

In another embodiment, compounds I are of formula I.A2 wherein Z is 1-CH₃-1,2,4-triazol-3-yl (Z-43), R³ is CH₃ and Q is Q4, and R^{a} and R⁴ are as per any row of Table E below, which compounds are named I.A2-Z43-Q4-E-1 to I.A2-Z43-Q4-E-78.

In another embodiment, compounds I are of formula I.B1 wherein Z is 1,2,4-triazol-3-yl (Z-41) and Q is Q1, and R^{a} and R⁴ are as per any row of Table E below, which compounds are named I.B1-Z41-Q1-E-1 to I.B1-Z41-Q1-E-78.

In another embodiment, compounds I are of formula I.B1 wherein Z is 1,2,4-triazol-3-yl (Z-41) and Q is Q2, and R^{a} and R⁴ are as per any row of Table E below, which compounds are named I.B1-Z41-Q2-E-1 to I.B1-Z41-Q2-E-78.

In another embodiment, compounds I are of formula I.B1 wherein Z is 1,2,4-triazol-3-yl (Z-41) and Q is Q3, and R^{a} and R⁴ are as per any row of Table E below, which compounds are named I.B1-Z41-Q3-E-1 to I.B1-Z41-Q3-E-78.

In another embodiment, compounds I are of formula I.B1 wherein Z is 1,2,4-triazol-3-yl (Z-41) and Q is Q4, and R^{a} and R⁴ are as per any row of Table E below, which compounds are named I.B1-Z41-Q4-E-1 to I.B1-Z41-Q4-E-78.

In another embodiment, compounds I are of formula I.B2 wherein Z is 1,2,4-triazol-3-yl (Z-41) and Q is Q1, and R^{a} and R⁴ are as per any row of Table E below, which compounds are named I.B2-Z41-Q1-E-1 to I.B2-Z41-Q1-E-78.

In another embodiment, compounds I are of formula I.B2 wherein Z is 1,2,4-triazol-3-yl (Z-41) and Q is Q2, and R^{a} and R⁴ are as per any row of Table E below, which compounds are named I.B2-Z41-Q2-E-1 to I.B2-Z41-Q2-E-78.

In another embodiment, compounds I are of formula I.B2 wherein Z is 1,2,4-triazol-3-yl (Z-41) and Q is Q3, and R^{a} and R⁴ are as per any row of Table E below, which compounds are named I.B2-Z41-Q3-E-1 to I.B2-Z41-Q3-E-78.

In another embodiment, compounds I are of formula I.B2 wherein Z is 1,2,4-triazol-3-yl (Z-41) and Q is Q4, and R^{a} and R⁴ are as per any row of Table E below, which compounds are named I.B2-Z41-Q4-E-1 to I.B2-Z41-Q4-E-78.

In another embodiment, compounds I are of formula I.B1 wherein Z is 1-CH₃-1,2,4-triazol-3-yl (Z-43) and Q is Q1, and R^{a} and R⁴ are as per any row of Table E below, which compounds are named I.B1-Z43-Q1-E-1 to I.B1-Z43-Q1-E-78.

In another embodiment, compounds I are of formula I.B1 wherein Z is 1-CH₃-1,2,4-triazol-3-yl (Z-43) and Q is Q2, and R^{a} and R⁴ are as per any row of Table E below, which compounds are named I.B1-Z43-Q2-E-1 to I.B1-Z43-Q2-E-78.

In another embodiment, compounds I are of formula I.B1 wherein Z is 1-CH₃-1,2,4-triazol-3-yl (Z-43) and Q is Q3, and R^{a} and R⁴ are as per any row of Table E below, which compounds are named I.B1-Z43-Q3-E-1 to I.B1-Z43-Q3-E-78.

In another embodiment, compounds I are of formula I.B1 wherein Z is 1-CH₃-1,2,4-triazol-3-yl (Z-43) and Q is Q4, and R^{a} and R⁴ are as per any row of Table E below, which compounds are named I.B1-Z43-Q4-E-1 to I.B1-Z43-Q4-E-78.

In another embodiment, compounds I are of formula I.B2 wherein Z is 1-CH₃-1,2,4-triazol-3-yl (Z-43) and Q is Q1, and R^{a} and R⁴ are as per any row of Table E below, which compounds are named I.B2-Z43-Q1-E-1 to I.B2-Z43-Q1-E-78.

In another embodiment, compounds I are of formula I.B2 wherein Z is 1-CH₃-1,2,4-triazol-3-yl (Z-43) and Q is Q2, and R^{a} and R⁴ are as per any row of Table E below, which compounds are named I.B2-Z43-Q2-E-1 to I.B2-Z43-Q2-E-78.

In another embodiment, compounds I are of formula I.B2 wherein Z is 1-CH₃-1,2,4-triazol-3-yl (Z-43) and Q is Q3, and R^{a} and R⁴ are as per any row of Table E below, which compounds are named I.B2-Z43-Q3-E-1 to I.B2-Z43-Q3-E-78.

In another embodiment, compounds I are of formula I.B2 wherein Z is 1-CH₃-1,2,4-triazol-3-yl (Z-43) and Q is Q4, and R^{a} and R⁴ are as per any row of Table E below, which compounds are named I.B2-Z43-Q4-E-1 to I.B2-Z43-Q4-E-78.

**Table E:**

| **No.** | **(R^{a})₀₋₁** | **R⁴** |
|---|---|---|
| E-1 | - | CH₃ |
| E-2 | 1-F | CH₃ |
| E-3 | 1-CI | CH₃ |
| E-4 | 1-CH₃ | CH₃ |
| E-5 | 1-CHF₂ | CH₃ |
| E-6 | 1-CF₃ | CH₃ |
| E-7 | 1-OCH₃ | CH₃ |
| E-8 | - | C₂H₅ |
| E-9 | 1-F | C₂H₅ |
| E-10 | 1-CI | C₂H₅ |
| E-11 | 1-CH₃ | C₂H₅ |
| E-12 | 1-CHF₂ | C₂H₅ |
| E-13 | 1-CF₃ | C₂H₅ |
| E-14 | 1-OCH₃ | C₂H₅ |
| E-15 | - | CHF₂ |
| E-16 | 1-F | CHF₂ |
| E-17 | 1-CI | CHF₂ |
| E-18 | 1-CH₃ | CHF₂ |
| E-19 | 1-CHF₂ | CHF₂ |
| E-20 | 1-CF₃ | CHF₂ |
| E-21 | 1-OCH₃ | CHF₂ |
| E-22 | - | CH₂CF₃ |
| E-23 | 1-F | CH₂CF₃ |
| E-24 | 1-CI | CH₂CF₃ |
| E-25 | 1-CH₃ | CH₂CF₃ |
| E-26 | 1-CHF₂ | CH₂CF₃ |
| E-27 | 1-CF₃ | CH₂CF₃ |
| E-28 | 1-OCH₃ | CH₂CF₃ |
| E-29 | - | CH₂C₃H₅ |
| E-30 | 1-F | CH₂C₃H₅ |
| E-31 | 1-CI | CH₂C₃H₅ |
| E-32 | 1-CH₃ | CH₂C₃H₅ |
| E-33 | 1-CHF₂ | CH₂C₃H₅ |
| E-34 | 1-CF₃ | CH₂C₃H₅ |
| E-35 | 1-OCH₃ | CH₂C₃H₅ |
| E-36 | - | CH₂OCH₃ |
| E-37 | 1-F | CH₂OCH₃ |
| E-38 | 1-CI | CH₂OCH₃ |
| E-39 | 1-CH₃ | CH₂OCH₃ |
| E-40 | 1-CHF₂ | CH₂OCH₃ |
| E-41 | 1-CF₃ | CH₂OCH₃ |
| E-42 | 1-OCH₃ | CH₂OCH₃ |
| E-43 | 5-F | CH₃ |
| E-44 | 5-Cl | CH₃ |
| E-45 | 5-CH₃ | CH₃ |
| E-46 | 5-CHF₂ | CH₃ |
| E-47 | 5-CF₃ | CH₃ |
| E-48 | 5-OCH₃ | CH₃ |
| E-49 | 5-F | C₂H₅ |
| E-50 | 5-CI | C₂H₅ |
| E-51 | 5-CH₃ | C₂H₅ |
| E-52 | 5-CHF₂ | C₂H₅ |
| E-53 | 5-CF₃ | C₂H₅ |
| E-54 | 5-OCH₃ | C₂H₅ |
| E-55 | 5-F | CHF₂ |
| E-56 | 5-CI | CHF₂ |
| E-57 | 5-CH₃ | CHF₂ |
| E-58 | 5-CHF₂ | CHF₂ |
| E-59 | 5-CF₃ | CHF₂ |
| E-60 | 5-OCH₃ | CHF₂ |
| E-61 | 5-F | CH₂CF₃ |
| E-62 | 5-CI | CH₂CF₃ |
| E-63 | 5-CH₃ | CH₂CF₃ |
| E-64 | 5-CHF₂ | CH₂CF₃ |
| E-65 | 5-CF₃ | CH₂CF₃ |
| E-66 | 5-OCH₃ | CH₂CF₃ |
| E-67 | 5-F | CH₂C₃H₅ |
| E-68 | 5-CI | CH₂C₃H₅ |
| E-69 | 5-CH₃ | CH₂C₃H₅ |
| E-70 | 5-CHF₂ | CH₂C₃H₅ |
| E-71 | 5-CF₃ | CH₂C₃H₅ |
| E-72 | 5-OCH₃ | CH₂C₃H₅ |
| E-73 | 5-F | CH₂OCH₃ |
| E-74 | 5-CI | CH₂OCH₃ |
| E-75 | 5-CH₃ | CH₂OCH₃ |
| E-76 | 5-CHF₂ | CH₂OCH₃ |
| E-77 | 5-CF₃ | CH₂OCH₃ |
| E-78 | 5-OCH₃ | CH₂OCH₃ |

In another embodiment, compounds I are of formula I.A1 wherein Z is oxazol-4-yl (Z-46), R³ is CH₃ and Q is Q1, and R^{a} and R⁴ are as per any row of Table F below, which compounds are named I.A1-Z46-Q1-F-1 to I.A1-Z46-Q1-F-78.

In another embodiment, compounds I are of formula I.A1 wherein Z is oxazol-4-yl (Z-46), R³ is CH₃ and Q is Q2, and R^{a} and R⁴ are as per any row of Table F below, which compounds are named I.A1-Z46-Q2-F-1 to I.A1-Z46-Q2-F-78.

In another embodiment, compounds I are of formula I.A1 wherein Z is oxazol-4-yl (Z-46), R³ is CH₃ and Q is Q3, and R^{a} and R⁴ are as per any row of Table F below, which compounds are named I.A1-Z46-Q3-F-1 to I.A1-Z46-Q3-F-78.

In another embodiment, compounds I are of formula I.A1 wherein Z is oxazol-4-yl (Z-46), R³ is CH₃ and Q is Q4, and R^{a} and R⁴ are as per any row of Table F below, which compounds are named I.A1-Z46-Q4-F-1 to I.A1-Z46-Q4-F-78.

In another embodiment, compounds I are of formula I.A2 wherein Z is oxazol-4-yl (Z-46), R³ is CH₃ and Q is Q1, and R^{a} and R⁴ are as per any row of Table F below, which compounds are named I.A2-Z46-Q1-F-1 to I.A2-Z46-Q1-F-78.

In another embodiment, compounds I are of formula I.A2 wherein Z is oxazol-4-yl (Z-46), R³ is CH₃ and Q is Q2, and R^{a} and R⁴ are as per any row of Table F below, which compounds are named I.A2-Z46-Q2-F-1 to I.A2-Z46-Q2-F-78.

In another embodiment, compounds I are of formula I.A2 wherein Z is oxazol-4-yl (Z-46), R³ is CH₃ and Q is Q3, and R^{a} and R⁴ are as per any row of Table F below, which compounds are named I.A2-Z46-Q3-F-1 to I.A2-Z46-Q3-F-78.

In another embodiment, compounds I are of formula I.A2 wherein Z is oxazol-4-yl (Z-46), R³ is CH₃ and Q is Q4, and R^{a} and R⁴ are as per any row of Table F below, which compounds are named I.A2-Z46-Q4-F-1 to I.A2-Z46-Q4-F-78.

In another embodiment, compounds I are of formula I.A1 wherein Z is thiazol-4-yl (Z-48), R³ is CH₃ and Q is Q1, and R^{a} and R⁴ are as per any row of Table F below, which compounds are named I.A1-Z48-Q1-F-1 to I.A1-Z48-Q1-F-78.

In another embodiment, compounds I are of formula I.A1 wherein Z is thiazol-4-yl (Z-48), R³ is CH₃ and Q is Q2, and R^{a} and R⁴ are as per any row of Table F below, which compounds are named I.A1-Z48-Q2-F-1 to I.A1-Z48-Q2-F-78.

In another embodiment, compounds I are of formula I.A1 wherein Z is thiazol-4-yl (Z-48), R³ is CH₃ and Q is Q3, and R^{a} and R⁴ are as per any row of Table F below, which compounds are named I.A1-Z48-Q3-F-1 to I.A1-Z48-Q3-F-78.

In another embodiment, compounds I are of formula I.A1 wherein Z is thiazol-4-yl (Z-48), R³ is CH₃ and Q is Q4, and R^{a} and R⁴ are as per any row of Table F below, which compounds are named I.A1-Z48-Q4-F-1 to I.A1-Z48-Q4-F-78.

In another embodiment, compounds I are of formula I.A2 wherein Z is thiazol-4-yl (Z-48), R³ is CH₃ and Q is Q1, and R^{a} and R⁴ are as per any row of Table F below, which compounds are named I.A2-Z48-Q1-F-1 to I.A2-Z48-Q1-F-78.

In another embodiment, compounds I are of formula I.A2 wherein Z is thiazol-4-yl (Z-48), R³ is CH₃ and Q is Q2, and R^{a} and R⁴ are as per any row of Table F below, which compounds are named I.A2-Z48-Q2-F-1 to I.A2-Z48-Q2-F-78.

In another embodiment, compounds I are of formula I.A2 wherein Z is thiazol-4-yl (Z-48), R³ is CH₃ and Q is Q3, and R^{a} and R⁴ are as per any row of Table F below, which compounds are named I.A2-Z48-Q3-F-1 to I.A2-Z48-Q3-F-78.

In another embodiment, compounds I are of formula I.A2 wherein Z is thiazol-4-yl (Z-48), R³ is CH₃ and Q is Q4, and R^{a} and R⁴ are as per any row of Table F below, which compounds are named I.A2-Z48-Q4-F-1 to I.A2-Z48-Q4-F-78.

In another embodiment, compounds I are of formula I.B1 wherein Z is oxazol-4-yl (Z-46) and Q is Q1, and R^{a} and R⁴ are as per any row of Table F below, which compounds are named I.B1-Z46-Q1-F-1 to I.B1-Z46-Q1-F-78.

In another embodiment, compounds I are of formula I.B1 wherein Z is oxazol-4-yl (Z-46) and Q is Q2, and R^{a} and R⁴ are as per any row of Table F below, which compounds are named I.B1-Z46-Q2-F-1 to I.B1-Z46-Q2-F-78.

In another embodiment, compounds I are of formula I.B1 wherein Z is oxazol-4-yl (Z-46) and Q is Q3, and R^{a} and R⁴ are as per any row of Table F below, which compounds are named I.B1-Z46-Q3-F-1 to I.B1-Z46-Q3-F-78.

In another embodiment, compounds I are of formula I.B1 wherein Z is oxazol-4-yl (Z-46) and Q is Q4, and R^{a} and R⁴ are as per any row of Table F below, which compounds are named I.B1-Z46-Q4-F-1 to I.B1-Z46-Q4-F-78.

In another embodiment, compounds I are of formula I.B2 wherein Z is oxazol-4-yl (Z-46) and Q is Q1, and R^{a} and R⁴ are as per any row of Table F below, which compounds are named I.B2-Z46-Q1-F-1 to I.B2-Z46-Q1-F-78.

In another embodiment, compounds I are of formula I.B2 wherein Z is oxazol-4-yl (Z-46) and Q is Q2, and R^{a} and R⁴ are as per any row of Table F below, which compounds are named I.B2-Z46-Q2-F-1 to I.B2-Z46-Q2-F-78.

In another embodiment, compounds I are of formula I.B2 wherein Z is oxazol-4-yl (Z-46) and Q is Q3, and R^{a} and R⁴ are as per any row of Table F below, which compounds are named I.B2-Z46-Q3-F-1 to I.B2-Z46-Q3-F-78.

In another embodiment, compounds I are of formula I.B2 wherein Z is oxazol-4-yl (Z-46) and Q is Q4, and R^{a} and R⁴ are as per any row of Table F below, which compounds are named I.B2-Z46-Q4-F-1 to I.B2-Z46-Q4-F-78.

In another embodiment, compounds I are of formula I.B1 wherein Z is thiazol-4-yl (Z-48) and Q is Q1, and R^{a} and R⁴ are as per any row of Table F below, which compounds are named I.B1-Z48-Q1-F-1 to I.B1-Z48-Q1-F-78.

In another embodiment, compounds I are of formula I.B1 wherein Z is thiazol-4-yl (Z-48) and Q is Q2, and R^{a} and R⁴ are as per any row of Table F below, which compounds are named I.B1-Z48-Q2-F-1 to I.B1-Z48-Q2-F-78.

In another embodiment, compounds I are of formula I.B1 wherein Z is thiazol-4-yl (Z-48) and Q is Q3, and R^{a} and R⁴ are as per any row of Table F below, which compounds are named I.B1-Z48-Q3-F-1 to I.B1-Z48-Q3-F-78.

In another embodiment, compounds I are of formula I.B1 wherein Z is thiazol-4-yl (Z-48) and Q is Q4, and R^{a} and R⁴ are as per any row of Table F below, which compounds are named I.B1-Z48-Q4-F-1 to I.B1-Z48-Q4-F-78.

In another embodiment, compounds I are of formula I.B2 wherein Z is thiazol-4-yl (Z-48) and Q is Q1, and R^{a} and R⁴ are as per any row of Table F below, which compounds are named I.B2-Z48-Q1-F-1 to I.B2-Z48-Q1-F-78.

In another embodiment, compounds I are of formula I.B2 wherein Z is thiazol-4-yl (Z-48) and Q is Q2, and R^{a} and R⁴ are as per any row of Table F below, which compounds are named I.B2-Z48-Q2-F-1 to I.B2-Z48-Q2-F-78.

In another embodiment, compounds I are of formula I.B2 wherein Z is thiazol-4-yl (Z-48) and Q is Q3, and R^{a} and R⁴ are as per any row of Table F below, which compounds are named I.B2-Z48-Q3-F-1 to I.B2-Z48-Q3-F-78.

In another embodiment, compounds I are of formula I.B2 wherein Z is thiazol-4-yl (Z-48) and Q is Q4, and R^{a} and R⁴ are as per any row of Table F below, which compounds are named I.B2-Z48-Q4-F-1 to I.B2-Z48-Q4-F-78.

**Table F:**

| **No.** | **(R^{a})₀₋₁** | **R⁴** |
|---|---|---|
| F-1 | - | CH₃ |
| F-2 | 2-F | CH₃ |
| F-3 | 2-CI | CH₃ |
| F-4 | 2-CH₃ | CH₃ |
| F-5 | 2-CHF₂ | CH₃ |
| F-6 | 2-CF₃ | CH₃ |
| F-7 | 2-OCH₃ | CH₃ |
| F-8 | - | C₂H₅ |
| F-9 | 2-F | C₂H₅ |
| F-10 | 2-CI | C₂H₅ |
| F-11 | 2-CH₃ | C₂H₅ |
| F-12 | 2-CHF₂ | C₂H₅ |
| F-13 | 2-CF₃ | C₂H₅ |
| F-14 | 2-OCH₃ | C₂H₅ |
| F-15 | - | CHF₂ |
| F-16 | 2-F | CHF₂ |
| F-17 | 2-CI | CHF₂ |
| F-18 | 2-CH₃ | CHF₂ |
| F-19 | 2-CHF₂ | CHF₂ |
| F-20 | 2-CF₃ | CHF₂ |
| F-21 | 2-OCH₃ | CHF₂ |
| F-22 | - | CH₂CF₃ |
| F-23 | 2-F | CH₂CF₃ |
| F-24 | 2-CI | CH₂CF₃ |
| F-25 | 2-CH₃ | CH₂CF₃ |
| F-26 | 2-CHF₂ | CH₂CF₃ |
| F-27 | 2-CF₃ | CH₂CF₃ |
| F-28 | 2-OCH₃ | CH₂CF₃ |
| F-29 | - | CH₂C₃H₅ |
| F-30 | 2-F | CH₂C₃H₅ |
| F-31 | 2-CI | CH₂C₃H₅ |
| F-32 | 2-CH₃ | CH₂C₃H₅ |
| F-33 | 2-CHF₂ | CH₂C₃H₅ |
| F-34 | 2-CF₃ | CH₂C₃H₅ |
| F-35 | 2-OCH₃ | CH₂C₃H₅ |
| F-36 | - | CH₂OCH₃ |
| F-37 | 2-F | CH₂OCH₃ |
| F-38 | 2-CI | CH₂OCH₃ |
| F-39 | 2-CH₃ | CH₂OCH₃ |
| F-40 | 2-CHF₂ | CH₂OCH₃ |
| F-41 | 2-CF₃ | CH₂OCH₃ |
| F-42 | 2-OCH₃ | CH₂OCH₃ |
| F-43 | 5-F | CH₃ |
| F-44 | 5-CI | CH₃ |
| F-45 | 5-CH₃ | CH₃ |
| F-46 | 5-CHF₂ | CH₃ |
| F-47 | 5-CF₃ | CH₃ |
| F-48 | 5-OCH₃ | CH₃ |
| F-49 | 5-F | C₂H₅ |
| F-50 | 5-CI | C₂H₅ |
| F-51 | 5-CH₃ | C₂H₅ |
| F-52 | 5-CHF₂ | C₂H₅ |
| F-53 | 5-CF₃ | C₂H₅ |
| F-54 | 5-OCH₃ | C₂H₅ |
| F-55 | 5-F | CHF₂ |
| F-56 | 5-CI | CHF₂ |
| F-57 | 5-CH₃ | CHF₂ |
| F-58 | 5-CHF₂ | CHF₂ |
| F-59 | 5-CF₃ | CHF₂ |
| F-60 | 5-OCH₃ | CHF₂ |
| F-61 | 5-F | CH₂CF₃ |
| F-62 | 5-Cl | CH₂CF₃ |
| F-63 | 5-CH₃ | CH₂CF₃ |
| F-64 | 5-CHF₂ | CH₂CF₃ |
| F-65 | 5-CF₃ | CH₂CF₃ |
| F-66 | 5-OCH₃ | CH₂CF₃ |
| F-67 | 5-F | CH₂C₃H₅ |
| F-68 | 5-Cl | CH₂C₃H₅ |
| F-69 | 5-CH₃ | CH₂C₃H₅ |
| F-70 | 5-CHF₂ | CH₂C₃H₅ |
| F-71 | 5-CF₃ | CH₂C₃H₅ |
| F-72 | 5-OCH₃ | CH₂C₃H₅ |
| F-73 | 5-F | CH₂OCH₃ |
| F-74 | 5-Cl | CH₂OCH₃ |
| F-75 | 5-CH₃ | CH₂OCH₃ |
| F-76 | 5-CHF₂ | CH₂OCH₃ |
| F-77 | 5-CF₃ | CH₂OCH₃ |
| F-78 | 5-OCH₃ | CH₂OCH₃ |

In another embodiment, compounds I are of formula I.A1 wherein Z is pyrazol-3-yl (Z-29), R³ is CH₃ and Q is Q1, and R^{a} and R⁴ are as per any row of Table G below, which compounds are named I.A1-Z29-Q1-G-1 to I.A1-Z29-Q1-G-78.

In another embodiment, compounds I are of formula I.A1 wherein Z is pyrazol-3-yl (Z-29), R³ is CH₃ and Q is Q2, and R^{a} and R⁴ are as per any row of Table G below, which compounds are named I.A1-Z29-Q2-G-1 to I.A1-Z29-Q2-G-78.

In another embodiment, compounds I are of formula I.A1 wherein Z is pyrazol-3-yl (Z-29), R³ is CH₃ and Q is Q3, and R^{a} and R⁴ are as per any row of Table G below, which compounds are named I.A1-Z29-Q3-G-1 to I.A1-Z29-Q3-G-78.

In another embodiment, compounds I are of formula I.A1 wherein Z is pyrazol-3-yl (Z-29), R³ is CH₃ and Q is Q4, and R^{a} and R⁴ are as per any row of Table G below, which compounds are named I.A1-Z29-Q4-G-1 to I.A1-Z29-Q4-G-78.

In another embodiment, compounds I are of formula I.A2 wherein Z is pyrazol-3-yl (Z-29), R³ is CH₃ and Q is Q1, and R^{a} and R⁴ are as per any row of Table G below, which compounds are named I.A2-Z29-Q1-G-1 to I.A2-Z29-Q1-G-78.

In another embodiment, compounds I are of formula I.A2 wherein Z is pyrazol-3-yl (Z-29), R³ is CH₃ and Q is Q2, and R^{a} and R⁴ are as per any row of Table G below, which compounds are named I.A2-Z29-Q2-G-1 to I.A2-Z29-Q2-G-78.

In another embodiment, compounds I are of formula I.A2 wherein Z is pyrazol-3-yl (Z-29), R³ is CH₃ and Q is Q3, and R^{a} and R⁴ are as per any row of Table G below, which compounds are named I.A2-Z29-Q3-G-1 to I.A2-Z29-Q3-G-78.

In another embodiment, compounds I are of formula I.A2 wherein Z is pyrazol-3-yl (Z-29), R³ is CH₃ and Q is Q4, and R^{a} and R⁴ are as per any row of Table G below, which compounds are named I.A2-Z29-Q4-G-1 to I.A2-Z29-Q4-G-78.

In another embodiment, compounds I are of formula I.A1 wherein Z is 1-CH₃-pyrazol-3-yl (Z-32), R³ is CH₃ and Q is Q1, and R^{a} and R⁴ are as per any row of Table G below, which compounds are named I.A1-Z32-Q1-G-1 to I.A1-Z32-Q1-G-78.

In another embodiment, compounds I are of formula I.A1 wherein Z is 1-CH₃-pyrazol-3-yl (Z-32), R³ is CH₃ and Q is Q2, and R^{a} and R⁴ are as per any row of Table G below, which compounds are named I.A1-Z32-Q2-G-1 to I.A1-Z32-Q2-G-78.

In another embodiment, compounds I are of formula I.A1 wherein Z is 1-CH₃-pyrazol-3-yl (Z-32), R³ is CH₃ and Q is Q3, and R^{a} and R⁴ are as per any row of Table G below, which compounds are named I.A1-Z32-Q3-G-1 to I.A1-Z32-Q3-G-78.

In another embodiment, compounds I are of formula I.A1 wherein Z is 1-CH₃-pyrazol-3-yl (Z-32), R³ is CH₃ and Q is Q4, and R^{a} and R⁴ are as per any row of Table G below, which compounds are named I.A1-Z32-Q4-G-1 to I.A1-Z32-Q4-G-78.

In another embodiment, compounds I are of formula I.A2 wherein Z is 1-CH₃-pyrazol-3-yl (Z-32), R³ is CH₃ and Q is Q1, and R^{a} and R⁴ are as per any row of Table G below, which compounds are named I.A2-Z32-Q1-G-1 to I.A2-Z32-Q1-G-78.

In another embodiment, compounds I are of formula I.A2 wherein Z is 1-CH₃-pyrazol-3-yl (Z-32), R³ is CH₃ and Q is Q2, and R^{a} and R⁴ are as per any row of Table G below, which compounds are named I.A2-Z32-Q2-G-1 to I.A2-Z32-Q2-G-78.

In another embodiment, compounds I are of formula I.A2 wherein Z is 1-CH₃-pyrazol-3-yl (Z-32), R³ is CH₃ and Q is Q3, and R^{a} and R⁴ are as per any row of Table G below, which compounds are named I.A2-Z32-Q3-G-1 to I.A2-Z32-Q3-G-78.

In another embodiment, compounds I are of formula I.A2 wherein Z is 1-CH₃-pyrazol-3-yl (Z-32), R³ is CH₃ and Q is Q4, and R^{a} and R⁴ are as per any row of Table G below, which compounds are named I.A2-Z32-Q4-G-1 to I.A2-Z32-Q4-G-78.

In another embodiment, compounds I are of formula I.B1 wherein Z is pyrazol-3-yl (Z-29) and Q is Q1, and R^{a} and R⁴ are as per any row of Table G below, which compounds are named I.B1-Z29-Q1-G-1 to I.B1-Z29-Q1-G-78.

In another embodiment, compounds I are of formula I.B1 wherein Z is pyrazol-3-yl (Z-29) and Q is Q2, and R^{a} and R⁴ are as per any row of Table G below, which compounds are named I.B1-Z29-Q2-G-1 to I.B1-Z29-Q2-G-78.

In another embodiment, compounds I are of formula I.B1 wherein Z is pyrazol-3-yl (Z-29) and Q is Q3, and R^{a} and R⁴ are as per any row of Table G below, which compounds are named I.B1-Z29-Q3-G-1 to I.B1-Z29-Q3-G-78.

In another embodiment, compounds I are of formula I.B1 wherein Z is pyrazol-3-yl (Z-29) and Q is Q4, and R^{a} and R⁴ are as per any row of Table G below, which compounds are named I.B1-Z29-Q4-G-1 to I.B1-Z29-Q4-G-78.

In another embodiment, compounds I are of formula I.B2 wherein Z is pyrazol-3-yl (Z-29) and Q is Q1, and R^{a} and R⁴ are as per any row of Table G below, which compounds are named I.B2-Z29-Q1-G-1 to I.B2-Z29-Q1-G-78.

In another embodiment, compounds I are of formula I.B2 wherein Z is pyrazol-3-yl (Z-29) and Q is Q2, and R^{a} and R⁴ are as per any row of Table G below, which compounds are named I.B2-Z29-Q2-G-1 to I.B2-Z29-Q2-G-78.

In another embodiment, compounds I are of formula I.B2 wherein Z is pyrazol-3-yl (Z-29) and Q is Q3, and R^{a} and R⁴ are as per any row of Table G below, which compounds are named I.B2-Z29-Q3-G-1 to I.B2-Z29-Q3-G-78.

In another embodiment, compounds I are of formula I.B2 wherein Z is pyrazol-3-yl (Z-29) and Q is Q4, and R^{a} and R⁴ are as per any row of Table G below, which compounds are named I.B2-Z29-Q4-G-1 to I.B2-Z29-Q4-G-78.

In another embodiment, compounds I are of formula I.B1 wherein Z is 1-CH₃-pyrazol-3-yl (Z-32) and Q is Q1, and R^{a} and R⁴ are as per any row of Table G below, which compounds are named I.B1-Z32-Q1-G-1 to I.B1-Z32-Q1-G-78.

In another embodiment, compounds I are of formula I.B1 wherein Z is 1-CH₃-pyrazol-3-yl (Z-32) and Q is Q2, and R^{a} and R⁴ are as per any row of Table G below, which compounds are named I.B1-Z32-Q2-G-1 to I.B1-Z32-Q2-G-78.

In another embodiment, compounds I are of formula I.B1 wherein Z is 1-CH₃-pyrazol-3-yl (Z-32) and Q is Q3, and R^{a} and R⁴ are as per any row of Table G below, which compounds are named I.B1-Z32-Q3-G-1 to I.B1-Z32-Q3-G-78.

In another embodiment, compounds I are of formula I.B1 wherein Z is 1-CH₃-pyrazol-3-yl (Z-32) and Q is Q4, and R^{a} and R⁴ are as per any row of Table G below, which compounds are named I.B1-Z32-Q4-G-1 to I.B1-Z32-Q4-G-78.

In another embodiment, compounds I are of formula I.B2 wherein Z is 1-CH₃-pyrazol-3-yl (Z-32) and Q is Q1, and R^{a} and R⁴ are as per any row of Table G below, which compounds are named I.B2-Z32-Q1-G-1 to I.B2-Z32-Q1-G-78.

In another embodiment, compounds I are of formula I.B2 wherein Z is 1-CH₃-pyrazol-3-yl (Z-32) and Q is Q2, and R^{a} and R⁴ are as per any row of Table G below, which compounds are named I.B2-Z32-Q2-G-1 to I.B2-Z32-Q2-G-78.

In another embodiment, compounds I are of formula I.B2 wherein Z is 1-CH₃-pyrazol-3-yl (Z-32) and Q is Q3, and R^{a} and R⁴ are as per any row of Table G below, which compounds are named I.B2-Z32-Q3-G-1 to I.B2-Z32-Q3-G-78.

In another embodiment, compounds I are of formula I.B2 wherein Z is 1-CH₃-pyrazol-3-yl (Z-32) and Q is Q4, and R^{a} and R⁴ are as per any row of Table G below, which compounds are named I.B2-Z32-Q4-G-1 to I.B2-Z32-Q4-G-78.

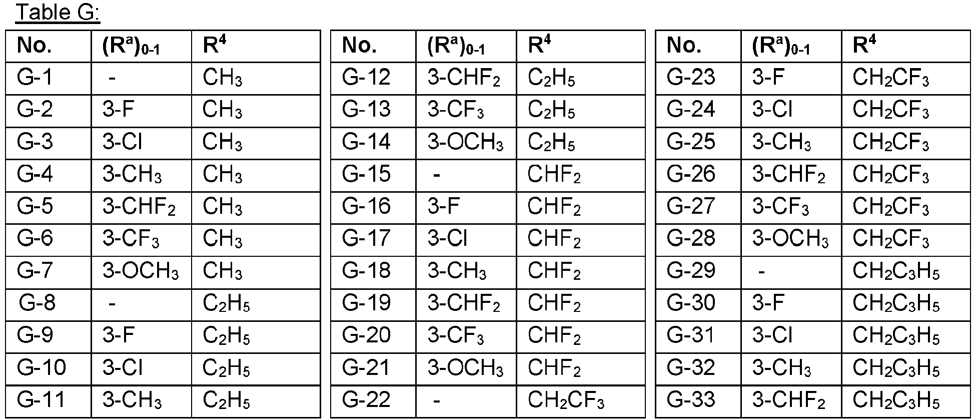

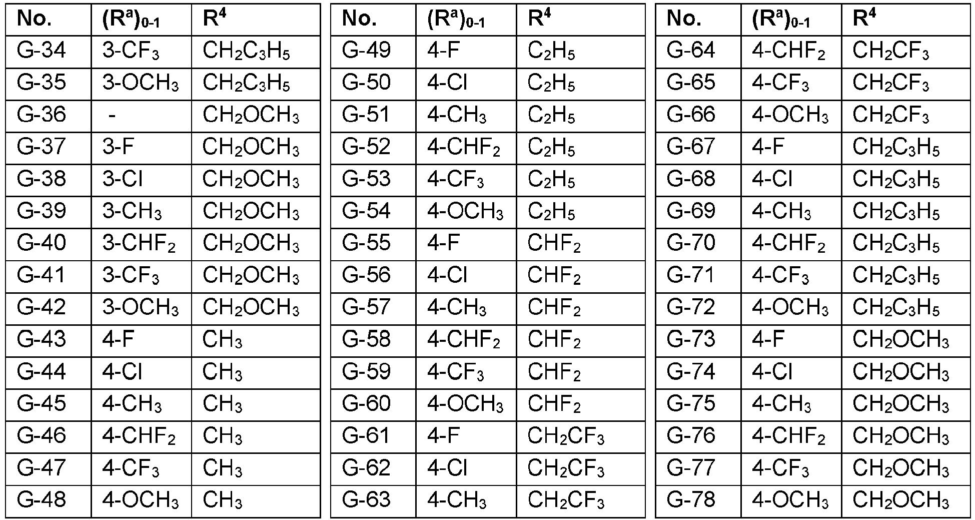

In another embodiment, compounds I are of formula I.A1 wherein Z is pyrazol-4-yl (Z-28), R³ is CH₃ and Q is Q1, and R^{a} and R⁴ are as per any row of Table H below, which compounds are named I.A1-Z28-Q1-H-1 to I.A1-Z28-Q1-H-78.

In another embodiment, compounds I are of formula I.A1 wherein Z is pyrazol-4-yl (Z-28), R³ is CH₃ and Q is Q2, and R^{a} and R⁴ are as per any row of Table H below, which compounds are named I.A1-Z28-Q2-H-1 to I.A1-Z28-Q2-H-78.

In another embodiment, compounds I are of formula I.A1 wherein Z is pyrazol-4-yl (Z-28), R³ is CH₃ and Q is Q3, and R^{a} and R⁴ are as per any row of Table H below, which compounds are named I.A1-Z28-Q3-H-1 to I.A1-Z28-Q3-H-78.

In another embodiment, compounds I are of formula I.A1 wherein Z is pyrazol-4-yl (Z-28), R³ is CH₃ and Q is Q4, and R^{a} and R⁴ are as per any row of Table H below, which compounds are named I.A1-Z28-Q4-H-1 to I.A1-Z28-Q4-H-78.

In another embodiment, compounds I are of formula I.A2 wherein Z is pyrazol-4-yl (Z-28), R³ is CH₃ and Q is Q1, and R^{a} and R⁴ are as per any row of Table H below, which compounds are named I.A2-Z28-Q1-H-1 to I.A2-Z28-Q1-H-78.

In another embodiment, compounds I are of formula I.A2 wherein Z is pyrazol-4-yl (Z-28), R³ is CH₃ and Q is Q2, and R^{a} and R⁴ are as per any row of Table H below, which compounds are named I.A2-Z28-Q2-H-1 to I.A2-Z28-Q2-H-78.

In another embodiment, compounds I are of formula I.A2 wherein Z is pyrazol-4-yl (Z-28), R³ is CH₃ and Q is Q3, and R^{a} and R⁴ are as per any row of Table H below, which compounds are named I.A2-Z28-Q3-H-1 to I.A2-Z28-Q3-H-78.

In another embodiment, compounds I are of formula I.A2 wherein Z is pyrazol-4-yl (Z-28), R³ is CH₃ and Q is Q4, and R^{a} and R⁴ are as per any row of Table H below, which compounds are named I.A2-Z28-Q4-H-1 to I.A2-Z28-Q4-H-78.

In another embodiment, compounds I are of formula I.A1 wherein Z is 1-CH₃-pyrazol-4-yl (Z-31), R³ is CH₃ and Q is Q1, and R^{a} and R⁴ are as per any row of Table H below, which compounds are named I.A1-Z31-Q1-H-1 to I.A1-Z31-Q1-H-78.

In another embodiment, compounds I are of formula I.A1 wherein Z is 1-CH₃-pyrazol-4-yl (Z-31), R³ is CH₃ and Q is Q2, and R^{a} and R⁴ are as per any row of Table H below, which compounds are named I.A1-Z31-Q2-H-1 to I.A1-Z31-Q2-H-78.

In another embodiment, compounds I are of formula I.A1 wherein Z is 1-CH₃-pyrazol-4-yl (Z-31), R³ is CH₃ and Q is Q3, and R^{a} and R⁴ are as per any row of Table H below, which compounds are named I.A1-Z31-Q3-H-1 to I.A1-Z31-Q3-H-78.

In another embodiment, compounds I are of formula I.A1 wherein Z is 1-CH₃-pyrazol-4-yl (Z-31), R³ is CH₃ and Q is Q4, and R^{a} and R⁴ are as per any row of Table H below, which compounds are named I.A1-Z31-Q4-H-1 to I.A1-Z31-Q4-H-78.

In another embodiment, compounds I are of formula I.A2 wherein Z is 1-CH₃-pyrazol-4-yl (Z-31), R³ is CH₃ and Q is Q1, and R^{a} and R⁴ are as per any row of Table H below, which compounds are named I.A2-Z31-Q1-H-1 to I.A2-Z31-Q1-H-78.

In another embodiment, compounds I are of formula I.A2 wherein Z is 1-CH₃-pyrazol-4-yl (Z-31), R³ is CH₃ and Q is Q2, and R^{a} and R⁴ are as per any row of Table H below, which compounds are named I.A2-Z31-Q2-H-1 to I.A2-Z31-Q2-H-78.

In another embodiment, compounds I are of formula I.A2 wherein Z is 1-CH₃-pyrazol-4-yl (Z-31), R³ is CH₃ and Q is Q3, and R^{a} and R⁴ are as per any row of Table H below, which compounds are named I.A2-Z31-Q3-H-1 to I.A2-Z31-Q3-H-78.

In another embodiment, compounds I are of formula I.A2 wherein Z is 1-CH₃-pyrazol-4-yl (Z-31), R³ is CH₃ and Q is Q4, and R^{a} and R⁴ are as per any row of Table H below, which compounds are named I.A2-Z31-Q4-H-1 to I.A2-Z31-Q4-H-78.

In another embodiment, compounds I are of formula I.A1 wherein Z is 1,2,4-triazol-1-yl (Z-40), R³ is CH₃ and Q is Q1, and R^{a} and R⁴ are as per any row of Table H below, which compounds are named I.A1-Z40-Q1-H-1 to I.A1-Z40-Q1-H-78.

In another embodiment, compounds I are of formula I.A1 wherein Z is 1,2,4-triazol-1-yl (Z-40), R³ is CH₃ and Q is Q2, and R^{a} and R⁴ are as per any row of Table H below, which compounds are named I.A1-Z40-Q2-H-1 to I.A1-Z40-Q2-H-78.

In another embodiment, compounds I are of formula I.A1 wherein Z is 1,2,4-triazol-1-yl (Z-40), R³ is CH₃ and Q is Q3, and R^{a} and R⁴ are as per any row of Table H below, which compounds are named I.A1-Z40-Q3-H-1 to I.A1-Z40-Q3-H-78.

In another embodiment, compounds I are of formula I.A1 wherein Z is 1,2,4-triazol-1-yl (Z-40), R³ is CH₃ and Q is Q4, and R^{a} and R⁴ are as per any row of Table H below, which compounds are named I.A1-Z40-Q4-H-1 to I.A1-Z40-Q4-H-78.

In another embodiment, compounds I are of formula I.A2 wherein Z is 1,2,4-triazol-1-yl (Z-40), R³ is CH₃ and Q is Q1, and R^{a} and R⁴ are as per any row of Table H below, which compounds are named I.A2-Z40-Q1-H-1 to I.A2-Z40-Q1-H-78.

In another embodiment, compounds I are of formula I.A2 wherein Z is 1,2,4-triazol-1-yl (Z-40), R³ is CH₃ and Q is Q2, and R^{a} and R⁴ are as per any row of Table H below, which compounds are named I.A2-Z40-Q2-H-1 to I.A2-Z40-Q2-H-78.

In another embodiment, compounds I are of formula I.A2 wherein Z is 1,2,4-triazol-1-yl (Z-40), R³ is CH₃ and Q is Q3, and R^{a} and R⁴ are as per any row of Table H below, which compounds are named I.A2-Z40-Q3-H-1 to I.A2-Z40-Q3-H-78.

In another embodiment, compounds I are of formula I.A2 wherein Z is 1,2,4-triazol-1-yl (Z-40), R³ is CH₃ and Q is Q4, and R^{a} and R⁴ are as per any row of Table H below, which compounds are named I.A2-Z40-Q4-H-1 to I.A2-Z40-Q4-H-78.

In another embodiment, compounds I are of formula I.A1 wherein Z is isoxazol-4-yl (Z-50), R³ is CH₃ and Q is Q1, and R^{a} and R⁴ are as per any row of Table H below, which compounds are named I.A1-Z50-Q1-H-1 to I.A1-Z50-Q1-H-78.

In another embodiment, compounds I are of formula I.A1 wherein Z is isoxazol-4-yl (Z-50), R³ is CH₃ and Q is Q2, and R^{a} and R⁴ are as per any row of Table H below, which compounds are named I.A1-Z50-Q2-H-1 to I.A1-Z50-Q2-H-78.

In another embodiment, compounds I are of formula I.A1 wherein Z is isoxazol-4-yl (Z-50), R³ is CH₃ and Q is Q3, and R^{a} and R⁴ are as per any row of Table H below, which compounds are named I.A1-Z50-Q3-H-1 to I.A1-Z50-Q3-H-78.

In another embodiment, compounds I are of formula I.A1 wherein Z is isoxazol-4-yl (Z-50), R³ is CH₃ and Q is Q4, and R^{a} and R⁴ are as per any row of Table H below, which compounds are named I.A1-Z50-Q4-H-1 to I.A1-Z50-Q4-H-78.

In another embodiment, compounds I are of formula I.A2 wherein Z is isoxazol-4-yl (Z-50), R³ is CH₃ and Q is Q1, and R^{a} and R⁴ are as per any row of Table H below, which compounds are named I.A2-Z50-Q1-H-1 to I.A2-Z50-Q1-H-78.

In another embodiment, compounds I are of formula I.A2 wherein Z is isoxazol-4-yl (Z-50), R³ is CH₃ and Q is Q2, and R^{a} and R⁴ are as per any row of Table H below, which compounds are named I.A2-Z50-Q2-H-1 to I.A2-Z50-Q2-H-78.

In another embodiment, compounds I are of formula I.A2 wherein Z is isoxazol-4-yl (Z-50), R³ is CH₃ and Q is Q3, and R^{a} and R⁴ are as per any row of Table H below, which compounds are named I.A2-Z50-Q3-H-1 to I.A2-Z50-Q3-H-78.

In another embodiment, compounds I are of formula I.A2 wherein Z is isoxazol-4-yl (Z-50), R³ is CH₃ and Q is Q4, and R^{a} and R⁴ are as per any row of Table H below, which compounds are named I.A2-Z50-Q4-H-1 to I.A2-Z50-Q4-H-78.

In another embodiment, compounds I are of formula I.A1 wherein Z is isothiazol-4-yl (Z-52), R³ is CH₃ and Q is Q1, and R^{a} and R⁴ are as per any row of Table H below, which compounds are named I.A1-Z52-Q1-H-1 to I.A1-Z52-Q1-H-78.

In another embodiment, compounds I are of formula I.A1 wherein Z is isothiazol-4-yl (Z-52), R³ is CH₃ and Q is Q2, and R^{a} and R⁴ are as per any row of Table H below, which compounds are named I.A1-Z52-Q2-H-1 to I.A1-Z52-Q2-H-78.

In another embodiment, compounds I are of formula I.A1 wherein Z is isothiazol-4-yl (Z-52), R³ is CH₃ and Q is Q3, and R^{a} and R⁴ are as per any row of Table H below, which compounds are named I.A1-Z52-Q3-H-1 to I.A1-Z52-Q3-H-78.

In another embodiment, compounds I are of formula I.A1 wherein Z is isothiazol-4-yl (Z-52), R³ is CH₃ and Q is Q4, and R^{a} and R⁴ are as per any row of Table H below, which compounds are named I.A1-Z52-Q4-H-1 to I.A1-Z52-Q4-H-78.

In another embodiment, compounds I are of formula I.A2 wherein Z is isothiazol-4-yl (Z-52), R³ is CH₃ and Q is Q1, and R^{a} and R⁴ are as per any row of Table H below, which compounds are named I.A2-Z52-Q1-H-1 to I.A2-Z52-Q1-H-78.

In another embodiment, compounds I are of formula I.A2 wherein Z is isothiazol-4-yl (Z-52), R³ is CH₃ and Q is Q2, and R^{a} and R⁴ are as per any row of Table H below, which compounds are named I.A2-Z52-Q2-H-1 to I.A2-Z52-Q2-H-78.

In another embodiment, compounds I are of formula I.A2 wherein Z is isothiazol-4-yl (Z-52), R³ is CH₃ and Q is Q3, and R^{a} and R⁴ are as per any row of Table H below, which compounds are named I.A2-Z52-Q3-H-1 to I.A2-Z52-Q3-H-78.

In another embodiment, compounds I are of formula I.A2 wherein Z is isothiazol-4-yl (Z-52), R³ is CH₃ and Q is Q4, and R^{a} and R⁴ are as per any row of Table H below, which compounds are named I.A2-Z52-Q4-H-1 to I.A2-Z52-Q4-H-78.

In another embodiment, compounds I are of formula I.B1 wherein Z is pyrazol-4-yl (Z-28) and Q is Q1, and R^{a} and R⁴ are as per any row of Table H below, which compounds are named I.B1-Z28-Q1-H-1 to I.B1-Z28-Q1-H-78.

In another embodiment, compounds I are of formula I.B1 wherein Z is pyrazol-4-yl (Z-28) and Q is Q2, and R^{a} and R⁴ are as per any row of Table H below, which compounds are named I.B1-Z28-Q2-H-1 to I.B1-Z28-Q2-H-78.

In another embodiment, compounds I are of formula I.B1 wherein Z is pyrazol-4-yl (Z-28) and Q is Q3, and R^{a} and R⁴ are as per any row of Table H below, which compounds are named I.B1-Z28-Q3-H-1 to I.B1-Z28-Q3-H-78.

In another embodiment, compounds I are of formula I.B1 wherein Z is pyrazol-4-yl (Z-28) and Q is Q4, and R^{a} and R⁴ are as per any row of Table H below, which compounds are named I.B1-Z28-Q4-H-1 to I.B1-Z28-Q4-H-78.

In another embodiment, compounds I are of formula I.B2 wherein Z is pyrazol-4-yl (Z-28) and Q is Q1, and R^{a} and R⁴ are as per any row of Table H below, which compounds are named I.B2-Z28-Q1-H-1 to I.B2-Z28-Q1-H-78.

In another embodiment, compounds I are of formula I.B2 wherein Z is pyrazol-4-yl (Z-28) and Q is Q2, and R^{a} and R⁴ are as per any row of Table H below, which compounds are named I.B2-Z28-Q2-H-1 to I.B2-Z28-Q2-H-78.

In another embodiment, compounds I are of formula I.B2 wherein Z is pyrazol-4-yl (Z-28) and Q is Q3, and R^{a} and R⁴ are as per any row of Table H below, which compounds are named I.B2-Z28-Q3-H-1 to I.B2-Z28-Q3-H-78.

In another embodiment, compounds I are of formula I.B2 wherein Z is pyrazol-4-yl (Z-28) and Q is Q4, and R^{a} and R⁴ are as per any row of Table H below, which compounds are named I.B2-Z28-Q4-H-1 to I.B2-Z28-Q4-H-78.

In another embodiment, compounds I are of formula I.B1 wherein Z is 1-CH₃-pyrazol-4-yl (Z-31) and Q is Q1, and R^{a} and R⁴ are as per any row of Table H below, which compounds are named I.B1-Z31-Q1-H-1 to I.B1-Z31-Q1-H-78.

In another embodiment, compounds I are of formula I.B1 wherein Z is 1-CH₃-pyrazol-4-yl (Z-31) and Q is Q2, and R^{a} and R⁴ are as per any row of Table H below, which compounds are named I.B1-Z31-Q2-H-1 to I.B1-Z31-Q2-H-78.

In another embodiment, compounds I are of formula I.B1 wherein Z is 1-CH₃-pyrazol-4-yl (Z-31) and Q is Q3, and R^{a} and R⁴ are as per any row of Table H below, which compounds are named I.B1-Z31-Q3-H-1 to I.B1-Z31-Q3-H-78.

In another embodiment, compounds I are of formula I.B1 wherein Z is 1-CH₃-pyrazol-4-yl (Z-31) and Q is Q4, and R^{a} and R⁴ are as per any row of Table H below, which compounds are named I.B1-Z31-Q4-H-1 to I.B1-Z31-Q4-H-78.

In another embodiment, compounds I are of formula I.B2 wherein Z is 1-CH₃-pyrazol-4-yl (Z-31) and Q is Q1, and R^{a} and R⁴ are as per any row of Table H below, which compounds are named I.B2-Z31-Q1-H-1 to I.B2-Z31-Q1-H-78.

In another embodiment, compounds I are of formula I.B2 wherein Z is 1-CH₃-pyrazol-4-yl (Z-31) and Q is Q2, and R^{a} and R⁴ are as per any row of Table H below, which compounds are named I.B2-Z31-Q2-H-1 to I.B2-Z31-Q2-H-78.

In another embodiment, compounds I are of formula I.B2 wherein Z is 1-CH₃-pyrazol-4-yl (Z-31) and Q is Q3, and R^{a} and R⁴ are as per any row of Table H below, which compounds are named I.B2-Z31-Q3-H-1 to I.B2-Z31-Q3-H-78.

In another embodiment, compounds I are of formula I.B2 wherein Z is 1-CH₃-pyrazol-4-yl (Z-31) and Q is Q4, and R^{a} and R⁴ are as per any row of Table H below, which compounds are named I.B2-Z31-Q4-H-1 to I.B2-Z31-Q4-H-78.

In another embodiment, compounds I are of formula I.B1 wherein Z is 1,2,4-triazol-1-yl (Z-40) and Q is Q1, and R^{a} and R⁴ are as per any row of Table H below, which compounds are named I.B1-Z40-Q1-H-1 to I.B1-Z40-Q1-H-78.

In another embodiment, compounds I are of formula I.B1 wherein Z is 1,2,4-triazol-1-yl (Z-40) and Q is Q2, and R^{a} and R⁴ are as per any row of Table H below, which compounds are named I.B1-Z40-Q2-H-1 to I.B1-Z40-Q2-H-78.

In another embodiment, compounds I are of formula I.B1 wherein Z is 1,2,4-triazol-1-yl (Z-40) and Q is Q3, and R^{a} and R⁴ are as per any row of Table H below, which compounds are named I.B1-Z40-Q3-H-1 to I.B1-Z40-Q3-H-78.

In another embodiment, compounds I are of formula I.B1 wherein Z is 1,2,4-triazol-1-yl (Z-40) and Q is Q4, and R^{a} and R⁴ are as per any row of Table H below, which compounds are named I.B1-Z40-Q4-H-1 to I.B1-Z40-Q4-H-78.

In another embodiment, compounds I are of formula I.B2 wherein Z is 1,2,4-triazol-1-yl (Z-40) and Q is Q1, and R^{a} and R⁴ are as per any row of Table H below, which compounds are named I.B2-Z40-Q1-H-1 to I.B2-Z40-Q1-H-78.

In another embodiment, compounds I are of formula I.B2 wherein Z is 1,2,4-triazol-1-yl (Z-40) and Q is Q2, and R^{a} and R⁴ are as per any row of Table H below, which compounds are named I.B2-Z40-Q2-H-1 to I.B2-Z40-Q2-H-78.

In another embodiment, compounds I are of formula I.B2 wherein Z is 1,2,4-triazol-1-yl (Z-40) and Q is Q3, and R^{a} and R⁴ are as per any row of Table H below, which compounds are named I.B2-Z40-Q3-H-1 to I.B2-Z40-Q3-H-78.

In another embodiment, compounds I are of formula I.B2 wherein Z is 1,2,4-triazol-1-yl (Z-40) and Q is Q4, and R^{a} and R⁴ are as per any row of Table H below, which compounds are named I.B2-Z40-Q4-H-1 to I.B2-Z40-Q4-H-78.

In another embodiment, compounds I are of formula I.B1 wherein Z is isoxazol-4-yl (Z-50) and Q is Q1, and R^{a} and R⁴ are as per any row of Table H below, which compounds are named I.B1-Z50-Q1-H-1 to I.B1-Z50-Q1-H-78.

In another embodiment, compounds I are of formula I.B1 wherein Z is isoxazol-4-yl (Z-50) and Q is Q2, and R^{a} and R⁴ are as per any row of Table H below, which compounds are named I.B1-Z50-Q2-H-1 to I.B1-Z50-Q2-H-78.

In another embodiment, compounds I are of formula I.B1 wherein Z is isoxazol-4-yl (Z-50) and Q is Q3, and R^{a} and R⁴ are as per any row of Table H below, which compounds are named I.B1-Z50-Q3-H-1 to I.B1-Z50-Q3-H-78.

In another embodiment, compounds I are of formula I.B1 wherein Z is isoxazol-4-yl (Z-50) and Q is Q4, and R^{a} and R⁴ are as per any row of Table H below, which compounds are named I.B1-Z50-Q4-H-1 to I.B1-Z50-Q4-H-78.

In another embodiment, compounds I are of formula I.B2 wherein Z is isoxazol-4-yl (Z-50) and Q is Q1, and R^{a} and R⁴ are as per any row of Table H below, which compounds are named I.B2-Z50-Q1-H-1 to I.B2-Z50-Q1-H-78.

In another embodiment, compounds I are of formula I.B2 wherein Z is isoxazol-4-yl (Z-50) and Q is Q2, and R^{a} and R⁴ are as per any row of Table H below, which compounds are named I.B2-Z50-Q2-H-1 to I.B2-Z50-Q2-H-78.

In another embodiment, compounds I are of formula I.B2 wherein Z is isoxazol-4-yl (Z-50) and Q is Q3, and R^{a} and R⁴ are as per any row of Table H below, which compounds are named I.B2-Z50-Q3-H-1 to I.B2-Z50-Q3-H-78.

In another embodiment, compounds I are of formula I.B2 wherein Z is isoxazol-4-yl (Z-50) and Q is Q4, and R^{a} and R⁴ are as per any row of Table H below, which compounds are named I.B2-Z50-Q4-H-1 to I.B2-Z50-Q4-H-78.

In another embodiment, compounds I are of formula I.B1 wherein Z is isothiazol-4-yl (Z-52) and Q is Q1, and R^{a} and R⁴ are as per any row of Table H below, which compounds are named I.B1-Z52-Q1-H-1 to I.B1-Z52-Q1-H-78.

In another embodiment, compounds I are of formula I.B1 wherein Z is isothiazol-4-yl (Z-52) and Q is Q2, and R^{a} and R⁴ are as per any row of Table H below, which compounds are named I.B1-Z52-Q2-H-1 to I.B1-Z52-Q2-H-78.

In another embodiment, compounds I are of formula I.B1 wherein Z is isothiazol-4-yl (Z-52) and Q is Q3, and R^{a} and R⁴ are as per any row of Table H below, which compounds are named I.B1-Z52-Q3-H-1 to I.B1-Z52-Q3-H-78.

In another embodiment, compounds I are of formula I.B1 wherein Z is isothiazol-4-yl (Z-52) and Q is Q4, and R^{a} and R⁴ are as per any row of Table H below, which compounds are named I.B1-Z52-Q4-H-1 to I.B1-Z52-Q4-H-78.

In another embodiment, compounds I are of formula I.B2 wherein Z is isothiazol-4-yl (Z-52) and Q is Q1, and R^{a} and R⁴ are as per any row of Table H below, which compounds are named I.B2-Z52-Q1-H-1 to I.B2-Z52-Q1-H-78.

In another embodiment, compounds I are of formula I.B2 wherein Z is isothiazol-4-yl (Z-52) and Q is Q2, and R^{a} and R⁴ are as per any row of Table H below, which compounds are named I.B2-Z52-Q2-H-1 to I.B2-Z52-Q2-H-78.

In another embodiment, compounds I are of formula I.B2 wherein Z is isothiazol-4-yl (Z-52) and Q is Q3, and R^{a} and R⁴ are as per any row of Table H below, which compounds are named I.B2-Z52-Q3-H-1 to I.B2-Z52-Q3-H-78.

In another embodiment, compounds I are of formula I.B2 wherein Z is isothiazol-4-yl (Z-52) and Q is Q4, and R^{a} and R⁴ are as per any row of Table H below, which compounds are named I.B2-Z52-Q4-H-1 to I.B2-Z52-Q4-H-78.

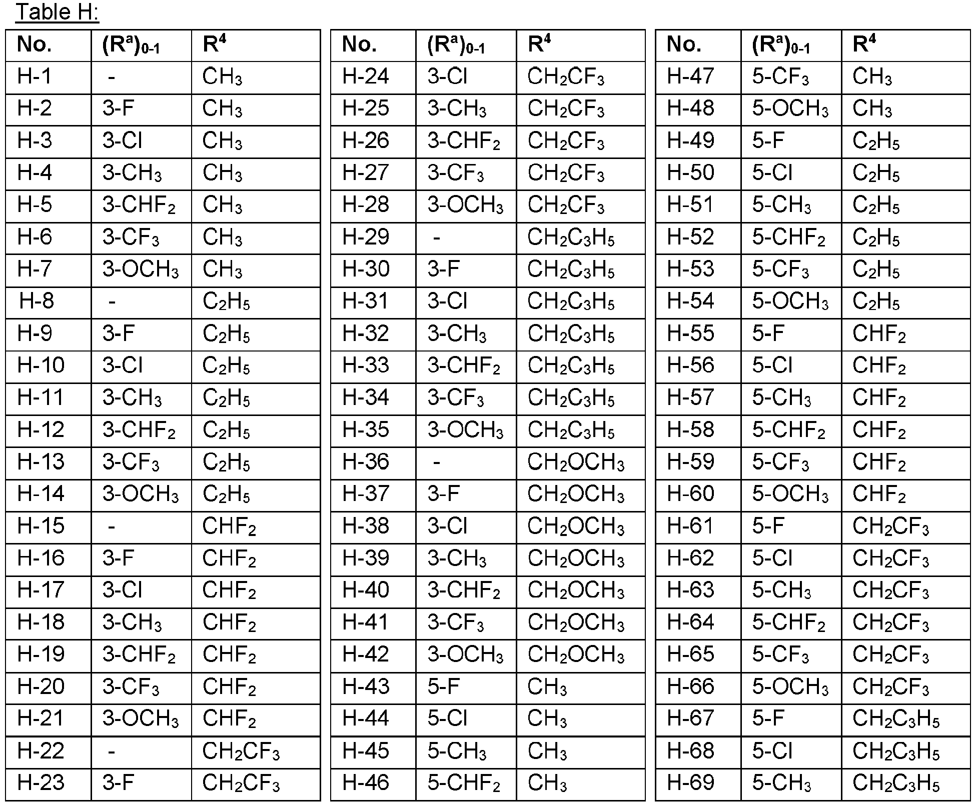

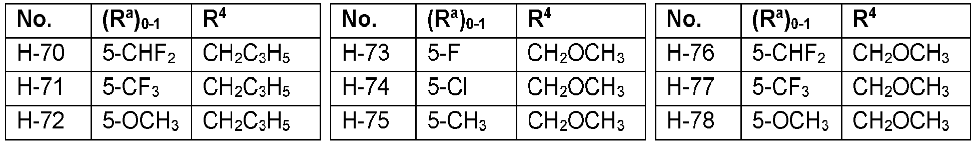

In another embodiment, compounds I are of formula I.A1 wherein Z is pyrazol-3-yl (Z-27), R³ is CH₃ and Q is Q1, and R^{a} and R⁴ are as per any row of Table J below, which compounds are named I.A1-Z27-Q1-J-1 to I.A1-Z27-Q1-J-78.

In another embodiment, compounds I are of formula I.A1 wherein Z is pyrazol-3-yl (Z-27), R³ is CH₃ and Q is Q2, and R^{a} and R⁴ are as per any row of Table J below, which compounds are named I.A1-Z27-Q2-J-1 to I.A1-Z27-Q2-J-78.

In another embodiment, compounds I are of formula I.A1 wherein Z is pyrazol-3-yl (Z-27), R³ is CH₃ and Q is Q3, and R^{a} and R⁴ are as per any row of Table J below, which compounds are named I.A1-Z27-Q3-J-1 to I.A1-Z27-Q3-J-78.

In another embodiment, compounds I are of formula I.A1 wherein Z is pyrazol-3-yl (Z-27), R³ is CH₃ and Q is Q4, and R^{a} and R⁴ are as per any row of Table J below, which compounds are named I.A1-Z27-Q4-J-1 to I.A1-Z27-Q4-J-78.

In another embodiment, compounds I are of formula I.A2 wherein Z is pyrazol-3-yl (Z-27), R³ is CH₃ and Q is Q1, and R^{a} and R⁴ are as per any row of Table J below, which compounds are named I.A2-Z27-Q1-J-1 to I.A2-Z27-Q1-J-78.

In another embodiment, compounds I are of formula I.A2 wherein Z is pyrazol-3-yl (Z-27), R³ is CH₃ and Q is Q2, and R^{a} and R⁴ are as per any row of Table J below, which compounds are named I.A2-Z27-Q2-J-1 to I.A2-Z27-Q2-J-78.

In another embodiment, compounds I are of formula I.A2 wherein Z is pyrazol-3-yl (Z-27), R³ is CH₃ and Q is Q3, and R^{a} and R⁴ are as per any row of Table J below, which compounds are named I.A2-Z27-Q3-J-1 to I.A2-Z27-Q3-J-78.

In another embodiment, compounds I are of formula I.A2 wherein Z is pyrazol-3-yl (Z-27), R³ is CH₃ and Q is Q4, and R^{a} and R⁴ are as per any row of Table J below, which compounds are named I.A2-Z27-Q4-J-1 to I.A2-Z27-Q4-J-78.

In another embodiment, compounds I are of formula I.A1 wherein Z is 1-CH₃-pyrazol-3-yl (Z-30), R³ is CH₃ and Q is Q1, and R^{a} and R⁴ are as per any row of Table J below, which compounds are named I.A1-Z30-Q1-J-1 to I.A1-Z30-Q1-J-78.

In another embodiment, compounds I are of formula I.A1 wherein Z is 1-CH₃-pyrazol-3-yl (Z-30), R³ is CH₃ and Q is Q2, and R^{a} and R⁴ are as per any row of Table J below, which compounds are named I.A1-Z30-Q2-J-1 to I.A1-Z30-Q2-J-78.

In another embodiment, compounds I are of formula I.A1 wherein Z is 1-CH₃-pyrazol-3-yl (Z-30), R³ is CH₃ and Q is Q3, and R^{a} and R⁴ are as per any row of Table J below, which compounds are named I.A1-Z30-Q3-J-1 to I.A1-Z30-Q3-J-78.

In another embodiment, compounds I are of formula I.A1 wherein Z is 1-CH₃-pyrazol-3-yl (Z-30), R³ is CH₃ and Q is Q4, and R^{a} and R⁴ are as per any row of Table J below, which compounds are named I.A1-Z30-Q4-J-1 to I.A1-Z30-Q4-J-78.

In another embodiment, compounds I are of formula I.A2 wherein Z is 1-CH₃-pyrazol-3-yl (Z-30), R³ is CH₃ and Q is Q1, and R^{a} and R⁴ are as per any row of Table J below, which compounds are named I.A2-Z30-Q1-J-1 to I.A2-Z30-Q1-J-78.

In another embodiment, compounds I are of formula I.A2 wherein Z is 1-CH₃-pyrazol-3-yl (Z-30), R³ is CH₃ and Q is Q2, and R^{a} and R⁴ are as per any row of Table J below, which compounds are named I.A2-Z30-Q2-J-1 to I.A2-Z30-Q2-J-78.

In another embodiment, compounds I are of formula I.A2 wherein Z is 1-CH₃-pyrazol-3-yl (Z-30), R³ is CH₃ and Q is Q3, and R^{a} and R⁴ are as per any row of Table J below, which compounds are named I.A2-Z30-Q3-J-1 to I.A2-Z30-Q3-J-78.

In another embodiment, compounds I are of formula I.A2 wherein Z is 1-CH₃-pyrazol-3-yl (Z-30), R³ is CH₃ and Q is Q4, and R^{a} and R⁴ are as per any row of Table J below, which compounds are named I.A2-Z30-Q4-J-1 to I.A2-Z30-Q4-J-78.

In another embodiment, compounds I are of formula I.A1 wherein Z is isoxazol-3-yl (Z-49), R³ is CH₃ and Q is Q1, and R^{a} and R⁴ are as per any row of Table J below, which compounds are named I.A1-Z49-Q1-J-1 to I.A1-Z49-Q1-J-78.

In another embodiment, compounds I are of formula I.A1 wherein Z is isoxazol-3-yl (Z-49), R³ is CH₃ and Q is Q2, and R^{a} and R⁴ are as per any row of Table J below, which compounds are named I.A1-Z49-Q2-J-1 to I.A1-Z49-Q2-J-78.

In another embodiment, compounds I are of formula I.A1 wherein Z is isoxazol-3-yl (Z-49), R³ is CH₃ and Q is Q3, and R^{a} and R⁴ are as per any row of Table J below, which compounds are named I.A1-Z49-Q3-J-1 to I.A1-Z49-Q3-J-78.

In another embodiment, compounds I are of formula I.A1 wherein Z is isoxazol-3-yl (Z-49), R³ is CH₃ and Q is Q4, and R^{a} and R⁴ are as per any row of Table J below, which compounds are named I.A1-Z49-Q4-J-1 to I.A1-Z49-Q4-J-78.

In another embodiment, compounds I are of formula I.A2 wherein Z is isoxazol-3-yl (Z-49), R³ is CH₃ and Q is Q1, and R^{a} and R⁴ are as per any row of Table J below, which compounds are named I.A2-Z49-Q1-J-1 to I.A2-Z49-Q1-J-78.

In another embodiment, compounds I are of formula I.A2 wherein Z is isoxazol-3-yl (Z-49), R³ is CH₃ and Q is Q2, and R^{a} and R⁴ are as per any row of Table J below, which compounds are named I.A2-Z49-Q2-J-1 to I.A2-Z49-Q2-J-78.

In another embodiment, compounds I are of formula I.A2 wherein Z is isoxazol-3-yl (Z-49), R³ is CH₃ and Q is Q3, and R^{a} and R⁴ are as per any row of Table J below, which compounds are named I.A2-Z49-Q3-J-1 to I.A2-Z49-Q3-J-78.

In another embodiment, compounds I are of formula I.A2 wherein Z is isoxazol-3-yl (Z-49), R³ is CH₃ and Q is Q4, and R^{a} and R⁴ are as per any row of Table J below, which compounds are named I.A2-Z49-Q4-J-1 to I.A2-Z49-Q4-J-78.

In another embodiment, compounds I are of formula I.A1 wherein Z is isothiazol-3-yl (Z-51), R³ is CH₃ and Q is Q1, and R^{a} and R⁴ are as per any row of Table J below, which compounds are named I.A1-Z51-Q1-J-1 to I.A1-Z51-Q1-J-78.

In another embodiment, compounds I are of formula I.A1 wherein Z is isothiazol-3-yl (Z-51), R³ is CH₃ and Q is Q2, and R^{a} and R⁴ are as per any row of Table J below, which compounds are named I.A1-Z51-Q2-J-1 to I.A1-Z51-Q2-J-78.

In another embodiment, compounds I are of formula I.A1 wherein Z is isothiazol-3-yl (Z-51), R³ is CH₃ and Q is Q3, and R^{a} and R⁴ are as per any row of Table J below, which compounds are named I.A1-Z51-Q3-J-1 to I.A1-Z51-Q3-J-78.

In another embodiment, compounds I are of formula I.A1 wherein Z is isothiazol-3-yl (Z-51), R³ is CH₃ and Q is Q4, and R^{a} and R⁴ are as per any row of Table J below, which compounds are named I.A1-Z51-Q4-J-1 to I.A1-Z51-Q4-J-78.

In another embodiment, compounds I are of formula I.A2 wherein Z is isothiazol-3-yl (Z-51), R³ is CH₃ and Q is Q1, and R^{a} and R⁴ are as per any row of Table J below, which compounds are named I.A2-Z51-Q1-J-1 to I.A2-Z51-Q1-J-78.

In another embodiment, compounds I are of formula I.A2 wherein Z is isothiazol-3-yl (Z-51), R³ is CH₃ and Q is Q2, and R^{a} and R⁴ are as per any row of Table J below, which compounds are named I.A2-Z51-Q2-J-1 to I.A2-Z51-Q2-J-78.

In another embodiment, compounds I are of formula I.A2 wherein Z is isothiazol-3-yl (Z-51), R³ is CH₃ and Q is Q3, and R^{a} and R⁴ are as per any row of Table J below, which compounds are named I.A2-Z51-Q3-J-1 to I.A2-Z51-Q3-J-78.

In another embodiment, compounds I are of formula I.A2 wherein Z is isothiazol-3-yl (Z-51), R³ is CH₃ and Q is Q4, and R^{a} and R⁴ are as per any row of Table J below, which compounds are named I.A2-Z51-Q4-J-1 to I.A2-Z51-Q4-J-78.

In another embodiment, compounds I are of formula I.A1 wherein Z is oxazol-2-yl (Z-45), R³ is CH₃ and Q is Q1, and R^{a} and R⁴ are as per any row of Table J below, which compounds are named I.A1-Z45-Q1-J-1 to I.A1-Z45-Q1-J-78.

In another embodiment, compounds I are of formula I.A1 wherein Z is oxazol-2-yl (Z-45), R³ is CH₃ and Q is Q2, and R^{a} and R⁴ are as per any row of Table J below, which compounds are named I.A1-Z45-Q2-J-1 to I.A1-Z45-Q2-J-78.

In another embodiment, compounds I are of formula I.A1 wherein Z is oxazol-2-yl (Z-45), R³ is CH₃ and Q is Q3, and R^{a} and R⁴ are as per any row of Table J below, which compounds are named I.A1-Z45-Q3-J-1 to I.A1-Z45-Q3-J-78.

In another embodiment, compounds I are of formula I.A1 wherein Z is oxazol-2-yl (Z-45), R³ is CH₃ and Q is Q4, and R^{a} and R⁴ are as per any row of Table J below, which compounds are named I.A1-Z45-Q4-J-1 to I.A1-Z45-Q4-J-78.

In another embodiment, compounds I are of formula I.A2 wherein Z is oxazol-2-yl (Z-45), R³ is CH₃ and Q is Q1, and R^{a} and R⁴ are as per any row of Table J below, which compounds are named I.A2-Z45-Q1-J-1 to I.A2-Z45-Q1-J-78.

In another embodiment, compounds I are of formula I.A2 wherein Z is oxazol-2-yl (Z-45), R³ is CH₃ and Q is Q2, and R^{a} and R⁴ are as per any row of Table J below, which compounds are named I.A2-Z45-Q2-J-1 to I.A2-Z45-Q2-J-78.

In another embodiment, compounds I are of formula I.A2 wherein Z is oxazol-2-yl (Z-45), R³ is CH₃ and Q is Q3, and R^{a} and R⁴ are as per any row of Table J below, which compounds are named I.A2-Z45-Q3-J-1 to I.A2-Z45-Q3-J-78.

In another embodiment, compounds I are of formula I.A2 wherein Z is oxazol-2-yl (Z-45), R³ is CH₃ and Q is Q4, and R^{a} and R⁴ are as per any row of Table J below, which compounds are named I.A2-Z45-Q4-J-1 to I.A2-Z45-Q4-J-78.

In another embodiment, compounds I are of formula I.A1 wherein Z is thiazol-2-yl (Z-47), R³ is CH₃ and Q is Q1, and R^{a} and R⁴ are as per any row of Table J below, which compounds are named I.A1-Z47-Q1-J-1 to I.A1-Z47-Q1-J-78.

In another embodiment, compounds I are of formula I.A1 wherein Z is thiazol-2-yl (Z-47), R³ is CH₃ and Q is Q2, and R^{a} and R⁴ are as per any row of Table J below, which compounds are named I.A1-Z47-Q2-J-1 to I.A1-Z47-Q2-J-78.

In another embodiment, compounds I are of formula I.A1 wherein Z is thiazol-2-yl (Z-47), R³ is CH₃ and Q is Q3, and R^{a} and R⁴ are as per any row of Table J below, which compounds are named I.A1-Z47-Q3-J-1 to I.A1-Z47-Q3-J-78.

In another embodiment, compounds I are of formula I.A1 wherein Z is thiazol-2-yl (Z-47), R³ is CH₃ and Q is Q4, and R^{a} and R⁴ are as per any row of Table J below, which compounds are named I.A1-Z47-Q4-J-1 to I.A1-Z47-Q4-J-78.

In another embodiment, compounds I are of formula I.A2 wherein Z is thiazol-2-yl (Z-47), R³ is CH₃ and Q is Q1, and R^{a} and R⁴ are as per any row of Table J below, which compounds are named I.A2-Z47-Q1-J-1 to I.A2-Z47-Q1-J-78.

In another embodiment, compounds I are of formula I.A2 wherein Z is thiazol-2-yl (Z-47), R³ is CH₃ and Q is Q2, and R^{a} and R⁴ are as per any row of Table J below, which compounds are named I.A2-Z47-Q2-J-1 to I.A2-Z47-Q2-J-78.

In another embodiment, compounds I are of formula I.A2 wherein Z is thiazol-2-yl (Z-47), R³ is CH₃ and Q is Q3, and R^{a} and R⁴ are as per any row of Table J below, which compounds are named I.A2-Z47-Q3-J-1 to I.A2-Z47-Q3-J-78.

In another embodiment, compounds I are of formula I.A2 wherein Z is thiazol-2-yl (Z-47), R³ is CH₃ and Q is Q4, and R^{a} and R⁴ are as per any row of Table J below, which compounds are named I.A2-Z47-Q4-J-1 to I.A2-Z47-Q4-J-78.

In another embodiment, compounds I are of formula I.B1 wherein Z is pyrazol-3-yl (Z-27) and Q is Q1, and R^{a} and R⁴ are as per any row of Table J below, which compounds are named I.B1-Z27-Q1-J-1 to I.B1-Z27-Q1-J-78.

In another embodiment, compounds I are of formula I.B1 wherein Z is pyrazol-3-yl (Z-27) and Q is Q2, and R^{a} and R⁴ are as per any row of Table J below, which compounds are named I.B1-Z27-Q2-J-1 to I.B1-Z27-Q2-J-78.

In another embodiment, compounds I are of formula I.B1 wherein Z is pyrazol-3-yl (Z-27) and Q is Q3, and R^{a} and R⁴ are as per any row of Table J below, which compounds are named I.B1-Z27-Q3-J-1 to I.B1-Z27-Q3-J-78.

In another embodiment, compounds I are of formula I.B1 wherein Z is pyrazol-3-yl (Z-27) and Q is Q4, and R^{a} and R⁴ are as per any row of Table J below, which compounds are named I.B1-Z27-Q4-J-1 to I.B1-Z27-Q4-J-78.

In another embodiment, compounds I are of formula I.B2 wherein Z is pyrazol-3-yl (Z-27) and Q is Q1, and R^{a} and R⁴ are as per any row of Table J below, which compounds are named I.B2-Z27-Q1-J-1 to I.B2-Z27-Q1-J-78.

In another embodiment, compounds I are of formula I.B2 wherein Z is pyrazol-3-yl (Z-27) and Q is Q2, and R^{a} and R⁴ are as per any row of Table J below, which compounds are named I.B2-Z27-Q2-J-1 to I.B2-Z27-Q2-J-78.

In another embodiment, compounds I are of formula I.B2 wherein Z is pyrazol-3-yl (Z-27) and Q is Q3, and R^{a} and R⁴ are as per any row of Table J below, which compounds are named I.B2-Z27-Q3-J-1 to I.B2-Z27-Q3-J-78.

In another embodiment, compounds I are of formula I.B2 wherein Z is pyrazol-3-yl (Z-27) and Q is Q4, and R^{a} and R⁴ are as per any row of Table J below, which compounds are named I.B2-Z27-Q4-J-1 to I.B2-Z27-Q4-J-78.

In another embodiment, compounds I are of formula I.B1 wherein Z is 1-CH₃-pyrazol-3-yl (Z-30) and Q is Q1, and R^{a} and R⁴ are as per any row of Table J below, which compounds are named I.B1-Z30-Q1-J-1 to I.B1-Z30-Q1-J-78.

In another embodiment, compounds I are of formula I.B1 wherein Z is 1-CH₃-pyrazol-3-yl (Z-30) and Q is Q2, and R^{a} and R⁴ are as per any row of Table J below, which compounds are named I.B1-Z30-Q2-J-1 to I.B1-Z30-Q2-J-78.

In another embodiment, compounds I are of formula I.B1 wherein Z is 1-CH₃-pyrazol-3-yl (Z-30) and Q is Q3, and R^{a} and R⁴ are as per any row of Table J below, which compounds are named I.B1-Z30-Q3-J-1 to I.B1-Z30-Q3-J-78.

In another embodiment, compounds I are of formula I.B1 wherein Z is 1-CH₃-pyrazol-3-yl (Z-30) and Q is Q4, and R^{a} and R⁴ are as per any row of Table J below, which compounds are named I.B1-Z30-Q4-J-1 to I.B1-Z30-Q4-J-78.

In another embodiment, compounds I are of formula I.B2 wherein Z is 1-CH₃-pyrazol-3-yl (Z-30) and Q is Q1, and R^{a} and R⁴ are as per any row of Table J below, which compounds are named I.B2-Z30-Q1-J-1 to I.B2-Z30-Q1-J-78.

In another embodiment, compounds I are of formula I.B2 wherein Z is 1-CH₃-pyrazol-3-yl (Z-30) and Q is Q2, and R^{a} and R⁴ are as per any row of Table J below, which compounds are named I.B2-Z30-Q2-J-1 to I.B2-Z30-Q2-J-78.

In another embodiment, compounds I are of formula I.B2 wherein Z is 1-CH₃-pyrazol-3-yl (Z-30) and Q is Q3, and R^{a} and R⁴ are as per any row of Table J below, which compounds are named I.B2-Z30-Q3-J-1 to I.B2-Z30-Q3-J-78.

In another embodiment, compounds I are of formula I.B2 wherein Z is 1-CH₃-pyrazol-3-yl (Z-30) and Q is Q4, and R^{a} and R⁴ are as per any row of Table J below, which compounds are named I.B2-Z30-Q4-J-1 to I.B2-Z30-Q4-J-78.

In another embodiment, compounds I are of formula I.B1 wherein Z is isoxazol-3-yl (Z-49) and Q is Q1, and R^{a} and R⁴ are as per any row of Table J below, which compounds are named I.B1-Z49-Q1-J-1 to I.B1-Z49-Q1-J-78.

In another embodiment, compounds I are of formula I.B1 wherein Z is isoxazol-3-yl (Z-49) and Q is Q2, and R^{a} and R⁴ are as per any row of Table J below, which compounds are named I.B1-Z49-Q2-J-1 to I.B1-Z49-Q2-J-78.

In another embodiment, compounds I are of formula I.B1 wherein Z is isoxazol-3-yl (Z-49) and Q is Q3, and R^{a} and R⁴ are as per any row of Table J below, which compounds are named I.B1-Z49-Q3-J-1 to I.B1-Z49-Q3-J-78.

In another embodiment, compounds I are of formula I.B1 wherein Z is isoxazol-3-yl (Z-49) and Q is Q4, and R^{a} and R⁴ are as per any row of Table J below, which compounds are named I.B1-Z49-Q4-J-1 to I.B1-Z49-Q4-J-78.

In another embodiment, compounds I are of formula I.B2 wherein Z is isoxazol-3-yl (Z-49) and Q is Q1, and R^{a} and R⁴ are as per any row of Table J below, which compounds are named I.B2-Z49-Q1-J-1 to I.B2-Z49-Q1-J-78.

In another embodiment, compounds I are of formula I.B2 wherein Z is isoxazol-3-yl (Z-49) and Q is Q2, and R^{a} and R⁴ are as per any row of Table J below, which compounds are named I.B2-Z49-Q2-J-1 to I.B2-Z49-Q2-J-78.

In another embodiment, compounds I are of formula I.B2 wherein Z is isoxazol-3-yl (Z-49) and Q is Q3, and R^{a} and R⁴ are as per any row of Table J below, which compounds are named I.B2-Z49-Q3-J-1 to I.B2-Z49-Q3-J-78.

In another embodiment, compounds I are of formula I.B2 wherein Z is isoxazol-3-yl (Z-49) and Q is Q4, and R^{a} and R⁴ are as per any row of Table J below, which compounds are named I.B2-Z49-Q4-J-1 to I.B2-Z49-Q4-J-78.

In another embodiment, compounds I are of formula I.B1 wherein Z is isothiazol-3-yl (Z-51) and Q is Q1, and R^{a} and R⁴ are as per any row of Table J below, which compounds are named I.B1-Z51-Q1-J-1 to I.B1-Z51-Q1-J-78.

In another embodiment, compounds I are of formula I.B1 wherein Z is isothiazol-3-yl (Z-51) and Q is Q2, and R^{a} and R⁴ are as per any row of Table J below, which compounds are named I.B1-Z51-Q2-J-1 to I.B1-Z51-Q2-J-78.

In another embodiment, compounds I are of formula I.B1 wherein Z is isothiazol-3-yl (Z-51) and Q is Q3, and R^{a} and R⁴ are as per any row of Table J below, which compounds are named I.B1-Z51-Q3-J-1 to I.B1-Z51-Q3-J-78.

In another embodiment, compounds I are of formula I.B1 wherein Z is isothiazol-3-yl (Z-51) and Q is Q4, and R^{a} and R⁴ are as per any row of Table J below, which compounds are named I.B1-Z51-Q4-J-1 to I.B1-Z51-Q4-J-78.

In another embodiment, compounds I are of formula I.B2 wherein Z is isothiazol-3-yl (Z-51) and Q is Q1, and R^{a} and R⁴ are as per any row of Table J below, which compounds are named I.B2-Z51-Q1-J-1 to I.B2-Z51-Q1-J-78.

In another embodiment, compounds I are of formula I.B2 wherein Z is isothiazol-3-yl (Z-51) and Q is Q2, and R^{a} and R⁴ are as per any row of Table J below, which compounds are named I.B2-Z51-Q2-J-1 to I.B2-Z51-Q2-J-78.

In another embodiment, compounds I are of formula I.B2 wherein Z is isothiazol-3-yl (Z-51) and Q is Q3, and R^{a} and R⁴ are as per any row of Table J below, which compounds are named I.B2-Z51-Q3-J-1 to I.B2-Z51-Q3-J-78.

In another embodiment, compounds I are of formula I.B2 wherein Z is isothiazol-3-yl (Z-51) and Q is Q4, and R^{a} and R⁴ are as per any row of Table J below, which compounds are named I.B2-Z51-Q4-J-1 to I.B2-Z51-Q4-J-78.

In another embodiment, compounds I are of formula I.B1 wherein Z is oxazol-2-yl (Z-45) and Q is Q1, and R^{a} and R⁴ are as per any row of Table J below, which compounds are named I.B1-Z45-Q1-J-1 to I.B1-Z45-Q1-J-78.

In another embodiment, compounds I are of formula I.B1 wherein Z is oxazol-2-yl (Z-45) and Q is Q2, and R^{a} and R⁴ are as per any row of Table J below, which compounds are named I.B1-Z45-Q2-J-1 to I.B1-Z45-Q2-J-78.

In another embodiment, compounds I are of formula I.B1 wherein Z is oxazol-2-yl (Z-45) and Q is Q3, and R^{a} and R⁴ are as per any row of Table J below, which compounds are named I.B1-Z45-Q3-J-1 to I.B1-Z45-Q3-J-78.

In another embodiment, compounds I are of formula I.B1 wherein Z is oxazol-2-yl (Z-45) and Q is Q4, and R^{a} and R⁴ are as per any row of Table J below, which compounds are named I.B1-Z45-Q4-J-1 to I.B1-Z45-Q4-J-78.

In another embodiment, compounds I are of formula I.B2 wherein Z is oxazol-2-yl (Z-45) and Q is Q1, and R^{a} and R⁴ are as per any row of Table J below, which compounds are named I.B2-Z45-Q1-J-1 to I.B2-Z45-Q1-J-78.

In another embodiment, compounds I are of formula I.B2 wherein Z is oxazol-2-yl (Z-45) and Q is Q2, and R^{a} and R⁴ are as per any row of Table J below, which compounds are named I.B2-Z45-Q2-J-1 to I.B2-Z45-Q2-J-78.

In another embodiment, compounds I are of formula I.B2 wherein Z is oxazol-2-yl (Z-45) and Q is Q3, and R^{a} and R⁴ are as per any row of Table J below, which compounds are named I.B2-Z45-Q3-J-1 to I.B2-Z45-Q3-J-78.

In another embodiment, compounds I are of formula I.B2 wherein Z is oxazol-2-yl (Z-45) and Q is Q4, and R^{a} and R⁴ are as per any row of Table J below, which compounds are named I.B2-Z45-Q4-J-1 to I.B2-Z45-Q4-J-78.

In another embodiment, compounds I are of formula I.B1 wherein Z is thiazol-2-yl (Z-47) and Q is Q1, and R^{a} and R⁴ are as per any row of Table J below, which compounds are named I.B1-Z47-Q1-J-1 to I.B1-Z47-Q1-J-78.

In another embodiment, compounds I are of formula I.B1 wherein Z is thiazol-2-yl (Z-47) and Q is Q2, and R^{a} and R⁴ are as per any row of Table J below, which compounds are named I.B1-Z47-Q2-J-1 to I.B1-Z47-Q2-J-78.

In another embodiment, compounds I are of formula I.B1 wherein Z is thiazol-2-yl (Z-47) and Q is Q3, and R^{a} and R⁴ are as per any row of Table J below, which compounds are named I.B1-Z47-Q3-J-1 to I.B1-Z47-Q3-J-78.

In another embodiment, compounds I are of formula I.B1 wherein Z is thiazol-2-yl (Z-47) and Q is Q4, and R^{a} and R⁴ are as per any row of Table J below, which compounds are named I.B1-Z47-Q4-J-1 to I.B1-Z47-Q4-J-78.

In another embodiment, compounds I are of formula I.B2 wherein Z is thiazol-2-yl (Z-47) and Q is Q1, and R^{a} and R⁴ are as per any row of Table J below, which compounds are named I.B2-Z47-Q1-J-1 to I.B2-Z47-Q1-J-78.

In another embodiment, compounds I are of formula I.B2 wherein Z is thiazol-2-yl (Z-47) and Q is Q2, and R^{a} and R⁴ are as per any row of Table J below, which compounds are named I.B2-Z47-Q2-J-1 to I.B2-Z47-Q2-J-78.

In another embodiment, compounds I are of formula I.B2 wherein Z is thiazol-2-yl (Z-47) and Q is Q3, and R^{a} and R⁴ are as per any row of Table J below, which compounds are named I.B2-Z47-Q3-J-1 to I.B2-Z47-Q3-J-78.

In another embodiment, compounds I are of formula I.B2 wherein Z is thiazol-2-yl (Z-47) and Q is Q4, and R^{a} and R⁴ are as per any row of Table J below, which compounds are named I.B2-Z47-Q4-J-1 to I.B2-Z47-Q4-J-78.

**Table J:**

| **No.** | **(R^{a})₀₋₁** | **R⁴** |
|---|---|---|
| J-1 | - | CH₃ |
| J-2 | 4-F | CH₃ |
| J-3 | 4-CI | CH₃ |
| J-4 | 4-CH₃ | CH₃ |
| J-5 | 4-CHF₂ | CH₃ |
| J-6 | 4-CF₃ | CH₃ |
| J-7 | 4-OCH₃ | CH₃ |
| J-8 | - | C₂H₅ |
| J-9 | 4-F | C₂H₅ |
| J-10 | 4-Cl | C₂H₅ |
| J-11 | 4-CH₃ | C₂H₅ |
| J-12 | 4-CHF₂ | C₂H₅ |
| J-13 | 4-CF₃ | C₂H₅ |
| J-14 | 4-OCH₃ | C₂H₅ |
| J-15 | - | CHF₂ |
| J-16 | 4-F | CHF₂ |
| J-17 | 4-Cl | CHF₂ |
| J-18 | 4-CH₃ | CHF₂ |
| J-19 | 4-CHF₂ | CHF₂ |
| J-20 | 4-CF₃ | CHF₂ |
| J-21 | 4-OCH₃ | CHF₂ |
| J-22 | - | CH₂CF₃ |
| J-23 | 4-F | CH₂CF₃ |
| J-24 | 4-Cl | CH₂CF₃ |
| J-25 | 4-CH₃ | CH₂CF₃ |
| J-26 | 4-CHF₂ | CH₂CF₃ |
| J-27 | 4-CF₃ | CH₂CF₃ |
| J-28 | 4-OCH₃ | CH₂CF₃ |
| J-29 | - | CH₂C₃H₅ |
| J-30 | 4-F | CH₂C₃H₅ |
| J-31 | 4-Cl | CH₂C₃H₅ |
| J-32 | 4-CH₃ | CH₂C₃H₅ |
| J-33 | 4-CHF₂ | CH₂C₃H₅ |
| J-34 | 4-CF₃ | CH₂C₃H₅ |
| J-35 | 4-OCH₃ | CH₂C₃H₅ |
| J-36 | - | CH₂OCH₃ |
| J-37 | 4-F | CH₂OCH₃ |
| J-38 | 4-Cl | CH₂OCH₃ |
| J-39 | 4-CH₃ | CH₂OCH₃ |
| J-40 | 4-CHF₂ | CH₂OCH₃ |
| J-41 | 4-CF₃ | CH₂OCH₃ |
| J-42 | 4-OCH₃ | CH₂OCH₃ |
| J-43 | 5-F | CH₃ |
| J-44 | 5-Cl | CH₃ |
| J-45 | 5-CH₃ | CH₃ |
| J-46 | 5-CHF₂ | CH₃ |
| J-47 | 5-CF₃ | CH₃ |
| J-48 | 5-OCH₃ | CH₃ |
| J-49 | 5-F | C₂H₅ |
| J-50 | 5-Cl | C₂H₅ |
| J-51 | 5-CH₃ | C₂H₅ |
| J-52 | 5-CHF₂ | C₂H₅ |
| J-53 | 5-CF₃ | C₂H₅ |
| J-54 | 5-OCH₃ | C₂H₅ |
| J-55 | 5-F | CHF₂ |
| J-56 | 5-Cl | CHF₂ |
| J-57 | 5-CH₃ | CHF₂ |
| J-58 | 5-CHF₂ | CHF₂ |
| J-59 | 5-CF₃ | CHF₂ |
| J-60 | 5-OCH₃ | CHF₂ |
| J-61 | 5-F | CH₂CF₃ |
| J-62 | 5-Cl | CH₂CF₃ |
| J-63 | 5-CH₃ | CH₂CF₃ |
| J-64 | 5-CHF₂ | CH₂CF₃ |
| J-65 | 5-CF₃ | CH₂CF₃ |
| J-66 | 5-OCH₃ | CH₂CF₃ |
| J-67 | 5-F | CH₂C₃H₅ |
| J-68 | 5-Cl | CH₂C₃H₅ |
| J-69 | 5-CH₃ | CH₂C₃H₅ |
| J-70 | 5-CHF₂ | CH₂C₃H₅ |
| J-71 | 5-CF₃ | CH₂C₃H₅ |
| J-72 | 5-OCH₃ | CH₂C₃H₅ |
| J-73 | 5-F | CH₂OCH₃ |
| J-74 | 5-Cl | CH₂OCH₃ |
| J-75 | 5-CH₃ | CH₂OCH₃ |
| J-76 | 5-CHF₂ | CH₂OCH₃ |
| J-77 | 5-CF₃ | CH₂OCH₃ |
| J-78 | 5-OCH₃ | CH₂OCH₃ |

In another embodiment, compounds I are of formula I.A1 wherein Z is pyrimidin-6-yl (Z-22), R³ is CH₃ and Q is Q1, and R^{a} and R⁴ are as per any row of Table K below, which compounds are named I.A1-Z22-Q1-K-1 to I.A1-Z22-Q1-K-114.

In another embodiment, compounds I are of formula I.A1 wherein Z is pyrimidin-6-yl (Z-22), R³ is CH₃ and Q is Q2, and R^{a} and R⁴ are as per any row of Table K below, which compounds are named I.A1-Z22-Q2-K-1 to I.A1-Z22-Q2-K-114.

In another embodiment, compounds I are of formula I.A1 wherein Z is pyrimidin-6-yl (Z-22), R³ is CH₃ and Q is Q3, and R^{a} and R⁴ are as per any row of Table K below, which compounds are named I.A1-Z22-Q3-K-1 to I.A1-Z22-Q3-K-114.

In another embodiment, compounds I are of formula I.A1 wherein Z is pyrimidin-6-yl (Z-22), R³ is CH₃ and Q is Q4, and R^{a} and R⁴ are as per any row of Table K below, which compounds are named I.A1-Z22-Q4-K-1 to I.A1-Z22-Q4-K-114.

In another embodiment, compounds I are of formula I.A2 wherein Z is pyrimidin-6-yl (Z-22), R³ is CH₃ and Q is Q1, and R^{a} and R⁴ are as per any row of Table K below, which compounds are named I.A2-Z22-Q1-K-1 to I.A2-Z22-Q1-K-114.

In another embodiment, compounds I are of formula I.A2 wherein Z is pyrimidin-6-yl (Z-22), R³ is CH₃ and Q is Q2, and R^{a} and R⁴ are as per any row of Table K below, which compounds are named I.A2-Z22-Q2-K-1 to I.A2-Z22-Q2-K-114.

In another embodiment, compounds I are of formula I.A2 wherein Z is pyrimidin-6-yl (Z-22), R³ is CH₃ and Q is Q3, and R^{a} and R⁴ are as per any row of Table K below, which compounds are named I.A2-Z22-Q3-K-1 to I.A2-Z22-Q3-K-114.

In another embodiment, compounds I are of formula I.A2 wherein Z is pyrimidin-6-yl (Z-22), R³ is CH₃ and Q is Q4, and R^{a} and R⁴ are as per any row of Table K below, which compounds are named I.A2-Z22-Q4-K-1 to I.A2-Z22-Q4-K-114.

In another embodiment, compounds I are of formula I.A1 wherein Z is azol-3-yl (Z-25), R³ is CH₃ and Q is Q1, and R^{a} and R⁴ are as per any row of Table K below, which compounds are named I.A1-Z25-Q1-K-1 to I.A1-Z25-Q1-K-114.

In another embodiment, compounds I are of formula I.A1 wherein Z is azol-3-yl (Z-25), R³ is CH₃ and Q is Q2, and R^{a} and R⁴ are as per any row of Table K below, which compounds are named I.A1-Z25-Q2-K-1 to I.A1-Z25-Q2-K-114.

In another embodiment, compounds I are of formula I.A1 wherein Z is azol-3-yl (Z-25), R³ is CH₃ and Q is Q3, and R^{a} and R⁴ are as per any row of Table K below, which compounds are named I.A1-Z25-Q3-K-1 to I.A1-Z25-Q3-K-114.

In another embodiment, compounds I are of formula I.A1 wherein Z is azol-3-yl (Z-25), R³ is CH₃ and Q is Q4, and R^{a} and R⁴ are as per any row of Table K below, which compounds are named I.A1-Z25-Q4-K-1 to I.A1-Z25-Q4-K-114.

In another embodiment, compounds I are of formula I.A2 wherein Z is azol-3-yl (Z-25), R³ is CH₃ and Q is Q1, and R^{a} and R⁴ are as per any row of Table K below, which compounds are named I.A2-Z25-Q1-K-1 to I.A2-Z25-Q1-K-114.

In another embodiment, compounds I are of formula I.A2 wherein Z is azol-3-yl (Z-25), R³ is CH₃ and Q is Q2, and R^{a} and R⁴ are as per any row of Table K below, which compounds are named I.A2-Z25-Q2-K-1 to I.A2-Z25-Q2-K-114.

In another embodiment, compounds I are of formula I.A2 wherein Z is azol-3-yl (Z-25), R³ is CH₃ and Q is Q3, and R^{a} and R⁴ are as per any row of Table K below, which compounds are named I.A2-Z25-Q3-K-1 to I.A2-Z25-Q3-K-114.

In another embodiment, compounds I are of formula I.A2 wherein Z is azol-3-yl (Z-25), R³ is CH₃ and Q is Q4, and R^{a} and R⁴ are as per any row of Table K below, which compounds are named I.A2-Z25-Q4-K-1 to I.A2-Z25-Q4-K-114.

In another embodiment, compounds I are of formula I.A1 wherein Z is imidazol-1-yl (Z-33), R³ is CH₃ and Q is Q1, and R^{a} and R⁴ are as per any row of Table K below, which compounds are named I.A1-Z33-Q1-K-1 to I.A1-Z33-Q1-K-114.

In another embodiment, compounds I are of formula I.A1 wherein Z is imidazol-1-yl (Z-33), R³ is CH₃ and Q is Q2, and R^{a} and R⁴ are as per any row of Table K below, which compounds are named I.A1-Z33-Q2-K-1 to I.A1-Z33-Q2-K-114.

In another embodiment, compounds I are of formula I.A1 wherein Z is imidazol-1-yl (Z-33), R³ is CH₃ and Q is Q3, and R^{a} and R⁴ are as per any row of Table K below, which compounds are named I.A1-Z33-Q3-K-1 to I.A1-Z33-Q3-K-114.

In another embodiment, compounds I are of formula I.A1 wherein Z is imidazol-1-yl (Z-33), R³ is CH₃ and Q is Q4, and R^{a} and R⁴ are as per any row of Table K below, which compounds are named I.A1-Z33-Q4-K-1 to I.A1-Z33-Q4-K-114.

In another embodiment, compounds I are of formula I.A2 wherein Z is imidazol-1-yl (Z-33), R³ is CH₃ and Q is Q1, and R^{a} and R⁴ are as per any row of Table K below, which compounds are named I.A2-Z33-Q1-K-1 to I.A2-Z33-Q1-K-114.

In another embodiment, compounds I are of formula I.A2 wherein Z is imidazol-1-yl (Z-33), R³ is CH₃ and Q is Q2, and R^{a} and R⁴ are as per any row of Table K below, which compounds are named I.A2-Z33-Q2-K-1 to I.A2-Z33-Q2-K-114.

In another embodiment, compounds I are of formula I.A2 wherein Z is imidazol-1-yl (Z-33), R³ is CH₃ and Q is Q3, and R^{a} and R⁴ are as per any row of Table K below, which compounds are named I.A2-Z33-Q3-K-1 to I.A2-Z33-Q3-K-114.

In another embodiment, compounds I are of formula I.A2 wherein Z is imidazol-1-yl (Z-33), R³ is CH₃ and Q is Q4, and R^{a} and R⁴ are as per any row of Table K below, which compounds are named I.A2-Z33-Q4-K-1 to I.A2-Z33-Q4-K-114.

In another embodiment, compounds I are of formula I.A1 wherein Z is furan-3-yl (Z-54), R³ is CH₃ and Q is Q1, and R^{a} and R⁴ are as per any row of Table K below, which compounds are named I.A1-Z54-Q1-K-1 to I.A1-Z54-Q1-K-114.

In another embodiment, compounds I are of formula I.A1 wherein Z is furan-3-yl (Z-54), R³ is CH₃ and Q is Q2, and R^{a} and R⁴ are as per any row of Table K below, which compounds are named I.A1-Z54-Q2-K-1 to I.A1-Z54-Q2-K-114.

In another embodiment, compounds I are of formula I.A1 wherein Z is furan-3-yl (Z-54), R³ is CH₃ and Q is Q3, and R^{a} and R⁴ are as per any row of Table K below, which compounds are named I.A1-Z54-Q3-K-1 to I.A1-Z54-Q3-K-114.

In another embodiment, compounds I are of formula I.A1 wherein Z is furan-3-yl (Z-54), R³ is CH₃ and Q is Q4, and R^{a} and R⁴ are as per any row of Table K below, which compounds are named I.A1-Z54-Q4-K-1 to I.A1-Z54-Q4-K-114.

In another embodiment, compounds I are of formula I.A2 wherein Z is furan-3-yl (Z-54), R³ is CH₃ and Q is Q1, and R^{a} and R⁴ are as per any row of Table K below, which compounds are named I.A2-Z54-Q1-K-1 to I.A2-Z54-Q1-K-114.

In another embodiment, compounds I are of formula I.A2 wherein Z is furan-3-yl (Z-54), R³ is CH₃ and Q is Q2, and R^{a} and R⁴ are as per any row of Table K below, which compounds are named I.A2-Z54-Q2-K-1 to I.A2-Z54-Q2-K-114.

In another embodiment, compounds I are of formula I.A2 wherein Z is furan-3-yl (Z-54), R³ is CH₃ and Q is Q3, and R^{a} and R⁴ are as per any row of Table K below, which compounds are named I.A2-Z54-Q3-K-1 to I.A2-Z54-Q3-K-114.

In another embodiment, compounds I are of formula I.A2 wherein Z is furan-3-yl (Z-54), R³ is CH₃ and Q is Q4, and R^{a} and R⁴ are as per any row of Table K below, which compounds are named I.A2-Z54-Q4-K-1 to I.A2-Z54-Q4-K-114.

In another embodiment, compounds I are of formula I.A1 wherein Z is thien-3-yl (Z-58), R³ is CH₃ and Q is Q1, and R^{a} and R⁴ are as per any row of Table K below, which compounds are named I.A1-Z58-Q1-K-1 to I.A1-Z58-Q1-K-114.

In another embodiment, compounds I are of formula I.A1 wherein Z is thien-3-yl (Z-58), R³ is CH₃ and Q is Q2, and R^{a} and R⁴ are as per any row of Table K below, which compounds are named I.A1-Z58-Q2-K-1 to I.A1-Z58-Q2-K-114.

In another embodiment, compounds I are of formula I.A1 wherein Z is thien-3-yl (Z-58), R³ is CH₃ and Q is Q3, and R^{a} and R⁴ are as per any row of Table K below, which compounds are named I.A1-Z58-Q3-K-1 to I.A1-Z58-Q3-K-114.

In another embodiment, compounds I are of formula I.A1 wherein Z is thien-3-yl (Z-58), R³ is CH₃ and Q is Q4, and R^{a} and R⁴ are as per any row of Table K below, which compounds are named I.A1-Z58-Q4-K-1 to I.A1-Z58-Q4-K-114.

In another embodiment, compounds I are of formula I.A2 wherein Z is thien-3-yl (Z-58), R³ is CH₃ and Q is Q1, and R^{a} and R⁴ are as per any row of Table K below, which compounds are named I.A2-Z58-Q1-K-1 to I.A2-Z58-Q1-K-114.

In another embodiment, compounds I are of formula I.A2 wherein Z is thien-3-yl (Z-58), R³ is CH₃ and Q is Q2, and R^{a} and R⁴ are as per any row of Table K below, which compounds are named I.A2-Z58-Q2-K-1 to I.A2-Z58-Q2-K-114.

In another embodiment, compounds I are of formula I.A2 wherein Z is thien-3-yl (Z-58), R³ is CH₃ and Q is Q3, and R^{a} and R⁴ are as per any row of Table K below, which compounds are named I.A2-Z58-Q3-K-1 to I.A2-Z58-Q3-K-114.

In another embodiment, compounds I are of formula I.A2 wherein Z is thien-3-yl (Z-58), R³ is CH₃ and Q is Q4, and R^{a} and R⁴ are as per any row of Table K below, which compounds are named I.A2-Z58-Q4-K-1 to I.A2-Z58-Q4-K-114.

In another embodiment, compounds I are of formula I.B1 wherein Z is pyrimidin-6-yl (Z-22) and Q is Q1, and R^{a} and R⁴ are as per any row of Table K below, which compounds are named I.B1-Z22-Q1-K-1 to I.B1-Z22-Q1-K-114.

In another embodiment, compounds I are of formula I.B1 wherein Z is pyrimidin-6-yl (Z-22) and Q is Q2, and R^{a} and R⁴ are as per any row of Table K below, which compounds are named I.B1-Z22-Q2-K-1 to I.B1-Z22-Q2-K-114.

In another embodiment, compounds I are of formula I.B1 wherein Z is pyrimidin-6-yl (Z-22) and Q is Q3, and R^{a} and R⁴ are as per any row of Table K below, which compounds are named I.B1-Z22-Q3-K-1 to I.B1-Z22-Q3-K-114.

In another embodiment, compounds I are of formula I.B1 wherein Z is pyrimidin-6-yl (Z-22) and Q is Q4, and R^{a} and R⁴ are as per any row of Table K below, which compounds are named I.B1-Z22-Q4-K-1 to I.B1-Z22-Q4-K-114.

In another embodiment, compounds I are of formula I.B2 wherein Z is pyrimidin-6-yl (Z-22) and Q is Q1, and R^{a} and R⁴ are as per any row of Table K below, which compounds are named I.B2-Z22-Q1-K-1 to I.B2-Z22-Q1-K-114.

In another embodiment, compounds I are of formula I.B2 wherein Z is pyrimidin-6-yl (Z-22) and Q is Q2, and R^{a} and R⁴ are as per any row of Table K below, which compounds are named I.B2-Z22-Q2-K-1 to I.B2-Z22-Q2-K-114.

In another embodiment, compounds I are of formula I.B2 wherein Z is pyrimidin-6-yl (Z-22) and Q is Q3, and R^{a} and R⁴ are as per any row of Table K below, which compounds are named I.B2-Z22-Q3-K-1 to I.B2-Z22-Q3-K-114.

In another embodiment, compounds I are of formula I.B2 wherein Z is pyrimidin-6-yl (Z-22) and Q is Q4, and R^{a} and R⁴ are as per any row of Table K below, which compounds are named I.B2-Z22-Q4-K-1 to I.B2-Z22-Q4-K-114.

In another embodiment, compounds I are of formula I.B1 wherein Z is azol-3-yl (Z-25) and Q is Q1, and R^{a} and R⁴ are as per any row of Table K below, which compounds are named I.B1-Z25-Q1-K-1 to I.B1-Z25-Q1-K-114.

In another embodiment, compounds I are of formula I.B1 wherein Z is azol-3-yl (Z-25) and Q is Q2, and R^{a} and R⁴ are as per any row of Table K below, which compounds are named I.B1-Z25-Q2-K-1 to I.B1-Z25-Q2-K-114.

In another embodiment, compounds I are of formula I.B1 wherein Z is azol-3-yl (Z-25) and Q is Q3, and R^{a} and R⁴ are as per any row of Table K below, which compounds are named I.B1-Z25-Q3-K-1 to I.B1-Z25-Q3-K-114.

In another embodiment, compounds I are of formula I.B1 wherein Z is azol-3-yl (Z-25) and Q is Q4, and R^{a} and R⁴ are as per any row of Table K below, which compounds are named I.B1-Z25-Q4-K-1 to I.B1-Z25-Q4-K-114.

In another embodiment, compounds I are of formula I.B2 wherein Z is azol-3-yl (Z-25) and Q is Q1, and R^{a} and R⁴ are as per any row of Table K below, which compounds are named I.B2-Z25-Q1-K-1 to I.B2-Z25-Q1-K-114.

In another embodiment, compounds I are of formula I.B2 wherein Z is azol-3-yl (Z-25) and Q is Q2, and R^{a} and R⁴ are as per any row of Table K below, which compounds are named I.B2-Z25-Q2-K-1 to I.B2-Z25-Q2-K-114.

In another embodiment, compounds I are of formula I.B2 wherein Z is azol-3-yl (Z-25) and Q is Q3, and R^{a} and R⁴ are as per any row of Table K below, which compounds are named I.B2-Z25-Q3-K-1 to I.B2-Z25-Q3-K-114.

In another embodiment, compounds I are of formula I.B2 wherein Z is azol-3-yl (Z-25) and Q is Q4, and R^{a} and R⁴ are as per any row of Table K below, which compounds are named I.B2-Z25-Q4-K-1 to I.B2-Z25-Q4-K-114.

In another embodiment, compounds I are of formula I.B1 wherein Z is imidazol-1-yl (Z-33) and Q is Q1, and R^{a} and R⁴ are as per any row of Table K below, which compounds are named I.B1-Z33-Q1-K-1 to I.B1-Z33-Q1-K-114.

In another embodiment, compounds I are of formula I.B1 wherein Z is imidazol-1-yl (Z-33) and Q is Q2, and R^{a} and R⁴ are as per any row of Table K below, which compounds are named I.B1-Z33-Q2-K-1 to I.B1-Z33-Q2-K-114.

In another embodiment, compounds I are of formula I.B1 wherein Z is imidazol-1-yl (Z-33) and Q is Q3, and R^{a} and R⁴ are as per any row of Table K below, which compounds are named I.B1-Z33-Q3-K-1 to I.B1-Z33-Q3-K-114.

In another embodiment, compounds I are of formula I.B1 wherein Z is imidazol-1-yl (Z-33) and Q is Q4, and R^{a} and R⁴ are as per any row of Table K below, which compounds are named I.B1-Z33-Q4-K-1 to I.B1-Z33-Q4-K-114.

In another embodiment, compounds I are of formula I.B2 wherein Z is imidazol-1-yl (Z-33) and Q is Q1, and R^{a} and R⁴ are as per any row of Table K below, which compounds are named I.B2-Z33-Q1-K-1 to I.B2-Z33-Q1-K-114.

In another embodiment, compounds I are of formula I.B2 wherein Z is imidazol-1-yl (Z-33) and Q is Q2, and R^{a} and R⁴ are as per any row of Table K below, which compounds are named I.B2-Z33-Q2-K-1 to I.B2-Z33-Q2-K-114.

In another embodiment, compounds I are of formula I.B2 wherein Z is imidazol-1-yl (Z-33) and Q is Q3, and R^{a} and R⁴ are as per any row of Table K below, which compounds are named I.B2-Z33-Q3-K-1 to I.B2-Z33-Q3-K-114.

In another embodiment, compounds I are of formula I.B2 wherein Z is imidazol-1-yl (Z-33) and Q is Q4, and R^{a} and R⁴ are as per any row of Table K below, which compounds are named I.B2-Z33-Q4-K-1 to I.B2-Z33-Q4-K-114.

In another embodiment, compounds I are of formula I.B1 wherein Z is furan-3-yl (Z-54) and Q is Q1, and R^{a} and R⁴ are as per any row of Table K below, which compounds are named I.B1-Z54-Q1-K-1 to I.B1-Z54-Q1-K-114.

In another embodiment, compounds I are of formula I.B1 wherein Z is furan-3-yl (Z-54) and Q is Q2, and R^{a} and R⁴ are as per any row of Table K below, which compounds are named I.B1-Z54-Q2-K-1 to I.B1-Z54-Q2-K-114.

In another embodiment, compounds I are of formula I.B1 wherein Z is furan-3-yl (Z-54) and Q is Q3, and R^{a} and R⁴ are as per any row of Table K below, which compounds are named I.B1-Z54-Q3-K-1 to I.B1-Z54-Q3-K-114.

In another embodiment, compounds I are of formula I.B1 wherein Z is furan-3-yl (Z-54) and Q is Q4, and R^{a} and R⁴ are as per any row of Table K below, which compounds are named I.B1-Z54-Q4-K-1 to I.B1-Z54-Q4-K-114.

In another embodiment, compounds I are of formula I.B2 wherein Z is furan-3-yl (Z-54) and Q is Q1, and R^{a} and R⁴ are as per any row of Table K below, which compounds are named I.B2-Z54-Q1-K-1 to I.B2-Z54-Q1-K-114.

In another embodiment, compounds I are of formula I.B2 wherein Z is furan-3-yl (Z-54) and Q is Q2, and R^{a} and R⁴ are as per any row of Table K below, which compounds are named I.B2-Z54-Q2-K-1 to I.B2-Z54-Q2-K-114.

In another embodiment, compounds I are of formula I.B2 wherein Z is furan-3-yl (Z-54) and Q is Q3, and R^{a} and R⁴ are as per any row of Table K below, which compounds are named I.B2-Z54-Q3-K-1 to I.B2-Z54-Q3-K-114.

In another embodiment, compounds I are of formula I.B2 wherein Z is furan-3-yl (Z-54) and Q is Q4, and R^{a} and R⁴ are as per any row of Table K below, which compounds are named I.B2-Z54-Q4-K-1 to I.B2-Z54-Q4-K-114.

In another embodiment, compounds I are of formula I.B1 wherein Z is thien-3-yl (Z-58) and Q is Q1, and R^{a} and R⁴ are as per any row of Table K below, which compounds are named I.B1-Z58-Q1-K-1 to I.B1-Z58-Q1-K-114.

In another embodiment, compounds I are of formula I.B1 wherein Z is thien-3-yl (Z-58) and Q is Q2, and R^{a} and R⁴ are as per any row of Table K below, which compounds are named I.B1-Z58-Q2-K-1 to I.B1-Z58-Q2-K-114.

In another embodiment, compounds I are of formula I.B1 wherein Z is thien-3-yl (Z-58) and Q is Q3, and R^{a} and R⁴ are as per any row of Table K below, which compounds are named I.B1-Z58-Q3-K-1 to I.B1-Z58-Q3-K-114.

In another embodiment, compounds I are of formula I.B1 wherein Z is thien-3-yl (Z-58) and Q is Q4, and R^{a} and R⁴ are as per any row of Table K below, which compounds are named I.B1-Z58-Q4-K-1 to I.B1-Z58-Q4-K-114.

In another embodiment, compounds I are of formula I.B2 wherein Z is thien-3-yl (Z-58) and Q is Q1, and R^{a} and R⁴ are as per any row of Table K below, which compounds are named I.B2-Z58-Q1-K-1 to I.B2-Z58-Q1-K-114.

In another embodiment, compounds I are of formula I.B2 wherein Z is thien-3-yl (Z-58) and Q is Q2, and R^{a} and R⁴ are as per any row of Table K below, which compounds are named I.B2-Z58-Q2-K-1 to I.B2-Z58-Q2-K-114.

In another embodiment, compounds I are of formula I.B2 wherein Z is thien-3-yl (Z-58) and Q is Q3, and R^{a} and R⁴ are as per any row of Table K below, which compounds are named I.B2-Z58-Q3-K-1 to I.B2-Z58-Q3-K-114.

In another embodiment, compounds I are of formula I.B2 wherein Z is thien-3-yl (Z-58) and Q is Q4, and R^{a} and R⁴ are as per any row of Table K below, which compounds are named I.B2-Z58-Q4-K-1 to I.B2-Z58-Q4-K-114.

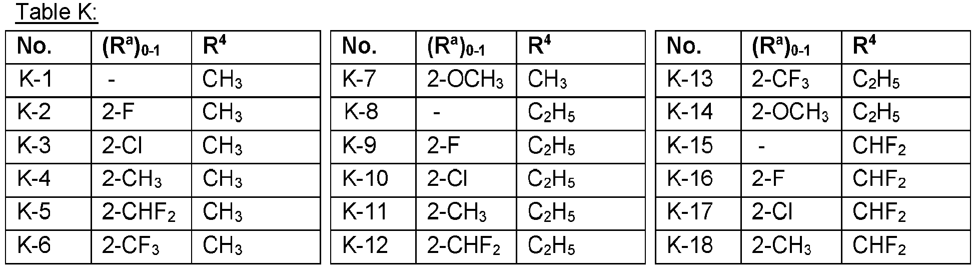

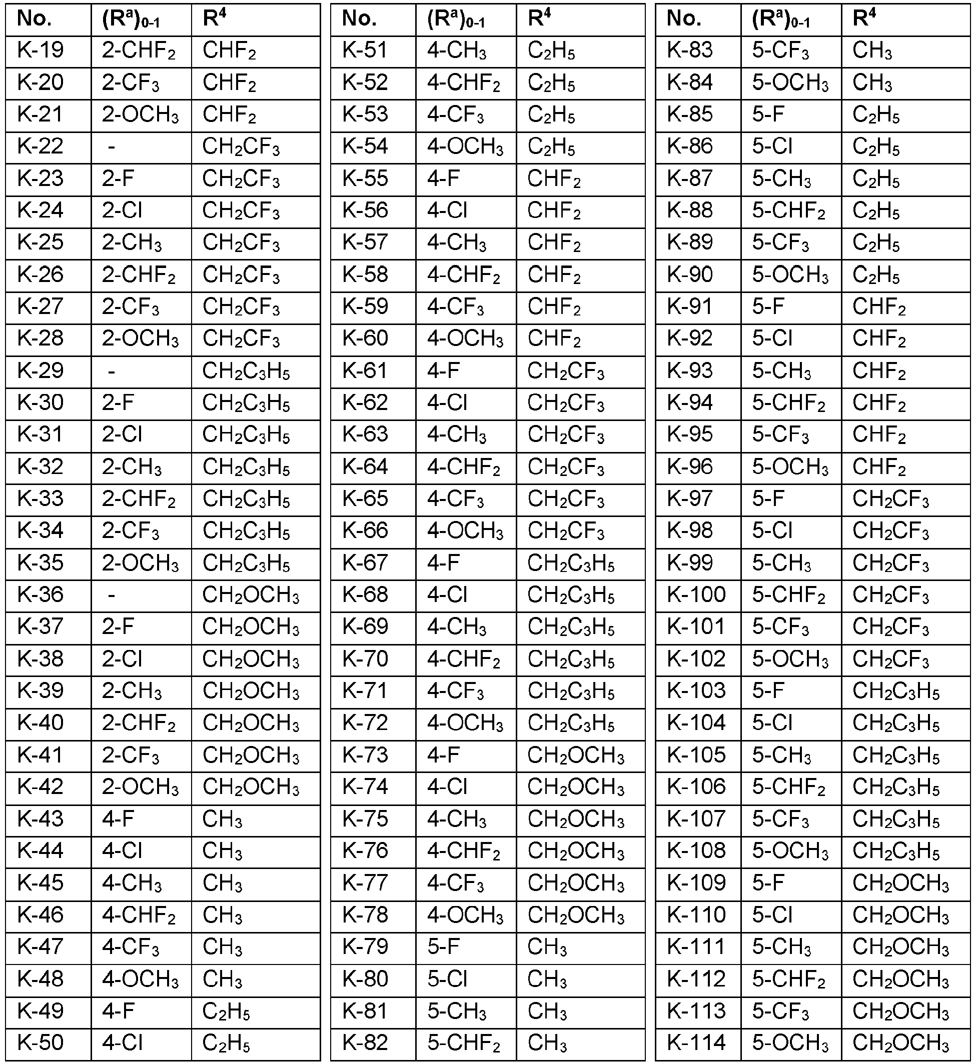

In another embodiment, compounds I are of formula I.A1 wherein Z is pyrimidin-5-yl (Z-21), R³ is CH₃ and Q is Q1, and R^{a} and R⁴ are as per any row of Table L below, which compounds are named I.A1-Z21-Q1-L-1 to I.A1-Z21-Q1-L-114.

In another embodiment, compounds I are of formula I.A1 wherein Z is pyrimidin-5-yl (Z-21), R³ is CH₃ and Q is Q2, and R^{a} and R⁴ are as per any row of Table L below, which compounds are named I.A1-Z21-Q2-L-1 to I.A1-Z21-Q2-L-114.

In another embodiment, compounds I are of formula I.A1 wherein Z is pyrimidin-5-yl (Z-21), R³ is CH₃ and Q is Q3, and R^{a} and R⁴ are as per any row of Table L below, which compounds are named I.A1-Z21-Q3-L-1 to I.A1-Z21-Q3-L-114.

In another embodiment, compounds I are of formula I.A1 wherein Z is pyrimidin-5-yl (Z-21), R³ is CH₃ and Q is Q4, and R^{a} and R⁴ are as per any row of Table L below, which compounds are named I.A1-Z21-Q4-L-1 to I.A1-Z21-Q4-L-114.

In another embodiment, compounds I are of formula I.A2 wherein Z is pyrimidin-5-yl (Z-21), R³ is CH₃ and Q is Q1, and R^{a} and R⁴ are as per any row of Table L below, which compounds are named I.A2-Z21-Q1-L-1 to I.A2-Z21-Q1-L-114.

In another embodiment, compounds I are of formula I.A2 wherein Z is pyrimidin-5-yl (Z-21), R³ is CH₃ and Q is Q2, and R^{a} and R⁴ are as per any row of Table L below, which compounds are named I.A2-Z21-Q2-L-1 to I.A2-Z21-Q2-L-114.

In another embodiment, compounds I are of formula I.A2 wherein Z is pyrimidin-5-yl (Z-21), R³ is CH₃ and Q is Q3, and R^{a} and R⁴ are as per any row of Table L below, which compounds are named I.A2-Z21-Q3-L-1 to I.A2-Z21-Q3-L-114.

In another embodiment, compounds I are of formula I.A2 wherein Z is pyrimidin-5-yl (Z-21), R³ is CH₃ and Q is Q4, and R^{a} and R⁴ are as per any row of Table L below, which compounds are named I.A2-Z21-Q4-L-1 to I.A2-Z21-Q4-L-114.

In another embodiment, compounds I are of formula I.A1 wherein Z is imidazol-5-yl (Z-36), R³ is CH₃ and Q is Q1, and R^{a} and R⁴ are as per any row of Table L below, which compounds are named I.A1-Z36-Q1-L-1 to I.A1-Z36-Q1-L-114.

In another embodiment, compounds I are of formula I.A1 wherein Z is imidazol-5-yl (Z-36), R³ is CH₃ and Q is Q2, and R^{a} and R⁴ are as per any row of Table L below, which compounds are named I.A1-Z36-Q2-L-1 to I.A1-Z36-Q2-L-114.

In another embodiment, compounds I are of formula I.A1 wherein Z is imidazol-5-yl (Z-36), R³ is CH₃ and Q is Q3, and R^{a} and R⁴ are as per any row of Table L below, which compounds are named I.A1-Z36-Q3-L-1 to I.A1-Z36-Q3-L-114.

In another embodiment, compounds I are of formula I.A1 wherein Z is imidazol-5-yl (Z-36), R³ is CH₃ and Q is Q4, and R^{a} and R⁴ are as per any row of Table L below, which compounds are named I.A1-Z36-Q4-L-1 to I.A1-Z36-Q4-L-114.

In another embodiment, compounds I are of formula I.A2 wherein Z is imidazol-5-yl (Z-36), R³ is CH₃ and Q is Q1, and R^{a} and R⁴ are as per any row of Table L below, which compounds are named I.A2-Z36-Q1-L-1 to I.A2-Z36-Q1-L-114.

In another embodiment, compounds I are of formula I.A2 wherein Z is imidazol-5-yl (Z-36), R³ is CH₃ and Q is Q2, and R^{a} and R⁴ are as per any row of Table L below, which compounds are named I.A2-Z36-Q2-L-1 to I.A2-Z36-Q2-L-114.

In another embodiment, compounds I are of formula I.A2 wherein Z is imidazol-5-yl (Z-36), R³ is CH₃ and Q is Q3, and R^{a} and R⁴ are as per any row of Table L below, which compounds are named I.A2-Z36-Q3-L-1 to I.A2-Z36-Q3-L-114.

In another embodiment, compounds I are of formula I.A2 wherein Z is imidazol-5-yl (Z-36), R³ is CH₃ and Q is Q4, and R^{a} and R⁴ are as per any row of Table L below, which compounds are named I.A2-Z36-Q4-L-1 to I.A2-Z36-Q4-L-114.

In another embodiment, compounds I are of formula I.A1 wherein Z is 1-CH₃-imidazol-5-yl (Z-39), R³ is CH₃ and Q is Q1, and R^{a} and R⁴ are as per any row of Table L below, which compounds are named I.A1-Z39-Q1-L-1 to I.A1-Z39-Q1-L-114.

In another embodiment, compounds I are of formula I.A1 wherein Z is 1-CH₃-imidazol-5-yl (Z-39), R³ is CH₃ and Q is Q2, and R^{a} and R⁴ are as per any row of Table L below, which compounds are named I.A1-Z39-Q2-L-1 to I.A1-Z39-Q2-L-114.

In another embodiment, compounds I are of formula I.A1 wherein Z is 1-CH₃-imidazol-5-yl (Z-39), R³ is CH₃ and Q is Q3, and R^{a} and R⁴ are as per any row of Table L below, which compounds are named I.A1-Z39-Q3-L-1 to I.A1-Z39-Q3-L-114.

In another embodiment, compounds I are of formula I.A1 wherein Z is 1-CH₃-imidazol-5-yl (Z-39), R³ is CH₃ and Q is Q4, and R^{a} and R⁴ are as per any row of Table L below, which compounds are named I.A1-Z39-Q4-L-1 to I.A1-Z39-Q4-L-114.

In another embodiment, compounds I are of formula I.A2 wherein Z is 1-CH₃-imidazol-5-yl (Z-39), R³ is CH₃ and Q is Q1, and R^{a} and R⁴ are as per any row of Table L below, which compounds are named I.A2-Z39-Q1-L-1 to I.A2-Z39-Q1-L-114.

In another embodiment, compounds I are of formula I.A2 wherein Z is 1-CH₃-imidazol-5-yl (Z-39), R³ is CH₃ and Q is Q2, and R^{a} and R⁴ are as per any row of Table L below, which compounds are named I.A2-Z39-Q2-L-1 to I.A2-Z39-Q2-L-114.

In another embodiment, compounds I are of formula I.A2 wherein Z is 1-CH₃-imidazol-5-yl (Z-39), R³ is CH₃ and Q is Q3, and R^{a} and R⁴ are as per any row of Table L below, which compounds are named I.A2-Z39-Q3-L-1 to I.A2-Z39-Q3-L-114.

In another embodiment, compounds I are of formula I.A2 wherein Z is 1-CH₃-imidazol-5-yl (Z-39), R³ is CH₃ and Q is Q4, and R^{a} and R⁴ are as per any row of Table L below, which compounds are named I.A2-Z39-Q4-L-1 to I.A2-Z39-Q4-L-114.

In another embodiment, compounds I are of formula I.B1 wherein Z is pyrimidin-5-yl (Z-21) and Q is Q1, and R^{a} and R⁴ are as per any row of Table L below, which compounds are named I.B1-Z21-Q1-L-1 to I.B1-Z21-Q1-L-114.

In another embodiment, compounds I are of formula I.B1 wherein Z is pyrimidin-5-yl (Z-21) and Q is Q2, and R^{a} and R⁴ are as per any row of Table L below, which compounds are named I.B1-Z21-Q2-L-1 to I.B1-Z21-Q2-L-114.

In another embodiment, compounds I are of formula I.B1 wherein Z is pyrimidin-5-yl (Z-21) and Q is Q3, and R^{a} and R⁴ are as per any row of Table L below, which compounds are named I.B1-Z21-Q3-L-1 to I.B1-Z21-Q3-L-114.

In another embodiment, compounds I are of formula I.B1 wherein Z is pyrimidin-5-yl (Z-21) and Q is Q4, and R^{a} and R⁴ are as per any row of Table L below, which compounds are named I.B1-Z21-Q4-L-1 to I.B1-Z21-Q4-L-114.

In another embodiment, compounds I are of formula I.B2 wherein Z is pyrimidin-5-yl (Z-21) and Q is Q1, and R^{a} and R⁴ are as per any row of Table L below, which compounds are named I.B2-Z21-Q1-L-1 to I.B2-Z21-Q1-L-114.

In another embodiment, compounds I are of formula I.B2 wherein Z is pyrimidin-5-yl (Z-21) and Q is Q2, and R^{a} and R⁴ are as per any row of Table L below, which compounds are named I.B2-Z21-Q2-L-1 to I.B2-Z21-Q2-L-114.

In another embodiment, compounds I are of formula I.B2 wherein Z is pyrimidin-5-yl (Z-21) and Q is Q3, and R^{a} and R⁴ are as per any row of Table L below, which compounds are named I.B2-Z21-Q3-L-1 to I.B2-Z21-Q3-L-114.

In another embodiment, compounds I are of formula I.B2 wherein Z is pyrimidin-5-yl (Z-21) and Q is Q4, and R^{a} and R⁴ are as per any row of Table L below, which compounds are named I.B2-Z21-Q4-L-1 to I.B2-Z21-Q4-L-114.

In another embodiment, compounds I are of formula I.B1 wherein Z is imidazol-5-yl (Z-36) and Q is Q1, and R^{a} and R⁴ are as per any row of Table L below, which compounds are named I.B1-Z36-Q1-L-1 to I.B1-Z36-Q1-L-114.

In another embodiment, compounds I are of formula I.B1 wherein Z is imidazol-5-yl (Z-36) and Q is Q2, and R^{a} and R⁴ are as per any row of Table L below, which compounds are named I.B1-Z36-Q2-L-1 to I.B1-Z36-Q2-L-114.

In another embodiment, compounds I are of formula I.B1 wherein Z is imidazol-5-yl (Z-36) and Q is Q3, and R^{a} and R⁴ are as per any row of Table L below, which compounds are named I.B1-Z36-Q3-L-1 to I.B1-Z36-Q3-L-114.

In another embodiment, compounds I are of formula I.B1 wherein Z is imidazol-5-yl (Z-36) and Q is Q4, and R^{a} and R⁴ are as per any row of Table L below, which compounds are named I.B1-Z36-Q4-L-1 to I.B1-Z36-Q4-L-114.

In another embodiment, compounds I are of formula I.B2 wherein Z is imidazol-5-yl (Z-36) and Q is Q1, and R^{a} and R⁴ are as per any row of Table L below, which compounds are named I.B2-Z36-Q1-L-1 to I.B2-Z36-Q1-L-114.

In another embodiment, compounds I are of formula I.B2 wherein Z is imidazol-5-yl (Z-36) and Q is Q2, and R^{a} and R⁴ are as per any row of Table L below, which compounds are named I.B2-Z36-Q2-L-1 to I.B2-Z36-Q2-L-114.

In another embodiment, compounds I are of formula I.B2 wherein Z is imidazol-5-yl (Z-36) and Q is Q3, and R^{a} and R⁴ are as per any row of Table L below, which compounds are named I.B2-Z36-Q3-L-1 to I.B2-Z36-Q3-L-114.

In another embodiment, compounds I are of formula I.B2 wherein Z is imidazol-5-yl (Z-36) and Q is Q4, and R^{a} and R⁴ are as per any row of Table L below, which compounds are named I.B2-Z36-Q4-L-1 to I.B2-Z36-Q4-L-114.

In another embodiment, compounds I are of formula I.B1 wherein Z is 1-CH₃-imidazol-5-yl (Z-39) and Q is Q1, and R^{a} and R⁴ are as per any row of Table L below, which compounds are named I.B1-Z39-Q1-L-1 to I.B1-Z39-Q1-L-114.

In another embodiment, compounds I are of formula I.B1 wherein Z is 1-CH₃-imidazol-5-yl (Z-39) and Q is Q2, and R^{a} and R⁴ are as per any row of Table L below, which compounds are named I.B1-Z39-Q2-L-1 to I.B1-Z39-Q2-L-114.

In another embodiment, compounds I are of formula I.B1 wherein Z is 1-CH₃-imidazol-5-yl (Z-39) and Q is Q3, and R^{a} and R⁴ are as per any row of Table L below, which compounds are named I.B1-Z39-Q3-L-1 to I.B1-Z39-Q3-L-114.

In another embodiment, compounds I are of formula I.B1 wherein Z is 1-CH₃-imidazol-5-yl (Z-39) and Q is Q4, and R^{a} and R⁴ are as per any row of Table L below, which compounds are named I.B1-Z39-Q4-L-1 to I.B1-Z39-Q4-L-114.

In another embodiment, compounds I are of formula I.B2 wherein Z is 1-CH₃-imidazol-5-yl (Z-39) and Q is Q1, and R^{a} and R⁴ are as per any row of Table L below, which compounds are named I.B2-Z39-Q1-L-1 to I.B2-Z39-Q1-L-114.

In another embodiment, compounds I are of formula I.B2 wherein Z is 1-CH₃-imidazol-5-yl (Z-39) and Q is Q2, and R^{a} and R⁴ are as per any row of Table L below, which compounds are named I.B2-Z39-Q2-L-1 to I.B2-Z39-Q2-L-114.

In another embodiment, compounds I are of formula I.B2 wherein Z is 1-CH₃-imidazol-5-yl (Z-39) and Q is Q3, and R^{a} and R⁴ are as per any row of Table L below, which compounds are named I.B2-Z39-Q3-L-1 to I.B2-Z39-Q3-L-114.

In another embodiment, compounds I are of formula I.B2 wherein Z is 1-CH₃-imidazol-5-yl (Z-39) and Q is Q4, and R^{a} and R⁴ are as per any row of Table L below, which compounds are named I.B2-Z39-Q4-L-1 to I.B2-Z39-Q4-L-114.

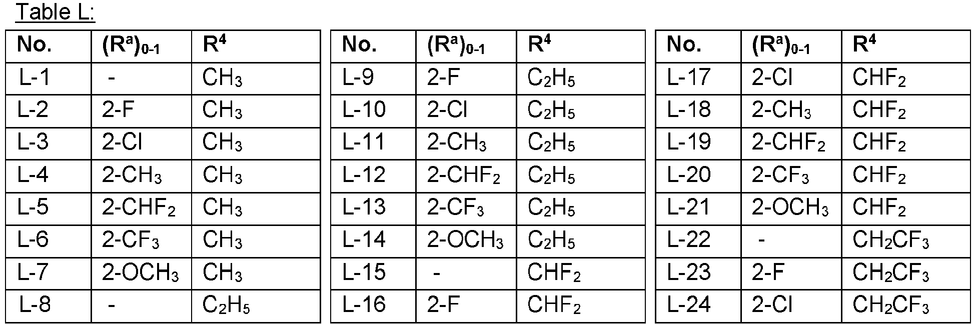

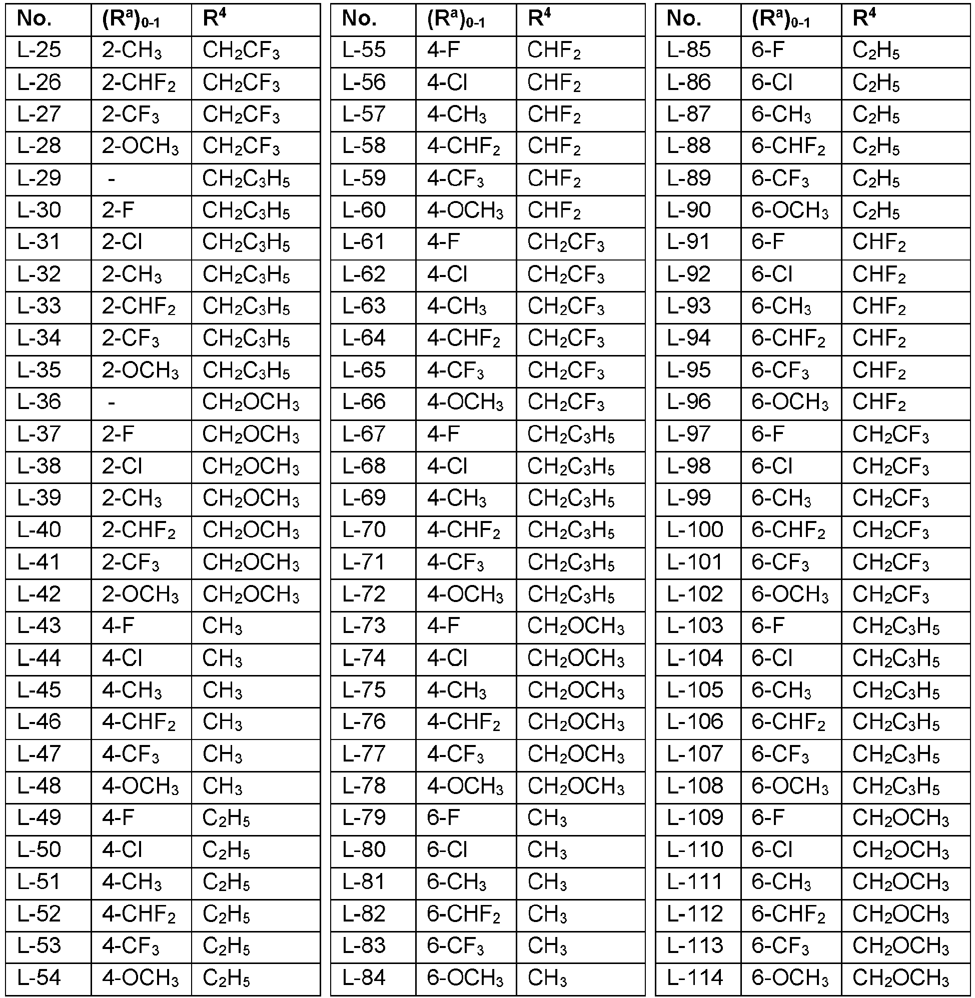

In another embodiment, compounds I are of formula I.A1 wherein Z is pyrimidin-4-yl (Z-20), R³ is CH₃ and Q is Q1, and R^{a} and R⁴ are as per any row of Table M below, which compounds are named I.A1-Z20-Q1-M-1 to I.A1-Z20-Q1-M-114.

In another embodiment, compounds I are of formula I.A1 wherein Z is pyrimidin-4-yl (Z-20), R³ is CH₃ and Q is Q2, and R^{a} and R⁴ are as per any row of Table M below, which compounds are named I.A1-Z20-Q2-M-1 to I.A1-Z20-Q2-M-114.

In another embodiment, compounds I are of formula I.A1 wherein Z is pyrimidin-4-yl (Z-20), R³ is CH₃ and Q is Q3, and R^{a} and R⁴ are as per any row of Table M below, which compounds are named I.A1-Z20-Q3-M-1 to I.A1-Z20-Q3-M-114.

In another embodiment, compounds I are of formula I.A1 wherein Z is pyrimidin-4-yl (Z-20), R³ is CH₃ and Q is Q4, and R^{a} and R⁴ are as per any row of Table M below, which compounds are named I.A1-Z20-Q4-M-1 to I.A1-Z20-Q4-M-114.

In another embodiment, compounds I are of formula I.A2 wherein Z is pyrimidin-4-yl (Z-20), R³ is CH₃ and Q is Q1, and R^{a} and R⁴ are as per any row of Table M below, which compounds are named I.A2-Z20-Q1-M-1 to I.A2-Z20-Q1-M-114.

In another embodiment, compounds I are of formula I.A2 wherein Z is pyrimidin-4-yl (Z-20), R³ is CH₃ and Q is Q2, and R^{a} and R⁴ are as per any row of Table M below, which compounds are named I.A2-Z20-Q2-M-1 to I.A2-Z20-Q2-M-114.

In another embodiment, compounds I are of formula I.A2 wherein Z is pyrimidin-4-yl (Z-20), R³ is CH₃ and Q is Q3, and R^{a} and R⁴ are as per any row of Table M below, which compounds are named I.A2-Z20-Q3-M-1 to I.A2-Z20-Q3-M-114.

In another embodiment, compounds I are of formula I.A2 wherein Z is pyrimidin-4-yl (Z-20), R³ is CH₃ and Q is Q4, and R^{a} and R⁴ are as per any row of Table M below, which compounds are named I.A2-Z20-Q4-M-1 to I.A2-Z20-Q4-M-114.

In another embodiment, compounds I are of formula I.A1 wherein Z is imidazol-4-yl (Z-35), R³ is CH₃ and Q is Q1, and R^{a} and R⁴ are as per any row of Table M below, which compounds are named I.A1-Z35-Q1-M-1 to I.A1-Z35-Q1-M-114.

In another embodiment, compounds I are of formula I.A1 wherein Z is imidazol-4-yl (Z-35), R³ is CH₃ and Q is Q2, and R^{a} and R⁴ are as per any row of Table M below, which compounds are named I.A1-Z35-Q2-M-1 to I.A1-Z35-Q2-M-114.

In another embodiment, compounds I are of formula I.A1 wherein Z is imidazol-4-yl (Z-35), R³ is CH₃ and Q is Q3, and R^{a} and R⁴ are as per any row of Table M below, which compounds are named I.A1-Z35-Q3-M-1 to I.A1-Z35-Q3-M-114.

In another embodiment, compounds I are of formula I.A1 wherein Z is imidazol-4-yl (Z-35), R³ is CH₃ and Q is Q4, and R^{a} and R⁴ are as per any row of Table M below, which compounds are named I.A1-Z35-Q4-M-1 to I.A1-Z35-Q4-M-114.

In another embodiment, compounds I are of formula I.A2 wherein Z is imidazol-4-yl (Z-35), R³ is CH₃ and Q is Q1, and R^{a} and R⁴ are as per any row of Table M below, which compounds are named I.A2-Z35-Q1-M-1 to I.A2-Z35-Q1-M-114.

In another embodiment, compounds I are of formula I.A2 wherein Z is imidazol-4-yl (Z-35), R³ is CH₃ and Q is Q2, and R^{a} and R⁴ are as per any row of Table M below, which compounds are named I.A2-Z35-Q2-M-1 to I.A2-Z35-Q2-M-114.

In another embodiment, compounds I are of formula I.A2 wherein Z is imidazol-4-yl (Z-35), R³ is CH₃ and Q is Q3, and R^{a} and R⁴ are as per any row of Table M below, which compounds are named I.A2-Z35-Q3-M-1 to I.A2-Z35-Q3-M-114.

In another embodiment, compounds I are of formula I.A2 wherein Z is imidazol-4-yl (Z-35), R³ is CH₃ and Q is Q4, and R^{a} and R⁴ are as per any row of Table M below, which compounds are named I.A2-Z35-Q4-M-1 to I.A2-Z35-Q4-M-114.

In another embodiment, compounds I are of formula I.A1 wherein Z is 1-CH₃-imidazol-4-yl (Z-38), R³ is CH₃ and Q is Q1, and R^{a} and R⁴ are as per any row of Table M below, which compounds are named I.A1-Z38-Q1-M-1 to I.A1-Z38-Q1-M-114.

In another embodiment, compounds I are of formula I.A1 wherein Z is 1-CH₃-imidazol-4-yl (Z-38), R³ is CH₃ and Q is Q2, and R^{a} and R⁴ are as per any row of Table M below, which compounds are named I.A1-Z38-Q2-M-1 to I.A1-Z38-Q2-M-114.

In another embodiment, compounds I are of formula I.A1 wherein Z is 1-CH₃-imidazol-4-yl (Z-38), R³ is CH₃ and Q is Q3, and R^{a} and R⁴ are as per any row of Table M below, which compounds are named I.A1-Z38-Q3-M-1 to I.A1-Z38-Q3-M-114.

In another embodiment, compounds I are of formula I.A1 wherein Z is 1-CH₃-imidazol-4-yl (Z-38), R³ is CH₃ and Q is Q4, and R^{a} and R⁴ are as per any row of Table M below, which compounds are named I.A1-Z38-Q4-M-1 to I.A1-Z38-Q4-M-114.

In another embodiment, compounds I are of formula I.A2 wherein Z is 1-CH₃-imidazol-4-yl (Z-38), R³ is CH₃ and Q is Q1, and R^{a} and R⁴ are as per any row of Table M below, which compounds are named I.A2-Z38-Q1-M-1 to I.A2-Z38-Q1-M-114.

In another embodiment, compounds I are of formula I.A2 wherein Z is 1-CH₃-imidazol-4-yl (Z-38), R³ is CH₃ and Q is Q2, and R^{a} and R⁴ are as per any row of Table M below, which compounds are named I.A2-Z38-Q2-M-1 to I.A2-Z38-Q2-M-114.

In another embodiment, compounds I are of formula I.A2 wherein Z is 1-CH₃-imidazol-4-yl (Z-38), R³ is CH₃ and Q is Q3, and R^{a} and R⁴ are as per any row of Table M below, which compounds are named I.A2-Z38-Q3-M-1 to I.A2-Z38-Q3-M-114.

In another embodiment, compounds I are of formula I.A2 wherein Z is 1-CH₃-imidazol-4-yl (Z-38), R³ is CH₃ and Q is Q4, and R^{a} and R⁴ are as per any row of Table M below, which compounds are named I.A2-Z38-Q4-M-1 to I.A2-Z38-Q4-M-114.

In another embodiment, compounds I are of formula I.B1 wherein Z is pyrimidin-4-yl (Z-20) and Q is Q1, and R^{a} and R⁴ are as per any row of Table M below, which compounds are named I.B1-Z20-Q1-M-1 to I.B1-Z20-Q1-M-114.

In another embodiment, compounds I are of formula I.B1 wherein Z is pyrimidin-4-yl (Z-20) and Q is Q2, and R^{a} and R⁴ are as per any row of Table M below, which compounds are named I.B1-Z20-Q2-M-1 to I.B1-Z20-Q2-M-114.

In another embodiment, compounds I are of formula I.B1 wherein Z is pyrimidin-4-yl (Z-20) and Q is Q3, and R^{a} and R⁴ are as per any row of Table M below, which compounds are named I.B1-Z20-Q3-M-1 to I.B1-Z20-Q3-M-114.

In another embodiment, compounds I are of formula I.B1 wherein Z is pyrimidin-4-yl (Z-20) and Q is Q4, and R^{a} and R⁴ are as per any row of Table M below, which compounds are named I.B1-Z20-Q4-M-1 to I.B1-Z20-Q4-M-114.

In another embodiment, compounds I are of formula I.B2 wherein Z is pyrimidin-4-yl (Z-20) and Q is Q1, and R^{a} and R⁴ are as per any row of Table M below, which compounds are named I.B2-Z20-Q1-M-1 to I.B2-Z20-Q1-M-114.

In another embodiment, compounds I are of formula I.B2 wherein Z is pyrimidin-4-yl (Z-20) and Q is Q2, and R^{a} and R⁴ are as per any row of Table M below, which compounds are named I.B2-Z20-Q2-M-1 to I.B2-Z20-Q2-M-114.

In another embodiment, compounds I are of formula I.B2 wherein Z is pyrimidin-4-yl (Z-20) and Q is Q3, and R^{a} and R⁴ are as per any row of Table M below, which compounds are named I.B2-Z20-Q3-M-1 to I.B2-Z20-Q3-M-114.

In another embodiment, compounds I are of formula I.B2 wherein Z is pyrimidin-4-yl (Z-20) and Q is Q4, and R^{a} and R⁴ are as per any row of Table M below, which compounds are named I.B2-Z20-Q4-M-1 to I.B2-Z20-Q4-M-114.

In another embodiment, compounds I are of formula I.B1 wherein Z is imidazol-4-yl (Z-35) and Q is Q1, and R^{a} and R⁴ are as per any row of Table M below, which compounds are named I.B1-Z35-Q1-M-1 to I.B1-Z35-Q1-M-114.

In another embodiment, compounds I are of formula I.B1 wherein Z is imidazol-4-yl (Z-35) and Q is Q2, and R^{a} and R⁴ are as per any row of Table M below, which compounds are named I.B1-Z35-Q2-M-1 to I.B1-Z35-Q2-M-114.

In another embodiment, compounds I are of formula I.B1 wherein Z is imidazol-4-yl (Z-35) and Q is Q3, and R^{a} and R⁴ are as per any row of Table M below, which compounds are named I.B1-Z35-Q3-M-1 to I.B1-Z35-Q3-M-114.

In another embodiment, compounds I are of formula I.B1 wherein Z is imidazol-4-yl (Z-35) and Q is Q4, and R^{a} and R⁴ are as per any row of Table M below, which compounds are named I.B1-Z35-Q4-M-1 to I.B1-Z35-Q4-M-114.

In another embodiment, compounds I are of formula I.B2 wherein Z is imidazol-4-yl (Z-35) and Q is Q1, and R^{a} and R⁴ are as per any row of Table M below, which compounds are named I.B2-Z35-Q1-M-1 to I.B2-Z35-Q1-M-114.

In another embodiment, compounds I are of formula I.B2 wherein Z is imidazol-4-yl (Z-35) and Q is Q2, and R^{a} and R⁴ are as per any row of Table M below, which compounds are named I.B2-Z35-Q2-M-1 to I.B2-Z35-Q2-M-114.

In another embodiment, compounds I are of formula I.B2 wherein Z is imidazol-4-yl (Z-35) and Q is Q3, and R^{a} and R⁴ are as per any row of Table M below, which compounds are named I.B2-Z35-Q3-M-1 to I.B2-Z35-Q3-M-114.

In another embodiment, compounds I are of formula I.B2 wherein Z is imidazol-4-yl (Z-35) and Q is Q4, and R^{a} and R⁴ are as per any row of Table M below, which compounds are named I.B2-Z35-Q4-M-1 to I.B2-Z35-Q4-M-114.

In another embodiment, compounds I are of formula I.B1 wherein Z is 1-CH₃-imidazol-4-yl (Z-38) and Q is Q1, and R^{a} and R⁴ are as per any row of Table M below, which compounds are named I.B1-Z38-Q1-M-1 to I.B1-Z38-Q1-M-114.

In another embodiment, compounds I are of formula I.B1 wherein Z is 1-CH₃-imidazol-4-yl (Z-38) and Q is Q2, and R^{a} and R⁴ are as per any row of Table M below, which compounds are named I.B1-Z38-Q2-M-1 to I.B1-Z38-Q2-M-114.

In another embodiment, compounds I are of formula I.B1 wherein Z is 1-CH₃-imidazol-4-yl (Z-38) and Q is Q3, and R^{a} and R⁴ are as per any row of Table M below, which compounds are named I.B1-Z38-Q3-M-1 to I.B1-Z38-Q3-M-114.

In another embodiment, compounds I are of formula I.B1 wherein Z is 1-CH₃-imidazol-4-yl (Z-38) and Q is Q4, and R^{a} and R⁴ are as per any row of Table M below, which compounds are named I.B1-Z38-Q4-M-1 to I.B1-Z38-Q4-M-114.

In another embodiment, compounds I are of formula I.B2 wherein Z is 1-CH₃-imidazol-4-yl (Z-38) and Q is Q1, and R^{a} and R⁴ are as per any row of Table M below, which compounds are named I.B2-Z38-Q1-M-1 to I.B2-Z38-Q1-M-114.

In another embodiment, compounds I are of formula I.B2 wherein Z is 1-CH₃-imidazol-4-yl (Z-38) and Q is Q2, and R^{a} and R⁴ are as per any row of Table M below, which compounds are named I.B2-Z38-Q2-M-1 to I.B2-Z38-Q2-M-114.

In another embodiment, compounds I are of formula I.B2 wherein Z is 1-CH₃-imidazol-4-yl (Z-38) and Q is Q3, and R^{a} and R⁴ are as per any row of Table M below, which compounds are named I.B2-Z38-Q3-M-1 to I.B2-Z38-Q3-M-114.

In another embodiment, compounds I are of formula I.B2 wherein Z is 1-CH₃-imidazol-4-yl (Z-38) and Q is Q4, and R^{a} and R⁴ are as per any row of Table M below, which compounds are named I.B2-Z38-Q4-M-1 to I.B2-Z38-Q4-M-114.

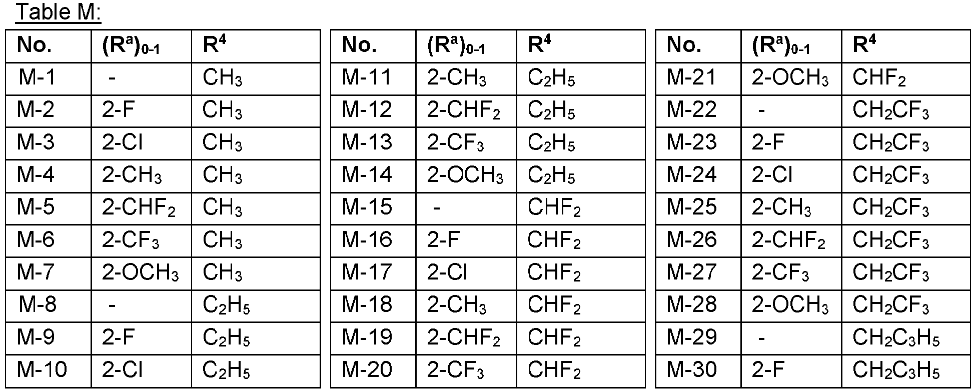

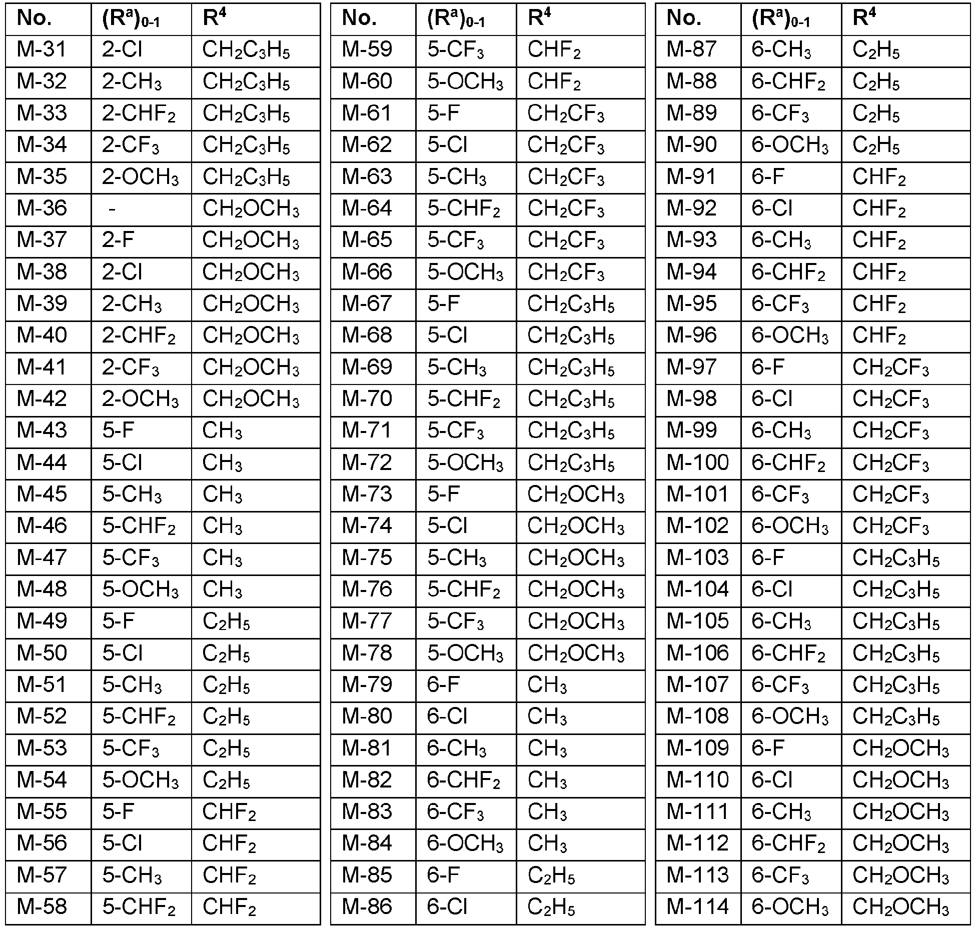

In another embodiment, compounds I are of formula I.A1 wherein Z is azol-2-yl (Z-24), R³ is CH₃ and Q is Q1, and R^{a} and R⁴ are as per any row of Table N below, which compounds are named I.A1-Z24-Q1-N-1 to I.A1-Z24-Q1-N-114.

In another embodiment, compounds I are of formula I.A1 wherein Z is azol-2-yl (Z-24), R³ is CH₃ and Q is Q2, and R^{a} and R⁴ are as per any row of Table N below, which compounds are named I.A1-Z24-Q2-N-1 to I.A1-Z24-Q2-N-114.

In another embodiment, compounds I are of formula I.A1 wherein Z is azol-2-yl (Z-24), R³ is CH₃ and Q is Q3, and R^{a} and R⁴ are as per any row of Table N below, which compounds are named I.A1-Z24-Q3-N-1 to I.A1-Z24-Q3-N-114.

In another embodiment, compounds I are of formula I.A1 wherein Z is azol-2-yl (Z-24), R³ is CH₃ and Q is Q4, and R^{a} and R⁴ are as per any row of Table N below, which compounds are named I.A1-Z24-Q4-N-1 to I.A1-Z24-Q4-N-114.

In another embodiment, compounds I are of formula I.A2 wherein Z is azol-2-yl (Z-24), R³ is CH₃ and Q is Q1, and R^{a} and R⁴ are as per any row of Table N below, which compounds are named I.A2-Z24-Q1-N-1 to I.A2-Z24-Q1-N-114.

In another embodiment, compounds I are of formula I.A2 wherein Z is azol-2-yl (Z-24), R³ is CH₃ and Q is Q2, and R^{a} and R⁴ are as per any row of Table N below, which compounds are named I.A2-Z24-Q2-N-1 to I.A2-Z24-Q2-N-114.

In another embodiment, compounds I are of formula I.A2 wherein Z is azol-2-yl (Z-24), R³ is CH₃ and Q is Q3, and R^{a} and R⁴ are as per any row of Table N below, which compounds are named I.A2-Z24-Q3-N-1 to I.A2-Z24-Q3-N-114.

In another embodiment, compounds I are of formula I.A2 wherein Z is azol-2-yl (Z-24), R³ is CH₃ and Q is Q4, and R^{a} and R⁴ are as per any row of Table N below, which compounds are named I.A2-Z24-Q4-N-1 to I.A2-Z24-Q4-N-114.

In another embodiment, compounds I are of formula I.A1 wherein Z is pyrazol-1-yl (Z-26), R³ is CH₃ and Q is Q1, and R^{a} and R⁴ are as per any row of Table N below, which compounds are named I.A1-Z26-Q1-N-1 to I.A1-Z26-Q1-N-114.

In another embodiment, compounds I are of formula I.A1 wherein Z is pyrazol-1-yl (Z-26), R³ is CH₃ and Q is Q2, and R^{a} and R⁴ are as per any row of Table N below, which compounds are named I.A1-Z26-Q2-N-1 to I.A1-Z26-Q2-N-114.

In another embodiment, compounds I are of formula I.A1 wherein Z is pyrazol-1-yl (Z-26), R³ is CH₃ and Q is Q3, and R^{a} and R⁴ are as per any row of Table N below, which compounds are named I.A1-Z26-Q3-N-1 to I.A1-Z26-Q3-N-114.

In another embodiment, compounds I are of formula I.A1 wherein Z is pyrazol-1-yl (Z-26), R³ is CH₃ and Q is Q4, and R^{a} and R⁴ are as per any row of Table N below, which compounds are named I.A1-Z26-Q4-N-1 to I.A1-Z26-Q4-N-114.

In another embodiment, compounds I are of formula I.A2 wherein Z is pyrazol-1-yl (Z-26), R³ is CH₃ and Q is Q1, and R^{a} and R⁴ are as per any row of Table N below, which compounds are named I.A2-Z26-Q1-N-1 to I.A2-Z26-Q1-N-114.

In another embodiment, compounds I are of formula I.A2 wherein Z is pyrazol-1-yl (Z-26), R³ is CH₃ and Q is Q2, and R^{a} and R⁴ are as per any row of Table N below, which compounds are named I.A2-Z26-Q2-N-1 to I.A2-Z26-Q2-N-114.

In another embodiment, compounds I are of formula I.A2 wherein Z is pyrazol-1-yl (Z-26), R³ is CH₃ and Q is Q3, and R^{a} and R⁴ are as per any row of Table N below, which compounds are named I.A2-Z26-Q3-N-1 to I.A2-Z26-Q3-N-114.

In another embodiment, compounds I are of formula I.A2 wherein Z is pyrazol-1-yl (Z-26), R³ is CH₃ and Q is Q4, and R^{a} and R⁴ are as per any row of Table N below, which compounds are named I.A2-Z26-Q4-N-1 to I.A2-Z26-Q4-N-114.

In another embodiment, compounds I are of formula I.A1 wherein Z is furan-2-yl (Z-53), R³ is CH₃ and Q is Q1, and R^{a} and R⁴ are as per any row of Table N below, which compounds are named I.A1-Z53-Q1-N-1 to I.A1-Z53-Q1-N-114.

In another embodiment, compounds I are of formula I.A1 wherein Z is furan-2-yl (Z-53), R³ is CH₃ and Q is Q2, and R^{a} and R⁴ are as per any row of Table N below, which compounds are named I.A1-Z53-Q2-N-1 to I.A1-Z53-Q2-N-114.

In another embodiment, compounds I are of formula I.A1 wherein Z is furan-2-yl (Z-53), R³ is CH₃ and Q is Q3, and R^{a} and R⁴ are as per any row of Table N below, which compounds are named I.A1-Z53-Q3-N-1 to I.A1-Z53-Q3-N-114.

In another embodiment, compounds I are of formula I.A1 wherein Z is furan-2-yl (Z-53), R³ is CH₃ and Q is Q4, and R^{a} and R⁴ are as per any row of Table N below, which compounds are named I.A1-Z53-Q4-N-1 to I.A1-Z53-Q4-N-114.

In another embodiment, compounds I are of formula I.A2 wherein Z is furan-2-yl (Z-53), R³ is CH₃ and Q is Q1, and R^{a} and R⁴ are as per any row of Table N below, which compounds are named I.A2-Z53-Q1-N-1 to I.A2-Z53-Q1-N-114.

In another embodiment, compounds I are of formula I.A2 wherein Z is furan-2-yl (Z-53), R³ is CH₃ and Q is Q2, and R^{a} and R⁴ are as per any row of Table N below, which compounds are named I.A2-Z53-Q2-N-1 to I.A2-Z53-Q2-N-114.

In another embodiment, compounds I are of formula I.A2 wherein Z is furan-2-yl (Z-53), R³ is CH₃ and Q is Q3, and R^{a} and R⁴ are as per any row of Table N below, which compounds are named I.A2-Z53-Q3-N-1 to I.A2-Z53-Q3-N-114.

In another embodiment, compounds I are of formula I.A2 wherein Z is furan-2-yl (Z-53), R³ is CH₃ and Q is Q4, and R^{a} and R⁴ are as per any row of Table N below, which compounds are named I.A2-Z53-Q4-N-1 to I.A2-Z53-Q4-N-114.

In another embodiment, compounds I are of formula I.A1 wherein Z is thien-2-yl (Z-57), R³ is CH₃ and Q is Q1, and R^{a} and R⁴ are as per any row of Table N below, which compounds are named I.A1-Z57-Q1-N-1 to I.A1-Z57-Q1-N-114.

In another embodiment, compounds I are of formula I.A1 wherein Z is thien-2-yl (Z-57), R³ is CH₃ and Q is Q2, and R^{a} and R⁴ are as per any row of Table N below, which compounds are named I.A1-Z57-Q2-N-1 to I.A1-Z57-Q2-N-114.

In another embodiment, compounds I are of formula I.A1 wherein Z is thien-2-yl (Z-57), R³ is CH₃ and Q is Q3, and R^{a} and R⁴ are as per any row of Table N below, which compounds are named I.A1-Z57-Q3-N-1 to I.A1-Z57-Q3-N-114.

In another embodiment, compounds I are of formula I.A1 wherein Z is thien-2-yl (Z-57), R³ is CH₃ and Q is Q4, and R^{a} and R⁴ are as per any row of Table N below, which compounds are named I.A1-Z57-Q4-N-1 to I.A1-Z57-Q4-N-114.

In another embodiment, compounds I are of formula I.A2 wherein Z is thien-2-yl (Z-57), R³ is CH₃ and Q is Q1, and R^{a} and R⁴ are as per any row of Table N below, which compounds are named I.A2-Z57-Q1-N-1 to I.A2-Z57-Q1-N-114.

In another embodiment, compounds I are of formula I.A2 wherein Z is thien-2-yl (Z-57), R³ is CH₃ and Q is Q2, and R^{a} and R⁴ are as per any row of Table N below, which compounds are named I.A2-Z57-Q2-N-1 to I.A2-Z57-Q2-N-114.

In another embodiment, compounds I are of formula I.A2 wherein Z is thien-2-yl (Z-57), R³ is CH₃ and Q is Q3, and R^{a} and R⁴ are as per any row of Table N below, which compounds are named I.A2-Z57-Q3-N-1 to I.A2-Z57-Q3-N-114.

In another embodiment, compounds I are of formula I.A2 wherein Z is thien-2-yl (Z-57), R³ is CH₃ and Q is Q4, and R^{a} and R⁴ are as per any row of Table N below, which compounds are named I.A2-Z57-Q4-N-1 to I.A2-Z57-Q4-N-114.

In another embodiment, compounds I are of formula I.B1 wherein Z is azol-2-yl (Z-24) and Q is Q1, and R^{a} and R⁴ are as per any row of Table N below, which compounds are named I.B1-Z24-Q1-N-1 to I.B1-Z24-Q1-N-114.

In another embodiment, compounds I are of formula I.B1 wherein Z is azol-2-yl (Z-24) and Q is Q2, and R^{a} and R⁴ are as per any row of Table N below, which compounds are named I.B1-Z24-Q2-N-1 to I.B1-Z24-Q2-N-114.

In another embodiment, compounds I are of formula I.B1 wherein Z is azol-2-yl (Z-24) and Q is Q3, and R^{a} and R⁴ are as per any row of Table N below, which compounds are named I.B1-Z24-Q3-N-1 to I.B1-Z24-Q3-N-114.

In another embodiment, compounds I are of formula I.B1 wherein Z is azol-2-yl (Z-24) and Q is Q4, and R^{a} and R⁴ are as per any row of Table N below, which compounds are named I.B1-Z24-Q4-N-1 to I.B1-Z24-Q4-N-114.

In another embodiment, compounds I are of formula I.B2 wherein Z is azol-2-yl (Z-24) and Q is Q1, and R^{a} and R⁴ are as per any row of Table N below, which compounds are named I.B2-Z24-Q1-N-1 to I.B2-Z24-Q1-N-114.

In another embodiment, compounds I are of formula I.B2 wherein Z is azol-2-yl (Z-24) and Q is Q2, and R^{a} and R⁴ are as per any row of Table N below, which compounds are named I.B2-Z24-Q2-N-1 to I.B2-Z24-Q2-N-114.

In another embodiment, compounds I are of formula I.B2 wherein Z is azol-2-yl (Z-24) and Q is Q3, and R^{a} and R⁴ are as per any row of Table N below, which compounds are named I.B2-Z24-Q3-N-1 to I.B2-Z24-Q3-N-114.

In another embodiment, compounds I are of formula I.B2 wherein Z is azol-2-yl (Z-24) and Q is Q4, and R^{a} and R⁴ are as per any row of Table N below, which compounds are named I.B2-Z24-Q4-N-1 to I.B2-Z24-Q4-N-114.

In another embodiment, compounds I are of formula I.B1 wherein Z is pyrazol-1-yl (Z-26) and Q is Q1, and R^{a} and R⁴ are as per any row of Table N below, which compounds are named I.B1-Z26-Q1-N-1 to I.B1-Z26-Q1-N-114.

In another embodiment, compounds I are of formula I.B1 wherein Z is pyrazol-1-yl (Z-26) and Q is Q2, and R^{a} and R⁴ are as per any row of Table N below, which compounds are named I.B1-Z26-Q2-N-1 to I.B1-Z26-Q2-N-114.

In another embodiment, compounds I are of formula I.B1 wherein Z is pyrazol-1-yl (Z-26) and Q is Q3, and R^{a} and R⁴ are as per any row of Table N below, which compounds are named I.B1-Z26-Q3-N-1 to I.B1-Z26-Q3-N-114.

In another embodiment, compounds I are of formula I.B1 wherein Z is pyrazol-1-yl (Z-26) and Q is Q4, and R^{a} and R⁴ are as per any row of Table N below, which compounds are named I.B1-Z26-Q4-N-1 to I.B1-Z26-Q4-N-114.

In another embodiment, compounds I are of formula I.B2 wherein Z is pyrazol-1-yl (Z-26) and Q is Q1, and R^{a} and R⁴ are as per any row of Table N below, which compounds are named I.B2-Z26-Q1-N-1 to I.B2-Z26-Q1-N-114.

In another embodiment, compounds I are of formula I.B2 wherein Z is pyrazol-1-yl (Z-26) and Q is Q2, and R^{a} and R⁴ are as per any row of Table N below, which compounds are named I.B2-Z26-Q2-N-1 to I.B2-Z26-Q2-N-114.

In another embodiment, compounds I are of formula I.B2 wherein Z is pyrazol-1-yl (Z-26) and Q is Q3, and R^{a} and R⁴ are as per any row of Table N below, which compounds are named I.B2-Z26-Q3-N-1 to I.B2-Z26-Q3-N-114.

In another embodiment, compounds I are of formula I.B2 wherein Z is pyrazol-1-yl (Z-26) and Q is Q4, and R^{a} and R⁴ are as per any row of Table N below, which compounds are named I.B2-Z26-Q4-N-1 to I.B2-Z26-Q4-N-114.

In another embodiment, compounds I are of formula I.B1 wherein Z is furan-2-yl (Z-53) and Q is Q1, and R^{a} and R⁴ are as per any row of Table N below, which compounds are named I.B1-Z53-Q1-N-1 to I.B1-Z53-Q1-N-114.

In another embodiment, compounds I are of formula I.B1 wherein Z is furan-2-yl (Z-53) and Q is Q2, and R^{a} and R⁴ are as per any row of Table N below, which compounds are named I.B1-Z53-Q2-N-1 to I.B1-Z53-Q2-N-114.

In another embodiment, compounds I are of formula I.B1 wherein Z is furan-2-yl (Z-53) and Q is Q3, and R^{a} and R⁴ are as per any row of Table N below, which compounds are named I.B1-Z53-Q3-N-1 to I.B1-Z53-Q3-N-114.

In another embodiment, compounds I are of formula I.B1 wherein Z is furan-2-yl (Z-53) and Q is Q4, and R^{a} and R⁴ are as per any row of Table N below, which compounds are named I.B1-Z53-Q4-N-1 to I.B1-Z53-Q4-N-114.

In another embodiment, compounds I are of formula I.B2 wherein Z is furan-2-yl (Z-53) and Q is Q1, and R^{a} and R⁴ are as per any row of Table N below, which compounds are named I.B2-Z53-Q1-N-1 to I.B2-Z53-Q1-N-114.

In another embodiment, compounds I are of formula I.B2 wherein Z is furan-2-yl (Z-53) and Q is Q2, and R^{a} and R⁴ are as per any row of Table N below, which compounds are named I.B2-Z53-Q2-N-1 to I.B2-Z53-Q2-N-114.

In another embodiment, compounds I are of formula I.B2 wherein Z is furan-2-yl (Z-53) and Q is Q3, and R^{a} and R⁴ are as per any row of Table N below, which compounds are named I.B2-Z53-Q3-N-1 to I.B2-Z53-Q3-N-114.

In another embodiment, compounds I are of formula I.B2 wherein Z is furan-2-yl (Z-53) and Q is Q4, and R^{a} and R⁴ are as per any row of Table N below, which compounds are named I.B2-Z53-Q4-N-1 to I.B2-Z53-Q4-N-114.

In another embodiment, compounds I are of formula I.B1 wherein Z is thien-2-yl (Z-57) and Q is Q1, and R^{a} and R⁴ are as per any row of Table N below, which compounds are named I.B1-Z57-Q1-N-1 to I.B1-Z57-Q1-N-114.

In another embodiment, compounds I are of formula I.B1 wherein Z is thien-2-yl (Z-57) and Q is Q2, and R^{a} and R⁴ are as per any row of Table N below, which compounds are named I.B1-Z57-Q2-N-1 to I.B1-Z57-Q2-N-114.

In another embodiment, compounds I are of formula I.B1 wherein Z is thien-2-yl (Z-57) and Q is Q3, and R^{a} and R⁴ are as per any row of Table N below, which compounds are named I.B1-Z57-Q3-N-1 to I.B1-Z57-Q3-N-114.

In another embodiment, compounds I are of formula I.B1 wherein Z is thien-2-yl (Z-57) and Q is Q4, and R^{a} and R⁴ are as per any row of Table N below, which compounds are named I.B1-Z57-Q4-N-1 to I.B1-Z57-Q4-N-114.

In another embodiment, compounds I are of formula I.B2 wherein Z is thien-2-yl (Z-57) and Q is Q1, and R^{a} and R⁴ are as per any row of Table N below, which compounds are named I.B2-Z57-Q1-N-1 to I.B2-Z57-Q1-N-114.

In another embodiment, compounds I are of formula I.B2 wherein Z is thien-2-yl (Z-57) and Q is Q2, and R^{a} and R⁴ are as per any row of Table N below, which compounds are named I.B2-Z57-Q2-N-1 to I.B2-Z57-Q2-N-114.

In another embodiment, compounds I are of formula I.B2 wherein Z is thien-2-yl (Z-57) and Q is Q3, and R^{a} and R⁴ are as per any row of Table N below, which compounds are named I.B2-Z57-Q3-N-1 to I.B2-Z57-Q3-N-114.

In another embodiment, compounds I are of formula I.B2 wherein Z is thien-2-yl (Z-57) and Q is Q4, and R^{a} and R⁴ are as per any row of Table N below, which compounds are named I.B2-Z57-Q4-N-1 to I.B2-Z57-Q4-N-114.

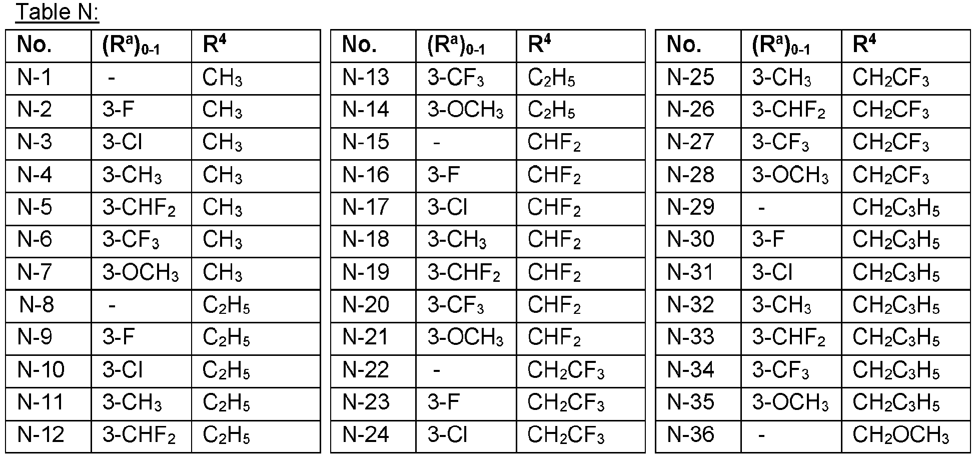

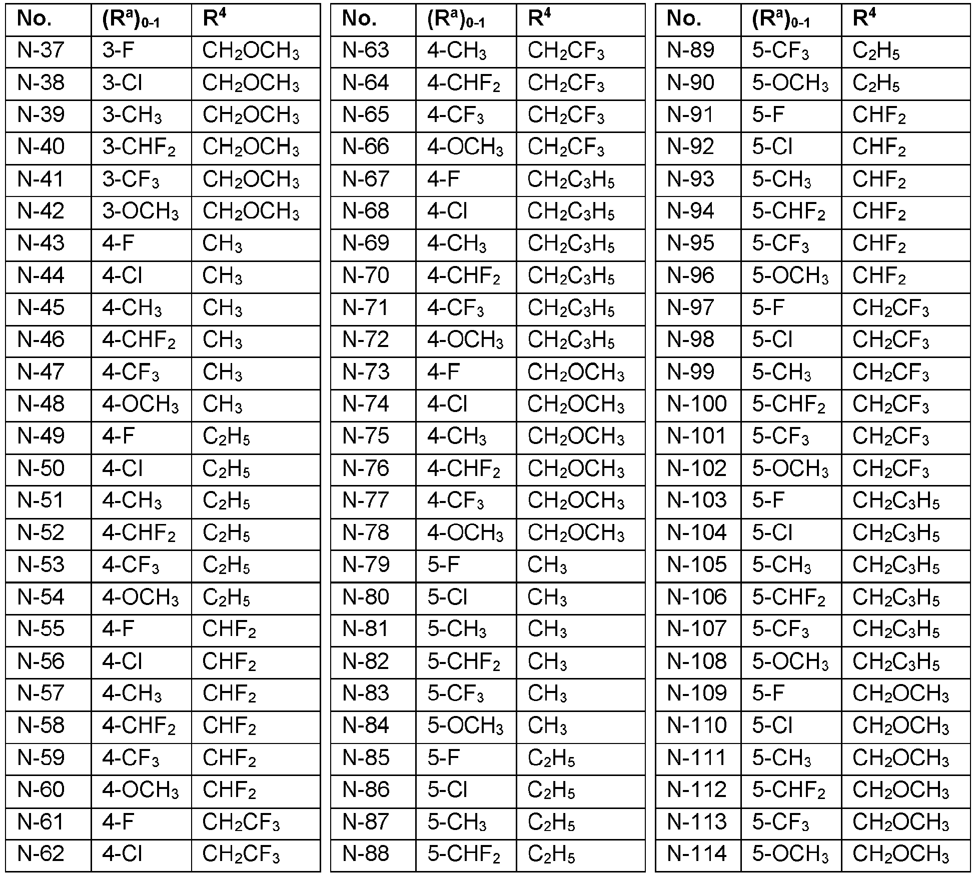

In another embodiment, compounds I are of formula I.A1 wherein Z is pyrimidin-2-yl (Z-19), R³ is CH₃ and Q is Q1, and R^{a} and R⁴ are as per any row of Table O below, which compounds are named I.A1-Z19-Q1-O-1 to I.A1-Z19-Q1-O-114.

In another embodiment, compounds I are of formula I.A1 wherein Z is pyrimidin-2-yl (Z-19), R³ is CH₃ and Q is Q2, and R^{a} and R⁴ are as per any row of Table O below, which compounds are named I.A1-Z19-Q2-O-1 to I.A1-Z19-Q2-O-114.

In another embodiment, compounds I are of formula I.A1 wherein Z is pyrimidin-2-yl (Z-19), R³ is CH₃ and Q is Q3, and R^{a} and R⁴ are as per any row of Table O below, which compounds are named I.A1-Z19-Q3-O-1 to I.A1-Z19-Q3-O-114.

In another embodiment, compounds I are of formula I.A1 wherein Z is pyrimidin-2-yl (Z-19), R³ is CH₃ and Q is Q4, and R^{a} and R⁴ are as per any row of Table O below, which compounds are named I.A1-Z19-Q4-O-1 to I.A1-Z19-Q4-O-114.

In another embodiment, compounds I are of formula I.A2 wherein Z is pyrimidin-2-yl (Z-19), R³ is CH₃ and Q is Q1, and R^{a} and R⁴ are as per any row of Table O below, which compounds are named I.A2-Z19-Q1-O-1 to I.A2-Z19-Q1-O-114.

In another embodiment, compounds I are of formula I.A2 wherein Z is pyrimidin-2-yl (Z-19), R³ is CH₃ and Q is Q2, and R^{a} and R⁴ are as per any row of Table O below, which compounds are named I.A2-Z19-Q2-O-1 to I.A2-Z19-Q2-O-114.

In another embodiment, compounds I are of formula I.A2 wherein Z is pyrimidin-2-yl (Z-19), R³ is CH₃ and Q is Q3, and R^{a} and R⁴ are as per any row of Table O below, which compounds are named I.A2-Z19-Q3-O-1 to I.A2-Z19-Q3-O-114.

In another embodiment, compounds I are of formula I.A2 wherein Z is pyrimidin-2-yl (Z-19), R³ is CH₃ and Q is Q4, and R^{a} and R⁴ are as per any row of Table O below, which compounds are named I.A2-Z19-Q4-O-1 to I.A2-Z19-Q4-O-114.

In another embodiment, compounds I are of formula I.A1 wherein Z is imidazol-2-yl (Z-34), R³ is CH₃ and Q is Q1, and R^{a} and R⁴ are as per any row of Table O below, which compounds are named I.A1-Z34-Q1-O-1 to I.A1-Z34-Q1-O-114.

In another embodiment, compounds I are of formula I.A1 wherein Z is imidazol-2-yl (Z-34), R³ is CH₃ and Q is Q2, and R^{a} and R⁴ are as per any row of Table O below, which compounds are named I.A1-Z34-Q2-O-1 to I.A1-Z34-Q2-O-114.

In another embodiment, compounds I are of formula I.A1 wherein Z is imidazol-2-yl (Z-34), R³ is CH₃ and Q is Q3, and R^{a} and R⁴ are as per any row of Table O below, which compounds are named I.A1-Z34-Q3-O-1 to I.A1-Z34-Q3-O-114.

In another embodiment, compounds I are of formula I.A1 wherein Z is imidazol-2-yl (Z-34), R³ is CH₃ and Q is Q4, and R^{a} and R⁴ are as per any row of Table O below, which compounds are named I.A1-Z34-Q4-O-1 to I.A1-Z34-Q4-O-114.

In another embodiment, compounds I are of formula I.A2 wherein Z is imidazol-2-yl (Z-34), R³ is CH₃ and Q is Q1, and R^{a} and R⁴ are as per any row of Table O below, which compounds are named I.A2-Z34-Q1-O-1 to I.A2-Z34-Q1-O-114.

In another embodiment, compounds I are of formula I.A2 wherein Z is imidazol-2-yl (Z-34), R³ is CH₃ and Q is Q2, and R^{a} and R⁴ are as per any row of Table O below, which compounds are named I.A2-Z34-Q2-O-1 to I.A2-Z34-Q2-O-114.

In another embodiment, compounds I are of formula I.A2 wherein Z is imidazol-2-yl (Z-34), R³ is CH₃ and Q is Q3, and R^{a} and R⁴ are as per any row of Table O below, which compounds are named I.A2-Z34-Q3-O-1 to I.A2-Z34-Q3-O-114.

In another embodiment, compounds I are of formula I.A2 wherein Z is imidazol-2-yl (Z-34), R³ is CH₃ and Q is Q4, and R^{a} and R⁴ are as per any row of Table O below, which compounds are named I.A2-Z34-Q4-O-1 to I.A2-Z34-Q4-O-114.

In another embodiment, compounds I are of formula I.A1 wherein Z is 1-CH₃-imidazol-2-yl (Z-37), R³ is CH₃ and Q is Q1, and R^{a} and R⁴ are as per any row of Table O below, which compounds are named I.A1-Z37-Q1-O-1 to I.A1-Z37-Q1-O-114.

In another embodiment, compounds I are of formula I.A1 wherein Z is 1-CH₃-imidazol-2-yl (Z-37), R³ is CH₃ and Q is Q2, and R^{a} and R⁴ are as per any row of Table O below, which compounds are named I.A1-Z37-Q2-O-1 to I.A1-Z37-Q2-O-114.

In another embodiment, compounds I are of formula I.A1 wherein Z is 1-CH₃-imidazol-2-yl (Z-37), R³ is CH₃ and Q is Q3, and R^{a} and R⁴ are as per any row of Table O below, which compounds are named I.A1-Z37-Q3-O-1 to I.A1-Z37-Q3-O-114.

In another embodiment, compounds I are of formula I.A1 wherein Z is 1-CH₃-imidazol-2-yl (Z-37), R³ is CH₃ and Q is Q4, and R^{a} and R⁴ are as per any row of Table O below, which compounds are named I.A1-Z37-Q4-O-1 to I.A1-Z37-Q4-O-114.

In another embodiment, compounds I are of formula I.A2 wherein Z is 1-CH₃-imidazol-2-yl (Z-37), R³ is CH₃ and Q is Q1, and R^{a} and R⁴ are as per any row of Table O below, which compounds are named I.A2-Z37-Q1-O-1 to I.A2-Z37-Q1-O-114.

In another embodiment, compounds I are of formula I.A2 wherein Z is 1-CH₃-imidazol-2-yl (Z-37), R³ is CH₃ and Q is Q2, and R^{a} and R⁴ are as per any row of Table O below, which compounds are named I.A2-Z37-Q2-O-1 to I.A2-Z37-Q2-O-114.

In another embodiment, compounds I are of formula I.A2 wherein Z is 1-CH₃-imidazol-2-yl (Z-37), R³ is CH₃ and Q is Q3, and R^{a} and R⁴ are as per any row of Table O below, which compounds are named I.A2-Z37-Q3-O-1 to I.A2-Z37-Q3-O-114.

In another embodiment, compounds I are of formula I.A2 wherein Z is 1-CH₃-imidazol-2-yl (Z-37), R³ is CH₃ and Q is Q4, and R^{a} and R⁴ are as per any row of Table O below, which compounds are named I.A2-Z37-Q4-O-1 to I.A2-Z37-Q4-O-114.

In another embodiment, compounds I are of formula I.B1 wherein Z is pyrimidin-2-yl (Z-19) and Q is Q1, and R^{a} and R⁴ are as per any row of Table O below, which compounds are named I.B1-Z19-Q1-O-1 to I.B1-Z19-Q1-O-114.

In another embodiment, compounds I are of formula I.B1 wherein Z is pyrimidin-2-yl (Z-19) and Q is Q2, and R^{a} and R⁴ are as per any row of Table O below, which compounds are named I.B1-Z19-Q2-O-1 to I.B1-Z19-Q2-O-114.

In another embodiment, compounds I are of formula I.B1 wherein Z is pyrimidin-2-yl (Z-19) and Q is Q3, and R^{a} and R⁴ are as per any row of Table O below, which compounds are named I.B1-Z19-Q3-O-1 to I.B1-Z19-Q3-O-114.

In another embodiment, compounds I are of formula I.B1 wherein Z is pyrimidin-2-yl (Z-19) and Q is Q4, and R^{a} and R⁴ are as per any row of Table O below, which compounds are named I.B1-Z19-Q4-O-1 to I.B1-Z19-Q4-O-114.

In another embodiment, compounds I are of formula I.B2 wherein Z is pyrimidin-2-yl (Z-19) and Q is Q1, and R^{a} and R⁴ are as per any row of Table O below, which compounds are named I.B2-Z19-Q1-O-1 to I.B2-Z19-Q1-O-114.

In another embodiment, compounds I are of formula I.B2 wherein Z is pyrimidin-2-yl (Z-19) and Q is Q2, and R^{a} and R⁴ are as per any row of Table O below, which compounds are named I.B2-Z19-Q2-O-1 to I.B2-Z19-Q2-O-114.

In another embodiment, compounds I are of formula I.B2 wherein Z is pyrimidin-2-yl (Z-19) and Q is Q3, and R^{a} and R⁴ are as per any row of Table O below, which compounds are named I.B2-Z19-Q3-O-1 to I.B2-Z19-Q3-O-114.

In another embodiment, compounds I are of formula I.B2 wherein Z is pyrimidin-2-yl (Z-19) and Q is Q4, and R^{a} and R⁴ are as per any row of Table O below, which compounds are named I.B2-Z19-Q4-O-1 to I.B2-Z19-Q4-O-114.

In another embodiment, compounds I are of formula I.B1 wherein Z is imidazol-2-yl (Z-34) and Q is Q1, and R^{a} and R⁴ are as per any row of Table O below, which compounds are named I.B1-Z34-Q1-O-1 to I.B1-Z34-Q1-O-114.

In another embodiment, compounds I are of formula I.B1 wherein Z is imidazol-2-yl (Z-34) and Q is Q2, and R^{a} and R⁴ are as per any row of Table O below, which compounds are named I.B1-Z34-Q2-O-1 to I.B1-Z34-Q2-O-114.

In another embodiment, compounds I are of formula I.B1 wherein Z is imidazol-2-yl (Z-34) and Q is Q3, and R^{a} and R⁴ are as per any row of Table O below, which compounds are named I.B1-Z34-Q3-O-1 to I.B1-Z34-Q3-O-114.

In another embodiment, compounds I are of formula I.B1 wherein Z is imidazol-2-yl (Z-34) and Q is Q4, and R^{a} and R⁴ are as per any row of Table O below, which compounds are named I.B1-Z34-Q4-O-1 to I.B1-Z34-Q4-O-114.

In another embodiment, compounds I are of formula I.B2 wherein Z is imidazol-2-yl (Z-34) and Q is Q1, and R^{a} and R⁴ are as per any row of Table O below, which compounds are named I.B2-Z34-Q1-O-1 to I.B2-Z34-Q1-O-114.

In another embodiment, compounds I are of formula I.B2 wherein Z is imidazol-2-yl (Z-34) and Q is Q2, and R^{a} and R⁴ are as per any row of Table O below, which compounds are named I.B2-Z34-Q2-O-1 to I.B2-Z34-Q2-O-114.

In another embodiment, compounds I are of formula I.B2 wherein Z is imidazol-2-yl (Z-34) and Q is Q3, and R^{a} and R⁴ are as per any row of Table O below, which compounds are named I.B2-Z34-Q3-O-1 to I.B2-Z34-Q3-O-114.

In another embodiment, compounds I are of formula I.B2 wherein Z is imidazol-2-yl (Z-34) and Q is Q4, and R^{a} and R⁴ are as per any row of Table O below, which compounds are named I.B2-Z34-Q4-O-1 to I.B2-Z34-Q4-O-114.

In another embodiment, compounds I are of formula I.B1 wherein Z is 1-CH₃-imidazol-2-yl (Z-37) and Q is Q1, and R^{a} and R⁴ are as per any row of Table O below, which compounds are named I.B1-Z37-Q1-O-1 to I.B1-Z37-Q1-O-114.

In another embodiment, compounds I are of formula I.B1 wherein Z is 1-CH₃-imidazol-2-yl (Z-37) and Q is Q2, and R^{a} and R⁴ are as per any row of Table O below, which compounds are named I.B1-Z37-Q2-O-1 to I.B1-Z37-Q2-O-114.

In another embodiment, compounds I are of formula I.B1 wherein Z is 1-CH₃-imidazol-2-yl (Z-37) and Q is Q3, and R^{a} and R⁴ are as per any row of Table O below, which compounds are named I.B1-Z37-Q3-O-1 to I.B1-Z37-Q3-O-114.

In another embodiment, compounds I are of formula I.B1 wherein Z is 1-CH₃-imidazol-2-yl (Z-37) and Q is Q4, and R^{a} and R⁴ are as per any row of Table O below, which compounds are named I.B1-Z37-Q4-O-1 to I.B1-Z37-Q4-O-114.

In another embodiment, compounds I are of formula I.B2 wherein Z is 1-CH₃-imidazol-2-yl (Z-37) and Q is Q1, and R^{a} and R⁴ are as per any row of Table O below, which compounds are named I.B2-Z37-Q1-O-1 to I.B2-Z37-Q1-O-114.

In another embodiment, compounds I are of formula I.B2 wherein Z is 1-CH₃-imidazol-2-yl (Z-37) and Q is Q2, and R^{a} and R⁴ are as per any row of Table O below, which compounds are named I.B2-Z37-Q2-O-1 to I.B2-Z37-Q2-O-114.

In another embodiment, compounds I are of formula I.B2 wherein Z is 1-CH₃-imidazol-2-yl (Z-37) and Q is Q3, and R^{a} and R⁴ are as per any row of Table O below, which compounds are named I.B2-Z37-Q3-O-1 to I.B2-Z37-Q3-O-114.

In another embodiment, compounds I are of formula I.B2 wherein Z is 1-CH₃-imidazol-2-yl (Z-37) and Q is Q4, and R^{a} and R⁴ are as per any row of Table O below, which compounds are named I.B2-Z37-Q4-O-1 to I.B2-Z37-Q4-O-114.

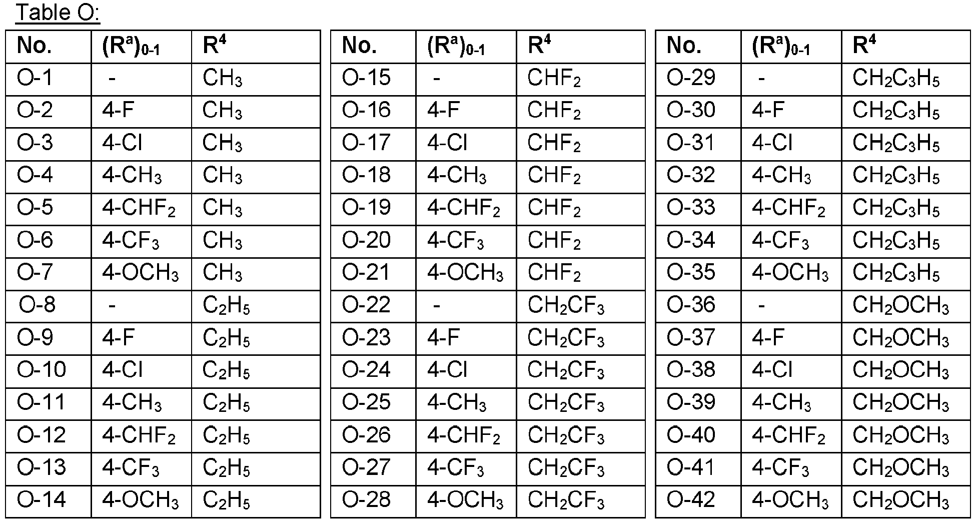

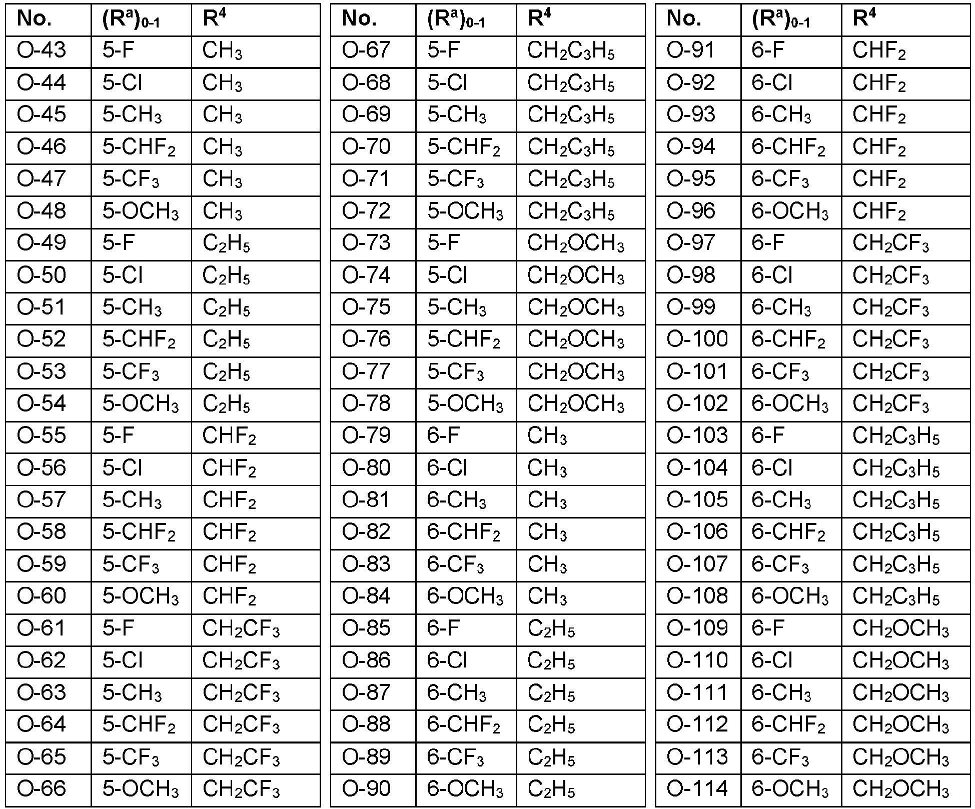

In another embodiment, compounds I are of formula I.A1 wherein Z is azol-1-yl (Z-23), R³ is CH₃ and Q is Q1, and R^{a} and R⁴ are as per any row of Table P below, which compounds are named I.A1-Z23-Q1-P-1 to I.A1-Z23-Q1-P-150.

In another embodiment, compounds I are of formula I.A1 wherein Z is azol-1-yl (Z-23), R³ is CH₃ and Q is Q2, and R^{a} and R⁴ are as per any row of Table P below, which compounds are named I.A1-Z23-Q2-P-1 to I.A1-Z23-Q2-P-150.

In another embodiment, compounds I are of formula I.A1 wherein Z is azol-1-yl (Z-23), R³ is CH₃ and Q is Q3, and R^{a} and R⁴ are as per any row of Table P below, which compounds are named I.A1-Z23-Q3-P-1 to I.A1-Z23-Q3-P-150.

In another embodiment, compounds I are of formula I.A1 wherein Z is azol-1-yl (Z-23), R³ is CH₃ and Q is Q4, and R^{a} and R⁴ are as per any row of Table P below, which compounds are named I.A1-Z23-Q4-P-1 to I.A1-Z23-Q4-P-150.

In another embodiment, compounds I are of formula I.A2 wherein Z is azol-1-yl (Z-23), R³ is CH₃ and Q is Q1, and R^{a} and R⁴ are as per any row of Table P below, which compounds are named I.A2-Z23-Q1-P-1 to I.A2-Z23-Q1-P-150.

In another embodiment, compounds I are of formula I.A2 wherein Z is azol-1-yl (Z-23), R³ is CH₃ and Q is Q2, and R^{a} and R⁴ are as per any row of Table P below, which compounds are named I.A2-Z23-Q2-P-1 to I.A2-Z23-Q2-P-150.

In another embodiment, compounds I are of formula I.A2 wherein Z is azol-1-yl (Z-23), R³ is CH₃ and Q is Q3, and R^{a} and R⁴ are as per any row of Table P below, which compounds are named I.A2-Z23-Q3-P-1 to I.A2-Z23-Q3-P-150.

In another embodiment, compounds I are of formula I.A2 wherein Z is azol-1-yl (Z-23), R³ is CH₃ and Q is Q4, and R^{a} and R⁴ are as per any row of Table P below, which compounds are named I.A2-Z23-Q4-P-1 to I.A2-Z23-Q4-P-150.

In another embodiment, compounds I are of formula I.B1 wherein Z is azol-1-yl (Z-23) and Q is Q1, and R^{a} and R⁴ are as per any row of Table P below, which compounds are named I.B1-Z23-Q1-P-1 to I.B1-Z23-Q1-P-150.

In another embodiment, compounds I are of formula I.B1 wherein Z is azol-1-yl (Z-23) and Q is Q2, and R^{a} and R⁴ are as per any row of Table P below, which compounds are named I.B1-Z23-Q2-P-1 to I.B1-Z23-Q2-P-150.

In another embodiment, compounds I are of formula I.B1 wherein Z is azol-1-yl (Z-23) and Q is Q3, and R^{a} and R⁴ are as per any row of Table P below, which compounds are named I.B1-Z23-Q3-P-1 to I.B1-Z23-Q3-P-150.

In another embodiment, compounds I are of formula I.B1 wherein Z is azol-1-yl (Z-23) and Q is Q4, and R^{a} and R⁴ are as per any row of Table P below, which compounds are named I.B1-Z23-Q4-P-1 to I.B1-Z23-Q4-P-150.

In another embodiment, compounds I are of formula I.B2 wherein Z is azol-1-yl (Z-23) and Q is Q1, and R^{a} and R⁴ are as per any row of Table P below, which compounds are named I.B2-Z23-Q1-P-1 to I.B2-Z23-Q1-P-150.

In another embodiment, compounds I are of formula I.B2 wherein Z is azol-1-yl (Z-23) and Q is Q2, and R^{a} and R⁴ are as per any row of Table P below, which compounds are named I.B2-Z23-Q2-P-1 to I.B2-Z23-Q2-P-150.

In another embodiment, compounds I are of formula I.B2 wherein Z is azol-1-yl (Z-23) and Q is Q3, and R^{a} and R⁴ are as per any row of Table P below, which compounds are named I.B2-Z23-Q3-P-1 to I.B2-Z23-Q3-P-150.

In another embodiment, compounds I are of formula I.B2 wherein Z is azol-1-yl (Z-23) and Q is Q4, and R^{a} and R⁴ are as per any row of Table P below, which compounds are named I.B2-Z23-Q4-P-1 to I.B2-Z23-Q4-P-150.

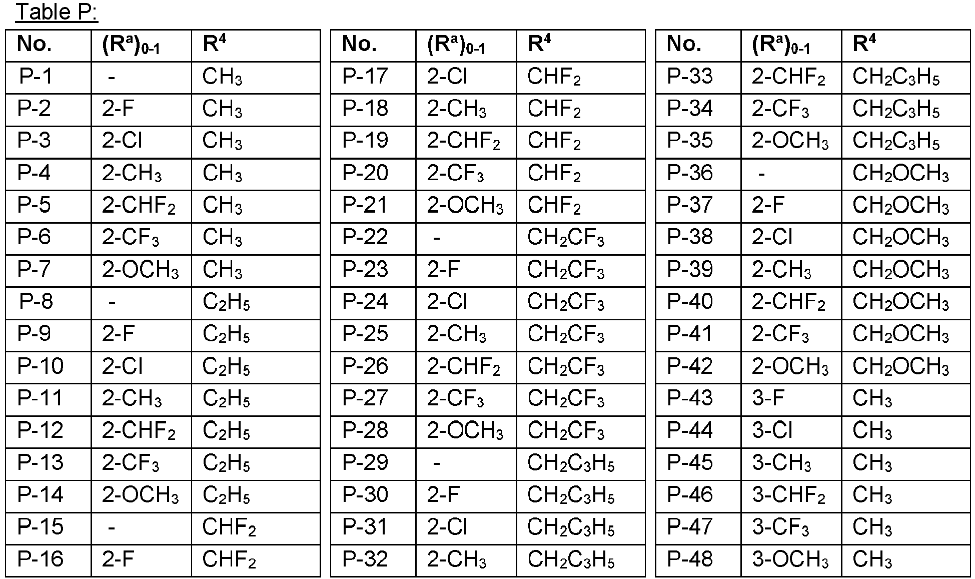

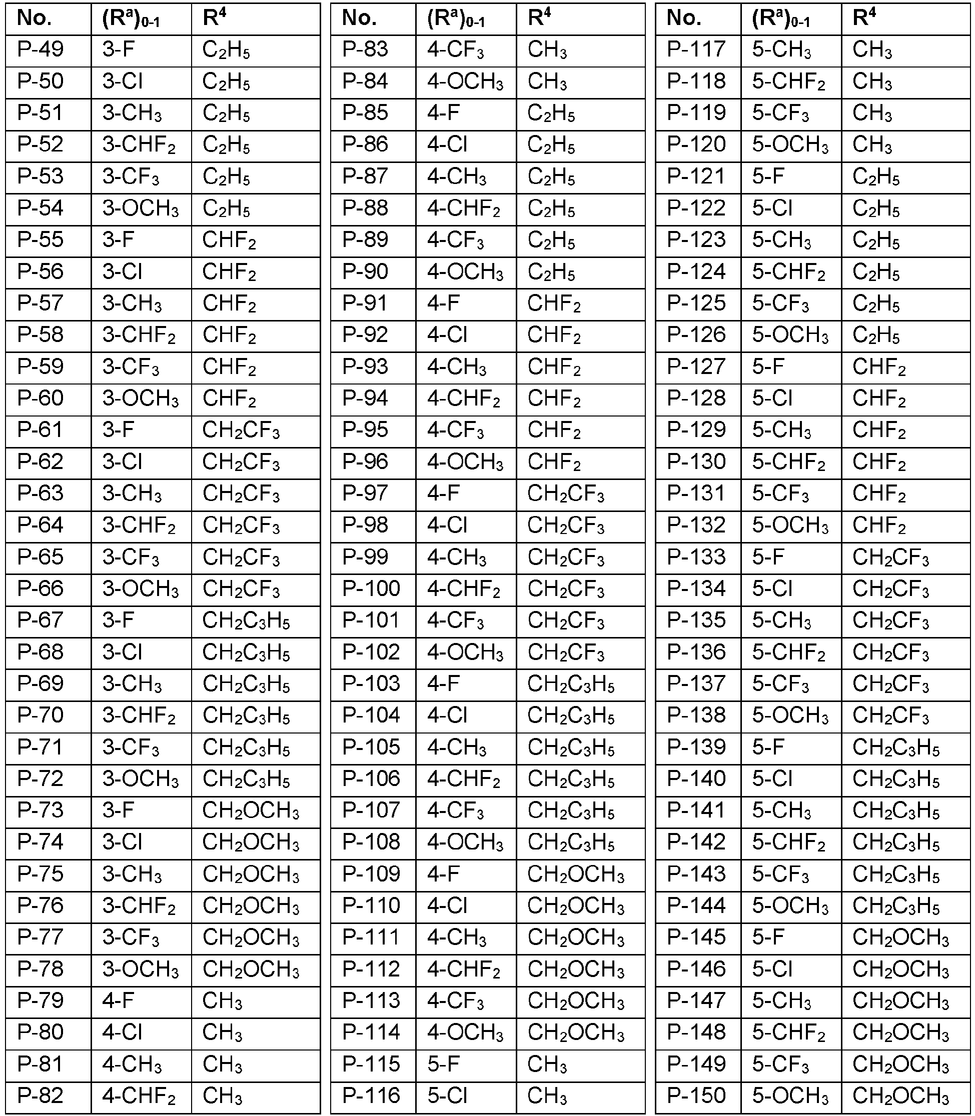

In another embodiment, compounds I are of formula I.A1 wherein Z is pyridin-4-yl (Z-18), R³ is CH₃ and Q is Q1, and R^{a} and R⁴ are as per any row of Table Q below, which compounds are named I.A1-Z18-Q1-Q-1 to I.A1-Z18-Q1-Q-150.

In another embodiment, compounds I are of formula I.A1 wherein Z is pyridin-4-yl (Z-18), R³ is CH₃ and Q is Q2, and R^{a} and R⁴ are as per any row of Table Q below, which compounds are named I.A1-Z18-Q2-Q-1 to I.A1-Z18-Q2-Q-150.

In another embodiment, compounds I are of formula I.A1 wherein Z is pyridin-4-yl (Z-18), R³ is CH₃ and Q is Q3, and R^{a} and R⁴ are as per any row of Table Q below, which compounds are named I.A1-Z18-Q3-Q-1 to I.A1-Z18-Q3-Q-150.

In another embodiment, compounds I are of formula I.A1 wherein Z is pyridin-4-yl (Z-18), R³ is CH₃ and Q is Q4, and R^{a} and R⁴ are as per any row of Table Q below, which compounds are named I.A1-Z18-Q4-Q-1 to I.A1-Z18-Q4-Q-150.

In another embodiment, compounds I are of formula I.A2 wherein Z is pyridin-4-yl (Z-18), R³ is CH₃ and Q is Q1, and R^{a} and R⁴ are as per any row of Table Q below, which compounds are named I.A2-Z18-Q1-Q-1 to I.A2-Z18-Q1-Q-150.

In another embodiment, compounds I are of formula I.A2 wherein Z is pyridin-4-yl (Z-18), R³ is CH₃ and Q is Q2, and R^{a} and R⁴ are as per any row of Table Q below, which compounds are named I.A2-Z18-Q2-Q-1 to I.A2-Z18-Q2-Q-150.

In another embodiment, compounds I are of formula I.A2 wherein Z is pyridin-4-yl (Z-18), R³ is CH₃ and Q is Q3, and R^{a} and R⁴ are as per any row of Table Q below, which compounds are named I.A2-Z18-Q3-Q-1 to I.A2-Z18-Q3-Q-150.

In another embodiment, compounds I are of formula I.A2 wherein Z is pyridin-4-yl (Z-18), R³ is CH₃ and Q is Q4, and R^{a} and R⁴ are as per any row of Table Q below, which compounds are named I.A2-Z18-Q4-Q-1 to I.A2-Z18-Q4-Q-150.

In another embodiment, compounds I are of formula I.B1 wherein Z is pyridin-4-yl (Z-18) and Q is Q1, and R^{a} and R⁴ are as per any row of Table Q below, which compounds are named I.B1-Z18-Q1-Q-1 to I.B1-Z18-Q1-Q-150.

In another embodiment, compounds I are of formula I.B1 wherein Z is pyridin-4-yl (Z-18) and Q is Q2, and R^{a} and R⁴ are as per any row of Table Q below, which compounds are named I.B1-Z18-Q2-Q-1 to I.B1-Z18-Q2-Q-150.

In another embodiment, compounds I are of formula I.B1 wherein Z is pyridin-4-yl (Z-18) and Q is Q3, and R^{a} and R⁴ are as per any row of Table Q below, which compounds are named I.B1-Z18-Q3-Q-1 to I.B1-Z18-Q3-Q-150.

In another embodiment, compounds I are of formula I.B1 wherein Z is pyridin-4-yl (Z-18) and Q is Q4, and R^{a} and R⁴ are as per any row of Table Q below, which compounds are named I.B1-Z18-Q4-Q-1 to I.B1-Z18-Q4-Q-150.

In another embodiment, compounds I are of formula I.B2 wherein Z is pyridin-4-yl (Z-18) and Q is Q1, and R^{a} and R⁴ are as per any row of Table Q below, which compounds are named I.B2-Z18-Q1-Q-1 to I.B2-Z18-Q1-Q-150.

In another embodiment, compounds I are of formula I.B2 wherein Z is pyridin-4-yl (Z-18) and Q is Q2, and R^{a} and R⁴ are as per any row of Table Q below, which compounds are named I.B2-Z18-Q2-Q-1 to I.B2-Z18-Q2-Q-150.

In another embodiment, compounds I are of formula I.B2 wherein Z is pyridin-4-yl (Z-18) and Q is Q3, and R^{a} and R⁴ are as per any row of Table Q below, which compounds are named I.B2-Z18-Q3-Q-1 to I.B2-Z18-Q3-Q-150.

In another embodiment, compounds I are of formula I.B2 wherein Z is pyridin-4-yl (Z-18) and Q is Q4, and R^{a} and R⁴ are as per any row of Table Q below, which compounds are named I.B2-Z18-Q4-Q-1 to I.B2-Z18-Q4-Q-150.

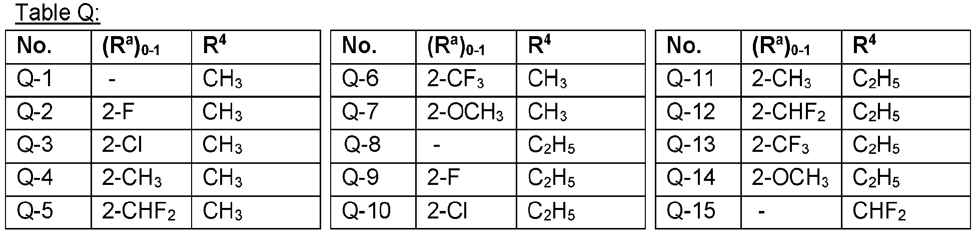

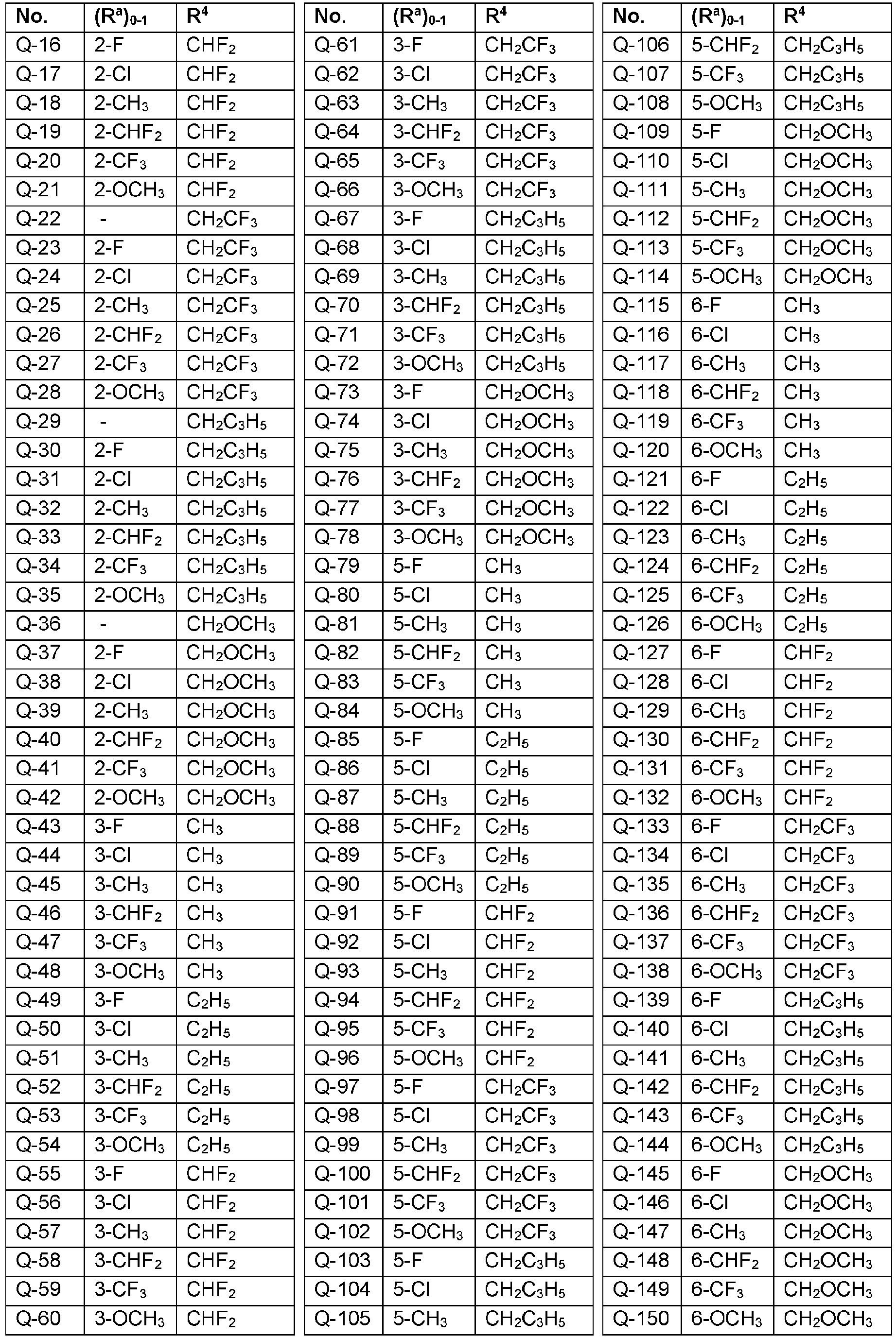

In another embodiment, compounds I are of formula I.A1 wherein Z is pyridin-3-yl (Z-17), R³ is CH₃ and Q is Q1, and R^{a} and R⁴ are as per any row of Table R below, which compounds are named I.A1-Z17-Q1-R-1 to I.A1-Z17-Q1-R-150.

In another embodiment, compounds I are of formula I.A1 wherein Z is pyridin-3-yl (Z-17), R³ is CH₃ and Q is Q2, and R^{a} and R⁴ are as per any row of Table R below, which compounds are named I.A1-Z17-Q2-R-1 to I.A1-Z17-Q2-R-150.

In another embodiment, compounds I are of formula I.A1 wherein Z is pyridin-3-yl (Z-17), R³ is CH₃ and Q is Q3, and R^{a} and R⁴ are as per any row of Table R below, which compounds are named I.A1-Z17-Q3-R-1 to I.A1-Z17-Q3-R-150.

In another embodiment, compounds I are of formula I.A1 wherein Z is pyridin-3-yl (Z-17), R³ is CH₃ and Q is Q4, and R^{a} and R⁴ are as per any row of Table R below, which compounds are named I.A1-Z17-Q4-R-1 to I.A1-Z17-Q4-R-150.

In another embodiment, compounds I are of formula I.A2 wherein Z is pyridin-3-yl (Z-17), R³ is CH₃ and Q is Q1, and R^{a} and R⁴ are as per any row of Table R below, which compounds are named I.A2-Z17-Q1-R-1 to I.A2-Z17-Q1-R-150.

In another embodiment, compounds I are of formula I.A2 wherein Z is pyridin-3-yl (Z-17), R³ is CH₃ and Q is Q2, and R^{a} and R⁴ are as per any row of Table R below, which compounds are named I.A2-Z17-Q2-R-1 to I.A2-Z17-Q2-R-150.

In another embodiment, compounds I are of formula I.A2 wherein Z is pyridin-3-yl (Z-17), R³ is CH₃ and Q is Q3, and R^{a} and R⁴ are as per any row of Table R below, which compounds are named I.A2-Z17-Q3-R-1 to I.A2-Z17-Q3-R-150.

In another embodiment, compounds I are of formula I.A2 wherein Z is pyridin-3-yl (Z-17), R³ is CH₃ and Q is Q4, and R^{a} and R⁴ are as per any row of Table R below, which compounds are named I.A2-Z17-Q4-R-1 to I.A2-Z17-Q4-R-150.

In another embodiment, compounds I are of formula I.B1 wherein Z is pyridin-3-yl (Z-17) and Q is Q1, and R^{a} and R⁴ are as per any row of Table R below, which compounds are named I.B1-Z17-Q1-R-1 to I.B1-Z17-Q1-R-150.

In another embodiment, compounds I are of formula I.B1 wherein Z is pyridin-3-yl (Z-17) and Q is Q2, and R^{a} and R⁴ are as per any row of Table R below, which compounds are named I.B1-Z17-Q2-R-1 to I.B1-Z17-Q2-R-150.

In another embodiment, compounds I are of formula I.B1 wherein Z is pyridin-3-yl (Z-17) and Q is Q3, and R^{a} and R⁴ are as per any row of Table R below, which compounds are named I.B1-Z17-Q3-R-1 to I.B1-Z17-Q3-R-150.

In another embodiment, compounds I are of formula I.B1 wherein Z is pyridin-3-yl (Z-17) and Q is Q4, and R^{a} and R⁴ are as per any row of Table R below, which compounds are named I.B1-Z17-Q4-R-1 to I.B1-Z17-Q4-R-150.

In another embodiment, compounds I are of formula I.B2 wherein Z is pyridin-3-yl (Z-17) and Q is Q1, and R^{a} and R⁴ are as per any row of Table R below, which compounds are named I.B2-Z17-Q1-R-1 to I.B2-Z17-Q1-R-150.

In another embodiment, compounds I are of formula I.B2 wherein Z is pyridin-3-yl (Z-17) and Q is Q2, and R^{a} and R⁴ are as per any row of Table R below, which compounds are named I.B2-Z17-Q2-R-1 to I.B2-Z17-Q2-R-150.

In another embodiment, compounds I are of formula I.B2 wherein Z is pyridin-3-yl (Z-17) and Q is Q3, and R^{a} and R⁴ are as per any row of Table R below, which compounds are named I.B2-Z17-Q3-R-1 to I.B2-Z17-Q3-R-150.

In another embodiment, compounds I are of formula I.B2 wherein Z is pyridin-3-yl (Z-17) and Q is Q4, and R^{a} and R⁴ are as per any row of Table R below, which compounds are named I.B2-Z17-Q4-R-1 to I.B2-Z17-Q4-R-150.

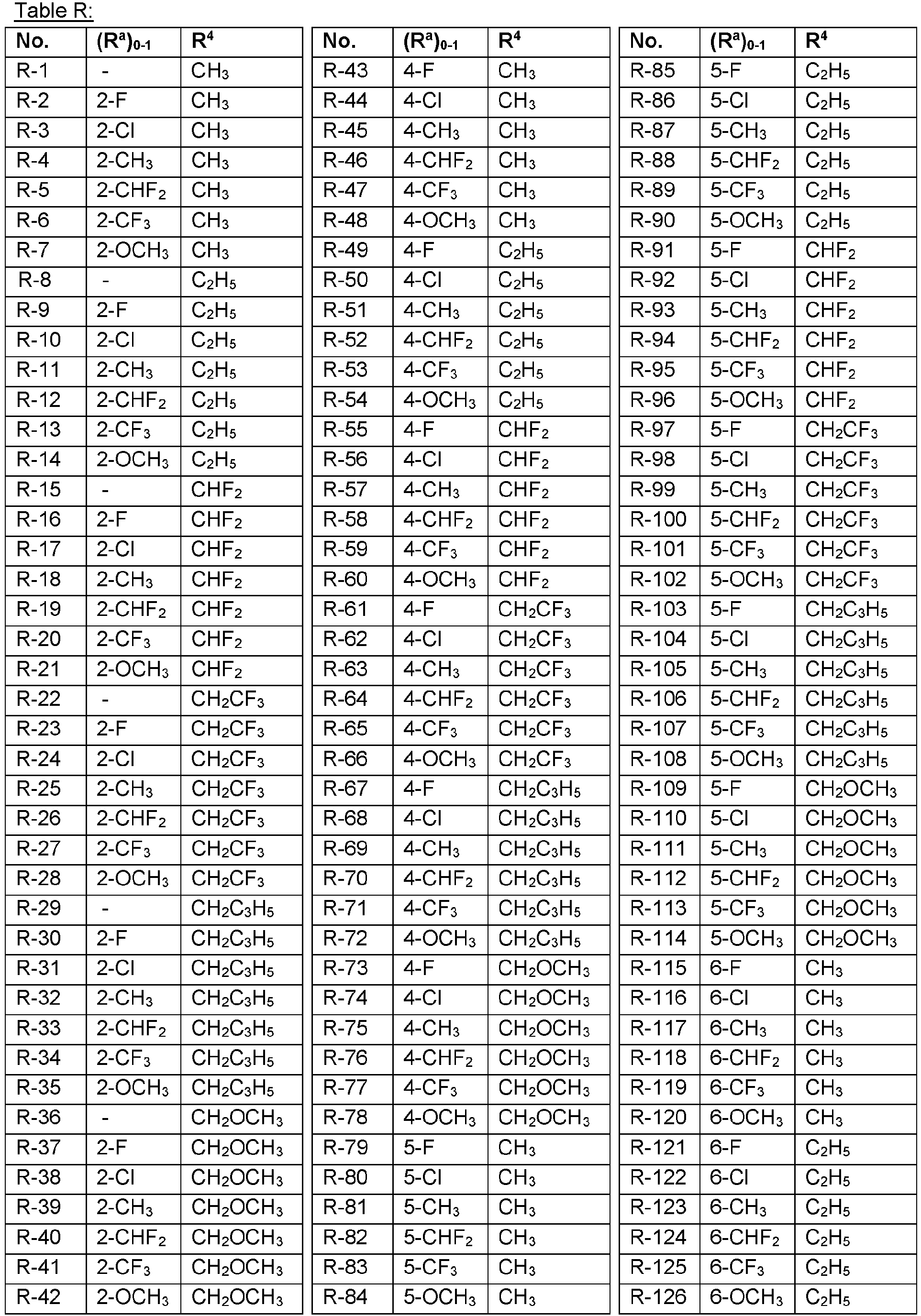

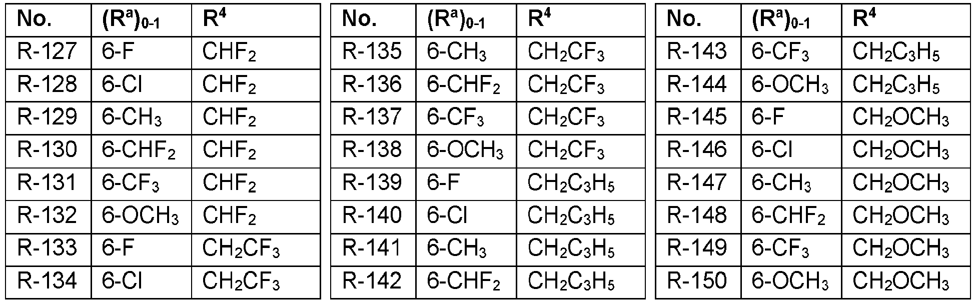

In another embodiment, compounds I are of formula I.A1 wherein Z is pyridin-2-yl (Z-16), R³ is CH₃ and Q is Q1, and R^{a} and R⁴ are as per any row of Table T below, which compounds are named I.A1-Z16-Q1-T-1 to I.A1-Z16-Q1-T-150.

In another embodiment, compounds I are of formula I.A1 wherein Z is pyridin-2-yl (Z-16), R³ is CH₃ and Q is Q2, and R^{a} and R⁴ are as per any row of Table T below, which compounds are named I.A1-Z16-Q2-T-1 to I.A1-Z16-Q2-T-150.

In another embodiment, compounds I are of formula I.A1 wherein Z is pyridin-2-yl (Z-16), R³ is CH₃ and Q is Q3, and R^{a} and R⁴ are as per any row of Table T below, which compounds are named I.A1-Z16-Q3-T-1 to I.A1-Z16-Q3-T-150.

In another embodiment, compounds I are of formula I.A1 wherein Z is pyridin-2-yl (Z-16), R³ is CH₃ and Q is Q4, and R^{a} and R⁴ are as per any row of Table T below, which compounds are named I.A1-Z16-Q4-T-1 to I.A1-Z16-Q4-T-150.

In another embodiment, compounds I are of formula I.A2 wherein Z is pyridin-2-yl (Z-16), R³ is CH₃ and Q is Q1, and R^{a} and R⁴ are as per any row of Table T below, which compounds are named I.A2-Z16-Q1-T-1 to I.A2-Z16-Q1-T-150.

In another embodiment, compounds I are of formula I.A2 wherein Z is pyridin-2-yl (Z-16), R³ is CH₃ and Q is Q2, and R^{a} and R⁴ are as per any row of Table T below, which compounds are named I.A2-Z16-Q2-T-1 to I.A2-Z16-Q2-T-150.

In another embodiment, compounds I are of formula I.A2 wherein Z is pyridin-2-yl (Z-16), R³ is CH₃ and Q is Q3, and R^{a} and R⁴ are as per any row of Table T below, which compounds are named I.A2-Z16-Q3-T-1 to I.A2-Z16-Q3-T-150.

In another embodiment, compounds I are of formula I.A2 wherein Z is pyridin-2-yl (Z-16), R³ is CH₃ and Q is Q4, and R^{a} and R⁴ are as per any row of Table T below, which compounds are named I.A2-Z16-Q4-T-1 to I.A2-Z16-Q4-T-150.

In another embodiment, compounds I are of formula I.B1 wherein Z is pyridin-2-yl (Z-16) and Q is Q1, and R^{a} and R⁴ are as per any row of Table T below, which compounds are named I.B1-Z16-Q1-T-1 to I.B1-Z16-Q1-T-150.

In another embodiment, compounds I are of formula I.B1 wherein Z is pyridin-2-yl (Z-16) and Q is Q2, and R^{a} and R⁴ are as per any row of Table T below, which compounds are named I.B1-Z16-Q2-T-1 to I.B1-Z16-Q2-T-150.

In another embodiment, compounds I are of formula I.B1 wherein Z is pyridin-2-yl (Z-16) and Q is Q3, and R^{a} and R⁴ are as per any row of Table T below, which compounds are named I.B1-Z16-Q3-T-1 to I.B1-Z16-Q3-T-150.

In another embodiment, compounds I are of formula I.B1 wherein Z is pyridin-2-yl (Z-16) and Q is Q4, and R^{a} and R⁴ are as per any row of Table T below, which compounds are named I.B1-Z16-Q4-T-1 to I.B1-Z16-Q4-T-150.

In another embodiment, compounds I are of formula I.B2 wherein Z is pyridin-2-yl (Z-16) and Q is Q1, and R^{a} and R⁴ are as per any row of Table T below, which compounds are named I.B2-Z16-Q1-T-1 to I.B2-Z16-Q1-T-150.

In another embodiment, compounds I are of formula I.B2 wherein Z is pyridin-2-yl (Z-16) and Q is Q2, and R^{a} and R⁴ are as per any row of Table T below, which compounds are named I.B2-Z16-Q2-T-1 to I.B2-Z16-Q2-T-150.

In another embodiment, compounds I are of formula I.B2 wherein Z is pyridin-2-yl (Z-16) and Q is Q3, and R^{a} and R⁴ are as per any row of Table T below, which compounds are named I.B2-Z16-Q3-T-1 to I.B2-Z16-Q3-T-150.

In another embodiment, compounds I are of formula I.B2 wherein Z is pyridin-2-yl (Z-16) and Q is Q4, and R^{a} and R⁴ are as per any row of Table T below, which compounds are named I.B2-Z16-Q4-T-1 to I.B2-Z16-Q4-T-150.

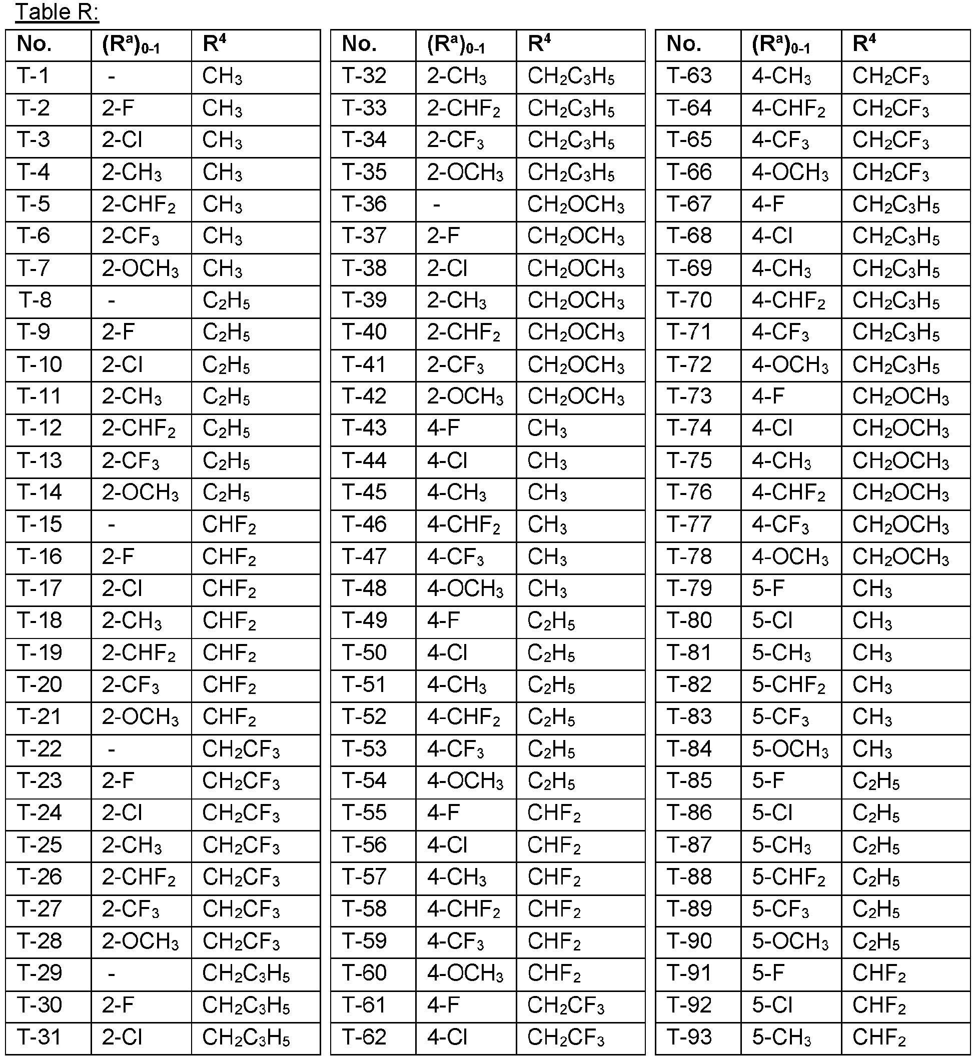

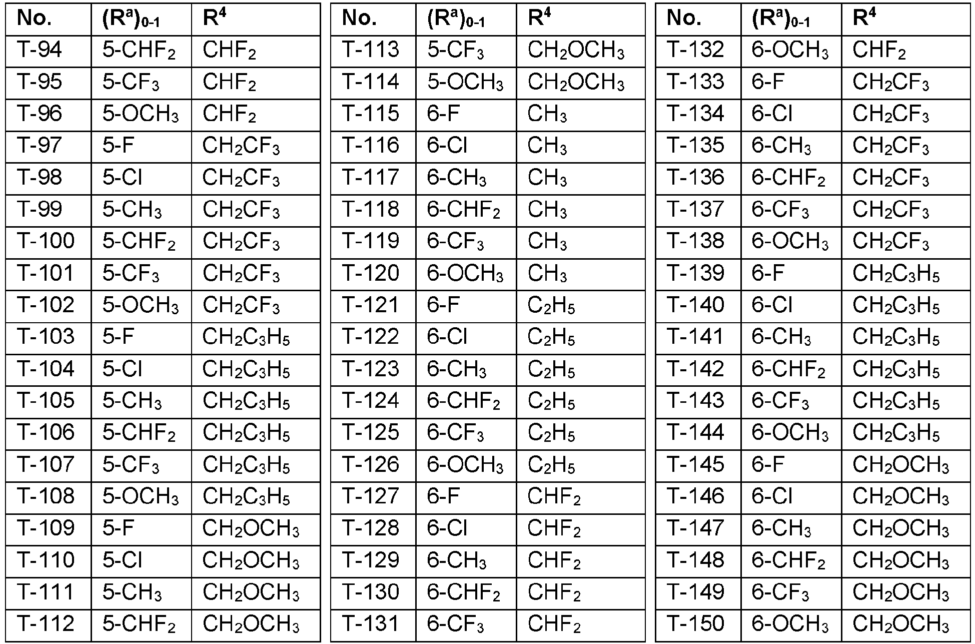

In another embodiment, compounds I are of formula I.A1 wherein Z is Z-59, R³ is CH₃ and Q is Q1, and R and R⁴ are as per any row of Table U below, which compounds are named I.A1-Z59-Q1-U-1 to I.A1-Z59-Q1-U-42.

In another embodiment, compounds I are of formula I.A1 wherein Z is Z-59, R³ is CH₃ and Q is Q2, and R and R⁴ are as per any row of Table U below, which compounds are named I.A1-Z59-Q2-U-1 to I.A1-Z59-Q2-U-42.

In another embodiment, compounds I are of formula I.A1 wherein Z is Z-59, R³ is CH₃ and Q is Q3, and R and R⁴ are as per any row of Table U below, which compounds are named I.A1-Z59-Q3-U-1 to I.A1-Z59-Q3-U-42.

In another embodiment, compounds I are of formula I.A1 wherein Z is Z-59, R³ is CH₃ and Q is Q4, and R and R⁴ are as per any row of Table U below, which compounds are named I.A1-Z59-Q4-U-1 to I.A1-Z59-Q4-U-42.

In another embodiment, compounds I are of formula I.A2 wherein Z is Z-59, R³ is CH₃ and Q is Q1, and R and R⁴ are as per any row of Table U below, which compounds are named I.A2-Z59-Q1-U-1 to I.A2-Z59-Q1-U-42.

In another embodiment, compounds I are of formula I.A2 wherein Z is Z-59, R³ is CH₃ and Q is Q2, and R and R⁴ are as per any row of Table U below, which compounds are named I.A2-Z59-Q2-U-1 to I.A2-Z59-Q2-U-42.

In another embodiment, compounds I are of formula I.A2 wherein Z is Z-59, R³ is CH₃ and Q is Q3, and R and R⁴ are as per any row of Table U below, which compounds are named I.A2-Z59-Q3-U-1 to I.A2-Z59-Q3-U-42.

In another embodiment, compounds I are of formula I.A2 wherein Z is Z-59, R³ is CH₃ and Q is Q4, and R and R⁴ are as per any row of Table U below, which compounds are named I.A2-Z59-Q4-U-1 to I.A2-Z59-Q4-U-42.

In another embodiment, compounds I are of formula I.A1 wherein Z is Z-60, R³ is CH₃ and Q is Q1, and R and R⁴ are as per any row of Table U below, which compounds are named I.A1-Z60-Q1-U-1 to I.A1-Z60-Q1-U-42.

In another embodiment, compounds I are of formula I.A1 wherein Z is Z-60, R³ is CH₃ and Q is Q2, and R and R⁴ are as per any row of Table U below, which compounds are named I.A1-Z60-Q2-U-1 to I.A1-Z60-Q2-U-42.

In another embodiment, compounds I are of formula I.A1 wherein Z is Z-60, R³ is CH₃ and Q is Q3, and R and R⁴ are as per any row of Table U below, which compounds are named I.A1-Z60-Q3-U-1 to I.A1-Z60-Q3-U-42.

In another embodiment, compounds I are of formula I.A1 wherein Z is Z-60, R³ is CH₃ and Q is Q4, and R and R⁴ are as per any row of Table U below, which compounds are named I.A1-Z60-Q4-U-1 to I.A1-Z60-Q4-U-42.

In another embodiment, compounds I are of formula I.A2 wherein Z is Z-60, R³ is CH₃ and Q is Q1, and R and R⁴ are as per any row of Table U below, which compounds are named I.A2-Z60-Q1-U-1 to I.A2-Z60-Q1-U-42.

In another embodiment, compounds I are of formula I.A2 wherein Z is Z-60, R³ is CH₃ and Q is Q2, and R and R⁴ are as per any row of Table U below, which compounds are named I.A2-Z60-Q2-U-1 to I.A2-Z60-Q2-U-42.

In another embodiment, compounds I are of formula I.A2 wherein Z is Z-60, R³ is CH₃ and Q is Q3, and R and R⁴ are as per any row of Table U below, which compounds are named I.A2-Z60-Q3-U-1 to I.A2-Z60-Q3-U-42.

In another embodiment, compounds I are of formula I.A2 wherein Z is Z-60, R³ is CH₃ and Q is Q4, and R and R⁴ are as per any row of Table U below, which compounds are named I.A2-Z60-Q4-U-1 to I.A2-Z60-Q4-U-42.

In another embodiment, compounds I are of formula I.A1 wherein Z is Z-61, R³ is CH₃ and Q is Q1, and R and R⁴ are as per any row of Table U below, which compounds are named I.A1-Z61-Q1-U-1 to I.A1-Z61-Q1-U-42.

In another embodiment, compounds I are of formula I.A1 wherein Z is Z-61, R³ is CH₃ and Q is Q2, and R and R⁴ are as per any row of Table U below, which compounds are named I.A1-Z61-Q2-U-1 to I.A1-Z61-Q2-U-42.

In another embodiment, compounds I are of formula I.A1 wherein Z is Z-61, R³ is CH₃ and Q is Q3, and R and R⁴ are as per any row of Table U below, which compounds are named I.A1-Z61-Q3-U-1 to I.A1-Z61-Q3-U-42.

In another embodiment, compounds I are of formula I.A1 wherein Z is Z-61, R³ is CH₃ and Q is Q4, and R and R⁴ are as per any row of Table U below, which compounds are named I.A1-Z61-Q4-U-1 to I.A1-Z61-Q4-U-42.

In another embodiment, compounds I are of formula I.A2 wherein Z is Z-61, R³ is CH₃ and Q is Q1, and R and R⁴ are as per any row of Table U below, which compounds are named I.A2-Z61-Q1-U-1 to I.A2-Z61-Q1-U-42.

In another embodiment, compounds I are of formula I.A2 wherein Z is Z-61, R³ is CH₃ and Q is Q2, and R and R⁴ are as per any row of Table U below, which compounds are named I.A2-Z61-Q2-U-1 to I.A2-Z61-Q2-U-42.

In another embodiment, compounds I are of formula I.A2 wherein Z is Z-61, R³ is CH₃ and Q is Q3, and R and R⁴ are as per any row of Table U below, which compounds are named I.A2-Z61-Q3-U-1 to I.A2-Z61-Q3-U-42.

In another embodiment, compounds I are of formula I.A2 wherein Z is Z-61, R³ is CH₃ and Q is Q4, and R and R⁴ are as per any row of Table U below, which compounds are named I.A2-Z61-Q4-U-1 to I.A2-Z61-Q4-U-42.

In another embodiment, compounds I are of formula I.A1 wherein Z is Z-62, R³ is CH₃ and Q is Q1, and R and R⁴ are as per any row of Table U below, which compounds are named I.A1-Z62-Q1-U-1 to I.A1-Z62-Q1-U-42.

In another embodiment, compounds I are of formula I.A1 wherein Z is Z-62, R³ is CH₃ and Q is Q2, and R and R⁴ are as per any row of Table U below, which compounds are named I.A1-Z62-Q2-U-1 to I.A1-Z62-Q2-U-42.

In another embodiment, compounds I are of formula I.A1 wherein Z is Z-62, R³ is CH₃ and Q is Q3, and R and R⁴ are as per any row of Table U below, which compounds are named I.A1-Z62-Q3-U-1 to I.A1-Z62-Q3-U-42.

In another embodiment, compounds I are of formula I.A1 wherein Z is Z-62, R³ is CH₃ and Q is Q4, and R and R⁴ are as per any row of Table U below, which compounds are named I.A1-Z62-Q4-U-1 to I.A1-Z62-Q4-U-42.

In another embodiment, compounds I are of formula I.A2 wherein Z is Z-62, R³ is CH₃ and Q is Q1, and R and R⁴ are as per any row of Table U below, which compounds are named I.A2-Z62-Q1-U-1 to I.A2-Z62-Q1-U-42.

In another embodiment, compounds I are of formula I.A2 wherein Z is Z-62, R³ is CH₃ and Q is Q2, and R and R⁴ are as per any row of Table U below, which compounds are named I.A2-Z62-Q2-U-1 to I.A2-Z62-Q2-U-42.

In another embodiment, compounds I are of formula I.A2 wherein Z is Z-62, R³ is CH₃ and Q is Q3, and R and R⁴ are as per any row of Table U below, which compounds are named I.A2-Z62-Q3-U-1 to I.A2-Z62-Q3-U-42.

In another embodiment, compounds I are of formula I.A2 wherein Z is Z-62, R³ is CH₃ and Q is Q4, and R and R⁴ are as per any row of Table U below, which compounds are named I.A2-Z62-Q4-U-1 to I.A2-Z62-Q4-U-42.

In another embodiment, compounds I are of formula I.A1 wherein Z is Z-63, R³ is CH₃ and Q is Q1, and R and R⁴ are as per any row of Table U below, which compounds are named I.A1-Z63-Q1-U-1 to I.A1-Z63-Q1-U-42.

In another embodiment, compounds I are of formula I.A1 wherein Z is Z-63, R³ is CH₃ and Q is Q2, and R and R⁴ are as per any row of Table U below, which compounds are named I.A1-Z63-Q2-U-1 to I.A1-Z63-Q2-U-42.

In another embodiment, compounds I are of formula I.A1 wherein Z is Z-63, R³ is CH₃ and Q is Q3, and R and R⁴ are as per any row of Table U below, which compounds are named I.A1-Z63-Q3-U-1 to I.A1-Z63-Q3-U-42.

In another embodiment, compounds I are of formula I.A1 wherein Z is Z-63, R³ is CH₃ and Q is Q4, and R and R⁴ are as per any row of Table U below, which compounds are named I.A1-Z63-Q4-U-1 to I.A1-Z63-Q4-U-42.

In another embodiment, compounds I are of formula I.A2 wherein Z is Z-63, R³ is CH₃ and Q is Q1, and R and R⁴ are as per any row of Table U below, which compounds are named I.A2-Z63-Q1-U-1 to I.A2-Z63-Q1-U-42.

In another embodiment, compounds I are of formula I.A2 wherein Z is Z-63, R³ is CH₃ and Q is Q2, and R and R⁴ are as per any row of Table U below, which compounds are named I.A2-Z63-Q2-U-1 to I.A2-Z63-Q2-U-42.

In another embodiment, compounds I are of formula I.A2 wherein Z is Z-63, R³ is CH₃ and Q is Q3, and R and R⁴ are as per any row of Table U below, which compounds are named I.A2-Z63-Q3-U-1 to I.A2-Z63-Q3-U-42.

In another embodiment, compounds I are of formula I.A2 wherein Z is Z-63, R³ is CH₃ and Q is Q4, and R and R⁴ are as per any row of Table U below, which compounds are named I.A2-Z63-Q4-U-1 to I.A2-Z63-Q4-U-42.

In another embodiment, compounds I are of formula I.A1 wherein Z is Z-64, R³ is CH₃ and Q is Q1, and R and R⁴ are as per any row of Table U below, which compounds are named I.A1-Z64-Q1-U-1 to I.A1-Z64-Q1-U-42.

In another embodiment, compounds I are of formula I.A1 wherein Z is Z-64, R³ is CH₃ and Q is Q2, and R and R⁴ are as per any row of Table U below, which compounds are named I.A1-Z64-Q2-U-1 to I.A1-Z64-Q2-U-42.

In another embodiment, compounds I are of formula I.A1 wherein Z is Z-64, R³ is CH₃ and Q is Q3, and R and R⁴ are as per any row of Table U below, which compounds are named I.A1-Z64-Q3-U-1 to I.A1-Z64-Q3-U-42.

In another embodiment, compounds I are of formula I.A1 wherein Z is Z-64, R³ is CH₃ and Q is Q4, and R and R⁴ are as per any row of Table U below, which compounds are named I.A1-Z64-Q4-U-1 to I.A1-Z64-Q4-U-42.

In another embodiment, compounds I are of formula I.A2 wherein Z is Z-64, R³ is CH₃ and Q is Q1, and R and R⁴ are as per any row of Table U below, which compounds are named I.A2-Z64-Q1-U-1 to I.A2-Z64-Q1-U-42.

In another embodiment, compounds I are of formula I.A2 wherein Z is Z-64, R³ is CH₃ and Q is Q2, and R and R⁴ are as per any row of Table U below, which compounds are named I.A2-Z64-Q2-U-1 to I.A2-Z64-Q2-U-42.

In another embodiment, compounds I are of formula I.A2 wherein Z is Z-64, R³ is CH₃ and Q is Q3, and R and R⁴ are as per any row of Table U below, which compounds are named I.A2-Z64-Q3-U-1 to I.A2-Z64-Q3-U-42.

In another embodiment, compounds I are of formula I.A2 wherein Z is Z-64, R³ is CH₃ and Q is Q4, and R and R⁴ are as per any row of Table U below, which compounds are named I.A2-Z64-Q4-U-1 to I.A2-Z64-Q4-U-42.

In another embodiment, compounds I are of formula I.A1 wherein Z is Z-65, R³ is CH₃ and Q is Q1, and R and R⁴ are as per any row of Table U below, which compounds are named I.A1-Z65-Q1-U-1 to I.A1-Z65-Q1-U-42.

In another embodiment, compounds I are of formula I.A1 wherein Z is Z-65, R³ is CH₃ and Q is Q2, and R and R⁴ are as per any row of Table U below, which compounds are named I.A1-Z65-Q2-U-1 to I.A1-Z65-Q2-U-42.

In another embodiment, compounds I are of formula I.A1 wherein Z is Z-65, R³ is CH₃ and Q is Q3, and R and R⁴ are as per any row of Table U below, which compounds are named I.A1-Z65-Q3-U-1 to I.A1-Z65-Q3-U-42.

In another embodiment, compounds I are of formula I.A1 wherein Z is Z-65, R³ is CH₃ and Q is Q4, and R and R⁴ are as per any row of Table U below, which compounds are named I.A1-Z65-Q4-U-1 to I.A1-Z65-Q4-U-42.

In another embodiment, compounds I are of formula I.A2 wherein Z is Z-65, R³ is CH₃ and Q is Q1, and R and R⁴ are as per any row of Table U below, which compounds are named I.A2-Z65-Q1-U-1 to I.A2-Z65-Q1-U-42.

In another embodiment, compounds I are of formula I.A2 wherein Z is Z-65, R³ is CH₃ and Q is Q2, and R and R⁴ are as per any row of Table U below, which compounds are named I.A2-Z65-Q2-U-1 to I.A2-Z65-Q2-U-42.

In another embodiment, compounds I are of formula I.A2 wherein Z is Z-65, R³ is CH₃ and Q is Q3, and R and R⁴ are as per any row of Table U below, which compounds are named I.A2-Z65-Q3-U-1 to I.A2-Z65-Q3-U-42.

In another embodiment, compounds I are of formula I.A2 wherein Z is Z-65, R³ is CH₃ and Q is Q4, and R and R⁴ are as per any row of Table U below, which compounds are named I.A2-Z65-Q4-U-1 to I.A2-Z65-Q4-U-42.

In another embodiment, compounds I are of formula I.A1 wherein Z is Z-66, R³ is CH₃ and Q is Q1, and R and R⁴ are as per any row of Table U below, which compounds are named I.A1-Z66-Q1-U-1 to I.A1-Z66-Q1-U-42.

In another embodiment, compounds I are of formula I.A1 wherein Z is Z-66, R³ is CH₃ and Q is Q2, and R and R⁴ are as per any row of Table U below, which compounds are named I.A1-Z66-Q2-U-1 to I.A1-Z66-Q2-U-42.

In another embodiment, compounds I are of formula I.A1 wherein Z is Z-66, R³ is CH₃ and Q is Q3, and R and R⁴ are as per any row of Table U below, which compounds are named I.A1-Z66-Q3-U-1 to I.A1-Z66-Q3-U-42.

In another embodiment, compounds I are of formula I.A1 wherein Z is Z-66, R³ is CH₃ and Q is Q4, and R and R⁴ are as per any row of Table U below, which compounds are named I.A1-Z66-Q4-U-1 to I.A1-Z66-Q4-U-42.

In another embodiment, compounds I are of formula I.A2 wherein Z is Z-66, R³ is CH₃ and Q is Q1, and R and R⁴ are as per any row of Table U below, which compounds are named I.A2-Z66-Q1-U-1 to I.A2-Z66-Q1-U-42.

In another embodiment, compounds I are of formula I.A2 wherein Z is Z-66, R³ is CH₃ and Q is Q2, and R and R⁴ are as per any row of Table U below, which compounds are named I.A2-Z66-Q2-U-1 to I.A2-Z66-Q2-U-42.

In another embodiment, compounds I are of formula I.A2 wherein Z is Z-66, R³ is CH₃ and Q is Q3, and R and R⁴ are as per any row of Table U below, which compounds are named I.A2-Z66-Q3-U-1 to I.A2-Z66-Q3-U-42.

In another embodiment, compounds I are of formula I.A2 wherein Z is Z-66, R³ is CH₃ and Q is Q4, and R and R⁴ are as per any row of Table U below, which compounds are named I.A2-Z66-Q4-U-1 to I.A2-Z66-Q4-U-42.

In another embodiment, compounds I are of formula I.A1 wherein Z is Z-67, R³ is CH₃ and Q is Q1, and R and R⁴ are as per any row of Table U below, which compounds are named I.A1-Z67-Q1-U-1 to I.A1-Z67-Q1-U-42.

In another embodiment, compounds I are of formula I.A1 wherein Z is Z-67, R³ is CH₃ and Q is Q2, and R and R⁴ are as per any row of Table U below, which compounds are named I.A1-Z67-Q2-U-1 to I.A1-Z67-Q2-U-42.

In another embodiment, compounds I are of formula I.A1 wherein Z is Z-67, R³ is CH₃ and Q is Q3, and R and R⁴ are as per any row of Table U below, which compounds are named I.A1-Z67-Q3-U-1 to I.A1-Z67-Q3-U-42.

In another embodiment, compounds I are of formula I.A1 wherein Z is Z-67, R³ is CH₃ and Q is Q4, and R and R⁴ are as per any row of Table U below, which compounds are named I.A1-Z67-Q4-U-1 to I.A1-Z67-Q4-U-42.

In another embodiment, compounds I are of formula I.A2 wherein Z is Z-67, R³ is CH₃ and Q is Q1, and R and R⁴ are as per any row of Table U below, which compounds are named I.A2-Z67-Q1-U-1 to I.A2-Z67-Q1-U-42.

In another embodiment, compounds I are of formula I.A2 wherein Z is Z-67, R³ is CH₃ and Q is Q2, and R and R⁴ are as per any row of Table U below, which compounds are named I.A2-Z67-Q2-U-1 to I.A2-Z67-Q2-U-42.

In another embodiment, compounds I are of formula I.A2 wherein Z is Z-67, R³ is CH₃ and Q is Q3, and R and R⁴ are as per any row of Table U below, which compounds are named I.A2-Z67-Q3-U-1 to I.A2-Z67-Q3-U-42.

In another embodiment, compounds I are of formula I.A2 wherein Z is Z-67, R³ is CH₃ and Q is Q4, and R and R⁴ are as per any row of Table U below, which compounds are named I.A2-Z67-Q4-U-1 to I.A2-Z67-Q4-U-42.

In another embodiment, compounds I are of formula I.A1 wherein Z is Z-68, R³ is CH₃ and Q is Q1, and R and R⁴ are as per any row of Table U below, which compounds are named I.A1-Z68-Q1-U-1 to I.A1-Z68-Q1-U-42.

In another embodiment, compounds I are of formula I.A1 wherein Z is Z-68, R³ is CH₃ and Q is Q2, and R and R⁴ are as per any row of Table U below, which compounds are named I.A1-Z68-Q2-U-1 to I.A1-Z68-Q2-U-42.

In another embodiment, compounds I are of formula I.A1 wherein Z is Z-68, R³ is CH₃ and Q is Q3, and R and R⁴ are as per any row of Table U below, which compounds are named I.A1-Z68-Q3-U-1 to I.A1-Z68-Q3-U-42.

In another embodiment, compounds I are of formula I.A1 wherein Z is Z-68, R³ is CH₃ and Q is Q4, and R and R⁴ are as per any row of Table U below, which compounds are named I.A1-Z68-Q4-U-1 to I.A1-Z68-Q4-U-42.

In another embodiment, compounds I are of formula I.A2 wherein Z is Z-68, R³ is CH₃ and Q is Q1, and R and R⁴ are as per any row of Table U below, which compounds are named I.A2-Z68-Q1-U-1 to I.A2-Z68-Q1-U-42.

In another embodiment, compounds I are of formula I.A2 wherein Z is Z-68, R³ is CH₃ and Q is Q2, and R and R⁴ are as per any row of Table U below, which compounds are named I.A2-Z68-Q2-U-1 to I.A2-Z68-Q2-U-42.

In another embodiment, compounds I are of formula I.A2 wherein Z is Z-68, R³ is CH₃ and Q is Q3, and R and R⁴ are as per any row of Table U below, which compounds are named I.A2-Z68-Q3-U-1 to I.A2-Z68-Q3-U-42.

In another embodiment, compounds I are of formula I.A2 wherein Z is Z-68, R³ is CH₃ and Q is Q4, and R and R⁴ are as per any row of Table U below, which compounds are named I.A2-Z68-Q4-U-1 to I.A2-Z68-Q4-U-42.

In another embodiment, compounds I are of formula I.A1 wherein Z is Z-69, R³ is CH₃ and Q is Q1, and R and R⁴ are as per any row of Table U below, which compounds are named I.A1-Z69-Q1-U-1 to I.A1-Z69-Q1-U-42.

In another embodiment, compounds I are of formula I.A1 wherein Z is Z-69, R³ is CH₃ and Q is Q2, and R and R⁴ are as per any row of Table U below, which compounds are named I.A1-Z69-Q2-U-1 to I.A1-Z69-Q2-U-42.

In another embodiment, compounds I are of formula I.A1 wherein Z is Z-69, R³ is CH₃ and Q is Q3, and R and R⁴ are as per any row of Table U below, which compounds are named I.A1-Z69-Q3-U-1 to I.A1-Z69-Q3-U-42.

In another embodiment, compounds I are of formula I.A1 wherein Z is Z-69, R³ is CH₃ and Q is Q4, and R and R⁴ are as per any row of Table U below, which compounds are named I.A1-Z69-Q4-U-1 to I.A1-Z69-Q4-U-42.

In another embodiment, compounds I are of formula I.A2 wherein Z is Z-69, R³ is CH₃ and Q is Q1, and R and R⁴ are as per any row of Table U below, which compounds are named I.A2-Z69-Q1-U-1 to I.A2-Z69-Q1-U-42.

In another embodiment, compounds I are of formula I.A2 wherein Z is Z-69, R³ is CH₃ and Q is Q2, and R and R⁴ are as per any row of Table U below, which compounds are named I.A2-Z69-Q2-U-1 to I.A2-Z69-Q2-U-42.

In another embodiment, compounds I are of formula I.A2 wherein Z is Z-69, R³ is CH₃ and Q is Q3, and R and R⁴ are as per any row of Table U below, which compounds are named I.A2-Z69-Q3-U-1 to I.A2-Z69-Q3-U-42.

In another embodiment, compounds I are of formula I.A2 wherein Z is Z-69, R³ is CH₃ and Q is Q4, and R and R⁴ are as per any row of Table U below, which compounds are named I.A2-Z69-Q4-U-1 to I.A2-Z69-Q4-U-42.

In another embodiment, compounds I are of formula I.A1 wherein Z is Z-70, R³ is CH₃ and Q is Q1, and R and R⁴ are as per any row of Table U below, which compounds are named I.A1-Z70-Q1-U-1 to I.A1-Z70-Q1-U-42.

In another embodiment, compounds I are of formula I.A1 wherein Z is Z-70, R³ is CH₃ and Q is Q2, and R and R⁴ are as per any row of Table U below, which compounds are named I.A1-Z70-Q2-U-1 to I.A1-Z70-Q2-U-42.

In another embodiment, compounds I are of formula I.A1 wherein Z is Z-70, R³ is CH₃ and Q is Q3, and R and R⁴ are as per any row of Table U below, which compounds are named I.A1-Z70-Q3-U-1 to I.A1-Z70-Q3-U-42.

In another embodiment, compounds I are of formula I.A1 wherein Z is Z-70, R³ is CH₃ and Q is Q4, and R and R⁴ are as per any row of Table U below, which compounds are named I.A1-Z70-Q4-U-1 to I.A1-Z70-Q4-U-42.

In another embodiment, compounds I are of formula I.A2 wherein Z is Z-70, R³ is CH₃ and Q is Q1, and R and R⁴ are as per any row of Table U below, which compounds are named I.A2-Z70-Q1-U-1 to I.A2-Z70-Q1-U-42.

In another embodiment, compounds I are of formula I.A2 wherein Z is Z-70, R³ is CH₃ and Q is Q2, and R and R⁴ are as per any row of Table U below, which compounds are named I.A2-Z70-Q2-U-1 to I.A2-Z70-Q2-U-42.

In another embodiment, compounds I are of formula I.A2 wherein Z is Z-70, R³ is CH₃ and Q is Q3, and R and R⁴ are as per any row of Table U below, which compounds are named I.A2-Z70-Q3-U-1 to I.A2-Z70-Q3-U-42.

In another embodiment, compounds I are of formula I.A2 wherein Z is Z-70, R³ is CH₃ and Q is Q4, and R and R⁴ are as per any row of Table U below, which compounds are named I.A2-Z70-Q4-U-1 to I.A2-Z70-Q4-U-42.

In another embodiment, compounds I are of formula I.A1 wherein Z is Z-71, R³ is CH₃ and Q is Q1, and R and R⁴ are as per any row of Table U below, which compounds are named I.A1-Z71-Q1-U-1 to I.A1-Z71-Q1-U-42.

In another embodiment, compounds I are of formula I.A1 wherein Z is Z-71, R³ is CH₃ and Q is Q2, and R and R⁴ are as per any row of Table U below, which compounds are named I.A1-Z71-Q2-U-1 to I.A1-Z71-Q2-U-42.

In another embodiment, compounds I are of formula I.A1 wherein Z is Z-71, R³ is CH₃ and Q is Q3, and R and R⁴ are as per any row of Table U below, which compounds are named I.A1-Z71-Q3-U-1 to I.A1-Z71-Q3-U-42.

In another embodiment, compounds I are of formula I.A1 wherein Z is Z-71, R³ is CH₃ and Q is Q4, and R and R⁴ are as per any row of Table U below, which compounds are named I.A1-Z71-Q4-U-1 to I.A1-Z71-Q4-U-42.

In another embodiment, compounds I are of formula I.A2 wherein Z is Z-71, R³ is CH₃ and Q is Q1, and R and R⁴ are as per any row of Table U below, which compounds are named I.A2-Z71-Q1-U-1 to I.A2-Z71-Q1-U-42.

In another embodiment, compounds I are of formula I.A2 wherein Z is Z-71, R³ is CH₃ and Q is Q2, and R and R⁴ are as per any row of Table U below, which compounds are named I.A2-Z71-Q2-U-1 to I.A2-Z71-Q2-U-42.

In another embodiment, compounds I are of formula I.A2 wherein Z is Z-71, R³ is CH₃ and Q is Q3, and R and R⁴ are as per any row of Table U below, which compounds are named I.A2-Z71-Q3-U-1 to I.A2-Z71-Q3-U-42.

In another embodiment, compounds I are of formula I.A2 wherein Z is Z-71, R³ is CH₃ and Q is Q4, and R and R⁴ are as per any row of Table U below, which compounds are named I.A2-Z71-Q4-U-1 to I.A2-Z71-Q4-U-42.

In another embodiment, compounds I are of formula I.A1 wherein Z is Z-72, R³ is CH₃ and Q is Q1, and R and R⁴ are as per any row of Table U below, which compounds are named I.A1-Z72-Q1-U-1 to I.A1-Z72-Q1-U-42.

In another embodiment, compounds I are of formula I.A1 wherein Z is Z-72, R³ is CH₃ and Q is Q2, and R and R⁴ are as per any row of Table U below, which compounds are named I.A1-Z72-Q2-U-1 to I.A1-Z72-Q2-U-42.

In another embodiment, compounds I are of formula I.A1 wherein Z is Z-72, R³ is CH₃ and Q is Q3, and R and R⁴ are as per any row of Table U below, which compounds are named I.A1-Z72-Q3-U-1 to I.A1-Z72-Q3-U-42.

In another embodiment, compounds I are of formula I.A1 wherein Z is Z-72, R³ is CH₃ and Q is Q4, and R and R⁴ are as per any row of Table U below, which compounds are named I.A1-Z72-Q4-U-1 to I.A1-Z72-Q4-U-42.

In another embodiment, compounds I are of formula I.A2 wherein Z is Z-72, R³ is CH₃ and Q is Q1, and R and R⁴ are as per any row of Table U below, which compounds are named I.A2-Z72-Q1-U-1 to I.A2-Z72-Q1-U-42.

In another embodiment, compounds I are of formula I.A2 wherein Z is Z-72, R³ is CH₃ and Q is Q2, and R and R⁴ are as per any row of Table U below, which compounds are named I.A2-Z72-Q2-U-1 to I.A2-Z72-Q2-U-42.

In another embodiment, compounds I are of formula I.A2 wherein Z is Z-72, R³ is CH₃ and Q is Q3, and R and R⁴ are as per any row of Table U below, which compounds are named I.A2-Z72-Q3-U-1 to I.A2-Z72-Q3-U-42.

In another embodiment, compounds I are of formula I.A2 wherein Z is Z-72, R³ is CH₃ and Q is Q4, and R and R⁴ are as per any row of Table U below, which compounds are named I.A2-Z72-Q4-U-1 to I.A2-Z72-Q4-U-42.

In another embodiment, compounds I are of formula I.A1 wherein Z is Z-73, R³ is CH₃ and Q is Q1, and R and R⁴ are as per any row of Table U below, which compounds are named I.A1-Z73-Q1-U-1 to I.A1-Z73-Q1-U-42.

In another embodiment, compounds I are of formula I.A1 wherein Z is Z-73, R³ is CH₃ and Q is Q2, and R and R⁴ are as per any row of Table U below, which compounds are named I.A1-Z73-Q2-U-1 to I.A1-Z73-Q2-U-42.

In another embodiment, compounds I are of formula I.A1 wherein Z is Z-73, R³ is CH₃ and Q is Q3, and R and R⁴ are as per any row of Table U below, which compounds are named I.A1-Z73-Q3-U-1 to I.A1-Z73-Q3-U-42.

In another embodiment, compounds I are of formula I.A1 wherein Z is Z-73, R³ is CH₃ and Q is Q4, and R and R⁴ are as per any row of Table U below, which compounds are named I.A1-Z73-Q4-U-1 to I.A1-Z73-Q4-U-42.

In another embodiment, compounds I are of formula I.A2 wherein Z is Z-73, R³ is CH₃ and Q is Q1, and R and R⁴ are as per any row of Table U below, which compounds are named I.A2-Z73-Q1-U-1 to I.A2-Z73-Q1-U-42.

In another embodiment, compounds I are of formula I.A2 wherein Z is Z-73, R³ is CH₃ and Q is Q2, and R and R⁴ are as per any row of Table U below, which compounds are named I.A2-Z73-Q2-U-1 to I.A2-Z73-Q2-U-42.

In another embodiment, compounds I are of formula I.A2 wherein Z is Z-73, R³ is CH₃ and Q is Q3, and R and R⁴ are as per any row of Table U below, which compounds are named I.A2-Z73-Q3-U-1 to I.A2-Z73-Q3-U-42.

In another embodiment, compounds I are of formula I.A2 wherein Z is Z-73, R³ is CH₃ and Q is Q4, and R and R⁴ are as per any row of Table U below, which compounds are named I.A2-Z73-Q4-U-1 to I.A2-Z73-Q4-U-42.

In another embodiment, compounds I are of formula I.A1 wherein Z is Z-74, R³ is CH₃ and Q is Q1, and R and R⁴ are as per any row of Table U below, which compounds are named I.A1-Z74-Q1-U-1 to I.A1-Z74-Q1-U-42.

In another embodiment, compounds I are of formula I.A1 wherein Z is Z-74, R³ is CH₃ and Q is Q2, and R and R⁴ are as per any row of Table U below, which compounds are named I.A1-Z74-Q2-U-1 to I.A1-Z74-Q2-U-42.

In another embodiment, compounds I are of formula I.A1 wherein Z is Z-74, R³ is CH₃ and Q is Q3, and R and R⁴ are as per any row of Table U below, which compounds are named I.A1-Z74-Q3-U-1 to I.A1-Z74-Q3-U-42.

In another embodiment, compounds I are of formula I.A1 wherein Z is Z-74, R³ is CH₃ and Q is Q4, and R and R⁴ are as per any row of Table U below, which compounds are named I.A1-Z74-Q4-U-1 to I.A1-Z74-Q4-U-42.

In another embodiment, compounds I are of formula I.A2 wherein Z is Z-74, R³ is CH₃ and Q is Q1, and R and R⁴ are as per any row of Table U below, which compounds are named I.A2-Z74-Q1-U-1 to I.A2-Z74-Q1-U-42.

In another embodiment, compounds I are of formula I.A2 wherein Z is Z-74, R³ is CH₃ and Q is Q2, and R and R⁴ are as per any row of Table U below, which compounds are named I.A2-Z74-Q2-U-1 to I.A2-Z74-Q2-U-42.

In another embodiment, compounds I are of formula I.A2 wherein Z is Z-74, R³ is CH₃ and Q is Q3, and R and R⁴ are as per any row of Table U below, which compounds are named I.A2-Z74-Q3-U-1 to I.A2-Z74-Q3-U-42.

In another embodiment, compounds I are of formula I.A2 wherein Z is Z-74, R³ is CH₃ and Q is Q4, and R and R⁴ are as per any row of Table U below, which compounds are named I.A2-Z74-Q4-U-1 to I.A2-Z74-Q4-U-42.

In another embodiment, compounds I are of formula I.A1 wherein Z is Z-75, R³ is CH₃ and Q is Q1, and R and R⁴ are as per any row of Table U below, which compounds are named I.A1-Z75-Q1-U-1 to I.A1-Z75-Q1-U-42.

In another embodiment, compounds I are of formula I.A1 wherein Z is Z-75, R³ is CH₃ and Q is Q2, and R and R⁴ are as per any row of Table U below, which compounds are named I.A1-Z75-Q2-U-1 to I.A1-Z75-Q2-U-42.

In another embodiment, compounds I are of formula I.A1 wherein Z is Z-75, R³ is CH₃ and Q is Q3, and R and R⁴ are as per any row of Table U below, which compounds are named I.A1-Z75-Q3-U-1 to I.A1-Z75-Q3-U-42.

In another embodiment, compounds I are of formula I.A1 wherein Z is Z-75, R³ is CH₃ and Q is Q4, and R and R⁴ are as per any row of Table U below, which compounds are named I.A1-Z75-Q4-U-1 to I.A1-Z75-Q4-U-42.

In another embodiment, compounds I are of formula I.A2 wherein Z is Z-75, R³ is CH₃ and Q is Q1, and R and R⁴ are as per any row of Table U below, which compounds are named I.A2-Z75-Q1-U-1 to I.A2-Z75-Q1-U-42.

In another embodiment, compounds I are of formula I.A2 wherein Z is Z-75, R³ is CH₃ and Q is Q2, and R and R⁴ are as per any row of Table U below, which compounds are named I.A2-Z75-Q2-U-1 to I.A2-Z75-Q2-U-42.

In another embodiment, compounds I are of formula I.A2 wherein Z is Z-75, R³ is CH₃ and Q is Q3, and R and R⁴ are as per any row of Table U below, which compounds are named I.A2-Z75-Q3-U-1 to I.A2-Z75-Q3-U-42.

In another embodiment, compounds I are of formula I.A2 wherein Z is Z-75, R³ is CH₃ and Q is Q4, and R and R⁴ are as per any row of Table U below, which compounds are named I.A2-Z75-Q4-U-1 to I.A2-Z75-Q4-U-42.

In another embodiment, compounds I are of formula I.A1 wherein Z is Z-76, R³ is CH₃ and Q is Q1, and R and R⁴ are as per any row of Table U below, which compounds are named I.A1-Z76-Q1-U-1 to I.A1-Z76-Q1-U-42.

In another embodiment, compounds I are of formula I.A1 wherein Z is Z-76, R³ is CH₃ and Q is Q2, and R and R⁴ are as per any row of Table U below, which compounds are named I.A1-Z76-Q2-U-1 to I.A1-Z76-Q2-U-42.

In another embodiment, compounds I are of formula I.A1 wherein Z is Z-76, R³ is CH₃ and Q is Q3, and R and R⁴ are as per any row of Table U below, which compounds are named I.A1-Z76-Q3-U-1 to I.A1-Z76-Q3-U-42.

In another embodiment, compounds I are of formula I.A1 wherein Z is Z-76, R³ is CH₃ and Q is Q4, and R and R⁴ are as per any row of Table U below, which compounds are named I.A1-Z76-Q4-U-1 to I.A1-Z76-Q4-U-42.

In another embodiment, compounds I are of formula I.A2 wherein Z is Z-76, R³ is CH₃ and Q is Q1, and R and R⁴ are as per any row of Table U below, which compounds are named I.A2-Z76-Q1-U-1 to I.A2-Z76-Q1-U-42.

In another embodiment, compounds I are of formula I.A2 wherein Z is Z-76, R³ is CH₃ and Q is Q2, and R and R⁴ are as per any row of Table U below, which compounds are named I.A2-Z76-Q2-U-1 to I.A2-Z76-Q2-U-42.

In another embodiment, compounds I are of formula I.A2 wherein Z is Z-76, R³ is CH₃ and Q is Q3, and R and R⁴ are as per any row of Table U below, which compounds are named I.A2-Z76-Q3-U-1 to I.A2-Z76-Q3-U-42.

In another embodiment, compounds I are of formula I.A2 wherein Z is Z-76, R³ is CH₃ and Q is Q4, and R and R⁴ are as per any row of Table U below, which compounds are named I.A2-Z76-Q4-U-1 to I.A2-Z76-Q4-U-42.

In another embodiment, compounds I are of formula I.A1 wherein Z is Z-77, R³ is CH₃ and Q is Q1, and R and R⁴ are as per any row of Table U below, which compounds are named I.A1-Z77-Q1-U-1 to I.A1-Z77-Q1-U-42.

In another embodiment, compounds I are of formula I.A1 wherein Z is Z-77, R³ is CH₃ and Q is Q2, and R and R⁴ are as per any row of Table U below, which compounds are named I.A1-Z77-Q2-U-1 to I.A1-Z77-Q2-U-42.

In another embodiment, compounds I are of formula I.A1 wherein Z is Z-77, R³ is CH₃ and Q is Q3, and R and R⁴ are as per any row of Table U below, which compounds are named I.A1-Z77-Q3-U-1 to I.A1-Z77-Q3-U-42.

In another embodiment, compounds I are of formula I.A1 wherein Z is Z-77, R³ is CH₃ and Q is Q4, and R and R⁴ are as per any row of Table U below, which compounds are named I.A1-Z77-Q4-U-1 to I.A1-Z77-Q4-U-42.

In another embodiment, compounds I are of formula I.A2 wherein Z is Z-77, R³ is CH₃ and Q is Q1, and R and R⁴ are as per any row of Table U below, which compounds are named I.A2-Z77-Q1-U-1 to I.A2-Z77-Q1-U-42.

In another embodiment, compounds I are of formula I.A2 wherein Z is Z-77, R³ is CH₃ and Q is Q2, and R and R⁴ are as per any row of Table U below, which compounds are named I.A2-Z77-Q2-U-1 to I.A2-Z77-Q2-U-42.

In another embodiment, compounds I are of formula I.A2 wherein Z is Z-77, R³ is CH₃ and Q is Q3, and R and R⁴ are as per any row of Table U below, which compounds are named I.A2-Z77-Q3-U-1 to I.A2-Z77-Q3-U-42.

In another embodiment, compounds I are of formula I.A2 wherein Z is Z-77, R³ is CH₃ and Q is Q4, and R and R⁴ are as per any row of Table U below, which compounds are named I.A2-Z77-Q4-U-1 to I.A2-Z77-Q4-U-42.

In another embodiment, compounds I are of formula I.A1 wherein Z is Z-78, R³ is CH₃ and Q is Q1, and R and R⁴ are as per any row of Table U below, which compounds are named I.A1-Z78-Q1-U-1 to I.A1-Z78-Q1-U-42.

In another embodiment, compounds I are of formula I.A1 wherein Z is Z-78, R³ is CH₃ and Q is Q2, and R and R⁴ are as per any row of Table U below, which compounds are named I.A1-Z78-Q2-U-1 to I.A1-Z78-Q2-U-42.

In another embodiment, compounds I are of formula I.A1 wherein Z is Z-78, R³ is CH₃ and Q is Q3, and R and R⁴ are as per any row of Table U below, which compounds are named I.A1-Z78-Q3-U-1 to I.A1-Z78-Q3-U-42.

In another embodiment, compounds I are of formula I.A1 wherein Z is Z-78, R³ is CH₃ and Q is Q4, and R and R⁴ are as per any row of Table U below, which compounds are named I.A1-Z78-Q4-U-1 to I.A1-Z78-Q4-U-42.

In another embodiment, compounds I are of formula I.A2 wherein Z is Z-78, R³ is CH₃ and Q is Q1, and R and R⁴ are as per any row of Table U below, which compounds are named I.A2-Z78-Q1-U-1 to I.A2-Z78-Q1-U-42.

In another embodiment, compounds I are of formula I.A2 wherein Z is Z-78, R³ is CH₃ and Q is Q2, and R and R⁴ are as per any row of Table U below, which compounds are named I.A2-Z78-Q2-U-1 to I.A2-Z78-Q2-U-42.

In another embodiment, compounds I are of formula I.A2 wherein Z is Z-78, R³ is CH₃ and Q is Q3, and R and R⁴ are as per any row of Table U below, which compounds are named I.A2-Z78-Q3-U-1 to I.A2-Z78-Q3-U-42.

In another embodiment, compounds I are of formula I.A2 wherein Z is Z-78, R³ is CH₃ and Q is Q4, and R and R⁴ are as per any row of Table U below, which compounds are named I.A2-Z78-Q4-U-1 to I.A2-Z78-Q4-U-42.

In another embodiment, compounds I are of formula I.A1 wherein Z is Z-79, R³ is CH₃ and Q is Q1, and R and R⁴ are as per any row of Table U below, which compounds are named I.A1-Z79-Q1-U-1 to I.A1-Z79-Q1-U-42.

In another embodiment, compounds I are of formula I.A1 wherein Z is Z-79, R³ is CH₃ and Q is Q2, and R and R⁴ are as per any row of Table U below, which compounds are named I.A1-Z79-Q2-U-1 to I.A1-Z79-Q2-U-42.

In another embodiment, compounds I are of formula I.A1 wherein Z is Z-79, R³ is CH₃ and Q is Q3, and R and R⁴ are as per any row of Table U below, which compounds are named I.A1-Z79-Q3-U-1 to I.A1-Z79-Q3-U-42.

In another embodiment, compounds I are of formula I.A1 wherein Z is Z-79, R³ is CH₃ and Q is Q4, and R and R⁴ are as per any row of Table U below, which compounds are named I.A1-Z79-Q4-U-1 to I.A1-Z79-Q4-U-42.

In another embodiment, compounds I are of formula I.A2 wherein Z is Z-79, R³ is CH₃ and Q is Q1, and R and R⁴ are as per any row of Table U below, which compounds are named I.A2-Z79-Q1-U-1 to I.A2-Z79-Q1-U-42.

In another embodiment, compounds I are of formula I.A2 wherein Z is Z-79, R³ is CH₃ and Q is Q2, and R and R⁴ are as per any row of Table U below, which compounds are named I.A2-Z79-Q2-U-1 to I.A2-Z79-Q2-U-42.

In another embodiment, compounds I are of formula I.A2 wherein Z is Z-79, R³ is CH₃ and Q is Q3, and R and R⁴ are as per any row of Table U below, which compounds are named I.A2-Z79-Q3-U-1 to I.A2-Z79-Q3-U-42.

In another embodiment, compounds I are of formula I.A2 wherein Z is Z-79, R³ is CH₃ and Q is Q4, and R and R⁴ are as per any row of Table U below, which compounds are named I.A2-Z79-Q4-U-1 to I.A2-Z79-Q4-U-42.

In another embodiment, compounds I are of formula I.A1 wherein Z is Z-80, R³ is CH₃ and Q is Q1, and R and R⁴ are as per any row of Table U below, which compounds are named I.A1-Z80-Q1-U-1 to I.A1-Z80-Q1-U-42.

In another embodiment, compounds I are of formula I.A1 wherein Z is Z-80, R³ is CH₃ and Q is Q2, and R and R⁴ are as per any row of Table U below, which compounds are named I.A1-Z80-Q2-U-1 to I.A1-Z80-Q2-U-42.

In another embodiment, compounds I are of formula I.A1 wherein Z is Z-80, R³ is CH₃ and Q is Q3, and R and R⁴ are as per any row of Table U below, which compounds are named I.A1-Z80-Q3-U-1 to I.A1-Z80-Q3-U-42.

In another embodiment, compounds I are of formula I.A1 wherein Z is Z-80, R³ is CH₃ and Q is Q4, and R and R⁴ are as per any row of Table U below, which compounds are named I.A1-Z80-Q4-U-1 to I.A1-Z80-Q4-U-42.

In another embodiment, compounds I are of formula I.A2 wherein Z is Z-80, R³ is CH₃ and Q is Q1, and R and R⁴ are as per any row of Table U below, which compounds are named I.A2-Z80-Q1-U-1 to I.A2-Z80-Q1-U-42.

In another embodiment, compounds I are of formula I.A2 wherein Z is Z-80, R³ is CH₃ and Q is Q2, and R and R⁴ are as per any row of Table U below, which compounds are named I.A2-Z80-Q2-U-1 to I.A2-Z80-Q2-U-42.

In another embodiment, compounds I are of formula I.A2 wherein Z is Z-80, R³ is CH₃ and Q is Q3, and R and R⁴ are as per any row of Table U below, which compounds are named I.A2-Z80-Q3-U-1 to I.A2-Z80-Q3-U-42.

In another embodiment, compounds I are of formula I.A2 wherein Z is Z-80, R³ is CH₃ and Q is Q4, and R and R⁴ are as per any row of Table U below, which compounds are named I.A2-Z80-Q4-U-1 to I.A2-Z80-Q4-U-42.

In another embodiment, compounds I are of formula I.A1 wherein Z is Z-81, R³ is CH₃ and Q is Q1, and R and R⁴ are as per any row of Table U below, which compounds are named I.A1-Z81-Q1-U-1 to I.A1-Z81-Q1-U-42.

In another embodiment, compounds I are of formula I.A1 wherein Z is Z-81, R³ is CH₃ and Q is Q2, and R and R⁴ are as per any row of Table U below, which compounds are named I.A1-Z81-Q2-U-1 to I.A1-Z81-Q2-U-42.

In another embodiment, compounds I are of formula I.A1 wherein Z is Z-81, R³ is CH₃ and Q is Q3, and R and R⁴ are as per any row of Table U below, which compounds are named I.A1-Z81-Q3-U-1 to I.A1-Z81-Q3-U-42.

In another embodiment, compounds I are of formula I.A1 wherein Z is Z-81, R³ is CH₃ and Q is Q4, and R and R⁴ are as per any row of Table U below, which compounds are named I.A1-Z81-Q4-U-1 to I.A1-Z81-Q4-U-42.

In another embodiment, compounds I are of formula I.A2 wherein Z is Z-81, R³ is CH₃ and Q is Q1, and R and R⁴ are as per any row of Table U below, which compounds are named I.A2-Z81-Q1-U-1 to I.A2-Z81-Q1-U-42.

In another embodiment, compounds I are of formula I.A2 wherein Z is Z-81, R³ is CH₃ and Q is Q2, and R and R⁴ are as per any row of Table U below, which compounds are named I.A2-Z81-Q2-U-1 to I.A2-Z81-Q2-U-42.

In another embodiment, compounds I are of formula I.A2 wherein Z is Z-81, R³ is CH₃ and Q is Q3, and R and R⁴ are as per any row of Table U below, which compounds are named I.A2-Z81-Q3-U-1 to I.A2-Z81-Q3-U-42.

In another embodiment, compounds I are of formula I.A2 wherein Z is Z-81, R³ is CH₃ and Q is Q4, and R and R⁴ are as per any row of Table U below, which compounds are named I.A2-Z81-Q4-U-1 to I.A2-Z81-Q4-U-42.

In another embodiment, compounds I are of formula I.A1 wherein Z is Z-82, R³ is CH₃ and Q is Q1, and R and R⁴ are as per any row of Table U below, which compounds are named I.A1-Z82-Q1-U-1 to I.A1-Z82-Q1-U-42.

In another embodiment, compounds I are of formula I.A1 wherein Z is Z-82, R³ is CH₃ and Q is Q2, and R and R⁴ are as per any row of Table U below, which compounds are named I.A1-Z82-Q2-U-1 to I.A1-Z82-Q2-U-42.

In another embodiment, compounds I are of formula I.A1 wherein Z is Z-82, R³ is CH₃ and Q is Q3, and R and R⁴ are as per any row of Table U below, which compounds are named I.A1-Z82-Q3-U-1 to I.A1-Z82-Q3-U-42.

In another embodiment, compounds I are of formula I.A1 wherein Z is Z-82, R³ is CH₃ and Q is Q4, and R and R⁴ are as per any row of Table U below, which compounds are named I.A1-Z82-Q4-U-1 to I.A1-Z82-Q4-U-42.

In another embodiment, compounds I are of formula I.A2 wherein Z is Z-82, R³ is CH₃ and Q is Q1, and R and R⁴ are as per any row of Table U below, which compounds are named I.A2-Z82-Q1-U-1 to I.A2-Z82-Q1-U-42.

In another embodiment, compounds I are of formula I.A2 wherein Z is Z-82, R³ is CH₃ and Q is Q2, and R and R⁴ are as per any row of Table U below, which compounds are named I.A2-Z82-Q2-U-1 to I.A2-Z82-Q2-U-42.

In another embodiment, compounds I are of formula I.A2 wherein Z is Z-82, R³ is CH₃ and Q is Q3, and R and R⁴ are as per any row of Table U below, which compounds are named I.A2-Z82-Q3-U-1 to I.A2-Z82-Q3-U-42.

In another embodiment, compounds I are of formula I.A2 wherein Z is Z-82, R³ is CH₃ and Q is Q4, and R and R⁴ are as per any row of Table U below, which compounds are named I.A2-Z82-Q4-U-1 to I.A2-Z82-Q4-U-42.

In another embodiment, compounds I are of formula I.A1 wherein Z is Z-83, R³ is CH₃ and Q is Q1, and R and R⁴ are as per any row of Table U below, which compounds are named I.A1-Z83-Q1-U-1 to I.A1-Z83-Q1-U-42.

In another embodiment, compounds I are of formula I.A1 wherein Z is Z-83, R³ is CH₃ and Q is Q2, and R and R⁴ are as per any row of Table U below, which compounds are named I.A1-Z83-Q2-U-1 to I.A1-Z83-Q2-U-42.

In another embodiment, compounds I are of formula I.A1 wherein Z is Z-83, R³ is CH₃ and Q is Q3, and R and R⁴ are as per any row of Table U below, which compounds are named I.A1-Z83-Q3-U-1 to I.A1-Z83-Q3-U-42.

In another embodiment, compounds I are of formula I.A1 wherein Z is Z-83, R³ is CH₃ and Q is Q4, and R and R⁴ are as per any row of Table U below, which compounds are named I.A1-Z83-Q4-U-1 to I.A1-Z83-Q4-U-42.

In another embodiment, compounds I are of formula I.A2 wherein Z is Z-83, R³ is CH₃ and Q is Q1, and R and R⁴ are as per any row of Table U below, which compounds are named I.A2-Z83-Q1-U-1 to I.A2-Z83-Q1-U-42.

In another embodiment, compounds I are of formula I.A2 wherein Z is Z-83, R³ is CH₃ and Q is Q2, and R and R⁴ are as per any row of Table U below, which compounds are named I.A2-Z83-Q2-U-1 to I.A2-Z83-Q2-U-42.

In another embodiment, compounds I are of formula I.A2 wherein Z is Z-83, R³ is CH₃ and Q is Q3, and R and R⁴ are as per any row of Table U below, which compounds are named I.A2-Z83-Q3-U-1 to I.A2-Z83-Q3-U-42.

In another embodiment, compounds I are of formula I.A2 wherein Z is Z-83, R³ is CH₃ and Q is Q4, and R and R⁴ are as per any row of Table U below, which compounds are named I.A2-Z83-Q4-U-1 to I.A2-Z83-Q4-U-42.

In another embodiment, compounds I are of formula I.A1 wherein Z is Z-84, R³ is CH₃ and Q is Q1, and R and R⁴ are as per any row of Table U below, which compounds are named I.A1-Z84-Q1-U-1 to I.A1-Z84-Q1-U-42.

In another embodiment, compounds I are of formula I.A1 wherein Z is Z-84, R³ is CH₃ and Q is Q2, and R and R⁴ are as per any row of Table U below, which compounds are named I.A1-Z84-Q2-U-1 to I.A1-Z84-Q2-U-42.

In another embodiment, compounds I are of formula I.A1 wherein Z is Z-84, R³ is CH₃ and Q is Q3, and R and R⁴ are as per any row of Table U below, which compounds are named I.A1-Z84-Q3-U-1 to I.A1-Z84-Q3-U-42.

In another embodiment, compounds I are of formula I.A1 wherein Z is Z-84, R³ is CH₃ and Q is Q4, and R and R⁴ are as per any row of Table U below, which compounds are named I.A1-Z84-Q4-U-1 to I.A1-Z84-Q4-U-42.

In another embodiment, compounds I are of formula I.A2 wherein Z is Z-84, R³ is CH₃ and Q is Q1, and R and R⁴ are as per any row of Table U below, which compounds are named I.A2-Z84-Q1-U-1 to I.A2-Z84-Q1-U-42.

In another embodiment, compounds I are of formula I.A2 wherein Z is Z-84, R³ is CH₃ and Q is Q2, and R and R⁴ are as per any row of Table U below, which compounds are named I.A2-Z84-Q2-U-1 to I.A2-Z84-Q2-U-42.

In another embodiment, compounds I are of formula I.A2 wherein Z is Z-84, R³ is CH₃ and Q is Q3, and R and R⁴ are as per any row of Table U below, which compounds are named I.A2-Z84-Q3-U-1 to I.A2-Z84-Q3-U-42.

In another embodiment, compounds I are of formula I.A2 wherein Z is Z-84, R³ is CH₃ and Q is Q4, and R and R⁴ are as per any row of Table U below, which compounds are named I.A2-Z84-Q4-U-1 to I.A2-Z84-Q4-U-42.

In another embodiment, compounds I are of formula I.A1 wherein Z is Z-85, R³ is CH₃ and Q is Q1, and R and R⁴ are as per any row of Table U below, which compounds are named I.A1-Z85-Q1-U-1 to I.A1-Z85-Q1-U-42.

In another embodiment, compounds I are of formula I.A1 wherein Z is Z-85, R³ is CH₃ and Q is Q2, and R and R⁴ are as per any row of Table U below, which compounds are named I.A1-Z85-Q2-U-1 to I.A1-Z85-Q2-U-42.

In another embodiment, compounds I are of formula I.A1 wherein Z is Z-85, R³ is CH₃ and Q is Q3, and R and R⁴ are as per any row of Table U below, which compounds are named I.A1-Z85-Q3-U-1 to I.A1-Z85-Q3-U-42.

In another embodiment, compounds I are of formula I.A1 wherein Z is Z-85, R³ is CH₃ and Q is Q4, and R and R⁴ are as per any row of Table U below, which compounds are named I.A1-Z85-Q4-U-1 to I.A1-Z85-Q4-U-42.

In another embodiment, compounds I are of formula I.A2 wherein Z is Z-85, R³ is CH₃ and Q is Q1, and R and R⁴ are as per any row of Table U below, which compounds are named I.A2-Z85-Q1-U-1 to I.A2-Z85-Q1-U-42.

In another embodiment, compounds I are of formula I.A2 wherein Z is Z-85, R³ is CH₃ and Q is Q2, and R and R⁴ are as per any row of Table U below, which compounds are named I.A2-Z85-Q2-U-1 to I.A2-Z85-Q2-U-42.

In another embodiment, compounds I are of formula I.A2 wherein Z is Z-85, R³ is CH₃ and Q is Q3, and R and R⁴ are as per any row of Table U below, which compounds are named I.A2-Z85-Q3-U-1 to I.A2-Z85-Q3-U-42.

In another embodiment, compounds I are of formula I.A2 wherein Z is Z-85, R³ is CH₃ and Q is Q4, and R and R⁴ are as per any row of Table U below, which compounds are named I.A2-Z85-Q4-U-1 to I.A2-Z85-Q4-U-42.

In another embodiment, compounds I are of formula I.A1 wherein Z is Z-86, R³ is CH₃ and Q is Q1, and R and R⁴ are as per any row of Table U below, which compounds are named I.A1-Z86-Q1-U-1 to I.A1-Z86-Q1-U-42.

In another embodiment, compounds I are of formula I.A1 wherein Z is Z-86, R³ is CH₃ and Q is Q2, and R and R⁴ are as per any row of Table U below, which compounds are named I.A1-Z86-Q2-U-1 to I.A1-Z86-Q2-U-42.

In another embodiment, compounds I are of formula I.A1 wherein Z is Z-86, R³ is CH₃ and Q is Q3, and R and R⁴ are as per any row of Table U below, which compounds are named I.A1-Z86-Q3-U-1 to I.A1-Z86-Q3-U-42.

In another embodiment, compounds I are of formula I.A1 wherein Z is Z-86, R³ is CH₃ and Q is Q4, and R and R⁴ are as per any row of Table U below, which compounds are named I.A1-Z86-Q4-U-1 to I.A1-Z86-Q4-U-42.

In another embodiment, compounds I are of formula I.A2 wherein Z is Z-86, R³ is CH₃ and Q is Q1, and R and R⁴ are as per any row of Table U below, which compounds are named I.A2-Z86-Q1-U-1 to I.A2-Z86-Q1-U-42.

In another embodiment, compounds I are of formula I.A2 wherein Z is Z-86, R³ is CH₃ and Q is Q2, and R and R⁴ are as per any row of Table U below, which compounds are named I.A2-Z86-Q2-U-1 to I.A2-Z86-Q2-U-42.

In another embodiment, compounds I are of formula I.A2 wherein Z is Z-86, R³ is CH₃ and Q is Q3, and R and R⁴ are as per any row of Table U below, which compounds are named I.A2-Z86-Q3-U-1 to I.A2-Z86-Q3-U-42.

In another embodiment, compounds I are of formula I.A2 wherein Z is Z-86, R³ is CH₃ and Q is Q4, and R and R⁴ are as per any row of Table U below, which compounds are named I.A2-Z86-Q4-U-1 to I.A2-Z86-Q4-U-42.

In another embodiment, compounds I are of formula I.A1 wherein Z is Z-87, R³ is CH₃ and Q is Q1, and R and R⁴ are as per any row of Table U below, which compounds are named I.A1-Z87-Q1-U-1 to I.A1-Z87-Q1-U-42.

In another embodiment, compounds I are of formula I.A1 wherein Z is Z-87, R³ is CH₃ and Q is Q2, and R and R⁴ are as per any row of Table U below, which compounds are named I.A1-Z87-Q2-U-1 to I.A1-Z87-Q2-U-42.

In another embodiment, compounds I are of formula I.A1 wherein Z is Z-87, R³ is CH₃ and Q is Q3, and R and R⁴ are as per any row of Table U below, which compounds are named I.A1-Z87-Q3-U-1 to I.A1-Z87-Q3-U-42.

In another embodiment, compounds I are of formula I.A1 wherein Z is Z-87, R³ is CH₃ and Q is Q4, and R and R⁴ are as per any row of Table U below, which compounds are named I.A1-Z87-Q4-U-1 to I.A1-Z87-Q4-U-42.

In another embodiment, compounds I are of formula I.A2 wherein Z is Z-87, R³ is CH₃ and Q is Q1, and R and R⁴ are as per any row of Table U below, which compounds are named I.A2-Z87-Q1-U-1 to I.A2-Z87-Q1-U-42.

In another embodiment, compounds I are of formula I.A2 wherein Z is Z-87, R³ is CH₃ and Q is Q2, and R and R⁴ are as per any row of Table U below, which compounds are named I.A2-Z87-Q2-U-1 to I.A2-Z87-Q2-U-42.

In another embodiment, compounds I are of formula I.A2 wherein Z is Z-87, R³ is CH₃ and Q is Q3, and R and R⁴ are as per any row of Table U below, which compounds are named I.A2-Z87-Q3-U-1 to I.A2-Z87-Q3-U-42.

In another embodiment, compounds I are of formula I.A2 wherein Z is Z-87, R³ is CH₃ and Q is Q4, and R and R⁴ are as per any row of Table U below, which compounds are named I.A2-Z87-Q4-U-1 to I.A2-Z87-Q4-U-42.

In another embodiment, compounds I are of formula I.A1 wherein Z is Z-88, R³ is CH₃ and Q is Q1, and R and R⁴ are as per any row of Table U below, which compounds are named I.A1-Z88-Q1-U-1 to I.A1-Z88-Q1-U-42.

In another embodiment, compounds I are of formula I.A1 wherein Z is Z-88, R³ is CH₃ and Q is Q2, and R and R⁴ are as per any row of Table U below, which compounds are named I.A1-Z88-Q2-U-1 to I.A1-Z88-Q2-U-42.

In another embodiment, compounds I are of formula I.A1 wherein Z is Z-88, R³ is CH₃ and Q is Q3, and R and R⁴ are as per any row of Table U below, which compounds are named I.A1-Z88-Q3-U-1 to I.A1-Z88-Q3-U-42.

In another embodiment, compounds I are of formula I.A1 wherein Z is Z-88, R³ is CH₃ and Q is Q4, and R and R⁴ are as per any row of Table U below, which compounds are named I.A1-Z88-Q4-U-1 to I.A1-Z88-Q4-U-42.

In another embodiment, compounds I are of formula I.A2 wherein Z is Z-88, R³ is CH₃ and Q is Q1, and R and R⁴ are as per any row of Table U below, which compounds are named I.A2-Z88-Q1-U-1 to I.A2-Z88-Q1-U-42.

In another embodiment, compounds I are of formula I.A2 wherein Z is Z-88, R³ is CH₃ and Q is Q2, and R and R⁴ are as per any row of Table U below, which compounds are named I.A2-Z88-Q2-U-1 to I.A2-Z88-Q2-U-42.

In another embodiment, compounds I are of formula I.A2 wherein Z is Z-88, R³ is CH₃ and Q is Q3, and R and R⁴ are as per any row of Table U below, which compounds are named I.A2-Z88-Q3-U-1 to I.A2-Z88-Q3-U-42.

In another embodiment, compounds I are of formula I.A2 wherein Z is Z-88, R³ is CH₃ and Q is Q4, and R and R⁴ are as per any row of Table U below, which compounds are named I.A2-Z88-Q4-U-1 to I.A2-Z88-Q4-U-42.

In another embodiment, compounds I are of formula I.A1 wherein Z is Z-89, R³ is CH₃ and Q is Q1, and R and R⁴ are as per any row of Table U below, which compounds are named I.A1-Z89-Q1-U-1 to I.A1-Z89-Q1-U-42.

In another embodiment, compounds I are of formula I.A1 wherein Z is Z-89, R³ is CH₃ and Q is Q2, and R and R⁴ are as per any row of Table U below, which compounds are named I.A1-Z89-Q2-U-1 to I.A1-Z89-Q2-U-42.

In another embodiment, compounds I are of formula I.A1 wherein Z is Z-89, R³ is CH₃ and Q is Q3, and R and R⁴ are as per any row of Table U below, which compounds are named I.A1-Z89-Q3-U-1 to I.A1-Z89-Q3-U-42.

In another embodiment, compounds I are of formula I.A1 wherein Z is Z-89, R³ is CH₃ and Q is Q4, and R and R⁴ are as per any row of Table U below, which compounds are named I.A1-Z89-Q4-U-1 to I.A1-Z89-Q4-U-42.

In another embodiment, compounds I are of formula I.A2 wherein Z is Z-89, R³ is CH₃ and Q is Q1, and R and R⁴ are as per any row of Table U below, which compounds are named I.A2-Z89-Q1-U-1 to I.A2-Z89-Q1-U-42.

In another embodiment, compounds I are of formula I.A2 wherein Z is Z-89, R³ is CH₃ and Q is Q2, and R and R⁴ are as per any row of Table U below, which compounds are named I.A2-Z89-Q2-U-1 to I.A2-Z89-Q2-U-42.

In another embodiment, compounds I are of formula I.A2 wherein Z is Z-89, R³ is CH₃ and Q is Q3, and R and R⁴ are as per any row of Table U below, which compounds are named I.A2-Z89-Q3-U-1 to I.A2-Z89-Q3-U-42.

In another embodiment, compounds I are of formula I.A2 wherein Z is Z-89, R³ is CH₃ and Q is Q4, and R and R⁴ are as per any row of Table U below, which compounds are named I.A2-Z89-Q4-U-1 to I.A2-Z89-Q4-U-42.

In another embodiment, compounds I are of formula I.A1 wherein Z is Z-90, R³ is CH₃ and Q is Q1, and R and R⁴ are as per any row of Table U below, which compounds are named I.A1-Z90-Q1-U-1 to I.A1-Z90-Q1-U-42.

In another embodiment, compounds I are of formula I.A1 wherein Z is Z-90, R³ is CH₃ and Q is Q2, and R and R⁴ are as per any row of Table U below, which compounds are named I.A1-Z90-Q2-U-1 to I.A1-Z90-Q2-U-42.

In another embodiment, compounds I are of formula I.A1 wherein Z is Z-90, R³ is CH₃ and Q is Q3, and R and R⁴ are as per any row of Table U below, which compounds are named I.A1-Z90-Q3-U-1 to I.A1-Z90-Q3-U-42.

In another embodiment, compounds I are of formula I.A1 wherein Z is Z-90, R³ is CH₃ and Q is Q4, and R and R⁴ are as per any row of Table U below, which compounds are named I.A1-Z90-Q4-U-1 to I.A1-Z90-Q4-U-42.

In another embodiment, compounds I are of formula I.A2 wherein Z is Z-90, R³ is CH₃ and Q is Q1, and R and R⁴ are as per any row of Table U below, which compounds are named I.A2-Z90-Q1-U-1 to I.A2-Z90-Q1-U-42.

In another embodiment, compounds I are of formula I.A2 wherein Z is Z-90, R³ is CH₃ and Q is Q2, and R and R⁴ are as per any row of Table U below, which compounds are named I.A2-Z90-Q2-U-1 to I.A2-Z90-Q2-U-42.

In another embodiment, compounds I are of formula I.A2 wherein Z is Z-90, R³ is CH₃ and Q is Q3, and R and R⁴ are as per any row of Table U below, which compounds are named I.A2-Z90-Q3-U-1 to I.A2-Z90-Q3-U-42.

In another embodiment, compounds I are of formula I.A2 wherein Z is Z-90, R³ is CH₃ and Q is Q4, and R and R⁴ are as per any row of Table U below, which compounds are named I.A2-Z90-Q4-U-1 to I.A2-Z90-Q4-U-42.

In another embodiment, compounds I are of formula I.A1 wherein Z is Z-91, R³ is CH₃ and Q is Q1, and R and R⁴ are as per any row of Table U below, which compounds are named I.A1-Z91-Q1-U-1 to I.A1-Z91-Q1-U-42.

In another embodiment, compounds I are of formula I.A1 wherein Z is Z-91, R³ is CH₃ and Q is Q2, and R and R⁴ are as per any row of Table U below, which compounds are named I.A1-Z91-Q2-U-1 to I.A1-Z91-Q2-U-42.

In another embodiment, compounds I are of formula I.A1 wherein Z is Z-91, R³ is CH₃ and Q is Q3, and R and R⁴ are as per any row of Table U below, which compounds are named I.A1-Z91-Q3-U-1 to I.A1-Z91-Q3-U-42.

In another embodiment, compounds I are of formula I.A1 wherein Z is Z-91, R³ is CH₃ and Q is Q4, and R and R⁴ are as per any row of Table U below, which compounds are named I.A1-Z91-Q4-U-1 to I.A1-Z91-Q4-U-42.

In another embodiment, compounds I are of formula I.A2 wherein Z is Z-91, R³ is CH₃ and Q is Q1, and R and R⁴ are as per any row of Table U below, which compounds are named I.A2-Z91-Q1-U-1 to I.A2-Z91-Q1-U-42.

In another embodiment, compounds I are of formula I.A2 wherein Z is Z-91, R³ is CH₃ and Q is Q2, and R and R⁴ are as per any row of Table U below, which compounds are named I.A2-Z91-Q2-U-1 to I.A2-Z91-Q2-U-42.

In another embodiment, compounds I are of formula I.A2 wherein Z is Z-91, R³ is CH₃ and Q is Q3, and R and R⁴ are as per any row of Table U below, which compounds are named I.A2-Z91-Q3-U-1 to I.A2-Z91-Q3-U-42.

In another embodiment, compounds I are of formula I.A2 wherein Z is Z-91, R³ is CH₃ and Q is Q4, and R and R⁴ are as per any row of Table U below, which compounds are named I.A2-Z91-Q4-U-1 to I.A2-Z91-Q4-U-42.

In another embodiment, compounds I are of formula I.A1 wherein Z is Z-92, R³ is CH₃ and Q is Q1, and R and R⁴ are as per any row of Table U below, which compounds are named I.A1-Z92-Q1-U-1 to I.A1-Z92-Q1-U-42.

In another embodiment, compounds I are of formula I.A1 wherein Z is Z-92, R³ is CH₃ and Q is Q2, and R and R⁴ are as per any row of Table U below, which compounds are named I.A1-Z92-Q2-U-1 to I.A1-Z92-Q2-U-42.

In another embodiment, compounds I are of formula I.A1 wherein Z is Z-92, R³ is CH₃ and Q is Q3, and R and R⁴ are as per any row of Table U below, which compounds are named I.A1-Z92-Q3-U-1 to I.A1-Z92-Q3-U-42.

In another embodiment, compounds I are of formula I.A1 wherein Z is Z-92, R³ is CH₃ and Q is Q4, and R and R⁴ are as per any row of Table U below, which compounds are named I.A1-Z92-Q4-U-1 to I.A1-Z92-Q4-U-42.

In another embodiment, compounds I are of formula I.A2 wherein Z is Z-92, R³ is CH₃ and Q is Q1, and R and R⁴ are as per any row of Table U below, which compounds are named I.A2-Z92-Q1-U-1 to I.A2-Z92-Q1-U-42.

In another embodiment, compounds I are of formula I.A2 wherein Z is Z-92, R³ is CH₃ and Q is Q2, and R and R⁴ are as per any row of Table U below, which compounds are named I.A2-Z92-Q2-U-1 to I.A2-Z92-Q2-U-42.

In another embodiment, compounds I are of formula I.A2 wherein Z is Z-92, R³ is CH₃ and Q is Q3, and R and R⁴ are as per any row of Table U below, which compounds are named I.A2-Z92-Q3-U-1 to I.A2-Z92-Q3-U-42.

In another embodiment, compounds I are of formula I.A2 wherein Z is Z-92, R³ is CH₃ and Q is Q4, and R and R⁴ are as per any row of Table U below, which compounds are named I.A2-Z92-Q4-U-1 to I.A2-Z92-Q4-U-42.

In another embodiment, compounds I are of formula I.A1 wherein Z is Z-93, R³ is CH₃ and Q is Q1, and R and R⁴ are as per any row of Table U below, which compounds are named I.A1-Z93-Q1-U-1 to I.A1-Z93-Q1-U-42.

In another embodiment, compounds I are of formula I.A1 wherein Z is Z-93, R³ is CH₃ and Q is Q2, and R and R⁴ are as per any row of Table U below, which compounds are named I.A1-Z93-Q2-U-1 to I.A1-Z93-Q2-U-42.

In another embodiment, compounds I are of formula I.A1 wherein Z is Z-93, R³ is CH₃ and Q is Q3, and R and R⁴ are as per any row of Table U below, which compounds are named I.A1-Z93-Q3-U-1 to I.A1-Z93-Q3-U-42.

In another embodiment, compounds I are of formula I.A1 wherein Z is Z-93, R³ is CH₃ and Q is Q4, and R and R⁴ are as per any row of Table U below, which compounds are named I.A1-Z93-Q4-U-1 to I.A1-Z93-Q4-U-42.

In another embodiment, compounds I are of formula I.A2 wherein Z is Z-93, R³ is CH₃ and Q is Q1, and R and R⁴ are as per any row of Table U below, which compounds are named I.A2-Z93-Q1-U-1 to I.A2-Z93-Q1-U-42.

In another embodiment, compounds I are of formula I.A2 wherein Z is Z-93, R³ is CH₃ and Q is Q2, and R and R⁴ are as per any row of Table U below, which compounds are named I.A2-Z93-Q2-U-1 to I.A2-Z93-Q2-U-42.

In another embodiment, compounds I are of formula I.A2 wherein Z is Z-93, R³ is CH₃ and Q is Q3, and R and R⁴ are as per any row of Table U below, which compounds are named I.A2-Z93-Q3-U-1 to I.A2-Z93-Q3-U-42.

In another embodiment, compounds I are of formula I.A2 wherein Z is Z-93, R³ is CH₃ and Q is Q4, and R and R⁴ are as per any row of Table U below, which compounds are named I.A2-Z93-Q4-U-1 to I.A2-Z93-Q4-U-42.

In another embodiment, compounds I are of formula I.A1 wherein Z is Z-94, R³ is CH₃ and Q is Q1, and R and R⁴ are as per any row of Table U below, which compounds are named I.A1-Z94-Q1-U-1 to I.A1-Z94-Q1-U-42.

In another embodiment, compounds I are of formula I.A1 wherein Z is Z-94, R³ is CH₃ and Q is Q2, and R and R⁴ are as per any row of Table U below, which compounds are named I.A1-Z94-Q2-U-1 to I.A1-Z94-Q2-U-42.

In another embodiment, compounds I are of formula I.A1 wherein Z is Z-94, R³ is CH₃ and Q is Q3, and R and R⁴ are as per any row of Table U below, which compounds are named I.A1-Z94-Q3-U-1 to I.A1-Z94-Q3-U-42.

In another embodiment, compounds I are of formula I.A1 wherein Z is Z-94, R³ is CH₃ and Q is Q4, and R and R⁴ are as per any row of Table U below, which compounds are named I.A1-Z94-Q4-U-1 to I.A1-Z94-Q4-U-42.

In another embodiment, compounds I are of formula I.A2 wherein Z is Z-94, R³ is CH₃ and Q is Q1, and R and R⁴ are as per any row of Table U below, which compounds are named I.A2-Z94-Q1-U-1 to I.A2-Z94-Q1-U-42.

In another embodiment, compounds I are of formula I.A2 wherein Z is Z-94, R³ is CH₃ and Q is Q2, and R and R⁴ are as per any row of Table U below, which compounds are named I.A2-Z94-Q2-U-1 to I.A2-Z94-Q2-U-42.

In another embodiment, compounds I are of formula I.A2 wherein Z is Z-94, R³ is CH₃ and Q is Q3, and R and R⁴ are as per any row of Table U below, which compounds are named I.A2-Z94-Q3-U-1 to I.A2-Z94-Q3-U-42.

In another embodiment, compounds I are of formula I.A2 wherein Z is Z-94, R³ is CH₃ and Q is Q4, and R and R⁴ are as per any row of Table U below, which compounds are named I.A2-Z94-Q4-U-1 to I.A2-Z94-Q4-U-42.

In another embodiment, compounds I are of formula I.A1 wherein Z is Z-95, R³ is CH₃ and Q is Q1, and R and R⁴ are as per any row of Table U below, which compounds are named I.A1-Z95-Q1-U-1 to I.A1-Z95-Q1-U-42.

In another embodiment, compounds I are of formula I.A1 wherein Z is Z-95, R³ is CH₃ and Q is Q2, and R and R⁴ are as per any row of Table U below, which compounds are named I.A1-Z95-Q2-U-1 to I.A1-Z95-Q2-U-42.

In another embodiment, compounds I are of formula I.A1 wherein Z is Z-95, R³ is CH₃ and Q is Q3, and R and R⁴ are as per any row of Table U below, which compounds are named I.A1-Z95-Q3-U-1 to I.A1-Z95-Q3-U-42.

In another embodiment, compounds I are of formula I.A1 wherein Z is Z-95, R³ is CH₃ and Q is Q4, and R and R⁴ are as per any row of Table U below, which compounds are named I.A1-Z95-Q4-U-1 to I.A1-Z95-Q4-U-42.

In another embodiment, compounds I are of formula I.A2 wherein Z is Z-95, R³ is CH₃ and Q is Q1, and R and R⁴ are as per any row of Table U below, which compounds are named I.A2-Z95-Q1-U-1 to I.A2-Z95-Q1-U-42.

In another embodiment, compounds I are of formula I.A2 wherein Z is Z-95, R³ is CH₃ and Q is Q2, and R and R⁴ are as per any row of Table U below, which compounds are named I.A2-Z95-Q2-U-1 to I.A2-Z95-Q2-U-42.

In another embodiment, compounds I are of formula I.A2 wherein Z is Z-95, R³ is CH₃ and Q is Q3, and R and R⁴ are as per any row of Table U below, which compounds are named I.A2-Z95-Q3-U-1 to I.A2-Z95-Q3-U-42.

In another embodiment, compounds I are of formula I.A2 wherein Z is Z-95, R³ is CH₃ and Q is Q4, and R and R⁴ are as per any row of Table U below, which compounds are named I.A2-Z95-Q4-U-1 to I.A2-Z95-Q4-U-42.

In another embodiment, compounds I are of formula I.A1 wherein Z is Z-96, R³ is CH₃ and Q is Q1, and R and R⁴ are as per any row of Table U below, which compounds are named I.A1-Z96-Q1-U-1 to I.A1-Z96-Q1-U-42.

In another embodiment, compounds I are of formula I.A1 wherein Z is Z-96, R³ is CH₃ and Q is Q2, and R and R⁴ are as per any row of Table U below, which compounds are named I.A1-Z96-Q2-U-1 to I.A1-Z96-Q2-U-42.

In another embodiment, compounds I are of formula I.A1 wherein Z is Z-96, R³ is CH₃ and Q is Q3, and R and R⁴ are as per any row of Table U below, which compounds are named I.A1-Z96-Q3-U-1 to I.A1-Z96-Q3-U-42.

In another embodiment, compounds I are of formula I.A1 wherein Z is Z-96, R³ is CH₃ and Q is Q4, and R and R⁴ are as per any row of Table U below, which compounds are named I.A1-Z96-Q4-U-1 to I.A1-Z96-Q4-U-42.

In another embodiment, compounds I are of formula I.A2 wherein Z is Z-96, R³ is CH₃ and Q is Q1, and R and R⁴ are as per any row of Table U below, which compounds are named I.A2-Z96-Q1-U-1 to I.A2-Z96-Q1-U-42.

In another embodiment, compounds I are of formula I.A2 wherein Z is Z-96, R³ is CH₃ and Q is Q2, and R and R⁴ are as per any row of Table U below, which compounds are named I.A2-Z96-Q2-U-1 to I.A2-Z96-Q2-U-42.

In another embodiment, compounds I are of formula I.A2 wherein Z is Z-96, R³ is CH₃ and Q is Q3, and R and R⁴ are as per any row of Table U below, which compounds are named I.A2-Z96-Q3-U-1 to I.A2-Z96-Q3-U-42.

In another embodiment, compounds I are of formula I.A2 wherein Z is Z-96, R³ is CH₃ and Q is Q4, and R and R⁴ are as per any row of Table U below, which compounds are named I.A2-Z96-Q4-U-1 to I.A2-Z96-Q4-U-42.

In another embodiment, compounds I are of formula I.A1 wherein Z is Z-97, R³ is CH₃ and Q is Q1, and R and R⁴ are as per any row of Table U below, which compounds are named I.A1-Z97-Q1-U-1 to I.A1-Z97-Q1-U-42.

In another embodiment, compounds I are of formula I.A1 wherein Z is Z-97, R³ is CH₃ and Q is Q2, and R and R⁴ are as per any row of Table U below, which compounds are named I.A1-Z97-Q2-U-1 to I.A1-Z97-Q2-U-42.

In another embodiment, compounds I are of formula I.A1 wherein Z is Z-97, R³ is CH₃ and Q is Q3, and R and R⁴ are as per any row of Table U below, which compounds are named I.A1-Z97-Q3-U-1 to I.A1-Z97-Q3-U-42.

In another embodiment, compounds I are of formula I.A1 wherein Z is Z-97, R³ is CH₃ and Q is Q4, and R and R⁴ are as per any row of Table U below, which compounds are named I.A1-Z97-Q4-U-1 to I.A1-Z97-Q4-U-42.

In another embodiment, compounds I are of formula I.A2 wherein Z is Z-97, R³ is CH₃ and Q is Q1, and R and R⁴ are as per any row of Table U below, which compounds are named I.A2-Z97-Q1-U-1 to I.A2-Z97-Q1-U-42.

In another embodiment, compounds I are of formula I.A2 wherein Z is Z-97, R³ is CH₃ and Q is Q2, and R and R⁴ are as per any row of Table U below, which compounds are named I.A2-Z97-Q2-U-1 to I.A2-Z97-Q2-U-42.

In another embodiment, compounds I are of formula I.A2 wherein Z is Z-97, R³ is CH₃ and Q is Q3, and R and R⁴ are as per any row of Table U below, which compounds are named I.A2-Z97-Q3-U-1 to I.A2-Z97-Q3-U-42.

In another embodiment, compounds I are of formula I.A2 wherein Z is Z-97, R³ is CH₃ and Q is Q4, and R and R⁴ are as per any row of Table U below, which compounds are named I.A2-Z97-Q4-U-1 to I.A2-Z97-Q4-U-42.

In another embodiment, compounds I are of formula I.A1 wherein Z is Z-98, R³ is CH₃ and Q is Q1, and R and R⁴ are as per any row of Table U below, which compounds are named I.A1-Z98-Q1-U-1 to I.A1-Z98-Q1-U-42.

In another embodiment, compounds I are of formula I.A1 wherein Z is Z-98, R³ is CH₃ and Q is Q2, and R and R⁴ are as per any row of Table U below, which compounds are named I.A1-Z98-Q2-U-1 to I.A1-Z98-Q2-U-42.

In another embodiment, compounds I are of formula I.A1 wherein Z is Z-98, R³ is CH₃ and Q is Q3, and R and R⁴ are as per any row of Table U below, which compounds are named I.A1-Z98-Q3-U-1 to I.A1-Z98-Q3-U-42.

In another embodiment, compounds I are of formula I.A1 wherein Z is Z-98, R³ is CH₃ and Q is Q4, and R and R⁴ are as per any row of Table U below, which compounds are named I.A1-Z98-Q4-U-1 to I.A1-Z98-Q4-U-42.

In another embodiment, compounds I are of formula I.A2 wherein Z is Z-98, R³ is CH₃ and Q is Q1, and R and R⁴ are as per any row of Table U below, which compounds are named I.A2-Z98-Q1-U-1 to I.A2-Z98-Q1-U-42.

In another embodiment, compounds I are of formula I.A2 wherein Z is Z-98, R³ is CH₃ and Q is Q2, and R and R⁴ are as per any row of Table U below, which compounds are named I.A2-Z98-Q2-U-1 to I.A2-Z98-Q2-U-42.

In another embodiment, compounds I are of formula I.A2 wherein Z is Z-98, R³ is CH₃ and Q is Q3, and R and R⁴ are as per any row of Table U below, which compounds are named I.A2-Z98-Q3-U-1 to I.A2-Z98-Q3-U-42.

In another embodiment, compounds I are of formula I.A2 wherein Z is Z-98, R³ is CH₃ and Q is Q4, and R and R⁴ are as per any row of Table U below, which compounds are named I.A2-Z98-Q4-U-1 to I.A2-Z98-Q4-U-42.

In another embodiment, compounds I are of formula I.A1 wherein Z is Z-99, R³ is CH₃ and Q is Q1, and R and R⁴ are as per any row of Table U below, which compounds are named I.A1-Z99-Q1-U-1 to I.A1-Z99-Q1-U-42.

In another embodiment, compounds I are of formula I.A1 wherein Z is Z-99, R³ is CH₃ and Q is Q2, and R and R⁴ are as per any row of Table U below, which compounds are named I.A1-Z99-Q2-U-1 to I.A1-Z99-Q2-U-42.

In another embodiment, compounds I are of formula I.A1 wherein Z is Z-99, R³ is CH₃ and Q is Q3, and R and R⁴ are as per any row of Table U below, which compounds are named I.A1-Z99-Q3-U-1 to I.A1-Z99-Q3-U-42.

In another embodiment, compounds I are of formula I.A1 wherein Z is Z-99, R³ is CH₃ and Q is Q4, and R and R⁴ are as per any row of Table U below, which compounds are named I.A1-Z99-Q4-U-1 to I.A1-Z99-Q4-U-42.

In another embodiment, compounds I are of formula I.A2 wherein Z is Z-99, R³ is CH₃ and Q is Q1, and R and R⁴ are as per any row of Table U below, which compounds are named I.A2-Z99-Q1-U-1 to I.A2-Z99-Q1-U-42.

In another embodiment, compounds I are of formula I.A2 wherein Z is Z-99, R³ is CH₃ and Q is Q2, and R and R⁴ are as per any row of Table U below, which compounds are named I.A2-Z99-Q2-U-1 to I.A2-Z99-Q2-U-42.

In another embodiment, compounds I are of formula I.A2 wherein Z is Z-99, R³ is CH₃ and Q is Q3, and R and R⁴ are as per any row of Table U below, which compounds are named I.A2-Z99-Q3-U-1 to I.A2-Z99-Q3-U-42.

In another embodiment, compounds I are of formula I.A2 wherein Z is Z-99, R³ is CH₃ and Q is Q4, and R and R⁴ are as per any row of Table U below, which compounds are named I.A2-Z99-Q4-U-1 to I.A2-Z99-Q4-U-42.

In another embodiment, compounds I are of formula I.A1 wherein Z is Z-100, R³ is CH₃ and Q is Q1, and R and R⁴ are as per any row of Table U below, which compounds are named I.A1-Z100-Q1-U-1 to I.A1-Z100-Q1-U-42.

In another embodiment, compounds I are of formula I.A1 wherein Z is Z-100, R³ is CH₃ and Q is Q2, and R and R⁴ are as per any row of Table U below, which compounds are named I.A1-Z100-Q2-U-1 to I.A1-Z100-Q2-U-42.

In another embodiment, compounds I are of formula I.A1 wherein Z is Z-100, R³ is CH₃ and Q is Q3, and R and R⁴ are as per any row of Table U below, which compounds are named I.A1-Z100-Q3-U-1 to I.A1-Z100-Q3-U-42.

In another embodiment, compounds I are of formula I.A1 wherein Z is Z-100, R³ is CH₃ and Q is Q4, and R and R⁴ are as per any row of Table U below, which compounds are named I.A1-Z100-Q4-U-1 to I.A1-Z100-Q4-U-42.

In another embodiment, compounds I are of formula I.A2 wherein Z is Z-100, R³ is CH₃ and Q is Q1, and R and R⁴ are as per any row of Table U below, which compounds are named I.A2-Z100-Q1-U-1 to I.A2-Z100-Q1-U-42.

In another embodiment, compounds I are of formula I.A2 wherein Z is Z-100, R³ is CH₃ and Q is Q2, and R and R⁴ are as per any row of Table U below, which compounds are named I.A2-Z100-Q2-U-1 to I.A2-Z100-Q2-U-42.

In another embodiment, compounds I are of formula I.A2 wherein Z is Z-100, R³ is CH₃ and Q is Q3, and R and R⁴ are as per any row of Table U below, which compounds are named I.A2-Z100-Q3-U-1 to I.A2-Z100-Q3-U-42.

In another embodiment, compounds I are of formula I.A2 wherein Z is Z-100, R³ is CH₃ and Q is Q4, and R and R⁴ are as per any row of Table U below, which compounds are named I.A2-Z100-Q4-U-1 to I.A2-Z100-Q4-U-42.

In another embodiment, compounds I are of formula I.A1 wherein Z is Z-101, R³ is CH₃ and Q is Q1, and R and R⁴ are as per any row of Table U below, which compounds are named I.A1-Z101-Q1-U-1 to I.A1-Z101-Q1-U-42.

In another embodiment, compounds I are of formula I.A1 wherein Z is Z-101, R³ is CH₃ and Q is Q2, and R and R⁴ are as per any row of Table U below, which compounds are named I.A1-Z101-Q2-U-1 to I.A1-Z101-Q2-U-42.

In another embodiment, compounds I are of formula I.A1 wherein Z is Z-101, R³ is CH₃ and Q is Q3, and R and R⁴ are as per any row of Table U below, which compounds are named I.A1-Z101-Q3-U-1 to I.A1-Z101-Q3-U-42.

In another embodiment, compounds I are of formula I.A1 wherein Z is Z-101, R³ is CH₃ and Q is Q4, and R and R⁴ are as per any row of Table U below, which compounds are named I.A1-Z101-Q4-U-1 to I.A1-Z101-Q4-U-42.

In another embodiment, compounds I are of formula I.A2 wherein Z is Z-101, R³ is CH₃ and Q is Q1, and R and R⁴ are as per any row of Table U below, which compounds are named I.A2-Z101-Q1-U-1 to I.A2-Z101-Q1-U-42.

In another embodiment, compounds I are of formula I.A2 wherein Z is Z-101, R³ is CH₃ and Q is Q2, and R and R⁴ are as per any row of Table U below, which compounds are named I.A2-Z101-Q2-U-1 to I.A2-Z101-Q2-U-42.

In another embodiment, compounds I are of formula I.A2 wherein Z is Z-101, R³ is CH₃ and Q is Q3, and R and R⁴ are as per any row of Table U below, which compounds are named I.A2-Z101-Q3-U-1 to I.A2-Z101-Q3-U-42.

In another embodiment, compounds I are of formula I.A2 wherein Z is Z-101, R³ is CH₃ and Q is Q4, and R and R⁴ are as per any row of Table U below, which compounds are named I.A2-Z101-Q4-U-1 to I.A2-Z101-Q4-U-42.

In another embodiment, compounds I are of formula I.A1 wherein Z is Z-102, R³ is CH₃ and Q is Q1, and R and R⁴ are as per any row of Table U below, which compounds are named I.A1-Z102-Q1-U-1 to I.A1-Z102-Q1-U-42.

In another embodiment, compounds I are of formula I.A1 wherein Z is Z-102, R³ is CH₃ and Q is Q2, and R and R⁴ are as per any row of Table U below, which compounds are named I.A1-Z102-Q2-U-1 to I.A1-Z102-Q2-U-42.

In another embodiment, compounds I are of formula I.A1 wherein Z is Z-102, R³ is CH₃ and Q is Q3, and R and R⁴ are as per any row of Table U below, which compounds are named I.A1-Z102-Q3-U-1 to I.A1-Z102-Q3-U-42.

In another embodiment, compounds I are of formula I.A1 wherein Z is Z-102, R³ is CH₃ and Q is Q4, and R and R⁴ are as per any row of Table U below, which compounds are named I.A1-Z102-Q4-U-1 to I.A1-Z102-Q4-U-42.

In another embodiment, compounds I are of formula I.A2 wherein Z is Z-102, R³ is CH₃ and Q is Q1, and R and R⁴ are as per any row of Table U below, which compounds are named I.A2-Z102-Q1-U-1 to I.A2-Z102-Q1-U-42.

In another embodiment, compounds I are of formula I.A2 wherein Z is Z-102, R³ is CH₃ and Q is Q2, and R and R⁴ are as per any row of Table U below, which compounds are named I.A2-Z102-Q2-U-1 to I.A2-Z102-Q2-U-42.

In another embodiment, compounds I are of formula I.A2 wherein Z is Z-102, R³ is CH₃ and Q is Q3, and R and R⁴ are as per any row of Table U below, which compounds are named I.A2-Z102-Q3-U-1 to I.A2-Z102-Q3-U-42.

In another embodiment, compounds I are of formula I.A2 wherein Z is Z-102, R³ is CH₃ and Q is Q4, and R and R⁴ are as per any row of Table U below, which compounds are named I.A2-Z102-Q4-U-1 to I.A2-Z102-Q4-U-42.

In another embodiment, compounds I are of formula I.A1 wherein Z is Z-103, R³ is CH₃ and Q is Q1, and R and R⁴ are as per any row of Table U below, which compounds are named I.A1-Z103-Q1-U-1 to I.A1-Z103-Q1-U-42.

In another embodiment, compounds I are of formula I.A1 wherein Z is Z-103, R³ is CH₃ and Q is Q2, and R and R⁴ are as per any row of Table U below, which compounds are named I.A1-Z103-Q2-U-1 to I.A1-Z103-Q2-U-42.

In another embodiment, compounds I are of formula I.A1 wherein Z is Z-103, R³ is CH₃ and Q is Q3, and R and R⁴ are as per any row of Table U below, which compounds are named I.A1-Z103-Q3-U-1 to I.A1-Z103-Q3-U-42.

In another embodiment, compounds I are of formula I.A1 wherein Z is Z-103, R³ is CH₃ and Q is Q4, and R and R⁴ are as per any row of Table U below, which compounds are named I.A1-Z103-Q4-U-1 to I.A1-Z103-Q4-U-42.

In another embodiment, compounds I are of formula I.A2 wherein Z is Z-103, R³ is CH₃ and Q is Q1, and R and R⁴ are as per any row of Table U below, which compounds are named I.A2-Z103-Q1-U-1 to I.A2-Z103-Q1-U-42.

In another embodiment, compounds I are of formula I.A2 wherein Z is Z-103, R³ is CH₃ and Q is Q2, and R and R⁴ are as per any row of Table U below, which compounds are named I.A2-Z103-Q2-U-1 to I.A2-Z103-Q2-U-42.

In another embodiment, compounds I are of formula I.A2 wherein Z is Z-103, R³ is CH₃ and Q is Q3, and R and R⁴ are as per any row of Table U below, which compounds are named I.A2-Z103-Q3-U-1 to I.A2-Z103-Q3-U-42.

In another embodiment, compounds I are of formula I.A2 wherein Z is Z-103, R³ is CH₃ and Q is Q4, and R and R⁴ are as per any row of Table U below, which compounds are named I.A2-Z103-Q4-U-1 to I.A2-Z103-Q4-U-42.

In another embodiment, compounds I are of formula I.A1 wherein Z is Z-104, R³ is CH₃ and Q is Q1, and R and R⁴ are as per any row of Table U below, which compounds are named I.A1-Z104-Q1-U-1 to I.A1-Z104-Q1-U-42.

In another embodiment, compounds I are of formula I.A1 wherein Z is Z-104, R³ is CH₃ and Q is Q2, and R and R⁴ are as per any row of Table U below, which compounds are named I.A1-Z104-Q2-U-1 to I.A1-Z104-Q2-U-42.

In another embodiment, compounds I are of formula I.A1 wherein Z is Z-104, R³ is CH₃ and Q is Q3, and R and R⁴ are as per any row of Table U below, which compounds are named I.A1-Z104-Q3-U-1 to I.A1-Z104-Q3-U-42.

In another embodiment, compounds I are of formula I.A1 wherein Z is Z-104, R³ is CH₃ and Q is Q4, and R and R⁴ are as per any row of Table U below, which compounds are named I.A1-Z104-Q4-U-1 to I.A1-Z104-Q4-U-42.

In another embodiment, compounds I are of formula I.A2 wherein Z is Z-104, R³ is CH₃ and Q is Q1, and R and R⁴ are as per any row of Table U below, which compounds are named I.A2-Z104-Q1-U-1 to I.A2-Z104-Q1-U-42.

In another embodiment, compounds I are of formula I.A2 wherein Z is Z-104, R³ is CH₃ and Q is Q2, and R and R⁴ are as per any row of Table U below, which compounds are named I.A2-Z104-Q2-U-1 to I.A2-Z104-Q2-U-42.

In another embodiment, compounds I are of formula I.A2 wherein Z is Z-104, R³ is CH₃ and Q is Q3, and R and R⁴ are as per any row of Table U below, which compounds are named I.A2-Z104-Q3-U-1 to I.A2-Z104-Q3-U-42.

In another embodiment, compounds I are of formula I.A2 wherein Z is Z-104, R³ is CH₃ and Q is Q4, and R and R⁴ are as per any row of Table U below, which compounds are named I.A2-Z104-Q4-U-1 to I.A2-Z104-Q4-U-42.

In another embodiment, compounds I are of formula I.A1 wherein Z is Z-105, R³ is CH₃ and Q is Q1, and R and R⁴ are as per any row of Table U below, which compounds are named I.A1-Z105-Q1-U-1 to I.A1-Z105-Q1-U-42.

In another embodiment, compounds I are of formula I.A1 wherein Z is Z-105, R³ is CH₃ and Q is Q2, and R and R⁴ are as per any row of Table U below, which compounds are named I.A1-Z105-Q2-U-1 to I.A1-Z105-Q2-U-42.

In another embodiment, compounds I are of formula I.A1 wherein Z is Z-105, R³ is CH₃ and Q is Q3, and R and R⁴ are as per any row of Table U below, which compounds are named I.A1-Z105-Q3-U-1 to I.A1-Z105-Q3-U-42.

In another embodiment, compounds I are of formula I.A1 wherein Z is Z-105, R³ is CH₃ and Q is Q4, and R and R⁴ are as per any row of Table U below, which compounds are named I.A1-Z105-Q4-U-1 to I.A1-Z105-Q4-U-42.

In another embodiment, compounds I are of formula I.A2 wherein Z is Z-105, R³ is CH₃ and Q is Q1, and R and R⁴ are as per any row of Table U below, which compounds are named I.A2-Z105-Q1-U-1 to I.A2-Z105-Q1-U-42.

In another embodiment, compounds I are of formula I.A2 wherein Z is Z-105, R³ is CH₃ and Q is Q2, and R and R⁴ are as per any row of Table U below, which compounds are named I.A2-Z105-Q2-U-1 to I.A2-Z105-Q2-U-42.

In another embodiment, compounds I are of formula I.A2 wherein Z is Z-105, R³ is CH₃ and Q is Q3, and R and R⁴ are as per any row of Table U below, which compounds are named I.A2-Z105-Q3-U-1 to I.A2-Z105-Q3-U-42.

In another embodiment, compounds I are of formula I.A2 wherein Z is Z-105, R³ is CH₃ and Q is Q4, and R and R⁴ are as per any row of Table U below, which compounds are named I.A2-Z105-Q4-U-1 to I.A2-Z105-Q4-U-42.

In another embodiment, compounds I are of formula I.A1 wherein Z is Z-106, R³ is CH₃ and Q is Q1, and R and R⁴ are as per any row of Table U below, which compounds are named I.A1-Z106-Q1-U-1 to I.A1-Z106-Q1-U-42.

In another embodiment, compounds I are of formula I.A1 wherein Z is Z-106, R³ is CH₃ and Q is Q2, and R and R⁴ are as per any row of Table U below, which compounds are named I.A1-Z106-Q2-U-1 to I.A1-Z106-Q2-U-42.

In another embodiment, compounds I are of formula I.A1 wherein Z is Z-106, R³ is CH₃ and Q is Q3, and R and R⁴ are as per any row of Table U below, which compounds are named I.A1-Z106-Q3-U-1 to I.A1-Z106-Q3-U-42.

In another embodiment, compounds I are of formula I.A1 wherein Z is Z-106, R³ is CH₃ and Q is Q4, and R and R⁴ are as per any row of Table U below, which compounds are named I.A1-Z106-Q4-U-1 to I.A1-Z106-Q4-U-42.

In another embodiment, compounds I are of formula I.A2 wherein Z is Z-106, R³ is CH₃ and Q is Q1, and R and R⁴ are as per any row of Table U below, which compounds are named I.A2-Z106-Q1-U-1 to I.A2-Z106-Q1-U-42.

In another embodiment, compounds I are of formula I.A2 wherein Z is Z-106, R³ is CH₃ and Q is Q2, and R and R⁴ are as per any row of Table U below, which compounds are named I.A2-Z106-Q2-U-1 to I.A2-Z106-Q2-U-42.

In another embodiment, compounds I are of formula I.A2 wherein Z is Z-106, R³ is CH₃ and Q is Q3, and R and R⁴ are as per any row of Table U below, which compounds are named I.A2-Z106-Q3-U-1 to I.A2-Z106-Q3-U-42.

In another embodiment, compounds I are of formula I.A2 wherein Z is Z-106, R³ is CH₃ and Q is Q4, and R and R⁴ are as per any row of Table U below, which compounds are named I.A2-Z106-Q4-U-1 to I.A2-Z106-Q4-U-42.

In another embodiment, compounds I are of formula I.A1 wherein Z is Z-107, R³ is CH₃ and Q is Q1, and R and R⁴ are as per any row of Table U below, which compounds are named I.A1-Z107-Q1-U-1 to I.A1-Z107-Q1-U-42.

In another embodiment, compounds I are of formula I.A1 wherein Z is Z-107, R³ is CH₃ and Q is Q2, and R and R⁴ are as per any row of Table U below, which compounds are named I.A1-Z107-Q2-U-1 to I.A1-Z107-Q2-U-42.

In another embodiment, compounds I are of formula I.A1 wherein Z is Z-107, R³ is CH₃ and Q is Q3, and R and R⁴ are as per any row of Table U below, which compounds are named I.A1-Z107-Q3-U-1 to I.A1-Z107-Q3-U-42.

In another embodiment, compounds I are of formula I.A1 wherein Z is Z-107, R³ is CH₃ and Q is Q4, and R and R⁴ are as per any row of Table U below, which compounds are named I.A1-Z107-Q4-U-1 to I.A1-Z107-Q4-U-42.

In another embodiment, compounds I are of formula I.A2 wherein Z is Z-107, R³ is CH₃ and Q is Q1, and R and R⁴ are as per any row of Table U below, which compounds are named I.A2-Z107-Q1-U-1 to I.A2-Z107-Q1-U-42.

In another embodiment, compounds I are of formula I.A2 wherein Z is Z-107, R³ is CH₃ and Q is Q2, and R and R⁴ are as per any row of Table U below, which compounds are named I.A2-Z107-Q2-U-1 to I.A2-Z107-Q2-U-42.

In another embodiment, compounds I are of formula I.A2 wherein Z is Z-107, R³ is CH₃ and Q is Q3, and R and R⁴ are as per any row of Table U below, which compounds are named I.A2-Z107-Q3-U-1 to I.A2-Z107-Q3-U-42.

In another embodiment, compounds I are of formula I.A2 wherein Z is Z-107, R³ is CH₃ and Q is Q4, and R and R⁴ are as per any row of Table U below, which compounds are named I.A2-Z107-Q4-U-1 to I.A2-Z107-Q4-U-42.

In another embodiment, compounds I are of formula I.A1 wherein Z is Z-108, R³ is CH₃ and Q is Q1, and R and R⁴ are as per any row of Table U below, which compounds are named I.A1-Z108-Q1-U-1 to I.A1-Z108-Q1-U-42.

In another embodiment, compounds I are of formula I.A1 wherein Z is Z-108, R³ is CH₃ and Q is Q2, and R and R⁴ are as per any row of Table U below, which compounds are named I.A1-Z108-Q2-U-1 to I.A1-Z108-Q2-U-42.

In another embodiment, compounds I are of formula I.A1 wherein Z is Z-108, R³ is CH₃ and Q is Q3, and R and R⁴ are as per any row of Table U below, which compounds are named I.A1-Z108-Q3-U-1 to I.A1-Z108-Q3-U-42.

In another embodiment, compounds I are of formula I.A1 wherein Z is Z-108, R³ is CH₃ and Q is Q4, and R and R⁴ are as per any row of Table U below, which compounds are named I.A1-Z108-Q4-U-1 to I.A1-Z108-Q4-U-42.

In another embodiment, compounds I are of formula I.A2 wherein Z is Z-108, R³ is CH₃ and Q is Q1, and R and R⁴ are as per any row of Table U below, which compounds are named I.A2-Z108-Q1-U-1 to I.A2-Z108-Q1-U-42.

In another embodiment, compounds I are of formula I.A2 wherein Z is Z-108, R³ is CH₃ and Q is Q2, and R and R⁴ are as per any row of Table U below, which compounds are named I.A2-Z108-Q2-U-1 to I.A2-Z108-Q2-U-42.

In another embodiment, compounds I are of formula I.A2 wherein Z is Z-108, R³ is CH₃ and Q is Q3, and R and R⁴ are as per any row of Table U below, which compounds are named I.A2-Z108-Q3-U-1 to I.A2-Z108-Q3-U-42.

In another embodiment, compounds I are of formula I.A2 wherein Z is Z-108, R³ is CH₃ and Q is Q4, and R and R⁴ are as per any row of Table U below, which compounds are named I.A2-Z108-Q4-U-1 to I.A2-Z108-Q4-U-42.

In another embodiment, compounds I are of formula I.A1 wherein Z is Z-109, R³ is CH₃ and Q is Q1, and R and R⁴ are as per any row of Table U below, which compounds are named I.A1-Z109-Q1-U-1 to I.A1-Z109-Q1-U-42.

In another embodiment, compounds I are of formula I.A1 wherein Z is Z-109, R³ is CH₃ and Q is Q2, and R and R⁴ are as per any row of Table U below, which compounds are named I.A1-Z109-Q2-U-1 to I.A1-Z109-Q2-U-42.

In another embodiment, compounds I are of formula I.A1 wherein Z is Z-109, R³ is CH₃ and Q is Q3, and R and R⁴ are as per any row of Table U below, which compounds are named I.A1-Z109-Q3-U-1 to I.A1-Z109-Q3-U-42.

In another embodiment, compounds I are of formula I.A1 wherein Z is Z-109, R³ is CH₃ and Q is Q4, and R and R⁴ are as per any row of Table U below, which compounds are named I.A1-Z109-Q4-U-1 to I.A1-Z109-Q4-U-42.

In another embodiment, compounds I are of formula I.A2 wherein Z is Z-109, R³ is CH₃ and Q is Q1, and R and R⁴ are as per any row of Table U below, which compounds are named I.A2-Z109-Q1-U-1 to I.A2-Z109-Q1-U-42.

In another embodiment, compounds I are of formula I.A2 wherein Z is Z-109, R³ is CH₃ and Q is Q2, and R and R⁴ are as per any row of Table U below, which compounds are named I.A2-Z109-Q2-U-1 to I.A2-Z109-Q2-U-42.

In another embodiment, compounds I are of formula I.A2 wherein Z is Z-109, R³ is CH₃ and Q is Q3, and R and R⁴ are as per any row of Table U below, which compounds are named I.A2-Z109-Q3-U-1 to I.A2-Z109-Q3-U-42.

In another embodiment, compounds I are of formula I.A2 wherein Z is Z-109, R³ is CH₃ and Q is Q4, and R and R⁴ are as per any row of Table U below, which compounds are named I.A2-Z109-Q4-U-1 to I.A2-Z109-Q4-U-42.

In another embodiment, compounds I are of formula I.A1 wherein Z is Z-110, R³ is CH₃ and Q is Q1, and R and R⁴ are as per any row of Table U below, which compounds are named I.A1-Z110-Q1-U-1 to I.A1-Z110-Q1-U-42.

In another embodiment, compounds I are of formula I.A1 wherein Z is Z-110, R³ is CH₃ and Q is Q2, and R and R⁴ are as per any row of Table U below, which compounds are named I.A1-Z110-Q2-U-1 to I.A1-Z110-Q2-U-42.

In another embodiment, compounds I are of formula I.A1 wherein Z is Z-110, R³ is CH₃ and Q is Q3, and R and R⁴ are as per any row of Table U below, which compounds are named I.A1-Z110-Q3-U-1 to I.A1-Z110-Q3-U-42.

In another embodiment, compounds I are of formula I.A1 wherein Z is Z-110, R³ is CH₃ and Q is Q4, and R and R⁴ are as per any row of Table U below, which compounds are named I.A1-Z110-Q4-U-1 to I.A1-Z110-Q4-U-42.

In another embodiment, compounds I are of formula I.A2 wherein Z is Z-110, R³ is CH₃ and Q is Q1, and R and R⁴ are as per any row of Table U below, which compounds are named I.A2-Z110-Q1-U-1 to I.A2-Z110-Q1-U-42.

In another embodiment, compounds I are of formula I.A2 wherein Z is Z-110, R³ is CH₃ and Q is Q2, and R and R⁴ are as per any row of Table U below, which compounds are named I.A2-Z110-Q2-U-1 to I.A2-Z110-Q2-U-42.

In another embodiment, compounds I are of formula I.A2 wherein Z is Z-110, R³ is CH₃ and Q is Q3, and R and R⁴ are as per any row of Table U below, which compounds are named I.A2-Z110-Q3-U-1 to I.A2-Z110-Q3-U-42.

In another embodiment, compounds I are of formula I.A2 wherein Z is Z-110, R³ is CH₃ and Q is Q4, and R and R⁴ are as per any row of Table U below, which compounds are named I.A2-Z110-Q4-U-1 to I.A2-Z110-Q4-U-42.

In another embodiment, compounds I are of formula I.A1 wherein Z is Z-111, R³ is CH₃ and Q is Q1, and R and R⁴ are as per any row of Table U below, which compounds are named I.A1-Z111-Q1-U-1 to I.A1-Z111-Q1-U-42.

In another embodiment, compounds I are of formula I.A1 wherein Z is Z-111, R³ is CH₃ and Q is Q2, and R and R⁴ are as per any row of Table U below, which compounds are named I.A1-Z111-Q2-U-1 to I.A1-Z111-Q2-U-42.

In another embodiment, compounds I are of formula I.A1 wherein Z is Z-111, R³ is CH₃ and Q is Q3, and R and R⁴ are as per any row of Table U below, which compounds are named I.A1-Z111-Q3-U-1 to I.A1-Z111-Q3-U-42.

In another embodiment, compounds I are of formula I.A1 wherein Z is Z-111, R³ is CH₃ and Q is Q4, and R and R⁴ are as per any row of Table U below, which compounds are named I.A1-Z111-Q4-U-1 to I.A1-Z111-Q4-U-42.

In another embodiment, compounds I are of formula I.A2 wherein Z is Z-111, R³ is CH₃ and Q is Q1, and R and R⁴ are as per any row of Table U below, which compounds are named I.A2-Z111-Q1-U-1 to I.A2-Z111-Q1-U-42.

In another embodiment, compounds I are of formula I.A2 wherein Z is Z-111, R³ is CH₃ and Q is Q2, and R and R⁴ are as per any row of Table U below, which compounds are named I.A2-Z111-Q2-U-1 to I.A2-Z111-Q2-U-42.

In another embodiment, compounds I are of formula I.A2 wherein Z is Z-111, R³ is CH₃ and Q is Q3, and R and R⁴ are as per any row of Table U below, which compounds are named I.A2-Z111-Q3-U-1 to I.A2-Z111-Q3-U-42.

In another embodiment, compounds I are of formula I.A2 wherein Z is Z-111, R³ is CH₃ and Q is Q4, and R and R⁴ are as per any row of Table U below, which compounds are named I.A2-Z111-Q4-U-1 to I.A2-Z111-Q4-U-42.

In another embodiment, compounds I are of formula I.A1 wherein Z is Z-112, R³ is CH₃ and Q is Q1, and R and R⁴ are as per any row of Table U below, which compounds are named I.A1-Z112-Q1-U-1 to I.A1-Z112-Q1-U-42.

In another embodiment, compounds I are of formula I.A1 wherein Z is Z-112, R³ is CH₃ and Q is Q2, and R and R⁴ are as per any row of Table U below, which compounds are named I.A1-Z112-Q2-U-1 to I.A1-Z112-Q2-U-42.

In another embodiment, compounds I are of formula I.A1 wherein Z is Z-112, R³ is CH₃ and Q is Q3, and R and R⁴ are as per any row of Table U below, which compounds are named I.A1-Z112-Q3-U-1 to I.A1-Z112-Q3-U-42.

In another embodiment, compounds I are of formula I.A1 wherein Z is Z-112, R³ is CH₃ and Q is Q4, and R and R⁴ are as per any row of Table U below, which compounds are named I.A1-Z112-Q4-U-1 to I.A1-Z112-Q4-U-42.

In another embodiment, compounds I are of formula I.A2 wherein Z is Z-112, R³ is CH₃ and Q is Q1, and R and R⁴ are as per any row of Table U below, which compounds are named I.A2-Z112-Q1-U-1 to I.A2-Z112-Q1-U-42.

In another embodiment, compounds I are of formula I.A2 wherein Z is Z-112, R³ is CH₃ and Q is Q2, and R and R⁴ are as per any row of Table U below, which compounds are named I.A2-Z112-Q2-U-1 to I.A2-Z112-Q2-U-42.

In another embodiment, compounds I are of formula I.A2 wherein Z is Z-112, R³ is CH₃ and Q is Q3, and R and R⁴ are as per any row of Table U below, which compounds are named I.A2-Z112-Q3-U-1 to I.A2-Z112-Q3-U-42.

In another embodiment, compounds I are of formula I.A2 wherein Z is Z-112, R³ is CH₃ and Q is Q4, and R and R⁴ are as per any row of Table U below, which compounds are named I.A2-Z112-Q4-U-1 to I.A2-Z112-Q4-U-42.

In another embodiment, compounds I are of formula I.A1 wherein Z is Z-113, R³ is CH₃ and Q is Q1, and R and R⁴ are as per any row of Table U below, which compounds are named I.A1-Z113-Q1-U-1 to I.A1-Z113-Q1-U-42.

In another embodiment, compounds I are of formula I.A1 wherein Z is Z-113, R³ is CH₃ and Q is Q2, and R and R⁴ are as per any row of Table U below, which compounds are named I.A1-Z113-Q2-U-1 to I.A1-Z113-Q2-U-42.

In another embodiment, compounds I are of formula I.A1 wherein Z is Z-113, R³ is CH₃ and Q is Q3, and R and R⁴ are as per any row of Table U below, which compounds are named I.A1-Z113-Q3-U-1 to I.A1-Z113-Q3-U-42.

In another embodiment, compounds I are of formula I.A1 wherein Z is Z-113, R³ is CH₃ and Q is Q4, and R and R⁴ are as per any row of Table U below, which compounds are named I.A1-Z113-Q4-U-1 to I.A1-Z113-Q4-U-42.

In another embodiment, compounds I are of formula I.A2 wherein Z is Z-113, R³ is CH₃ and Q is Q1, and R and R⁴ are as per any row of Table U below, which compounds are named I.A2-Z113-Q1-U-1 to I.A2-Z113-Q1-U-42.

In another embodiment, compounds I are of formula I.A2 wherein Z is Z-113, R³ is CH₃ and Q is Q2, and R and R⁴ are as per any row of Table U below, which compounds are named I.A2-Z113-Q2-U-1 to I.A2-Z113-Q2-U-42.

In another embodiment, compounds I are of formula I.A2 wherein Z is Z-113, R³ is CH₃ and Q is Q3, and R and R⁴ are as per any row of Table U below, which compounds are named I.A2-Z113-Q3-U-1 to I.A2-Z113-Q3-U-42.

In another embodiment, compounds I are of formula I.A2 wherein Z is Z-113, R³ is CH₃ and Q is Q4, and R and R⁴ are as per any row of Table U below, which compounds are named I.A2-Z113-Q4-U-1 to I.A2-Z113-Q4-U-42.

In another embodiment, compounds I are of formula I.A1 wherein Z is Z-114, R³ is CH₃ and Q is Q1, and R and R⁴ are as per any row of Table U below, which compounds are named I.A1-Z114-Q1-U-1 to I.A1-Z114-Q1-U-42.

In another embodiment, compounds I are of formula I.A1 wherein Z is Z-114, R³ is CH₃ and Q is Q2, and R and R⁴ are as per any row of Table U below, which compounds are named I.A1-Z114-Q2-U-1 to I.A1-Z114-Q2-U-42.

In another embodiment, compounds I are of formula I.A1 wherein Z is Z-114, R³ is CH₃ and Q is Q3, and R and R⁴ are as per any row of Table U below, which compounds are named I.A1-Z114-Q3-U-1 to I.A1-Z114-Q3-U-42.

In another embodiment, compounds I are of formula I.A1 wherein Z is Z-114, R³ is CH₃ and Q is Q4, and R and R⁴ are as per any row of Table U below, which compounds are named I.A1-Z114-Q4-U-1 to I.A1-Z114-Q4-U-42.

In another embodiment, compounds I are of formula I.A2 wherein Z is Z-114, R³ is CH₃ and Q is Q1, and R and R⁴ are as per any row of Table U below, which compounds are named I.A2-Z114-Q1-U-1 to I.A2-Z114-Q1-U-42.

In another embodiment, compounds I are of formula I.A2 wherein Z is Z-114, R³ is CH₃ and Q is Q2, and R and R⁴ are as per any row of Table U below, which compounds are named I.A2-Z114-Q2-U-1 to I.A2-Z114-Q2-U-42.

In another embodiment, compounds I are of formula I.A2 wherein Z is Z-114, R³ is CH₃ and Q is Q3, and R and R⁴ are as per any row of Table U below, which compounds are named I.A2-Z114-Q3-U-1 to I.A2-Z114-Q3-U-42.

In another embodiment, compounds I are of formula I.A2 wherein Z is Z-114, R³ is CH₃ and Q is Q4, and R and R⁴ are as per any row of Table U below, which compounds are named I.A2-Z114-Q4-U-1 to I.A2-Z114-Q4-U-42.

In another embodiment, compounds I are of formula I.A1 wherein Z is Z-115, R³ is CH₃ and Q is Q1, and R and R⁴ are as per any row of Table U below, which compounds are named I.A1-Z115-Q1-U-1 to I.A1-Z115-Q1-U-42.

In another embodiment, compounds I are of formula I.A1 wherein Z is Z-115, R³ is CH₃ and Q is Q2, and R and R⁴ are as per any row of Table U below, which compounds are named I.A1-Z115-Q2-U-1 to I.A1-Z115-Q2-U-42.

In another embodiment, compounds I are of formula I.A1 wherein Z is Z-115, R³ is CH₃ and Q is Q3, and R and R⁴ are as per any row of Table U below, which compounds are named I.A1-Z115-Q3-U-1 to I.A1-Z115-Q3-U-42.

In another embodiment, compounds I are of formula I.A1 wherein Z is Z-115, R³ is CH₃ and Q is Q4, and R and R⁴ are as per any row of Table U below, which compounds are named I.A1-Z115-Q4-U-1 to I.A1-Z115-Q4-U-42.

In another embodiment, compounds I are of formula I.A2 wherein Z is Z-115, R³ is CH₃ and Q is Q1, and R and R⁴ are as per any row of Table U below, which compounds are named I.A2-Z115-Q1-U-1 to I.A2-Z115-Q1-U-42.

In another embodiment, compounds I are of formula I.A2 wherein Z is Z-115, R³ is CH₃ and Q is Q2, and R and R⁴ are as per any row of Table U below, which compounds are named I.A2-Z115-Q2-U-1 to I.A2-Z115-Q2-U-42.

In another embodiment, compounds I are of formula I.A2 wherein Z is Z-115, R³ is CH₃ and Q is Q3, and R and R⁴ are as per any row of Table U below, which compounds are named I.A2-Z115-Q3-U-1 to I.A2-Z115-Q3-U-42.

In another embodiment, compounds I are of formula I.A2 wherein Z is Z-115, R³ is CH₃ and Q is Q4, and R and R⁴ are as per any row of Table U below, which compounds are named I.A2-Z115-Q4-U-1 to I.A2-Z115-Q4-U-42.

In another embodiment, compounds I are of formula I.A1 wherein Z is Z-116, R³ is CH₃ and Q is Q1, and R and R⁴ are as per any row of Table U below, which compounds are named I.A1-Z116-Q1-U-1 to I.A1-Z116-Q1-U-42.

In another embodiment, compounds I are of formula I.A1 wherein Z is Z-116, R³ is CH₃ and Q is Q2, and R and R⁴ are as per any row of Table U below, which compounds are named I.A1-Z116-Q2-U-1 to I.A1-Z116-Q2-U-42.

In another embodiment, compounds I are of formula I.A1 wherein Z is Z-116, R³ is CH₃ and Q is Q3, and R and R⁴ are as per any row of Table U below, which compounds are named I.A1-Z116-Q3-U-1 to I.A1-Z116-Q3-U-42.

In another embodiment, compounds I are of formula I.A1 wherein Z is Z-116, R³ is CH₃ and Q is Q4, and R and R⁴ are as per any row of Table U below, which compounds are named I.A1-Z116-Q4-U-1 to I.A1-Z116-Q4-U-42.

In another embodiment, compounds I are of formula I.A2 wherein Z is Z-116, R³ is CH₃ and Q is Q1, and R and R⁴ are as per any row of Table U below, which compounds are named I.A2-Z116-Q1-U-1 to I.A2-Z116-Q1-U-42.

In another embodiment, compounds I are of formula I.A2 wherein Z is Z-116, R³ is CH₃ and Q is Q2, and R and R⁴ are as per any row of Table U below, which compounds are named I.A2-Z116-Q2-U-1 to I.A2-Z116-Q2-U-42.

In another embodiment, compounds I are of formula I.A2 wherein Z is Z-116, R³ is CH₃ and Q is Q3, and R and R⁴ are as per any row of Table U below, which compounds are named I.A2-Z116-Q3-U-1 to I.A2-Z116-Q3-U-42.

In another embodiment, compounds I are of formula I.A2 wherein Z is Z-116, R³ is CH₃ and Q is Q4, and R and R⁴ are as per any row of Table U below, which compounds are named I.A2-Z116-Q4-U-1 to I.A2-Z116-Q4-U-42.

In another embodiment, compounds I are of formula I.A1 wherein Z is Z-117, R³ is CH₃ and Q is Q1, and R and R⁴ are as per any row of Table U below, which compounds are named I.A1-Z117-Q1-U-1 to I.A1-Z117-Q1-U-42.

In another embodiment, compounds I are of formula I.A1 wherein Z is Z-117, R³ is CH₃ and Q is Q2, and R and R⁴ are as per any row of Table U below, which compounds are named I.A1-Z117-Q2-U-1 to I.A1-Z117-Q2-U-42.

In another embodiment, compounds I are of formula I.A1 wherein Z is Z-117, R³ is CH₃ and Q is Q3, and R and R⁴ are as per any row of Table U below, which compounds are named I.A1-Z117-Q3-U-1 to I.A1-Z117-Q3-U-42.

In another embodiment, compounds I are of formula I.A1 wherein Z is Z-117, R³ is CH₃ and Q is Q4, and R and R⁴ are as per any row of Table U below, which compounds are named I.A1-Z117-Q4-U-1 to I.A1-Z117-Q4-U-42.

In another embodiment, compounds I are of formula I.A2 wherein Z is Z-117, R³ is CH₃ and Q is Q1, and R and R⁴ are as per any row of Table U below, which compounds are named I.A2-Z117-Q1-U-1 to I.A2-Z117-Q1-U-42.

In another embodiment, compounds I are of formula I.A2 wherein Z is Z-117, R³ is CH₃ and Q is Q2, and R and R⁴ are as per any row of Table U below, which compounds are named I.A2-Z117-Q2-U-1 to I.A2-Z117-Q2-U-42.

In another embodiment, compounds I are of formula I.A2 wherein Z is Z-117, R³ is CH₃ and Q is Q3, and R and R⁴ are as per any row of Table U below, which compounds are named I.A2-Z117-Q3-U-1 to I.A2-Z117-Q3-U-42.

In another embodiment, compounds I are of formula I.A2 wherein Z is Z-117, R³ is CH₃ and Q is Q4, and R and R⁴ are as per any row of Table U below, which compounds are named I.A2-Z117-Q4-U-1 to I.A2-Z117-Q4-U-42.

In another embodiment, compounds I are of formula I.A1 wherein Z is Z-118, R³ is CH₃ and Q is Q1, and R and R⁴ are as per any row of Table U below, which compounds are named I.A1-Z118-Q1-U-1 to I.A1-Z118-Q1-U-42.

In another embodiment, compounds I are of formula I.A1 wherein Z is Z-118, R³ is CH₃ and Q is Q2, and R and R⁴ are as per any row of Table U below, which compounds are named I.A1-Z118-Q2-U-1 to I.A1-Z118-Q2-U-42.

In another embodiment, compounds I are of formula I.A1 wherein Z is Z-118, R³ is CH₃ and Q is Q3, and R and R⁴ are as per any row of Table U below, which compounds are named I.A1-Z118-Q3-U-1 to I.A1-Z118-Q3-U-42.

In another embodiment, compounds I are of formula I.A1 wherein Z is Z-118, R³ is CH₃ and Q is Q4, and R and R⁴ are as per any row of Table U below, which compounds are named I.A1-Z118-Q4-U-1 to I.A1-Z118-Q4-U-42.

In another embodiment, compounds I are of formula I.A2 wherein Z is Z-118, R³ is CH₃ and Q is Q1, and R and R⁴ are as per any row of Table U below, which compounds are named I.A2-Z118-Q1-U-1 to I.A2-Z118-Q1-U-42.

In another embodiment, compounds I are of formula I.A2 wherein Z is Z-118, R³ is CH₃ and Q is Q2, and R and R⁴ are as per any row of Table U below, which compounds are named I.A2-Z118-Q2-U-1 to I.A2-Z118-Q2-U-42.

In another embodiment, compounds I are of formula I.A2 wherein Z is Z-118, R³ is CH₃ and Q is Q3, and R and R⁴ are as per any row of Table U below, which compounds are named I.A2-Z118-Q3-U-1 to I.A2-Z118-Q3-U-42.

In another embodiment, compounds I are of formula I.A2 wherein Z is Z-118, R³ is CH₃ and Q is Q4, and R and R⁴ are as per any row of Table U below, which compounds are named I.A2-Z118-Q4-U-1 to I.A2-Z118-Q4-U-42.

In another embodiment, compounds I are of formula I.A1 wherein Z is Z-119, R³ is CH₃ and Q is Q1, and R and R⁴ are as per any row of Table U below, which compounds are named I.A1-Z119-Q1-U-1 to I.A1-Z119-Q1-U-42.

In another embodiment, compounds I are of formula I.A1 wherein Z is Z-119, R³ is CH₃ and Q is Q2, and R and R⁴ are as per any row of Table U below, which compounds are named I.A1-Z119-Q2-U-1 to I.A1-Z119-Q2-U-42.

In another embodiment, compounds I are of formula I.A1 wherein Z is Z-119, R³ is CH₃ and Q is Q3, and R and R⁴ are as per any row of Table U below, which compounds are named I.A1-Z119-Q3-U-1 to I.A1-Z119-Q3-U-42.

In another embodiment, compounds I are of formula I.A1 wherein Z is Z-119, R³ is CH₃ and Q is Q4, and R and R⁴ are as per any row of Table U below, which compounds are named I.A1-Z119-Q4-U-1 to I.A1-Z119-Q4-U-42.

In another embodiment, compounds I are of formula I.A2 wherein Z is Z-119, R³ is CH₃ and Q is Q1, and R and R⁴ are as per any row of Table U below, which compounds are named I.A2-Z119-Q1-U-1 to I.A2-Z119-Q1-U-42.

In another embodiment, compounds I are of formula I.A2 wherein Z is Z-119, R³ is CH₃ and Q is Q2, and R and R⁴ are as per any row of Table U below, which compounds are named I.A2-Z119-Q2-U-1 to I.A2-Z119-Q2-U-42.

In another embodiment, compounds I are of formula I.A2 wherein Z is Z-119, R³ is CH₃ and Q is Q3, and R and R⁴ are as per any row of Table U below, which compounds are named I.A2-Z119-Q3-U-1 to I.A2-Z119-Q3-U-42.

In another embodiment, compounds I are of formula I.A2 wherein Z is Z-119, R³ is CH₃ and Q is Q4, and R and R⁴ are as per any row of Table U below, which compounds are named I.A2-Z119-Q4-U-1 to I.A2-Z119-Q4-U-42.

In another embodiment, compounds I are of formula I.A1 wherein Z is Z-120, R³ is CH₃ and Q is Q1, and R and R⁴ are as per any row of Table U below, which compounds are named I.A1-Z120-Q1-U-1 to I.A1-Z120-Q1-U-42.

In another embodiment, compounds I are of formula I.A1 wherein Z is Z-120, R³ is CH₃ and Q is Q2, and R and R⁴ are as per any row of Table U below, which compounds are named I.A1-Z120-Q2-U-1 to I.A1-Z120-Q2-U-42.

In another embodiment, compounds I are of formula I.A1 wherein Z is Z-120, R³ is CH₃ and Q is Q3, and R and R⁴ are as per any row of Table U below, which compounds are named I.A1-Z120-Q3-U-1 to I.A1-Z120-Q3-U-42.

In another embodiment, compounds I are of formula I.A1 wherein Z is Z-120, R³ is CH₃ and Q is Q4, and R and R⁴ are as per any row of Table U below, which compounds are named I.A1-Z120-Q4-U-1 to I.A1-Z120-Q4-U-42.

In another embodiment, compounds I are of formula I.A2 wherein Z is Z-120, R³ is CH₃ and Q is Q1, and R and R⁴ are as per any row of Table U below, which compounds are named I.A2-Z120-Q1-U-1 to I.A2-Z120-Q1-U-42.

In another embodiment, compounds I are of formula I.A2 wherein Z is Z-120, R³ is CH₃ and Q is Q2, and R and R⁴ are as per any row of Table U below, which compounds are named I.A2-Z120-Q2-U-1 to I.A2-Z120-Q2-U-42.

In another embodiment, compounds I are of formula I.A2 wherein Z is Z-120, R³ is CH₃ and Q is Q3, and R and R⁴ are as per any row of Table U below, which compounds are named I.A2-Z120-Q3-U-1 to I.A2-Z120-Q3-U-42.

In another embodiment, compounds I are of formula I.A2 wherein Z is Z-120, R³ is CH₃ and Q is Q4, and R and R⁴ are as per any row of Table U below, which compounds are named I.A2-Z120-Q4-U-1 to I.A2-Z120-Q4-U-42.

In another embodiment, compounds I are of formula I.A1 wherein Z is Z-121, R³ is CH₃ and Q is Q1, and R and R⁴ are as per any row of Table U below, which compounds are named I.A1-Z121-Q1-U-1 to I.A1-Z121-Q1-U-42.

In another embodiment, compounds I are of formula I.A1 wherein Z is Z-121, R³ is CH₃ and Q is Q2, and R and R⁴ are as per any row of Table U below, which compounds are named I.A1-Z121-Q2-U-1 to I.A1-Z121-Q2-U-42.

In another embodiment, compounds I are of formula I.A1 wherein Z is Z-121, R³ is CH₃ and Q is Q3, and R and R⁴ are as per any row of Table U below, which compounds are named I.A1-Z121-Q3-U-1 to I.A1-Z121-Q3-U-42.

In another embodiment, compounds I are of formula I.A1 wherein Z is Z-121, R³ is CH₃ and Q is Q4, and R and R⁴ are as per any row of Table U below, which compounds are named I.A1-Z121-Q4-U-1 to I.A1-Z121-Q4-U-42.

In another embodiment, compounds I are of formula I.A2 wherein Z is Z-121, R³ is CH₃ and Q is Q1, and R and R⁴ are as per any row of Table U below, which compounds are named I.A2-Z121-Q1-U-1 to I.A2-Z121-Q1-U-42.

In another embodiment, compounds I are of formula I.A2 wherein Z is Z-121, R³ is CH₃ and Q is Q2, and R and R⁴ are as per any row of Table U below, which compounds are named I.A2-Z121-Q2-U-1 to I.A2-Z121-Q2-U-42.

In another embodiment, compounds I are of formula I.A2 wherein Z is Z-121, R³ is CH₃ and Q is Q3, and R and R⁴ are as per any row of Table U below, which compounds are named I.A2-Z121-Q3-U-1 to I.A2-Z121-Q3-U-42.

In another embodiment, compounds I are of formula I.A2 wherein Z is Z-121, R³ is CH₃ and Q is Q4, and R and R⁴ are as per any row of Table U below, which compounds are named I.A2-Z121-Q4-U-1 to I.A2-Z121-Q4-U-42.

In another embodiment, compounds I are of formula I.A1 wherein Z is Z-122, R³ is CH₃ and Q is Q1, and R and R⁴ are as per any row of Table U below, which compounds are named I.A1-Z122-Q1-U-1 to I.A1-Z122-Q1-U-42.

In another embodiment, compounds I are of formula I.A1 wherein Z is Z-122, R³ is CH₃ and Q is Q2, and R and R⁴ are as per any row of Table U below, which compounds are named I.A1-Z122-Q2-U-1 to I.A1-Z122-Q2-U-42.

In another embodiment, compounds I are of formula I.A1 wherein Z is Z-122, R³ is CH₃ and Q is Q3, and R and R⁴ are as per any row of Table U below, which compounds are named I.A1-Z122-Q3-U-1 to I.A1-Z122-Q3-U-42.

In another embodiment, compounds I are of formula I.A1 wherein Z is Z-122, R³ is CH₃ and Q is Q4, and R and R⁴ are as per any row of Table U below, which compounds are named I.A1-Z122-Q4-U-1 to I.A1-Z122-Q4-U-42.

In another embodiment, compounds I are of formula I.A2 wherein Z is Z-122, R³ is CH₃ and Q is Q1, and R and R⁴ are as per any row of Table U below, which compounds are named I.A2-Z122-Q1-U-1 to I.A2-Z122-Q1-U-42.

In another embodiment, compounds I are of formula I.A2 wherein Z is Z-122, R³ is CH₃ and Q is Q2, and R and R⁴ are as per any row of Table U below, which compounds are named I.A2-Z122-Q2-U-1 to I.A2-Z122-Q2-U-42.

In another embodiment, compounds I are of formula I.A2 wherein Z is Z-122, R³ is CH₃ and Q is Q3, and R and R⁴ are as per any row of Table U below, which compounds are named I.A2-Z122-Q3-U-1 to I.A2-Z122-Q3-U-42.

In another embodiment, compounds I are of formula I.A2 wherein Z is Z-122, R³ is CH₃ and Q is Q4, and R and R⁴ are as per any row of Table U below, which compounds are named I.A2-Z122-Q4-U-1 to I.A2-Z122-Q4-U-42.

In another embodiment, compounds I are of formula I.A1 wherein Z is Z-123, R³ is CH₃ and Q is Q1, and R and R⁴ are as per any row of Table U below, which compounds are named I.A1-Z123-Q1-U-1 to I.A1-Z123-Q1-U-42.

In another embodiment, compounds I are of formula I.A1 wherein Z is Z-123, R³ is CH₃ and Q is Q2, and R and R⁴ are as per any row of Table U below, which compounds are named I.A1-Z123-Q2-U-1 to I.A1-Z123-Q2-U-42.

In another embodiment, compounds I are of formula I.A1 wherein Z is Z-123, R³ is CH₃ and Q is Q3, and R and R⁴ are as per any row of Table U below, which compounds are named I.A1-Z123-Q3-U-1 to I.A1-Z123-Q3-U-42.

In another embodiment, compounds I are of formula I.A1 wherein Z is Z-123, R³ is CH₃ and Q is Q4, and R and R⁴ are as per any row of Table U below, which compounds are named I.A1-Z123-Q4-U-1 to I.A1-Z123-Q4-U-42.

In another embodiment, compounds I are of formula I.A2 wherein Z is Z-123, R³ is CH₃ and Q is Q1, and R and R⁴ are as per any row of Table U below, which compounds are named I.A2-Z123-Q1-U-1 to I.A2-Z123-Q1-U-42.

In another embodiment, compounds I are of formula I.A2 wherein Z is Z-123, R³ is CH₃ and Q is Q2, and R and R⁴ are as per any row of Table U below, which compounds are named I.A2-Z123-Q2-U-1 to I.A2-Z123-Q2-U-42.

In another embodiment, compounds I are of formula I.A2 wherein Z is Z-123, R³ is CH₃ and Q is Q3, and R and R⁴ are as per any row of Table U below, which compounds are named I.A2-Z123-Q3-U-1 to I.A2-Z123-Q3-U-42.

In another embodiment, compounds I are of formula I.A2 wherein Z is Z-123, R³ is CH₃ and Q is Q4, and R and R⁴ are as per any row of Table U below, which compounds are named I.A2-Z123-Q4-U-1 to I.A2-Z123-Q4-U-42.

In another embodiment, compounds I are of formula I.A1 wherein Z is Z-124, R³ is CH₃ and Q is Q1, and R and R⁴ are as per any row of Table U below, which compounds are named I.A1-Z124-Q1-U-1 to I.A1-Z124-Q1-U-42.

In another embodiment, compounds I are of formula I.A1 wherein Z is Z-124, R³ is CH₃ and Q is Q2, and R and R⁴ are as per any row of Table U below, which compounds are named I.A1-Z124-Q2-U-1 to I.A1-Z124-Q2-U-42.

In another embodiment, compounds I are of formula I.A1 wherein Z is Z-124, R³ is CH₃ and Q is Q3, and R and R⁴ are as per any row of Table U below, which compounds are named I.A1-Z124-Q3-U-1 to I.A1-Z124-Q3-U-42.

In another embodiment, compounds I are of formula I.A1 wherein Z is Z-124, R³ is CH₃ and Q is Q4, and R and R⁴ are as per any row of Table U below, which compounds are named I.A1-Z124-Q4-U-1 to I.A1-Z124-Q4-U-42.

In another embodiment, compounds I are of formula I.A2 wherein Z is Z-124, R³ is CH₃ and Q is Q1, and R and R⁴ are as per any row of Table U below, which compounds are named I.A2-Z124-Q1-U-1 to I.A2-Z124-Q1-U-42.

In another embodiment, compounds I are of formula I.A2 wherein Z is Z-124, R³ is CH₃ and Q is Q2, and R and R⁴ are as per any row of Table U below, which compounds are named I.A2-Z124-Q2-U-1 to I.A2-Z124-Q2-U-42.

In another embodiment, compounds I are of formula I.A2 wherein Z is Z-124, R³ is CH₃ and Q is Q3, and R and R⁴ are as per any row of Table U below, which compounds are named I.A2-Z124-Q3-U-1 to I.A2-Z124-Q3-U-42.

In another embodiment, compounds I are of formula I.A2 wherein Z is Z-124, R³ is CH₃ and Q is Q4, and R and R⁴ are as per any row of Table U below, which compounds are named I.A2-Z124-Q4-U-1 to I.A2-Z124-Q4-U-42.

In another embodiment, compounds I are of formula I.A1 wherein Z is Z-125, R³ is CH₃ and Q is Q1, and R and R⁴ are as per any row of Table U below, which compounds are named I.A1-Z125-Q1-U-1 to I.A1-Z125-Q1-U-42.

In another embodiment, compounds I are of formula I.A1 wherein Z is Z-125, R³ is CH₃ and Q is Q2, and R and R⁴ are as per any row of Table U below, which compounds are named I.A1-Z125-Q2-U-1 to I.A1-Z125-Q2-U-42.

In another embodiment, compounds I are of formula I.A1 wherein Z is Z-125, R³ is CH₃ and Q is Q3, and R and R⁴ are as per any row of Table U below, which compounds are named I.A1-Z125-Q3-U-1 to I.A1-Z125-Q3-U-42.

In another embodiment, compounds I are of formula I.A1 wherein Z is Z-125, R³ is CH₃ and Q is Q4, and R and R⁴ are as per any row of Table U below, which compounds are named I.A1-Z125-Q4-U-1 to I.A1-Z125-Q4-U-42.

In another embodiment, compounds I are of formula I.A2 wherein Z is Z-125, R³ is CH₃ and Q is Q1, and R and R⁴ are as per any row of Table U below, which compounds are named I.A2-Z125-Q1-U-1 to I.A2-Z125-Q1-U-42.

In another embodiment, compounds I are of formula I.A2 wherein Z is Z-125, R³ is CH₃ and Q is Q2, and R and R⁴ are as per any row of Table U below, which compounds are named I.A2-Z125-Q2-U-1 to I.A2-Z125-Q2-U-42.

In another embodiment, compounds I are of formula I.A2 wherein Z is Z-125, R³ is CH₃ and Q is Q3, and R and R⁴ are as per any row of Table U below, which compounds are named I.A2-Z125-Q3-U-1 to I.A2-Z125-Q3-U-42.

In another embodiment, compounds I are of formula I.A2 wherein Z is Z-125, R³ is CH₃ and Q is Q4, and R and R⁴ are as per any row of Table U below, which compounds are named I.A2-Z125-Q4-U-1 to I.A2-Z125-Q4-U-42.

In another embodiment, compounds I are of formula I.A1 wherein Z is Z-126, R³ is CH₃ and Q is Q1, and R and R⁴ are as per any row of Table U below, which compounds are named I.A1-Z126-Q1-U-1 to I.A1-Z126-Q1-U-42.

In another embodiment, compounds I are of formula I.A1 wherein Z is Z-126, R³ is CH₃ and Q is Q2, and R and R⁴ are as per any row of Table U below, which compounds are named I.A1-Z126-Q2-U-1 to I.A1-Z126-Q2-U-42.

In another embodiment, compounds I are of formula I.A1 wherein Z is Z-126, R³ is CH₃ and Q is Q3, and R and R⁴ are as per any row of Table U below, which compounds are named I.A1-Z126-Q3-U-1 to I.A1-Z126-Q3-U-42.

In another embodiment, compounds I are of formula I.A1 wherein Z is Z-126, R³ is CH₃ and Q is Q4, and R and R⁴ are as per any row of Table U below, which compounds are named I.A1-Z126-Q4-U-1 to I.A1-Z126-Q4-U-42.

In another embodiment, compounds I are of formula I.A2 wherein Z is Z-126, R³ is CH₃ and Q is Q1, and R and R⁴ are as per any row of Table U below, which compounds are named I.A2-Z126-Q1-U-1 to I.A2-Z126-Q1-U-42.

In another embodiment, compounds I are of formula I.A2 wherein Z is Z-126, R³ is CH₃ and Q is Q2, and R and R⁴ are as per any row of Table U below, which compounds are named I.A2-Z126-Q2-U-1 to I.A2-Z126-Q2-U-42.

In another embodiment, compounds I are of formula I.A2 wherein Z is Z-126, R³ is CH₃ and Q is Q3, and R and R⁴ are as per any row of Table U below, which compounds are named I.A2-Z126-Q3-U-1 to I.A2-Z126-Q3-U-42.

In another embodiment, compounds I are of formula I.A2 wherein Z is Z-126, R³ is CH₃ and Q is Q4, and R and R⁴ are as per any row of Table U below, which compounds are named I.A2-Z126-Q4-U-1 to I.A2-Z126-Q4-U-42.

In another embodiment, compounds I are of formula I.A1 wherein Z is Z-127, R³ is CH₃ and Q is Q1, and R and R⁴ are as per any row of Table U below, which compounds are named I.A1-Z127-Q1-U-1 to I.A1-Z127-Q1-U-42.

In another embodiment, compounds I are of formula I.A1 wherein Z is Z-127, R³ is CH₃ and Q is Q2, and R and R⁴ are as per any row of Table U below, which compounds are named I.A1-Z127-Q2-U-1 to I.A1-Z127-Q2-U-42.

In another embodiment, compounds I are of formula I.A1 wherein Z is Z-127, R³ is CH₃ and Q is Q3, and R and R⁴ are as per any row of Table U below, which compounds are named I.A1-Z127-Q3-U-1 to I.A1-Z127-Q3-U-42.

In another embodiment, compounds I are of formula I.A1 wherein Z is Z-127, R³ is CH₃ and Q is Q4, and R and R⁴ are as per any row of Table U below, which compounds are named I.A1-Z127-Q4-U-1 to I.A1-Z127-Q4-U-42.

In another embodiment, compounds I are of formula I.A2 wherein Z is Z-127, R³ is CH₃ and Q is Q1, and R and R⁴ are as per any row of Table U below, which compounds are named I.A2-Z127-Q1-U-1 to I.A2-Z127-Q1-U-42.

In another embodiment, compounds I are of formula I.A2 wherein Z is Z-127, R³ is CH₃ and Q is Q2, and R and R⁴ are as per any row of Table U below, which compounds are named I.A2-Z127-Q2-U-1 to I.A2-Z127-Q2-U-42.

In another embodiment, compounds I are of formula I.A2 wherein Z is Z-127, R³ is CH₃ and Q is Q3, and R and R⁴ are as per any row of Table U below, which compounds are named I.A2-Z127-Q3-U-1 to I.A2-Z127-Q3-U-42.

In another embodiment, compounds I are of formula I.A2 wherein Z is Z-127, R³ is CH₃ and Q is Q4, and R and R⁴ are as per any row of Table U below, which compounds are named I.A2-Z127-Q4-U-1 to I.A2-Z127-Q4-U-42.

In another embodiment, compounds I are of formula I.A1 wherein Z is Z-128, R³ is CH₃ and Q is Q1, and R and R⁴ are as per any row of Table U below, which compounds are named I.A1-Z128-Q1-U-1 to I.A1-Z128-Q1-U-42.

In another embodiment, compounds I are of formula I.A1 wherein Z is Z-128, R³ is CH₃ and Q is Q2, and R and R⁴ are as per any row of Table U below, which compounds are named I.A1-Z128-Q2-U-1 to I.A1-Z128-Q2-U-42.

In another embodiment, compounds I are of formula I.A1 wherein Z is Z-128, R³ is CH₃ and Q is Q3, and R and R⁴ are as per any row of Table U below, which compounds are named I.A1-Z128-Q3-U-1 to I.A1-Z128-Q3-U-42.

In another embodiment, compounds I are of formula I.A1 wherein Z is Z-128, R³ is CH₃ and Q is Q4, and R and R⁴ are as per any row of Table U below, which compounds are named I.A1-Z128-Q4-U-1 to I.A1-Z128-Q4-U-42.

In another embodiment, compounds I are of formula I.A2 wherein Z is Z-128, R³ is CH₃ and Q is Q1, and R and R⁴ are as per any row of Table U below, which compounds are named I.A2-Z128-Q1-U-1 to I.A2-Z128-Q1-U-42.

In another embodiment, compounds I are of formula I.A2 wherein Z is Z-128, R³ is CH₃ and Q is Q2, and R and R⁴ are as per any row of Table U below, which compounds are named I.A2-Z128-Q2-U-1 to I.A2-Z128-Q2-U-42.

In another embodiment, compounds I are of formula I.A2 wherein Z is Z-128, R³ is CH₃ and Q is Q3, and R and R⁴ are as per any row of Table U below, which compounds are named I.A2-Z128-Q3-U-1 to I.A2-Z128-Q3-U-42.

In another embodiment, compounds I are of formula I.A2 wherein Z is Z-128, R³ is CH₃ and Q is Q4, and R and R⁴ are as per any row of Table U below, which compounds are named I.A2-Z128-Q4-U-1 to I.A2-Z128-Q4-U-42.

In another embodiment, compounds I are of formula I.A1 wherein Z is Z-129, R³ is CH₃ and Q is Q1, and R and R⁴ are as per any row of Table U below, which compounds are named I.A1-Z129-Q1-U-1 to I.A1-Z129-Q1-U-42.

In another embodiment, compounds I are of formula I.A1 wherein Z is Z-129, R³ is CH₃ and Q is Q2, and R and R⁴ are as per any row of Table U below, which compounds are named I.A1-Z129-Q2-U-1 to I.A1-Z129-Q2-U-42.

In another embodiment, compounds I are of formula I.A1 wherein Z is Z-129, R³ is CH₃ and Q is Q3, and R and R⁴ are as per any row of Table U below, which compounds are named I.A1-Z129-Q3-U-1 to I.A1-Z129-Q3-U-42.

In another embodiment, compounds I are of formula I.A1 wherein Z is Z-129, R³ is CH₃ and Q is Q4, and R and R⁴ are as per any row of Table U below, which compounds are named I.A1-Z129-Q4-U-1 to I.A1-Z129-Q4-U-42.

In another embodiment, compounds I are of formula I.A2 wherein Z is Z-129, R³ is CH₃ and Q is Q1, and R and R⁴ are as per any row of Table U below, which compounds are named I.A2-Z129-Q1-U-1 to I.A2-Z129-Q1-U-42.

In another embodiment, compounds I are of formula I.A2 wherein Z is Z-129, R³ is CH₃ and Q is Q2, and R and R⁴ are as per any row of Table U below, which compounds are named I.A2-Z129-Q2-U-1 to I.A2-Z129-Q2-U-42.

In another embodiment, compounds I are of formula I.A2 wherein Z is Z-129, R³ is CH₃ and Q is Q3, and R and R⁴ are as per any row of Table U below, which compounds are named I.A2-Z129-Q3-U-1 to I.A2-Z129-Q3-U-42.

In another embodiment, compounds I are of formula I.A2 wherein Z is Z-129, R³ is CH₃ and Q is Q4, and R and R⁴ are as per any row of Table U below, which compounds are named I.A2-Z129-Q4-U-1 to I.A2-Z129-Q4-U-42.

In another embodiment, compounds I are of formula I.A1 wherein Z is Z-130, R³ is CH₃ and Q is Q1, and R and R⁴ are as per any row of Table U below, which compounds are named I.A1-Z130-Q1-U-1 to I.A1-Z130-Q1-U-42.

In another embodiment, compounds I are of formula I.A1 wherein Z is Z-130, R³ is CH₃ and Q is Q2, and R and R⁴ are as per any row of Table U below, which compounds are named I.A1-Z130-Q2-U-1 to lA1-Z130-Q2-U-42.

In another embodiment, compounds I are of formula I.A1 wherein Z is Z-130, R³ is CH₃ and Q is Q3, and R and R⁴ are as per any row of Table U below, which compounds are named I.A1-Z130-Q3-U-1 to I.Al-Zl30-Q3-U-42.

In another embodiment, compounds I are of formula I.A1 wherein Z is Z-130, R³ is CH₃ and Q is Q4, and R and R⁴ are as per any row of Table U below, which compounds are named I.A1-Z130-Q4-U-1 to lA1-Z130-Q4-U-42.

In another embodiment, compounds I are of formula I.A2 wherein Z is Z-130, R³ is CH₃ and Q is Q1, and R and R⁴ are as per any row of Table U below, which compounds are named I.A2-Z130-Q1-U-1 to I.A2-Z130-Q1-U-42.

In another embodiment, compounds I are of formula I.A2 wherein Z is Z-130, R³ is CH₃ and Q is Q2, and R and R⁴ are as per any row of Table U below, which compounds are named I.A2-Z130-Q2-U-1 to I.A2-Z130-Q2-U-42.

In another embodiment, compounds I are of formula I.A2 wherein Z is Z-130, R³ is CH₃ and Q is Q3, and R and R⁴ are as per any row of Table U below, which compounds are named I.A2-Z130-Q3-U-1 to I.A2-Z130-Q3-U-42.

In another embodiment, compounds I are of formula I.A2 wherein Z is Z-130, R³ is CH₃ and Q is Q4, and R and R⁴ are as per any row of Table U below, which compounds are named I.A2-Z130-Q4-U-1 to I.A2-Z130-Q4-U-42.

In another embodiment, compounds I are of formula I.A1 wherein Z is Z-131, R³ is CH₃ and Q is Q1, and R and R⁴ are as per any row of Table U below, which compounds are named I.A1-Z131-Q1-U-1 to I.A1-Z131-Q1-U-42.

In another embodiment, compounds I are of formula I.A1 wherein Z is Z-131, R³ is CH₃ and Q is Q2, and R and R⁴ are as per any row of Table U below, which compounds are named I.A1-Z131-Q2-U-1 to I.A1-Z131-Q2-U-42.

In another embodiment, compounds I are of formula I.A1 wherein Z is Z-131, R³ is CH₃ and Q is Q3, and R and R⁴ are as per any row of Table U below, which compounds are named I.A1-Z131-Q3-U-1 to I.A1-Z131-Q3-U-42.

In another embodiment, compounds I are of formula I.A1 wherein Z is Z-131, R³ is CH₃ and Q is Q4, and R and R⁴ are as per any row of Table U below, which compounds are named I.A1-Z131-Q4-U-1 to I.A1-Z131-Q4-U-42.

In another embodiment, compounds I are of formula I.A2 wherein Z is Z-131, R³ is CH₃ and Q is Q1, and R and R⁴ are as per any row of Table U below, which compounds are named I.A2-Z131-Q1-U-1 to I.A2-Z131-Q1-U-42.

In another embodiment, compounds I are of formula I.A2 wherein Z is Z-131, R³ is CH₃ and Q is Q2, and R and R⁴ are as per any row of Table U below, which compounds are named I.A2-Z131-Q2-U-1 to I.A2-Z131-Q2-U-42.

In another embodiment, compounds I are of formula I.A2 wherein Z is Z-131, R³ is CH₃ and Q is Q3, and R and R⁴ are as per any row of Table U below, which compounds are named I.A2-Z131-Q3-U-1 to I.A2-Z131-Q3-U-42.

In another embodiment, compounds I are of formula I.A2 wherein Z is Z-131, R³ is CH₃ and Q is Q4, and R and R⁴ are as per any row of Table U below, which compounds are named I.A2-Z131-Q4-U-1 to I.A2-Z131-Q4-U-42.

In another embodiment, compounds I are of formula I.A1 wherein Z is Z-132, R³ is CH₃ and Q is Q1, and R and R⁴ are as per any row of Table U below, which compounds are named I.A1-Z132-Q1-U-1 to I.A1-Z132-Q1-U-42.

In another embodiment, compounds I are of formula I.A1 wherein Z is Z-132, R³ is CH₃ and Q is Q2, and R and R⁴ are as per any row of Table U below, which compounds are named I.A1-Z132-Q2-U-1 to lA1-Z132-Q2-U-42.

In another embodiment, compounds I are of formula I.A1 wherein Z is Z-132, R³ is CH₃ and Q is Q3, and R and R⁴ are as per any row of Table U below, which compounds are named I.A1-Z132-Q3-U-1 to I.A 1-Z132-Q3-U-42.

In another embodiment, compounds I are of formula I.A1 wherein Z is Z-132, R³ is CH₃ and Q is Q4, and R and R⁴ are as per any row of Table U below, which compounds are named I.A1-Z132-Q4-U-1 to lA1-Z132-Q4-U-42.

In another embodiment, compounds I are of formula I.A2 wherein Z is Z-132, R³ is CH₃ and Q is Q1, and R and R⁴ are as per any row of Table U below, which compounds are named I.A2-Z132-Q1-U-1 to I.A2-Z132-Q1-U-42.

In another embodiment, compounds I are of formula I.A2 wherein Z is Z-132, R³ is CH₃ and Q is Q2, and R and R⁴ are as per any row of Table U below, which compounds are named I.A2-Z132-Q2-U-1 to I.A2-Z132-Q2-U-42.

In another embodiment, compounds I are of formula I.A2 wherein Z is Z-132, R³ is CH₃ and Q is Q3, and R and R⁴ are as per any row of Table U below, which compounds are named I.A2-Z132-Q3-U-1 to I.A2-Z132-Q3-U-42.

In another embodiment, compounds I are of formula I.A2 wherein Z is Z-132, R³ is CH₃ and Q is Q4, and R and R⁴ are as per any row of Table U below, which compounds are named I.A2-Z132-Q4-U-1 to I.A2-Z132-Q4-U-42.

In another embodiment, compounds I are of formula I.A1 wherein Z is Z-133, R³ is CH₃ and Q is Q1, and R and R⁴ are as per any row of Table U below, which compounds are named I.A1-Z133-Q1-U-1 to I.A1-Z133-Q1-U-42.

In another embodiment, compounds I are of formula I.A1 wherein Z is Z-133, R³ is CH₃ and Q is Q2, and R and R⁴ are as per any row of Table U below, which compounds are named I.A1-Z133-Q2-U-1 to lA1-Z133-Q2-U-42.

In another embodiment, compounds I are of formula I.A1 wherein Z is Z-133, R³ is CH₃ and Q is Q3, and R and R⁴ are as per any row of Table U below, which compounds are named I.A1-Z133-Q3-U-1 to I.A1-Z133-Q3-U-42.

In another embodiment, compounds I are of formula I.A1 wherein Z is Z-133, R³ is CH₃ and Q is Q4, and R and R⁴ are as per any row of Table U below, which compounds are named I.A1-Z133-Q4-U-1 to lA1-Z133-Q4-U-42.

In another embodiment, compounds I are of formula I.A2 wherein Z is Z-133, R³ is CH₃ and Q is Q1, and R and R⁴ are as per any row of Table U below, which compounds are named I.A2-Z133-Q1-U-1 to I.A2-Z133-Q1-U-42.

In another embodiment, compounds I are of formula I.A2 wherein Z is Z-133, R³ is CH₃ and Q is Q2, and R and R⁴ are as per any row of Table U below, which compounds are named I.A2-Z133-Q2-U-1 to I.A2-Z133-Q2-U-42.

In another embodiment, compounds I are of formula I.A2 wherein Z is Z-133, R³ is CH₃ and Q is Q3, and R and R⁴ are as per any row of Table U below, which compounds are named I.A2-Z133-Q3-U-1 to I.A2-Z133-Q3-U-42.

In another embodiment, compounds I are of formula I.A2 wherein Z is Z-133, R³ is CH₃ and Q is Q4, and R and R⁴ are as per any row of Table U below, which compounds are named I.A2-Z133-Q4-U-1 to I.A2-Z133-Q4-U-42.

In another embodiment, compounds I are of formula I.A1 wherein Z is Z-134, R³ is CH₃ and Q is Q1, and R and R⁴ are as per any row of Table U below, which compounds are named I.A1-Z134-Q1-U-1 to I.A1-Z134-Q1-U-42.

In another embodiment, compounds I are of formula I.A1 wherein Z is Z-134, R³ is CH₃ and Q is Q2, and R and R⁴ are as per any row of Table U below, which compounds are named I.A1-Z134-Q2-U-1 to I.A1-Z134-Q2-U-42.

In another embodiment, compounds I are of formula I.A1 wherein Z is Z-134, R³ is CH₃ and Q is Q3, and R and R⁴ are as per any row of Table U below, which compounds are named I.A1-Z134-Q3-U-1 to I.A 1-Z134-Q3-U-42.

In another embodiment, compounds I are of formula I.A1 wherein Z is Z-134, R³ is CH₃ and Q is Q4, and R and R⁴ are as per any row of Table U below, which compounds are named I.A1-Z134-Q4-U-1 to I.A1-Z134-Q4-U-42.

In another embodiment, compounds I are of formula I.A2 wherein Z is Z-134, R³ is CH₃ and Q is Q1, and R and R⁴ are as per any row of Table U below, which compounds are named I.A2-Z134-Q1-U-1 to I.A2-Z134-Q1-U-42.

In another embodiment, compounds I are of formula I.A2 wherein Z is Z-134, R³ is CH₃ and Q is Q2, and R and R⁴ are as per any row of Table U below, which compounds are named I.A2-Z134-Q2-U-1 to I.A2-Z134-Q2-U-42.

In another embodiment, compounds I are of formula I.A2 wherein Z is Z-134, R³ is CH₃ and Q is Q3, and R and R⁴ are as per any row of Table U below, which compounds are named I.A2-Z134-Q3-U-1 to I.A2-Z134-Q3-U-42.

In another embodiment, compounds I are of formula I.A2 wherein Z is Z-134, R³ is CH₃ and Q is Q4, and R and R⁴ are as per any row of Table U below, which compounds are named I.A2-Z134-Q4-U-1 to I.A2-Z134-Q4-U-42.

In another embodiment, compounds I are of formula I.A1 wherein Z is Z-135, R³ is CH₃ and Q is Q1, and R and R⁴ are as per any row of Table U below, which compounds are named I.A1-Z135-Q1-U-1 to I.A1-Z135-Q1-U-42.

In another embodiment, compounds I are of formula I.A1 wherein Z is Z-135, R³ is CH₃ and Q is Q2, and R and R⁴ are as per any row of Table U below, which compounds are named I.A1-Z135-Q2-U-1 to I.A1-Z135-Q2-U-42.

In another embodiment, compounds I are of formula I.A1 wherein Z is Z-135, R³ is CH₃ and Q is Q3, and R and R⁴ are as per any row of Table U below, which compounds are named I.A1-Z135-Q3-U-1 to I.A1-Z135-Q3-U-42.

In another embodiment, compounds I are of formula I.A1 wherein Z is Z-135, R³ is CH₃ and Q is Q4, and R and R⁴ are as per any row of Table U below, which compounds are named I.A1-Z135-Q4-U-1 to I.A1-Z135-Q4-U-42.

In another embodiment, compounds I are of formula I.A2 wherein Z is Z-135, R³ is CH₃ and Q is Q1, and R and R⁴ are as per any row of Table U below, which compounds are named I.A2-Z135-Q1-U-1 to I.A2-Z135-Q1-U-42.

In another embodiment, compounds I are of formula I.A2 wherein Z is Z-135, R³ is CH₃ and Q is Q2, and R and R⁴ are as per any row of Table U below, which compounds are named I.A2-Z135-Q2-U-1 to I.A2-Z135-Q2-U-42.

In another embodiment, compounds I are of formula I.A2 wherein Z is Z-135, R³ is CH₃ and Q is Q3, and R and R⁴ are as per any row of Table U below, which compounds are named I.A2-Z135-Q3-U-1 to I.A2-Z135-Q3-U-42.

In another embodiment, compounds I are of formula I.A2 wherein Z is Z-135, R³ is CH₃ and Q is Q4, and R and R⁴ are as per any row of Table U below, which compounds are named I.A2-Z135-Q4-U-1 to I.A2-Z135-Q4-U-42.

In another embodiment, compounds I are of formula I.A1 wherein Z is Z-136, R³ is CH₃ and Q is Q1, and R and R⁴ are as per any row of Table U below, which compounds are named I.A1-Z136-Q1-U-1 to I.A1-Z136-Q1-U-42.

In another embodiment, compounds I are of formula I.A1 wherein Z is Z-136, R³ is CH₃ and Q is Q2, and R and R⁴ are as per any row of Table U below, which compounds are named I.A1-Z136-Q2-U-1 to I.A1-Z136-Q2-U-42.

In another embodiment, compounds I are of formula I.A1 wherein Z is Z-136, R³ is CH₃ and Q is Q3, and R and R⁴ are as per any row of Table U below, which compounds are named I.A1-Z136-Q3-U-1 to I.A1-Z136-Q3-U-42.

In another embodiment, compounds I are of formula I.A1 wherein Z is Z-136, R³ is CH₃ and Q is Q4, and R and R⁴ are as per any row of Table U below, which compounds are named I.A1-Z136-Q4-U-1 to I.A1-Z136-Q4-U-42.

In another embodiment, compounds I are of formula I.A2 wherein Z is Z-136, R³ is CH₃ and Q is Q1, and R and R⁴ are as per any row of Table U below, which compounds are named I.A2-Z136-Q1-U-1 to I.A2-Z136-Q1-U-42.

In another embodiment, compounds I are of formula I.A2 wherein Z is Z-136, R³ is CH₃ and Q is Q2, and R and R⁴ are as per any row of Table U below, which compounds are named I.A2-Z136-Q2-U-1 to I.A2-Z136-Q2-U-42.

In another embodiment, compounds I are of formula I.A2 wherein Z is Z-136, R³ is CH₃ and Q is Q3, and R and R⁴ are as per any row of Table U below, which compounds are named I.A2-Z136-Q3-U-1 to I.A2-Z136-Q3-U-42.

In another embodiment, compounds I are of formula I.A2 wherein Z is Z-136, R³ is CH₃ and Q is Q4, and R and R⁴ are as per any row of Table U below, which compounds are named I.A2-Z136-Q4-U-1 to I.A2-Z136-Q4-U-42.

In another embodiment, compounds I are of formula I.A1 wherein Z is Z-137, R³ is CH₃ and Q is Q1, and R and R⁴ are as per any row of Table U below, which compounds are named I.A1-Z137-Q1-U-1 to I.A1-Z137-Q1-U-42.

In another embodiment, compounds I are of formula I.A1 wherein Z is Z-137, R³ is CH₃ and Q is Q2, and R and R⁴ are as per any row of Table U below, which compounds are named I.A1-Z137-Q2-U-1 to I.A1-Z137-Q2-U-42.

In another embodiment, compounds I are of formula I.A1 wherein Z is Z-137, R³ is CH₃ and Q is Q3, and R and R⁴ are as per any row of Table U below, which compounds are named I.A1-Z137-Q3-U-1 to I.A1-Z137-Q3-U-42.

In another embodiment, compounds I are of formula I.A1 wherein Z is Z-137, R³ is CH₃ and Q is Q4, and R and R⁴ are as per any row of Table U below, which compounds are named I.A1-Z137-Q4-U-1 to I.A1-Z137-Q4-U-42.

In another embodiment, compounds I are of formula I.A2 wherein Z is Z-137, R³ is CH₃ and Q is Q1, and R and R⁴ are as per any row of Table U below, which compounds are named I.A2-Z137-Q1-U-1 to I.A2-Z137-Q1-U-42.

In another embodiment, compounds I are of formula I.A2 wherein Z is Z-137, R³ is CH₃ and Q is Q2, and R and R⁴ are as per any row of Table U below, which compounds are named I.A2-Z137-Q2-U-1 to I.A2-Z137-Q2-U-42.

In another embodiment, compounds I are of formula I.A2 wherein Z is Z-137, R³ is CH₃ and Q is Q3, and R and R⁴ are as per any row of Table U below, which compounds are named I.A2-Z137-Q3-U-1 to I.A2-Z137-Q3-U-42.

In another embodiment, compounds I are of formula I.A2 wherein Z is Z-137, R³ is CH₃ and Q is Q4, and R and R⁴ are as per any row of Table U below, which compounds are named I.A2-Z137-Q4-U-1 to I.A2-Z137-Q4-U-42.

In another embodiment, compounds I are of formula I.A1 wherein Z is Z-138, R³ is CH₃ and Q is Q1, and R and R⁴ are as per any row of Table U below, which compounds are named I.A1-Z138-Q1-U-1 to I.A1-Z138-Q1-U-42.

In another embodiment, compounds I are of formula I.A1 wherein Z is Z-138, R³ is CH₃ and Q is Q2, and R and R⁴ are as per any row of Table U below, which compounds are named I.A1-Z138-Q2-U-1 to I.A1-Z138-Q2-U-42.

In another embodiment, compounds I are of formula I.A1 wherein Z is Z-138, R³ is CH₃ and Q is Q3, and R and R⁴ are as per any row of Table U below, which compounds are named I.A1-Z138-Q3-U-1 to I.A1-Z138-Q3-U-42.

In another embodiment, compounds I are of formula I.A1 wherein Z is Z-138, R³ is CH₃ and Q is Q4, and R and R⁴ are as per any row of Table U below, which compounds are named I.A1-Z138-Q4-U-1 to I.A1-Z138-Q4-U-42.

In another embodiment, compounds I are of formula I.A2 wherein Z is Z-138, R³ is CH₃ and Q is Q1, and R and R⁴ are as per any row of Table U below, which compounds are named I.A2-Z138-Q1-U-1 to I.A2-Z138-Q1-U-42.

In another embodiment, compounds I are of formula I.A2 wherein Z is Z-138, R³ is CH₃ and Q is Q2, and R and R⁴ are as per any row of Table U below, which compounds are named I.A2-Z138-Q2-U-1 to I.A2-Z138-Q2-U-42.

In another embodiment, compounds I are of formula I.A2 wherein Z is Z-138, R³ is CH₃ and Q is Q3, and R and R⁴ are as per any row of Table U below, which compounds are named I.A2-Z138-Q3-U-1 to I.A2-Z138-Q3-U-42.

In another embodiment, compounds I are of formula I.A2 wherein Z is Z-138, R³ is CH₃ and Q is Q4, and R and R⁴ are as per any row of Table U below, which compounds are named I.A2-Z138-Q4-U-1 to I.A2-Z138-Q4-U-42.

In another embodiment, compounds I are of formula I.A1 wherein Z is Z-139, R³ is CH₃ and Q is Q1, and R and R⁴ are as per any row of Table U below, which compounds are named I.A1-Z139-Q1-U-1 to I.A1-Z139-Q1-U-42.

In another embodiment, compounds I are of formula I.A1 wherein Z is Z-139, R³ is CH₃ and Q is Q2, and R and R⁴ are as per any row of Table U below, which compounds are named I.A1-Z139-Q2-U-1 to I.A1-Z139-Q2-U-42.

In another embodiment, compounds I are of formula I.A1 wherein Z is Z-139, R³ is CH₃ and Q is Q3, and R and R⁴ are as per any row of Table U below, which compounds are named I.A1-Z139-Q3-U-1 to I.A1-Z139-Q3-U-42.

In another embodiment, compounds I are of formula I.A1 wherein Z is Z-139, R³ is CH₃ and Q is Q4, and R and R⁴ are as per any row of Table U below, which compounds are named I.A1-Z139-Q4-U-1 to I.A1-Z139-Q4-U-42.

In another embodiment, compounds I are of formula I.A2 wherein Z is Z-139, R³ is CH₃ and Q is Q1, and R and R⁴ are as per any row of Table U below, which compounds are named I.A2-Z139-Q1-U-1 to I.A2-Z139-Q1-U-42.

In another embodiment, compounds I are of formula I.A2 wherein Z is Z-139, R³ is CH₃ and Q is Q2, and R and R⁴ are as per any row of Table U below, which compounds are named I.A2-Z139-Q2-U-1 to I.A2-Z139-Q2-U-42.

In another embodiment, compounds I are of formula I.A2 wherein Z is Z-139, R³ is CH₃ and Q is Q3, and R and R⁴ are as per any row of Table U below, which compounds are named I.A2-Z139-Q3-U-1 to I.A2-Z139-Q3-U-42.

In another embodiment, compounds I are of formula I.A2 wherein Z is Z-139, R³ is CH₃ and Q is Q4, and R and R⁴ are as per any row of Table U below, which compounds are named I.A2-Z139-Q4-U-1 to I.A2-Z139-Q4-U-42.

In another embodiment, compounds I are of formula I.A1 wherein Z is Z-140, R³ is CH₃ and Q is Q1, and R and R⁴ are as per any row of Table U below, which compounds are named I.A1-Z140-Q1-U-1 to I.A1-Z140-Q1-U-42.

In another embodiment, compounds I are of formula I.A1 wherein Z is Z-140, R³ is CH₃ and Q is Q2, and R and R⁴ are as per any row of Table U below, which compounds are named I.A1-Z140-Q2-U-1 to I.A1-Z140-Q2-U-42.

In another embodiment, compounds I are of formula I.A1 wherein Z is Z-140, R³ is CH₃ and Q is Q3, and R and R⁴ are as per any row of Table U below, which compounds are named I.A1-Z140-Q3-U-1 to I.A1-Z140-Q3-U-42.

In another embodiment, compounds I are of formula I.A1 wherein Z is Z-140, R³ is CH₃ and Q is Q4, and R and R⁴ are as per any row of Table U below, which compounds are named I.A1-Z140-Q4-U-1 to I.A1-Z140-Q4-U-42.

In another embodiment, compounds I are of formula I.A2 wherein Z is Z-140, R³ is CH₃ and Q is Q1, and R and R⁴ are as per any row of Table U below, which compounds are named I.A2-Z140-Q1-U-1 to I.A2-Z140-Q1-U-42.

In another embodiment, compounds I are of formula I.A2 wherein Z is Z-140, R³ is CH₃ and Q is Q2, and R and R⁴ are as per any row of Table U below, which compounds are named I.A2-Z140-Q2-U-1 to I.A2-Z140-Q2-U-42.

In another embodiment, compounds I are of formula I.A2 wherein Z is Z-140, R³ is CH₃ and Q is Q3, and R and R⁴ are as per any row of Table U below, which compounds are named I.A2-Z140-Q3-U-1 to I.A2-Z140-Q3-U-42.

In another embodiment, compounds I are of formula I.A2 wherein Z is Z-140, R³ is CH₃ and Q is Q4, and R and R⁴ are as per any row of Table U below, which compounds are named I.A2-Z140-Q4-U-1 to I.A2-Z140-Q4-U-42.

In another embodiment, compounds I are of formula I.A1 wherein Z is Z-141, R³ is CH₃ and Q is Q1, and R and R⁴ are as per any row of Table U below, which compounds are named I.A1-Z141-Q1-U-1 to I.A1-Z141-Q1-U-42.

In another embodiment, compounds I are of formula I.A1 wherein Z is Z-141, R³ is CH₃ and Q is Q2, and R and R⁴ are as per any row of Table U below, which compounds are named I.A1-Z141-Q2-U-1 to I.A1-Z141-Q2-U-42.

In another embodiment, compounds I are of formula I.A1 wherein Z is Z-141, R³ is CH₃ and Q is Q3, and R and R⁴ are as per any row of Table U below, which compounds are named I.A1-Z141-Q3-U-1 to I.A1-Z141-Q3-U-42.

In another embodiment, compounds I are of formula I.A1 wherein Z is Z-141, R³ is CH₃ and Q is Q4, and R and R⁴ are as per any row of Table U below, which compounds are named I.A1-Z141-Q4-U-1 to I.A1-Z141-Q4-U-42.

In another embodiment, compounds I are of formula I.A2 wherein Z is Z-141, R³ is CH₃ and Q is Q1, and R and R⁴ are as per any row of Table U below, which compounds are named I.A2-Z141-Q1-U-1 to I.A2-Z141-Q1-U-42.

In another embodiment, compounds I are of formula I.A2 wherein Z is Z-141, R³ is CH₃ and Q is Q2, and R and R⁴ are as per any row of Table U below, which compounds are named I.A2-Z141-Q2-U-1 to I.A2-Z141-Q2-U-42.

In another embodiment, compounds I are of formula I.A2 wherein Z is Z-141, R³ is CH₃ and Q is Q3, and R and R⁴ are as per any row of Table U below, which compounds are named I.A2-Z141-Q3-U-1 to I.A2-Z141-Q3-U-42.

In another embodiment, compounds I are of formula I.A2 wherein Z is Z-141, R³ is CH₃ and Q is Q4, and R and R⁴ are as per any row of Table U below, which compounds are named I.A2-Z141-Q4-U-1 to I.A2-Z141-Q4-U-42.

In another embodiment, compounds I are of formula I.A1 wherein Z is Z-142, R³ is CH₃ and Q is Q1, and R and R⁴ are as per any row of Table U below, which compounds are named I.A1-Z142-Q1-U-1 to I.A1-Z142-Q1-U-42.

In another embodiment, compounds I are of formula I.A1 wherein Z is Z-142, R³ is CH₃ and Q is Q2, and R and R⁴ are as per any row of Table U below, which compounds are named I.A1-Z142-Q2-U-1 to I.A1-Z142-Q2-U-42.

In another embodiment, compounds I are of formula I.A1 wherein Z is Z-142, R³ is CH₃ and Q is Q3, and R and R⁴ are as per any row of Table U below, which compounds are named I.A1-Z142-Q3-U-1 to I.A1-Z142-Q3-U-42.

In another embodiment, compounds I are of formula I.A1 wherein Z is Z-142, R³ is CH₃ and Q is Q4, and R and R⁴ are as per any row of Table U below, which compounds are named I.A1-Z142-Q4-U-1 to I.A1-Z142-Q4-U-42.

In another embodiment, compounds I are of formula I.A2 wherein Z is Z-142, R³ is CH₃ and Q is Q1, and R and R⁴ are as per any row of Table U below, which compounds are named I.A2-Z142-Q1-U-1 to I.A2-Z142-Q1-U-42.

In another embodiment, compounds I are of formula I.A2 wherein Z is Z-142, R³ is CH₃ and Q is Q2, and R and R⁴ are as per any row of Table U below, which compounds are named I.A2-Z142-Q2-U-1 to I.A2-Z142-Q2-U-42.

In another embodiment, compounds I are of formula I.A2 wherein Z is Z-142, R³ is CH₃ and Q is Q3, and R and R⁴ are as per any row of Table U below, which compounds are named I.A2-Z142-Q3-U-1 to I.A2-Z142-Q3-U-42.

In another embodiment, compounds I are of formula I.A2 wherein Z is Z-142, R³ is CH₃ and Q is Q4, and R and R⁴ are as per any row of Table U below, which compounds are named I.A2-Z142-Q4-U-1 to I.A2-Z142-Q4-U-42.

In another embodiment, compounds I are of formula I.A1 wherein Z is Z-143, R³ is CH₃ and Q is Q1, and R and R⁴ are as per any row of Table U below, which compounds are named I.A1-Z143-Q1-U-1 to I.A1-Z143-Q1-U-42.

In another embodiment, compounds I are of formula I.A1 wherein Z is Z-143, R³ is CH₃ and Q is Q2, and R and R⁴ are as per any row of Table U below, which compounds are named I.A1-Z143-Q2-U-1 to I.A1-Z143-Q2-U-42.

In another embodiment, compounds I are of formula I.A1 wherein Z is Z-143, R³ is CH₃ and Q is Q3, and R and R⁴ are as per any row of Table U below, which compounds are named I.A1-Z143-Q3-U-1 to I.A1-Z143-Q3-U-42.

In another embodiment, compounds I are of formula I.A1 wherein Z is Z-143, R³ is CH₃ and Q is Q4, and R and R⁴ are as per any row of Table U below, which compounds are named I.A1-Z143-Q4-U-1 to I.A1-Z143-Q4-U-42.

In another embodiment, compounds I are of formula I.A2 wherein Z is Z-143, R³ is CH₃ and Q is Q1, and R and R⁴ are as per any row of Table U below, which compounds are named I.A2-Z143-Q1-U-1 to I.A2-Z143-Q1-U-42.

In another embodiment, compounds I are of formula I.A2 wherein Z is Z-143, R³ is CH₃ and Q is Q2, and R and R⁴ are as per any row of Table U below, which compounds are named I.A2-Z143-Q2-U-1 to I.A2-Z143-Q2-U-42.

In another embodiment, compounds I are of formula I.A2 wherein Z is Z-143, R³ is CH₃ and Q is Q3, and R and R⁴ are as per any row of Table U below, which compounds are named I.A2-Z143-Q3-U-1 to I.A2-Z143-Q3-U-42.

In another embodiment, compounds I are of formula I.A2 wherein Z is Z-143, R³ is CH₃ and Q is Q4, and R and R⁴ are as per any row of Table U below, which compounds are named I.A2-Z143-Q4-U-1 to I.A2-Z143-Q4-U-42.

In another embodiment, compounds I are of formula I.A1 wherein Z is Z-144, R³ is CH₃ and Q is Q1, and R and R⁴ are as per any row of Table U below, which compounds are named I.A1-Z144-Q1-U-1 to I.A1-Z144-Q1-U-42.

In another embodiment, compounds I are of formula I.A1 wherein Z is Z-144, R³ is CH₃ and Q is Q2, and R and R⁴ are as per any row of Table U below, which compounds are named I.A1-Z144-Q2-U-1 to I.A1-Z144-Q2-U-42.

In another embodiment, compounds I are of formula I.A1 wherein Z is Z-144, R³ is CH₃ and Q is Q3, and R and R⁴ are as per any row of Table U below, which compounds are named I.A1-Z144-Q3-U-1 to I.A1-Z144-Q3-U-42.

In another embodiment, compounds I are of formula I.A1 wherein Z is Z-144, R³ is CH₃ and Q is Q4, and R and R⁴ are as per any row of Table U below, which compounds are named I.A1-Z144-Q4-U-1 to I.A1-Z144-Q4-U-42.

In another embodiment, compounds I are of formula I.A2 wherein Z is Z-144, R³ is CH₃ and Q is Q1, and R and R⁴ are as per any row of Table U below, which compounds are named I.A2-Z144-Q1-U-1 to I.A2-Z144-Q1-U-42.

In another embodiment, compounds I are of formula I.A2 wherein Z is Z-144, R³ is CH₃ and Q is Q2, and R and R⁴ are as per any row of Table U below, which compounds are named I.A2-Z144-Q2-U-1 to I.A2-Z144-Q2-U-42.

In another embodiment, compounds I are of formula I.A2 wherein Z is Z-144, R³ is CH₃ and Q is Q3, and R and R⁴ are as per any row of Table U below, which compounds are named I.A2-Z144-Q3-U-1 to I.A2-Z144-Q3-U-42.

In another embodiment, compounds I are of formula I.A2 wherein Z is Z-144, R³ is CH₃ and Q is Q4, and R and R⁴ are as per any row of Table U below, which compounds are named I.A2-Z144-Q4-U-1 to I.A2-Z144-Q4-U-42.

In another embodiment, compounds I are of formula I.A1 wherein Z is Z-145, R³ is CH₃ and Q is Q1, and R and R⁴ are as per any row of Table U below, which compounds are named I.A1-Z145-Q1-U-1 to I.A1-Z145-Q1-U-42.

In another embodiment, compounds I are of formula I.A1 wherein Z is Z-145, R³ is CH₃ and Q is Q2, and R and R⁴ are as per any row of Table U below, which compounds are named I.A1-Z145-Q2-U-1 to I.A1-Z145-Q2-U-42.

In another embodiment, compounds I are of formula I.A1 wherein Z is Z-145, R³ is CH₃ and Q is Q3, and R and R⁴ are as per any row of Table U below, which compounds are named I.A1-Z145-Q3-U-1 to I.A1-Z145-Q3-U-42.

In another embodiment, compounds I are of formula I.A1 wherein Z is Z-145, R³ is CH₃ and Q is Q4, and R and R⁴ are as per any row of Table U below, which compounds are named I.A1-Z145-Q4-U-1 to I.A1-Z145-Q4-U-42.

In another embodiment, compounds I are of formula I.A2 wherein Z is Z-145, R³ is CH₃ and Q is Q1, and R and R⁴ are as per any row of Table U below, which compounds are named I.A2-Z145-Q1-U-1 to I.A2-Z145-Q1-U-42.

In another embodiment, compounds I are of formula I.A2 wherein Z is Z-145, R³ is CH₃ and Q is Q2, and R and R⁴ are as per any row of Table U below, which compounds are named I.A2-Z145-Q2-U-1 to I.A2-Z145-Q2-U-42.

In another embodiment, compounds I are of formula I.A2 wherein Z is Z-145, R³ is CH₃ and Q is Q3, and R and R⁴ are as per any row of Table U below, which compounds are named I.A2-Z145-Q3-U-1 to I.A2-Z145-Q3-U-42.

In another embodiment, compounds I are of formula I.A2 wherein Z is Z-145, R³ is CH₃ and Q is Q4, and R and R⁴ are as per any row of Table U below, which compounds are named I.A2-Z145-Q4-U-1 to I.A2-Z145-Q4-U-42.

In another embodiment, compounds I are of formula I.A1 wherein Z is Z-146, R³ is CH₃ and Q is Q1, and R and R⁴ are as per any row of Table U below, which compounds are named I.A1-Z146-Q1-U-1 to I.A1-Z146-Q1-U-42.

In another embodiment, compounds I are of formula I.A1 wherein Z is Z-146, R³ is CH₃ and Q is Q2, and R and R⁴ are as per any row of Table U below, which compounds are named I.A1-Z146-Q2-U-1 to I.A1-Z146-Q2-U-42.

In another embodiment, compounds I are of formula I.A1 wherein Z is Z-146, R³ is CH₃ and Q is Q3, and R and R⁴ are as per any row of Table U below, which compounds are named I.A1-Z146-Q3-U-1 to I.A1-Z146-Q3-U-42.

In another embodiment, compounds I are of formula I.A1 wherein Z is Z-146, R³ is CH₃ and Q is Q4, and R and R⁴ are as per any row of Table U below, which compounds are named I.A1-Z146-Q4-U-1 to I.A1-Z146-Q4-U-42.

In another embodiment, compounds I are of formula I.A2 wherein Z is Z-146, R³ is CH₃ and Q is Q1, and R and R⁴ are as per any row of Table U below, which compounds are named I.A2-Z146-Q1-U-1 to I.A2-Z146-Q1-U-42.

In another embodiment, compounds I are of formula I.A2 wherein Z is Z-146, R³ is CH₃ and Q is Q2, and R and R⁴ are as per any row of Table U below, which compounds are named I.A2-Z146-Q2-U-1 to I.A2-Z146-Q2-U-42.

In another embodiment, compounds I are of formula I.A2 wherein Z is Z-146, R³ is CH₃ and Q is Q3, and R and R⁴ are as per any row of Table U below, which compounds are named I.A2-Z146-Q3-U-1 to I.A2-Z146-Q3-U-42.

In another embodiment, compounds I are of formula I.A2 wherein Z is Z-146, R³ is CH₃ and Q is Q4, and R and R⁴ are as per any row of Table U below, which compounds are named I.A2-Z146-Q4-U-1 to I.A2-Z146-Q4-U-42.

In another embodiment, compounds I are of formula I.A1 wherein Z is Z-147, R³ is CH₃ and Q is Q1, and R and R⁴ are as per any row of Table U below, which compounds are named I.A1-Z147-Q1-U-1 to I.A1-Z147-Q1-U-42.

In another embodiment, compounds I are of formula I.A1 wherein Z is Z-147, R³ is CH₃ and Q is Q2, and R and R⁴ are as per any row of Table U below, which compounds are named I.A1-Z147-Q2-U-1 to I.A1-Z147-Q2-U-42.

In another embodiment, compounds I are of formula I.A1 wherein Z is Z-147, R³ is CH₃ and Q is Q3, and R and R⁴ are as per any row of Table U below, which compounds are named I.A1-Z147-Q3-U-1 to I.A1-Z147-Q3-U-42.

In another embodiment, compounds I are of formula I.A1 wherein Z is Z-147, R³ is CH₃ and Q is Q4, and R and R⁴ are as per any row of Table U below, which compounds are named I.A1-Z147-Q4-U-1 to I.A1-Z147-Q4-U-42.

In another embodiment, compounds I are of formula I.A2 wherein Z is Z-147, R³ is CH₃ and Q is Q1, and R and R⁴ are as per any row of Table U below, which compounds are named I.A2-Z147-Q1-U-1 to I.A2-Z147-Q1-U-42.

In another embodiment, compounds I are of formula I.A2 wherein Z is Z-147, R³ is CH₃ and Q is Q2, and R and R⁴ are as per any row of Table U below, which compounds are named I.A2-Z147-Q2-U-1 to I.A2-Z147-Q2-U-42.

In another embodiment, compounds I are of formula I.A2 wherein Z is Z-147, R³ is CH₃ and Q is Q3, and R and R⁴ are as per any row of Table U below, which compounds are named I.A2-Z147-Q3-U-1 to I.A2-Z147-Q3-U-42.

In another embodiment, compounds I are of formula I.A2 wherein Z is Z-147, R³ is CH₃ and Q is Q4, and R and R⁴ are as per any row of Table U below, which compounds are named I.A2-Z147-Q4-U-1 to I.A2-Z147-Q4-U-42.

In another embodiment, compounds I are of formula I.A1 wherein Z is Z-148, R³ is CH₃ and Q is Q1, and R and R⁴ are as per any row of Table U below, which compounds are named I.A1-Z148-Q1-U-1 to I.A1-Z148-Q1-U-42.

In another embodiment, compounds I are of formula I.A1 wherein Z is Z-148, R³ is CH₃ and Q is Q2, and R and R⁴ are as per any row of Table U below, which compounds are named I.A1-Z148-Q2-U-1 to I.A1-Z148-Q2-U-42.

In another embodiment, compounds I are of formula I.A1 wherein Z is Z-148, R³ is CH₃ and Q is Q3, and R and R⁴ are as per any row of Table U below, which compounds are named I.A1-Z148-Q3-U-1 to I.A1-Z148-Q3-U-42.

In another embodiment, compounds I are of formula I.A1 wherein Z is Z-148, R³ is CH₃ and Q is Q4, and R and R⁴ are as per any row of Table U below, which compounds are named I.A1-Z148-Q4-U-1 to I.A1-Z148-Q4-U-42.

In another embodiment, compounds I are of formula I.A2 wherein Z is Z-148, R³ is CH₃ and Q is Q1, and R and R⁴ are as per any row of Table U below, which compounds are named I.A2-Z148-Q1-U-1 to I.A2-Z148-Q1-U-42.

In another embodiment, compounds I are of formula I.A2 wherein Z is Z-148, R³ is CH₃ and Q is Q2, and R and R⁴ are as per any row of Table U below, which compounds are named I.A2-Z148-Q2-U-1 to I.A2-Z148-Q2-U-42.

In another embodiment, compounds I are of formula I.A2 wherein Z is Z-148, R³ is CH₃ and Q is Q3, and R and R⁴ are as per any row of Table U below, which compounds are named I.A2-Z148-Q3-U-1 to I.A2-Z148-Q3-U-42.

In another embodiment, compounds I are of formula I.A2 wherein Z is Z-148, R³ is CH₃ and Q is Q4, and R and R⁴ are as per any row of Table U below, which compounds are named I.A2-Z148-Q4-U-1 to I.A2-Z148-Q4-U-42.

In another embodiment, compounds I are of formula I.A1 wherein Z is Z-149, R³ is CH₃ and Q is Q1, and R and R⁴ are as per any row of Table U below, which compounds are named I.A1-Z149-Q1-U-1 to I.A1-Z149-Q1-U-42.

In another embodiment, compounds I are of formula I.A1 wherein Z is Z-149, R³ is CH₃ and Q is Q2, and R and R⁴ are as per any row of Table U below, which compounds are named I.A1-Z149-Q2-U-1 to I.A1-Z149-Q2-U-42.

In another embodiment, compounds I are of formula I.A1 wherein Z is Z-149, R³ is CH₃ and Q is Q3, and R and R⁴ are as per any row of Table U below, which compounds are named I.A1-Z149-Q3-U-1 to I.A1-Z149-Q3-U-42.

In another embodiment, compounds I are of formula I.A1 wherein Z is Z-149, R³ is CH₃ and Q is Q4, and R and R⁴ are as per any row of Table U below, which compounds are named I.A1-Z149-Q4-U-1 to I.A1-Z149-Q4-U-42.

In another embodiment, compounds I are of formula I.A2 wherein Z is Z-149, R³ is CH₃ and Q is Q1, and R and R⁴ are as per any row of Table U below, which compounds are named I.A2-Z149-Q1-U-1 to I.A2-Z149-Q1-U-42.

In another embodiment, compounds I are of formula I.A2 wherein Z is Z-149, R³ is CH₃ and Q is Q2, and R and R⁴ are as per any row of Table U below, which compounds are named I.A2-Z149-Q2-U-1 to I.A2-Z149-Q2-U-42.

In another embodiment, compounds I are of formula I.A2 wherein Z is Z-149, R³ is CH₃ and Q is Q3, and R and R⁴ are as per any row of Table U below, which compounds are named I.A2-Z149-Q3-U-1 to I.A2-Z149-Q3-U-42.

In another embodiment, compounds I are of formula I.A2 wherein Z is Z-149, R³ is CH₃ and Q is Q4, and R and R⁴ are as per any row of Table U below, which compounds are named I.A2-Z149-Q4-U-1 to I.A2-Z149-Q4-U-42.

In another embodiment, compounds I are of formula I.A1 wherein Z is Z-150, R³ is CH₃ and Q is Q1, and R and R⁴ are as per any row of Table U below, which compounds are named I.A1-Z150-Q1-U-1 to I.A1-Z150-Q1-U-42.

In another embodiment, compounds I are of formula I.A1 wherein Z is Z-150, R³ is CH₃ and Q is Q2, and R and R⁴ are as per any row of Table U below, which compounds are named I.A1-Z150-Q2-U-1 to I.A1-Z150-Q2-U-42.

In another embodiment, compounds I are of formula I.A1 wherein Z is Z-150, R³ is CH₃ and Q is Q3, and R and R⁴ are as per any row of Table U below, which compounds are named I.A1-Z150-Q3-U-1 to I.A1-Z150-Q3-U-42.

In another embodiment, compounds I are of formula I.A1 wherein Z is Z-150, R³ is CH₃ and Q is Q4, and R and R⁴ are as per any row of Table U below, which compounds are named I.A1-Z150-Q4-U-1 to I.A1-Z150-Q4-U-42.

In another embodiment, compounds I are of formula I.A2 wherein Z is Z-150, R³ is CH₃ and Q is Q1, and R and R⁴ are as per any row of Table U below, which compounds are named I.A2-Z150-Q1-U-1 to I.A2-Z150-Q1-U-42.

In another embodiment, compounds I are of formula I.A2 wherein Z is Z-150, R³ is CH₃ and Q is Q2, and R and R⁴ are as per any row of Table U below, which compounds are named I.A2-Z150-Q2-U-1 to I.A2-Z150-Q2-U-42.

In another embodiment, compounds I are of formula I.A2 wherein Z is Z-150, R³ is CH₃ and Q is Q3, and R and R⁴ are as per any row of Table U below, which compounds are named I.A2-Z150-Q3-U-1 to I.A2-Z150-Q3-U-42.

In another embodiment, compounds I are of formula I.A2 wherein Z is Z-150, R³ is CH₃ and Q is Q4, and R and R⁴ are as per any row of Table U below, which compounds are named I.A2-Z150-Q4-U-1 to I.A2-Z150-Q4-U-42.

In another embodiment, compounds I are of formula I.A1 wherein Z is Z-151, R³ is CH₃ and Q is Q1, and R and R⁴ are as per any row of Table U below, which compounds are named I.A1-Z151-Q1-U-1 to I.A1-Z151-Q1-U-42.

In another embodiment, compounds I are of formula I.A1 wherein Z is Z-151, R³ is CH₃ and Q is Q2, and R and R⁴ are as per any row of Table U below, which compounds are named I.A1-Z151-Q2-U-1 to I.A1-Z151-Q2-U-42.

In another embodiment, compounds I are of formula I.A1 wherein Z is Z-151, R³ is CH₃ and Q is Q3, and R and R⁴ are as per any row of Table U below, which compounds are named I.A1-Z151-Q3-U-1 to I.A1-Z151-Q3-U-42.

In another embodiment, compounds I are of formula I.A1 wherein Z is Z-151, R³ is CH₃ and Q is Q4, and R and R⁴ are as per any row of Table U below, which compounds are named I.A1-Z151-Q4-U-1 to I.A1-Z151-Q4-U-42.

In another embodiment, compounds I are of formula I.A2 wherein Z is Z-151, R³ is CH₃ and Q is Q1, and R and R⁴ are as per any row of Table U below, which compounds are named I.A2-Z151-Q1-U-1 to I.A2-Z151-Q1-U-42.

In another embodiment, compounds I are of formula I.A2 wherein Z is Z-151, R³ is CH₃ and Q is Q2, and R and R⁴ are as per any row of Table U below, which compounds are named I.A2-Z151-Q2-U-1 to I.A2-Z151-Q2-U-42.

In another embodiment, compounds I are of formula I.A2 wherein Z is Z-151, R³ is CH₃ and Q is Q3, and R and R⁴ are as per any row of Table U below, which compounds are named I.A2-Z151-Q3-U-1 to I.A2-Z151-Q3-U-42.

In another embodiment, compounds I are of formula I.A2 wherein Z is Z-151, R³ is CH₃ and Q is Q4, and R and R⁴ are as per any row of Table U below, which compounds are named I.A2-Z151-Q4-U-1 to I.A2-Z151-Q4-U-42.

In another embodiment, compounds I are of formula I.A1 wherein Z is Z-152, R³ is CH₃ and Q is Q1, and R and R⁴ are as per any row of Table U below, which compounds are named I.A1-Z152-Q1-U-1 to I.A1-Z152-Q1-U-42.

In another embodiment, compounds I are of formula I.A1 wherein Z is Z-152, R³ is CH₃ and Q is Q2, and R and R⁴ are as per any row of Table U below, which compounds are named I.A1-Z152-Q2-U-1 to I.A1-Z152-Q2-U-42.

In another embodiment, compounds I are of formula I.A1 wherein Z is Z-152, R³ is CH₃ and Q is Q3, and R and R⁴ are as per any row of Table U below, which compounds are named I.A1-Z152-Q3-U-1 to I.A1-Z152-Q3-U-42.

In another embodiment, compounds I are of formula I.A1 wherein Z is Z-152, R³ is CH₃ and Q is Q4, and R and R⁴ are as per any row of Table U below, which compounds are named I.A1-Z152-Q4-U-1 to I.A1-Z152-Q4-U-42.

In another embodiment, compounds I are of formula I.A2 wherein Z is Z-152, R³ is CH₃ and Q is Q1, and R and R⁴ are as per any row of Table U below, which compounds are named I.A2-Z152-Q1-U-1 to I.A2-Z152-Q1-U-42.

In another embodiment, compounds I are of formula I.A2 wherein Z is Z-152, R³ is CH₃ and Q is Q2, and R and R⁴ are as per any row of Table U below, which compounds are named I.A2-Z152-Q2-U-1 to I.A2-Z152-Q2-U-42.

In another embodiment, compounds I are of formula I.A2 wherein Z is Z-152, R³ is CH₃ and Q is Q3, and R and R⁴ are as per any row of Table U below, which compounds are named I.A2-Z152-Q3-U-1 to I.A2-Z152-Q3-U-42.

In another embodiment, compounds I are of formula I.A2 wherein Z is Z-152, R³ is CH₃ and Q is Q4, and R and R⁴ are as per any row of Table U below, which compounds are named I.A2-Z152-Q4-U-1 to I.A2-Z152-Q4-U-42.

In another embodiment, compounds I are of formula I.A1 wherein Z is Z-153, R³ is CH₃ and Q is Q1, and R and R⁴ are as per any row of Table U below, which compounds are named I.A1-Z153-Q1-U-1 to I.A1-Z153-Q1-U-42.

In another embodiment, compounds I are of formula I.A1 wherein Z is Z-153, R³ is CH₃ and Q is Q2, and R and R⁴ are as per any row of Table U below, which compounds are named I.A1-Z153-Q2-U-1 to I.A1-Z153-Q2-U-42.

In another embodiment, compounds I are of formula I.A1 wherein Z is Z-153, R³ is CH₃ and Q is Q3, and R and R⁴ are as per any row of Table U below, which compounds are named I.A1-Z153-Q3-U-1 to I.A1-Z153-Q3-U-42.

In another embodiment, compounds I are of formula I.A1 wherein Z is Z-153, R³ is CH₃ and Q is Q4, and R and R⁴ are as per any row of Table U below, which compounds are named I.A1-Z153-Q4-U-1 to I.A1-Z153-Q4-U-42.

In another embodiment, compounds I are of formula I.A2 wherein Z is Z-153, R³ is CH₃ and Q is Q1, and R and R⁴ are as per any row of Table U below, which compounds are named I.A2-Z153-Q1-U-1 to I.A2-Z153-Q1-U-42.

In another embodiment, compounds I are of formula I.A2 wherein Z is Z-153, R³ is CH₃ and Q is Q2, and R and R⁴ are as per any row of Table U below, which compounds are named I.A2-Z153-Q2-U-1 to I.A2-Z153-Q2-U-42.

In another embodiment, compounds I are of formula I.A2 wherein Z is Z-153, R³ is CH₃ and Q is Q3, and R and R⁴ are as per any row of Table U below, which compounds are named I.A2-Z153-Q3-U-1 to I.A2-Z153-Q3-U-42.

In another embodiment, compounds I are of formula I.A2 wherein Z is Z-153, R³ is CH₃ and Q is Q4, and R and R⁴ are as per any row of Table U below, which compounds are named I.A2-Z153-Q4-U-1 to I.A2-Z153-Q4-U-42.

In another embodiment, compounds I are of formula I.A1 wherein Z is Z-154, R³ is CH₃ and Q is Q1, and R and R⁴ are as per any row of Table U below, which compounds are named I.A1-Z154-Q1-U-1 to I.A1-Z154-Q1-U-42.

In another embodiment, compounds I are of formula I.A1 wherein Z is Z-154, R³ is CH₃ and Q is Q2, and R and R⁴ are as per any row of Table U below, which compounds are named I.A1-Z154-Q2-U-1 to I.A1-Z154-Q2-U-42.

In another embodiment, compounds I are of formula I.A1 wherein Z is Z-154, R³ is CH₃ and Q is Q3, and R and R⁴ are as per any row of Table U below, which compounds are named I.A1-Z154-Q3-U-1 to I.A1-Z154-Q3-U-42.

In another embodiment, compounds I are of formula I.A1 wherein Z is Z-154, R³ is CH₃ and Q is Q4, and R and R⁴ are as per any row of Table U below, which compounds are named I.A1-Z154-Q4-U-1 to I.A1-Z154-Q4-U-42.

In another embodiment, compounds I are of formula I.A2 wherein Z is Z-154, R³ is CH₃ and Q is Q1, and R and R⁴ are as per any row of Table U below, which compounds are named I.A2-Z154-Q1-U-1 to I.A2-Z154-Q1-U-42.

In another embodiment, compounds I are of formula I.A2 wherein Z is Z-154, R³ is CH₃ and Q is Q2, and R and R⁴ are as per any row of Table U below, which compounds are named I.A2-Z154-Q2-U-1 to I.A2-Z154-Q2-U-42.

In another embodiment, compounds I are of formula I.A2 wherein Z is Z-154, R³ is CH₃ and Q is Q3, and R and R⁴ are as per any row of Table U below, which compounds are named I.A2-Z154-Q3-U-1 to I.A2-Z154-Q3-U-42.

In another embodiment, compounds I are of formula I.A2 wherein Z is Z-154, R³ is CH₃ and Q is Q4, and R and R⁴ are as per any row of Table U below, which compounds are named I.A2-Z154-Q4-U-1 to I.A2-Z154-Q4-U-42.

In another embodiment, compounds I are of formula I.A1 wherein Z is Z-155, R³ is CH₃ and Q is Q1, and R and R⁴ are as per any row of Table U below, which compounds are named I.A1-Z155-Q1-U-1 to I.A1-Z155-Q1-U-42.

In another embodiment, compounds I are of formula I.A1 wherein Z is Z-155, R³ is CH₃ and Q is Q2, and R and R⁴ are as per any row of Table U below, which compounds are named I.A1-Z155-Q2-U-1 to I.A1-Z155-Q2-U-42.

In another embodiment, compounds I are of formula I.A1 wherein Z is Z-155, R³ is CH₃ and Q is Q3, and R and R⁴ are as per any row of Table U below, which compounds are named I.A1-Z155-Q3-U-1 to I.A1-Z155-Q3-U-42.

In another embodiment, compounds I are of formula I.A1 wherein Z is Z-155, R³ is CH₃ and Q is Q4, and R and R⁴ are as per any row of Table U below, which compounds are named I.A1-Z155-Q4-U-1 to I.A1-Z155-Q4-U-42.

In another embodiment, compounds I are of formula I.A2 wherein Z is Z-155, R³ is CH₃ and Q is Q1, and R and R⁴ are as per any row of Table U below, which compounds are named I.A2-Z155-Q1-U-1 to I.A2-Z155-Q1-U-42.

In another embodiment, compounds I are of formula I.A2 wherein Z is Z-155, R³ is CH₃ and Q is Q2, and R and R⁴ are as per any row of Table U below, which compounds are named I.A2-Z155-Q2-U-1 to I.A2-Z155-Q2-U-42.

In another embodiment, compounds I are of formula I.A2 wherein Z is Z-155, R³ is CH₃ and Q is Q3, and R and R⁴ are as per any row of Table U below, which compounds are named I.A2-Z155-Q3-U-1 to I.A2-Z155-Q3-U-42.

In another embodiment, compounds I are of formula I.A2 wherein Z is Z-155, R³ is CH₃ and Q is Q4, and R and R⁴ are as per any row of Table U below, which compounds are named I.A2-Z155-Q4-U-1 to I.A2-Z155-Q4-U-42.

In another embodiment, compounds I are of formula I.A1 wherein Z is Z-156, R³ is CH₃ and Q is Q1, and R and R⁴ are as per any row of Table U below, which compounds are named I.A1-Z156-Q1-U-1 to I.A1-Z156-Q1-U-42.

In another embodiment, compounds I are of formula I.A1 wherein Z is Z-156, R³ is CH₃ and Q is Q2, and R and R⁴ are as per any row of Table U below, which compounds are named I.A1-Z156-Q2-U-1 to I.A1-Z156-Q2-U-42.

In another embodiment, compounds I are of formula I.A1 wherein Z is Z-156, R³ is CH₃ and Q is Q3, and R and R⁴ are as per any row of Table U below, which compounds are named I.A1-Z156-Q3-U-1 to I.A1-Z156-Q3-U-42.

In another embodiment, compounds I are of formula I.A1 wherein Z is Z-156, R³ is CH₃ and Q is Q4, and R and R⁴ are as per any row of Table U below, which compounds are named I.A1-Z156-Q4-U-1 to I.A1-Z156-Q4-U-42.

In another embodiment, compounds I are of formula I.A2 wherein Z is Z-156, R³ is CH₃ and Q is Q1, and R and R⁴ are as per any row of Table U below, which compounds are named I.A2-Z156-Q1-U-1 to I.A2-Z156-Q1-U-42.

In another embodiment, compounds I are of formula I.A2 wherein Z is Z-156, R³ is CH₃ and Q is Q2, and R and R⁴ are as per any row of Table U below, which compounds are named I.A2-Z156-Q2-U-1 to I.A2-Z156-Q2-U-42.

In another embodiment, compounds I are of formula I.A2 wherein Z is Z-156, R³ is CH₃ and Q is Q3, and R and R⁴ are as per any row of Table U below, which compounds are named I.A2-Z156-Q3-U-1 to I.A2-Z156-Q3-U-42.

In another embodiment, compounds I are of formula I.A2 wherein Z is Z-156, R³ is CH₃ and Q is Q4, and R and R⁴ are as per any row of Table U below, which compounds are named I.A2-Z156-Q4-U-1 to I.A2-Z156-Q4-U-42.

In another embodiment, compounds I are of formula I.A1 wherein Z is Z-157, R³ is CH₃ and Q is Q1, and R and R⁴ are as per any row of Table U below, which compounds are named I.A1-Z157-Q1-U-1 to I.A1-Z157-Q1-U-42.

In another embodiment, compounds I are of formula I.A1 wherein Z is Z-157, R³ is CH₃ and Q is Q2, and R and R⁴ are as per any row of Table U below, which compounds are named I.A1-Z157-Q2-U-1 to I.A1-Z157-Q2-U-42.

In another embodiment, compounds I are of formula I.A1 wherein Z is Z-157, R³ is CH₃ and Q is Q3, and R and R⁴ are as per any row of Table U below, which compounds are named I.A1-Z157-Q3-U-1 to I.A1-Z157-Q3-U-42.

In another embodiment, compounds I are of formula I.A1 wherein Z is Z-157, R³ is CH₃ and Q is Q4, and R and R⁴ are as per any row of Table U below, which compounds are named I.A1-Z157-Q4-U-1 to I.A1-Z157-Q4-U-42.

In another embodiment, compounds I are of formula I.A2 wherein Z is Z-157, R³ is CH₃ and Q is Q1, and R and R⁴ are as per any row of Table U below, which compounds are named I.A2-Z157-Q1-U-1 to I.A2-Z157-Q1-U-42.

In another embodiment, compounds I are of formula I.A2 wherein Z is Z-157, R³ is CH₃ and Q is Q2, and R and R⁴ are as per any row of Table U below, which compounds are named I.A2-Z157-Q2-U-1 to I.A2-Z157-Q2-U-42.

In another embodiment, compounds I are of formula I.A2 wherein Z is Z-157, R³ is CH₃ and Q is Q3, and R and R⁴ are as per any row of Table U below, which compounds are named I.A2-Z157-Q3-U-1 to I.A2-Z157-Q3-U-42.

In another embodiment, compounds I are of formula I.A2 wherein Z is Z-157, R³ is CH₃ and Q is Q4, and R and R⁴ are as per any row of Table U below, which compounds are named I.A2-Z157-Q4-U-1 to I.A2-Z157-Q4-U-42.

In another embodiment, compounds I are of formula I.A1 wherein Z is Z-158, R³ is CH₃ and Q is Q1, and R and R⁴ are as per any row of Table U below, which compounds are named I.A1-Z158-Q1-U-1 to I.A1-Z158-Q1-U-42.

In another embodiment, compounds I are of formula I.A1 wherein Z is Z-158, R³ is CH₃ and Q is Q2, and R and R⁴ are as per any row of Table U below, which compounds are named I.A1-Z158-Q2-U-1 to I.A1-Z158-Q2-U-42.

In another embodiment, compounds I are of formula I.A1 wherein Z is Z-158, R³ is CH₃ and Q is Q3, and R and R⁴ are as per any row of Table U below, which compounds are named I.A1-Z158-Q3-U-1 to I.A1-Z158-Q3-U-42.

In another embodiment, compounds I are of formula I.A1 wherein Z is Z-158, R³ is CH₃ and Q is Q4, and R and R⁴ are as per any row of Table U below, which compounds are named I.A1-Z158-Q4-U-1 to I.A1-Z158-Q4-U-42.

In another embodiment, compounds I are of formula I.A2 wherein Z is Z-158, R³ is CH₃ and Q is Q1, and R and R⁴ are as per any row of Table U below, which compounds are named I.A2-Z158-Q1-U-1 to I.A2-Z158-Q1-U-42.

In another embodiment, compounds I are of formula I.A2 wherein Z is Z-158, R³ is CH₃ and Q is Q2, and R and R⁴ are as per any row of Table U below, which compounds are named I.A2-Z158-Q2-U-1 to I.A2-Z158-Q2-U-42.

In another embodiment, compounds I are of formula I.A2 wherein Z is Z-158, R³ is CH₃ and Q is Q3, and R and R⁴ are as per any row of Table U below, which compounds are named I.A2-Z158-Q3-U-1 to I.A2-Z158-Q3-U-42.

In another embodiment, compounds I are of formula I.A2 wherein Z is Z-158, R³ is CH₃ and Q is Q4, and R and R⁴ are as per any row of Table U below, which compounds are named I.A2-Z158-Q4-U-1 to I.A2-Z158-Q4-U-42.

In another embodiment, compounds I are of formula I.A1 wherein Z is Z-159, R³ is CH₃ and Q is Q1, and R and R⁴ are as per any row of Table U below, which compounds are named I.A1-Z159-Q1-U-1 to I.A1-Z159-Q1-U-42.

In another embodiment, compounds I are of formula I.A1 wherein Z is Z-159, R³ is CH₃ and Q is Q2, and R and R⁴ are as per any row of Table U below, which compounds are named I.A1-Z159-Q2-U-1 to I.A1-Z159-Q2-U-42.

In another embodiment, compounds I are of formula I.A1 wherein Z is Z-159, R³ is CH₃ and Q is Q3, and R and R⁴ are as per any row of Table U below, which compounds are named I.A1-Z159-Q3-U-1 to I.A1-Z159-Q3-U-42.

In another embodiment, compounds I are of formula I.A1 wherein Z is Z-159, R³ is CH₃ and Q is Q4, and R and R⁴ are as per any row of Table U below, which compounds are named I.A1-Z159-Q4-U-1 to I.A1-Z159-Q4-U-42.

In another embodiment, compounds I are of formula I.A2 wherein Z is Z-159, R³ is CH₃ and Q is Q1, and R and R⁴ are as per any row of Table U below, which compounds are named I.A2-Z159-Q1-U-1 to I.A2-Z159-Q1-U-42.

In another embodiment, compounds I are of formula I.A2 wherein Z is Z-159, R³ is CH₃ and Q is Q2, and R and R⁴ are as per any row of Table U below, which compounds are named I.A2-Z159-Q2-U-1 to I.A2-Z159-Q2-U-42.

In another embodiment, compounds I are of formula I.A2 wherein Z is Z-159, R³ is CH₃ and Q is Q3, and R and R⁴ are as per any row of Table U below, which compounds are named I.A2-Z159-Q3-U-1 to I.A2-Z159-Q3-U-42.

In another embodiment, compounds I are of formula I.A2 wherein Z is Z-159, R³ is CH₃ and Q is Q4, and R and R⁴ are as per any row of Table U below, which compounds are named I.A2-Z159-Q4-U-1 to I.A2-Z159-Q4-U-42.

In another embodiment, compounds I are of formula I.A1 wherein Z is Z-160, R³ is CH₃ and Q is Q1, and R and R⁴ are as per any row of Table U below, which compounds are named I.A1-Z160-Q1-U-1 to I.A1-Z160-Q1-U-42.

In another embodiment, compounds I are of formula I.A1 wherein Z is Z-160, R³ is CH₃ and Q is Q2, and R and R⁴ are as per any row of Table U below, which compounds are named I.A1-Z160-Q2-U-1 to I.A1-Z160-Q2-U-42.

In another embodiment, compounds I are of formula I.A1 wherein Z is Z-160, R³ is CH₃ and Q is Q3, and R and R⁴ are as per any row of Table U below, which compounds are named I.A1-Z160-Q3-U-1 to I.A1-Z160-Q3-U-42.

In another embodiment, compounds I are of formula I.A1 wherein Z is Z-160, R³ is CH₃ and Q is Q4, and R and R⁴ are as per any row of Table U below, which compounds are named I.A1-Z160-Q4-U-1 to I.A1-Z160-Q4-U-42.

In another embodiment, compounds I are of formula I.A2 wherein Z is Z-160, R³ is CH₃ and Q is Q1, and R and R⁴ are as per any row of Table U below, which compounds are named I.A2-Z160-Q1-U-1 to I.A2-Z160-Q1-U-42.

In another embodiment, compounds I are of formula I.A2 wherein Z is Z-160, R³ is CH₃ and Q is Q2, and R and R⁴ are as per any row of Table U below, which compounds are named I.A2-Z160-Q2-U-1 to I.A2-Z160-Q2-U-42.

In another embodiment, compounds I are of formula I.A2 wherein Z is Z-160, R³ is CH₃ and Q is Q3, and R and R⁴ are as per any row of Table U below, which compounds are named I.A2-Z160-Q3-U-1 to I.A2-Z160-Q3-U-42.

In another embodiment, compounds I are of formula I.A2 wherein Z is Z-160, R³ is CH₃ and Q is Q4, and R and R⁴ are as per any row of Table U below, which compounds are named I.A2-Z160-Q4-U-1 to I.A2-Z160-Q4-U-42.

In another embodiment, compounds I are of formula I.A1 wherein Z is Z-161, R³ is CH₃ and Q is Q1, and R and R⁴ are as per any row of Table U below, which compounds are named I.A1-Z161-Q1-U-1 to I.A1-Z161-Q1-U-42.

In another embodiment, compounds I are of formula I.A1 wherein Z is Z-161, R³ is CH₃ and Q is Q2, and R and R⁴ are as per any row of Table U below, which compounds are named I.A1-Z161-Q2-U-1 to I.A1-Z161-Q2-U-42.

In another embodiment, compounds I are of formula I.A1 wherein Z is Z-161, R³ is CH₃ and Q is Q3, and R and R⁴ are as per any row of Table U below, which compounds are named I.A1-Z161-Q3-U-1 to I.A1-Z161-Q3-U-42.

In another embodiment, compounds I are of formula I.A1 wherein Z is Z-161, R³ is CH₃ and Q is Q4, and R and R⁴ are as per any row of Table U below, which compounds are named I.A1-Z161-Q4-U-1 to I.A1-Z161-Q4-U-42.

In another embodiment, compounds I are of formula I.A2 wherein Z is Z-161, R³ is CH₃ and Q is Q1, and R and R⁴ are as per any row of Table U below, which compounds are named I.A2-Z161-Q1-U-1 to I.A2-Z161-Q1-U-42.

In another embodiment, compounds I are of formula I.A2 wherein Z is Z-161, R³ is CH₃ and Q is Q2, and R and R⁴ are as per any row of Table U below, which compounds are named I.A2-Z161-Q2-U-1 to I.A2-Z161-Q2-U-42.

In another embodiment, compounds I are of formula I.A2 wherein Z is Z-161, R³ is CH₃ and Q is Q3, and R and R⁴ are as per any row of Table U below, which compounds are named I.A2-Z161-Q3-U-1 to I.A2-Z161-Q3-U-42.

In another embodiment, compounds I are of formula I.A2 wherein Z is Z-161, R³ is CH₃ and Q is Q4, and R and R⁴ are as per any row of Table U below, which compounds are named I.A2-Z161-Q4-U-1 to I.A2-Z161-Q4-U-42.

In another embodiment, compounds I are of formula I.A1 wherein Z is Z-162, R³ is CH₃ and Q is Q1, and R and R⁴ are as per any row of Table U below, which compounds are named I.A1-Z162-Q1-U-1 to I.A1-Z162-Q1-U-42.

In another embodiment, compounds I are of formula I.A1 wherein Z is Z-162, R³ is CH₃ and Q is Q2, and R and R⁴ are as per any row of Table U below, which compounds are named I.A1-Z162-Q2-U-1 to I.A1-Z162-Q2-U-42.

In another embodiment, compounds I are of formula I.A1 wherein Z is Z-162, R³ is CH₃ and Q is Q3, and R and R⁴ are as per any row of Table U below, which compounds are named I.A1-Z162-Q3-U-1 to I.A1-Z162-Q3-U-42.

In another embodiment, compounds I are of formula I.A1 wherein Z is Z-162, R³ is CH₃ and Q is Q4, and R and R⁴ are as per any row of Table U below, which compounds are named I.A1-Z162-Q4-U-1 to I.A1-Z162-Q4-U-42.

In another embodiment, compounds I are of formula I.A2 wherein Z is Z-162, R³ is CH₃ and Q is Q1, and R and R⁴ are as per any row of Table U below, which compounds are named I.A2-Z162-Q1-U-1 to I.A2-Z162-Q1-U-42.

In another embodiment, compounds I are of formula I.A2 wherein Z is Z-162, R³ is CH₃ and Q is Q2, and R and R⁴ are as per any row of Table U below, which compounds are named I.A2-Z162-Q2-U-1 to I.A2-Z162-Q2-U-42.

In another embodiment, compounds I are of formula I.A2 wherein Z is Z-162, R³ is CH₃ and Q is Q3, and R and R⁴ are as per any row of Table U below, which compounds are named I.A2-Z162-Q3-U-1 to I.A2-Z162-Q3-U-42.

In another embodiment, compounds I are of formula I.A2 wherein Z is Z-162, R³ is CH₃ and Q is Q4, and R and R⁴ are as per any row of Table U below, which compounds are named I.A2-Z162-Q4-U-1 to I.A2-Z162-Q4-U-42.

In another embodiment, compounds I are of formula I.A1 wherein Z is Z-163, R³ is CH₃ and Q is Q1, and R and R⁴ are as per any row of Table U below, which compounds are named I.A1-Z163-Q1-U-1 to I.A1-Z163-Q1-U-42.

In another embodiment, compounds I are of formula I.A1 wherein Z is Z-163, R³ is CH₃ and Q is Q2, and R and R⁴ are as per any row of Table U below, which compounds are named I.A1-Z163-Q2-U-1 to I.A1-Z163-Q2-U-42.

In another embodiment, compounds I are of formula I.A1 wherein Z is Z-163, R³ is CH₃ and Q is Q3, and R and R⁴ are as per any row of Table U below, which compounds are named I.A1-Z163-Q3-U-1 to I.A1-Z163-Q3-U-42.

In another embodiment, compounds I are of formula I.A1 wherein Z is Z-163, R³ is CH₃ and Q is Q4, and R and R⁴ are as per any row of Table U below, which compounds are named I.A1-Z163-Q4-U-1 to I.A1-Z163-Q4-U-42.

In another embodiment, compounds I are of formula I.A2 wherein Z is Z-163, R³ is CH₃ and Q is Q1, and R and R⁴ are as per any row of Table U below, which compounds are named I.A2-Z163-Q1-U-1 to I.A2-Z163-Q1-U-42.

In another embodiment, compounds I are of formula I.A2 wherein Z is Z-163, R³ is CH₃ and Q is Q2, and R and R⁴ are as per any row of Table U below, which compounds are named I.A2-Z163-Q2-U-1 to I.A2-Z163-Q2-U-42.

In another embodiment, compounds I are of formula I.A2 wherein Z is Z-163, R³ is CH₃ and Q is Q3, and R and R⁴ are as per any row of Table U below, which compounds are named I.A2-Z163-Q3-U-1 to I.A2-Z163-Q3-U-42.

In another embodiment, compounds I are of formula I.A2 wherein Z is Z-163, R³ is CH₃ and Q is Q4, and R and R⁴ are as per any row of Table U below, which compounds are named I.A2-Z163-Q4-U-1 to I.A2-Z163-Q4-U-42.

In another embodiment, compounds I are of formula I.A1 wherein Z is Z-164, R³ is CH₃ and Q is Q1, and R and R⁴ are as per any row of Table U below, which compounds are named I.A1-Z164-Q1-U-1 to I.A1-Z164-Q1-U-42.

In another embodiment, compounds I are of formula I.A1 wherein Z is Z-164, R³ is CH₃ and Q is Q2, and R and R⁴ are as per any row of Table U below, which compounds are named I.A1-Z164-Q2-U-1 to I.A1-Z164-Q2-U-42.

In another embodiment, compounds I are of formula I.A1 wherein Z is Z-164, R³ is CH₃ and Q is Q3, and R and R⁴ are as per any row of Table U below, which compounds are named I.A1-Z164-Q3-U-1 to I.A1-Z164-Q3-U-42.

In another embodiment, compounds I are of formula I.A1 wherein Z is Z-164, R³ is CH₃ and Q is Q4, and R and R⁴ are as per any row of Table U below, which compounds are named I.A1-Z164-Q4-U-1 to I.A1-Z164-Q4-U-42.

In another embodiment, compounds I are of formula I.A2 wherein Z is Z-164, R³ is CH₃ and Q is Q1, and R and R⁴ are as per any row of Table U below, which compounds are named I.A2-Z164-Q1-U-1 to I.A2-Z164-Q1-U-42.

In another embodiment, compounds I are of formula I.A2 wherein Z is Z-164, R³ is CH₃ and Q is Q2, and R and R⁴ are as per any row of Table U below, which compounds are named I.A2-Z164-Q2-U-1 to I.A2-Z164-Q2-U-42.

In another embodiment, compounds I are of formula I.A2 wherein Z is Z-164, R³ is CH₃ and Q is Q3, and R and R⁴ are as per any row of Table U below, which compounds are named I.A2-Z164-Q3-U-1 to I.A2-Z164-Q3-U-42.

In another embodiment, compounds I are of formula I.A2 wherein Z is Z-164, R³ is CH₃ and Q is Q4, and R and R⁴ are as per any row of Table U below, which compounds are named I.A2-Z164-Q4-U-1 to I.A2-Z164-Q4-U-42.

In another embodiment, compounds I are of formula I.A1 wherein Z is Z-165, R³ is CH₃ and Q is Q1, and R and R⁴ are as per any row of Table U below, which compounds are named I.A1-Z165-Q1-U-1 to I.A1-Z165-Q1-U-42.

In another embodiment, compounds I are of formula I.A1 wherein Z is Z-165, R³ is CH₃ and Q is Q2, and R and R⁴ are as per any row of Table U below, which compounds are named I.A1-Z165-Q2-U-1 to I.A1-Z165-Q2-U-42.

In another embodiment, compounds I are of formula I.A1 wherein Z is Z-165, R³ is CH₃ and Q is Q3, and R and R⁴ are as per any row of Table U below, which compounds are named I.A1-Z165-Q3-U-1 to I.A1-Z165-Q3-U-42.

In another embodiment, compounds I are of formula I.A1 wherein Z is Z-165, R³ is CH₃ and Q is Q4, and R and R⁴ are as per any row of Table U below, which compounds are named I.A1-Z165-Q4-U-1 to I.A1-Z165-Q4-U-42.

In another embodiment, compounds I are of formula I.A2 wherein Z is Z-165, R³ is CH₃ and Q is Q1, and R and R⁴ are as per any row of Table U below, which compounds are named I.A2-Z165-Q1-U-1 to I.A2-Z165-Q1-U-42.

In another embodiment, compounds I are of formula I.A2 wherein Z is Z-165, R³ is CH₃ and Q is Q2, and R and R⁴ are as per any row of Table U below, which compounds are named I.A2-Z165-Q2-U-1 to I.A2-Z165-Q2-U-42.

In another embodiment, compounds I are of formula I.A2 wherein Z is Z-165, R³ is CH₃ and Q is Q3, and R and R⁴ are as per any row of Table U below, which compounds are named I.A2-Z165-Q3-U-1 to I.A2-Z165-Q3-U-42.

In another embodiment, compounds I are of formula I.A2 wherein Z is Z-165, R³ is CH₃ and Q is Q4, and R and R⁴ are as per any row of Table U below, which compounds are named I.A2-Z165-Q4-U-1 to I.A2-Z165-Q4-U-42.

In another embodiment, compounds I are of formula I.A1 wherein Z is Z-166, R³ is CH₃ and Q is Q1, and R and R⁴ are as per any row of Table U below, which compounds are named I.A1-Z166-Q1-U-1 to I.A1-Z166-Q1-U-42.

In another embodiment, compounds I are of formula I.A1 wherein Z is Z-166, R³ is CH₃ and Q is Q2, and R and R⁴ are as per any row of Table U below, which compounds are named I.A1-Z166-Q2-U-1 to I.A1-Z166-Q2-U-42.

In another embodiment, compounds I are of formula I.A1 wherein Z is Z-166, R³ is CH₃ and Q is Q3, and R and R⁴ are as per any row of Table U below, which compounds are named I.A1-Z166-Q3-U-1 to I.A1-Z166-Q3-U-42.

In another embodiment, compounds I are of formula I.A1 wherein Z is Z-166, R³ is CH₃ and Q is Q4, and R and R⁴ are as per any row of Table U below, which compounds are named I.A1-Z166-Q4-U-1 to I.A1-Z166-Q4-U-42.

In another embodiment, compounds I are of formula I.A2 wherein Z is Z-166, R³ is CH₃ and Q is Q1, and R and R⁴ are as per any row of Table U below, which compounds are named I.A2-Z166-Q1-U-1 to I.A2-Z166-Q1-U-42.

In another embodiment, compounds I are of formula I.A2 wherein Z is Z-166, R³ is CH₃ and Q is Q2, and R and R⁴ are as per any row of Table U below, which compounds are named I.A2-Z166-Q2-U-1 to I.A2-Z166-Q2-U-42.

In another embodiment, compounds I are of formula I.A2 wherein Z is Z-166, R³ is CH₃ and Q is Q3, and R and R⁴ are as per any row of Table U below, which compounds are named I.A2-Z166-Q3-U-1 to I.A2-Z166-Q3-U-42.

In another embodiment, compounds I are of formula I.A2 wherein Z is Z-166, R³ is CH₃ and Q is Q4, and R and R⁴ are as per any row of Table U below, which compounds are named I.A2-Z166-Q4-U-1 to I.A2-Z166-Q4-U-42.

In another embodiment, compounds I are of formula I.A1 wherein Z is Z-167, R³ is CH₃ and Q is Q1, and R and R⁴ are as per any row of Table U below, which compounds are named I.A1-Z167-Q1-U-1 to I.A1-Z167-Q1-U-42.

In another embodiment, compounds I are of formula I.A1 wherein Z is Z-167, R³ is CH₃ and Q is Q2, and R and R⁴ are as per any row of Table U below, which compounds are named I.A1-Z167-Q2-U-1 to I.A1-Z167-Q2-U-42.

In another embodiment, compounds I are of formula I.A1 wherein Z is Z-167, R³ is CH₃ and Q is Q3, and R and R⁴ are as per any row of Table U below, which compounds are named I.A1-Z167-Q3-U-1 to I.A1-Z167-Q3-U-42.

In another embodiment, compounds I are of formula I.A1 wherein Z is Z-167, R³ is CH₃ and Q is Q4, and R and R⁴ are as per any row of Table U below, which compounds are named I.A1-Z167-Q4-U-1 to I.A1-Z167-Q4-U-42.

In another embodiment, compounds I are of formula I.A2 wherein Z is Z-167, R³ is CH₃ and Q is Q1, and R and R⁴ are as per any row of Table U below, which compounds are named I.A2-Z167-Q1-U-1 to I.A2-Z167-Q1-U-42.

In another embodiment, compounds I are of formula I.A2 wherein Z is Z-167, R³ is CH₃ and Q is Q2, and R and R⁴ are as per any row of Table U below, which compounds are named I.A2-Z167-Q2-U-1 to I.A2-Z167-Q2-U-42.

In another embodiment, compounds I are of formula I.A2 wherein Z is Z-167, R³ is CH₃ and Q is Q3, and R and R⁴ are as per any row of Table U below, which compounds are named I.A2-Z167-Q3-U-1 to I.A2-Z167-Q3-U-42.

In another embodiment, compounds I are of formula I.A2 wherein Z is Z-167, R³ is CH₃ and Q is Q4, and R and R⁴ are as per any row of Table U below, which compounds are named I.A2-Z167-Q4-U-1 to I.A2-Z167-Q4-U-42.

In another embodiment, compounds I are of formula I.A1 wherein Z is Z-168, R³ is CH₃ and Q is Q1, and R and R⁴ are as per any row of Table U below, which compounds are named I.A1-Z168-Q1-U-1 to I.A1-Z168-Q1-U-42.

In another embodiment, compounds I are of formula I.A1 wherein Z is Z-168, R³ is CH₃ and Q is Q2, and R and R⁴ are as per any row of Table U below, which compounds are named I.A1-Z168-Q2-U-1 to I.A1-Z168-Q2-U-42.

In another embodiment, compounds I are of formula I.A1 wherein Z is Z-168, R³ is CH₃ and Q is Q3, and R and R⁴ are as per any row of Table U below, which compounds are named I.A1-Z168-Q3-U-1 to I.A1-Z168-Q3-U-42.

In another embodiment, compounds I are of formula I.A1 wherein Z is Z-168, R³ is CH₃ and Q is Q4, and R and R⁴ are as per any row of Table U below, which compounds are named I.A1-Z168-Q4-U-1 to I.A1-Z168-Q4-U-42.

In another embodiment, compounds I are of formula I.A2 wherein Z is Z-168, R³ is CH₃ and Q is Q1, and R and R⁴ are as per any row of Table U below, which compounds are named I.A2-Z168-Q1-U-1 to I.A2-Z168-Q1-U-42.

In another embodiment, compounds I are of formula I.A2 wherein Z is Z-168, R³ is CH₃ and Q is Q2, and R and R⁴ are as per any row of Table U below, which compounds are named I.A2-Z168-Q2-U-1 to I.A2-Z168-Q2-U-42.

In another embodiment, compounds I are of formula I.A2 wherein Z is Z-168, R³ is CH₃ and Q is Q3, and R and R⁴ are as per any row of Table U below, which compounds are named I.A2-Z168-Q3-U-1 to I.A2-Z168-Q3-U-42.

In another embodiment, compounds I are of formula I.A2 wherein Z is Z-168, R³ is CH₃ and Q is Q4, and R and R⁴ are as per any row of Table U below, which compounds are named I.A2-Z168-Q4-U-1 to I.A2-Z168-Q4-U-42.

In another embodiment, compounds I are of formula I.A1 wherein Z is Z-169, R³ is CH₃ and Q is Q1, and R and R⁴ are as per any row of Table U below, which compounds are named I.A1-Z169-Q1-U-1 to I.A1-Z169-Q1-U-42.

In another embodiment, compounds I are of formula I.A1 wherein Z is Z-169, R³ is CH₃ and Q is Q2, and R and R⁴ are as per any row of Table U below, which compounds are named I.A1-Z169-Q2-U-1 to I.A1-Z169-Q2-U-42.

In another embodiment, compounds I are of formula I.A1 wherein Z is Z-169, R³ is CH₃ and Q is Q3, and R and R⁴ are as per any row of Table U below, which compounds are named I.A1-Z169-Q3-U-1 to I.A1-Z169-Q3-U-42.

In another embodiment, compounds I are of formula I.A1 wherein Z is Z-169, R³ is CH₃ and Q is Q4, and R and R⁴ are as per any row of Table U below, which compounds are named I.A1-Z169-Q4-U-1 to I.A1-Z169-Q4-U-42.

In another embodiment, compounds I are of formula I.A2 wherein Z is Z-169, R³ is CH₃ and Q is Q1, and R and R⁴ are as per any row of Table U below, which compounds are named I.A2-Z169-Q1-U-1 to I.A2-Z169-Q1-U-42.

In another embodiment, compounds I are of formula I.A2 wherein Z is Z-169, R³ is CH₃ and Q is Q2, and R and R⁴ are as per any row of Table U below, which compounds are named I.A2-Z169-Q2-U-1 to I.A2-Z169-Q2-U-42.

In another embodiment, compounds I are of formula I.A2 wherein Z is Z-169, R³ is CH₃ and Q is Q3, and R and R⁴ are as per any row of Table U below, which compounds are named I.A2-Z169-Q3-U-1 to I.A2-Z169-Q3-U-42.

In another embodiment, compounds I are of formula I.A2 wherein Z is Z-169, R³ is CH₃ and Q is Q4, and R and R⁴ are as per any row of Table U below, which compounds are named I.A2-Z169-Q4-U-1 to I.A2-Z169-Q4-U-42.

In another embodiment, compounds I are of formula I.A1 wherein Z is Z-170, R³ is CH₃ and Q is Q1, and R and R⁴ are as per any row of Table U below, which compounds are named I.A1-Z170-Q1-U-1 to I.A1-Z170-Q1-U-42.

In another embodiment, compounds I are of formula I.A1 wherein Z is Z-170, R³ is CH₃ and Q is Q2, and R and R⁴ are as per any row of Table U below, which compounds are named I.A1-Z170-Q2-U-1 to I.A1-Z170-Q2-U-42.

In another embodiment, compounds I are of formula I.A1 wherein Z is Z-170, R³ is CH₃ and Q is Q3, and R and R⁴ are as per any row of Table U below, which compounds are named I.A1-Z170-Q3-U-1 to I.A1-Z170-Q3-U-42.

In another embodiment, compounds I are of formula I.A1 wherein Z is Z-170, R³ is CH₃ and Q is Q4, and R and R⁴ are as per any row of Table U below, which compounds are named I.A1-Z170-Q4-U-1 to I.A1-Z170-Q4-U-42.

In another embodiment, compounds I are of formula I.A2 wherein Z is Z-170, R³ is CH₃ and Q is Q1, and R and R⁴ are as per any row of Table U below, which compounds are named I.A2-Z170-Q1-U-1 to I.A2-Z170-Q1-U-42.

In another embodiment, compounds I are of formula I.A2 wherein Z is Z-170, R³ is CH₃ and Q is Q2, and R and R⁴ are as per any row of Table U below, which compounds are named I.A2-Z170-Q2-U-1 to I.A2-Z170-Q2-U-42.

In another embodiment, compounds I are of formula I.A2 wherein Z is Z-170, R³ is CH₃ and Q is Q3, and R and R⁴ are as per any row of Table U below, which compounds are named I.A2-Z170-Q3-U-1 to I.A2-Z170-Q3-U-42.

In another embodiment, compounds I are of formula I.A2 wherein Z is Z-170, R³ is CH₃ and Q is Q4, and R and R⁴ are as per any row of Table U below, which compounds are named I.A2-Z170-Q4-U-1 to I.A2-Z170-Q4-U-42.

In another embodiment, compounds I are of formula I.A1 wherein Z is Z-171, R³ is CH₃ and Q is Q1, and R and R⁴ are as per any row of Table U below, which compounds are named I.A1-Z171-Q1-U-1 to I.A1-Z171-Q1-U-42.

In another embodiment, compounds I are of formula I.A1 wherein Z is Z-171, R³ is CH₃ and Q is Q2, and R and R⁴ are as per any row of Table U below, which compounds are named I.A1-Z171-Q2-U-1 to I.A1-Z171-Q2-U-42.

In another embodiment, compounds I are of formula I.A1 wherein Z is Z-171, R³ is CH₃ and Q is Q3, and R and R⁴ are as per any row of Table U below, which compounds are named I.A1-Z171-Q3-U-1 to I.A1-Z171-Q3-U-42.

In another embodiment, compounds I are of formula I.A1 wherein Z is Z-171, R³ is CH₃ and Q is Q4, and R and R⁴ are as per any row of Table U below, which compounds are named I.A1-Z171-Q4-U-1 to I.A1-Z171-Q4-U-42.

In another embodiment, compounds I are of formula I.A2 wherein Z is Z-171, R³ is CH₃ and Q is Q1, and R and R⁴ are as per any row of Table U below, which compounds are named I.A2-Z171-Q1-U-1 to I.A2-Z171-Q1-U-42.

In another embodiment, compounds I are of formula I.A2 wherein Z is Z-171, R³ is CH₃ and Q is Q2, and R and R⁴ are as per any row of Table U below, which compounds are named I.A2-Z171-Q2-U-1 to I.A2-Z171-Q2-U-42.

In another embodiment, compounds I are of formula I.A2 wherein Z is Z-171, R³ is CH₃ and Q is Q3, and R and R⁴ are as per any row of Table U below, which compounds are named I.A2-Z171-Q3-U-1 to I.A2-Z171-Q3-U-42.

In another embodiment, compounds I are of formula I.A2 wherein Z is Z-171, R³ is CH₃ and Q is Q4, and R and R⁴ are as per any row of Table U below, which compounds are named I.A2-Z171-Q4-U-1 to I.A2-Z171-Q4-U-42.

In another embodiment, compounds I are of formula I.A1 wherein Z is Z-172, R³ is CH₃ and Q is Q1, and R and R⁴ are as per any row of Table U below, which compounds are named I.A1-Z172-Q1-U-1 to I.A1-Z172-Q1-U-42.

In another embodiment, compounds I are of formula I.A1 wherein Z is Z-172, R³ is CH₃ and Q is Q2, and R and R⁴ are as per any row of Table U below, which compounds are named I.A1-Z172-Q2-U-1 to I.A1-Z172-Q2-U-42.

In another embodiment, compounds I are of formula I.A1 wherein Z is Z-172, R³ is CH₃ and Q is Q3, and R and R⁴ are as per any row of Table U below, which compounds are named I.A1-Z172-Q3-U-1 to I.A1-Z172-Q3-U-42.

In another embodiment, compounds I are of formula I.A1 wherein Z is Z-172, R³ is CH₃ and Q is Q4, and R and R⁴ are as per any row of Table U below, which compounds are named I.A1-Z172-Q4-U-1 to I.A1-Z172-Q4-U-42.

In another embodiment, compounds I are of formula I.A2 wherein Z is Z-172, R³ is CH₃ and Q is Q1, and R and R⁴ are as per any row of Table U below, which compounds are named I.A2-Z172-Q1-U-1 to I.A2-Z172-Q1-U-42.

In another embodiment, compounds I are of formula I.A2 wherein Z is Z-172, R³ is CH₃ and Q is Q2, and R and R⁴ are as per any row of Table U below, which compounds are named I.A2-Z172-Q2-U-1 to I.A2-Z172-Q2-U-42.

In another embodiment, compounds I are of formula I.A2 wherein Z is Z-172, R³ is CH₃ and Q is Q3, and R and R⁴ are as per any row of Table U below, which compounds are named I.A2-Z172-Q3-U-1 to I.A2-Z172-Q3-U-42.

In another embodiment, compounds I are of formula I.A2 wherein Z is Z-172, R³ is CH₃ and Q is Q4, and R and R⁴ are as per any row of Table U below, which compounds are named I.A2-Z172-Q4-U-1 to I.A2-Z172-Q4-U-42.

In another embodiment, compounds I are of formula I.A1 wherein Z is Z-173, R³ is CH₃ and Q is Q1, and R and R⁴ are as per any row of Table U below, which compounds are named I.A1-Z173-Q1-U-1 to I.A1-Z173-Q1-U-42.

In another embodiment, compounds I are of formula I.A1 wherein Z is Z-173, R³ is CH₃ and Q is Q2, and R and R⁴ are as per any row of Table U below, which compounds are named I.A1-Z173-Q2-U-1 to I.A1-Z173-Q2-U-42.

In another embodiment, compounds I are of formula I.A1 wherein Z is Z-173, R³ is CH₃ and Q is Q3, and R and R⁴ are as per any row of Table U below, which compounds are named I.A1-Z173-Q3-U-1 to I.A1-Z173-Q3-U-42.

In another embodiment, compounds I are of formula I.A1 wherein Z is Z-173, R³ is CH₃ and Q is Q4, and R and R⁴ are as per any row of Table U below, which compounds are named I.A1-Z173-Q4-U-1 to I.A1-Z173-Q4-U-42.

In another embodiment, compounds I are of formula I.A2 wherein Z is Z-173, R³ is CH₃ and Q is Q1, and R and R⁴ are as per any row of Table U below, which compounds are named I.A2-Z173-Q1-U-1 to I.A2-Z173-Q1-U-42.

In another embodiment, compounds I are of formula I.A2 wherein Z is Z-173, R³ is CH₃ and Q is Q2, and R and R⁴ are as per any row of Table U below, which compounds are named I.A2-Z173-Q2-U-1 to I.A2-Z173-Q2-U-42.

In another embodiment, compounds I are of formula I.A2 wherein Z is Z-173, R³ is CH₃ and Q is Q3, and R and R⁴ are as per any row of Table U below, which compounds are named I.A2-Z173-Q3-U-1 to I.A2-Z173-Q3-U-42.

In another embodiment, compounds I are of formula I.A2 wherein Z is Z-173, R³ is CH₃ and Q is Q4, and R and R⁴ are as per any row of Table U below, which compounds are named I.A2-Z173-Q4-U-1 to I.A2-Z173-Q4-U-42.

In another embodiment, compounds I are of formula I.A1 wherein Z is Z-174, R³ is CH₃ and Q is Q1, and R and R⁴ are as per any row of Table U below, which compounds are named I.A1-Z174-Q1-U-1 to I.A1-Z174-Q1-U-42.

In another embodiment, compounds I are of formula I.A1 wherein Z is Z-174, R³ is CH₃ and Q is Q2, and R and R⁴ are as per any row of Table U below, which compounds are named I.A1-Z174-Q2-U-1 to I.A1-Z174-Q2-U-42.

In another embodiment, compounds I are of formula I.A1 wherein Z is Z-174, R³ is CH₃ and Q is Q3, and R and R⁴ are as per any row of Table U below, which compounds are named I.A1-Z174-Q3-U-1 to I.A1-Z174-Q3-U-42.

In another embodiment, compounds I are of formula I.A1 wherein Z is Z-174, R³ is CH₃ and Q is Q4, and R and R⁴ are as per any row of Table U below, which compounds are named I.A1-Z174-Q4-U-1 to I.A1-Z174-Q4-U-42.

In another embodiment, compounds I are of formula I.A2 wherein Z is Z-174, R³ is CH₃ and Q is Q1, and R and R⁴ are as per any row of Table U below, which compounds are named I.A2-Z174-Q1-U-1 to I.A2-Z174-Q1-U-42.

In another embodiment, compounds I are of formula I.A2 wherein Z is Z-174, R³ is CH₃ and Q is Q2, and R and R⁴ are as per any row of Table U below, which compounds are named I.A2-Z174-Q2-U-1 to I.A2-Z174-Q2-U-42.

In another embodiment, compounds I are of formula I.A2 wherein Z is Z-174, R³ is CH₃ and Q is Q3, and R and R⁴ are as per any row of Table U below, which compounds are named I.A2-Z174-Q3-U-1 to I.A2-Z174-Q3-U-42.

In another embodiment, compounds I are of formula I.A2 wherein Z is Z-174, R³ is CH₃ and Q is Q4, and R and R⁴ are as per any row of Table U below, which compounds are named I.A2-Z174-Q4-U-1 to I.A2-Z174-Q4-U-42.

In another embodiment, compounds I are of formula I.A1 wherein Z is Z-175, R³ is CH₃ and Q is Q1, and R and R⁴ are as per any row of Table U below, which compounds are named I.A1-Z175-Q1-U-1 to I.A1-Z175-Q1-U-42.

In another embodiment, compounds I are of formula I.A1 wherein Z is Z-175, R³ is CH₃ and Q is Q2, and R and R⁴ are as per any row of Table U below, which compounds are named I.A1-Z175-Q2-U-1 to I.A1-Z175-Q2-U-42.

In another embodiment, compounds I are of formula I.A1 wherein Z is Z-175, R³ is CH₃ and Q is Q3, and R and R⁴ are as per any row of Table U below, which compounds are named I.A1-Z175-Q3-U-1 to I.A1-Z175-Q3-U-42.

In another embodiment, compounds I are of formula I.A1 wherein Z is Z-175, R³ is CH₃ and Q is Q4, and R and R⁴ are as per any row of Table U below, which compounds are named I.A1-Z175-Q4-U-1 to I.A1-Z175-Q4-U-42.

In another embodiment, compounds I are of formula I.A2 wherein Z is Z-175, R³ is CH₃ and Q is Q1, and R and R⁴ are as per any row of Table U below, which compounds are named I.A2-Z175-Q1-U-1 to I.A2-Z175-Q1-U-42.

In another embodiment, compounds I are of formula I.A2 wherein Z is Z-175, R³ is CH₃ and Q is Q2, and R and R⁴ are as per any row of Table U below, which compounds are named I.A2-Z175-Q2-U-1 to I.A2-Z175-Q2-U-42.

In another embodiment, compounds I are of formula I.A2 wherein Z is Z-175, R³ is CH₃ and Q is Q3, and R and R⁴ are as per any row of Table U below, which compounds are named I.A2-Z175-Q3-U-1 to I.A2-Z175-Q3-U-42.

In another embodiment, compounds I are of formula I.A2 wherein Z is Z-175, R³ is CH₃ and Q is Q4, and R and R⁴ are as per any row of Table U below, which compounds are named I.A2-Z175-Q4-U-1 to I.A2-Z175-Q4-U-42.

In another embodiment, compounds I are of formula I.A1 wherein Z is Z-176, R³ is CH₃ and Q is Q1, and R and R⁴ are as per any row of Table U below, which compounds are named I.A1-Z176-Q1-U-1 to I.A1-Z176-Q1-U-42.

In another embodiment, compounds I are of formula I.A1 wherein Z is Z-176, R³ is CH₃ and Q is Q2, and R and R⁴ are as per any row of Table U below, which compounds are named I.A1-Z176-Q2-U-1 to I.A1-Z176-Q2-U-42.

In another embodiment, compounds I are of formula I.A1 wherein Z is Z-176, R³ is CH₃ and Q is Q3, and R and R⁴ are as per any row of Table U below, which compounds are named I.A1-Z176-Q3-U-1 to I.A1-Z176-Q3-U-42.

In another embodiment, compounds I are of formula I.A1 wherein Z is Z-176, R³ is CH₃ and Q is Q4, and R and R⁴ are as per any row of Table U below, which compounds are named I.A1-Z176-Q4-U-1 to I.A1-Z176-Q4-U-42.

In another embodiment, compounds I are of formula I.A2 wherein Z is Z-176, R³ is CH₃ and Q is Q1, and R and R⁴ are as per any row of Table U below, which compounds are named I.A2-Z176-Q1-U-1 to I.A2-Z176-Q1-U-42.

In another embodiment, compounds I are of formula I.A2 wherein Z is Z-176, R³ is CH₃ and Q is Q2, and R and R⁴ are as per any row of Table U below, which compounds are named I.A2-Z176-Q2-U-1 to I.A2-Z176-Q2-U-42.

In another embodiment, compounds I are of formula I.A2 wherein Z is Z-176, R³ is CH₃ and Q is Q3, and R and R⁴ are as per any row of Table U below, which compounds are named I.A2-Z176-Q3-U-1 to I.A2-Z176-Q3-U-42.

In another embodiment, compounds I are of formula I.A2 wherein Z is Z-176, R³ is CH₃ and Q is Q4, and R and R⁴ are as per any row of Table U below, which compounds are named I.A2-Z176-Q4-U-1 to I.A2-Z176-Q4-U-42.

In another embodiment, compounds I are of formula I.A1 wherein Z is Z-177, R³ is CH₃ and Q is Q1, and R and R⁴ are as per any row of Table U below, which compounds are named I.A1-Z177-Q1-U-1 to I.A1-Z177-Q1-U-42.

In another embodiment, compounds I are of formula I.A1 wherein Z is Z-177, R³ is CH₃ and Q is Q2, and R and R⁴ are as per any row of Table U below, which compounds are named I.A1-Z177-Q2-U-1 to I.A1-Z177-Q2-U-42.

In another embodiment, compounds I are of formula I.A1 wherein Z is Z-177, R³ is CH₃ and Q is Q3, and R and R⁴ are as per any row of Table U below, which compounds are named I.A1-Z177-Q3-U-1 to I.A1-Z177-Q3-U-42.

In another embodiment, compounds I are of formula I.A1 wherein Z is Z-177, R³ is CH₃ and Q is Q4, and R and R⁴ are as per any row of Table U below, which compounds are named I.A1-Z177-Q4-U-1 to I.A1-Z177-Q4-U-42.

In another embodiment, compounds I are of formula I.A2 wherein Z is Z-177, R³ is CH₃ and Q is Q1, and R and R⁴ are as per any row of Table U below, which compounds are named I.A2-Z177-Q1-U-1 to I.A2-Z177-Q1-U-42.

In another embodiment, compounds I are of formula I.A2 wherein Z is Z-177, R³ is CH₃ and Q is Q2, and R and R⁴ are as per any row of Table U below, which compounds are named I.A2-Z177-Q2-U-1 to I.A2-Z177-Q2-U-42.

In another embodiment, compounds I are of formula I.A2 wherein Z is Z-177, R³ is CH₃ and Q is Q3, and R and R⁴ are as per any row of Table U below, which compounds are named I.A2-Z177-Q3-U-1 to I.A2-Z177-Q3-U-42.

In another embodiment, compounds I are of formula I.A2 wherein Z is Z-177, R³ is CH₃ and Q is Q4, and R and R⁴ are as per any row of Table U below, which compounds are named I.A2-Z177-Q4-U-1 to I.A2-Z177-Q4-U-42.

In another embodiment, compounds I are of formula I.A1 wherein Z is Z-178, R³ is CH₃ and Q is Q1, and R and R⁴ are as per any row of Table U below, which compounds are named I.A1-Z178-Q1-U-1 to I.A1-Z178-Q1-U-42.

In another embodiment, compounds I are of formula I.A1 wherein Z is Z-178, R³ is CH₃ and Q is Q2, and R and R⁴ are as per any row of Table U below, which compounds are named I.A1-Z178-Q2-U-1 to I.A1-Z178-Q2-U-42.

In another embodiment, compounds I are of formula I.A1 wherein Z is Z-178, R³ is CH₃ and Q is Q3, and R and R⁴ are as per any row of Table U below, which compounds are named I.A1-Z178-Q3-U-1 to I.A1-Z178-Q3-U-42.

In another embodiment, compounds I are of formula I.A1 wherein Z is Z-178, R³ is CH₃ and Q is Q4, and R and R⁴ are as per any row of Table U below, which compounds are named I.A1-Z178-Q4-U-1 to I.A1-Z178-Q4-U-42.

In another embodiment, compounds I are of formula I.A2 wherein Z is Z-178, R³ is CH₃ and Q is Q1, and R and R⁴ are as per any row of Table U below, which compounds are named I.A2-Z178-Q1-U-1 to I.A2-Z178-Q1-U-42.

In another embodiment, compounds I are of formula I.A2 wherein Z is Z-178, R³ is CH₃ and Q is Q2, and R and R⁴ are as per any row of Table U below, which compounds are named I.A2-Z178-Q2-U-1 to I.A2-Z178-Q2-U-42.

In another embodiment, compounds I are of formula I.A2 wherein Z is Z-178, R³ is CH₃ and Q is Q3, and R and R⁴ are as per any row of Table U below, which compounds are named I.A2-Z178-Q3-U-1 to I.A2-Z178-Q3-U-42.

In another embodiment, compounds I are of formula I.A2 wherein Z is Z-178, R³ is CH₃ and Q is Q4, and R and R⁴ are as per any row of Table U below, which compounds are named I.A2-Z178-Q4-U-1 to I.A2-Z178-Q4-U-42.

In another embodiment, compounds I are of formula I.A1 wherein Z is Z-179, R³ is CH₃ and Q is Q1, and R and R⁴ are as per any row of Table U below, which compounds are named I.A1-Z179-Q1-U-1 to I.A1-Z179-Q1-U-42.

In another embodiment, compounds I are of formula I.A1 wherein Z is Z-179, R³ is CH₃ and Q is Q2, and R and R⁴ are as per any row of Table U below, which compounds are named I.A1-Z179-Q2-U-1 to I.A1-Z179-Q2-U-42.

In another embodiment, compounds I are of formula I.A1 wherein Z is Z-179, R³ is CH₃ and Q is Q3, and R and R⁴ are as per any row of Table U below, which compounds are named I.A1-Z179-Q3-U-1 to I.A1-Z179-Q3-U-42.

In another embodiment, compounds I are of formula I.A1 wherein Z is Z-179, R³ is CH₃ and Q is Q4, and R and R⁴ are as per any row of Table U below, which compounds are named I.A1-Z179-Q4-U-1 to I.A1-Z179-Q4-U-42.

In another embodiment, compounds I are of formula I.A2 wherein Z is Z-179, R³ is CH₃ and Q is Q1, and R and R⁴ are as per any row of Table U below, which compounds are named I.A2-Z179-Q1-U-1 to I.A2-Z179-Q1-U-42.

In another embodiment, compounds I are of formula I.A2 wherein Z is Z-179, R³ is CH₃ and Q is Q2, and R and R⁴ are as per any row of Table U below, which compounds are named I.A2-Z179-Q2-U-1 to I.A2-Z179-Q2-U-42.

In another embodiment, compounds I are of formula I.A2 wherein Z is Z-179, R³ is CH₃ and Q is Q3, and R and R⁴ are as per any row of Table U below, which compounds are named I.A2-Z179-Q3-U-1 to I.A2-Z179-Q3-U-42.

In another embodiment, compounds I are of formula I.A2 wherein Z is Z-179, R³ is CH₃ and Q is Q4, and R and R⁴ are as per any row of Table U below, which compounds are named I.A2-Z179-Q4-U-1 to I.A2-Z179-Q4-U-42.

In another embodiment, compounds I are of formula I.A1 wherein Z is Z-180, R³ is CH₃ and Q is Q1, and R and R⁴ are as per any row of Table U below, which compounds are named I.A1-Z180-Q1-U-1 to I.A1-Z180-Q1-U-42.

In another embodiment, compounds I are of formula I.A1 wherein Z is Z-180, R³ is CH₃ and Q is Q2, and R and R⁴ are as per any row of Table U below, which compounds are named I.A1-Z180-Q2-U-1 to I.A1-Z180-Q2-U-42.

In another embodiment, compounds I are of formula I.A1 wherein Z is Z-180, R³ is CH₃ and Q is Q3, and R and R⁴ are as per any row of Table U below, which compounds are named I.A1-Z180-Q3-U-1 to I.A1-Z180-Q3-U-42.

In another embodiment, compounds I are of formula I.A1 wherein Z is Z-180, R³ is CH₃ and Q is Q4, and R and R⁴ are as per any row of Table U below, which compounds are named I.A1-Z180-Q4-U-1 to I.A1-Z180-Q4-U-42.

In another embodiment, compounds I are of formula I.A2 wherein Z is Z-180, R³ is CH₃ and Q is Q1, and R and R⁴ are as per any row of Table U below, which compounds are named I.A2-Z180-Q1-U-1 to I.A2-Z180-Q1-U-42.

In another embodiment, compounds I are of formula I.A2 wherein Z is Z-180, R³ is CH₃ and Q is Q2, and R and R⁴ are as per any row of Table U below, which compounds are named I.A2-Z180-Q2-U-1 to I.A2-Z180-Q2-U-42.

In another embodiment, compounds I are of formula I.A2 wherein Z is Z-180, R³ is CH₃ and Q is Q3, and R and R⁴ are as per any row of Table U below, which compounds are named I.A2-Z180-Q3-U-1 to I.A2-Z180-Q3-U-42.

In another embodiment, compounds I are of formula I.A2 wherein Z is Z-180, R³ is CH₃ and Q is Q4, and R and R⁴ are as per any row of Table U below, which compounds are named I.A2-Z180-Q4-U-1 to I.A2-Z180-Q4-U-42.

In another embodiment, compounds I are of formula I.A1 wherein Z is Z-181, R³ is CH₃ and Q is Q1, and R and R⁴ are as per any row of Table U below, which compounds are named I.A1-Z181-Q1-U-1 to I.A1-Z181-Q1-U-42.

In another embodiment, compounds I are of formula I.A1 wherein Z is Z-181, R³ is CH₃ and Q is Q2, and R and R⁴ are as per any row of Table U below, which compounds are named I.A1-Z181-Q2-U-1 to I.A1-Z181-Q2-U-42.

In another embodiment, compounds I are of formula I.A1 wherein Z is Z-181, R³ is CH₃ and Q is Q3, and R and R⁴ are as per any row of Table U below, which compounds are named I.A1-Z181-Q3-U-1 to I.A1-Z181-Q3-U-42.

In another embodiment, compounds I are of formula I.A1 wherein Z is Z-181, R³ is CH₃ and Q is Q4, and R and R⁴ are as per any row of Table U below, which compounds are named I.A1-Z181-Q4-U-1 to I.A1-Z181-Q4-U-42.

In another embodiment, compounds I are of formula I.A2 wherein Z is Z-181, R³ is CH₃ and Q is Q1, and R and R⁴ are as per any row of Table U below, which compounds are named I.A2-Z181-Q1-U-1 to I.A2-Z181-Q1-U-42.

In another embodiment, compounds I are of formula I.A2 wherein Z is Z-181, R³ is CH₃ and Q is Q2, and R and R⁴ are as per any row of Table U below, which compounds are named I.A2-Z181-Q2-U-1 to I.A2-Z181-Q2-U-42.

In another embodiment, compounds I are of formula I.A2 wherein Z is Z-181, R³ is CH₃ and Q is Q3, and R and R⁴ are as per any row of Table U below, which compounds are named I.A2-Z181-Q3-U-1 to I.A2-Z181-Q3-U-42.

In another embodiment, compounds I are of formula I.A2 wherein Z is Z-181, R³ is CH₃ and Q is Q4, and R and R⁴ are as per any row of Table U below, which compounds are named I.A2-Z181-Q4-U-1 to I.A2-Z181-Q4-U-42.

In another embodiment, compounds I are of formula I.A1 wherein Z is Z-182, R³ is CH₃ and Q is Q1, and R and R⁴ are as per any row of Table U below, which compounds are named I.A1-Z182-Q1-U-1 to I.A1-Z182-Q1-U-42.

In another embodiment, compounds I are of formula I.A1 wherein Z is Z-182, R³ is CH₃ and Q is Q2, and R and R⁴ are as per any row of Table U below, which compounds are named I.A1-Z182-Q2-U-1 to I.A1-Z182-Q2-U-42.

In another embodiment, compounds I are of formula I.A1 wherein Z is Z-182, R³ is CH₃ and Q is Q3, and R and R⁴ are as per any row of Table U below, which compounds are named I.A1-Z182-Q3-U-1 to I.A1-Z182-Q3-U-42.

In another embodiment, compounds I are of formula I.A1 wherein Z is Z-182, R³ is CH₃ and Q is Q4, and R and R⁴ are as per any row of Table U below, which compounds are named I.A1-Z182-Q4-U-1 to I.A1-Z182-Q4-U-42.

In another embodiment, compounds I are of formula I.A2 wherein Z is Z-182, R³ is CH₃ and Q is Q1, and R and R⁴ are as per any row of Table U below, which compounds are named I.A2-Z182-Q1-U-1 to I.A2-Z182-Q1-U-42.

In another embodiment, compounds I are of formula I.A2 wherein Z is Z-182, R³ is CH₃ and Q is Q2, and R and R⁴ are as per any row of Table U below, which compounds are named I.A2-Z182-Q2-U-1 to I.A2-Z182-Q2-U-42.

In another embodiment, compounds I are of formula I.A2 wherein Z is Z-182, R³ is CH₃ and Q is Q3, and R and R⁴ are as per any row of Table U below, which compounds are named I.A2-Z182-Q3-U-1 to I.A2-Z182-Q3-U-42.

In another embodiment, compounds I are of formula I.A2 wherein Z is Z-182, R³ is CH₃ and Q is Q4, and R and R⁴ are as per any row of Table U below, which compounds are named I.A2-Z182-Q4-U-1 to I.A2-Z182-Q4-U-42.

In another embodiment, compounds I are of formula I.A1 wherein Z is Z-183, R³ is CH₃ and Q is Q1, and R and R⁴ are as per any row of Table U below, which compounds are named I.A1-Z183-Q1-U-1 to I.A1-Z183-Q1-U-42.

In another embodiment, compounds I are of formula I.A1 wherein Z is Z-183, R³ is CH₃ and Q is Q2, and R and R⁴ are as per any row of Table U below, which compounds are named I.A1-Z183-Q2-U-1 to I.A1-Z183-Q2-U-42.

In another embodiment, compounds I are of formula I.A1 wherein Z is Z-183, R³ is CH₃ and Q is Q3, and R and R⁴ are as per any row of Table U below, which compounds are named I.A1-Z183-Q3-U-1 to I.A1-Z183-Q3-U-42.

In another embodiment, compounds I are of formula I.A1 wherein Z is Z-183, R³ is CH₃ and Q is Q4, and R and R⁴ are as per any row of Table U below, which compounds are named I.A1-Z183-Q4-U-1 to I.A1-Z183-Q4-U-42.

In another embodiment, compounds I are of formula I.A2 wherein Z is Z-183, R³ is CH₃ and Q is Q1, and R and R⁴ are as per any row of Table U below, which compounds are named I.A2-Z183-Q1-U-1 to I.A2-Z183-Q1-U-42.

In another embodiment, compounds I are of formula I.A2 wherein Z is Z-183, R³ is CH₃ and Q is Q2, and R and R⁴ are as per any row of Table U below, which compounds are named I.A2-Z183-Q2-U-1 to I.A2-Z183-Q2-U-42.

In another embodiment, compounds I are of formula I.A2 wherein Z is Z-183, R³ is CH₃ and Q is Q3, and R and R⁴ are as per any row of Table U below, which compounds are named I.A2-Z183-Q3-U-1 to I.A2-Z183-Q3-U-42.

In another embodiment, compounds I are of formula I.A2 wherein Z is Z-183, R³ is CH₃ and Q is Q4, and R and R⁴ are as per any row of Table U below, which compounds are named I.A2-Z183-Q4-U-1 to I.A2-Z183-Q4-U-42.

In another embodiment, compounds I are of formula I.A1 wherein Z is Z-184, R³ is CH₃ and Q is Q1, and R and R⁴ are as per any row of Table U below, which compounds are named I.A1-Z184-Q1-U-1 to I.A1-Z184-Q1-U-42.

In another embodiment, compounds I are of formula I.A1 wherein Z is Z-184, R³ is CH₃ and Q is Q2, and R and R⁴ are as per any row of Table U below, which compounds are named I.A1-Z184-Q2-U-1 to I.A1-Z184-Q2-U-42.

In another embodiment, compounds I are of formula I.A1 wherein Z is Z-184, R³ is CH₃ and Q is Q3, and R and R⁴ are as per any row of Table U below, which compounds are named I.A1-Z184-Q3-U-1 to I.A1-Z184-Q3-U-42.

In another embodiment, compounds I are of formula I.A1 wherein Z is Z-184, R³ is CH₃ and Q is Q4, and R and R⁴ are as per any row of Table U below, which compounds are named I.A1-Z184-Q4-U-1 to I.A1-Z184-Q4-U-42.

In another embodiment, compounds I are of formula I.A2 wherein Z is Z-184, R³ is CH₃ and Q is Q1, and R and R⁴ are as per any row of Table U below, which compounds are named I.A2-Z184-Q1-U-1 to I.A2-Z184-Q1-U-42.

In another embodiment, compounds I are of formula I.A2 wherein Z is Z-184, R³ is CH₃ and Q is Q2, and R and R⁴ are as per any row of Table U below, which compounds are named I.A2-Z184-Q2-U-1 to I.A2-Z184-Q2-U-42.

In another embodiment, compounds I are of formula I.A2 wherein Z is Z-184, R³ is CH₃ and Q is Q3, and R and R⁴ are as per any row of Table U below, which compounds are named I.A2-Z184-Q3-U-1 to I.A2-Z184-Q3-U-42.

In another embodiment, compounds I are of formula I.A2 wherein Z is Z-184, R³ is CH₃ and Q is Q4, and R and R⁴ are as per any row of Table U below, which compounds are named I.A2-Z184-Q4-U-1 to I.A2-Z184-Q4-U-42.

In another embodiment, compounds I are of formula I.A1 wherein Z is Z-185, R³ is CH₃ and Q is Q1, and R and R⁴ are as per any row of Table U below, which compounds are named I.A1-Z185-Q1-U-1 to I.A1-Z185-Q1-U-42.

In another embodiment, compounds I are of formula I.A1 wherein Z is Z-185, R³ is CH₃ and Q is Q2, and R and R⁴ are as per any row of Table U below, which compounds are named I.A1-Z185-Q2-U-1 to I.A1-Z185-Q2-U-42.

In another embodiment, compounds I are of formula I.A1 wherein Z is Z-185, R³ is CH₃ and Q is Q3, and R and R⁴ are as per any row of Table U below, which compounds are named I.A1-Z185-Q3-U-1 to I.A1-Z185-Q3-U-42.

In another embodiment, compounds I are of formula I.A1 wherein Z is Z-185, R³ is CH₃ and Q is Q4, and R and R⁴ are as per any row of Table U below, which compounds are named I.A1-Z185-Q4-U-1 to I.A1-Z185-Q4-U-42.

In another embodiment, compounds I are of formula I.A2 wherein Z is Z-185, R³ is CH₃ and Q is Q1, and R and R⁴ are as per any row of Table U below, which compounds are named I.A2-Z185-Q1-U-1 to I.A2-Z185-Q1-U-42.

In another embodiment, compounds I are of formula I.A2 wherein Z is Z-185, R³ is CH₃ and Q is Q2, and R and R⁴ are as per any row of Table U below, which compounds are named I.A2-Z185-Q2-U-1 to I.A2-Z185-Q2-U-42.

In another embodiment, compounds I are of formula I.A2 wherein Z is Z-185, R³ is CH₃ and Q is Q3, and R and R⁴ are as per any row of Table U below, which compounds are named I.A2-Z185-Q3-U-1 to I.A2-Z185-Q3-U-42.

In another embodiment, compounds I are of formula I.A2 wherein Z is Z-185, R³ is CH₃ and Q is Q4, and R and R⁴ are as per any row of Table U below, which compounds are named I.A2-Z185-Q4-U-1 to I.A2-Z185-Q4-U-42.

In another embodiment, compounds I are of formula I.A1 wherein Z is Z-186, R³ is CH₃ and Q is Q1, and R and R⁴ are as per any row of Table U below, which compounds are named I.A1-Z186-Q1-U-1 to I.A1-Z186-Q1-U-42.

In another embodiment, compounds I are of formula I.A1 wherein Z is Z-186, R³ is CH₃ and Q is Q2, and R and R⁴ are as per any row of Table U below, which compounds are named I.A1-Z186-Q2-U-1 to I.A1-Z186-Q2-U-42.

In another embodiment, compounds I are of formula I.A1 wherein Z is Z-186, R³ is CH₃ and Q is Q3, and R and R⁴ are as per any row of Table U below, which compounds are named I.A1-Z186-Q3-U-1 to I.A1-Z186-Q3-U-42.

In another embodiment, compounds I are of formula I.A1 wherein Z is Z-186, R³ is CH₃ and Q is Q4, and R and R⁴ are as per any row of Table U below, which compounds are named I.A1-Z186-Q4-U-1 to I.A1-Z186-Q4-U-42.

In another embodiment, compounds I are of formula I.A2 wherein Z is Z-186, R³ is CH₃ and Q is Q1, and R and R⁴ are as per any row of Table U below, which compounds are named I.A2-Z186-Q1-U-1 to I.A2-Z186-Q1-U-42.

In another embodiment, compounds I are of formula I.A2 wherein Z is Z-186, R³ is CH₃ and Q is Q2, and R and R⁴ are as per any row of Table U below, which compounds are named I.A2-Z186-Q2-U-1 to I.A2-Z186-Q2-U-42.

In another embodiment, compounds I are of formula I.A2 wherein Z is Z-186, R³ is CH₃ and Q is Q3, and R and R⁴ are as per any row of Table U below, which compounds are named I.A2-Z186-Q3-U-1 to I.A2-Z186-Q3-U-42.

In another embodiment, compounds I are of formula I.A2 wherein Z is Z-186, R³ is CH₃ and Q is Q4, and R and R⁴ are as per any row of Table U below, which compounds are named I.A2-Z186-Q4-U-1 to I.A2-Z186-Q4-U-42.

In another embodiment, compounds I are of formula I.A1 wherein Z is Z-187, R³ is CH₃ and Q is Q1, and R and R⁴ are as per any row of Table U below, which compounds are named I.A1-Z187-Q1-U-1 to I.A1-Z187-Q1-U-42.

In another embodiment, compounds I are of formula I.A1 wherein Z is Z-187, R³ is CH₃ and Q is Q2, and R and R⁴ are as per any row of Table U below, which compounds are named I.A1-Z187-Q2-U-1 to I.A1-Z187-Q2-U-42.

In another embodiment, compounds I are of formula I.A1 wherein Z is Z-187, R³ is CH₃ and Q is Q3, and R and R⁴ are as per any row of Table U below, which compounds are named I.A1-Z187-Q3-U-1 to I.A1-Z187-Q3-U-42.

In another embodiment, compounds I are of formula I.A1 wherein Z is Z-187, R³ is CH₃ and Q is Q4, and R and R⁴ are as per any row of Table U below, which compounds are named I.A1-Z187-Q4-U-1 to I.A1-Z187-Q4-U-42.

In another embodiment, compounds I are of formula I.A2 wherein Z is Z-187, R³ is CH₃ and Q is Q1, and R and R⁴ are as per any row of Table U below, which compounds are named I.A2-Z187-Q1-U-1 to I.A2-Z187-Q1-U-42.

In another embodiment, compounds I are of formula I.A2 wherein Z is Z-187, R³ is CH₃ and Q is Q2, and R and R⁴ are as per any row of Table U below, which compounds are named I.A2-Z187-Q2-U-1 to I.A2-Z187-Q2-U-42.

In another embodiment, compounds I are of formula I.A2 wherein Z is Z-187, R³ is CH₃ and Q is Q3, and R and R⁴ are as per any row of Table U below, which compounds are named I.A2-Z187-Q3-U-1 to I.A2-Z187-Q3-U-42.

In another embodiment, compounds I are of formula I.A2 wherein Z is Z-187, R³ is CH₃ and Q is Q4, and R and R⁴ are as per any row of Table U below, which compounds are named I.A2-Z187-Q4-U-1 to I.A2-Z187-Q4-U-42.

In another embodiment, compounds I are of formula I.A1 wherein Z is Z-188, R³ is CH₃ and Q is Q1, and R and R⁴ are as per any row of Table U below, which compounds are named I.A1-Z188-Q1-U-1 to I.A1-Z188-Q1-U-42.

In another embodiment, compounds I are of formula I.A1 wherein Z is Z-188, R³ is CH₃ and Q is Q2, and R and R⁴ are as per any row of Table U below, which compounds are named I.A1-Z188-Q2-U-1 to I.A1-Z188-Q2-U-42.

In another embodiment, compounds I are of formula I.A1 wherein Z is Z-188, R³ is CH₃ and Q is Q3, and R and R⁴ are as per any row of Table U below, which compounds are named I.A1-Z188-Q3-U-1 to I.A1-Z188-Q3-U-42.

In another embodiment, compounds I are of formula I.A1 wherein Z is Z-188, R³ is CH₃ and Q is Q4, and R and R⁴ are as per any row of Table U below, which compounds are named I.A1-Z188-Q4-U-1 to I.A1-Z188-Q4-U-42.

In another embodiment, compounds I are of formula I.A2 wherein Z is Z-188, R³ is CH₃ and Q is Q1, and R and R⁴ are as per any row of Table U below, which compounds are named I.A2-Z188-Q1-U-1 to I.A2-Z188-Q1-U-42.

In another embodiment, compounds I are of formula I.A2 wherein Z is Z-188, R³ is CH₃ and Q is Q2, and R and R⁴ are as per any row of Table U below, which compounds are named I.A2-Z188-Q2-U-1 to I.A2-Z188-Q2-U-42.

In another embodiment, compounds I are of formula I.A2 wherein Z is Z-188, R³ is CH₃ and Q is Q3, and R and R⁴ are as per any row of Table U below, which compounds are named I.A2-Z188-Q3-U-1 to I.A2-Z188-Q3-U-42.

In another embodiment, compounds I are of formula I.A2 wherein Z is Z-188, R³ is CH₃ and Q is Q4, and R and R⁴ are as per any row of Table U below, which compounds are named I.A2-Z188-Q4-U-1 to I.A2-Z188-Q4-U-42.

In another embodiment, compounds I are of formula I.A1 wherein Z is Z-189, R³ is CH₃ and Q is Q1, and R and R⁴ are as per any row of Table U below, which compounds are named I.A1-Z189-Q1-U-1 to I.A1-Z189-Q1-U-42.

In another embodiment, compounds I are of formula I.A1 wherein Z is Z-189, R³ is CH₃ and Q is Q2, and R and R⁴ are as per any row of Table U below, which compounds are named I.A1-Z189-Q2-U-1 to I.A1-Z189-Q2-U-42.

In another embodiment, compounds I are of formula I.A1 wherein Z is Z-189, R³ is CH₃ and Q is Q3, and R and R⁴ are as per any row of Table U below, which compounds are named I.A1-Z189-Q3-U-1 to I.A1-Z189-Q3-U-42.

In another embodiment, compounds I are of formula I.A1 wherein Z is Z-189, R³ is CH₃ and Q is Q4, and R and R⁴ are as per any row of Table U below, which compounds are named I.A1-Z189-Q4-U-1 to I.A1-Z189-Q4-U-42.

In another embodiment, compounds I are of formula I.A2 wherein Z is Z-189, R³ is CH₃ and Q is Q1, and R and R⁴ are as per any row of Table U below, which compounds are named I.A2-Z189-Q1-U-1 to I.A2-Z189-Q1-U-42.

In another embodiment, compounds I are of formula I.A2 wherein Z is Z-189, R³ is CH₃ and Q is Q2, and R and R⁴ are as per any row of Table U below, which compounds are named I.A2-Z189-Q2-U-1 to I.A2-Z189-Q2-U-42.

In another embodiment, compounds I are of formula I.A2 wherein Z is Z-189, R³ is CH₃ and Q is Q3, and R and R⁴ are as per any row of Table U below, which compounds are named I.A2-Z189-Q3-U-1 to I.A2-Z189-Q3-U-42.

In another embodiment, compounds I are of formula I.A2 wherein Z is Z-189, R³ is CH₃ and Q is Q4, and R and R⁴ are as per any row of Table U below, which compounds are named I.A2-Z189-Q4-U-1 to I.A2-Z189-Q4-U-42.

In another embodiment, compounds I are of formula I.A1 wherein Z is Z-190, R³ is CH₃ and Q is Q1, and R and R⁴ are as per any row of Table U below, which compounds are named I.A1-Z190-Q1-U-1 to I.A1-Z190-Q1-U-42.

In another embodiment, compounds I are of formula I.A1 wherein Z is Z-190, R³ is CH₃ and Q is Q2, and R and R⁴ are as per any row of Table U below, which compounds are named I.A1-Z190-Q2-U-1 to I.A1-Z190-Q2-U-42.

In another embodiment, compounds I are of formula I.A1 wherein Z is Z-190, R³ is CH₃ and Q is Q3, and R and R⁴ are as per any row of Table U below, which compounds are named I.A1-Z190-Q3-U-1 to I.A1-Z190-Q3-U-42.

In another embodiment, compounds I are of formula I.A1 wherein Z is Z-190, R³ is CH₃ and Q is Q4, and R and R⁴ are as per any row of Table U below, which compounds are named I.A1-Z190-Q4-U-1 to I.A1-Z190-Q4-U-42.

In another embodiment, compounds I are of formula I.A2 wherein Z is Z-190, R³ is CH₃ and Q is Q1, and R and R⁴ are as per any row of Table U below, which compounds are named I.A2-Z190-Q1-U-1 to I.A2-Z190-Q1-U-42.

In another embodiment, compounds I are of formula I.A2 wherein Z is Z-190, R³ is CH₃ and Q is Q2, and R and R⁴ are as per any row of Table U below, which compounds are named I.A2-Z190-Q2-U-1 to I.A2-Z190-Q2-U-42.

In another embodiment, compounds I are of formula I.A2 wherein Z is Z-190, R³ is CH₃ and Q is Q3, and R and R⁴ are as per any row of Table U below, which compounds are named I.A2-Z190-Q3-U-1 to I.A2-Z190-Q3-U-42.

In another embodiment, compounds I are of formula I.A2 wherein Z is Z-190, R³ is CH₃ and Q is Q4, and R and R⁴ are as per any row of Table U below, which compounds are named I.A2-Z190-Q4-U-1 to I.A2-Z190-Q4-U-42.

In another embodiment, compounds I are of formula I.A1 wherein Z is Z-191, R³ is CH₃ and Q is Q1, and R and R⁴ are as per any row of Table U below, which compounds are named I.A1-Z191-Q1-U-1 to I.A1-Z191-Q1-U-42.

In another embodiment, compounds I are of formula I.A1 wherein Z is Z-191, R³ is CH₃ and Q is Q2, and R and R⁴ are as per any row of Table U below, which compounds are named I.A1-Z191-Q2-U-1 to I.A1-Z191-Q2-U-42.

In another embodiment, compounds I are of formula I.A1 wherein Z is Z-191, R³ is CH₃ and Q is Q3, and R and R⁴ are as per any row of Table U below, which compounds are named I.A1-Z191-Q3-U-1 to I.A1-Z191-Q3-U-42.

In another embodiment, compounds I are of formula I.A1 wherein Z is Z-191, R³ is CH₃ and Q is Q4, and R and R⁴ are as per any row of Table U below, which compounds are named I.A1-Z191-Q4-U-1 to I.A1-Z191-Q4-U-42.

In another embodiment, compounds I are of formula I.A2 wherein Z is Z-191, R³ is CH₃ and Q is Q1, and R and R⁴ are as per any row of Table U below, which compounds are named I.A2-Z191-Q1-U-1 to I.A2-Z191-Q1-U-42.

In another embodiment, compounds I are of formula I.A2 wherein Z is Z-191, R³ is CH₃ and Q is Q2, and R and R⁴ are as per any row of Table U below, which compounds are named I.A2-Z191-Q2-U-1 to I.A2-Z191-Q2-U-42.

In another embodiment, compounds I are of formula I.A2 wherein Z is Z-191, R³ is CH₃ and Q is Q3, and R and R⁴ are as per any row of Table U below, which compounds are named I.A2-Z191-Q3-U-1 to I.A2-Z191-Q3-U-42.

In another embodiment, compounds I are of formula I.A2 wherein Z is Z-191, R³ is CH₃ and Q is Q4, and R and R⁴ are as per any row of Table U below, which compounds are named I.A2-Z191-Q4-U-1 to I.A2-Z191-Q4-U-42.

In another embodiment, compounds I are of formula I.A1 wherein Z is Z-192, R³ is CH₃ and Q is Q1, and R and R⁴ are as per any row of Table U below, which compounds are named I.A1-Z192-Q1-U-1 to I.A1-Z192-Q1-U-42.

In another embodiment, compounds I are of formula I.A1 wherein Z is Z-192, R³ is CH₃ and Q is Q2, and R and R⁴ are as per any row of Table U below, which compounds are named I.A1-Z192-Q2-U-1 to I.A1-Z192-Q2-U-42.

In another embodiment, compounds I are of formula I.A1 wherein Z is Z-192, R³ is CH₃ and Q is Q3, and R and R⁴ are as per any row of Table U below, which compounds are named I.A1-Z192-Q3-U-1 to I.A1-Z192-Q3-U-42.

In another embodiment, compounds I are of formula I.A1 wherein Z is Z-192, R³ is CH₃ and Q is Q4, and R and R⁴ are as per any row of Table U below, which compounds are named I.A1-Z192-Q4-U-1 to I.A1-Z192-Q4-U-42.

In another embodiment, compounds I are of formula I.A2 wherein Z is Z-192, R³ is CH₃ and Q is Q1, and R and R⁴ are as per any row of Table U below, which compounds are named I.A2-Z192-Q1-U-1 to I.A2-Z192-Q1-U-42.

In another embodiment, compounds I are of formula I.A2 wherein Z is Z-192, R³ is CH₃ and Q is Q2, and R and R⁴ are as per any row of Table U below, which compounds are named I.A2-Z192-Q2-U-1 to I.A2-Z192-Q2-U-42.

In another embodiment, compounds I are of formula I.A2 wherein Z is Z-192, R³ is CH₃ and Q is Q3, and R and R⁴ are as per any row of Table U below, which compounds are named I.A2-Z192-Q3-U-1 to I.A2-Z192-Q3-U-42.

In another embodiment, compounds I are of formula I.A2 wherein Z is Z-192, R³ is CH₃ and Q is Q4, and R and R⁴ are as per any row of Table U below, which compounds are named I.A2-Z192-Q4-U-1 to I.A2-Z192-Q4-U-42.

In another embodiment, compounds I are of formula I.A1 wherein Z is Z-193, R³ is CH₃ and Q is Q1, and R and R⁴ are as per any row of Table U below, which compounds are named I.A1-Z193-Q1-U-1 to I.A1-Z193-Q1-U-42.

In another embodiment, compounds I are of formula I.A1 wherein Z is Z-193, R³ is CH₃ and Q is Q2, and R and R⁴ are as per any row of Table U below, which compounds are named I.A1-Z193-Q2-U-1 to I.A1-Z193-Q2-U-42.

In another embodiment, compounds I are of formula I.A1 wherein Z is Z-193, R³ is CH₃ and Q is Q3, and R and R⁴ are as per any row of Table U below, which compounds are named I.A1-Z193-Q3-U-1 to I.A1-Z193-Q3-U-42.

In another embodiment, compounds I are of formula I.A1 wherein Z is Z-193, R³ is CH₃ and Q is Q4, and R and R⁴ are as per any row of Table U below, which compounds are named I.A1-Z193-Q4-U-1 to I.A1-Z193-Q4-U-42.

In another embodiment, compounds I are of formula I.A2 wherein Z is Z-193, R³ is CH₃ and Q is Q1, and R and R⁴ are as per any row of Table U below, which compounds are named I.A2-Z193-Q1-U-1 to LA2-Z193-Q1-U-42.

In another embodiment, compounds I are of formula I.A2 wherein Z is Z-193, R³ is CH₃ and Q is Q2, and R and R⁴ are as per any row of Table U below, which compounds are named I.A2-Z193-Q2-U-1 to I.A2-Z193-Q2-U-42.

In another embodiment, compounds I are of formula I.A2 wherein Z is Z-193, R³ is CH₃ and Q is Q3, and R and R⁴ are as per any row of Table U below, which compounds are named I.A2-Z193-Q3-U-1 to I.A2-Z193-Q3-U-42.

In another embodiment, compounds I are of formula I.A2 wherein Z is Z-193, R³ is CH₃ and Q is Q4, and R and R⁴ are as per any row of Table U below, which compounds are named I.A2-Z193-Q4-U-1 to I.A2-Z193-Q4-U-42.

In another embodiment, compounds I are of formula I.A1 wherein Z is Z-194, R³ is CH₃ and Q is Q1, and R and R⁴ are as per any row of Table U below, which compounds are named I.A1-Z194-Q1-U-1 to I.A1-Z194-Q1-U-42.

In another embodiment, compounds I are of formula I.A1 wherein Z is Z-194, R³ is CH₃ and Q is Q2, and R and R⁴ are as per any row of Table U below, which compounds are named I.A1-Z194-Q2-U-1 to I.A1-Z194-Q2-U-42.

In another embodiment, compounds I are of formula I.A1 wherein Z is Z-194, R³ is CH₃ and Q is Q3, and R and R⁴ are as per any row of Table U below, which compounds are named I.A1-Z194-Q3-U-1 to I.A1-Z194-Q3-U-42.

In another embodiment, compounds I are of formula I.A1 wherein Z is Z-194, R³ is CH₃ and Q is Q4, and R and R⁴ are as per any row of Table U below, which compounds are named I.A1-Z194-Q4-U-1 to lA1-Z194-Q4-U-42.

In another embodiment, compounds I are of formula I.A2 wherein Z is Z-194, R³ is CH₃ and Q is Q1, and R and R⁴ are as per any row of Table U below, which compounds are named I.A2-Z194-Q1-U-1 to I.A2-Z194-Q1-U-42.

In another embodiment, compounds I are of formula I.A2 wherein Z is Z-194, R³ is CH₃ and Q is Q2, and R and R⁴ are as per any row of Table U below, which compounds are named I.A2-Z194-Q2-U-1 to I.A2-Z194-Q2-U-42.

In another embodiment, compounds I are of formula I.A2 wherein Z is Z-194, R³ is CH₃ and Q is Q3, and R and R⁴ are as per any row of Table U below, which compounds are named I.A2-Z194-Q3-U-1 to I.A2-Z194-Q3-U-42.

In another embodiment, compounds I are of formula I.A2 wherein Z is Z-194, R³ is CH₃ and Q is Q4, and R and R⁴ are as per any row of Table U below, which compounds are named I.A2-Z194-Q4-U-1 to I.A2-Z194-Q4-U-42.

In another embodiment, compounds I are of formula I.A1 wherein Z is Z-195, R³ is CH₃ and Q is Q1, and R and R⁴ are as per any row of Table U below, which compounds are named I.A1-Z195-Q1-U-1 to I.A1-Z195-Q1-U-42.

In another embodiment, compounds I are of formula I.A1 wherein Z is Z-195, R³ is CH₃ and Q is Q2, and R and R⁴ are as per any row of Table U below, which compounds are named I.A1-Z195-Q2-U-1 to I.A1-Z195-Q2-U-42.

In another embodiment, compounds I are of formula I.A1 wherein Z is Z-195, R³ is CH₃ and Q is Q3, and R and R⁴ are as per any row of Table U below, which compounds are named I.A1-Z195-Q3-U-1 to I.A1-Z195-Q3-U-42.

In another embodiment, compounds I are of formula I.A1 wherein Z is Z-195, R³ is CH₃ and Q is Q4, and R and R⁴ are as per any row of Table U below, which compounds are named I.A1-Z195-Q4-U-1 to I.A1-Z195-Q4-U-42.

In another embodiment, compounds I are of formula I.A2 wherein Z is Z-195, R³ is CH₃ and Q is Q1, and R and R⁴ are as per any row of Table U below, which compounds are named I.A2-Z195-Q1-U-1 to I.A2-Z195-Q1-U-42.

In another embodiment, compounds I are of formula I.A2 wherein Z is Z-195, R³ is CH₃ and Q is Q2, and R and R⁴ are as per any row of Table U below, which compounds are named I.A2-Z195-Q2-U-1 to I.A2-Z195-Q2-U-42.

In another embodiment, compounds I are of formula I.A2 wherein Z is Z-195, R³ is CH₃ and Q is Q3, and R and R⁴ are as per any row of Table U below, which compounds are named I.A2-Z195-Q3-U-1 to I.A2-Z195-Q3-U-42.

In another embodiment, compounds I are of formula I.A2 wherein Z is Z-195, R³ is CH₃ and Q is Q4, and R and R⁴ are as per any row of Table U below, which compounds are named I.A2-Z195-Q4-U-1 to I.A2-Z195-Q4-U-42.

In another embodiment, compounds I are of formula I.A1 wherein Z is Z-196, R³ is CH₃ and Q is Q1, and R and R⁴ are as per any row of Table U below, which compounds are named I.A1-Z196-Q1-U-1 to I.A1-Z196-Q1-U-42.

In another embodiment, compounds I are of formula I.A1 wherein Z is Z-196, R³ is CH₃ and Q is Q2, and R and R⁴ are as per any row of Table U below, which compounds are named I.A1-Z196-Q2-U-1 to I.A1-Z196-Q2-U-42.

In another embodiment, compounds I are of formula I.A1 wherein Z is Z-196, R³ is CH₃ and Q is Q3, and R and R⁴ are as per any row of Table U below, which compounds are named I.A1-Z196-Q3-U-1 to I.A1-Z196-Q3-U-42.

In another embodiment, compounds I are of formula I.A1 wherein Z is Z-196, R³ is CH₃ and Q is Q4, and R and R⁴ are as per any row of Table U below, which compounds are named I.A1-Z196-Q4-U-1 to I.A1-Z196-Q4-U-42.

In another embodiment, compounds I are of formula I.A2 wherein Z is Z-196, R³ is CH₃ and Q is Q1, and R and R⁴ are as per any row of Table U below, which compounds are named I.A2-Z196-Q1-U-1 to I.A2-Z196-Q1-U-42.

In another embodiment, compounds I are of formula I.A2 wherein Z is Z-196, R³ is CH₃ and Q is Q2, and R and R⁴ are as per any row of Table U below, which compounds are named I.A2-Z196-Q2-U-1 to I.A2-Z196-Q2-U-42.

In another embodiment, compounds I are of formula I.A2 wherein Z is Z-196, R³ is CH₃ and Q is Q3, and R and R⁴ are as per any row of Table U below, which compounds are named I.A2-Z196-Q3-U-1 to I.A2-Z196-Q3-U-42.

In another embodiment, compounds I are of formula I.A2 wherein Z is Z-196, R³ is CH₃ and Q is Q4, and R and R⁴ are as per any row of Table U below, which compounds are named I.A2-Z196-Q4-U-1 to I.A2-Z196-Q4-U-42.

In another embodiment, compounds I are of formula I.A1 wherein Z is Z-197, R³ is CH₃ and Q is Q1, and R and R⁴ are as per any row of Table U below, which compounds are named I.A1-Z197-Q1-U-1 to I.A1-Z197-Q1-U-42.

In another embodiment, compounds I are of formula I.A1 wherein Z is Z-197, R³ is CH₃ and Q is Q2, and R and R⁴ are as per any row of Table U below, which compounds are named I.A1-Z197-Q2-U-1 to I.A1-Z197-Q2-U-42.

In another embodiment, compounds I are of formula I.A1 wherein Z is Z-197, R³ is CH₃ and Q is Q3, and R and R⁴ are as per any row of Table U below, which compounds are named I.A1-Z197-Q3-U-1 to I.A1-Z197-Q3-U-42.

In another embodiment, compounds I are of formula I.A1 wherein Z is Z-197, R³ is CH₃ and Q is Q4, and R and R⁴ are as per any row of Table U below, which compounds are named I.A1-Z197-Q4-U-1 to I.A1-Z197-Q4-U-42.

In another embodiment, compounds I are of formula I.A2 wherein Z is Z-197, R³ is CH₃ and Q is Q1, and R and R⁴ are as per any row of Table U below, which compounds are named I.A2-Z197-Q1-U-1 to I.A2-Z197-Q1-U-42.

In another embodiment, compounds I are of formula I.A2 wherein Z is Z-197, R³ is CH₃ and Q is Q2, and R and R⁴ are as per any row of Table U below, which compounds are named I.A2-Z197-Q2-U-1 to I.A2-Z197-Q2-U-42.

In another embodiment, compounds I are of formula I.A2 wherein Z is Z-197, R³ is CH₃ and Q is Q3, and R and R⁴ are as per any row of Table U below, which compounds are named I.A2-Z197-Q3-U-1 to I.A2-Z197-Q3-U-42.

In another embodiment, compounds I are of formula I.A2 wherein Z is Z-197, R³ is CH₃ and Q is Q4, and R and R⁴ are as per any row of Table U below, which compounds are named I.A2-Z197-Q4-U-1 to I.A2-Z197-Q4-U-42.

In another embodiment, compounds I are of formula I.A1 wherein Z is Z-198, R³ is CH₃ and Q is Q1, and R and R⁴ are as per any row of Table U below, which compounds are named I.A1-Z198-Q1-U-1 to I.A1-Z198-Q1-U-42.

In another embodiment, compounds I are of formula I.A1 wherein Z is Z-198, R³ is CH₃ and Q is Q2, and R and R⁴ are as per any row of Table U below, which compounds are named I.A1-Z198-Q2-U-1 to I.A1-Z198-Q2-U-42.

In another embodiment, compounds I are of formula I.A1 wherein Z is Z-198, R³ is CH₃ and Q is Q3, and R and R⁴ are as per any row of Table U below, which compounds are named I.A1-Z198-Q3-U-1 to I.A1-Z198-Q3-U-42.

In another embodiment, compounds I are of formula I.A1 wherein Z is Z-198, R³ is CH₃ and Q is Q4, and R and R⁴ are as per any row of Table U below, which compounds are named I.A1-Z198-Q4-U-1 to I.A1-Z198-Q4-U-42.

In another embodiment, compounds I are of formula I.A2 wherein Z is Z-198, R³ is CH₃ and Q is Q1, and R and R⁴ are as per any row of Table U below, which compounds are named I.A2-Z198-Q1-U-1 to I.A2-Z198-Q1-U-42.

In another embodiment, compounds I are of formula I.A2 wherein Z is Z-198, R³ is CH₃ and Q is Q2, and R and R⁴ are as per any row of Table U below, which compounds are named I.A2-Z198-Q2-U-1 to I.A2-Z198-Q2-U-42.

In another embodiment, compounds I are of formula I.A2 wherein Z is Z-198, R³ is CH₃ and Q is Q3, and R and R⁴ are as per any row of Table U below, which compounds are named I.A2-Z198-Q3-U-1 to I.A2-Z198-Q3-U-42.

In another embodiment, compounds I are of formula I.A2 wherein Z is Z-198, R³ is CH₃ and Q is Q4, and R and R⁴ are as per any row of Table U below, which compounds are named I.A2-Z198-Q4-U-1 to I.A2-Z198-Q4-U-42.

In another embodiment, compounds I are of formula I.A1 wherein Z is Z-199, R³ is CH₃ and Q is Q1, and R and R⁴ are as per any row of Table U below, which compounds are named I.A1-Z199-Q1-U-1 to I.A1-Z199-Q1-U-42.

In another embodiment, compounds I are of formula I.A1 wherein Z is Z-199, R³ is CH₃ and Q is Q2, and R and R⁴ are as per any row of Table U below, which compounds are named I.A1-Z199-Q2-U-1 to I.A1-Z199-Q2-U-42.

In another embodiment, compounds I are of formula I.A1 wherein Z is Z-199, R³ is CH₃ and Q is Q3, and R and R⁴ are as per any row of Table U below, which compounds are named I.A1-Z199-Q3-U-1 to I.A1-Z199-Q3-U-42.

In another embodiment, compounds I are of formula I.A1 wherein Z is Z-199, R³ is CH₃ and Q is Q4, and R and R⁴ are as per any row of Table U below, which compounds are named I.A1-Z199-Q4-U-1 to I.A1-Z199-Q4-U-42.

In another embodiment, compounds I are of formula I.A2 wherein Z is Z-199, R³ is CH₃ and Q is Q1, and R and R⁴ are as per any row of Table U below, which compounds are named I.A2-Z199-Q1-U-1 to I.A2-Z199-Q1-U-42.

In another embodiment, compounds I are of formula I.A2 wherein Z is Z-199, R³ is CH₃ and Q is Q2, and R and R⁴ are as per any row of Table U below, which compounds are named I.A2-Z199-Q2-U-1 to I.A2-Z199-Q2-U-42.

In another embodiment, compounds I are of formula I.A2 wherein Z is Z-199, R³ is CH₃ and Q is Q3, and R and R⁴ are as per any row of Table U below, which compounds are named I.A2-Z199-Q3-U-1 to I.A2-Z199-Q3-U-42.

In another embodiment, compounds I are of formula I.A2 wherein Z is Z-199, R³ is CH₃ and Q is Q4, and R and R⁴ are as per any row of Table U below, which compounds are named I.A2-Z199-Q4-U-1 to I.A2-Z199-Q4-U-42.

In another embodiment, compounds I are of formula I.A1 wherein Z is Z-200, R³ is CH₃ and Q is Q1, and R and R⁴ are as per any row of Table U below, which compounds are named I.A1-Z200-Q1-U-1 to I.A1-Z200-Q1-U-42.

In another embodiment, compounds I are of formula I.A1 wherein Z is Z-200, R³ is CH₃ and Q is Q2, and R and R⁴ are as per any row of Table U below, which compounds are named I.A1-Z200-Q2-U-1 to I.A1-Z200-Q2-U-42.

In another embodiment, compounds I are of formula I.A1 wherein Z is Z-200, R³ is CH₃ and Q is Q3, and R and R⁴ are as per any row of Table U below, which compounds are named I.A1-Z200-Q3-U-1 to I.A1-Z200-Q3-U-42.

In another embodiment, compounds I are of formula I.A1 wherein Z is Z-200, R³ is CH₃ and Q is Q4, and R and R⁴ are as per any row of Table U below, which compounds are named I.A1-Z200-Q4-U-1 to I.A1-Z200-Q4-U-42.

In another embodiment, compounds I are of formula I.A2 wherein Z is Z-200, R³ is CH₃ and Q is Q1, and R and R⁴ are as per any row of Table U below, which compounds are named I.A2-Z200-Q1-U-1 to I.A2-Z200-Q1-U-42.

In another embodiment, compounds I are of formula I.A2 wherein Z is Z-200, R³ is CH₃ and Q is Q2, and R and R⁴ are as per any row of Table U below, which compounds are named I.A2-Z200-Q2-U-1 to I.A2-Z200-Q2-U-42.

In another embodiment, compounds I are of formula I.A2 wherein Z is Z-200, R³ is CH₃ and Q is Q3, and R and R⁴ are as per any row of Table U below, which compounds are named I.A2-Z200-Q3-U-1 to I.A2-Z200-Q3-U-42.

In another embodiment, compounds I are of formula I.A2 wherein Z is Z-200, R³ is CH₃ and Q is Q4, and R and R⁴ are as per any row of Table U below, which compounds are named I.A2-Z200-Q4-U-1 to I.A2-Z200-Q4-U-42.

In another embodiment, compounds I are of formula I.A1 wherein Z is Z-201, R³ is CH₃ and Q is Q1, and R and R⁴ are as per any row of Table U below, which compounds are named I.A1-Z201-Q1-U-1 to I.A1-Z201-Q1-U-42.

In another embodiment, compounds I are of formula I.A1 wherein Z is Z-201, R³ is CH₃ and Q is Q2, and R and R⁴ are as per any row of Table U below, which compounds are named I.A1-Z201-Q2-U-1 to I.A1-Z201-Q2-U-42.

In another embodiment, compounds I are of formula I.A1 wherein Z is Z-201, R³ is CH₃ and Q is Q3, and R and R⁴ are as per any row of Table U below, which compounds are named I.A1-Z201-Q3-U-1 to I.A1-Z201-Q3-U-42.

In another embodiment, compounds I are of formula I.A1 wherein Z is Z-201, R³ is CH₃ and Q is Q4, and R and R⁴ are as per any row of Table U below, which compounds are named I.A1-Z201-Q4-U-1 to I.A1-Z201-Q4-U-42.

In another embodiment, compounds I are of formula I.A2 wherein Z is Z-201, R³ is CH₃ and Q is Q1, and R and R⁴ are as per any row of Table U below, which compounds are named I.A2-Z201-Q1-U-1 to I.A2-Z201-Q1-U-42.

In another embodiment, compounds I are of formula I.A2 wherein Z is Z-201, R³ is CH₃ and Q is Q2, and R and R⁴ are as per any row of Table U below, which compounds are named I.A2-Z201-Q2-U-1 to I.A2-Z201-Q2-U-42.

In another embodiment, compounds I are of formula I.A2 wherein Z is Z-201, R³ is CH₃ and Q is Q3, and R and R⁴ are as per any row of Table U below, which compounds are named I.A2-Z201-Q3-U-1 to I.A2-Z201-Q3-U-42.

In another embodiment, compounds I are of formula I.A2 wherein Z is Z-201, R³ is CH₃ and Q is Q4, and R and R⁴ are as per any row of Table U below, which compounds are named I.A2-Z201-Q4-U-1 to I.A2-Z201-Q4-U-42.

In another embodiment, compounds I are of formula I.A1 wherein Z is Z-202, R³ is CH₃ and Q is Q1, and R and R⁴ are as per any row of Table U below, which compounds are named I.A1-Z202-Q1-U-1 to I.A1-Z202-Q1-U-42.

In another embodiment, compounds I are of formula I.A1 wherein Z is Z-202, R³ is CH₃ and Q is Q2, and R and R⁴ are as per any row of Table U below, which compounds are named I.A1-Z202-Q2-U-1 to I.A1-Z202-Q2-U-42.

In another embodiment, compounds I are of formula I.A1 wherein Z is Z-202, R³ is CH₃ and Q is Q3, and R and R⁴ are as per any row of Table U below, which compounds are named I.A1-Z202-Q3-U-1 to I.A1-Z202-Q3-U-42.

In another embodiment, compounds I are of formula I.A1 wherein Z is Z-202, R³ is CH₃ and Q is Q4, and R and R⁴ are as per any row of Table U below, which compounds are named I.A1-Z202-Q4-U-1 to I.A1-Z202-Q4-U-42.

In another embodiment, compounds I are of formula I.A2 wherein Z is Z-202, R³ is CH₃ and Q is Q1, and R and R⁴ are as per any row of Table U below, which compounds are named I.A2-Z202-Q1-U-1 to I.A2-Z202-Q1-U-42.

In another embodiment, compounds I are of formula I.A2 wherein Z is Z-202, R³ is CH₃ and Q is Q2, and R and R⁴ are as per any row of Table U below, which compounds are named I.A2-Z202-Q2-U-1 to I.A2-Z202-Q2-U-42.

In another embodiment, compounds I are of formula I.A2 wherein Z is Z-202, R³ is CH₃ and Q is Q3, and R and R⁴ are as per any row of Table U below, which compounds are named I.A2-Z202-Q3-U-1 to I.A2-Z202-Q3-U-42.

In another embodiment, compounds I are of formula I.A2 wherein Z is Z-202, R³ is CH₃ and Q is Q4, and R and R⁴ are as per any row of Table U below, which compounds are named I.A2-Z202-Q4-U-1 to I.A2-Z202-Q4-U-42.

In another embodiment, compounds I are of formula I.A1 wherein Z is Z-203, R³ is CH₃ and Q is Q1, and R and R⁴ are as per any row of Table U below, which compounds are named I.A1-Z203-Q1-U-1 to I.A1-Z203-Q1-U-42.

In another embodiment, compounds I are of formula I.A1 wherein Z is Z-203, R³ is CH₃ and Q is Q2, and R and R⁴ are as per any row of Table U below, which compounds are named I.A1-Z203-Q2-U-1 to I.A1-Z203-Q2-U-42.

In another embodiment, compounds I are of formula I.A1 wherein Z is Z-203, R³ is CH₃ and Q is Q3, and R and R⁴ are as per any row of Table U below, which compounds are named I.A1-Z203-Q3-U-1 to I.A1-Z203-Q3-U-42.

In another embodiment, compounds I are of formula I.A1 wherein Z is Z-203, R³ is CH₃ and Q is Q4, and R and R⁴ are as per any row of Table U below, which compounds are named I.A1-Z203-Q4-U-1 to I.A1-Z203-Q4-U-42.

In another embodiment, compounds I are of formula I.A2 wherein Z is Z-203, R³ is CH₃ and Q is Q1, and R and R⁴ are as per any row of Table U below, which compounds are named I.A2-Z203-Q1-U-1 to I.A2-Z203-Q1-U-42.

In another embodiment, compounds I are of formula I.A2 wherein Z is Z-203, R³ is CH₃ and Q is Q2, and R and R⁴ are as per any row of Table U below, which compounds are named I.A2-Z203-Q2-U-1 to I.A2-Z203-Q2-U-42.

In another embodiment, compounds I are of formula I.A2 wherein Z is Z-203, R³ is CH₃ and Q is Q3, and R and R⁴ are as per any row of Table U below, which compounds are named I.A2-Z203-Q3-U-1 to I.A2-Z203-Q3-U-42.

In another embodiment, compounds I are of formula I.A2 wherein Z is Z-203, R³ is CH₃ and Q is Q4, and R and R⁴ are as per any row of Table U below, which compounds are named I.A2-Z203-Q4-U-1 to I.A2-Z203-Q4-U-42.

In another embodiment, compounds I are of formula I.A1 wherein Z is Z-204, R³ is CH₃ and Q is Q1, and R and R⁴ are as per any row of Table U below, which compounds are named I.A1-Z204-Q1-U-1 to I.A1-Z204-Q1-U-42.

In another embodiment, compounds I are of formula I.A1 wherein Z is Z-204, R³ is CH₃ and Q is Q2, and R and R⁴ are as per any row of Table U below, which compounds are named I.A1-Z204-Q2-U-1 to I.A1-Z204-Q2-U-42.

In another embodiment, compounds I are of formula I.A1 wherein Z is Z-204, R³ is CH₃ and Q is Q3, and R and R⁴ are as per any row of Table U below, which compounds are named I.A1-Z204-Q3-U-1 to I.A1-Z204-Q3-U-42.

In another embodiment, compounds I are of formula I.A1 wherein Z is Z-204, R³ is CH₃ and Q is Q4, and R and R⁴ are as per any row of Table U below, which compounds are named I.A1-Z204-Q4-U-1 to I.A1-Z204-Q4-U-42.

In another embodiment, compounds I are of formula I.A2 wherein Z is Z-204, R³ is CH₃ and Q is Q1, and R and R⁴ are as per any row of Table U below, which compounds are named I.A2-Z204-Q1-U-1 to I.A2-Z204-Q1-U-42.

In another embodiment, compounds I are of formula I.A2 wherein Z is Z-204, R³ is CH₃ and Q is Q2, and R and R⁴ are as per any row of Table U below, which compounds are named I.A2-Z204-Q2-U-1 to I.A2-Z204-Q2-U-42.

In another embodiment, compounds I are of formula I.A2 wherein Z is Z-204, R³ is CH₃ and Q is Q3, and R and R⁴ are as per any row of Table U below, which compounds are named I.A2-Z204-Q3-U-1 to I.A2-Z204-Q3-U-42.

In another embodiment, compounds I are of formula I.A2 wherein Z is Z-204, R³ is CH₃ and Q is Q4, and R and R⁴ are as per any row of Table U below, which compounds are named I.A2-Z204-Q4-U-1 to I.A2-Z204-Q4-U-42.

In another embodiment, compounds I are of formula I.A1 wherein Z is Z-205, R³ is CH₃ and Q is Q1, and R and R⁴ are as per any row of Table U below, which compounds are named I.A1-Z205-Q1-U-1 to I.A1-Z205-Q1-U-42.

In another embodiment, compounds I are of formula I.A1 wherein Z is Z-205, R³ is CH₃ and Q is Q2, and R and R⁴ are as per any row of Table U below, which compounds are named I.A1-Z205-Q2-U-1 to I.A1-Z205-Q2-U-42.

In another embodiment, compounds I are of formula I.A1 wherein Z is Z-205, R³ is CH₃ and Q is Q3, and R and R⁴ are as per any row of Table U below, which compounds are named I.A1-Z205-Q3-U-1 to I.A1-Z205-Q3-U-42.

In another embodiment, compounds I are of formula I.A1 wherein Z is Z-205, R³ is CH₃ and Q is Q4, and R and R⁴ are as per any row of Table U below, which compounds are named I.A1-Z205-Q4-U-1 to I.A1-Z205-Q4-U-42.

In another embodiment, compounds I are of formula I.A2 wherein Z is Z-205, R³ is CH₃ and Q is Q1, and R and R⁴ are as per any row of Table U below, which compounds are named I.A2-Z205-Q1-U-1 to I.A2-Z205-Q1-U-42.

In another embodiment, compounds I are of formula I.A2 wherein Z is Z-205, R³ is CH₃ and Q is Q2, and R and R⁴ are as per any row of Table U below, which compounds are named I.A2-Z205-Q2-U-1 to I.A2-Z205-Q2-U-42.

In another embodiment, compounds I are of formula I.A2 wherein Z is Z-205, R³ is CH₃ and Q is Q3, and R and R⁴ are as per any row of Table U below, which compounds are named I.A2-Z205-Q3-U-1 to I.A2-Z205-Q3-U-42.

In another embodiment, compounds I are of formula I.A2 wherein Z is Z-205, R³ is CH₃ and Q is Q4, and R and R⁴ are as per any row of Table U below, which compounds are named I.A2-Z205-Q4-U-1 to I.A2-Z205-Q4-U-42.

In another embodiment, compounds I are of formula I.A1 wherein Z is Z-206, R³ is CH₃ and Q is Q1, and R and R⁴ are as per any row of Table U below, which compounds are named I.A1-Z206-Q1-U-1 to I.A1-Z206-Q1-U-42.

In another embodiment, compounds I are of formula I.A1 wherein Z is Z-206, R³ is CH₃ and Q is Q2, and R and R⁴ are as per any row of Table U below, which compounds are named I.A1-Z206-Q2-U-1 to I.A1-Z206-Q2-U-42.

In another embodiment, compounds I are of formula I.A1 wherein Z is Z-206, R³ is CH₃ and Q is Q3, and R and R⁴ are as per any row of Table U below, which compounds are named I.A1-Z206-Q3-U-1 to I.A1-Z206-Q3-U-42.

In another embodiment, compounds I are of formula I.A1 wherein Z is Z-206, R³ is CH₃ and Q is Q4, and R and R⁴ are as per any row of Table U below, which compounds are named I.A1-Z206-Q4-U-1 to I.A1-Z206-Q4-U-42.

In another embodiment, compounds I are of formula I.A2 wherein Z is Z-206, R³ is CH₃ and Q is Q1, and R and R⁴ are as per any row of Table U below, which compounds are named I.A2-Z206-Q1-U-1 to I.A2-Z206-Q1-U-42.

In another embodiment, compounds I are of formula I.A2 wherein Z is Z-206, R³ is CH₃ and Q is Q2, and R and R⁴ are as per any row of Table U below, which compounds are named I.A2-Z206-Q2-U-1 to I.A2-Z206-Q2-U-42.

In another embodiment, compounds I are of formula I.A2 wherein Z is Z-206, R³ is CH₃ and Q is Q3, and R and R⁴ are as per any row of Table U below, which compounds are named I.A2-Z206-Q3-U-1 to I.A2-Z206-Q3-U-42.

In another embodiment, compounds I are of formula I.A2 wherein Z is Z-206, R³ is CH₃ and Q is Q4, and R and R⁴ are as per any row of Table U below, which compounds are named I.A2-Z206-Q4-U-1 to I.A2-Z206-Q4-U-42.

In another embodiment, compounds I are of formula I.A1 wherein Z is Z-207, R³ is CH₃ and Q is Q1, and R and R⁴ are as per any row of Table U below, which compounds are named I.A1-Z207-Q1-U-1 to I.A1-Z207-Q1-U-42.

In another embodiment, compounds I are of formula I.A1 wherein Z is Z-207, R³ is CH₃ and Q is Q2, and R and R⁴ are as per any row of Table U below, which compounds are named I.A1-Z207-Q2-U-1 to I.A1-Z207-Q2-U-42.

In another embodiment, compounds I are of formula I.A1 wherein Z is Z-207, R³ is CH₃ and Q is Q3, and R and R⁴ are as per any row of Table U below, which compounds are named I.A1-Z207-Q3-U-1 to I.A1-Z207-Q3-U-42.

In another embodiment, compounds I are of formula I.A1 wherein Z is Z-207, R³ is CH₃ and Q is Q4, and R and R⁴ are as per any row of Table U below, which compounds are named I.A1-Z207-Q4-U-1 to I.A1-Z207-Q4-U-42.

In another embodiment, compounds I are of formula I.A2 wherein Z is Z-207, R³ is CH₃ and Q is Q1, and R and R⁴ are as per any row of Table U below, which compounds are named I.A2-Z207-Q1-U-1 to I.A2-Z207-Q1-U-42.

In another embodiment, compounds I are of formula I.A2 wherein Z is Z-207, R³ is CH₃ and Q is Q2, and R and R⁴ are as per any row of Table U below, which compounds are named I.A2-Z207-Q2-U-1 to I.A2-Z207-Q2-U-42.

In another embodiment, compounds I are of formula I.A2 wherein Z is Z-207, R³ is CH₃ and Q is Q3, and R and R⁴ are as per any row of Table U below, which compounds are named I.A2-Z207-Q3-U-1 to I.A2-Z207-Q3-U-42.

In another embodiment, compounds I are of formula I.A2 wherein Z is Z-207, R³ is CH₃ and Q is Q4, and R and R⁴ are as per any row of Table U below, which compounds are named I.A2-Z207-Q4-U-1 to I.A2-Z207-Q4-U-42.

In another embodiment, compounds I are of formula I.A1 wherein Z is Z-208, R³ is CH₃ and Q is Q1, and R and R⁴ are as per any row of Table U below, which compounds are named I.A1-Z208-Q1-U-1 to I.A1-Z208-Q1-U-42.

In another embodiment, compounds I are of formula I.A1 wherein Z is Z-208, R³ is CH₃ and Q is Q2, and R and R⁴ are as per any row of Table U below, which compounds are named I.A1-Z208-Q2-U-1 to I.A1-Z208-Q2-U-42.

In another embodiment, compounds I are of formula I.A1 wherein Z is Z-208, R³ is CH₃ and Q is Q3, and R and R⁴ are as per any row of Table U below, which compounds are named I.A1-Z208-Q3-U-1 to I.A1-Z208-Q3-U-42.

In another embodiment, compounds I are of formula I.A1 wherein Z is Z-208, R³ is CH₃ and Q is Q4, and R and R⁴ are as per any row of Table U below, which compounds are named I.A1-Z208-Q4-U-1 to I.A1-Z208-Q4-U-42.

In another embodiment, compounds I are of formula I.A2 wherein Z is Z-208, R³ is CH₃ and Q is Q1, and R and R⁴ are as per any row of Table U below, which compounds are named I.A2-Z208-Q1-U-1 to I.A2-Z208-Q1-U-42.

In another embodiment, compounds I are of formula I.A2 wherein Z is Z-208, R³ is CH₃ and Q is Q2, and R and R⁴ are as per any row of Table U below, which compounds are named I.A2-Z208-Q2-U-1 to I.A2-Z208-Q2-U-42.

In another embodiment, compounds I are of formula I.A2 wherein Z is Z-208, R³ is CH₃ and Q is Q3, and R and R⁴ are as per any row of Table U below, which compounds are named I.A2-Z208-Q3-U-1 to I.A2-Z208-Q3-U-42.

In another embodiment, compounds I are of formula I.A2 wherein Z is Z-208, R³ is CH₃ and Q is Q4, and R and R⁴ are as per any row of Table U below, which compounds are named I.A2-Z208-Q4-U-1 to I.A2-Z208-Q4-U-42.

In another embodiment, compounds I are of formula I.A1 wherein Z is Z-209, R³ is CH₃ and Q is Q1, and R and R⁴ are as per any row of Table U below, which compounds are named I.A1-Z209-Q1-U-1 to I.A1-Z209-Q1-U-42.

In another embodiment, compounds I are of formula I.A1 wherein Z is Z-209, R³ is CH₃ and Q is Q2, and R and R⁴ are as per any row of Table U below, which compounds are named I.A1-Z209-Q2-U-1 to I.A1-Z209-Q2-U-42.

In another embodiment, compounds I are of formula I.A1 wherein Z is Z-209, R³ is CH₃ and Q is Q3, and R and R⁴ are as per any row of Table U below, which compounds are named I.A1-Z209-Q3-U-1 to I.A1-Z209-Q3-U-42.

In another embodiment, compounds I are of formula I.A1 wherein Z is Z-209, R³ is CH₃ and Q is Q4, and R and R⁴ are as per any row of Table U below, which compounds are named I.A1-Z209-Q4-U-1 to I.A1-Z209-Q4-U-42.

In another embodiment, compounds I are of formula I.A2 wherein Z is Z-209, R³ is CH₃ and Q is Q1, and R and R⁴ are as per any row of Table U below, which compounds are named I.A2-Z209-Q1-U-1 to I.A2-Z209-Q1-U-42.

In another embodiment, compounds I are of formula I.A2 wherein Z is Z-209, R³ is CH₃ and Q is Q2, and R and R⁴ are as per any row of Table U below, which compounds are named I.A2-Z209-Q2-U-1 to I.A2-Z209-Q2-U-42.

In another embodiment, compounds I are of formula I.A2 wherein Z is Z-209, R³ is CH₃ and Q is Q3, and R and R⁴ are as per any row of Table U below, which compounds are named I.A2-Z209-Q3-U-1 to I.A2-Z209-Q3-U-42.

In another embodiment, compounds I are of formula I.A2 wherein Z is Z-209, R³ is CH₃ and Q is Q4, and R and R⁴ are as per any row of Table U below, which compounds are named I.A2-Z209-Q4-U-1 to I.A2-Z209-Q4-U-42.

In another embodiment, compounds I are of formula I.A1 wherein Z is Z-210, R³ is CH₃ and Q is Q1, and R and R⁴ are as per any row of Table U below, which compounds are named I.A1-Z210-Q1-U-1 to I.A1-Z210-Q1-U-42.

In another embodiment, compounds I are of formula I.A1 wherein Z is Z-210, R³ is CH₃ and Q is Q2, and R and R⁴ are as per any row of Table U below, which compounds are named I.A1-Z210-Q2-U-1 to I.A1-Z210-Q2-U-42.

In another embodiment, compounds I are of formula I.A1 wherein Z is Z-210, R³ is CH₃ and Q is Q3, and R and R⁴ are as per any row of Table U below, which compounds are named I.A1-Z210-Q3-U-1 to I.A1-Z210-Q3-U-42.

In another embodiment, compounds I are of formula I.A1 wherein Z is Z-210, R³ is CH₃ and Q is Q4, and R and R⁴ are as per any row of Table U below, which compounds are named I.A1-Z210-Q4-U-1 to I.A1-Z210-Q4-U-42.

In another embodiment, compounds I are of formula I.A2 wherein Z is Z-210, R³ is CH₃ and Q is Q1, and R and R⁴ are as per any row of Table U below, which compounds are named I.A2-Z210-Q1-U-1 to I.A2-Z210-Q1-U-42.

In another embodiment, compounds I are of formula I.A2 wherein Z is Z-210, R³ is CH₃ and Q is Q2, and R and R⁴ are as per any row of Table U below, which compounds are named I.A2-Z210-Q2-U-1 to I.A2-Z210-Q2-U-42.

In another embodiment, compounds I are of formula I.A2 wherein Z is Z-210, R³ is CH₃ and Q is Q3, and R and R⁴ are as per any row of Table U below, which compounds are named I.A2-Z210-Q3-U-1 to I.A2-Z210-Q3-U-42.

In another embodiment, compounds I are of formula I.A2 wherein Z is Z-210, R³ is CH₃ and Q is Q4, and R and R⁴ are as per any row of Table U below, which compounds are named I.A2-Z210-Q4-U-1 to I.A2-Z210-Q4-U-42.

In another embodiment, compounds I are of formula I.A1 wherein Z is Z-211, R³ is CH₃ and Q is Q1, and R and R⁴ are as per any row of Table U below, which compounds are named I.A1-Z211-Q1-U-1 to I.A1-Z211-Q1-U-42.

In another embodiment, compounds I are of formula I.A1 wherein Z is Z-211, R³ is CH₃ and Q is Q2, and R and R⁴ are as per any row of Table U below, which compounds are named I.A1-Z211-Q2-U-1 to I.A1-Z211-Q2-U-42.

In another embodiment, compounds I are of formula I.A1 wherein Z is Z-211, R³ is CH₃ and Q is Q3, and R and R⁴ are as per any row of Table U below, which compounds are named I.A1-Z211-Q3-U-1 to I.A1-Z211-Q3-U-42.

In another embodiment, compounds I are of formula I.A1 wherein Z is Z-211, R³ is CH₃ and Q is Q4, and R and R⁴ are as per any row of Table U below, which compounds are named I.A1-Z211-Q4-U-1 to I.A1-Z211-Q4-U-42.

In another embodiment, compounds I are of formula I.A2 wherein Z is Z-211, R³ is CH₃ and Q is Q1, and R and R⁴ are as per any row of Table U below, which compounds are named I.A2-Z211-Q1-U-1 to I.A2-Z211-Q1-U-42.

In another embodiment, compounds I are of formula I.A2 wherein Z is Z-211, R³ is CH₃ and Q is Q2, and R and R⁴ are as per any row of Table U below, which compounds are named I.A2-Z211-Q2-U-1 to I.A2-Z211-Q2-U-42.

In another embodiment, compounds I are of formula I.A2 wherein Z is Z-211, R³ is CH₃ and Q is Q3, and R and R⁴ are as per any row of Table U below, which compounds are named I.A2-Z211-Q3-U-1 to I.A2-Z211-Q3-U-42.

In another embodiment, compounds I are of formula I.A2 wherein Z is Z-211, R³ is CH₃ and Q is Q4, and R and R⁴ are as per any row of Table U below, which compounds are named I.A2-Z211-Q4-U-1 to I.A2-Z211-Q4-U-42.

In another embodiment, compounds I are of formula I.A1 wherein Z is Z-212, R³ is CH₃ and Q is Q1, and R and R⁴ are as per any row of Table U below, which compounds are named I.A1-Z212-Q1-U-1 to I.A1-Z212-Q1-U-42.

In another embodiment, compounds I are of formula I.A1 wherein Z is Z-212, R³ is CH₃ and Q is Q2, and R and R⁴ are as per any row of Table U below, which compounds are named I.A1-Z212-Q2-U-1 to I.A1-Z212-Q2-U-42.

In another embodiment, compounds I are of formula I.A1 wherein Z is Z-212, R³ is CH₃ and Q is Q3, and R and R⁴ are as per any row of Table U below, which compounds are named I.A1-Z212-Q3-U-1 to I.A1-Z212-Q3-U-42.

In another embodiment, compounds I are of formula I.A1 wherein Z is Z-212, R³ is CH₃ and Q is Q4, and R and R⁴ are as per any row of Table U below, which compounds are named I.A1-Z212-Q4-U-1 to I.A1-Z212-Q4-U-42.

In another embodiment, compounds I are of formula I.A2 wherein Z is Z-212, R³ is CH₃ and Q is Q1, and R and R⁴ are as per any row of Table U below, which compounds are named I.A2-Z212-Q1-U-1 to I.A2-Z212-Q1-U-42.

In another embodiment, compounds I are of formula I.A2 wherein Z is Z-212, R³ is CH₃ and Q is Q2, and R and R⁴ are as per any row of Table U below, which compounds are named I.A2-Z212-Q2-U-1 to I.A2-Z212-Q2-U-42.

In another embodiment, compounds I are of formula I.A2 wherein Z is Z-212, R³ is CH₃ and Q is Q3, and R and R⁴ are as per any row of Table U below, which compounds are named I.A2-Z212-Q3-U-1 to I.A2-Z212-Q3-U-42.

In another embodiment, compounds I are of formula I.A2 wherein Z is Z-212, R³ is CH₃ and Q is Q4, and R and R⁴ are as per any row of Table U below, which compounds are named I.A2-Z212-Q4-U-1 to I.A2-Z212-Q4-U-42.

In another embodiment, compounds I are of formula I.A1 wherein Z is Z-213, R³ is CH₃ and Q is Q1, and R and R⁴ are as per any row of Table U below, which compounds are named I.A1-Z213-Q1-U-1 to I.A1-Z213-Q1-U-42.

In another embodiment, compounds I are of formula I.A1 wherein Z is Z-213, R³ is CH₃ and Q is Q2, and R and R⁴ are as per any row of Table U below, which compounds are named I.A1-Z213-Q2-U-1 to I.A1-Z213-Q2-U-42.

In another embodiment, compounds I are of formula I.A1 wherein Z is Z-213, R³ is CH₃ and Q is Q3, and R and R⁴ are as per any row of Table U below, which compounds are named I.A1-Z213-Q3-U-1 to I.A1-Z213-Q3-U-42.

In another embodiment, compounds I are of formula I.A1 wherein Z is Z-213, R³ is CH₃ and Q is Q4, and R and R⁴ are as per any row of Table U below, which compounds are named I.A1-Z213-Q4-U-1 to I.A1-Z213-Q4-U-42.

In another embodiment, compounds I are of formula I.A2 wherein Z is Z-213, R³ is CH₃ and Q is Q1, and R and R⁴ are as per any row of Table U below, which compounds are named I.A2-Z213-Q1-U-1 to I.A2-Z213-Q1-U-42.

In another embodiment, compounds I are of formula I.A2 wherein Z is Z-213, R³ is CH₃ and Q is Q2, and R and R⁴ are as per any row of Table U below, which compounds are named I.A2-Z213-Q2-U-1 to I.A2-Z213-Q2-U-42.

In another embodiment, compounds I are of formula I.A2 wherein Z is Z-213, R³ is CH₃ and Q is Q3, and R and R⁴ are as per any row of Table U below, which compounds are named I.A2-Z213-Q3-U-1 to I.A2-Z213-Q3-U-42.

In another embodiment, compounds I are of formula I.A2 wherein Z is Z-213, R³ is CH₃ and Q is Q4, and R and R⁴ are as per any row of Table U below, which compounds are named I.A2-Z213-Q4-U-1 to I.A2-Z213-Q4-U-42.

In another embodiment, compounds I are of formula I.A1 wherein Z is Z-214, R³ is CH₃ and Q is Q1, and R and R⁴ are as per any row of Table U below, which compounds are named I.A1-Z214-Q1-U-1 to I.A1-Z214-Q1-U-42.

In another embodiment, compounds I are of formula I.A1 wherein Z is Z-214, R³ is CH₃ and Q is Q2, and R and R⁴ are as per any row of Table U below, which compounds are named I.A1-Z214-Q2-U-1 to I.A1-Z214-Q2-U-42.

In another embodiment, compounds I are of formula I.A1 wherein Z is Z-214, R³ is CH₃ and Q is Q3, and R and R⁴ are as per any row of Table U below, which compounds are named I.A1-Z214-Q3-U-1 to I.A1-Z214-Q3-U-42.

In another embodiment, compounds I are of formula I.A1 wherein Z is Z-214, R³ is CH₃ and Q is Q4, and R and R⁴ are as per any row of Table U below, which compounds are named I.A1-Z214-Q4-U-1 to I.A1-Z214-Q4-U-42.

In another embodiment, compounds I are of formula I.A2 wherein Z is Z-214, R³ is CH₃ and Q is Q1, and R and R⁴ are as per any row of Table U below, which compounds are named I.A2-Z214-Q1-U-1 to I.A2-Z214-Q1-U-42.

In another embodiment, compounds I are of formula I.A2 wherein Z is Z-214, R³ is CH₃ and Q is Q2, and R and R⁴ are as per any row of Table U below, which compounds are named I.A2-Z214-Q2-U-1 to I.A2-Z214-Q2-U-42.

In another embodiment, compounds I are of formula I.A2 wherein Z is Z-214, R³ is CH₃ and Q is Q3, and R and R⁴ are as per any row of Table U below, which compounds are named I.A2-Z214-Q3-U-1 to I.A2-Z214-Q3-U-42.

In another embodiment, compounds I are of formula I.A2 wherein Z is Z-214, R³ is CH₃ and Q is Q4, and R and R⁴ are as per any row of Table U below, which compounds are named I.A2-Z214-Q4-U-1 to I.A2-Z214-Q4-U-42.

In another embodiment, compounds I are of formula I.A1 wherein Z is Z-215, R³ is CH₃ and Q is Q1, and R and R⁴ are as per any row of Table U below, which compounds are named I.A1-Z215-Q1-U-1 to I.A1-Z215-Q1-U-42.

In another embodiment, compounds I are of formula I.A1 wherein Z is Z-215, R³ is CH₃ and Q is Q2, and R and R⁴ are as per any row of Table U below, which compounds are named I.A1-Z215-Q2-U-1 to I.A1-Z215-Q2-U-42.

In another embodiment, compounds I are of formula I.A1 wherein Z is Z-215, R³ is CH₃ and Q is Q3, and R and R⁴ are as per any row of Table U below, which compounds are named I.A1-Z215-Q3-U-1 to I.A1-Z215-Q3-U-42.

In another embodiment, compounds I are of formula I.A1 wherein Z is Z-215, R³ is CH₃ and Q is Q4, and R and R⁴ are as per any row of Table U below, which compounds are named I.A1-Z215-Q4-U-1 to I.A1-Z215-Q4-U-42.

In another embodiment, compounds I are of formula I.A2 wherein Z is Z-215, R³ is CH₃ and Q is Q1, and R and R⁴ are as per any row of Table U below, which compounds are named I.A2-Z215-Q1-U-1 to I.A2-Z215-Q1-U-42.

In another embodiment, compounds I are of formula I.A2 wherein Z is Z-215, R³ is CH₃ and Q is Q2, and R and R⁴ are as per any row of Table U below, which compounds are named I.A2-Z215-Q2-U-1 to I.A2-Z215-Q2-U-42.

In another embodiment, compounds I are of formula I.A2 wherein Z is Z-215, R³ is CH₃ and Q is Q3, and R and R⁴ are as per any row of Table U below, which compounds are named I.A2-Z215-Q3-U-1 to I.A2-Z215-Q3-U-42.

In another embodiment, compounds I are of formula I.A2 wherein Z is Z-215, R³ is CH₃ and Q is Q4, and R and R⁴ are as per any row of Table U below, which compounds are named I.A2-Z215-Q4-U-1 to I.A2-Z215-Q4-U-42.

In another embodiment, compounds I are of formula I.A1 wherein Z is Z-216, R³ is CH₃ and Q is Q1, and R and R⁴ are as per any row of Table U below, which compounds are named I.A1-Z216-Q1-U-1 to I.A1-Z216-Q1-U-42.

In another embodiment, compounds I are of formula I.A1 wherein Z is Z-216, R³ is CH₃ and Q is Q2, and R and R⁴ are as per any row of Table U below, which compounds are named I.A1-Z216-Q2-U-1 to I.A1-Z216-Q2-U-42.

In another embodiment, compounds I are of formula I.A1 wherein Z is Z-216, R³ is CH₃ and Q is Q3, and R and R⁴ are as per any row of Table U below, which compounds are named I.A1-Z216-Q3-U-1 to I.A1-Z216-Q3-U-42.

In another embodiment, compounds I are of formula I.A1 wherein Z is Z-216, R³ is CH₃ and Q is Q4, and R and R⁴ are as per any row of Table U below, which compounds are named I.A1-Z216-Q4-U-1 to I.A1-Z216-Q4-U-42.

In another embodiment, compounds I are of formula I.A2 wherein Z is Z-216, R³ is CH₃ and Q is Q1, and R and R⁴ are as per any row of Table U below, which compounds are named I.A2-Z216-Q1-U-1 to I.A2-Z216-Q1-U-42.

In another embodiment, compounds I are of formula I.A2 wherein Z is Z-216, R³ is CH₃ and Q is Q2, and R and R⁴ are as per any row of Table U below, which compounds are named I.A2-Z216-Q2-U-1 to I.A2-Z216-Q2-U-42.

In another embodiment, compounds I are of formula I.A2 wherein Z is Z-216, R³ is CH₃ and Q is Q3, and R and R⁴ are as per any row of Table U below, which compounds are named I.A2-Z216-Q3-U-1 to I.A2-Z216-Q3-U-42.

In another embodiment, compounds I are of formula I.A2 wherein Z is Z-216, R³ is CH₃ and Q is Q4, and R and R⁴ are as per any row of Table U below, which compounds are named I.A2-Z216-Q4-U-1 to I.A2-Z216-Q4-U-42.

In another embodiment, compounds I are of formula I.A1 wherein Z is Z-217, R³ is CH₃ and Q is Q1, and R and R⁴ are as per any row of Table U below, which compounds are named I.A1-Z217-Q1-U-1 to I.A1-Z217-Q1-U-42.

In another embodiment, compounds I are of formula I.A1 wherein Z is Z-217, R³ is CH₃ and Q is Q2, and R and R⁴ are as per any row of Table U below, which compounds are named I.A1-Z217-Q2-U-1 to I.A1-Z217-Q2-U-42.

In another embodiment, compounds I are of formula I.A1 wherein Z is Z-217, R³ is CH₃ and Q is Q3, and R and R⁴ are as per any row of Table U below, which compounds are named I.A1-Z217-Q3-U-1 to I.A1-Z217-Q3-U-42.

In another embodiment, compounds I are of formula I.A1 wherein Z is Z-217, R³ is CH₃ and Q is Q4, and R and R⁴ are as per any row of Table U below, which compounds are named I.A1-Z217-Q4-U-1 to I.A1-Z217-Q4-U-42.

In another embodiment, compounds I are of formula I.A2 wherein Z is Z-217, R³ is CH₃ and Q is Q1, and R and R⁴ are as per any row of Table U below, which compounds are named I.A2-Z217-Q1-U-1 to I.A2-Z217-Q1-U-42.

In another embodiment, compounds I are of formula I.A2 wherein Z is Z-217, R³ is CH₃ and Q is Q2, and R and R⁴ are as per any row of Table U below, which compounds are named I.A2-Z217-Q2-U-1 to I.A2-Z217-Q2-U-42.

In another embodiment, compounds I are of formula I.A2 wherein Z is Z-217, R³ is CH₃ and Q is Q3, and R and R⁴ are as per any row of Table U below, which compounds are named I.A2-Z217-Q3-U-1 to I.A2-Z217-Q3-U-42.

In another embodiment, compounds I are of formula I.A2 wherein Z is Z-217, R³ is CH₃ and Q is Q4, and R and R⁴ are as per any row of Table U below, which compounds are named I.A2-Z217-Q4-U-1 to I.A2-Z217-Q4-U-42.

In another embodiment, compounds I are of formula I.A1 wherein Z is Z-218, R³ is CH₃ and Q is Q1, and R and R⁴ are as per any row of Table U below, which compounds are named I.A1-Z218-Q1-U-1 to I.A1-Z218-Q1-U-42.

In another embodiment, compounds I are of formula I.A1 wherein Z is Z-218, R³ is CH₃ and Q is Q2, and R and R⁴ are as per any row of Table U below, which compounds are named I.A1-Z218-Q2-U-1 to I.A1-Z218-Q2-U-42.

In another embodiment, compounds I are of formula I.A1 wherein Z is Z-218, R³ is CH₃ and Q is Q3, and R and R⁴ are as per any row of Table U below, which compounds are named I.A1-Z218-Q3-U-1 to I.A1-Z218-Q3-U-42.

In another embodiment, compounds I are of formula I.A1 wherein Z is Z-218, R³ is CH₃ and Q is Q4, and R and R⁴ are as per any row of Table U below, which compounds are named I.A1-Z218-Q4-U-1 to I.A1-Z218-Q4-U-42.

In another embodiment, compounds I are of formula I.A2 wherein Z is Z-218, R³ is CH₃ and Q is Q1, and R and R⁴ are as per any row of Table U below, which compounds are named I.A2-Z218-Q1-U-1 to I.A2-Z218-Q1-U-42.

In another embodiment, compounds I are of formula I.A2 wherein Z is Z-218, R³ is CH₃ and Q is Q2, and R and R⁴ are as per any row of Table U below, which compounds are named I.A2-Z218-Q2-U-1 to I.A2-Z218-Q2-U-42.

In another embodiment, compounds I are of formula I.A2 wherein Z is Z-218, R³ is CH₃ and Q is Q3, and R and R⁴ are as per any row of Table U below, which compounds are named I.A2-Z218-Q3-U-1 to I.A2-Z218-Q3-U-42.

In another embodiment, compounds I are of formula I.A2 wherein Z is Z-218, R³ is CH₃ and Q is Q4, and R and R⁴ are as per any row of Table U below, which compounds are named I.A2-Z218-Q4-U-1 to I.A2-Z218-Q4-U-42.

In another embodiment, compounds I are of formula I.A1 wherein Z is Z-219, R³ is CH₃ and Q is Q1, and R and R⁴ are as per any row of Table U below, which compounds are named I.A1-Z219-Q1-U-1 to I.A1-Z219-Q1-U-42.

In another embodiment, compounds I are of formula I.A1 wherein Z is Z-219, R³ is CH₃ and Q is Q2, and R and R⁴ are as per any row of Table U below, which compounds are named I.A1-Z219-Q2-U-1 to I.A1-Z219-Q2-U-42.

In another embodiment, compounds I are of formula I.A1 wherein Z is Z-219, R³ is CH₃ and Q is Q3, and R and R⁴ are as per any row of Table U below, which compounds are named I.A1-Z219-Q3-U-1 to I.A1-Z219-Q3-U-42.

In another embodiment, compounds I are of formula I.A1 wherein Z is Z-219, R³ is CH₃ and Q is Q4, and R and R⁴ are as per any row of Table U below, which compounds are named I.A1-Z219-Q4-U-1 to I.A1-Z219-Q4-U-42.

In another embodiment, compounds I are of formula I.A2 wherein Z is Z-219, R³ is CH₃ and Q is Q1, and R and R⁴ are as per any row of Table U below, which compounds are named I.A2-Z219-Q1-U-1 to I.A2-Z219-Q1-U-42.

In another embodiment, compounds I are of formula I.A2 wherein Z is Z-219, R³ is CH₃ and Q is Q2, and R and R⁴ are as per any row of Table U below, which compounds are named I.A2-Z219-Q2-U-1 to I.A2-Z219-Q2-U-42.

In another embodiment, compounds I are of formula I.A2 wherein Z is Z-219, R³ is CH₃ and Q is Q3, and R and R⁴ are as per any row of Table U below, which compounds are named I.A2-Z219-Q3-U-1 to I.A2-Z219-Q3-U-42.

In another embodiment, compounds I are of formula I.A2 wherein Z is Z-219, R³ is CH₃ and Q is Q4, and R and R⁴ are as per any row of Table U below, which compounds are named I.A2-Z219-Q4-U-1 to I.A2-Z219-Q4-U-42.

In another embodiment, compounds I are of formula I.A1 wherein Z is Z-220, R³ is CH₃ and Q is Q1, and R and R⁴ are as per any row of Table U below, which compounds are named I.A1-Z220-Q1-U-1 to I.A1-Z220-Q1-U-42.

In another embodiment, compounds I are of formula I.A1 wherein Z is Z-220, R³ is CH₃ and Q is Q2, and R and R⁴ are as per any row of Table U below, which compounds are named I.A1-Z220-Q2-U-1 to I.A1-Z220-Q2-U-42.

In another embodiment, compounds I are of formula I.A1 wherein Z is Z-220, R³ is CH₃ and Q is Q3, and R and R⁴ are as per any row of Table U below, which compounds are named I.A1-Z220-Q3-U-1 to I.A1-Z220-Q3-U-42.

In another embodiment, compounds I are of formula I.A1 wherein Z is Z-220, R³ is CH₃ and Q is Q4, and R and R⁴ are as per any row of Table U below, which compounds are named I.A1-Z220-Q4-U-1 to I.A1-Z220-Q4-U-42.

In another embodiment, compounds I are of formula I.A2 wherein Z is Z-220, R³ is CH₃ and Q is Q1, and R and R⁴ are as per any row of Table U below, which compounds are named I.A2-Z220-Q1-U-1 to I.A2-Z220-Q1-U-42.

In another embodiment, compounds I are of formula I.A2 wherein Z is Z-220, R³ is CH₃ and Q is Q2, and R and R⁴ are as per any row of Table U below, which compounds are named I.A2-Z220-Q2-U-1 to I.A2-Z220-Q2-U-42.

In another embodiment, compounds I are of formula I.A2 wherein Z is Z-220, R³ is CH₃ and Q is Q3, and R and R⁴ are as per any row of Table U below, which compounds are named I.A2-Z220-Q3-U-1 to I.A2-Z220-Q3-U-42.

In another embodiment, compounds I are of formula I.A2 wherein Z is Z-220, R³ is CH₃ and Q is Q4, and R and R⁴ are as per any row of Table U below, which compounds are named I.A2-Z220-Q4-U-1 to I.A2-Z220-Q4-U-42.

In another embodiment, compounds I are of formula I.A1 wherein Z is Z-221, R³ is CH₃ and Q is Q1, and R and R⁴ are as per any row of Table U below, which compounds are named I.A1-Z221-Q1-U-1 to I.A1-Z221-Q1-U-42.

In another embodiment, compounds I are of formula I.A1 wherein Z is Z-221, R³ is CH₃ and Q is Q2, and R and R⁴ are as per any row of Table U below, which compounds are named I.A1-Z221-Q2-U-1 to I.A1-Z221-Q2-U-42.

In another embodiment, compounds I are of formula I.A1 wherein Z is Z-221, R³ is CH₃ and Q is Q3, and R and R⁴ are as per any row of Table U below, which compounds are named I.A1-Z221-Q3-U-1 to I,A1-Z221-Q3-U-42.

In another embodiment, compounds I are of formula I.A1 wherein Z is Z-221, R³ is CH₃ and Q is Q4, and R and R⁴ are as per any row of Table U below, which compounds are named I.A1-Z221-Q4-U-1 to I.A1-Z221-Q4-U-42.

In another embodiment, compounds I are of formula I.A2 wherein Z is Z-221, R³ is CH₃ and Q is Q1, and R and R⁴ are as per any row of Table U below, which compounds are named I.A2-Z221-Q1-U-1 to I.A2-Z221-Q1-U-42.

In another embodiment, compounds I are of formula I.A2 wherein Z is Z-221, R³ is CH₃ and Q is Q2, and R and R⁴ are as per any row of Table U below, which compounds are named I.A2-Z221-Q2-U-1 to I.A2-Z221-Q2-U-42.

In another embodiment, compounds I are of formula I.A2 wherein Z is Z-221, R³ is CH₃ and Q is Q3, and R and R⁴ are as per any row of Table U below, which compounds are named I.A2-Z221-Q3-U-1 to I.A2-Z221-Q3-U-42.

In another embodiment, compounds I are of formula I.A2 wherein Z is Z-221, R³ is CH₃ and Q is Q4, and R and R⁴ are as per any row of Table U below, which compounds are named I.A2-Z221-Q4-U-1 to I.A2-Z221-Q4-U-42.

In another embodiment, compounds I are of formula I.A1 wherein Z is Z-222, R³ is CH₃ and Q is Q1, and R and R⁴ are as per any row of Table U below, which compounds are named I.A1-Z222-Q1-U-1 to I.A1-Z222-Q1-U-42.

In another embodiment, compounds I are of formula I.A1 wherein Z is Z-222, R³ is CH₃ and Q is Q2, and R and R⁴ are as per any row of Table U below, which compounds are named I.A1-Z222-Q2-U-1 to I.A1-Z222-Q2-U-42.

In another embodiment, compounds I are of formula I.A1 wherein Z is Z-222, R³ is CH₃ and Q is Q3, and R and R⁴ are as per any row of Table U below, which compounds are named I.A1-Z222-Q3-U-1 to I.A1-Z222-Q3-U-42.

In another embodiment, compounds I are of formula I.A1 wherein Z is Z-222, R³ is CH₃ and Q is Q4, and R and R⁴ are as per any row of Table U below, which compounds are named I.A1-Z222-Q4-U-1 to I.A1-Z222-Q4-U-42.

In another embodiment, compounds I are of formula I.A2 wherein Z is Z-222, R³ is CH₃ and Q is Q1, and R and R⁴ are as per any row of Table U below, which compounds are named I.A2-Z222-Q1-U-1 to I.A2-Z222-Q1-U-42.

In another embodiment, compounds I are of formula I.A2 wherein Z is Z-222, R³ is CH₃ and Q is Q2, and R and R⁴ are as per any row of Table U below, which compounds are named I.A2-Z222-Q2-U-1 to I.A2-Z222-Q2-U-42.

In another embodiment, compounds I are of formula I.A2 wherein Z is Z-222, R³ is CH₃ and Q is Q3, and R and R⁴ are as per any row of Table U below, which compounds are named I.A2-Z222-Q3-U-1 to I.A2-Z222-Q3-U-42.

In another embodiment, compounds I are of formula I.A2 wherein Z is Z-222, R³ is CH₃ and Q is Q4, and R and R⁴ are as per any row of Table U below, which compounds are named I.A2-Z222-Q4-U-1 to I.A2-Z222-Q4-U-42.

In another embodiment, compounds I are of formula I.A1 wherein Z is Z-223, R³ is CH₃ and Q is Q1, and R and R⁴ are as per any row of Table U below, which compounds are named I.A1-Z223-Q1-U-1 to I.A1-Z223-Q1-U-42.

In another embodiment, compounds I are of formula I.A1 wherein Z is Z-223, R³ is CH₃ and Q is Q2, and R and R⁴ are as per any row of Table U below, which compounds are named I.A1-Z223-Q2-U-1 to I.A1-Z223-Q2-U-42.

In another embodiment, compounds I are of formula I.A1 wherein Z is Z-223, R³ is CH₃ and Q is Q3, and R and R⁴ are as per any row of Table U below, which compounds are named I.A1-Z223-Q3-U-1 to I.A1-Z223-Q3-U-42.

In another embodiment, compounds I are of formula I.A1 wherein Z is Z-223, R³ is CH₃ and Q is Q4, and R and R⁴ are as per any row of Table U below, which compounds are named I.A1-Z223-Q4-U-1 to I.A1-Z223-Q4-U-42.

In another embodiment, compounds I are of formula I.A2 wherein Z is Z-223, R³ is CH₃ and Q is Q1, and R and R⁴ are as per any row of Table U below, which compounds are named I.A2-Z223-Q1-U-1 to I.A2-Z223-Q1-U-42.

In another embodiment, compounds I are of formula I.A2 wherein Z is Z-223, R³ is CH₃ and Q is Q2, and R and R⁴ are as per any row of Table U below, which compounds are named I.A2-Z223-Q2-U-1 to I.A2-Z223-Q2-U-42.

In another embodiment, compounds I are of formula I.A2 wherein Z is Z-223, R³ is CH₃ and Q is Q3, and R and R⁴ are as per any row of Table U below, which compounds are named I.A2-Z223-Q3-U-1 to I.A2-Z223-Q3-U-42.

In another embodiment, compounds I are of formula I.A2 wherein Z is Z-223, R³ is CH₃ and Q is Q4, and R and R⁴ are as per any row of Table U below, which compounds are named I.A2-Z223-Q4-U-1 to I.A2-Z223-Q4-U-42.

In another embodiment, compounds I are of formula I.A1 wherein Z is Z-224, R³ is CH₃ and Q is Q1, and R and R⁴ are as per any row of Table U below, which compounds are named I.A1-Z224-Q1-U-1 to I.A1-Z224-Q1-U-42.

In another embodiment, compounds I are of formula I.A1 wherein Z is Z-224, R³ is CH₃ and Q is Q2, and R and R⁴ are as per any row of Table U below, which compounds are named I.A1-Z224-Q2-U-1 to I.A1-Z224-Q2-U-42.

In another embodiment, compounds I are of formula I.A1 wherein Z is Z-224, R³ is CH₃ and Q is Q3, and R and R⁴ are as per any row of Table U below, which compounds are named I.A1-Z224-Q3-U-1 to I.A1-Z224-Q3-U-42.

In another embodiment, compounds I are of formula I.A1 wherein Z is Z-224, R³ is CH₃ and Q is Q4, and R and R⁴ are as per any row of Table U below, which compounds are named I.A1-Z224-Q4-U-1 to I.A1-Z224-Q4-U-42.

In another embodiment, compounds I are of formula I.A2 wherein Z is Z-224, R³ is CH₃ and Q is Q1, and R and R⁴ are as per any row of Table U below, which compounds are named I.A2-Z224-Q1-U-1 to I.A2-Z224-Q1-U-42.

In another embodiment, compounds I are of formula I.A2 wherein Z is Z-224, R³ is CH₃ and Q is Q2, and R and R⁴ are as per any row of Table U below, which compounds are named I.A2-Z224-Q2-U-1 to I.A2-Z224-Q2-U-42.

In another embodiment, compounds I are of formula I.A2 wherein Z is Z-224, R³ is CH₃ and Q is Q3, and R and R⁴ are as per any row of Table U below, which compounds are named I.A2-Z224-Q3-U-1 to I.A2-Z224-Q3-U-42.

In another embodiment, compounds I are of formula I.A2 wherein Z is Z-224, R³ is CH₃ and Q is Q4, and R and R⁴ are as per any row of Table U below, which compounds are named I.A2-Z224-Q4-U-1 to I.A2-Z224-Q4-U-42.

In another embodiment, compounds I are of formula I.A1 wherein Z is Z-225, R³ is CH₃ and Q is Q1, and R and R⁴ are as per any row of Table U below, which compounds are named I.A1-Z225-Q1-U-1 to I.A1-Z225-Q1-U-42.

In another embodiment, compounds I are of formula I.A1 wherein Z is Z-225, R³ is CH₃ and Q is Q2, and R and R⁴ are as per any row of Table U below, which compounds are named I.A1-Z225-Q2-U-1 to I.A1-Z225-Q2-U-42.

In another embodiment, compounds I are of formula I.A1 wherein Z is Z-225, R³ is CH₃ and Q is Q3, and R and R⁴ are as per any row of Table U below, which compounds are named I.A1-Z225-Q3-U-1 to I.A1-Z225-Q3-U-42.

In another embodiment, compounds I are of formula I.A1 wherein Z is Z-225, R³ is CH₃ and Q is Q4, and R and R⁴ are as per any row of Table U below, which compounds are named I.A1-Z225-Q4-U-1 to I.A1-Z225-Q4-U-42.

In another embodiment, compounds I are of formula I.A2 wherein Z is Z-225, R³ is CH₃ and Q is Q1, and R and R⁴ are as per any row of Table U below, which compounds are named I.A2-Z225-Q1-U-1 to I.A2-Z225-Q1-U-42.

In another embodiment, compounds I are of formula I.A2 wherein Z is Z-225, R³ is CH₃ and Q is Q2, and R and R⁴ are as per any row of Table U below, which compounds are named I.A2-Z225-Q2-U-1 to I.A2-Z225-Q2-U-42.

In another embodiment, compounds I are of formula I.A2 wherein Z is Z-225, R³ is CH₃ and Q is Q3, and R and R⁴ are as per any row of Table U below, which compounds are named I.A2-Z225-Q3-U-1 to I.A2-Z225-Q3-U-42.

In another embodiment, compounds I are of formula I.A2 wherein Z is Z-225, R³ is CH₃ and Q is Q4, and R and R⁴ are as per any row of Table U below, which compounds are named I.A2-Z225-Q4-U-1 to I.A2-Z225-Q4-U-42.

In another embodiment, compounds I are of formula I.A1 wherein Z is Z-226, R³ is CH₃ and Q is Q1, and R and R⁴ are as per any row of Table U below, which compounds are named I.A1-Z226-Q1-U-1 to I.A1-Z226-Q1-U-42.

In another embodiment, compounds I are of formula I.A1 wherein Z is Z-226, R³ is CH₃ and Q is Q2, and R and R⁴ are as per any row of Table U below, which compounds are named I.A1-Z226-Q2-U-1 to I.A1-Z226-Q2-U-42.

In another embodiment, compounds I are of formula I.A1 wherein Z is Z-226, R³ is CH₃ and Q is Q3, and R and R⁴ are as per any row of Table U below, which compounds are named I.A1-Z226-Q3-U-1 to I.A1-Z226-Q3-U-42.

In another embodiment, compounds I are of formula I.A1 wherein Z is Z-226, R³ is CH₃ and Q is Q4, and R and R⁴ are as per any row of Table U below, which compounds are named I.A1-Z226-Q4-U-1 to I.A1-Z226-Q4-U-42.

In another embodiment, compounds I are of formula I.A2 wherein Z is Z-226, R³ is CH₃ and Q is Q1, and R and R⁴ are as per any row of Table U below, which compounds are named I.A2-Z226-Q1-U-1 to I.A2-Z226-Q1-U-42.

In another embodiment, compounds I are of formula I.A2 wherein Z is Z-226, R³ is CH₃ and Q is Q2, and R and R⁴ are as per any row of Table U below, which compounds are named I.A2-Z226-Q2-U-1 to I.A2-Z226-Q2-U-42.

In another embodiment, compounds I are of formula I.A2 wherein Z is Z-226, R³ is CH₃ and Q is Q3, and R and R⁴ are as per any row of Table U below, which compounds are named I.A2-Z226-Q3-U-1 to I.A2-Z226-Q3-U-42.

In another embodiment, compounds I are of formula I.A2 wherein Z is Z-226, R³ is CH₃ and Q is Q4, and R and R⁴ are as per any row of Table U below, which compounds are named I.A2-Z226-Q4-U-1 to I.A2-Z226-Q4-U-42.

In another embodiment, compounds I are of formula I.A1 wherein Z is Z-227, R³ is CH₃ and Q is Q1, and R and R⁴ are as per any row of Table U below, which compounds are named I.A1-Z227-Q1-U-1 to I.A1-Z227-Q1-U-42.

In another embodiment, compounds I are of formula I.A1 wherein Z is Z-227, R³ is CH₃ and Q is Q2, and R and R⁴ are as per any row of Table U below, which compounds are named I.A1-Z227-Q2-U-1 to I.A1-Z227-Q2-U-42.

In another embodiment, compounds I are of formula I.A1 wherein Z is Z-227, R³ is CH₃ and Q is Q3, and R and R⁴ are as per any row of Table U below, which compounds are named I.A1-Z227-Q3-U-1 to I.A1-Z227-Q3-U-42.

In another embodiment, compounds I are of formula I.A1 wherein Z is Z-227, R³ is CH₃ and Q is Q4, and R and R⁴ are as per any row of Table U below, which compounds are named I.A1-Z227-Q4-U-1 to I.A1-Z227-Q4-U-42.

In another embodiment, compounds I are of formula I.A2 wherein Z is Z-227, R³ is CH₃ and Q is Q1, and R and R⁴ are as per any row of Table U below, which compounds are named I.A2-Z227-Q1-U-1 to I.A2-Z227-Q1-U-42.

In another embodiment, compounds I are of formula I.A2 wherein Z is Z-227, R³ is CH₃ and Q is Q2, and R and R⁴ are as per any row of Table U below, which compounds are named I.A2-Z227-Q2-U-1 to I.A2-Z227-Q2-U-42.

In another embodiment, compounds I are of formula I.A2 wherein Z is Z-227, R³ is CH₃ and Q is Q3, and R and R⁴ are as per any row of Table U below, which compounds are named I.A2-Z227-Q3-U-1 to I.A2-Z227-Q3-U-42.

In another embodiment, compounds I are of formula I.A2 wherein Z is Z-227, R³ is CH₃ and Q is Q4, and R and R⁴ are as per any row of Table U below, which compounds are named I.A2-Z227-Q4-U-1 to I.A2-Z227-Q4-U-42.

In another embodiment, compounds I are of formula I.A1 wherein Z is Z-228, R³ is CH₃ and Q is Q1, and R and R⁴ are as per any row of Table U below, which compounds are named I.A1-Z228-Q1-U-1 to I.A1-Z228-Q1-U-42.

In another embodiment, compounds I are of formula I.A1 wherein Z is Z-228, R³ is CH₃ and Q is Q2, and R and R⁴ are as per any row of Table U below, which compounds are named I.A1-Z228-Q2-U-1 to I.A1-Z228-Q2-U-42.

In another embodiment, compounds I are of formula I.A1 wherein Z is Z-228, R³ is CH₃ and Q is Q3, and R and R⁴ are as per any row of Table U below, which compounds are named I.A1-Z228-Q3-U-1 to I.A1-Z228-Q3-U-42.

In another embodiment, compounds I are of formula I.A1 wherein Z is Z-228, R³ is CH₃ and Q is Q4, and R and R⁴ are as per any row of Table U below, which compounds are named I.A1-Z228-Q4-U-1 to I.A1-Z228-Q4-U-42.

In another embodiment, compounds I are of formula I.A2 wherein Z is Z-228, R³ is CH₃ and Q is Q1, and R and R⁴ are as per any row of Table U below, which compounds are named I.A2-Z228-Q1-U-1 to I.A2-Z228-Q1-U-42.

In another embodiment, compounds I are of formula I.A2 wherein Z is Z-228, R³ is CH₃ and Q is Q2, and R and R⁴ are as per any row of Table U below, which compounds are named I.A2-Z228-Q2-U-1 to I.A2-Z228-Q2-U-42.

In another embodiment, compounds I are of formula I.A2 wherein Z is Z-228, R³ is CH₃ and Q is Q3, and R and R⁴ are as per any row of Table U below, which compounds are named I.A2-Z228-Q3-U-1 to I.A2-Z228-Q3-U-42.

In another embodiment, compounds I are of formula I.A2 wherein Z is Z-228, R³ is CH₃ and Q is Q4, and R and R⁴ are as per any row of Table U below, which compounds are named I.A2-Z228-Q4-U-1 to I.A2-Z228-Q4-U-42.

In another embodiment, compounds I are of formula I.A1 wherein Z is Z-229, R³ is CH₃ and Q is Q1, and R and R⁴ are as per any row of Table U below, which compounds are named I.A1-Z229-Q1-U-1 to I.A1-Z229-Q1-U-42.

In another embodiment, compounds I are of formula I.A1 wherein Z is Z-229, R³ is CH₃ and Q is Q2, and R and R⁴ are as per any row of Table U below, which compounds are named I.A1-Z229-Q2-U-1 to I.A1-Z229-Q2-U-42.

In another embodiment, compounds I are of formula I.A1 wherein Z is Z-229, R³ is CH₃ and Q is Q3, and R and R⁴ are as per any row of Table U below, which compounds are named I.A1-Z229-Q3-U-1 to I.A1-Z229-Q3-U-42.

In another embodiment, compounds I are of formula I.A1 wherein Z is Z-229, R³ is CH₃ and Q is Q4, and R and R⁴ are as per any row of Table U below, which compounds are named I.A1-Z229-Q4-U-1 to I.A1-Z229-Q4-U-42.

In another embodiment, compounds I are of formula I.A2 wherein Z is Z-229, R³ is CH₃ and Q is Q1, and R and R⁴ are as per any row of Table U below, which compounds are named I.A2-Z229-Q1-U-1 to I.A2-Z229-Q1-U-42.

In another embodiment, compounds I are of formula I.A2 wherein Z is Z-229, R³ is CH₃ and Q is Q2, and R and R⁴ are as per any row of Table U below, which compounds are named I.A2-Z229-Q2-U-1 to I.A2-Z229-Q2-U-42.

In another embodiment, compounds I are of formula I.A2 wherein Z is Z-229, R³ is CH₃ and Q is Q3, and R and R⁴ are as per any row of Table U below, which compounds are named I.A2-Z229-Q3-U-1 to I.A2-Z229-Q3-U-42.

In another embodiment, compounds I are of formula I.A2 wherein Z is Z-229, R³ is CH₃ and Q is Q4, and R and R⁴ are as per any row of Table U below, which compounds are named I.A2-Z229-Q4-U-1 to I.A2-Z229-Q4-U-42.

In another embodiment, compounds I are of formula I.A1 wherein Z is Z-230, R³ is CH₃ and Q is Q1, and R and R⁴ are as per any row of Table U below, which compounds are named I.A1-Z230-Q1-U-1 to I.A1-Z230-Q1-U-42.

In another embodiment, compounds I are of formula I.A1 wherein Z is Z-230, R³ is CH₃ and Q is Q2, and R and R⁴ are as per any row of Table U below, which compounds are named I.A1-Z230-Q2-U-1 to I.A1-Z230-Q2-U-42.

In another embodiment, compounds I are of formula I.A1 wherein Z is Z-230, R³ is CH₃ and Q is Q3, and R and R⁴ are as per any row of Table U below, which compounds are named I.A1-Z230-Q3-U-1 to I.A1-Z230-Q3-U-42.

In another embodiment, compounds I are of formula I.A1 wherein Z is Z-230, R³ is CH₃ and Q is Q4, and R and R⁴ are as per any row of Table U below, which compounds are named I.A1-Z230-Q4-U-1 to I.A1-Z230-Q4-U-42.

In another embodiment, compounds I are of formula I.A2 wherein Z is Z-230, R³ is CH₃ and Q is Q1, and R and R⁴ are as per any row of Table U below, which compounds are named I.A2-Z230-Q1-U-1 to I.A2-Z230-Q1-U-42.

In another embodiment, compounds I are of formula I.A2 wherein Z is Z-230, R³ is CH₃ and Q is Q2, and R and R⁴ are as per any row of Table U below, which compounds are named I.A2-Z230-Q2-U-1 to I.A2-Z230-Q2-U-42.

In another embodiment, compounds I are of formula I.A2 wherein Z is Z-230, R³ is CH₃ and Q is Q3, and R and R⁴ are as per any row of Table U below, which compounds are named I.A2-Z230-Q3-U-1 to I.A2-Z230-Q3-U-42.

In another embodiment, compounds I are of formula I.A2 wherein Z is Z-230, R³ is CH₃ and Q is Q4, and R and R⁴ are as per any row of Table U below, which compounds are named I.A2-Z230-Q4-U-1 to I.A2-Z230-Q4-U-42.

In another embodiment, compounds I are of formula I.A1 wherein Z is Z-231, R³ is CH₃ and Q is Q1, and R and R⁴ are as per any row of Table U below, which compounds are named I.A1-Z231-Q1-U-1 to I.A1-Z231-Q1-U-42.

In another embodiment, compounds I are of formula I.A1 wherein Z is Z-231, R³ is CH₃ and Q is Q2, and R and R⁴ are as per any row of Table U below, which compounds are named I.A1-Z231-Q2-U-1 to I.A1-Z231-Q2-U-42.

In another embodiment, compounds I are of formula I.A1 wherein Z is Z-231, R³ is CH₃ and Q is Q3, and R and R⁴ are as per any row of Table U below, which compounds are named I.A1-Z231-Q3-U-1 to I.A1-Z231-Q3-U-42.

In another embodiment, compounds I are of formula I.A1 wherein Z is Z-231, R³ is CH₃ and Q is Q4, and R and R⁴ are as per any row of Table U below, which compounds are named I.A1-Z231-Q4-U-1 to I.A1-Z231-Q4-U-42.

In another embodiment, compounds I are of formula I.A2 wherein Z is Z-231, R³ is CH₃ and Q is Q1, and R and R⁴ are as per any row of Table U below, which compounds are named I.A2-Z231-Q1-U-1 to I.A2-Z231-Q1-U-42.

In another embodiment, compounds I are of formula I.A2 wherein Z is Z-231, R³ is CH₃ and Q is Q2, and R and R⁴ are as per any row of Table U below, which compounds are named I.A2-Z231-Q2-U-1 to I.A2-Z231-Q2-U-42.

In another embodiment, compounds I are of formula I.A2 wherein Z is Z-231, R³ is CH₃ and Q is Q3, and R and R⁴ are as per any row of Table U below, which compounds are named I.A2-Z231-Q3-U-1 to I.A2-Z231-Q3-U-42.

In another embodiment, compounds I are of formula I.A2 wherein Z is Z-231, R³ is CH₃ and Q is Q4, and R and R⁴ are as per any row of Table U below, which compounds are named I.A2-Z231-Q4-U-1 to I.A2-Z231-Q4-U-42.

In another embodiment, compounds I are of formula I.A1 wherein Z is Z-232, R³ is CH₃ and Q is Q1, and R and R⁴ are as per any row of Table U below, which compounds are named I.A1-Z232-Q1-U-1 to I.A1-Z232-Q1-U-42.

In another embodiment, compounds I are of formula I.A1 wherein Z is Z-232, R³ is CH₃ and Q is Q2, and R and R⁴ are as per any row of Table U below, which compounds are named I.A1-Z232-Q2-U-1 to I.A1-Z232-Q2-U-42.

In another embodiment, compounds I are of formula I.A1 wherein Z is Z-232, R³ is CH₃ and Q is Q3, and R and R⁴ are as per any row of Table U below, which compounds are named I.A1-Z232-Q3-U-1 to I.A1-Z232-Q3-U-42.

In another embodiment, compounds I are of formula I.A1 wherein Z is Z-232, R³ is CH₃ and Q is Q4, and R and R⁴ are as per any row of Table U below, which compounds are named I.A1-Z232-Q4-U-1 to I.A1-Z232-Q4-U-42.

In another embodiment, compounds I are of formula I.A2 wherein Z is Z-232, R³ is CH₃ and Q is Q1, and R and R⁴ are as per any row of Table U below, which compounds are named I.A2-Z232-Q1-U-1 to I.A2-Z232-Q1-U-42.

In another embodiment, compounds I are of formula I.A2 wherein Z is Z-232, R³ is CH₃ and Q is Q2, and R and R⁴ are as per any row of Table U below, which compounds are named I.A2-Z232-Q2-U-1 to I.A2-Z232-Q2-U-42.

In another embodiment, compounds I are of formula I.A2 wherein Z is Z-232, R³ is CH₃ and Q is Q3, and R and R⁴ are as per any row of Table U below, which compounds are named I.A2-Z232-Q3-U-1 to I.A2-Z232-Q3-U-42.

In another embodiment, compounds I are of formula I.A2 wherein Z is Z-232, R³ is CH₃ and Q is Q4, and R and R⁴ are as per any row of Table U below, which compounds are named I.A2-Z232-Q4-U-1 to I.A2-Z232-Q4-U-42.

In another embodiment, compounds I are of formula I.A1 wherein Z is Z-233, R³ is CH₃ and Q is Q1, and R and R⁴ are as per any row of Table U below, which compounds are named I.A1-Z233-Q1-U-1 to I.A1-Z233-Q1-U-42.

In another embodiment, compounds I are of formula I.A1 wherein Z is Z-233, R³ is CH₃ and Q is Q2, and R and R⁴ are as per any row of Table U below, which compounds are named I.A1-Z233-Q2-U-1 to I.A1-Z233-Q2-U-42.

In another embodiment, compounds I are of formula I.A1 wherein Z is Z-233, R³ is CH₃ and Q is Q3, and R and R⁴ are as per any row of Table U below, which compounds are named I.A1-Z233-Q3-U-1 to I.A1-Z233-Q3-U-42.

In another embodiment, compounds I are of formula I.A1 wherein Z is Z-233, R³ is CH₃ and Q is Q4, and R and R⁴ are as per any row of Table U below, which compounds are named I.A1-Z233-Q4-U-1 to I.A1-Z233-Q4-U-42.

In another embodiment, compounds I are of formula I.A2 wherein Z is Z-233, R³ is CH₃ and Q is Q1, and R and R⁴ are as per any row of Table U below, which compounds are named I.A2-Z233-Q1-U-1 to I.A2-Z233-Q1-U-42.

In another embodiment, compounds I are of formula I.A2 wherein Z is Z-233, R³ is CH₃ and Q is Q2, and R and R⁴ are as per any row of Table U below, which compounds are named I.A2-Z233-Q2-U-1 to I.A2-Z233-Q2-U-42.

In another embodiment, compounds I are of formula I.A2 wherein Z is Z-233, R³ is CH₃ and Q is Q3, and R and R⁴ are as per any row of Table U below, which compounds are named I.A2-Z233-Q3-U-1 to I.A2-Z233-Q3-U-42.

In another embodiment, compounds I are of formula I.A2 wherein Z is Z-233, R³ is CH₃ and Q is Q4, and R and R⁴ are as per any row of Table U below, which compounds are named I.A2-Z233-Q4-U-1 to I.A2-Z233-Q4-U-42.

In another embodiment, compounds I are of formula I.A1 wherein Z is Z-234, R³ is CH₃ and Q is Q1, and R and R⁴ are as per any row of Table U below, which compounds are named I.A1-Z234-Q1-U-1 to I.A1-Z234-Q1-U-42.

In another embodiment, compounds I are of formula I.A1 wherein Z is Z-234, R³ is CH₃ and Q is Q2, and R and R⁴ are as per any row of Table U below, which compounds are named I.A1-Z234-Q2-U-1 to I.A1-Z234-Q2-U-42.

In another embodiment, compounds I are of formula I.A1 wherein Z is Z-234, R³ is CH₃ and Q is Q3, and R and R⁴ are as per any row of Table U below, which compounds are named I.A1-Z234-Q3-U-1 to I.A1-Z234-Q3-U-42.

In another embodiment, compounds I are of formula I.A1 wherein Z is Z-234, R³ is CH₃ and Q is Q4, and R and R⁴ are as per any row of Table U below, which compounds are named I.A1-Z234-Q4-U-1 to I.A1-Z234-Q4-U-42.

In another embodiment, compounds I are of formula I.A2 wherein Z is Z-234, R³ is CH₃ and Q is Q1, and R and R⁴ are as per any row of Table U below, which compounds are named I.A2-Z234-Q1-U-1 to I.A2-Z234-Q1-U-42.

In another embodiment, compounds I are of formula I.A2 wherein Z is Z-234, R³ is CH₃ and Q is Q2, and R and R⁴ are as per any row of Table U below, which compounds are named I.A2-Z234-Q2-U-1 to I.A2-Z234-Q2-U-42.

In another embodiment, compounds I are of formula I.A2 wherein Z is Z-234, R³ is CH₃ and Q is Q3, and R and R⁴ are as per any row of Table U below, which compounds are named I.A2-Z234-Q3-U-1 to I.A2-Z234-Q3-U-42.

In another embodiment, compounds I are of formula I.A2 wherein Z is Z-234, R³ is CH₃ and Q is Q4, and R and R⁴ are as per any row of Table U below, which compounds are named I.A2-Z234-Q4-U-1 to I.A2-Z234-Q4-U-42.

In another embodiment, compounds I are of formula I.A1 wherein Z is Z-235, R³ is CH₃ and Q is Q1, and R and R⁴ are as per any row of Table U below, which compounds are named I.A1-Z235-Q1-U-1 to I.A1-Z235-Q1-U-42.

In another embodiment, compounds I are of formula I.A1 wherein Z is Z-235, R³ is CH₃ and Q is Q2, and R and R⁴ are as per any row of Table U below, which compounds are named I.A1-Z235-Q2-U-1 to I.A1-Z235-Q2-U-42.

In another embodiment, compounds I are of formula I.A1 wherein Z is Z-235, R³ is CH₃ and Q is Q3, and R and R⁴ are as per any row of Table U below, which compounds are named I.A1-Z235-Q3-U-1 to I.A1-Z235-Q3-U-42.

In another embodiment, compounds I are of formula I.A1 wherein Z is Z-235, R³ is CH₃ and Q is Q4, and R and R⁴ are as per any row of Table U below, which compounds are named I.A1-Z235-Q4-U-1 to I.A1-Z235-Q4-U-42.

In another embodiment, compounds I are of formula I.A2 wherein Z is Z-235, R³ is CH₃ and Q is Q1, and R and R⁴ are as per any row of Table U below, which compounds are named I.A2-Z235-Q1-U-1 to I.A2-Z235-Q1-U-42.

In another embodiment, compounds I are of formula I.A2 wherein Z is Z-235, R³ is CH₃ and Q is Q2, and R and R⁴ are as per any row of Table U below, which compounds are named I.A2-Z235-Q2-U-1 to I.A2-Z235-Q2-U-42.

In another embodiment, compounds I are of formula I.A2 wherein Z is Z-235, R³ is CH₃ and Q is Q3, and R and R⁴ are as per any row of Table U below, which compounds are named I.A2-Z235-Q3-U-1 to I.A2-Z235-Q3-U-42.

In another embodiment, compounds I are of formula I.A2 wherein Z is Z-235, R³ is CH₃ and Q is Q4, and R and R⁴ are as per any row of Table U below, which compounds are named I.A2-Z235-Q4-U-1 to I.A2-Z235-Q4-U-42.

In another embodiment, compounds I are of formula I.A1 wherein Z is Z-236, R³ is CH₃ and Q is Q1, and R and R⁴ are as per any row of Table U below, which compounds are named I.A1-Z236-Q1-U-1 to I.A1-Z236-Q1-U-42.

In another embodiment, compounds I are of formula I.A1 wherein Z is Z-236, R³ is CH₃ and Q is Q2, and R and R⁴ are as per any row of Table U below, which compounds are named I.A1-Z236-Q2-U-1 to I.A1-Z236-Q2-U-42.

In another embodiment, compounds I are of formula I.A1 wherein Z is Z-236, R³ is CH₃ and Q is Q3, and R and R⁴ are as per any row of Table U below, which compounds are named I.A1-Z236-Q3-U-1 to I.A1-Z236-Q3-U-42.

In another embodiment, compounds I are of formula I.A1 wherein Z is Z-236, R³ is CH₃ and Q is Q4, and R and R⁴ are as per any row of Table U below, which compounds are named I.A1-Z236-Q4-U-1 to I.A1-Z236-Q4-U-42.

In another embodiment, compounds I are of formula I.A2 wherein Z is Z-236, R³ is CH₃ and Q is Q1, and R and R⁴ are as per any row of Table U below, which compounds are named I.A2-Z236-Q1-U-1 to I.A2-Z236-Q1-U-42.

In another embodiment, compounds I are of formula I.A2 wherein Z is Z-236, R³ is CH₃ and Q is Q2, and R and R⁴ are as per any row of Table U below, which compounds are named I.A2-Z236-Q2-U-1 to I.A2-Z236-Q2-U-42.

In another embodiment, compounds I are of formula I.A2 wherein Z is Z-236, R³ is CH₃ and Q is Q3, and R and R⁴ are as per any row of Table U below, which compounds are named I.A2-Z236-Q3-U-1 to I.A2-Z236-Q3-U-42.

In another embodiment, compounds I are of formula I.A2 wherein Z is Z-236, R³ is CH₃ and Q is Q4, and R and R⁴ are as per any row of Table U below, which compounds are named I.A2-Z236-Q4-U-1 to I.A2-Z236-Q4-U-42.

In another embodiment, compounds I are of formula I.A1 wherein Z is Z-237, R³ is CH₃ and Q is Q1, and R and R⁴ are as per any row of Table U below, which compounds are named I.A1-Z237-Q1-U-1 to I.A1-Z237-Q1-U-42.

In another embodiment, compounds I are of formula I.A1 wherein Z is Z-237, R³ is CH₃ and Q is Q2, and R and R⁴ are as per any row of Table U below, which compounds are named I.A1-Z237-Q2-U-1 to I.A1-Z237-Q2-U-42.

In another embodiment, compounds I are of formula I.A1 wherein Z is Z-237, R³ is CH₃ and Q is Q3, and R and R⁴ are as per any row of Table U below, which compounds are named I.A1-Z237-Q3-U-1 to I.A1-Z237-Q3-U-42.

In another embodiment, compounds I are of formula I.A1 wherein Z is Z-237, R³ is CH₃ and Q is Q4, and R and R⁴ are as per any row of Table U below, which compounds are named I.A1-Z237-Q4-U-1 to I.A1-Z237-Q4-U-42.

In another embodiment, compounds I are of formula I.A2 wherein Z is Z-237, R³ is CH₃ and Q is Q1, and R and R⁴ are as per any row of Table U below, which compounds are named I.A2-Z237-Q1-U-1 to I.A2-Z237-Q1-U-42.

In another embodiment, compounds I are of formula I.A2 wherein Z is Z-237, R³ is CH₃ and Q is Q2, and R and R⁴ are as per any row of Table U below, which compounds are named I.A2-Z237-Q2-U-1 to I.A2-Z237-Q2-U-42.

In another embodiment, compounds I are of formula I.A2 wherein Z is Z-237, R³ is CH₃ and Q is Q3, and R and R⁴ are as per any row of Table U below, which compounds are named I.A2-Z237-Q3-U-1 to I.A2-Z237-Q3-U-42.

In another embodiment, compounds I are of formula I.A2 wherein Z is Z-237, R³ is CH₃ and Q is Q4, and R and R⁴ are as per any row of Table U below, which compounds are named I.A2-Z237-Q4-U-1 to I.A2-Z237-Q4-U-42.

In another embodiment, compounds I are of formula I.A1 wherein Z is Z-238, R³ is CH₃ and Q is Q1, and R and R⁴ are as per any row of Table U below, which compounds are named I.A1-Z238-Q1-U-1 to I.A1-Z238-Q1-U-42.

In another embodiment, compounds I are of formula I.A1 wherein Z is Z-238, R³ is CH₃ and Q is Q2, and R and R⁴ are as per any row of Table U below, which compounds are named I.A1-Z238-Q2-U-1 to I.A1-Z238-Q2-U-42.

In another embodiment, compounds I are of formula I.A1 wherein Z is Z-238, R³ is CH₃ and Q is Q3, and R and R⁴ are as per any row of Table U below, which compounds are named I.A1-Z238-Q3-U-1 to I.A1-Z238-Q3-U-42.

In another embodiment, compounds I are of formula I.A1 wherein Z is Z-238, R³ is CH₃ and Q is Q4, and R and R⁴ are as per any row of Table U below, which compounds are named I.A1-Z238-Q4-U-1 to I.A1-Z238-Q4-U-42.

In another embodiment, compounds I are of formula I.A2 wherein Z is Z-238, R³ is CH₃ and Q is Q1, and R and R⁴ are as per any row of Table U below, which compounds are named I.A2-Z238-Q1-U-1 to I.A2-Z238-Q1-U-42.

In another embodiment, compounds I are of formula I.A2 wherein Z is Z-238, R³ is CH₃ and Q is Q2, and R and R⁴ are as per any row of Table U below, which compounds are named I.A2-Z238-Q2-U-1 to I.A2-Z238-Q2-U-42.

In another embodiment, compounds I are of formula I.A2 wherein Z is Z-238, R³ is CH₃ and Q is Q3, and R and R⁴ are as per any row of Table U below, which compounds are named I.A2-Z238-Q3-U-1 to I.A2-Z238-Q3-U-42.

In another embodiment, compounds I are of formula I.A2 wherein Z is Z-238, R³ is CH₃ and Q is Q4, and R and R⁴ are as per any row of Table U below, which compounds are named I.A2-Z238-Q4-U-1 to I.A2-Z238-Q4-U-42.

In another embodiment, compounds I are of formula I.A1 wherein Z is Z-239, R³ is CH₃ and Q is Q1, and R and R⁴ are as per any row of Table U below, which compounds are named I.A1-Z239-Q1-U-1 to I.A1-Z239-Q1-U-42.

In another embodiment, compounds I are of formula I.A1 wherein Z is Z-239, R³ is CH₃ and Q is Q2, and R and R⁴ are as per any row of Table U below, which compounds are named I.A1-Z239-Q2-U-1 to I.A1-Z239-Q2-U-42.

In another embodiment, compounds I are of formula I.A1 wherein Z is Z-239, R³ is CH₃ and Q is Q3, and R and R⁴ are as per any row of Table U below, which compounds are named I.A1-Z239-Q3-U-1 to I.A1-Z239-Q3-U-42.

In another embodiment, compounds I are of formula I.A1 wherein Z is Z-239, R³ is CH₃ and Q is Q4, and R and R⁴ are as per any row of Table U below, which compounds are named I.A1-Z239-Q4-U-1 to I.A1-Z239-Q4-U-42.

In another embodiment, compounds I are of formula I.A2 wherein Z is Z-239, R³ is CH₃ and Q is Q1, and R and R⁴ are as per any row of Table U below, which compounds are named I.A2-Z239-Q1-U-1 to I.A2-Z239-Q1-U-42.

In another embodiment, compounds I are of formula I.A2 wherein Z is Z-239, R³ is CH₃ and Q is Q2, and R and R⁴ are as per any row of Table U below, which compounds are named I.A2-Z239-Q2-U-1 to I.A2-Z239-Q2-U-42.

In another embodiment, compounds I are of formula I.A2 wherein Z is Z-239, R³ is CH₃ and Q is Q3, and R and R⁴ are as per any row of Table U below, which compounds are named I.A2-Z239-Q3-U-1 to I.A2-Z239-Q3-U-42.

In another embodiment, compounds I are of formula I.A2 wherein Z is Z-239, R³ is CH₃ and Q is Q4, and R and R⁴ are as per any row of Table U below, which compounds are named I.A2-Z239-Q4-U-1 to I.A2-Z239-Q4-U-42.

In another embodiment, compounds I are of formula I.A1 wherein Z is Z-240, R³ is CH₃ and Q is Q1, and R and R⁴ are as per any row of Table U below, which compounds are named I.A1-Z240-Q1-U-1 to I.A1-Z240-Q1-U-42.

In another embodiment, compounds I are of formula I.A1 wherein Z is Z-240, R³ is CH₃ and Q is Q2, and R and R⁴ are as per any row of Table U below, which compounds are named I.A1-Z240-Q2-U-1 to I.A1-Z240-Q2-U-42.

In another embodiment, compounds I are of formula I.A1 wherein Z is Z-240, R³ is CH₃ and Q is Q3, and R and R⁴ are as per any row of Table U below, which compounds are named I.A1-Z240-Q3-U-1 to I.A1-Z240-Q3-U-42.

In another embodiment, compounds I are of formula I.A1 wherein Z is Z-240, R³ is CH₃ and Q is Q4, and R and R⁴ are as per any row of Table U below, which compounds are named I.A1-Z240-Q4-U-1 to I.A1-Z240-Q4-U-42.

In another embodiment, compounds I are of formula I.A2 wherein Z is Z-240, R³ is CH₃ and Q is Q1, and R and R⁴ are as per any row of Table U below, which compounds are named I.A2-Z240-Q1-U-1 to I.A2-Z240-Q1-U-42.

In another embodiment, compounds I are of formula I.A2 wherein Z is Z-240, R³ is CH₃ and Q is Q2, and R and R⁴ are as per any row of Table U below, which compounds are named I.A2-Z240-Q2-U-1 to I.A2-Z240-Q2-U-42.

In another embodiment, compounds I are of formula I.A2 wherein Z is Z-240, R³ is CH₃ and Q is Q3, and R and R⁴ are as per any row of Table U below, which compounds are named I.A2-Z240-Q3-U-1 to I.A2-Z240-Q3-U-42.

In another embodiment, compounds I are of formula I.A2 wherein Z is Z-240, R³ is CH₃ and Q is Q4, and R and R⁴ are as per any row of Table U below, which compounds are named I.A2-Z240-Q4-U-1 to I.A2-Z240-Q4-U-42.

In another embodiment, compounds I are of formula I.A1 wherein Z is Z-241, R³ is CH₃ and Q is Q1, and R and R⁴ are as per any row of Table U below, which compounds are named I.A1-Z241-Q1-U-1 to I.A1-Z241-Q1-U-42.

In another embodiment, compounds I are of formula I.A1 wherein Z is Z-241, R³ is CH₃ and Q is Q2, and R and R⁴ are as per any row of Table U below, which compounds are named I.A1-Z241-Q2-U-1 to I.A1-Z241-Q2-U-42.

In another embodiment, compounds I are of formula I.A1 wherein Z is Z-241, R³ is CH₃ and Q is Q3, and R and R⁴ are as per any row of Table U below, which compounds are named I.A1-Z241-Q3-U-1 to I.A1-Z241-Q3-U-42.

In another embodiment, compounds I are of formula I.A1 wherein Z is Z-241, R³ is CH₃ and Q is Q4, and R and R⁴ are as per any row of Table U below, which compounds are named I.A1-Z241-Q4-U-1 to I.A1-Z241-Q4-U-42.

In another embodiment, compounds I are of formula I.A2 wherein Z is Z-241, R³ is CH₃ and Q is Q1, and R and R⁴ are as per any row of Table U below, which compounds are named I.A2-Z241-Q1-U-1 to I.A2-Z241-Q1-U-42.

In another embodiment, compounds I are of formula I.A2 wherein Z is Z-241, R³ is CH₃ and Q is Q2, and R and R⁴ are as per any row of Table U below, which compounds are named I.A2-Z241-Q2-U-1 to I.A2-Z241-Q2-U-42.

In another embodiment, compounds I are of formula I.A2 wherein Z is Z-241, R³ is CH₃ and Q is Q3, and R and R⁴ are as per any row of Table U below, which compounds are named I.A2-Z241-Q3-U-1 to I.A2-Z241-Q3-U-42.

In another embodiment, compounds I are of formula I.A2 wherein Z is Z-241, R³ is CH₃ and Q is Q4, and R and R⁴ are as per any row of Table U below, which compounds are named I.A2-Z241-Q4-U-1 to I.A2-Z241-Q4-U-42.

In another embodiment, compounds I are of formula I.A1 wherein Z is Z-242, R³ is CH₃ and Q is Q1, and R and R⁴ are as per any row of Table U below, which compounds are named I.A1-Z242-Q1-U-1 to I.A1-Z242-Q1-U-42.

In another embodiment, compounds I are of formula I.A1 wherein Z is Z-242, R³ is CH₃ and Q is Q2, and R and R⁴ are as per any row of Table U below, which compounds are named I.A1-Z242-Q2-U-1 to I.A1-Z242-Q2-U-42.

In another embodiment, compounds I are of formula I.A1 wherein Z is Z-242, R³ is CH₃ and Q is Q3, and R and R⁴ are as per any row of Table U below, which compounds are named I.A1-Z242-Q3-U-1 to I.A1-Z242-Q3-U-42.

In another embodiment, compounds I are of formula I.A1 wherein Z is Z-242, R³ is CH₃ and Q is Q4, and R and R⁴ are as per any row of Table U below, which compounds are named I.A1-Z242-Q4-U-1 to I.A1-Z242-Q4-U-42.

In another embodiment, compounds I are of formula I.A2 wherein Z is Z-242, R³ is CH₃ and Q is Q1, and R and R⁴ are as per any row of Table U below, which compounds are named I.A2-Z242-Q1-U-1 to I.A2-Z242-Q1-U-42.

In another embodiment, compounds I are of formula I.A2 wherein Z is Z-242, R³ is CH₃ and Q is Q2, and R and R⁴ are as per any row of Table U below, which compounds are named I.A2-Z242-Q2-U-1 to I.A2-Z242-Q2-U-42.

In another embodiment, compounds I are of formula I.A2 wherein Z is Z-242, R³ is CH₃ and Q is Q3, and R and R⁴ are as per any row of Table U below, which compounds are named I.A2-Z242-Q3-U-1 to I.A2-Z242-Q3-U-42.

In another embodiment, compounds I are of formula I.A2 wherein Z is Z-242, R³ is CH₃ and Q is Q4, and R and R⁴ are as per any row of Table U below, which compounds are named I.A2-Z242-Q4-U-1 to I.A2-Z242-Q4-U-42.

In another embodiment, compounds I are of formula I.A1 wherein Z is Z-243, R³ is CH₃ and Q is Q1, and R and R⁴ are as per any row of Table U below, which compounds are named I.A1-Z243-Q1-U-1 to I.A1-Z243-Q1-U-42.

In another embodiment, compounds I are of formula I.A1 wherein Z is Z-243, R³ is CH₃ and Q is Q2, and R and R⁴ are as per any row of Table U below, which compounds are named I.A1-Z243-Q2-U-1 to I.A1-Z243-Q2-U-42.

In another embodiment, compounds I are of formula I.A1 wherein Z is Z-243, R³ is CH₃ and Q is Q3, and R and R⁴ are as per any row of Table U below, which compounds are named I.A1-Z243-Q3-U-1 to I.A1-Z243-Q3-U-42.

In another embodiment, compounds I are of formula I.A1 wherein Z is Z-243, R³ is CH₃ and Q is Q4, and R and R⁴ are as per any row of Table U below, which compounds are named I.A1-Z243-Q4-U-1 to I.A1-Z243-Q4-U-42.

In another embodiment, compounds I are of formula I.A2 wherein Z is Z-243, R³ is CH₃ and Q is Q1, and R and R⁴ are as per any row of Table U below, which compounds are named I.A2-Z243-Q1-U-1 to I.A2-Z243-Q1-U-42.

In another embodiment, compounds I are of formula I.A2 wherein Z is Z-243, R³ is CH₃ and Q is Q2, and R and R⁴ are as per any row of Table U below, which compounds are named I.A2-Z243-Q2-U-1 to I.A2-Z243-Q2-U-42.

In another embodiment, compounds I are of formula I.A2 wherein Z is Z-243, R³ is CH₃ and Q is Q3, and R and R⁴ are as per any row of Table U below, which compounds are named I.A2-Z243-Q3-U-1 to I.A2-Z243-Q3-U-42.

In another embodiment, compounds I are of formula I.A2 wherein Z is Z-243, R³ is CH₃ and Q is Q4, and R and R⁴ are as per any row of Table U below, which compounds are named I.A2-Z243-Q4-U-1 to I.A2-Z243-Q4-U-42.

In another embodiment, compounds I are of formula I.A1 wherein Z is Z-244, R³ is CH₃ and Q is Q1, and R and R⁴ are as per any row of Table U below, which compounds are named I.A1-Z244-Q1-U-1 to I.A1-Z244-Q1-U-42.

In another embodiment, compounds I are of formula I.A1 wherein Z is Z-244, R³ is CH₃ and Q is Q2, and R and R⁴ are as per any row of Table U below, which compounds are named I.A1-Z244-Q2-U-1 to I.A1-Z244-Q2-U-42.

In another embodiment, compounds I are of formula I.A1 wherein Z is Z-244, R³ is CH₃ and Q is Q3, and R and R⁴ are as per any row of Table U below, which compounds are named I.A1-Z244-Q3-U-1 to I.A1-Z244-Q3-U-42.

In another embodiment, compounds I are of formula I.A1 wherein Z is Z-244, R³ is CH₃ and Q is Q4, and R and R⁴ are as per any row of Table U below, which compounds are named I.A1-Z244-Q4-U-1 to I.A1-Z244-Q4-U-42.

In another embodiment, compounds I are of formula I.A2 wherein Z is Z-244, R³ is CH₃ and Q is Q1, and R and R⁴ are as per any row of Table U below, which compounds are named I.A2-Z244-Q1-U-1 to I.A2-Z244-Q1-U-42.

In another embodiment, compounds I are of formula I.A2 wherein Z is Z-244, R³ is CH₃ and Q is Q2, and R and R⁴ are as per any row of Table U below, which compounds are named I.A2-Z244-Q2-U-1 to I.A2-Z244-Q2-U-42.

In another embodiment, compounds I are of formula I.A2 wherein Z is Z-244, R³ is CH₃ and Q is Q3, and R and R⁴ are as per any row of Table U below, which compounds are named I.A2-Z244-Q3-U-1 to I.A2-Z244-Q3-U-42.

In another embodiment, compounds I are of formula I.A2 wherein Z is Z-244, R³ is CH₃ and Q is Q4, and R and R⁴ are as per any row of Table U below, which compounds are named I.A2-Z244-Q4-U-1 to I.A2-Z244-Q4-U-42.

In another embodiment, compounds I are of formula I.A1 wherein Z is Z-245, R³ is CH₃ and Q is Q1, and R and R⁴ are as per any row of Table U below, which compounds are named I.A1-Z245-Q1-U-1 to I.A1-Z245-Q1-U-42.

In another embodiment, compounds I are of formula I.A1 wherein Z is Z-245, R³ is CH₃ and Q is Q2, and R and R⁴ are as per any row of Table U below, which compounds are named I.A1-Z245-Q2-U-1 to I.A1-Z245-Q2-U-42.

In another embodiment, compounds I are of formula I.A1 wherein Z is Z-245, R³ is CH₃ and Q is Q3, and R and R⁴ are as per any row of Table U below, which compounds are named I.A1-Z245-Q3-U-1 to I.A1-Z245-Q3-U-42.

In another embodiment, compounds I are of formula I.A1 wherein Z is Z-245, R³ is CH₃ and Q is Q4, and R and R⁴ are as per any row of Table U below, which compounds are named I.A1-Z245-Q4-U-1 to I.A1-Z245-Q4-U-42.

In another embodiment, compounds I are of formula I.A2 wherein Z is Z-245, R³ is CH₃ and Q is Q1, and R and R⁴ are as per any row of Table U below, which compounds are named I.A2-Z245-Q1-U-1 to I.A2-Z245-Q1-U-42.

In another embodiment, compounds I are of formula I.A2 wherein Z is Z-245, R³ is CH₃ and Q is Q2, and R and R⁴ are as per any row of Table U below, which compounds are named I.A2-Z245-Q2-U-1 to I.A2-Z245-Q2-U-42.

In another embodiment, compounds I are of formula I.A2 wherein Z is Z-245, R³ is CH₃ and Q is Q3, and R and R⁴ are as per any row of Table U below, which compounds are named I.A2-Z245-Q3-U-1 to I.A2-Z245-Q3-U-42.

In another embodiment, compounds I are of formula I.A2 wherein Z is Z-245, R³ is CH₃ and Q is Q4, and R and R⁴ are as per any row of Table U below, which compounds are named I.A2-Z245-Q4-U-1 to I.A2-Z245-Q4-U-42.

In another embodiment, compounds I are of formula I.A1 wherein Z is Z-246, R³ is CH₃ and Q is Q1, and R and R⁴ are as per any row of Table U below, which compounds are named I.A1-Z246-Q1-U-1 to I.A1-Z246-Q1-U-42.

In another embodiment, compounds I are of formula I.A1 wherein Z is Z-246, R³ is CH₃ and Q is Q2, and R and R⁴ are as per any row of Table U below, which compounds are named I.A1-Z246-Q2-U-1 to I.A1-Z246-Q2-U-42.

In another embodiment, compounds I are of formula I.A1 wherein Z is Z-246, R³ is CH₃ and Q is Q3, and R and R⁴ are as per any row of Table U below, which compounds are named I.A1-Z246-Q3-U-1 to I.A1-Z246-Q3-U-42.

In another embodiment, compounds I are of formula I.A1 wherein Z is Z-246, R³ is CH₃ and Q is Q4, and R and R⁴ are as per any row of Table U below, which compounds are named I.A1-Z246-Q4-U-1 to I.A1-Z246-Q4-U-42.

In another embodiment, compounds I are of formula I.A2 wherein Z is Z-246, R³ is CH₃ and Q is Q1, and R and R⁴ are as per any row of Table U below, which compounds are named I.A2-Z246-Q1-U-1 to I.A2-Z246-Q1-U-42.

In another embodiment, compounds I are of formula I.A2 wherein Z is Z-246, R³ is CH₃ and Q is Q2, and R and R⁴ are as per any row of Table U below, which compounds are named I.A2-Z246-Q2-U-1 to I.A2-Z246-Q2-U-42.

In another embodiment, compounds I are of formula I.A2 wherein Z is Z-246, R³ is CH₃ and Q is Q3, and R and R⁴ are as per any row of Table U below, which compounds are named I.A2-Z246-Q3-U-1 to I.A2-Z246-Q3-U-42.

In another embodiment, compounds I are of formula I.A2 wherein Z is Z-246, R³ is CH₃ and Q is Q4, and R and R⁴ are as per any row of Table U below, which compounds are named I.A2-Z246-Q4-U-1 to I.A2-Z246-Q4-U-42.

In another embodiment, compounds I are of formula I.A1 wherein Z is Z-247, R³ is CH₃ and Q is Q1, and R and R⁴ are as per any row of Table U below, which compounds are named I.A1-Z247-Q1-U-1 to I.A1-Z247-Q1-U-42.

In another embodiment, compounds I are of formula I.A1 wherein Z is Z-247, R³ is CH₃ and Q is Q2, and R and R⁴ are as per any row of Table U below, which compounds are named I.A1-Z247-Q2-U-1 to I.A1-Z247-Q2-U-42.

In another embodiment, compounds I are of formula I.A1 wherein Z is Z-247, R³ is CH₃ and Q is Q3, and R and R⁴ are as per any row of Table U below, which compounds are named I.A1-Z247-Q3-U-1 to I.A1-Z247-Q3-U-42.

In another embodiment, compounds I are of formula I.A1 wherein Z is Z-247, R³ is CH₃ and Q is Q4, and R and R⁴ are as per any row of Table U below, which compounds are named I.A1-Z247-Q4-U-1 to I.A1-Z247-Q4-U-42.

In another embodiment, compounds I are of formula I.A2 wherein Z is Z-247, R³ is CH₃ and Q is Q1, and R and R⁴ are as per any row of Table U below, which compounds are named I.A2-Z247-Q1-U-1 to I.A2-Z247-Q1-U-42.

In another embodiment, compounds I are of formula I.A2 wherein Z is Z-247, R³ is CH₃ and Q is Q2, and R and R⁴ are as per any row of Table U below, which compounds are named I.A2-Z247-Q2-U-1 to I.A2-Z247-Q2-U-42.

In another embodiment, compounds I are of formula I.A2 wherein Z is Z-247, R³ is CH₃ and Q is Q3, and R and R⁴ are as per any row of Table U below, which compounds are named I.A2-Z247-Q3-U-1 to I.A2-Z247-Q3-U-42.

In another embodiment, compounds I are of formula I.A2 wherein Z is Z-247, R³ is CH₃ and Q is Q4, and R and R⁴ are as per any row of Table U below, which compounds are named I.A2-Z247-Q4-U-1 to I.A2-Z247-Q4-U-42.

In another embodiment, compounds I are of formula I.A1 wherein Z is Z-248, R³ is CH₃ and Q is Q1, and R and R⁴ are as per any row of Table U below, which compounds are named I.A1-Z248-Q1-U-1 to I.A1-Z248-Q1-U-42.

In another embodiment, compounds I are of formula I.A1 wherein Z is Z-248, R³ is CH₃ and Q is Q2, and R and R⁴ are as per any row of Table U below, which compounds are named I.A1-Z248-Q2-U-1 to I.A1-Z248-Q2-U-42.

In another embodiment, compounds I are of formula I.A1 wherein Z is Z-248, R³ is CH₃ and Q is Q3, and R and R⁴ are as per any row of Table U below, which compounds are named I.A1-Z248-Q3-U-1 to I.A1-Z248-Q3-U-42.

In another embodiment, compounds I are of formula I.A1 wherein Z is Z-248, R³ is CH₃ and Q is Q4, and R and R⁴ are as per any row of Table U below, which compounds are named I.A1-Z248-Q4-U-1 to I.A1-Z248-Q4-U-42.

In another embodiment, compounds I are of formula I.A2 wherein Z is Z-248, R³ is CH₃ and Q is Q1, and R and R⁴ are as per any row of Table U below, which compounds are named I.A2-Z248-Q1-U-1 to I.A2-Z248-Q1-U-42.

In another embodiment, compounds I are of formula I.A2 wherein Z is Z-248, R³ is CH₃ and Q is Q2, and R and R⁴ are as per any row of Table U below, which compounds are named I.A2-Z248-Q2-U-1 to I.A2-Z248-Q2-U-42.

In another embodiment, compounds I are of formula I.A2 wherein Z is Z-248, R³ is CH₃ and Q is Q3, and R and R⁴ are as per any row of Table U below, which compounds are named I.A2-Z248-Q3-U-1 to I.A2-Z248-Q3-U-42.

In another embodiment, compounds I are of formula I.A2 wherein Z is Z-248, R³ is CH₃ and Q is Q4, and R and R⁴ are as per any row of Table U below, which compounds are named I.A2-Z248-Q4-U-1 to I.A2-Z248-Q4-U-42.

In another embodiment, compounds I are of formula I.A1 wherein Z is Z-249, R³ is CH₃ and Q is Q1, and R and R⁴ are as per any row of Table U below, which compounds are named I.A1-Z249-Q1-U-1 to I.A1-Z249-Q1-U-42.

In another embodiment, compounds I are of formula I.A1 wherein Z is Z-249, R³ is CH₃ and Q is Q2, and R and R⁴ are as per any row of Table U below, which compounds are named I.A1-Z249-Q2-U-1 to I.A1-Z249-Q2-U-42.

In another embodiment, compounds I are of formula I.A1 wherein Z is Z-249, R³ is CH₃ and Q is Q3, and R and R⁴ are as per any row of Table U below, which compounds are named I.A1-Z249-Q3-U-1 to I.A1-Z249-Q3-U-42.

In another embodiment, compounds I are of formula I.A1 wherein Z is Z-249, R³ is CH₃ and Q is Q4, and R and R⁴ are as per any row of Table U below, which compounds are named I.A1-Z249-Q4-U-1 to I.A1-Z249-Q4-U-42.

In another embodiment, compounds I are of formula I.A2 wherein Z is Z-249, R³ is CH₃ and Q is Q1, and R and R⁴ are as per any row of Table U below, which compounds are named I.A2-Z249-Q1-U-1 to I.A2-Z249-Q1-U-42.

In another embodiment, compounds I are of formula I.A2 wherein Z is Z-249, R³ is CH₃ and Q is Q2, and R and R⁴ are as per any row of Table U below, which compounds are named I.A2-Z249-Q2-U-1 to I.A2-Z249-Q2-U-42.

In another embodiment, compounds I are of formula I.A2 wherein Z is Z-249, R³ is CH₃ and Q is Q3, and R and R⁴ are as per any row of Table U below, which compounds are named I.A2-Z249-Q3-U-1 to I.A2-Z249-Q3-U-42.

In another embodiment, compounds I are of formula I.A2 wherein Z is Z-249, R³ is CH₃ and Q is Q4, and R and R⁴ are as per any row of Table U below, which compounds are named I.A2-Z249-Q4-U-1 to I.A2-Z249-Q4-U-42.

In another embodiment, compounds I are of formula I.A1 wherein Z is Z-250, R³ is CH₃ and Q is Q1, and R and R⁴ are as per any row of Table U below, which compounds are named I.A1-Z250-Q1-U-1 to I.A1-Z250-Q1-U-42.

In another embodiment, compounds I are of formula I.A1 wherein Z is Z-250, R³ is CH₃ and Q is Q2, and R and R⁴ are as per any row of Table U below, which compounds are named I.A1-Z250-Q2-U-1 to I.A1-Z250-Q2-U-42.

In another embodiment, compounds I are of formula I.A1 wherein Z is Z-250, R³ is CH₃ and Q is Q3, and R and R⁴ are as per any row of Table U below, which compounds are named I.A1-Z250-Q3-U-1 to I.A1-Z250-Q3-U-42.

In another embodiment, compounds I are of formula I.A1 wherein Z is Z-250, R³ is CH₃ and Q is Q4, and R and R⁴ are as per any row of Table U below, which compounds are named I.A1-Z250-Q4-U-1 to I.A1-Z250-Q4-U-42.

In another embodiment, compounds I are of formula I.A2 wherein Z is Z-250, R³ is CH₃ and Q is Q1, and R and R⁴ are as per any row of Table U below, which compounds are named I.A2-Z250-Q1-U-1 to I.A2-Z250-Q1-U-42.

In another embodiment, compounds I are of formula I.A2 wherein Z is Z-250, R³ is CH₃ and Q is Q2, and R and R⁴ are as per any row of Table U below, which compounds are named I.A2-Z250-Q2-U-1 to I.A2-Z250-Q2-U-42.

In another embodiment, compounds I are of formula I.A2 wherein Z is Z-250, R³ is CH₃ and Q is Q3, and R and R⁴ are as per any row of Table U below, which compounds are named I.A2-Z250-Q3-U-1 to I.A2-Z250-Q3-U-42.

In another embodiment, compounds I are of formula I.A2 wherein Z is Z-250, R³ is CH₃ and Q is Q4, and R and R⁴ are as per any row of Table U below, which compounds are named I.A2-Z250-Q4-U-1 to I.A2-Z250-Q4-U-42.

In another embodiment, compounds I are of formula I.A1 wherein Z is Z-251, R³ is CH₃ and Q is Q1, and R and R⁴ are as per any row of Table U below, which compounds are named I.A1-Z251-Q1-U-1 to I.A1-Z251-Q1-U-42.

In another embodiment, compounds I are of formula I.A1 wherein Z is Z-251, R³ is CH₃ and Q is Q2, and R and R⁴ are as per any row of Table U below, which compounds are named I.A1-Z251-Q2-U-1 to I.A1-Z251-Q2-U-42.

In another embodiment, compounds I are of formula I.A1 wherein Z is Z-251, R³ is CH₃ and Q is Q3, and R and R⁴ are as per any row of Table U below, which compounds are named I.A1-Z251-Q3-U-1 to I.A1-Z251-Q3-U-42.

In another embodiment, compounds I are of formula I.A1 wherein Z is Z-251, R³ is CH₃ and Q is Q4, and R and R⁴ are as per any row of Table U below, which compounds are named I.A1-Z251-Q4-U-1 to I.A1-Z251-Q4-U-42.

In another embodiment, compounds I are of formula I.A2 wherein Z is Z-251, R³ is CH₃ and Q is Q1, and R and R⁴ are as per any row of Table U below, which compounds are named I.A2-Z251-Q1-U-1 to I.A2-Z251-Q1-U-42.

In another embodiment, compounds I are of formula I.A2 wherein Z is Z-251, R³ is CH₃ and Q is Q2, and R and R⁴ are as per any row of Table U below, which compounds are named I.A2-Z251-Q2-U-1 to I.A2-Z251-Q2-U-42.

In another embodiment, compounds I are of formula I.A2 wherein Z is Z-251, R³ is CH₃ and Q is Q3, and R and R⁴ are as per any row of Table U below, which compounds are named I.A2-Z251-Q3-U-1 to I.A2-Z251-Q3-U-42.

In another embodiment, compounds I are of formula I.A2 wherein Z is Z-251, R³ is CH₃ and Q is Q4, and R and R⁴ are as per any row of Table U below, which compounds are named I.A2-Z251-Q4-U-1 to I.A2-Z251-Q4-U-42.

In another embodiment, compounds I are of formula I.A1 wherein Z is Z-252, R³ is CH₃ and Q is Q1, and R and R⁴ are as per any row of Table U below, which compounds are named I.A1-Z252-Q1-U-1 to I.A1-Z252-Q1-U-42.

In another embodiment, compounds I are of formula I.A1 wherein Z is Z-252, R³ is CH₃ and Q is Q2, and R and R⁴ are as per any row of Table U below, which compounds are named I.A1-Z252-Q2-U-1 to I.A1-Z252-Q2-U-42.

In another embodiment, compounds I are of formula I.A1 wherein Z is Z-252, R³ is CH₃ and Q is Q3, and R and R⁴ are as per any row of Table U below, which compounds are named I.A1-Z252-Q3-U-1 to I.A1-Z252-Q3-U-42.

In another embodiment, compounds I are of formula I.A1 wherein Z is Z-252, R³ is CH₃ and Q is Q4, and R and R⁴ are as per any row of Table U below, which compounds are named I.A1-Z252-Q4-U-1 to I.A1-Z252-Q4-U-42.

In another embodiment, compounds I are of formula I.A2 wherein Z is Z-252, R³ is CH₃ and Q is Q1, and R and R⁴ are as per any row of Table U below, which compounds are named I.A2-Z252-Q1-U-1 to I.A2-Z252-Q1-U-42.

In another embodiment, compounds I are of formula I.A2 wherein Z is Z-252, R³ is CH₃ and Q is Q2, and R and R⁴ are as per any row of Table U below, which compounds are named I.A2-Z252-Q2-U-1 to I.A2-Z252-Q2-U-42.

In another embodiment, compounds I are of formula I.A2 wherein Z is Z-252, R³ is CH₃ and Q is Q3, and R and R⁴ are as per any row of Table U below, which compounds are named I.A2-Z252-Q3-U-1 to I.A2-Z252-Q3-U-42.

In another embodiment, compounds I are of formula I.A2 wherein Z is Z-252, R³ is CH₃ and Q is Q4, and R and R⁴ are as per any row of Table U below, which compounds are named I.A2-Z252-Q4-U-1 to I.A2-Z252-Q4-U-42.

In another embodiment, compounds I are of formula I.A1 wherein Z is Z-253, R³ is CH₃ and Q is Q1, and R and R⁴ are as per any row of Table U below, which compounds are named I.A1-Z253-Q1-U-1 to I.A1-Z253-Q1-U-42.

In another embodiment, compounds I are of formula I.A1 wherein Z is Z-253, R³ is CH₃ and Q is Q2, and R and R⁴ are as per any row of Table U below, which compounds are named I.A1-Z253-Q2-U-1 to I.A1-Z253-Q2-U-42.

In another embodiment, compounds I are of formula I.A1 wherein Z is Z-253, R³ is CH₃ and Q is Q3, and R and R⁴ are as per any row of Table U below, which compounds are named I.A1-Z253-Q3-U-1 to I.A1-Z253-Q3-U-42.

In another embodiment, compounds I are of formula I.A1 wherein Z is Z-253, R³ is CH₃ and Q is Q4, and R and R⁴ are as per any row of Table U below, which compounds are named I.A1-Z253-Q4-U-1 to I.A1-Z253-Q4-U-42.

In another embodiment, compounds I are of formula I.A2 wherein Z is Z-253, R³ is CH₃ and Q is Q1, and R and R⁴ are as per any row of Table U below, which compounds are named I.A2-Z253-Q1-U-1 to I.A2-Z253-Q1-U-42.

In another embodiment, compounds I are of formula I.A2 wherein Z is Z-253, R³ is CH₃ and Q is Q2, and R and R⁴ are as per any row of Table U below, which compounds are named I.A2-Z253-Q2-U-1 to I.A2-Z253-Q2-U-42.

In another embodiment, compounds I are of formula I.A2 wherein Z is Z-253, R³ is CH₃ and Q is Q3, and R and R⁴ are as per any row of Table U below, which compounds are named I.A2-Z253-Q3-U-1 to I.A2-Z253-Q3-U-42.

In another embodiment, compounds I are of formula I.A2 wherein Z is Z-253, R³ is CH₃ and Q is Q4, and R and R⁴ are as per any row of Table U below, which compounds are named I.A2-Z253-Q4-U-1 to I.A2-Z253-Q4-U-42.

In another embodiment, compounds I are of formula I.A1 wherein Z is Z-254, R³ is CH₃ and Q is Q1, and R and R⁴ are as per any row of Table U below, which compounds are named I.A1-Z254-Q1-U-1 to I.A1-Z254-Q1-U-42.

In another embodiment, compounds I are of formula I.A1 wherein Z is Z-254, R³ is CH₃ and Q is Q2, and R and R⁴ are as per any row of Table U below, which compounds are named I.A1-Z254-Q2-U-1 to I.A1-Z254-Q2-U-42.

In another embodiment, compounds I are of formula I.A1 wherein Z is Z-254, R³ is CH₃ and Q is Q3, and R and R⁴ are as per any row of Table U below, which compounds are named I.A1-Z254-Q3-U-1 to I.A1-Z254-Q3-U-42.

In another embodiment, compounds I are of formula I.A1 wherein Z is Z-254, R³ is CH₃ and Q is Q4, and R and R⁴ are as per any row of Table U below, which compounds are named I.A1-Z254-Q4-U-1 to I.A1-Z254-Q4-U-42.

In another embodiment, compounds I are of formula I.A2 wherein Z is Z-254, R³ is CH₃ and Q is Q1, and R and R⁴ are as per any row of Table U below, which compounds are named I.A2-Z254-Q1-U-1 to I.A2-Z254-Q1-U-42.

In another embodiment, compounds I are of formula I.A2 wherein Z is Z-254, R³ is CH₃ and Q is Q2, and R and R⁴ are as per any row of Table U below, which compounds are named I.A2-Z254-Q2-U-1 to I.A2-Z254-Q2-U-42.

In another embodiment, compounds I are of formula I.A2 wherein Z is Z-254, R³ is CH₃ and Q is Q3, and R and R⁴ are as per any row of Table U below, which compounds are named I.A2-Z254-Q3-U-1 to I.A2-Z254-Q3-U-42.

In another embodiment, compounds I are of formula I.A2 wherein Z is Z-254, R³ is CH₃ and Q is Q4, and R and R⁴ are as per any row of Table U below, which compounds are named I.A2-Z254-Q4-U-1 to I.A2-Z254-Q4-U-42.

In another embodiment, compounds I are of formula I.A1 wherein Z is Z-255, R³ is CH₃ and Q is Q1, and R and R⁴ are as per any row of Table U below, which compounds are named I.A1-Z255-Q1-U-1 to I.A1-Z255-Q1-U-42.

In another embodiment, compounds I are of formula I.A1 wherein Z is Z-255, R³ is CH₃ and Q is Q2, and R and R⁴ are as per any row of Table U below, which compounds are named I.A1-Z255-Q2-U-1 to I.A1-Z255-Q2-U-42.

In another embodiment, compounds I are of formula I.A1 wherein Z is Z-255, R³ is CH₃ and Q is Q3, and R and R⁴ are as per any row of Table U below, which compounds are named I.A1-Z255-Q3-U-1 to I.A1-Z255-Q3-U-42.

In another embodiment, compounds I are of formula I.A1 wherein Z is Z-255, R³ is CH₃ and Q is Q4, and R and R⁴ are as per any row of Table U below, which compounds are named I.A1-Z255-Q4-U-1 to I.A1-Z255-Q4-U-42.

In another embodiment, compounds I are of formula I.A2 wherein Z is Z-255, R³ is CH₃ and Q is Q1, and R and R⁴ are as per any row of Table U below, which compounds are named I.A2-Z255-Q1-U-1 to I.A2-Z255-Q1-U-42.

In another embodiment, compounds I are of formula I.A2 wherein Z is Z-255, R³ is CH₃ and Q is Q2, and R and R⁴ are as per any row of Table U below, which compounds are named I.A2-Z255-Q2-U-1 to I.A2-Z255-Q2-U-42.

In another embodiment, compounds I are of formula I.A2 wherein Z is Z-255, R³ is CH₃ and Q is Q3, and R and R⁴ are as per any row of Table U below, which compounds are named I.A2-Z255-Q3-U-1 to I.A2-Z255-Q3-U-42.

In another embodiment, compounds I are of formula I.A2 wherein Z is Z-255, R³ is CH₃ and Q is Q4, and R and R⁴ are as per any row of Table U below, which compounds are named I.A2-Z255-Q4-U-1 to I.A2-Z255-Q4-U-42.

In another embodiment, compounds I are of formula I.A1 wherein Z is Z-256, R³ is CH₃ and Q is Q1, and R and R⁴ are as per any row of Table U below, which compounds are named I.A1-Z256-Q1-U-1 to I.A1-Z256-Q1-U-42.

In another embodiment, compounds I are of formula I.A1 wherein Z is Z-256, R³ is CH₃ and Q is Q2, and R and R⁴ are as per any row of Table U below, which compounds are named I.A1-Z256-Q2-U-1 to I.A1-Z256-Q2-U-42.

In another embodiment, compounds I are of formula I.A1 wherein Z is Z-256, R³ is CH₃ and Q is Q3, and R and R⁴ are as per any row of Table U below, which compounds are named I.A1-Z256-Q3-U-1 to I.A1-Z256-Q3-U-42.

In another embodiment, compounds I are of formula I.A1 wherein Z is Z-256, R³ is CH₃ and Q is Q4, and R and R⁴ are as per any row of Table U below, which compounds are named I.A1-Z256-Q4-U-1 to I.A1-Z256-Q4-U-42.

In another embodiment, compounds I are of formula I.A2 wherein Z is Z-256, R³ is CH₃ and Q is Q1, and R and R⁴ are as per any row of Table U below, which compounds are named I.A2-Z256-Q1-U-1 to I.A2-Z256-Q1-U-42.

In another embodiment, compounds I are of formula I.A2 wherein Z is Z-256, R³ is CH₃ and Q is Q2, and R and R⁴ are as per any row of Table U below, which compounds are named I.A2-Z256-Q2-U-1 to I.A2-Z256-Q2-U-42.

In another embodiment, compounds I are of formula I.A2 wherein Z is Z-256, R³ is CH₃ and Q is Q3, and R and R⁴ are as per any row of Table U below, which compounds are named I.A2-Z256-Q3-U-1 to I.A2-Z256-Q3-U-42.

In another embodiment, compounds I are of formula I.A2 wherein Z is Z-256, R³ is CH₃ and Q is Q4, and R and R⁴ are as per any row of Table U below, which compounds are named I.A2-Z256-Q4-U-1 to I.A2-Z256-Q4-U-42.

In another embodiment, compounds I are of formula I.A1 wherein Z is Z-257, R³ is CH₃ and Q is Q1, and R and R⁴ are as per any row of Table U below, which compounds are named I.A1-Z257-Q1-U-1 to I.A1-Z257-Q1-U-42.

In another embodiment, compounds I are of formula I.A1 wherein Z is Z-257, R³ is CH₃ and Q is Q2, and R and R⁴ are as per any row of Table U below, which compounds are named I.A1-Z257-Q2-U-1 to I.A1-Z257-Q2-U-42.

In another embodiment, compounds I are of formula I.A1 wherein Z is Z-257, R³ is CH₃ and Q is Q3, and R and R⁴ are as per any row of Table U below, which compounds are named I.A1-Z257-Q3-U-1 to I.A1-Z257-Q3-U-42.

In another embodiment, compounds I are of formula I.A1 wherein Z is Z-257, R³ is CH₃ and Q is Q4, and R and R⁴ are as per any row of Table U below, which compounds are named I.A1-Z257-Q4-U-1 to I.A1-Z257-Q4-U-42.

In another embodiment, compounds I are of formula I.A2 wherein Z is Z-257, R³ is CH₃ and Q is Q1, and R and R⁴ are as per any row of Table U below, which compounds are named I.A2-Z257-Q1-U-1 to I.A2-Z257-Q1-U-42.

In another embodiment, compounds I are of formula I.A2 wherein Z is Z-257, R³ is CH₃ and Q is Q2, and R and R⁴ are as per any row of Table U below, which compounds are named I.A2-Z257-Q2-U-1 to I.A2-Z257-Q2-U-42.

In another embodiment, compounds I are of formula I.A2 wherein Z is Z-257, R³ is CH₃ and Q is Q3, and R and R⁴ are as per any row of Table U below, which compounds are named I.A2-Z257-Q3-U-1 to I.A2-Z257-Q3-U-42.

In another embodiment, compounds I are of formula I.A2 wherein Z is Z-257, R³ is CH₃ and Q is Q4, and R and R⁴ are as per any row of Table U below, which compounds are named I.A2-Z257-Q4-U-1 to I.A2-Z257-Q4-U-42.

In another embodiment, compounds I are of formula I.A1 wherein Z is Z-258, R³ is CH₃ and Q is Q1, and R and R⁴ are as per any row of Table U below, which compounds are named I.A1-Z258-Q1-U-1 to I.A1-Z258-Q1-U-42.

In another embodiment, compounds I are of formula I.A1 wherein Z is Z-258, R³ is CH₃ and Q is Q2, and R and R⁴ are as per any row of Table U below, which compounds are named I.A1-Z258-Q2-U-1 to I.A1-Z258-Q2-U-42.

In another embodiment, compounds I are of formula I.A1 wherein Z is Z-258, R³ is CH₃ and Q is Q3, and R and R⁴ are as per any row of Table U below, which compounds are named I.A1-Z258-Q3-U-1 to I.A1-Z258-Q3-U-42.

In another embodiment, compounds I are of formula I.A1 wherein Z is Z-258, R³ is CH₃ and Q is Q4, and R and R⁴ are as per any row of Table U below, which compounds are named I.A1-Z258-Q4-U-1 to I.A1-Z258-Q4-U-42.

In another embodiment, compounds I are of formula I.A2 wherein Z is Z-258, R³ is CH₃ and Q is Q1, and R and R⁴ are as per any row of Table U below, which compounds are named I.A2-Z258-Q1-U-1 to I.A2-Z258-Q1-U-42.

In another embodiment, compounds I are of formula I.A2 wherein Z is Z-258, R³ is CH₃ and Q is Q2, and R and R⁴ are as per any row of Table U below, which compounds are named I.A2-Z258-Q2-U-1 to I.A2-Z258-Q2-U-42.

In another embodiment, compounds I are of formula I.A2 wherein Z is Z-258, R³ is CH₃ and Q is Q3, and R and R⁴ are as per any row of Table U below, which compounds are named I.A2-Z258-Q3-U-1 to I.A2-Z258-Q3-U-42.

In another embodiment, compounds I are of formula I.A2 wherein Z is Z-258, R³ is CH₃ and Q is Q4, and R and R⁴ are as per any row of Table U below, which compounds are named I.A2-Z258-Q4-U-1 to I.A2-Z258-Q4-U-42.

In another embodiment, compounds I are of formula I.A1 wherein Z is Z-259, R³ is CH₃ and Q is Q1, and R and R⁴ are as per any row of Table U below, which compounds are named I.A1-Z259-Q1-U-1 to I.A1-Z259-Q1-U-42.

In another embodiment, compounds I are of formula I.A1 wherein Z is Z-259, R³ is CH₃ and Q is Q2, and R and R⁴ are as per any row of Table U below, which compounds are named I.A1-Z259-Q2-U-1 to I.A1-Z259-Q2-U-42.

In another embodiment, compounds I are of formula I.A1 wherein Z is Z-259, R³ is CH₃ and Q is Q3, and R and R⁴ are as per any row of Table U below, which compounds are named I.A1-Z259-Q3-U-1 to I.A1-Z259-Q3-U-42.

In another embodiment, compounds I are of formula I.A1 wherein Z is Z-259, R³ is CH₃ and Q is Q4, and R and R⁴ are as per any row of Table U below, which compounds are named I.A1-Z259-Q4-U-1 to I.A1-Z259-Q4-U-42.

In another embodiment, compounds I are of formula I.A2 wherein Z is Z-259, R³ is CH₃ and Q is Q1, and R and R⁴ are as per any row of Table U below, which compounds are named I.A2-Z259-Q1-U-1 to I.A2-Z259-Q1-U-42.

In another embodiment, compounds I are of formula I.A2 wherein Z is Z-259, R³ is CH₃ and Q is Q2, and R and R⁴ are as per any row of Table U below, which compounds are named I.A2-Z259-Q2-U-1 to I.A2-Z259-Q2-U-42.

In another embodiment, compounds I are of formula I.A2 wherein Z is Z-259, R³ is CH₃ and Q is Q3, and R and R⁴ are as per any row of Table U below, which compounds are named I.A2-Z259-Q3-U-1 to I.A2-Z259-Q3-U-42.

In another embodiment, compounds I are of formula I.A2 wherein Z is Z-259, R³ is CH₃ and Q is Q4, and R and R⁴ are as per any row of Table U below, which compounds are named I.A2-Z259-Q4-U-1 to I.A2-Z259-Q4-U-42.

In another embodiment, compounds I are of formula I.A1 wherein Z is Z-260, R³ is CH₃ and Q is Q1, and R and R⁴ are as per any row of Table U below, which compounds are named I.A1-Z260-Q1-U-1 to I.A1-Z260-Q1-U-42.

In another embodiment, compounds I are of formula I.A1 wherein Z is Z-260, R³ is CH₃ and Q is Q2, and R and R⁴ are as per any row of Table U below, which compounds are named I.A1-Z260-Q2-U-1 to I.A1-Z260-Q2-U-42.

In another embodiment, compounds I are of formula I.A1 wherein Z is Z-260, R³ is CH₃ and Q is Q3, and R and R⁴ are as per any row of Table U below, which compounds are named I.A1-Z260-Q3-U-1 to I.A1-Z260-Q3-U-42.

In another embodiment, compounds I are of formula I.A1 wherein Z is Z-260, R³ is CH₃ and Q is Q4, and R and R⁴ are as per any row of Table U below, which compounds are named I.A1-Z260-Q4-U-1 to I.A1-Z260-Q4-U-42.

In another embodiment, compounds I are of formula I.A2 wherein Z is Z-260, R³ is CH₃ and Q is Q1, and R and R⁴ are as per any row of Table U below, which compounds are named I.A2-Z260-Q1-U-1 to I.A2-Z260-Q1-U-42.

In another embodiment, compounds I are of formula I.A2 wherein Z is Z-260, R³ is CH₃ and Q is Q2, and R and R⁴ are as per any row of Table U below, which compounds are named I.A2-Z260-Q2-U-1 to I.A2-Z260-Q2-U-42.

In another embodiment, compounds I are of formula I.A2 wherein Z is Z-260, R³ is CH₃ and Q is Q3, and R and R⁴ are as per any row of Table U below, which compounds are named I.A2-Z260-Q3-U-1 to I.A2-Z260-Q3-U-42.

In another embodiment, compounds I are of formula I.A2 wherein Z is Z-260, R³ is CH₃ and Q is Q4, and R and R⁴ are as per any row of Table U below, which compounds are named I.A2-Z260-Q4-U-1 to I.A2-Z260-Q4-U-42.

In another embodiment, compounds I are of formula I.A1 wherein Z is Z-261, R³ is CH₃ and Q is Q1, and R and R⁴ are as per any row of Table U below, which compounds are named I.A1-Z261-Q1-U-1 to I.A1-Z261-Q1-U-42.

In another embodiment, compounds I are of formula I.A1 wherein Z is Z-261, R³ is CH₃ and Q is Q2, and R and R⁴ are as per any row of Table U below, which compounds are named I.A1-Z261-Q2-U-1 to I.A1-Z261-Q2-U-42.

In another embodiment, compounds I are of formula I.A1 wherein Z is Z-261, R³ is CH₃ and Q is Q3, and R and R⁴ are as per any row of Table U below, which compounds are named I.A1-Z261-Q3-U-1 to I.A1-Z261-Q3-U-42.

In another embodiment, compounds I are of formula I.A1 wherein Z is Z-261, R³ is CH₃ and Q is Q4, and R and R⁴ are as per any row of Table U below, which compounds are named I.A1-Z261-Q4-U-1 to I.A1-Z261-Q4-U-42.

In another embodiment, compounds I are of formula I.A2 wherein Z is Z-261, R³ is CH₃ and Q is Q1, and R and R⁴ are as per any row of Table U below, which compounds are named I.A2-Z261-Q1-U-1 to I.A2-Z261-Q1-U-42.

In another embodiment, compounds I are of formula I.A2 wherein Z is Z-261, R³ is CH₃ and Q is Q2, and R and R⁴ are as per any row of Table U below, which compounds are named I.A2-Z261-Q2-U-1 to I.A2-Z261-Q2-U-42.

In another embodiment, compounds I are of formula I.A2 wherein Z is Z-261, R³ is CH₃ and Q is Q3, and R and R⁴ are as per any row of Table U below, which compounds are named I.A2-Z261-Q3-U-1 to I.A2-Z261-Q3-U-42.

In another embodiment, compounds I are of formula I.A2 wherein Z is Z-261, R³ is CH₃ and Q is Q4, and R and R⁴ are as per any row of Table U below, which compounds are named I.A2-Z261-Q4-U-1 to I.A2-Z261-Q4-U-42.

In another embodiment, compounds I are of formula I.A1 wherein Z is Z-262, R³ is CH₃ and Q is Q1, and R and R⁴ are as per any row of Table U below, which compounds are named I.A1-Z262-Q1-U-1 to I.A1-Z262-Q1-U-42.

In another embodiment, compounds I are of formula I.A1 wherein Z is Z-262, R³ is CH₃ and Q is Q2, and R and R⁴ are as per any row of Table U below, which compounds are named I.A1-Z262-Q2-U-1 to I.A1-Z262-Q2-U-42.

In another embodiment, compounds I are of formula I.A1 wherein Z is Z-262, R³ is CH₃ and Q is Q3, and R and R⁴ are as per any row of Table U below, which compounds are named I.A1-Z262-Q3-U-1 to I.A1-Z262-Q3-U-42.

In another embodiment, compounds I are of formula I.A1 wherein Z is Z-262, R³ is CH₃ and Q is Q4, and R and R⁴ are as per any row of Table U below, which compounds are named I.A1-Z262-Q4-U-1 to I.A1-Z262-Q4-U-42.

In another embodiment, compounds I are of formula I.A2 wherein Z is Z-262, R³ is CH₃ and Q is Q1, and R and R⁴ are as per any row of Table U below, which compounds are named I.A2-Z262-Q1-U-1 to I.A2-Z262-Q1-U-42.

In another embodiment, compounds I are of formula I.A2 wherein Z is Z-262, R³ is CH₃ and Q is Q2, and R and R⁴ are as per any row of Table U below, which compounds are named I.A2-Z262-Q2-U-1 to I.A2-Z262-Q2-U-42.

In another embodiment, compounds I are of formula I.A2 wherein Z is Z-262, R³ is CH₃ and Q is Q3, and R and R⁴ are as per any row of Table U below, which compounds are named I.A2-Z262-Q3-U-1 to I.A2-Z262-Q3-U-42.

In another embodiment, compounds I are of formula I.A2 wherein Z is Z-262, R³ is CH₃ and Q is Q4, and R and R⁴ are as per any row of Table U below, which compounds are named I.A2-Z262-Q4-U-1 to I.A2-Z262-Q4-U-42.

In another embodiment, compounds I are of formula I.A1 wherein Z is Z-263, R³ is CH₃ and Q is Q1, and R and R⁴ are as per any row of Table U below, which compounds are named I.A1-Z263-Q1-U-1 to I.A1-Z263-Q1-U-42.

In another embodiment, compounds I are of formula I.A1 wherein Z is Z-263, R³ is CH₃ and Q is Q2, and R and R⁴ are as per any row of Table U below, which compounds are named I.A1-Z263-Q2-U-1 to I.A1-Z263-Q2-U-42.

In another embodiment, compounds I are of formula I.A1 wherein Z is Z-263, R³ is CH₃ and Q is Q3, and R and R⁴ are as per any row of Table U below, which compounds are named I.A1-Z263-Q3-U-1 to I.A1-Z263-Q3-U-42.

In another embodiment, compounds I are of formula I.A1 wherein Z is Z-263, R³ is CH₃ and Q is Q4, and R and R⁴ are as per any row of Table U below, which compounds are named I.A1-Z263-Q4-U-1 to I.A1-Z263-Q4-U-42.

In another embodiment, compounds I are of formula I.A2 wherein Z is Z-263, R³ is CH₃ and Q is Q1, and R and R⁴ are as per any row of Table U below, which compounds are named I.A2-Z263-Q1-U-1 to I.A2-Z263-Q1-U-42.

In another embodiment, compounds I are of formula I.A2 wherein Z is Z-263, R³ is CH₃ and Q is Q2, and R and R⁴ are as per any row of Table U below, which compounds are named I.A2-Z263-Q2-U-1 to I.A2-Z263-Q2-U-42.

In another embodiment, compounds I are of formula I.A2 wherein Z is Z-263, R³ is CH₃ and Q is Q3, and R and R⁴ are as per any row of Table U below, which compounds are named I.A2-Z263-Q3-U-1 to I.A2-Z263-Q3-U-42.

In another embodiment, compounds I are of formula I.A2 wherein Z is Z-263, R³ is CH₃ and Q is Q4, and R and R⁴ are as per any row of Table U below, which compounds are named I.A2-Z263-Q4-U-1 to I.A2-Z263-Q4-U-42.

In another embodiment, compounds I are of formula I.A1 wherein Z is Z-264, R³ is CH₃ and Q is Q1, and R and R⁴ are as per any row of Table U below, which compounds are named I.A1-Z264-Q1-U-1 to I.A1-Z264-Q1-U-42.

In another embodiment, compounds I are of formula I.A1 wherein Z is Z-264, R³ is CH₃ and Q is Q2, and R and R⁴ are as per any row of Table U below, which compounds are named I.A1-Z264-Q2-U-1 to I.A1-Z264-Q2-U-42.

In another embodiment, compounds I are of formula I.A1 wherein Z is Z-264, R³ is CH₃ and Q is Q3, and R and R⁴ are as per any row of Table U below, which compounds are named I.A1-Z264-Q3-U-1 to I.A1-Z264-Q3-U-42.

In another embodiment, compounds I are of formula I.A1 wherein Z is Z-264, R³ is CH₃ and Q is Q4, and R and R⁴ are as per any row of Table U below, which compounds are named I.A1-Z264-Q4-U-1 to I.A1-Z264-Q4-U-42.

In another embodiment, compounds I are of formula I.A2 wherein Z is Z-264, R³ is CH₃ and Q is Q1, and R and R⁴ are as per any row of Table U below, which compounds are named I.A2-Z264-Q1-U-1 to I.A2-Z264-Q1-U-42.

In another embodiment, compounds I are of formula I.A2 wherein Z is Z-264, R³ is CH₃ and Q is Q2, and R and R⁴ are as per any row of Table U below, which compounds are named I.A2-Z264-Q2-U-1 to I.A2-Z264-Q2-U-42.

In another embodiment, compounds I are of formula I.A2 wherein Z is Z-264, R³ is CH₃ and Q is Q3, and R and R⁴ are as per any row of Table U below, which compounds are named I.A2-Z264-Q3-U-1 to I.A2-Z264-Q3-U-42.

In another embodiment, compounds I are of formula I.A2 wherein Z is Z-264, R³ is CH₃ and Q is Q4, and R and R⁴ are as per any row of Table U below, which compounds are named I.A2-Z264-Q4-U-1 to I.A2-Z264-Q4-U-42.

In another embodiment, compounds I are of formula I.A1 wherein Z is Z-265, R³ is CH₃ and Q is Q1, and R and R⁴ are as per any row of Table U below, which compounds are named I.A1-Z265-Q1-U-1 to I.A1-Z265-Q1-U-42.

In another embodiment, compounds I are of formula I.A1 wherein Z is Z-265, R³ is CH₃ and Q is Q2, and R and R⁴ are as per any row of Table U below, which compounds are named I.A1-Z265-Q2-U-1 to I.A1-Z265-Q2-U-42.

In another embodiment, compounds I are of formula I.A1 wherein Z is Z-265, R³ is CH₃ and Q is Q3, and R and R⁴ are as per any row of Table U below, which compounds are named I.A1-Z265-Q3-U-1 to I.A1-Z265-Q3-U-42.

In another embodiment, compounds I are of formula I.A1 wherein Z is Z-265, R³ is CH₃ and Q is Q4, and R and R⁴ are as per any row of Table U below, which compounds are named I.A1-Z265-Q4-U-1 to I.A1-Z265-Q4-U-42.

In another embodiment, compounds I are of formula I.A2 wherein Z is Z-265, R³ is CH₃ and Q is Q1, and R and R⁴ are as per any row of Table U below, which compounds are named I.A2-Z265-Q1-U-1 to I.A2-Z265-Q1-U-42.

In another embodiment, compounds I are of formula I.A2 wherein Z is Z-265, R³ is CH₃ and Q is Q2, and R and R⁴ are as per any row of Table U below, which compounds are named I.A2-Z265-Q2-U-1 to I.A2-Z265-Q2-U-42.

In another embodiment, compounds I are of formula I.A2 wherein Z is Z-265, R³ is CH₃ and Q is Q3, and R and R⁴ are as per any row of Table U below, which compounds are named I.A2-Z265-Q3-U-1 to I.A2-Z265-Q3-U-42.

In another embodiment, compounds I are of formula I.A2 wherein Z is Z-265, R³ is CH₃ and Q is Q4, and R and R⁴ are as per any row of Table U below, which compounds are named I.A2-Z265-Q4-U-1 to I.A2-Z265-Q4-U-42.

In another embodiment, compounds I are of formula I.A1 wherein Z is Z-266, R³ is CH₃ and Q is Q1, and R and R⁴ are as per any row of Table U below, which compounds are named I.A1-Z266-Q1-U-1 to I.A1-Z266-Q1-U-42.

In another embodiment, compounds I are of formula I.A1 wherein Z is Z-266, R³ is CH₃ and Q is Q2, and R and R⁴ are as per any row of Table U below, which compounds are named I.A1-Z266-Q2-U-1 to I.A1-Z266-Q2-U-42.

In another embodiment, compounds I are of formula I.A1 wherein Z is Z-266, R³ is CH₃ and Q is Q3, and R and R⁴ are as per any row of Table U below, which compounds are named I.A1-Z266-Q3-U-1 to I.A1-Z266-Q3-U-42.

In another embodiment, compounds I are of formula I.A1 wherein Z is Z-266, R³ is CH₃ and Q is Q4, and R and R⁴ are as per any row of Table U below, which compounds are named I.A1-Z266-Q4-U-1 to I.A1-Z266-Q4-U-42.

In another embodiment, compounds I are of formula I.A2 wherein Z is Z-266, R³ is CH₃ and Q is Q1, and R and R⁴ are as per any row of Table U below, which compounds are named I.A2-Z266-Q1-U-1 to I.A2-Z266-Q1-U-42.

In another embodiment, compounds I are of formula I.A2 wherein Z is Z-266, R³ is CH₃ and Q is Q2, and R and R⁴ are as per any row of Table U below, which compounds are named I.A2-Z266-Q2-U-1 to I.A2-Z266-Q2-U-42.

In another embodiment, compounds I are of formula I.A2 wherein Z is Z-266, R³ is CH₃ and Q is Q3, and R and R⁴ are as per any row of Table U below, which compounds are named I.A2-Z266-Q3-U-1 to I.A2-Z266-Q3-U-42.

In another embodiment, compounds I are of formula I.A2 wherein Z is Z-266, R³ is CH₃ and Q is Q4, and R and R⁴ are as per any row of Table U below, which compounds are named I.A2-Z266-Q4-U-1 to I.A2-Z266-Q4-U-42.

In another embodiment, compounds I are of formula I.A1 wherein Z is Z-267, R³ is CH₃ and Q is Q1, and R and R⁴ are as per any row of Table U below, which compounds are named I.A1-Z267-Q1-U-1 to I.A1-Z267-Q1-U-42.

In another embodiment, compounds I are of formula I.A1 wherein Z is Z-267, R³ is CH₃ and Q is Q2, and R and R⁴ are as per any row of Table U below, which compounds are named I.A1-Z267-Q2-U-1 to I.A1-Z267-Q2-U-42.

In another embodiment, compounds I are of formula I.A1 wherein Z is Z-267, R³ is CH₃ and Q is Q3, and R and R⁴ are as per any row of Table U below, which compounds are named I.A1-Z267-Q3-U-1 to I.A1-Z267-Q3-U-42.

In another embodiment, compounds I are of formula I.A1 wherein Z is Z-267, R³ is CH₃ and Q is Q4, and R and R⁴ are as per any row of Table U below, which compounds are named I.A1-Z267-Q4-U-1 to I.A1-Z267-Q4-U-42.

In another embodiment, compounds I are of formula I.A2 wherein Z is Z-267, R³ is CH₃ and Q is Q1, and R and R⁴ are as per any row of Table U below, which compounds are named I.A2-Z267-Q1-U-1 to I.A2-Z267-Q1-U-42.

In another embodiment, compounds I are of formula I.A2 wherein Z is Z-267, R³ is CH₃ and Q is Q2, and R and R⁴ are as per any row of Table U below, which compounds are named I.A2-Z267-Q2-U-1 to I.A2-Z267-Q2-U-42.

In another embodiment, compounds I are of formula I.A2 wherein Z is Z-267, R³ is CH₃ and Q is Q3, and R and R⁴ are as per any row of Table U below, which compounds are named I.A2-Z267-Q3-U-1 to I.A2-Z267-Q3-U-42.

In another embodiment, compounds I are of formula I.A2 wherein Z is Z-267, R³ is CH₃ and Q is Q4, and R and R⁴ are as per any row of Table U below, which compounds are named I.A2-Z267-Q4-U-1 to I.A2-Z267-Q4-U-42.

In another embodiment, compounds I are of formula I.A1 wherein Z is Z-268, R³ is CH₃ and Q is Q1, and R and R⁴ are as per any row of Table U below, which compounds are named I.A1-Z268-Q1-U-1 to I.A1-Z268-Q1-U-42.

In another embodiment, compounds I are of formula I.A1 wherein Z is Z-268, R³ is CH₃ and Q is Q2, and R and R⁴ are as per any row of Table U below, which compounds are named I.A1-Z268-Q2-U-1 to I.A1-Z268-Q2-U-42.

In another embodiment, compounds I are of formula I.A1 wherein Z is Z-268, R³ is CH₃ and Q is Q3, and R and R⁴ are as per any row of Table U below, which compounds are named I.A1-Z268-Q3-U-1 to I.A1-Z268-Q3-U-42.

In another embodiment, compounds I are of formula I.A1 wherein Z is Z-268, R³ is CH₃ and Q is Q4, and R and R⁴ are as per any row of Table U below, which compounds are named I.A1-Z268-Q4-U-1 to I.A1-Z268-Q4-U-42.

In another embodiment, compounds I are of formula I.A2 wherein Z is Z-268, R³ is CH₃ and Q is Q1, and R and R⁴ are as per any row of Table U below, which compounds are named I.A2-Z268-Q1-U-1 to I.A2-Z268-Q1-U-42.

In another embodiment, compounds I are of formula I.A2 wherein Z is Z-268, R³ is CH₃ and Q is Q2, and R and R⁴ are as per any row of Table U below, which compounds are named I.A2-Z268-Q2-U-1 to I.A2-Z268-Q2-U-42.

In another embodiment, compounds I are of formula I.A2 wherein Z is Z-268, R³ is CH₃ and Q is Q3, and R and R⁴ are as per any row of Table U below, which compounds are named I.A2-Z268-Q3-U-1 to I.A2-Z268-Q3-U-42.

In another embodiment, compounds I are of formula I.A2 wherein Z is Z-268, R³ is CH₃ and Q is Q4, and R and R⁴ are as per any row of Table U below, which compounds are named I.A2-Z268-Q4-U-1 to I.A2-Z268-Q4-U-42.

In another embodiment, compounds I are of formula I.A1 wherein Z is Z-269, R³ is CH₃ and Q is Q1, and R and R⁴ are as per any row of Table U below, which compounds are named I.A1-Z269-Q1-U-1 to I.A1-Z269-Q1-U-42.

In another embodiment, compounds I are of formula I.A1 wherein Z is Z-269, R³ is CH₃ and Q is Q2, and R and R⁴ are as per any row of Table U below, which compounds are named I.A1-Z269-Q2-U-1 to I.A1-Z269-Q2-U-42.

In another embodiment, compounds I are of formula I.A1 wherein Z is Z-269, R³ is CH₃ and Q is Q3, and R and R⁴ are as per any row of Table U below, which compounds are named I.A1-Z269-Q3-U-1 to I.A1-Z269-Q3-U-42.

In another embodiment, compounds I are of formula I.A1 wherein Z is Z-269, R³ is CH₃ and Q is Q4, and R and R⁴ are as per any row of Table U below, which compounds are named I.A1-Z269-Q4-U-1 to I.A1-Z269-Q4-U-42.

In another embodiment, compounds I are of formula I.A2 wherein Z is Z-269, R³ is CH₃ and Q is Q1, and R and R⁴ are as per any row of Table U below, which compounds are named I.A2-Z269-Q1-U-1 to I.A2-Z269-Q1-U-42.

In another embodiment, compounds I are of formula I.A2 wherein Z is Z-269, R³ is CH₃ and Q is Q2, and R and R⁴ are as per any row of Table U below, which compounds are named I.A2-Z269-Q2-U-1 to I.A2-Z269-Q2-U-42.

In another embodiment, compounds I are of formula I.A2 wherein Z is Z-269, R³ is CH₃ and Q is Q3, and R and R⁴ are as per any row of Table U below, which compounds are named I.A2-Z269-Q3-U-1 to I.A2-Z269-Q3-U-42.

In another embodiment, compounds I are of formula I.A2 wherein Z is Z-269, R³ is CH₃ and Q is Q4, and R and R⁴ are as per any row of Table U below, which compounds are named I.A2-Z269-Q4-U-1 to I.A2-Z269-Q4-U-42.

In another embodiment, compounds I are of formula I.A1 wherein Z is Z-270, R³ is CH₃ and Q is Q1, and R and R⁴ are as per any row of Table U below, which compounds are named I.A1-Z270-Q1-U-1 to I.A1-Z270-Q1-U-42.

In another embodiment, compounds I are of formula I.A1 wherein Z is Z-270, R³ is CH₃ and Q is Q2, and R and R⁴ are as per any row of Table U below, which compounds are named I.A1-Z270-Q2-U-1 to I.A1-Z270-Q2-U-42.

In another embodiment, compounds I are of formula I.A1 wherein Z is Z-270, R³ is CH₃ and Q is Q3, and R and R⁴ are as per any row of Table U below, which compounds are named I.A1-Z270-Q3-U-1 to I.A1-Z270-Q3-U-42.

In another embodiment, compounds I are of formula I.A1 wherein Z is Z-270, R³ is CH₃ and Q is Q4, and R and R⁴ are as per any row of Table U below, which compounds are named I.A1-Z270-Q4-U-1 to I.A1-Z270-Q4-U-42.

In another embodiment, compounds I are of formula I.A2 wherein Z is Z-270, R³ is CH₃ and Q is Q1, and R and R⁴ are as per any row of Table U below, which compounds are named I.A2-Z270-Q1-U-1 to I.A2-Z270-Q1-U-42.

In another embodiment, compounds I are of formula I.A2 wherein Z is Z-270, R³ is CH₃ and Q is Q2, and R and R⁴ are as per any row of Table U below, which compounds are named I.A2-Z270-Q2-U-1 to I.A2-Z270-Q2-U-42.

In another embodiment, compounds I are of formula I.A2 wherein Z is Z-270, R³ is CH₃ and Q is Q3, and R and R⁴ are as per any row of Table U below, which compounds are named I.A2-Z270-Q3-U-1 to I.A2-Z270-Q3-U-42.

In another embodiment, compounds I are of formula I.A2 wherein Z is Z-270, R³ is CH₃ and Q is Q4, and R and R⁴ are as per any row of Table U below, which compounds are named I.A2-Z270-Q4-U-1 to I.A2-Z270-Q4-U-42.

In another embodiment, compounds I are of formula I.A1 wherein Z is Z-271, R³ is CH₃ and Q is Q1, and R and R⁴ are as per any row of Table U below, which compounds are named I.A1-Z271-Q1-U-1 to I.A1-Z271-Q1-U-42.

In another embodiment, compounds I are of formula I.A1 wherein Z is Z-271, R³ is CH₃ and Q is Q2, and R and R⁴ are as per any row of Table U below, which compounds are named I.A1-Z271-Q2-U-1 to I.A1-Z271-Q2-U-42.

In another embodiment, compounds I are of formula I.A1 wherein Z is Z-271, R³ is CH₃ and Q is Q3, and R and R⁴ are as per any row of Table U below, which compounds are named I.A1-Z271-Q3-U-1 to I.A1-Z271-Q3-U-42.

In another embodiment, compounds I are of formula I.A1 wherein Z is Z-271, R³ is CH₃ and Q is Q4, and R and R⁴ are as per any row of Table U below, which compounds are named I.A1-Z271-Q4-U-1 to I.A1-Z271-Q4-U-42.

In another embodiment, compounds I are of formula I.A2 wherein Z is Z-271, R³ is CH₃ and Q is Q1, and R and R⁴ are as per any row of Table U below, which compounds are named I.A2-Z271-Q1-U-1 to I.A2-Z271-Q1-U-42.

In another embodiment, compounds I are of formula I.A2 wherein Z is Z-271, R³ is CH₃ and Q is Q2, and R and R⁴ are as per any row of Table U below, which compounds are named I.A2-Z271-Q2-U-1 to I.A2-Z271-Q2-U-42.

In another embodiment, compounds I are of formula I.A2 wherein Z is Z-271, R³ is CH₃ and Q is Q3, and R and R⁴ are as per any row of Table U below, which compounds are named I.A2-Z271-Q3-U-1 to I.A2-Z271-Q3-U-42.

In another embodiment, compounds I are of formula I.A2 wherein Z is Z-271, R³ is CH₃ and Q is Q4, and R and R⁴ are as per any row of Table U below, which compounds are named I.A2-Z271-Q4-U-1 to I.A2-Z271-Q4-U-42.

In another embodiment, compounds I are of formula I.A1 wherein Z is Z-272, R³ is CH₃ and Q is Q1, and R and R⁴ are as per any row of Table U below, which compounds are named I.A1-Z272-Q1-U-1 to I.A1-Z272-Q1-U-42.

In another embodiment, compounds I are of formula I.A1 wherein Z is Z-272, R³ is CH₃ and Q is Q2, and R and R⁴ are as per any row of Table U below, which compounds are named I.A1-Z272-Q2-U-1 to I.A1-Z272-Q2-U-42.

In another embodiment, compounds I are of formula I.A1 wherein Z is Z-272, R³ is CH₃ and Q is Q3, and R and R⁴ are as per any row of Table U below, which compounds are named I.A1-Z272-Q3-U-1 to I.A1-Z272-Q3-U-42.

In another embodiment, compounds I are of formula I.A1 wherein Z is Z-272, R³ is CH₃ and Q is Q4, and R and R⁴ are as per any row of Table U below, which compounds are named I.A1-Z272-Q4-U-1 to I.A1-Z272-Q4-U-42.

In another embodiment, compounds I are of formula I.A2 wherein Z is Z-272, R³ is CH₃ and Q is Q1, and R and R⁴ are as per any row of Table U below, which compounds are named I.A2-Z272-Q1-U-1 to I.A2-Z272-Q1-U-42.

In another embodiment, compounds I are of formula I.A2 wherein Z is Z-272, R³ is CH₃ and Q is Q2, and R and R⁴ are as per any row of Table U below, which compounds are named I.A2-Z272-Q2-U-1 to I.A2-Z272-Q2-U-42.

In another embodiment, compounds I are of formula I.A2 wherein Z is Z-272, R³ is CH₃ and Q is Q3, and R and R⁴ are as per any row of Table U below, which compounds are named I.A2-Z272-Q3-U-1 to I.A2-Z272-Q3-U-42.

In another embodiment, compounds I are of formula I.A2 wherein Z is Z-272, R³ is CH₃ and Q is Q4, and R and R⁴ are as per any row of Table U below, which compounds are named I.A2-Z272-Q4-U-1 to I.A2-Z272-Q4-U-42.

In another embodiment, compounds I are of formula I.A1 wherein Z is Z-273, R³ is CH₃ and Q is Q1, and R and R⁴ are as per any row of Table U below, which compounds are named I.A1-Z273-Q1-U-1 to I.A1-Z273-Q1-U-42.

In another embodiment, compounds I are of formula I.A1 wherein Z is Z-273, R³ is CH₃ and Q is Q2, and R and R⁴ are as per any row of Table U below, which compounds are named I.A1-Z273-Q2-U-1 to I.A1-Z273-Q2-U-42.

In another embodiment, compounds I are of formula I.A1 wherein Z is Z-273, R³ is CH₃ and Q is Q3, and R and R⁴ are as per any row of Table U below, which compounds are named I.A1-Z273-Q3-U-1 to I.A1-Z273-Q3-U-42.

In another embodiment, compounds I are of formula I.A1 wherein Z is Z-273, R³ is CH₃ and Q is Q4, and R and R⁴ are as per any row of Table U below, which compounds are named I.A1-Z273-Q4-U-1 to I.A1-Z273-Q4-U-42.

In another embodiment, compounds I are of formula I.A2 wherein Z is Z-273, R³ is CH₃ and Q is Q1, and R and R⁴ are as per any row of Table U below, which compounds are named I.A2-Z273-Q1-U-1 to I.A2-Z273-Q1-U-42.

In another embodiment, compounds I are of formula I.A2 wherein Z is Z-273, R³ is CH₃ and Q is Q2, and R and R⁴ are as per any row of Table U below, which compounds are named I.A2-Z273-Q2-U-1 to I.A2-Z273-Q2-U-42.

In another embodiment, compounds I are of formula I.A2 wherein Z is Z-273, R³ is CH₃ and Q is Q3, and R and R⁴ are as per any row of Table U below, which compounds are named I.A2-Z273-Q3-U-1 to I.A2-Z273-Q3-U-42.

In another embodiment, compounds I are of formula I.A2 wherein Z is Z-273, R³ is CH₃ and Q is Q4, and R and R⁴ are as per any row of Table U below, which compounds are named I.A2-Z273-Q4-U-1 to I.A2-Z273-Q4-U-42.

In another embodiment, compounds I are of formula I.A1 wherein Z is Z-274, R³ is CH₃ and Q is Q1, and R and R⁴ are as per any row of Table U below, which compounds are named I.A1-Z274-Q1-U-1 to I.A1-Z274-Q1-U-42.

In another embodiment, compounds I are of formula I.A1 wherein Z is Z-274, R³ is CH₃ and Q is Q2, and R and R⁴ are as per any row of Table U below, which compounds are named I.A1-Z274-Q2-U-1 to I.A1-Z274-Q2-U-42.

In another embodiment, compounds I are of formula I.A1 wherein Z is Z-274, R³ is CH₃ and Q is Q3, and R and R⁴ are as per any row of Table U below, which compounds are named I.A1-Z274-Q3-U-1 to I.A1-Z274-Q3-U-42.

In another embodiment, compounds I are of formula I.A1 wherein Z is Z-274, R³ is CH₃ and Q is Q4, and R and R⁴ are as per any row of Table U below, which compounds are named I.A1-Z274-Q4-U-1 to I.A1-Z274-Q4-U-42.

In another embodiment, compounds I are of formula I.A2 wherein Z is Z-274, R³ is CH₃ and Q is Q1, and R and R⁴ are as per any row of Table U below, which compounds are named I.A2-Z274-Q1-U-1 to I.A2-Z274-Q1-U-42.

In another embodiment, compounds I are of formula I.A2 wherein Z is Z-274, R³ is CH₃ and Q is Q2, and R and R⁴ are as per any row of Table U below, which compounds are named I.A2-Z274-Q2-U-1 to I.A2-Z274-Q2-U-42.

In another embodiment, compounds I are of formula I.A2 wherein Z is Z-274, R³ is CH₃ and Q is Q3, and R and R⁴ are as per any row of Table U below, which compounds are named I.A2-Z274-Q3-U-1 to I.A2-Z274-Q3-U-42.

In another embodiment, compounds I are of formula I.A2 wherein Z is Z-274, R³ is CH₃ and Q is Q4, and R and R⁴ are as per any row of Table U below, which compounds are named I.A2-Z274-Q4-U-1 to I.A2-Z274-Q4-U-42.

In another embodiment, compounds I are of formula I.A1 wherein Z is Z-275, R³ is CH₃ and Q is Q1, and R and R⁴ are as per any row of Table U below, which compounds are named I.A1-Z275-Q1-U-1 to I.A1-Z275-Q1-U-42.

In another embodiment, compounds I are of formula I.A1 wherein Z is Z-275, R³ is CH₃ and Q is Q2, and R and R⁴ are as per any row of Table U below, which compounds are named I.A1-Z275-Q2-U-1 to I.A1-Z275-Q2-U-42.

In another embodiment, compounds I are of formula I.A1 wherein Z is Z-275, R³ is CH₃ and Q is Q3, and R and R⁴ are as per any row of Table U below, which compounds are named I.A1-Z275-Q3-U-1 to I.A1-Z275-Q3-U-42.

In another embodiment, compounds I are of formula I.A1 wherein Z is Z-275, R³ is CH₃ and Q is Q4, and R and R⁴ are as per any row of Table U below, which compounds are named I.A1-Z275-Q4-U-1 to I.A1-Z275-Q4-U-42.

In another embodiment, compounds I are of formula I.A2 wherein Z is Z-275, R³ is CH₃ and Q is Q1, and R and R⁴ are as per any row of Table U below, which compounds are named I.A2-Z275-Q1-U-1 to I.A2-Z275-Q1-U-42.

In another embodiment, compounds I are of formula I.A2 wherein Z is Z-275, R³ is CH₃ and Q is Q2, and R and R⁴ are as per any row of Table U below, which compounds are named I.A2-Z275-Q2-U-1 to I.A2-Z275-Q2-U-42.

In another embodiment, compounds I are of formula I.A2 wherein Z is Z-275, R³ is CH₃ and Q is Q3, and R and R⁴ are as per any row of Table U below, which compounds are named I.A2-Z275-Q3-U-1 to I.A2-Z275-Q3-U-42.

In another embodiment, compounds I are of formula I.A2 wherein Z is Z-275, R³ is CH₃ and Q is Q4, and R and R⁴ are as per any row of Table U below, which compounds are named I.A2-Z275-Q4-U-1 to I.A2-Z275-Q4-U-42.

In another embodiment, compounds I are of formula I.A1 wherein Z is Z-276, R³ is CH₃ and Q is Q1, and R and R⁴ are as per any row of Table U below, which compounds are named I.A1-Z276-Q1-U-1 to I.A1-Z276-Q1-U-42.

In another embodiment, compounds I are of formula I.A1 wherein Z is Z-276, R³ is CH₃ and Q is Q2, and R and R⁴ are as per any row of Table U below, which compounds are named I.A1-Z276-Q2-U-1 to I.A1-Z276-Q2-U-42.

In another embodiment, compounds I are of formula I.A1 wherein Z is Z-276, R³ is CH₃ and Q is Q3, and R and R⁴ are as per any row of Table U below, which compounds are named I.A1-Z276-Q3-U-1 to I.A1-Z276-Q3-U-42.

In another embodiment, compounds I are of formula I.A1 wherein Z is Z-276, R³ is CH₃ and Q is Q4, and R and R⁴ are as per any row of Table U below, which compounds are named I.A1-Z276-Q4-U-1 to I.A1-Z276-Q4-U-42.

In another embodiment, compounds I are of formula I.A2 wherein Z is Z-276, R³ is CH₃ and Q is Q1, and R and R⁴ are as per any row of Table U below, which compounds are named I.A2-Z276-Q1-U-1 to I.A2-Z276-Q1-U-42.

In another embodiment, compounds I are of formula I.A2 wherein Z is Z-276, R³ is CH₃ and Q is Q2, and R and R⁴ are as per any row of Table U below, which compounds are named I.A2-Z276-Q2-U-1 to I.A2-Z276-Q2-U-42.

In another embodiment, compounds I are of formula I.A2 wherein Z is Z-276, R³ is CH₃ and Q is Q3, and R and R⁴ are as per any row of Table U below, which compounds are named I.A2-Z276-Q3-U-1 to I.A2-Z276-Q3-U-42.

In another embodiment, compounds I are of formula I.A2 wherein Z is Z-276, R³ is CH₃ and Q is Q4, and R and R⁴ are as per any row of Table U below, which compounds are named I.A2-Z276-Q4-U-1 to I.A2-Z276-Q4-U-42.

In another embodiment, compounds I are of formula I.A1 wherein Z is Z-277, R³ is CH₃ and Q is Q1, and R and R⁴ are as per any row of Table U below, which compounds are named I.A1-Z277-Q1-U-1 to I.A1-Z277-Q1-U-42.

In another embodiment, compounds I are of formula I.A1 wherein Z is Z-277, R³ is CH₃ and Q is Q2, and R and R⁴ are as per any row of Table U below, which compounds are named I.A1-Z277-Q2-U-1 to I.A1-Z277-Q2-U-42.

In another embodiment, compounds I are of formula I.A1 wherein Z is Z-277, R³ is CH₃ and Q is Q3, and R and R⁴ are as per any row of Table U below, which compounds are named I.A1-Z277-Q3-U-1 to I.A1-Z277-Q3-U-42.

In another embodiment, compounds I are of formula I.A1 wherein Z is Z-277, R³ is CH₃ and Q is Q4, and R and R⁴ are as per any row of Table U below, which compounds are named I.A1-Z277-Q4-U-1 to I.A1-Z277-Q4-U-42.

In another embodiment, compounds I are of formula I.A2 wherein Z is Z-277, R³ is CH₃ and Q is Q1, and R and R⁴ are as per any row of Table U below, which compounds are named I.A2-Z277-Q1-U-1 to I.A2-Z277-Q1-U-42.

In another embodiment, compounds I are of formula I.A2 wherein Z is Z-277, R³ is CH₃ and Q is Q2, and R and R⁴ are as per any row of Table U below, which compounds are named I.A2-Z277-Q2-U-1 to I.A2-Z277-Q2-U-42.

In another embodiment, compounds I are of formula I.A2 wherein Z is Z-277, R³ is CH₃ and Q is Q3, and R and R⁴ are as per any row of Table U below, which compounds are named I.A2-Z277-Q3-U-1 to I.A2-Z277-Q3-U-42.

In another embodiment, compounds I are of formula I.A2 wherein Z is Z-277, R³ is CH₃ and Q is Q4, and R and R⁴ are as per any row of Table U below, which compounds are named I.A2-Z277-Q4-U-1 to I.A2-Z277-Q4-U-42.

In another embodiment, compounds I are of formula I.A1 wherein Z is Z-278, R³ is CH₃ and Q is Q1, and R and R⁴ are as per any row of Table U below, which compounds are named I.A1-Z278-Q1-U-1 to I.A1-Z278-Q1-U-42.

In another embodiment, compounds I are of formula I.A1 wherein Z is Z-278, R³ is CH₃ and Q is Q2, and R and R⁴ are as per any row of Table U below, which compounds are named I.A1-Z278-Q2-U-1 to I.A1-Z278-Q2-U-42.

In another embodiment, compounds I are of formula I.A1 wherein Z is Z-278, R³ is CH₃ and Q is Q3, and R and R⁴ are as per any row of Table U below, which compounds are named I.A1-Z278-Q3-U-1 to I.A1-Z278-Q3-U-42.

In another embodiment, compounds I are of formula I.A1 wherein Z is Z-278, R³ is CH₃ and Q is Q4, and R and R⁴ are as per any row of Table U below, which compounds are named I.A1-Z278-Q4-U-1 to I.A1-Z278-Q4-U-42.

In another embodiment, compounds I are of formula I.A2 wherein Z is Z-278, R³ is CH₃ and Q is Q1, and R and R⁴ are as per any row of Table U below, which compounds are named I.A2-Z278-Q1-U-1 to I.A2-Z278-Q1-U-42.

In another embodiment, compounds I are of formula I.A2 wherein Z is Z-278, R³ is CH₃ and Q is Q2, and R and R⁴ are as per any row of Table U below, which compounds are named I.A2-Z278-Q2-U-1 to I.A2-Z278-Q2-U-42.

In another embodiment, compounds I are of formula I.A2 wherein Z is Z-278, R³ is CH₃ and Q is Q3, and R and R⁴ are as per any row of Table U below, which compounds are named I.A2-Z278-Q3-U-1 to I.A2-Z278-Q3-U-42.

In another embodiment, compounds I are of formula I.A2 wherein Z is Z-278, R³ is CH₃ and Q is Q4, and R and R⁴ are as per any row of Table U below, which compounds are named I.A2-Z278-Q4-U-1 to I.A2-Z278-Q4-U-42.

In another embodiment, compounds I are of formula I.A1 wherein Z is Z-279, R³ is CH₃ and Q is Q1, and R and R⁴ are as per any row of Table U below, which compounds are named I.A1-Z279-Q1-U-1 to I.A1-Z279-Q1-U-42.

In another embodiment, compounds I are of formula I.A1 wherein Z is Z-279, R³ is CH₃ and Q is Q2, and R and R⁴ are as per any row of Table U below, which compounds are named I.A1-Z279-Q2-U-1 to I.A1-Z279-Q2-U-42.

In another embodiment, compounds I are of formula I.A1 wherein Z is Z-279, R³ is CH₃ and Q is Q3, and R and R⁴ are as per any row of Table U below, which compounds are named I.A1-Z279-Q3-U-1 to I.A1-Z279-Q3-U-42.

In another embodiment, compounds I are of formula I.A1 wherein Z is Z-279, R³ is CH₃ and Q is Q4, and R and R⁴ are as per any row of Table U below, which compounds are named I.A1-Z279-Q4-U-1 to I.A1-Z279-Q4-U-42.

In another embodiment, compounds I are of formula I.A2 wherein Z is Z-279, R³ is CH₃ and Q is Q1, and R and R⁴ are as per any row of Table U below, which compounds are named I.A2-Z279-Q1-U-1 to I.A2-Z279-Q1-U-42.

In another embodiment, compounds I are of formula I.A2 wherein Z is Z-279, R³ is CH₃ and Q is Q2, and R and R⁴ are as per any row of Table U below, which compounds are named I.A2-Z279-Q2-U-1 to I.A2-Z279-Q2-U-42.

In another embodiment, compounds I are of formula I.A2 wherein Z is Z-279, R³ is CH₃ and Q is Q3, and R and R⁴ are as per any row of Table U below, which compounds are named I.A2-Z279-Q3-U-1 to I.A2-Z279-Q3-U-42.

In another embodiment, compounds I are of formula I.A2 wherein Z is Z-279, R³ is CH₃ and Q is Q4, and R and R⁴ are as per any row of Table U below, which compounds are named I.A2-Z279-Q4-U-1 to I.A2-Z279-Q4-U-42.

In another embodiment, compounds I are of formula I.A1 wherein Z is Z-280, R³ is CH₃ and Q is Q1, and R and R⁴ are as per any row of Table U below, which compounds are named I.A1-Z280-Q1-U-1 to I.A1-Z280-Q1-U-42.

In another embodiment, compounds I are of formula I.A1 wherein Z is Z-280, R³ is CH₃ and Q is Q2, and R and R⁴ are as per any row of Table U below, which compounds are named I.A1-Z280-Q2-U-1 to I.A1-Z280-Q2-U-42.

In another embodiment, compounds I are of formula I.A1 wherein Z is Z-280, R³ is CH₃ and Q is Q3, and R and R⁴ are as per any row of Table U below, which compounds are named I.A1-Z280-Q3-U-1 to I.A1-Z280-Q3-U-42.

In another embodiment, compounds I are of formula I.A1 wherein Z is Z-280, R³ is CH₃ and Q is Q4, and R and R⁴ are as per any row of Table U below, which compounds are named I.A1-Z280-Q4-U-1 to I.A1-Z280-Q4-U-42.

In another embodiment, compounds I are of formula I.A2 wherein Z is Z-280, R³ is CH₃ and Q is Q1, and R and R⁴ are as per any row of Table U below, which compounds are named I.A2-Z280-Q1-U-1 to I.A2-Z280-Q1-U-42.

In another embodiment, compounds I are of formula I.A2 wherein Z is Z-280, R³ is CH₃ and Q is Q2, and R and R⁴ are as per any row of Table U below, which compounds are named I.A2-Z280-Q2-U-1 to I.A2-Z280-Q2-U-42.

In another embodiment, compounds I are of formula I.A2 wherein Z is Z-280, R³ is CH₃ and Q is Q3, and R and R⁴ are as per any row of Table U below, which compounds are named I.A2-Z280-Q3-U-1 to I.A2-Z280-Q3-U-42.

In another embodiment, compounds I are of formula I.A2 wherein Z is Z-280, R³ is CH₃ and Q is Q4, and R and R⁴ are as per any row of Table U below, which compounds are named I.A2-Z280-Q4-U-1 to I.A2-Z280-Q4-U-42.

In another embodiment, compounds I are of formula I.A1 wherein Z is Z-281, R³ is CH₃ and Q is Q1, and R and R⁴ are as per any row of Table U below, which compounds are named I.A1-Z281-Q1-U-1 to I.A1-Z281-Q1-U-42.

In another embodiment, compounds I are of formula I.A1 wherein Z is Z-281, R³ is CH₃ and Q is Q2, and R and R⁴ are as per any row of Table U below, which compounds are named I.A1-Z281-Q2-U-1 to I.A1-Z281-Q2-U-42.

In another embodiment, compounds I are of formula I.A1 wherein Z is Z-281, R³ is CH₃ and Q is Q3, and R and R⁴ are as per any row of Table U below, which compounds are named I.A1-Z281-Q3-U-1 to I.A1-Z281-Q3-U-42.

In another embodiment, compounds I are of formula I.A1 wherein Z is Z-281, R³ is CH₃ and Q is Q4, and R and R⁴ are as per any row of Table U below, which compounds are named I.A1-Z281-Q4-U-1 to I.A1-Z281-Q4-U-42.

In another embodiment, compounds I are of formula I.A2 wherein Z is Z-281, R³ is CH₃ and Q is Q1, and R and R⁴ are as per any row of Table U below, which compounds are named I.A2-Z281-Q1-U-1 to I.A2-Z281-Q1-U-42.

In another embodiment, compounds I are of formula I.A2 wherein Z is Z-281, R³ is CH₃ and Q is Q2, and R and R⁴ are as per any row of Table U below, which compounds are named I.A2-Z281-Q2-U-1 to I.A2-Z281-Q2-U-42.

In another embodiment, compounds I are of formula I.A2 wherein Z is Z-281, R³ is CH₃ and Q is Q3, and R and R⁴ are as per any row of Table U below, which compounds are named I.A2-Z281-Q3-U-1 to I.A2-Z281-Q3-U-42.

In another embodiment, compounds I are of formula I.A2 wherein Z is Z-281, R³ is CH₃ and Q is Q4, and R and R⁴ are as per any row of Table U below, which compounds are named I.A2-Z281-Q4-U-1 to I.A2-Z281-Q4-U-42.

In another embodiment, compounds I are of formula I.A1 wherein Z is Z-282, R³ is CH₃ and Q is Q1, and R and R⁴ are as per any row of Table U below, which compounds are named I.A1-Z282-Q1-U-1 to I.A1-Z282-Q1-U-42.

In another embodiment, compounds I are of formula I.A1 wherein Z is Z-282, R³ is CH₃ and Q is Q2, and R and R⁴ are as per any row of Table U below, which compounds are named I.A1-Z282-Q2-U-1 to I.A1-Z282-Q2-U-42.

In another embodiment, compounds I are of formula I.A1 wherein Z is Z-282, R³ is CH₃ and Q is Q3, and R and R⁴ are as per any row of Table U below, which compounds are named I.A1-Z282-Q3-U-1 to I.A1-Z282-Q3-U-42.

In another embodiment, compounds I are of formula I.A1 wherein Z is Z-282, R³ is CH₃ and Q is Q4, and R and R⁴ are as per any row of Table U below, which compounds are named I.A1-Z282-Q4-U-1 to I.A1-Z282-Q4-U-42.

In another embodiment, compounds I are of formula I.A2 wherein Z is Z-282, R³ is CH₃ and Q is Q1, and R and R⁴ are as per any row of Table U below, which compounds are named I.A2-Z282-Q1-U-1 to I.A2-Z282-Q1-U-42.

In another embodiment, compounds I are of formula I.A2 wherein Z is Z-282, R³ is CH₃ and Q is Q2, and R and R⁴ are as per any row of Table U below, which compounds are named I.A2-Z282-Q2-U-1 to I.A2-Z282-Q2-U-42.

In another embodiment, compounds I are of formula I.A2 wherein Z is Z-282, R³ is CH₃ and Q is Q3, and R and R⁴ are as per any row of Table U below, which compounds are named I.A2-Z282-Q3-U-1 to I.A2-Z282-Q3-U-42.

In another embodiment, compounds I are of formula I.A2 wherein Z is Z-282, R³ is CH₃ and Q is Q4, and R and R⁴ are as per any row of Table U below, which compounds are named I.A2-Z282-Q4-U-1 to I.A2-Z282-Q4-U-42.

In another embodiment, compounds I are of formula I.A1 wherein Z is Z-283, R³ is CH₃ and Q is Q1, and R and R⁴ are as per any row of Table U below, which compounds are named I.A1-Z283-Q1-U-1 to I.A1-Z283-Q1-U-42.

In another embodiment, compounds I are of formula I.A1 wherein Z is Z-283, R³ is CH₃ and Q is Q2, and R and R⁴ are as per any row of Table U below, which compounds are named I.A1-Z283-Q2-U-1 to I.A1-Z283-Q2-U-42.

In another embodiment, compounds I are of formula I.A1 wherein Z is Z-283, R³ is CH₃ and Q is Q3, and R and R⁴ are as per any row of Table U below, which compounds are named I.A1-Z283-Q3-U-1 to I.A1-Z283-Q3-U-42.

In another embodiment, compounds I are of formula I.A1 wherein Z is Z-283, R³ is CH₃ and Q is Q4, and R and R⁴ are as per any row of Table U below, which compounds are named I.A1-Z283-Q4-U-1 to I.A1-Z283-Q4-U-42.

In another embodiment, compounds I are of formula I.A2 wherein Z is Z-283, R³ is CH₃ and Q is Q1, and R and R⁴ are as per any row of Table U below, which compounds are named I.A2-Z283-Q1-U-1 to I.A2-Z283-Q1-U-42.

In another embodiment, compounds I are of formula I.A2 wherein Z is Z-283, R³ is CH₃ and Q is Q2, and R and R⁴ are as per any row of Table U below, which compounds are named I.A2-Z283-Q2-U-1 to I.A2-Z283-Q2-U-42.

In another embodiment, compounds I are of formula I.A2 wherein Z is Z-283, R³ is CH₃ and Q is Q3, and R and R⁴ are as per any row of Table U below, which compounds are named I.A2-Z283-Q3-U-1 to I.A2-Z283-Q3-U-42.

In another embodiment, compounds I are of formula I.A2 wherein Z is Z-283, R³ is CH₃ and Q is Q4, and R and R⁴ are as per any row of Table U below, which compounds are named I.A2-Z283-Q4-U-1 to I.A2-Z283-Q4-U-42.

In another embodiment, compounds I are of formula I.A1 wherein Z is Z-284, R³ is CH₃ and Q is Q1, and R and R⁴ are as per any row of Table U below, which compounds are named I.A1-Z284-Q1-U-1 to I.A1-Z284-Q1-U-42.

In another embodiment, compounds I are of formula I.A1 wherein Z is Z-284, R³ is CH₃ and Q is Q2, and R and R⁴ are as per any row of Table U below, which compounds are named I.A1-Z284-Q2-U-1 to I.A1-Z284-Q2-U-42.

In another embodiment, compounds I are of formula I.A1 wherein Z is Z-284, R³ is CH₃ and Q is Q3, and R and R⁴ are as per any row of Table U below, which compounds are named I.A1-Z284-Q3-U-1 to I.A1-Z284-Q3-U-42.

In another embodiment, compounds I are of formula I.A1 wherein Z is Z-284, R³ is CH₃ and Q is Q4, and R and R⁴ are as per any row of Table U below, which compounds are named I.A1-Z284-Q4-U-1 to I.A1-Z284-Q4-U-42.

In another embodiment, compounds I are of formula I.A2 wherein Z is Z-284, R³ is CH₃ and Q is Q1, and R and R⁴ are as per any row of Table U below, which compounds are named I.A2-Z284-Q1-U-1 to I.A2-Z284-Q1-U-42.

In another embodiment, compounds I are of formula I.A2 wherein Z is Z-284, R³ is CH₃ and Q is Q2, and R and R⁴ are as per any row of Table U below, which compounds are named I.A2-Z284-Q2-U-1 to I.A2-Z284-Q2-U-42.

In another embodiment, compounds I are of formula I.A2 wherein Z is Z-284, R³ is CH₃ and Q is Q3, and R and R⁴ are as per any row of Table U below, which compounds are named I.A2-Z284-Q3-U-1 to I.A2-Z284-Q3-U-42.

In another embodiment, compounds I are of formula I.A2 wherein Z is Z-284, R³ is CH₃ and Q is Q4, and R and R⁴ are as per any row of Table U below, which compounds are named I.A2-Z284-Q4-U-1 to I.A2-Z284-Q4-U-42.

In another embodiment, compounds I are of formula I.A1 wherein Z is Z-285, R³ is CH₃ and Q is Q1, and R and R⁴ are as per any row of Table U below, which compounds are named I.A1-Z285-Q1-U-1 to I.A1-Z285-Q1-U-42.

In another embodiment, compounds I are of formula I.A1 wherein Z is Z-285, R³ is CH₃ and Q is Q2, and R and R⁴ are as per any row of Table U below, which compounds are named I.A1-Z285-Q2-U-1 to I.A1-Z285-Q2-U-42.

In another embodiment, compounds I are of formula I.A1 wherein Z is Z-285, R³ is CH₃ and Q is Q3, and R and R⁴ are as per any row of Table U below, which compounds are named I.A1-Z285-Q3-U-1 to I.A1-Z285-Q3-U-42.

In another embodiment, compounds I are of formula I.A1 wherein Z is Z-285, R³ is CH₃ and Q is Q4, and R and R⁴ are as per any row of Table U below, which compounds are named I.A1-Z285-Q4-U-1 to I.A1-Z285-Q4-U-42.

In another embodiment, compounds I are of formula I.A2 wherein Z is Z-285, R³ is CH₃ and Q is Q1, and R and R⁴ are as per any row of Table U below, which compounds are named I.A2-Z285-Q1-U-1 to I.A2-Z285-Q1-U-42.

In another embodiment, compounds I are of formula I.A2 wherein Z is Z-285, R³ is CH₃ and Q is Q2, and R and R⁴ are as per any row of Table U below, which compounds are named I.A2-Z285-Q2-U-1 to I.A2-Z285-Q2-U-42.

In another embodiment, compounds I are of formula I.A2 wherein Z is Z-285, R³ is CH₃ and Q is Q3, and R and R⁴ are as per any row of Table U below, which compounds are named I.A2-Z285-Q3-U-1 to I.A2-Z285-Q3-U-42.

In another embodiment, compounds I are of formula I.A2 wherein Z is Z-285, R³ is CH₃ and Q is Q4, and R and R⁴ are as per any row of Table U below, which compounds are named I.A2-Z285-Q4-U-1 to I.A2-Z285-Q4-U-42.

In another embodiment, compounds I are of formula I.A1 wherein Z is Z-286, R³ is CH₃ and Q is Q1, and R and R⁴ are as per any row of Table U below, which compounds are named I.A1-Z286-Q1-U-1 to I.A1-Z286-Q1-U-42.

In another embodiment, compounds I are of formula I.A1 wherein Z is Z-286, R³ is CH₃ and Q is Q2, and R and R⁴ are as per any row of Table U below, which compounds are named I.A1-Z286-Q2-U-1 to I.A1-Z286-Q2-U-42.

In another embodiment, compounds I are of formula I.A1 wherein Z is Z-286, R³ is CH₃ and Q is Q3, and R and R⁴ are as per any row of Table U below, which compounds are named I.A1-Z286-Q3-U-1 to I.A1-Z286-Q3-U-42.

In another embodiment, compounds I are of formula I.A1 wherein Z is Z-286, R³ is CH₃ and Q is Q4, and R and R⁴ are as per any row of Table U below, which compounds are named I.A1-Z286-Q4-U-1 to I.A1-Z286-Q4-U-42.

In another embodiment, compounds I are of formula I.A2 wherein Z is Z-286, R³ is CH₃ and Q is Q1, and R and R⁴ are as per any row of Table U below, which compounds are named I.A2-Z286-Q1-U-1 to I.A2-Z286-Q1-U-42.

In another embodiment, compounds I are of formula I.A2 wherein Z is Z-286, R³ is CH₃ and Q is Q2, and R and R⁴ are as per any row of Table U below, which compounds are named I.A2-Z286-Q2-U-1 to I.A2-Z286-Q2-U-42.

In another embodiment, compounds I are of formula I.A2 wherein Z is Z-286, R³ is CH₃ and Q is Q3, and R and R⁴ are as per any row of Table U below, which compounds are named I.A2-Z286-Q3-U-1 to I.A2-Z286-Q3-U-42.

In another embodiment, compounds I are of formula I.A2 wherein Z is Z-286, R³ is CH₃ and Q is Q4, and R and R⁴ are as per any row of Table U below, which compounds are named I.A2-Z286-Q4-U-1 to I.A2-Z286-Q4-U-42.

In another embodiment, compounds I are of formula I.A1 wherein Z is Z-287, R³ is CH₃ and Q is Q1, and R and R⁴ are as per any row of Table U below, which compounds are named I.A1-Z287-Q1-U-1 to I.A1-Z287-Q1-U-42.

In another embodiment, compounds I are of formula I.A1 wherein Z is Z-287, R³ is CH₃ and Q is Q2, and R and R⁴ are as per any row of Table U below, which compounds are named I.A1-Z287-Q2-U-1 to I.A1-Z287-Q2-U-42.

In another embodiment, compounds I are of formula I.A1 wherein Z is Z-287, R³ is CH₃ and Q is Q3, and R and R⁴ are as per any row of Table U below, which compounds are named I.A1-Z287-Q3-U-1 to I.A1-Z287-Q3-U-42.

In another embodiment, compounds I are of formula I.A1 wherein Z is Z-287, R³ is CH₃ and Q is Q4, and R and R⁴ are as per any row of Table U below, which compounds are named I.A1-Z287-Q4-U-1 to I.A1-Z287-Q4-U-42.

In another embodiment, compounds I are of formula I.A2 wherein Z is Z-287, R³ is CH₃ and Q is Q1, and R and R⁴ are as per any row of Table U below, which compounds are named I.A2-Z287-Q1-U-1 to I.A2-Z287-Q1-U-42.

In another embodiment, compounds I are of formula I.A2 wherein Z is Z-287, R³ is CH₃ and Q is Q2, and R and R⁴ are as per any row of Table U below, which compounds are named I.A2-Z287-Q2-U-1 to I.A2-Z287-Q2-U-42.

In another embodiment, compounds I are of formula I.A2 wherein Z is Z-287, R³ is CH₃ and Q is Q3, and R and R⁴ are as per any row of Table U below, which compounds are named I.A2-Z287-Q3-U-1 to I.A2-Z287-Q3-U-42.

In another embodiment, compounds I are of formula I.A2 wherein Z is Z-287, R³ is CH₃ and Q is Q4, and R and R⁴ are as per any row of Table U below, which compounds are named I.A2-Z287-Q4-U-1 to I.A2-Z287-Q4-U-42.

In another embodiment, compounds I are of formula I.A1 wherein Z is Z-288, R³ is CH₃ and Q is Q1, and R and R⁴ are as per any row of Table U below, which compounds are named I.A1-Z288-Q1-U-1 to I.A1-Z288-Q1-U-42.

In another embodiment, compounds I are of formula I.A1 wherein Z is Z-288, R³ is CH₃ and Q is Q2, and R and R⁴ are as per any row of Table U below, which compounds are named I.A1-Z288-Q2-U-1 to I.A1-Z288-Q2-U-42.

In another embodiment, compounds I are of formula I.A1 wherein Z is Z-288, R³ is CH₃ and Q is Q3, and R and R⁴ are as per any row of Table U below, which compounds are named I.A1-Z288-Q3-U-1 to I.A1-Z288-Q3-U-42.

In another embodiment, compounds I are of formula I.A1 wherein Z is Z-288, R³ is CH₃ and Q is Q4, and R and R⁴ are as per any row of Table U below, which compounds are named I.A1-Z288-Q4-U-1 to I.A1-Z288-Q4-U-42.

In another embodiment, compounds I are of formula I.A2 wherein Z is Z-288, R³ is CH₃ and Q is Q1, and R and R⁴ are as per any row of Table U below, which compounds are named I.A2-Z288-Q1-U-1 to I.A2-Z288-Q1-U-42.

In another embodiment, compounds I are of formula I.A2 wherein Z is Z-288, R³ is CH₃ and Q is Q2, and R and R⁴ are as per any row of Table U below, which compounds are named I.A2-Z288-Q2-U-1 to I.A2-Z288-Q2-U-42.

In another embodiment, compounds I are of formula I.A2 wherein Z is Z-288, R³ is CH₃ and Q is Q3, and R and R⁴ are as per any row of Table U below, which compounds are named I.A2-Z288-Q3-U-1 to I.A2-Z288-Q3-U-42.

In another embodiment, compounds I are of formula I.A2 wherein Z is Z-288, R³ is CH₃ and Q is Q4, and R and R⁴ are as per any row of Table U below, which compounds are named I.A2-Z288-Q4-U-1 to I.A2-Z288-Q4-U-42.

In another embodiment, compounds I are of formula I.A1 wherein Z is Z-289, R³ is CH₃ and Q is Q1, and R and R⁴ are as per any row of Table U below, which compounds are named I.A1-Z289-Q1-U-1 to I.A1-Z289-Q1-U-42.

In another embodiment, compounds I are of formula I.A1 wherein Z is Z-289, R³ is CH₃ and Q is Q2, and R and R⁴ are as per any row of Table U below, which compounds are named I.A1-Z289-Q2-U-1 to I.A1-Z289-Q2-U-42.

In another embodiment, compounds I are of formula I.A1 wherein Z is Z-289, R³ is CH₃ and Q is Q3, and R and R⁴ are as per any row of Table U below, which compounds are named I.A1-Z289-Q3-U-1 to I.A1-Z289-Q3-U-42.

In another embodiment, compounds I are of formula I.A1 wherein Z is Z-289, R³ is CH₃ and Q is Q4, and R and R⁴ are as per any row of Table U below, which compounds are named I.A1-Z289-Q4-U-1 to I.A1-Z289-Q4-U-42.

In another embodiment, compounds I are of formula I.A2 wherein Z is Z-289, R³ is CH₃ and Q is Q1, and R and R⁴ are as per any row of Table U below, which compounds are named I.A2-Z289-Q1-U-1 to I.A2-Z289-Q1-U-42.

In another embodiment, compounds I are of formula I.A2 wherein Z is Z-289, R³ is CH₃ and Q is Q2, and R and R⁴ are as per any row of Table U below, which compounds are named I.A2-Z289-Q2-U-1 to I.A2-Z289-Q2-U-42.

In another embodiment, compounds I are of formula I.A2 wherein Z is Z-289, R³ is CH₃ and Q is Q3, and R and R⁴ are as per any row of Table U below, which compounds are named I.A2-Z289-Q3-U-1 to I.A2-Z289-Q3-U-42.

In another embodiment, compounds I are of formula I.A2 wherein Z is Z-289, R³ is CH₃ and Q is Q4, and R and R⁴ are as per any row of Table U below, which compounds are named I.A2-Z289-Q4-U-1 to I.A2-Z289-Q4-U-42.

In another embodiment, compounds I are of formula I.A1 wherein Z is Z-290, R³ is CH₃ and Q is Q1, and R and R⁴ are as per any row of Table U below, which compounds are named I.A1-Z290-Q1-U-1 to I.A1-Z290-Q1-U-42.

In another embodiment, compounds I are of formula I.A1 wherein Z is Z-290, R³ is CH₃ and Q is Q2, and R and R⁴ are as per any row of Table U below, which compounds are named I.A1-Z290-Q2-U-1 to I.A1-Z290-Q2-U-42.

In another embodiment, compounds I are of formula I.A1 wherein Z is Z-290, R³ is CH₃ and Q is Q3, and R and R⁴ are as per any row of Table U below, which compounds are named I.A1-Z290-Q3-U-1 to I.A1-Z290-Q3-U-42.

In another embodiment, compounds I are of formula I.A1 wherein Z is Z-290, R³ is CH₃ and Q is Q4, and R and R⁴ are as per any row of Table U below, which compounds are named I.A1-Z290-Q4-U-1 to I.A1-Z290-Q4-U-42.

In another embodiment, compounds I are of formula I.A2 wherein Z is Z-290, R³ is CH₃ and Q is Q1, and R and R⁴ are as per any row of Table U below, which compounds are named I.A2-Z290-Q1-U-1 to I.A2-Z290-Q1-U-42.

In another embodiment, compounds I are of formula I.A2 wherein Z is Z-290, R³ is CH₃ and Q is Q2, and R and R⁴ are as per any row of Table U below, which compounds are named I.A2-Z290-Q2-U-1 to I.A2-Z290-Q2-U-42.

In another embodiment, compounds I are of formula I.A2 wherein Z is Z-290, R³ is CH₃ and Q is Q3, and R and R⁴ are as per any row of Table U below, which compounds are named I.A2-Z290-Q3-U-1 to I.A2-Z290-Q3-U-42.

In another embodiment, compounds I are of formula I.A2 wherein Z is Z-290, R³ is CH₃ and Q is Q4, and R and R⁴ are as per any row of Table U below, which compounds are named I.A2-Z290-Q4-U-1 to I.A2-Z290-Q4-U-42.

In another embodiment, compounds I are of formula I.A1 wherein Z is Z-291, R³ is CH₃ and Q is Q1, and R and R⁴ are as per any row of Table U below, which compounds are named I.A1-Z291-Q1-U-1 to I.A1-Z291-Q1-U-42.

In another embodiment, compounds I are of formula I.A1 wherein Z is Z-291, R³ is CH₃ and Q is Q2, and R and R⁴ are as per any row of Table U below, which compounds are named I.A1-Z291-Q2-U-1 to I.A1-Z291-Q2-U-42.

In another embodiment, compounds I are of formula I.A1 wherein Z is Z-291, R³ is CH₃ and Q is Q3, and R and R⁴ are as per any row of Table U below, which compounds are named I.A1-Z291-Q3-U-1 to I.A1-Z291-Q3-U-42.

In another embodiment, compounds I are of formula I.A1 wherein Z is Z-291, R³ is CH₃ and Q is Q4, and R and R⁴ are as per any row of Table U below, which compounds are named I.A1-Z291-Q4-U-1 to I.A1-Z291-Q4-U-42.

In another embodiment, compounds I are of formula I.A2 wherein Z is Z-291, R³ is CH₃ and Q is Q1, and R and R⁴ are as per any row of Table U below, which compounds are named I.A2-Z291-Q1-U-1 to I.A2-Z291-Q1-U-42.

In another embodiment, compounds I are of formula I.A2 wherein Z is Z-291, R³ is CH₃ and Q is Q2, and R and R⁴ are as per any row of Table U below, which compounds are named I.A2-Z291-Q2-U-1 to I.A2-Z291-Q2-U-42.

In another embodiment, compounds I are of formula I.A2 wherein Z is Z-291, R³ is CH₃ and Q is Q3, and R and R⁴ are as per any row of Table U below, which compounds are named I.A2-Z291-Q3-U-1 to I.A2-Z291-Q3-U-42.

In another embodiment, compounds I are of formula I.A2 wherein Z is Z-291, R³ is CH₃ and Q is Q4, and R and R⁴ are as per any row of Table U below, which compounds are named I.A2-Z291-Q4-U-1 to I.A2-Z291-Q4-U-42.

In another embodiment, compounds I are of formula I.A1 wherein Z is Z-292, R³ is CH₃ and Q is Q1, and R and R⁴ are as per any row of Table U below, which compounds are named I.A1-Z292-Q1-U-1 to I.A1-Z292-Q1-U-42.

In another embodiment, compounds I are of formula I.A1 wherein Z is Z-292, R³ is CH₃ and Q is Q2, and R and R⁴ are as per any row of Table U below, which compounds are named I.A1-Z292-Q2-U-1 to I.A1-Z292-Q2-U-42.

In another embodiment, compounds I are of formula I.A1 wherein Z is Z-292, R³ is CH₃ and Q is Q3, and R and R⁴ are as per any row of Table U below, which compounds are named I.A1-Z292-Q3-U-1 to I.A1-Z292-Q3-U-42.

In another embodiment, compounds I are of formula I.A1 wherein Z is Z-292, R³ is CH₃ and Q is Q4, and R and R⁴ are as per any row of Table U below, which compounds are named I.A1-Z292-Q4-U-1 to I.A1-Z292-Q4-U-42.

In another embodiment, compounds I are of formula I.A2 wherein Z is Z-292, R³ is CH₃ and Q is Q1, and R and R⁴ are as per any row of Table U below, which compounds are named I.A2-Z292-Q1-U-1 to I.A2-Z292-Q1-U-42.

In another embodiment, compounds I are of formula I.A2 wherein Z is Z-292, R³ is CH₃ and Q is Q2, and R and R⁴ are as per any row of Table U below, which compounds are named I.A2-Z292-Q2-U-1 to I.A2-Z292-Q2-U-42.

In another embodiment, compounds I are of formula I.A2 wherein Z is Z-292, R³ is CH₃ and Q is Q3, and R and R⁴ are as per any row of Table U below, which compounds are named I.A2-Z292-Q3-U-1 to I.A2-Z292-Q3-U-42.

In another embodiment, compounds I are of formula I.A2 wherein Z is Z-292, R³ is CH₃ and Q is Q4, and R and R⁴ are as per any row of Table U below, which compounds are named I.A2-Z292-Q4-U-1 to I.A2-Z292-Q4-U-42.

In another embodiment, compounds I are of formula I.A1 wherein Z is Z-293, R³ is CH₃ and Q is Q1, and R and R⁴ are as per any row of Table U below, which compounds are named I.A1-Z293-Q1-U-1 to I.A1-Z293-Q1-U-42.

In another embodiment, compounds I are of formula I.A1 wherein Z is Z-293, R³ is CH₃ and Q is Q2, and R and R⁴ are as per any row of Table U below, which compounds are named I.A1-Z293-Q2-U-1 to I.A1-Z293-Q2-U-42.

In another embodiment, compounds I are of formula I.A1 wherein Z is Z-293, R³ is CH₃ and Q is Q3, and R and R⁴ are as per any row of Table U below, which compounds are named I.A1-Z293-Q3-U-1 to I.A1-Z293-Q3-U-42.

In another embodiment, compounds I are of formula I.A1 wherein Z is Z-293, R³ is CH₃ and Q is Q4, and R and R⁴ are as per any row of Table U below, which compounds are named I.A1-Z293-Q4-U-1 to I.A1-Z293-Q4-U-42.

In another embodiment, compounds I are of formula I.A2 wherein Z is Z-293, R³ is CH₃ and Q is Q1, and R and R⁴ are as per any row of Table U below, which compounds are named I.A2-Z293-Q1-U-1 to I.A2-Z293-Q1-U-42.

In another embodiment, compounds I are of formula I.A2 wherein Z is Z-293, R³ is CH₃ and Q is Q2, and R and R⁴ are as per any row of Table U below, which compounds are named I.A2-Z293-Q2-U-1 to I.A2-Z293-Q2-U-42.

In another embodiment, compounds I are of formula I.A2 wherein Z is Z-293, R³ is CH₃ and Q is Q3, and R and R⁴ are as per any row of Table U below, which compounds are named I.A2-Z293-Q3-U-1 to I.A2-Z293-Q3-U-42.

In another embodiment, compounds I are of formula I.A2 wherein Z is Z-293, R³ is CH₃ and Q is Q4, and R and R⁴ are as per any row of Table U below, which compounds are named I.A2-Z293-Q4-U-1 to I.A2-Z293-Q4-U-42.

In another embodiment, compounds I are of formula I.A1 wherein Z is Z-294, R³ is CH₃ and Q is Q1, and R and R⁴ are as per any row of Table U below, which compounds are named I.A1-Z294-Q1-U-1 to I.A1-Z294-Q1-U-42.

In another embodiment, compounds I are of formula I.A1 wherein Z is Z-294, R³ is CH₃ and Q is Q2, and R and R⁴ are as per any row of Table U below, which compounds are named I.A1-Z294-Q2-U-1 to I.A1-Z294-Q2-U-42.

In another embodiment, compounds I are of formula I.A1 wherein Z is Z-294, R³ is CH₃ and Q is Q3, and R and R⁴ are as per any row of Table U below, which compounds are named I.A1-Z294-Q3-U-1 to I.A1-Z294-Q3-U-42.

In another embodiment, compounds I are of formula I.A1 wherein Z is Z-294, R³ is CH₃ and Q is Q4, and R and R⁴ are as per any row of Table U below, which compounds are named I.A1-Z294-Q4-U-1 to I.A1-Z294-Q4-U-42.

In another embodiment, compounds I are of formula I.A2 wherein Z is Z-294, R³ is CH₃ and Q is Q1, and R and R⁴ are as per any row of Table U below, which compounds are named I.A2-Z294-Q1-U-1 to I.A2-Z294-Q1-U-42.

In another embodiment, compounds I are of formula I.A2 wherein Z is Z-294, R³ is CH₃ and Q is Q2, and R and R⁴ are as per any row of Table U below, which compounds are named I.A2-Z294-Q2-U-1 to I.A2-Z294-Q2-U-42.

In another embodiment, compounds I are of formula I.A2 wherein Z is Z-294, R³ is CH₃ and Q is Q3, and R and R⁴ are as per any row of Table U below, which compounds are named I.A2-Z294-Q3-U-1 to I.A2-Z294-Q3-U-42.

In another embodiment, compounds I are of formula I.A2 wherein Z is Z-294, R³ is CH₃ and Q is Q4, and R and R⁴ are as per any row of Table U below, which compounds are named I.A2-Z294-Q4-U-1 to I.A2-Z294-Q4-U-42.

In another embodiment, compounds I are of formula I.A1 wherein Z is Z-295, R³ is CH₃ and Q is Q1, and R and R⁴ are as per any row of Table U below, which compounds are named I.A1-Z295-Q1-U-1 to I.A1-Z295-Q1-U-42.

In another embodiment, compounds I are of formula I.A1 wherein Z is Z-295, R³ is CH₃ and Q is Q2, and R and R⁴ are as per any row of Table U below, which compounds are named I.A1-Z295-Q2-U-1 to I.A1-Z295-Q2-U-42.

In another embodiment, compounds I are of formula I.A1 wherein Z is Z-295, R³ is CH₃ and Q is Q3, and R and R⁴ are as per any row of Table U below, which compounds are named I.A1-Z295-Q3-U-1 to I.A1-Z295-Q3-U-42.

In another embodiment, compounds I are of formula I.A1 wherein Z is Z-295, R³ is CH₃ and Q is Q4, and R and R⁴ are as per any row of Table U below, which compounds are named I.A1-Z295-Q4-U-1 to I.A1-Z295-Q4-U-42.

In another embodiment, compounds I are of formula I.A2 wherein Z is Z-295, R³ is CH₃ and Q is Q1, and R and R⁴ are as per any row of Table U below, which compounds are named I.A2-Z295-Q1-U-1 to I.A2-Z295-Q1-U-42.

In another embodiment, compounds I are of formula I.A2 wherein Z is Z-295, R³ is CH₃ and Q is Q2, and R and R⁴ are as per any row of Table U below, which compounds are named I.A2-Z295-Q2-U-1 to I.A2-Z295-Q2-U-42.

In another embodiment, compounds I are of formula I.A2 wherein Z is Z-295, R³ is CH₃ and Q is Q3, and R and R⁴ are as per any row of Table U below, which compounds are named I.A2-Z295-Q3-U-1 to I.A2-Z295-Q3-U-42.

In another embodiment, compounds I are of formula I.A2 wherein Z is Z-295, R³ is CH₃ and Q is Q4, and R and R⁴ are as per any row of Table U below, which compounds are named I.A2-Z295-Q4-U-1 to I.A2-Z295-Q4-U-42.

In another embodiment, compounds I are of formula I.A1 wherein Z is Z-296, R³ is CH₃ and Q is Q1, and R and R⁴ are as per any row of Table U below, which compounds are named I.A1-Z296-Q1-U-1 to I.A1-Z296-Q1-U-42.

In another embodiment, compounds I are of formula I.A1 wherein Z is Z-296, R³ is CH₃ and Q is Q2, and R and R⁴ are as per any row of Table U below, which compounds are named I.A1-Z296-Q2-U-1 to I.A1-Z296-Q2-U-42.

In another embodiment, compounds I are of formula I.A1 wherein Z is Z-296, R³ is CH₃ and Q is Q3, and R and R⁴ are as per any row of Table U below, which compounds are named I.A1-Z296-Q3-U-1 to I.A1-Z296-Q3-U-42.

In another embodiment, compounds I are of formula I.A1 wherein Z is Z-296, R³ is CH₃ and Q is Q4, and R and R⁴ are as per any row of Table U below, which compounds are named I.A1-Z296-Q4-U-1 to I.A1-Z296-Q4-U-42.

In another embodiment, compounds I are of formula I.A2 wherein Z is Z-296, R³ is CH₃ and Q is Q1, and R and R⁴ are as per any row of Table U below, which compounds are named I.A2-Z296-Q1-U-1 to I.A2-Z296-Q1-U-42.

In another embodiment, compounds I are of formula I.A2 wherein Z is Z-296, R³ is CH₃ and Q is Q2, and R and R⁴ are as per any row of Table U below, which compounds are named I.A2-Z296-Q2-U-1 to I.A2-Z296-Q2-U-42.

In another embodiment, compounds I are of formula I.A2 wherein Z is Z-296, R³ is CH₃ and Q is Q3, and R and R⁴ are as per any row of Table U below, which compounds are named I.A2-Z296-Q3-U-1 to I.A2-Z296-Q3-U-42.

In another embodiment, compounds I are of formula I.A2 wherein Z is Z-296, R³ is CH₃ and Q is Q4, and R and R⁴ are as per any row of Table U below, which compounds are named I.A2-Z296-Q4-U-1 to I.A2-Z296-Q4-U-42.

In another embodiment, compounds I are of formula I.A1 wherein Z is Z-297, R³ is CH₃ and Q is Q1, and R and R⁴ are as per any row of Table U below, which compounds are named I.A1-Z297-Q1-U-1 to I.A1-Z297-Q1-U-42.

In another embodiment, compounds I are of formula I.A1 wherein Z is Z-297, R³ is CH₃ and Q is Q2, and R and R⁴ are as per any row of Table U below, which compounds are named I.A1-Z297-Q2-U-1 to I.A1-Z297-Q2-U-42.

In another embodiment, compounds I are of formula I.A1 wherein Z is Z-297, R³ is CH₃ and Q is Q3, and R and R⁴ are as per any row of Table U below, which compounds are named I.A1-Z297-Q3-U-1 to I.A1-Z297-Q3-U-42.

In another embodiment, compounds I are of formula I.A1 wherein Z is Z-297, R³ is CH₃ and Q is Q4, and R and R⁴ are as per any row of Table U below, which compounds are named I.A1-Z297-Q4-U-1 to I.A1-Z297-Q4-U-42.

In another embodiment, compounds I are of formula I.A2 wherein Z is Z-297, R³ is CH₃ and Q is Q1, and R and R⁴ are as per any row of Table U below, which compounds are named I.A2-Z297-Q1-U-1 to I.A2-Z297-Q1-U-42.

In another embodiment, compounds I are of formula I.A2 wherein Z is Z-297, R³ is CH₃ and Q is Q2, and R and R⁴ are as per any row of Table U below, which compounds are named I.A2-Z297-Q2-U-1 to I.A2-Z297-Q2-U-42.

In another embodiment, compounds I are of formula I.A2 wherein Z is Z-297, R³ is CH₃ and Q is Q3, and R and R⁴ are as per any row of Table U below, which compounds are named I.A2-Z297-Q3-U-1 to I.A2-Z297-Q3-U-42.

In another embodiment, compounds I are of formula I.A2 wherein Z is Z-297, R³ is CH₃ and Q is Q4, and R and R⁴ are as per any row of Table U below, which compounds are named I.A2-Z297-Q4-U-1 to I.A2-Z297-Q4-U-42.

In another embodiment, compounds I are of formula I.A1 wherein Z is Z-298, R³ is CH₃ and Q is Q1, and R and R⁴ are as per any row of Table U below, which compounds are named I.A1-Z298-Q1-U-1 to I.A1-Z298-Q1-U-42.

In another embodiment, compounds I are of formula I.A1 wherein Z is Z-298, R³ is CH₃ and Q is Q2, and R and R⁴ are as per any row of Table U below, which compounds are named I.A1-Z298-Q2-U-1 to I.A1-Z298-Q2-U-42.

In another embodiment, compounds I are of formula I.A1 wherein Z is Z-298, R³ is CH₃ and Q is Q3, and R and R⁴ are as per any row of Table U below, which compounds are named I.A1-Z298-Q3-U-1 to I.A1-Z298-Q3-U-42.

In another embodiment, compounds I are of formula I.A1 wherein Z is Z-298, R³ is CH₃ and Q is Q4, and R and R⁴ are as per any row of Table U below, which compounds are named I.A1-Z298-Q4-U-1 to I.A1-Z298-Q4-U-42.

In another embodiment, compounds I are of formula I.A2 wherein Z is Z-298, R³ is CH₃ and Q is Q1, and R and R⁴ are as per any row of Table U below, which compounds are named I.A2-Z298-Q1-U-1 to I.A2-Z298-Q1-U-42.

In another embodiment, compounds I are of formula I.A2 wherein Z is Z-298, R³ is CH₃ and Q is Q2, and R and R⁴ are as per any row of Table U below, which compounds are named I.A2-Z298-Q2-U-1 to I.A2-Z298-Q2-U-42.

In another embodiment, compounds I are of formula I.A2 wherein Z is Z-298, R³ is CH₃ and Q is Q3, and R and R⁴ are as per any row of Table U below, which compounds are named I.A2-Z298-Q3-U-1 to I.A2-Z298-Q3-U-42.

In another embodiment, compounds I are of formula I.A2 wherein Z is Z-298, R³ is CH₃ and Q is Q4, and R and R⁴ are as per any row of Table U below, which compounds are named I.A2-Z298-Q4-U-1 to I.A2-Z298-Q4-U-42.

In another embodiment, compounds I are of formula I.A1 wherein Z is Z-299, R³ is CH₃ and Q is Q1, and R and R⁴ are as per any row of Table U below, which compounds are named I.A1-Z299-Q1-U-1 to I.A1-Z299-Q1-U-42.

In another embodiment, compounds I are of formula I.A1 wherein Z is Z-299, R³ is CH₃ and Q is Q2, and R and R⁴ are as per any row of Table U below, which compounds are named I.A1-Z299-Q2-U-1 to I.A1-Z299-Q2-U-42.

In another embodiment, compounds I are of formula I.A1 wherein Z is Z-299, R³ is CH₃ and Q is Q3, and R and R⁴ are as per any row of Table U below, which compounds are named I.A1-Z299-Q3-U-1 to I.A1-Z299-Q3-U-42.

In another embodiment, compounds I are of formula I.A1 wherein Z is Z-299, R³ is CH₃ and Q is Q4, and R and R⁴ are as per any row of Table U below, which compounds are named I.A1-Z299-Q4-U-1 to I.A1-Z299-Q4-U-42.

In another embodiment, compounds I are of formula I.A2 wherein Z is Z-299, R³ is CH₃ and Q is Q1, and R and R⁴ are as per any row of Table U below, which compounds are named I.A2-Z299-Q1-U-1 to I.A2-Z299-Q1-U-42.

In another embodiment, compounds I are of formula I.A2 wherein Z is Z-299, R³ is CH₃ and Q is Q2, and R and R⁴ are as per any row of Table U below, which compounds are named I.A2-Z299-Q2-U-1 to I.A2-Z299-Q2-U-42.

In another embodiment, compounds I are of formula I.A2 wherein Z is Z-299, R³ is CH₃ and Q is Q3, and R and R⁴ are as per any row of Table U below, which compounds are named I.A2-Z299-Q3-U-1 to I.A2-Z299-Q3-U-42.

In another embodiment, compounds I are of formula I.A2 wherein Z is Z-299, R³ is CH₃ and Q is Q4, and R and R⁴ are as per any row of Table U below, which compounds are named I.A2-Z299-Q4-U-1 to I.A2-Z299-Q4-U-42.

In another embodiment, compounds I are of formula I.A1 wherein Z is Z-300, R³ is CH₃ and Q is Q1, and R and R⁴ are as per any row of Table U below, which compounds are named I.A1-Z300-Q1-U-1 to I.A1-Z300-Q1-U-42.

In another embodiment, compounds I are of formula I.A1 wherein Z is Z-300, R³ is CH₃ and Q is Q2, and R and R⁴ are as per any row of Table U below, which compounds are named I.A1-Z300-Q2-U-1 to I,A1 -Z300-Q2-U-42.

In another embodiment, compounds I are of formula I.A1 wherein Z is Z-300, R³ is CH₃ and Q is Q3, and R and R⁴ are as per any row of Table U below, which compounds are named I.A1-Z300-Q3-U-1 to I.A1-Z300-Q3-U-42.

In another embodiment, compounds I are of formula I.A1 wherein Z is Z-300, R³ is CH₃ and Q is Q4, and R and R⁴ are as per any row of Table U below, which compounds are named I.A1-Z300-Q4-U-1 to I.A1-Z300-Q4-U-42.

In another embodiment, compounds I are of formula I.A2 wherein Z is Z-300, R³ is CH₃ and Q is Q1, and R and R⁴ are as per any row of Table U below, which compounds are named I.A2-Z300-Q1-U-1 to LA2-Z300-Q1-U-42.

In another embodiment, compounds I are of formula I.A2 wherein Z is Z-300, R³ is CH₃ and Q is Q2, and R and R⁴ are as per any row of Table U below, which compounds are named I.A2-Z300-Q2-U-1 to I.A2-Z300-Q2-U-42.

In another embodiment, compounds I are of formula I.A2 wherein Z is Z-300, R³ is CH₃ and Q is Q3, and R and R⁴ are as per any row of Table U below, which compounds are named I.A2-Z300-Q3-U-1 to I.A2-Z300-Q3-U-42.

In another embodiment, compounds I are of formula I.A2 wherein Z is Z-300, R³ is CH₃ and Q is Q4, and R and R⁴ are as per any row of Table U below, which compounds are named I.A2-Z300-Q4-U-1 to I.A2-Z300-Q4-U-42.

In another embodiment, compounds I are of formula I.A1 wherein Z is Z-301, R³ is CH₃ and Q is Q1, and R and R⁴ are as per any row of Table U below, which compounds are named I.A1-Z301-Q1-U-1 to I.A1-Z301-Q1-U-42.

In another embodiment, compounds I are of formula I.A1 wherein Z is Z-301, R³ is CH₃ and Q is Q2, and R and R⁴ are as per any row of Table U below, which compounds are named I.A1-Z301-Q2-U-1 to I.A1-Z301-Q2-U-42.

In another embodiment, compounds I are of formula I.A1 wherein Z is Z-301, R³ is CH₃ and Q is Q3, and R and R⁴ are as per any row of Table U below, which compounds are named I.A1-Z301-Q3-U-1 to I.A1-Z301-Q3-U-42.

In another embodiment, compounds I are of formula I.A1 wherein Z is Z-301, R³ is CH₃ and Q is Q4, and R and R⁴ are as per any row of Table U below, which compounds are named I.A1-Z301-Q4-U-1 to I.A1-Z301-Q4-U-42.

In another embodiment, compounds I are of formula I.A2 wherein Z is Z-301, R³ is CH₃ and Q is Q1, and R and R⁴ are as per any row of Table U below, which compounds are named I.A2-Z301-Q1-U-1 to I.A2-Z301-Q1-U-42.

In another embodiment, compounds I are of formula I.A2 wherein Z is Z-301, R³ is CH₃ and Q is Q2, and R and R⁴ are as per any row of Table U below, which compounds are named I.A2-Z301-Q2-U-1 to I.A2-Z301-Q2-U-42.

In another embodiment, compounds I are of formula I.A2 wherein Z is Z-301, R³ is CH₃ and Q is Q3, and R and R⁴ are as per any row of Table U below, which compounds are named I.A2-Z301-Q3-U-1 to I.A2-Z301-Q3-U-42.

In another embodiment, compounds I are of formula I.A2 wherein Z is Z-301, R³ is CH₃ and Q is Q4, and R and R⁴ are as per any row of Table U below, which compounds are named I.A2-Z301-Q4-U-1 to I.A2-Z301-Q4-U-42.

In another embodiment, compounds I are of formula I.A1 wherein Z is Z-302, R³ is CH₃ and Q is Q1, and R and R⁴ are as per any row of Table U below, which compounds are named I.A1-Z302-Q1-U-1 to I.A1-Z302-Q1-U-42.

In another embodiment, compounds I are of formula I.A1 wherein Z is Z-302, R³ is CH₃ and Q is Q2, and R and R⁴ are as per any row of Table U below, which compounds are named I.A1-Z302-Q2-U-1 to I.A1-Z302-Q2-U-42.

In another embodiment, compounds I are of formula I.A1 wherein Z is Z-302, R³ is CH₃ and Q is Q3, and R and R⁴ are as per any row of Table U below, which compounds are named I.A1-Z302-Q3-U-1 to I.A1-Z302-Q3-U-42.

In another embodiment, compounds I are of formula I.A1 wherein Z is Z-302, R³ is CH₃ and Q is Q4, and R and R⁴ are as per any row of Table U below, which compounds are named I.A1-Z302-Q4-U-1 to I.A1-Z302-Q4-U-42.

In another embodiment, compounds I are of formula I.A2 wherein Z is Z-302, R³ is CH₃ and Q is Q1, and R and R⁴ are as per any row of Table U below, which compounds are named I.A2-Z302-Q1-U-1 to I.A2-Z302-Q1-U-42.

In another embodiment, compounds I are of formula I.A2 wherein Z is Z-302, R³ is CH₃ and Q is Q2, and R and R⁴ are as per any row of Table U below, which compounds are named I.A2-Z302-Q2-U-1 to I.A2-Z302-Q2-U-42.

In another embodiment, compounds I are of formula I.A2 wherein Z is Z-302, R³ is CH₃ and Q is Q3, and R and R⁴ are as per any row of Table U below, which compounds are named I.A2-Z302-Q3-U-1 to I.A2-Z302-Q3-U-42.

In another embodiment, compounds I are of formula I.A2 wherein Z is Z-302, R³ is CH₃ and Q is Q4, and R and R⁴ are as per any row of Table U below, which compounds are named I.A2-Z302-Q4-U-1 to I.A2-Z302-Q4-U-42.

In another embodiment, compounds I are of formula I.A1 wherein Z is Z-303, R³ is CH₃ and Q is Q1, and R and R⁴ are as per any row of Table U below, which compounds are named I.A1-Z303-Q1-U-1 to I.A1-Z303-Q1-U-42.

In another embodiment, compounds I are of formula I.A1 wherein Z is Z-303, R³ is CH₃ and Q is Q2, and R and R⁴ are as per any row of Table U below, which compounds are named I.A1-Z303-Q2-U-1 to I.A1-Z303-Q2-U-42.

In another embodiment, compounds I are of formula I.A1 wherein Z is Z-303, R³ is CH₃ and Q is Q3, and R and R⁴ are as per any row of Table U below, which compounds are named I.A1-Z303-Q3-U-1 to I.A1-Z303-Q3-U-42.

In another embodiment, compounds I are of formula I.A1 wherein Z is Z-303, R³ is CH₃ and Q is Q4, and R and R⁴ are as per any row of Table U below, which compounds are named I.A1-Z303-Q4-U-1 to I.A1-Z303-Q4-U-42.

In another embodiment, compounds I are of formula I.A2 wherein Z is Z-303, R³ is CH₃ and Q is Q1, and R and R⁴ are as per any row of Table U below, which compounds are named I.A2-Z303-Q1-U-1 to I.A2-Z303-Q1-U-42.

In another embodiment, compounds I are of formula I.A2 wherein Z is Z-303, R³ is CH₃ and Q is Q2, and R and R⁴ are as per any row of Table U below, which compounds are named I.A2-Z303-Q2-U-1 to I.A2-Z303-Q2-U-42.

In another embodiment, compounds I are of formula I.A2 wherein Z is Z-303, R³ is CH₃ and Q is Q3, and R and R⁴ are as per any row of Table U below, which compounds are named I.A2-Z303-Q3-U-1 to I.A2-Z303-Q3-U-42.

In another embodiment, compounds I are of formula I.A2 wherein Z is Z-303, R³ is CH₃ and Q is Q4, and R and R⁴ are as per any row of Table U below, which compounds are named I.A2-Z303-Q4-U-1 to I.A2-Z303-Q4-U-42.

In another embodiment, compounds I are of formula I.A1 wherein Z is Z-304, R³ is CH₃ and Q is Q1, and R and R⁴ are as per any row of Table U below, which compounds are named I.A1-Z304-Q1-U-1 to I.A1-Z304-Q1-U-42.

In another embodiment, compounds I are of formula I.A1 wherein Z is Z-304, R³ is CH₃ and Q is Q2, and R and R⁴ are as per any row of Table U below, which compounds are named I.A1-Z304-Q2-U-1 to I.A1-Z304-Q2-U-42.

In another embodiment, compounds I are of formula I.A1 wherein Z is Z-304, R³ is CH₃ and Q is Q3, and R and R⁴ are as per any row of Table U below, which compounds are named I.A1-Z304-Q3-U-1 to I.A1-Z304-Q3-U-42.

In another embodiment, compounds I are of formula I.A1 wherein Z is Z-304, R³ is CH₃ and Q is Q4, and R and R⁴ are as per any row of Table U below, which compounds are named I.A1-Z304-Q4-U-1 to I.A1-Z304-Q4-U-42.

In another embodiment, compounds I are of formula I.A2 wherein Z is Z-304, R³ is CH₃ and Q is Q1, and R and R⁴ are as per any row of Table U below, which compounds are named I.A2-Z304-Q1-U-1 to I.A2-Z304-Q1-U-42.

In another embodiment, compounds I are of formula I.A2 wherein Z is Z-304, R³ is CH₃ and Q is Q2, and R and R⁴ are as per any row of Table U below, which compounds are named I.A2-Z304-Q2-U-1 to I.A2-Z304-Q2-U-42.

In another embodiment, compounds I are of formula I.A2 wherein Z is Z-304, R³ is CH₃ and Q is Q3, and R and R⁴ are as per any row of Table U below, which compounds are named I.A2-Z304-Q3-U-1 to I.A2-Z304-Q3-U-42.

In another embodiment, compounds I are of formula I.A2 wherein Z is Z-304, R³ is CH₃ and Q is Q4, and R and R⁴ are as per any row of Table U below, which compounds are named I.A2-Z304-Q4-U-1 to I.A2-Z304-Q4-U-42.

In another embodiment, compounds I are of formula I.A1 wherein Z is Z-305, R³ is CH₃ and Q is Q1, and R and R⁴ are as per any row of Table U below, which compounds are named I.A1-Z305-Q1-U-1 to I.A1-Z305-Q1-U-42.

In another embodiment, compounds I are of formula I.A1 wherein Z is Z-305, R³ is CH₃ and Q is Q2, and R and R⁴ are as per any row of Table U below, which compounds are named I.A1-Z305-Q2-U-1 to I.A1-Z305-Q2-U-42.

In another embodiment, compounds I are of formula I.A1 wherein Z is Z-305, R³ is CH₃ and Q is Q3, and R and R⁴ are as per any row of Table U below, which compounds are named I.A1-Z305-Q3-U-1 to I.A1-Z305-Q3-U-42.

In another embodiment, compounds I are of formula I.A1 wherein Z is Z-305, R³ is CH₃ and Q is Q4, and R and R⁴ are as per any row of Table U below, which compounds are named I.A1-Z305-Q4-U-1 to I.A1-Z305-Q4-U-42.

In another embodiment, compounds I are of formula I.A2 wherein Z is Z-305, R³ is CH₃ and Q is Q1, and R and R⁴ are as per any row of Table U below, which compounds are named I.A2-Z305-Q1-U-1 to I.A2-Z305-Q1-U-42.

In another embodiment, compounds I are of formula I.A2 wherein Z is Z-305, R³ is CH₃ and Q is Q2, and R and R⁴ are as per any row of Table U below, which compounds are named I.A2-Z305-Q2-U-1 to I.A2-Z305-Q2-U-42.

In another embodiment, compounds I are of formula I.A2 wherein Z is Z-305, R³ is CH₃ and Q is Q3, and R and R⁴ are as per any row of Table U below, which compounds are named I.A2-Z305-Q3-U-1 to I.A2-Z305-Q3-U-42.

In another embodiment, compounds I are of formula I.A2 wherein Z is Z-305, R³ is CH₃ and Q is Q4, and R and R⁴ are as per any row of Table U below, which compounds are named I.A2-Z305-Q4-U-1 to I.A2-Z305-Q4-U-42.

In another embodiment, compounds I are of formula I.A1 wherein Z is Z-306, R³ is CH₃ and Q is Q1, and R and R⁴ are as per any row of Table U below, which compounds are named I.A1-Z306-Q1-U-1 to I.A1-Z306-Q1-U-42.

In another embodiment, compounds I are of formula I.A1 wherein Z is Z-306, R³ is CH₃ and Q is Q2, and R and R⁴ are as per any row of Table U below, which compounds are named I.A1-Z306-Q2-U-1 to I.A1-Z306-Q2-U-42.

In another embodiment, compounds I are of formula I.A1 wherein Z is Z-306, R³ is CH₃ and Q is Q3, and R and R⁴ are as per any row of Table U below, which compounds are named I.A1-Z306-Q3-U-1 to I.A1-Z306-Q3-U-42.

In another embodiment, compounds I are of formula I.A1 wherein Z is Z-306, R³ is CH₃ and Q is Q4, and R and R⁴ are as per any row of Table U below, which compounds are named I.A1-Z306-Q4-U-1 to I.A1-Z306-Q4-U-42.

In another embodiment, compounds I are of formula I.A2 wherein Z is Z-306, R³ is CH₃ and Q is Q1, and R and R⁴ are as per any row of Table U below, which compounds are named I.A2-Z306-Q1-U-1 to I.A2-Z306-Q1-U-42.

In another embodiment, compounds I are of formula I.A2 wherein Z is Z-306, R³ is CH₃ and Q is Q2, and R and R⁴ are as per any row of Table U below, which compounds are named I.A2-Z306-Q2-U-1 to I.A2-Z306-Q2-U-42.

In another embodiment, compounds I are of formula I.A2 wherein Z is Z-306, R³ is CH₃ and Q is Q3, and R and R⁴ are as per any row of Table U below, which compounds are named I.A2-Z306-Q3-U-1 to I.A2-Z306-Q3-U-42.

In another embodiment, compounds I are of formula I.A2 wherein Z is Z-306, R³ is CH₃ and Q is Q4, and R and R⁴ are as per any row of Table U below, which compounds are named I.A2-Z306-Q4-U-1 to I.A2-Z306-Q4-U-42.

In another embodiment, compounds I are of formula I.A1 wherein Z is Z-307, R³ is CH₃ and Q is Q1, and R and R⁴ are as per any row of Table U below, which compounds are named I.A1-Z307-Q1-U-1 to I.A1-Z307-Q1-U-42.

In another embodiment, compounds I are of formula I.A1 wherein Z is Z-307, R³ is CH₃ and Q is Q2, and R and R⁴ are as per any row of Table U below, which compounds are named I.A1-Z307-Q2-U-1 to I.A1-Z307-Q2-U-42.

In another embodiment, compounds I are of formula I.A1 wherein Z is Z-307, R³ is CH₃ and Q is Q3, and R and R⁴ are as per any row of Table U below, which compounds are named I.A1-Z307-Q3-U-1 to I.A1-Z307-Q3-U-42.

In another embodiment, compounds I are of formula I.A1 wherein Z is Z-307, R³ is CH₃ and Q is Q4, and R and R⁴ are as per any row of Table U below, which compounds are named I.A1-Z307-Q4-U-1 to I.A1-Z307-Q4-U-42.

In another embodiment, compounds I are of formula I.A2 wherein Z is Z-307, R³ is CH₃ and Q is Q1, and R and R⁴ are as per any row of Table U below, which compounds are named I.A2-Z307-Q1-U-1 to I.A2-Z307-Q1-U-42.

In another embodiment, compounds I are of formula I.A2 wherein Z is Z-307, R³ is CH₃ and Q is Q2, and R and R⁴ are as per any row of Table U below, which compounds are named I.A2-Z307-Q2-U-1 to I.A2-Z307-Q2-U-42.

In another embodiment, compounds I are of formula I.A2 wherein Z is Z-307, R³ is CH₃ and Q is Q3, and R and R⁴ are as per any row of Table U below, which compounds are named I.A2-Z307-Q3-U-1 to I.A2-Z307-Q3-U-42.

In another embodiment, compounds I are of formula I.A2 wherein Z is Z-307, R³ is CH₃ and Q is Q4, and R and R⁴ are as per any row of Table U below, which compounds are named I.A2-Z307-Q4-U-1 to I.A2-Z307-Q4-U-42.

In another embodiment, compounds I are of formula I.A1 wherein Z is Z-308, R³ is CH₃ and Q is Q1, and R and R⁴ are as per any row of Table U below, which compounds are named I.A1-Z308-Q1-U-1 to I.A1-Z308-Q1-U-42.

In another embodiment, compounds I are of formula I.A1 wherein Z is Z-308, R³ is CH₃ and Q is Q2, and R and R⁴ are as per any row of Table U below, which compounds are named I.A1-Z308-Q2-U-1 to I.A1-Z308-Q2-U-42.

In another embodiment, compounds I are of formula I.A1 wherein Z is Z-308, R³ is CH₃ and Q is Q3, and R and R⁴ are as per any row of Table U below, which compounds are named I.A1-Z308-Q3-U-1 to I.A1-Z308-Q3-U-42.

In another embodiment, compounds I are of formula I.A1 wherein Z is Z-308, R³ is CH₃ and Q is Q4, and R and R⁴ are as per any row of Table U below, which compounds are named I.A1-Z308-Q4-U-1 to I.A1-Z308-Q4-U-42.

In another embodiment, compounds I are of formula I.A2 wherein Z is Z-308, R³ is CH₃ and Q is Q1, and R and R⁴ are as per any row of Table U below, which compounds are named I.A2-Z308-Q1-U-1 to I.A2-Z308-Q1-U-42.

In another embodiment, compounds I are of formula I.A2 wherein Z is Z-308, R³ is CH₃ and Q is Q2, and R and R⁴ are as per any row of Table U below, which compounds are named I.A2-Z308-Q2-U-1 to I.A2-Z308-Q2-U-42.

In another embodiment, compounds I are of formula I.A2 wherein Z is Z-308, R³ is CH₃ and Q is Q3, and R and R⁴ are as per any row of Table U below, which compounds are named I.A2-Z308-Q3-U-1 to I.A2-Z308-Q3-U-42.

In another embodiment, compounds I are of formula I.A2 wherein Z is Z-308, R³ is CH₃ and Q is Q4, and R and R⁴ are as per any row of Table U below, which compounds are named I.A2-Z308-Q4-U-1 to I.A2-Z308-Q4-U-42.

In another embodiment, compounds I are of formula I.A1 wherein Z is Z-309, R³ is CH₃ and Q is Q1, and R and R⁴ are as per any row of Table U below, which compounds are named I.A1-Z309-Q1-U-1 to I.A1-Z309-Q1-U-42.

In another embodiment, compounds I are of formula I.A1 wherein Z is Z-309, R³ is CH₃ and Q is Q2, and R and R⁴ are as per any row of Table U below, which compounds are named I.A1-Z309-Q2-U-1 to I.A1-Z309-Q2-U-42.

In another embodiment, compounds I are of formula I.A1 wherein Z is Z-309, R³ is CH₃ and Q is Q3, and R and R⁴ are as per any row of Table U below, which compounds are named I.A1-Z309-Q3-U-1 to I.A1-Z309-Q3-U-42.

In another embodiment, compounds I are of formula I.A1 wherein Z is Z-309, R³ is CH₃ and Q is Q4, and R and R⁴ are as per any row of Table U below, which compounds are named I.A1-Z309-Q4-U-1 to I.A1-Z309-Q4-U-42.

In another embodiment, compounds I are of formula I.A2 wherein Z is Z-309, R³ is CH₃ and Q is Q1, and R and R⁴ are as per any row of Table U below, which compounds are named I.A2-Z309-Q1-U-1 to I.A2-Z309-Q1-U-42.

In another embodiment, compounds I are of formula I.A2 wherein Z is Z-309, R³ is CH₃ and Q is Q2, and R and R⁴ are as per any row of Table U below, which compounds are named I.A2-Z309-Q2-U-1 to I.A2-Z309-Q2-U-42.

In another embodiment, compounds I are of formula I.A2 wherein Z is Z-309, R³ is CH₃ and Q is Q3, and R and R⁴ are as per any row of Table U below, which compounds are named I.A2-Z309-Q3-U-1 to I.A2-Z309-Q3-U-42.

In another embodiment, compounds I are of formula I.A2 wherein Z is Z-309, R³ is CH₃ and Q is Q4, and R and R⁴ are as per any row of Table U below, which compounds are named I.A2-Z309-Q4-U-1 to I.A2-Z309-Q4-U-42.

In another embodiment, compounds I are of formula I.A1 wherein Z is Z-310, R³ is CH₃ and Q is Q1, and R and R⁴ are as per any row of Table U below, which compounds are named I.A1-Z310-Q1-U-1 to I.A1-Z310-Q1-U-42.

In another embodiment, compounds I are of formula I.A1 wherein Z is Z-310, R³ is CH₃ and Q is Q2, and R and R⁴ are as per any row of Table U below, which compounds are named I.A1-Z310-Q2-U-1 to I.A1-Z310-Q2-U-42.

In another embodiment, compounds I are of formula I.A1 wherein Z is Z-310, R³ is CH₃ and Q is Q3, and R and R⁴ are as per any row of Table U below, which compounds are named I.A1-Z310-Q3-U-1 to I.A1-Z310-Q3-U-42.

In another embodiment, compounds I are of formula I.A1 wherein Z is Z-310, R³ is CH₃ and Q is Q4, and R and R⁴ are as per any row of Table U below, which compounds are named I.A1-Z310-Q4-U-1 to I.A1-Z310-Q4-U-42.

In another embodiment, compounds I are of formula I.A2 wherein Z is Z-310, R³ is CH₃ and Q is Q1, and R and R⁴ are as per any row of Table U below, which compounds are named I.A2-Z310-Q1-U-1 to LA2-Z310-Q1-U-42.

In another embodiment, compounds I are of formula I.A2 wherein Z is Z-310, R³ is CH₃ and Q is Q2, and R and R⁴ are as per any row of Table U below, which compounds are named I.A2-Z310-Q2-U-1 to I.A2-Z310-Q2-U-42.

In another embodiment, compounds I are of formula I.A2 wherein Z is Z-310, R³ is CH₃ and Q is Q3, and R and R⁴ are as per any row of Table U below, which compounds are named I.A2-Z310-Q3-U-1 to I.A2-Z310-Q3-U-42.

In another embodiment, compounds I are of formula I.A2 wherein Z is Z-310, R³ is CH₃ and Q is Q4, and R and R⁴ are as per any row of Table U below, which compounds are named I.A2-Z310-Q4-U-1 to I.A2-Z310-Q4-U-42.

In another embodiment, compounds I are of formula I.A1 wherein Z is Z-311, R³ is CH₃ and Q is Q1, and R and R⁴ are as per any row of Table U below, which compounds are named I.A1-Z311-Q1-U-1 to I.A1-Z311-Q1-U-42.

In another embodiment, compounds I are of formula I.A1 wherein Z is Z-311, R³ is CH₃ and Q is Q2, and R and R⁴ are as per any row of Table U below, which compounds are named I.A1-Z311-Q2-U-1 to I.A1-Z311-Q2-U-42.

In another embodiment, compounds I are of formula I.A1 wherein Z is Z-311, R³ is CH₃ and Q is Q3, and R and R⁴ are as per any row of Table U below, which compounds are named I.A1-Z311-Q3-U-1 to I.A1-Z311-Q3-U-42.

In another embodiment, compounds I are of formula I.A1 wherein Z is Z-311, R³ is CH₃ and Q is Q4, and R and R⁴ are as per any row of Table U below, which compounds are named I.A1-Z311-Q4-U-1 to I.A1-Z311-Q4-U-42.

In another embodiment, compounds I are of formula I.A2 wherein Z is Z-311, R³ is CH₃ and Q is Q1, and R and R⁴ are as per any row of Table U below, which compounds are named LA2-Z311-Q1-U-1 to I.A2-Z311-Q1-U-42.

In another embodiment, compounds I are of formula I.A2 wherein Z is Z-311, R³ is CH₃ and Q is Q2, and R and R⁴ are as per any row of Table U below, which compounds are named I.A2-Z311-Q2-U-1 to I.A2-Z311-Q2-U-42.

In another embodiment, compounds I are of formula I.A2 wherein Z is Z-311, R³ is CH₃ and Q is Q3, and R and R⁴ are as per any row of Table U below, which compounds are named I.A2-Z311-Q3-U-1 to I.A2-Z311-Q3-U-42.

In another embodiment, compounds I are of formula I.A2 wherein Z is Z-311, R³ is CH₃ and Q is Q4, and R and R⁴ are as per any row of Table U below, which compounds are named I.A2-Z311-Q4-U-1 to I.A2-Z311-Q4-U-42.

In another embodiment, compounds I are of formula I.A1 wherein Z is Z-312, R³ is CH₃ and Q is Q1, and R and R⁴ are as per any row of Table U below, which compounds are named I.A1-Z312-Q1-U-1 to I.A1-Z312-Q1-U-42.

In another embodiment, compounds I are of formula I.A1 wherein Z is Z-312, R³ is CH₃ and Q is Q2, and R and R⁴ are as per any row of Table U below, which compounds are named I.A1-Z312-Q2-U-1 to I.A1-Z312-Q2-U-42.

In another embodiment, compounds I are of formula I.A1 wherein Z is Z-312, R³ is CH₃ and Q is Q3, and R and R⁴ are as per any row of Table U below, which compounds are named I.A1-Z312-Q3-U-1 to I.A1-Z312-Q3-U-42.

In another embodiment, compounds I are of formula I.A1 wherein Z is Z-312, R³ is CH₃ and Q is Q4, and R and R⁴ are as per any row of Table U below, which compounds are named I.A1-Z312-Q4-U-1 to I.A1-Z312-Q4-U-42.

In another embodiment, compounds I are of formula I.A2 wherein Z is Z-312, R³ is CH₃ and Q is Q1, and R and R⁴ are as per any row of Table U below, which compounds are named I.A2-Z312-Q1-U-1 to LA2-Z312-Q1-U-42.

In another embodiment, compounds I are of formula I.A2 wherein Z is Z-312, R³ is CH₃ and Q is Q2, and R and R⁴ are as per any row of Table U below, which compounds are named I.A2-Z312-Q2-U-1 to I.A2-Z312-Q2-U-42.

In another embodiment, compounds I are of formula I.A2 wherein Z is Z-312, R³ is CH₃ and Q is Q3, and R and R⁴ are as per any row of Table U below, which compounds are named I.A2-Z312-Q3-U-1 to I.A2-Z312-Q3-U-42.

In another embodiment, compounds I are of formula I.A2 wherein Z is Z-312, R³ is CH₃ and Q is Q4, and R and R⁴ are as per any row of Table U below, which compounds are named I.A2-Z312-Q4-U-1 to I.A2-Z312-Q4-U-42.

In another embodiment, compounds I are of formula I.A1 wherein Z is Z-313, R³ is CH₃ and Q is Q1, and R and R⁴ are as per any row of Table U below, which compounds are named I.A1-Z313-Q1-U-1 to I.A1-Z313-Q1-U-42.

In another embodiment, compounds I are of formula I.A1 wherein Z is Z-313, R³ is CH₃ and Q is Q2, and R and R⁴ are as per any row of Table U below, which compounds are named I.A1-Z313-Q2-U-1 to I.A1-Z313-Q2-U-42.

In another embodiment, compounds I are of formula I.A1 wherein Z is Z-313, R³ is CH₃ and Q is Q3, and R and R⁴ are as per any row of Table U below, which compounds are named I.A1-Z313-Q3-U-1 to I.A1-Z313-Q3-U-42.

In another embodiment, compounds I are of formula I.A1 wherein Z is Z-313, R³ is CH₃ and Q is Q4, and R and R⁴ are as per any row of Table U below, which compounds are named I.A1-Z313-Q4-U-1 to I.A1-Z313-Q4-U-42.

In another embodiment, compounds I are of formula I.A2 wherein Z is Z-313, R³ is CH₃ and Q is Q1, and R and R⁴ are as per any row of Table U below, which compounds are named I.A2-Z313-Q1-U-1 to I.A2-Z313-Q1-U-42.

In another embodiment, compounds I are of formula I.A2 wherein Z is Z-313, R³ is CH₃ and Q is Q2, and R and R⁴ are as per any row of Table U below, which compounds are named I.A2-Z313-Q2-U-1 to I.A2-Z313-Q2-U-42.

In another embodiment, compounds I are of formula I.A2 wherein Z is Z-313, R³ is CH₃ and Q is Q3, and R and R⁴ are as per any row of Table U below, which compounds are named I.A2-Z313-Q3-U-1 to I.A2-Z313-Q3-U-42.

In another embodiment, compounds I are of formula I.A2 wherein Z is Z-313, R³ is CH₃ and Q is Q4, and R and R⁴ are as per any row of Table U below, which compounds are named I.A2-Z313-Q4-U-1 to I.A2-Z313-Q4-U-42.

In another embodiment, compounds I are of formula I.A1 wherein Z is Z-314, R³ is CH₃ and Q is Q1, and R and R⁴ are as per any row of Table U below, which compounds are named I.A1-Z314-Q1-U-1 to I.A1-Z314-Q1-U-42.

In another embodiment, compounds I are of formula I.A1 wherein Z is Z-314, R³ is CH₃ and Q is Q2, and R and R⁴ are as per any row of Table U below, which compounds are named I.A1-Z314-Q2-U-1 to I,A1-Z314-Q2-U-42.

In another embodiment, compounds I are of formula I.A1 wherein Z is Z-314, R³ is CH₃ and Q is Q3, and R and R⁴ are as per any row of Table U below, which compounds are named I.A1-Z314-Q3-U-1 to I.A1-Z314-Q3-U-42.

In another embodiment, compounds I are of formula I.A1 wherein Z is Z-314, R³ is CH₃ and Q is Q4, and R and R⁴ are as per any row of Table U below, which compounds are named I.A1-Z314-Q4-U-1 to I.A1-Z314-Q4-U-42.

In another embodiment, compounds I are of formula I.A2 wherein Z is Z-314, R³ is CH₃ and Q is Q1, and R and R⁴ are as per any row of Table U below, which compounds are named I.A2-Z314-Q1-U-1 to I.A2-Z314-Q1-U-42.

In another embodiment, compounds I are of formula I.A2 wherein Z is Z-314, R³ is CH₃ and Q is Q2, and R and R⁴ are as per any row of Table U below, which compounds are named I.A2-Z314-Q2-U-1 to I.A2-Z314-Q2-U-42.

In another embodiment, compounds I are of formula I.A2 wherein Z is Z-314, R³ is CH₃ and Q is Q3, and R and R⁴ are as per any row of Table U below, which compounds are named I.A2-Z314-Q3-U-1 to I.A2-Z314-Q3-U-42.

In another embodiment, compounds I are of formula I.A2 wherein Z is Z-314, R³ is CH₃ and Q is Q4, and R and R⁴ are as per any row of Table U below, which compounds are named I.A2-Z314-Q4-U-1 to I.A2-Z314-Q4-U-42.

In another embodiment, compounds I are of formula I.A1 wherein Z is Z-315, R³ is CH₃ and Q is Q1, and R and R⁴ are as per any row of Table U below, which compounds are named I.A1-Z315-Q1-U-1 to I.A1-Z315-Q1-U-42.

In another embodiment, compounds I are of formula I.A1 wherein Z is Z-315, R³ is CH₃ and Q is Q2, and R and R⁴ are as per any row of Table U below, which compounds are named I.A1-Z315-Q2-U-1 to I.A1-Z315-Q2-U-42.

In another embodiment, compounds I are of formula I.A1 wherein Z is Z-315, R³ is CH₃ and Q is Q3, and R and R⁴ are as per any row of Table U below, which compounds are named I.A1-Z315-Q3-U-1 to I.A1-Z315-Q3-U-42.

In another embodiment, compounds I are of formula I.A1 wherein Z is Z-315, R³ is CH₃ and Q is Q4, and R and R⁴ are as per any row of Table U below, which compounds are named I.A1-Z315-Q4-U-1 to I.A1-Z315-Q4-U-42.

In another embodiment, compounds I are of formula I.A2 wherein Z is Z-315, R³ is CH₃ and Q is Q1, and R and R⁴ are as per any row of Table U below, which compounds are named I.A2-Z315-Q1-U-1 to I.A2-Z315-Q1-U-42.

In another embodiment, compounds I are of formula I.A2 wherein Z is Z-315, R³ is CH₃ and Q is Q2, and R and R⁴ are as per any row of Table U below, which compounds are named I.A2-Z315-Q2-U-1 to I.A2-Z315-Q2-U-42.

In another embodiment, compounds I are of formula I.A2 wherein Z is Z-315, R³ is CH₃ and Q is Q3, and R and R⁴ are as per any row of Table U below, which compounds are named I.A2-Z315-Q3-U-1 to I.A2-Z315-Q3-U-42.

In another embodiment, compounds I are of formula I.A2 wherein Z is Z-315, R³ is CH₃ and Q is Q4, and R and R⁴ are as per any row of Table U below, which compounds are named I.A2-Z315-Q4-U-1 to I.A2-Z315-Q4-U-42.

In another embodiment, compounds I are of formula I.A1 wherein Z is Z-316, R³ is CH₃ and Q is Q1, and R and R⁴ are as per any row of Table U below, which compounds are named I.A1-Z316-Q1-U-1 to I.A1-Z316-Q1-U-42.

In another embodiment, compounds I are of formula I.A1 wherein Z is Z-316, R³ is CH₃ and Q is Q2, and R and R⁴ are as per any row of Table U below, which compounds are named I.A1-Z316-Q2-U-1 to I.A1-Z316-Q2-U-42.

In another embodiment, compounds I are of formula I.A1 wherein Z is Z-316, R³ is CH₃ and Q is Q3, and R and R⁴ are as per any row of Table U below, which compounds are named I.A1-Z316-Q3-U-1 to I.A1-Z316-Q3-U-42.

In another embodiment, compounds I are of formula I.A1 wherein Z is Z-316, R³ is CH₃ and Q is Q4, and R and R⁴ are as per any row of Table U below, which compounds are named I.A1-Z316-Q4-U-1 to I.A1-Z316-Q4-U-42.

In another embodiment, compounds I are of formula I.A2 wherein Z is Z-316, R³ is CH₃ and Q is Q1, and R and R⁴ are as per any row of Table U below, which compounds are named I.A2-Z316-Q1-U-1 to LA2-Z316-Q1-U-42.

In another embodiment, compounds I are of formula I.A2 wherein Z is Z-316, R³ is CH₃ and Q is Q2, and R and R⁴ are as per any row of Table U below, which compounds are named I.A2-Z316-Q2-U-1 to I.A2-Z316-Q2-U-42.

In another embodiment, compounds I are of formula I.A2 wherein Z is Z-316, R³ is CH₃ and Q is Q3, and R and R⁴ are as per any row of Table U below, which compounds are named I.A2-Z316-Q3-U-1 to I.A2-Z316-Q3-U-42.

In another embodiment, compounds I are of formula I.A2 wherein Z is Z-316, R³ is CH₃ and Q is Q4, and R and R⁴ are as per any row of Table U below, which compounds are named I.A2-Z316-Q4-U-1 to I.A2-Z316-Q4-U-42.

In another embodiment, compounds I are of formula I.A1 wherein Z is Z-317, R³ is CH₃ and Q is Q1, and R and R⁴ are as per any row of Table U below, which compounds are named I.A1-Z317-Q1-U-1 to I.A1-Z317-Q1-U-42.

In another embodiment, compounds I are of formula I.A1 wherein Z is Z-317, R³ is CH₃ and Q is Q2, and R and R⁴ are as per any row of Table U below, which compounds are named I.A1-Z317-Q2-U-1 to I.A1-Z317-Q2-U-42.

In another embodiment, compounds I are of formula I.A1 wherein Z is Z-317, R³ is CH₃ and Q is Q3, and R and R⁴ are as per any row of Table U below, which compounds are named I.A1-Z317-Q3-U-1 to I.A1-Z317-Q3-U-42.

In another embodiment, compounds I are of formula I.A1 wherein Z is Z-317, R³ is CH₃ and Q is Q4, and R and R⁴ are as per any row of Table U below, which compounds are named I.A1-Z317-Q4-U-1 to I.A1-Z317-Q4-U-42.

In another embodiment, compounds I are of formula I.A2 wherein Z is Z-317, R³ is CH₃ and Q is Q1, and R and R⁴ are as per any row of Table U below, which compounds are named I.A2-Z317-Q1-U-1 to I.A2-Z317-Q1-U-42.

In another embodiment, compounds I are of formula I.A2 wherein Z is Z-317, R³ is CH₃ and Q is Q2, and R and R⁴ are as per any row of Table U below, which compounds are named I.A2-Z317-Q2-U-1 to I.A2-Z317-Q2-U-42.

In another embodiment, compounds I are of formula I.A2 wherein Z is Z-317, R³ is CH₃ and Q is Q3, and R and R⁴ are as per any row of Table U below, which compounds are named I.A2-Z317-Q3-U-1 to I.A2-Z317-Q3-U-42.

In another embodiment, compounds I are of formula I.A2 wherein Z is Z-317, R³ is CH₃ and Q is Q4, and R and R⁴ are as per any row of Table U below, which compounds are named I.A2-Z317-Q4-U-1 to I.A2-Z317-Q4-U-42.

In another embodiment, compounds I are of formula I.A1 wherein Z is Z-318, R³ is CH₃ and Q is Q1, and R and R⁴ are as per any row of Table U below, which compounds are named I.A1-Z318-Q1-U-1 to I.A1-Z318-Q1-U-42.

In another embodiment, compounds I are of formula I.A1 wherein Z is Z-318, R³ is CH₃ and Q is Q2, and R and R⁴ are as per any row of Table U below, which compounds are named I.A1-Z318-Q2-U-1 to I.A1-Z318-Q2-U-42.

In another embodiment, compounds I are of formula I.A1 wherein Z is Z-318, R³ is CH₃ and Q is Q3, and R and R⁴ are as per any row of Table U below, which compounds are named I.A1-Z318-Q3-U-1 to I.A1-Z318-Q3-U-42.

In another embodiment, compounds I are of formula I.A1 wherein Z is Z-318, R³ is CH₃ and Q is Q4, and R and R⁴ are as per any row of Table U below, which compounds are named I.A1-Z318-Q4-U-1 to I.A1-Z318-Q4-U-42.

In another embodiment, compounds I are of formula I.A2 wherein Z is Z-318, R³ is CH₃ and Q is Q1, and R and R⁴ are as per any row of Table U below, which compounds are named I.A2-Z318-Q1-U-1 to I.A2-Z318-Q1-U-42.

In another embodiment, compounds I are of formula I.A2 wherein Z is Z-318, R³ is CH₃ and Q is Q2, and R and R⁴ are as per any row of Table U below, which compounds are named I.A2-Z318-Q2-U-1 to I.A2-Z318-Q2-U-42.

In another embodiment, compounds I are of formula I.A2 wherein Z is Z-318, R³ is CH₃ and Q is Q3, and R and R⁴ are as per any row of Table U below, which compounds are named I.A2-Z318-Q3-U-1 to I.A2-Z318-Q3-U-42.

In another embodiment, compounds I are of formula I.A2 wherein Z is Z-318, R³ is CH₃ and Q is Q4, and R and R⁴ are as per any row of Table U below, which compounds are named I.A2-Z318-Q4-U-1 to I.A2-Z318-Q4-U-42.

In another embodiment, compounds I are of formula I.A1 wherein Z is Z-319, R³ is CH₃ and Q is Q1, and R and R⁴ are as per any row of Table U below, which compounds are named I.A1-Z319-Q1-U-1 to I.A1-Z319-Q1-U-42.

In another embodiment, compounds I are of formula I.A1 wherein Z is Z-319, R³ is CH₃ and Q is Q2, and R and R⁴ are as per any row of Table U below, which compounds are named I.A1-Z319-Q2-U-1 to I.A1-Z319-Q2-U-42.

In another embodiment, compounds I are of formula I.A1 wherein Z is Z-319, R³ is CH₃ and Q is Q3, and R and R⁴ are as per any row of Table U below, which compounds are named I.A1-Z319-Q3-U-1 to I.A1-Z319-Q3-U-42.

In another embodiment, compounds I are of formula I.A1 wherein Z is Z-319, R³ is CH₃ and Q is Q4, and R and R⁴ are as per any row of Table U below, which compounds are named I.A1-Z319-Q4-U-1 to I.A1-Z319-Q4-U-42.

In another embodiment, compounds I are of formula I.A2 wherein Z is Z-319, R³ is CH₃ and Q is Q1, and R and R⁴ are as per any row of Table U below, which compounds are named I.A2-Z319-Q1-U-1 to I.A2-Z319-Q1-U-42.

In another embodiment, compounds I are of formula I.A2 wherein Z is Z-319, R³ is CH₃ and Q is Q2, and R and R⁴ are as per any row of Table U below, which compounds are named I.A2-Z319-Q2-U-1 to I.A2-Z319-Q2-U-42.

In another embodiment, compounds I are of formula I.A2 wherein Z is Z-319, R³ is CH₃ and Q is Q3, and R and R⁴ are as per any row of Table U below, which compounds are named I.A2-Z319-Q3-U-1 to I.A2-Z319-Q3-U-42.

In another embodiment, compounds I are of formula I.A2 wherein Z is Z-319, R³ is CH₃ and Q is Q4, and R and R⁴ are as per any row of Table U below, which compounds are named I.A2-Z319-Q4-U-1 to I.A2-Z319-Q4-U-42.

In another embodiment, compounds I are of formula I.A1 wherein Z is Z-320, R³ is CH₃ and Q is Q1, and R and R⁴ are as per any row of Table U below, which compounds are named I.A1-Z320-Q1-U-1 to I.A1-Z320-Q1-U-42.

In another embodiment, compounds I are of formula I.A1 wherein Z is Z-320, R³ is CH₃ and Q is Q2, and R and R⁴ are as per any row of Table U below, which compounds are named I.A1-Z320-Q2-U-1 to I.A1-Z320-Q2-U-42.

In another embodiment, compounds I are of formula I.A1 wherein Z is Z-320, R³ is CH₃ and Q is Q3, and R and R⁴ are as per any row of Table U below, which compounds are named I.A1-Z320-Q3-U-1 to I.A1-Z320-Q3-U-42.

In another embodiment, compounds I are of formula I.A1 wherein Z is Z-320, R³ is CH₃ and Q is Q4, and R and R⁴ are as per any row of Table U below, which compounds are named I.A1-Z320-Q4-U-1 to I.A1-Z320-Q4-U-42.

In another embodiment, compounds I are of formula I.A2 wherein Z is Z-320, R³ is CH₃ and Q is Q1, and R and R⁴ are as per any row of Table U below, which compounds are named I.A2-Z320-Q1-U-1 to I.A2-Z320-Q1-U-42.

In another embodiment, compounds I are of formula I.A2 wherein Z is Z-320, R³ is CH₃ and Q is Q2, and R and R⁴ are as per any row of Table U below, which compounds are named I.A2-Z320-Q2-U-1 to I.A2-Z320-Q2-U-42.

In another embodiment, compounds I are of formula I.A2 wherein Z is Z-320, R³ is CH₃ and Q is Q3, and R and R⁴ are as per any row of Table U below, which compounds are named I.A2-Z320-Q3-U-1 to I.A2-Z320-Q3-U-42.

In another embodiment, compounds I are of formula I.A2 wherein Z is Z-320, R³ is CH₃ and Q is Q4, and R and R⁴ are as per any row of Table U below, which compounds are named I.A2-Z320-Q4-U-1 to I.A2-Z320-Q4-U-42.

In another embodiment, compounds I are of formula I.A1 wherein Z is Z-321, R³ is CH₃ and Q is Q1, and R and R⁴ are as per any row of Table U below, which compounds are named I.A1-Z321-Q1-U-1 to I.A1-Z321-Q1-U-42.

In another embodiment, compounds I are of formula I.A1 wherein Z is Z-321, R³ is CH₃ and Q is Q2, and R and R⁴ are as per any row of Table U below, which compounds are named I.A1-Z321-Q2-U-1 to I,A1-Z321-Q2-U-42.

In another embodiment, compounds I are of formula I.A1 wherein Z is Z-321, R³ is CH₃ and Q is Q3, and R and R⁴ are as per any row of Table U below, which compounds are named I.A1-Z321-Q3-U-1 to I.A1-Z321-Q3-U-42.

In another embodiment, compounds I are of formula I.A1 wherein Z is Z-321, R³ is CH₃ and Q is Q4, and R and R⁴ are as per any row of Table U below, which compounds are named I.A1-Z321-Q4-U-1 to I.A1-Z321-Q4-U-42.

In another embodiment, compounds I are of formula I.A2 wherein Z is Z-321, R³ is CH₃ and Q is Q1, and R and R⁴ are as per any row of Table U below, which compounds are named I.A2-Z321-Q1-U-1 to I.A2-Z321-Q1-U-42.

In another embodiment, compounds I are of formula I.A2 wherein Z is Z-321, R³ is CH₃ and Q is Q2, and R and R⁴ are as per any row of Table U below, which compounds are named I.A2-Z321-Q2-U-1 to I.A2-Z321-Q2-U-42.

In another embodiment, compounds I are of formula I.A2 wherein Z is Z-321, R³ is CH₃ and Q is Q3, and R and R⁴ are as per any row of Table U below, which compounds are named I.A2-Z321-Q3-U-1 to I.A2-Z321-Q3-U-42.

In another embodiment, compounds I are of formula I.A2 wherein Z is Z-321, R³ is CH₃ and Q is Q4, and R and R⁴ are as per any row of Table U below, which compounds are named I.A2-Z321-Q4-U-1 to I.A2-Z321-Q4-U-42.

In another embodiment, compounds I are of formula I.A1 wherein Z is Z-322, R³ is CH₃ and Q is Q1, and R and R⁴ are as per any row of Table U below, which compounds are named I.A1-Z322-Q1-U-1 to I.A1-Z322-Q1-U-42.

In another embodiment, compounds I are of formula I.A1 wherein Z is Z-322, R³ is CH₃ and Q is Q2, and R and R⁴ are as per any row of Table U below, which compounds are named I.A1-Z322-Q2-U-1 to I.A1-Z322-Q2-U-42.

In another embodiment, compounds I are of formula I.A1 wherein Z is Z-322, R³ is CH₃ and Q is Q3, and R and R⁴ are as per any row of Table U below, which compounds are named I.A1-Z322-Q3-U-1 to I.A1-Z322-Q3-U-42.

In another embodiment, compounds I are of formula I.A1 wherein Z is Z-322, R³ is CH₃ and Q is Q4, and R and R⁴ are as per any row of Table U below, which compounds are named I.A1-Z322-Q4-U-1 to I.A1-Z322-Q4-U-42.

In another embodiment, compounds I are of formula I.A2 wherein Z is Z-322, R³ is CH₃ and Q is Q1, and R and R⁴ are as per any row of Table U below, which compounds are named I.A2-Z322-Q1-U-1 to I.A2-Z322-Q1-U-42.

In another embodiment, compounds I are of formula I.A2 wherein Z is Z-322, R³ is CH₃ and Q is Q2, and R and R⁴ are as per any row of Table U below, which compounds are named I.A2-Z322-Q2-U-1 to I.A2-Z322-Q2-U-42.

In another embodiment, compounds I are of formula I.A2 wherein Z is Z-322, R³ is CH₃ and Q is Q3, and R and R⁴ are as per any row of Table U below, which compounds are named I.A2-Z322-Q3-U-1 to I.A2-Z322-Q3-U-42.

In another embodiment, compounds I are of formula I.A2 wherein Z is Z-322, R³ is CH₃ and Q is Q4, and R and R⁴ are as per any row of Table U below, which compounds are named I.A2-Z322-Q4-U-1 to I.A2-Z322-Q4-U-42.

In another embodiment, compounds I are of formula I.B1 wherein Z is Z-59 and Q is Q1, and R and R⁴ are as per any row of Table U below, which compounds are named I.B1-Z59-Q1-U-1 to I.B1-Z59-Q1-U-42.

In another embodiment, compounds I are of formula I.B1 wherein Z is Z-59 and Q is Q2, and R and R⁴ are as per any row of Table U below, which compounds are named I.B1-Z59-Q2-U-1 to I.B1-Z59-Q2-U-42.

In another embodiment, compounds I are of formula I.B1 wherein Z is Z-59 and Q is Q3, and R and R⁴ are as per any row of Table U below, which compounds are named I.B1-Z59-Q3-U-1 to I.B1-Z59-Q3-U-42.

In another embodiment, compounds I are of formula I.B1 wherein Z is Z-59 and Q is Q4, and R and R⁴ are as per any row of Table U below, which compounds are named I.B1-Z59-Q4-U-1 to I.B1-Z59-Q4-U-42.

In another embodiment, compounds I are of formula I.B2 wherein Z is Z-59 and Q is Q1, and R and R⁴ are as per any row of Table U below, which compounds are named I.B2-Z59-Q1-U-1 to I.B2-Z59-Q1-U-42.

In another embodiment, compounds I are of formula I.B2 wherein Z is Z-59 and Q is Q2, and R and R⁴ are as per any row of Table U below, which compounds are named I.B2-Z59-Q2-U-1 to I.B2-Z59-Q2-U-42.

In another embodiment, compounds I are of formula I.B2 wherein Z is Z-59 and Q is Q3, and R and R⁴ are as per any row of Table U below, which compounds are named I.B2-Z59-Q3-U-1 to I.B2-Z59-Q3-U-42.

In another embodiment, compounds I are of formula I.B2 wherein Z is Z-59 and Q is Q4, and R and R⁴ are as per any row of Table U below, which compounds are named I.B2-Z59-Q4-U-1 to I.B2-Z59-Q4-U-42.

In another embodiment, compounds I are of formula I.B1 wherein Z is Z-60 and Q is Q1, and R and R⁴ are as per any row of Table U below, which compounds are named I.B1-Z60-Q1-U-1 to I.B1-Z60-Q1-U-42.

In another embodiment, compounds I are of formula I.B1 wherein Z is Z-60 and Q is Q2, and R and R⁴ are as per any row of Table U below, which compounds are named I.B1-Z60-Q2-U-1 to I.B1-Z60-Q2-U-42.

In another embodiment, compounds I are of formula I.B1 wherein Z is Z-60 and Q is Q3, and R and R⁴ are as per any row of Table U below, which compounds are named I.B1-Z60-Q3-U-1 to I.B1-Z60-Q3-U-42.

In another embodiment, compounds I are of formula I.B1 wherein Z is Z-60 and Q is Q4, and R and R⁴ are as per any row of Table U below, which compounds are named I.B1-Z60-Q4-U-1 to I.B1-Z60-Q4-U-42.

In another embodiment, compounds I are of formula I.B2 wherein Z is Z-60 and Q is Q1, and R and R⁴ are as per any row of Table U below, which compounds are named I.B2-Z60-Q1-U-1 to I.B2-Z60-Q1-U-42.

In another embodiment, compounds I are of formula I.B2 wherein Z is Z-60 and Q is Q2, and R and R⁴ are as per any row of Table U below, which compounds are named I.B2-Z60-Q2-U-1 to I.B2-Z60-Q2-U-42.

In another embodiment, compounds I are of formula I.B2 wherein Z is Z-60 and Q is Q3, and R and R⁴ are as per any row of Table U below, which compounds are named I.B2-Z60-Q3-U-1 to I.B2-Z60-Q3-U-42.

In another embodiment, compounds I are of formula I.B2 wherein Z is Z-60 and Q is Q4, and R and R⁴ are as per any row of Table U below, which compounds are named I.B2-Z60-Q4-U-1 to I.B2-Z60-Q4-U-42.

In another embodiment, compounds I are of formula I.B1 wherein Z is Z-61 and Q is Q1, and R and R⁴ are as per any row of Table U below, which compounds are named I.B1-Z61-Q1-U-1 to I.B1-Z61-Q1-U-42.

In another embodiment, compounds I are of formula I.B1 wherein Z is Z-61 and Q is Q2, and R and R⁴ are as per any row of Table U below, which compounds are named I.B1-Z61-Q2-U-1 to I.B1-Z61-Q2-U-42.

In another embodiment, compounds I are of formula I.B1 wherein Z is Z-61 and Q is Q3, and R and R⁴ are as per any row of Table U below, which compounds are named I.B1-Z61-Q3-U-1 to I.B1-Z61-Q3-U-42.

In another embodiment, compounds I are of formula I.B1 wherein Z is Z-61 and Q is Q4, and R and R⁴ are as per any row of Table U below, which compounds are named I.B1-Z61-Q4-U-1 to I.B1-Z61-Q4-U-42.

In another embodiment, compounds I are of formula I.B2 wherein Z is Z-61 and Q is Q1, and R and R⁴ are as per any row of Table U below, which compounds are named I.B2-Z61-Q1-U-1 to I.B2-Z61-Q1-U-42.

In another embodiment, compounds I are of formula I.B2 wherein Z is Z-61 and Q is Q2, and R and R⁴ are as per any row of Table U below, which compounds are named I.B2-Z61-Q2-U-1 to I.B2-Z61-Q2-U-42.

In another embodiment, compounds I are of formula I.B2 wherein Z is Z-61 and Q is Q3, and R and R⁴ are as per any row of Table U below, which compounds are named I.B2-Z61-Q3-U-1 to I.B2-Z61-Q3-U-42.

In another embodiment, compounds I are of formula I.B2 wherein Z is Z-61 and Q is Q4, and R and R⁴ are as per any row of Table U below, which compounds are named I.B2-Z61-Q4-U-1 to I.B2-Z61-Q4-U-42.

In another embodiment, compounds I are of formula I.B1 wherein Z is Z-62 and Q is Q1, and R and R⁴ are as per any row of Table U below, which compounds are named I.B1-Z62-Q1-U-1 to I.B1-Z62-Q1-U-42.

In another embodiment, compounds I are of formula I.B1 wherein Z is Z-62 and Q is Q2, and R and R⁴ are as per any row of Table U below, which compounds are named I.B1-Z62-Q2-U-1 to I.B1-Z62-Q2-U-42.

In another embodiment, compounds I are of formula I.B1 wherein Z is Z-62 and Q is Q3, and R and R⁴ are as per any row of Table U below, which compounds are named I.B1-Z62-Q3-U-1 to I.B1-Z62-Q3-U-42.

In another embodiment, compounds I are of formula I.B1 wherein Z is Z-62 and Q is Q4, and R and R⁴ are as per any row of Table U below, which compounds are named I.B1-Z62-Q4-U-1 to I.B1-Z62-Q4-U-42.

In another embodiment, compounds I are of formula I.B2 wherein Z is Z-62 and Q is Q1, and R and R⁴ are as per any row of Table U below, which compounds are named I.B2-Z62-Q1-U-1 to I.B2-Z62-Q1-U-42.

In another embodiment, compounds I are of formula I.B2 wherein Z is Z-62 and Q is Q2, and R and R⁴ are as per any row of Table U below, which compounds are named I.B2-Z62-Q2-U-1 to I.B2-Z62-Q2-U-42.

In another embodiment, compounds I are of formula I.B2 wherein Z is Z-62 and Q is Q3, and R and R⁴ are as per any row of Table U below, which compounds are named I.B2-Z62-Q3-U-1 to I.B2-Z62-Q3-U-42.

In another embodiment, compounds I are of formula I.B2 wherein Z is Z-62 and Q is Q4, and R and R⁴ are as per any row of Table U below, which compounds are named I.B2-Z62-Q4-U-1 to I.B2-Z62-Q4-U-42.

In another embodiment, compounds I are of formula I.B1 wherein Z is Z-63 and Q is Q1, and R and R⁴ are as per any row of Table U below, which compounds are named I.B1-Z63-Q1-U-1 to I.B1-Z63-Q1-U-42.

In another embodiment, compounds I are of formula I.B1 wherein Z is Z-63 and Q is Q2, and R and R⁴ are as per any row of Table U below, which compounds are named I.B1-Z63-Q2-U-1 to I.B1-Z63-Q2-U-42.

In another embodiment, compounds I are of formula I.B1 wherein Z is Z-63 and Q is Q3, and R and R⁴ are as per any row of Table U below, which compounds are named I.B1-Z63-Q3-U-1 to I.B1-Z63-Q3-U-42.

In another embodiment, compounds I are of formula I.B1 wherein Z is Z-63 and Q is Q4, and R and R⁴ are as per any row of Table U below, which compounds are named I.B1-Z63-Q4-U-1 to I.B1-Z63-Q4-U-42.

In another embodiment, compounds I are of formula I.B2 wherein Z is Z-63 and Q is Q1, and R and R⁴ are as per any row of Table U below, which compounds are named I.B2-Z63-Q1-U-1 to I.B2-Z63-Q1-U-42.

In another embodiment, compounds I are of formula I.B2 wherein Z is Z-63 and Q is Q2, and R and R⁴ are as per any row of Table U below, which compounds are named I.B2-Z63-Q2-U-1 to I.B2-Z63-Q2-U-42.

In another embodiment, compounds I are of formula I.B2 wherein Z is Z-63 and Q is Q3, and R and R⁴ are as per any row of Table U below, which compounds are named I.B2-Z63-Q3-U-1 to I.B2-Z63-Q3-U-42.

In another embodiment, compounds I are of formula I.B2 wherein Z is Z-63 and Q is Q4, and R and R⁴ are as per any row of Table U below, which compounds are named I.B2-Z63-Q4-U-1 to I.B2-Z63-Q4-U-42.

In another embodiment, compounds I are of formula I.B1 wherein Z is Z-64 and Q is Q1, and R and R⁴ are as per any row of Table U below, which compounds are named I.B1-Z64-Q1-U-1 to I.B1-Z64-Q1-U-42.

In another embodiment, compounds I are of formula I.B1 wherein Z is Z-64 and Q is Q2, and R and R⁴ are as per any row of Table U below, which compounds are named I.B1-Z64-Q2-U-1 to I.B1-Z64-Q2-U-42.

In another embodiment, compounds I are of formula I.B1 wherein Z is Z-64 and Q is Q3, and R and R⁴ are as per any row of Table U below, which compounds are named I.B1-Z64-Q3-U-1 to I.B1-Z64-Q3-U-42.

In another embodiment, compounds I are of formula I.B1 wherein Z is Z-64 and Q is Q4, and R and R⁴ are as per any row of Table U below, which compounds are named I.B1-Z64-Q4-U-1 to I.B1-Z64-Q4-U-42.

In another embodiment, compounds I are of formula I.B2 wherein Z is Z-64 and Q is Q1, and R and R⁴ are as per any row of Table U below, which compounds are named I.B2-Z64-Q1-U-1 to I.B2-Z64-Q1-U-42.

In another embodiment, compounds I are of formula I.B2 wherein Z is Z-64 and Q is Q2, and R and R⁴ are as per any row of Table U below, which compounds are named I.B2-Z64-Q2-U-1 to I.B2-Z64-Q2-U-42.

In another embodiment, compounds I are of formula I.B2 wherein Z is Z-64 and Q is Q3, and R and R⁴ are as per any row of Table U below, which compounds are named I.B2-Z64-Q3-U-1 to I.B2-Z64-Q3-U-42.

In another embodiment, compounds I are of formula I.B2 wherein Z is Z-64 and Q is Q4, and R and R⁴ are as per any row of Table U below, which compounds are named I.B2-Z64-Q4-U-1 to I.B2-Z64-Q4-U-42.

In another embodiment, compounds I are of formula I.B1 wherein Z is Z-65 and Q is Q1, and R and R⁴ are as per any row of Table U below, which compounds are named I.B1-Z65-Q1-U-1 to I.B1-Z65-Q1-U-42.

In another embodiment, compounds I are of formula I.B1 wherein Z is Z-65 and Q is Q2, and R and R⁴ are as per any row of Table U below, which compounds are named I.B1-Z65-Q2-U-1 to I.B1-Z65-Q2-U-42.

In another embodiment, compounds I are of formula I.B1 wherein Z is Z-65 and Q is Q3, and R and R⁴ are as per any row of Table U below, which compounds are named I.B1-Z65-Q3-U-1 to I.B1-Z65-Q3-U-42.

In another embodiment, compounds I are of formula I.B1 wherein Z is Z-65 and Q is Q4, and R and R⁴ are as per any row of Table U below, which compounds are named I.B1-Z65-Q4-U-1 to I.B1-Z65-Q4-U-42.

In another embodiment, compounds I are of formula I.B2 wherein Z is Z-65 and Q is Q1, and R and R⁴ are as per any row of Table U below, which compounds are named I.B2-Z65-Q1-U-1 to I.B2-Z65-Q1-U-42.

In another embodiment, compounds I are of formula I.B2 wherein Z is Z-65 and Q is Q2, and R and R⁴ are as per any row of Table U below, which compounds are named I.B2-Z65-Q2-U-1 to I.B2-Z65-Q2-U-42.

In another embodiment, compounds I are of formula I.B2 wherein Z is Z-65 and Q is Q3, and R and R⁴ are as per any row of Table U below, which compounds are named I.B2-Z65-Q3-U-1 to I.B2-Z65-Q3-U-42.

In another embodiment, compounds I are of formula I.B2 wherein Z is Z-65 and Q is Q4, and R and R⁴ are as per any row of Table U below, which compounds are named I.B2-Z65-Q4-U-1 to I.B2-Z65-Q4-U-42.

In another embodiment, compounds I are of formula I.B1 wherein Z is Z-66 and Q is Q1, and R and R⁴ are as per any row of Table U below, which compounds are named I.B1-Z66-Q1-U-1 to I.B1-Z66-Q1-U-42.

In another embodiment, compounds I are of formula I.B1 wherein Z is Z-66 and Q is Q2, and R and R⁴ are as per any row of Table U below, which compounds are named I.B1-Z66-Q2-U-1 to I.B1-Z66-Q2-U-42.

In another embodiment, compounds I are of formula I.B1 wherein Z is Z-66 and Q is Q3, and R and R⁴ are as per any row of Table U below, which compounds are named I.B1-Z66-Q3-U-1 to I.B1-Z66-Q3-U-42.

In another embodiment, compounds I are of formula I.B1 wherein Z is Z-66 and Q is Q4, and R and R⁴ are as per any row of Table U below, which compounds are named I.B1-Z66-Q4-U-1 to I.B1-Z66-Q4-U-42.

In another embodiment, compounds I are of formula I.B2 wherein Z is Z-66 and Q is Q1, and R and R⁴ are as per any row of Table U below, which compounds are named I.B2-Z66-Q1-U-1 to I.B2-Z66-Q1-U-42.

In another embodiment, compounds I are of formula I.B2 wherein Z is Z-66 and Q is Q2, and R and R⁴ are as per any row of Table U below, which compounds are named I.B2-Z66-Q2-U-1 to I.B2-Z66-Q2-U-42.

In another embodiment, compounds I are of formula I.B2 wherein Z is Z-66 and Q is Q3, and R and R⁴ are as per any row of Table U below, which compounds are named I.B2-Z66-Q3-U-1 to I.B2-Z66-Q3-U-42.

In another embodiment, compounds I are of formula I.B2 wherein Z is Z-66 and Q is Q4, and R and R⁴ are as per any row of Table U below, which compounds are named I.B2-Z66-Q4-U-1 to I.B2-Z66-Q4-U-42.

In another embodiment, compounds I are of formula I.B1 wherein Z is Z-67 and Q is Q1, and R and R⁴ are as per any row of Table U below, which compounds are named I.B1-Z67-Q1-U-1 to I.B1-Z67-Q1-U-42.

In another embodiment, compounds I are of formula I.B1 wherein Z is Z-67 and Q is Q2, and R and R⁴ are as per any row of Table U below, which compounds are named I.B1-Z67-Q2-U-1 to I.B1-Z67-Q2-U-42.

In another embodiment, compounds I are of formula I.B1 wherein Z is Z-67 and Q is Q3, and R and R⁴ are as per any row of Table U below, which compounds are named I.B1-Z67-Q3-U-1 to I.B1-Z67-Q3-U-42.

In another embodiment, compounds I are of formula I.B1 wherein Z is Z-67 and Q is Q4, and R and R⁴ are as per any row of Table U below, which compounds are named I.B1-Z67-Q4-U-1 to I.B1-Z67-Q4-U-42.

In another embodiment, compounds I are of formula I.B2 wherein Z is Z-67 and Q is Q1, and R and R⁴ are as per any row of Table U below, which compounds are named I.B2-Z67-Q1-U-1 to I.B2-Z67-Q1-U-42.

In another embodiment, compounds I are of formula I.B2 wherein Z is Z-67 and Q is Q2, and R and R⁴ are as per any row of Table U below, which compounds are named I.B2-Z67-Q2-U-1 to I.B2-Z67-Q2-U-42.

In another embodiment, compounds I are of formula I.B2 wherein Z is Z-67 and Q is Q3, and R and R⁴ are as per any row of Table U below, which compounds are named I.B2-Z67-Q3-U-1 to I.B2-Z67-Q3-U-42.

In another embodiment, compounds I are of formula I.B2 wherein Z is Z-67 and Q is Q4, and R and R⁴ are as per any row of Table U below, which compounds are named I.B2-Z67-Q4-U-1 to I.B2-Z67-Q4-U-42.

In another embodiment, compounds I are of formula I.B1 wherein Z is Z-68 and Q is Q1, and R and R⁴ are as per any row of Table U below, which compounds are named I.B1-Z68-Q1-U-1 to I.B1-Z68-Q1-U-42.

In another embodiment, compounds I are of formula I.B1 wherein Z is Z-68 and Q is Q2, and R and R⁴ are as per any row of Table U below, which compounds are named I.B1-Z68-Q2-U-1 to I.B1-Z68-Q2-U-42.

In another embodiment, compounds I are of formula I.B1 wherein Z is Z-68 and Q is Q3, and R and R⁴ are as per any row of Table U below, which compounds are named I.B1-Z68-Q3-U-1 to I.B1-Z68-Q3-U-42.

In another embodiment, compounds I are of formula I.B1 wherein Z is Z-68 and Q is Q4, and R and R⁴ are as per any row of Table U below, which compounds are named I.B1-Z68-Q4-U-1 to I.B1-Z68-Q4-U-42.

In another embodiment, compounds I are of formula I.B2 wherein Z is Z-68 and Q is Q1, and R and R⁴ are as per any row of Table U below, which compounds are named I.B2-Z68-Q1-U-1 to I.B2-Z68-Q1-U-42.

In another embodiment, compounds I are of formula I.B2 wherein Z is Z-68 and Q is Q2, and R and R⁴ are as per any row of Table U below, which compounds are named I.B2-Z68-Q2-U-1 to I.B2-Z68-Q2-U-42.

In another embodiment, compounds I are of formula I.B2 wherein Z is Z-68 and Q is Q3, and R and R⁴ are as per any row of Table U below, which compounds are named I.B2-Z68-Q3-U-1 to I.B2-Z68-Q3-U-42.

In another embodiment, compounds I are of formula I.B2 wherein Z is Z-68 and Q is Q4, and R and R⁴ are as per any row of Table U below, which compounds are named I.B2-Z68-Q4-U-1 to I.B2-Z68-Q4-U-42.

In another embodiment, compounds I are of formula I.B1 wherein Z is Z-69 and Q is Q1, and R and R⁴ are as per any row of Table U below, which compounds are named I.B1-Z69-Q1-U-1 to I.B1-Z69-Q1-U-42.

In another embodiment, compounds I are of formula I.B1 wherein Z is Z-69 and Q is Q2, and R and R⁴ are as per any row of Table U below, which compounds are named I.B1-Z69-Q2-U-1 to I.B1-Z69-Q2-U-42.

In another embodiment, compounds I are of formula I.B1 wherein Z is Z-69 and Q is Q3, and R and R⁴ are as per any row of Table U below, which compounds are named I.B1-Z69-Q3-U-1 to I.B1-Z69-Q3-U-42.

In another embodiment, compounds I are of formula I.B1 wherein Z is Z-69 and Q is Q4, and R and R⁴ are as per any row of Table U below, which compounds are named I.B1-Z69-Q4-U-1 to I.B1-Z69-Q4-U-42.

In another embodiment, compounds I are of formula I.B2 wherein Z is Z-69 and Q is Q1, and R and R⁴ are as per any row of Table U below, which compounds are named I.B2-Z69-Q1-U-1 to I.B2-Z69-Q1-U-42.

In another embodiment, compounds I are of formula I.B2 wherein Z is Z-69 and Q is Q2, and R and R⁴ are as per any row of Table U below, which compounds are named I.B2-Z69-Q2-U-1 to I.B2-Z69-Q2-U-42.

In another embodiment, compounds I are of formula I.B2 wherein Z is Z-69 and Q is Q3, and R and R⁴ are as per any row of Table U below, which compounds are named I.B2-Z69-Q3-U-1 to I.B2-Z69-Q3-U-42.

In another embodiment, compounds I are of formula I.B2 wherein Z is Z-69 and Q is Q4, and R and R⁴ are as per any row of Table U below, which compounds are named I.B2-Z69-Q4-U-1 to I.B2-Z69-Q4-U-42.

In another embodiment, compounds I are of formula I.B1 wherein Z is Z-70 and Q is Q1, and R and R⁴ are as per any row of Table U below, which compounds are named I.B1-Z70-Q1-U-1 to I.B1-Z70-Q1-U-42.

In another embodiment, compounds I are of formula I.B1 wherein Z is Z-70 and Q is Q2, and R and R⁴ are as per any row of Table U below, which compounds are named I.B1-Z70-Q2-U-1 to I.B1-Z70-Q2-U-42.

In another embodiment, compounds I are of formula I.B1 wherein Z is Z-70 and Q is Q3, and R and R⁴ are as per any row of Table U below, which compounds are named I.B1-Z70-Q3-U-1 to I.B1-Z70-Q3-U-42.

In another embodiment, compounds I are of formula I.B1 wherein Z is Z-70 and Q is Q4, and R and R⁴ are as per any row of Table U below, which compounds are named I.B1-Z70-Q4-U-1 to I.B1-Z70-Q4-U-42.

In another embodiment, compounds I are of formula I.B2 wherein Z is Z-70 and Q is Q1, and R and R⁴ are as per any row of Table U below, which compounds are named I.B2-Z70-Q1-U-1 to I.B2-Z70-Q1-U-42.

In another embodiment, compounds I are of formula I.B2 wherein Z is Z-70 and Q is Q2, and R and R⁴ are as per any row of Table U below, which compounds are named I.B2-Z70-Q2-U-1 to I.B2-Z70-Q2-U-42.

In another embodiment, compounds I are of formula I.B2 wherein Z is Z-70 and Q is Q3, and R and R⁴ are as per any row of Table U below, which compounds are named I.B2-Z70-Q3-U-1 to I.B2-Z70-Q3-U-42.

In another embodiment, compounds I are of formula I.B2 wherein Z is Z-70 and Q is Q4, and R and R⁴ are as per any row of Table U below, which compounds are named I.B2-Z70-Q4-U-1 to I.B2-Z70-Q4-U-42.

In another embodiment, compounds I are of formula I.B1 wherein Z is Z-71 and Q is Q1, and R and R⁴ are as per any row of Table U below, which compounds are named I.B1-Z71-Q1-U-1 to I.B1-Z71-Q1-U-42.

In another embodiment, compounds I are of formula I.B1 wherein Z is Z-71 and Q is Q2, and R and R⁴ are as per any row of Table U below, which compounds are named I.B1-Z71-Q2-U-1 to I.B1-Z71-Q2-U-42.

In another embodiment, compounds I are of formula I.B1 wherein Z is Z-71 and Q is Q3, and R and R⁴ are as per any row of Table U below, which compounds are named I.B1-Z71-Q3-U-1 to I.B1-Z71-Q3-U-42.

In another embodiment, compounds I are of formula I.B1 wherein Z is Z-71 and Q is Q4, and R and R⁴ are as per any row of Table U below, which compounds are named I.B1-Z71-Q4-U-1 to I.B1-Z71-Q4-U-42.

In another embodiment, compounds I are of formula I.B2 wherein Z is Z-71 and Q is Q1, and R and R⁴ are as per any row of Table U below, which compounds are named I.B2-Z71-Q1-U-1 to I.B2-Z71-Q1-U-42.

In another embodiment, compounds I are of formula I.B2 wherein Z is Z-71 and Q is Q2, and R and R⁴ are as per any row of Table U below, which compounds are named I.B2-Z71-Q2-U-1 to I.B2-Z71-Q2-U-42.

In another embodiment, compounds I are of formula I.B2 wherein Z is Z-71 and Q is Q3, and R and R⁴ are as per any row of Table U below, which compounds are named I.B2-Z71-Q3-U-1 to I.B2-Z71-Q3-U-42.

In another embodiment, compounds I are of formula I.B2 wherein Z is Z-71 and Q is Q4, and R and R⁴ are as per any row of Table U below, which compounds are named I.B2-Z71-Q4-U-1 to I.B2-Z71-Q4-U-42.

In another embodiment, compounds I are of formula I.B1 wherein Z is Z-72 and Q is Q1, and R and R⁴ are as per any row of Table U below, which compounds are named I.B1-Z72-Q1-U-1 to I.B1-Z72-Q1-U-42.

In another embodiment, compounds I are of formula I.B1 wherein Z is Z-72 and Q is Q2, and R and R⁴ are as per any row of Table U below, which compounds are named I.B1-Z72-Q2-U-1 to I.B1-Z72-Q2-U-42.

In another embodiment, compounds I are of formula I.B1 wherein Z is Z-72 and Q is Q3, and R and R⁴ are as per any row of Table U below, which compounds are named I.B1-Z72-Q3-U-1 to I.B1-Z72-Q3-U-42.

In another embodiment, compounds I are of formula I.B1 wherein Z is Z-72 and Q is Q4, and R and R⁴ are as per any row of Table U below, which compounds are named I.B1-Z72-Q4-U-1 to I.B1-Z72-Q4-U-42.

In another embodiment, compounds I are of formula I.B2 wherein Z is Z-72 and Q is Q1, and R and R⁴ are as per any row of Table U below, which compounds are named I.B2-Z72-Q1-U-1 to I.B2-Z72-Q1-U-42.

In another embodiment, compounds I are of formula I.B2 wherein Z is Z-72 and Q is Q2, and R and R⁴ are as per any row of Table U below, which compounds are named I.B2-Z72-Q2-U-1 to I.B2-Z72-Q2-U-42.

In another embodiment, compounds I are of formula I.B2 wherein Z is Z-72 and Q is Q3, and R and R⁴ are as per any row of Table U below, which compounds are named I.B2-Z72-Q3-U-1 to I.B2-Z72-Q3-U-42.

In another embodiment, compounds I are of formula I.B2 wherein Z is Z-72 and Q is Q4, and R and R⁴ are as per any row of Table U below, which compounds are named I.B2-Z72-Q4-U-1 to I.B2-Z72-Q4-U-42.

In another embodiment, compounds I are of formula I.B1 wherein Z is Z-73 and Q is Q1, and R and R⁴ are as per any row of Table U below, which compounds are named I.B1-Z73-Q1-U-1 to I.B1-Z73-Q1-U-42.

In another embodiment, compounds I are of formula I.B1 wherein Z is Z-73 and Q is Q2, and R and R⁴ are as per any row of Table U below, which compounds are named I.B1-Z73-Q2-U-1 to I.B1-Z73-Q2-U-42.

In another embodiment, compounds I are of formula I.B1 wherein Z is Z-73 and Q is Q3, and R and R⁴ are as per any row of Table U below, which compounds are named I.B1-Z73-Q3-U-1 to I.B1-Z73-Q3-U-42.

In another embodiment, compounds I are of formula I.B1 wherein Z is Z-73 and Q is Q4, and R and R⁴ are as per any row of Table U below, which compounds are named I.B1-Z73-Q4-U-1 to I.B1-Z73-Q4-U-42.

In another embodiment, compounds I are of formula I.B2 wherein Z is Z-73 and Q is Q1, and R and R⁴ are as per any row of Table U below, which compounds are named I.B2-Z73-Q1-U-1 to I.B2-Z73-Q1-U-42.

In another embodiment, compounds I are of formula I.B2 wherein Z is Z-73 and Q is Q2, and R and R⁴ are as per any row of Table U below, which compounds are named I.B2-Z73-Q2-U-1 to I.B2-Z73-Q2-U-42.

In another embodiment, compounds I are of formula I.B2 wherein Z is Z-73 and Q is Q3, and R and R⁴ are as per any row of Table U below, which compounds are named I.B2-Z73-Q3-U-1 to I.B2-Z73-Q3-U-42.

In another embodiment, compounds I are of formula I.B2 wherein Z is Z-73 and Q is Q4, and R and R⁴ are as per any row of Table U below, which compounds are named I.B2-Z73-Q4-U-1 to I.B2-Z73-Q4-U-42.

In another embodiment, compounds I are of formula I.B1 wherein Z is Z-74 and Q is Q1, and R and R⁴ are as per any row of Table U below, which compounds are named I.B1-Z74-Q1-U-1 to I.B1-Z74-Q1-U-42.

In another embodiment, compounds I are of formula I.B1 wherein Z is Z-74 and Q is Q2, and R and R⁴ are as per any row of Table U below, which compounds are named I.B1-Z74-Q2-U-1 to I.B1-Z74-Q2-U-42.

In another embodiment, compounds I are of formula I.B1 wherein Z is Z-74 and Q is Q3, and R and R⁴ are as per any row of Table U below, which compounds are named I.B1-Z74-Q3-U-1 to I.B1-Z74-Q3-U-42.

In another embodiment, compounds I are of formula I.B1 wherein Z is Z-74 and Q is Q4, and R and R⁴ are as per any row of Table U below, which compounds are named I.B1-Z74-Q4-U-1 to I.B1-Z74-Q4-U-42.

In another embodiment, compounds I are of formula I.B2 wherein Z is Z-74 and Q is Q1, and R and R⁴ are as per any row of Table U below, which compounds are named I.B2-Z74-Q1-U-1 to I.B2-Z74-Q1-U-42.

In another embodiment, compounds I are of formula I.B2 wherein Z is Z-74 and Q is Q2, and R and R⁴ are as per any row of Table U below, which compounds are named I.B2-Z74-Q2-U-1 to I.B2-Z74-Q2-U-42.

In another embodiment, compounds I are of formula I.B2 wherein Z is Z-74 and Q is Q3, and R and R⁴ are as per any row of Table U below, which compounds are named I.B2-Z74-Q3-U-1 to I.B2-Z74-Q3-U-42.

In another embodiment, compounds I are of formula I.B2 wherein Z is Z-74 and Q is Q4, and R and R⁴ are as per any row of Table U below, which compounds are named I.B2-Z74-Q4-U-1 to I.B2-Z74-Q4-U-42.

In another embodiment, compounds I are of formula I.B1 wherein Z is Z-75 and Q is Q1, and R and R⁴ are as per any row of Table U below, which compounds are named I.B1-Z75-Q1-U-1 to I.B1-Z75-Q1-U-42.

In another embodiment, compounds I are of formula I.B1 wherein Z is Z-75 and Q is Q2, and R and R⁴ are as per any row of Table U below, which compounds are named I.B1-Z75-Q2-U-1 to I.B1-Z75-Q2-U-42.

In another embodiment, compounds I are of formula I.B1 wherein Z is Z-75 and Q is Q3, and R and R⁴ are as per any row of Table U below, which compounds are named I.B1-Z75-Q3-U-1 to I.B1-Z75-Q3-U-42.

In another embodiment, compounds I are of formula I.B1 wherein Z is Z-75 and Q is Q4, and R and R⁴ are as per any row of Table U below, which compounds are named I.B1-Z75-Q4-U-1 to I.B1-Z75-Q4-U-42.

In another embodiment, compounds I are of formula I.B2 wherein Z is Z-75 and Q is Q1, and R and R⁴ are as per any row of Table U below, which compounds are named I.B2-Z75-Q1-U-1 to I.B2-Z75-Q1-U-42.

In another embodiment, compounds I are of formula I.B2 wherein Z is Z-75 and Q is Q2, and R and R⁴ are as per any row of Table U below, which compounds are named I.B2-Z75-Q2-U-1 to I.B2-Z75-Q2-U-42.

In another embodiment, compounds I are of formula I.B2 wherein Z is Z-75 and Q is Q3, and R and R⁴ are as per any row of Table U below, which compounds are named I.B2-Z75-Q3-U-1 to I.B2-Z75-Q3-U-42.

In another embodiment, compounds I are of formula I.B2 wherein Z is Z-75 and Q is Q4, and R and R⁴ are as per any row of Table U below, which compounds are named I.B2-Z75-Q4-U-1 to I.B2-Z75-Q4-U-42.

In another embodiment, compounds I are of formula I.B1 wherein Z is Z-76 and Q is Q1, and R and R⁴ are as per any row of Table U below, which compounds are named I.B1-Z76-Q1-U-1 to I.B1-Z76-Q1-U-42.

In another embodiment, compounds I are of formula I.B1 wherein Z is Z-76 and Q is Q2, and R and R⁴ are as per any row of Table U below, which compounds are named I.B1-Z76-Q2-U-1 to I.B1-Z76-Q2-U-42.

In another embodiment, compounds I are of formula I.B1 wherein Z is Z-76 and Q is Q3, and R and R⁴ are as per any row of Table U below, which compounds are named I.B1-Z76-Q3-U-1 to I.B1-Z76-Q3-U-42.

In another embodiment, compounds I are of formula I.B1 wherein Z is Z-76 and Q is Q4, and R and R⁴ are as per any row of Table U below, which compounds are named I.B1-Z76-Q4-U-1 to I.B1-Z76-Q4-U-42.

In another embodiment, compounds I are of formula I.B2 wherein Z is Z-76 and Q is Q1, and R and R⁴ are as per any row of Table U below, which compounds are named I.B2-Z76-Q1-U-1 to I.B2-Z76-Q1-U-42.

In another embodiment, compounds I are of formula I.B2 wherein Z is Z-76 and Q is Q2, and R and R⁴ are as per any row of Table U below, which compounds are named I.B2-Z76-Q2-U-1 to I.B2-Z76-Q2-U-42.

In another embodiment, compounds I are of formula I.B2 wherein Z is Z-76 and Q is Q3, and R and R⁴ are as per any row of Table U below, which compounds are named I.B2-Z76-Q3-U-1 to I.B2-Z76-Q3-U-42.

In another embodiment, compounds I are of formula I.B2 wherein Z is Z-76 and Q is Q4, and R and R⁴ are as per any row of Table U below, which compounds are named I.B2-Z76-Q4-U-1 to I.B2-Z76-Q4-U-42.

In another embodiment, compounds I are of formula I.B1 wherein Z is Z-77 and Q is Q1, and R and R⁴ are as per any row of Table U below, which compounds are named I.B1-Z77-Q1-U-1 to I.B1-Z77-Q1-U-42.

In another embodiment, compounds I are of formula I.B1 wherein Z is Z-77 and Q is Q2, and R and R⁴ are as per any row of Table U below, which compounds are named I.B1-Z77-Q2-U-1 to I.B1-Z77-Q2-U-42.

In another embodiment, compounds I are of formula I.B1 wherein Z is Z-77 and Q is Q3, and R and R⁴ are as per any row of Table U below, which compounds are named I.B1-Z77-Q3-U-1 to I.B1-Z77-Q3-U-42.

In another embodiment, compounds I are of formula I.B1 wherein Z is Z-77 and Q is Q4, and R and R⁴ are as per any row of Table U below, which compounds are named I.B1-Z77-Q4-U-1 to I.B1-Z77-Q4-U-42.

In another embodiment, compounds I are of formula I.B2 wherein Z is Z-77 and Q is Q1, and R and R⁴ are as per any row of Table U below, which compounds are named I.B2-Z77-Q1-U-1 to I.B2-Z77-Q1-U-42.

In another embodiment, compounds I are of formula I.B2 wherein Z is Z-77 and Q is Q2, and R and R⁴ are as per any row of Table U below, which compounds are named I.B2-Z77-Q2-U-1 to I.B2-Z77-Q2-U-42.

In another embodiment, compounds I are of formula I.B2 wherein Z is Z-77 and Q is Q3, and R and R⁴ are as per any row of Table U below, which compounds are named I.B2-Z77-Q3-U-1 to I.B2-Z77-Q3-U-42.

In another embodiment, compounds I are of formula I.B2 wherein Z is Z-77 and Q is Q4, and R and R⁴ are as per any row of Table U below, which compounds are named I.B2-Z77-Q4-U-1 to I.B2-Z77-Q4-U-42.

In another embodiment, compounds I are of formula I.B1 wherein Z is Z-78 and Q is Q1, and R and R⁴ are as per any row of Table U below, which compounds are named I.B1-Z78-Q1-U-1 to I.B1-Z78-Q1-U-42.

In another embodiment, compounds I are of formula I.B1 wherein Z is Z-78 and Q is Q2, and R and R⁴ are as per any row of Table U below, which compounds are named I.B1-Z78-Q2-U-1 to I.B1-Z78-Q2-U-42.

In another embodiment, compounds I are of formula I.B1 wherein Z is Z-78 and Q is Q3, and R and R⁴ are as per any row of Table U below, which compounds are named I.B1-Z78-Q3-U-1 to I.B1-Z78-Q3-U-42.

In another embodiment, compounds I are of formula I.B1 wherein Z is Z-78 and Q is Q4, and R and R⁴ are as per any row of Table U below, which compounds are named I.B1-Z78-Q4-U-1 to I.B1-Z78-Q4-U-42.

In another embodiment, compounds I are of formula I.B2 wherein Z is Z-78 and Q is Q1, and R and R⁴ are as per any row of Table U below, which compounds are named I.B2-Z78-Q1-U-1 to I.B2-Z78-Q1-U-42.

In another embodiment, compounds I are of formula I.B2 wherein Z is Z-78 and Q is Q2, and R and R⁴ are as per any row of Table U below, which compounds are named I.B2-Z78-Q2-U-1 to I.B2-Z78-Q2-U-42.

In another embodiment, compounds I are of formula I.B2 wherein Z is Z-78 and Q is Q3, and R and R⁴ are as per any row of Table U below, which compounds are named I.B2-Z78-Q3-U-1 to I.B2-Z78-Q3-U-42.

In another embodiment, compounds I are of formula I.B2 wherein Z is Z-78 and Q is Q4, and R and R⁴ are as per any row of Table U below, which compounds are named I.B2-Z78-Q4-U-1 to I.B2-Z78-Q4-U-42.

In another embodiment, compounds I are of formula I.B1 wherein Z is Z-79 and Q is Q1, and R and R⁴ are as per any row of Table U below, which compounds are named I.B1-Z79-Q1-U-1 to I.B1-Z79-Q1-U-42.

In another embodiment, compounds I are of formula I.B1 wherein Z is Z-79 and Q is Q2, and R and R⁴ are as per any row of Table U below, which compounds are named I.B1-Z79-Q2-U-1 to I.B1-Z79-Q2-U-42.

In another embodiment, compounds I are of formula I.B1 wherein Z is Z-79 and Q is Q3, and R and R⁴ are as per any row of Table U below, which compounds are named I.B1-Z79-Q3-U-1 to I.B1-Z79-Q3-U-42.

In another embodiment, compounds I are of formula I.B1 wherein Z is Z-79 and Q is Q4, and R and R⁴ are as per any row of Table U below, which compounds are named I.B1-Z79-Q4-U-1 to I.B1-Z79-Q4-U-42.

In another embodiment, compounds I are of formula I.B2 wherein Z is Z-79 and Q is Q1, and R and R⁴ are as per any row of Table U below, which compounds are named I.B2-Z79-Q1-U-1 to I.B2-Z79-Q1-U-42.

In another embodiment, compounds I are of formula I.B2 wherein Z is Z-79 and Q is Q2, and R and R⁴ are as per any row of Table U below, which compounds are named I.B2-Z79-Q2-U-1 to I.B2-Z79-Q2-U-42.

In another embodiment, compounds I are of formula I.B2 wherein Z is Z-79 and Q is Q3, and R and R⁴ are as per any row of Table U below, which compounds are named I.B2-Z79-Q3-U-1 to I.B2-Z79-Q3-U-42.

In another embodiment, compounds I are of formula I.B2 wherein Z is Z-79 and Q is Q4, and R and R⁴ are as per any row of Table U below, which compounds are named I.B2-Z79-Q4-U-1 to I.B2-Z79-Q4-U-42.

In another embodiment, compounds I are of formula I.B1 wherein Z is Z-80 and Q is Q1, and R and R⁴ are as per any row of Table U below, which compounds are named I.B1-Z80-Q1-U-1 to I.B1-Z80-Q1-U-42.

In another embodiment, compounds I are of formula I.B1 wherein Z is Z-80 and Q is Q2, and R and R⁴ are as per any row of Table U below, which compounds are named I.B1-Z80-Q2-U-1 to I.B1-Z80-Q2-U-42.

In another embodiment, compounds I are of formula I.B1 wherein Z is Z-80 and Q is Q3, and R and R⁴ are as per any row of Table U below, which compounds are named I.B1-Z80-Q3-U-1 to I.B1-Z80-Q3-U-42.

In another embodiment, compounds I are of formula I.B1 wherein Z is Z-80 and Q is Q4, and R and R⁴ are as per any row of Table U below, which compounds are named I.B1-Z80-Q4-U-1 to I.B1-Z80-Q4-U-42.

In another embodiment, compounds I are of formula I.B2 wherein Z is Z-80 and Q is Q1, and R and R⁴ are as per any row of Table U below, which compounds are named I.B2-Z80-Q1-U-1 to I.B2-Z80-Q1-U-42.

In another embodiment, compounds I are of formula I.B2 wherein Z is Z-80 and Q is Q2, and R and R⁴ are as per any row of Table U below, which compounds are named I.B2-Z80-Q2-U-1 to I.B2-Z80-Q2-U-42.

In another embodiment, compounds I are of formula I.B2 wherein Z is Z-80 and Q is Q3, and R and R⁴ are as per any row of Table U below, which compounds are named I.B2-Z80-Q3-U-1 to I.B2-Z80-Q3-U-42.

In another embodiment, compounds I are of formula I.B2 wherein Z is Z-80 and Q is Q4, and R and R⁴ are as per any row of Table U below, which compounds are named I.B2-Z80-Q4-U-1 to I.B2-Z80-Q4-U-42.

In another embodiment, compounds I are of formula I.B1 wherein Z is Z-81 and Q is Q1, and R and R⁴ are as per any row of Table U below, which compounds are named I.B1-Z81-Q1-U-1 to I.B1-Z81-Q1-U-42.

In another embodiment, compounds I are of formula I.B1 wherein Z is Z-81 and Q is Q2, and R and R⁴ are as per any row of Table U below, which compounds are named I.B1-Z81-Q2-U-1 to I.B1-Z81-Q2-U-42.

In another embodiment, compounds I are of formula I.B1 wherein Z is Z-81 and Q is Q3, and R and R⁴ are as per any row of Table U below, which compounds are named I.B1-Z81-Q3-U-1 to I.B1-Z81-Q3-U-42.

In another embodiment, compounds I are of formula I.B1 wherein Z is Z-81 and Q is Q4, and R and R⁴ are as per any row of Table U below, which compounds are named I.B1-Z81-Q4-U-1 to I.B1-Z81-Q4-U-42.

In another embodiment, compounds I are of formula I.B2 wherein Z is Z-81 and Q is Q1, and R and R⁴ are as per any row of Table U below, which compounds are named I.B2-Z81-Q1-U-1 to I.B2-Z81-Q1-U-42.

In another embodiment, compounds I are of formula I.B2 wherein Z is Z-81 and Q is Q2, and R and R⁴ are as per any row of Table U below, which compounds are named I.B2-Z81-Q2-U-1 to I.B2-Z81-Q2-U-42.

In another embodiment, compounds I are of formula I.B2 wherein Z is Z-81 and Q is Q3, and R and R⁴ are as per any row of Table U below, which compounds are named I.B2-Z81-Q3-U-1 to I.B2-Z81-Q3-U-42.

In another embodiment, compounds I are of formula I.B2 wherein Z is Z-81 and Q is Q4, and R and R⁴ are as per any row of Table U below, which compounds are named I.B2-Z81-Q4-U-1 to I.B2-Z81-Q4-U-42.

In another embodiment, compounds I are of formula I.B1 wherein Z is Z-82 and Q is Q1, and R and R⁴ are as per any row of Table U below, which compounds are named I.B1-Z82-Q1-U-1 to I.B1-Z82-Q1-U-42.

In another embodiment, compounds I are of formula I.B1 wherein Z is Z-82 and Q is Q2, and R and R⁴ are as per any row of Table U below, which compounds are named I.B1-Z82-Q2-U-1 to I.B1-Z82-Q2-U-42.

In another embodiment, compounds I are of formula I.B1 wherein Z is Z-82 and Q is Q3, and R and R⁴ are as per any row of Table U below, which compounds are named I.B1-Z82-Q3-U-1 to I.B1-Z82-Q3-U-42.

In another embodiment, compounds I are of formula I.B1 wherein Z is Z-82 and Q is Q4, and R and R⁴ are as per any row of Table U below, which compounds are named I.B1-Z82-Q4-U-1 to I.B1-Z82-Q4-U-42.

In another embodiment, compounds I are of formula I.B2 wherein Z is Z-82 and Q is Q1, and R and R⁴ are as per any row of Table U below, which compounds are named I.B2-Z82-Q1-U-1 to I.B2-Z82-Q1-U-42.

In another embodiment, compounds I are of formula I.B2 wherein Z is Z-82 and Q is Q2, and R and R⁴ are as per any row of Table U below, which compounds are named I.B2-Z82-Q2-U-1 to I.B2-Z82-Q2-U-42.

In another embodiment, compounds I are of formula I.B2 wherein Z is Z-82 and Q is Q3, and R and R⁴ are as per any row of Table U below, which compounds are named I.B2-Z82-Q3-U-1 to I.B2-Z82-Q3-U-42.

In another embodiment, compounds I are of formula I.B2 wherein Z is Z-82 and Q is Q4, and R and R⁴ are as per any row of Table U below, which compounds are named I.B2-Z82-Q4-U-1 to I.B2-Z82-Q4-U-42.

In another embodiment, compounds I are of formula I.B1 wherein Z is Z-83 and Q is Q1, and R and R⁴ are as per any row of Table U below, which compounds are named I.B1-Z83-Q1-U-1 to I.B1-Z83-Q1-U-42.

In another embodiment, compounds I are of formula I.B1 wherein Z is Z-83 and Q is Q2, and R and R⁴ are as per any row of Table U below, which compounds are named I.B1-Z83-Q2-U-1 to I.B1-Z83-Q2-U-42.

In another embodiment, compounds I are of formula I.B1 wherein Z is Z-83 and Q is Q3, and R and R⁴ are as per any row of Table U below, which compounds are named I.B1-Z83-Q3-U-1 to I.B1-Z83-Q3-U-42.

In another embodiment, compounds I are of formula I.B1 wherein Z is Z-83 and Q is Q4, and R and R⁴ are as per any row of Table U below, which compounds are named I.B1-Z83-Q4-U-1 to I.B1-Z83-Q4-U-42.

In another embodiment, compounds I are of formula I.B2 wherein Z is Z-83 and Q is Q1, and R and R⁴ are as per any row of Table U below, which compounds are named I.B2-Z83-Q1-U-1 to I.B2-Z83-Q1-U-42.

In another embodiment, compounds I are of formula I.B2 wherein Z is Z-83 and Q is Q2, and R and R⁴ are as per any row of Table U below, which compounds are named I.B2-Z83-Q2-U-1 to I.B2-Z83-Q2-U-42.

In another embodiment, compounds I are of formula I.B2 wherein Z is Z-83 and Q is Q3, and R and R⁴ are as per any row of Table U below, which compounds are named I.B2-Z83-Q3-U-1 to I.B2-Z83-Q3-U-42.

In another embodiment, compounds I are of formula I.B2 wherein Z is Z-83 and Q is Q4, and R and R⁴ are as per any row of Table U below, which compounds are named I.B2-Z83-Q4-U-1 to I.B2-Z83-Q4-U-42.

In another embodiment, compounds I are of formula I.B1 wherein Z is Z-84 and Q is Q1, and R and R⁴ are as per any row of Table U below, which compounds are named I.B1-Z84-Q1-U-1 to I.B1-Z84-Q1-U-42.

In another embodiment, compounds I are of formula I.B1 wherein Z is Z-84 and Q is Q2, and R and R⁴ are as per any row of Table U below, which compounds are named I.B1-Z84-Q2-U-1 to I.B1-Z84-Q2-U-42.

In another embodiment, compounds I are of formula I.B1 wherein Z is Z-84 and Q is Q3, and R and R⁴ are as per any row of Table U below, which compounds are named I.B1-Z84-Q3-U-1 to I.B1-Z84-Q3-U-42.

In another embodiment, compounds I are of formula I.B1 wherein Z is Z-84 and Q is Q4, and R and R⁴ are as per any row of Table U below, which compounds are named I.B1-Z84-Q4-U-1 to I.B1-Z84-Q4-U-42.

In another embodiment, compounds I are of formula I.B2 wherein Z is Z-84 and Q is Q1, and R and R⁴ are as per any row of Table U below, which compounds are named I.B2-Z84-Q1-U-1 to I.B2-Z84-Q1-U-42.

In another embodiment, compounds I are of formula I.B2 wherein Z is Z-84 and Q is Q2, and R and R⁴ are as per any row of Table U below, which compounds are named I.B2-Z84-Q2-U-1 to I.B2-Z84-Q2-U-42.

In another embodiment, compounds I are of formula I.B2 wherein Z is Z-84 and Q is Q3, and R and R⁴ are as per any row of Table U below, which compounds are named I.B2-Z84-Q3-U-1 to I.B2-Z84-Q3-U-42.

In another embodiment, compounds I are of formula I.B2 wherein Z is Z-84 and Q is Q4, and R and R⁴ are as per any row of Table U below, which compounds are named I.B2-Z84-Q4-U-1 to I.B2-Z84-Q4-U-42.

In another embodiment, compounds I are of formula I.B1 wherein Z is Z-85 and Q is Q1, and R and R⁴ are as per any row of Table U below, which compounds are named I.B1-Z85-Q1-U-1 to I.B1-Z85-Q1-U-42.

In another embodiment, compounds I are of formula I.B1 wherein Z is Z-85 and Q is Q2, and R and R⁴ are as per any row of Table U below, which compounds are named I.B1-Z85-Q2-U-1 to I.B1-Z85-Q2-U-42.

In another embodiment, compounds I are of formula I.B1 wherein Z is Z-85 and Q is Q3, and R and R⁴ are as per any row of Table U below, which compounds are named I.B1-Z85-Q3-U-1 to I.B1-Z85-Q3-U-42.

In another embodiment, compounds I are of formula I.B1 wherein Z is Z-85 and Q is Q4, and R and R⁴ are as per any row of Table U below, which compounds are named I.B1-Z85-Q4-U-1 to I.B1-Z85-Q4-U-42.

In another embodiment, compounds I are of formula I.B2 wherein Z is Z-85 and Q is Q1, and R and R⁴ are as per any row of Table U below, which compounds are named I.B2-Z85-Q1-U-1 to I.B2-Z85-Q1-U-42.

In another embodiment, compounds I are of formula I.B2 wherein Z is Z-85 and Q is Q2, and R and R⁴ are as per any row of Table U below, which compounds are named I.B2-Z85-Q2-U-1 to I.B2-Z85-Q2-U-42.

In another embodiment, compounds I are of formula I.B2 wherein Z is Z-85 and Q is Q3, and R and R⁴ are as per any row of Table U below, which compounds are named I.B2-Z85-Q3-U-1 to I.B2-Z85-Q3-U-42.

In another embodiment, compounds I are of formula I.B2 wherein Z is Z-85 and Q is Q4, and R and R⁴ are as per any row of Table U below, which compounds are named I.B2-Z85-Q4-U-1 to I.B2-Z85-Q4-U-42.

In another embodiment, compounds I are of formula I.B1 wherein Z is Z-86 and Q is Q1, and R and R⁴ are as per any row of Table U below, which compounds are named I.B1-Z86-Q1-U-1 to I.B1-Z86-Q1-U-42.

In another embodiment, compounds I are of formula I.B1 wherein Z is Z-86 and Q is Q2, and R and R⁴ are as per any row of Table U below, which compounds are named I.B1-Z86-Q2-U-1 to I.B1-Z86-Q2-U-42.

In another embodiment, compounds I are of formula I.B1 wherein Z is Z-86 and Q is Q3, and R and R⁴ are as per any row of Table U below, which compounds are named I.B1-Z86-Q3-U-1 to I.B1-Z86-Q3-U-42.

In another embodiment, compounds I are of formula I.B1 wherein Z is Z-86 and Q is Q4, and R and R⁴ are as per any row of Table U below, which compounds are named I.B1-Z86-Q4-U-1 to I.B1-Z86-Q4-U-42.

In another embodiment, compounds I are of formula I.B2 wherein Z is Z-86 and Q is Q1, and R and R⁴ are as per any row of Table U below, which compounds are named I.B2-Z86-Q1-U-1 to I.B2-Z86-Q1-U-42.

In another embodiment, compounds I are of formula I.B2 wherein Z is Z-86 and Q is Q2, and R and R⁴ are as per any row of Table U below, which compounds are named I.B2-Z86-Q2-U-1 to I.B2-Z86-Q2-U-42.

In another embodiment, compounds I are of formula I.B2 wherein Z is Z-86 and Q is Q3, and R and R⁴ are as per any row of Table U below, which compounds are named I.B2-Z86-Q3-U-1 to I.B2-Z86-Q3-U-42.

In another embodiment, compounds I are of formula I.B2 wherein Z is Z-86 and Q is Q4, and R and R⁴ are as per any row of Table U below, which compounds are named I.B2-Z86-Q4-U-1 to I.B2-Z86-Q4-U-42.

In another embodiment, compounds I are of formula I.B1 wherein Z is Z-87 and Q is Q1, and R and R⁴ are as per any row of Table U below, which compounds are named I.B1-Z87-Q1-U-1 to I.B1-Z87-Q1-U-42.

In another embodiment, compounds I are of formula I.B1 wherein Z is Z-87 and Q is Q2, and R and R⁴ are as per any row of Table U below, which compounds are named I.B1-Z87-Q2-U-1 to I.B1-Z87-Q2-U-42.

In another embodiment, compounds I are of formula I.B1 wherein Z is Z-87 and Q is Q3, and R and R⁴ are as per any row of Table U below, which compounds are named I.B1-Z87-Q3-U-1 to I.B1-Z87-Q3-U-42.

In another embodiment, compounds I are of formula I.B1 wherein Z is Z-87 and Q is Q4, and R and R⁴ are as per any row of Table U below, which compounds are named I.B1-Z87-Q4-U-1 to I.B1-Z87-Q4-U-42.

In another embodiment, compounds I are of formula I.B2 wherein Z is Z-87 and Q is Q1, and R and R⁴ are as per any row of Table U below, which compounds are named I.B2-Z87-Q1-U-1 to I.B2-Z87-Q1-U-42.

In another embodiment, compounds I are of formula I.B2 wherein Z is Z-87 and Q is Q2, and R and R⁴ are as per any row of Table U below, which compounds are named I.B2-Z87-Q2-U-1 to I.B2-Z87-Q2-U-42.

In another embodiment, compounds I are of formula I.B2 wherein Z is Z-87 and Q is Q3, and R and R⁴ are as per any row of Table U below, which compounds are named I.B2-Z87-Q3-U-1 to I.B2-Z87-Q3-U-42.

In another embodiment, compounds I are of formula I.B2 wherein Z is Z-87 and Q is Q4, and R and R⁴ are as per any row of Table U below, which compounds are named I.B2-Z87-Q4-U-1 to I.B2-Z87-Q4-U-42.

In another embodiment, compounds I are of formula I.B1 wherein Z is Z-88 and Q is Q1, and R and R⁴ are as per any row of Table U below, which compounds are named I.B1-Z88-Q1-U-1 to I.B1-Z88-Q1-U-42.

In another embodiment, compounds I are of formula I.B1 wherein Z is Z-88 and Q is Q2, and R and R⁴ are as per any row of Table U below, which compounds are named I.B1-Z88-Q2-U-1 to I.B1-Z88-Q2-U-42.

In another embodiment, compounds I are of formula I.B1 wherein Z is Z-88 and Q is Q3, and R and R⁴ are as per any row of Table U below, which compounds are named I.B1-Z88-Q3-U-1 to I.B1-Z88-Q3-U-42.

In another embodiment, compounds I are of formula I.B1 wherein Z is Z-88 and Q is Q4, and R and R⁴ are as per any row of Table U below, which compounds are named I.B1-Z88-Q4-U-1 to I.B1-Z88-Q4-U-42.

In another embodiment, compounds I are of formula I.B2 wherein Z is Z-88 and Q is Q1, and R and R⁴ are as per any row of Table U below, which compounds are named I.B2-Z88-Q1-U-1 to I.B2-Z88-Q1-U-42.

In another embodiment, compounds I are of formula I.B2 wherein Z is Z-88 and Q is Q2, and R and R⁴ are as per any row of Table U below, which compounds are named I.B2-Z88-Q2-U-1 to I.B2-Z88-Q2-U-42.

In another embodiment, compounds I are of formula I.B2 wherein Z is Z-88 and Q is Q3, and R and R⁴ are as per any row of Table U below, which compounds are named I.B2-Z88-Q3-U-1 to I.B2-Z88-Q3-U-42.

In another embodiment, compounds I are of formula I.B2 wherein Z is Z-88 and Q is Q4, and R and R⁴ are as per any row of Table U below, which compounds are named I.B2-Z88-Q4-U-1 to I.B2-Z88-Q4-U-42.

In another embodiment, compounds I are of formula I.B1 wherein Z is Z-89 and Q is Q1, and R and R⁴ are as per any row of Table U below, which compounds are named I.B1-Z89-Q1-U-1 to I.B1-Z89-Q1-U-42.

In another embodiment, compounds I are of formula I.B1 wherein Z is Z-89 and Q is Q2, and R and R⁴ are as per any row of Table U below, which compounds are named I.B1-Z89-Q2-U-1 to I.B1-Z89-Q2-U-42.

In another embodiment, compounds I are of formula I.B1 wherein Z is Z-89 and Q is Q3, and R and R⁴ are as per any row of Table U below, which compounds are named I.B1-Z89-Q3-U-1 to I.B1-Z89-Q3-U-42.

In another embodiment, compounds I are of formula I.B1 wherein Z is Z-89 and Q is Q4, and R and R⁴ are as per any row of Table U below, which compounds are named I.B1-Z89-Q4-U-1 to I.B1-Z89-Q4-U-42.

In another embodiment, compounds I are of formula I.B2 wherein Z is Z-89 and Q is Q1, and R and R⁴ are as per any row of Table U below, which compounds are named I.B2-Z89-Q1-U-1 to I.B2-Z89-Q1-U-42.

In another embodiment, compounds I are of formula I.B2 wherein Z is Z-89 and Q is Q2, and R and R⁴ are as per any row of Table U below, which compounds are named I.B2-Z89-Q2-U-1 to I.B2-Z89-Q2-U-42.

In another embodiment, compounds I are of formula I.B2 wherein Z is Z-89 and Q is Q3, and R and R⁴ are as per any row of Table U below, which compounds are named I.B2-Z89-Q3-U-1 to I.B2-Z89-Q3-U-42.

In another embodiment, compounds I are of formula I.B2 wherein Z is Z-89 and Q is Q4, and R and R⁴ are as per any row of Table U below, which compounds are named I.B2-Z89-Q4-U-1 to I.B2-Z89-Q4-U-42.

In another embodiment, compounds I are of formula I.B1 wherein Z is Z-90 and Q is Q1, and R and R⁴ are as per any row of Table U below, which compounds are named I.B1-Z90-Q1-U-1 to I.B1-Z90-Q1-U-42.

In another embodiment, compounds I are of formula I.B1 wherein Z is Z-90 and Q is Q2, and R and R⁴ are as per any row of Table U below, which compounds are named I.B1-Z90-Q2-U-1 to I.B1-Z90-Q2-U-42.

In another embodiment, compounds I are of formula I.B1 wherein Z is Z-90 and Q is Q3, and R and R⁴ are as per any row of Table U below, which compounds are named I.B1-Z90-Q3-U-1 to I.B1-Z90-Q3-U-42.

In another embodiment, compounds I are of formula I.B1 wherein Z is Z-90 and Q is Q4, and R and R⁴ are as per any row of Table U below, which compounds are named I.B1-Z90-Q4-U-1 to I.B1-Z90-Q4-U-42.

In another embodiment, compounds I are of formula I.B2 wherein Z is Z-90 and Q is Q1, and R and R⁴ are as per any row of Table U below, which compounds are named I.B2-Z90-Q1-U-1 to I.B2-Z90-Q1-U-42.

In another embodiment, compounds I are of formula I.B2 wherein Z is Z-90 and Q is Q2, and R and R⁴ are as per any row of Table U below, which compounds are named I.B2-Z90-Q2-U-1 to I.B2-Z90-Q2-U-42.

In another embodiment, compounds I are of formula I.B2 wherein Z is Z-90 and Q is Q3, and R and R⁴ are as per any row of Table U below, which compounds are named I.B2-Z90-Q3-U-1 to I.B2-Z90-Q3-U-42.

In another embodiment, compounds I are of formula I.B2 wherein Z is Z-90 and Q is Q4, and R and R⁴ are as per any row of Table U below, which compounds are named I.B2-Z90-Q4-U-1 to I.B2-Z90-Q4-U-42.

In another embodiment, compounds I are of formula I.B1 wherein Z is Z-91 and Q is Q1, and R and R⁴ are as per any row of Table U below, which compounds are named I.B1-Z91-Q1-U-1 to I.B1-Z91-Q1-U-42.

In another embodiment, compounds I are of formula I.B1 wherein Z is Z-91 and Q is Q2, and R and R⁴ are as per any row of Table U below, which compounds are named I.B1-Z91-Q2-U-1 to I.B1-Z91-Q2-U-42.

In another embodiment, compounds I are of formula I.B1 wherein Z is Z-91 and Q is Q3, and R and R⁴ are as per any row of Table U below, which compounds are named I.B1-Z91-Q3-U-1 to I.B1-Z91-Q3-U-42.

In another embodiment, compounds I are of formula I.B1 wherein Z is Z-91 and Q is Q4, and R and R⁴ are as per any row of Table U below, which compounds are named I.B1-Z91-Q4-U-1 to I.B1-Z91-Q4-U-42.

In another embodiment, compounds I are of formula I.B2 wherein Z is Z-91 and Q is Q1, and R and R⁴ are as per any row of Table U below, which compounds are named I.B2-Z91-Q1-U-1 to I.B2-Z91-Q1-U-42.

In another embodiment, compounds I are of formula I.B2 wherein Z is Z-91 and Q is Q2, and R and R⁴ are as per any row of Table U below, which compounds are named I.B2-Z91-Q2-U-1 to I.B2-Z91-Q2-U-42.

In another embodiment, compounds I are of formula I.B2 wherein Z is Z-91 and Q is Q3, and R and R⁴ are as per any row of Table U below, which compounds are named I.B2-Z91-Q3-U-1 to I.B2-Z91-Q3-U-42.

In another embodiment, compounds I are of formula I.B2 wherein Z is Z-91 and Q is Q4, and R and R⁴ are as per any row of Table U below, which compounds are named I.B2-Z91-Q4-U-1 to I.B2-Z91-Q4-U-42.

In another embodiment, compounds I are of formula I.B1 wherein Z is Z-92 and Q is Q1, and R and R⁴ are as per any row of Table U below, which compounds are named I.B1-Z92-Q1-U-1 to I.B1-Z92-Q1-U-42.

In another embodiment, compounds I are of formula I.B1 wherein Z is Z-92 and Q is Q2, and R and R⁴ are as per any row of Table U below, which compounds are named I.B1-Z92-Q2-U-1 to I.B1-Z92-Q2-U-42.

In another embodiment, compounds I are of formula I.B1 wherein Z is Z-92 and Q is Q3, and R and R⁴ are as per any row of Table U below, which compounds are named I.B1-Z92-Q3-U-1 to I.B1-Z92-Q3-U-42.

In another embodiment, compounds I are of formula I.B1 wherein Z is Z-92 and Q is Q4, and R and R⁴ are as per any row of Table U below, which compounds are named I.B1-Z92-Q4-U-1 to I.B1-Z92-Q4-U-42.

In another embodiment, compounds I are of formula I.B2 wherein Z is Z-92 and Q is Q1, and R and R⁴ are as per any row of Table U below, which compounds are named I.B2-Z92-Q1-U-1 to I.B2-Z92-Q1-U-42.

In another embodiment, compounds I are of formula I.B2 wherein Z is Z-92 and Q is Q2, and R and R⁴ are as per any row of Table U below, which compounds are named I.B2-Z92-Q2-U-1 to I.B2-Z92-Q2-U-42.

In another embodiment, compounds I are of formula I.B2 wherein Z is Z-92 and Q is Q3, and R and R⁴ are as per any row of Table U below, which compounds are named I.B2-Z92-Q3-U-1 to I.B2-Z92-Q3-U-42.

In another embodiment, compounds I are of formula I.B2 wherein Z is Z-92 and Q is Q4, and R and R⁴ are as per any row of Table U below, which compounds are named I.B2-Z92-Q4-U-1 to I.B2-Z92-Q4-U-42.

In another embodiment, compounds I are of formula I.B1 wherein Z is Z-93 and Q is Q1, and R and R⁴ are as per any row of Table U below, which compounds are named I.B1-Z93-Q1-U-1 to I.B1-Z93-Q1-U-42.

In another embodiment, compounds I are of formula I.B1 wherein Z is Z-93 and Q is Q2, and R and R⁴ are as per any row of Table U below, which compounds are named I.B1-Z93-Q2-U-1 to I.B1-Z93-Q2-U-42.

In another embodiment, compounds I are of formula I.B1 wherein Z is Z-93 and Q is Q3, and R and R⁴ are as per any row of Table U below, which compounds are named I.B1-Z93-Q3-U-1 to I.B1-Z93-Q3-U-42.

In another embodiment, compounds I are of formula I.B1 wherein Z is Z-93 and Q is Q4, and R and R⁴ are as per any row of Table U below, which compounds are named I.B1-Z93-Q4-U-1 to I.B1-Z93-Q4-U-42.

In another embodiment, compounds I are of formula I.B2 wherein Z is Z-93 and Q is Q1, and R and R⁴ are as per any row of Table U below, which compounds are named I.B2-Z93-Q1-U-1 to I.B2-Z93-Q1-U-42.

In another embodiment, compounds I are of formula I.B2 wherein Z is Z-93 and Q is Q2, and R and R⁴ are as per any row of Table U below, which compounds are named I.B2-Z93-Q2-U-1 to I.B2-Z93-Q2-U-42.

In another embodiment, compounds I are of formula I.B2 wherein Z is Z-93 and Q is Q3, and R and R⁴ are as per any row of Table U below, which compounds are named I.B2-Z93-Q3-U-1 to I.B2-Z93-Q3-U-42.

In another embodiment, compounds I are of formula I.B2 wherein Z is Z-93 and Q is Q4, and R and R⁴ are as per any row of Table U below, which compounds are named I.B2-Z93-Q4-U-1 to I.B2-Z93-Q4-U-42.

In another embodiment, compounds I are of formula I.B1 wherein Z is Z-94 and Q is Q1, and R and R⁴ are as per any row of Table U below, which compounds are named I.B1-Z94-Q1-U-1 to I.B1-Z94-Q1-U-42.

In another embodiment, compounds I are of formula I.B1 wherein Z is Z-94 and Q is Q2, and R and R⁴ are as per any row of Table U below, which compounds are named I.B1-Z94-Q2-U-1 to I.B1-Z94-Q2-U-42.

In another embodiment, compounds I are of formula I.B1 wherein Z is Z-94 and Q is Q3, and R and R⁴ are as per any row of Table U below, which compounds are named I.B1-Z94-Q3-U-1 to I.B1-Z94-Q3-U-42.

In another embodiment, compounds I are of formula I.B1 wherein Z is Z-94 and Q is Q4, and R and R⁴ are as per any row of Table U below, which compounds are named I.B1-Z94-Q4-U-1 to I.B1-Z94-Q4-U-42.

In another embodiment, compounds I are of formula I.B2 wherein Z is Z-94 and Q is Q1, and R and R⁴ are as per any row of Table U below, which compounds are named I.B2-Z94-Q1-U-1 to I.B2-Z94-Q1-U-42.

In another embodiment, compounds I are of formula I.B2 wherein Z is Z-94 and Q is Q2, and R and R⁴ are as per any row of Table U below, which compounds are named I.B2-Z94-Q2-U-1 to I.B2-Z94-Q2-U-42.

In another embodiment, compounds I are of formula I.B2 wherein Z is Z-94 and Q is Q3, and R and R⁴ are as per any row of Table U below, which compounds are named I.B2-Z94-Q3-U-1 to I.B2-Z94-Q3-U-42.

In another embodiment, compounds I are of formula I.B2 wherein Z is Z-94 and Q is Q4, and R and R⁴ are as per any row of Table U below, which compounds are named I.B2-Z94-Q4-U-1 to I.B2-Z94-Q4-U-42.

In another embodiment, compounds I are of formula I.B1 wherein Z is Z-95 and Q is Q1, and R and R⁴ are as per any row of Table U below, which compounds are named I.B1-Z95-Q1-U-1 to I.B1-Z95-Q1-U-42.

In another embodiment, compounds I are of formula I.B1 wherein Z is Z-95 and Q is Q2, and R and R⁴ are as per any row of Table U below, which compounds are named I.B1-Z95-Q2-U-1 to I.B1-Z95-Q2-U-42.

In another embodiment, compounds I are of formula I.B1 wherein Z is Z-95 and Q is Q3, and R and R⁴ are as per any row of Table U below, which compounds are named I.B1-Z95-Q3-U-1 to I.B1-Z95-Q3-U-42.

In another embodiment, compounds I are of formula I.B1 wherein Z is Z-95 and Q is Q4, and R and R⁴ are as per any row of Table U below, which compounds are named I.B1-Z95-Q4-U-1 to I.B1-Z95-Q4-U-42.

In another embodiment, compounds I are of formula I.B2 wherein Z is Z-95 and Q is Q1, and R and R⁴ are as per any row of Table U below, which compounds are named I.B2-Z95-Q1-U-1 to I.B2-Z95-Q1-U-42.

In another embodiment, compounds I are of formula I.B2 wherein Z is Z-95 and Q is Q2, and R and R⁴ are as per any row of Table U below, which compounds are named I.B2-Z95-Q2-U-1 to I.B2-Z95-Q2-U-42.

In another embodiment, compounds I are of formula I.B2 wherein Z is Z-95 and Q is Q3, and R and R⁴ are as per any row of Table U below, which compounds are named I.B2-Z95-Q3-U-1 to I.B2-Z95-Q3-U-42.

In another embodiment, compounds I are of formula I.B2 wherein Z is Z-95 and Q is Q4, and R and R⁴ are as per any row of Table U below, which compounds are named I.B2-Z95-Q4-U-1 to I.B2-Z95-Q4-U-42.

In another embodiment, compounds I are of formula I.B1 wherein Z is Z-96 and Q is Q1, and R and R⁴ are as per any row of Table U below, which compounds are named I.B1-Z96-Q1-U-1 to I.B1-Z96-Q1-U-42.

In another embodiment, compounds I are of formula I.B1 wherein Z is Z-96 and Q is Q2, and R and R⁴ are as per any row of Table U below, which compounds are named I.B1-Z96-Q2-U-1 to I.B1-Z96-Q2-U-42.

In another embodiment, compounds I are of formula I.B1 wherein Z is Z-96 and Q is Q3, and R and R⁴ are as per any row of Table U below, which compounds are named I.B1-Z96-Q3-U-1 to I.B1-Z96-Q3-U-42.

In another embodiment, compounds I are of formula I.B1 wherein Z is Z-96 and Q is Q4, and R and R⁴ are as per any row of Table U below, which compounds are named I.B1-Z96-Q4-U-1 to I.B1-Z96-Q4-U-42.

In another embodiment, compounds I are of formula I.B2 wherein Z is Z-96 and Q is Q1, and R and R⁴ are as per any row of Table U below, which compounds are named I.B2-Z96-Q1-U-1 to I.B2-Z96-Q1-U-42.

In another embodiment, compounds I are of formula I.B2 wherein Z is Z-96 and Q is Q2, and R and R⁴ are as per any row of Table U below, which compounds are named I.B2-Z96-Q2-U-1 to I.B2-Z96-Q2-U-42.

In another embodiment, compounds I are of formula I.B2 wherein Z is Z-96 and Q is Q3, and R and R⁴ are as per any row of Table U below, which compounds are named I.B2-Z96-Q3-U-1 to I.B2-Z96-Q3-U-42.

In another embodiment, compounds I are of formula I.B2 wherein Z is Z-96 and Q is Q4, and R and R⁴ are as per any row of Table U below, which compounds are named I.B2-Z96-Q4-U-1 to I.B2-Z96-Q4-U-42.

In another embodiment, compounds I are of formula I.B1 wherein Z is Z-97 and Q is Q1, and R and R⁴ are as per any row of Table U below, which compounds are named I.B1-Z97-Q1-U-1 to I.B1-Z97-Q1-U-42.

In another embodiment, compounds I are of formula I.B1 wherein Z is Z-97 and Q is Q2, and R and R⁴ are as per any row of Table U below, which compounds are named I.B1-Z97-Q2-U-1 to I.B1-Z97-Q2-U-42.

In another embodiment, compounds I are of formula I.B1 wherein Z is Z-97 and Q is Q3, and R and R⁴ are as per any row of Table U below, which compounds are named I.B1-Z97-Q3-U-1 to I.B1-Z97-Q3-U-42.

In another embodiment, compounds I are of formula I.B1 wherein Z is Z-97 and Q is Q4, and R and R⁴ are as per any row of Table U below, which compounds are named I.B1-Z97-Q4-U-1 to I.B1-Z97-Q4-U-42.

In another embodiment, compounds I are of formula I.B2 wherein Z is Z-97 and Q is Q1, and R and R⁴ are as per any row of Table U below, which compounds are named I.B2-Z97-Q1-U-1 to I.B2-Z97-Q1-U-42.

In another embodiment, compounds I are of formula I.B2 wherein Z is Z-97 and Q is Q2, and R and R⁴ are as per any row of Table U below, which compounds are named I.B2-Z97-Q2-U-1 to I.B2-Z97-Q2-U-42.

In another embodiment, compounds I are of formula I.B2 wherein Z is Z-97 and Q is Q3, and R and R⁴ are as per any row of Table U below, which compounds are named I.B2-Z97-Q3-U-1 to I.B2-Z97-Q3-U-42.

In another embodiment, compounds I are of formula I.B2 wherein Z is Z-97 and Q is Q4, and R and R⁴ are as per any row of Table U below, which compounds are named I.B2-Z97-Q4-U-1 to I.B2-Z97-Q4-U-42.

In another embodiment, compounds I are of formula I.B1 wherein Z is Z-98 and Q is Q1, and R and R⁴ are as per any row of Table U below, which compounds are named I.B1-Z98-Q1-U-1 to I.B1-Z98-Q1-U-42.

In another embodiment, compounds I are of formula I.B1 wherein Z is Z-98 and Q is Q2, and R and R⁴ are as per any row of Table U below, which compounds are named I.B1-Z98-Q2-U-1 to I.B1-Z98-Q2-U-42.

In another embodiment, compounds I are of formula I.B1 wherein Z is Z-98 and Q is Q3, and R and R⁴ are as per any row of Table U below, which compounds are named I.B1-Z98-Q3-U-1 to I.B1-Z98-Q3-U-42.

In another embodiment, compounds I are of formula I.B1 wherein Z is Z-98 and Q is Q4, and R and R⁴ are as per any row of Table U below, which compounds are named I.B1-Z98-Q4-U-1 to I.B1-Z98-Q4-U-42.

In another embodiment, compounds I are of formula I.B2 wherein Z is Z-98 and Q is Q1, and R and R⁴ are as per any row of Table U below, which compounds are named I.B2-Z98-Q1-U-1 to I.B2-Z98-Q1-U-42.

In another embodiment, compounds I are of formula I.B2 wherein Z is Z-98 and Q is Q2, and R and R⁴ are as per any row of Table U below, which compounds are named I.B2-Z98-Q2-U-1 to I.B2-Z98-Q2-U-42.

In another embodiment, compounds I are of formula I.B2 wherein Z is Z-98 and Q is Q3, and R and R⁴ are as per any row of Table U below, which compounds are named I.B2-Z98-Q3-U-1 to I.B2-Z98-Q3-U-42.

In another embodiment, compounds I are of formula I.B2 wherein Z is Z-98 and Q is Q4, and R and R⁴ are as per any row of Table U below, which compounds are named I.B2-Z98-Q4-U-1 to I.B2-Z98-Q4-U-42.

In another embodiment, compounds I are of formula I.B1 wherein Z is Z-99 and Q is Q1, and R and R⁴ are as per any row of Table U below, which compounds are named I.B1-Z99-Q1-U-1 to I.B1-Z99-Q1-U-42.

In another embodiment, compounds I are of formula I.B1 wherein Z is Z-99 and Q is Q2, and R and R⁴ are as per any row of Table U below, which compounds are named I.B1-Z99-Q2-U-1 to I.B1-Z99-Q2-U-42.

In another embodiment, compounds I are of formula I.B1 wherein Z is Z-99 and Q is Q3, and R and R⁴ are as per any row of Table U below, which compounds are named I.B1-Z99-Q3-U-1 to I.B1-Z99-Q3-U-42.

In another embodiment, compounds I are of formula I.B1 wherein Z is Z-99 and Q is Q4, and R and R⁴ are as per any row of Table U below, which compounds are named I.B1-Z99-Q4-U-1 to I.B1-Z99-Q4-U-42.

In another embodiment, compounds I are of formula I.B2 wherein Z is Z-99 and Q is Q1, and R and R⁴ are as per any row of Table U below, which compounds are named I.B2-Z99-Q1-U-1 to I.B2-Z99-Q1-U-42.

In another embodiment, compounds I are of formula I.B2 wherein Z is Z-99 and Q is Q2, and R and R⁴ are as per any row of Table U below, which compounds are named I.B2-Z99-Q2-U-1 to I.B2-Z99-Q2-U-42.

In another embodiment, compounds I are of formula I.B2 wherein Z is Z-99 and Q is Q3, and R and R⁴ are as per any row of Table U below, which compounds are named I.B2-Z99-Q3-U-1 to I.B2-Z99-Q3-U-42.

In another embodiment, compounds I are of formula I.B2 wherein Z is Z-99 and Q is Q4, and R and R⁴ are as per any row of Table U below, which compounds are named I.B2-Z99-Q4-U-1 to I.B2-Z99-Q4-U-42.

In another embodiment, compounds I are of formula I.B1 wherein Z is Z-100 and Q is Q1, and R and R⁴ are as per any row of Table U below, which compounds are named I.B1-Z100-Q1-U-1 to I.B1-Z100-Q1-U-42.

In another embodiment, compounds I are of formula I.B1 wherein Z is Z-100 and Q is Q2, and R and R⁴ are as per any row of Table U below, which compounds are named I.B1-Z100-Q2-U-1 to I.B1-Z100-Q2-U-42.

In another embodiment, compounds I are of formula I.B1 wherein Z is Z-100 and Q is Q3, and R and R⁴ are as per any row of Table U below, which compounds are named I.B1-Z100-Q3-U-1 to I.B1-Z100-Q3-U-42.

In another embodiment, compounds I are of formula I.B1 wherein Z is Z-100 and Q is Q4, and R and R⁴ are as per any row of Table U below, which compounds are named I.B1-Z100-Q4-U-1 to I.B1-Z100-Q4-U-42.

In another embodiment, compounds I are of formula I.B2 wherein Z is Z-100 and Q is Q1, and R and R⁴ are as per any row of Table U below, which compounds are named I.B2-Z100-Q1-U-1 to I.B2-Z100-Q1-U-42.

In another embodiment, compounds I are of formula I.B2 wherein Z is Z-100 and Q is Q2, and R and R⁴ are as per any row of Table U below, which compounds are named I.B2-Z100-Q2-U-1 to I.B2-Z100-Q2-U-42.

In another embodiment, compounds I are of formula I.B2 wherein Z is Z-100 and Q is Q3, and R and R⁴ are as per any row of Table U below, which compounds are named I.B2-Z100-Q3-U-1 to I.B2-Z100-Q3-U-42.

In another embodiment, compounds I are of formula I.B2 wherein Z is Z-100 and Q is Q4, and R and R⁴ are as per any row of Table U below, which compounds are named I.B2-Z100-Q4-U-1 to I.B2-Z100-Q4-U-42.

In another embodiment, compounds I are of formula I.B1 wherein Z is Z-101 and Q is Q1, and R and R⁴ are as per any row of Table U below, which compounds are named I.B1-Z101-Q1-U-1 to I.B1-Z101-Q1-U-42.

In another embodiment, compounds I are of formula I.B1 wherein Z is Z-101 and Q is Q2, and R and R⁴ are as per any row of Table U below, which compounds are named I.B1-Z101-Q2-U-1 to I.B1-Z101-Q2-U-42.

In another embodiment, compounds I are of formula I.B1 wherein Z is Z-101 and Q is Q3, and R and R⁴ are as per any row of Table U below, which compounds are named I.B1-Z101-Q3-U-1 to I.B1-Z101-Q3-U-42.

In another embodiment, compounds I are of formula I.B1 wherein Z is Z-101 and Q is Q4, and R and R⁴ are as per any row of Table U below, which compounds are named I.B1-Z101-Q4-U-1 to I.B1-Z101-Q4-U-42.

In another embodiment, compounds I are of formula I.B2 wherein Z is Z-101 and Q is Q1, and R and R⁴ are as per any row of Table U below, which compounds are named I.B2-Z101-Q1-U-1 to I.B2-Z101-Q1-U-42.

In another embodiment, compounds I are of formula I.B2 wherein Z is Z-101 and Q is Q2, and R and R⁴ are as per any row of Table U below, which compounds are named I.B2-Z101-Q2-U-1 to I.B2-Z101-Q2-U-42.

In another embodiment, compounds I are of formula I.B2 wherein Z is Z-101 and Q is Q3, and R and R⁴ are as per any row of Table U below, which compounds are named I.B2-Z101-Q3-U-1 to I.B2-Z101-Q3-U-42.

In another embodiment, compounds I are of formula I.B2 wherein Z is Z-101 and Q is Q4, and R and R⁴ are as per any row of Table U below, which compounds are named I.B2-Z101-Q4-U-1 to I.B2-Z101-Q4-U-42.

In another embodiment, compounds I are of formula I.B1 wherein Z is Z-102 and Q is Q1, and R and R⁴ are as per any row of Table U below, which compounds are named I.B1-Z102-Q1-U-1 to I.B1-Z102-Q1-U-42.

In another embodiment, compounds I are of formula I.B1 wherein Z is Z-102 and Q is Q2, and R and R⁴ are as per any row of Table U below, which compounds are named I.B1-Z102-Q2-U-1 to I.B1-Z102-Q2-U-42.

In another embodiment, compounds I are of formula I.B1 wherein Z is Z-102 and Q is Q3, and R and R⁴ are as per any row of Table U below, which compounds are named I.B1-Z102-Q3-U-1 to I.B1-Z102-Q3-U-42.

In another embodiment, compounds I are of formula I.B1 wherein Z is Z-102 and Q is Q4, and R and R⁴ are as per any row of Table U below, which compounds are named I.B1-Z102-Q4-U-1 to I.B1-Z102-Q4-U-42.

In another embodiment, compounds I are of formula I.B2 wherein Z is Z-102 and Q is Q1, and R and R⁴ are as per any row of Table U below, which compounds are named I.B2-Z102-Q1-U-1 to I.B2-Z102-Q1-U-42.

In another embodiment, compounds I are of formula I.B2 wherein Z is Z-102 and Q is Q2, and R and R⁴ are as per any row of Table U below, which compounds are named I.B2-Z102-Q2-U-1 to I.B2-Z102-Q2-U-42.

In another embodiment, compounds I are of formula I.B2 wherein Z is Z-102 and Q is Q3, and R and R⁴ are as per any row of Table U below, which compounds are named I.B2-Z102-Q3-U-1 to I.B2-Z102-Q3-U-42.

In another embodiment, compounds I are of formula I.B2 wherein Z is Z-102 and Q is Q4, and R and R⁴ are as per any row of Table U below, which compounds are named I.B2-Z102-Q4-U-1 to I.B2-Z102-Q4-U-42.

In another embodiment, compounds I are of formula I.B1 wherein Z is Z-103 and Q is Q1, and R and R⁴ are as per any row of Table U below, which compounds are named I.B1-Z103-Q1-U-1 to I.B1-Z103-Q1-U-42.

In another embodiment, compounds I are of formula I.B1 wherein Z is Z-103 and Q is Q2, and R and R⁴ are as per any row of Table U below, which compounds are named I.B1-Z103-Q2-U-1 to I.B1-Z103-Q2-U-42.

In another embodiment, compounds I are of formula I.B1 wherein Z is Z-103 and Q is Q3, and R and R⁴ are as per any row of Table U below, which compounds are named I.B1-Z103-Q3-U-1 to I.B1-Z103-Q3-U-42.

In another embodiment, compounds I are of formula I.B1 wherein Z is Z-103 and Q is Q4, and R and R⁴ are as per any row of Table U below, which compounds are named I.B1-Z103-Q4-U-1 to I.B1-Z103-Q4-U-42.

In another embodiment, compounds I are of formula I.B2 wherein Z is Z-103 and Q is Q1, and R and R⁴ are as per any row of Table U below, which compounds are named I.B2-Z103-Q1-U-1 to I.B2-Z103-Q1-U-42.

In another embodiment, compounds I are of formula I.B2 wherein Z is Z-103 and Q is Q2, and R and R⁴ are as per any row of Table U below, which compounds are named I.B2-Z103-Q2-U-1 to I.B2-Z103-Q2-U-42.

In another embodiment, compounds I are of formula I.B2 wherein Z is Z-103 and Q is Q3, and R and R⁴ are as per any row of Table U below, which compounds are named I.B2-Z103-Q3-U-1 to I.B2-Z103-Q3-U-42.

In another embodiment, compounds I are of formula I.B2 wherein Z is Z-103 and Q is Q4, and R and R⁴ are as per any row of Table U below, which compounds are named I.B2-Z103-Q4-U-1 to I.B2-Z103-Q4-U-42.

In another embodiment, compounds I are of formula I.B1 wherein Z is Z-104 and Q is Q1, and R and R⁴ are as per any row of Table U below, which compounds are named I.B1-Z104-Q1-U-1 to I.B1-Z104-Q1-U-42.

In another embodiment, compounds I are of formula I.B1 wherein Z is Z-104 and Q is Q2, and R and R⁴ are as per any row of Table U below, which compounds are named I.B1-Z104-Q2-U-1 to I.B1-Z104-Q2-U-42.

In another embodiment, compounds I are of formula I.B1 wherein Z is Z-104 and Q is Q3, and R and R⁴ are as per any row of Table U below, which compounds are named I.B1-Z104-Q3-U-1 to I.B1-Z104-Q3-U-42.

In another embodiment, compounds I are of formula I.B1 wherein Z is Z-104 and Q is Q4, and R and R⁴ are as per any row of Table U below, which compounds are named I.B1-Z104-Q4-U-1 to I.B1-Z104-Q4-U-42.

In another embodiment, compounds I are of formula I.B2 wherein Z is Z-104 and Q is Q1, and R and R⁴ are as per any row of Table U below, which compounds are named I.B2-Z104-Q1-U-1 to I.B2-Z104-Q1-U-42.

In another embodiment, compounds I are of formula I.B2 wherein Z is Z-104 and Q is Q2, and R and R⁴ are as per any row of Table U below, which compounds are named I.B2-Z104-Q2-U-1 to I.B2-Z104-Q2-U-42.

In another embodiment, compounds I are of formula I.B2 wherein Z is Z-104 and Q is Q3, and R and R⁴ are as per any row of Table U below, which compounds are named I.B2-Z104-Q3-U-1 to I.B2-Z104-Q3-U-42.

In another embodiment, compounds I are of formula I.B2 wherein Z is Z-104 and Q is Q4, and R and R⁴ are as per any row of Table U below, which compounds are named I.B2-Z104-Q4-U-1 to I.B2-Z104-Q4-U-42.

In another embodiment, compounds I are of formula I.B1 wherein Z is Z-105 and Q is Q1, and R and R⁴ are as per any row of Table U below, which compounds are named I.B1-Z105-Q1-U-1 to I.B1-Z105-Q1-U-42.

In another embodiment, compounds I are of formula I.B1 wherein Z is Z-105 and Q is Q2, and R and R⁴ are as per any row of Table U below, which compounds are named I.B1-Z105-Q2-U-1 to I.B1-Z105-Q2-U-42.

In another embodiment, compounds I are of formula I.B1 wherein Z is Z-105 and Q is Q3, and R and R⁴ are as per any row of Table U below, which compounds are named I.B1-Z105-Q3-U-1 to I.B1-Z105-Q3-U-42.

In another embodiment, compounds I are of formula I.B1 wherein Z is Z-105 and Q is Q4, and R and R⁴ are as per any row of Table U below, which compounds are named I.B1-Z105-Q4-U-1 to I.B1-Z105-Q4-U-42.

In another embodiment, compounds I are of formula I.B2 wherein Z is Z-105 and Q is Q1, and R and R⁴ are as per any row of Table U below, which compounds are named I.B2-Z105-Q1-U-1 to I.B2-Z105-Q1-U-42.

In another embodiment, compounds I are of formula I.B2 wherein Z is Z-105 and Q is Q2, and R and R⁴ are as per any row of Table U below, which compounds are named I.B2-Z105-Q2-U-1 to I.B2-Z105-Q2-U-42.

In another embodiment, compounds I are of formula I.B2 wherein Z is Z-105 and Q is Q3, and R and R⁴ are as per any row of Table U below, which compounds are named I.B2-Z105-Q3-U-1 to I.B2-Z105-Q3-U-42.

In another embodiment, compounds I are of formula I.B2 wherein Z is Z-105 and Q is Q4, and R and R⁴ are as per any row of Table U below, which compounds are named I.B2-Z105-Q4-U-1 to I.B2-Z105-Q4-U-42.

In another embodiment, compounds I are of formula I.B1 wherein Z is Z-106 and Q is Q1, and R and R⁴ are as per any row of Table U below, which compounds are named I.B1-Z106-Q1-U-1 to I.B1-Z106-Q1-U-42.

In another embodiment, compounds I are of formula I.B1 wherein Z is Z-106 and Q is Q2, and R and R⁴ are as per any row of Table U below, which compounds are named I.B1-Z106-Q2-U-1 to I.B1-Z106-Q2-U-42.

In another embodiment, compounds I are of formula I.B1 wherein Z is Z-106 and Q is Q3, and R and R⁴ are as per any row of Table U below, which compounds are named I.B1-Z106-Q3-U-1 to I.B1-Z106-Q3-U-42.

In another embodiment, compounds I are of formula I.B1 wherein Z is Z-106 and Q is Q4, and R and R⁴ are as per any row of Table U below, which compounds are named I.B1-Z106-Q4-U-1 to I.B1-Z106-Q4-U-42.

In another embodiment, compounds I are of formula I.B2 wherein Z is Z-106 and Q is Q1, and R and R⁴ are as per any row of Table U below, which compounds are named I.B2-Z106-Q1-U-1 to I.B2-Z106-Q1-U-42.

In another embodiment, compounds I are of formula I.B2 wherein Z is Z-106 and Q is Q2, and R and R⁴ are as per any row of Table U below, which compounds are named I.B2-Z106-Q2-U-1 to I.B2-Z106-Q2-U-42.

In another embodiment, compounds I are of formula I.B2 wherein Z is Z-106 and Q is Q3, and R and R⁴ are as per any row of Table U below, which compounds are named I.B2-Z106-Q3-U-1 to I.B2-Z106-Q3-U-42.

In another embodiment, compounds I are of formula I.B2 wherein Z is Z-106 and Q is Q4, and R and R⁴ are as per any row of Table U below, which compounds are named I.B2-Z106-Q4-U-1 to I.B2-Z106-Q4-U-42.

In another embodiment, compounds I are of formula I.B1 wherein Z is Z-107 and Q is Q1, and R and R⁴ are as per any row of Table U below, which compounds are named I.B1-Z107-Q1-U-1 to I.B1-Z107-Q1-U-42.

In another embodiment, compounds I are of formula I.B1 wherein Z is Z-107 and Q is Q2, and R and R⁴ are as per any row of Table U below, which compounds are named I.B1-Z107-Q2-U-1 to I.B1-Z107-Q2-U-42.

In another embodiment, compounds I are of formula I.B1 wherein Z is Z-107 and Q is Q3, and R and R⁴ are as per any row of Table U below, which compounds are named I.B1-Z107-Q3-U-1 to I.B1-Z107-Q3-U-42.

In another embodiment, compounds I are of formula I.B1 wherein Z is Z-107 and Q is Q4, and R and R⁴ are as per any row of Table U below, which compounds are named I.B1-Z107-Q4-U-1 to I.B1-Z107-Q4-U-42.

In another embodiment, compounds I are of formula I.B2 wherein Z is Z-107 and Q is Q1, and R and R⁴ are as per any row of Table U below, which compounds are named I.B2-Z107-Q1-U-1 to I.B2-Z107-Q1-U-42.

In another embodiment, compounds I are of formula I.B2 wherein Z is Z-107 and Q is Q2, and R and R⁴ are as per any row of Table U below, which compounds are named I.B2-Z107-Q2-U-1 to I.B2-Z107-Q2-U-42.

In another embodiment, compounds I are of formula I.B2 wherein Z is Z-107 and Q is Q3, and R and R⁴ are as per any row of Table U below, which compounds are named I.B2-Z107-Q3-U-1 to I.B2-Z107-Q3-U-42.

In another embodiment, compounds I are of formula I.B2 wherein Z is Z-107 and Q is Q4, and R and R⁴ are as per any row of Table U below, which compounds are named I.B2-Z107-Q4-U-1 to I.B2-Z107-Q4-U-42.

In another embodiment, compounds I are of formula I.B1 wherein Z is Z-108 and Q is Q1, and R and R⁴ are as per any row of Table U below, which compounds are named I.B1-Z108-Q1-U-1 to I.B1-Z108-Q1-U-42.

In another embodiment, compounds I are of formula I.B1 wherein Z is Z-108 and Q is Q2, and R and R⁴ are as per any row of Table U below, which compounds are named I.B1-Z108-Q2-U-1 to I.B1-Z108-Q2-U-42.

In another embodiment, compounds I are of formula I.B1 wherein Z is Z-108 and Q is Q3, and R and R⁴ are as per any row of Table U below, which compounds are named I.B1-Z108-Q3-U-1 to I.B1-Z108-Q3-U-42.

In another embodiment, compounds I are of formula I.B1 wherein Z is Z-108 and Q is Q4, and R and R⁴ are as per any row of Table U below, which compounds are named I.B1-Z108-Q4-U-1 to I.B1-Z108-Q4-U-42.

In another embodiment, compounds I are of formula I.B2 wherein Z is Z-108 and Q is Q1, and R and R⁴ are as per any row of Table U below, which compounds are named I.B2-Z108-Q1-U-1 to I.B2-Z108-Q1-U-42.

In another embodiment, compounds I are of formula I.B2 wherein Z is Z-108 and Q is Q2, and R and R⁴ are as per any row of Table U below, which compounds are named I.B2-Z108-Q2-U-1 to I.B2-Z108-Q2-U-42.

In another embodiment, compounds I are of formula I.B2 wherein Z is Z-108 and Q is Q3, and R and R⁴ are as per any row of Table U below, which compounds are named I.B2-Z108-Q3-U-1 to I.B2-Z108-Q3-U-42.

In another embodiment, compounds I are of formula I.B2 wherein Z is Z-108 and Q is Q4, and R and R⁴ are as per any row of Table U below, which compounds are named I.B2-Z108-Q4-U-1 to I.B2-Z108-Q4-U-42.

In another embodiment, compounds I are of formula I.B1 wherein Z is Z-109 and Q is Q1, and R and R⁴ are as per any row of Table U below, which compounds are named I.B1-Z109-Q1-U-1 to I.B1-Z109-Q1-U-42.

In another embodiment, compounds I are of formula I.B1 wherein Z is Z-109 and Q is Q2, and R and R⁴ are as per any row of Table U below, which compounds are named I.B1-Z109-Q2-U-1 to I.B1-Z109-Q2-U-42.

In another embodiment, compounds I are of formula I.B1 wherein Z is Z-109 and Q is Q3, and R and R⁴ are as per any row of Table U below, which compounds are named I.B1-Z109-Q3-U-1 to I.B1-Z109-Q3-U-42.

In another embodiment, compounds I are of formula I.B1 wherein Z is Z-109 and Q is Q4, and R and R⁴ are as per any row of Table U below, which compounds are named I.B1-Z109-Q4-U-1 to I.B1-Z109-Q4-U-42.

In another embodiment, compounds I are of formula I.B2 wherein Z is Z-109 and Q is Q1, and R and R⁴ are as per any row of Table U below, which compounds are named I.B2-Z109-Q1-U-1 to I.B2-Z109-Q1-U-42.

In another embodiment, compounds I are of formula I.B2 wherein Z is Z-109 and Q is Q2, and R and R⁴ are as per any row of Table U below, which compounds are named I.B2-Z109-Q2-U-1 to I.B2-Z109-Q2-U-42.

In another embodiment, compounds I are of formula I.B2 wherein Z is Z-109 and Q is Q3, and R and R⁴ are as per any row of Table U below, which compounds are named I.B2-Z109-Q3-U-1 to I.B2-Z109-Q3-U-42.

In another embodiment, compounds I are of formula I.B2 wherein Z is Z-109 and Q is Q4, and R and R⁴ are as per any row of Table U below, which compounds are named I.B2-Z109-Q4-U-1 to I.B2-Z109-Q4-U-42.

In another embodiment, compounds I are of formula I.B1 wherein Z is Z-110 and Q is Q1, and R and R⁴ are as per any row of Table U below, which compounds are named I.B1-Z110-Q1-U-1 to I.B1-Z110-Q1-U-42.

In another embodiment, compounds I are of formula I.B1 wherein Z is Z-110 and Q is Q2, and R and R⁴ are as per any row of Table U below, which compounds are named I.B1-Z110-Q2-U-1 to I.B1-Z110-Q2-U-42.

In another embodiment, compounds I are of formula I.B1 wherein Z is Z-110 and Q is Q3, and R and R⁴ are as per any row of Table U below, which compounds are named I.B1-Z110-Q3-U-1 to I.B1-Z110-Q3-U-42.

In another embodiment, compounds I are of formula I.B1 wherein Z is Z-110 and Q is Q4, and R and R⁴ are as per any row of Table U below, which compounds are named I.B1-Z110-Q4-U-1 to I.B1-Z110-Q4-U-42.

In another embodiment, compounds I are of formula I.B2 wherein Z is Z-110 and Q is Q1, and R and R⁴ are as per any row of Table U below, which compounds are named I.B2-Z110-Q1-U-1 to I.B2-Z110-Q1-U-42.

In another embodiment, compounds I are of formula I.B2 wherein Z is Z-110 and Q is Q2, and R and R⁴ are as per any row of Table U below, which compounds are named I.B2-Z110-Q2-U-1 to I.B2-Z110-Q2-U-42.

In another embodiment, compounds I are of formula I.B2 wherein Z is Z-110 and Q is Q3, and R and R⁴ are as per any row of Table U below, which compounds are named I.B2-Z110-Q3-U-1 to I.B2-Z110-Q3-U-42.

In another embodiment, compounds I are of formula I.B2 wherein Z is Z-110 and Q is Q4, and R and R⁴ are as per any row of Table U below, which compounds are named I.B2-Z110-Q4-U-1 to I.B2-Z110-Q4-U-42.

In another embodiment, compounds I are of formula I.B1 wherein Z is Z-111 and Q is Q1, and R and R⁴ are as per any row of Table U below, which compounds are named I.B1-Z111-Q1-U-1 to I.B1-Z111-Q1-U-42.

In another embodiment, compounds I are of formula I.B1 wherein Z is Z-111 and Q is Q2, and R and R⁴ are as per any row of Table U below, which compounds are named I.B1-Z111-Q2-U-1 to I.B1-Z111-Q2-U-42.

In another embodiment, compounds I are of formula I.B1 wherein Z is Z-111 and Q is Q3, and R and R⁴ are as per any row of Table U below, which compounds are named I.B1-Z111-Q3-U-1 to I.B1-Z111-Q3-U-42.

In another embodiment, compounds I are of formula I.B1 wherein Z is Z-111 and Q is Q4, and R and R⁴ are as per any row of Table U below, which compounds are named I.B1-Z111-Q4-U-1 to I.B1-Z111-Q4-U-42.

In another embodiment, compounds I are of formula I.B2 wherein Z is Z-111 and Q is Q1, and R and R⁴ are as per any row of Table U below, which compounds are named I.B2-Z111-Q1-U-1 to I.B2-Z111-Q1-U-42.

In another embodiment, compounds I are of formula I.B2 wherein Z is Z-111 and Q is Q2, and R and R⁴ are as per any row of Table U below, which compounds are named I.B2-Z111-Q2-U-1 to I.B2-Z111-Q2-U-42.

In another embodiment, compounds I are of formula I.B2 wherein Z is Z-111 and Q is Q3, and R and R⁴ are as per any row of Table U below, which compounds are named I.B2-Z111-Q3-U-1 to I.B2-Z111-Q3-U-42.

In another embodiment, compounds I are of formula I.B2 wherein Z is Z-111 and Q is Q4, and R and R⁴ are as per any row of Table U below, which compounds are named I.B2-Z111-Q4-U-1 to I.B2-Z111-Q4-U-42.

In another embodiment, compounds I are of formula I.B1 wherein Z is Z-112 and Q is Q1, and R and R⁴ are as per any row of Table U below, which compounds are named I.B1-Z112-Q1-U-1 to I.B1-Z112-Q1-U-42.

In another embodiment, compounds I are of formula I.B1 wherein Z is Z-112 and Q is Q2, and R and R⁴ are as per any row of Table U below, which compounds are named I.B1-Z112-Q2-U-1 to I.B1-Z112-Q2-U-42.

In another embodiment, compounds I are of formula I.B1 wherein Z is Z-112 and Q is Q3, and R and R⁴ are as per any row of Table U below, which compounds are named I.B1-Z112-Q3-U-1 to I.B1-Z112-Q3-U-42.

In another embodiment, compounds I are of formula I.B1 wherein Z is Z-112 and Q is Q4, and R and R⁴ are as per any row of Table U below, which compounds are named I.B1-Z112-Q4-U-1 to I.B1-Z112-Q4-U-42.

In another embodiment, compounds I are of formula I.B2 wherein Z is Z-112 and Q is Q1, and R and R⁴ are as per any row of Table U below, which compounds are named I.B2-Z112-Q1-U-1 to I.B2-Z112-Q1-U-42.

In another embodiment, compounds I are of formula I.B2 wherein Z is Z-112 and Q is Q2, and R and R⁴ are as per any row of Table U below, which compounds are named I.B2-Z112-Q2-U-1 to I.B2-Z112-Q2-U-42.

In another embodiment, compounds I are of formula I.B2 wherein Z is Z-112 and Q is Q3, and R and R⁴ are as per any row of Table U below, which compounds are named I.B2-Z112-Q3-U-1 to I.B2-Z112-Q3-U-42.

In another embodiment, compounds I are of formula I.B2 wherein Z is Z-112 and Q is Q4, and R and R⁴ are as per any row of Table U below, which compounds are named I.B2-Z112-Q4-U-1 to I.B2-Z112-Q4-U-42.

In another embodiment, compounds I are of formula I.B1 wherein Z is Z-113 and Q is Q1, and R and R⁴ are as per any row of Table U below, which compounds are named I.B1-Z113-Q1-U-1 to I.B1-Z113-Q1-U-42.

In another embodiment, compounds I are of formula I.B1 wherein Z is Z-113 and Q is Q2, and R and R⁴ are as per any row of Table U below, which compounds are named I.B1-Z113-Q2-U-1 to I.B1-Z113-Q2-U-42.

In another embodiment, compounds I are of formula I.B1 wherein Z is Z-113 and Q is Q3, and R and R⁴ are as per any row of Table U below, which compounds are named I.B1-Z113-Q3-U-1 to I.B1-Z113-Q3-U-42.

In another embodiment, compounds I are of formula I.B1 wherein Z is Z-113 and Q is Q4, and R and R⁴ are as per any row of Table U below, which compounds are named I.B1-Z113-Q4-U-1 to I.B1-Z113-Q4-U-42.

In another embodiment, compounds I are of formula I.B2 wherein Z is Z-113 and Q is Q1, and R and R⁴ are as per any row of Table U below, which compounds are named I.B2-Z113-Q1-U-1 to I.B2-Z113-Q1-U-42.

In another embodiment, compounds I are of formula I.B2 wherein Z is Z-113 and Q is Q2, and R and R⁴ are as per any row of Table U below, which compounds are named I.B2-Z113-Q2-U-1 to I.B2-Z113-Q2-U-42.

In another embodiment, compounds I are of formula I.B2 wherein Z is Z-113 and Q is Q3, and R and R⁴ are as per any row of Table U below, which compounds are named I.B2-Z113-Q3-U-1 to I.B2-Z113-Q3-U-42.

In another embodiment, compounds I are of formula I.B2 wherein Z is Z-113 and Q is Q4, and R and R⁴ are as per any row of Table U below, which compounds are named I.B2-Z113-Q4-U-1 to I.B2-Z113-Q4-U-42.

In another embodiment, compounds I are of formula I.B1 wherein Z is Z-114 and Q is Q1, and R and R⁴ are as per any row of Table U below, which compounds are named I.B1-Z114-Q1-U-1 to I.B1-Z114-Q1-U-42.

In another embodiment, compounds I are of formula I.B1 wherein Z is Z-114 and Q is Q2, and R and R⁴ are as per any row of Table U below, which compounds are named I.B1-Z114-Q2-U-1 to I.B1-Z114-Q2-U-42.

In another embodiment, compounds I are of formula I.B1 wherein Z is Z-114 and Q is Q3, and R and R⁴ are as per any row of Table U below, which compounds are named I.B1-Z114-Q3-U-1 to I.B1-Z114-Q3-U-42.

In another embodiment, compounds I are of formula I.B1 wherein Z is Z-114 and Q is Q4, and R and R⁴ are as per any row of Table U below, which compounds are named I.B1-Z114-Q4-U-1 to I.B1-Z114-Q4-U-42.

In another embodiment, compounds I are of formula I.B2 wherein Z is Z-114 and Q is Q1, and R and R⁴ are as per any row of Table U below, which compounds are named I.B2-Z114-Q1-U-1 to I.B2-Z114-Q1-U-42.

In another embodiment, compounds I are of formula I.B2 wherein Z is Z-114 and Q is Q2, and R and R⁴ are as per any row of Table U below, which compounds are named I.B2-Z114-Q2-U-1 to I.B2-Z114-Q2-U-42.

In another embodiment, compounds I are of formula I.B2 wherein Z is Z-114 and Q is Q3, and R and R⁴ are as per any row of Table U below, which compounds are named I.B2-Z114-Q3-U-1 to I.B2-Z114-Q3-U-42.

In another embodiment, compounds I are of formula I.B2 wherein Z is Z-114 and Q is Q4, and R and R⁴ are as per any row of Table U below, which compounds are named I.B2-Z114-Q4-U-1 to I.B2-Z114-Q4-U-42.

In another embodiment, compounds I are of formula I.B1 wherein Z is Z-115 and Q is Q1, and R and R⁴ are as per any row of Table U below, which compounds are named I.B1-Z115-Q1-U-1 to I.B1-Z115-Q1-U-42.

In another embodiment, compounds I are of formula I.B1 wherein Z is Z-115 and Q is Q2, and R and R⁴ are as per any row of Table U below, which compounds are named I.B1-Z115-Q2-U-1 to I.B1-Z115-Q2-U-42.

In another embodiment, compounds I are of formula I.B1 wherein Z is Z-115 and Q is Q3, and R and R⁴ are as per any row of Table U below, which compounds are named I.B1-Z115-Q3-U-1 to I.B1-Z115-Q3-U-42.

In another embodiment, compounds I are of formula I.B1 wherein Z is Z-115 and Q is Q4, and R and R⁴ are as per any row of Table U below, which compounds are named I.B1-Z115-Q4-U-1 to I.B1-Z115-Q4-U-42.

In another embodiment, compounds I are of formula I.B2 wherein Z is Z-115 and Q is Q1, and R and R⁴ are as per any row of Table U below, which compounds are named I.B2-Z115-Q1-U-1 to I.B2-Z115-Q1-U-42.

In another embodiment, compounds I are of formula I.B2 wherein Z is Z-115 and Q is Q2, and R and R⁴ are as per any row of Table U below, which compounds are named I.B2-Z115-Q2-U-1 to I.B2-Z115-Q2-U-42.

In another embodiment, compounds I are of formula I.B2 wherein Z is Z-115 and Q is Q3, and R and R⁴ are as per any row of Table U below, which compounds are named I.B2-Z115-Q3-U-1 to I.B2-Z115-Q3-U-42.

In another embodiment, compounds I are of formula I.B2 wherein Z is Z-115 and Q is Q4, and R and R⁴ are as per any row of Table U below, which compounds are named I.B2-Z115-Q4-U-1 to I.B2-Z115-Q4-U-42.

In another embodiment, compounds I are of formula I.B1 wherein Z is Z-116 and Q is Q1, and R and R⁴ are as per any row of Table U below, which compounds are named I.B1-Z116-Q1-U-1 to I.B1-Z116-Q1-U-42.

In another embodiment, compounds I are of formula I.B1 wherein Z is Z-116 and Q is Q2, and R and R⁴ are as per any row of Table U below, which compounds are named I.B1-Z116-Q2-U-1 to I.B1-Z116-Q2-U-42.

In another embodiment, compounds I are of formula I.B1 wherein Z is Z-116 and Q is Q3, and R and R⁴ are as per any row of Table U below, which compounds are named I.B1-Z116-Q3-U-1 to I.B1-Z116-Q3-U-42.

In another embodiment, compounds I are of formula I.B1 wherein Z is Z-116 and Q is Q4, and R and R⁴ are as per any row of Table U below, which compounds are named I.B1-Z116-Q4-U-1 to I.B1-Z116-Q4-U-42.

In another embodiment, compounds I are of formula I.B2 wherein Z is Z-116 and Q is Q1, and R and R⁴ are as per any row of Table U below, which compounds are named I.B2-Z116-Q1-U-1 to I.B2-Z116-Q1-U-42.

In another embodiment, compounds I are of formula I.B2 wherein Z is Z-116 and Q is Q2, and R and R⁴ are as per any row of Table U below, which compounds are named I.B2-Z116-Q2-U-1 to I.B2-Z116-Q2-U-42.

In another embodiment, compounds I are of formula I.B2 wherein Z is Z-116 and Q is Q3, and R and R⁴ are as per any row of Table U below, which compounds are named I.B2-Z116-Q3-U-1 to I.B2-Z116-Q3-U-42.

In another embodiment, compounds I are of formula I.B2 wherein Z is Z-116 and Q is Q4, and R and R⁴ are as per any row of Table U below, which compounds are named I.B2-Z116-Q4-U-1 to I.B2-Z116-Q4-U-42.

In another embodiment, compounds I are of formula I.B1 wherein Z is Z-117 and Q is Q1, and R and R⁴ are as per any row of Table U below, which compounds are named I.B1-Z117-Q1-U-1 to I.B1-Z117-Q1-U-42.

In another embodiment, compounds I are of formula I.B1 wherein Z is Z-117 and Q is Q2, and R and R⁴ are as per any row of Table U below, which compounds are named I.B1-Z117-Q2-U-1 to I.B1-Z117-Q2-U-42.

In another embodiment, compounds I are of formula I.B1 wherein Z is Z-117 and Q is Q3, and R and R⁴ are as per any row of Table U below, which compounds are named I.B1-Z117-Q3-U-1 to I.B1-Z117-Q3-U-42.

In another embodiment, compounds I are of formula I.B1 wherein Z is Z-117 and Q is Q4, and R and R⁴ are as per any row of Table U below, which compounds are named I.B1-Z117-Q4-U-1 to I.B1-Z117-Q4-U-42.

In another embodiment, compounds I are of formula I.B2 wherein Z is Z-117 and Q is Q1, and R and R⁴ are as per any row of Table U below, which compounds are named I.B2-Z117-Q1-U-1 to I.B2-Z117-Q1-U-42.

In another embodiment, compounds I are of formula I.B2 wherein Z is Z-117 and Q is Q2, and R and R⁴ are as per any row of Table U below, which compounds are named I.B2-Z117-Q2-U-1 to I.B2-Z117-Q2-U-42.

In another embodiment, compounds I are of formula I.B2 wherein Z is Z-117 and Q is Q3, and R and R⁴ are as per any row of Table U below, which compounds are named I.B2-Z117-Q3-U-1 to I.B2-Z117-Q3-U-42.

In another embodiment, compounds I are of formula I.B2 wherein Z is Z-117 and Q is Q4, and R and R⁴ are as per any row of Table U below, which compounds are named I.B2-Z117-Q4-U-1 to I.B2-Z117-Q4-U-42.

In another embodiment, compounds I are of formula I.B1 wherein Z is Z-118 and Q is Q1, and R and R⁴ are as per any row of Table U below, which compounds are named I.B1-Z118-Q1-U-1 to I.B1-Z118-Q1-U-42.

In another embodiment, compounds I are of formula I.B1 wherein Z is Z-118 and Q is Q2, and R and R⁴ are as per any row of Table U below, which compounds are named I.B1-Z118-Q2-U-1 to I.B1-Z118-Q2-U-42.

In another embodiment, compounds I are of formula I.B1 wherein Z is Z-118 and Q is Q3, and R and R⁴ are as per any row of Table U below, which compounds are named I.B1-Z118-Q3-U-1 to I.B1-Z118-Q3-U-42.

In another embodiment, compounds I are of formula I.B1 wherein Z is Z-118 and Q is Q4, and R and R⁴ are as per any row of Table U below, which compounds are named I.B1-Z118-Q4-U-1 to I.B1-Z118-Q4-U-42.

In another embodiment, compounds I are of formula I.B2 wherein Z is Z-118 and Q is Q1, and R and R⁴ are as per any row of Table U below, which compounds are named I.B2-Z118-Q1-U-1 to I.B2-Z118-Q1-U-42.

In another embodiment, compounds I are of formula I.B2 wherein Z is Z-118 and Q is Q2, and R and R⁴ are as per any row of Table U below, which compounds are named I.B2-Z118-Q2-U-1 to I.B2-Z118-Q2-U-42.

In another embodiment, compounds I are of formula I.B2 wherein Z is Z-118 and Q is Q3, and R and R⁴ are as per any row of Table U below, which compounds are named I.B2-Z118-Q3-U-1 to I.B2-Z118-Q3-U-42.

In another embodiment, compounds I are of formula I.B2 wherein Z is Z-118 and Q is Q4, and R and R⁴ are as per any row of Table U below, which compounds are named I.B2-Z118-Q4-U-1 to I.B2-Z118-Q4-U-42.

In another embodiment, compounds I are of formula I.B1 wherein Z is Z-119 and Q is Q1, and R and R⁴ are as per any row of Table U below, which compounds are named I.B1-Z119-Q1-U-1 to I.B1-Z119-Q1-U-42.

In another embodiment, compounds I are of formula I.B1 wherein Z is Z-119 and Q is Q2, and R and R⁴ are as per any row of Table U below, which compounds are named I.B1-Z119-Q2-U-1 to I.B1-Z119-Q2-U-42.

In another embodiment, compounds I are of formula I.B1 wherein Z is Z-119 and Q is Q3, and R and R⁴ are as per any row of Table U below, which compounds are named I.B1-Z119-Q3-U-1 to I.B1-Z119-Q3-U-42.

In another embodiment, compounds I are of formula I.B1 wherein Z is Z-119 and Q is Q4, and R and R⁴ are as per any row of Table U below, which compounds are named I.B1-Z119-Q4-U-1 to I.B1-Z119-Q4-U-42.

In another embodiment, compounds I are of formula I.B2 wherein Z is Z-119 and Q is Q1, and R and R⁴ are as per any row of Table U below, which compounds are named I.B2-Z119-Q1-U-1 to I.B2-Z119-Q1-U-42.

In another embodiment, compounds I are of formula I.B2 wherein Z is Z-119 and Q is Q2, and R and R⁴ are as per any row of Table U below, which compounds are named I.B2-Z119-Q2-U-1 to I.B2-Z119-Q2-U-42.

In another embodiment, compounds I are of formula I.B2 wherein Z is Z-119 and Q is Q3, and R and R⁴ are as per any row of Table U below, which compounds are named I.B2-Z119-Q3-U-1 to I.B2-Z119-Q3-U-42.

In another embodiment, compounds I are of formula I.B2 wherein Z is Z-119 and Q is Q4, and R and R⁴ are as per any row of Table U below, which compounds are named I.B2-Z119-Q4-U-1 to I.B2-Z119-Q4-U-42.

In another embodiment, compounds I are of formula I.B1 wherein Z is Z-120 and Q is Q1, and R and R⁴ are as per any row of Table U below, which compounds are named I.B1-Z120-Q1-U-1 to I.B1-Z120-Q1-U-42.

In another embodiment, compounds I are of formula I.B1 wherein Z is Z-120 and Q is Q2, and R and R⁴ are as per any row of Table U below, which compounds are named I.B1-Z120-Q2-U-1 to I.B1-Z120-Q2-U-42.

In another embodiment, compounds I are of formula I.B1 wherein Z is Z-120 and Q is Q3, and R and R⁴ are as per any row of Table U below, which compounds are named I.B1-Z120-Q3-U-1 to I.B1-Z120-Q3-U-42.

In another embodiment, compounds I are of formula I.B1 wherein Z is Z-120 and Q is Q4, and R and R⁴ are as per any row of Table U below, which compounds are named I.B1-Z120-Q4-U-1 to I.B1-Z120-Q4-U-42.

In another embodiment, compounds I are of formula I.B2 wherein Z is Z-120 and Q is Q1, and R and R⁴ are as per any row of Table U below, which compounds are named I.B2-Z120-Q1-U-1 to I.B2-Z120-Q1-U-42.

In another embodiment, compounds I are of formula I.B2 wherein Z is Z-120 and Q is Q2, and R and R⁴ are as per any row of Table U below, which compounds are named I.B2-Z120-Q2-U-1 to I.B2-Z120-Q2-U-42.

In another embodiment, compounds I are of formula I.B2 wherein Z is Z-120 and Q is Q3, and R and R⁴ are as per any row of Table U below, which compounds are named I.B2-Z120-Q3-U-1 to I.B2-Z120-Q3-U-42.

In another embodiment, compounds I are of formula I.B2 wherein Z is Z-120 and Q is Q4, and R and R⁴ are as per any row of Table U below, which compounds are named I.B2-Z120-Q4-U-1 to I.B2-Z120-Q4-U-42.

In another embodiment, compounds I are of formula I.B1 wherein Z is Z-121 and Q is Q1, and R and R⁴ are as per any row of Table U below, which compounds are named I.B1-Z121-Q1-U-1 to I.B1-Z121-Q1-U-42.

In another embodiment, compounds I are of formula I.B1 wherein Z is Z-121 and Q is Q2, and R and R⁴ are as per any row of Table U below, which compounds are named I.B1-Z121-Q2-U-1 to I.B1-Z121-Q2-U-42.

In another embodiment, compounds I are of formula I.B1 wherein Z is Z-121 and Q is Q3, and R and R⁴ are as per any row of Table U below, which compounds are named I.B1-Z121-Q3-U-1 to I.B1-Z121-Q3-U-42.

In another embodiment, compounds I are of formula I.B1 wherein Z is Z-121 and Q is Q4, and R and R⁴ are as per any row of Table U below, which compounds are named I.B1-Z121-Q4-U-1 to I.B1-Z121-Q4-U-42.

In another embodiment, compounds I are of formula I.B2 wherein Z is Z-121 and Q is Q1, and R and R⁴ are as per any row of Table U below, which compounds are named I.B2-Z121-Q1-U-1 to I.B2-Z121-Q1-U-42.

In another embodiment, compounds I are of formula I.B2 wherein Z is Z-121 and Q is Q2, and R and R⁴ are as per any row of Table U below, which compounds are named I.B2-Z121-Q2-U-1 to I.B2-Z121-Q2-U-42.

In another embodiment, compounds I are of formula I.B2 wherein Z is Z-121 and Q is Q3, and R and R⁴ are as per any row of Table U below, which compounds are named I.B2-Z121-Q3-U-1 to I.B2-Z121-Q3-U-42.

In another embodiment, compounds I are of formula I.B2 wherein Z is Z-121 and Q is Q4, and R and R⁴ are as per any row of Table U below, which compounds are named I.B2-Z121-Q4-U-1 to I.B2-Z121-Q4-U-42.

In another embodiment, compounds I are of formula I.B1 wherein Z is Z-122 and Q is Q1, and R and R⁴ are as per any row of Table U below, which compounds are named I.B1-Z122-Q1-U-1 to I.B1-Z122-Q1-U-42.

In another embodiment, compounds I are of formula I.B1 wherein Z is Z-122 and Q is Q2, and R and R⁴ are as per any row of Table U below, which compounds are named I.B1-Z122-Q2-U-1 to I.B1-Z122-Q2-U-42.

In another embodiment, compounds I are of formula I.B1 wherein Z is Z-122 and Q is Q3, and R and R⁴ are as per any row of Table U below, which compounds are named I.B1-Z122-Q3-U-1 to I.B1-Z122-Q3-U-42.

In another embodiment, compounds I are of formula I.B1 wherein Z is Z-122 and Q is Q4, and R and R⁴ are as per any row of Table U below, which compounds are named I.B1-Z122-Q4-U-1 to I.B1-Z122-Q4-U-42.

In another embodiment, compounds I are of formula I.B2 wherein Z is Z-122 and Q is Q1, and R and R⁴ are as per any row of Table U below, which compounds are named I.B2-Z122-Q1-U-1 to I.B2-Z122-Q1-U-42.

In another embodiment, compounds I are of formula I.B2 wherein Z is Z-122 and Q is Q2, and R and R⁴ are as per any row of Table U below, which compounds are named I.B2-Z122-Q2-U-1 to I.B2-Z122-Q2-U-42.

In another embodiment, compounds I are of formula I.B2 wherein Z is Z-122 and Q is Q3, and R and R⁴ are as per any row of Table U below, which compounds are named I.B2-Z122-Q3-U-1 to I.B2-Z122-Q3-U-42.

In another embodiment, compounds I are of formula I.B2 wherein Z is Z-122 and Q is Q4, and R and R⁴ are as per any row of Table U below, which compounds are named I.B2-Z122-Q4-U-1 to I.B2-Z122-Q4-U-42.

In another embodiment, compounds I are of formula I.B1 wherein Z is Z-123 and Q is Q1, and R and R⁴ are as per any row of Table U below, which compounds are named I.B1-Z123-Q1-U-1 to I.B1-Z123-Q1-U-42.

In another embodiment, compounds I are of formula I.B1 wherein Z is Z-123 and Q is Q2, and R and R⁴ are as per any row of Table U below, which compounds are named I.B1-Z123-Q2-U-1 to I.B1-Z123-Q2-U-42.

In another embodiment, compounds I are of formula I.B1 wherein Z is Z-123 and Q is Q3, and R and R⁴ are as per any row of Table U below, which compounds are named I.B1-Z123-Q3-U-1 to I.B1-Z123-Q3-U-42.

In another embodiment, compounds I are of formula I.B1 wherein Z is Z-123 and Q is Q4, and R and R⁴ are as per any row of Table U below, which compounds are named I.B1-Z123-Q4-U-1 to I.B1-Z123-Q4-U-42.

In another embodiment, compounds I are of formula I.B2 wherein Z is Z-123 and Q is Q1, and R and R⁴ are as per any row of Table U below, which compounds are named I.B2-Z123-Q1-U-1 to I.B2-Z123-Q1-U-42.

In another embodiment, compounds I are of formula I.B2 wherein Z is Z-123 and Q is Q2, and R and R⁴ are as per any row of Table U below, which compounds are named I.B2-Z123-Q2-U-1 to I.B2-Z123-Q2-U-42.

In another embodiment, compounds I are of formula I.B2 wherein Z is Z-123 and Q is Q3, and R and R⁴ are as per any row of Table U below, which compounds are named I.B2-Z123-Q3-U-1 to I.B2-Z123-Q3-U-42.

In another embodiment, compounds I are of formula I.B2 wherein Z is Z-123 and Q is Q4, and R and R⁴ are as per any row of Table U below, which compounds are named I.B2-Z123-Q4-U-1 to I.B2-Z123-Q4-U-42.

In another embodiment, compounds I are of formula I.B1 wherein Z is Z-124 and Q is Q1, and R and R⁴ are as per any row of Table U below, which compounds are named I.B1-Z124-Q1-U-1 to I.B1-Z124-Q1-U-42.

In another embodiment, compounds I are of formula I.B1 wherein Z is Z-124 and Q is Q2, and R and R⁴ are as per any row of Table U below, which compounds are named I.B1-Z124-Q2-U-1 to I.B1-Z124-Q2-U-42.

In another embodiment, compounds I are of formula I.B1 wherein Z is Z-124 and Q is Q3, and R and R⁴ are as per any row of Table U below, which compounds are named I.B1-Z124-Q3-U-1 to I.B1-Z124-Q3-U-42.

In another embodiment, compounds I are of formula I.B1 wherein Z is Z-124 and Q is Q4, and R and R⁴ are as per any row of Table U below, which compounds are named I.B1-Z124-Q4-U-1 to I.B1-Z124-Q4-U-42.

In another embodiment, compounds I are of formula I.B2 wherein Z is Z-124 and Q is Q1, and R and R⁴ are as per any row of Table U below, which compounds are named I.B2-Z124-Q1-U-1 to I.B2-Z124-Q1-U-42.

In another embodiment, compounds I are of formula I.B2 wherein Z is Z-124 and Q is Q2, and R and R⁴ are as per any row of Table U below, which compounds are named I.B2-Z124-Q2-U-1 to I.B2-Z124-Q2-U-42.

In another embodiment, compounds I are of formula I.B2 wherein Z is Z-124 and Q is Q3, and R and R⁴ are as per any row of Table U below, which compounds are named I.B2-Z124-Q3-U-1 to I.B2-Z124-Q3-U-42.

In another embodiment, compounds I are of formula I.B2 wherein Z is Z-124 and Q is Q4, and R and R⁴ are as per any row of Table U below, which compounds are named I.B2-Z124-Q4-U-1 to I.B2-Z124-Q4-U-42.

In another embodiment, compounds I are of formula I.B1 wherein Z is Z-125 and Q is Q1, and R and R⁴ are as per any row of Table U below, which compounds are named I.B1-Z125-Q1-U-1 to I.B1-Z125-Q1-U-42.

In another embodiment, compounds I are of formula I.B1 wherein Z is Z-125 and Q is Q2, and R and R⁴ are as per any row of Table U below, which compounds are named I.B1-Z125-Q2-U-1 to I.B1-Z125-Q2-U-42.

In another embodiment, compounds I are of formula I.B1 wherein Z is Z-125 and Q is Q3, and R and R⁴ are as per any row of Table U below, which compounds are named I.B1-Z125-Q3-U-1 to I.B1-Z125-Q3-U-42.

In another embodiment, compounds I are of formula I.B1 wherein Z is Z-125 and Q is Q4, and R and R⁴ are as per any row of Table U below, which compounds are named I.B1-Z125-Q4-U-1 to I.B1-Z125-Q4-U-42.

In another embodiment, compounds I are of formula I.B2 wherein Z is Z-125 and Q is Q1, and R and R⁴ are as per any row of Table U below, which compounds are named I.B2-Z125-Q1-U-1 to I.B2-Z125-Q1-U-42.

In another embodiment, compounds I are of formula I.B2 wherein Z is Z-125 and Q is Q2, and R and R⁴ are as per any row of Table U below, which compounds are named I.B2-Z125-Q2-U-1 to I.B2-Z125-Q2-U-42.

In another embodiment, compounds I are of formula I.B2 wherein Z is Z-125 and Q is Q3, and R and R⁴ are as per any row of Table U below, which compounds are named I.B2-Z125-Q3-U-1 to I.B2-Z125-Q3-U-42.

In another embodiment, compounds I are of formula I.B2 wherein Z is Z-125 and Q is Q4, and R and R⁴ are as per any row of Table U below, which compounds are named I.B2-Z125-Q4-U-1 to I.B2-Z125-Q4-U-42.

In another embodiment, compounds I are of formula I.B1 wherein Z is Z-126 and Q is Q1, and R and R⁴ are as per any row of Table U below, which compounds are named I.B1-Z126-Q1-U-1 to I.B1-Z126-Q1-U-42.

In another embodiment, compounds I are of formula I.B1 wherein Z is Z-126 and Q is Q2, and R and R⁴ are as per any row of Table U below, which compounds are named I.B1-Z126-Q2-U-1 to I.B1-Z126-Q2-U-42.

In another embodiment, compounds I are of formula I.B1 wherein Z is Z-126 and Q is Q3, and R and R⁴ are as per any row of Table U below, which compounds are named I.B1-Z126-Q3-U-1 to I.B1-Z126-Q3-U-42.

In another embodiment, compounds I are of formula I.B1 wherein Z is Z-126 and Q is Q4, and R and R⁴ are as per any row of Table U below, which compounds are named I.B1-Z126-Q4-U-1 to I.B1-Z126-Q4-U-42.

In another embodiment, compounds I are of formula I.B2 wherein Z is Z-126 and Q is Q1, and R and R⁴ are as per any row of Table U below, which compounds are named I.B2-Z126-Q1-U-1 to I.B2-Z126-Q1-U-42.

In another embodiment, compounds I are of formula I.B2 wherein Z is Z-126 and Q is Q2, and R and R⁴ are as per any row of Table U below, which compounds are named I.B2-Z126-Q2-U-1 to I.B2-Z126-Q2-U-42.

In another embodiment, compounds I are of formula I.B2 wherein Z is Z-126 and Q is Q3, and R and R⁴ are as per any row of Table U below, which compounds are named I.B2-Z126-Q3-U-1 to I.B2-Z126-Q3-U-42.

In another embodiment, compounds I are of formula I.B2 wherein Z is Z-126 and Q is Q4, and R and R⁴ are as per any row of Table U below, which compounds are named I.B2-Z126-Q4-U-1 to I.B2-Z126-Q4-U-42.

In another embodiment, compounds I are of formula I.B1 wherein Z is Z-127 and Q is Q1, and R and R⁴ are as per any row of Table U below, which compounds are named I.B1-Z127-Q1-U-1 to I.B1-Z127-Q1-U-42.

In another embodiment, compounds I are of formula I.B1 wherein Z is Z-127 and Q is Q2, and R and R⁴ are as per any row of Table U below, which compounds are named I.B1-Z127-Q2-U-1 to I.B1-Z127-Q2-U-42.

In another embodiment, compounds I are of formula I.B1 wherein Z is Z-127 and Q is Q3, and R and R⁴ are as per any row of Table U below, which compounds are named I.B1-Z127-Q3-U-1 to I.B1-Z127-Q3-U-42.

In another embodiment, compounds I are of formula I.B1 wherein Z is Z-127 and Q is Q4, and R and R⁴ are as per any row of Table U below, which compounds are named I.B1-Z127-Q4-U-1 to I.B1-Z127-Q4-U-42.

In another embodiment, compounds I are of formula I.B2 wherein Z is Z-127 and Q is Q1, and R and R⁴ are as per any row of Table U below, which compounds are named I.B2-Z127-Q1-U-1 to I.B2-Z127-Q1-U-42.

In another embodiment, compounds I are of formula I.B2 wherein Z is Z-127 and Q is Q2, and R and R⁴ are as per any row of Table U below, which compounds are named I.B2-Z127-Q2-U-1 to I.B2-Z127-Q2-U-42.

In another embodiment, compounds I are of formula I.B2 wherein Z is Z-127 and Q is Q3, and R and R⁴ are as per any row of Table U below, which compounds are named I.B2-Z127-Q3-U-1 to I.B2-Z127-Q3-U-42.

In another embodiment, compounds I are of formula I.B2 wherein Z is Z-127 and Q is Q4, and R and R⁴ are as per any row of Table U below, which compounds are named I.B2-Z127-Q4-U-1 to I.B2-Z127-Q4-U-42.

In another embodiment, compounds I are of formula I.B1 wherein Z is Z-128 and Q is Q1, and R and R⁴ are as per any row of Table U below, which compounds are named I.B1-Z128-Q1-U-1 to I.B1-Z128-Q1-U-42.

In another embodiment, compounds I are of formula I.B1 wherein Z is Z-128 and Q is Q2, and R and R⁴ are as per any row of Table U below, which compounds are named I.B1-Z128-Q2-U-1 to I.B1-Z128-Q2-U-42.

In another embodiment, compounds I are of formula I.B1 wherein Z is Z-128 and Q is Q3, and R and R⁴ are as per any row of Table U below, which compounds are named I.B1-Z128-Q3-U-1 to I.B1-Z128-Q3-U-42.

In another embodiment, compounds I are of formula I.B1 wherein Z is Z-128 and Q is Q4, and R and R⁴ are as per any row of Table U below, which compounds are named I.B1-Z128-Q4-U-1 to I.B1-Z128-Q4-U-42.

In another embodiment, compounds I are of formula I.B2 wherein Z is Z-128 and Q is Q1, and R and R⁴ are as per any row of Table U below, which compounds are named I.B2-Z128-Q1-U-1 to I.B2-Z128-Q1-U-42.

In another embodiment, compounds I are of formula I.B2 wherein Z is Z-128 and Q is Q2, and R and R⁴ are as per any row of Table U below, which compounds are named I.B2-Z128-Q2-U-1 to I.B2-Z128-Q2-U-42.

In another embodiment, compounds I are of formula I.B2 wherein Z is Z-128 and Q is Q3, and R and R⁴ are as per any row of Table U below, which compounds are named I.B2-Z128-Q3-U-1 to I.B2-Z128-Q3-U-42.

In another embodiment, compounds I are of formula I.B2 wherein Z is Z-128 and Q is Q4, and R and R⁴ are as per any row of Table U below, which compounds are named I.B2-Z128-Q4-U-1 to I.B2-Z128-Q4-U-42.

In another embodiment, compounds I are of formula I.B1 wherein Z is Z-129 and Q is Q1, and R and R⁴ are as per any row of Table U below, which compounds are named I.B1-Z129-Q1-U-1 to I.B1-Z129-Q1-U-42.

In another embodiment, compounds I are of formula I.B1 wherein Z is Z-129 and Q is Q2, and R and R⁴ are as per any row of Table U below, which compounds are named I.B1-Z129-Q2-U-1 to I.B1-Z129-Q2-U-42.

In another embodiment, compounds I are of formula I.B1 wherein Z is Z-129 and Q is Q3, and R and R⁴ are as per any row of Table U below, which compounds are named I.B1-Z129-Q3-U-1 to I.B1-Z129-Q3-U-42.

In another embodiment, compounds I are of formula I.B1 wherein Z is Z-129 and Q is Q4, and R and R⁴ are as per any row of Table U below, which compounds are named I.B1-Z129-Q4-U-1 to I.B1-Z129-Q4-U-42.

In another embodiment, compounds I are of formula I.B2 wherein Z is Z-129 and Q is Q1, and R and R⁴ are as per any row of Table U below, which compounds are named I.B2-Z129-Q1-U-1 to I.B2-Z129-Q1-U-42.

In another embodiment, compounds I are of formula I.B2 wherein Z is Z-129 and Q is Q2, and R and R⁴ are as per any row of Table U below, which compounds are named I.B2-Z129-Q2-U-1 to I.B2-Z129-Q2-U-42.

In another embodiment, compounds I are of formula I.B2 wherein Z is Z-129 and Q is Q3, and R and R⁴ are as per any row of Table U below, which compounds are named I.B2-Z129-Q3-U-1 to I.B2-Z129-Q3-U-42.

In another embodiment, compounds I are of formula I.B2 wherein Z is Z-129 and Q is Q4, and R and R⁴ are as per any row of Table U below, which compounds are named I.B2-Z129-Q4-U-1 to I.B2-Z129-Q4-U-42.

In another embodiment, compounds I are of formula I.B1 wherein Z is Z-130 and Q is Q1, and R and R⁴ are as per any row of Table U below, which compounds are named I.B1-Z130-Q1-U-1 to I.B1-Z130-Q1-U-42.

In another embodiment, compounds I are of formula I.B1 wherein Z is Z-130 and Q is Q2, and R and R⁴ are as per any row of Table U below, which compounds are named I.B1-Z130-Q2-U-1 to I.B1-Z130-Q2-U-42.

In another embodiment, compounds I are of formula I.B1 wherein Z is Z-130 and Q is Q3, and R and R⁴ are as per any row of Table U below, which compounds are named I.B1-Z130-Q3-U-1 to I.B1-Z130-Q3-U-42.

In another embodiment, compounds I are of formula I.B1 wherein Z is Z-130 and Q is Q4, and R and R⁴ are as per any row of Table U below, which compounds are named I.B1-Z130-Q4-U-1 to I.B1-Z130-Q4-U-42.

In another embodiment, compounds I are of formula I.B2 wherein Z is Z-130 and Q is Q1, and R and R⁴ are as per any row of Table U below, which compounds are named I.B2-Z130-Q1-U-1 to I.B2-Z130-Q1-U-42.

In another embodiment, compounds I are of formula I.B2 wherein Z is Z-130 and Q is Q2, and R and R⁴ are as per any row of Table U below, which compounds are named I.B2-Z130-Q2-U-1 to I.B2-Z130-Q2-U-42.

In another embodiment, compounds I are of formula I.B2 wherein Z is Z-130 and Q is Q3, and R and R⁴ are as per any row of Table U below, which compounds are named I.B2-Z130-Q3-U-1 to I.B2-Z130-Q3-U-42.

In another embodiment, compounds I are of formula I.B2 wherein Z is Z-130 and Q is Q4, and R and R⁴ are as per any row of Table U below, which compounds are named I.B2-Z130-Q4-U-1 to I.B2-Z130-Q4-U-42.

In another embodiment, compounds I are of formula I.B1 wherein Z is Z-131 and Q is Q1, and R and R⁴ are as per any row of Table U below, which compounds are named I.B1-Z131-Q1-U-1 to I.B1-Z131-Q1-U-42.

In another embodiment, compounds I are of formula I.B1 wherein Z is Z-131 and Q is Q2, and R and R⁴ are as per any row of Table U below, which compounds are named I.B1-Z131-Q2-U-1 to I.B1-Z131-Q2-U-42.

In another embodiment, compounds I are of formula I.B1 wherein Z is Z-131 and Q is Q3, and R and R⁴ are as per any row of Table U below, which compounds are named I.B1-Z131-Q3-U-1 to I.B1-Z131-Q3-U-42.

In another embodiment, compounds I are of formula I.B1 wherein Z is Z-131 and Q is Q4, and R and R⁴ are as per any row of Table U below, which compounds are named I.B1-Z131-Q4-U-1 to I.B1-Z131-Q4-U-42.

In another embodiment, compounds I are of formula I.B2 wherein Z is Z-131 and Q is Q1, and R and R⁴ are as per any row of Table U below, which compounds are named I.B2-Z131-Q1-U-1 to I.B2-Z131-Q1-U-42.

In another embodiment, compounds I are of formula I.B2 wherein Z is Z-131 and Q is Q2, and R and R⁴ are as per any row of Table U below, which compounds are named I.B2-Z131-Q2-U-1 to I.B2-Z131-Q2-U-42.

In another embodiment, compounds I are of formula I.B2 wherein Z is Z-131 and Q is Q3, and R and R⁴ are as per any row of Table U below, which compounds are named I.B2-Z131-Q3-U-1 to I.B2-Z131-Q3-U-42.

In another embodiment, compounds I are of formula I.B2 wherein Z is Z-131 and Q is Q4, and R and R⁴ are as per any row of Table U below, which compounds are named I.B2-Z131-Q4-U-1 to I.B2-Z131-Q4-U-42.

In another embodiment, compounds I are of formula I.B1 wherein Z is Z-132 and Q is Q1, and R and R⁴ are as per any row of Table U below, which compounds are named I.B1-Z132-Q1-U-1 to I.B1-Z132-Q1-U-42.

In another embodiment, compounds I are of formula I.B1 wherein Z is Z-132 and Q is Q2, and R and R⁴ are as per any row of Table U below, which compounds are named I.B1-Z132-Q2-U-1 to I.B1-Z132-Q2-U-42.

In another embodiment, compounds I are of formula I.B1 wherein Z is Z-132 and Q is Q3, and R and R⁴ are as per any row of Table U below, which compounds are named I.B1-Z132-Q3-U-1 to I.B1-Z132-Q3-U-42.

In another embodiment, compounds I are of formula I.B1 wherein Z is Z-132 and Q is Q4, and R and R⁴ are as per any row of Table U below, which compounds are named I.B1-Z132-Q4-U-1 to I.B1-Z132-Q4-U-42.

In another embodiment, compounds I are of formula I.B2 wherein Z is Z-132 and Q is Q1, and R and R⁴ are as per any row of Table U below, which compounds are named I.B2-Z132-Q1-U-1 to I.B2-Z132-Q1-U-42.

In another embodiment, compounds I are of formula I.B2 wherein Z is Z-132 and Q is Q2, and R and R⁴ are as per any row of Table U below, which compounds are named I.B2-Z132-Q2-U-1 to I.B2-Z132-Q2-U-42.

In another embodiment, compounds I are of formula I.B2 wherein Z is Z-132 and Q is Q3, and R and R⁴ are as per any row of Table U below, which compounds are named I.B2-Z132-Q3-U-1 to I.B2-Z132-Q3-U-42.

In another embodiment, compounds I are of formula I.B2 wherein Z is Z-132 and Q is Q4, and R and R⁴ are as per any row of Table U below, which compounds are named I.B2-Z132-Q4-U-1 to I.B2-Z132-Q4-U-42.

In another embodiment, compounds I are of formula I.B1 wherein Z is Z-133 and Q is Q1, and R and R⁴ are as per any row of Table U below, which compounds are named I.B1-Z133-Q1-U-1 to I.B1-Z133-Q1-U-42.

In another embodiment, compounds I are of formula I.B1 wherein Z is Z-133 and Q is Q2, and R and R⁴ are as per any row of Table U below, which compounds are named I.B1-Z133-Q2-U-1 to I.B1-Z133-Q2-U-42.

In another embodiment, compounds I are of formula I.B1 wherein Z is Z-133 and Q is Q3, and R and R⁴ are as per any row of Table U below, which compounds are named I.B1-Z133-Q3-U-1 to I.B1-Z133-Q3-U-42.

In another embodiment, compounds I are of formula I.B1 wherein Z is Z-133 and Q is Q4, and R and R⁴ are as per any row of Table U below, which compounds are named I.B1-Z133-Q4-U-1 to I.B1-Z133-Q4-U-42.

In another embodiment, compounds I are of formula I.B2 wherein Z is Z-133 and Q is Q1, and R and R⁴ are as per any row of Table U below, which compounds are named I.B2-Z133-Q1-U-1 to I.B2-Z133-Q1-U-42.

In another embodiment, compounds I are of formula I.B2 wherein Z is Z-133 and Q is Q2, and R and R⁴ are as per any row of Table U below, which compounds are named I.B2-Z133-Q2-U-1 to I.B2-Z133-Q2-U-42.

In another embodiment, compounds I are of formula I.B2 wherein Z is Z-133 and Q is Q3, and R and R⁴ are as per any row of Table U below, which compounds are named I.B2-Z133-Q3-U-1 to I.B2-Z133-Q3-U-42.

In another embodiment, compounds I are of formula I.B2 wherein Z is Z-133 and Q is Q4, and R and R⁴ are as per any row of Table U below, which compounds are named I.B2-Z133-Q4-U-1 to I.B2-Z133-Q4-U-42.

In another embodiment, compounds I are of formula I.B1 wherein Z is Z-134 and Q is Q1, and R and R⁴ are as per any row of Table U below, which compounds are named I.B1-Z134-Q1-U-1 to I.B1-Z134-Q1-U-42.

In another embodiment, compounds I are of formula I.B1 wherein Z is Z-134 and Q is Q2, and R and R⁴ are as per any row of Table U below, which compounds are named I.B1-Z134-Q2-U-1 to I.B1-Z134-Q2-U-42.

In another embodiment, compounds I are of formula I.B1 wherein Z is Z-134 and Q is Q3, and R and R⁴ are as per any row of Table U below, which compounds are named I.B1-Z134-Q3-U-1 to I.B1-Z134-Q3-U-42.

In another embodiment, compounds I are of formula I.B1 wherein Z is Z-134 and Q is Q4, and R and R⁴ are as per any row of Table U below, which compounds are named I.B1-Z134-Q4-U-1 to I.B1-Z134-Q4-U-42.

In another embodiment, compounds I are of formula I.B2 wherein Z is Z-134 and Q is Q1, and R and R⁴ are as per any row of Table U below, which compounds are named I.B2-Z134-Q1-U-1 to I.B2-Z134-Q1-U-42.

In another embodiment, compounds I are of formula I.B2 wherein Z is Z-134 and Q is Q2, and R and R⁴ are as per any row of Table U below, which compounds are named I.B2-Z134-Q2-U-1 to I.B2-Z134-Q2-U-42.

In another embodiment, compounds I are of formula I.B2 wherein Z is Z-134 and Q is Q3, and R and R⁴ are as per any row of Table U below, which compounds are named I.B2-Z134-Q3-U-1 to I.B2-Z134-Q3-U-42.

In another embodiment, compounds I are of formula I.B2 wherein Z is Z-134 and Q is Q4, and R and R⁴ are as per any row of Table U below, which compounds are named I.B2-Z134-Q4-U-1 to I.B2-Z134-Q4-U-42.

In another embodiment, compounds I are of formula I.B1 wherein Z is Z-135 and Q is Q1, and R and R⁴ are as per any row of Table U below, which compounds are named I.B1-Z135-Q1-U-1 to I.B1-Z135-Q1-U-42.

In another embodiment, compounds I are of formula I.B1 wherein Z is Z-135 and Q is Q2, and R and R⁴ are as per any row of Table U below, which compounds are named I.B1-Z135-Q2-U-1 to I.B1-Z135-Q2-U-42.

In another embodiment, compounds I are of formula I.B1 wherein Z is Z-135 and Q is Q3, and R and R⁴ are as per any row of Table U below, which compounds are named I.B1-Z135-Q3-U-1 to I.B1-Z135-Q3-U-42.

In another embodiment, compounds I are of formula I.B1 wherein Z is Z-135 and Q is Q4, and R and R⁴ are as per any row of Table U below, which compounds are named I.B1-Z135-Q4-U-1 to I.B1-Z135-Q4-U-42.

In another embodiment, compounds I are of formula I.B2 wherein Z is Z-135 and Q is Q1, and R and R⁴ are as per any row of Table U below, which compounds are named I.B2-Z135-Q1-U-1 to I.B2-Z135-Q1-U-42.

In another embodiment, compounds I are of formula I.B2 wherein Z is Z-135 and Q is Q2, and R and R⁴ are as per any row of Table U below, which compounds are named I.B2-Z135-Q2-U-1 to I.B2-Z135-Q2-U-42.

In another embodiment, compounds I are of formula I.B2 wherein Z is Z-135 and Q is Q3, and R and R⁴ are as per any row of Table U below, which compounds are named I.B2-Z135-Q3-U-1 to I.B2-Z135-Q3-U-42.

In another embodiment, compounds I are of formula I.B2 wherein Z is Z-135 and Q is Q4, and R and R⁴ are as per any row of Table U below, which compounds are named I.B2-Z135-Q4-U-1 to I.B2-Z135-Q4-U-42.

In another embodiment, compounds I are of formula I.B1 wherein Z is Z-136 and Q is Q1, and R and R⁴ are as per any row of Table U below, which compounds are named I.B1-Z136-Q1-U-1 to I.B1-Z136-Q1-U-42.

In another embodiment, compounds I are of formula I.B1 wherein Z is Z-136 and Q is Q2, and R and R⁴ are as per any row of Table U below, which compounds are named I.B1-Z136-Q2-U-1 to I.B1-Z136-Q2-U-42.

In another embodiment, compounds I are of formula I.B1 wherein Z is Z-136 and Q is Q3, and R and R⁴ are as per any row of Table U below, which compounds are named I.B1-Z136-Q3-U-1 to I.B1-Z136-Q3-U-42.

In another embodiment, compounds I are of formula I.B1 wherein Z is Z-136 and Q is Q4, and R and R⁴ are as per any row of Table U below, which compounds are named I.B1-Z136-Q4-U-1 to I.B1-Z136-Q4-U-42.

In another embodiment, compounds I are of formula I.B2 wherein Z is Z-136 and Q is Q1, and R and R⁴ are as per any row of Table U below, which compounds are named I.B2-Z136-Q1-U-1 to I.B2-Z136-Q1-U-42.

In another embodiment, compounds I are of formula I.B2 wherein Z is Z-136 and Q is Q2, and R and R⁴ are as per any row of Table U below, which compounds are named I.B2-Z136-Q2-U-1 to I.B2-Z136-Q2-U-42.

In another embodiment, compounds I are of formula I.B2 wherein Z is Z-136 and Q is Q3, and R and R⁴ are as per any row of Table U below, which compounds are named I.B2-Z136-Q3-U-1 to I.B2-Z136-Q3-U-42.

In another embodiment, compounds I are of formula I.B2 wherein Z is Z-136 and Q is Q4, and R and R⁴ are as per any row of Table U below, which compounds are named I.B2-Z136-Q4-U-1 to I.B2-Z136-Q4-U-42.

In another embodiment, compounds I are of formula I.B1 wherein Z is Z-137 and Q is Q1, and R and R⁴ are as per any row of Table U below, which compounds are named I.B1-Z137-Q1-U-1 to I.B1-Z137-Q1-U-42.

In another embodiment, compounds I are of formula I.B1 wherein Z is Z-137 and Q is Q2, and R and R⁴ are as per any row of Table U below, which compounds are named I.B1-Z137-Q2-U-1 to I.B1-Z137-Q2-U-42.

In another embodiment, compounds I are of formula I.B1 wherein Z is Z-137 and Q is Q3, and R and R⁴ are as per any row of Table U below, which compounds are named I.B1-Z137-Q3-U-1 to I.B1-Z137-Q3-U-42.

In another embodiment, compounds I are of formula I.B1 wherein Z is Z-137 and Q is Q4, and R and R⁴ are as per any row of Table U below, which compounds are named I.B1-Z137-Q4-U-1 to I.B1-Z137-Q4-U-42.

In another embodiment, compounds I are of formula I.B2 wherein Z is Z-137 and Q is Q1, and R and R⁴ are as per any row of Table U below, which compounds are named I.B2-Z137-Q1-U-1 to I.B2-Z137-Q1-U-42.

In another embodiment, compounds I are of formula I.B2 wherein Z is Z-137 and Q is Q2, and R and R⁴ are as per any row of Table U below, which compounds are named I.B2-Z137-Q2-U-1 to I.B2-Z137-Q2-U-42.

In another embodiment, compounds I are of formula I.B2 wherein Z is Z-137 and Q is Q3, and R and R⁴ are as per any row of Table U below, which compounds are named I.B2-Z137-Q3-U-1 to I.B2-Z137-Q3-U-42.

In another embodiment, compounds I are of formula I.B2 wherein Z is Z-137 and Q is Q4, and R and R⁴ are as per any row of Table U below, which compounds are named I.B2-Z137-Q4-U-1 to I.B2-Z137-Q4-U-42.

In another embodiment, compounds I are of formula I.B1 wherein Z is Z-138 and Q is Q1, and R and R⁴ are as per any row of Table U below, which compounds are named I.B1-Z138-Q1-U-1 to I.B1-Z138-Q1-U-42.

In another embodiment, compounds I are of formula I.B1 wherein Z is Z-138 and Q is Q2, and R and R⁴ are as per any row of Table U below, which compounds are named I.B1-Z138-Q2-U-1 to I.B1-Z138-Q2-U-42.

In another embodiment, compounds I are of formula I.B1 wherein Z is Z-138 and Q is Q3, and R and R⁴ are as per any row of Table U below, which compounds are named I.B1-Z138-Q3-U-1 to I.B1-Z138-Q3-U-42.

In another embodiment, compounds I are of formula I.B1 wherein Z is Z-138 and Q is Q4, and R and R⁴ are as per any row of Table U below, which compounds are named I.B1-Z138-Q4-U-1 to I.B1-Z138-Q4-U-42.

In another embodiment, compounds I are of formula I.B2 wherein Z is Z-138 and Q is Q1, and R and R⁴ are as per any row of Table U below, which compounds are named I.B2-Z138-Q1-U-1 to I.B2-Z138-Q1-U-42.

In another embodiment, compounds I are of formula I.B2 wherein Z is Z-138 and Q is Q2, and R and R⁴ are as per any row of Table U below, which compounds are named I.B2-Z138-Q2-U-1 to I.B2-Z138-Q2-U-42.

In another embodiment, compounds I are of formula I.B2 wherein Z is Z-138 and Q is Q3, and R and R⁴ are as per any row of Table U below, which compounds are named I.B2-Z138-Q3-U-1 to I.B2-Z138-Q3-U-42.

In another embodiment, compounds I are of formula I.B2 wherein Z is Z-138 and Q is Q4, and R and R⁴ are as per any row of Table U below, which compounds are named I.B2-Z138-Q4-U-1 to I.B2-Z138-Q4-U-42.

In another embodiment, compounds I are of formula I.B1 wherein Z is Z-139 and Q is Q1, and R and R⁴ are as per any row of Table U below, which compounds are named I.B1-Z139-Q1-U-1 to I.B1-Z139-Q1-U-42.

In another embodiment, compounds I are of formula I.B1 wherein Z is Z-139 and Q is Q2, and R and R⁴ are as per any row of Table U below, which compounds are named I.B1-Z139-Q2-U-1 to I.B1-Z139-Q2-U-42.

In another embodiment, compounds I are of formula I.B1 wherein Z is Z-139 and Q is Q3, and R and R⁴ are as per any row of Table U below, which compounds are named I.B1-Z139-Q3-U-1 to I.B1-Z139-Q3-U-42.

In another embodiment, compounds I are of formula I.B1 wherein Z is Z-139 and Q is Q4, and R and R⁴ are as per any row of Table U below, which compounds are named I.B1-Z139-Q4-U-1 to I.B1-Z139-Q4-U-42.

In another embodiment, compounds I are of formula I.B2 wherein Z is Z-139 and Q is Q1, and R and R⁴ are as per any row of Table U below, which compounds are named I.B2-Z139-Q1-U-1 to I.B2-Z139-Q1-U-42.

In another embodiment, compounds I are of formula I.B2 wherein Z is Z-139 and Q is Q2, and R and R⁴ are as per any row of Table U below, which compounds are named I.B2-Z139-Q2-U-1 to I.B2-Z139-Q2-U-42.

In another embodiment, compounds I are of formula I.B2 wherein Z is Z-139 and Q is Q3, and R and R⁴ are as per any row of Table U below, which compounds are named I.B2-Z139-Q3-U-1 to I.B2-Z139-Q3-U-42.

In another embodiment, compounds I are of formula I.B2 wherein Z is Z-139 and Q is Q4, and R and R⁴ are as per any row of Table U below, which compounds are named I.B2-Z139-Q4-U-1 to I.B2-Z139-Q4-U-42.

In another embodiment, compounds I are of formula I.B1 wherein Z is Z-140 and Q is Q1, and R and R⁴ are as per any row of Table U below, which compounds are named I.B1-Z140-Q1-U-1 to I.B1-Z140-Q1-U-42.

In another embodiment, compounds I are of formula I.B1 wherein Z is Z-140 and Q is Q2, and R and R⁴ are as per any row of Table U below, which compounds are named I.B1-Z140-Q2-U-1 to I.B1-Z140-Q2-U-42.

In another embodiment, compounds I are of formula I.B1 wherein Z is Z-140 and Q is Q3, and R and R⁴ are as per any row of Table U below, which compounds are named I.B1-Z140-Q3-U-1 to I.B1-Z140-Q3-U-42.

In another embodiment, compounds I are of formula I.B1 wherein Z is Z-140 and Q is Q4, and R and R⁴ are as per any row of Table U below, which compounds are named I.B1-Z140-Q4-U-1 to I.B1-Z140-Q4-U-42.

In another embodiment, compounds I are of formula I.B2 wherein Z is Z-140 and Q is Q1, and R and R⁴ are as per any row of Table U below, which compounds are named I.B2-Z140-Q1-U-1 to I.B2-Z140-Q1-U-42.

In another embodiment, compounds I are of formula I.B2 wherein Z is Z-140 and Q is Q2, and R and R⁴ are as per any row of Table U below, which compounds are named I.B2-Z140-Q2-U-1 to I.B2-Z140-Q2-U-42.

In another embodiment, compounds I are of formula I.B2 wherein Z is Z-140 and Q is Q3, and R and R⁴ are as per any row of Table U below, which compounds are named I.B2-Z140-Q3-U-1 to I.B2-Z140-Q3-U-42.

In another embodiment, compounds I are of formula I.B2 wherein Z is Z-140 and Q is Q4, and R and R⁴ are as per any row of Table U below, which compounds are named I.B2-Z140-Q4-U-1 to I.B2-Z140-Q4-U-42.

In another embodiment, compounds I are of formula I.B1 wherein Z is Z-141 and Q is Q1, and R and R⁴ are as per any row of Table U below, which compounds are named I.B1-Z141-Q1-U-1 to I.B1-Z141-Q1-U-42.

In another embodiment, compounds I are of formula I.B1 wherein Z is Z-141 and Q is Q2, and R and R⁴ are as per any row of Table U below, which compounds are named I.B1-Z141-Q2-U-1 to I.B1-Z141-Q2-U-42.

In another embodiment, compounds I are of formula I.B1 wherein Z is Z-141 and Q is Q3, and R and R⁴ are as per any row of Table U below, which compounds are named I.B1-Z141-Q3-U-1 to I.B1-Z141-Q3-U-42.

In another embodiment, compounds I are of formula I.B1 wherein Z is Z-141 and Q is Q4, and R and R⁴ are as per any row of Table U below, which compounds are named I.B1-Z141-Q4-U-1 to I.B1-Z141-Q4-U-42.

In another embodiment, compounds I are of formula I.B2 wherein Z is Z-141 and Q is Q1, and R and R⁴ are as per any row of Table U below, which compounds are named I.B2-Z141-Q1-U-1 to I.B2-Z141-Q1-U-42.

In another embodiment, compounds I are of formula I.B2 wherein Z is Z-141 and Q is Q2, and R and R⁴ are as per any row of Table U below, which compounds are named I.B2-Z141-Q2-U-1 to I.B2-Z141-Q2-U-42.

In another embodiment, compounds I are of formula I.B2 wherein Z is Z-141 and Q is Q3, and R and R⁴ are as per any row of Table U below, which compounds are named I.B2-Z141-Q3-U-1 to I.B2-Z141-Q3-U-42.

In another embodiment, compounds I are of formula I.B2 wherein Z is Z-141 and Q is Q4, and R and R⁴ are as per any row of Table U below, which compounds are named I.B2-Z141-Q4-U-1 to I.B2-Z141-Q4-U-42.

In another embodiment, compounds I are of formula I.B1 wherein Z is Z-142 and Q is Q1, and R and R⁴ are as per any row of Table U below, which compounds are named I.B1-Z142-Q1-U-1 to I.B1-Z142-Q1-U-42.

In another embodiment, compounds I are of formula I.B1 wherein Z is Z-142 and Q is Q2, and R and R⁴ are as per any row of Table U below, which compounds are named I.B1-Z142-Q2-U-1 to I.B1-Z142-Q2-U-42.

In another embodiment, compounds I are of formula I.B1 wherein Z is Z-142 and Q is Q3, and R and R⁴ are as per any row of Table U below, which compounds are named I.B1-Z142-Q3-U-1 to I.B1-Z142-Q3-U-42.

In another embodiment, compounds I are of formula I.B1 wherein Z is Z-142 and Q is Q4, and R and R⁴ are as per any row of Table U below, which compounds are named I.B1-Z142-Q4-U-1 to I.B1-Z142-Q4-U-42.

In another embodiment, compounds I are of formula I.B2 wherein Z is Z-142 and Q is Q1, and R and R⁴ are as per any row of Table U below, which compounds are named I.B2-Z142-Q1-U-1 to I.B2-Z142-Q1-U-42.

In another embodiment, compounds I are of formula I.B2 wherein Z is Z-142 and Q is Q2, and R and R⁴ are as per any row of Table U below, which compounds are named I.B2-Z142-Q2-U-1 to I.B2-Z142-Q2-U-42.

In another embodiment, compounds I are of formula I.B2 wherein Z is Z-142 and Q is Q3, and R and R⁴ are as per any row of Table U below, which compounds are named I.B2-Z142-Q3-U-1 to I.B2-Z142-Q3-U-42.

In another embodiment, compounds I are of formula I.B2 wherein Z is Z-142 and Q is Q4, and R and R⁴ are as per any row of Table U below, which compounds are named I.B2-Z142-Q4-U-1 to I.B2-Z142-Q4-U-42.

In another embodiment, compounds I are of formula I.B1 wherein Z is Z-143 and Q is Q1, and R and R⁴ are as per any row of Table U below, which compounds are named I.B1-Z143-Q1-U-1 to I.B1-Z143-Q1-U-42.

In another embodiment, compounds I are of formula I.B1 wherein Z is Z-143 and Q is Q2, and R and R⁴ are as per any row of Table U below, which compounds are named I.B1-Z143-Q2-U-1 to I.B1-Z143-Q2-U-42.

In another embodiment, compounds I are of formula I.B1 wherein Z is Z-143 and Q is Q3, and R and R⁴ are as per any row of Table U below, which compounds are named I.B1-Z143-Q3-U-1 to I.B1-Z143-Q3-U-42.

In another embodiment, compounds I are of formula I.B1 wherein Z is Z-143 and Q is Q4, and R and R⁴ are as per any row of Table U below, which compounds are named I.B1-Z143-Q4-U-1 to I.B1-Z143-Q4-U-42.

In another embodiment, compounds I are of formula I.B2 wherein Z is Z-143 and Q is Q1, and R and R⁴ are as per any row of Table U below, which compounds are named I.B2-Z143-Q1-U-1 to I.B2-Z143-Q1-U-42.

In another embodiment, compounds I are of formula I.B2 wherein Z is Z-143 and Q is Q2, and R and R⁴ are as per any row of Table U below, which compounds are named I.B2-Z143-Q2-U-1 to I.B2-Z143-Q2-U-42.

In another embodiment, compounds I are of formula I.B2 wherein Z is Z-143 and Q is Q3, and R and R⁴ are as per any row of Table U below, which compounds are named I.B2-Z143-Q3-U-1 to I.B2-Z143-Q3-U-42.

In another embodiment, compounds I are of formula I.B2 wherein Z is Z-143 and Q is Q4, and R and R⁴ are as per any row of Table U below, which compounds are named I.B2-Z143-Q4-U-1 to I.B2-Z143-Q4-U-42.

In another embodiment, compounds I are of formula I.B1 wherein Z is Z-144 and Q is Q1, and R and R⁴ are as per any row of Table U below, which compounds are named I.B1-Z144-Q1-U-1 to I.B1-Z144-Q1-U-42.

In another embodiment, compounds I are of formula I.B1 wherein Z is Z-144 and Q is Q2, and R and R⁴ are as per any row of Table U below, which compounds are named I.B1-Z144-Q2-U-1 to I.B1-Z144-Q2-U-42.

In another embodiment, compounds I are of formula I.B1 wherein Z is Z-144 and Q is Q3, and R and R⁴ are as per any row of Table U below, which compounds are named I.B1-Z144-Q3-U-1 to I.B1-Z144-Q3-U-42.

In another embodiment, compounds I are of formula I.B1 wherein Z is Z-144 and Q is Q4, and R and R⁴ are as per any row of Table U below, which compounds are named I.B1-Z144-Q4-U-1 to I.B1-Z144-Q4-U-42.

In another embodiment, compounds I are of formula I.B2 wherein Z is Z-144 and Q is Q1, and R and R⁴ are as per any row of Table U below, which compounds are named I.B2-Z144-Q1-U-1 to I.B2-Z144-Q1-U-42.

In another embodiment, compounds I are of formula I.B2 wherein Z is Z-144 and Q is Q2, and R and R⁴ are as per any row of Table U below, which compounds are named I.B2-Z144-Q2-U-1 to I.B2-Z144-Q2-U-42.

In another embodiment, compounds I are of formula I.B2 wherein Z is Z-144 and Q is Q3, and R and R⁴ are as per any row of Table U below, which compounds are named I.B2-Z144-Q3-U-1 to I.B2-Z144-Q3-U-42.

In another embodiment, compounds I are of formula I.B2 wherein Z is Z-144 and Q is Q4, and R and R⁴ are as per any row of Table U below, which compounds are named I.B2-Z144-Q4-U-1 to I.B2-Z144-Q4-U-42.

In another embodiment, compounds I are of formula I.B1 wherein Z is Z-145 and Q is Q1, and R and R⁴ are as per any row of Table U below, which compounds are named I.B1-Z145-Q1-U-1 to I.B1-Z145-Q1-U-42.

In another embodiment, compounds I are of formula I.B1 wherein Z is Z-145 and Q is Q2, and R and R⁴ are as per any row of Table U below, which compounds are named I.B1-Z145-Q2-U-1 to I.B1-Z145-Q2-U-42.

In another embodiment, compounds I are of formula I.B1 wherein Z is Z-145 and Q is Q3, and R and R⁴ are as per any row of Table U below, which compounds are named I.B1-Z145-Q3-U-1 to I.B1-Z145-Q3-U-42.

In another embodiment, compounds I are of formula I.B1 wherein Z is Z-145 and Q is Q4, and R and R⁴ are as per any row of Table U below, which compounds are named I.B1-Z145-Q4-U-1 to I.B1-Z145-Q4-U-42.

In another embodiment, compounds I are of formula I.B2 wherein Z is Z-145 and Q is Q1, and R and R⁴ are as per any row of Table U below, which compounds are named I.B2-Z145-Q1-U-1 to I.B2-Z145-Q1-U-42.

In another embodiment, compounds I are of formula I.B2 wherein Z is Z-145 and Q is Q2, and R and R⁴ are as per any row of Table U below, which compounds are named I.B2-Z145-Q2-U-1 to I.B2-Z145-Q2-U-42.

In another embodiment, compounds I are of formula I.B2 wherein Z is Z-145 and Q is Q3, and R and R⁴ are as per any row of Table U below, which compounds are named I.B2-Z145-Q3-U-1 to I.B2-Z145-Q3-U-42.

In another embodiment, compounds I are of formula I.B2 wherein Z is Z-145 and Q is Q4, and R and R⁴ are as per any row of Table U below, which compounds are named I.B2-Z145-Q4-U-1 to I.B2-Z145-Q4-U-42.

In another embodiment, compounds I are of formula I.B1 wherein Z is Z-146 and Q is Q1, and R and R⁴ are as per any row of Table U below, which compounds are named I.B1-Z146-Q1-U-1 to I.B1-Z146-Q1-U-42.

In another embodiment, compounds I are of formula I.B1 wherein Z is Z-146 and Q is Q2, and R and R⁴ are as per any row of Table U below, which compounds are named I.B1-Z146-Q2-U-1 to I.B1-Z146-Q2-U-42.

In another embodiment, compounds I are of formula I.B1 wherein Z is Z-146 and Q is Q3, and R and R⁴ are as per any row of Table U below, which compounds are named I.B1-Z146-Q3-U-1 to I.B1-Z146-Q3-U-42.

In another embodiment, compounds I are of formula I.B1 wherein Z is Z-146 and Q is Q4, and R and R⁴ are as per any row of Table U below, which compounds are named I.B1-Z146-Q4-U-1 to I.B1-Z146-Q4-U-42.

In another embodiment, compounds I are of formula I.B2 wherein Z is Z-146 and Q is Q1, and R and R⁴ are as per any row of Table U below, which compounds are named I.B2-Z146-Q1-U-1 to I.B2-Z146-Q1-U-42.

In another embodiment, compounds I are of formula I.B2 wherein Z is Z-146 and Q is Q2, and R and R⁴ are as per any row of Table U below, which compounds are named I.B2-Z146-Q2-U-1 to I.B2-Z146-Q2-U-42.

In another embodiment, compounds I are of formula I.B2 wherein Z is Z-146 and Q is Q3, and R and R⁴ are as per any row of Table U below, which compounds are named I.B2-Z146-Q3-U-1 to I.B2-Z146-Q3-U-42.

In another embodiment, compounds I are of formula I.B2 wherein Z is Z-146 and Q is Q4, and R and R⁴ are as per any row of Table U below, which compounds are named I.B2-Z146-Q4-U-1 to I.B2-Z146-Q4-U-42.

In another embodiment, compounds I are of formula I.B1 wherein Z is Z-147 and Q is Q1, and R and R⁴ are as per any row of Table U below, which compounds are named I.B1-Z147-Q1-U-1 to I.B1-Z147-Q1-U-42.

In another embodiment, compounds I are of formula I.B1 wherein Z is Z-147 and Q is Q2, and R and R⁴ are as per any row of Table U below, which compounds are named I.B1-Z147-Q2-U-1 to I.B1-Z147-Q2-U-42.

In another embodiment, compounds I are of formula I.B1 wherein Z is Z-147 and Q is Q3, and R and R⁴ are as per any row of Table U below, which compounds are named I.B1-Z147-Q3-U-1 to I.B1-Z147-Q3-U-42.

In another embodiment, compounds I are of formula I.B1 wherein Z is Z-147 and Q is Q4, and R and R⁴ are as per any row of Table U below, which compounds are named I.B1-Z147-Q4-U-1 to I.B1-Z147-Q4-U-42.

In another embodiment, compounds I are of formula I.B2 wherein Z is Z-147 and Q is Q1, and R and R⁴ are as per any row of Table U below, which compounds are named I.B2-Z147-Q1-U-1 to I.B2-Z147-Q1-U-42.

In another embodiment, compounds I are of formula I.B2 wherein Z is Z-147 and Q is Q2, and R and R⁴ are as per any row of Table U below, which compounds are named I.B2-Z147-Q2-U-1 to I.B2-Z147-Q2-U-42.

In another embodiment, compounds I are of formula I.B2 wherein Z is Z-147 and Q is Q3, and R and R⁴ are as per any row of Table U below, which compounds are named I.B2-Z147-Q3-U-1 to I.B2-Z147-Q3-U-42.

In another embodiment, compounds I are of formula I.B2 wherein Z is Z-147 and Q is Q4, and R and R⁴ are as per any row of Table U below, which compounds are named I.B2-Z147-Q4-U-1 to I.B2-Z147-Q4-U-42.

In another embodiment, compounds I are of formula I.B1 wherein Z is Z-148 and Q is Q1, and R and R⁴ are as per any row of Table U below, which compounds are named I.B1-Z148-Q1-U-1 to I.B1-Z148-Q1-U-42.

In another embodiment, compounds I are of formula I.B1 wherein Z is Z-148 and Q is Q2, and R and R⁴ are as per any row of Table U below, which compounds are named I.B1-Z148-Q2-U-1 to I.B1-Z148-Q2-U-42.

In another embodiment, compounds I are of formula I.B1 wherein Z is Z-148 and Q is Q3, and R and R⁴ are as per any row of Table U below, which compounds are named I.B1-Z148-Q3-U-1 to I.B1-Z148-Q3-U-42.

In another embodiment, compounds I are of formula I.B1 wherein Z is Z-148 and Q is Q4, and R and R⁴ are as per any row of Table U below, which compounds are named I.B1-Z148-Q4-U-1 to I.B1-Z148-Q4-U-42.

In another embodiment, compounds I are of formula I.B2 wherein Z is Z-148 and Q is Q1, and R and R⁴ are as per any row of Table U below, which compounds are named I.B2-Z148-Q1-U-1 to I.B2-Z148-Q1-U-42.

In another embodiment, compounds I are of formula I.B2 wherein Z is Z-148 and Q is Q2, and R and R⁴ are as per any row of Table U below, which compounds are named I.B2-Z148-Q2-U-1 to I.B2-Z148-Q2-U-42.

In another embodiment, compounds I are of formula I.B2 wherein Z is Z-148 and Q is Q3, and R and R⁴ are as per any row of Table U below, which compounds are named I.B2-Z148-Q3-U-1 to I.B2-Z148-Q3-U-42.

In another embodiment, compounds I are of formula I.B2 wherein Z is Z-148 and Q is Q4, and R and R⁴ are as per any row of Table U below, which compounds are named I.B2-Z148-Q4-U-1 to I.B2-Z148-Q4-U-42.

In another embodiment, compounds I are of formula I.B1 wherein Z is Z-149 and Q is Q1, and R and R⁴ are as per any row of Table U below, which compounds are named I.B1-Z149-Q1-U-1 to I.B1-Z149-Q1-U-42.

In another embodiment, compounds I are of formula I.B1 wherein Z is Z-149 and Q is Q2, and R and R⁴ are as per any row of Table U below, which compounds are named I.B1-Z149-Q2-U-1 to I.B1-Z149-Q2-U-42.

In another embodiment, compounds I are of formula I.B1 wherein Z is Z-149 and Q is Q3, and R and R⁴ are as per any row of Table U below, which compounds are named I.B1-Z149-Q3-U-1 to I.B1-Z149-Q3-U-42.

In another embodiment, compounds I are of formula I.B1 wherein Z is Z-149 and Q is Q4, and R and R⁴ are as per any row of Table U below, which compounds are named I.B1-Z149-Q4-U-1 to I.B1-Z149-Q4-U-42.

In another embodiment, compounds I are of formula I.B2 wherein Z is Z-149 and Q is Q1, and R and R⁴ are as per any row of Table U below, which compounds are named I.B2-Z149-Q1-U-1 to I.B2-Z149-Q1-U-42.

In another embodiment, compounds I are of formula I.B2 wherein Z is Z-149 and Q is Q2, and R and R⁴ are as per any row of Table U below, which compounds are named I.B2-Z149-Q2-U-1 to I.B2-Z149-Q2-U-42.

In another embodiment, compounds I are of formula I.B2 wherein Z is Z-149 and Q is Q3, and R and R⁴ are as per any row of Table U below, which compounds are named I.B2-Z149-Q3-U-1 to I.B2-Z149-Q3-U-42.

In another embodiment, compounds I are of formula I.B2 wherein Z is Z-149 and Q is Q4, and R and R⁴ are as per any row of Table U below, which compounds are named I.B2-Z149-Q4-U-1 to I.B2-Z149-Q4-U-42.

In another embodiment, compounds I are of formula I.B1 wherein Z is Z-150 and Q is Q1, and R and R⁴ are as per any row of Table U below, which compounds are named I.B1-Z150-Q1-U-1 to I.B1-Z150-Q1-U-42.

In another embodiment, compounds I are of formula I.B1 wherein Z is Z-150 and Q is Q2, and R and R⁴ are as per any row of Table U below, which compounds are named I.B1-Z150-Q2-U-1 to I.B1-Z150-Q2-U-42.

In another embodiment, compounds I are of formula I.B1 wherein Z is Z-150 and Q is Q3, and R and R⁴ are as per any row of Table U below, which compounds are named I.B1-Z150-Q3-U-1 to I.B1-Z150-Q3-U-42.

In another embodiment, compounds I are of formula I.B1 wherein Z is Z-150 and Q is Q4, and R and R⁴ are as per any row of Table U below, which compounds are named I.B1-Z150-Q4-U-1 to I.B1-Z150-Q4-U-42.

In another embodiment, compounds I are of formula I.B2 wherein Z is Z-150 and Q is Q1, and R and R⁴ are as per any row of Table U below, which compounds are named I.B2-Z150-Q1-U-1 to I.B2-Z150-Q1-U-42.

In another embodiment, compounds I are of formula I.B2 wherein Z is Z-150 and Q is Q2, and R and R⁴ are as per any row of Table U below, which compounds are named I.B2-Z150-Q2-U-1 to I.B2-Z150-Q2-U-42.

In another embodiment, compounds I are of formula I.B2 wherein Z is Z-150 and Q is Q3, and R and R⁴ are as per any row of Table U below, which compounds are named I.B2-Z150-Q3-U-1 to I.B2-Z150-Q3-U-42.

In another embodiment, compounds I are of formula I.B2 wherein Z is Z-150 and Q is Q4, and R and R⁴ are as per any row of Table U below, which compounds are named I.B2-Z150-Q4-U-1 to I.B2-Z150-Q4-U-42.

In another embodiment, compounds I are of formula I.B1 wherein Z is Z-151 and Q is Q1, and R and R⁴ are as per any row of Table U below, which compounds are named I.B1-Z151-Q1-U-1 to I.B1-Z151-Q1-U-42.

In another embodiment, compounds I are of formula I.B1 wherein Z is Z-151 and Q is Q2, and R and R⁴ are as per any row of Table U below, which compounds are named I.B1-Z151-Q2-U-1 to I.B1-Z151-Q2-U-42.

In another embodiment, compounds I are of formula I.B1 wherein Z is Z-151 and Q is Q3, and R and R⁴ are as per any row of Table U below, which compounds are named I.B1-Z151-Q3-U-1 to I.B1-Z151-Q3-U-42.

In another embodiment, compounds I are of formula I.B1 wherein Z is Z-151 and Q is Q4, and R and R⁴ are as per any row of Table U below, which compounds are named I.B1-Z151-Q4-U-1 to I.B1-Z151-Q4-U-42.

In another embodiment, compounds I are of formula I.B2 wherein Z is Z-151 and Q is Q1, and R and R⁴ are as per any row of Table U below, which compounds are named I.B2-Z151-Q1-U-1 to I.B2-Z151-Q1-U-42.

In another embodiment, compounds I are of formula I.B2 wherein Z is Z-151 and Q is Q2, and R and R⁴ are as per any row of Table U below, which compounds are named I.B2-Z151-Q2-U-1 to I.B2-Z151-Q2-U-42.

In another embodiment, compounds I are of formula I.B2 wherein Z is Z-151 and Q is Q3, and R and R⁴ are as per any row of Table U below, which compounds are named I.B2-Z151-Q3-U-1 to I.B2-Z151-Q3-U-42.

In another embodiment, compounds I are of formula I.B2 wherein Z is Z-151 and Q is Q4, and R and R⁴ are as per any row of Table U below, which compounds are named I.B2-Z151-Q4-U-1 to I.B2-Z151-Q4-U-42.

In another embodiment, compounds I are of formula I.B1 wherein Z is Z-152 and Q is Q1, and R and R⁴ are as per any row of Table U below, which compounds are named I.B1-Z152-Q1-U-1 to I.B1-Z152-Q1-U-42.

In another embodiment, compounds I are of formula I.B1 wherein Z is Z-152 and Q is Q2, and R and R⁴ are as per any row of Table U below, which compounds are named I.B1-Z152-Q2-U-1 to I.B1-Z152-Q2-U-42.

In another embodiment, compounds I are of formula I.B1 wherein Z is Z-152 and Q is Q3, and R and R⁴ are as per any row of Table U below, which compounds are named I.B1-Z152-Q3-U-1 to I.B1-Z152-Q3-U-42.

In another embodiment, compounds I are of formula I.B1 wherein Z is Z-152 and Q is Q4, and R and R⁴ are as per any row of Table U below, which compounds are named I.B1-Z152-Q4-U-1 to I.B1-Z152-Q4-U-42.

In another embodiment, compounds I are of formula I.B2 wherein Z is Z-152 and Q is Q1, and R and R⁴ are as per any row of Table U below, which compounds are named I.B2-Z152-Q1-U-1 to I.B2-Z152-Q1-U-42.

In another embodiment, compounds I are of formula I.B2 wherein Z is Z-152 and Q is Q2, and R and R⁴ are as per any row of Table U below, which compounds are named I.B2-Z152-Q2-U-1 to I.B2-Z152-Q2-U-42.

In another embodiment, compounds I are of formula I.B2 wherein Z is Z-152 and Q is Q3, and R and R⁴ are as per any row of Table U below, which compounds are named I.B2-Z152-Q3-U-1 to I.B2-Z152-Q3-U-42.

In another embodiment, compounds I are of formula I.B2 wherein Z is Z-152 and Q is Q4, and R and R⁴ are as per any row of Table U below, which compounds are named I.B2-Z152-Q4-U-1 to I.B2-Z152-Q4-U-42.

In another embodiment, compounds I are of formula I.B1 wherein Z is Z-153 and Q is Q1, and R and R⁴ are as per any row of Table U below, which compounds are named I.B1-Z153-Q1-U-1 to I.B1-Z153-Q1-U-42.

In another embodiment, compounds I are of formula I.B1 wherein Z is Z-153 and Q is Q2, and R and R⁴ are as per any row of Table U below, which compounds are named I.B1-Z153-Q2-U-1 to I.B1-Z153-Q2-U-42.

In another embodiment, compounds I are of formula I.B1 wherein Z is Z-153 and Q is Q3, and R and R⁴ are as per any row of Table U below, which compounds are named I.B1-Z153-Q3-U-1 to I.B1-Z153-Q3-U-42.

In another embodiment, compounds I are of formula I.B1 wherein Z is Z-153 and Q is Q4, and R and R⁴ are as per any row of Table U below, which compounds are named I.B1-Z153-Q4-U-1 to I.B1-Z153-Q4-U-42.

In another embodiment, compounds I are of formula I.B2 wherein Z is Z-153 and Q is Q1, and R and R⁴ are as per any row of Table U below, which compounds are named I.B2-Z153-Q1-U-1 to I.B2-Z153-Q1-U-42.

In another embodiment, compounds I are of formula I.B2 wherein Z is Z-153 and Q is Q2, and R and R⁴ are as per any row of Table U below, which compounds are named I.B2-Z153-Q2-U-1 to I.B2-Z153-Q2-U-42.

In another embodiment, compounds I are of formula I.B2 wherein Z is Z-153 and Q is Q3, and R and R⁴ are as per any row of Table U below, which compounds are named I.B2-Z153-Q3-U-1 to I.B2-Z153-Q3-U-42.

In another embodiment, compounds I are of formula I.B2 wherein Z is Z-153 and Q is Q4, and R and R⁴ are as per any row of Table U below, which compounds are named I.B2-Z153-Q4-U-1 to I.B2-Z153-Q4-U-42.

In another embodiment, compounds I are of formula I.B1 wherein Z is Z-154 and Q is Q1, and R and R⁴ are as per any row of Table U below, which compounds are named I.B1-Z154-Q1-U-1 to I.B1-Z154-Q1-U-42.

In another embodiment, compounds I are of formula I.B1 wherein Z is Z-154 and Q is Q2, and R and R⁴ are as per any row of Table U below, which compounds are named I.B1-Z154-Q2-U-1 to I.B1-Z154-Q2-U-42.

In another embodiment, compounds I are of formula I.B1 wherein Z is Z-154 and Q is Q3, and R and R⁴ are as per any row of Table U below, which compounds are named I.B1-Z154-Q3-U-1 to I.B1-Z154-Q3-U-42.

In another embodiment, compounds I are of formula I.B1 wherein Z is Z-154 and Q is Q4, and R and R⁴ are as per any row of Table U below, which compounds are named I.B1-Z154-Q4-U-1 to I.B1-Z154-Q4-U-42.

In another embodiment, compounds I are of formula I.B2 wherein Z is Z-154 and Q is Q1, and R and R⁴ are as per any row of Table U below, which compounds are named I.B2-Z154-Q1-U-1 to I.B2-Z154-Q1-U-42.

In another embodiment, compounds I are of formula I.B2 wherein Z is Z-154 and Q is Q2, and R and R⁴ are as per any row of Table U below, which compounds are named I.B2-Z154-Q2-U-1 to I.B2-Z154-Q2-U-42.

In another embodiment, compounds I are of formula I.B2 wherein Z is Z-154 and Q is Q3, and R and R⁴ are as per any row of Table U below, which compounds are named I.B2-Z154-Q3-U-1 to I.B2-Z154-Q3-U-42.

In another embodiment, compounds I are of formula I.B2 wherein Z is Z-154 and Q is Q4, and R and R⁴ are as per any row of Table U below, which compounds are named I.B2-Z154-Q4-U-1 to I.B2-Z154-Q4-U-42.

In another embodiment, compounds I are of formula I.B1 wherein Z is Z-155 and Q is Q1, and R and R⁴ are as per any row of Table U below, which compounds are named I.B1-Z155-Q1-U-1 to I.B1-Z155-Q1-U-42.

In another embodiment, compounds I are of formula I.B1 wherein Z is Z-155 and Q is Q2, and R and R⁴ are as per any row of Table U below, which compounds are named I.B1-Z155-Q2-U-1 to I.B1-Z155-Q2-U-42.

In another embodiment, compounds I are of formula I.B1 wherein Z is Z-155 and Q is Q3, and R and R⁴ are as per any row of Table U below, which compounds are named I.B1-Z155-Q3-U-1 to I.B1-Z155-Q3-U-42.

In another embodiment, compounds I are of formula I.B1 wherein Z is Z-155 and Q is Q4, and R and R⁴ are as per any row of Table U below, which compounds are named I.B1-Z155-Q4-U-1 to I.B1-Z155-Q4-U-42.

In another embodiment, compounds I are of formula I.B2 wherein Z is Z-155 and Q is Q1, and R and R⁴ are as per any row of Table U below, which compounds are named I.B2-Z155-Q1-U-1 to I.B2-Z155-Q1-U-42.

In another embodiment, compounds I are of formula I.B2 wherein Z is Z-155 and Q is Q2, and R and R⁴ are as per any row of Table U below, which compounds are named I.B2-Z155-Q2-U-1 to I.B2-Z155-Q2-U-42.

In another embodiment, compounds I are of formula I.B2 wherein Z is Z-155 and Q is Q3, and R and R⁴ are as per any row of Table U below, which compounds are named I.B2-Z155-Q3-U-1 to I.B2-Z155-Q3-U-42.

In another embodiment, compounds I are of formula I.B2 wherein Z is Z-155 and Q is Q4, and R and R⁴ are as per any row of Table U below, which compounds are named I.B2-Z155-Q4-U-1 to I.B2-Z155-Q4-U-42.

In another embodiment, compounds I are of formula I.B1 wherein Z is Z-156 and Q is Q1, and R and R⁴ are as per any row of Table U below, which compounds are named I.B1-Z156-Q1-U-1 to I.B1-Z156-Q1-U-42.

In another embodiment, compounds I are of formula I.B1 wherein Z is Z-156 and Q is Q2, and R and R⁴ are as per any row of Table U below, which compounds are named I.B1-Z156-Q2-U-1 to I.B1-Z156-Q2-U-42.

In another embodiment, compounds I are of formula I.B1 wherein Z is Z-156 and Q is Q3, and R and R⁴ are as per any row of Table U below, which compounds are named I.B1-Z156-Q3-U-1 to I.B1-Z156-Q3-U-42.

In another embodiment, compounds I are of formula I.B1 wherein Z is Z-156 and Q is Q4, and R and R⁴ are as per any row of Table U below, which compounds are named I.B1-Z156-Q4-U-1 to I.B1-Z156-Q4-U-42.

In another embodiment, compounds I are of formula I.B2 wherein Z is Z-156 and Q is Q1, and R and R⁴ are as per any row of Table U below, which compounds are named I.B2-Z156-Q1-U-1 to I.B2-Z156-Q1-U-42.

In another embodiment, compounds I are of formula I.B2 wherein Z is Z-156 and Q is Q2, and R and R⁴ are as per any row of Table U below, which compounds are named I.B2-Z156-Q2-U-1 to I.B2-Z156-Q2-U-42.

In another embodiment, compounds I are of formula I.B2 wherein Z is Z-156 and Q is Q3, and R and R⁴ are as per any row of Table U below, which compounds are named I.B2-Z156-Q3-U-1 to I.B2-Z156-Q3-U-42.

In another embodiment, compounds I are of formula I.B2 wherein Z is Z-156 and Q is Q4, and R and R⁴ are as per any row of Table U below, which compounds are named I.B2-Z156-Q4-U-1 to I.B2-Z156-Q4-U-42.

In another embodiment, compounds I are of formula I.B1 wherein Z is Z-157 and Q is Q1, and R and R⁴ are as per any row of Table U below, which compounds are named I.B1-Z157-Q1-U-1 to I.B1-Z157-Q1-U-42.

In another embodiment, compounds I are of formula I.B1 wherein Z is Z-157 and Q is Q2, and R and R⁴ are as per any row of Table U below, which compounds are named I.B1-Z157-Q2-U-1 to I.B1-Z157-Q2-U-42.

In another embodiment, compounds I are of formula I.B1 wherein Z is Z-157 and Q is Q3, and R and R⁴ are as per any row of Table U below, which compounds are named I.B1-Z157-Q3-U-1 to I.B1-Z157-Q3-U-42.

In another embodiment, compounds I are of formula I.B1 wherein Z is Z-157 and Q is Q4, and R and R⁴ are as per any row of Table U below, which compounds are named I.B1-Z157-Q4-U-1 to I.B1-Z157-Q4-U-42.

In another embodiment, compounds I are of formula I.B2 wherein Z is Z-157 and Q is Q1, and R and R⁴ are as per any row of Table U below, which compounds are named I.B2-Z157-Q1-U-1 to I.B2-Z157-Q1-U-42.

In another embodiment, compounds I are of formula I.B2 wherein Z is Z-157 and Q is Q2, and R and R⁴ are as per any row of Table U below, which compounds are named I.B2-Z157-Q2-U-1 to I.B2-Z157-Q2-U-42.

In another embodiment, compounds I are of formula I.B2 wherein Z is Z-157 and Q is Q3, and R and R⁴ are as per any row of Table U below, which compounds are named I.B2-Z157-Q3-U-1 to I.B2-Z157-Q3-U-42.

In another embodiment, compounds I are of formula I.B2 wherein Z is Z-157 and Q is Q4, and R and R⁴ are as per any row of Table U below, which compounds are named I.B2-Z157-Q4-U-1 to I.B2-Z157-Q4-U-42.

In another embodiment, compounds I are of formula I.B1 wherein Z is Z-158 and Q is Q1, and R and R⁴ are as per any row of Table U below, which compounds are named I.B1-Z158-Q1-U-1 to I.B1-Z158-Q1-U-42.

In another embodiment, compounds I are of formula I.B1 wherein Z is Z-158 and Q is Q2, and R and R⁴ are as per any row of Table U below, which compounds are named I.B1-Z158-Q2-U-1 to I.B1-Z158-Q2-U-42.

In another embodiment, compounds I are of formula I.B1 wherein Z is Z-158 and Q is Q3, and R and R⁴ are as per any row of Table U below, which compounds are named I.B1-Z158-Q3-U-1 to I.B1-Z158-Q3-U-42.

In another embodiment, compounds I are of formula I.B1 wherein Z is Z-158 and Q is Q4, and R and R⁴ are as per any row of Table U below, which compounds are named I.B1-Z158-Q4-U-1 to I.B1-Z158-Q4-U-42.

In another embodiment, compounds I are of formula I.B2 wherein Z is Z-158 and Q is Q1, and R and R⁴ are as per any row of Table U below, which compounds are named I.B2-Z158-Q1-U-1 to I.B2-Z158-Q1-U-42.

In another embodiment, compounds I are of formula I.B2 wherein Z is Z-158 and Q is Q2, and R and R⁴ are as per any row of Table U below, which compounds are named I.B2-Z158-Q2-U-1 to I.B2-Z158-Q2-U-42.

In another embodiment, compounds I are of formula I.B2 wherein Z is Z-158 and Q is Q3, and R and R⁴ are as per any row of Table U below, which compounds are named I.B2-Z158-Q3-U-1 to I.B2-Z158-Q3-U-42.

In another embodiment, compounds I are of formula I.B2 wherein Z is Z-158 and Q is Q4, and R and R⁴ are as per any row of Table U below, which compounds are named I.B2-Z158-Q4-U-1 to I.B2-Z158-Q4-U-42.

In another embodiment, compounds I are of formula I.B1 wherein Z is Z-159 and Q is Q1, and R and R⁴ are as per any row of Table U below, which compounds are named I.B1-Z159-Q1-U-1 to I.B1-Z159-Q1-U-42.

In another embodiment, compounds I are of formula I.B1 wherein Z is Z-159 and Q is Q2, and R and R⁴ are as per any row of Table U below, which compounds are named I.B1-Z159-Q2-U-1 to I.B1-Z159-Q2-U-42.

In another embodiment, compounds I are of formula I.B1 wherein Z is Z-159 and Q is Q3, and R and R⁴ are as per any row of Table U below, which compounds are named I.B1-Z159-Q3-U-1 to I.B1-Z159-Q3-U-42.

In another embodiment, compounds I are of formula I.B1 wherein Z is Z-159 and Q is Q4, and R and R⁴ are as per any row of Table U below, which compounds are named I.B1-Z159-Q4-U-1 to I.B1-Z159-Q4-U-42.

In another embodiment, compounds I are of formula I.B2 wherein Z is Z-159 and Q is Q1, and R and R⁴ are as per any row of Table U below, which compounds are named I.B2-Z159-Q1-U-1 to I.B2-Z159-Q1-U-42.

In another embodiment, compounds I are of formula I.B2 wherein Z is Z-159 and Q is Q2, and R and R⁴ are as per any row of Table U below, which compounds are named I.B2-Z159-Q2-U-1 to I.B2-Z159-Q2-U-42.

In another embodiment, compounds I are of formula I.B2 wherein Z is Z-159 and Q is Q3, and R and R⁴ are as per any row of Table U below, which compounds are named I.B2-Z159-Q3-U-1 to I.B2-Z159-Q3-U-42.

In another embodiment, compounds I are of formula I.B2 wherein Z is Z-159 and Q is Q4, and R and R⁴ are as per any row of Table U below, which compounds are named I.B2-Z159-Q4-U-1 to I.B2-Z159-Q4-U-42.

In another embodiment, compounds I are of formula I.B1 wherein Z is Z-160 and Q is Q1, and R and R⁴ are as per any row of Table U below, which compounds are named I.B1-Z160-Q1-U-1 to I.B1-Z160-Q1-U-42.

In another embodiment, compounds I are of formula I.B1 wherein Z is Z-160 and Q is Q2, and R and R⁴ are as per any row of Table U below, which compounds are named I.B1-Z160-Q2-U-1 to I.B1-Z160-Q2-U-42.

In another embodiment, compounds I are of formula I.B1 wherein Z is Z-160 and Q is Q3, and R and R⁴ are as per any row of Table U below, which compounds are named I.B1-Z160-Q3-U-1 to I.B1-Z160-Q3-U-42.

In another embodiment, compounds I are of formula I.B1 wherein Z is Z-160 and Q is Q4, and R and R⁴ are as per any row of Table U below, which compounds are named I.B1-Z160-Q4-U-1 to I.B1-Z160-Q4-U-42.

In another embodiment, compounds I are of formula I.B2 wherein Z is Z-160 and Q is Q1, and R and R⁴ are as per any row of Table U below, which compounds are named I.B2-Z160-Q1-U-1 to I.B2-Z160-Q1-U-42.

In another embodiment, compounds I are of formula I.B2 wherein Z is Z-160 and Q is Q2, and R and R⁴ are as per any row of Table U below, which compounds are named I.B2-Z160-Q2-U-1 to I.B2-Z160-Q2-U-42.

In another embodiment, compounds I are of formula I.B2 wherein Z is Z-160 and Q is Q3, and R and R⁴ are as per any row of Table U below, which compounds are named I.B2-Z160-Q3-U-1 to I.B2-Z160-Q3-U-42.

In another embodiment, compounds I are of formula I.B2 wherein Z is Z-160 and Q is Q4, and R and R⁴ are as per any row of Table U below, which compounds are named I.B2-Z160-Q4-U-1 to I.B2-Z160-Q4-U-42.

In another embodiment, compounds I are of formula I.B1 wherein Z is Z-161 and Q is Q1, and R and R⁴ are as per any row of Table U below, which compounds are named I.B1-Z161-Q1-U-1 to I.B1-Z161-Q1-U-42.

In another embodiment, compounds I are of formula I.B1 wherein Z is Z-161 and Q is Q2, and R and R⁴ are as per any row of Table U below, which compounds are named I.B1-Z161-Q2-U-1 to I.B1-Z161-Q2-U-42.

In another embodiment, compounds I are of formula I.B1 wherein Z is Z-161 and Q is Q3, and R and R⁴ are as per any row of Table U below, which compounds are named I.B1-Z161-Q3-U-1 to I.B1-Z161-Q3-U-42.

In another embodiment, compounds I are of formula I.B1 wherein Z is Z-161 and Q is Q4, and R and R⁴ are as per any row of Table U below, which compounds are named I.B1-Z161-Q4-U-1 to I.B1-Z161-Q4-U-42.

In another embodiment, compounds I are of formula I.B2 wherein Z is Z-161 and Q is Q1, and R and R⁴ are as per any row of Table U below, which compounds are named I.B2-Z161-Q1-U-1 to I.B2-Z161-Q1-U-42.

In another embodiment, compounds I are of formula I.B2 wherein Z is Z-161 and Q is Q2, and R and R⁴ are as per any row of Table U below, which compounds are named I.B2-Z161-Q2-U-1 to I.B2-Z161-Q2-U-42.

In another embodiment, compounds I are of formula I.B2 wherein Z is Z-161 and Q is Q3, and R and R⁴ are as per any row of Table U below, which compounds are named I.B2-Z161-Q3-U-1 to I.B2-Z161-Q3-U-42.

In another embodiment, compounds I are of formula I.B2 wherein Z is Z-161 and Q is Q4, and R and R⁴ are as per any row of Table U below, which compounds are named I.B2-Z161-Q4-U-1 to I.B2-Z161-Q4-U-42.

In another embodiment, compounds I are of formula I.B1 wherein Z is Z-162 and Q is Q1, and R and R⁴ are as per any row of Table U below, which compounds are named I.B1-Z162-Q1-U-1 to I.B1-Z162-Q1-U-42.

In another embodiment, compounds I are of formula I.B1 wherein Z is Z-162 and Q is Q2, and R and R⁴ are as per any row of Table U below, which compounds are named I.B1-Z162-Q2-U-1 to I.B1-Z162-Q2-U-42.

In another embodiment, compounds I are of formula I.B1 wherein Z is Z-162 and Q is Q3, and R and R⁴ are as per any row of Table U below, which compounds are named I.B1-Z162-Q3-U-1 to I.B1-Z162-Q3-U-42.

In another embodiment, compounds I are of formula I.B1 wherein Z is Z-162 and Q is Q4, and R and R⁴ are as per any row of Table U below, which compounds are named I.B1-Z162-Q4-U-1 to I.B1-Z162-Q4-U-42.

In another embodiment, compounds I are of formula I.B2 wherein Z is Z-162 and Q is Q1, and R and R⁴ are as per any row of Table U below, which compounds are named I.B2-Z162-Q1-U-1 to I.B2-Z162-Q1-U-42.

In another embodiment, compounds I are of formula I.B2 wherein Z is Z-162 and Q is Q2, and R and R⁴ are as per any row of Table U below, which compounds are named I.B2-Z162-Q2-U-1 to I.B2-Z162-Q2-U-42.

In another embodiment, compounds I are of formula I.B2 wherein Z is Z-162 and Q is Q3, and R and R⁴ are as per any row of Table U below, which compounds are named I.B2-Z162-Q3-U-1 to I.B2-Z162-Q3-U-42.

In another embodiment, compounds I are of formula I.B2 wherein Z is Z-162 and Q is Q4, and R and R⁴ are as per any row of Table U below, which compounds are named I.B2-Z162-Q4-U-1 to I.B2-Z162-Q4-U-42.

In another embodiment, compounds I are of formula I.B1 wherein Z is Z-163 and Q is Q1, and R and R⁴ are as per any row of Table U below, which compounds are named I.B1-Z163-Q1-U-1 to I.B1-Z163-Q1-U-42.

In another embodiment, compounds I are of formula I.B1 wherein Z is Z-163 and Q is Q2, and R and R⁴ are as per any row of Table U below, which compounds are named I.B1-Z163-Q2-U-1 to I.B1-Z163-Q2-U-42.

In another embodiment, compounds I are of formula I.B1 wherein Z is Z-163 and Q is Q3, and R and R⁴ are as per any row of Table U below, which compounds are named I.B1-Z163-Q3-U-1 to I.B1-Z163-Q3-U-42.

In another embodiment, compounds I are of formula I.B1 wherein Z is Z-163 and Q is Q4, and R and R⁴ are as per any row of Table U below, which compounds are named I.B1-Z163-Q4-U-1 to I.B1-Z163-Q4-U-42.

In another embodiment, compounds I are of formula I.B2 wherein Z is Z-163 and Q is Q1, and R and R⁴ are as per any row of Table U below, which compounds are named I.B2-Z163-Q1-U-1 to I.B2-Z163-Q1-U-42.

In another embodiment, compounds I are of formula I.B2 wherein Z is Z-163 and Q is Q2, and R and R⁴ are as per any row of Table U below, which compounds are named I.B2-Z163-Q2-U-1 to I.B2-Z163-Q2-U-42.

In another embodiment, compounds I are of formula I.B2 wherein Z is Z-163 and Q is Q3, and R and R⁴ are as per any row of Table U below, which compounds are named I.B2-Z163-Q3-U-1 to I.B2-Z163-Q3-U-42.

In another embodiment, compounds I are of formula I.B2 wherein Z is Z-163 and Q is Q4, and R and R⁴ are as per any row of Table U below, which compounds are named I.B2-Z163-Q4-U-1 to I.B2-Z163-Q4-U-42.

In another embodiment, compounds I are of formula I.B1 wherein Z is Z-164 and Q is Q1, and R and R⁴ are as per any row of Table U below, which compounds are named I.B1-Z164-Q1-U-1 to I.B1-Z164-Q1-U-42.

In another embodiment, compounds I are of formula I.B1 wherein Z is Z-164 and Q is Q2, and R and R⁴ are as per any row of Table U below, which compounds are named I.B1-Z164-Q2-U-1 to I.B1-Z164-Q2-U-42.

In another embodiment, compounds I are of formula I.B1 wherein Z is Z-164 and Q is Q3, and R and R⁴ are as per any row of Table U below, which compounds are named I.B1-Z164-Q3-U-1 to I.B1-Z164-Q3-U-42.

In another embodiment, compounds I are of formula I.B1 wherein Z is Z-164 and Q is Q4, and R and R⁴ are as per any row of Table U below, which compounds are named I.B1-Z164-Q4-U-1 to I.B1-Z164-Q4-U-42.

In another embodiment, compounds I are of formula I.B2 wherein Z is Z-164 and Q is Q1, and R and R⁴ are as per any row of Table U below, which compounds are named I.B2-Z164-Q1-U-1 to I.B2-Z164-Q1-U-42.

In another embodiment, compounds I are of formula I.B2 wherein Z is Z-164 and Q is Q2, and R and R⁴ are as per any row of Table U below, which compounds are named I.B2-Z164-Q2-U-1 to I.B2-Z164-Q2-U-42.

In another embodiment, compounds I are of formula I.B2 wherein Z is Z-164 and Q is Q3, and R and R⁴ are as per any row of Table U below, which compounds are named I.B2-Z164-Q3-U-1 to I.B2-Z164-Q3-U-42.

In another embodiment, compounds I are of formula I.B2 wherein Z is Z-164 and Q is Q4, and R and R⁴ are as per any row of Table U below, which compounds are named I.B2-Z164-Q4-U-1 to I.B2-Z164-Q4-U-42.

In another embodiment, compounds I are of formula I.B1 wherein Z is Z-165 and Q is Q1, and R and R⁴ are as per any row of Table U below, which compounds are named I.B1-Z165-Q1-U-1 to I.B1-Z165-Q1-U-42.

In another embodiment, compounds I are of formula I.B1 wherein Z is Z-165 and Q is Q2, and R and R⁴ are as per any row of Table U below, which compounds are named I.B1-Z165-Q2-U-1 to I.B1-Z165-Q2-U-42.

In another embodiment, compounds I are of formula I.B1 wherein Z is Z-165 and Q is Q3, and R and R⁴ are as per any row of Table U below, which compounds are named I.B1-Z165-Q3-U-1 to I.B1-Z165-Q3-U-42.

In another embodiment, compounds I are of formula I.B1 wherein Z is Z-165 and Q is Q4, and R and R⁴ are as per any row of Table U below, which compounds are named I.B1-Z165-Q4-U-1 to I.B1-Z165-Q4-U-42.

In another embodiment, compounds I are of formula I.B2 wherein Z is Z-165 and Q is Q1, and R and R⁴ are as per any row of Table U below, which compounds are named I.B2-Z165-Q1-U-1 to I.B2-Z165-Q1-U-42.

In another embodiment, compounds I are of formula I.B2 wherein Z is Z-165 and Q is Q2, and R and R⁴ are as per any row of Table U below, which compounds are named I.B2-Z165-Q2-U-1 to I.B2-Z165-Q2-U-42.

In another embodiment, compounds I are of formula I.B2 wherein Z is Z-165 and Q is Q3, and R and R⁴ are as per any row of Table U below, which compounds are named I.B2-Z165-Q3-U-1 to I.B2-Z165-Q3-U-42.

In another embodiment, compounds I are of formula I.B2 wherein Z is Z-165 and Q is Q4, and R and R⁴ are as per any row of Table U below, which compounds are named I.B2-Z165-Q4-U-1 to I.B2-Z165-Q4-U-42.

In another embodiment, compounds I are of formula I.B1 wherein Z is Z-166 and Q is Q1, and R and R⁴ are as per any row of Table U below, which compounds are named I.B1-Z166-Q1-U-1 to I.B1-Z166-Q1-U-42.

In another embodiment, compounds I are of formula I.B1 wherein Z is Z-166 and Q is Q2, and R and R⁴ are as per any row of Table U below, which compounds are named I.B1-Z166-Q2-U-1 to I.B1-Z166-Q2-U-42.

In another embodiment, compounds I are of formula I.B1 wherein Z is Z-166 and Q is Q3, and R and R⁴ are as per any row of Table U below, which compounds are named I.B1-Z166-Q3-U-1 to I.B1-Z166-Q3-U-42.

In another embodiment, compounds I are of formula I.B1 wherein Z is Z-166 and Q is Q4, and R and R⁴ are as per any row of Table U below, which compounds are named I.B1-Z166-Q4-U-1 to I.B1-Z166-Q4-U-42.

In another embodiment, compounds I are of formula I.B2 wherein Z is Z-166 and Q is Q1, and R and R⁴ are as per any row of Table U below, which compounds are named I.B2-Z166-Q1-U-1 to I.B2-Z166-Q1-U-42.

In another embodiment, compounds I are of formula I.B2 wherein Z is Z-166 and Q is Q2, and R and R⁴ are as per any row of Table U below, which compounds are named I.B2-Z166-Q2-U-1 to I.B2-Z166-Q2-U-42.

In another embodiment, compounds I are of formula I.B2 wherein Z is Z-166 and Q is Q3, and R and R⁴ are as per any row of Table U below, which compounds are named I.B2-Z166-Q3-U-1 to I.B2-Z166-Q3-U-42.

In another embodiment, compounds I are of formula I.B2 wherein Z is Z-166 and Q is Q4, and R and R⁴ are as per any row of Table U below, which compounds are named I.B2-Z166-Q4-U-1 to I.B2-Z166-Q4-U-42.

In another embodiment, compounds I are of formula I.B1 wherein Z is Z-167 and Q is Q1, and R and R⁴ are as per any row of Table U below, which compounds are named I.B1-Z167-Q1-U-1 to I.B1-Z167-Q1-U-42.

In another embodiment, compounds I are of formula I.B1 wherein Z is Z-167 and Q is Q2, and R and R⁴ are as per any row of Table U below, which compounds are named I.B1-Z167-Q2-U-1 to I.B1-Z167-Q2-U-42.

In another embodiment, compounds I are of formula I.B1 wherein Z is Z-167 and Q is Q3, and R and R⁴ are as per any row of Table U below, which compounds are named I.B1-Z167-Q3-U-1 to I.B1-Z167-Q3-U-42.

In another embodiment, compounds I are of formula I.B1 wherein Z is Z-167 and Q is Q4, and R and R⁴ are as per any row of Table U below, which compounds are named I.B1-Z167-Q4-U-1 to I.B1-Z167-Q4-U-42.

In another embodiment, compounds I are of formula I.B2 wherein Z is Z-167 and Q is Q1, and R and R⁴ are as per any row of Table U below, which compounds are named I.B2-Z167-Q1-U-1 to I.B2-Z167-Q1-U-42.

In another embodiment, compounds I are of formula I.B2 wherein Z is Z-167 and Q is Q2, and R and R⁴ are as per any row of Table U below, which compounds are named I.B2-Z167-Q2-U-1 to I.B2-Z167-Q2-U-42.

In another embodiment, compounds I are of formula I.B2 wherein Z is Z-167 and Q is Q3, and R and R⁴ are as per any row of Table U below, which compounds are named I.B2-Z167-Q3-U-1 to I.B2-Z167-Q3-U-42.

In another embodiment, compounds I are of formula I.B2 wherein Z is Z-167 and Q is Q4, and R and R⁴ are as per any row of Table U below, which compounds are named I.B2-Z167-Q4-U-1 to I.B2-Z167-Q4-U-42.

In another embodiment, compounds I are of formula I.B1 wherein Z is Z-168 and Q is Q1, and R and R⁴ are as per any row of Table U below, which compounds are named I.B1-Z168-Q1-U-1 to I.B1-Z168-Q1-U-42.

In another embodiment, compounds I are of formula I.B1 wherein Z is Z-168 and Q is Q2, and R and R⁴ are as per any row of Table U below, which compounds are named I.B1-Z168-Q2-U-1 to I.B1-Z168-Q2-U-42.

In another embodiment, compounds I are of formula I.B1 wherein Z is Z-168 and Q is Q3, and R and R⁴ are as per any row of Table U below, which compounds are named I.B1-Z168-Q3-U-1 to I.B1-Z168-Q3-U-42.

In another embodiment, compounds I are of formula I.B1 wherein Z is Z-168 and Q is Q4, and R and R⁴ are as per any row of Table U below, which compounds are named I.B1-Z168-Q4-U-1 to I.B1-Z168-Q4-U-42.

In another embodiment, compounds I are of formula I.B2 wherein Z is Z-168 and Q is Q1, and R and R⁴ are as per any row of Table U below, which compounds are named I.B2-Z168-Q1-U-1 to I.B2-Z168-Q1-U-42.

In another embodiment, compounds I are of formula I.B2 wherein Z is Z-168 and Q is Q2, and R and R⁴ are as per any row of Table U below, which compounds are named I.B2-Z168-Q2-U-1 to I.B2-Z168-Q2-U-42.

In another embodiment, compounds I are of formula I.B2 wherein Z is Z-168 and Q is Q3, and R and R⁴ are as per any row of Table U below, which compounds are named I.B2-Z168-Q3-U-1 to I.B2-Z168-Q3-U-42.

In another embodiment, compounds I are of formula I.B2 wherein Z is Z-168 and Q is Q4, and R and R⁴ are as per any row of Table U below, which compounds are named I.B2-Z168-Q4-U-1 to I.B2-Z168-Q4-U-42.

In another embodiment, compounds I are of formula I.B1 wherein Z is Z-169 and Q is Q1, and R and R⁴ are as per any row of Table U below, which compounds are named I.B1-Z169-Q1-U-1 to I.B1-Z169-Q1-U-42.

In another embodiment, compounds I are of formula I.B1 wherein Z is Z-169 and Q is Q2, and R and R⁴ are as per any row of Table U below, which compounds are named I.B1-Z169-Q2-U-1 to I.B1-Z169-Q2-U-42.

In another embodiment, compounds I are of formula I.B1 wherein Z is Z-169 and Q is Q3, and R and R⁴ are as per any row of Table U below, which compounds are named I.B1-Z169-Q3-U-1 to I.B1-Z169-Q3-U-42.

In another embodiment, compounds I are of formula I.B1 wherein Z is Z-169 and Q is Q4, and R and R⁴ are as per any row of Table U below, which compounds are named I.B1-Z169-Q4-U-1 to I.B1-Z169-Q4-U-42.

In another embodiment, compounds I are of formula I.B2 wherein Z is Z-169 and Q is Q1, and R and R⁴ are as per any row of Table U below, which compounds are named I.B2-Z169-Q1-U-1 to I.B2-Z169-Q1-U-42.

In another embodiment, compounds I are of formula I.B2 wherein Z is Z-169 and Q is Q2, and R and R⁴ are as per any row of Table U below, which compounds are named I.B2-Z169-Q2-U-1 to I.B2-Z169-Q2-U-42.

In another embodiment, compounds I are of formula I.B2 wherein Z is Z-169 and Q is Q3, and R and R⁴ are as per any row of Table U below, which compounds are named I.B2-Z169-Q3-U-1 to I.B2-Z169-Q3-U-42.

In another embodiment, compounds I are of formula I.B2 wherein Z is Z-169 and Q is Q4, and R and R⁴ are as per any row of Table U below, which compounds are named I.B2-Z169-Q4-U-1 to I.B2-Z169-Q4-U-42.

In another embodiment, compounds I are of formula I.B1 wherein Z is Z-170 and Q is Q1, and R and R⁴ are as per any row of Table U below, which compounds are named I.B1-Z170-Q1-U-1 to I.B1-Z170-Q1-U-42.

In another embodiment, compounds I are of formula I.B1 wherein Z is Z-170 and Q is Q2, and R and R⁴ are as per any row of Table U below, which compounds are named I.B1-Z170-Q2-U-1 to I.B1-Z170-Q2-U-42.

In another embodiment, compounds I are of formula I.B1 wherein Z is Z-170 and Q is Q3, and R and R⁴ are as per any row of Table U below, which compounds are named I.B1-Z170-Q3-U-1 to I.B1-Z170-Q3-U-42.

In another embodiment, compounds I are of formula I.B1 wherein Z is Z-170 and Q is Q4, and R and R⁴ are as per any row of Table U below, which compounds are named I.B1-Z170-Q4-U-1 to I.B1-Z170-Q4-U-42.

In another embodiment, compounds I are of formula I.B2 wherein Z is Z-170 and Q is Q1, and R and R⁴ are as per any row of Table U below, which compounds are named I.B2-Z170-Q1-U-1 to I.B2-Z170-Q1-U-42.

In another embodiment, compounds I are of formula I.B2 wherein Z is Z-170 and Q is Q2, and R and R⁴ are as per any row of Table U below, which compounds are named I.B2-Z170-Q2-U-1 to I.B2-Z170-Q2-U-42.

In another embodiment, compounds I are of formula I.B2 wherein Z is Z-170 and Q is Q3, and R and R⁴ are as per any row of Table U below, which compounds are named I.B2-Z170-Q3-U-1 to I.B2-Z170-Q3-U-42.

In another embodiment, compounds I are of formula I.B2 wherein Z is Z-170 and Q is Q4, and R and R⁴ are as per any row of Table U below, which compounds are named I.B2-Z170-Q4-U-1 to I.B2-Z170-Q4-U-42.

In another embodiment, compounds I are of formula I.B1 wherein Z is Z-171 and Q is Q1, and R and R⁴ are as per any row of Table U below, which compounds are named I.B1-Z171-Q1-U-1 to I.B1-Z171-Q1-U-42.

In another embodiment, compounds I are of formula I.B1 wherein Z is Z-171 and Q is Q2, and R and R⁴ are as per any row of Table U below, which compounds are named I.B1-Z171-Q2-U-1 to I.B1-Z171-Q2-U-42.

In another embodiment, compounds I are of formula I.B1 wherein Z is Z-171 and Q is Q3, and R and R⁴ are as per any row of Table U below, which compounds are named I.B1-Z171-Q3-U-1 to I.B1-Z171-Q3-U-42.

In another embodiment, compounds I are of formula I.B1 wherein Z is Z-171 and Q is Q4, and R and R⁴ are as per any row of Table U below, which compounds are named I.B1-Z171-Q4-U-1 to I.B1-Z171-Q4-U-42.

In another embodiment, compounds I are of formula I.B2 wherein Z is Z-171 and Q is Q1, and R and R⁴ are as per any row of Table U below, which compounds are named I.B2-Z171-Q1-U-1 to I.B2-Z171-Q1-U-42.

In another embodiment, compounds I are of formula I.B2 wherein Z is Z-171 and Q is Q2, and R and R⁴ are as per any row of Table U below, which compounds are named I.B2-Z171-Q2-U-1 to I.B2-Z171-Q2-U-42.

In another embodiment, compounds I are of formula I.B2 wherein Z is Z-171 and Q is Q3, and R and R⁴ are as per any row of Table U below, which compounds are named I.B2-Z171-Q3-U-1 to I.B2-Z171-Q3-U-42.

In another embodiment, compounds I are of formula I.B2 wherein Z is Z-171 and Q is Q4, and R and R⁴ are as per any row of Table U below, which compounds are named I.B2-Z171-Q4-U-1 to I.B2-Z171-Q4-U-42.

In another embodiment, compounds I are of formula I.B1 wherein Z is Z-172 and Q is Q1, and R and R⁴ are as per any row of Table U below, which compounds are named I.B1-Z172-Q1-U-1 to I.B1-Z172-Q1-U-42.

In another embodiment, compounds I are of formula I.B1 wherein Z is Z-172 and Q is Q2, and R and R⁴ are as per any row of Table U below, which compounds are named I.B1-Z172-Q2-U-1 to I.B1-Z172-Q2-U-42.

In another embodiment, compounds I are of formula I.B1 wherein Z is Z-172 and Q is Q3, and R and R⁴ are as per any row of Table U below, which compounds are named I.B1-Z172-Q3-U-1 to I.B1-Z172-Q3-U-42.

In another embodiment, compounds I are of formula I.B1 wherein Z is Z-172 and Q is Q4, and R and R⁴ are as per any row of Table U below, which compounds are named I.B1-Z172-Q4-U-1 to I.B1-Z172-Q4-U-42.

In another embodiment, compounds I are of formula I.B2 wherein Z is Z-172 and Q is Q1, and R and R⁴ are as per any row of Table U below, which compounds are named I.B2-Z172-Q1-U-1 to I.B2-Z172-Q1-U-42.

In another embodiment, compounds I are of formula I.B2 wherein Z is Z-172 and Q is Q2, and R and R⁴ are as per any row of Table U below, which compounds are named I.B2-Z172-Q2-U-1 to I.B2-Z172-Q2-U-42.

In another embodiment, compounds I are of formula I.B2 wherein Z is Z-172 and Q is Q3, and R and R⁴ are as per any row of Table U below, which compounds are named I.B2-Z172-Q3-U-1 to I.B2-Z172-Q3-U-42.

In another embodiment, compounds I are of formula I.B2 wherein Z is Z-172 and Q is Q4, and R and R⁴ are as per any row of Table U below, which compounds are named I.B2-Z172-Q4-U-1 to I.B2-Z172-Q4-U-42.

In another embodiment, compounds I are of formula I.B1 wherein Z is Z-173 and Q is Q1, and R and R⁴ are as per any row of Table U below, which compounds are named I.B1-Z173-Q1-U-1 to I.B1-Z173-Q1-U-42.

In another embodiment, compounds I are of formula I.B1 wherein Z is Z-173 and Q is Q2, and R and R⁴ are as per any row of Table U below, which compounds are named I.B1-Z173-Q2-U-1 to I.B1-Z173-Q2-U-42.

In another embodiment, compounds I are of formula I.B1 wherein Z is Z-173 and Q is Q3, and R and R⁴ are as per any row of Table U below, which compounds are named I.B1-Z173-Q3-U-1 to I.B1-Z173-Q3-U-42.

In another embodiment, compounds I are of formula I.B1 wherein Z is Z-173 and Q is Q4, and R and R⁴ are as per any row of Table U below, which compounds are named I.B1-Z173-Q4-U-1 to I.B1-Z173-Q4-U-42.

In another embodiment, compounds I are of formula I.B2 wherein Z is Z-173 and Q is Q1, and R and R⁴ are as per any row of Table U below, which compounds are named I.B2-Z173-Q1-U-1 to I.B2-Z173-Q1-U-42.

In another embodiment, compounds I are of formula I.B2 wherein Z is Z-173 and Q is Q2, and R and R⁴ are as per any row of Table U below, which compounds are named I.B2-Z173-Q2-U-1 to I.B2-Z173-Q2-U-42.

In another embodiment, compounds I are of formula I.B2 wherein Z is Z-173 and Q is Q3, and R and R⁴ are as per any row of Table U below, which compounds are named I.B2-Z173-Q3-U-1 to I.B2-Z173-Q3-U-42.

In another embodiment, compounds I are of formula I.B2 wherein Z is Z-173 and Q is Q4, and R and R⁴ are as per any row of Table U below, which compounds are named I.B2-Z173-Q4-U-1 to I.B2-Z173-Q4-U-42.

In another embodiment, compounds I are of formula I.B1 wherein Z is Z-174 and Q is Q1, and R and R⁴ are as per any row of Table U below, which compounds are named I.B1-Z174-Q1-U-1 to I.B1-Z174-Q1-U-42.

In another embodiment, compounds I are of formula I.B1 wherein Z is Z-174 and Q is Q2, and R and R⁴ are as per any row of Table U below, which compounds are named I.B1-Z174-Q2-U-1 to I.B1-Z174-Q2-U-42.

In another embodiment, compounds I are of formula I.B1 wherein Z is Z-174 and Q is Q3, and R and R⁴ are as per any row of Table U below, which compounds are named I.B1-Z174-Q3-U-1 to I.B1-Z174-Q3-U-42.

In another embodiment, compounds I are of formula I.B1 wherein Z is Z-174 and Q is Q4, and R and R⁴ are as per any row of Table U below, which compounds are named I.B1-Z174-Q4-U-1 to I.B1-Z174-Q4-U-42.

In another embodiment, compounds I are of formula I.B2 wherein Z is Z-174 and Q is Q1, and R and R⁴ are as per any row of Table U below, which compounds are named I.B2-Z174-Q1-U-1 to I.B2-Z174-Q1-U-42.

In another embodiment, compounds I are of formula I.B2 wherein Z is Z-174 and Q is Q2, and R and R⁴ are as per any row of Table U below, which compounds are named I.B2-Z174-Q2-U-1 to I.B2-Z174-Q2-U-42.

In another embodiment, compounds I are of formula I.B2 wherein Z is Z-174 and Q is Q3, and R and R⁴ are as per any row of Table U below, which compounds are named I.B2-Z174-Q3-U-1 to I.B2-Z174-Q3-U-42.

In another embodiment, compounds I are of formula I.B2 wherein Z is Z-174 and Q is Q4, and R and R⁴ are as per any row of Table U below, which compounds are named I.B2-Z174-Q4-U-1 to I.B2-Z174-Q4-U-42.

In another embodiment, compounds I are of formula I.B1 wherein Z is Z-175 and Q is Q1, and R and R⁴ are as per any row of Table U below, which compounds are named I.B1-Z175-Q1-U-1 to I.B1-Z175-Q1-U-42.

In another embodiment, compounds I are of formula I.B1 wherein Z is Z-175 and Q is Q2, and R and R⁴ are as per any row of Table U below, which compounds are named I.B1-Z175-Q2-U-1 to I.B1-Z175-Q2-U-42.

In another embodiment, compounds I are of formula I.B1 wherein Z is Z-175 and Q is Q3, and R and R⁴ are as per any row of Table U below, which compounds are named I.B1-Z175-Q3-U-1 to I.B1-Z175-Q3-U-42.

In another embodiment, compounds I are of formula I.B1 wherein Z is Z-175 and Q is Q4, and R and R⁴ are as per any row of Table U below, which compounds are named I.B1-Z175-Q4-U-1 to I.B1-Z175-Q4-U-42.

In another embodiment, compounds I are of formula I.B2 wherein Z is Z-175 and Q is Q1, and R and R⁴ are as per any row of Table U below, which compounds are named I.B2-Z175-Q1-U-1 to I.B2-Z175-Q1-U-42.

In another embodiment, compounds I are of formula I.B2 wherein Z is Z-175 and Q is Q2, and R and R⁴ are as per any row of Table U below, which compounds are named I.B2-Z175-Q2-U-1 to I.B2-Z175-Q2-U-42.

In another embodiment, compounds I are of formula I.B2 wherein Z is Z-175 and Q is Q3, and R and R⁴ are as per any row of Table U below, which compounds are named I.B2-Z175-Q3-U-1 to I.B2-Z175-Q3-U-42.

In another embodiment, compounds I are of formula I.B2 wherein Z is Z-175 and Q is Q4, and R and R⁴ are as per any row of Table U below, which compounds are named I.B2-Z175-Q4-U-1 to I.B2-Z175-Q4-U-42.

In another embodiment, compounds I are of formula I.B1 wherein Z is Z-176 and Q is Q1, and R and R⁴ are as per any row of Table U below, which compounds are named I.B1-Z176-Q1-U-1 to I.B1-Z176-Q1-U-42.

In another embodiment, compounds I are of formula I.B1 wherein Z is Z-176 and Q is Q2, and R and R⁴ are as per any row of Table U below, which compounds are named I.B1-Z176-Q2-U-1 to I.B1-Z176-Q2-U-42.

In another embodiment, compounds I are of formula I.B1 wherein Z is Z-176 and Q is Q3, and R and R⁴ are as per any row of Table U below, which compounds are named I.B1-Z176-Q3-U-1 to I.B1-Z176-Q3-U-42.

In another embodiment, compounds I are of formula I.B1 wherein Z is Z-176 and Q is Q4, and R and R⁴ are as per any row of Table U below, which compounds are named I.B1-Z176-Q4-U-1 to I.B1-Z176-Q4-U-42.

In another embodiment, compounds I are of formula I.B2 wherein Z is Z-176 and Q is Q1, and R and R⁴ are as per any row of Table U below, which compounds are named I.B2-Z176-Q1-U-1 to I.B2-Z176-Q1-U-42.

In another embodiment, compounds I are of formula I.B2 wherein Z is Z-176 and Q is Q2, and R and R⁴ are as per any row of Table U below, which compounds are named I.B2-Z176-Q2-U-1 to I.B2-Z176-Q2-U-42.

In another embodiment, compounds I are of formula I.B2 wherein Z is Z-176 and Q is Q3, and R and R⁴ are as per any row of Table U below, which compounds are named I.B2-Z176-Q3-U-1 to I.B2-Z176-Q3-U-42.

In another embodiment, compounds I are of formula I.B2 wherein Z is Z-176 and Q is Q4, and R and R⁴ are as per any row of Table U below, which compounds are named I.B2-Z176-Q4-U-1 to I.B2-Z176-Q4-U-42.

In another embodiment, compounds I are of formula I.B1 wherein Z is Z-177 and Q is Q1, and R and R⁴ are as per any row of Table U below, which compounds are named I.B1-Z177-Q1-U-1 to I.B1-Z177-Q1-U-42.

In another embodiment, compounds I are of formula I.B1 wherein Z is Z-177 and Q is Q2, and R and R⁴ are as per any row of Table U below, which compounds are named I.B1-Z177-Q2-U-1 to I.B1-Z177-Q2-U-42.

In another embodiment, compounds I are of formula I.B1 wherein Z is Z-177 and Q is Q3, and R and R⁴ are as per any row of Table U below, which compounds are named I.B1-Z177-Q3-U-1 to I.B1-Z177-Q3-U-42.

In another embodiment, compounds I are of formula I.B1 wherein Z is Z-177 and Q is Q4, and R and R⁴ are as per any row of Table U below, which compounds are named I.B1-Z177-Q4-U-1 to I.B1-Z177-Q4-U-42.

In another embodiment, compounds I are of formula I.B2 wherein Z is Z-177 and Q is Q1, and R and R⁴ are as per any row of Table U below, which compounds are named I.B2-Z177-Q1-U-1 to I.B2-Z177-Q1-U-42.

In another embodiment, compounds I are of formula I.B2 wherein Z is Z-177 and Q is Q2, and R and R⁴ are as per any row of Table U below, which compounds are named I.B2-Z177-Q2-U-1 to I.B2-Z177-Q2-U-42.

In another embodiment, compounds I are of formula I.B2 wherein Z is Z-177 and Q is Q3, and R and R⁴ are as per any row of Table U below, which compounds are named I.B2-Z177-Q3-U-1 to I.B2-Z177-Q3-U-42.

In another embodiment, compounds I are of formula I.B2 wherein Z is Z-177 and Q is Q4, and R and R⁴ are as per any row of Table U below, which compounds are named I.B2-Z177-Q4-U-1 to I.B2-Z177-Q4-U-42.

In another embodiment, compounds I are of formula I.B1 wherein Z is Z-178 and Q is Q1, and R and R⁴ are as per any row of Table U below, which compounds are named I.B1-Z178-Q1-U-1 to I.B1-Z178-Q1-U-42.

In another embodiment, compounds I are of formula I.B1 wherein Z is Z-178 and Q is Q2, and R and R⁴ are as per any row of Table U below, which compounds are named I.B1-Z178-Q2-U-1 to I.B1-Z178-Q2-U-42.

In another embodiment, compounds I are of formula I.B1 wherein Z is Z-178 and Q is Q3, and R and R⁴ are as per any row of Table U below, which compounds are named I.B1-Z178-Q3-U-1 to I.B1-Z178-Q3-U-42.

In another embodiment, compounds I are of formula I.B1 wherein Z is Z-178 and Q is Q4, and R and R⁴ are as per any row of Table U below, which compounds are named I.B1-Z178-Q4-U-1 to I.B1-Z178-Q4-U-42.

In another embodiment, compounds I are of formula I.B2 wherein Z is Z-178 and Q is Q1, and R and R⁴ are as per any row of Table U below, which compounds are named I.B2-Z178-Q1-U-1 to I.B2-Z178-Q1-U-42.

In another embodiment, compounds I are of formula I.B2 wherein Z is Z-178 and Q is Q2, and R and R⁴ are as per any row of Table U below, which compounds are named I.B2-Z178-Q2-U-1 to I.B2-Z178-Q2-U-42.

In another embodiment, compounds I are of formula I.B2 wherein Z is Z-178 and Q is Q3, and R and R⁴ are as per any row of Table U below, which compounds are named I.B2-Z178-Q3-U-1 to I.B2-Z178-Q3-U-42.

In another embodiment, compounds I are of formula I.B2 wherein Z is Z-178 and Q is Q4, and R and R⁴ are as per any row of Table U below, which compounds are named I.B2-Z178-Q4-U-1 to I.B2-Z178-Q4-U-42.

In another embodiment, compounds I are of formula I.B1 wherein Z is Z-179 and Q is Q1, and R and R⁴ are as per any row of Table U below, which compounds are named I.B1-Z179-Q1-U-1 to I.B1-Z179-Q1-U-42.

In another embodiment, compounds I are of formula I.B1 wherein Z is Z-179 and Q is Q2, and R and R⁴ are as per any row of Table U below, which compounds are named I.B1-Z179-Q2-U-1 to I.B1-Z179-Q2-U-42.

In another embodiment, compounds I are of formula I.B1 wherein Z is Z-179 and Q is Q3, and R and R⁴ are as per any row of Table U below, which compounds are named I.B1-Z179-Q3-U-1 to I.B1-Z179-Q3-U-42.

In another embodiment, compounds I are of formula I.B1 wherein Z is Z-179 and Q is Q4, and R and R⁴ are as per any row of Table U below, which compounds are named I.B1-Z179-Q4-U-1 to I.B1-Z179-Q4-U-42.

In another embodiment, compounds I are of formula I.B2 wherein Z is Z-179 and Q is Q1, and R and R⁴ are as per any row of Table U below, which compounds are named I.B2-Z179-Q1-U-1 to I.B2-Z179-Q1-U-42.

In another embodiment, compounds I are of formula I.B2 wherein Z is Z-179 and Q is Q2, and R and R⁴ are as per any row of Table U below, which compounds are named I.B2-Z179-Q2-U-1 to I.B2-Z179-Q2-U-42.

In another embodiment, compounds I are of formula I.B2 wherein Z is Z-179 and Q is Q3, and R and R⁴ are as per any row of Table U below, which compounds are named I.B2-Z179-Q3-U-1 to I.B2-Z179-Q3-U-42.

In another embodiment, compounds I are of formula I.B2 wherein Z is Z-179 and Q is Q4, and R and R⁴ are as per any row of Table U below, which compounds are named I.B2-Z179-Q4-U-1 to I.B2-Z179-Q4-U-42.

In another embodiment, compounds I are of formula I.B1 wherein Z is Z-180 and Q is Q1, and R and R⁴ are as per any row of Table U below, which compounds are named I.B1-Z180-Q1-U-1 to I.B1-Z180-Q1-U-42.

In another embodiment, compounds I are of formula I.B1 wherein Z is Z-180 and Q is Q2, and R and R⁴ are as per any row of Table U below, which compounds are named I.B1-Z180-Q2-U-1 to I.B1-Z180-Q2-U-42.

In another embodiment, compounds I are of formula I.B1 wherein Z is Z-180 and Q is Q3, and R and R⁴ are as per any row of Table U below, which compounds are named I.B1-Z180-Q3-U-1 to I.B1-Z180-Q3-U-42.

In another embodiment, compounds I are of formula I.B1 wherein Z is Z-180 and Q is Q4, and R and R⁴ are as per any row of Table U below, which compounds are named I.B1-Z180-Q4-U-1 to I.B1-Z180-Q4-U-42.

In another embodiment, compounds I are of formula I.B2 wherein Z is Z-180 and Q is Q1, and R and R⁴ are as per any row of Table U below, which compounds are named I.B2-Z180-Q1-U-1 to I.B2-Z180-Q1-U-42.

In another embodiment, compounds I are of formula I.B2 wherein Z is Z-180 and Q is Q2, and R and R⁴ are as per any row of Table U below, which compounds are named I.B2-Z180-Q2-U-1 to I.B2-Z180-Q2-U-42.

In another embodiment, compounds I are of formula I.B2 wherein Z is Z-180 and Q is Q3, and R and R⁴ are as per any row of Table U below, which compounds are named I.B2-Z180-Q3-U-1 to I.B2-Z180-Q3-U-42.

In another embodiment, compounds I are of formula I.B2 wherein Z is Z-180 and Q is Q4, and R and R⁴ are as per any row of Table U below, which compounds are named I.B2-Z180-Q4-U-1 to I.B2-Z180-Q4-U-42.

In another embodiment, compounds I are of formula I.B1 wherein Z is Z-181 and Q is Q1, and R and R⁴ are as per any row of Table U below, which compounds are named I.B1-Z181-Q1-U-1 to I.B1-Z181-Q1-U-42.

In another embodiment, compounds I are of formula I.B1 wherein Z is Z-181 and Q is Q2, and R and R⁴ are as per any row of Table U below, which compounds are named I.B1-Z181-Q2-U-1 to I.B1-Z181-Q2-U-42.

In another embodiment, compounds I are of formula I.B1 wherein Z is Z-181 and Q is Q3, and R and R⁴ are as per any row of Table U below, which compounds are named I.B1-Z181-Q3-U-1 to I.B1-Z181-Q3-U-42.

In another embodiment, compounds I are of formula I.B1 wherein Z is Z-181 and Q is Q4, and R and R⁴ are as per any row of Table U below, which compounds are named I.B1-Z181-Q4-U-1 to I.B1-Z181-Q4-U-42.

In another embodiment, compounds I are of formula I.B2 wherein Z is Z-181 and Q is Q1, and R and R⁴ are as per any row of Table U below, which compounds are named I.B2-Z181-Q1-U-1 to I.B2-Z181-Q1-U-42.

In another embodiment, compounds I are of formula I.B2 wherein Z is Z-181 and Q is Q2, and R and R⁴ are as per any row of Table U below, which compounds are named I.B2-Z181-Q2-U-1 to I.B2-Z181-Q2-U-42.

In another embodiment, compounds I are of formula I.B2 wherein Z is Z-181 and Q is Q3, and R and R⁴ are as per any row of Table U below, which compounds are named I.B2-Z181-Q3-U-1 to I.B2-Z181-Q3-U-42.

In another embodiment, compounds I are of formula I.B2 wherein Z is Z-181 and Q is Q4, and R and R⁴ are as per any row of Table U below, which compounds are named I.B2-Z181-Q4-U-1 to I.B2-Z181-Q4-U-42.

In another embodiment, compounds I are of formula I.B1 wherein Z is Z-182 and Q is Q1, and R and R⁴ are as per any row of Table U below, which compounds are named I.B1-Z182-Q1-U-1 to I.B1-Z182-Q1-U-42.

In another embodiment, compounds I are of formula I.B1 wherein Z is Z-182 and Q is Q2, and R and R⁴ are as per any row of Table U below, which compounds are named I.B1-Z182-Q2-U-1 to I.B1-Z182-Q2-U-42.

In another embodiment, compounds I are of formula I.B1 wherein Z is Z-182 and Q is Q3, and R and R⁴ are as per any row of Table U below, which compounds are named I.B1-Z182-Q3-U-1 to I.B1-Z182-Q3-U-42.

In another embodiment, compounds I are of formula I.B1 wherein Z is Z-182 and Q is Q4, and R and R⁴ are as per any row of Table U below, which compounds are named I.B1-Z182-Q4-U-1 to I.B1-Z182-Q4-U-42.

In another embodiment, compounds I are of formula I.B2 wherein Z is Z-182 and Q is Q1, and R and R⁴ are as per any row of Table U below, which compounds are named I.B2-Z182-Q1-U-1 to I.B2-Z182-Q1-U-42.

In another embodiment, compounds I are of formula I.B2 wherein Z is Z-182 and Q is Q2, and R and R⁴ are as per any row of Table U below, which compounds are named I.B2-Z182-Q2-U-1 to I.B2-Z182-Q2-U-42.

In another embodiment, compounds I are of formula I.B2 wherein Z is Z-182 and Q is Q3, and R and R⁴ are as per any row of Table U below, which compounds are named I.B2-Z182-Q3-U-1 to I.B2-Z182-Q3-U-42.

In another embodiment, compounds I are of formula I.B2 wherein Z is Z-182 and Q is Q4, and R and R⁴ are as per any row of Table U below, which compounds are named I.B2-Z182-Q4-U-1 to I.B2-Z182-Q4-U-42.

In another embodiment, compounds I are of formula I.B1 wherein Z is Z-183 and Q is Q1, and R and R⁴ are as per any row of Table U below, which compounds are named I.B1-Z183-Q1-U-1 to I.B1-Z183-Q1-U-42.

In another embodiment, compounds I are of formula I.B1 wherein Z is Z-183 and Q is Q2, and R and R⁴ are as per any row of Table U below, which compounds are named I.B1-Z183-Q2-U-1 to I.B1-Z183-Q2-U-42.

In another embodiment, compounds I are of formula I.B1 wherein Z is Z-183 and Q is Q3, and R and R⁴ are as per any row of Table U below, which compounds are named I.B1-Z183-Q3-U-1 to I.B1-Z183-Q3-U-42.

In another embodiment, compounds I are of formula I.B1 wherein Z is Z-183 and Q is Q4, and R and R⁴ are as per any row of Table U below, which compounds are named I.B1-Z183-Q4-U-1 to I.B1-Z183-Q4-U-42.

In another embodiment, compounds I are of formula I.B2 wherein Z is Z-183 and Q is Q1, and R and R⁴ are as per any row of Table U below, which compounds are named I.B2-Z183-Q1-U-1 to I.B2-Z183-Q1-U-42.

In another embodiment, compounds I are of formula I.B2 wherein Z is Z-183 and Q is Q2, and R and R⁴ are as per any row of Table U below, which compounds are named I.B2-Z183-Q2-U-1 to I.B2-Z183-Q2-U-42.

In another embodiment, compounds I are of formula I.B2 wherein Z is Z-183 and Q is Q3, and R and R⁴ are as per any row of Table U below, which compounds are named I.B2-Z183-Q3-U-1 to I.B2-Z183-Q3-U-42.

In another embodiment, compounds I are of formula I.B2 wherein Z is Z-183 and Q is Q4, and R and R⁴ are as per any row of Table U below, which compounds are named I.B2-Z183-Q4-U-1 to I.B2-Z183-Q4-U-42.

In another embodiment, compounds I are of formula I.B1 wherein Z is Z-184 and Q is Q1, and R and R⁴ are as per any row of Table U below, which compounds are named I.B1-Z184-Q1-U-1 to I.B1-Z184-Q1-U-42.

In another embodiment, compounds I are of formula I.B1 wherein Z is Z-184 and Q is Q2, and R and R⁴ are as per any row of Table U below, which compounds are named I.B1-Z184-Q2-U-1 to I.B1-Z184-Q2-U-42.

In another embodiment, compounds I are of formula I.B1 wherein Z is Z-184 and Q is Q3, and R and R⁴ are as per any row of Table U below, which compounds are named I.B1-Z184-Q3-U-1 to I.B1-Z184-Q3-U-42.

In another embodiment, compounds I are of formula I.B1 wherein Z is Z-184 and Q is Q4, and R and R⁴ are as per any row of Table U below, which compounds are named I.B1-Z184-Q4-U-1 to I.B1-Z184-Q4-U-42.

In another embodiment, compounds I are of formula I.B2 wherein Z is Z-184 and Q is Q1, and R and R⁴ are as per any row of Table U below, which compounds are named I.B2-Z184-Q1-U-1 to I.B2-Z184-Q1-U-42.

In another embodiment, compounds I are of formula I.B2 wherein Z is Z-184 and Q is Q2, and R and R⁴ are as per any row of Table U below, which compounds are named I.B2-Z184-Q2-U-1 to I.B2-Z184-Q2-U-42.

In another embodiment, compounds I are of formula I.B2 wherein Z is Z-184 and Q is Q3, and R and R⁴ are as per any row of Table U below, which compounds are named I.B2-Z184-Q3-U-1 to I.B2-Z184-Q3-U-42.

In another embodiment, compounds I are of formula I.B2 wherein Z is Z-184 and Q is Q4, and R and R⁴ are as per any row of Table U below, which compounds are named I.B2-Z184-Q4-U-1 to I.B2-Z184-Q4-U-42.

In another embodiment, compounds I are of formula I.B1 wherein Z is Z-185 and Q is Q1, and R and R⁴ are as per any row of Table U below, which compounds are named I.B1-Z185-Q1-U-1 to I.B1-Z185-Q1-U-42.

In another embodiment, compounds I are of formula I.B1 wherein Z is Z-185 and Q is Q2, and R and R⁴ are as per any row of Table U below, which compounds are named I.B1-Z185-Q2-U-1 to I.B1-Z185-Q2-U-42.

In another embodiment, compounds I are of formula I.B1 wherein Z is Z-185 and Q is Q3, and R and R⁴ are as per any row of Table U below, which compounds are named I.B1-Z185-Q3-U-1 to I.B1-Z185-Q3-U-42.

In another embodiment, compounds I are of formula I.B1 wherein Z is Z-185 and Q is Q4, and R and R⁴ are as per any row of Table U below, which compounds are named I.B1-Z185-Q4-U-1 to I.B1-Z185-Q4-U-42.

In another embodiment, compounds I are of formula I.B2 wherein Z is Z-185 and Q is Q1, and R and R⁴ are as per any row of Table U below, which compounds are named I.B2-Z185-Q1-U-1 to I.B2-Z185-Q1-U-42.

In another embodiment, compounds I are of formula I.B2 wherein Z is Z-185 and Q is Q2, and R and R⁴ are as per any row of Table U below, which compounds are named I.B2-Z185-Q2-U-1 to I.B2-Z185-Q2-U-42.

In another embodiment, compounds I are of formula I.B2 wherein Z is Z-185 and Q is Q3, and R and R⁴ are as per any row of Table U below, which compounds are named I.B2-Z185-Q3-U-1 to I.B2-Z185-Q3-U-42.

In another embodiment, compounds I are of formula I.B2 wherein Z is Z-185 and Q is Q4, and R and R⁴ are as per any row of Table U below, which compounds are named I.B2-Z185-Q4-U-1 to I.B2-Z185-Q4-U-42.

In another embodiment, compounds I are of formula I.B1 wherein Z is Z-186 and Q is Q1, and R and R⁴ are as per any row of Table U below, which compounds are named I.B1-Z186-Q1-U-1 to I.B1-Z186-Q1-U-42.

In another embodiment, compounds I are of formula I.B1 wherein Z is Z-186 and Q is Q2, and R and R⁴ are as per any row of Table U below, which compounds are named I.B1-Z186-Q2-U-1 to I.B1-Z186-Q2-U-42.

In another embodiment, compounds I are of formula I.B1 wherein Z is Z-186 and Q is Q3, and R and R⁴ are as per any row of Table U below, which compounds are named I.B1-Z186-Q3-U-1 to I.B1-Z186-Q3-U-42.

In another embodiment, compounds I are of formula I.B1 wherein Z is Z-186 and Q is Q4, and R and R⁴ are as per any row of Table U below, which compounds are named I.B1-Z186-Q4-U-1 to I.B1-Z186-Q4-U-42.

In another embodiment, compounds I are of formula I.B2 wherein Z is Z-186 and Q is Q1, and R and R⁴ are as per any row of Table U below, which compounds are named I.B2-Z186-Q1-U-1 to I.B2-Z186-Q1-U-42.

In another embodiment, compounds I are of formula I.B2 wherein Z is Z-186 and Q is Q2, and R and R⁴ are as per any row of Table U below, which compounds are named I.B2-Z186-Q2-U-1 to I.B2-Z186-Q2-U-42.

In another embodiment, compounds I are of formula I.B2 wherein Z is Z-186 and Q is Q3, and R and R⁴ are as per any row of Table U below, which compounds are named I.B2-Z186-Q3-U-1 to I.B2-Z186-Q3-U-42.

In another embodiment, compounds I are of formula I.B2 wherein Z is Z-186 and Q is Q4, and R and R⁴ are as per any row of Table U below, which compounds are named I.B2-Z186-Q4-U-1 to I.B2-Z186-Q4-U-42.

In another embodiment, compounds I are of formula I.B1 wherein Z is Z-187 and Q is Q1, and R and R⁴ are as per any row of Table U below, which compounds are named I.B1-Z187-Q1-U-1 to I.B1-Z187-Q1-U-42.

In another embodiment, compounds I are of formula I.B1 wherein Z is Z-187 and Q is Q2, and R and R⁴ are as per any row of Table U below, which compounds are named I.B1-Z187-Q2-U-1 to I.B1-Z187-Q2-U-42.

In another embodiment, compounds I are of formula I.B1 wherein Z is Z-187 and Q is Q3, and R and R⁴ are as per any row of Table U below, which compounds are named I.B1-Z187-Q3-U-1 to I.B1-Z187-Q3-U-42.

In another embodiment, compounds I are of formula I.B1 wherein Z is Z-187 and Q is Q4, and R and R⁴ are as per any row of Table U below, which compounds are named I.B1-Z187-Q4-U-1 to I.B1-Z187-Q4-U-42.

In another embodiment, compounds I are of formula I.B2 wherein Z is Z-187 and Q is Q1, and R and R⁴ are as per any row of Table U below, which compounds are named I.B2-Z187-Q1-U-1 to I.B2-Z187-Q1-U-42.

In another embodiment, compounds I are of formula I.B2 wherein Z is Z-187 and Q is Q2, and R and R⁴ are as per any row of Table U below, which compounds are named I.B2-Z187-Q2-U-1 to I.B2-Z187-Q2-U-42.

In another embodiment, compounds I are of formula I.B2 wherein Z is Z-187 and Q is Q3, and R and R⁴ are as per any row of Table U below, which compounds are named I.B2-Z187-Q3-U-1 to I.B2-Z187-Q3-U-42.

In another embodiment, compounds I are of formula I.B2 wherein Z is Z-187 and Q is Q4, and R and R⁴ are as per any row of Table U below, which compounds are named I.B2-Z187-Q4-U-1 to I.B2-Z187-Q4-U-42.

In another embodiment, compounds I are of formula I.B1 wherein Z is Z-188 and Q is Q1, and R and R⁴ are as per any row of Table U below, which compounds are named I.B1-Z188-Q1-U-1 to I.B1-Z188-Q1-U-42.

In another embodiment, compounds I are of formula I.B1 wherein Z is Z-188 and Q is Q2, and R and R⁴ are as per any row of Table U below, which compounds are named I.B1-Z188-Q2-U-1 to I.B1-Z188-Q2-U-42.

In another embodiment, compounds I are of formula I.B1 wherein Z is Z-188 and Q is Q3, and R and R⁴ are as per any row of Table U below, which compounds are named I.B1-Z188-Q3-U-1 to I.B1-Z188-Q3-U-42.

In another embodiment, compounds I are of formula I.B1 wherein Z is Z-188 and Q is Q4, and R and R⁴ are as per any row of Table U below, which compounds are named I.B1-Z188-Q4-U-1 to I.B1-Z188-Q4-U-42.

In another embodiment, compounds I are of formula I.B2 wherein Z is Z-188 and Q is Q1, and R and R⁴ are as per any row of Table U below, which compounds are named I.B2-Z188-Q1-U-1 to I.B2-Z188-Q1-U-42.

In another embodiment, compounds I are of formula I.B2 wherein Z is Z-188 and Q is Q2, and R and R⁴ are as per any row of Table U below, which compounds are named I.B2-Z188-Q2-U-1 to I.B2-Z188-Q2-U-42.

In another embodiment, compounds I are of formula I.B2 wherein Z is Z-188 and Q is Q3, and R and R⁴ are as per any row of Table U below, which compounds are named I.B2-Z188-Q3-U-1 to I.B2-Z188-Q3-U-42.

In another embodiment, compounds I are of formula I.B2 wherein Z is Z-188 and Q is Q4, and R and R⁴ are as per any row of Table U below, which compounds are named I.B2-Z188-Q4-U-1 to I.B2-Z188-Q4-U-42.

In another embodiment, compounds I are of formula I.B1 wherein Z is Z-189 and Q is Q1, and R and R⁴ are as per any row of Table U below, which compounds are named I.B1-Z189-Q1-U-1 to I.B1-Z189-Q1-U-42.

In another embodiment, compounds I are of formula I.B1 wherein Z is Z-189 and Q is Q2, and R and R⁴ are as per any row of Table U below, which compounds are named I.B1-Z189-Q2-U-1 to I.B1-Z189-Q2-U-42.

In another embodiment, compounds I are of formula I.B1 wherein Z is Z-189 and Q is Q3, and R and R⁴ are as per any row of Table U below, which compounds are named I.B1-Z189-Q3-U-1 to I.B1-Z189-Q3-U-42.

In another embodiment, compounds I are of formula I.B1 wherein Z is Z-189 and Q is Q4, and R and R⁴ are as per any row of Table U below, which compounds are named I.B1-Z189-Q4-U-1 to I.B1-Z189-Q4-U-42.

In another embodiment, compounds I are of formula I.B2 wherein Z is Z-189 and Q is Q1, and R and R⁴ are as per any row of Table U below, which compounds are named I.B2-Z189-Q1-U-1 to I.B2-Z189-Q1-U-42.

In another embodiment, compounds I are of formula I.B2 wherein Z is Z-189 and Q is Q2, and R and R⁴ are as per any row of Table U below, which compounds are named I.B2-Z189-Q2-U-1 to I.B2-Z189-Q2-U-42.

In another embodiment, compounds I are of formula I.B2 wherein Z is Z-189 and Q is Q3, and R and R⁴ are as per any row of Table U below, which compounds are named I.B2-Z189-Q3-U-1 to I.B2-Z189-Q3-U-42.

In another embodiment, compounds I are of formula I.B2 wherein Z is Z-189 and Q is Q4, and R and R⁴ are as per any row of Table U below, which compounds are named I.B2-Z189-Q4-U-1 to I.B2-Z189-Q4-U-42.

In another embodiment, compounds I are of formula I.B1 wherein Z is Z-190 and Q is Q1, and R and R⁴ are as per any row of Table U below, which compounds are named I.B1-Z190-Q1-U-1 to I.B1-Z190-Q1-U-42.

In another embodiment, compounds I are of formula I.B1 wherein Z is Z-190 and Q is Q2, and R and R⁴ are as per any row of Table U below, which compounds are named I.B1-Z190-Q2-U-1 to I.B1-Z190-Q2-U-42.

In another embodiment, compounds I are of formula I.B1 wherein Z is Z-190 and Q is Q3, and R and R⁴ are as per any row of Table U below, which compounds are named I.B1-Z190-Q3-U-1 to I.B1-Z190-Q3-U-42.

In another embodiment, compounds I are of formula I.B1 wherein Z is Z-190 and Q is Q4, and R and R⁴ are as per any row of Table U below, which compounds are named I.B1-Z190-Q4-U-1 to I.B1-Z190-Q4-U-42.

In another embodiment, compounds I are of formula I.B2 wherein Z is Z-190 and Q is Q1, and R and R⁴ are as per any row of Table U below, which compounds are named I.B2-Z190-Q1-U-1 to I.B2-Z190-Q1-U-42.

In another embodiment, compounds I are of formula I.B2 wherein Z is Z-190 and Q is Q2, and R and R⁴ are as per any row of Table U below, which compounds are named I.B2-Z190-Q2-U-1 to I.B2-Z190-Q2-U-42.

In another embodiment, compounds I are of formula I.B2 wherein Z is Z-190 and Q is Q3, and R and R⁴ are as per any row of Table U below, which compounds are named I.B2-Z190-Q3-U-1 to I.B2-Z190-Q3-U-42.

In another embodiment, compounds I are of formula I.B2 wherein Z is Z-190 and Q is Q4, and R and R⁴ are as per any row of Table U below, which compounds are named I.B2-Z190-Q4-U-1 to I.B2-Z190-Q4-U-42.

In another embodiment, compounds I are of formula I.B1 wherein Z is Z-191 and Q is Q1, and R and R⁴ are as per any row of Table U below, which compounds are named I.B1-Z191-Q1-U-1 to I.B1-Z191-Q1-U-42.

In another embodiment, compounds I are of formula I.B1 wherein Z is Z-191 and Q is Q2, and R and R⁴ are as per any row of Table U below, which compounds are named I.B1-Z191-Q2-U-1 to I.B1-Z191-Q2-U-42.

In another embodiment, compounds I are of formula I.B1 wherein Z is Z-191 and Q is Q3, and R and R⁴ are as per any row of Table U below, which compounds are named I.B1-Z191-Q3-U-1 to I.B1-Z191-Q3-U-42.

In another embodiment, compounds I are of formula I.B1 wherein Z is Z-191 and Q is Q4, and R and R⁴ are as per any row of Table U below, which compounds are named I.B1-Z191-Q4-U-1 to I.B1-Z191-Q4-U-42.

In another embodiment, compounds I are of formula I.B2 wherein Z is Z-191 and Q is Q1, and R and R⁴ are as per any row of Table U below, which compounds are named I.B2-Z191-Q1-U-1 to I.B2-Z191-Q1-U-42.

In another embodiment, compounds I are of formula I.B2 wherein Z is Z-191 and Q is Q2, and R and R⁴ are as per any row of Table U below, which compounds are named I.B2-Z191-Q2-U-1 to I.B2-Z191-Q2-U-42.

In another embodiment, compounds I are of formula I.B2 wherein Z is Z-191 and Q is Q3, and R and R⁴ are as per any row of Table U below, which compounds are named I.B2-Z191-Q3-U-1 to I.B2-Z191-Q3-U-42.

In another embodiment, compounds I are of formula I.B2 wherein Z is Z-191 and Q is Q4, and R and R⁴ are as per any row of Table U below, which compounds are named I.B2-Z191-Q4-U-1 to I.B2-Z191-Q4-U-42.

In another embodiment, compounds I are of formula I.B1 wherein Z is Z-192 and Q is Q1, and R and R⁴ are as per any row of Table U below, which compounds are named I.B1-Z192-Q1-U-1 to I.B1-Z192-Q1-U-42.

In another embodiment, compounds I are of formula I.B1 wherein Z is Z-192 and Q is Q2, and R and R⁴ are as per any row of Table U below, which compounds are named I.B1-Z192-Q2-U-1 to I.B1-Z192-Q2-U-42.

In another embodiment, compounds I are of formula I.B1 wherein Z is Z-192 and Q is Q3, and R and R⁴ are as per any row of Table U below, which compounds are named I.B1-Z192-Q3-U-1 to I.B1-Z192-Q3-U-42.

In another embodiment, compounds I are of formula I.B1 wherein Z is Z-192 and Q is Q4, and R and R⁴ are as per any row of Table U below, which compounds are named I.B1-Z192-Q4-U-1 to I.B1-Z192-Q4-U-42.

In another embodiment, compounds I are of formula I.B2 wherein Z is Z-192 and Q is Q1, and R and R⁴ are as per any row of Table U below, which compounds are named I.B2-Z192-Q1-U-1 to I.B2-Z192-Q1-U-42.

In another embodiment, compounds I are of formula I.B2 wherein Z is Z-192 and Q is Q2, and R and R⁴ are as per any row of Table U below, which compounds are named I.B2-Z192-Q2-U-1 to I.B2-Z192-Q2-U-42.

In another embodiment, compounds I are of formula I.B2 wherein Z is Z-192 and Q is Q3, and R and R⁴ are as per any row of Table U below, which compounds are named I.B2-Z192-Q3-U-1 to I.B2-Z192-Q3-U-42.

In another embodiment, compounds I are of formula I.B2 wherein Z is Z-192 and Q is Q4, and R and R⁴ are as per any row of Table U below, which compounds are named I.B2-Z192-Q4-U-1 to I.B2-Z192-Q4-U-42.

In another embodiment, compounds I are of formula I.B1 wherein Z is Z-193 and Q is Q1, and R and R⁴ are as per any row of Table U below, which compounds are named I.B1-Z193-Q1-U-1 to I.B1-Z193-Q1-U-42.

In another embodiment, compounds I are of formula I.B1 wherein Z is Z-193 and Q is Q2, and R and R⁴ are as per any row of Table U below, which compounds are named I.B1-Z193-Q2-U-1 to I.B1-Z193-Q2-U-42.

In another embodiment, compounds I are of formula I.B1 wherein Z is Z-193 and Q is Q3, and R and R⁴ are as per any row of Table U below, which compounds are named I.B1-Z193-Q3-U-1 to I.B1-Z193-Q3-U-42.

In another embodiment, compounds I are of formula I.B1 wherein Z is Z-193 and Q is Q4, and R and R⁴ are as per any row of Table U below, which compounds are named I.B1-Z193-Q4-U-1 to I.B1-Z193-Q4-U-42.

In another embodiment, compounds I are of formula I.B2 wherein Z is Z-193 and Q is Q1, and R and R⁴ are as per any row of Table U below, which compounds are named I.B2-Z193-Q1-U-1 to I.B2-Z193-Q1-U-42.

In another embodiment, compounds I are of formula I.B2 wherein Z is Z-193 and Q is Q2, and R and R⁴ are as per any row of Table U below, which compounds are named I.B2-Z193-Q2-U-1 to I.B2-Z193-Q2-U-42.

In another embodiment, compounds I are of formula I.B2 wherein Z is Z-193 and Q is Q3, and R and R⁴ are as per any row of Table U below, which compounds are named I.B2-Z193-Q3-U-1 to I.B2-Z193-Q3-U-42.

In another embodiment, compounds I are of formula I.B2 wherein Z is Z-193 and Q is Q4, and R and R⁴ are as per any row of Table U below, which compounds are named I.B2-Z193-Q4-U-1 to I.B2-Z193-Q4-U-42.

In another embodiment, compounds I are of formula I.B1 wherein Z is Z-194 and Q is Q1, and R and R⁴ are as per any row of Table U below, which compounds are named I.B1-Z194-Q1-U-1 to I.B1-Z194-Q1-U-42.

In another embodiment, compounds I are of formula I.B1 wherein Z is Z-194 and Q is Q2, and R and R⁴ are as per any row of Table U below, which compounds are named I.B1-Z194-Q2-U-1 to I.B1-Z194-Q2-U-42.

In another embodiment, compounds I are of formula I.B1 wherein Z is Z-194 and Q is Q3, and R and R⁴ are as per any row of Table U below, which compounds are named I.B1-Z194-Q3-U-1 to I.B1-Z194-Q3-U-42.

In another embodiment, compounds I are of formula I.B1 wherein Z is Z-194 and Q is Q4, and R and R⁴ are as per any row of Table U below, which compounds are named I.B1-Z194-Q4-U-1 to I.B1-Z194-Q4-U-42.

In another embodiment, compounds I are of formula I.B2 wherein Z is Z-194 and Q is Q1, and R and R⁴ are as per any row of Table U below, which compounds are named I.B2-Z194-Q1-U-1 to I.B2-Z194-Q1-U-42.

In another embodiment, compounds I are of formula I.B2 wherein Z is Z-194 and Q is Q2, and R and R⁴ are as per any row of Table U below, which compounds are named I.B2-Z194-Q2-U-1 to I.B2-Z194-Q2-U-42.

In another embodiment, compounds I are of formula I.B2 wherein Z is Z-194 and Q is Q3, and R and R⁴ are as per any row of Table U below, which compounds are named I.B2-Z194-Q3-U-1 to I.B2-Z194-Q3-U-42.

In another embodiment, compounds I are of formula I.B2 wherein Z is Z-194 and Q is Q4, and R and R⁴ are as per any row of Table U below, which compounds are named I.B2-Z194-Q4-U-1 to I.B2-Z194-Q4-U-42.

In another embodiment, compounds I are of formula I.B1 wherein Z is Z-195 and Q is Q1, and R and R⁴ are as per any row of Table U below, which compounds are named I.B1-Z195-Q1-U-1 to I.B1-Z195-Q1-U-42.

In another embodiment, compounds I are of formula I.B1 wherein Z is Z-195 and Q is Q2, and R and R⁴ are as per any row of Table U below, which compounds are named I.B1-Z195-Q2-U-1 to I.B1-Z195-Q2-U-42.

In another embodiment, compounds I are of formula I.B1 wherein Z is Z-195 and Q is Q3, and R and R⁴ are as per any row of Table U below, which compounds are named I.B1-Z195-Q3-U-1 to I.B1-Z195-Q3-U-42.

In another embodiment, compounds I are of formula I.B1 wherein Z is Z-195 and Q is Q4, and R and R⁴ are as per any row of Table U below, which compounds are named I.B1-Z195-Q4-U-1 to I.B1-Z195-Q4-U-42.

In another embodiment, compounds I are of formula I.B2 wherein Z is Z-195 and Q is Q1, and R and R⁴ are as per any row of Table U below, which compounds are named I.B2-Z195-Q1-U-1 to I.B2-Z195-Q1-U-42.

In another embodiment, compounds I are of formula I.B2 wherein Z is Z-195 and Q is Q2, and R and R⁴ are as per any row of Table U below, which compounds are named I.B2-Z195-Q2-U-1 to I.B2-Z195-Q2-U-42.

In another embodiment, compounds I are of formula I.B2 wherein Z is Z-195 and Q is Q3, and R and R⁴ are as per any row of Table U below, which compounds are named I.B2-Z195-Q3-U-1 to I.B2-Z195-Q3-U-42.

In another embodiment, compounds I are of formula I.B2 wherein Z is Z-195 and Q is Q4, and R and R⁴ are as per any row of Table U below, which compounds are named I.B2-Z195-Q4-U-1 to I.B2-Z195-Q4-U-42.

In another embodiment, compounds I are of formula I.B1 wherein Z is Z-196 and Q is Q1, and R and R⁴ are as per any row of Table U below, which compounds are named I.B1-Z196-Q1-U-1 to I.B1-Z196-Q1-U-42.

In another embodiment, compounds I are of formula I.B1 wherein Z is Z-196 and Q is Q2, and R and R⁴ are as per any row of Table U below, which compounds are named I.B1-Z196-Q2-U-1 to I.B1-Z196-Q2-U-42.

In another embodiment, compounds I are of formula I.B1 wherein Z is Z-196 and Q is Q3, and R and R⁴ are as per any row of Table U below, which compounds are named I.B1-Z196-Q3-U-1 to I.B1-Z196-Q3-U-42.

In another embodiment, compounds I are of formula I.B1 wherein Z is Z-196 and Q is Q4, and R and R⁴ are as per any row of Table U below, which compounds are named I.B1-Z196-Q4-U-1 to I.B1-Z196-Q4-U-42.

In another embodiment, compounds I are of formula I.B2 wherein Z is Z-196 and Q is Q1, and R and R⁴ are as per any row of Table U below, which compounds are named I.B2-Z196-Q1-U-1 to I.B2-Z196-Q1-U-42.

In another embodiment, compounds I are of formula I.B2 wherein Z is Z-196 and Q is Q2, and R and R⁴ are as per any row of Table U below, which compounds are named I.B2-Z196-Q2-U-1 to I.B2-Z196-Q2-U-42.

In another embodiment, compounds I are of formula I.B2 wherein Z is Z-196 and Q is Q3, and R and R⁴ are as per any row of Table U below, which compounds are named I.B2-Z196-Q3-U-1 to I.B2-Z196-Q3-U-42.

In another embodiment, compounds I are of formula I.B2 wherein Z is Z-196 and Q is Q4, and R and R⁴ are as per any row of Table U below, which compounds are named I.B2-Z196-Q4-U-1 to I.B2-Z196-Q4-U-42.

In another embodiment, compounds I are of formula I.B1 wherein Z is Z-197 and Q is Q1, and R and R⁴ are as per any row of Table U below, which compounds are named I.B1-Z197-Q1-U-1 to I.B1-Z197-Q1-U-42.

In another embodiment, compounds I are of formula I.B1 wherein Z is Z-197 and Q is Q2, and R and R⁴ are as per any row of Table U below, which compounds are named I.B1-Z197-Q2-U-1 to I.B1-Z197-Q2-U-42.

In another embodiment, compounds I are of formula I.B1 wherein Z is Z-197 and Q is Q3, and R and R⁴ are as per any row of Table U below, which compounds are named I.B1-Z197-Q3-U-1 to I.B1-Z197-Q3-U-42.

In another embodiment, compounds I are of formula I.B1 wherein Z is Z-197 and Q is Q4, and R and R⁴ are as per any row of Table U below, which compounds are named I.B1-Z197-Q4-U-1 to I.B1-Z197-Q4-U-42.

In another embodiment, compounds I are of formula I.B2 wherein Z is Z-197 and Q is Q1, and R and R⁴ are as per any row of Table U below, which compounds are named I.B2-Z197-Q1-U-1 to I.B2-Z197-Q1-U-42.

In another embodiment, compounds I are of formula I.B2 wherein Z is Z-197 and Q is Q2, and R and R⁴ are as per any row of Table U below, which compounds are named I.B2-Z197-Q2-U-1 to I.B2-Z197-Q2-U-42.

In another embodiment, compounds I are of formula I.B2 wherein Z is Z-197 and Q is Q3, and R and R⁴ are as per any row of Table U below, which compounds are named I.B2-Z197-Q3-U-1 to I.B2-Z197-Q3-U-42.

In another embodiment, compounds I are of formula I.B2 wherein Z is Z-197 and Q is Q4, and R and R⁴ are as per any row of Table U below, which compounds are named I.B2-Z197-Q4-U-1 to I.B2-Z197-Q4-U-42.

In another embodiment, compounds I are of formula I.B1 wherein Z is Z-198 and Q is Q1, and R and R⁴ are as per any row of Table U below, which compounds are named I.B1-Z198-Q1-U-1 to I.B1-Z198-Q1-U-42.

In another embodiment, compounds I are of formula I.B1 wherein Z is Z-198 and Q is Q2, and R and R⁴ are as per any row of Table U below, which compounds are named I.B1-Z198-Q2-U-1 to I.B1-Z198-Q2-U-42.

In another embodiment, compounds I are of formula I.B1 wherein Z is Z-198 and Q is Q3, and R and R⁴ are as per any row of Table U below, which compounds are named I.B1-Z198-Q3-U-1 to I.B1-Z198-Q3-U-42.

In another embodiment, compounds I are of formula I.B1 wherein Z is Z-198 and Q is Q4, and R and R⁴ are as per any row of Table U below, which compounds are named I.B1-Z198-Q4-U-1 to I.B1-Z198-Q4-U-42.

In another embodiment, compounds I are of formula I.B2 wherein Z is Z-198 and Q is Q1, and R and R⁴ are as per any row of Table U below, which compounds are named I.B2-Z198-Q1-U-1 to I.B2-Z198-Q1-U-42.

In another embodiment, compounds I are of formula I.B2 wherein Z is Z-198 and Q is Q2, and R and R⁴ are as per any row of Table U below, which compounds are named I.B2-Z198-Q2-U-1 to I.B2-Z198-Q2-U-42.

In another embodiment, compounds I are of formula I.B2 wherein Z is Z-198 and Q is Q3, and R and R⁴ are as per any row of Table U below, which compounds are named I.B2-Z198-Q3-U-1 to I.B2-Z198-Q3-U-42.

In another embodiment, compounds I are of formula I.B2 wherein Z is Z-198 and Q is Q4, and R and R⁴ are as per any row of Table U below, which compounds are named I.B2-Z198-Q4-U-1 to I.B2-Z198-Q4-U-42.

In another embodiment, compounds I are of formula I.B1 wherein Z is Z-199 and Q is Q1, and R and R⁴ are as per any row of Table U below, which compounds are named I.B1-Z199-Q1-U-1 to I.B1-Z199-Q1-U-42.

In another embodiment, compounds I are of formula I.B1 wherein Z is Z-199 and Q is Q2, and R and R⁴ are as per any row of Table U below, which compounds are named I.B1-Z199-Q2-U-1 to I.B1-Z199-Q2-U-42.

In another embodiment, compounds I are of formula I.B1 wherein Z is Z-199 and Q is Q3, and R and R⁴ are as per any row of Table U below, which compounds are named I.B1-Z199-Q3-U-1 to I.B1-Z199-Q3-U-42.

In another embodiment, compounds I are of formula I.B1 wherein Z is Z-199 and Q is Q4, and R and R⁴ are as per any row of Table U below, which compounds are named I.B1-Z199-Q4-U-1 to I.B1-Z199-Q4-U-42.

In another embodiment, compounds I are of formula I.B2 wherein Z is Z-199 and Q is Q1, and R and R⁴ are as per any row of Table U below, which compounds are named I.B2-Z199-Q1-U-1 to I.B2-Z199-Q1-U-42.

In another embodiment, compounds I are of formula I.B2 wherein Z is Z-199 and Q is Q2, and R and R⁴ are as per any row of Table U below, which compounds are named I.B2-Z199-Q2-U-1 to I.B2-Z199-Q2-U-42.

In another embodiment, compounds I are of formula I.B2 wherein Z is Z-199 and Q is Q3, and R and R⁴ are as per any row of Table U below, which compounds are named I.B2-Z199-Q3-U-1 to I.B2-Z199-Q3-U-42.

In another embodiment, compounds I are of formula I.B2 wherein Z is Z-199 and Q is Q4, and R and R⁴ are as per any row of Table U below, which compounds are named I.B2-Z199-Q4-U-1 to I.B2-Z199-Q4-U-42.

In another embodiment, compounds I are of formula I.B1 wherein Z is Z-200 and Q is Q1, and R and R⁴ are as per any row of Table U below, which compounds are named I.B1-Z200-Q1-U-1 to I.B1-Z200-Q1-U-42.

In another embodiment, compounds I are of formula I.B1 wherein Z is Z-200 and Q is Q2, and R and R⁴ are as per any row of Table U below, which compounds are named I.B1-Z200-Q2-U-1 to I.B1-Z200-Q2-U-42.

In another embodiment, compounds I are of formula I.B1 wherein Z is Z-200 and Q is Q3, and R and R⁴ are as per any row of Table U below, which compounds are named I.B1-Z200-Q3-U-1 to I.B1-Z200-Q3-U-42.

In another embodiment, compounds I are of formula I.B1 wherein Z is Z-200 and Q is Q4, and R and R⁴ are as per any row of Table U below, which compounds are named I.B1-Z200-Q4-U-1 to I.B1-Z200-Q4-U-42.

In another embodiment, compounds I are of formula I.B2 wherein Z is Z-200 and Q is Q1, and R and R⁴ are as per any row of Table U below, which compounds are named I.B2-Z200-Q1-U-1 to I.B2-Z200-Q1-U-42.

In another embodiment, compounds I are of formula I.B2 wherein Z is Z-200 and Q is Q2, and R and R⁴ are as per any row of Table U below, which compounds are named I.B2-Z200-Q2-U-1 to I.B2-Z200-Q2-U-42.

In another embodiment, compounds I are of formula I.B2 wherein Z is Z-200 and Q is Q3, and R and R⁴ are as per any row of Table U below, which compounds are named I.B2-Z200-Q3-U-1 to I.B2-Z200-Q3-U-42.

In another embodiment, compounds I are of formula I.B2 wherein Z is Z-200 and Q is Q4, and R and R⁴ are as per any row of Table U below, which compounds are named I.B2-Z200-Q4-U-1 to I.B2-Z200-Q4-U-42.

In another embodiment, compounds I are of formula I.B1 wherein Z is Z-201 and Q is Q1, and R and R⁴ are as per any row of Table U below, which compounds are named I.B1-Z201-Q1-U-1 to I.B1-Z201-Q1-U-42.

In another embodiment, compounds I are of formula I.B1 wherein Z is Z-201 and Q is Q2, and R and R⁴ are as per any row of Table U below, which compounds are named I.B1-Z201-Q2-U-1 to I.B1-Z201-Q2-U-42.

In another embodiment, compounds I are of formula I.B1 wherein Z is Z-201 and Q is Q3, and R and R⁴ are as per any row of Table U below, which compounds are named I.B1-Z201-Q3-U-1 to I.B1-Z201-Q3-U-42.

In another embodiment, compounds I are of formula I.B1 wherein Z is Z-201 and Q is Q4, and R and R⁴ are as per any row of Table U below, which compounds are named I.B1-Z201-Q4-U-1 to I.B1-Z201-Q4-U-42.

In another embodiment, compounds I are of formula I.B2 wherein Z is Z-201 and Q is Q1, and R and R⁴ are as per any row of Table U below, which compounds are named I.B2-Z201-Q1-U-1 to I.B2-Z201-Q1-U-42.

In another embodiment, compounds I are of formula I.B2 wherein Z is Z-201 and Q is Q2, and R and R⁴ are as per any row of Table U below, which compounds are named I.B2-Z201-Q2-U-1 to I.B2-Z201-Q2-U-42.

In another embodiment, compounds I are of formula I.B2 wherein Z is Z-201 and Q is Q3, and R and R⁴ are as per any row of Table U below, which compounds are named I.B2-Z201-Q3-U-1 to I.B2-Z201-Q3-U-42.

In another embodiment, compounds I are of formula I.B2 wherein Z is Z-201 and Q is Q4, and R and R⁴ are as per any row of Table U below, which compounds are named I.B2-Z201-Q4-U-1 to I.B2-Z201-Q4-U-42.

In another embodiment, compounds I are of formula I.B1 wherein Z is Z-202 and Q is Q1, and R and R⁴ are as per any row of Table U below, which compounds are named I.B1-Z202-Q1-U-1 to I.B1-Z202-Q1-U-42.

In another embodiment, compounds I are of formula I.B1 wherein Z is Z-202 and Q is Q2, and R and R⁴ are as per any row of Table U below, which compounds are named I.B1-Z202-Q2-U-1 to I.B1-Z202-Q2-U-42.

In another embodiment, compounds I are of formula I.B1 wherein Z is Z-202 and Q is Q3, and R and R⁴ are as per any row of Table U below, which compounds are named I.B1-Z202-Q3-U-1 to I.B1-Z202-Q3-U-42.

In another embodiment, compounds I are of formula I.B1 wherein Z is Z-202 and Q is Q4, and R and R⁴ are as per any row of Table U below, which compounds are named I.B1-Z202-Q4-U-1 to I.B1-Z202-Q4-U-42.

In another embodiment, compounds I are of formula I.B2 wherein Z is Z-202 and Q is Q1, and R and R⁴ are as per any row of Table U below, which compounds are named I.B2-Z202-Q1-U-1 to I.B2-Z202-Q1-U-42.

In another embodiment, compounds I are of formula I.B2 wherein Z is Z-202 and Q is Q2, and R and R⁴ are as per any row of Table U below, which compounds are named I.B2-Z202-Q2-U-1 to I.B2-Z202-Q2-U-42.

In another embodiment, compounds I are of formula I.B2 wherein Z is Z-202 and Q is Q3, and R and R⁴ are as per any row of Table U below, which compounds are named I.B2-Z202-Q3-U-1 to I.B2-Z202-Q3-U-42.

In another embodiment, compounds I are of formula I.B2 wherein Z is Z-202 and Q is Q4, and R and R⁴ are as per any row of Table U below, which compounds are named I.B2-Z202-Q4-U-1 to I.B2-Z202-Q4-U-42.

In another embodiment, compounds I are of formula I.B1 wherein Z is Z-203 and Q is Q1, and R and R⁴ are as per any row of Table U below, which compounds are named I.B1-Z203-Q1-U-1 to I.B1-Z203-Q1-U-42.

In another embodiment, compounds I are of formula I.B1 wherein Z is Z-203 and Q is Q2, and R and R⁴ are as per any row of Table U below, which compounds are named I.B1-Z203-Q2-U-1 to I.B1-Z203-Q2-U-42.

In another embodiment, compounds I are of formula I.B1 wherein Z is Z-203 and Q is Q3, and R and R⁴ are as per any row of Table U below, which compounds are named I.B1-Z203-Q3-U-1 to I.B1-Z203-Q3-U-42.

In another embodiment, compounds I are of formula I.B1 wherein Z is Z-203 and Q is Q4, and R and R⁴ are as per any row of Table U below, which compounds are named I.B1-Z203-Q4-U-1 to I.B1-Z203-Q4-U-42.

In another embodiment, compounds I are of formula I.B2 wherein Z is Z-203 and Q is Q1, and R and R⁴ are as per any row of Table U below, which compounds are named I.B2-Z203-Q1-U-1 to I.B2-Z203-Q1-U-42.

In another embodiment, compounds I are of formula I.B2 wherein Z is Z-203 and Q is Q2, and R and R⁴ are as per any row of Table U below, which compounds are named I.B2-Z203-Q2-U-1 to I.B2-Z203-Q2-U-42.

In another embodiment, compounds I are of formula I.B2 wherein Z is Z-203 and Q is Q3, and R and R⁴ are as per any row of Table U below, which compounds are named I.B2-Z203-Q3-U-1 to I.B2-Z203-Q3-U-42.

In another embodiment, compounds I are of formula I.B2 wherein Z is Z-203 and Q is Q4, and R and R⁴ are as per any row of Table U below, which compounds are named I.B2-Z203-Q4-U-1 to I.B2-Z203-Q4-U-42.

In another embodiment, compounds I are of formula I.B1 wherein Z is Z-204 and Q is Q1, and R and R⁴ are as per any row of Table U below, which compounds are named I.B1-Z204-Q1-U-1 to I.B1-Z204-Q1-U-42.

In another embodiment, compounds I are of formula I.B1 wherein Z is Z-204 and Q is Q2, and R and R⁴ are as per any row of Table U below, which compounds are named I.B1-Z204-Q2-U-1 to I.B1-Z204-Q2-U-42.

In another embodiment, compounds I are of formula I.B1 wherein Z is Z-204 and Q is Q3, and R and R⁴ are as per any row of Table U below, which compounds are named I.B1-Z204-Q3-U-1 to I.B1-Z204-Q3-U-42.

In another embodiment, compounds I are of formula I.B1 wherein Z is Z-204 and Q is Q4, and R and R⁴ are as per any row of Table U below, which compounds are named I.B1-Z204-Q4-U-1 to I.B1-Z204-Q4-U-42.

In another embodiment, compounds I are of formula I.B2 wherein Z is Z-204 and Q is Q1, and R and R⁴ are as per any row of Table U below, which compounds are named I.B2-Z204-Q1-U-1 to I.B2-Z204-Q1-U-42.

In another embodiment, compounds I are of formula I.B2 wherein Z is Z-204 and Q is Q2, and R and R⁴ are as per any row of Table U below, which compounds are named I.B2-Z204-Q2-U-1 to I.B2-Z204-Q2-U-42.

In another embodiment, compounds I are of formula I.B2 wherein Z is Z-204 and Q is Q3, and R and R⁴ are as per any row of Table U below, which compounds are named I.B2-Z204-Q3-U-1 to I.B2-Z204-Q3-U-42.

In another embodiment, compounds I are of formula I.B2 wherein Z is Z-204 and Q is Q4, and R and R⁴ are as per any row of Table U below, which compounds are named I.B2-Z204-Q4-U-1 to I.B2-Z204-Q4-U-42.

In another embodiment, compounds I are of formula I.B1 wherein Z is Z-205 and Q is Q1, and R and R⁴ are as per any row of Table U below, which compounds are named I.B1-Z205-Q1-U-1 to I.B1-Z205-Q1-U-42.

In another embodiment, compounds I are of formula I.B1 wherein Z is Z-205 and Q is Q2, and R and R⁴ are as per any row of Table U below, which compounds are named I.B1-Z205-Q2-U-1 to I.B1-Z205-Q2-U-42.

In another embodiment, compounds I are of formula I.B1 wherein Z is Z-205 and Q is Q3, and R and R⁴ are as per any row of Table U below, which compounds are named I.B1-Z205-Q3-U-1 to I.B1-Z205-Q3-U-42.

In another embodiment, compounds I are of formula I.B1 wherein Z is Z-205 and Q is Q4, and R and R⁴ are as per any row of Table U below, which compounds are named I.B1-Z205-Q4-U-1 to I.B1-Z205-Q4-U-42.

In another embodiment, compounds I are of formula I.B2 wherein Z is Z-205 and Q is Q1, and R and R⁴ are as per any row of Table U below, which compounds are named I.B2-Z205-Q1-U-1 to I.B2-Z205-Q1-U-42.

In another embodiment, compounds I are of formula I.B2 wherein Z is Z-205 and Q is Q2, and R and R⁴ are as per any row of Table U below, which compounds are named I.B2-Z205-Q2-U-1 to I.B2-Z205-Q2-U-42.

In another embodiment, compounds I are of formula I.B2 wherein Z is Z-205 and Q is Q3, and R and R⁴ are as per any row of Table U below, which compounds are named I.B2-Z205-Q3-U-1 to I.B2-Z205-Q3-U-42.

In another embodiment, compounds I are of formula I.B2 wherein Z is Z-205 and Q is Q4, and R and R⁴ are as per any row of Table U below, which compounds are named I.B2-Z205-Q4-U-1 to I.B2-Z205-Q4-U-42.

In another embodiment, compounds I are of formula I.B1 wherein Z is Z-206 and Q is Q1, and R and R⁴ are as per any row of Table U below, which compounds are named I.B1-Z206-Q1-U-1 to I.B1-Z206-Q1-U-42.

In another embodiment, compounds I are of formula I.B1 wherein Z is Z-206 and Q is Q2, and R and R⁴ are as per any row of Table U below, which compounds are named I.B1-Z206-Q2-U-1 to I.B1-Z206-Q2-U-42.

In another embodiment, compounds I are of formula I.B1 wherein Z is Z-206 and Q is Q3, and R and R⁴ are as per any row of Table U below, which compounds are named I.B1-Z206-Q3-U-1 to I.B1-Z206-Q3-U-42.

In another embodiment, compounds I are of formula I.B1 wherein Z is Z-206 and Q is Q4, and R and R⁴ are as per any row of Table U below, which compounds are named I.B1-Z206-Q4-U-1 to I.B1-Z206-Q4-U-42.

In another embodiment, compounds I are of formula I.B2 wherein Z is Z-206 and Q is Q1, and R and R⁴ are as per any row of Table U below, which compounds are named I.B2-Z206-Q1-U-1 to I.B2-Z206-Q1-U-42.

In another embodiment, compounds I are of formula I.B2 wherein Z is Z-206 and Q is Q2, and R and R⁴ are as per any row of Table U below, which compounds are named I.B2-Z206-Q2-U-1 to I.B2-Z206-Q2-U-42.

In another embodiment, compounds I are of formula I.B2 wherein Z is Z-206 and Q is Q3, and R and R⁴ are as per any row of Table U below, which compounds are named I.B2-Z206-Q3-U-1 to I.B2-Z206-Q3-U-42.

In another embodiment, compounds I are of formula I.B2 wherein Z is Z-206 and Q is Q4, and R and R⁴ are as per any row of Table U below, which compounds are named I.B2-Z206-Q4-U-1 to I.B2-Z206-Q4-U-42.

In another embodiment, compounds I are of formula I.B1 wherein Z is Z-207 and Q is Q1, and R and R⁴ are as per any row of Table U below, which compounds are named I.B1-Z207-Q1-U-1 to I.B1-Z207-Q1-U-42.

In another embodiment, compounds I are of formula I.B1 wherein Z is Z-207 and Q is Q2, and R and R⁴ are as per any row of Table U below, which compounds are named I.B1-Z207-Q2-U-1 to I.B1-Z207-Q2-U-42.

In another embodiment, compounds I are of formula I.B1 wherein Z is Z-207 and Q is Q3, and R and R⁴ are as per any row of Table U below, which compounds are named I.B1-Z207-Q3-U-1 to I.B1-Z207-Q3-U-42.

In another embodiment, compounds I are of formula I.B1 wherein Z is Z-207 and Q is Q4, and R and R⁴ are as per any row of Table U below, which compounds are named I.B1-Z207-Q4-U-1 to I.B1-Z207-Q4-U-42.

In another embodiment, compounds I are of formula I.B2 wherein Z is Z-207 and Q is Q1, and R and R⁴ are as per any row of Table U below, which compounds are named I.B2-Z207-Q1-U-1 to I.B2-Z207-Q1-U-42.

In another embodiment, compounds I are of formula I.B2 wherein Z is Z-207 and Q is Q2, and R and R⁴ are as per any row of Table U below, which compounds are named I.B2-Z207-Q2-U-1 to I.B2-Z207-Q2-U-42.

In another embodiment, compounds I are of formula I.B2 wherein Z is Z-207 and Q is Q3, and R and R⁴ are as per any row of Table U below, which compounds are named I.B2-Z207-Q3-U-1 to I.B2-Z207-Q3-U-42.

In another embodiment, compounds I are of formula I.B2 wherein Z is Z-207 and Q is Q4, and R and R⁴ are as per any row of Table U below, which compounds are named I.B2-Z207-Q4-U-1 to I.B2-Z207-Q4-U-42.

In another embodiment, compounds I are of formula I.B1 wherein Z is Z-208 and Q is Q1, and R and R⁴ are as per any row of Table U below, which compounds are named I.B1-Z208-Q1-U-1 to I.B1-Z208-Q1-U-42.

In another embodiment, compounds I are of formula I.B1 wherein Z is Z-208 and Q is Q2, and R and R⁴ are as per any row of Table U below, which compounds are named I.B1-Z208-Q2-U-1 to I.B1-Z208-Q2-U-42.

In another embodiment, compounds I are of formula I.B1 wherein Z is Z-208 and Q is Q3, and R and R⁴ are as per any row of Table U below, which compounds are named I.B1-Z208-Q3-U-1 to I.B1-Z208-Q3-U-42.

In another embodiment, compounds I are of formula I.B1 wherein Z is Z-208 and Q is Q4, and R and R⁴ are as per any row of Table U below, which compounds are named I.B1-Z208-Q4-U-1 to I.B1-Z208-Q4-U-42.

In another embodiment, compounds I are of formula I.B2 wherein Z is Z-208 and Q is Q1, and R and R⁴ are as per any row of Table U below, which compounds are named I.B2-Z208-Q1-U-1 to I.B2-Z208-Q1-U-42.

In another embodiment, compounds I are of formula I.B2 wherein Z is Z-208 and Q is Q2, and R and R⁴ are as per any row of Table U below, which compounds are named I.B2-Z208-Q2-U-1 to I.B2-Z208-Q2-U-42.

In another embodiment, compounds I are of formula I.B2 wherein Z is Z-208 and Q is Q3, and R and R⁴ are as per any row of Table U below, which compounds are named I.B2-Z208-Q3-U-1 to I.B2-Z208-Q3-U-42.

In another embodiment, compounds I are of formula I.B2 wherein Z is Z-208 and Q is Q4, and R and R⁴ are as per any row of Table U below, which compounds are named I.B2-Z208-Q4-U-1 to I.B2-Z208-Q4-U-42.

In another embodiment, compounds I are of formula I.B1 wherein Z is Z-209 and Q is Q1, and R and R⁴ are as per any row of Table U below, which compounds are named I.B1-Z209-Q1-U-1 to I.B1-Z209-Q1-U-42.

In another embodiment, compounds I are of formula I.B1 wherein Z is Z-209 and Q is Q2, and R and R⁴ are as per any row of Table U below, which compounds are named I.B1-Z209-Q2-U-1 to I.B1-Z209-Q2-U-42.

In another embodiment, compounds I are of formula I.B1 wherein Z is Z-209 and Q is Q3, and R and R⁴ are as per any row of Table U below, which compounds are named I.B1-Z209-Q3-U-1 to I.B1-Z209-Q3-U-42.

In another embodiment, compounds I are of formula I.B1 wherein Z is Z-209 and Q is Q4, and R and R⁴ are as per any row of Table U below, which compounds are named I.B1-Z209-Q4-U-1 to I.B1-Z209-Q4-U-42.

In another embodiment, compounds I are of formula I.B2 wherein Z is Z-209 and Q is Q1, and R and R⁴ are as per any row of Table U below, which compounds are named I.B2-Z209-Q1-U-1 to I.B2-Z209-Q1-U-42.

In another embodiment, compounds I are of formula I.B2 wherein Z is Z-209 and Q is Q2, and R and R⁴ are as per any row of Table U below, which compounds are named I.B2-Z209-Q2-U-1 to I.B2-Z209-Q2-U-42.

In another embodiment, compounds I are of formula I.B2 wherein Z is Z-209 and Q is Q3, and R and R⁴ are as per any row of Table U below, which compounds are named I.B2-Z209-Q3-U-1 to I.B2-Z209-Q3-U-42.

In another embodiment, compounds I are of formula I.B2 wherein Z is Z-209 and Q is Q4, and R and R⁴ are as per any row of Table U below, which compounds are named I.B2-Z209-Q4-U-1 to I.B2-Z209-Q4-U-42.

In another embodiment, compounds I are of formula I.B1 wherein Z is Z-210 and Q is Q1, and R and R⁴ are as per any row of Table U below, which compounds are named I.B1-Z210-Q1-U-1 to I.B1-Z210-Q1-U-42.

In another embodiment, compounds I are of formula I.B1 wherein Z is Z-210 and Q is Q2, and R and R⁴ are as per any row of Table U below, which compounds are named I.B1-Z210-Q2-U-1 to I.B1-Z210-Q2-U-42.

In another embodiment, compounds I are of formula I.B1 wherein Z is Z-210 and Q is Q3, and R and R⁴ are as per any row of Table U below, which compounds are named I.B1-Z210-Q3-U-1 to I.B1-Z210-Q3-U-42.

In another embodiment, compounds I are of formula I.B1 wherein Z is Z-210 and Q is Q4, and R and R⁴ are as per any row of Table U below, which compounds are named I.B1-Z210-Q4-U-1 to I.B1-Z210-Q4-U-42.

In another embodiment, compounds I are of formula I.B2 wherein Z is Z-210 and Q is Q1, and R and R⁴ are as per any row of Table U below, which compounds are named I.B2-Z210-Q1-U-1 to I.B2-Z210-Q1-U-42.

In another embodiment, compounds I are of formula I.B2 wherein Z is Z-210 and Q is Q2, and R and R⁴ are as per any row of Table U below, which compounds are named I.B2-Z210-Q2-U-1 to I. B2-Z210-Q2-U-42.

In another embodiment, compounds I are of formula I.B2 wherein Z is Z-210 and Q is Q3, and R and R⁴ are as per any row of Table U below, which compounds are named I.B2-Z210-Q3-U-1 to I.B2-Z210-Q3-U-42.

In another embodiment, compounds I are of formula I.B2 wherein Z is Z-210 and Q is Q4, and R and R⁴ are as per any row of Table U below, which compounds are named I.B2-Z210-Q4-U-1 to I.B2-Z210-Q4-U-42.

In another embodiment, compounds I are of formula I.B1 wherein Z is Z-211 and Q is Q1, and R and R⁴ are as per any row of Table U below, which compounds are named I.B1-Z211-Q1-U-1 to I.B1-Z211-Q1-U-42.

In another embodiment, compounds I are of formula I.B1 wherein Z is Z-211 and Q is Q2, and R and R⁴ are as per any row of Table U below, which compounds are named I.B1-Z211-Q2-U-1 to I.B1-Z211-Q2-U-42.

In another embodiment, compounds I are of formula I.B1 wherein Z is Z-211 and Q is Q3, and R and R⁴ are as per any row of Table U below, which compounds are named I.B1-Z211-Q3-U-1 to I.B1-Z211-Q3-U-42.

In another embodiment, compounds I are of formula I.B1 wherein Z is Z-211 and Q is Q4, and R and R⁴ are as per any row of Table U below, which compounds are named I.B1-Z211-Q4-U-1 to I.B1-Z211-Q4-U-42.

In another embodiment, compounds I are of formula I.B2 wherein Z is Z-211 and Q is Q1, and R and R⁴ are as per any row of Table U below, which compounds are named I.B2-Z211-Q1-U-1 to I.B2-Z211-Q1-U-42.

In another embodiment, compounds I are of formula I.B2 wherein Z is Z-211 and Q is Q2, and R and R⁴ are as per any row of Table U below, which compounds are named I.B2-Z211-Q2-U-1 to I.B2-Z211-Q2-U-42.

In another embodiment, compounds I are of formula I.B2 wherein Z is Z-211 and Q is Q3, and R and R⁴ are as per any row of Table U below, which compounds are named I.B2-Z211-Q3-U-1 to I.B2-Z211-Q3-U-42.

In another embodiment, compounds I are of formula I.B2 wherein Z is Z-211 and Q is Q4, and R and R⁴ are as per any row of Table U below, which compounds are named I.B2-Z211-Q4-U-1 to I.B2-Z211-Q4-U-42.

In another embodiment, compounds I are of formula I.B1 wherein Z is Z-212 and Q is Q1, and R and R⁴ are as per any row of Table U below, which compounds are named I.B1-Z212-Q1-U-1 to I.B1-Z212-Q1-U-42.

In another embodiment, compounds I are of formula I.B1 wherein Z is Z-212 and Q is Q2, and R and R⁴ are as per any row of Table U below, which compounds are named I.B1-Z212-Q2-U-1 to I.B1-Z212-Q2-U-42.

In another embodiment, compounds I are of formula I.B1 wherein Z is Z-212 and Q is Q3, and R and R⁴ are as per any row of Table U below, which compounds are named I.B1-Z212-Q3-U-1 to I.B1-Z212-Q3-U-42.

In another embodiment, compounds I are of formula I.B1 wherein Z is Z-212 and Q is Q4, and R and R⁴ are as per any row of Table U below, which compounds are named I.B1-Z212-Q4-U-1 to I.B1-Z212-Q4-U-42.

In another embodiment, compounds I are of formula I.B2 wherein Z is Z-212 and Q is Q1, and R and R⁴ are as per any row of Table U below, which compounds are named I.B2-Z212-Q1-U-1 to I.B2-Z212-Q1-U-42.

In another embodiment, compounds I are of formula I.B2 wherein Z is Z-212 and Q is Q2, and R and R⁴ are as per any row of Table U below, which compounds are named I.B2-Z212-Q2-U-1 to I.B2-Z212-Q2-U-42.

In another embodiment, compounds I are of formula I.B2 wherein Z is Z-212 and Q is Q3, and R and R⁴ are as per any row of Table U below, which compounds are named I.B2-Z212-Q3-U-1 to I.B2-Z212-Q3-U-42.

In another embodiment, compounds I are of formula I.B2 wherein Z is Z-212 and Q is Q4, and R and R⁴ are as per any row of Table U below, which compounds are named I.B2-Z212-Q4-U-1 to I.B2-Z212-Q4-U-42.

In another embodiment, compounds I are of formula I.B1 wherein Z is Z-213 and Q is Q1, and R and R⁴ are as per any row of Table U below, which compounds are named I.B1-Z213-Q1-U-1 to I.B1-Z213-Q1-U-42.

In another embodiment, compounds I are of formula I.B1 wherein Z is Z-213 and Q is Q2, and R and R⁴ are as per any row of Table U below, which compounds are named I.B1-Z213-Q2-U-1 to I.B1-Z213-Q2-U-42.

In another embodiment, compounds I are of formula I.B1 wherein Z is Z-213 and Q is Q3, and R and R⁴ are as per any row of Table U below, which compounds are named I.B1-Z213-Q3-U-1 to I.B1-Z213-Q3-U-42.

In another embodiment, compounds I are of formula I.B1 wherein Z is Z-213 and Q is Q4, and R and R⁴ are as per any row of Table U below, which compounds are named I.B1-Z213-Q4-U-1 to I.B1-Z213-Q4-U-42.

In another embodiment, compounds I are of formula I.B2 wherein Z is Z-213 and Q is Q1, and R and R⁴ are as per any row of Table U below, which compounds are named I.B2-Z213-Q1-U-1 to I.B2-Z213-Q1-U-42.

In another embodiment, compounds I are of formula I.B2 wherein Z is Z-213 and Q is Q2, and R and R⁴ are as per any row of Table U below, which compounds are named I.B2-Z213-Q2-U-1 to I.B2-Z213-Q2-U-42.

In another embodiment, compounds I are of formula I.B2 wherein Z is Z-213 and Q is Q3, and R and R⁴ are as per any row of Table U below, which compounds are named I.B2-Z213-Q3-U-1 to I.B2-Z213-Q3-U-42.

In another embodiment, compounds I are of formula I.B2 wherein Z is Z-213 and Q is Q4, and R and R⁴ are as per any row of Table U below, which compounds are named I.B2-Z213-Q4-U-1 to I.B2-Z213-Q4-U-42.

In another embodiment, compounds I are of formula I.B1 wherein Z is Z-214 and Q is Q1, and R and R⁴ are as per any row of Table U below, which compounds are named I.B1-Z214-Q1-U-1 to I.B1-Z214-Q1-U-42.

In another embodiment, compounds I are of formula I.B1 wherein Z is Z-214 and Q is Q2, and R and R⁴ are as per any row of Table U below, which compounds are named I.B1-Z214-Q2-U-1 to I.B1-Z214-Q2-U-42.

In another embodiment, compounds I are of formula I.B1 wherein Z is Z-214 and Q is Q3, and R and R⁴ are as per any row of Table U below, which compounds are named I.B1-Z214-Q3-U-1 to I.B1-Z214-Q3-U-42.

In another embodiment, compounds I are of formula I.B1 wherein Z is Z-214 and Q is Q4, and R and R⁴ are as per any row of Table U below, which compounds are named I.B1-Z214-Q4-U-1 to I.B1-Z214-Q4-U-42.

In another embodiment, compounds I are of formula I.B2 wherein Z is Z-214 and Q is Q1, and R and R⁴ are as per any row of Table U below, which compounds are named I.B2-Z214-Q1-U-1 to I.B2-Z214-Q1-U-42.

In another embodiment, compounds I are of formula I.B2 wherein Z is Z-214 and Q is Q2, and R and R⁴ are as per any row of Table U below, which compounds are named I.B2-Z214-Q2-U-1 to I.B2-Z214-Q2-U-42.

In another embodiment, compounds I are of formula I.B2 wherein Z is Z-214 and Q is Q3, and R and R⁴ are as per any row of Table U below, which compounds are named I.B2-Z214-Q3-U-1 to I.B2-Z214-Q3-U-42.

In another embodiment, compounds I are of formula I.B2 wherein Z is Z-214 and Q is Q4, and R and R⁴ are as per any row of Table U below, which compounds are named I.B2-Z214-Q4-U-1 to I.B2-Z214-Q4-U-42.

In another embodiment, compounds I are of formula I.B1 wherein Z is Z-215 and Q is Q1, and R and R⁴ are as per any row of Table U below, which compounds are named I.B1-Z215-Q1-U-1 to I.B1-Z215-Q1-U-42.

In another embodiment, compounds I are of formula I.B1 wherein Z is Z-215 and Q is Q2, and R and R⁴ are as per any row of Table U below, which compounds are named I.B1-Z215-Q2-U-1 to I.B1-Z215-Q2-U-42.

In another embodiment, compounds I are of formula I.B1 wherein Z is Z-215 and Q is Q3, and R and R⁴ are as per any row of Table U below, which compounds are named I.B1-Z215-Q3-U-1 to I.B1-Z215-Q3-U-42.

In another embodiment, compounds I are of formula I.B1 wherein Z is Z-215 and Q is Q4, and R and R⁴ are as per any row of Table U below, which compounds are named I.B1-Z215-Q4-U-1 to I.B1-Z215-Q4-U-42.

In another embodiment, compounds I are of formula I.B2 wherein Z is Z-215 and Q is Q1, and R and R⁴ are as per any row of Table U below, which compounds are named I.B2-Z215-Q1-U-1 to I.B2-Z215-Q1-U-42.

In another embodiment, compounds I are of formula I.B2 wherein Z is Z-215 and Q is Q2, and R and R⁴ are as per any row of Table U below, which compounds are named I.B2-Z215-Q2-U-1 to I.B2-Z215-Q2-U-42.

In another embodiment, compounds I are of formula I.B2 wherein Z is Z-215 and Q is Q3, and R and R⁴ are as per any row of Table U below, which compounds are named I.B2-Z215-Q3-U-1 to I.B2-Z215-Q3-U-42.

In another embodiment, compounds I are of formula I.B2 wherein Z is Z-215 and Q is Q4, and R and R⁴ are as per any row of Table U below, which compounds are named I.B2-Z215-Q4-U-1 to I.B2-Z215-Q4-U-42.

In another embodiment, compounds I are of formula I.B1 wherein Z is Z-216 and Q is Q1, and R and R⁴ are as per any row of Table U below, which compounds are named I.B1-Z216-Q1-U-1 to I.B1-Z216-Q1-U-42.

In another embodiment, compounds I are of formula I.B1 wherein Z is Z-216 and Q is Q2, and R and R⁴ are as per any row of Table U below, which compounds are named I.B1-Z216-Q2-U-1 to I.B1-Z216-Q2-U-42.

In another embodiment, compounds I are of formula I.B1 wherein Z is Z-216 and Q is Q3, and R and R⁴ are as per any row of Table U below, which compounds are named I.B1-Z216-Q3-U-1 to I.B1-Z216-Q3-U-42.

In another embodiment, compounds I are of formula I.B1 wherein Z is Z-216 and Q is Q4, and R and R⁴ are as per any row of Table U below, which compounds are named I.B1-Z216-Q4-U-1 to I.B1-Z216-Q4-U-42.

In another embodiment, compounds I are of formula I.B2 wherein Z is Z-216 and Q is Q1, and R and R⁴ are as per any row of Table U below, which compounds are named I.B2-Z216-Q1-U-1 to I.B2-Z216-Q1-U-42.

In another embodiment, compounds I are of formula I.B2 wherein Z is Z-216 and Q is Q2, and R and R⁴ are as per any row of Table U below, which compounds are named I.B2-Z216-Q2-U-1 to I.B2-Z216-Q2-U-42.

In another embodiment, compounds I are of formula I.B2 wherein Z is Z-216 and Q is Q3, and R and R⁴ are as per any row of Table U below, which compounds are named I.B2-Z216-Q3-U-1 to I.B2-Z216-Q3-U-42.

In another embodiment, compounds I are of formula I.B2 wherein Z is Z-216 and Q is Q4, and R and R⁴ are as per any row of Table U below, which compounds are named I.B2-Z216-Q4-U-1 to I.B2-Z216-Q4-U-42.

In another embodiment, compounds I are of formula I.B1 wherein Z is Z-217 and Q is Q1, and R and R⁴ are as per any row of Table U below, which compounds are named I.B1-Z217-Q1-U-1 to I.B1-Z217-Q1-U-42.

In another embodiment, compounds I are of formula I.B1 wherein Z is Z-217 and Q is Q2, and R and R⁴ are as per any row of Table U below, which compounds are named I.B1-Z217-Q2-U-1 to I.B1-Z217-Q2-U-42.

In another embodiment, compounds I are of formula I.B1 wherein Z is Z-217 and Q is Q3, and R and R⁴ are as per any row of Table U below, which compounds are named I.B1-Z217-Q3-U-1 to I.B1-Z217-Q3-U-42.

In another embodiment, compounds I are of formula I.B1 wherein Z is Z-217 and Q is Q4, and R and R⁴ are as per any row of Table U below, which compounds are named I.B1-Z217-Q4-U-1 to I.B1-Z217-Q4-U-42.

In another embodiment, compounds I are of formula I.B2 wherein Z is Z-217 and Q is Q1, and R and R⁴ are as per any row of Table U below, which compounds are named I.B2-Z217-Q1-U-1 to I.B2-Z217-Q1-U-42.

In another embodiment, compounds I are of formula I.B2 wherein Z is Z-217 and Q is Q2, and R and R⁴ are as per any row of Table U below, which compounds are named I.B2-Z217-Q2-U-1 to I.B2-Z217-Q2-U-42.

In another embodiment, compounds I are of formula I.B2 wherein Z is Z-217 and Q is Q3, and R and R⁴ are as per any row of Table U below, which compounds are named I.B2-Z217-Q3-U-1 to I.B2-Z217-Q3-U-42.

In another embodiment, compounds I are of formula I.B2 wherein Z is Z-217 and Q is Q4, and R and R⁴ are as per any row of Table U below, which compounds are named I.B2-Z217-Q4-U-1 to I.B2-Z217-Q4-U-42.

In another embodiment, compounds I are of formula I.B1 wherein Z is Z-218 and Q is Q1, and R and R⁴ are as per any row of Table U below, which compounds are named I.B1-Z218-Q1-U-1 to I.B1-Z218-Q1-U-42.

In another embodiment, compounds I are of formula I.B1 wherein Z is Z-218 and Q is Q2, and R and R⁴ are as per any row of Table U below, which compounds are named I.B1-Z218-Q2-U-1 to I.B1-Z218-Q2-U-42.

In another embodiment, compounds I are of formula I.B1 wherein Z is Z-218 and Q is Q3, and R and R⁴ are as per any row of Table U below, which compounds are named I.B1-Z218-Q3-U-1 to I.B1-Z218-Q3-U-42.

In another embodiment, compounds I are of formula I.B1 wherein Z is Z-218 and Q is Q4, and R and R⁴ are as per any row of Table U below, which compounds are named I.B1-Z218-Q4-U-1 to I.B1-Z218-Q4-U-42.

In another embodiment, compounds I are of formula I.B2 wherein Z is Z-218 and Q is Q1, and R and R⁴ are as per any row of Table U below, which compounds are named I.B2-Z218-Q1-U-1 to I.B2-Z218-Q1-U-42.

In another embodiment, compounds I are of formula I.B2 wherein Z is Z-218 and Q is Q2, and R and R⁴ are as per any row of Table U below, which compounds are named I.B2-Z218-Q2-U-1 to I.B2-Z218-Q2-U-42.

In another embodiment, compounds I are of formula I.B2 wherein Z is Z-218 and Q is Q3, and R and R⁴ are as per any row of Table U below, which compounds are named I.B2-Z218-Q3-U-1 to I.B2-Z218-Q3-U-42.

In another embodiment, compounds I are of formula I.B2 wherein Z is Z-218 and Q is Q4, and R and R⁴ are as per any row of Table U below, which compounds are named I.B2-Z218-Q4-U-1 to I.B2-Z218-Q4-U-42.

In another embodiment, compounds I are of formula I.B1 wherein Z is Z-219 and Q is Q1, and R and R⁴ are as per any row of Table U below, which compounds are named I.B1-Z219-Q1-U-1 to I.B1-Z219-Q1-U-42.

In another embodiment, compounds I are of formula I.B1 wherein Z is Z-219 and Q is Q2, and R and R⁴ are as per any row of Table U below, which compounds are named I.B1-Z219-Q2-U-1 to I.B1-Z219-Q2-U-42.

In another embodiment, compounds I are of formula I.B1 wherein Z is Z-219 and Q is Q3, and R and R⁴ are as per any row of Table U below, which compounds are named I.B1-Z219-Q3-U-1 to I.B1-Z219-Q3-U-42.

In another embodiment, compounds I are of formula I.B1 wherein Z is Z-219 and Q is Q4, and R and R⁴ are as per any row of Table U below, which compounds are named I.B1-Z219-Q4-U-1 to I.B1-Z219-Q4-U-42.

In another embodiment, compounds I are of formula I.B2 wherein Z is Z-219 and Q is Q1, and R and R⁴ are as per any row of Table U below, which compounds are named I.B2-Z219-Q1-U-1 to I.B2-Z219-Q1-U-42.

In another embodiment, compounds I are of formula I.B2 wherein Z is Z-219 and Q is Q2, and R and R⁴ are as per any row of Table U below, which compounds are named I.B2-Z219-Q2-U-1 to I.B2-Z219-Q2-U-42.

In another embodiment, compounds I are of formula I.B2 wherein Z is Z-219 and Q is Q3, and R and R⁴ are as per any row of Table U below, which compounds are named I.B2-Z219-Q3-U-1 to I.B2-Z219-Q3-U-42.

In another embodiment, compounds I are of formula I.B2 wherein Z is Z-219 and Q is Q4, and R and R⁴ are as per any row of Table U below, which compounds are named I.B2-Z219-Q4-U-1 to I.B2-Z219-Q4-U-42.

In another embodiment, compounds I are of formula I.B1 wherein Z is Z-220 and Q is Q1, and R and R⁴ are as per any row of Table U below, which compounds are named I.B1-Z220-Q1-U-1 to I.B1-Z220-Q1-U-42.

In another embodiment, compounds I are of formula I.B1 wherein Z is Z-220 and Q is Q2, and R and R⁴ are as per any row of Table U below, which compounds are named I.B1-Z220-Q2-U-1 to I.B1-Z220-Q2-U-42.

In another embodiment, compounds I are of formula I.B1 wherein Z is Z-220 and Q is Q3, and R and R⁴ are as per any row of Table U below, which compounds are named I.B1-Z220-Q3-U-1 to I.B1-Z220-Q3-U-42.

In another embodiment, compounds I are of formula I.B1 wherein Z is Z-220 and Q is Q4, and R and R⁴ are as per any row of Table U below, which compounds are named I.B1-Z220-Q4-U-1 to I.B1-Z220-Q4-U-42.

In another embodiment, compounds I are of formula I.B2 wherein Z is Z-220 and Q is Q1, and R and R⁴ are as per any row of Table U below, which compounds are named I.B2-Z220-Q1-U-1 to I.B2-Z220-Q1-U-42.

In another embodiment, compounds I are of formula I.B2 wherein Z is Z-220 and Q is Q2, and R and R⁴ are as per any row of Table U below, which compounds are named I.B2-Z220-Q2-U-1 to I.B2-Z220-Q2-U-42.

In another embodiment, compounds I are of formula I.B2 wherein Z is Z-220 and Q is Q3, and R and R⁴ are as per any row of Table U below, which compounds are named I.B2-Z220-Q3-U-1 to I.B2-Z220-Q3-U-42.

In another embodiment, compounds I are of formula I.B2 wherein Z is Z-220 and Q is Q4, and R and R⁴ are as per any row of Table U below, which compounds are named I.B2-Z220-Q4-U-1 to I.B2-Z220-Q4-U-42.

In another embodiment, compounds I are of formula I.B1 wherein Z is Z-221 and Q is Q1, and R and R⁴ are as per any row of Table U below, which compounds are named I.B1-Z221-Q1-U-1 to I.B1-Z221-Q1-U-42.

In another embodiment, compounds I are of formula I.B1 wherein Z is Z-221 and Q is Q2, and R and R⁴ are as per any row of Table U below, which compounds are named I.B1-Z221-Q2-U-1 to I.B1-Z221-Q2-U-42.

In another embodiment, compounds I are of formula I.B1 wherein Z is Z-221 and Q is Q3, and R and R⁴ are as per any row of Table U below, which compounds are named I.B1-Z221-Q3-U-1 to I.B1-Z221-Q3-U-42.

In another embodiment, compounds I are of formula I.B1 wherein Z is Z-221 and Q is Q4, and R and R⁴ are as per any row of Table U below, which compounds are named I.B1-Z221-Q4-U-1 to I.B1-Z221-Q4-U-42.

In another embodiment, compounds I are of formula I.B2 wherein Z is Z-221 and Q is Q1, and R and R⁴ are as per any row of Table U below, which compounds are named I.B2-Z221-Q1-U-1 to I.B2-Z221-Q1-U-42.

In another embodiment, compounds I are of formula I.B2 wherein Z is Z-221 and Q is Q2, and R and R⁴ are as per any row of Table U below, which compounds are named I.B2-Z221-Q2-U-1 to I.B2-Z221-Q2-U-42.

In another embodiment, compounds I are of formula I.B2 wherein Z is Z-221 and Q is Q3, and R and R⁴ are as per any row of Table U below, which compounds are named I.B2-Z221-Q3-U-1 to I.B2-Z221-Q3-U-42.

In another embodiment, compounds I are of formula I.B2 wherein Z is Z-221 and Q is Q4, and R and R⁴ are as per any row of Table U below, which compounds are named I.B2-Z221-Q4-U-1 to I.B2-Z221-Q4-U-42.

In another embodiment, compounds I are of formula I.B1 wherein Z is Z-222 and Q is Q1, and R and R⁴ are as per any row of Table U below, which compounds are named I.B1-Z222-Q1-U-1 to I.B1-Z222-Q1-U-42.

In another embodiment, compounds I are of formula I.B1 wherein Z is Z-222 and Q is Q2, and R and R⁴ are as per any row of Table U below, which compounds are named I.B1-Z222-Q2-U-1 to I.B1-Z222-Q2-U-42.

In another embodiment, compounds I are of formula I.B1 wherein Z is Z-222 and Q is Q3, and R and R⁴ are as per any row of Table U below, which compounds are named I.B1-Z222-Q3-U-1 to I.B1-Z222-Q3-U-42.

In another embodiment, compounds I are of formula I.B1 wherein Z is Z-222 and Q is Q4, and R and R⁴ are as per any row of Table U below, which compounds are named I.B1-Z222-Q4-U-1 to I.B1-Z222-Q4-U-42.

In another embodiment, compounds I are of formula I.B2 wherein Z is Z-222 and Q is Q1, and R and R⁴ are as per any row of Table U below, which compounds are named I.B2-Z222-Q1-U-1 to I.B2-Z222-Q1-U-42.

In another embodiment, compounds I are of formula I.B2 wherein Z is Z-222 and Q is Q2, and R and R⁴ are as per any row of Table U below, which compounds are named I.B2-Z222-Q2-U-1 to I.B2-Z222-Q2-U-42.

In another embodiment, compounds I are of formula I.B2 wherein Z is Z-222 and Q is Q3, and R and R⁴ are as per any row of Table U below, which compounds are named I.B2-Z222-Q3-U-1 to I.B2-Z222-Q3-U-42.

In another embodiment, compounds I are of formula I.B2 wherein Z is Z-222 and Q is Q4, and R and R⁴ are as per any row of Table U below, which compounds are named I.B2-Z222-Q4-U-1 to I.B2-Z222-Q4-U-42.

In another embodiment, compounds I are of formula I.B1 wherein Z is Z-223 and Q is Q1, and R and R⁴ are as per any row of Table U below, which compounds are named I.B1-Z223-Q1-U-1 to I.B1-Z223-Q1-U-42.

In another embodiment, compounds I are of formula I.B1 wherein Z is Z-223 and Q is Q2, and R and R⁴ are as per any row of Table U below, which compounds are named I.B1-Z223-Q2-U-1 to I.B1-Z223-Q2-U-42.

In another embodiment, compounds I are of formula I.B1 wherein Z is Z-223 and Q is Q3, and R and R⁴ are as per any row of Table U below, which compounds are named I.B1-Z223-Q3-U-1 to I.B1-Z223-Q3-U-42.

In another embodiment, compounds I are of formula I.B1 wherein Z is Z-223 and Q is Q4, and R and R⁴ are as per any row of Table U below, which compounds are named I.B1-Z223-Q4-U-1 to I.B1-Z223-Q4-U-42.

In another embodiment, compounds I are of formula I.B2 wherein Z is Z-223 and Q is Q1, and R and R⁴ are as per any row of Table U below, which compounds are named I.B2-Z223-Q1-U-1 to I.B2-Z223-Q1-U-42.

In another embodiment, compounds I are of formula I.B2 wherein Z is Z-223 and Q is Q2, and R and R⁴ are as per any row of Table U below, which compounds are named I.B2-Z223-Q2-U-1 to I.B2-Z223-Q2-U-42.

In another embodiment, compounds I are of formula I.B2 wherein Z is Z-223 and Q is Q3, and R and R⁴ are as per any row of Table U below, which compounds are named I.B2-Z223-Q3-U-1 to I.B2-Z223-Q3-U-42.

In another embodiment, compounds I are of formula I.B2 wherein Z is Z-223 and Q is Q4, and R and R⁴ are as per any row of Table U below, which compounds are named I.B2-Z223-Q4-U-1 to I.B2-Z223-Q4-U-42.

In another embodiment, compounds I are of formula I.B1 wherein Z is Z-224 and Q is Q1, and R and R⁴ are as per any row of Table U below, which compounds are named I.B1-Z224-Q1-U-1 to I.B1-Z224-Q1-U-42.

In another embodiment, compounds I are of formula I.B1 wherein Z is Z-224 and Q is Q2, and R and R⁴ are as per any row of Table U below, which compounds are named I.B1-Z224-Q2-U-1 to I.B1-Z224-Q2-U-42.

In another embodiment, compounds I are of formula I.B1 wherein Z is Z-224 and Q is Q3, and R and R⁴ are as per any row of Table U below, which compounds are named I.B1-Z224-Q3-U-1 to I.B1-Z224-Q3-U-42.

In another embodiment, compounds I are of formula I.B1 wherein Z is Z-224 and Q is Q4, and R and R⁴ are as per any row of Table U below, which compounds are named I.B1-Z224-Q4-U-1 to I.B1-Z224-Q4-U-42.

In another embodiment, compounds I are of formula I.B2 wherein Z is Z-224 and Q is Q1, and R and R⁴ are as per any row of Table U below, which compounds are named I.B2-Z224-Q1-U-1 to I.B2-Z224-Q1-U-42.

In another embodiment, compounds I are of formula I.B2 wherein Z is Z-224 and Q is Q2, and R and R⁴ are as per any row of Table U below, which compounds are named I.B2-Z224-Q2-U-1 to I.B2-Z224-Q2-U-42.

In another embodiment, compounds I are of formula I.B2 wherein Z is Z-224 and Q is Q3, and R and R⁴ are as per any row of Table U below, which compounds are named I.B2-Z224-Q3-U-1 to I.B2-Z224-Q3-U-42.

In another embodiment, compounds I are of formula I.B2 wherein Z is Z-224 and Q is Q4, and R and R⁴ are as per any row of Table U below, which compounds are named I.B2-Z224-Q4-U-1 to I.B2-Z224-Q4-U-42.

In another embodiment, compounds I are of formula I.B1 wherein Z is Z-225 and Q is Q1, and R and R⁴ are as per any row of Table U below, which compounds are named I.B1-Z225-Q1-U-1 to I.B1-Z225-Q1-U-42.

In another embodiment, compounds I are of formula I.B1 wherein Z is Z-225 and Q is Q2, and R and R⁴ are as per any row of Table U below, which compounds are named I.B1-Z225-Q2-U-1 to I.B1-Z225-Q2-U-42.

In another embodiment, compounds I are of formula I.B1 wherein Z is Z-225 and Q is Q3, and R and R⁴ are as per any row of Table U below, which compounds are named I.B1-Z225-Q3-U-1 to I.B1-Z225-Q3-U-42.

In another embodiment, compounds I are of formula I.B1 wherein Z is Z-225 and Q is Q4, and R and R⁴ are as per any row of Table U below, which compounds are named I.B1-Z225-Q4-U-1 to I.B1-Z225-Q4-U-42.

In another embodiment, compounds I are of formula I.B2 wherein Z is Z-225 and Q is Q1, and R and R⁴ are as per any row of Table U below, which compounds are named I.B2-Z225-Q1-U-1 to I.B2-Z225-Q1-U-42.

In another embodiment, compounds I are of formula I.B2 wherein Z is Z-225 and Q is Q2, and R and R⁴ are as per any row of Table U below, which compounds are named I.B2-Z225-Q2-U-1 to I.B2-Z225-Q2-U-42.

In another embodiment, compounds I are of formula I.B2 wherein Z is Z-225 and Q is Q3, and R and R⁴ are as per any row of Table U below, which compounds are named I.B2-Z225-Q3-U-1 to I.B2-Z225-Q3-U-42.

In another embodiment, compounds I are of formula I.B2 wherein Z is Z-225 and Q is Q4, and R and R⁴ are as per any row of Table U below, which compounds are named I.B2-Z225-Q4-U-1 to I.B2-Z225-Q4-U-42.

In another embodiment, compounds I are of formula I.B1 wherein Z is Z-226 and Q is Q1, and R and R⁴ are as per any row of Table U below, which compounds are named I.B1-Z226-Q1-U-1 to I.B1-Z226-Q1-U-42.

In another embodiment, compounds I are of formula I.B1 wherein Z is Z-226 and Q is Q2, and R and R⁴ are as per any row of Table U below, which compounds are named I.B1-Z226-Q2-U-1 to I.B1-Z226-Q2-U-42.

In another embodiment, compounds I are of formula I.B1 wherein Z is Z-226 and Q is Q3, and R and R⁴ are as per any row of Table U below, which compounds are named I.B1-Z226-Q3-U-1 to I.B1-Z226-Q3-U-42.

In another embodiment, compounds I are of formula I.B1 wherein Z is Z-226 and Q is Q4, and R and R⁴ are as per any row of Table U below, which compounds are named I.B1-Z226-Q4-U-1 to I.B1-Z226-Q4-U-42.

In another embodiment, compounds I are of formula I.B2 wherein Z is Z-226 and Q is Q1, and R and R⁴ are as per any row of Table U below, which compounds are named I.B2-Z226-Q1-U-1 to I.B2-Z226-Q1-U-42.

In another embodiment, compounds I are of formula I.B2 wherein Z is Z-226 and Q is Q2, and R and R⁴ are as per any row of Table U below, which compounds are named I.B2-Z226-Q2-U-1 to I.B2-Z226-Q2-U-42.

In another embodiment, compounds I are of formula I.B2 wherein Z is Z-226 and Q is Q3, and R and R⁴ are as per any row of Table U below, which compounds are named I.B2-Z226-Q3-U-1 to I.B2-Z226-Q3-U-42.

In another embodiment, compounds I are of formula I.B2 wherein Z is Z-226 and Q is Q4, and R and R⁴ are as per any row of Table U below, which compounds are named I.B2-Z226-Q4-U-1 to I.B2-Z226-Q4-U-42.

In another embodiment, compounds I are of formula I.B1 wherein Z is Z-227 and Q is Q1, and R and R⁴ are as per any row of Table U below, which compounds are named I.B1-Z227-Q1-U-1 to I.B1-Z227-Q1-U-42.

In another embodiment, compounds I are of formula I.B1 wherein Z is Z-227 and Q is Q2, and R and R⁴ are as per any row of Table U below, which compounds are named I.B1-Z227-Q2-U-1 to I.B1-Z227-Q2-U-42.

In another embodiment, compounds I are of formula I.B1 wherein Z is Z-227 and Q is Q3, and R and R⁴ are as per any row of Table U below, which compounds are named I.B1-Z227-Q3-U-1 to I.B1-Z227-Q3-U-42.

In another embodiment, compounds I are of formula I.B1 wherein Z is Z-227 and Q is Q4, and R and R⁴ are as per any row of Table U below, which compounds are named I.B1-Z227-Q4-U-1 to I.B1-Z227-Q4-U-42.

In another embodiment, compounds I are of formula I.B2 wherein Z is Z-227 and Q is Q1, and R and R⁴ are as per any row of Table U below, which compounds are named I.B2-Z227-Q1-U-1 to I.B2-Z227-Q1-U-42.

In another embodiment, compounds I are of formula I.B2 wherein Z is Z-227 and Q is Q2, and R and R⁴ are as per any row of Table U below, which compounds are named I.B2-Z227-Q2-U-1 to I.B2-Z227-Q2-U-42.

In another embodiment, compounds I are of formula I.B2 wherein Z is Z-227 and Q is Q3, and R and R⁴ are as per any row of Table U below, which compounds are named I.B2-Z227-Q3-U-1 to I.B2-Z227-Q3-U-42.

In another embodiment, compounds I are of formula I.B2 wherein Z is Z-227 and Q is Q4, and R and R⁴ are as per any row of Table U below, which compounds are named I.B2-Z227-Q4-U-1 to I.B2-Z227-Q4-U-42.

In another embodiment, compounds I are of formula I.B1 wherein Z is Z-228 and Q is Q1, and R and R⁴ are as per any row of Table U below, which compounds are named I.B1-Z228-Q1-U-1 to I.B1-Z228-Q1-U-42.

In another embodiment, compounds I are of formula I.B1 wherein Z is Z-228 and Q is Q2, and R and R⁴ are as per any row of Table U below, which compounds are named I.B1-Z228-Q2-U-1 to I.B1-Z228-Q2-U-42.

In another embodiment, compounds I are of formula I.B1 wherein Z is Z-228 and Q is Q3, and R and R⁴ are as per any row of Table U below, which compounds are named I.B1-Z228-Q3-U-1 to I.B1-Z228-Q3-U-42.

In another embodiment, compounds I are of formula I.B1 wherein Z is Z-228 and Q is Q4, and R and R⁴ are as per any row of Table U below, which compounds are named I.B1-Z228-Q4-U-1 to I.B1-Z228-Q4-U-42.

In another embodiment, compounds I are of formula I.B2 wherein Z is Z-228 and Q is Q1, and R and R⁴ are as per any row of Table U below, which compounds are named I.B2-Z228-Q1-U-1 to I.B2-Z228-Q1-U-42.

In another embodiment, compounds I are of formula I.B2 wherein Z is Z-228 and Q is Q2, and R and R⁴ are as per any row of Table U below, which compounds are named I.B2-Z228-Q2-U-1 to I.B2-Z228-Q2-U-42.

In another embodiment, compounds I are of formula I.B2 wherein Z is Z-228 and Q is Q3, and R and R⁴ are as per any row of Table U below, which compounds are named I.B2-Z228-Q3-U-1 to I.B2-Z228-Q3-U-42.

In another embodiment, compounds I are of formula I.B2 wherein Z is Z-228 and Q is Q4, and R and R⁴ are as per any row of Table U below, which compounds are named I.B2-Z228-Q4-U-1 to I.B2-Z228-Q4-U-42.

In another embodiment, compounds I are of formula I.B1 wherein Z is Z-229 and Q is Q1, and R and R⁴ are as per any row of Table U below, which compounds are named I.B1-Z229-Q1-U-1 to I.B1-Z229-Q1-U-42.

In another embodiment, compounds I are of formula I.B1 wherein Z is Z-229 and Q is Q2, and R and R⁴ are as per any row of Table U below, which compounds are named I.B1-Z229-Q2-U-1 to I.B1-Z229-Q2-U-42.

In another embodiment, compounds I are of formula I.B1 wherein Z is Z-229 and Q is Q3, and R and R⁴ are as per any row of Table U below, which compounds are named I.B1-Z229-Q3-U-1 to I.B1-Z229-Q3-U-42.

In another embodiment, compounds I are of formula I.B1 wherein Z is Z-229 and Q is Q4, and R and R⁴ are as per any row of Table U below, which compounds are named I.B1-Z229-Q4-U-1 to I.B1-Z229-Q4-U-42.

In another embodiment, compounds I are of formula I.B2 wherein Z is Z-229 and Q is Q1, and R and R⁴ are as per any row of Table U below, which compounds are named I.B2-Z229-Q1-U-1 to I.B2-Z229-Q1-U-42.

In another embodiment, compounds I are of formula I.B2 wherein Z is Z-229 and Q is Q2, and R and R⁴ are as per any row of Table U below, which compounds are named I.B2-Z229-Q2-U-1 to I.B2-Z229-Q2-U-42.

In another embodiment, compounds I are of formula I.B2 wherein Z is Z-229 and Q is Q3, and R and R⁴ are as per any row of Table U below, which compounds are named I.B2-Z229-Q3-U-1 to I.B2-Z229-Q3-U-42.

In another embodiment, compounds I are of formula I.B2 wherein Z is Z-229 and Q is Q4, and R and R⁴ are as per any row of Table U below, which compounds are named I.B2-Z229-Q4-U-1 to I.B2-Z229-Q4-U-42.

In another embodiment, compounds I are of formula I.B1 wherein Z is Z-230 and Q is Q1, and R and R⁴ are as per any row of Table U below, which compounds are named I.B1-Z230-Q1-U-1 to I.B1-Z230-Q1-U-42.

In another embodiment, compounds I are of formula I.B1 wherein Z is Z-230 and Q is Q2, and R and R⁴ are as per any row of Table U below, which compounds are named I.B1-Z230-Q2-U-1 to I.B1-Z230-Q2-U-42.

In another embodiment, compounds I are of formula I.B1 wherein Z is Z-230 and Q is Q3, and R and R⁴ are as per any row of Table U below, which compounds are named I.B1-Z230-Q3-U-1 to I.B1-Z230-Q3-U-42.

In another embodiment, compounds I are of formula I.B1 wherein Z is Z-230 and Q is Q4, and R and R⁴ are as per any row of Table U below, which compounds are named I.B1-Z230-Q4-U-1 to I.B1-Z230-Q4-U-42.

In another embodiment, compounds I are of formula I.B2 wherein Z is Z-230 and Q is Q1, and R and R⁴ are as per any row of Table U below, which compounds are named I.B2-Z230-Q1-U-1 to I.B2-Z230-Q1-U-42.

In another embodiment, compounds I are of formula I.B2 wherein Z is Z-230 and Q is Q2, and R and R⁴ are as per any row of Table U below, which compounds are named I.B2-Z230-Q2-U-1 to I.B2-Z230-Q2-U-42.

In another embodiment, compounds I are of formula I.B2 wherein Z is Z-230 and Q is Q3, and R and R⁴ are as per any row of Table U below, which compounds are named I.B2-Z230-Q3-U-1 to I.B2-Z230-Q3-U-42.

In another embodiment, compounds I are of formula I.B2 wherein Z is Z-230 and Q is Q4, and R and R⁴ are as per any row of Table U below, which compounds are named I.B2-Z230-Q4-U-1 to I.B2-Z230-Q4-U-42.

In another embodiment, compounds I are of formula I.B1 wherein Z is Z-231 and Q is Q1, and R and R⁴ are as per any row of Table U below, which compounds are named I.B1-Z231-Q1-U-1 to I.B1-Z231-Q1-U-42.

In another embodiment, compounds I are of formula I.B1 wherein Z is Z-231 and Q is Q2, and R and R⁴ are as per any row of Table U below, which compounds are named I.B1-Z231-Q2-U-1 to I.B1-Z231-Q2-U-42.

In another embodiment, compounds I are of formula I.B1 wherein Z is Z-231 and Q is Q3, and R and R⁴ are as per any row of Table U below, which compounds are named I.B1-Z231-Q3-U-1 to I.B1-Z231-Q3-U-42.

In another embodiment, compounds I are of formula I.B1 wherein Z is Z-231 and Q is Q4, and R and R⁴ are as per any row of Table U below, which compounds are named I.B1-Z231-Q4-U-1 to I.B1-Z231-Q4-U-42.

In another embodiment, compounds I are of formula I.B2 wherein Z is Z-231 and Q is Q1, and R and R⁴ are as per any row of Table U below, which compounds are named I.B2-Z231-Q1-U-1 to I.B2-Z231-Q1-U-42.

In another embodiment, compounds I are of formula I.B2 wherein Z is Z-231 and Q is Q2, and R and R⁴ are as per any row of Table U below, which compounds are named I.B2-Z231-Q2-U-1 to I.B2-Z231-Q2-U-42.

In another embodiment, compounds I are of formula I.B2 wherein Z is Z-231 and Q is Q3, and R and R⁴ are as per any row of Table U below, which compounds are named I.B2-Z231-Q3-U-1 to I.B2-Z231-Q3-U-42.

In another embodiment, compounds I are of formula I.B2 wherein Z is Z-231 and Q is Q4, and R and R⁴ are as per any row of Table U below, which compounds are named I.B2-Z231-Q4-U-1 to I.B2-Z231-Q4-U-42.

In another embodiment, compounds I are of formula I.B1 wherein Z is Z-232 and Q is Q1, and R and R⁴ are as per any row of Table U below, which compounds are named I.B1-Z232-Q1-U-1 to I.B1-Z232-Q1-U-42.

In another embodiment, compounds I are of formula I.B1 wherein Z is Z-232 and Q is Q2, and R and R⁴ are as per any row of Table U below, which compounds are named I.B1-Z232-Q2-U-1 to I.B1-Z232-Q2-U-42.

In another embodiment, compounds I are of formula I.B1 wherein Z is Z-232 and Q is Q3, and R and R⁴ are as per any row of Table U below, which compounds are named I.B1-Z232-Q3-U-1 to I.B1-Z232-Q3-U-42.

In another embodiment, compounds I are of formula I.B1 wherein Z is Z-232 and Q is Q4, and R and R⁴ are as per any row of Table U below, which compounds are named I.B1-Z232-Q4-U-1 to I.B1-Z232-Q4-U-42.

In another embodiment, compounds I are of formula I.B2 wherein Z is Z-232 and Q is Q1, and R and R⁴ are as per any row of Table U below, which compounds are named I.B2-Z232-Q1-U-1 to I.B2-Z232-Q1-U-42.

In another embodiment, compounds I are of formula I.B2 wherein Z is Z-232 and Q is Q2, and R and R⁴ are as per any row of Table U below, which compounds are named I.B2-Z232-Q2-U-1 to I.B2-Z232-Q2-U-42.

In another embodiment, compounds I are of formula I.B2 wherein Z is Z-232 and Q is Q3, and R and R⁴ are as per any row of Table U below, which compounds are named I.B2-Z232-Q3-U-1 to I.B2-Z232-Q3-U-42.

In another embodiment, compounds I are of formula I.B2 wherein Z is Z-232 and Q is Q4, and R and R⁴ are as per any row of Table U below, which compounds are named I.B2-Z232-Q4-U-1 to I.B2-Z232-Q4-U-42.

In another embodiment, compounds I are of formula I.B1 wherein Z is Z-233 and Q is Q1, and R and R⁴ are as per any row of Table U below, which compounds are named I.B1-Z233-Q1-U-1 to I.B1-Z233-Q1-U-42.

In another embodiment, compounds I are of formula I.B1 wherein Z is Z-233 and Q is Q2, and R and R⁴ are as per any row of Table U below, which compounds are named I.B1-Z233-Q2-U-1 to I.B1-Z233-Q2-U-42.

In another embodiment, compounds I are of formula I.B1 wherein Z is Z-233 and Q is Q3, and R and R⁴ are as per any row of Table U below, which compounds are named I.B1-Z233-Q3-U-1 to I.B1-Z233-Q3-U-42.

In another embodiment, compounds I are of formula I.B1 wherein Z is Z-233 and Q is Q4, and R and R⁴ are as per any row of Table U below, which compounds are named I.B1-Z233-Q4-U-1 to I.B1-Z233-Q4-U-42.

In another embodiment, compounds I are of formula I.B2 wherein Z is Z-233 and Q is Q1, and R and R⁴ are as per any row of Table U below, which compounds are named I.B2-Z233-Q1-U-1 to I.B2-Z233-Q1-U-42.

In another embodiment, compounds I are of formula I.B2 wherein Z is Z-233 and Q is Q2, and R and R⁴ are as per any row of Table U below, which compounds are named I.B2-Z233-Q2-U-1 to I.B2-Z233-Q2-U-42.

In another embodiment, compounds I are of formula I.B2 wherein Z is Z-233 and Q is Q3, and R and R⁴ are as per any row of Table U below, which compounds are named I.B2-Z233-Q3-U-1 to I.B2-Z233-Q3-U-42.

In another embodiment, compounds I are of formula I.B2 wherein Z is Z-233 and Q is Q4, and R and R⁴ are as per any row of Table U below, which compounds are named I.B2-Z233-Q4-U-1 to I.B2-Z233-Q4-U-42.

In another embodiment, compounds I are of formula I.B1 wherein Z is Z-234 and Q is Q1, and R and R⁴ are as per any row of Table U below, which compounds are named I.B1-Z234-Q1-U-1 to I.B1-Z234-Q1-U-42.

In another embodiment, compounds I are of formula I.B1 wherein Z is Z-234 and Q is Q2, and R and R⁴ are as per any row of Table U below, which compounds are named I.B1-Z234-Q2-U-1 to I.B1-Z234-Q2-U-42.

In another embodiment, compounds I are of formula I.B1 wherein Z is Z-234 and Q is Q3, and R and R⁴ are as per any row of Table U below, which compounds are named I.B1-Z234-Q3-U-1 to I.B1-Z234-Q3-U-42.

In another embodiment, compounds I are of formula I.B1 wherein Z is Z-234 and Q is Q4, and R and R⁴ are as per any row of Table U below, which compounds are named I.B1-Z234-Q4-U-1 to I.B1-Z234-Q4-U-42.

In another embodiment, compounds I are of formula I.B2 wherein Z is Z-234 and Q is Q1, and R and R⁴ are as per any row of Table U below, which compounds are named I.B2-Z234-Q1-U-1 to I.B2-Z234-Q1-U-42.

In another embodiment, compounds I are of formula I.B2 wherein Z is Z-234 and Q is Q2, and R and R⁴ are as per any row of Table U below, which compounds are named I.B2-Z234-Q2-U-1 to I.B2-Z234-Q2-U-42.

In another embodiment, compounds I are of formula I.B2 wherein Z is Z-234 and Q is Q3, and R and R⁴ are as per any row of Table U below, which compounds are named I.B2-Z234-Q3-U-1 to I.B2-Z234-Q3-U-42.

In another embodiment, compounds I are of formula I.B2 wherein Z is Z-234 and Q is Q4, and R and R⁴ are as per any row of Table U below, which compounds are named I.B2-Z234-Q4-U-1 to I.B2-Z234-Q4-U-42.

In another embodiment, compounds I are of formula I.B1 wherein Z is Z-235 and Q is Q1, and R and R⁴ are as per any row of Table U below, which compounds are named I.B1-Z235-Q1-U-1 to I.B1-Z235-Q1-U-42.

In another embodiment, compounds I are of formula I.B1 wherein Z is Z-235 and Q is Q2, and R and R⁴ are as per any row of Table U below, which compounds are named I.B1-Z235-Q2-U-1 to I.B1-Z235-Q2-U-42.

In another embodiment, compounds I are of formula I.B1 wherein Z is Z-235 and Q is Q3, and R and R⁴ are as per any row of Table U below, which compounds are named I.B1-Z235-Q3-U-1 to I.B1-Z235-Q3-U-42.

In another embodiment, compounds I are of formula I.B1 wherein Z is Z-235 and Q is Q4, and R and R⁴ are as per any row of Table U below, which compounds are named I.B1-Z235-Q4-U-1 to I.B1-Z235-Q4-U-42.

In another embodiment, compounds I are of formula I.B2 wherein Z is Z-235 and Q is Q1, and R and R⁴ are as per any row of Table U below, which compounds are named I.B2-Z235-Q1-U-1 to I.B2-Z235-Q1-U-42.

In another embodiment, compounds I are of formula I.B2 wherein Z is Z-235 and Q is Q2, and R and R⁴ are as per any row of Table U below, which compounds are named I.B2-Z235-Q2-U-1 to I.B2-Z235-Q2-U-42.

In another embodiment, compounds I are of formula I.B2 wherein Z is Z-235 and Q is Q3, and R and R⁴ are as per any row of Table U below, which compounds are named I.B2-Z235-Q3-U-1 to I.B2-Z235-Q3-U-42.

In another embodiment, compounds I are of formula I.B2 wherein Z is Z-235 and Q is Q4, and R and R⁴ are as per any row of Table U below, which compounds are named I.B2-Z235-Q4-U-1 to I.B2-Z235-Q4-U-42.

In another embodiment, compounds I are of formula I.B1 wherein Z is Z-236 and Q is Q1, and R and R⁴ are as per any row of Table U below, which compounds are named I.B1-Z236-Q1-U-1 to I.B1-Z236-Q1-U-42.

In another embodiment, compounds I are of formula I.B1 wherein Z is Z-236 and Q is Q2, and R and R⁴ are as per any row of Table U below, which compounds are named I.B1-Z236-Q2-U-1 to I.B1-Z236-Q2-U-42.

In another embodiment, compounds I are of formula I.B1 wherein Z is Z-236 and Q is Q3, and R and R⁴ are as per any row of Table U below, which compounds are named I.B1-Z236-Q3-U-1 to I.B1-Z236-Q3-U-42.

In another embodiment, compounds I are of formula I.B1 wherein Z is Z-236 and Q is Q4, and R and R⁴ are as per any row of Table U below, which compounds are named I.B1-Z236-Q4-U-1 to I.B1-Z236-Q4-U-42.

In another embodiment, compounds I are of formula I.B2 wherein Z is Z-236 and Q is Q1, and R and R⁴ are as per any row of Table U below, which compounds are named I.B2-Z236-Q1-U-1 to I.B2-Z236-Q1-U-42.

In another embodiment, compounds I are of formula I.B2 wherein Z is Z-236 and Q is Q2, and R and R⁴ are as per any row of Table U below, which compounds are named I.B2-Z236-Q2-U-1 to I.B2-Z236-Q2-U-42.

In another embodiment, compounds I are of formula I.B2 wherein Z is Z-236 and Q is Q3, and R and R⁴ are as per any row of Table U below, which compounds are named I.B2-Z236-Q3-U-1 to I.B2-Z236-Q3-U-42.

In another embodiment, compounds I are of formula I.B2 wherein Z is Z-236 and Q is Q4, and R and R⁴ are as per any row of Table U below, which compounds are named I.B2-Z236-Q4-U-1 to I.B2-Z236-Q4-U-42.

In another embodiment, compounds I are of formula I.B1 wherein Z is Z-237 and Q is Q1, and R and R⁴ are as per any row of Table U below, which compounds are named I.B1-Z237-Q1-U-1 to I.B1-Z237-Q1-U-42.

In another embodiment, compounds I are of formula I.B1 wherein Z is Z-237 and Q is Q2, and R and R⁴ are as per any row of Table U below, which compounds are named I.B1-Z237-Q2-U-1 to I.B1-Z237-Q2-U-42.

In another embodiment, compounds I are of formula I.B1 wherein Z is Z-237 and Q is Q3, and R and R⁴ are as per any row of Table U below, which compounds are named I.B1-Z237-Q3-U-1 to I.B1-Z237-Q3-U-42.

In another embodiment, compounds I are of formula I.B1 wherein Z is Z-237 and Q is Q4, and R and R⁴ are as per any row of Table U below, which compounds are named I.B1-Z237-Q4-U-1 to I.B1-Z237-Q4-U-42.

In another embodiment, compounds I are of formula I.B2 wherein Z is Z-237 and Q is Q1, and R and R⁴ are as per any row of Table U below, which compounds are named I.B2-Z237-Q1-U-1 to I.B2-Z237-Q1-U-42.

In another embodiment, compounds I are of formula I.B2 wherein Z is Z-237 and Q is Q2, and R and R⁴ are as per any row of Table U below, which compounds are named I.B2-Z237-Q2-U-1 to I.B2-Z237-Q2-U-42.

In another embodiment, compounds I are of formula I.B2 wherein Z is Z-237 and Q is Q3, and R and R⁴ are as per any row of Table U below, which compounds are named I.B2-Z237-Q3-U-1 to I.B2-Z237-Q3-U-42.

In another embodiment, compounds I are of formula I.B2 wherein Z is Z-237 and Q is Q4, and R and R⁴ are as per any row of Table U below, which compounds are named I.B2-Z237-Q4-U-1 to I.B2-Z237-Q4-U-42.

In another embodiment, compounds I are of formula I.B1 wherein Z is Z-238 and Q is Q1, and R and R⁴ are as per any row of Table U below, which compounds are named I.B1-Z238-Q1-U-1 to I.B1-Z238-Q1-U-42.

In another embodiment, compounds I are of formula I.B1 wherein Z is Z-238 and Q is Q2, and R and R⁴ are as per any row of Table U below, which compounds are named I.B1-Z238-Q2-U-1 to I.B1-Z238-Q2-U-42.

In another embodiment, compounds I are of formula I.B1 wherein Z is Z-238 and Q is Q3, and R and R⁴ are as per any row of Table U below, which compounds are named I.B1-Z238-Q3-U-1 to I.B1-Z238-Q3-U-42.

In another embodiment, compounds I are of formula I.B1 wherein Z is Z-238 and Q is Q4, and R and R⁴ are as per any row of Table U below, which compounds are named I.B1-Z238-Q4-U-1 to I.B1-Z238-Q4-U-42.

In another embodiment, compounds I are of formula I.B2 wherein Z is Z-238 and Q is Q1, and R and R⁴ are as per any row of Table U below, which compounds are named I.B2-Z238-Q1-U-1 to I.B2-Z238-Q1-U-42.

In another embodiment, compounds I are of formula I.B2 wherein Z is Z-238 and Q is Q2, and R and R⁴ are as per any row of Table U below, which compounds are named I.B2-Z238-Q2-U-1 to I.B2-Z238-Q2-U-42.

In another embodiment, compounds I are of formula I.B2 wherein Z is Z-238 and Q is Q3, and R and R⁴ are as per any row of Table U below, which compounds are named I.B2-Z238-Q3-U-1 to I.B2-Z238-Q3-U-42.

In another embodiment, compounds I are of formula I.B2 wherein Z is Z-238 and Q is Q4, and R and R⁴ are as per any row of Table U below, which compounds are named I.B2-Z238-Q4-U-1 to I.B2-Z238-Q4-U-42.

In another embodiment, compounds I are of formula I.B1 wherein Z is Z-239 and Q is Q1, and R and R⁴ are as per any row of Table U below, which compounds are named I.B1-Z239-Q1-U-1 to I.B1-Z239-Q1-U-42.

In another embodiment, compounds I are of formula I.B1 wherein Z is Z-239 and Q is Q2, and R and R⁴ are as per any row of Table U below, which compounds are named I.B1-Z239-Q2-U-1 to I.B1-Z239-Q2-U-42.

In another embodiment, compounds I are of formula I.B1 wherein Z is Z-239 and Q is Q3, and R and R⁴ are as per any row of Table U below, which compounds are named I.B1-Z239-Q3-U-1 to I.B1-Z239-Q3-U-42.

In another embodiment, compounds I are of formula I.B1 wherein Z is Z-239 and Q is Q4, and R and R⁴ are as per any row of Table U below, which compounds are named I.B1-Z239-Q4-U-1 to I.B1-Z239-Q4-U-42.

In another embodiment, compounds I are of formula I.B2 wherein Z is Z-239 and Q is Q1, and R and R⁴ are as per any row of Table U below, which compounds are named I.B2-Z239-Q1-U-1 to I.B2-Z239-Q1-U-42.

In another embodiment, compounds I are of formula I.B2 wherein Z is Z-239 and Q is Q2, and R and R⁴ are as per any row of Table U below, which compounds are named I.B2-Z239-Q2-U-1 to I.B2-Z239-Q2-U-42.

In another embodiment, compounds I are of formula I.B2 wherein Z is Z-239 and Q is Q3, and R and R⁴ are as per any row of Table U below, which compounds are named I.B2-Z239-Q3-U-1 to I.B2-Z239-Q3-U-42.

In another embodiment, compounds I are of formula I.B2 wherein Z is Z-239 and Q is Q4, and R and R⁴ are as per any row of Table U below, which compounds are named I.B2-Z239-Q4-U-1 to I.B2-Z239-Q4-U-42.

In another embodiment, compounds I are of formula I.B1 wherein Z is Z-240 and Q is Q1, and R and R⁴ are as per any row of Table U below, which compounds are named I.B1-Z240-Q1-U-1 to I.B1-Z240-Q1-U-42.

In another embodiment, compounds I are of formula I.B1 wherein Z is Z-240 and Q is Q2, and R and R⁴ are as per any row of Table U below, which compounds are named I.B1-Z240-Q2-U-1 to I.B1-Z240-Q2-U-42.

In another embodiment, compounds I are of formula I.B1 wherein Z is Z-240 and Q is Q3, and R and R⁴ are as per any row of Table U below, which compounds are named I.B1-Z240-Q3-U-1 to I.B1-Z240-Q3-U-42.

In another embodiment, compounds I are of formula I.B1 wherein Z is Z-240 and Q is Q4, and R and R⁴ are as per any row of Table U below, which compounds are named I.B1-Z240-Q4-U-1 to I.B1-Z240-Q4-U-42.

In another embodiment, compounds I are of formula I.B2 wherein Z is Z-240 and Q is Q1, and R and R⁴ are as per any row of Table U below, which compounds are named I.B2-Z240-Q1-U-1 to I.B2-Z240-Q1-U-42.

In another embodiment, compounds I are of formula I.B2 wherein Z is Z-240 and Q is Q2, and R and R⁴ are as per any row of Table U below, which compounds are named I.B2-Z240-Q2-U-1 to I.B2-Z240-Q2-U-42.

In another embodiment, compounds I are of formula I.B2 wherein Z is Z-240 and Q is Q3, and R and R⁴ are as per any row of Table U below, which compounds are named I.B2-Z240-Q3-U-1 to I.B2-Z240-Q3-U-42.

In another embodiment, compounds I are of formula I.B2 wherein Z is Z-240 and Q is Q4, and R and R⁴ are as per any row of Table U below, which compounds are named I.B2-Z240-Q4-U-1 to I.B2-Z240-Q4-U-42.

In another embodiment, compounds I are of formula I.B1 wherein Z is Z-241 and Q is Q1, and R and R⁴ are as per any row of Table U below, which compounds are named I.B1-Z241-Q1-U-1 to I.B1-Z241-Q1-U-42.

In another embodiment, compounds I are of formula I.B1 wherein Z is Z-241 and Q is Q2, and R and R⁴ are as per any row of Table U below, which compounds are named I.B1-Z241-Q2-U-1 to I.B1-Z241-Q2-U-42.

In another embodiment, compounds I are of formula I.B1 wherein Z is Z-241 and Q is Q3, and R and R⁴ are as per any row of Table U below, which compounds are named I.B1-Z241-Q3-U-1 to I.B1-Z241-Q3-U-42.

In another embodiment, compounds I are of formula I.B1 wherein Z is Z-241 and Q is Q4, and R and R⁴ are as per any row of Table U below, which compounds are named I.B1-Z241-Q4-U-1 to I.B1-Z241-Q4-U-42.

In another embodiment, compounds I are of formula I.B2 wherein Z is Z-241 and Q is Q1, and R and R⁴ are as per any row of Table U below, which compounds are named I.B2-Z241-Q1-U-1 to I.B2-Z241-Q1-U-42.

In another embodiment, compounds I are of formula I.B2 wherein Z is Z-241 and Q is Q2, and R and R⁴ are as per any row of Table U below, which compounds are named I.B2-Z241-Q2-U-1 to I.B2-Z241-Q2-U-42.

In another embodiment, compounds I are of formula I.B2 wherein Z is Z-241 and Q is Q3, and R and R⁴ are as per any row of Table U below, which compounds are named I.B2-Z241-Q3-U-1 to I.B2-Z241-Q3-U-42.

In another embodiment, compounds I are of formula I.B2 wherein Z is Z-241 and Q is Q4, and R and R⁴ are as per any row of Table U below, which compounds are named I.B2-Z241-Q4-U-1 to I.B2-Z241-Q4-U-42.

In another embodiment, compounds I are of formula I.B1 wherein Z is Z-242 and Q is Q1, and R and R⁴ are as per any row of Table U below, which compounds are named I.B1-Z242-Q1-U-1 to I.B1-Z242-Q1-U-42.

In another embodiment, compounds I are of formula I.B1 wherein Z is Z-242 and Q is Q2, and R and R⁴ are as per any row of Table U below, which compounds are named I.B1-Z242-Q2-U-1 to I.B1-Z242-Q2-U-42.

In another embodiment, compounds I are of formula I.B1 wherein Z is Z-242 and Q is Q3, and R and R⁴ are as per any row of Table U below, which compounds are named I.B1-Z242-Q3-U-1 to I.B1-Z242-Q3-U-42.

In another embodiment, compounds I are of formula I.B1 wherein Z is Z-242 and Q is Q4, and R and R⁴ are as per any row of Table U below, which compounds are named I.B1-Z242-Q4-U-1 to I.B1-Z242-Q4-U-42.

In another embodiment, compounds I are of formula I.B2 wherein Z is Z-242 and Q is Q1, and R and R⁴ are as per any row of Table U below, which compounds are named I.B2-Z242-Q1-U-1 to I.B2-Z242-Q1-U-42.

In another embodiment, compounds I are of formula I.B2 wherein Z is Z-242 and Q is Q2, and R and R⁴ are as per any row of Table U below, which compounds are named I.B2-Z242-Q2-U-1 to I.B2-Z242-Q2-U-42.

In another embodiment, compounds I are of formula I.B2 wherein Z is Z-242 and Q is Q3, and R and R⁴ are as per any row of Table U below, which compounds are named I.B2-Z242-Q3-U-1 to I.B2-Z242-Q3-U-42.

In another embodiment, compounds I are of formula I.B2 wherein Z is Z-242 and Q is Q4, and R and R⁴ are as per any row of Table U below, which compounds are named I.B2-Z242-Q4-U-1 to I.B2-Z242-Q4-U-42.

In another embodiment, compounds I are of formula I.B1 wherein Z is Z-243 and Q is Q1, and R and R⁴ are as per any row of Table U below, which compounds are named I.B1-Z243-Q1-U-1 to I.B1-Z243-Q1-U-42.

In another embodiment, compounds I are of formula I.B1 wherein Z is Z-243 and Q is Q2, and R and R⁴ are as per any row of Table U below, which compounds are named I.B1-Z243-Q2-U-1 to I.B1-Z243-Q2-U-42.

In another embodiment, compounds I are of formula I.B1 wherein Z is Z-243 and Q is Q3, and R and R⁴ are as per any row of Table U below, which compounds are named I.B1-Z243-Q3-U-1 to I.B1-Z243-Q3-U-42.

In another embodiment, compounds I are of formula I.B1 wherein Z is Z-243 and Q is Q4, and R and R⁴ are as per any row of Table U below, which compounds are named I.B1-Z243-Q4-U-1 to I.B1-Z243-Q4-U-42.

In another embodiment, compounds I are of formula I.B2 wherein Z is Z-243 and Q is Q1, and R and R⁴ are as per any row of Table U below, which compounds are named I.B2-Z243-Q1-U-1 to I.B2-Z243-Q1-U-42.

In another embodiment, compounds I are of formula I.B2 wherein Z is Z-243 and Q is Q2, and R and R⁴ are as per any row of Table U below, which compounds are named I.B2-Z243-Q2-U-1 to I.B2-Z243-Q2-U-42.

In another embodiment, compounds I are of formula I.B2 wherein Z is Z-243 and Q is Q3, and R and R⁴ are as per any row of Table U below, which compounds are named I.B2-Z243-Q3-U-1 to I.B2-Z243-Q3-U-42.

In another embodiment, compounds I are of formula I.B2 wherein Z is Z-243 and Q is Q4, and R and R⁴ are as per any row of Table U below, which compounds are named I.B2-Z243-Q4-U-1 to I.B2-Z243-Q4-U-42.

In another embodiment, compounds I are of formula I.B1 wherein Z is Z-244 and Q is Q1, and R and R⁴ are as per any row of Table U below, which compounds are named I.B1-Z244-Q1-U-1 to I.B1-Z244-Q1-U-42.

In another embodiment, compounds I are of formula I.B1 wherein Z is Z-244 and Q is Q2, and R and R⁴ are as per any row of Table U below, which compounds are named I.B1-Z244-Q2-U-1 to I.B1-Z244-Q2-U-42.

In another embodiment, compounds I are of formula I.B1 wherein Z is Z-244 and Q is Q3, and R and R⁴ are as per any row of Table U below, which compounds are named I.B1-Z244-Q3-U-1 to I.B1-Z244-Q3-U-42.

In another embodiment, compounds I are of formula I.B1 wherein Z is Z-244 and Q is Q4, and R and R⁴ are as per any row of Table U below, which compounds are named I.B1-Z244-Q4-U-1 to I.B1-Z244-Q4-U-42.

In another embodiment, compounds I are of formula I.B2 wherein Z is Z-244 and Q is Q1, and R and R⁴ are as per any row of Table U below, which compounds are named I.B2-Z244-Q1-U-1 to I.B2-Z244-Q1-U-42.

In another embodiment, compounds I are of formula I.B2 wherein Z is Z-244 and Q is Q2, and R and R⁴ are as per any row of Table U below, which compounds are named I.B2-Z244-Q2-U-1 to I.B2-Z244-Q2-U-42.

In another embodiment, compounds I are of formula I.B2 wherein Z is Z-244 and Q is Q3, and R and R⁴ are as per any row of Table U below, which compounds are named I.B2-Z244-Q3-U-1 to I.B2-Z244-Q3-U-42.

In another embodiment, compounds I are of formula I.B2 wherein Z is Z-244 and Q is Q4, and R and R⁴ are as per any row of Table U below, which compounds are named I.B2-Z244-Q4-U-1 to I.B2-Z244-Q4-U-42.

In another embodiment, compounds I are of formula I.B1 wherein Z is Z-245 and Q is Q1, and R and R⁴ are as per any row of Table U below, which compounds are named I.B1-Z245-Q1-U-1 to I.B1-Z245-Q1-U-42.

In another embodiment, compounds I are of formula I.B1 wherein Z is Z-245 and Q is Q2, and R and R⁴ are as per any row of Table U below, which compounds are named I.B1-Z245-Q2-U-1 to I.B1-Z245-Q2-U-42.

In another embodiment, compounds I are of formula I.B1 wherein Z is Z-245 and Q is Q3, and R and R⁴ are as per any row of Table U below, which compounds are named I.B1-Z245-Q3-U-1 to I.B1-Z245-Q3-U-42.

In another embodiment, compounds I are of formula I.B1 wherein Z is Z-245 and Q is Q4, and R and R⁴ are as per any row of Table U below, which compounds are named I.B1-Z245-Q4-U-1 to I.B1-Z245-Q4-U-42.

In another embodiment, compounds I are of formula I.B2 wherein Z is Z-245 and Q is Q1, and R and R⁴ are as per any row of Table U below, which compounds are named I.B2-Z245-Q1-U-1 to I.B2-Z245-Q1-U-42.

In another embodiment, compounds I are of formula I.B2 wherein Z is Z-245 and Q is Q2, and R and R⁴ are as per any row of Table U below, which compounds are named I.B2-Z245-Q2-U-1 to I.B2-Z245-Q2-U-42.

In another embodiment, compounds I are of formula I.B2 wherein Z is Z-245 and Q is Q3, and R and R⁴ are as per any row of Table U below, which compounds are named I.B2-Z245-Q3-U-1 to I.B2-Z245-Q3-U-42.

In another embodiment, compounds I are of formula I.B2 wherein Z is Z-245 and Q is Q4, and R and R⁴ are as per any row of Table U below, which compounds are named I.B2-Z245-Q4-U-1 to I.B2-Z245-Q4-U-42.

In another embodiment, compounds I are of formula I.B1 wherein Z is Z-246 and Q is Q1, and R and R⁴ are as per any row of Table U below, which compounds are named I.B1-Z246-Q1-U-1 to I.B1-Z246-Q1-U-42.

In another embodiment, compounds I are of formula I.B1 wherein Z is Z-246 and Q is Q2, and R and R⁴ are as per any row of Table U below, which compounds are named I.B1-Z246-Q2-U-1 to I.B1-Z246-Q2-U-42.

In another embodiment, compounds I are of formula I.B1 wherein Z is Z-246 and Q is Q3, and R and R⁴ are as per any row of Table U below, which compounds are named I.B1-Z246-Q3-U-1 to I.B1-Z246-Q3-U-42.

In another embodiment, compounds I are of formula I.B1 wherein Z is Z-246 and Q is Q4, and R and R⁴ are as per any row of Table U below, which compounds are named I.B1-Z246-Q4-U-1 to I.B1-Z246-Q4-U-42.

In another embodiment, compounds I are of formula I.B2 wherein Z is Z-246 and Q is Q1, and R and R⁴ are as per any row of Table U below, which compounds are named I.B2-Z246-Q1-U-1 to I.B2-Z246-Q1-U-42.

In another embodiment, compounds I are of formula I.B2 wherein Z is Z-246 and Q is Q2, and R and R⁴ are as per any row of Table U below, which compounds are named I.B2-Z246-Q2-U-1 to I.B2-Z246-Q2-U-42.

In another embodiment, compounds I are of formula I.B2 wherein Z is Z-246 and Q is Q3, and R and R⁴ are as per any row of Table U below, which compounds are named I.B2-Z246-Q3-U-1 to I.B2-Z246-Q3-U-42.

In another embodiment, compounds I are of formula I.B2 wherein Z is Z-246 and Q is Q4, and R and R⁴ are as per any row of Table U below, which compounds are named I.B2-Z246-Q4-U-1 to I.B2-Z246-Q4-U-42.

In another embodiment, compounds I are of formula I.B1 wherein Z is Z-247 and Q is Q1, and R and R⁴ are as per any row of Table U below, which compounds are named I.B1-Z247-Q1-U-1 to I.B1-Z247-Q1-U-42.

In another embodiment, compounds I are of formula I.B1 wherein Z is Z-247 and Q is Q2, and R and R⁴ are as per any row of Table U below, which compounds are named I.B1-Z247-Q2-U-1 to I.B1-Z247-Q2-U-42.

In another embodiment, compounds I are of formula I.B1 wherein Z is Z-247 and Q is Q3, and R and R⁴ are as per any row of Table U below, which compounds are named I.B1-Z247-Q3-U-1 to I.B1-Z247-Q3-U-42.

In another embodiment, compounds I are of formula I.B1 wherein Z is Z-247 and Q is Q4, and R and R⁴ are as per any row of Table U below, which compounds are named I.B1-Z247-Q4-U-1 to I.B1-Z247-Q4-U-42.

In another embodiment, compounds I are of formula I.B2 wherein Z is Z-247 and Q is Q1, and R and R⁴ are as per any row of Table U below, which compounds are named I.B2-Z247-Q1-U-1 to I.B2-Z247-Q1-U-42.

In another embodiment, compounds I are of formula I.B2 wherein Z is Z-247 and Q is Q2, and R and R⁴ are as per any row of Table U below, which compounds are named I.B2-Z247-Q2-U-1 to I.B2-Z247-Q2-U-42.

In another embodiment, compounds I are of formula I.B2 wherein Z is Z-247 and Q is Q3, and R and R⁴ are as per any row of Table U below, which compounds are named I.B2-Z247-Q3-U-1 to I.B2-Z247-Q3-U-42.

In another embodiment, compounds I are of formula I.B2 wherein Z is Z-247 and Q is Q4, and R and R⁴ are as per any row of Table U below, which compounds are named I.B2-Z247-Q4-U-1 to I.B2-Z247-Q4-U-42.

In another embodiment, compounds I are of formula I.B1 wherein Z is Z-248 and Q is Q1, and R and R⁴ are as per any row of Table U below, which compounds are named I.B1-Z248-Q1-U-1 to I.B1-Z248-Q1-U-42.

In another embodiment, compounds I are of formula I.B1 wherein Z is Z-248 and Q is Q2, and R and R⁴ are as per any row of Table U below, which compounds are named I.B1-Z248-Q2-U-1 to I.B1-Z248-Q2-U-42.

In another embodiment, compounds I are of formula I.B1 wherein Z is Z-248 and Q is Q3, and R and R⁴ are as per any row of Table U below, which compounds are named I.B1-Z248-Q3-U-1 to I.B1-Z248-Q3-U-42.

In another embodiment, compounds I are of formula I.B1 wherein Z is Z-248 and Q is Q4, and R and R⁴ are as per any row of Table U below, which compounds are named I.B1-Z248-Q4-U-1 to I.B1-Z248-Q4-U-42.

In another embodiment, compounds I are of formula I.B2 wherein Z is Z-248 and Q is Q1, and R and R⁴ are as per any row of Table U below, which compounds are named I.B2-Z248-Q1-U-1 to I.B2-Z248-Q1-U-42.

In another embodiment, compounds I are of formula I.B2 wherein Z is Z-248 and Q is Q2, and R and R⁴ are as per any row of Table U below, which compounds are named I.B2-Z248-Q2-U-1 to I.B2-Z248-Q2-U-42.

In another embodiment, compounds I are of formula I.B2 wherein Z is Z-248 and Q is Q3, and R and R⁴ are as per any row of Table U below, which compounds are named I.B2-Z248-Q3-U-1 to I.B2-Z248-Q3-U-42.

In another embodiment, compounds I are of formula I.B2 wherein Z is Z-248 and Q is Q4, and R and R⁴ are as per any row of Table U below, which compounds are named I.B2-Z248-Q4-U-1 to I.B2-Z248-Q4-U-42.

In another embodiment, compounds I are of formula I.B1 wherein Z is Z-249 and Q is Q1, and R and R⁴ are as per any row of Table U below, which compounds are named I.B1-Z249-Q1-U-1 to I.B1-Z249-Q1-U-42.

In another embodiment, compounds I are of formula I.B1 wherein Z is Z-249 and Q is Q2, and R and R⁴ are as per any row of Table U below, which compounds are named I.B1-Z249-Q2-U-1 to I.B1-Z249-Q2-U-42.

In another embodiment, compounds I are of formula I.B1 wherein Z is Z-249 and Q is Q3, and R and R⁴ are as per any row of Table U below, which compounds are named I.B1-Z249-Q3-U-1 to I.B1-Z249-Q3-U-42.

In another embodiment, compounds I are of formula I.B1 wherein Z is Z-249 and Q is Q4, and R and R⁴ are as per any row of Table U below, which compounds are named I.B1-Z249-Q4-U-1 to I.B1-Z249-Q4-U-42.

In another embodiment, compounds I are of formula I.B2 wherein Z is Z-249 and Q is Q1, and R and R⁴ are as per any row of Table U below, which compounds are named I.B2-Z249-Q1-U-1 to I.B2-Z249-Q1-U-42.

In another embodiment, compounds I are of formula I.B2 wherein Z is Z-249 and Q is Q2, and R and R⁴ are as per any row of Table U below, which compounds are named I.B2-Z249-Q2-U-1 to I.B2-Z249-Q2-U-42.

In another embodiment, compounds I are of formula I.B2 wherein Z is Z-249 and Q is Q3, and R and R⁴ are as per any row of Table U below, which compounds are named I.B2-Z249-Q3-U-1 to I.B2-Z249-Q3-U-42.

In another embodiment, compounds I are of formula I.B2 wherein Z is Z-249 and Q is Q4, and R and R⁴ are as per any row of Table U below, which compounds are named I.B2-Z249-Q4-U-1 to I.B2-Z249-Q4-U-42.

In another embodiment, compounds I are of formula I.B1 wherein Z is Z-250 and Q is Q1, and R and R⁴ are as per any row of Table U below, which compounds are named I.B1-Z250-Q1-U-1 to I.B1-Z250-Q1-U-42.

In another embodiment, compounds I are of formula I.B1 wherein Z is Z-250 and Q is Q2, and R and R⁴ are as per any row of Table U below, which compounds are named I.B1-Z250-Q2-U-1 to I.B1-Z250-Q2-U-42.

In another embodiment, compounds I are of formula I.B1 wherein Z is Z-250 and Q is Q3, and R and R⁴ are as per any row of Table U below, which compounds are named I.B1-Z250-Q3-U-1 to I.B1-Z250-Q3-U-42.

In another embodiment, compounds I are of formula I.B1 wherein Z is Z-250 and Q is Q4, and R and R⁴ are as per any row of Table U below, which compounds are named I.B1-Z250-Q4-U-1 to I.B1-Z250-Q4-U-42.

In another embodiment, compounds I are of formula I.B2 wherein Z is Z-250 and Q is Q1, and R and R⁴ are as per any row of Table U below, which compounds are named I.B2-Z250-Q1-U-1 to I.B2-Z250-Q1-U-42.

In another embodiment, compounds I are of formula I.B2 wherein Z is Z-250 and Q is Q2, and R and R⁴ are as per any row of Table U below, which compounds are named I.B2-Z250-Q2-U-1 to I.B2-Z250-Q2-U-42.

In another embodiment, compounds I are of formula I.B2 wherein Z is Z-250 and Q is Q3, and R and R⁴ are as per any row of Table U below, which compounds are named I.B2-Z250-Q3-U-1 to I.B2-Z250-Q3-U-42.

In another embodiment, compounds I are of formula I.B2 wherein Z is Z-250 and Q is Q4, and R and R⁴ are as per any row of Table U below, which compounds are named I.B2-Z250-Q4-U-1 to I.B2-Z250-Q4-U-42.

In another embodiment, compounds I are of formula I.B1 wherein Z is Z-251 and Q is Q1, and R and R⁴ are as per any row of Table U below, which compounds are named I.B1-Z251-Q1-U-1 to I.B1-Z251-Q1-U-42.

In another embodiment, compounds I are of formula I.B1 wherein Z is Z-251 and Q is Q2, and R and R⁴ are as per any row of Table U below, which compounds are named I.B1-Z251-Q2-U-1 to I.B1-Z251-Q2-U-42.

In another embodiment, compounds I are of formula I.B1 wherein Z is Z-251 and Q is Q3, and R and R⁴ are as per any row of Table U below, which compounds are named I.B1-Z251-Q3-U-1 to I.B1-Z251-Q3-U-42.

In another embodiment, compounds I are of formula I.B1 wherein Z is Z-251 and Q is Q4, and R and R⁴ are as per any row of Table U below, which compounds are named I.B1-Z251-Q4-U-1 to I.B1-Z251-Q4-U-42.

In another embodiment, compounds I are of formula I.B2 wherein Z is Z-251 and Q is Q1, and R and R⁴ are as per any row of Table U below, which compounds are named I.B2-Z251-Q1-U-1 to I.B2-Z251-Q1-U-42.

In another embodiment, compounds I are of formula I.B2 wherein Z is Z-251 and Q is Q2, and R and R⁴ are as per any row of Table U below, which compounds are named I.B2-Z251-Q2-U-1 to I.B2-Z251-Q2-U-42.

In another embodiment, compounds I are of formula I.B2 wherein Z is Z-251 and Q is Q3, and R and R⁴ are as per any row of Table U below, which compounds are named I.B2-Z251-Q3-U-1 to I.B2-Z251-Q3-U-42.

In another embodiment, compounds I are of formula I.B2 wherein Z is Z-251 and Q is Q4, and R and R⁴ are as per any row of Table U below, which compounds are named I.B2-Z251-Q4-U-1 to I.B2-Z251-Q4-U-42.

In another embodiment, compounds I are of formula I.B1 wherein Z is Z-252 and Q is Q1, and R and R⁴ are as per any row of Table U below, which compounds are named I.B1-Z252-Q1-U-1 to I.B1-Z252-Q1-U-42.

In another embodiment, compounds I are of formula I.B1 wherein Z is Z-252 and Q is Q2, and R and R⁴ are as per any row of Table U below, which compounds are named I.B1-Z252-Q2-U-1 to I.B1-Z252-Q2-U-42.

In another embodiment, compounds I are of formula I.B1 wherein Z is Z-252 and Q is Q3, and R and R⁴ are as per any row of Table U below, which compounds are named I.B1-Z252-Q3-U-1 to I.B1-Z252-Q3-U-42.

In another embodiment, compounds I are of formula I.B1 wherein Z is Z-252 and Q is Q4, and R and R⁴ are as per any row of Table U below, which compounds are named I.B1-Z252-Q4-U-1 to I.B1-Z252-Q4-U-42.

In another embodiment, compounds I are of formula I.B2 wherein Z is Z-252 and Q is Q1, and R and R⁴ are as per any row of Table U below, which compounds are named I.B2-Z252-Q1-U-1 to I.B2-Z252-Q1-U-42.

In another embodiment, compounds I are of formula I.B2 wherein Z is Z-252 and Q is Q2, and R and R⁴ are as per any row of Table U below, which compounds are named I.B2-Z252-Q2-U-1 to I.B2-Z252-Q2-U-42.

In another embodiment, compounds I are of formula I.B2 wherein Z is Z-252 and Q is Q3, and R and R⁴ are as per any row of Table U below, which compounds are named I.B2-Z252-Q3-U-1 to I.B2-Z252-Q3-U-42.

In another embodiment, compounds I are of formula I.B2 wherein Z is Z-252 and Q is Q4, and R and R⁴ are as per any row of Table U below, which compounds are named I.B2-Z252-Q4-U-1 to I.B2-Z252-Q4-U-42.

In another embodiment, compounds I are of formula I.B1 wherein Z is Z-253 and Q is Q1, and R and R⁴ are as per any row of Table U below, which compounds are named I.B1-Z253-Q1-U-1 to I.B1-Z253-Q1-U-42.

In another embodiment, compounds I are of formula I.B1 wherein Z is Z-253 and Q is Q2, and R and R⁴ are as per any row of Table U below, which compounds are named I.B1-Z253-Q2-U-1 to I.B1-Z253-Q2-U-42.

In another embodiment, compounds I are of formula I.B1 wherein Z is Z-253 and Q is Q3, and R and R⁴ are as per any row of Table U below, which compounds are named I.B1-Z253-Q3-U-1 to I.B1-Z253-Q3-U-42.

In another embodiment, compounds I are of formula I.B1 wherein Z is Z-253 and Q is Q4, and R and R⁴ are as per any row of Table U below, which compounds are named I.B1-Z253-Q4-U-1 to I.B1-Z253-Q4-U-42.

In another embodiment, compounds I are of formula I.B2 wherein Z is Z-253 and Q is Q1, and R and R⁴ are as per any row of Table U below, which compounds are named I.B2-Z253-Q1-U-1 to I.B2-Z253-Q1-U-42.

In another embodiment, compounds I are of formula I.B2 wherein Z is Z-253 and Q is Q2, and R and R⁴ are as per any row of Table U below, which compounds are named I.B2-Z253-Q2-U-1 to I.B2-Z253-Q2-U-42.

In another embodiment, compounds I are of formula I.B2 wherein Z is Z-253 and Q is Q3, and R and R⁴ are as per any row of Table U below, which compounds are named I.B2-Z253-Q3-U-1 to I.B2-Z253-Q3-U-42.

In another embodiment, compounds I are of formula I.B2 wherein Z is Z-253 and Q is Q4, and R and R⁴ are as per any row of Table U below, which compounds are named I.B2-Z253-Q4-U-1 to I.B2-Z253-Q4-U-42.

In another embodiment, compounds I are of formula I.B1 wherein Z is Z-254 and Q is Q1, and R and R⁴ are as per any row of Table U below, which compounds are named I.B1-Z254-Q1-U-1 to I.B1-Z254-Q1-U-42.

In another embodiment, compounds I are of formula I.B1 wherein Z is Z-254 and Q is Q2, and R and R⁴ are as per any row of Table U below, which compounds are named I.B1-Z254-Q2-U-1 to I.B1-Z254-Q2-U-42.

In another embodiment, compounds I are of formula I.B1 wherein Z is Z-254 and Q is Q3, and R and R⁴ are as per any row of Table U below, which compounds are named I.B1-Z254-Q3-U-1 to I.B1-Z254-Q3-U-42.

In another embodiment, compounds I are of formula I.B1 wherein Z is Z-254 and Q is Q4, and R and R⁴ are as per any row of Table U below, which compounds are named I.B1-Z254-Q4-U-1 to I.B1-Z254-Q4-U-42.

In another embodiment, compounds I are of formula I.B2 wherein Z is Z-254 and Q is Q1, and R and R⁴ are as per any row of Table U below, which compounds are named I.B2-Z254-Q1-U-1 to I.B2-Z254-Q1-U-42.

In another embodiment, compounds I are of formula I.B2 wherein Z is Z-254 and Q is Q2, and R and R⁴ are as per any row of Table U below, which compounds are named I.B2-Z254-Q2-U-1 to I.B2-Z254-Q2-U-42.

In another embodiment, compounds I are of formula I.B2 wherein Z is Z-254 and Q is Q3, and R and R⁴ are as per any row of Table U below, which compounds are named I.B2-Z254-Q3-U-1 to I.B2-Z254-Q3-U-42.

In another embodiment, compounds I are of formula I.B2 wherein Z is Z-254 and Q is Q4, and R and R⁴ are as per any row of Table U below, which compounds are named I.B2-Z254-Q4-U-1 to I.B2-Z254-Q4-U-42.

In another embodiment, compounds I are of formula I.B1 wherein Z is Z-255 and Q is Q1, and R and R⁴ are as per any row of Table U below, which compounds are named I.B1-Z255-Q1-U-1 to I.B1-Z255-Q1-U-42.

In another embodiment, compounds I are of formula I.B1 wherein Z is Z-255 and Q is Q2, and R and R⁴ are as per any row of Table U below, which compounds are named I.B1-Z255-Q2-U-1 to I.B1-Z255-Q2-U-42.

In another embodiment, compounds I are of formula I.B1 wherein Z is Z-255 and Q is Q3, and R and R⁴ are as per any row of Table U below, which compounds are named I.B1-Z255-Q3-U-1 to I.B1-Z255-Q3-U-42.

In another embodiment, compounds I are of formula I.B1 wherein Z is Z-255 and Q is Q4, and R and R⁴ are as per any row of Table U below, which compounds are named I.B1-Z255-Q4-U-1 to I.B1-Z255-Q4-U-42.

In another embodiment, compounds I are of formula I.B2 wherein Z is Z-255 and Q is Q1, and R and R⁴ are as per any row of Table U below, which compounds are named I.B2-Z255-Q1-U-1 to I.B2-Z255-Q1-U-42.

In another embodiment, compounds I are of formula I.B2 wherein Z is Z-255 and Q is Q2, and R and R⁴ are as per any row of Table U below, which compounds are named I.B2-Z255-Q2-U-1 to I.B2-Z255-Q2-U-42.

In another embodiment, compounds I are of formula I.B2 wherein Z is Z-255 and Q is Q3, and R and R⁴ are as per any row of Table U below, which compounds are named I.B2-Z255-Q3-U-1 to I.B2-Z255-Q3-U-42.

In another embodiment, compounds I are of formula I.B2 wherein Z is Z-255 and Q is Q4, and R and R⁴ are as per any row of Table U below, which compounds are named I.B2-Z255-Q4-U-1 to I.B2-Z255-Q4-U-42.

In another embodiment, compounds I are of formula I.B1 wherein Z is Z-256 and Q is Q1, and R and R⁴ are as per any row of Table U below, which compounds are named I.B1-Z256-Q1-U-1 to I.B1-Z256-Q1-U-42.

In another embodiment, compounds I are of formula I.B1 wherein Z is Z-256 and Q is Q2, and R and R⁴ are as per any row of Table U below, which compounds are named I.B1-Z256-Q2-U-1 to I.B1-Z256-Q2-U-42.

In another embodiment, compounds I are of formula I.B1 wherein Z is Z-256 and Q is Q3, and R and R⁴ are as per any row of Table U below, which compounds are named I.B1-Z256-Q3-U-1 to I.B1-Z256-Q3-U-42.

In another embodiment, compounds I are of formula I.B1 wherein Z is Z-256 and Q is Q4, and R and R⁴ are as per any row of Table U below, which compounds are named I.B1-Z256-Q4-U-1 to I.B1-Z256-Q4-U-42.

In another embodiment, compounds I are of formula I.B2 wherein Z is Z-256 and Q is Q1, and R and R⁴ are as per any row of Table U below, which compounds are named I.B2-Z256-Q1-U-1 to I.B2-Z256-Q1-U-42.

In another embodiment, compounds I are of formula I.B2 wherein Z is Z-256 and Q is Q2, and R and R⁴ are as per any row of Table U below, which compounds are named I.B2-Z256-Q2-U-1 to I.B2-Z256-Q2-U-42.

In another embodiment, compounds I are of formula I.B2 wherein Z is Z-256 and Q is Q3, and R and R⁴ are as per any row of Table U below, which compounds are named I.B2-Z256-Q3-U-1 to I.B2-Z256-Q3-U-42.

In another embodiment, compounds I are of formula I.B2 wherein Z is Z-256 and Q is Q4, and R and R⁴ are as per any row of Table U below, which compounds are named I.B2-Z256-Q4-U-1 to I.B2-Z256-Q4-U-42.

In another embodiment, compounds I are of formula I.B1 wherein Z is Z-257 and Q is Q1, and R and R⁴ are as per any row of Table U below, which compounds are named I.B1-Z257-Q1-U-1 to I.B1-Z257-Q1-U-42.

In another embodiment, compounds I are of formula I.B1 wherein Z is Z-257 and Q is Q2, and R and R⁴ are as per any row of Table U below, which compounds are named I.B1-Z257-Q2-U-1 to I.B1-Z257-Q2-U-42.

In another embodiment, compounds I are of formula I.B1 wherein Z is Z-257 and Q is Q3, and R and R⁴ are as per any row of Table U below, which compounds are named I.B1-Z257-Q3-U-1 to I.B1-Z257-Q3-U-42.

In another embodiment, compounds I are of formula I.B1 wherein Z is Z-257 and Q is Q4, and R and R⁴ are as per any row of Table U below, which compounds are named I.B1-Z257-Q4-U-1 to I.B1-Z257-Q4-U-42.

In another embodiment, compounds I are of formula I.B2 wherein Z is Z-257 and Q is Q1, and R and R⁴ are as per any row of Table U below, which compounds are named I.B2-Z257-Q1-U-1 to I.B2-Z257-Q1-U-42.

In another embodiment, compounds I are of formula I.B2 wherein Z is Z-257 and Q is Q2, and R and R⁴ are as per any row of Table U below, which compounds are named I.B2-Z257-Q2-U-1 to I.B2-Z257-Q2-U-42.

In another embodiment, compounds I are of formula I.B2 wherein Z is Z-257 and Q is Q3, and R and R⁴ are as per any row of Table U below, which compounds are named I.B2-Z257-Q3-U-1 to I.B2-Z257-Q3-U-42.

In another embodiment, compounds I are of formula I.B2 wherein Z is Z-257 and Q is Q4, and R and R⁴ are as per any row of Table U below, which compounds are named I.B2-Z257-Q4-U-1 to I.B2-Z257-Q4-U-42.

In another embodiment, compounds I are of formula I.B1 wherein Z is Z-258 and Q is Q1, and R and R⁴ are as per any row of Table U below, which compounds are named I.B1-Z258-Q1-U-1 to I.B1-Z258-Q1-U-42.

In another embodiment, compounds I are of formula I.B1 wherein Z is Z-258 and Q is Q2, and R and R⁴ are as per any row of Table U below, which compounds are named I.B1-Z258-Q2-U-1 to I.B1-Z258-Q2-U-42.

In another embodiment, compounds I are of formula I.B1 wherein Z is Z-258 and Q is Q3, and R and R⁴ are as per any row of Table U below, which compounds are named I.B1-Z258-Q3-U-1 to I.B1-Z258-Q3-U-42.

In another embodiment, compounds I are of formula I.B1 wherein Z is Z-258 and Q is Q4, and R and R⁴ are as per any row of Table U below, which compounds are named I.B1-Z258-Q4-U-1 to I.B1-Z258-Q4-U-42.

In another embodiment, compounds I are of formula I.B2 wherein Z is Z-258 and Q is Q1, and R and R⁴ are as per any row of Table U below, which compounds are named I.B2-Z258-Q1-U-1 to I.B2-Z258-Q1-U-42.

In another embodiment, compounds I are of formula I.B2 wherein Z is Z-258 and Q is Q2, and R and R⁴ are as per any row of Table U below, which compounds are named I.B2-Z258-Q2-U-1 to I.B2-Z258-Q2-U-42.

In another embodiment, compounds I are of formula I.B2 wherein Z is Z-258 and Q is Q3, and R and R⁴ are as per any row of Table U below, which compounds are named I.B2-Z258-Q3-U-1 to I.B2-Z258-Q3-U-42.

In another embodiment, compounds I are of formula I.B2 wherein Z is Z-258 and Q is Q4, and R and R⁴ are as per any row of Table U below, which compounds are named I.B2-Z258-Q4-U-1 to I.B2-Z258-Q4-U-42.

In another embodiment, compounds I are of formula I.B1 wherein Z is Z-259 and Q is Q1, and R and R⁴ are as per any row of Table U below, which compounds are named I.B1-Z259-Q1-U-1 to I.B1-Z259-Q1-U-42.

In another embodiment, compounds I are of formula I.B1 wherein Z is Z-259 and Q is Q2, and R and R⁴ are as per any row of Table U below, which compounds are named I.B1-Z259-Q2-U-1 to I.B1-Z259-Q2-U-42.

In another embodiment, compounds I are of formula I.B1 wherein Z is Z-259 and Q is Q3, and R and R⁴ are as per any row of Table U below, which compounds are named I.B1-Z259-Q3-U-1 to I.B1-Z259-Q3-U-42.

In another embodiment, compounds I are of formula I.B1 wherein Z is Z-259 and Q is Q4, and R and R⁴ are as per any row of Table U below, which compounds are named I.B1-Z259-Q4-U-1 to I.B1-Z259-Q4-U-42.

In another embodiment, compounds I are of formula I.B2 wherein Z is Z-259 and Q is Q1, and R and R⁴ are as per any row of Table U below, which compounds are named I.B2-Z259-Q1-U-1 to I.B2-Z259-Q1-U-42.

In another embodiment, compounds I are of formula I.B2 wherein Z is Z-259 and Q is Q2, and R and R⁴ are as per any row of Table U below, which compounds are named I.B2-Z259-Q2-U-1 to I.B2-Z259-Q2-U-42.

In another embodiment, compounds I are of formula I.B2 wherein Z is Z-259 and Q is Q3, and R and R⁴ are as per any row of Table U below, which compounds are named I.B2-Z259-Q3-U-1 to I.B2-Z259-Q3-U-42.

In another embodiment, compounds I are of formula I.B2 wherein Z is Z-259 and Q is Q4, and R and R⁴ are as per any row of Table U below, which compounds are named I.B2-Z259-Q4-U-1 to I.B2-Z259-Q4-U-42.

In another embodiment, compounds I are of formula I.B1 wherein Z is Z-260 and Q is Q1, and R and R⁴ are as per any row of Table U below, which compounds are named I.B1-Z260-Q1-U-1 to I.B1-Z260-Q1-U-42.

In another embodiment, compounds I are of formula I.B1 wherein Z is Z-260 and Q is Q2, and R and R⁴ are as per any row of Table U below, which compounds are named I.B1-Z260-Q2-U-1 to I.B1-Z260-Q2-U-42.

In another embodiment, compounds I are of formula I.B1 wherein Z is Z-260 and Q is Q3, and R and R⁴ are as per any row of Table U below, which compounds are named I.B1-Z260-Q3-U-1 to I.B1-Z260-Q3-U-42.

In another embodiment, compounds I are of formula I.B1 wherein Z is Z-260 and Q is Q4, and R and R⁴ are as per any row of Table U below, which compounds are named I.B1-Z260-Q4-U-1 to I.B1-Z260-Q4-U-42.

In another embodiment, compounds I are of formula I.B2 wherein Z is Z-260 and Q is Q1, and R and R⁴ are as per any row of Table U below, which compounds are named I.B2-Z260-Q1-U-1 to I.B2-Z260-Q1-U-42.

In another embodiment, compounds I are of formula I.B2 wherein Z is Z-260 and Q is Q2, and R and R⁴ are as per any row of Table U below, which compounds are named I.B2-Z260-Q2-U-1 to I.B2-Z260-Q2-U-42.

In another embodiment, compounds I are of formula I.B2 wherein Z is Z-260 and Q is Q3, and R and R⁴ are as per any row of Table U below, which compounds are named I.B2-Z260-Q3-U-1 to I.B2-Z260-Q3-U-42.

In another embodiment, compounds I are of formula I.B2 wherein Z is Z-260 and Q is Q4, and R and R⁴ are as per any row of Table U below, which compounds are named I.B2-Z260-Q4-U-1 to I.B2-Z260-Q4-U-42.

In another embodiment, compounds I are of formula I.B1 wherein Z is Z-261 and Q is Q1, and R and R⁴ are as per any row of Table U below, which compounds are named I.B1-Z261-Q1-U-1 to I.B1-Z261-Q1-U-42.

In another embodiment, compounds I are of formula I.B1 wherein Z is Z-261 and Q is Q2, and R and R⁴ are as per any row of Table U below, which compounds are named I.B1-Z261-Q2-U-1 to I.B1-Z261-Q2-U-42.

In another embodiment, compounds I are of formula I.B1 wherein Z is Z-261 and Q is Q3, and R and R⁴ are as per any row of Table U below, which compounds are named I.B1-Z261-Q3-U-1 to I.B1-Z261-Q3-U-42.

In another embodiment, compounds I are of formula I.B1 wherein Z is Z-261 and Q is Q4, and R and R⁴ are as per any row of Table U below, which compounds are named I.B1-Z261-Q4-U-1 to I.B1-Z261-Q4-U-42.

In another embodiment, compounds I are of formula I.B2 wherein Z is Z-261 and Q is Q1, and R and R⁴ are as per any row of Table U below, which compounds are named I.B2-Z261-Q1-U-1 to I.B2-Z261-Q1-U-42.

In another embodiment, compounds I are of formula I.B2 wherein Z is Z-261 and Q is Q2, and R and R⁴ are as per any row of Table U below, which compounds are named I.B2-Z261-Q2-U-1 to I.B2-Z261-Q2-U-42.

In another embodiment, compounds I are of formula I.B2 wherein Z is Z-261 and Q is Q3, and R and R⁴ are as per any row of Table U below, which compounds are named I.B2-Z261-Q3-U-1 to I.B2-Z261-Q3-U-42.

In another embodiment, compounds I are of formula I.B2 wherein Z is Z-261 and Q is Q4, and R and R⁴ are as per any row of Table U below, which compounds are named I.B2-Z261-Q4-U-1 to I.B2-Z261-Q4-U-42.

In another embodiment, compounds I are of formula I.B1 wherein Z is Z-262 and Q is Q1, and R and R⁴ are as per any row of Table U below, which compounds are named I.B1-Z262-Q1-U-1 to I.B1-Z262-Q1-U-42.

In another embodiment, compounds I are of formula I.B1 wherein Z is Z-262 and Q is Q2, and R and R⁴ are as per any row of Table U below, which compounds are named I.B1-Z262-Q2-U-1 to I.B1-Z262-Q2-U-42.

In another embodiment, compounds I are of formula I.B1 wherein Z is Z-262 and Q is Q3, and R and R⁴ are as per any row of Table U below, which compounds are named I.B1-Z262-Q3-U-1 to I.B1-Z262-Q3-U-42.

In another embodiment, compounds I are of formula I.B1 wherein Z is Z-262 and Q is Q4, and R and R⁴ are as per any row of Table U below, which compounds are named I.B1-Z262-Q4-U-1 to I.B1-Z262-Q4-U-42.

In another embodiment, compounds I are of formula I.B2 wherein Z is Z-262 and Q is Q1, and R and R⁴ are as per any row of Table U below, which compounds are named I.B2-Z262-Q1-U-1 to I.B2-Z262-Q1-U-42.

In another embodiment, compounds I are of formula I.B2 wherein Z is Z-262 and Q is Q2, and R and R⁴ are as per any row of Table U below, which compounds are named I.B2-Z262-Q2-U-1 to I.B2-Z262-Q2-U-42.

In another embodiment, compounds I are of formula I.B2 wherein Z is Z-262 and Q is Q3, and R and R⁴ are as per any row of Table U below, which compounds are named I.B2-Z262-Q3-U-1 to I.B2-Z262-Q3-U-42.

In another embodiment, compounds I are of formula I.B2 wherein Z is Z-262 and Q is Q4, and R and R⁴ are as per any row of Table U below, which compounds are named I.B2-Z262-Q4-U-1 to I.B2-Z262-Q4-U-42.

In another embodiment, compounds I are of formula I.B1 wherein Z is Z-263 and Q is Q1, and R and R⁴ are as per any row of Table U below, which compounds are named I.B1-Z263-Q1-U-1 to I.B1-Z263-Q1-U-42.

In another embodiment, compounds I are of formula I.B1 wherein Z is Z-263 and Q is Q2, and R and R⁴ are as per any row of Table U below, which compounds are named I.B1-Z263-Q2-U-1 to I.B1-Z263-Q2-U-42.

In another embodiment, compounds I are of formula I.B1 wherein Z is Z-263 and Q is Q3, and R and R⁴ are as per any row of Table U below, which compounds are named I.B1-Z263-Q3-U-1 to I.B1-Z263-Q3-U-42.

In another embodiment, compounds I are of formula I.B1 wherein Z is Z-263 and Q is Q4, and R and R⁴ are as per any row of Table U below, which compounds are named I.B1-Z263-Q4-U-1 to I.B1-Z263-Q4-U-42.

In another embodiment, compounds I are of formula I.B2 wherein Z is Z-263 and Q is Q1, and R and R⁴ are as per any row of Table U below, which compounds are named I.B2-Z263-Q1-U-1 to I.B2-Z263-Q1-U-42.

In another embodiment, compounds I are of formula I.B2 wherein Z is Z-263 and Q is Q2, and R and R⁴ are as per any row of Table U below, which compounds are named I.B2-Z263-Q2-U-1 to I.B2-Z263-Q2-U-42.

In another embodiment, compounds I are of formula I.B2 wherein Z is Z-263 and Q is Q3, and R and R⁴ are as per any row of Table U below, which compounds are named I.B2-Z263-Q3-U-1 to I.B2-Z263-Q3-U-42.

In another embodiment, compounds I are of formula I.B2 wherein Z is Z-263 and Q is Q4, and R and R⁴ are as per any row of Table U below, which compounds are named I.B2-Z263-Q4-U-1 to I.B2-Z263-Q4-U-42.

In another embodiment, compounds I are of formula I.B1 wherein Z is Z-264 and Q is Q1, and R and R⁴ are as per any row of Table U below, which compounds are named I.B1-Z264-Q1-U-1 to I.B1-Z264-Q1-U-42.

In another embodiment, compounds I are of formula I.B1 wherein Z is Z-264 and Q is Q2, and R and R⁴ are as per any row of Table U below, which compounds are named I.B1-Z264-Q2-U-1 to I.B1-Z264-Q2-U-42.

In another embodiment, compounds I are of formula I.B1 wherein Z is Z-264 and Q is Q3, and R and R⁴ are as per any row of Table U below, which compounds are named I.B1-Z264-Q3-U-1 to I.B1-Z264-Q3-U-42.

In another embodiment, compounds I are of formula I.B1 wherein Z is Z-264 and Q is Q4, and R and R⁴ are as per any row of Table U below, which compounds are named I.B1-Z264-Q4-U-1 to I.B1-Z264-Q4-U-42.

In another embodiment, compounds I are of formula I.B2 wherein Z is Z-264 and Q is Q1, and R and R⁴ are as per any row of Table U below, which compounds are named I.B2-Z264-Q1-U-1 to I.B2-Z264-Q1-U-42.

In another embodiment, compounds I are of formula I.B2 wherein Z is Z-264 and Q is Q2, and R and R⁴ are as per any row of Table U below, which compounds are named I.B2-Z264-Q2-U-1 to I.B2-Z264-Q2-U-42.

In another embodiment, compounds I are of formula I.B2 wherein Z is Z-264 and Q is Q3, and R and R⁴ are as per any row of Table U below, which compounds are named I.B2-Z264-Q3-U-1 to I.B2-Z264-Q3-U-42.

In another embodiment, compounds I are of formula I.B2 wherein Z is Z-264 and Q is Q4, and R and R⁴ are as per any row of Table U below, which compounds are named I.B2-Z264-Q4-U-1 to I.B2-Z264-Q4-U-42.

In another embodiment, compounds I are of formula I.B1 wherein Z is Z-265 and Q is Q1, and R and R⁴ are as per any row of Table U below, which compounds are named I.B1-Z265-Q1-U-1 to I.B1-Z265-Q1-U-42.

In another embodiment, compounds I are of formula I.B1 wherein Z is Z-265 and Q is Q2, and R and R⁴ are as per any row of Table U below, which compounds are named I.B1-Z265-Q2-U-1 to I.B1-Z265-Q2-U-42.

In another embodiment, compounds I are of formula I.B1 wherein Z is Z-265 and Q is Q3, and R and R⁴ are as per any row of Table U below, which compounds are named I.B1-Z265-Q3-U-1 to I.B1-Z265-Q3-U-42.

In another embodiment, compounds I are of formula I.B1 wherein Z is Z-265 and Q is Q4, and R and R⁴ are as per any row of Table U below, which compounds are named I.B1-Z265-Q4-U-1 to I.B1-Z265-Q4-U-42.

In another embodiment, compounds I are of formula I.B2 wherein Z is Z-265 and Q is Q1, and R and R⁴ are as per any row of Table U below, which compounds are named I.B2-Z265-Q1-U-1 to I.B2-Z265-Q1-U-42.

In another embodiment, compounds I are of formula I.B2 wherein Z is Z-265 and Q is Q2, and R and R⁴ are as per any row of Table U below, which compounds are named I.B2-Z265-Q2-U-1 to I.B2-Z265-Q2-U-42.

In another embodiment, compounds I are of formula I.B2 wherein Z is Z-265 and Q is Q3, and R and R⁴ are as per any row of Table U below, which compounds are named I.B2-Z265-Q3-U-1 to I.B2-Z265-Q3-U-42.

In another embodiment, compounds I are of formula I.B2 wherein Z is Z-265 and Q is Q4, and R and R⁴ are as per any row of Table U below, which compounds are named I.B2-Z265-Q4-U-1 to I.B2-Z265-Q4-U-42.

In another embodiment, compounds I are of formula I.B1 wherein Z is Z-266 and Q is Q1, and R and R⁴ are as per any row of Table U below, which compounds are named I.B1-Z266-Q1-U-1 to I.B1-Z266-Q1-U-42.

In another embodiment, compounds I are of formula I.B1 wherein Z is Z-266 and Q is Q2, and R and R⁴ are as per any row of Table U below, which compounds are named I.B1-Z266-Q2-U-1 to I.B1-Z266-Q2-U-42.

In another embodiment, compounds I are of formula I.B1 wherein Z is Z-266 and Q is Q3, and R and R⁴ are as per any row of Table U below, which compounds are named I.B1-Z266-Q3-U-1 to I.B1-Z266-Q3-U-42.

In another embodiment, compounds I are of formula I.B1 wherein Z is Z-266 and Q is Q4, and R and R⁴ are as per any row of Table U below, which compounds are named I.B1-Z266-Q4-U-1 to I.B1-Z266-Q4-U-42.

In another embodiment, compounds I are of formula I.B2 wherein Z is Z-266 and Q is Q1, and R and R⁴ are as per any row of Table U below, which compounds are named I.B2-Z266-Q1-U-1 to I.B2-Z266-Q1-U-42.

In another embodiment, compounds I are of formula I.B2 wherein Z is Z-266 and Q is Q2, and R and R⁴ are as per any row of Table U below, which compounds are named I.B2-Z266-Q2-U-1 to I.B2-Z266-Q2-U-42.

In another embodiment, compounds I are of formula I.B2 wherein Z is Z-266 and Q is Q3, and R and R⁴ are as per any row of Table U below, which compounds are named I.B2-Z266-Q3-U-1 to I.B2-Z266-Q3-U-42.

In another embodiment, compounds I are of formula I.B2 wherein Z is Z-266 and Q is Q4, and R and R⁴ are as per any row of Table U below, which compounds are named I.B2-Z266-Q4-U-1 to I.B2-Z266-Q4-U-42.

In another embodiment, compounds I are of formula I.B1 wherein Z is Z-267 and Q is Q1, and R and R⁴ are as per any row of Table U below, which compounds are named I.B1-Z267-Q1-U-1 to I.B1-Z267-Q1-U-42.

In another embodiment, compounds I are of formula I.B1 wherein Z is Z-267 and Q is Q2, and R and R⁴ are as per any row of Table U below, which compounds are named I.B1-Z267-Q2-U-1 to I.B1-Z267-Q2-U-42.

In another embodiment, compounds I are of formula I.B1 wherein Z is Z-267 and Q is Q3, and R and R⁴ are as per any row of Table U below, which compounds are named I.B1-Z267-Q3-U-1 to I.B1-Z267-Q3-U-42.

In another embodiment, compounds I are of formula I.B1 wherein Z is Z-267 and Q is Q4, and R and R⁴ are as per any row of Table U below, which compounds are named I.B1-Z267-Q4-U-1 to I.B1-Z267-Q4-U-42.

In another embodiment, compounds I are of formula I.B2 wherein Z is Z-267 and Q is Q1, and R and R⁴ are as per any row of Table U below, which compounds are named I.B2-Z267-Q1-U-1 to I.B2-Z267-Q1-U-42.

In another embodiment, compounds I are of formula I.B2 wherein Z is Z-267 and Q is Q2, and R and R⁴ are as per any row of Table U below, which compounds are named I.B2-Z267-Q2-U-1 to I.B2-Z267-Q2-U-42.

In another embodiment, compounds I are of formula I.B2 wherein Z is Z-267 and Q is Q3, and R and R⁴ are as per any row of Table U below, which compounds are named I.B2-Z267-Q3-U-1 to I.B2-Z267-Q3-U-42.

In another embodiment, compounds I are of formula I.B2 wherein Z is Z-267 and Q is Q4, and R and R⁴ are as per any row of Table U below, which compounds are named I.B2-Z267-Q4-U-1 to I.B2-Z267-Q4-U-42.

In another embodiment, compounds I are of formula I.B1 wherein Z is Z-268 and Q is Q1, and R and R⁴ are as per any row of Table U below, which compounds are named I.B1-Z268-Q1-U-1 to I.B1-Z268-Q1-U-42.

In another embodiment, compounds I are of formula I.B1 wherein Z is Z-268 and Q is Q2, and R and R⁴ are as per any row of Table U below, which compounds are named I.B1-Z268-Q2-U-1 to I.B1-Z268-Q2-U-42.

In another embodiment, compounds I are of formula I.B1 wherein Z is Z-268 and Q is Q3, and R and R⁴ are as per any row of Table U below, which compounds are named I.B1-Z268-Q3-U-1 to I.B1-Z268-Q3-U-42.

In another embodiment, compounds I are of formula I.B1 wherein Z is Z-268 and Q is Q4, and R and R⁴ are as per any row of Table U below, which compounds are named I.B1-Z268-Q4-U-1 to I.B1-Z268-Q4-U-42.

In another embodiment, compounds I are of formula I.B2 wherein Z is Z-268 and Q is Q1, and R and R⁴ are as per any row of Table U below, which compounds are named I.B2-Z268-Q1-U-1 to I.B2-Z268-Q1-U-42.

In another embodiment, compounds I are of formula I.B2 wherein Z is Z-268 and Q is Q2, and R and R⁴ are as per any row of Table U below, which compounds are named I.B2-Z268-Q2-U-1 to I.B2-Z268-Q2-U-42.

In another embodiment, compounds I are of formula I.B2 wherein Z is Z-268 and Q is Q3, and R and R⁴ are as per any row of Table U below, which compounds are named I.B2-Z268-Q3-U-1 to I.B2-Z268-Q3-U-42.

In another embodiment, compounds I are of formula I.B2 wherein Z is Z-268 and Q is Q4, and R and R⁴ are as per any row of Table U below, which compounds are named I.B2-Z268-Q4-U-1 to I.B2-Z268-Q4-U-42.

In another embodiment, compounds I are of formula I.B1 wherein Z is Z-269 and Q is Q1, and R and R⁴ are as per any row of Table U below, which compounds are named I.B1-Z269-Q1-U-1 to I.B1-Z269-Q1-U-42.

In another embodiment, compounds I are of formula I.B1 wherein Z is Z-269 and Q is Q2, and R and R⁴ are as per any row of Table U below, which compounds are named I.B1-Z269-Q2-U-1 to I.B1-Z269-Q2-U-42.

In another embodiment, compounds I are of formula I.B1 wherein Z is Z-269 and Q is Q3, and R and R⁴ are as per any row of Table U below, which compounds are named I.B1-Z269-Q3-U-1 to I.B1-Z269-Q3-U-42.

In another embodiment, compounds I are of formula I.B1 wherein Z is Z-269 and Q is Q4, and R and R⁴ are as per any row of Table U below, which compounds are named I.B1-Z269-Q4-U-1 to I.B1-Z269-Q4-U-42.

In another embodiment, compounds I are of formula I.B2 wherein Z is Z-269 and Q is Q1, and R and R⁴ are as per any row of Table U below, which compounds are named I.B2-Z269-Q1-U-1 to I.B2-Z269-Q1-U-42.

In another embodiment, compounds I are of formula I.B2 wherein Z is Z-269 and Q is Q2, and R and R⁴ are as per any row of Table U below, which compounds are named I.B2-Z269-Q2-U-1 to I.B2-Z269-Q2-U-42.

In another embodiment, compounds I are of formula I.B2 wherein Z is Z-269 and Q is Q3, and R and R⁴ are as per any row of Table U below, which compounds are named I.B2-Z269-Q3-U-1 to I.B2-Z269-Q3-U-42.

In another embodiment, compounds I are of formula I.B2 wherein Z is Z-269 and Q is Q4, and R and R⁴ are as per any row of Table U below, which compounds are named I.B2-Z269-Q4-U-1 to I.B2-Z269-Q4-U-42.

In another embodiment, compounds I are of formula I.B1 wherein Z is Z-270 and Q is Q1, and R and R⁴ are as per any row of Table U below, which compounds are named I.B1-Z270-Q1-U-1 to I.B1-Z270-Q1-U-42.

In another embodiment, compounds I are of formula I.B1 wherein Z is Z-270 and Q is Q2, and R and R⁴ are as per any row of Table U below, which compounds are named I.B1-Z270-Q2-U-1 to I.B1-Z270-Q2-U-42.

In another embodiment, compounds I are of formula I.B1 wherein Z is Z-270 and Q is Q3, and R and R⁴ are as per any row of Table U below, which compounds are named I.B1-Z270-Q3-U-1 to I.B1-Z270-Q3-U-42.

In another embodiment, compounds I are of formula I.B1 wherein Z is Z-270 and Q is Q4, and R and R⁴ are as per any row of Table U below, which compounds are named I.B1-Z270-Q4-U-1 to I.B1-Z270-Q4-U-42.

In another embodiment, compounds I are of formula I.B2 wherein Z is Z-270 and Q is Q1, and R and R⁴ are as per any row of Table U below, which compounds are named I.B2-Z270-Q1-U-1 to I.B2-Z270-Q1-U-42.

In another embodiment, compounds I are of formula I.B2 wherein Z is Z-270 and Q is Q2, and R and R⁴ are as per any row of Table U below, which compounds are named I.B2-Z270-Q2-U-1 to I.B2-Z270-Q2-U-42.

In another embodiment, compounds I are of formula I.B2 wherein Z is Z-270 and Q is Q3, and R and R⁴ are as per any row of Table U below, which compounds are named I.B2-Z270-Q3-U-1 to I.B2-Z270-Q3-U-42.

In another embodiment, compounds I are of formula I.B2 wherein Z is Z-270 and Q is Q4, and R and R⁴ are as per any row of Table U below, which compounds are named I.B2-Z270-Q4-U-1 to I.B2-Z270-Q4-U-42.

In another embodiment, compounds I are of formula I.B1 wherein Z is Z-271 and Q is Q1, and R and R⁴ are as per any row of Table U below, which compounds are named I.B1-Z271-Q1-U-1 to I.B1-Z271-Q1-U-42.

In another embodiment, compounds I are of formula I.B1 wherein Z is Z-271 and Q is Q2, and R and R⁴ are as per any row of Table U below, which compounds are named I.B1-Z271-Q2-U-1 to I.B1-Z271-Q2-U-42.

In another embodiment, compounds I are of formula I.B1 wherein Z is Z-271 and Q is Q3, and R and R⁴ are as per any row of Table U below, which compounds are named I.B1-Z271-Q3-U-1 to I.B1-Z271-Q3-U-42.

In another embodiment, compounds I are of formula I.B1 wherein Z is Z-271 and Q is Q4, and R and R⁴ are as per any row of Table U below, which compounds are named I.B1-Z271-Q4-U-1 to I.B1-Z271-Q4-U-42.

In another embodiment, compounds I are of formula I.B2 wherein Z is Z-271 and Q is Q1, and R and R⁴ are as per any row of Table U below, which compounds are named I.B2-Z271-Q1-U-1 to I.B2-Z271-Q1-U-42.

In another embodiment, compounds I are of formula I.B2 wherein Z is Z-271 and Q is Q2, and R and R⁴ are as per any row of Table U below, which compounds are named I.B2-Z271-Q2-U-1 to I.B2-Z271-Q2-U-42.

In another embodiment, compounds I are of formula I.B2 wherein Z is Z-271 and Q is Q3, and R and R⁴ are as per any row of Table U below, which compounds are named I.B2-Z271-Q3-U-1 to I.B2-Z271-Q3-U-42.

In another embodiment, compounds I are of formula I.B2 wherein Z is Z-271 and Q is Q4, and R and R⁴ are as per any row of Table U below, which compounds are named I.B2-Z271-Q4-U-1 to I.B2-Z271-Q4-U-42.

In another embodiment, compounds I are of formula I.B1 wherein Z is Z-272 and Q is Q1, and R and R⁴ are as per any row of Table U below, which compounds are named I.B1-Z272-Q1-U-1 to I.B1-Z272-Q1-U-42.

In another embodiment, compounds I are of formula I.B1 wherein Z is Z-272 and Q is Q2, and R and R⁴ are as per any row of Table U below, which compounds are named I.B1-Z272-Q2-U-1 to I.B1-Z272-Q2-U-42.

In another embodiment, compounds I are of formula I.B1 wherein Z is Z-272 and Q is Q3, and R and R⁴ are as per any row of Table U below, which compounds are named I.B1-Z272-Q3-U-1 to I.B1-Z272-Q3-U-42.

In another embodiment, compounds I are of formula I.B1 wherein Z is Z-272 and Q is Q4, and R and R⁴ are as per any row of Table U below, which compounds are named I.B1-Z272-Q4-U-1 to I.B1-Z272-Q4-U-42.

In another embodiment, compounds I are of formula I.B2 wherein Z is Z-272 and Q is Q1, and R and R⁴ are as per any row of Table U below, which compounds are named I.B2-Z272-Q1-U-1 to I.B2-Z272-Q1-U-42.

In another embodiment, compounds I are of formula I.B2 wherein Z is Z-272 and Q is Q2, and R and R⁴ are as per any row of Table U below, which compounds are named I.B2-Z272-Q2-U-1 to I.B2-Z272-Q2-U-42.

In another embodiment, compounds I are of formula I.B2 wherein Z is Z-272 and Q is Q3, and R and R⁴ are as per any row of Table U below, which compounds are named I.B2-Z272-Q3-U-1 to I.B2-Z272-Q3-U-42.

In another embodiment, compounds I are of formula I.B2 wherein Z is Z-272 and Q is Q4, and R and R⁴ are as per any row of Table U below, which compounds are named I.B2-Z272-Q4-U-1 to I.B2-Z272-Q4-U-42.

In another embodiment, compounds I are of formula I.B1 wherein Z is Z-273 and Q is Q1, and R and R⁴ are as per any row of Table U below, which compounds are named I.B1-Z273-Q1-U-1 to I.B1-Z273-Q1-U-42.

In another embodiment, compounds I are of formula I.B1 wherein Z is Z-273 and Q is Q2, and R and R⁴ are as per any row of Table U below, which compounds are named I.B1-Z273-Q2-U-1 to I.B1-Z273-Q2-U-42.

In another embodiment, compounds I are of formula I.B1 wherein Z is Z-273 and Q is Q3, and R and R⁴ are as per any row of Table U below, which compounds are named I.B1-Z273-Q3-U-1 to I.B1-Z273-Q3-U-42.

In another embodiment, compounds I are of formula I.B1 wherein Z is Z-273 and Q is Q4, and R and R⁴ are as per any row of Table U below, which compounds are named I.B1-Z273-Q4-U-1 to I.B1-Z273-Q4-U-42.

In another embodiment, compounds I are of formula I.B2 wherein Z is Z-273 and Q is Q1, and R and R⁴ are as per any row of Table U below, which compounds are named I.B2-Z273-Q1-U-1 to I.B2-Z273-Q1-U-42.

In another embodiment, compounds I are of formula I.B2 wherein Z is Z-273 and Q is Q2, and R and R⁴ are as per any row of Table U below, which compounds are named I.B2-Z273-Q2-U-1 to I.B2-Z273-Q2-U-42.

In another embodiment, compounds I are of formula I.B2 wherein Z is Z-273 and Q is Q3, and R and R⁴ are as per any row of Table U below, which compounds are named I.B2-Z273-Q3-U-1 to I.B2-Z273-Q3-U-42.

In another embodiment, compounds I are of formula I.B2 wherein Z is Z-273 and Q is Q4, and R and R⁴ are as per any row of Table U below, which compounds are named I.B2-Z273-Q4-U-1 to I.B2-Z273-Q4-U-42.

In another embodiment, compounds I are of formula I.B1 wherein Z is Z-274 and Q is Q1, and R and R⁴ are as per any row of Table U below, which compounds are named I.B1-Z274-Q1-U-1 to I.B1-Z274-Q1-U-42.

In another embodiment, compounds I are of formula I.B1 wherein Z is Z-274 and Q is Q2, and R and R⁴ are as per any row of Table U below, which compounds are named I.B1-Z274-Q2-U-1 to I.B1-Z274-Q2-U-42.

In another embodiment, compounds I are of formula I.B1 wherein Z is Z-274 and Q is Q3, and R and R⁴ are as per any row of Table U below, which compounds are named I.B1-Z274-Q3-U-1 to I.B1-Z274-Q3-U-42.

In another embodiment, compounds I are of formula I.B1 wherein Z is Z-274 and Q is Q4, and R and R⁴ are as per any row of Table U below, which compounds are named I.B1-Z274-Q4-U-1 to I.B1-Z274-Q4-U-42.

In another embodiment, compounds I are of formula I.B2 wherein Z is Z-274 and Q is Q1, and R and R⁴ are as per any row of Table U below, which compounds are named I.B2-Z274-Q1-U-1 to I.B2-Z274-Q1-U-42.

In another embodiment, compounds I are of formula I.B2 wherein Z is Z-274 and Q is Q2, and R and R⁴ are as per any row of Table U below, which compounds are named I.B2-Z274-Q2-U-1 to I.B2-Z274-Q2-U-42.

In another embodiment, compounds I are of formula I.B2 wherein Z is Z-274 and Q is Q3, and R and R⁴ are as per any row of Table U below, which compounds are named I.B2-Z274-Q3-U-1 to I.B2-Z274-Q3-U-42.

In another embodiment, compounds I are of formula I.B2 wherein Z is Z-274 and Q is Q4, and R and R⁴ are as per any row of Table U below, which compounds are named I.B2-Z274-Q4-U-1 to I.B2-Z274-Q4-U-42.

In another embodiment, compounds I are of formula I.B1 wherein Z is Z-275 and Q is Q1, and R and R⁴ are as per any row of Table U below, which compounds are named I.B1-Z275-Q1-U-1 to I.B1-Z275-Q1-U-42.

In another embodiment, compounds I are of formula I.B1 wherein Z is Z-275 and Q is Q2, and R and R⁴ are as per any row of Table U below, which compounds are named I.B1-Z275-Q2-U-1 to I.B1-Z275-Q2-U-42.

In another embodiment, compounds I are of formula I.B1 wherein Z is Z-275 and Q is Q3, and R and R⁴ are as per any row of Table U below, which compounds are named I.B1-Z275-Q3-U-1 to I.B1-Z275-Q3-U-42.

In another embodiment, compounds I are of formula I.B1 wherein Z is Z-275 and Q is Q4, and R and R⁴ are as per any row of Table U below, which compounds are named I.B1-Z275-Q4-U-1 to I.B1-Z275-Q4-U-42.

In another embodiment, compounds I are of formula I.B2 wherein Z is Z-275 and Q is Q1, and R and R⁴ are as per any row of Table U below, which compounds are named I.B2-Z275-Q1-U-1 to I.B2-Z275-Q1-U-42.

In another embodiment, compounds I are of formula I.B2 wherein Z is Z-275 and Q is Q2, and R and R⁴ are as per any row of Table U below, which compounds are named I.B2-Z275-Q2-U-1 to I.B2-Z275-Q2-U-42.

In another embodiment, compounds I are of formula I.B2 wherein Z is Z-275 and Q is Q3, and R and R⁴ are as per any row of Table U below, which compounds are named I.B2-Z275-Q3-U-1 to I.B2-Z275-Q3-U-42.

In another embodiment, compounds I are of formula I.B2 wherein Z is Z-275 and Q is Q4, and R and R⁴ are as per any row of Table U below, which compounds are named I.B2-Z275-Q4-U-1 to I.B2-Z275-Q4-U-42.

In another embodiment, compounds I are of formula I.B1 wherein Z is Z-276 and Q is Q1, and R and R⁴ are as per any row of Table U below, which compounds are named I.B1-Z276-Q1-U-1 to I.B1-Z276-Q1-U-42.

In another embodiment, compounds I are of formula I.B1 wherein Z is Z-276 and Q is Q2, and R and R⁴ are as per any row of Table U below, which compounds are named I.B1-Z276-Q2-U-1 to I.B1-Z276-Q2-U-42.

In another embodiment, compounds I are of formula I.B1 wherein Z is Z-276 and Q is Q3, and R and R⁴ are as per any row of Table U below, which compounds are named I.B1-Z276-Q3-U-1 to I.B1-Z276-Q3-U-42.

In another embodiment, compounds I are of formula I.B1 wherein Z is Z-276 and Q is Q4, and R and R⁴ are as per any row of Table U below, which compounds are named I.B1-Z276-Q4-U-1 to I.B1-Z276-Q4-U-42.

In another embodiment, compounds I are of formula I.B2 wherein Z is Z-276 and Q is Q1, and R and R⁴ are as per any row of Table U below, which compounds are named I.B2-Z276-Q1-U-1 to I.B2-Z276-Q1-U-42.

In another embodiment, compounds I are of formula I.B2 wherein Z is Z-276 and Q is Q2, and R and R⁴ are as per any row of Table U below, which compounds are named I.B2-Z276-Q2-U-1 to I.B2-Z276-Q2-U-42.

In another embodiment, compounds I are of formula I.B2 wherein Z is Z-276 and Q is Q3, and R and R⁴ are as per any row of Table U below, which compounds are named I.B2-Z276-Q3-U-1 to I.B2-Z276-Q3-U-42.

In another embodiment, compounds I are of formula I.B2 wherein Z is Z-276 and Q is Q4, and R and R⁴ are as per any row of Table U below, which compounds are named I.B2-Z276-Q4-U-1 to I.B2-Z276-Q4-U-42.

In another embodiment, compounds I are of formula I.B1 wherein Z is Z-277 and Q is Q1, and R and R⁴ are as per any row of Table U below, which compounds are named I.B1-Z277-Q1-U-1 to I.B1-Z277-Q1-U-42.

In another embodiment, compounds I are of formula I.B1 wherein Z is Z-277 and Q is Q2, and R and R⁴ are as per any row of Table U below, which compounds are named I.B1-Z277-Q2-U-1 to I.B1-Z277-Q2-U-42.

In another embodiment, compounds I are of formula I.B1 wherein Z is Z-277 and Q is Q3, and R and R⁴ are as per any row of Table U below, which compounds are named I.B1-Z277-Q3-U-1 to I.B1-Z277-Q3-U-42.

In another embodiment, compounds I are of formula I.B1 wherein Z is Z-277 and Q is Q4, and R and R⁴ are as per any row of Table U below, which compounds are named I.B1-Z277-Q4-U-1 to I.B1-Z277-Q4-U-42.

In another embodiment, compounds I are of formula I.B2 wherein Z is Z-277 and Q is Q1, and R and R⁴ are as per any row of Table U below, which compounds are named I.B2-Z277-Q1-U-1 to I.B2-Z277-Q1-U-42.

In another embodiment, compounds I are of formula I.B2 wherein Z is Z-277 and Q is Q2, and R and R⁴ are as per any row of Table U below, which compounds are named I.B2-Z277-Q2-U-1 to I.B2-Z277-Q2-U-42.

In another embodiment, compounds I are of formula I.B2 wherein Z is Z-277 and Q is Q3, and R and R⁴ are as per any row of Table U below, which compounds are named I.B2-Z277-Q3-U-1 to I.B2-Z277-Q3-U-42.

In another embodiment, compounds I are of formula I.B2 wherein Z is Z-277 and Q is Q4, and R and R⁴ are as per any row of Table U below, which compounds are named I.B2-Z277-Q4-U-1 to I.B2-Z277-Q4-U-42.

In another embodiment, compounds I are of formula I.B1 wherein Z is Z-278 and Q is Q1, and R and R⁴ are as per any row of Table U below, which compounds are named I.B1-Z278-Q1-U-1 to I.B1-Z278-Q1-U-42.

In another embodiment, compounds I are of formula I.B1 wherein Z is Z-278 and Q is Q2, and R and R⁴ are as per any row of Table U below, which compounds are named I.B1-Z278-Q2-U-1 to I.B1-Z278-Q2-U-42.

In another embodiment, compounds I are of formula I.B1 wherein Z is Z-278 and Q is Q3, and R and R⁴ are as per any row of Table U below, which compounds are named I.B1-Z278-Q3-U-1 to I.B1-Z278-Q3-U-42.

In another embodiment, compounds I are of formula I.B1 wherein Z is Z-278 and Q is Q4, and R and R⁴ are as per any row of Table U below, which compounds are named I.B1-Z278-Q4-U-1 to I.B1-Z278-Q4-U-42.

In another embodiment, compounds I are of formula I.B2 wherein Z is Z-278 and Q is Q1, and R and R⁴ are as per any row of Table U below, which compounds are named I.B2-Z278-Q1-U-1 to I.B2-Z278-Q1-U-42.

In another embodiment, compounds I are of formula I.B2 wherein Z is Z-278 and Q is Q2, and R and R⁴ are as per any row of Table U below, which compounds are named I.B2-Z278-Q2-U-1 to I.B2-Z278-Q2-U-42.

In another embodiment, compounds I are of formula I.B2 wherein Z is Z-278 and Q is Q3, and R and R⁴ are as per any row of Table U below, which compounds are named I.B2-Z278-Q3-U-1 to I.B2-Z278-Q3-U-42.

In another embodiment, compounds I are of formula I.B2 wherein Z is Z-278 and Q is Q4, and R and R⁴ are as per any row of Table U below, which compounds are named I.B2-Z278-Q4-U-1 to I.B2-Z278-Q4-U-42.

In another embodiment, compounds I are of formula I.B1 wherein Z is Z-279 and Q is Q1, and R and R⁴ are as per any row of Table U below, which compounds are named I.B1-Z279-Q1-U-1 to I.B1-Z279-Q1-U-42.

In another embodiment, compounds I are of formula I.B1 wherein Z is Z-279 and Q is Q2, and R and R⁴ are as per any row of Table U below, which compounds are named I.B1-Z279-Q2-U-1 to I.B1-Z279-Q2-U-42.

In another embodiment, compounds I are of formula I.B1 wherein Z is Z-279 and Q is Q3, and R and R⁴ are as per any row of Table U below, which compounds are named I.B1-Z279-Q3-U-1 to I.B1-Z279-Q3-U-42.

In another embodiment, compounds I are of formula I.B1 wherein Z is Z-279 and Q is Q4, and R and R⁴ are as per any row of Table U below, which compounds are named I.B1-Z279-Q4-U-1 to I.B1-Z279-Q4-U-42.

In another embodiment, compounds I are of formula I.B2 wherein Z is Z-279 and Q is Q1, and R and R⁴ are as per any row of Table U below, which compounds are named I.B2-Z279-Q1-U-1 to I.B2-Z279-Q1-U-42.

In another embodiment, compounds I are of formula I.B2 wherein Z is Z-279 and Q is Q2, and R and R⁴ are as per any row of Table U below, which compounds are named I.B2-Z279-Q2-U-1 to I.B2-Z279-Q2-U-42.

In another embodiment, compounds I are of formula I.B2 wherein Z is Z-279 and Q is Q3, and R and R⁴ are as per any row of Table U below, which compounds are named I.B2-Z279-Q3-U-1 to I.B2-Z279-Q3-U-42.

In another embodiment, compounds I are of formula I.B2 wherein Z is Z-279 and Q is Q4, and R and R⁴ are as per any row of Table U below, which compounds are named I.B2-Z279-Q4-U-1 to I.B2-Z279-Q4-U-42.

In another embodiment, compounds I are of formula I.B1 wherein Z is Z-280 and Q is Q1, and R and R⁴ are as per any row of Table U below, which compounds are named I.B1-Z280-Q1-U-1 to I.B1-Z280-Q1-U-42.

In another embodiment, compounds I are of formula I.B1 wherein Z is Z-280 and Q is Q2, and R and R⁴ are as per any row of Table U below, which compounds are named I.B1-Z280-Q2-U-1 to I.B1-Z280-Q2-U-42.

In another embodiment, compounds I are of formula I.B1 wherein Z is Z-280 and Q is Q3, and R and R⁴ are as per any row of Table U below, which compounds are named I.B1-Z280-Q3-U-1 to I.B1-Z280-Q3-U-42.

In another embodiment, compounds I are of formula I.B1 wherein Z is Z-280 and Q is Q4, and R and R⁴ are as per any row of Table U below, which compounds are named I.B1-Z280-Q4-U-1 to I.B1-Z280-Q4-U-42.

In another embodiment, compounds I are of formula I.B2 wherein Z is Z-280 and Q is Q1, and R and R⁴ are as per any row of Table U below, which compounds are named I.B2-Z280-Q1-U-1 to I.B2-Z280-Q1-U-42.

In another embodiment, compounds I are of formula I.B2 wherein Z is Z-280 and Q is Q2, and R and R⁴ are as per any row of Table U below, which compounds are named I.B2-Z280-Q2-U-1 to I.B2-Z280-Q2-U-42.

In another embodiment, compounds I are of formula I.B2 wherein Z is Z-280 and Q is Q3, and R and R⁴ are as per any row of Table U below, which compounds are named I.B2-Z280-Q3-U-1 to I.B2-Z280-Q3-U-42.

In another embodiment, compounds I are of formula I.B2 wherein Z is Z-280 and Q is Q4, and R and R⁴ are as per any row of Table U below, which compounds are named I.B2-Z280-Q4-U-1 to I.B2-Z280-Q4-U-42.

In another embodiment, compounds I are of formula I.B1 wherein Z is Z-281 and Q is Q1, and R and R⁴ are as per any row of Table U below, which compounds are named I.B1-Z281-Q1-U-1 to I.B1-Z281-Q1-U-42.

In another embodiment, compounds I are of formula I.B1 wherein Z is Z-281 and Q is Q2, and R and R⁴ are as per any row of Table U below, which compounds are named I.B1-Z281-Q2-U-1 to I.B1-Z281-Q2-U-42.

In another embodiment, compounds I are of formula I.B1 wherein Z is Z-281 and Q is Q3, and R and R⁴ are as per any row of Table U below, which compounds are named I.B1-Z281-Q3-U-1 to I.B1-Z281-Q3-U-42.

In another embodiment, compounds I are of formula I.B1 wherein Z is Z-281 and Q is Q4, and R and R⁴ are as per any row of Table U below, which compounds are named I.B1-Z281-Q4-U-1 to I.B1-Z281-Q4-U-42.

In another embodiment, compounds I are of formula I.B2 wherein Z is Z-281 and Q is Q1, and R and R⁴ are as per any row of Table U below, which compounds are named I.B2-Z281-Q1-U-1 to I.B2-Z281-Q1-U-42.

In another embodiment, compounds I are of formula I.B2 wherein Z is Z-281 and Q is Q2, and R and R⁴ are as per any row of Table U below, which compounds are named I.B2-Z281-Q2-U-1 to I.B2-Z281-Q2-U-42.

In another embodiment, compounds I are of formula I.B2 wherein Z is Z-281 and Q is Q3, and R and R⁴ are as per any row of Table U below, which compounds are named I.B2-Z281-Q3-U-1 to I.B2-Z281-Q3-U-42.

In another embodiment, compounds I are of formula I.B2 wherein Z is Z-281 and Q is Q4, and R and R⁴ are as per any row of Table U below, which compounds are named I.B2-Z281-Q4-U-1 to I.B2-Z281-Q4-U-42.

In another embodiment, compounds I are of formula I.B1 wherein Z is Z-282 and Q is Q1, and R and R⁴ are as per any row of Table U below, which compounds are named I.B1-Z282-Q1-U-1 to I.B1-Z282-Q1-U-42.

In another embodiment, compounds I are of formula I.B1 wherein Z is Z-282 and Q is Q2, and R and R⁴ are as per any row of Table U below, which compounds are named I.B1-Z282-Q2-U-1 to I.B1-Z282-Q2-U-42.

In another embodiment, compounds I are of formula I.B1 wherein Z is Z-282 and Q is Q3, and R and R⁴ are as per any row of Table U below, which compounds are named I.B1-Z282-Q3-U-1 to I.B1-Z282-Q3-U-42.

In another embodiment, compounds I are of formula I.B1 wherein Z is Z-282 and Q is Q4, and R and R⁴ are as per any row of Table U below, which compounds are named I.B1-Z282-Q4-U-1 to I.B1-Z282-Q4-U-42.

In another embodiment, compounds I are of formula I.B2 wherein Z is Z-282 and Q is Q1, and R and R⁴ are as per any row of Table U below, which compounds are named I.B2-Z282-Q1-U-1 to I.B2-Z282-Q1-U-42.

In another embodiment, compounds I are of formula I.B2 wherein Z is Z-282 and Q is Q2, and R and R⁴ are as per any row of Table U below, which compounds are named I.B2-Z282-Q2-U-1 to I.B2-Z282-Q2-U-42.

In another embodiment, compounds I are of formula I.B2 wherein Z is Z-282 and Q is Q3, and R and R⁴ are as per any row of Table U below, which compounds are named I.B2-Z282-Q3-U-1 to I.B2-Z282-Q3-U-42.

In another embodiment, compounds I are of formula I.B2 wherein Z is Z-282 and Q is Q4, and R and R⁴ are as per any row of Table U below, which compounds are named I.B2-Z282-Q4-U-1 to I.B2-Z282-Q4-U-42.

In another embodiment, compounds I are of formula I.B1 wherein Z is Z-283 and Q is Q1, and R and R⁴ are as per any row of Table U below, which compounds are named I.B1-Z283-Q1-U-1 to I.B1-Z283-Q1-U-42.

In another embodiment, compounds I are of formula I.B1 wherein Z is Z-283 and Q is Q2, and R and R⁴ are as per any row of Table U below, which compounds are named I.B1-Z283-Q2-U-1 to I.B1-Z283-Q2-U-42.

In another embodiment, compounds I are of formula I.B1 wherein Z is Z-283 and Q is Q3, and R and R⁴ are as per any row of Table U below, which compounds are named I.B1-Z283-Q3-U-1 to I.B1-Z283-Q3-U-42.

In another embodiment, compounds I are of formula I.B1 wherein Z is Z-283 and Q is Q4, and R and R⁴ are as per any row of Table U below, which compounds are named I.B1-Z283-Q4-U-1 to I.B1-Z283-Q4-U-42.

In another embodiment, compounds I are of formula I.B2 wherein Z is Z-283 and Q is Q1, and R and R⁴ are as per any row of Table U below, which compounds are named I.B2-Z283-Q1-U-1 to I.B2-Z283-Q1-U-42.

In another embodiment, compounds I are of formula I.B2 wherein Z is Z-283 and Q is Q2, and R and R⁴ are as per any row of Table U below, which compounds are named I.B2-Z283-Q2-U-1 to I.B2-Z283-Q2-U-42.

In another embodiment, compounds I are of formula I.B2 wherein Z is Z-283 and Q is Q3, and R and R⁴ are as per any row of Table U below, which compounds are named I.B2-Z283-Q3-U-1 to I.B2-Z283-Q3-U-42.

In another embodiment, compounds I are of formula I.B2 wherein Z is Z-283 and Q is Q4, and R and R⁴ are as per any row of Table U below, which compounds are named I.B2-Z283-Q4-U-1 to I.B2-Z283-Q4-U-42.

In another embodiment, compounds I are of formula I.B1 wherein Z is Z-284 and Q is Q1, and R and R⁴ are as per any row of Table U below, which compounds are named I.B1-Z284-Q1-U-1 to I.B1-Z284-Q1-U-42.

In another embodiment, compounds I are of formula I.B1 wherein Z is Z-284 and Q is Q2, and R and R⁴ are as per any row of Table U below, which compounds are named I.B1-Z284-Q2-U-1 to I.B1-Z284-Q2-U-42.

In another embodiment, compounds I are of formula I.B1 wherein Z is Z-284 and Q is Q3, and R and R⁴ are as per any row of Table U below, which compounds are named I.B1-Z284-Q3-U-1 to I.B1-Z284-Q3-U-42.

In another embodiment, compounds I are of formula I.B1 wherein Z is Z-284 and Q is Q4, and R and R⁴ are as per any row of Table U below, which compounds are named I.B1-Z284-Q4-U-1 to I.B1-Z284-Q4-U-42.

In another embodiment, compounds I are of formula I.B2 wherein Z is Z-284 and Q is Q1, and R and R⁴ are as per any row of Table U below, which compounds are named I.B2-Z284-Q1-U-1 to I.B2-Z284-Q1-U-42.

In another embodiment, compounds I are of formula I.B2 wherein Z is Z-284 and Q is Q2, and R and R⁴ are as per any row of Table U below, which compounds are named I.B2-Z284-Q2-U-1 to I.B2-Z284-Q2-U-42.

In another embodiment, compounds I are of formula I.B2 wherein Z is Z-284 and Q is Q3, and R and R⁴ are as per any row of Table U below, which compounds are named I.B2-Z284-Q3-U-1 to I.B2-Z284-Q3-U-42.

In another embodiment, compounds I are of formula I.B2 wherein Z is Z-284 and Q is Q4, and R and R⁴ are as per any row of Table U below, which compounds are named I.B2-Z284-Q4-U-1 to I.B2-Z284-Q4-U-42.

In another embodiment, compounds I are of formula I.B1 wherein Z is Z-285 and Q is Q1, and R and R⁴ are as per any row of Table U below, which compounds are named I.B1-Z285-Q1-U-1 to I.B1-Z285-Q1-U-42.

In another embodiment, compounds I are of formula I.B1 wherein Z is Z-285 and Q is Q2, and R and R⁴ are as per any row of Table U below, which compounds are named I.B1-Z285-Q2-U-1 to I.B1-Z285-Q2-U-42.

In another embodiment, compounds I are of formula I.B1 wherein Z is Z-285 and Q is Q3, and R and R⁴ are as per any row of Table U below, which compounds are named I.B1-Z285-Q3-U-1 to I.B1-Z285-Q3-U-42.

In another embodiment, compounds I are of formula I.B1 wherein Z is Z-285 and Q is Q4, and R and R⁴ are as per any row of Table U below, which compounds are named I.B1-Z285-Q4-U-1 to I.B1-Z285-Q4-U-42.

In another embodiment, compounds I are of formula I.B2 wherein Z is Z-285 and Q is Q1, and R and R⁴ are as per any row of Table U below, which compounds are named I.B2-Z285-Q1-U-1 to I.B2-Z285-Q1-U-42.

In another embodiment, compounds I are of formula I.B2 wherein Z is Z-285 and Q is Q2, and R and R⁴ are as per any row of Table U below, which compounds are named I.B2-Z285-Q2-U-1 to I.B2-Z285-Q2-U-42.

In another embodiment, compounds I are of formula I.B2 wherein Z is Z-285 and Q is Q3, and R and R⁴ are as per any row of Table U below, which compounds are named I.B2-Z285-Q3-U-1 to I.B2-Z285-Q3-U-42.

In another embodiment, compounds I are of formula I.B2 wherein Z is Z-285 and Q is Q4, and R and R⁴ are as per any row of Table U below, which compounds are named I.B2-Z285-Q4-U-1 to I.B2-Z285-Q4-U-42.

In another embodiment, compounds I are of formula I.B1 wherein Z is Z-286 and Q is Q1, and R and R⁴ are as per any row of Table U below, which compounds are named I.B1-Z286-Q1-U-1 to I.B1-Z286-Q1-U-42.

In another embodiment, compounds I are of formula I.B1 wherein Z is Z-286 and Q is Q2, and R and R⁴ are as per any row of Table U below, which compounds are named I.B1-Z286-Q2-U-1 to I.B1-Z286-Q2-U-42.

In another embodiment, compounds I are of formula I.B1 wherein Z is Z-286 and Q is Q3, and R and R⁴ are as per any row of Table U below, which compounds are named I.B1-Z286-Q3-U-1 to I.B1-Z286-Q3-U-42.

In another embodiment, compounds I are of formula I.B1 wherein Z is Z-286 and Q is Q4, and R and R⁴ are as per any row of Table U below, which compounds are named I.B1-Z286-Q4-U-1 to I.B1-Z286-Q4-U-42.

In another embodiment, compounds I are of formula I.B2 wherein Z is Z-286 and Q is Q1, and R and R⁴ are as per any row of Table U below, which compounds are named I.B2-Z286-Q1-U-1 to I.B2-Z286-Q1-U-42.

In another embodiment, compounds I are of formula I.B2 wherein Z is Z-286 and Q is Q2, and R and R⁴ are as per any row of Table U below, which compounds are named I.B2-Z286-Q2-U-1 to I.B2-Z286-Q2-U-42.

In another embodiment, compounds I are of formula I.B2 wherein Z is Z-286 and Q is Q3, and R and R⁴ are as per any row of Table U below, which compounds are named I.B2-Z286-Q3-U-1 to I.B2-Z286-Q3-U-42.

In another embodiment, compounds I are of formula I.B2 wherein Z is Z-286 and Q is Q4, and R and R⁴ are as per any row of Table U below, which compounds are named I.B2-Z286-Q4-U-1 to I.B2-Z286-Q4-U-42.

In another embodiment, compounds I are of formula I.B1 wherein Z is Z-287 and Q is Q1, and R and R⁴ are as per any row of Table U below, which compounds are named I.B1-Z287-Q1-U-1 to I.B1-Z287-Q1-U-42.

In another embodiment, compounds I are of formula I.B1 wherein Z is Z-287 and Q is Q2, and R and R⁴ are as per any row of Table U below, which compounds are named I.B1-Z287-Q2-U-1 to I.B1-Z287-Q2-U-42.

In another embodiment, compounds I are of formula I.B1 wherein Z is Z-287 and Q is Q3, and R and R⁴ are as per any row of Table U below, which compounds are named I.B1-Z287-Q3-U-1 to I.B1-Z287-Q3-U-42.

In another embodiment, compounds I are of formula I.B1 wherein Z is Z-287 and Q is Q4, and R and R⁴ are as per any row of Table U below, which compounds are named I.B1-Z287-Q4-U-1 to I.B1-Z287-Q4-U-42.

In another embodiment, compounds I are of formula I.B2 wherein Z is Z-287 and Q is Q1, and R and R⁴ are as per any row of Table U below, which compounds are named I.B2-Z287-Q1-U-1 to I.B2-Z287-Q1-U-42.

In another embodiment, compounds I are of formula I.B2 wherein Z is Z-287 and Q is Q2, and R and R⁴ are as per any row of Table U below, which compounds are named I.B2-Z287-Q2-U-1 to I.B2-Z287-Q2-U-42.

In another embodiment, compounds I are of formula I.B2 wherein Z is Z-287 and Q is Q3, and R and R⁴ are as per any row of Table U below, which compounds are named I.B2-Z287-Q3-U-1 to I.B2-Z287-Q3-U-42.

In another embodiment, compounds I are of formula I.B2 wherein Z is Z-287 and Q is Q4, and R and R⁴ are as per any row of Table U below, which compounds are named I.B2-Z287-Q4-U-1 to I.B2-Z287-Q4-U-42.

In another embodiment, compounds I are of formula I.B1 wherein Z is Z-288 and Q is Q1, and R and R⁴ are as per any row of Table U below, which compounds are named I.B1-Z288-Q1-U-1 to I.B1-Z288-Q1-U-42.

In another embodiment, compounds I are of formula I.B1 wherein Z is Z-288 and Q is Q2, and R and R⁴ are as per any row of Table U below, which compounds are named I.B1-Z288-Q2-U-1 to I.B1-Z288-Q2-U-42.

In another embodiment, compounds I are of formula I.B1 wherein Z is Z-288 and Q is Q3, and R and R⁴ are as per any row of Table U below, which compounds are named I.B1-Z288-Q3-U-1 to I.B1-Z288-Q3-U-42.

In another embodiment, compounds I are of formula I.B1 wherein Z is Z-288 and Q is Q4, and R and R⁴ are as per any row of Table U below, which compounds are named I.B1-Z288-Q4-U-1 to I.B1-Z288-Q4-U-42.

In another embodiment, compounds I are of formula I.B2 wherein Z is Z-288 and Q is Q1, and R and R⁴ are as per any row of Table U below, which compounds are named I.B2-Z288-Q1-U-1 to I.B2-Z288-Q1-U-42.

In another embodiment, compounds I are of formula I.B2 wherein Z is Z-288 and Q is Q2, and R and R⁴ are as per any row of Table U below, which compounds are named I.B2-Z288-Q2-U-1 to I.B2-Z288-Q2-U-42.

In another embodiment, compounds I are of formula I.B2 wherein Z is Z-288 and Q is Q3, and R and R⁴ are as per any row of Table U below, which compounds are named I.B2-Z288-Q3-U-1 to I.B2-Z288-Q3-U-42.

In another embodiment, compounds I are of formula I.B2 wherein Z is Z-288 and Q is Q4, and R and R⁴ are as per any row of Table U below, which compounds are named I.B2-Z288-Q4-U-1 to I.B2-Z288-Q4-U-42.

In another embodiment, compounds I are of formula I.B1 wherein Z is Z-289 and Q is Q1, and R and R⁴ are as per any row of Table U below, which compounds are named I.B1-Z289-Q1-U-1 to I.B1-Z289-Q1-U-42.

In another embodiment, compounds I are of formula I.B1 wherein Z is Z-289 and Q is Q2, and R and R⁴ are as per any row of Table U below, which compounds are named I.B1-Z289-Q2-U-1 to I.B1-Z289-Q2-U-42.

In another embodiment, compounds I are of formula I.B1 wherein Z is Z-289 and Q is Q3, and R and R⁴ are as per any row of Table U below, which compounds are named I.B1-Z289-Q3-U-1 to I.B1-Z289-Q3-U-42.

In another embodiment, compounds I are of formula I.B1 wherein Z is Z-289 and Q is Q4, and R and R⁴ are as per any row of Table U below, which compounds are named I.B1-Z289-Q4-U-1 to I.B1-Z289-Q4-U-42.

In another embodiment, compounds I are of formula I.B2 wherein Z is Z-289 and Q is Q1, and R and R⁴ are as per any row of Table U below, which compounds are named I.B2-Z289-Q1-U-1 to I.B2-Z289-Q1-U-42.

In another embodiment, compounds I are of formula I.B2 wherein Z is Z-289 and Q is Q2, and R and R⁴ are as per any row of Table U below, which compounds are named I.B2-Z289-Q2-U-1 to I.B2-Z289-Q2-U-42.

In another embodiment, compounds I are of formula I.B2 wherein Z is Z-289 and Q is Q3, and R and R⁴ are as per any row of Table U below, which compounds are named I.B2-Z289-Q3-U-1 to I.B2-Z289-Q3-U-42.

In another embodiment, compounds I are of formula I.B2 wherein Z is Z-289 and Q is Q4, and R and R⁴ are as per any row of Table U below, which compounds are named I.B2-Z289-Q4-U-1 to I.B2-Z289-Q4-U-42.

In another embodiment, compounds I are of formula I.B1 wherein Z is Z-290 and Q is Q1, and R and R⁴ are as per any row of Table U below, which compounds are named I.B1-Z290-Q1-U-1 to I.B1-Z290-Q1-U-42.

In another embodiment, compounds I are of formula I.B1 wherein Z is Z-290 and Q is Q2, and R and R⁴ are as per any row of Table U below, which compounds are named I.B1-Z290-Q2-U-1 to I.B1-Z290-Q2-U-42.

In another embodiment, compounds I are of formula I.B1 wherein Z is Z-290 and Q is Q3, and R and R⁴ are as per any row of Table U below, which compounds are named I.B1-Z290-Q3-U-1 to I.B1-Z290-Q3-U-42.

In another embodiment, compounds I are of formula I.B1 wherein Z is Z-290 and Q is Q4, and R and R⁴ are as per any row of Table U below, which compounds are named I.B1-Z290-Q4-U-1 to I.B1-Z290-Q4-U-42.

In another embodiment, compounds I are of formula I.B2 wherein Z is Z-290 and Q is Q1, and R and R⁴ are as per any row of Table U below, which compounds are named I.B2-Z290-Q1-U-1 to I.B2-Z290-Q1-U-42.

In another embodiment, compounds I are of formula I.B2 wherein Z is Z-290 and Q is Q2, and R and R⁴ are as per any row of Table U below, which compounds are named I.B2-Z290-Q2-U-1 to I.B2-Z290-Q2-U-42.

In another embodiment, compounds I are of formula I.B2 wherein Z is Z-290 and Q is Q3, and R and R⁴ are as per any row of Table U below, which compounds are named I.B2-Z290-Q3-U-1 to I.B2-Z290-Q3-U-42.

In another embodiment, compounds I are of formula I.B2 wherein Z is Z-290 and Q is Q4, and R and R⁴ are as per any row of Table U below, which compounds are named I.B2-Z290-Q4-U-1 to I.B2-Z290-Q4-U-42.

In another embodiment, compounds I are of formula I.B1 wherein Z is Z-291 and Q is Q1, and R and R⁴ are as per any row of Table U below, which compounds are named I.B1-Z291-Q1-U-1 to I.B1-Z291-Q1-U-42.

In another embodiment, compounds I are of formula I.B1 wherein Z is Z-291 and Q is Q2, and R and R⁴ are as per any row of Table U below, which compounds are named I.B1-Z291-Q2-U-1 to I.B1-Z291-Q2-U-42.

In another embodiment, compounds I are of formula I.B1 wherein Z is Z-291 and Q is Q3, and R and R⁴ are as per any row of Table U below, which compounds are named I.B1-Z291-Q3-U-1 to I.B1-Z291-Q3-U-42.

In another embodiment, compounds I are of formula I.B1 wherein Z is Z-291 and Q is Q4, and R and R⁴ are as per any row of Table U below, which compounds are named I.B1-Z291-Q4-U-1 to I.B1-Z291-Q4-U-42.

In another embodiment, compounds I are of formula I.B2 wherein Z is Z-291 and Q is Q1, and R and R⁴ are as per any row of Table U below, which compounds are named I.B2-Z291-Q1-U-1 to I.B2-Z291-Q1-U-42.

In another embodiment, compounds I are of formula I.B2 wherein Z is Z-291 and Q is Q2, and R and R⁴ are as per any row of Table U below, which compounds are named I.B2-Z291-Q2-U-1 to I.B2-Z291-Q2-U-42.

In another embodiment, compounds I are of formula I.B2 wherein Z is Z-291 and Q is Q3, and R and R⁴ are as per any row of Table U below, which compounds are named I.B2-Z291-Q3-U-1 to I.B2-Z291-Q3-U-42.

In another embodiment, compounds I are of formula I.B2 wherein Z is Z-291 and Q is Q4, and R and R⁴ are as per any row of Table U below, which compounds are named I.B2-Z291-Q4-U-1 to I.B2-Z291-Q4-U-42.

In another embodiment, compounds I are of formula I.B1 wherein Z is Z-292 and Q is Q1, and R and R⁴ are as per any row of Table U below, which compounds are named I.B1-Z292-Q1-U-1 to I.B1-Z292-Q1-U-42.

In another embodiment, compounds I are of formula I.B1 wherein Z is Z-292 and Q is Q2, and R and R⁴ are as per any row of Table U below, which compounds are named I.B1-Z292-Q2-U-1 to I.B1-Z292-Q2-U-42.

In another embodiment, compounds I are of formula I.B1 wherein Z is Z-292 and Q is Q3, and R and R⁴ are as per any row of Table U below, which compounds are named I.B1-Z292-Q3-U-1 to I.B1-Z292-Q3-U-42.

In another embodiment, compounds I are of formula I.B1 wherein Z is Z-292 and Q is Q4, and R and R⁴ are as per any row of Table U below, which compounds are named I.B1-Z292-Q4-U-1 to I.B1-Z292-Q4-U-42.

In another embodiment, compounds I are of formula I.B2 wherein Z is Z-292 and Q is Q1, and R and R⁴ are as per any row of Table U below, which compounds are named I.B2-Z292-Q1-U-1 to I.B2-Z292-Q1-U-42.

In another embodiment, compounds I are of formula I.B2 wherein Z is Z-292 and Q is Q2, and R and R⁴ are as per any row of Table U below, which compounds are named I.B2-Z292-Q2-U-1 to I.B2-Z292-Q2-U-42.

In another embodiment, compounds I are of formula I.B2 wherein Z is Z-292 and Q is Q3, and R and R⁴ are as per any row of Table U below, which compounds are named I.B2-Z292-Q3-U-1 to I.B2-Z292-Q3-U-42.

In another embodiment, compounds I are of formula I.B2 wherein Z is Z-292 and Q is Q4, and R and R⁴ are as per any row of Table U below, which compounds are named I.B2-Z292-Q4-U-1 to I.B2-Z292-Q4-U-42.

In another embodiment, compounds I are of formula I.B1 wherein Z is Z-293 and Q is Q1, and R and R⁴ are as per any row of Table U below, which compounds are named I.B1-Z293-Q1-U-1 to I.B1-Z293-Q1-U-42.

In another embodiment, compounds I are of formula I.B1 wherein Z is Z-293 and Q is Q2, and R and R⁴ are as per any row of Table U below, which compounds are named I.B1-Z293-Q2-U-1 to I.B1-Z293-Q2-U-42.

In another embodiment, compounds I are of formula I.B1 wherein Z is Z-293 and Q is Q3, and R and R⁴ are as per any row of Table U below, which compounds are named I.B1-Z293-Q3-U-1 to I.B1-Z293-Q3-U-42.

In another embodiment, compounds I are of formula I.B1 wherein Z is Z-293 and Q is Q4, and R and R⁴ are as per any row of Table U below, which compounds are named I.B1-Z293-Q4-U-1 to I.B1-Z293-Q4-U-42.

In another embodiment, compounds I are of formula I.B2 wherein Z is Z-293 and Q is Q1, and R and R⁴ are as per any row of Table U below, which compounds are named I.B2-Z293-Q1-U-1 to I.B2-Z293-Q1-U-42.

In another embodiment, compounds I are of formula I.B2 wherein Z is Z-293 and Q is Q2, and R and R⁴ are as per any row of Table U below, which compounds are named I.B2-Z293-Q2-U-1 to I.B2-Z293-Q2-U-42.

In another embodiment, compounds I are of formula I.B2 wherein Z is Z-293 and Q is Q3, and R and R⁴ are as per any row of Table U below, which compounds are named I.B2-Z293-Q3-U-1 to I.B2-Z293-Q3-U-42.

In another embodiment, compounds I are of formula I.B2 wherein Z is Z-293 and Q is Q4, and R and R⁴ are as per any row of Table U below, which compounds are named I.B2-Z293-Q4-U-1 to I.B2-Z293-Q4-U-42.

In another embodiment, compounds I are of formula I.B1 wherein Z is Z-294 and Q is Q1, and R and R⁴ are as per any row of Table U below, which compounds are named I.B1-Z294-Q1-U-1 to I.B1-Z294-Q1-U-42.

In another embodiment, compounds I are of formula I.B1 wherein Z is Z-294 and Q is Q2, and R and R⁴ are as per any row of Table U below, which compounds are named I.B1-Z294-Q2-U-1 to I.B1-Z294-Q2-U-42.

In another embodiment, compounds I are of formula I.B1 wherein Z is Z-294 and Q is Q3, and R and R⁴ are as per any row of Table U below, which compounds are named I.B1-Z294-Q3-U-1 to I.B1-Z294-Q3-U-42.

In another embodiment, compounds I are of formula I.B1 wherein Z is Z-294 and Q is Q4, and R and R⁴ are as per any row of Table U below, which compounds are named I.B1-Z294-Q4-U-1 to I.B1-Z294-Q4-U-42.

In another embodiment, compounds I are of formula I.B2 wherein Z is Z-294 and Q is Q1, and R and R⁴ are as per any row of Table U below, which compounds are named I.B2-Z294-Q1-U-1 to I.B2-Z294-Q1-U-42.

In another embodiment, compounds I are of formula I.B2 wherein Z is Z-294 and Q is Q2, and R and R⁴ are as per any row of Table U below, which compounds are named I.B2-Z294-Q2-U-1 to I.B2-Z294-Q2-U-42.

In another embodiment, compounds I are of formula I.B2 wherein Z is Z-294 and Q is Q3, and R and R⁴ are as per any row of Table U below, which compounds are named I.B2-Z294-Q3-U-1 to I.B2-Z294-Q3-U-42.

In another embodiment, compounds I are of formula I.B2 wherein Z is Z-294 and Q is Q4, and R and R⁴ are as per any row of Table U below, which compounds are named I.B2-Z294-Q4-U-1 to I.B2-Z294-Q4-U-42.

In another embodiment, compounds I are of formula I.B1 wherein Z is Z-295 and Q is Q1, and R and R⁴ are as per any row of Table U below, which compounds are named I.B1-Z295-Q1-U-1 to I.B1-Z295-Q1-U-42.

In another embodiment, compounds I are of formula I.B1 wherein Z is Z-295 and Q is Q2, and R and R⁴ are as per any row of Table U below, which compounds are named I.B1-Z295-Q2-U-1 to I.B1-Z295-Q2-U-42.

In another embodiment, compounds I are of formula I.B1 wherein Z is Z-295 and Q is Q3, and R and R⁴ are as per any row of Table U below, which compounds are named I.B1-Z295-Q3-U-1 to I.B1-Z295-Q3-U-42.

In another embodiment, compounds I are of formula I.B1 wherein Z is Z-295 and Q is Q4, and R and R⁴ are as per any row of Table U below, which compounds are named I.B1-Z295-Q4-U-1 to I.B1-Z295-Q4-U-42.

In another embodiment, compounds I are of formula I.B2 wherein Z is Z-295 and Q is Q1, and R and R⁴ are as per any row of Table U below, which compounds are named I.B2-Z295-Q1-U-1 to I.B2-Z295-Q1-U-42.

In another embodiment, compounds I are of formula I.B2 wherein Z is Z-295 and Q is Q2, and R and R⁴ are as per any row of Table U below, which compounds are named I.B2-Z295-Q2-U-1 to I.B2-Z295-Q2-U-42.

In another embodiment, compounds I are of formula I.B2 wherein Z is Z-295 and Q is Q3, and R and R⁴ are as per any row of Table U below, which compounds are named I.B2-Z295-Q3-U-1 to I.B2-Z295-Q3-U-42.

In another embodiment, compounds I are of formula I.B2 wherein Z is Z-295 and Q is Q4, and R and R⁴ are as per any row of Table U below, which compounds are named I.B2-Z295-Q4-U-1 to I.B2-Z295-Q4-U-42.

In another embodiment, compounds I are of formula I.B1 wherein Z is Z-296 and Q is Q1, and R and R⁴ are as per any row of Table U below, which compounds are named I.B1-Z296-Q1-U-1 to I.B1-Z296-Q1-U-42.

In another embodiment, compounds I are of formula I.B1 wherein Z is Z-296 and Q is Q2, and R and R⁴ are as per any row of Table U below, which compounds are named I.B1-Z296-Q2-U-1 to I.B1-Z296-Q2-U-42.

In another embodiment, compounds I are of formula I.B1 wherein Z is Z-296 and Q is Q3, and R and R⁴ are as per any row of Table U below, which compounds are named I.B1-Z296-Q3-U-1 to I.B1-Z296-Q3-U-42.

In another embodiment, compounds I are of formula I.B1 wherein Z is Z-296 and Q is Q4, and R and R⁴ are as per any row of Table U below, which compounds are named I.B1-Z296-Q4-U-1 to I.B1-Z296-Q4-U-42.

In another embodiment, compounds I are of formula I.B2 wherein Z is Z-296 and Q is Q1, and R and R⁴ are as per any row of Table U below, which compounds are named I.B2-Z296-Q1-U-1 to I.B2-Z296-Q1-U-42.

In another embodiment, compounds I are of formula I.B2 wherein Z is Z-296 and Q is Q2, and R and R⁴ are as per any row of Table U below, which compounds are named I.B2-Z296-Q2-U-1 to I.B2-Z296-Q2-U-42.

In another embodiment, compounds I are of formula I.B2 wherein Z is Z-296 and Q is Q3, and R and R⁴ are as per any row of Table U below, which compounds are named I.B2-Z296-Q3-U-1 to I.B2-Z296-Q3-U-42.

In another embodiment, compounds I are of formula I.B2 wherein Z is Z-296 and Q is Q4, and R and R⁴ are as per any row of Table U below, which compounds are named I.B2-Z296-Q4-U-1 to I.B2-Z296-Q4-U-42.

In another embodiment, compounds I are of formula I.B1 wherein Z is Z-297 and Q is Q1, and R and R⁴ are as per any row of Table U below, which compounds are named I.B1-Z297-Q1-U-1 to I.B1-Z297-Q1-U-42.

In another embodiment, compounds I are of formula I.B1 wherein Z is Z-297 and Q is Q2, and R and R⁴ are as per any row of Table U below, which compounds are named I.B1-Z297-Q2-U-1 to I.B1-Z297-Q2-U-42.

In another embodiment, compounds I are of formula I.B1 wherein Z is Z-297 and Q is Q3, and R and R⁴ are as per any row of Table U below, which compounds are named I.B1-Z297-Q3-U-1 to I.B1-Z297-Q3-U-42.

In another embodiment, compounds I are of formula I.B1 wherein Z is Z-297 and Q is Q4, and R and R⁴ are as per any row of Table U below, which compounds are named I.B1-Z297-Q4-U-1 to I.B1-Z297-Q4-U-42.

In another embodiment, compounds I are of formula I.B2 wherein Z is Z-297 and Q is Q1, and R and R⁴ are as per any row of Table U below, which compounds are named I.B2-Z297-Q1-U-1 to I.B2-Z297-Q1-U-42.

In another embodiment, compounds I are of formula I.B2 wherein Z is Z-297 and Q is Q2, and R and R⁴ are as per any row of Table U below, which compounds are named I.B2-Z297-Q2-U-1 to I.B2-Z297-Q2-U-42.

In another embodiment, compounds I are of formula I.B2 wherein Z is Z-297 and Q is Q3, and R and R⁴ are as per any row of Table U below, which compounds are named I.B2-Z297-Q3-U-1 to I.B2-Z297-Q3-U-42.

In another embodiment, compounds I are of formula I.B2 wherein Z is Z-297 and Q is Q4, and R and R⁴ are as per any row of Table U below, which compounds are named I.B2-Z297-Q4-U-1 to I.B2-Z297-Q4-U-42.

In another embodiment, compounds I are of formula I.B1 wherein Z is Z-298 and Q is Q1, and R and R⁴ are as per any row of Table U below, which compounds are named I.B1-Z298-Q1-U-1 to I.B1-Z298-Q1-U-42.

In another embodiment, compounds I are of formula I.B1 wherein Z is Z-298 and Q is Q2, and R and R⁴ are as per any row of Table U below, which compounds are named I.B1-Z298-Q2-U-1 to I.B1-Z298-Q2-U-42.

In another embodiment, compounds I are of formula I.B1 wherein Z is Z-298 and Q is Q3, and R and R⁴ are as per any row of Table U below, which compounds are named I.B1-Z298-Q3-U-1 to I.B1-Z298-Q3-U-42.

In another embodiment, compounds I are of formula I.B1 wherein Z is Z-298 and Q is Q4, and R and R⁴ are as per any row of Table U below, which compounds are named I.B1-Z298-Q4-U-1 to I.B1-Z298-Q4-U-42.

In another embodiment, compounds I are of formula I.B2 wherein Z is Z-298 and Q is Q1, and R and R⁴ are as per any row of Table U below, which compounds are named I.B2-Z298-Q1-U-1 to I.B2-Z298-Q1-U-42.

In another embodiment, compounds I are of formula I.B2 wherein Z is Z-298 and Q is Q2, and R and R⁴ are as per any row of Table U below, which compounds are named I.B2-Z298-Q2-U-1 to I.B2-Z298-Q2-U-42.

In another embodiment, compounds I are of formula I.B2 wherein Z is Z-298 and Q is Q3, and R and R⁴ are as per any row of Table U below, which compounds are named I.B2-Z298-Q3-U-1 to I.B2-Z298-Q3-U-42.

In another embodiment, compounds I are of formula I.B2 wherein Z is Z-298 and Q is Q4, and R and R⁴ are as per any row of Table U below, which compounds are named I.B2-Z298-Q4-U-1 to I.B2-Z298-Q4-U-42.

In another embodiment, compounds I are of formula I.B1 wherein Z is Z-299 and Q is Q1, and R and R⁴ are as per any row of Table U below, which compounds are named I.B1-Z299-Q1-U-1 to I.B1-Z299-Q1-U-42.

In another embodiment, compounds I are of formula I.B1 wherein Z is Z-299 and Q is Q2, and R and R⁴ are as per any row of Table U below, which compounds are named I.B1-Z299-Q2-U-1 to I.B1-Z299-Q2-U-42.

In another embodiment, compounds I are of formula I.B1 wherein Z is Z-299 and Q is Q3, and R and R⁴ are as per any row of Table U below, which compounds are named I.B1-Z299-Q3-U-1 to I.B1-Z299-Q3-U-42.

In another embodiment, compounds I are of formula I.B1 wherein Z is Z-299 and Q is Q4, and R and R⁴ are as per any row of Table U below, which compounds are named I.B1-Z299-Q4-U-1 to I.B1-Z299-Q4-U-42.

In another embodiment, compounds I are of formula I.B2 wherein Z is Z-299 and Q is Q1, and R and R⁴ are as per any row of Table U below, which compounds are named I.B2-Z299-Q1-U-1 to I.B2-Z299-Q1-U-42.

In another embodiment, compounds I are of formula I.B2 wherein Z is Z-299 and Q is Q2, and R and R⁴ are as per any row of Table U below, which compounds are named I.B2-Z299-Q2-U-1 to I.B2-Z299-Q2-U-42.

In another embodiment, compounds I are of formula I.B2 wherein Z is Z-299 and Q is Q3, and R and R⁴ are as per any row of Table U below, which compounds are named I.B2-Z299-Q3-U-1 to I.B2-Z299-Q3-U-42.

In another embodiment, compounds I are of formula I.B2 wherein Z is Z-299 and Q is Q4, and R and R⁴ are as per any row of Table U below, which compounds are named I.B2-Z299-Q4-U-1 to I.B2-Z299-Q4-U-42.

In another embodiment, compounds I are of formula I.B1 wherein Z is Z-300 and Q is Q1, and R and R⁴ are as per any row of Table U below, which compounds are named I.B1-Z300-Q1-U-1 to I.B1-Z300-Q1-U-42.

In another embodiment, compounds I are of formula I.B1 wherein Z is Z-300 and Q is Q2, and R and R⁴ are as per any row of Table U below, which compounds are named I.B1-Z300-Q2-U-1 to I.B1-Z300-Q2-U-42.

In another embodiment, compounds I are of formula I.B1 wherein Z is Z-300 and Q is Q3, and R and R⁴ are as per any row of Table U below, which compounds are named I.B1-Z300-Q3-U-1 to I.B1-Z300-Q3-U-42.

In another embodiment, compounds I are of formula I.B1 wherein Z is Z-300 and Q is Q4, and R and R⁴ are as per any row of Table U below, which compounds are named I.B1-Z300-Q4-U-1 to I.B1-Z300-Q4-U-42.

In another embodiment, compounds I are of formula I.B2 wherein Z is Z-300 and Q is Q1, and R and R⁴ are as per any row of Table U below, which compounds are named I.B2-Z300-Q1-U-1 to I.B2-Z300-Q1-U-42.

In another embodiment, compounds I are of formula I.B2 wherein Z is Z-300 and Q is Q2, and R and R⁴ are as per any row of Table U below, which compounds are named I.B2-Z300-Q2-U-1 to I.B2-Z300-Q2-U-42.

In another embodiment, compounds I are of formula I.B2 wherein Z is Z-300 and Q is Q3, and R and R⁴ are as per any row of Table U below, which compounds are named I.B2-Z300-Q3-U-1 to I.B2-Z300-Q3-U-42.

In another embodiment, compounds I are of formula I.B2 wherein Z is Z-300 and Q is Q4, and R and R⁴ are as per any row of Table U below, which compounds are named I.B2-Z300-Q4-U-1 to I.B2-Z300-Q4-U-42.

In another embodiment, compounds I are of formula I.B1 wherein Z is Z-301 and Q is Q1, and R and R⁴ are as per any row of Table U below, which compounds are named I.B1-Z301-Q1-U-1 to I.B1-Z301-Q1-U-42.

In another embodiment, compounds I are of formula I.B1 wherein Z is Z-301 and Q is Q2, and R and R⁴ are as per any row of Table U below, which compounds are named I.B1-Z301-Q2-U-1 to I.B1-Z301-Q2-U-42.

In another embodiment, compounds I are of formula I.B1 wherein Z is Z-301 and Q is Q3, and R and R⁴ are as per any row of Table U below, which compounds are named I.B1-Z301-Q3-U-1 to I.B1-Z301-Q3-U-42.

In another embodiment, compounds I are of formula I.B1 wherein Z is Z-301 and Q is Q4, and R and R⁴ are as per any row of Table U below, which compounds are named I.B1-Z301-Q4-U-1 to I.B1-Z301-Q4-U-42.

In another embodiment, compounds I are of formula I.B2 wherein Z is Z-301 and Q is Q1, and R and R⁴ are as per any row of Table U below, which compounds are named I.B2-Z301-Q1-U-1 to I.B2-Z301-Q1-U-42.

In another embodiment, compounds I are of formula I.B2 wherein Z is Z-301 and Q is Q2, and R and R⁴ are as per any row of Table U below, which compounds are named I.B2-Z301-Q2-U-1 to I.B2-Z301-Q2-U-42.

In another embodiment, compounds I are of formula I.B2 wherein Z is Z-301 and Q is Q3, and R and R⁴ are as per any row of Table U below, which compounds are named I.B2-Z301-Q3-U-1 to I.B2-Z301-Q3-U-42.

In another embodiment, compounds I are of formula I.B2 wherein Z is Z-301 and Q is Q4, and R and R⁴ are as per any row of Table U below, which compounds are named I.B2-Z301-Q4-U-1 to I.B2-Z301-Q4-U-42.

In another embodiment, compounds I are of formula I.B1 wherein Z is Z-302 and Q is Q1, and R and R⁴ are as per any row of Table U below, which compounds are named I.B1-Z302-Q1-U-1 to I.B1-Z302-Q1-U-42.

In another embodiment, compounds I are of formula I.B1 wherein Z is Z-302 and Q is Q2, and R and R⁴ are as per any row of Table U below, which compounds are named I.B1-Z302-Q2-U-1 to I.B1-Z302-Q2-U-42.

In another embodiment, compounds I are of formula I.B1 wherein Z is Z-302 and Q is Q3, and R and R⁴ are as per any row of Table U below, which compounds are named I.B1-Z302-Q3-U-1 to I.B1-Z302-Q3-U-42.

In another embodiment, compounds I are of formula I.B1 wherein Z is Z-302 and Q is Q4, and R and R⁴ are as per any row of Table U below, which compounds are named I.B1-Z302-Q4-U-1 to I.B1-Z302-Q4-U-42.

In another embodiment, compounds I are of formula I.B2 wherein Z is Z-302 and Q is Q1, and R and R⁴ are as per any row of Table U below, which compounds are named I.B2-Z302-Q1-U-1 to I.B2-Z302-Q1-U-42.

In another embodiment, compounds I are of formula I.B2 wherein Z is Z-302 and Q is Q2, and R and R⁴ are as per any row of Table U below, which compounds are named I.B2-Z302-Q2-U-1 to I.B2-Z302-Q2-U-42.

In another embodiment, compounds I are of formula I.B2 wherein Z is Z-302 and Q is Q3, and R and R⁴ are as per any row of Table U below, which compounds are named I.B2-Z302-Q3-U-1 to I.B2-Z302-Q3-U-42.

In another embodiment, compounds I are of formula I.B2 wherein Z is Z-302 and Q is Q4, and R and R⁴ are as per any row of Table U below, which compounds are named I.B2-Z302-Q4-U-1 to I.B2-Z302-Q4-U-42.

In another embodiment, compounds I are of formula I.B1 wherein Z is Z-303 and Q is Q1, and R and R⁴ are as per any row of Table U below, which compounds are named I.B1-Z303-Q1-U-1 to I.B1-Z303-Q1-U-42.

In another embodiment, compounds I are of formula I.B1 wherein Z is Z-303 and Q is Q2, and R and R⁴ are as per any row of Table U below, which compounds are named I.B1-Z303-Q2-U-1 to I.B1-Z303-Q2-U-42.

In another embodiment, compounds I are of formula I.B1 wherein Z is Z-303 and Q is Q3, and R and R⁴ are as per any row of Table U below, which compounds are named I.B1-Z303-Q3-U-1 to I.B1-Z303-Q3-U-42.

In another embodiment, compounds I are of formula I.B1 wherein Z is Z-303 and Q is Q4, and R and R⁴ are as per any row of Table U below, which compounds are named I.B1-Z303-Q4-U-1 to I.B1-Z303-Q4-U-42.

In another embodiment, compounds I are of formula I.B2 wherein Z is Z-303 and Q is Q1, and R and R⁴ are as per any row of Table U below, which compounds are named I.B2-Z303-Q1-U-1 to I.B2-Z303-Q1-U-42.

In another embodiment, compounds I are of formula I.B2 wherein Z is Z-303 and Q is Q2, and R and R⁴ are as per any row of Table U below, which compounds are named I.B2-Z303-Q2-U-1 to I.B2-Z303-Q2-U-42.

In another embodiment, compounds I are of formula I.B2 wherein Z is Z-303 and Q is Q3, and R and R⁴ are as per any row of Table U below, which compounds are named I.B2-Z303-Q3-U-1 to I.B2-Z303-Q3-U-42.

In another embodiment, compounds I are of formula I.B2 wherein Z is Z-303 and Q is Q4, and R and R⁴ are as per any row of Table U below, which compounds are named I.B2-Z303-Q4-U-1 to I.B2-Z303-Q4-U-42.

In another embodiment, compounds I are of formula I.B1 wherein Z is Z-304 and Q is Q1, and R and R⁴ are as per any row of Table U below, which compounds are named I.B1-Z304-Q1-U-1 to I.B1-Z304-Q1-U-42.

In another embodiment, compounds I are of formula I.B1 wherein Z is Z-304 and Q is Q2, and R and R⁴ are as per any row of Table U below, which compounds are named I.B1-Z304-Q2-U-1 to I.B1-Z304-Q2-U-42.

In another embodiment, compounds I are of formula I.B1 wherein Z is Z-304 and Q is Q3, and R and R⁴ are as per any row of Table U below, which compounds are named I.B1-Z304-Q3-U-1 to I.B1-Z304-Q3-U-42.

In another embodiment, compounds I are of formula I.B1 wherein Z is Z-304 and Q is Q4, and R and R⁴ are as per any row of Table U below, which compounds are named I.B1-Z304-Q4-U-1 to I.B1-Z304-Q4-U-42.

In another embodiment, compounds I are of formula I.B2 wherein Z is Z-304 and Q is Q1, and R and R⁴ are as per any row of Table U below, which compounds are named I.B2-Z304-Q1-U-1 to I.B2-Z304-Q1-U-42.

In another embodiment, compounds I are of formula I.B2 wherein Z is Z-304 and Q is Q2, and R and R⁴ are as per any row of Table U below, which compounds are named I.B2-Z304-Q2-U-1 to I.B2-Z304-Q2-U-42.

In another embodiment, compounds I are of formula I.B2 wherein Z is Z-304 and Q is Q3, and R and R⁴ are as per any row of Table U below, which compounds are named I.B2-Z304-Q3-U-1 to I.B2-Z304-Q3-U-42.

In another embodiment, compounds I are of formula I.B2 wherein Z is Z-304 and Q is Q4, and R and R⁴ are as per any row of Table U below, which compounds are named I.B2-Z304-Q4-U-1 to I.B2-Z304-Q4-U-42.

In another embodiment, compounds I are of formula I.B1 wherein Z is Z-305 and Q is Q1, and R and R⁴ are as per any row of Table U below, which compounds are named I.B1-Z305-Q1-U-1 to I.B1-Z305-Q1-U-42.

In another embodiment, compounds I are of formula I.B1 wherein Z is Z-305 and Q is Q2, and R and R⁴ are as per any row of Table U below, which compounds are named I.B1-Z305-Q2-U-1 to I.B1-Z305-Q2-U-42.

In another embodiment, compounds I are of formula I.B1 wherein Z is Z-305 and Q is Q3, and R and R⁴ are as per any row of Table U below, which compounds are named I.B1-Z305-Q3-U-1 to I.B1-Z305-Q3-U-42.

In another embodiment, compounds I are of formula I.B1 wherein Z is Z-305 and Q is Q4, and R and R⁴ are as per any row of Table U below, which compounds are named I.B1-Z305-Q4-U-1 to I.B1-Z305-Q4-U-42.

In another embodiment, compounds I are of formula I.B2 wherein Z is Z-305 and Q is Q1, and R and R⁴ are as per any row of Table U below, which compounds are named I.B2-Z305-Q1-U-1 to I.B2-Z305-Q1-U-42.

In another embodiment, compounds I are of formula I.B2 wherein Z is Z-305 and Q is Q2, and R and R⁴ are as per any row of Table U below, which compounds are named I.B2-Z305-Q2-U-1 to I.B2-Z305-Q2-U-42.

In another embodiment, compounds I are of formula I.B2 wherein Z is Z-305 and Q is Q3, and R and R⁴ are as per any row of Table U below, which compounds are named I.B2-Z305-Q3-U-1 to I.B2-Z305-Q3-U-42.

In another embodiment, compounds I are of formula I.B2 wherein Z is Z-305 and Q is Q4, and R and R⁴ are as per any row of Table U below, which compounds are named I.B2-Z305-Q4-U-1 to I.B2-Z305-Q4-U-42.

In another embodiment, compounds I are of formula I.B1 wherein Z is Z-306 and Q is Q1, and R and R⁴ are as per any row of Table U below, which compounds are named I.B1-Z306-Q1-U-1 to I.B1-Z306-Q1-U-42.

In another embodiment, compounds I are of formula I.B1 wherein Z is Z-306 and Q is Q2, and R and R⁴ are as per any row of Table U below, which compounds are named I.B1-Z306-Q2-U-1 to I.B1-Z306-Q2-U-42.

In another embodiment, compounds I are of formula I.B1 wherein Z is Z-306 and Q is Q3, and R and R⁴ are as per any row of Table U below, which compounds are named I.B1-Z306-Q3-U-1 to I.B1-Z306-Q3-U-42.

In another embodiment, compounds I are of formula I.B1 wherein Z is Z-306 and Q is Q4, and R and R⁴ are as per any row of Table U below, which compounds are named I.B1-Z306-Q4-U-1 to I.B1-Z306-Q4-U-42.

In another embodiment, compounds I are of formula I.B2 wherein Z is Z-306 and Q is Q1, and R and R⁴ are as per any row of Table U below, which compounds are named I.B2-Z306-Q1-U-1 to I.B2-Z306-Q1-U-42.

In another embodiment, compounds I are of formula I.B2 wherein Z is Z-306 and Q is Q2, and R and R⁴ are as per any row of Table U below, which compounds are named I.B2-Z306-Q2-U-1 to I.B2-Z306-Q2-U-42.

In another embodiment, compounds I are of formula I.B2 wherein Z is Z-306 and Q is Q3, and R and R⁴ are as per any row of Table U below, which compounds are named I.B2-Z306-Q3-U-1 to I.B2-Z306-Q3-U-42.

In another embodiment, compounds I are of formula I.B2 wherein Z is Z-306 and Q is Q4, and R and R⁴ are as per any row of Table U below, which compounds are named I.B2-Z306-Q4-U-1 to I.B2-Z306-Q4-U-42.

In another embodiment, compounds I are of formula I.B1 wherein Z is Z-307 and Q is Q1, and R and R⁴ are as per any row of Table U below, which compounds are named I.B1-Z307-Q1-U-1 to I.B1-Z307-Q1-U-42.

In another embodiment, compounds I are of formula I.B1 wherein Z is Z-307 and Q is Q2, and R and R⁴ are as per any row of Table U below, which compounds are named I.B1-Z307-Q2-U-1 to I.B1-Z307-Q2-U-42.

In another embodiment, compounds I are of formula I.B1 wherein Z is Z-307 and Q is Q3, and R and R⁴ are as per any row of Table U below, which compounds are named I.B1-Z307-Q3-U-1 to I.B1-Z307-Q3-U-42.

In another embodiment, compounds I are of formula I.B1 wherein Z is Z-307 and Q is Q4, and R and R⁴ are as per any row of Table U below, which compounds are named I.B1-Z307-Q4-U-1 to I.B1-Z307-Q4-U-42.

In another embodiment, compounds I are of formula I.B2 wherein Z is Z-307 and Q is Q1, and R and R⁴ are as per any row of Table U below, which compounds are named I.B2-Z307-Q1-U-1 to I.B2-Z307-Q1-U-42.

In another embodiment, compounds I are of formula I.B2 wherein Z is Z-307 and Q is Q2, and R and R⁴ are as per any row of Table U below, which compounds are named I.B2-Z307-Q2-U-1 to I.B2-Z307-Q2-U-42.

In another embodiment, compounds I are of formula I.B2 wherein Z is Z-307 and Q is Q3, and R and R⁴ are as per any row of Table U below, which compounds are named I.B2-Z307-Q3-U-1 to I.B2-Z307-Q3-U-42.

In another embodiment, compounds I are of formula I.B2 wherein Z is Z-307 and Q is Q4, and R and R⁴ are as per any row of Table U below, which compounds are named I.B2-Z307-Q4-U-1 to I.B2-Z307-Q4-U-42.

In another embodiment, compounds I are of formula I.B1 wherein Z is Z-308 and Q is Q1, and R and R⁴ are as per any row of Table U below, which compounds are named I.B1-Z308-Q1-U-1 to I.B1-Z308-Q1-U-42.

In another embodiment, compounds I are of formula I.B1 wherein Z is Z-308 and Q is Q2, and R and R⁴ are as per any row of Table U below, which compounds are named I.B1-Z308-Q2-U-1 to I.B1-Z308-Q2-U-42.

In another embodiment, compounds I are of formula I.B1 wherein Z is Z-308 and Q is Q3, and R and R⁴ are as per any row of Table U below, which compounds are named I.B1-Z308-Q3-U-1 to I.B1-Z308-Q3-U-42.

In another embodiment, compounds I are of formula I.B1 wherein Z is Z-308 and Q is Q4, and R and R⁴ are as per any row of Table U below, which compounds are named I.B1-Z308-Q4-U-1 to I.B1-Z308-Q4-U-42.

In another embodiment, compounds I are of formula I.B2 wherein Z is Z-308 and Q is Q1, and R and R⁴ are as per any row of Table U below, which compounds are named I.B2-Z308-Q1-U-1 to I.B2-Z308-Q1-U-42.

In another embodiment, compounds I are of formula I.B2 wherein Z is Z-308 and Q is Q2, and R and R⁴ are as per any row of Table U below, which compounds are named I.B2-Z308-Q2-U-1 to I.B2-Z308-Q2-U-42.

In another embodiment, compounds I are of formula I.B2 wherein Z is Z-308 and Q is Q3, and R and R⁴ are as per any row of Table U below, which compounds are named I.B2-Z308-Q3-U-1 to I.B2-Z308-Q3-U-42.

In another embodiment, compounds I are of formula I.B2 wherein Z is Z-308 and Q is Q4, and R and R⁴ are as per any row of Table U below, which compounds are named I.B2-Z308-Q4-U-1 to I.B2-Z308-Q4-U-42.

In another embodiment, compounds I are of formula I.B1 wherein Z is Z-309 and Q is Q1, and R and R⁴ are as per any row of Table U below, which compounds are named I.B1-Z309-Q1-U-1 to I.B1-Z309-Q1-U-42.

In another embodiment, compounds I are of formula I.B1 wherein Z is Z-309 and Q is Q2, and R and R⁴ are as per any row of Table U below, which compounds are named I.B1-Z309-Q2-U-1 to I.B1-Z309-Q2-U-42.

In another embodiment, compounds I are of formula I.B1 wherein Z is Z-309 and Q is Q3, and R and R⁴ are as per any row of Table U below, which compounds are named I.B1-Z309-Q3-U-1 to I.B1-Z309-Q3-U-42.

In another embodiment, compounds I are of formula I.B1 wherein Z is Z-309 and Q is Q4, and R and R⁴ are as per any row of Table U below, which compounds are named I.B1-Z309-Q4-U-1 to I.B1-Z309-Q4-U-42.

In another embodiment, compounds I are of formula I.B2 wherein Z is Z-309 and Q is Q1, and R and R⁴ are as per any row of Table U below, which compounds are named I.B2-Z309-Q1-U-1 to I.B2-Z309-Q1-U-42.

In another embodiment, compounds I are of formula I.B2 wherein Z is Z-309 and Q is Q2, and R and R⁴ are as per any row of Table U below, which compounds are named I.B2-Z309-Q2-U-1 to I.B2-Z309-Q2-U-42.

In another embodiment, compounds I are of formula I.B2 wherein Z is Z-309 and Q is Q3, and R and R⁴ are as per any row of Table U below, which compounds are named I.B2-Z309-Q3-U-1 to I.B2-Z309-Q3-U-42.

In another embodiment, compounds I are of formula I.B2 wherein Z is Z-309 and Q is Q4, and R and R⁴ are as per any row of Table U below, which compounds are named I.B2-Z309-Q4-U-1 to I.B2-Z309-Q4-U-42.

In another embodiment, compounds I are of formula I.B1 wherein Z is Z-310 and Q is Q1, and R and R⁴ are as per any row of Table U below, which compounds are named I.B1-Z310-Q1-U-1 to I.B1-Z310-Q1-U-42.

In another embodiment, compounds I are of formula I.B1 wherein Z is Z-310 and Q is Q2, and R and R⁴ are as per any row of Table U below, which compounds are named I.B1-Z310-Q2-U-1 to I.B1-Z310-Q2-U-42.

In another embodiment, compounds I are of formula I.B1 wherein Z is Z-310 and Q is Q3, and R and R⁴ are as per any row of Table U below, which compounds are named I.B1-Z310-Q3-U-1 to I.B1-Z310-Q3-U-42.

In another embodiment, compounds I are of formula I.B1 wherein Z is Z-310 and Q is Q4, and R and R⁴ are as per any row of Table U below, which compounds are named I.B1-Z310-Q4-U-1 to I.B1-Z310-Q4-U-42.

In another embodiment, compounds I are of formula I.B2 wherein Z is Z-310 and Q is Q1, and R and R⁴ are as per any row of Table U below, which compounds are named I.B2-Z310-Q1-U-1 to I.B2-Z310-Q1-U-42.

In another embodiment, compounds I are of formula I.B2 wherein Z is Z-310 and Q is Q2, and R and R⁴ are as per any row of Table U below, which compounds are named I.B2-Z310-Q2-U-1 to I. B2-Z310-Q2-U-42.

In another embodiment, compounds I are of formula I.B2 wherein Z is Z-310 and Q is Q3, and R and R⁴ are as per any row of Table U below, which compounds are named I.B2-Z310-Q3-U-1 to I. B2-Z310-Q3-U-42.

In another embodiment, compounds I are of formula I.B2 wherein Z is Z-310 and Q is Q4, and R and R⁴ are as per any row of Table U below, which compounds are named I.B2-Z310-Q4-U-1 to I.B2-Z310-Q4-U-42.

In another embodiment, compounds I are of formula I.B1 wherein Z is Z-311 and Q is Q1, and R and R⁴ are as per any row of Table U below, which compounds are named I.B1-Z311-Q1-U-1 to I.B1-Z311-Q1-U-42.

In another embodiment, compounds I are of formula I.B1 wherein Z is Z-311 and Q is Q2, and R and R⁴ are as per any row of Table U below, which compounds are named I.B1-Z311-Q2-U-1 to I.B1-Z311-Q2-U-42.

In another embodiment, compounds I are of formula I.B1 wherein Z is Z-311 and Q is Q3, and R and R⁴ are as per any row of Table U below, which compounds are named I.B1-Z311-Q3-U-1 to I.B1-Z311-Q3-U-42.

In another embodiment, compounds I are of formula I.B1 wherein Z is Z-311 and Q is Q4, and R and R⁴ are as per any row of Table U below, which compounds are named I.B1-Z311-Q4-U-1 to I.B1-Z311-Q4-U-42.

In another embodiment, compounds I are of formula I.B2 wherein Z is Z-311 and Q is Q1, and R and R⁴ are as per any row of Table U below, which compounds are named I.B2-Z311-Q1-U-1 to I.B2-Z311-Q1-U-42.

In another embodiment, compounds I are of formula I.B2 wherein Z is Z-311 and Q is Q2, and R and R⁴ are as per any row of Table U below, which compounds are named I.B2-Z311-Q2-U-1 to I.B2-Z311-Q2-U-42.

In another embodiment, compounds I are of formula I.B2 wherein Z is Z-311 and Q is Q3, and R and R⁴ are as per any row of Table U below, which compounds are named I.B2-Z311-Q3-U-1 to I.B2-Z311-Q3-U-42.

In another embodiment, compounds I are of formula I.B2 wherein Z is Z-311 and Q is Q4, and R and R⁴ are as per any row of Table U below, which compounds are named I.B2-Z311-Q4-U-1 to I.B2-Z311-Q4-U-42.

In another embodiment, compounds I are of formula I.B1 wherein Z is Z-312 and Q is Q1, and R and R⁴ are as per any row of Table U below, which compounds are named I.B1-Z312-Q1-U-1 to I.B1-Z312-Q1-U-42.

In another embodiment, compounds I are of formula I.B1 wherein Z is Z-312 and Q is Q2, and R and R⁴ are as per any row of Table U below, which compounds are named I.B1-Z312-Q2-U-1 to I.B1-Z312-Q2-U-42.

In another embodiment, compounds I are of formula I.B1 wherein Z is Z-312 and Q is Q3, and R and R⁴ are as per any row of Table U below, which compounds are named I.B1-Z312-Q3-U-1 to I.B1-Z312-Q3-U-42.

In another embodiment, compounds I are of formula I.B1 wherein Z is Z-312 and Q is Q4, and R and R⁴ are as per any row of Table U below, which compounds are named I.B1-Z312-Q4-U-1 to I.B1-Z312-Q4-U-42.

In another embodiment, compounds I are of formula I.B2 wherein Z is Z-312 and Q is Q1, and R and R⁴ are as per any row of Table U below, which compounds are named I.B2-Z312-Q1-U-1 to I.B2-Z312-Q1-U-42.

In another embodiment, compounds I are of formula I.B2 wherein Z is Z-312 and Q is Q2, and R and R⁴ are as per any row of Table U below, which compounds are named I.B2-Z312-Q2-U-1 to I.B2-Z312-Q2-U-42.

In another embodiment, compounds I are of formula I.B2 wherein Z is Z-312 and Q is Q3, and R and R⁴ are as per any row of Table U below, which compounds are named I.B2-Z312-Q3-U-1 to I.B2-Z312-Q3-U-42.

In another embodiment, compounds I are of formula I.B2 wherein Z is Z-312 and Q is Q4, and R and R⁴ are as per any row of Table U below, which compounds are named I.B2-Z312-Q4-U-1 to I.B2-Z312-Q4-U-42.

In another embodiment, compounds I are of formula I.B1 wherein Z is Z-313 and Q is Q1, and R and R⁴ are as per any row of Table U below, which compounds are named I.B1-Z313-Q1-U-1 to I.B1-Z313-Q1-U-42.

In another embodiment, compounds I are of formula I.B1 wherein Z is Z-313 and Q is Q2, and R and R⁴ are as per any row of Table U below, which compounds are named I.B1-Z313-Q2-U-1 to I.B1-Z313-Q2-U-42.

In another embodiment, compounds I are of formula I.B1 wherein Z is Z-313 and Q is Q3, and R and R⁴ are as per any row of Table U below, which compounds are named I.B1-Z313-Q3-U-1 to I.B1-Z313-Q3-U-42.

In another embodiment, compounds I are of formula I.B1 wherein Z is Z-313 and Q is Q4, and R and R⁴ are as per any row of Table U below, which compounds are named I.B1-Z313-Q4-U-1 to I.B1-Z313-Q4-U-42.

In another embodiment, compounds I are of formula I.B2 wherein Z is Z-313 and Q is Q1, and R and R⁴ are as per any row of Table U below, which compounds are named I.B2-Z313-Q1-U-1 to I.B2-Z313-Q1-U-42.

In another embodiment, compounds I are of formula I.B2 wherein Z is Z-313 and Q is Q2, and R and R⁴ are as per any row of Table U below, which compounds are named I.B2-Z313-Q2-U-1 to I.B2-Z313-Q2-U-42.

In another embodiment, compounds I are of formula I.B2 wherein Z is Z-313 and Q is Q3, and R and R⁴ are as per any row of Table U below, which compounds are named I.B2-Z313-Q3-U-1 to I.B2-Z313-Q3-U-42.

In another embodiment, compounds I are of formula I.B2 wherein Z is Z-313 and Q is Q4, and R and R⁴ are as per any row of Table U below, which compounds are named I.B2-Z313-Q4-U-1 to I.B2-Z313-Q4-U-42.

In another embodiment, compounds I are of formula I.B1 wherein Z is Z-314 and Q is Q1, and R and R⁴ are as per any row of Table U below, which compounds are named I.B1-Z314-Q1-U-1 to I.B1-Z314-Q1-U-42.

In another embodiment, compounds I are of formula I.B1 wherein Z is Z-314 and Q is Q2, and R and R⁴ are as per any row of Table U below, which compounds are named I.B1-Z314-Q2-U-1 to I.B1-Z314-Q2-U-42.

In another embodiment, compounds I are of formula I.B1 wherein Z is Z-314 and Q is Q3, and R and R⁴ are as per any row of Table U below, which compounds are named I.B1-Z314-Q3-U-1 to I.B1-Z314-Q3-U-42.

In another embodiment, compounds I are of formula I.B1 wherein Z is Z-314 and Q is Q4, and R and R⁴ are as per any row of Table U below, which compounds are named I.B1-Z314-Q4-U-1 to I.B1-Z314-Q4-U-42.

In another embodiment, compounds I are of formula I.B2 wherein Z is Z-314 and Q is Q1, and R and R⁴ are as per any row of Table U below, which compounds are named I.B2-Z314-Q1-U-1 to I.B2-Z314-Q1-U-42.

In another embodiment, compounds I are of formula I.B2 wherein Z is Z-314 and Q is Q2, and R and R⁴ are as per any row of Table U below, which compounds are named I.B2-Z314-Q2-U-1 to I.B2-Z314-Q2-U-42.

In another embodiment, compounds I are of formula I.B2 wherein Z is Z-314 and Q is Q3, and R and R⁴ are as per any row of Table U below, which compounds are named I.B2-Z314-Q3-U-1 to I.B2-Z314-Q3-U-42.

In another embodiment, compounds I are of formula I.B2 wherein Z is Z-314 and Q is Q4, and R and R⁴ are as per any row of Table U below, which compounds are named I.B2-Z314-Q4-U-1 to I.B2-Z314-Q4-U-42.

In another embodiment, compounds I are of formula I.B1 wherein Z is Z-315 and Q is Q1, and R and R⁴ are as per any row of Table U below, which compounds are named I.B1-Z315-Q1-U-1 to I.B1-Z315-Q1-U-42.

In another embodiment, compounds I are of formula I.B1 wherein Z is Z-315 and Q is Q2, and R and R⁴ are as per any row of Table U below, which compounds are named I.B1-Z315-Q2-U-1 to I.B1-Z315-Q2-U-42.

In another embodiment, compounds I are of formula I.B1 wherein Z is Z-315 and Q is Q3, and R and R⁴ are as per any row of Table U below, which compounds are named I.B1-Z315-Q3-U-1 to I.B1-Z315-Q3-U-42.

In another embodiment, compounds I are of formula I.B1 wherein Z is Z-315 and Q is Q4, and R and R⁴ are as per any row of Table U below, which compounds are named I.B1-Z315-Q4-U-1 to I.B1-Z315-Q4-U-42.

In another embodiment, compounds I are of formula I.B2 wherein Z is Z-315 and Q is Q1, and R and R⁴ are as per any row of Table U below, which compounds are named I.B2-Z315-Q1-U-1 to I.B2-Z315-Q1-U-42.

In another embodiment, compounds I are of formula I.B2 wherein Z is Z-315 and Q is Q2, and R and R⁴ are as per any row of Table U below, which compounds are named I.B2-Z315-Q2-U-1 to I.B2-Z315-Q2-U-42.

In another embodiment, compounds I are of formula I.B2 wherein Z is Z-315 and Q is Q3, and R and R⁴ are as per any row of Table U below, which compounds are named I.B2-Z315-Q3-U-1 to I.B2-Z315-Q3-U-42.

In another embodiment, compounds I are of formula I.B2 wherein Z is Z-315 and Q is Q4, and R and R⁴ are as per any row of Table U below, which compounds are named I.B2-Z315-Q4-U-1 to I.B2-Z315-Q4-U-42.

In another embodiment, compounds I are of formula I.B1 wherein Z is Z-316 and Q is Q1, and R and R⁴ are as per any row of Table U below, which compounds are named I.B1-Z316-Q1-U-1 to I.B1-Z316-Q1-U-42.

In another embodiment, compounds I are of formula I.B1 wherein Z is Z-316 and Q is Q2, and R and R⁴ are as per any row of Table U below, which compounds are named I.B1-Z316-Q2-U-1 to I.B1-Z316-Q2-U-42.

In another embodiment, compounds I are of formula I.B1 wherein Z is Z-316 and Q is Q3, and R and R⁴ are as per any row of Table U below, which compounds are named I.B1-Z316-Q3-U-1 to I.B1-Z316-Q3-U-42.

In another embodiment, compounds I are of formula I.B1 wherein Z is Z-316 and Q is Q4, and R and R⁴ are as per any row of Table U below, which compounds are named I.B1-Z316-Q4-U-1 to I.B1-Z316-Q4-U-42.

In another embodiment, compounds I are of formula I.B2 wherein Z is Z-316 and Q is Q1, and R and R⁴ are as per any row of Table U below, which compounds are named I.B2-Z316-Q1-U-1 to I.B2-Z316-Q1-U-42.

In another embodiment, compounds I are of formula I.B2 wherein Z is Z-316 and Q is Q2, and R and R⁴ are as per any row of Table U below, which compounds are named I.B2-Z316-Q2-U-1 to I.B2-Z316-Q2-U-42.

In another embodiment, compounds I are of formula I.B2 wherein Z is Z-316 and Q is Q3, and R and R⁴ are as per any row of Table U below, which compounds are named I.B2-Z316-Q3-U-1 to I.B2-Z316-Q3-U-42.

In another embodiment, compounds I are of formula I.B2 wherein Z is Z-316 and Q is Q4, and R and R⁴ are as per any row of Table U below, which compounds are named I.B2-Z316-Q4-U-1 to I.B2-Z316-Q4-U-42.

In another embodiment, compounds I are of formula I.B1 wherein Z is Z-317 and Q is Q1, and R and R⁴ are as per any row of Table U below, which compounds are named I.B1-Z317-Q1-U-1 to I.B1-Z317-Q1-U-42.

In another embodiment, compounds I are of formula I.B1 wherein Z is Z-317 and Q is Q2, and R and R⁴ are as per any row of Table U below, which compounds are named I.B1-Z317-Q2-U-1 to I.B1-Z317-Q2-U-42.

In another embodiment, compounds I are of formula I.B1 wherein Z is Z-317 and Q is Q3, and R and R⁴ are as per any row of Table U below, which compounds are named I.B1-Z317-Q3-U-1 to I.B1-Z317-Q3-U-42.

In another embodiment, compounds I are of formula I.B1 wherein Z is Z-317 and Q is Q4, and R and R⁴ are as per any row of Table U below, which compounds are named I.B1-Z317-Q4-U-1 to I.B1-Z317-Q4-U-42.

In another embodiment, compounds I are of formula I.B2 wherein Z is Z-317 and Q is Q1, and R and R⁴ are as per any row of Table U below, which compounds are named I.B2-Z317-Q1-U-1 to I.B2-Z317-Q1-U-42.

In another embodiment, compounds I are of formula I.B2 wherein Z is Z-317 and Q is Q2, and R and R⁴ are as per any row of Table U below, which compounds are named I.B2-Z317-Q2-U-1 to I.B2-Z317-Q2-U-42.

In another embodiment, compounds I are of formula I.B2 wherein Z is Z-317 and Q is Q3, and R and R⁴ are as per any row of Table U below, which compounds are named I.B2-Z317-Q3-U-1 to I.B2-Z317-Q3-U-42.

In another embodiment, compounds I are of formula I.B2 wherein Z is Z-317 and Q is Q4, and R and R⁴ are as per any row of Table U below, which compounds are named I.B2-Z317-Q4-U-1 to I.B2-Z317-Q4-U-42.

In another embodiment, compounds I are of formula I.B1 wherein Z is Z-318 and Q is Q1, and R and R⁴ are as per any row of Table U below, which compounds are named I.B1-Z318-Q1-U-1 to I.B1-Z318-Q1-U-42.

In another embodiment, compounds I are of formula I.B1 wherein Z is Z-318 and Q is Q2, and R and R⁴ are as per any row of Table U below, which compounds are named I.B1-Z318-Q2-U-1 to I.B1-Z318-Q2-U-42.

In another embodiment, compounds I are of formula I.B1 wherein Z is Z-318 and Q is Q3, and R and R⁴ are as per any row of Table U below, which compounds are named I.B1-Z318-Q3-U-1 to I.B1-Z318-Q3-U-42.

In another embodiment, compounds I are of formula I.B1 wherein Z is Z-318 and Q is Q4, and R and R⁴ are as per any row of Table U below, which compounds are named I.B1-Z318-Q4-U-1 to I.B1-Z318-Q4-U-42.

In another embodiment, compounds I are of formula I.B2 wherein Z is Z-318 and Q is Q1, and R and R⁴ are as per any row of Table U below, which compounds are named I.B2-Z318-Q1-U-1 to I.B2-Z318-Q1-U-42.

In another embodiment, compounds I are of formula I.B2 wherein Z is Z-318 and Q is Q2, and R and R⁴ are as per any row of Table U below, which compounds are named I.B2-Z318-Q2-U-1 to I.B2-Z318-Q2-U-42.

In another embodiment, compounds I are of formula I.B2 wherein Z is Z-318 and Q is Q3, and R and R⁴ are as per any row of Table U below, which compounds are named I.B2-Z318-Q3-U-1 to I.B2-Z318-Q3-U-42.

In another embodiment, compounds I are of formula I.B2 wherein Z is Z-318 and Q is Q4, and R and R⁴ are as per any row of Table U below, which compounds are named I.B2-Z318-Q4-U-1 to I.B2-Z318-Q4-U-42.

In another embodiment, compounds I are of formula I.B1 wherein Z is Z-319 and Q is Q1, and R and R⁴ are as per any row of Table U below, which compounds are named I.B1-Z319-Q1-U-1 to I.B1-Z319-Q1-U-42.

In another embodiment, compounds I are of formula I.B1 wherein Z is Z-319 and Q is Q2, and R and R⁴ are as per any row of Table U below, which compounds are named I.B1-Z319-Q2-U-1 to I.B1-Z319-Q2-U-42.

In another embodiment, compounds I are of formula I.B1 wherein Z is Z-319 and Q is Q3, and R and R⁴ are as per any row of Table U below, which compounds are named I.B1-Z319-Q3-U-1 to I.B1-Z319-Q3-U-42.

In another embodiment, compounds I are of formula I.B1 wherein Z is Z-319 and Q is Q4, and R and R⁴ are as per any row of Table U below, which compounds are named I.B1-Z319-Q4-U-1 to I.B1-Z319-Q4-U-42.

In another embodiment, compounds I are of formula I.B2 wherein Z is Z-319 and Q is Q1, and R and R⁴ are as per any row of Table U below, which compounds are named I.B2-Z319-Q1-U-1 to I.B2-Z319-Q1-U-42.

In another embodiment, compounds I are of formula I.B2 wherein Z is Z-319 and Q is Q2, and R and R⁴ are as per any row of Table U below, which compounds are named I.B2-Z319-Q2-U-1 to I.B2-Z319-Q2-U-42.

In another embodiment, compounds I are of formula I.B2 wherein Z is Z-319 and Q is Q3, and R and R⁴ are as per any row of Table U below, which compounds are named I.B2-Z319-Q3-U-1 to I.B2-Z319-Q3-U-42.

In another embodiment, compounds I are of formula I.B2 wherein Z is Z-319 and Q is Q4, and R and R⁴ are as per any row of Table U below, which compounds are named I.B2-Z319-Q4-U-1 to I.B2-Z319-Q4-U-42.

In another embodiment, compounds I are of formula I.B1 wherein Z is Z-320 and Q is Q1, and R and R⁴ are as per any row of Table U below, which compounds are named I.B1-Z320-Q1-U-1 to I.B1-Z320-Q1-U-42.

In another embodiment, compounds I are of formula I.B1 wherein Z is Z-320 and Q is Q2, and R and R⁴ are as per any row of Table U below, which compounds are named I.B1-Z320-Q2-U-1 to I.B1-Z320-Q2-U-42.

In another embodiment, compounds I are of formula I.B1 wherein Z is Z-320 and Q is Q3, and R and R⁴ are as per any row of Table U below, which compounds are named I.B1-Z320-Q3-U-1 to I.B1-Z320-Q3-U-42.

In another embodiment, compounds I are of formula I.B1 wherein Z is Z-320 and Q is Q4, and R and R⁴ are as per any row of Table U below, which compounds are named I.B1-Z320-Q4-U-1 to I.B1-Z320-Q4-U-42.

In another embodiment, compounds I are of formula I.B2 wherein Z is Z-320 and Q is Q1, and R and R⁴ are as per any row of Table U below, which compounds are named I.B2-Z320-Q1-U-1 to I.B2-Z320-Q1-U-42.

In another embodiment, compounds I are of formula I.B2 wherein Z is Z-320 and Q is Q2, and R and R⁴ are as per any row of Table U below, which compounds are named I.B2-Z320-Q2-U-1 to I.B2-Z320-Q2-U-42.

In another embodiment, compounds I are of formula I.B2 wherein Z is Z-320 and Q is Q3, and R and R⁴ are as per any row of Table U below, which compounds are named I.B2-Z320-Q3-U-1 to I.B2-Z320-Q3-U-42.

In another embodiment, compounds I are of formula I.B2 wherein Z is Z-320 and Q is Q4, and R and R⁴ are as per any row of Table U below, which compounds are named I.B2-Z320-Q4-U-1 to I.B2-Z320-Q4-U-42.

In another embodiment, compounds I are of formula I.B1 wherein Z is Z-321 and Q is Q1, and R and R⁴ are as per any row of Table U below, which compounds are named I.B1-Z321-Q1-U-1 to I.B1-Z321-Q1-U-42.

In another embodiment, compounds I are of formula I.B1 wherein Z is Z-321 and Q is Q2, and R and R⁴ are as per any row of Table U below, which compounds are named I.B1-Z321-Q2-U-1 to I.B1-Z321-Q2-U-42.

In another embodiment, compounds I are of formula I.B1 wherein Z is Z-321 and Q is Q3, and R and R⁴ are as per any row of Table U below, which compounds are named I.B1-Z321-Q3-U-1 to I.B1-Z321-Q3-U-42.

In another embodiment, compounds I are of formula I.B1 wherein Z is Z-321 and Q is Q4, and R and R⁴ are as per any row of Table U below, which compounds are named I.B1-Z321-Q4-U-1 to I.B1-Z321-Q4-U-42.

In another embodiment, compounds I are of formula I.B2 wherein Z is Z-321 and Q is Q1, and R and R⁴ are as per any row of Table U below, which compounds are named I.B2-Z321-Q1-U-1 to I.B2-Z321-Q1-U-42.

In another embodiment, compounds I are of formula I.B2 wherein Z is Z-321 and Q is Q2, and R and R⁴ are as per any row of Table U below, which compounds are named I.B2-Z321-Q2-U-1 to I.B2-Z321-Q2-U-42.

In another embodiment, compounds I are of formula I.B2 wherein Z is Z-321 and Q is Q3, and R and R⁴ are as per any row of Table U below, which compounds are named I.B2-Z321-Q3-U-1 to I.B2-Z321-Q3-U-42.

In another embodiment, compounds I are of formula I.B2 wherein Z is Z-321 and Q is Q4, and R and R⁴ are as per any row of Table U below, which compounds are named I.B2-Z321-Q4-U-1 to I.B2-Z321-Q4-U-42.

In another embodiment, compounds I are of formula I.B1 wherein Z is Z-322 and Q is Q1, and R and R⁴ are as per any row of Table U below, which compounds are named I.B1-Z322-Q1-U-1 to I.B1-Z322-Q1-U-42.

In another embodiment, compounds I are of formula I.B1 wherein Z is Z-322 and Q is Q2, and R and R⁴ are as per any row of Table U below, which compounds are named I.B1-Z322-Q2-U-1 to I.B1-Z322-Q2-U-42.

In another embodiment, compounds I are of formula I.B1 wherein Z is Z-322 and Q is Q3, and R and R⁴ are as per any row of Table U below, which compounds are named I.B1-Z322-Q3-U-1 to I.B1-Z322-Q3-U-42.

In another embodiment, compounds I are of formula I.B1 wherein Z is Z-322 and Q is Q4, and R and R⁴ are as per any row of Table U below, which compounds are named I.B1-Z322-Q4-U-1 to I.B1-Z322-Q4-U-42.

In another embodiment, compounds I are of formula I.B2 wherein Z is Z-322 and Q is Q1, and R and R⁴ are as per any row of Table U below, which compounds are named I.B2-Z322-Q1-U-1 to I.B2-Z322-Q1-U-42.

In another embodiment, compounds I are of formula I.B2 wherein Z is Z-322 and Q is Q2, and R and R⁴ are as per any row of Table U below, which compounds are named I.B2-Z322-Q2-U-1 to I.B2-Z322-Q2-U-42.

In another embodiment, compounds I are of formula I.B2 wherein Z is Z-322 and Q is Q3, and R and R⁴ are as per any row of Table U below, which compounds are named I.B2-Z322-Q3-U-1 to I.B2-Z322-Q3-U-42.

In another embodiment, compounds I are of formula I.B2 wherein Z is Z-322 and Q is Q4, and R and R⁴ are as per any row of Table U below, which compounds are named I.B2-Z322-Q4-U-1 to I.B2-Z322-Q4-U-42.

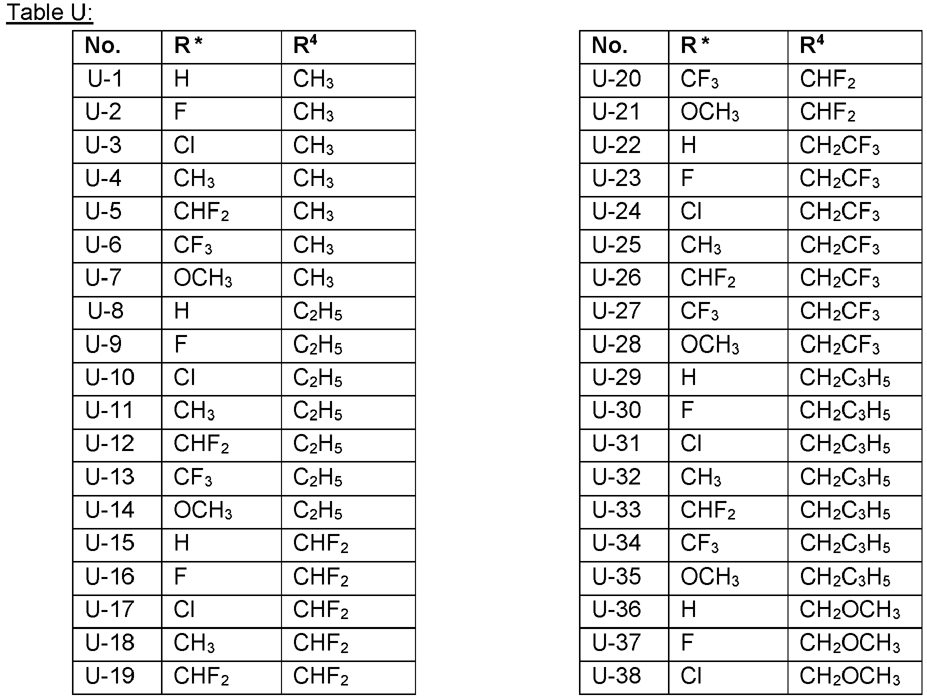

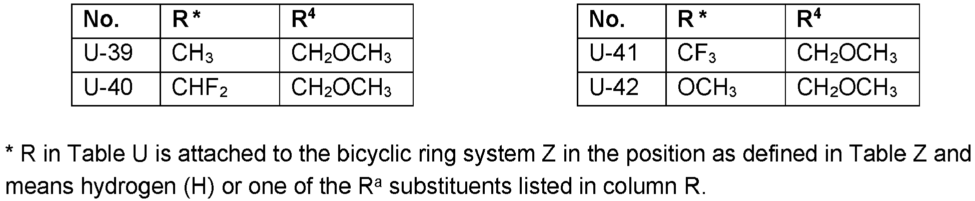

In another embodiment, compounds I are of formula I.A1 wherein Z is Z-323, R³ is CH₃ and Q is Q1, and R⁴ is as per any row of Table V below, which compounds are named I.A1-Z323-Q1-V-1 to I.A1-Z323-Q1-V-6.

In another embodiment, compounds I are of formula I.A1 wherein Z is Z-323, R³ is CH₃ and Q is Q2, and R⁴ is as per any row of Table V below, which compounds are named I.A1-Z323-Q2-V-1 to I.A1-Z323-Q2-V-6.

In another embodiment, compounds I are of formula I.A1 wherein Z is Z-323, R³ is CH₃ and Q is Q3, and R⁴ is as per any row of Table V below, which compounds are named I.A1-Z323-Q3-V-1 to I.A1-Z323-Q3-V-6.

In another embodiment, compounds I are of formula I.A1 wherein Z is Z-323, R³ is CH₃ and Q is Q4, and R⁴ is as per any row of Table V below, which compounds are named I.A1-Z323-Q4-V-1 to I.A1-Z323-Q4-V-6.

In another embodiment, compounds I are of formula I.A2 wherein Z is Z-323, R³ is CH₃ and Q is Q1, and R⁴ is as per any row of Table V below, which compounds are named I.A2-Z323-Q1-V-1 to I.A2-Z323-Q1-V-6.

In another embodiment, compounds I are of formula I.A2 wherein Z is Z-323, R³ is CH₃ and Q is Q2, and R⁴ is as per any row of Table V below, which compounds are named I.A2-Z323-Q2-V-1 to I.A2-Z323-Q2-V-6.

In another embodiment, compounds I are of formula I.A2 wherein Z is Z-323, R³ is CH₃ and Q is Q3, and R⁴ is as per any row of Table V below, which compounds are named I.A2-Z323-Q3-V-1 to I.A2-Z323-Q3-V-6.

In another embodiment, compounds I are of formula I.A2 wherein Z is Z-323, R³ is CH₃ and Q is Q4, and R⁴ is as per any row of Table V below, which compounds are named I.A2-Z323-Q4-V-1 to I.A2-Z323-Q4-V-6.

In another embodiment, compounds I are of formula I.A1 wherein Z is Z-324, R³ is CH₃ and Q is Q1, and R⁴ is as per any row of Table V below, which compounds are named I.A1-Z324-Q1-V-1 to I.A1-Z324-Q1-V-6.

In another embodiment, compounds I are of formula I.A1 wherein Z is Z-324, R³ is CH₃ and Q is Q2, and R⁴ is as per any row of Table V below, which compounds are named I.A1-Z324-Q2-V-1 to I.A1-Z324-Q2-V-6.

In another embodiment, compounds I are of formula I.A1 wherein Z is Z-324, R³ is CH₃ and Q is Q3, and R⁴ is as per any row of Table V below, which compounds are named I.A1-Z324-Q3-V-1 to I.A1-Z324-Q3-V-6.

In another embodiment, compounds I are of formula I.A1 wherein Z is Z-324, R³ is CH₃ and Q is Q4, and R⁴ is as per any row of Table V below, which compounds are named I.A1-Z324-Q4-V-1 to I.A1-Z324-Q4-V-6.

In another embodiment, compounds I are of formula I.A2 wherein Z is Z-324, R³ is CH₃ and Q is Q1, and R⁴ is as per any row of Table V below, which compounds are named I.A2-Z324-Q1-V-1 to I.A2-Z324-Q1-V-6.

In another embodiment, compounds I are of formula I.A2 wherein Z is Z-324, R³ is CH₃ and Q is Q2, and R⁴ is as per any row of Table V below, which compounds are named I.A2-Z324-Q2-V-1 to I.A2-Z324-Q2-V-6.

In another embodiment, compounds I are of formula I.A2 wherein Z is Z-324, R³ is CH₃ and Q is Q3, and R⁴ is as per any row of Table V below, which compounds are named I.A2-Z324-Q3-V-1 to I.A2-Z324-Q3-V-6.

In another embodiment, compounds I are of formula I.A2 wherein Z is Z-324, R³ is CH₃ and Q is Q4, and R⁴ is as per any row of Table V below, which compounds are named I.A2-Z324-Q4-V-1 to I.A2-Z324-Q4-V-6.

In another embodiment, compounds I are of formula I.A1 wherein Z is Z-325, R³ is CH₃ and Q is Q1, and R⁴ is as per any row of Table V below, which compounds are named I.A1-Z325-Q1-V-1 to I.A1-Z325-Q1-V-6.

In another embodiment, compounds I are of formula I.A1 wherein Z is Z-325, R³ is CH₃ and Q is Q2, and R⁴ is as per any row of Table V below, which compounds are named I.A1-Z325-Q2-V-1 to I.A1-Z325-Q2-V-6.

In another embodiment, compounds I are of formula I.A1 wherein Z is Z-325, R³ is CH₃ and Q is Q3, and R⁴ is as per any row of Table V below, which compounds are named I.A1-Z325-Q3-V-1 to I.A1-Z325-Q3-V-6.

In another embodiment, compounds I are of formula I.A1 wherein Z is Z-325, R³ is CH₃ and Q is Q4, and R⁴ is as per any row of Table V below, which compounds are named I.A1-Z325-Q4-V-1 to I.A1-Z325-Q4-V-6.

In another embodiment, compounds I are of formula I.A2 wherein Z is Z-325, R³ is CH₃ and Q is Q1, and R⁴ is as per any row of Table V below, which compounds are named I.A2-Z325-Q1-V-1 to I.A2-Z325-Q1-V-6.

In another embodiment, compounds I are of formula I.A2 wherein Z is Z-325, R³ is CH₃ and Q is Q2, and R⁴ is as per any row of Table V below, which compounds are named I.A2-Z325-Q2-V-1 to I.A2-Z325-Q2-V-6.

In another embodiment, compounds I are of formula I.A2 wherein Z is Z-325, R³ is CH₃ and Q is Q3, and R⁴ is as per any row of Table V below, which compounds are named I.A2-Z325-Q3-V-1 to I.A2-Z325-Q3-V-6.

In another embodiment, compounds I are of formula I.A2 wherein Z is Z-325, R³ is CH₃ and Q is Q4, and R⁴ is as per any row of Table V below, which compounds are named I.A2-Z325-Q4-V-1 to I.A2-Z325-Q4-V-6.

In another embodiment, compounds I are of formula I.A1 wherein Z is Z-326, R³ is CH₃ and Q is Q1, and R⁴ is as per any row of Table V below, which compounds are named I.A1-Z326-Q1-V-1 to I.A1-Z326-Q1-V-6.

In another embodiment, compounds I are of formula I.A1 wherein Z is Z-326, R³ is CH₃ and Q is Q2, and R⁴ is as per any row of Table V below, which compounds are named I.A1-Z326-Q2-V-1 to I.A1-Z326-Q2-V-6.

In another embodiment, compounds I are of formula I.A1 wherein Z is Z-326, R³ is CH₃ and Q is Q3, and R⁴ is as per any row of Table V below, which compounds are named I.A1-Z326-Q3-V-1 to I.A1-Z326-Q3-V-6.

In another embodiment, compounds I are of formula I.A1 wherein Z is Z-326, R³ is CH₃ and Q is Q4, and R⁴ is as per any row of Table V below, which compounds are named I.A1-Z326-Q4-V-1 to I.A1-Z326-Q4-V-6.

In another embodiment, compounds I are of formula I.A2 wherein Z is Z-326, R³ is CH₃ and Q is Q1, and R⁴ is as per any row of Table V below, which compounds are named I.A2-Z326-Q1-V-1 to I.A2-Z326-Q1-V-6.

In another embodiment, compounds I are of formula I.A2 wherein Z is Z-326, R³ is CH₃ and Q is Q2, and R⁴ is as per any row of Table V below, which compounds are named I.A2-Z326-Q2-V-1 to I.A2-Z326-Q2-V-6.

In another embodiment, compounds I are of formula I.A2 wherein Z is Z-326, R³ is CH₃ and Q is Q3, and R⁴ is as per any row of Table V below, which compounds are named I.A2-Z326-Q3-V-1 to I.A2-Z326-Q3-V-6.

In another embodiment, compounds I are of formula I.A2 wherein Z is Z-326, R³ is CH₃ and Q is Q4, and R⁴ is as per any row of Table V below, which compounds are named I.A2-Z326-Q4-V-1 to I.A2-Z326-Q4-V-6.

In another embodiment, compounds I are of formula I.A1 wherein Z is Z-327, R³ is CH₃ and Q is Q1, and R⁴ is as per any row of Table V below, which compounds are named I.A1-Z327-Q1-V-1 to I.A1-Z327-Q1-V-6.

In another embodiment, compounds I are of formula I.A1 wherein Z is Z-327, R³ is CH₃ and Q is Q2, and R⁴ is as per any row of Table V below, which compounds are named I.A1-Z327-Q2-V-1 to I.A1-Z327-Q2-V-6.

In another embodiment, compounds I are of formula I.A1 wherein Z is Z-327, R³ is CH₃ and Q is Q3, and R⁴ is as per any row of Table V below, which compounds are named I.A1-Z327-Q3-V-1 to I.A1-Z327-Q3-V-6.

In another embodiment, compounds I are of formula I.A1 wherein Z is Z-327, R³ is CH₃ and Q is Q4, and R⁴ is as per any row of Table V below, which compounds are named I.A1-Z327-Q4-V-1 to I.A1-Z327-Q4-V-6.

In another embodiment, compounds I are of formula I.A2 wherein Z is Z-327, R³ is CH₃ and Q is Q1, and R⁴ is as per any row of Table V below, which compounds are named I.A2-Z327-Q1-V-1 to I.A2-Z327-Q1-V-6.

In another embodiment, compounds I are of formula I.A2 wherein Z is Z-327, R³ is CH₃ and Q is Q2, and R⁴ is as per any row of Table V below, which compounds are named I.A2-Z327-Q2-V-1 to I.A2-Z327-Q2-V-6.

In another embodiment, compounds I are of formula I.A2 wherein Z is Z-327, R³ is CH₃ and Q is Q3, and R⁴ is as per any row of Table V below, which compounds are named I.A2-Z327-Q3-V-1 to I.A2-Z327-Q3-V-6.

In another embodiment, compounds I are of formula I.A2 wherein Z is Z-327, R³ is CH₃ and Q is Q4, and R⁴ is as per any row of Table V below, which compounds are named I.A2-Z327-Q4-V-1 to I.A2-Z327-Q4-V-6.

In another embodiment, compounds I are of formula I.A1 wherein Z is Z-328, R³ is CH₃ and Q is Q1, and R⁴ is as per any row of Table V below, which compounds are named I.A1-Z328-Q1-V-1 to I.A1-Z328-Q1-V-6.

In another embodiment, compounds I are of formula I.A1 wherein Z is Z-328, R³ is CH₃ and Q is Q2, and R⁴ is as per any row of Table V below, which compounds are named I.A1-Z328-Q2-V-1 to I.A1-Z328-Q2-V-6.

In another embodiment, compounds I are of formula I.A1 wherein Z is Z-328, R³ is CH₃ and Q is Q3, and R⁴ is as per any row of Table V below, which compounds are named I.A1-Z328-Q3-V-1 to I.A1-Z328-Q3-V-6.

In another embodiment, compounds I are of formula I.A1 wherein Z is Z-328, R³ is CH₃ and Q is Q4, and R⁴ is as per any row of Table V below, which compounds are named I.A1-Z328-Q4-V-1 to I.A1-Z328-Q4-V-6.

In another embodiment, compounds I are of formula I.A2 wherein Z is Z-328, R³ is CH₃ and Q is Q1, and R⁴ is as per any row of Table V below, which compounds are named I.A2-Z328-Q1-V-1 to I.A2-Z328-Q1-V-6.

In another embodiment, compounds I are of formula I.A2 wherein Z is Z-328, R³ is CH₃ and Q is Q2, and R⁴ is as per any row of Table V below, which compounds are named I.A2-Z328-Q2-V-1 to I.A2-Z328-Q2-V-6.

In another embodiment, compounds I are of formula I.A2 wherein Z is Z-328, R³ is CH₃ and Q is Q3, and R⁴ is as per any row of Table V below, which compounds are named I.A2-Z328-Q3-V-1 to I.A2-Z328-Q3-V-6.

In another embodiment, compounds I are of formula I.A2 wherein Z is Z-328, R³ is CH₃ and Q is Q4, and R⁴ is as per any row of Table V below, which compounds are named I.A2-Z328-Q4-V-1 to I.A2-Z328-Q4-V-6.

In another embodiment, compounds I are of formula I.A1 wherein Z is Z-329, R³ is CH₃ and Q is Q1, and R⁴ is as per any row of Table V below, which compounds are named I.A1-Z329-Q1-V-1 to I.A1-Z329-Q1-V-6.

In another embodiment, compounds I are of formula I.A1 wherein Z is Z-329, R³ is CH₃ and Q is Q2, and R⁴ is as per any row of Table V below, which compounds are named I.A1-Z329-Q2-V-1 to I.A1-Z329-Q2-V-6.

In another embodiment, compounds I are of formula I.A1 wherein Z is Z-329, R³ is CH₃ and Q is Q3, and R⁴ is as per any row of Table V below, which compounds are named I.A1-Z329-Q3-V-1 to I.A1-Z329-Q3-V-6.

In another embodiment, compounds I are of formula I.A1 wherein Z is Z-329, R³ is CH₃ and Q is Q4, and R⁴ is as per any row of Table V below, which compounds are named I.A1-Z329-Q4-V-1 to I.A1-Z329-Q4-V-6.

In another embodiment, compounds I are of formula I.A2 wherein Z is Z-329, R³ is CH₃ and Q is Q1, and R⁴ is as per any row of Table V below, which compounds are named I.A2-Z329-Q1-V-1 to I.A2-Z329-Q1-V-6.

In another embodiment, compounds I are of formula I.A2 wherein Z is Z-329, R³ is CH₃ and Q is Q2, and R⁴ is as per any row of Table V below, which compounds are named I.A2-Z329-Q2-V-1 to I.A2-Z329-Q2-V-6.

In another embodiment, compounds I are of formula I.A2 wherein Z is Z-329, R³ is CH₃ and Q is Q3, and R⁴ is as per any row of Table V below, which compounds are named I.A2-Z329-Q3-V-1 to I.A2-Z329-Q3-V-6.

In another embodiment, compounds I are of formula I.A2 wherein Z is Z-329, R³ is CH₃ and Q is Q4, and R⁴ is as per any row of Table V below, which compounds are named I.A2-Z329-Q4-V-1 to I.A2-Z329-Q4-V-6.

In another embodiment, compounds I are of formula I.A1 wherein Z is Z-330, R³ is CH₃ and Q is Q1, and R⁴ is as per any row of Table V below, which compounds are named I.A1-Z330-Q1-V-1 to I.A1-Z330-Q1-V-6.

In another embodiment, compounds I are of formula I.A1 wherein Z is Z-330, R³ is CH₃ and Q is Q2, and R⁴ is as per any row of Table V below, which compounds are named I.A1-Z330-Q2-V-1 to I.A1-Z330-Q2-V-6.

In another embodiment, compounds I are of formula I.A1 wherein Z is Z-330, R³ is CH₃ and Q is Q3, and R⁴ is as per any row of Table V below, which compounds are named I.A1-Z330-Q3-V-1 to I.A1-Z330-Q3-V-6.

In another embodiment, compounds I are of formula I.A1 wherein Z is Z-330, R³ is CH₃ and Q is Q4, and R⁴ is as per any row of Table V below, which compounds are named I.A1-Z330-Q4-V-1 to I.A1-Z330-Q4-V-6.

In another embodiment, compounds I are of formula I.A2 wherein Z is Z-330, R³ is CH₃ and Q is Q1, and R⁴ is as per any row of Table V below, which compounds are named I.A2-Z330-Q1-V-1 to I.A2-Z330-Q1-V-6.

In another embodiment, compounds I are of formula I.A2 wherein Z is Z-330, R³ is CH₃ and Q is Q2, and R⁴ is as per any row of Table V below, which compounds are named I.A2-Z330-Q2-V-1 to I.A2-Z330-Q2-V-6.

In another embodiment, compounds I are of formula I.A2 wherein Z is Z-330, R³ is CH₃ and Q is Q3, and R⁴ is as per any row of Table V below, which compounds are named I.A2-Z330-Q3-V-1 to I.A2-Z330-Q3-V-6.

In another embodiment, compounds I are of formula I.A2 wherein Z is Z-330, R³ is CH₃ and Q is Q4, and R⁴ is as per any row of Table V below, which compounds are named I.A2-Z330-Q4-V-1 to I.A2-Z330-Q4-V-6.

In another embodiment, compounds I are of formula I.A1 wherein Z is Z-331, R³ is CH₃ and Q is Q1, and R⁴ is as per any row of Table V below, which compounds are named I.A1-Z331-Q1-V-1 to I.A1-Z331-Q1-V-6.

In another embodiment, compounds I are of formula I.A1 wherein Z is Z-331, R³ is CH₃ and Q is Q2, and R⁴ is as per any row of Table V below, which compounds are named I.A1-Z331-Q2-V-1 to I.A1-Z331-Q2-V-6.

In another embodiment, compounds I are of formula I.A1 wherein Z is Z-331, R³ is CH₃ and Q is Q3, and R⁴ is as per any row of Table V below, which compounds are named I.A1-Z331-Q3-V-1 to I.A1-Z331-Q3-V-6.

In another embodiment, compounds I are of formula I.A1 wherein Z is Z-331, R³ is CH₃ and Q is Q4, and R⁴ is as per any row of Table V below, which compounds are named I.A1-Z331-Q4-V-1 to I.A1-Z331-Q4-V-6.

In another embodiment, compounds I are of formula I.A2 wherein Z is Z-331, R³ is CH₃ and Q is Q1, and R⁴ is as per any row of Table V below, which compounds are named I.A2-Z331-Q1-V-1 to I.A2-Z331-Q1-V-6.

In another embodiment, compounds I are of formula I.A2 wherein Z is Z-331, R³ is CH₃ and Q is Q2, and R⁴ is as per any row of Table V below, which compounds are named I.A2-Z331-Q2-V-1 to I.A2-Z331-Q2-V-6.

In another embodiment, compounds I are of formula I.A2 wherein Z is Z-331, R³ is CH₃ and Q is Q3, and R⁴ is as per any row of Table V below, which compounds are named I.A2-Z331-Q3-V-1 to I.A2-Z331-Q3-V-6.

In another embodiment, compounds I are of formula I.A2 wherein Z is Z-331, R³ is CH₃ and Q is Q4, and R⁴ is as per any row of Table V below, which compounds are named I.A2-Z331-Q4-V-1 to I.A2-Z331-Q4-V-6.

In another embodiment, compounds I are of formula I.A1 wherein Z is Z-332, R³ is CH₃ and Q is Q1, and R⁴ is as per any row of Table V below, which compounds are named I.A1-Z332-Q1-V-1 to I.A1-Z332-Q1-V-6.

In another embodiment, compounds I are of formula I.A1 wherein Z is Z-332, R³ is CH₃ and Q is Q2, and R⁴ is as per any row of Table V below, which compounds are named I.A1-Z332-Q2-V-1 to I.A1-Z332-Q2-V-6.

In another embodiment, compounds I are of formula I.A1 wherein Z is Z-332, R³ is CH₃ and Q is Q3, and R⁴ is as per any row of Table V below, which compounds are named I.A1-Z332-Q3-V-1 to I.A1-Z332-Q3-V-6.

In another embodiment, compounds I are of formula I.A1 wherein Z is Z-332, R³ is CH₃ and Q is Q4, and R⁴ is as per any row of Table V below, which compounds are named I.A1-Z332-Q4-V-1 to I.A1-Z332-Q4-V-6.

In another embodiment, compounds I are of formula I.A2 wherein Z is Z-332, R³ is CH₃ and Q is Q1, and R⁴ is as per any row of Table V below, which compounds are named I.A2-Z332-Q1-V-1 to I.A2-Z332-Q1-V-6.

In another embodiment, compounds I are of formula I.A2 wherein Z is Z-332, R³ is CH₃ and Q is Q2, and R⁴ is as per any row of Table V below, which compounds are named I.A2-Z332-Q2-V-1 to I.A2-Z332-Q2-V-6.

In another embodiment, compounds I are of formula I.A2 wherein Z is Z-332, R³ is CH₃ and Q is Q3, and R⁴ is as per any row of Table V below, which compounds are named I.A2-Z332-Q3-V-1 to I.A2-Z332-Q3-V-6.

In another embodiment, compounds I are of formula I.A2 wherein Z is Z-332, R³ is CH₃ and Q is Q4, and R⁴ is as per any row of Table V below, which compounds are named I.A2-Z332-Q4-V-1 to I.A2-Z332-Q4-V-6.

In another embodiment, compounds I are of formula I.A1 wherein Z is Z-333, R³ is CH₃ and Q is Q1, and R⁴ is as per any row of Table V below, which compounds are named I.A1-Z333-Q1-V-1 to I.A1-Z333-Q1-V-6.

In another embodiment, compounds I are of formula I.A1 wherein Z is Z-333, R³ is CH₃ and Q is Q2, and R⁴ is as per any row of Table V below, which compounds are named I.A1-Z333-Q2-V-1 to I.A1-Z333-Q2-V-6.

In another embodiment, compounds I are of formula I.A1 wherein Z is Z-333, R³ is CH₃ and Q is Q3, and R⁴ is as per any row of Table V below, which compounds are named I.A1-Z333-Q3-V-1 to I.A1-Z333-Q3-V-6.

In another embodiment, compounds I are of formula I.A1 wherein Z is Z-333, R³ is CH₃ and Q is Q4, and R⁴ is as per any row of Table V below, which compounds are named I.A1-Z333-Q4-V-1 to I.A1-Z333-Q4-V-6.

In another embodiment, compounds I are of formula I.A2 wherein Z is Z-333, R³ is CH₃ and Q is Q1, and R⁴ is as per any row of Table V below, which compounds are named I.A2-Z333-Q1-V-1 to I.A2-Z333-Q1-V-6.

In another embodiment, compounds I are of formula I.A2 wherein Z is Z-333, R³ is CH₃ and Q is Q2, and R⁴ is as per any row of Table V below, which compounds are named I.A2-Z333-Q2-V-1 to I.A2-Z333-Q2-V-6.

In another embodiment, compounds I are of formula I.A2 wherein Z is Z-333, R³ is CH₃ and Q is Q3, and R⁴ is as per any row of Table V below, which compounds are named I.A2-Z333-Q3-V-1 to I.A2-Z333-Q3-V-6.

In another embodiment, compounds I are of formula I.A2 wherein Z is Z-333, R³ is CH₃ and Q is Q4, and R⁴ is as per any row of Table V below, which compounds are named I.A2-Z333-Q4-V-1 to I.A2-Z333-Q4-V-6.

In another embodiment, compounds I are of formula I.A1 wherein Z is Z-334, R³ is CH₃ and Q is Q1, and R⁴ is as per any row of Table V below, which compounds are named I.A1-Z334-Q1-V-1 to I.A1-Z334-Q1-V-6.

In another embodiment, compounds I are of formula I.A1 wherein Z is Z-334, R³ is CH₃ and Q is Q2, and R⁴ is as per any row of Table V below, which compounds are named I.A1-Z334-Q2-V-1 to I.A1-Z334-Q2-V-6.

In another embodiment, compounds I are of formula I.A1 wherein Z is Z-334, R³ is CH₃ and Q is Q3, and R⁴ is as per any row of Table V below, which compounds are named I.A1-Z334-Q3-V-1 to I.A1-Z334-Q3-V-6.

In another embodiment, compounds I are of formula I.A1 wherein Z is Z-334, R³ is CH₃ and Q is Q4, and R⁴ is as per any row of Table V below, which compounds are named I.A1-Z334-Q4-V-1 to I.A1-Z334-Q4-V-6.

In another embodiment, compounds I are of formula I.A2 wherein Z is Z-334, R³ is CH₃ and Q is Q1, and R⁴ is as per any row of Table V below, which compounds are named I.A2-Z334-Q1-V-1 to I.A2-Z334-Q1-V-6.

In another embodiment, compounds I are of formula I.A2 wherein Z is Z-334, R³ is CH₃ and Q is Q2, and R⁴ is as per any row of Table V below, which compounds are named I.A2-Z334-Q2-V-1 to I.A2-Z334-Q2-V-6.

In another embodiment, compounds I are of formula I.A2 wherein Z is Z-334, R³ is CH₃ and Q is Q3, and R⁴ is as per any row of Table V below, which compounds are named I.A2-Z334-Q3-V-1 to I.A2-Z334-Q3-V-6.

In another embodiment, compounds I are of formula I.A2 wherein Z is Z-334, R³ is CH₃ and Q is Q4, and R⁴ is as per any row of Table V below, which compounds are named I.A2-Z334-Q4-V-1 to I.A2-Z334-Q4-V-6.

In another embodiment, compounds I are of formula I.A1 wherein Z is Z-335, R³ is CH₃ and Q is Q1, and R⁴ is as per any row of Table V below, which compounds are named I.A1-Z335-Q1-V-1 to I.A1-Z335-Q1-V-6.

In another embodiment, compounds I are of formula I.A1 wherein Z is Z-335, R³ is CH₃ and Q is Q2, and R⁴ is as per any row of Table V below, which compounds are named I.A1-Z335-Q2-V-1 to I.A1-Z335-Q2-V-6.

In another embodiment, compounds I are of formula I.A1 wherein Z is Z-335, R³ is CH₃ and Q is Q3, and R⁴ is as per any row of Table V below, which compounds are named I.A1-Z335-Q3-V-1 to I.A1-Z335-Q3-V-6.

In another embodiment, compounds I are of formula I.A1 wherein Z is Z-335, R³ is CH₃ and Q is Q4, and R⁴ is as per any row of Table V below, which compounds are named I.A1-Z335-Q4-V-1 to I.A1-Z335-Q4-V-6.

In another embodiment, compounds I are of formula I.A2 wherein Z is Z-335, R³ is CH₃ and Q is Q1, and R⁴ is as per any row of Table V below, which compounds are named I.A2-Z335-Q1-V-1 to I.A2-Z335-Q1-V-6.

In another embodiment, compounds I are of formula I.A2 wherein Z is Z-335, R³ is CH₃ and Q is Q2, and R⁴ is as per any row of Table V below, which compounds are named I.A2-Z335-Q2-V-1 to I.A2-Z335-Q2-V-6.

In another embodiment, compounds I are of formula I.A2 wherein Z is Z-335, R³ is CH₃ and Q is Q3, and R⁴ is as per any row of Table V below, which compounds are named I.A2-Z335-Q3-V-1 to I.A2-Z335-Q3-V-6.

In another embodiment, compounds I are of formula I.A2 wherein Z is Z-335, R³ is CH₃ and Q is Q4, and R⁴ is as per any row of Table V below, which compounds are named I.A2-Z335-Q4-V-1 to I.A2-Z335-Q4-V-6.

In another embodiment, compounds I are of formula I.A1 wherein Z is Z-336, R³ is CH₃ and Q is Q1, and R⁴ is as per any row of Table V below, which compounds are named I.A1-Z336-Q1-V-1 to I.A1-Z336-Q1-V-6.

In another embodiment, compounds I are of formula I.A1 wherein Z is Z-336, R³ is CH₃ and Q is Q2, and R⁴ is as per any row of Table V below, which compounds are named I.A1-Z336-Q2-V-1 to I.A1-Z336-Q2-V-6.

In another embodiment, compounds I are of formula I.A1 wherein Z is Z-336, R³ is CH₃ and Q is Q3, and R⁴ is as per any row of Table V below, which compounds are named I.A1-Z336-Q3-V-1 to I.A1-Z336-Q3-V-6.

In another embodiment, compounds I are of formula I.A1 wherein Z is Z-336, R³ is CH₃ and Q is Q4, and R⁴ is as per any row of Table V below, which compounds are named I.A1-Z336-Q4-V-1 to I.A1-Z336-Q4-V-6.

In another embodiment, compounds I are of formula I.A2 wherein Z is Z-336, R³ is CH₃ and Q is Q1, and R⁴ is as per any row of Table V below, which compounds are named I.A2-Z336-Q1-V-1 to I.A2-Z336-Q1-V-6.

In another embodiment, compounds I are of formula I.A2 wherein Z is Z-336, R³ is CH₃ and Q is Q2, and R⁴ is as per any row of Table V below, which compounds are named I.A2-Z336-Q2-V-1 to I.A2-Z336-Q2-V-6.

In another embodiment, compounds I are of formula I.A2 wherein Z is Z-336, R³ is CH₃ and Q is Q3, and R⁴ is as per any row of Table V below, which compounds are named I.A2-Z336-Q3-V-1 to I.A2-Z336-Q3-V-6.

In another embodiment, compounds I are of formula I.A2 wherein Z is Z-336, R³ is CH₃ and Q is Q4, and R⁴ is as per any row of Table V below, which compounds are named I.A2-Z336-Q4-V-1 to I.A2-Z336-Q4-V-6.

In another embodiment, compounds I are of formula I.A1 wherein Z is Z-337, R³ is CH₃ and Q is Q1, and R⁴ is as per any row of Table V below, which compounds are named I.A1-Z337-Q1-V-1 to I.A1-Z337-Q1-V-6.

In another embodiment, compounds I are of formula I.A1 wherein Z is Z-337, R³ is CH₃ and Q is Q2, and R⁴ is as per any row of Table V below, which compounds are named I.A1-Z337-Q2-V-1 to I.A1-Z337-Q2-V-6.

In another embodiment, compounds I are of formula I.A1 wherein Z is Z-337, R³ is CH₃ and Q is Q3, and R⁴ is as per any row of Table V below, which compounds are named I.A1-Z337-Q3-V-1 to I.A1-Z337-Q3-V-6.

In another embodiment, compounds I are of formula I.A1 wherein Z is Z-337, R³ is CH₃ and Q is Q4, and R⁴ is as per any row of Table V below, which compounds are named I.A1-Z337-Q4-V-1 to I.A1-Z337-Q4-V-6.

In another embodiment, compounds I are of formula I.A2 wherein Z is Z-337, R³ is CH₃ and Q is Q1, and R⁴ is as per any row of Table V below, which compounds are named I.A2-Z337-Q1-V-1 to I.A2-Z337-Q1-V-6.

In another embodiment, compounds I are of formula I.A2 wherein Z is Z-337, R³ is CH₃ and Q is Q2, and R⁴ is as per any row of Table V below, which compounds are named I.A2-Z337-Q2-V-1 to I.A2-Z337-Q2-V-6.

In another embodiment, compounds I are of formula I.A2 wherein Z is Z-337, R³ is CH₃ and Q is Q3, and R⁴ is as per any row of Table V below, which compounds are named I.A2-Z337-Q3-V-1 to I.A2-Z337-Q3-V-6.

In another embodiment, compounds I are of formula I.A2 wherein Z is Z-337, R³ is CH₃ and Q is Q4, and R⁴ is as per any row of Table V below, which compounds are named I.A2-Z337-Q4-V-1 to I.A2-Z337-Q4-V-6.

In another embodiment, compounds I are of formula I.A1 wherein Z is Z-338, R³ is CH₃ and Q is Q1, and R⁴ is as per any row of Table V below, which compounds are named I.A1-Z338-Q1-V-1 to I.A1-Z338-Q1-V-6.

In another embodiment, compounds I are of formula I.A1 wherein Z is Z-338, R³ is CH₃ and Q is Q2, and R⁴ is as per any row of Table V below, which compounds are named I.A1-Z338-Q2-V-1 to I.A1-Z338-Q2-V-6.

In another embodiment, compounds I are of formula I.A1 wherein Z is Z-338, R³ is CH₃ and Q is Q3, and R⁴ is as per any row of Table V below, which compounds are named I.A1-Z338-Q3-V-1 to I.A1-Z338-Q3-V-6.

In another embodiment, compounds I are of formula I.A1 wherein Z is Z-338, R³ is CH₃ and Q is Q4, and R⁴ is as per any row of Table V below, which compounds are named I.A1-Z338-Q4-V-1 to I.A1-Z338-Q4-V-6.

In another embodiment, compounds I are of formula I.A2 wherein Z is Z-338, R³ is CH₃ and Q is Q1, and R⁴ is as per any row of Table V below, which compounds are named I.A2-Z338-Q1-V-1 to I.A2-Z338-Q1-V-6.

In another embodiment, compounds I are of formula I.A2 wherein Z is Z-338, R³ is CH₃ and Q is Q2, and R⁴ is as per any row of Table V below, which compounds are named I.A2-Z338-Q2-V-1 to I.A2-Z338-Q2-V-6.

In another embodiment, compounds I are of formula I.A2 wherein Z is Z-338, R³ is CH₃ and Q is Q3, and R⁴ is as per any row of Table V below, which compounds are named I.A2-Z338-Q3-V-1 to I.A2-Z338-Q3-V-6.

In another embodiment, compounds I are of formula I.A2 wherein Z is Z-338, R³ is CH₃ and Q is Q4, and R⁴ is as per any row of Table V below, which compounds are named I.A2-Z338-Q4-V-1 to I.A2-Z338-Q4-V-6.

In another embodiment, compounds I are of formula I.A1 wherein Z is Z-339, R³ is CH₃ and Q is Q1, and R⁴ is as per any row of Table V below, which compounds are named I.A1-Z339-Q1-V-1 to I.A1-Z339-Q1-V-6.

In another embodiment, compounds I are of formula I.A1 wherein Z is Z-339, R³ is CH₃ and Q is Q2, and R⁴ is as per any row of Table V below, which compounds are named I.A1-Z339-Q2-V-1 to I.A1-Z339-Q2-V-6.

In another embodiment, compounds I are of formula I.A1 wherein Z is Z-339, R³ is CH₃ and Q is Q3, and R⁴ is as per any row of Table V below, which compounds are named I.A1-Z339-Q3-V-1 to I.A1-Z339-Q3-V-6.

In another embodiment, compounds I are of formula I.A1 wherein Z is Z-339, R³ is CH₃ and Q is Q4, and R⁴ is as per any row of Table V below, which compounds are named I.A1-Z339-Q4-V-1 to I.A1-Z339-Q4-V-6.

In another embodiment, compounds I are of formula I.A2 wherein Z is Z-339, R³ is CH₃ and Q is Q1, and R⁴ is as per any row of Table V below, which compounds are named I.A2-Z339-Q1-V-1 to I.A2-Z339-Q1-V-6.

In another embodiment, compounds I are of formula I.A2 wherein Z is Z-339, R³ is CH₃ and Q is Q2, and R⁴ is as per any row of Table V below, which compounds are named I.A2-Z339-Q2-V-1 to I.A2-Z339-Q2-V-6.

In another embodiment, compounds I are of formula I.A2 wherein Z is Z-339, R³ is CH₃ and Q is Q3, and R⁴ is as per any row of Table V below, which compounds are named I.A2-Z339-Q3-V-1 to I.A2-Z339-Q3-V-6.

In another embodiment, compounds I are of formula I.A2 wherein Z is Z-339, R³ is CH₃ and Q is Q4, and R⁴ is as per any row of Table V below, which compounds are named I.A2-Z339-Q4-V-1 to I.A2-Z339-Q4-V-6.

In another embodiment, compounds I are of formula I.A1 wherein Z is Z-340, R³ is CH₃ and Q is Q1, and R⁴ is as per any row of Table V below, which compounds are named I.A1-Z340-Q1-V-1 to I.A1-Z340-Q1-V-6.

In another embodiment, compounds I are of formula I.A1 wherein Z is Z-340, R³ is CH₃ and Q is Q2, and R⁴ is as per any row of Table V below, which compounds are named I.A1-Z340-Q2-V-1 to I.A1-Z340-Q2-V-6.

In another embodiment, compounds I are of formula I.A1 wherein Z is Z-340, R³ is CH₃ and Q is Q3, and R⁴ is as per any row of Table V below, which compounds are named I.A1-Z340-Q3-V-1 to I.A1-Z340-Q3-V-6.

In another embodiment, compounds I are of formula I.A1 wherein Z is Z-340, R³ is CH₃ and Q is Q4, and R⁴ is as per any row of Table V below, which compounds are named I.A1-Z340-Q4-V-1 to I.A1-Z340-Q4-V-6.

In another embodiment, compounds I are of formula I.A2 wherein Z is Z-340, R³ is CH₃ and Q is Q1, and R⁴ is as per any row of Table V below, which compounds are named I.A2-Z340-Q1-V-1 to I.A2-Z340-Q1-V-6.

In another embodiment, compounds I are of formula I.A2 wherein Z is Z-340, R³ is CH₃ and Q is Q2, and R⁴ is as per any row of Table V below, which compounds are named I.A2-Z340-Q2-V-1 to I.A2-Z340-Q2-V-6.

In another embodiment, compounds I are of formula I.A2 wherein Z is Z-340, R³ is CH₃ and Q is Q3, and R⁴ is as per any row of Table V below, which compounds are named I.A2-Z340-Q3-V-1 to I.A2-Z340-Q3-V-6.

In another embodiment, compounds I are of formula I.A2 wherein Z is Z-340, R³ is CH₃ and Q is Q4, and R⁴ is as per any row of Table V below, which compounds are named I.A2-Z340-Q4-V-1 to I.A2-Z340-Q4-V-6.

In another embodiment, compounds I are of formula I.A1 wherein Z is Z-341, R³ is CH₃ and Q is Q1, and R⁴ is as per any row of Table V below, which compounds are named I.A1-Z341-Q1-V-1 to I.A1-Z341-Q1-V-6.

In another embodiment, compounds I are of formula I.A1 wherein Z is Z-341, R³ is CH₃ and Q is Q2, and R⁴ is as per any row of Table V below, which compounds are named I.A1-Z341-Q2-V-1 to I.A1-Z341-Q2-V-6.

In another embodiment, compounds I are of formula I.A1 wherein Z is Z-341, R³ is CH₃ and Q is Q3, and R⁴ is as per any row of Table V below, which compounds are named I.A1-Z341-Q3-V-1 to I.A1-Z341-Q3-V-6.

In another embodiment, compounds I are of formula I.A1 wherein Z is Z-341, R³ is CH₃ and Q is Q4, and R⁴ is as per any row of Table V below, which compounds are named I.A1-Z341-Q4-V-1 to I.A1-Z341-Q4-V-6.

In another embodiment, compounds I are of formula I.A2 wherein Z is Z-341, R³ is CH₃ and Q is Q1, and R⁴ is as per any row of Table V below, which compounds are named I.A2-Z341-Q1-V-1 to I.A2-Z341-Q1-V-6.

In another embodiment, compounds I are of formula I.A2 wherein Z is Z-341, R³ is CH₃ and Q is Q2, and R⁴ is as per any row of Table V below, which compounds are named I.A2-Z341-Q2-V-1 to I.A2-Z341-Q2-V-6.

In another embodiment, compounds I are of formula I.A2 wherein Z is Z-341, R³ is CH₃ and Q is Q3, and R⁴ is as per any row of Table V below, which compounds are named I.A2-Z341-Q3-V-1 to I.A2-Z341-Q3-V-6.

In another embodiment, compounds I are of formula I.A2 wherein Z is Z-341, R³ is CH₃ and Q is Q4, and R⁴ is as per any row of Table V below, which compounds are named I.A2-Z341-Q4-V-1 to I.A2-Z341-Q4-V-6.

In another embodiment, compounds I are of formula I.A1 wherein Z is Z-342, R³ is CH₃ and Q is Q1, and R⁴ is as per any row of Table V below, which compounds are named I.A1-Z342-Q1-V-1 to I.A1-Z342-Q1-V-6.

In another embodiment, compounds I are of formula I.A1 wherein Z is Z-342, R³ is CH₃ and Q is Q2, and R⁴ is as per any row of Table V below, which compounds are named I.A1-Z342-Q2-V-1 to I.A1-Z342-Q2-V-6.

In another embodiment, compounds I are of formula I.A1 wherein Z is Z-342, R³ is CH₃ and Q is Q3, and R⁴ is as per any row of Table V below, which compounds are named I.A1-Z342-Q3-V-1 to I.A1-Z342-Q3-V-6.

In another embodiment, compounds I are of formula I.A1 wherein Z is Z-342, R³ is CH₃ and Q is Q4, and R⁴ is as per any row of Table V below, which compounds are named I.A1-Z342-Q4-V-1 to I.A1-Z342-Q4-V-6.

In another embodiment, compounds I are of formula I.A2 wherein Z is Z-342, R³ is CH₃ and Q is Q1, and R⁴ is as per any row of Table V below, which compounds are named I.A2-Z342-Q1-V-1 to I.A2-Z342-Q1-V-6.

In another embodiment, compounds I are of formula I.A2 wherein Z is Z-342, R³ is CH₃ and Q is Q2, and R⁴ is as per any row of Table V below, which compounds are named I.A2-Z342-Q2-V-1 to I.A2-Z342-Q2-V-6.

In another embodiment, compounds I are of formula I.A2 wherein Z is Z-342, R³ is CH₃ and Q is Q3, and R⁴ is as per any row of Table V below, which compounds are named I.A2-Z342-Q3-V-1 to I.A2-Z342-Q3-V-6.

In another embodiment, compounds I are of formula I.A2 wherein Z is Z-342, R³ is CH₃ and Q is Q4, and R⁴ is as per any row of Table V below, which compounds are named I.A2-Z342-Q4-V-1 to I.A2-Z342-Q4-V-6.

In another embodiment, compounds I are of formula I.B1 wherein Z is Z-323 and Q is Q1, and R⁴ is as per any row of Table V below, which compounds are named I.B1-Z323-Q1-V-1 to I.B1-Z323-Q1-V-6.

In another embodiment, compounds I are of formula I.B1 wherein Z is Z-323 and Q is Q2, and R⁴ is as per any row of Table V below, which compounds are named I.B1-Z323-Q2-V-1 to I.B1-Z323-Q2-V-6.

In another embodiment, compounds I are of formula I.B1 wherein Z is Z-323 and Q is Q3, and R⁴ is as per any row of Table V below, which compounds are named I.B1-Z323-Q3-V-1 to I.B1-Z323-Q3-V-6.

In another embodiment, compounds I are of formula I.B1 wherein Z is Z-323 and Q is Q4, and R⁴ is as per any row of Table V below, which compounds are named I.B1-Z323-Q4-V-1 to I.B1-Z323-Q4-V-6.

In another embodiment, compounds I are of formula I.B2 wherein Z is Z-323 and Q is Q1, and R⁴ is as per any row of Table V below, which compounds are named I.B2-Z323-Q1-V-1 to I.B2-Z323-Q1-V-6.

In another embodiment, compounds I are of formula I.B2 wherein Z is Z-323 and Q is Q2, and R⁴ is as per any row of Table V below, which compounds are named I.B2-Z323-Q2-V-1 to I. B2-Z323-Q2-V-6.

In another embodiment, compounds I are of formula I.B2 wherein Z is Z-323 and Q is Q3, and R⁴ is as per any row of Table V below, which compounds are named I.B2-Z323-Q3-V-1 to I. B2-Z323-Q3-V-6.

In another embodiment, compounds I are of formula I.B2 wherein Z is Z-323 and Q is Q4, and R⁴ is as per any row of Table V below, which compounds are named I.B2-Z323-Q4-V-1 to I. B2-Z323-Q4-V-6.

In another embodiment, compounds I are of formula I.B1 wherein Z is Z-324 and Q is Q1, and R⁴ is as per any row of Table V below, which compounds are named I.B1-Z324-Q1-V-1 to I.B1-Z324-Q1-V-6.

In another embodiment, compounds I are of formula I.B1 wherein Z is Z-324 and Q is Q2, and R⁴ is as per any row of Table V below, which compounds are named I.B1-Z324-Q2-V-1 to I.B1-Z324-Q2-V-6.

In another embodiment, compounds I are of formula I.B1 wherein Z is Z-324 and Q is Q3, and R⁴ is as per any row of Table V below, which compounds are named I.B1-Z324-Q3-V-1 to I.B1-Z324-Q3-V-6.

In another embodiment, compounds I are of formula I.B1 wherein Z is Z-324 and Q is Q4, and R⁴ is as per any row of Table V below, which compounds are named I.B1-Z324-Q4-V-1 to I.B1-Z324-Q4-V-6.

In another embodiment, compounds I are of formula I.B2 wherein Z is Z-324 and Q is Q1, and R⁴ is as per any row of Table V below, which compounds are named I.B2-Z324-Q1-V-1 to I.B2-Z324-Q1-V-6.

In another embodiment, compounds I are of formula I.B2 wherein Z is Z-324 and Q is Q2, and R⁴ is as per any row of Table V below, which compounds are named I.B2-Z324-Q2-V-1 to I. B2-Z324-Q2-V-6.

In another embodiment, compounds I are of formula I.B2 wherein Z is Z-324 and Q is Q3, and R⁴ is as per any row of Table V below, which compounds are named I.B2-Z324-Q3-V-1 to I. B2-Z324-Q3-V-6.

In another embodiment, compounds I are of formula I.B2 wherein Z is Z-324 and Q is Q4, and R⁴ is as per any row of Table V below, which compounds are named I.B2-Z324-Q4-V-1 to I. B2-Z324-Q4-V-6.

In another embodiment, compounds I are of formula I.B1 wherein Z is Z-325 and Q is Q1, and R⁴ is as per any row of Table V below, which compounds are named I.B1-Z325-Q1-V-1 to I.B1-Z325-Q1-V-6.

In another embodiment, compounds I are of formula I.B1 wherein Z is Z-325 and Q is Q2, and R⁴ is as per any row of Table V below, which compounds are named I.B1-Z325-Q2-V-1 to I.B1-Z325-Q2-V-6.

In another embodiment, compounds I are of formula I.B1 wherein Z is Z-325 and Q is Q3, and R⁴ is as per any row of Table V below, which compounds are named I.B1-Z325-Q3-V-1 to I.B1-Z325-Q3-V-6.

In another embodiment, compounds I are of formula I.B1 wherein Z is Z-325 and Q is Q4, and R⁴ is as per any row of Table V below, which compounds are named I.B1-Z325-Q4-V-1 to I.B1-Z325-Q4-V-6.

In another embodiment, compounds I are of formula I.B2 wherein Z is Z-325 and Q is Q1, and R⁴ is as per any row of Table V below, which compounds are named I.B2-Z325-Q1-V-1 to I.B2-Z325-Q1-V-6.

In another embodiment, compounds I are of formula I.B2 wherein Z is Z-325 and Q is Q2, and R⁴ is as per any row of Table V below, which compounds are named I.B2-Z325-Q2-V-1 to I. B2-Z325-Q2-V-6.

In another embodiment, compounds I are of formula I.B2 wherein Z is Z-325 and Q is Q3, and R⁴ is as per any row of Table V below, which compounds are named I.B2-Z325-Q3-V-1 to I. B2-Z325-Q3-V-6.

In another embodiment, compounds I are of formula I.B2 wherein Z is Z-325 and Q is Q4, and R⁴ is as per any row of Table V below, which compounds are named I.B2-Z325-Q4-V-1 to I. B2-Z325-Q4-V-6.

In another embodiment, compounds I are of formula I.B1 wherein Z is Z-326 and Q is Q1, and R⁴ is as per any row of Table V below, which compounds are named I.B1-Z326-Q1-V-1 to I.B1-Z326-Q1-V-6.

In another embodiment, compounds I are of formula I.B1 wherein Z is Z-326 and Q is Q2, and R⁴ is as per any row of Table V below, which compounds are named I.B1-Z326-Q2-V-1 to I.B1-Z326-Q2-V-6.

In another embodiment, compounds I are of formula I.B1 wherein Z is Z-326 and Q is Q3, and R⁴ is as per any row of Table V below, which compounds are named I.B1-Z326-Q3-V-1 to I.B1-Z326-Q3-V-6.

In another embodiment, compounds I are of formula I.B1 wherein Z is Z-326 and Q is Q4, and R⁴ is as per any row of Table V below, which compounds are named I.B1-Z326-Q4-V-1 to I.B1-Z326-Q4-V-6.

In another embodiment, compounds I are of formula I.B2 wherein Z is Z-326 and Q is Q1, and R⁴ is as per any row of Table V below, which compounds are named I.B2-Z326-Q1-V-1 to I.B2-Z326-Q1-V-6.

In another embodiment, compounds I are of formula I.B2 wherein Z is Z-326 and Q is Q2, and R⁴ is as per any row of Table V below, which compounds are named I.B2-Z326-Q2-V-1 to I. B2-Z326-Q2-V-6.

In another embodiment, compounds I are of formula I.B2 wherein Z is Z-326 and Q is Q3, and R⁴ is as per any row of Table V below, which compounds are named I.B2-Z326-Q3-V-1 to I. B2-Z326-Q3-V-6.

In another embodiment, compounds I are of formula I.B2 wherein Z is Z-326 and Q is Q4, and R⁴ is as per any row of Table V below, which compounds are named I.B2-Z326-Q4-V-1 to I. B2-Z326-Q4-V-6.

In another embodiment, compounds I are of formula I.B1 wherein Z is Z-327 and Q is Q1, and R⁴ is as per any row of Table V below, which compounds are named I.B1-Z327-Q1-V-1 to I.B1-Z327-Q1-V-6.

In another embodiment, compounds I are of formula I.B1 wherein Z is Z-327 and Q is Q2, and R⁴ is as per any row of Table V below, which compounds are named I.B1-Z327-Q2-V-1 to I.B1-Z327-Q2-V-6.

In another embodiment, compounds I are of formula I.B1 wherein Z is Z-327 and Q is Q3, and R⁴ is as per any row of Table V below, which compounds are named I.B1-Z327-Q3-V-1 to I.B1-Z327-Q3-V-6.

In another embodiment, compounds I are of formula I.B1 wherein Z is Z-327 and Q is Q4, and R⁴ is as per any row of Table V below, which compounds are named I.B1-Z327-Q4-V-1 to I.B1-Z327-Q4-V-6.

In another embodiment, compounds I are of formula I.B2 wherein Z is Z-327 and Q is Q1, and R⁴ is as per any row of Table V below, which compounds are named I.B2-Z327-Q1-V-1 to I.B2-Z327-Q1-V-6.

In another embodiment, compounds I are of formula I.B2 wherein Z is Z-327 and Q is Q2, and R⁴ is as per any row of Table V below, which compounds are named I.B2-Z327-Q2-V-1 to I.B2-Z327-Q2-V-6.

In another embodiment, compounds I are of formula I.B2 wherein Z is Z-327 and Q is Q3, and R⁴ is as per any row of Table V below, which compounds are named I.B2-Z327-Q3-V-1 to I.B2-Z327-Q3-V-6.

In another embodiment, compounds I are of formula I.B2 wherein Z is Z-327 and Q is Q4, and R⁴ is as per any row of Table V below, which compounds are named I.B2-Z327-Q4-V-1 to I.B2-Z327-Q4-V-6.

In another embodiment, compounds I are of formula I.B1 wherein Z is Z-328 and Q is Q1, and R⁴ is as per any row of Table V below, which compounds are named I.B1-Z328-Q1-V-1 to I.B1-Z328-Q1-V-6.

In another embodiment, compounds I are of formula I.B1 wherein Z is Z-328 and Q is Q2, and R⁴ is as per any row of Table V below, which compounds are named I.B1-Z328-Q2-V-1 to I.B1-Z328-Q2-V-6.

In another embodiment, compounds I are of formula I.B1 wherein Z is Z-328 and Q is Q3, and R⁴ is as per any row of Table V below, which compounds are named I.B1-Z328-Q3-V-1 to I.B1-Z328-Q3-V-6.

In another embodiment, compounds I are of formula I.B1 wherein Z is Z-328 and Q is Q4, and R⁴ is as per any row of Table V below, which compounds are named I.B1-Z328-Q4-V-1 to I.B1-Z328-Q4-V-6.

In another embodiment, compounds I are of formula I.B2 wherein Z is Z-328 and Q is Q1, and R⁴ is as per any row of Table V below, which compounds are named I.B2-Z328-Q1-V-1 to I.B2-Z328-Q1-V-6.

In another embodiment, compounds I are of formula I.B2 wherein Z is Z-328 and Q is Q2, and R⁴ is as per any row of Table V below, which compounds are named I.B2-Z328-Q2-V-1 to I. B2-Z328-Q2-V-6.

In another embodiment, compounds I are of formula I.B2 wherein Z is Z-328 and Q is Q3, and R⁴ is as per any row of Table V below, which compounds are named I.B2-Z328-Q3-V-1 to I. B2-Z328-Q3-V-6.

In another embodiment, compounds I are of formula I.B2 wherein Z is Z-328 and Q is Q4, and R⁴ is as per any row of Table V below, which compounds are named I.B2-Z328-Q4-V-1 to I.B2-Z328-Q4-V-6.

In another embodiment, compounds I are of formula I.B1 wherein Z is Z-329 and Q is Q1, and R⁴ is as per any row of Table V below, which compounds are named I.B1-Z329-Q1-V-1 to I.B1-Z329-Q1-V-6.

In another embodiment, compounds I are of formula I.B1 wherein Z is Z-329 and Q is Q2, and R⁴ is as per any row of Table V below, which compounds are named I.B1-Z329-Q2-V-1 to I.B1-Z329-Q2-V-6.

In another embodiment, compounds I are of formula I.B1 wherein Z is Z-329 and Q is Q3, and R⁴ is as per any row of Table V below, which compounds are named I.B1-Z329-Q3-V-1 to I.B1-Z329-Q3-V-6.

In another embodiment, compounds I are of formula I.B1 wherein Z is Z-329 and Q is Q4, and R⁴ is as per any row of Table V below, which compounds are named I.B1-Z329-Q4-V-1 to I.B1-Z329-Q4-V-6.

In another embodiment, compounds I are of formula I.B2 wherein Z is Z-329 and Q is Q1, and R⁴ is as per any row of Table V below, which compounds are named I.B2-Z329-Q1-V-1 to I.B2-Z329-Q1-V-6.

In another embodiment, compounds I are of formula I.B2 wherein Z is Z-329 and Q is Q2, and R⁴ is as per any row of Table V below, which compounds are named I.B2-Z329-Q2-V-1 to I. B2-Z329-Q2-V-6.

In another embodiment, compounds I are of formula I.B2 wherein Z is Z-329 and Q is Q3, and R⁴ is as per any row of Table V below, which compounds are named I.B2-Z329-Q3-V-1 to I. B2-Z329-Q3-V-6.

In another embodiment, compounds I are of formula I.B2 wherein Z is Z-329 and Q is Q4, and R⁴ is as per any row of Table V below, which compounds are named I.B2-Z329-Q4-V-1 to I. B2-Z329-Q4-V-6.

In another embodiment, compounds I are of formula I.B1 wherein Z is Z-330 and Q is Q1, and R⁴ is as per any row of Table V below, which compounds are named I.B1-Z330-Q1-V-1 to I.B1-Z330-Q1-V-6.

In another embodiment, compounds I are of formula I.B1 wherein Z is Z-330 and Q is Q2, and R⁴ is as per any row of Table V below, which compounds are named I.B1-Z330-Q2-V-1 to I.B1-Z330-Q2-V-6.

In another embodiment, compounds I are of formula I.B1 wherein Z is Z-330 and Q is Q3, and R⁴ is as per any row of Table V below, which compounds are named I.B1-Z330-Q3-V-1 to I.B1-Z330-Q3-V-6.

In another embodiment, compounds I are of formula I.B1 wherein Z is Z-330 and Q is Q4, and R⁴ is as per any row of Table V below, which compounds are named I.B1-Z330-Q4-V-1 to I.B1-Z330-Q4-V-6.

In another embodiment, compounds I are of formula I.B2 wherein Z is Z-330 and Q is Q1, and R⁴ is as per any row of Table V below, which compounds are named I.B2-Z330-Q1-V-1 to I.B2-Z330-Q1-V-6.

In another embodiment, compounds I are of formula I.B2 wherein Z is Z-330 and Q is Q2, and R⁴ is as per any row of Table V below, which compounds are named I.B2-Z330-Q2-V-1 to I. B2-Z330-Q2-V-6.

In another embodiment, compounds I are of formula I.B2 wherein Z is Z-330 and Q is Q3, and R⁴ is as per any row of Table V below, which compounds are named I.B2-Z330-Q3-V-1 to I. B2-Z330-Q3-V-6.

In another embodiment, compounds I are of formula I.B2 wherein Z is Z-330 and Q is Q4, and R⁴ is as per any row of Table V below, which compounds are named I.B2-Z330-Q4-V-1 to I. B2-Z330-Q4-V-6.

In another embodiment, compounds I are of formula I.B1 wherein Z is Z-331 and Q is Q1, and R⁴ is as per any row of Table V below, which compounds are named I.B1-Z331-Q1-V-1 to I.B1-Z331-Q1-V-6.

In another embodiment, compounds I are of formula I.B1 wherein Z is Z-331 and Q is Q2, and R⁴ is as per any row of Table V below, which compounds are named I.B1-Z331-Q2-V-1 to I.B1-Z331-Q2-V-6.

In another embodiment, compounds I are of formula I.B1 wherein Z is Z-331 and Q is Q3, and R⁴ is as per any row of Table V below, which compounds are named I.B1-Z331-Q3-V-1 to I.B1-Z331-Q3-V-6.

In another embodiment, compounds I are of formula I.B1 wherein Z is Z-331 and Q is Q4, and R⁴ is as per any row of Table V below, which compounds are named I.B1-Z331-Q4-V-1 to I.B1-Z331-Q4-V-6.

In another embodiment, compounds I are of formula I.B2 wherein Z is Z-331 and Q is Q1, and R⁴ is as per any row of Table V below, which compounds are named I.B2-Z331-Q1-V-1 to I.B2-Z331-Q1-V-6.

In another embodiment, compounds I are of formula I.B2 wherein Z is Z-331 and Q is Q2, and R⁴ is as per any row of Table V below, which compounds are named I.B2-Z331-Q2-V-1 to I.B2-Z331-Q2-V-6.

In another embodiment, compounds I are of formula I.B2 wherein Z is Z-331 and Q is Q3, and R⁴ is as per any row of Table V below, which compounds are named I.B2-Z331-Q3-V-1 to I.B2-Z331-Q3-V-6.

In another embodiment, compounds I are of formula I.B2 wherein Z is Z-331 and Q is Q4, and R⁴ is as per any row of Table V below, which compounds are named I.B2-Z331-Q4-V-1 to I.B2-Z331-Q4-V-6.

In another embodiment, compounds I are of formula I.B1 wherein Z is Z-332 and Q is Q1, and R⁴ is as per any row of Table V below, which compounds are named I.B1-Z332-Q1-V-1 to I.B1-Z332-Q1-V-6.

In another embodiment, compounds I are of formula I.B1 wherein Z is Z-332 and Q is Q2, and R⁴ is as per any row of Table V below, which compounds are named I.B1-Z332-Q2-V-1 to I.B1-Z332-Q2-V-6.

In another embodiment, compounds I are of formula I.B1 wherein Z is Z-332 and Q is Q3, and R⁴ is as per any row of Table V below, which compounds are named I.B1-Z332-Q3-V-1 to I.B1-Z332-Q3-V-6.

In another embodiment, compounds I are of formula I.B1 wherein Z is Z-332 and Q is Q4, and R⁴ is as per any row of Table V below, which compounds are named I.B1-Z332-Q4-V-1 to I.B1-Z332-Q4-V-6.

In another embodiment, compounds I are of formula I.B2 wherein Z is Z-332 and Q is Q1, and R⁴ is as per any row of Table V below, which compounds are named I.B2-Z332-Q1-V-1 to I.B2-Z332-Q1-V-6.

In another embodiment, compounds I are of formula I.B2 wherein Z is Z-332 and Q is Q2, and R⁴ is as per any row of Table V below, which compounds are named I.B2-Z332-Q2-V-1 to I.B2-Z332-Q2-V-6.

In another embodiment, compounds I are of formula I.B2 wherein Z is Z-332 and Q is Q3, and R⁴ is as per any row of Table V below, which compounds are named I.B2-Z332-Q3-V-1 to I.B2-Z332-Q3-V-6.

In another embodiment, compounds I are of formula I.B2 wherein Z is Z-332 and Q is Q4, and R⁴ is as per any row of Table V below, which compounds are named I.B2-Z332-Q4-V-1 to I.B2-Z332-Q4-V-6.

In another embodiment, compounds I are of formula I.B1 wherein Z is Z-333 and Q is Q1, and R⁴ is as per any row of Table V below, which compounds are named I.B1-Z333-Q1-V-1 to I.B1-Z333-Q1-V-6.

In another embodiment, compounds I are of formula I.B1 wherein Z is Z-333 and Q is Q2, and R⁴ is as per any row of Table V below, which compounds are named I.B1-Z333-Q2-V-1 to I.B1-Z333-Q2-V-6.

In another embodiment, compounds I are of formula I.B1 wherein Z is Z-333 and Q is Q3, and R⁴ is as per any row of Table V below, which compounds are named I.B1-Z333-Q3-V-1 to I.B1-Z333-Q3-V-6.

In another embodiment, compounds I are of formula I.B1 wherein Z is Z-333 and Q is Q4, and R⁴ is as per any row of Table V below, which compounds are named I.B1-Z333-Q4-V-1 to I.B1-Z333-Q4-V-6.

In another embodiment, compounds I are of formula I.B2 wherein Z is Z-333 and Q is Q1, and R⁴ is as per any row of Table V below, which compounds are named I.B2-Z333-Q1-V-1 to I.B2-Z333-Q1-V-6.

In another embodiment, compounds I are of formula I.B2 wherein Z is Z-333 and Q is Q2, and R⁴ is as per any row of Table V below, which compounds are named I.B2-Z333-Q2-V-1 to I.B2-Z333-Q2-V-6.

In another embodiment, compounds I are of formula I.B2 wherein Z is Z-333 and Q is Q3, and R⁴ is as per any row of Table V below, which compounds are named I.B2-Z333-Q3-V-1 to I.B2-Z333-Q3-V-6.

In another embodiment, compounds I are of formula I.B2 wherein Z is Z-333 and Q is Q4, and R⁴ is as per any row of Table V below, which compounds are named I.B2-Z333-Q4-V-1 to I.B2-Z333-Q4-V-6.

In another embodiment, compounds I are of formula I.B1 wherein Z is Z-334 and Q is Q1, and R⁴ is as per any row of Table V below, which compounds are named I.B1-Z334-Q1-V-1 to I.B1-Z334-Q1-V-6.

In another embodiment, compounds I are of formula I.B1 wherein Z is Z-334 and Q is Q2, and R⁴ is as per any row of Table V below, which compounds are named I.B1-Z334-Q2-V-1 to I.B1-Z334-Q2-V-6.

In another embodiment, compounds I are of formula I.B1 wherein Z is Z-334 and Q is Q3, and R⁴ is as per any row of Table V below, which compounds are named I.B1-Z334-Q3-V-1 to I.B1-Z334-Q3-V-6.

In another embodiment, compounds I are of formula I.B1 wherein Z is Z-334 and Q is Q4, and R⁴ is as per any row of Table V below, which compounds are named I.B1-Z334-Q4-V-1 to I.B1-Z334-Q4-V-6.

In another embodiment, compounds I are of formula I.B2 wherein Z is Z-334 and Q is Q1, and R⁴ is as per any row of Table V below, which compounds are named I.B2-Z334-Q1-V-1 to I.B2-Z334-Q1-V-6.

In another embodiment, compounds I are of formula I.B2 wherein Z is Z-334 and Q is Q2, and R⁴ is as per any row of Table V below, which compounds are named I.B2-Z334-Q2-V-1 to I.B2-Z334-Q2-V-6.

In another embodiment, compounds I are of formula I.B2 wherein Z is Z-334 and Q is Q3, and R⁴ is as per any row of Table V below, which compounds are named I.B2-Z334-Q3-V-1 to I.B2-Z334-Q3-V-6.

In another embodiment, compounds I are of formula I.B2 wherein Z is Z-334 and Q is Q4, and R⁴ is as per any row of Table V below, which compounds are named I.B2-Z334-Q4-V-1 to I.B2-Z334-Q4-V-6.

In another embodiment, compounds I are of formula I.B1 wherein Z is Z-335 and Q is Q1, and R⁴ is as per any row of Table V below, which compounds are named I.B1-Z335-Q1-V-1 to I.B1-Z335-Q1-V-6.

In another embodiment, compounds I are of formula I.B1 wherein Z is Z-335 and Q is Q2, and R⁴ is as per any row of Table V below, which compounds are named I.B1-Z335-Q2-V-1 to I.B1-Z335-Q2-V-6.

In another embodiment, compounds I are of formula I.B1 wherein Z is Z-335 and Q is Q3, and R⁴ is as per any row of Table V below, which compounds are named I.B1-Z335-Q3-V-1 to I.B1-Z335-Q3-V-6.

In another embodiment, compounds I are of formula I.B1 wherein Z is Z-335 and Q is Q4, and R⁴ is as per any row of Table V below, which compounds are named I.B1-Z335-Q4-V-1 to I.B1-Z335-Q4-V-6.

In another embodiment, compounds I are of formula I.B2 wherein Z is Z-335 and Q is Q1, and R⁴ is as per any row of Table V below, which compounds are named I.B2-Z335-Q1-V-1 to I.B2-Z335-Q1-V-6.

In another embodiment, compounds I are of formula I.B2 wherein Z is Z-335 and Q is Q2, and R⁴ is as per any row of Table V below, which compounds are named I.B2-Z335-Q2-V-1 to I. B2-Z335-Q2-V-6.

In another embodiment, compounds I are of formula I.B2 wherein Z is Z-335 and Q is Q3, and R⁴ is as per any row of Table V below, which compounds are named I.B2-Z335-Q3-V-1 to I. B2-Z335-Q3-V-6.

In another embodiment, compounds I are of formula I.B2 wherein Z is Z-335 and Q is Q4, and R⁴ is as per any row of Table V below, which compounds are named I.B2-Z335-Q4-V-1 to I. B2-Z335-Q4-V-6.

In another embodiment, compounds I are of formula I.B1 wherein Z is Z-336 and Q is Q1, and R⁴ is as per any row of Table V below, which compounds are named I.B1-Z336-Q1-V-1 to I.B1-Z336-Q1-V-6.

In another embodiment, compounds I are of formula I.B1 wherein Z is Z-336 and Q is Q2, and R⁴ is as per any row of Table V below, which compounds are named I.B1-Z336-Q2-V-1 to I.B1-Z336-Q2-V-6.

In another embodiment, compounds I are of formula I.B1 wherein Z is Z-336 and Q is Q3, and R⁴ is as per any row of Table V below, which compounds are named I.B1-Z336-Q3-V-1 to I.B1-Z336-Q3-V-6.

In another embodiment, compounds I are of formula I.B1 wherein Z is Z-336 and Q is Q4, and R⁴ is as per any row of Table V below, which compounds are named I.B1-Z336-Q4-V-1 to I.B1-Z336-Q4-V-6.

In another embodiment, compounds I are of formula I.B2 wherein Z is Z-336 and Q is Q1, and R⁴ is as per any row of Table V below, which compounds are named I.B2-Z336-Q1-V-1 to I.B2-Z336-Q1-V-6.

In another embodiment, compounds I are of formula I.B2 wherein Z is Z-336 and Q is Q2, and R⁴ is as per any row of Table V below, which compounds are named I.B2-Z336-Q2-V-1 to I.B2-Z336-Q2-V-6.

In another embodiment, compounds I are of formula I.B2 wherein Z is Z-336 and Q is Q3, and R⁴ is as per any row of Table V below, which compounds are named I.B2-Z336-Q3-V-1 to I. B2-Z336-Q3-V-6.

In another embodiment, compounds I are of formula I.B2 wherein Z is Z-336 and Q is Q4, and R⁴ is as per any row of Table V below, which compounds are named I.B2-Z336-Q4-V-1 to I.B2-Z336-Q4-V-6.

In another embodiment, compounds I are of formula I.B1 wherein Z is Z-337 and Q is Q1, and R⁴ is as per any row of Table V below, which compounds are named I.B1-Z337-Q1-V-1 to I.B1-Z337-Q1-V-6.

In another embodiment, compounds I are of formula I.B1 wherein Z is Z-337 and Q is Q2, and R⁴ is as per any row of Table V below, which compounds are named I.B1-Z337-Q2-V-1 to I.B1-Z337-Q2-V-6.

In another embodiment, compounds I are of formula I.B1 wherein Z is Z-337 and Q is Q3, and R⁴ is as per any row of Table V below, which compounds are named I.B1-Z337-Q3-V-1 to I.B1-Z337-Q3-V-6.

In another embodiment, compounds I are of formula I.B1 wherein Z is Z-337 and Q is Q4, and R⁴ is as per any row of Table V below, which compounds are named I.B1-Z337-Q4-V-1 to I.B1-Z337-Q4-V-6.

In another embodiment, compounds I are of formula I.B2 wherein Z is Z-337 and Q is Q1, and R⁴ is as per any row of Table V below, which compounds are named I.B2-Z337-Q1-V-1 to I.B2-Z337-Q1-V-6.

In another embodiment, compounds I are of formula I.B2 wherein Z is Z-337 and Q is Q2, and R⁴ is as per any row of Table V below, which compounds are named I.B2-Z337-Q2-V-1 to I.B2-Z337-Q2-V-6.

In another embodiment, compounds I are of formula I.B2 wherein Z is Z-337 and Q is Q3, and R⁴ is as per any row of Table V below, which compounds are named I.B2-Z337-Q3-V-1 to I.B2-Z337-Q3-V-6.

In another embodiment, compounds I are of formula I.B2 wherein Z is Z-337 and Q is Q4, and R⁴ is as per any row of Table V below, which compounds are named I.B2-Z337-Q4-V-1 to I.B2-Z337-Q4-V-6.

In another embodiment, compounds I are of formula I.B1 wherein Z is Z-338 and Q is Q1, and R⁴ is as per any row of Table V below, which compounds are named I.B1-Z338-Q1-V-1 to I.B1-Z338-Q1-V-6.

In another embodiment, compounds I are of formula I.B1 wherein Z is Z-338 and Q is Q2, and R⁴ is as per any row of Table V below, which compounds are named I.B1-Z338-Q2-V-1 to I.B1-Z338-Q2-V-6.

In another embodiment, compounds I are of formula I.B1 wherein Z is Z-338 and Q is Q3, and R⁴ is as per any row of Table V below, which compounds are named I.B1-Z338-Q3-V-1 to I.B1-Z338-Q3-V-6.

In another embodiment, compounds I are of formula I.B1 wherein Z is Z-338 and Q is Q4, and R⁴ is as per any row of Table V below, which compounds are named I.B1-Z338-Q4-V-1 to I.B1-Z338-Q4-V-6.

In another embodiment, compounds I are of formula I.B2 wherein Z is Z-338 and Q is Q1, and R⁴ is as per any row of Table V below, which compounds are named I.B2-Z338-Q1-V-1 to I.B2-Z338-Q1-V-6.

In another embodiment, compounds I are of formula I.B2 wherein Z is Z-338 and Q is Q2, and R⁴ is as per any row of Table V below, which compounds are named I.B2-Z338-Q2-V-1 to I.B2-Z338-Q2-V-6.

In another embodiment, compounds I are of formula I.B2 wherein Z is Z-338 and Q is Q3, and R⁴ is as per any row of Table V below, which compounds are named I.B2-Z338-Q3-V-1 to I.B2-Z338-Q3-V-6.

In another embodiment, compounds I are of formula I.B2 wherein Z is Z-338 and Q is Q4, and R⁴ is as per any row of Table V below, which compounds are named I.B2-Z338-Q4-V-1 to I.B2-Z338-Q4-V-6.

In another embodiment, compounds I are of formula I.B1 wherein Z is Z-339 and Q is Q1, and R⁴ is as per any row of Table V below, which compounds are named I.B1-Z339-Q1-V-1 to I.B1-Z339-Q1-V-6.

In another embodiment, compounds I are of formula I.B1 wherein Z is Z-339 and Q is Q2, and R⁴ is as per any row of Table V below, which compounds are named I.B1-Z339-Q2-V-1 to I.B1-Z339-Q2-V-6.

In another embodiment, compounds I are of formula I.B1 wherein Z is Z-339 and Q is Q3, and R⁴ is as per any row of Table V below, which compounds are named I.B1-Z339-Q3-V-1 to I.B1-Z339-Q3-V-6.

In another embodiment, compounds I are of formula I.B1 wherein Z is Z-339 and Q is Q4, and R⁴ is as per any row of Table V below, which compounds are named I.B1-Z339-Q4-V-1 to I.B1-Z339-Q4-V-6.

In another embodiment, compounds I are of formula I.B2 wherein Z is Z-339 and Q is Q1, and R⁴ is as per any row of Table V below, which compounds are named I.B2-Z339-Q1-V-1 to I.B2-Z339-Q1-V-6.

In another embodiment, compounds I are of formula I.B2 wherein Z is Z-339 and Q is Q2, and R⁴ is as per any row of Table V below, which compounds are named I.B2-Z339-Q2-V-1 to I.B2-Z339-Q2-V-6.

In another embodiment, compounds I are of formula I.B2 wherein Z is Z-339 and Q is Q3, and R⁴ is as per any row of Table V below, which compounds are named I.B2-Z339-Q3-V-1 to I.B2-Z339-Q3-V-6.

In another embodiment, compounds I are of formula I.B2 wherein Z is Z-339 and Q is Q4, and R⁴ is as per any row of Table V below, which compounds are named I.B2-Z339-Q4-V-1 to I.B2-Z339-Q4-V-6.

In another embodiment, compounds I are of formula I.B1 wherein Z is Z-340 and Q is Q1, and R⁴ is as per any row of Table V below, which compounds are named I.B1-Z340-Q1-V-1 to I.B1-Z340-Q1-V-6.

In another embodiment, compounds I are of formula I.B1 wherein Z is Z-340 and Q is Q2, and R⁴ is as per any row of Table V below, which compounds are named I.B1-Z340-Q2-V-1 to I.B1-Z340-Q2-V-6.

In another embodiment, compounds I are of formula I.B1 wherein Z is Z-340 and Q is Q3, and R⁴ is as per any row of Table V below, which compounds are named I.B1-Z340-Q3-V-1 to I.B1-Z340-Q3-V-6.

In another embodiment, compounds I are of formula I.B1 wherein Z is Z-340 and Q is Q4, and R⁴ is as per any row of Table V below, which compounds are named I.B1-Z340-Q4-V-1 to I.B1-Z340-Q4-V-6.

In another embodiment, compounds I are of formula I.B2 wherein Z is Z-340 and Q is Q1, and R⁴ is as per any row of Table V below, which compounds are named I.B2-Z340-Q1-V-1 to I.B2-Z340-Q1-V-6.

In another embodiment, compounds I are of formula I.B2 wherein Z is Z-340 and Q is Q2, and R⁴ is as per any row of Table V below, which compounds are named I.B2-Z340-Q2-V-1 to I.B2-Z340-Q2-V-6.

In another embodiment, compounds I are of formula I.B2 wherein Z is Z-340 and Q is Q3, and R⁴ is as per any row of Table V below, which compounds are named I.B2-Z340-Q3-V-1 to I.B2-Z340-Q3-V-6.

In another embodiment, compounds I are of formula I.B2 wherein Z is Z-340 and Q is Q4, and R⁴ is as per any row of Table V below, which compounds are named I.B2-Z340-Q4-V-1 to I.B2-Z340-Q4-V-6.

In another embodiment, compounds I are of formula I.B1 wherein Z is Z-341 and Q is Q1, and R⁴ is as per any row of Table V below, which compounds are named I.B1-Z341-Q1-V-1 to I.B1-Z341-Q1-V-6.

In another embodiment, compounds I are of formula I.B1 wherein Z is Z-341 and Q is Q2, and R⁴ is as per any row of Table V below, which compounds are named I.B1-Z341-Q2-V-1 to I.B1-Z341-Q2-V-6.

In another embodiment, compounds I are of formula I.B1 wherein Z is Z-341 and Q is Q3, and R⁴ is as per any row of Table V below, which compounds are named I.B1-Z341-Q3-V-1 to I.B1-Z341-Q3-V-6.

In another embodiment, compounds I are of formula I.B1 wherein Z is Z-341 and Q is Q4, and R⁴ is as per any row of Table V below, which compounds are named I.B1-Z341-Q4-V-1 to I.B1-Z341-Q4-V-6.

In another embodiment, compounds I are of formula I.B2 wherein Z is Z-341 and Q is Q1, and R⁴ is as per any row of Table V below, which compounds are named I.B2-Z341-Q1-V-1 to I.B2-Z341-Q1-V-6.

In another embodiment, compounds I are of formula I.B2 wherein Z is Z-341 and Q is Q2, and R⁴ is as per any row of Table V below, which compounds are named I.B2-Z341-Q2-V-1 to I.B2-Z341-Q2-V-6.

In another embodiment, compounds I are of formula I.B2 wherein Z is Z-341 and Q is Q3, and R⁴ is as per any row of Table V below, which compounds are named I.B2-Z341-Q3-V-1 to I.B2-Z341-Q3-V-6.

In another embodiment, compounds I are of formula I.B2 wherein Z is Z-341 and Q is Q4, and R⁴ is as per any row of Table V below, which compounds are named I.B2-Z341-Q4-V-1 to I.B2-Z341-Q4-V-6.

In another embodiment, compounds I are of formula I.B1 wherein Z is Z-342 and Q is Q1, and R⁴ is as per any row of Table V below, which compounds are named I.B1-Z342-Q1-V-1 to I.B1-Z342-Q 1-V-6.

In another embodiment, compounds I are of formula I.B1 wherein Z is Z-342 and Q is Q2, and R⁴ is as per any row of Table V below, which compounds are named I.B1-Z342-Q2-V-1 to I.B1-Z342-Q2-V-6.

In another embodiment, compounds I are of formula I.B1 wherein Z is Z-342 and Q is Q3, and R⁴ is as per any row of Table V below, which compounds are named I.B1-Z342-Q3-V-1 to I.B1-Z342-Q3-V-6.

In another embodiment, compounds I are of formula I.B1 wherein Z is Z-342 and Q is Q4, and R⁴ is as per any row of Table V below, which compounds are named I.B1-Z342-Q4-V-1 to I.B1-Z342-Q4-V-6.

In another embodiment, compounds I are of formula I.B2 wherein Z is Z-342 and Q is Q1, and R⁴ is as per any row of Table V below, which compounds are named I.B2-Z342-Q1-V-1 to I.B2-Z342-Q1-V-6.

In another embodiment, compounds I are of formula I.B2 wherein Z is Z-342 and Q is Q2, and R⁴ is as per any row of Table V below, which compounds are named I.B2-Z342-Q2-V-1 to I.B2-Z342-Q2-V-6.

In another embodiment, compounds I are of formula I.B2 wherein Z is Z-342 and Q is Q3, and R⁴ is as per any row of Table V below, which compounds are named I.B2-Z342-Q3-V-1 to I.B2-Z342-Q3-V-6.

In another embodiment, compounds I are of formula I.B2 wherein Z is Z-342 and Q is Q4, and R⁴ is as per any row of Table V below, which compounds are named I.B2-Z342-Q4-V-1 to I.B2-Z342-Q4-V-6.

**Table V:**

| **\No.** | **R⁴** |
|---|---|
| V-1 | CH₃ |
| V-2 | C₂H₅ |
| V-3 | CHF₂ |

| **No.** | **R⁴** |
|---|---|
| V-4 | CH₂CF₃ |
| V-5 | CH₂C₃H₅ |
| V-6 | CH₂OCH₃ |

The compounds can be obtained by various routes in analogy to prior art processes known (e.g. WO 93/15046, WO 95/21153, WO 2015/197458, WO 2001/019803) and, advantageously, by the synthesis shown in the following Schemes 1 to 4 and in the experimental part below.

A suitable method to prepare compounds I is illustrated in Scheme 1.

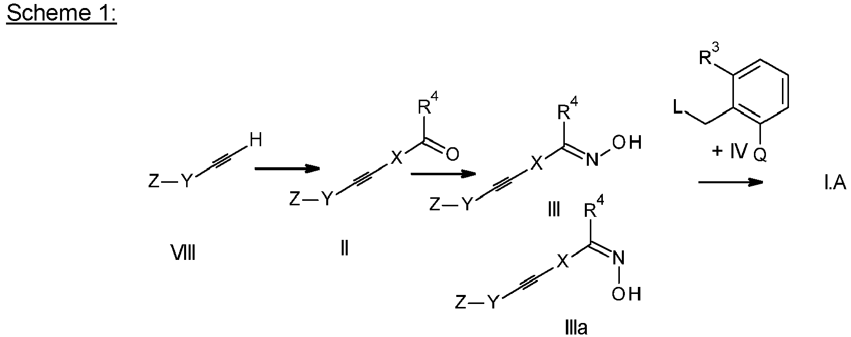

It starts with the formation of a ketone II from the corresponding acetylene compound VIII. Acetylenic proton is first abstracted by a base such as n-butyl lithium in a solvent such as tetrahydrofuran (THF) or 2-methyl-THF at reaction temperatures of -78 °C followed by quenching it with an electrophile such as ethyl acetate or an alkyl halide (where the keto group is optionally protected), in the presence of BF₃-diethyl etherate at -78 °C. The conversion of the ketone II to the corresponding oxime is performed using hydxroxylamine hydrochloride and a base such as pyridine or sodium acetate in polar solvents such as methanol-water mixture at reaction temperatures of about 10 to 25 °C for 2 to 4 h, preferably at about 15 °C. The reaction provides a mixture of E-oxime III and Z-oxime IIIa. The *E*/*Z* oxime mixture (III and IIIa) is subjected to a reaction with the intermediate IV, wherein L is a leaving group such as halogen, toluene-methanesulfonates, preferably L is Cl or Br, under basic conditions using bases such as sodium hydride, cesium carbonate or potassium carbonate and using an organic solvent such as dimethyl formamide (DMF) or acetonitrile, preferably cesium carbonate as base and acetonitrile (AcN) as solvent at room temperature (RT) of about 24 °C for 12h.

Intermediates IV, wherein Q is Q1 or Q2, are commercially available or can be obtained by various routes according to prior art processes known (see e.g. WO 93/15046, WO 95/21153). In intermediates IV, L is a leaving group such as halogen, toluene-methanesulfonates, preferably L is Cl or Br, in particular Br.

A general method for preparation of intermediate IV, wherein Q is Q3 is shown in Scheme 2.1.

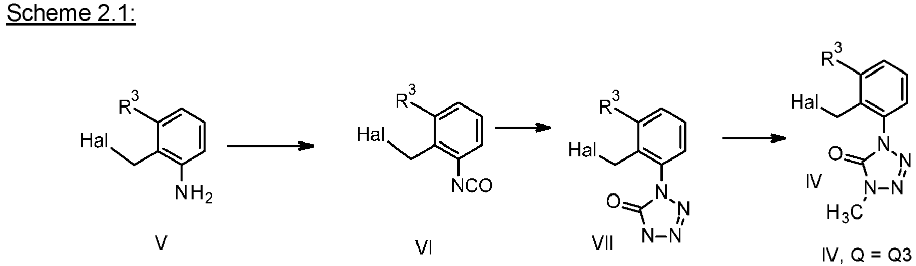

Compound VII can be obtained by ring closure of isocyanate VI with sodium azide in aprotic dipolar solvents like e.g. DMF (see e.g. WO2015005499). The isocyanates VI can be prepared from the corresponding anilines V with e.g. phosgene. Methylation of intermediate VI with methyl halides or sulfonates like methyl chloride, methyl bromide, methyl iodide or methyl tosylate, in the presence of a base like sodium carbonate or triethyl amine in solvents like e.g. DMF, DMSO or THF provides intermediate IV.

A general method for preparation of intermediate IV, wherein Q is Q4, is shown in Scheme 2.2.

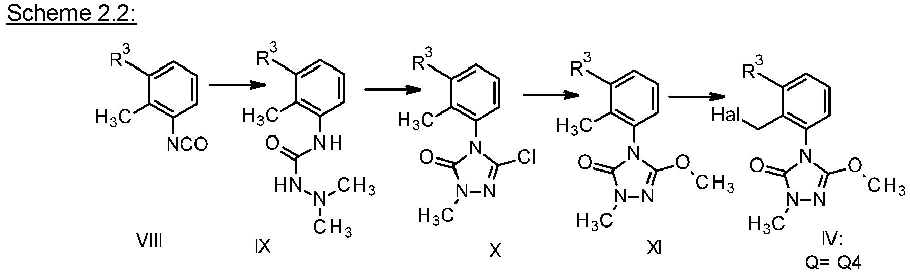

The isocyantes VIII can be obtained in a similar manner as described in scheme 2.1. Reaction with dimethyl hydrazine in e.g. toluene as solvent leads to the semicarbazones IX. Subsequent ring closure of intermediate IX can be achieved with phosgene or triphosgene as described e.g. in US5962436 to provide triazolinone X. This intermediate is further converted to intermediate XI by reaction with e.g. NaOMe in methanol. Ultimate side chain halogenation can be achieved by e.g. Wohl-Ziegler bromination with N-bromo succinimide in CCl₄ to provide intermediate IV.

Preparation of ketones II can also be carried out using other known methods (Scheme 3).

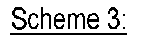

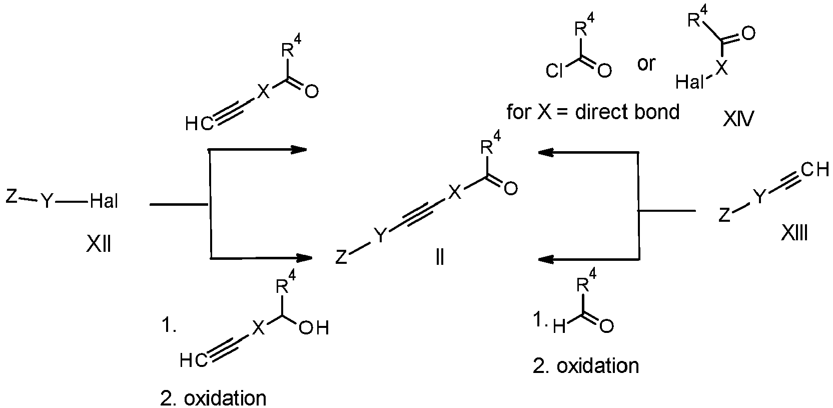

When X is a direct bond compounds II can be synthesized by the following methods:
Palladium and/or copper-catalyzed cross couplings of terminal acetylenic compounds XIII with acid chlorides are usually applied (J. Org. Chem. 2004, 69, 1615). Another possibility is by formation of base-catalyzed alumination of terminal alkynes XIII followed by reaction of it with acid chlorides (J. Org. Chem. 2005, 70, 6126-6128). Alternatively, ketones II can be obtained from acetylene VIII by abstraction of acetylenic proton followed by quenching it with an aldehyde and subsequent oxidation of alcohol (Org. Biomol. Chem. 2018, 16, 6659).

Ketones II can also be obtained from precursors XII, by palladium and copper-catalyzed cross couplings with terminal alkynes (Chem. Cat. Chem. 2015, 7, 3266) or with alkynyl alcohols followed by oxidation of the alcohol (Adv. Synth. Cat. 2017, 22, 4062).

When X is -CL¹L²- compounds II can be synthesized by the following methods: In precursor the acetylenic proton is deprotonated with a base and reacted with compound XIV. In some cases it might be necessary to protect the keto group with a classical protecting group like e.g. 1,3-dioxolane prior to reaction with halide XIV. Also here, ketones II can be obtained from precursors XII, by palladium and copper-catalyzed cross couplings with terminal alkynes (Chem. Cat. Chem. 2015, 7, 3266) or with alkynyl alcohols followed by oxidation of the alcohol (Adv. Synth. Cat. 2017, 22, 4062).

Compounds XIII are generally commercially available, otherwise accessible using methods known in prior art (Org. Lett. 2019, 21, 3990).

The Schemes 1 to 3 presented above describe the synthesis of compounds I wherein R³ is in ortho position to the methyl oxime side chain but can also be applied likewise to corresponding compounds wherein R³ is at different position of the phenyl ring.

The compounds I and the compositions thereof, respectively, are suitable as fungicides effective against a broad spectrum of phytopathogenic fungi, including soil-borne fungi, in particular from the classes of Plasmodiophoromycetes, Peronosporomycetes (syn. Oomycetes), Chytridiomycetes, Zygomycetes, Ascomycetes, Basidiomycetes, and Deuteromycetes (syn. Fungi imperfecti). They can be used in crop protection as foliar fungicides, fungicides for seed dressing, and soil fungicides.

The compounds I and the compositions thereof are preferably useful in the control of phytopathogenic fungi on various cultivated plants, such as cereals, e.g. wheat, rye, barley, triticale, oats, or rice; beet, e.g. sugar beet or fodder beet; fruits, e.g. pomes (apples, pears, etc.), stone fruits (e.g. plums, peaches, almonds, cherries), or soft fruits, also called berries (strawberries, raspberries, blackberries, gooseberries, etc.); leguminous plants, e.g. lentils, peas, alfalfa, or soybeans; oil plants, e.g. oilseed rape, mustard, olives, sunflowers, coconut, cocoa beans, castor oil plants, oil palms, ground nuts, or soybeans; cucurbits, e.g. squashes, cucumber, or melons; fiber plants, e.g. cotton, flax, hemp, or jute; citrus fruits, e.g. oranges, lemons, grapefruits, or mandarins; vegetables, e.g. spinach, lettuce, asparagus, cabbages, carrots, onions, tomatoes, potatoes, cucurbits, or paprika; lauraceous plants, e.g. avocados, cinnamon, or camphor; energy and raw material plants, e.g. corn, soybean, oilseed rape, sugar cane, or oil palm; corn; tobacco; nuts; coffee; tea; bananas; vines (table grapes and grape juice grape vines); hop; turf; sweet leaf (also called Stevia); natural rubber plants; or ornamental and forestry plants, e.g. flowers, shrubs, broad-leaved trees, or evergreens (conifers, eucalypts, etc.); on the plant propagation material, such as seeds; and on the crop material of these plants.

More preferably, compounds I and compositions thereof, respectively are used for controlling fungi on field crops, such as potatoes, sugar beets, tobacco, wheat, rye, barley, oats, rice, corn, cotton, soybeans, oilseed rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant, such as seeds; and vegetative plant materials, such as cuttings and tubers (e.g. potatoes), which can be used for the multiplication of the plant. This includes seeds, roots, fruits, tubers, bulbs, rhizomes, shoots, sprouts and other parts of plants; including seedlings and young plants to be transplanted after germination or after emergence from soil.

Preferably, treatment of plant propagation materials with compounds I and compositions thereof, respectively, is used for controlling fungi on cereals, such as wheat, rye, barley and oats; rice, corn, cotton and soybeans.

According to the invention all of the above cultivated plants are understood to comprise all species, subspecies, variants, varieties and/or hybrids which belong to the respective cultivated plants, including but not limited to winter and spring varieties, in particular in cereals such as wheat and barley, as well as oilseed rape, e.g. winter wheat, spring wheat, winter barley etc.

Corn is also known as Indian corn or maize (Zea *mays)* which comprises all kinds of corn such as field corn and sweet corn. According to the invention all maize or corn subspecies and/or varieties are comprised, in particular flour corn (Zea *mays* var.*amylacea),* popcorn (Zea *mays* var.*everta*)*,* dent corn (Zea *mays* var.*indentata),* flint corn (Zea *mays* var.*indurata),* sweet corn (Zea *mays* var.*saccharata* and var.*rugosa),* waxy corn (Zea *mays* var.*ceratina),* amylomaize (high amylose Zea *mays* varieties), pod corn or wild maize (Zea *mays* var.*tunicata)* and striped maize (Zea *mays* var.*japonica).*

Most soybean cultivars are classifiable into indeterminate and determinate growth habit, whereas *Glycine soja,* the wild progenitor of soybean, is indeterminate (PNAS 2010, 107 (19) 8563-8568). The indeterminate growth habit (Maturity Group, MG 00 to MG 4.9) is characterized by a continuation of vegetative growth after flowering begins whereas determinate soybean varieties (MG 5 to MG 8) characteristically have finished most of their vegetative growth when flowering begins. According to the invention all soybean cultivars or varieties are comprised, in particular indeterminate and determinate cultivars or varieties.

The term "cultivated plants" is to be understood as including plants which have been modified by mutagenesis or genetic engineering to provide a new trait to a plant or to modify an already present trait. Mutagenesis includes random mutagenesis using X-rays or mutagenic chemicals, but also targeted mutagenesis to create mutations at a specific locus of a plant genome. Targeted mutagenesis frequently uses oligonucleotides or proteins like CRISPR/Cas, zinc-finger nucleases, TALENs or meganucleases. Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant to add a trait or improve or modify a trait. These integrated genes are also referred to as transgenes, while plant comprising such transgenes are referred to as transgenic plants. The process of plant transformation usually produces several transformation events, wich differ in the genomic locus in which a transgene has been integrated. Plants comprising a specific transgene on a specific genomic locus are usually described as comprising a specific "event", which is referred to by a specific event name. Traits which have been introduced in plants or have been modified include herbicide tolerance, insect resistance, increased yield and tolerance to abiotic conditions, like drought. Herbicide tolerance has been created by using mutagenesis and genetic engineering. Plants which have been rendered tolerant to acetolactate synthase (ALS) inhibitor herbicides by mutagenesis and breeding are e.g. available under the name Clearfield^{®}. Herbicide tolerance to glyphosate, glufosinate, 2,4-D, dicamba, oxynil herbicides, like bromoxynil and ioxynil, sulfonylurea herbicides, ALS inhibitors and 4-hydroxyphenylpyruvate dioxygenase (HPPD) inhibitors, like isoxaflutole and mesotrione, has been created via the use of transgenes.

Transgenes to provide herbicide tolerance traits comprise: for tolerance to glyphosate: cp4 epsps, epsps grg23ace5, mepsps, 2mepsps, gat4601, gat4621, goxv247; for tolerance to glufosinate: pat and bar, for tolerance to 2,4-D: aad-1, aad-12; for tolerance to dicamba: dmo; for tolerance to oxynil herbicies: bxn; for tolerance to sulfonylurea herbicides: zm-hra, csr1-2, gm-hra, S4-HrA; for tolerance to ALS inhibitors: csr1-2; and for tolerance to HPPD inhibitors: hppdPF, W336, avhppd-03.

Transgenic corn events comprising herbicide tolerance genes include, but are not limited to, DAS40278, MON801, MON802, MON809, MON810, MON832, MON87411, MON87419, MON87427, MON88017, MON89034, NK603, GA21, MZHG0JG, HCEM485, VCO-∅1981-5, 676, 678, 680, 33121, 4114, 59122, 98140, Bt10, Bt176, CBH-351, DBT418, DLL25, MS3, MS6, MZIR098, T25, TC1507 and TC6275. Transgenic soybean events comprising herbicide tolerance genes include, but are not limited to, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHT∅H2, W62, W98, FG72 and CV127. Transgenic cotton events comprising herbicide tolerance genes include, but are not limited to, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40. Transgenic canola events comprising herbicide tolerance genes are for example, but not excluding others, MON88302, HCR-1, HCN10, HCN28, HCN92, MS1, MS8, PHY14, PHY23, PHY35, PHY36, RF1, RF2 and RF3.

Transgenes to provide insect resistance preferably are toxin genes of *Bacillus* spp.and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. In addition, transgenes of plant origin, such as genes coding for protease inhibitors, like CpTI and pinll, can be used. A further approach uses transgenes such as dvsnf7 to produce double-stranded RNA in plants.

Transgenic corn events comprising genes for insecticidal proteins or double stranded RNA include, but are not limited to, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098. Transgenic soybean events comprising genes for insecticidal proteins include, but are not limited to, MON87701, MON87751 and DAS-81419. Transgenic cotton events comprising genes for insecticidal proteins include, but are not limited to, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Cultivated plants with increased yield have been created by using the transgene athb17 (e.g. corn event MON87403), or bbx32 (e.g. soybean event MON87712).

Cultivated plants comprising a modified oil content have been created by using the transgenes: gm-fad2-1, Pj.D6D, Nc.Fad3, fad2-1A and fatb1-A (e.g. soybean events 260-05, MON87705 and MON87769).

Tolerance to abiotic conditions, such as drought, has been created by using the transgene cspB (corn event MON87460) and Hahb-4 (soybean event IND-∅∅41∅-5).

Traits are frequently combined by combining genes in a transformation event or by combining different events during the breeding process resulting in a cultivated plant with stacked traits. Preferred combinations of traits are combinations of herbicide tolerance traits to different groups of herbicides, combinations of insect tolerance to different kind of insects, in particular tolerance to lepidopteran and coleopteran insects, combinations of herbicide tolerance with one or several types of insect resistance, combinations of herbicide tolerance with increased yield as well as combinations of herbicide tolerance and tolerance to abiotic conditions.

Plants comprising singular or stacked traits as well as the genes and events providing these traits are well known in the art. For example, detailed information as to the mutagenized or integrated genes and the respective events are available from websites of the organizations "International Service for the Acquisition of Agri-biotech Applications (ISAAA)" (http://www.isaaa.org/gmapprovaldatabase) and the "Center for Environmental Risk Assessment (CERA)" (http://cera-gmc.org/GMCropDatabase). Further information on specific events and methods to detect them can be found for canola events MS1, MS8, RF3, GT73, MON88302, KK179 in WO01/031042, WO01/041558, WO01/041558, WO02/036831, WO11/153186, WO13/003558; for cotton events MON1445, MON15985, MON531 (MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527; for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571; for potato events E12, F10, J3, J55, V11, X17, Y9 in WO14/178910, WO14/178913, WO14/178941, WO14/179276, WO16/183445, WO17/062831, WO17/062825; for rice events LLRICE06, LLRICE601, LLRICE62 in WO00/026345, WO00/026356, WO00/026345; and for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 × DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compounds I and compositions thereof, respectively, on cultivated plants may result in effects which are specific to a cultivated plant comprising a certain transgene or event. These effects might involve changes in growth behavior or changed resistance to biotic or abiotic stress factors. Such effects may in particular comprise enhanced yield, enhanced resistance or tolerance to insects, nematodes, fungal, bacterial, mycoplasma, viral or viroid pathogens as well as early vigour, early or delayed ripening, cold or heat tolerance as well as changed amino acid or fatty acid spectrum or content.

The compounds I and compositions thereof, respectively, are particularly suitable for controlling the following causal agents of plant diseases:
*Albugo* spp. (white rust) on ornamentals, vegetables (e.g. *A. candida)* and sunflowers (e.g. *A. tragopogonis*); *Alternaria* spp. (Alternaria leaf spot) on vegetables (e.g. *A. dauci* or *A. porri*)*,* oilseed rape *(A. brassicicola* or *brassicae),* sugar beets (*A. tenuis*)*,* fruits (e.g. *A. grandis*)*,* rice, soybeans, potatoes and tomatoes (e.g. *A. solani, A. grandis* or *A. alternata*)*,* tomatoes (e.g. *A. solani* or *A. alternata*) and wheat (e.g. A. *triticina*)*; Aphanomyces* spp. on sugar beets and vegetables; *Ascochyta* spp. on cereals and vegetables, e.g. *A. tritici* (anthracnose) on wheat and *A. hordei* on barley; *Aureobasidium zeae* (syn. *Kapatiella* zeae) on corn; *Bipolaris* and *Drechslera* spp. (teleomorph: *Cochliobolus* spp.), e.g. Southern leaf blight (*D. maydis*) or Northern leaf blight (*B. zeicola*) on corn, e.g. spot blotch (*B. sorokiniana*) on cereals and e.g. *B*. *oryzae* on rice and turfs; *Blumeria* (formerly *Erysiphe) graminis* (powdery mildew) on cereals (e.g. on wheat or barley); *Botrytis cinerea* (teleomorph: *Botryotinia fuckeliana:* grey mold) on fruits and berries (e.g. strawberries), vegetables (e.g. lettuce, carrots, celery and cabbages); *B*. *squamosa* or *B*. *allii* on onion family), oilseed rape, ornamentals (e.g. *B eliptica*)*,* vines, forestry plants and wheat; *Bremia lactucae* (downy mildew) on lettuce; *Ceratocystis* (syn. *Ophiostoma*) spp. (rot or wilt) on broad-leaved trees and evergreens, e.g. C. *ulmi* (Dutch elm disease) on elms; *Cercospora* spp. (Cercospora leaf spots) on corn (e.g. Gray leaf spot: C. *zeae-maydis*)*,* rice, sugar beets (e.g. C. *beticola*)*,* sugar cane, vegetables, coffee, soybeans (e.g. C. *sojina* or *C. kikuchii*) and rice; *Cladobotryum (syn. Dactylium)* spp. (e.g. C. *mycophilum* (formerly *Dactylium dendroides,* teleomorph: *Nectria albertinii, Nectria rosella* syn. *Hypomyces rosellus*) on mushrooms; *Cladosporium* spp. on tomatoes (e.g. C. *fulvum:* leaf mold) and cereals, e.g. C. *herbarum* (black ear) on wheat; *Claviceps purpurea* (ergot) on cereals; *Cochliobolus* (anamorph: *Helminthosporium* of *Bipolaris*) spp. (leaf spots) on corn (C. *carbonum*)*,* cereals (e.g. C. *sativus,* anamorph: *B*. *sorokiniana*) and rice (e.g. C. *miyabeanus,* anamorph: *H. oryzae*); *Colletotrichum* (teleomorph: *Glomerella*) spp. (anthracnose) on cotton (e.g. C. *gossypii*)*,* corn (e.g. C. *graminicola:* Anthracnose stalk rot), soft fruits, potatoes (e.g. C. *coccodes:* black dot), beans (e.g. C. *lindemuthianum*)*,* soybeans (e.g. C. *truncatum* or C. *gloeosporioides*)*,* vegetables (e.g. C. *lagenarium* or C. *capsici*)*,* fruits (e.g. C. *acutatum*)*,* coffee (e.g. C. *coffeanum* or C. *kahawae*) and C. *gloeosporioides* on various crops; *Corticium* spp., e.g. C. *sasakii* (sheath blight) on rice; *Corynespora cassiicola* (leaf spots) on soybeans, cotton and ornamentals; *Cycloconium* spp., e.g. C. *oleaginum* on olive trees; *Cylindrocarpon* spp. (e.g. fruit tree canker or young vine decline, teleomorph: *Nectria* or *Neonectria* spp.) on fruit trees, vines (e.g. C. *liriodendri,* teleomorph: *Neonectria liriodendri:* Black Foot Disease) and ornamentals; *Dematophora* (teleomorph: *Rosellinia*) *necatrix* (root and stem rot) on soybeans; *Diaporthe* spp., e.g. *D. phaseolorum* (damping off) on soybeans; *Drechslera* (syn. *Helminthosporium,* teleomorph: *Pyrenophora*) spp. on corn, cereals, such as barley (e.g. D. *teres,* net blotch) and wheat (e.g. *D. tritici-repentis:* tan spot), rice and turf; Esca (dieback, apoplexy) on vines, caused by *Formitiporia* (syn. *Phellinus*) *punctata, F. mediterranea, Phaeomoniella chlamydospora* (formerly *Phaeoacremonium chlamydosporum), Phaeoacremonium aleophilum* and/or *Botryosphaeria obtusa; Elsinoe* spp. on pome fruits (E. *pyri*)*,* soft fruits (E. *veneta:* anthracnose) and vines (*E*. *ampelina:* anthracnose); *Entyloma oryzae* (leaf smut) on rice; *Epicoccum* spp. (black mold) on wheat; *Erysiphe* spp. (powdery mildew) on sugar beets (E. *betae*)*,* vegetables (e.g. *E. pisi*)*,* such as cucurbits (e.g. *E*. *cichoracearum*)*,* cabbages, oilseed rape (e.g. *E. cruciferarum*)*; Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e.g. *E. turcicum*); *Fusarium* (teleomorph: *Gibberella*) spp. (wilt, root or stem rot) on various plants, such as *F*. *graminearum* or *F. culmorum* (root rot, scab or head blight) on cereals (e.g. wheat or barley), *F*. *oxysporum* on tomatoes, *F. solani* (f. sp. *glycines* now syn. *F. virguliforme*) and *F. tucumaniae* and *F. brasiliense* each causing sudden death syndrome on soybeans, and *F. verticillioides* on corn; *Gaeumannomyces graminis* (take-all) on cereals (e.g. wheat or barley) and corn; *Gibberella* spp. on cereals (e.g. *G. zeae)* and rice (e.g. *G. fujikuroi:* Bakanae disease); *Glomerella cingulata* on vines, pome fruits and other plants and *G. gossypii* on cotton; Grainstaining complex on rice; *Guignardia bidwellii* (black rot) on vines; *Gymnosporangium* spp. on rosaceous plants and junipers, e.g. *G. sabinae* (rust) on pears; *Helminthosporium* spp. (syn. *Drechslera,* teleomorph: *Cochliobolus*) on corn, cereals, potatoes and rice; *Hemileia* spp., e.g. *H. vastatrix* (coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e.g. wheat or barley); *Microsphaera diffusa* (powdery mildew) on soybeans; *Monilinia* spp., e.g. *M. laxa, M. fructicola* and *M. fructigena* (syn. *Monilia* spp.: bloom and twig blight, brown rot) on stone fruits and other rosaceous plants; *Mycosphaerella* spp. on cereals, bananas, soft fruits and ground nuts, such as e.g. *M.graminicola* (anamorph: *Zymoseptoria tritici* formerly *Septoria tritici:* Septoria blotch) on wheat or *M. fijiensis* (syn. *Pseudocercospora fijiensis:* black Sigatoka disease) and *M*. *musicola* on bananas, *M. arachidicola* (syn. *M. arachidis* or *Cercospora arachidis), M. berkeleyi* on peanuts, *M. pisi* on peas and *M. brassiciola* on brassicas; *Peronospora* spp. (downy mildew) on cabbage (e.g. *P. brassicae*)*,* oilseed rape (e.g. *P. parasitica*)*,* onions (e.g. *P. destructor*)*,* tobacco (P. *tabacina*) and soybeans (e.g. *P. manshurica*)*; Phakopsora pachyrhizi* and *P*. *meibomiae* (soybean rust) on soybeans; *Phialophora* spp. e.g. on vines (e.g. *P. tracheiphila* and *P. tetraspora*) and soybeans (e.g. *P. gregata:* stem rot); *Phoma lingam* (syn. *Leptosphaeria biglobosa* and *L. maculans:* root and stem rot) on oilseed rape and cabbage, *P. betae* (root rot, leaf spot and damping-off) on sugar beets and *P*. *zeae-maydis* (syn. *Phyllostica zeae*) on corn; *Phomopsis* spp. on sunflowers, vines (e.g. *P*. *viticola:* can and leaf spot) and soybeans (e.g. stem rot: *P. phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e.g. *P. capsici*), soybeans (e.g. *P. megasperma,* syn. *P. sojae*)*,* potatoes and tomatoes (e.g. *P. infestans:* late blight) and broad-leaved trees (e.g. *P. ramorum:* sudden oak death); *Plasmodiophora brassicae* (club root) on cabbage,oilseed rape, radish and other plants; *Plasmopara* spp., e.g. *P*. *viticola* (grapevine downy mildew) on vines and *P*. *halstedii* on sunflowers; *Podosphaera* spp. (powdery mildew) on rosaceous plants, hop, pome and soft fruits (e.g. *P. leucotricha* on apples) and curcurbits (*P. xanthii)*; *Polymyxa* spp., e.g. on cereals, such as barley and wheat (P. *graminis*) and sugar beets (P. *betae*) and thereby transmitted viral diseases; *Pseudocercosporella herpotrichoides* (syn. *Oculimacula yallundae, O. acuformis:* eyespot, teleomorph: *Tapesia yallundae*) on cereals, e.g. wheat or barley; *Pseudoperonospora* (downy mildew) on various plants, e.g. *P. cubensis* on cucurbits or *P*. *humili* on hop; *Pseudopezicula tracheiphila* (red fire disease or 'rotbrenner', anamorph: *Phialophora*) on vines; *Puccinia* spp. (rusts) on various plants, e.g. *P. triticina* (brown or leaf rust), *P. striiformis* (stripe or yellow rust), *P. hordei* (dwarf rust), *P. graminis* (stem or black rust) or *P*. *recondita* (brown or leaf rust) on cereals, such as e.g. wheat, barley or rye, *P*. *kuehnii* (orange rust) on sugar cane and *P*. *asparagi* on asparagus; *Pyrenopeziza spp.,* e.g. *P. brassicae* on oilseed rape; *Pyrenophora* (anamorph: *Drechslera*) *tritici-repentis* (tan spot) on wheat or *P*. *teres* (net blotch) on barley; *Pyricularia* spp., e.g. *P*. *oryzae* (teleomorph: *Magnaporthe grisea:* rice blast) on rice and *P*. *grisea* on turf and cereals; *Pythium* spp. (damping-off) on turf, rice, corn, wheat, cotton, oilseed rape, sunflowers, soybeans, sugar beets, vegetables and various other plants (e.g. *P*. *ultimum* or *P*. *aphanidermatum*) and *P*. *oligandrum* on mushrooms; *Ramularia* spp., e.g. *R*. *collo-cygni* (Ramularia leaf spots, Physiological leaf spots) on barley, *R. areola* (teleomorph: *Mycosphaerella areola)* on cotton and *R. beticola* on sugar beets; *Rhizoctonia* spp. on cotton, rice, potatoes, turf, corn, oilseed rape, potatoes, sugar beets, vegetables and various other plants, e.g. *R. solani* (root and stem rot) on soybeans, *R. solani* (sheath blight) on rice or *R*. *cerealis* (Rhizoctonia spring blight) on wheat or barley; *Rhizopus stolonifer* (black mold, soft rot) on strawberries, carrots, cabbage, vines and tomatoes; *Rhynchosporium secalis* and *R*. *commune* (scald) on barley, rye and triticale; *Sarocladium oryzae* and S. *attenuatum* (sheath rot) on rice; *Sclerotinia* spp. (stem rot or white mold) on vegetables (S. *minor* and S. *sclerotiorum)* and field crops, such as oilseed rape, sunflowers (e.g. S. *sclerotiorum)* and soybeans, S. *rolfsii* (syn. *Athelia rolfsii)* on soybeans, peanut, vegetables, corn, cereals and ornamentals; *Septoria* spp. on various plants, e.g. S. *glycines* (brown spot) on soybeans, S. *tritici* (syn. *Zymoseptoria tritici,* Septoria blotch) on wheat and S. (syn. *Stagonospora) nodorum* (Stagonospora blotch) on cereals; *Uncinula* (syn. *Erysiphe) necator* (powdery mildew, anamorph: *Oidium tuckeri*) on vines; *Setosphaeria* spp. (leaf blight) on corn (e.g. S. *turcicum,* syn. *Helminthosporium turcicum)* and turf; *Sphacelotheca* spp. (smut) on corn, (e.g. S. *reiliana,* syn. *Ustilago reiliana:* head smut), sorghum und sugar cane; *Sphaerotheca fuliginea* (syn. *Podosphaera xanthii:* powdery mildew) on cucurbits; *Spongospora subterranea* (powdery scab) on potatoes and thereby transmitted viral diseases; *Stagonospora* spp. on cereals, e.g. S. *nodorum* (Stagonospora blotch, teleomorph: *Leptosphaeria* [syn. *Phaeosphaeria] nodorum,* syn. *Septoria nodorum)* on wheat; *Synchytrium endobioticum* on potatoes (potato wart disease); *Taphrina* spp., e.g. *T. deformans* (leaf curl disease) on peaches and *T. pruni* (plum pocket) on plums; *Thielaviopsis* spp. (black root rot) on tobacco, pome fruits, vegetables, soybeans and cotton, e.g. *T. basicola* (syn. *Chalara elegans); Tilletia* spp. (common bunt or stinking smut) on cereals, such as e.g. *T. tritici* (syn. *T. caries,* wheat bunt) and *T. controversa* (dwarf bunt) on wheat; *Trichoderma harzianum on mushrooms; Typhula incarnata* (grey snow mold) on barley or wheat; *Urocystis* spp., e.g. *U. occulta* (stem smut) on rye; *Uromyces* spp. (rust) on vegetables, such as beans (e.g. *U. appendiculatus,* syn. *U. phaseoli*)*,* sugar beets (e.g. *U. betae* or *U. beticola*) and on pulses (e.g. *U. vignae, U. pisi, U. viciae-fabae* and *U. fabae); Ustilago* spp. (loose smut) on cereals (e.g. *U. nuda* and *U. avaenae),* corn (e.g. *U. maydis:* corn smut) and sugar cane; *Venturia* spp. (scab) on apples (e.g. *V. inaequalis)* and pears; and *Verticillium* spp. (wilt) on various plants, such as fruits and ornamentals, vines, soft fruits, vegetables and field crops, e.g. *V. longisporum* on oilseed rape, *V. dahliae* on strawberries, oilseed rape, potatoes and tomatoes, and *V. fungicola* on mushrooms; *Zymoseptoria tritici* on cereals.

The compounds I and compositions thereof, respectively, are particularly suitable for controlling the following causal agents of plant diseases: rusts on soybean and cereals (e.g. *Phakopsora pachyrhizi* and *P*. *meibomiae* on soy; *Puccinia tritici* and *P*. *striiformis* on wheat); molds on specialty crops, soybean, oil seed rape and sunflowers (e.g. *Botrytis cinerea* on strawberries and vines, *Sclerotinia sclerotiorum, S. minor* and S. *rolfsii* on oil seed rape, sunflowers and soybean); Fusarium diseases on cereals (e.g. *Fusarium culmorum* and *F*. *graminearum* on wheat); downy mildews on specialty crops (e.g. *Plasmopara viticola* on vines, *Phytophthora infestans* on potatoes); powdery mildews on specialty crops and cereals (e.g. *Uncinula necator* on vines, *Erysiphe* spp. on various specialty crops, *Blumeria graminis* on cereals); and leaf spots on cereals, soybean and corn (e.g. *Septoria tritici* and S. *nodorum* on cereals, S. *glycines* on soybean, *Cercospora* spp. on corn and soybean).

Fungicide-resistant strains of the abovementioned phytopathgenic fungi have been reported, with strains resistant to one or more fungicides from various fungicidal mode of action classes being observed including but not limited to beta-tubulin assembly inhibitors, sterol demethyl-ation-inhibitors (DMI), quinone-outside-inhibitors (Qol) and succinate dehydrogenase inhibitors (SDHI) by target-site mutations. Examples of mutation sites of genes encoding the such target protein in each plant disease fungus include those indicated in Table MU below.

**Table MU:**

| | **Genes encoding target proteins of various kinds of fungicides** | | | | | | | |
|---|---|---|---|---|---|---|---|---|
| **Fungus** | **CytB** | **Cyp51** | **CesA3** | **SdhB** | **SdhC** | **SdhD** | **Os-1** | **Erg27** |
| *Alternaria alternata* | G143A | | | H277R/ Y | H134R | H133R | | |
| *Alternaria brassicicola* | | | | | | | E753K | |
| *Alternaria longipes* | | | | | | | G420D | |
| *Alternaria arborescens* | G143A | | | | | | | |
| *Alternaria solani* | F129L | | | H277R/ Y | | H133R | | |
| *Alternaria tomato* | G143A | | | | | | | |
| *Aspergillus flavus* | | Y132N, K197N, D282E, M288L, T469S, H399P, D411N, T454P | | | | | | |
| *Aspergillus fumigatus* | | N22D, S52T, G54E/K/ R/ V/W, Y68, Q88H, L98H, V101F, Y121F, N125I, G138C/ R/S Q141H, H147Y, F219S, M220K/I /T /V, T289A, S297T, P394L, Y431C, G432S, G434C, T440A, G448S, Y491H, F4951 | | | | | | |
| *Aspergillus parasiticus* | | G54W | | | | | | |
| *Botryotinia fuckeliana* | G143A | | | P225H/ F/L /T, N230I, H272L/ R/V /Y | P80H/L, A85V | H132R | I365N/R /S, V368F, Q369H/ P, N373S, T447S | S9G, F26S, P57A, T63I, G170R, V192I, L195F, N196T, A210G, I232M, P238S/ Δ, P250S, P269L, P298 Δ, V309M, A314V, S336C, V365A, E368D, N369D, E375K, A378T, L400F/S, Y408S, F412I/S/V/ C, A461S, R496T |
| *Candida albicans* | | A61V, Y132F/ H, K143E, S405F, F449S, G464S, R467K, I471T | | | | | | |
| *Glomerella graminicola* | G143A | | | | | | | |
| *Corynespora cassiicola* | G143A | | | | S73P | S89P, G109V | | |
| *Cercospora beticola* | G143A | E297K, I330T, P384S | | | | | | |
| *Cercospora sojina* | G143A | | | | | | | |
| *Cladsporium carpophilum* | G143A | | | | | | | |
| *Colletotrichu* m *graminicola* | G143A | | | | | | | |
| *Glomerella cingulata* | G143A | | | | | | | |
| *Stagonospor opsis cucurbitacear* um | | | | H277R/ Y | | | | |
| *Blumeria graminis f. sp. hordei* | G143A | Y136F, K147Q, S509T | | | | | | |
| *Blumeria graminis f. sp. tritici* | G143A | S79T, Y136F, K175N | | | | | | |
| *Eurotium oryzae* | | | | H249L/ N/Y | T90I | D124E | | |
| *Parastagono spora nodorum* | G143A | | | | | | | |
| *Monographell a nivalis* | G143A | | | | | | | |
| *Monilinia fructicola* | | Y136F | | | | | | |
| *Monilinia laxa* | | | | | | | | |
| *Microdochiu m majus, nivale* | G143A | | | | | | | |
| *Mycosphaere Ila fijiensis* | G143A | Y136F, A313G, A381G, Y461D, G462A, Y463D/ H/N | | | | | | |
| *Didymella rabiei* | G143A | | | | | | | |
| *Phakopsora pachyrhizi* | F129L | F120L, Y131F/ H, K142R, I145F, I475T | | | I86F | | | |
| *Phytophthora capsici* | | | Q1077 K, V1109 L/M | | | | | |
| *Phytophthora drechsleri* | | | V1109 L | | | | | |
| *Phytophthora infestans* | | | G1105 A/V, V1109 L | | | | | |
| *Plasmopara viticola* | F129L G143A | | G1105 S/V | | | | | |
| *Pleospora allii* | G143A | | | | | | F267L, L290S, T765R, Q777R | |
| *Podosphaera fusca* | G143A | | | | | | | |
| *Podosphaera xanthii* | G143A | | | | | | | |
| *Pseudoperon* o *spora cubensis* | G143A | | G1105 V/W | | | | | |
| *Puccinia triticina* | | Y134F | | | | | | |
| *Magnaporthe oryzae* | F129L G143A | | | | | | | |
| *Pyrenophora teres* | F129L | | | H277Y | K49E, R64K, N75S, G79R, H134R, S135R | D124E/ N, H134R, G138V, D145G | | |
| *Pyrenophorat ritici-repentis* | F129L G137R G143A | | | | | | | |
| *Pyrenopeziza brassicae* | | S508T | | | | | | |
| *Pythium aphanidermat* um | F129L | | | | | | | |
| *Thanatephor us cucumeris* | F129L G143A | | | | | | | |
| *Remularia collo-cygni* | G143A | | | | N87S, H146R, H153R | | | |
| *Rhynchospori um secalis* | G143A | | | | | | | |
| *Rhizoctonia solani* | F129L | | | | | | | |
| *Saccharomyc es cerevisiae* | | Y140F/ H | | | | | | |
| *Sclerotinia sclerotiorum* | | | | H273Y | H146R | H132R | | |
| *Zymoseptoria tritici* | F129L G137R G143A | L50S, D107V, D134G, V136A/ C/G Y137F, M145L, N178S, S188N, S208T, N284H, H303Y, | | N225I/T, H273Y, T268I/A, I269V | I29V, N33T, N34T, T79I/N, W80S, A84V, N86K/S/ A, G90R, R151T/ S, | I50F, M114V, D129E | | |
| | | A311G, G312A, A379G, I381V/ Δ, A410T, G412A, Y459C/ D/N /S/P/ Δ, G460D/ Δ, Y461D/ H/S, V490L, G510C, N513K, S524T | | | H152R, I161S | | | |
| *Erysiphe necator* | G143A | Y136F | | H242R | G169D | | | |
| *Ustilago maydis* | | | | H257L | | | | |
| *Venturia inaequalis* | G143A | | | T253I | H151R | | | |

Thus, compounds I are particularly useful to control such fungicide-resistant strains of phytopathogenic fungi described in Table M. Such strains may have one or more resistances derived from one or more mutations of one or more genes encoding target proteins of various kinds of the fungicides including but not limited to the mutations listed in Table M and/or a resistance derived from an overexpression of the target protein.

In addition, certain strains of fungi may have developed other type of resistances to fungicides some of which are called efflux type multidrug-resistant fungi.

The term of "efflux type multidrug resistant fungus" as used herein represents a fungus showing a resistance to multiple fungicides sometimes even from various fungicidal modes oof aciton by overexpressing various kinds of membrane transporters that are present on the cell membrane, and increasing an efflux pump function in exporting the plant disease control agent that is inflowed into cells outside of the cells. Examples of the membrane transporter include an ABC transporter and a MFS transporter, which are not limited thereto. As used herein, "ABC transporter" refers to an ATP-binding cassette transporter, and "MFS transporter" refers to a Major Facilitator Superfamily transporter. The overexpression of the membrane transporter is confirmed by usual method, for example, by measuring an amount of the membrane transporter or an amount of mRNA which corresponds to a gene encoding the membrane transporter.

It is sufficient that the efflux type multidrug resistant fungus shows any resistance to a plant disease control agent as a result of the overexpression of the membrane transporter regardless of the measured amount of mRNA. The measured amount of mRNA may be, for example, 5 fold, 20 fold, further 100 fold or more, relative to the mRNA amount of wild-type fungus.

The method of the present invention can be applied to control a plant disease that is caused by an efflux type multidrug resistant fungus.

The efflux type multidrug resistant fungus may have in addition one or more resistances derived from one or more mutations of one or more genes encoding target proteins of various kinds of the pfungicides including but not limited to the mutations listed in Table M and/or a resistance derived from an overexpression of the target protein.

Thus, compounds I are also particularly useful to control such efflux type multidrug-resistant fungi.

According to one embodiment of the invention, the invention also relates to a method for combating phytopathogenic fungi containing a mutation in the mitochondrial cytochrome b gene conferring resistance to Qo inhibitors, comprising: treating the phytopathogenic fungi containing a mutation in the mitochondrial cytochrome b gene conferring resistance to Qo inhibitors or the materials, plants, the soil or seeds that are at risk of being diseased from phytopathogenic fungi containing a mutation in the mitochondrial cytochrome b gene conferring resistance to Qo inhibitors with an effective amount of at least one compound I, or a composition comprising it thereof.

The term "phytopathogenic fungi containing a mutation in the mitochondrial cytochrome b gene conferring resistance to Qo inhibitors" ist be understood that at least 10% of the fungal isolates to be controlled contain a mutation in the mitochondrial cytochrome b gene conferring resistance to Qo inhibitors, more preferably at least 30%, even more preferably at least 50%, and most preferably at least 75% of the fungi, in particular between 90 and 100%.

It has been observed under field conditions that populations of phytopathogenic fungi apparently consisting of non-resistant strains can readily develop resistance. The compounds can be applied under such conditions, too, in order to prevent the formation of resistance and the spread of resistant strains altogether. In this regard it is useful that they have strong activity against non-resistant phytopathogenic fungi also.

According to another embodiment, the method for combating phytopathogenic fungi, comprises: a) identifying the phytopathogenic fungi containing a mutation in the mitochondrial cytochrome b gene conferring resistance to Qo inhibitors, or the materials, plants, the soil or seeds that are at risk of being diseased from phytopathogenic fungi as defined herein, and b) treating said fungi or the materials, plants, the soil or seeds with an effective amount of at least one compound I, or a composition comprising it thereof.

According to another embodiment of the invention, the invention also relates to a method for combating phytopathogenic fungi containing a mutation in the mitochondrial cytochrome b gene conferring resistance to Qo inhibitors, comprising: treating the phytopathogenic fungi whereof at least 10% contain a mutation in the mitochondrial cytochrome b gene conferring resistance to Qo inhibitors or the materials, plants, the soil or seeds that are at risk of being diseased from phytopathogenic fungi containing a mutation in the mitochondrial cytochrome b gene conferring resistance to Qo inhibitors with an effective amount of at least one compound I, or a composition comprising it thereof; more preferably at least 30%, even more preferably at least 50%, and most preferably at least 75% of the fungi contain a mutation in the mitochondrial cytochrome b gene conferring resistance to Qo inhibitors.

According to one embodiment of the use and the method for combating phytopathogenic fungi containing a mutation in the mitochondrial cytochrome b gene conferring resistance to Qo inhibitors, the mutation in the mitochondrial cytochrome b gene of the phytopathogenic fungi is G143A.

The mutation G143A in the cytochrome b (cytb, also referred to as cob) gene shall mean any substitution of nucleotides of codon 143 encoding "G" (glycine; e.g. GGT, GGC, GGA or GGG) that leads to a codon encoding "A" (alanine; e.g. GCT, GCC, GCA or GCG), for example the substitution of the second nucleotide of codon 143 'G' to 'C' (GGT to GCT), in the cytochrome b gene resulting in a single amino acid substitution in the position 143 from G (glycine) to A (alanine) (G143A) in the cytochrome b protein (Cytb). In the present invention, the mutation G143A in the cytochrome b gene shall be understood to be a single amino acid substitution in the position 143 from G (glycine) to A (alanine) (G143A) in the cytochrome b protein.

According to a further embodiment, the phytopathogenic fungi containing a mutation in the mitochondrial cytochrome b gene conferring resistance to Qo inhibitors, wherein the mutation in the mitochondrial cytochrome b gene of the phytopathogenic fungi is G143A, are selected from the group consisting of *Alternaria alternata, Blumeria graminis, Pyriculania oryzae* (also known as *Magnaporthe grisea*)*, Septoria tritici* (also known as *Mycosphaerella graminicola*)*, Mycosphaerella fijiensis, Venturia inaequalis, Pyrenophora teres, Pyrenophona tritici-repentis* and *Plasmopara viticola,* in particular *Septoria tritici.*

According to another embodiment of the use and the method for combating phytopathogenic fungi containing a mutation in the mitochondrial cytochrome b gene conferring resistance to Qo inhibitors, the mutation in the mitochondrial cytochrome b gene of the phytopathogenic fungi is F129L.

The mutation F129L in the cytochrome b (cytb, also referred to as cob) gene shall mean any substitution of nucleotides of codon 129 encoding "F" (phenylalanine; e.g. TTT or TTC) that leads to a codon encoding "L" (leucine; e.g. TTA, TTG, TTG, CTT, CTC, CTA or CTG), for example the substitution of the first nucleotide of codon 129 'T' to 'C' (TTT to CTT), in the cytochrome b gene resulting in a single amino acid substitution in the position 129 from F (phenylalanine) to L (leucine) (F129L) in the cytochrome b protein (Cytb). In the present invention, the mutation F129L in the cytochrome b gene shall be understood to be a single amino acid substitution in the position 129 from F (phenylalanine) to L (leucine) (F129L) in the cytochrome b protein.

Many other phytopathogenic fungi acquired the F129L mutation in the cytochrome b gene conferring resistance to Qo inhibitors, such as rusts, in particular soybean rust (*Phakopsora pachyrhizi* and *Phakopsora meibromiae*) as well as fungi from the genera Alternaria, Pyrenophora and Rhizoctonia. Preferred fungal species are *Alternaria solani, Phakopsora pachyrhizi, Phakopsora meibromiae, Pyrenophora teres, Pyrenophora tritici-repentis* and *Rhizoctonia solani;* in particular *Phakopsora pachyrhizi.*

In one aspect, the present invention relates to the method of protecting plants susceptible to and/or under attack by phytopathogenic fungi containing an amino acid substitution F129L in the mitochondrial cytochrome b protein conferring resistance to Qo inhibitors, which method comprises applying to said plants, treating plant propagation material of said plants with, and/or applying to said phytopathogenic fungi, at least one compound of formula I or a composition comprising at least one compound of formula I.

According to another embodiment, the method for combating phytopathogenic fungi, comprises: a) identifying the phytopathogenic fungi containing an amino acid substitution F129L in the mitochondrial cytochrome b protein conferring resistance to Qo inhibitors, or the materials, plants, the soil or seeds that are at risk of being diseased from phytopathogenic fungi as defined herein, and b) treating said fungi or the materials, plants, the soil or plant propagation material with an effective amount of at least one compound of formula I, or a composition comprising it thereof.

The term "phytopathogenic fungi an amino acid substitution F129L in the mitochondrial cytochrome b protein conferring resistance to Qo inhibitors" is to be understood that at least 10% of the fungal isolates to be controlled contain such F129L substitution in the mitochondrial cytochrome b protein conferring resistance to Qo inhibitors, preferably at least 30%, more preferably at least 50%, even more preferably at at least 75% of the fungi, most preferably between 90 and 100%; in particular between 95 and 100%.

The compounds I and compositions thereof, respectively, are also suitable for controlling harmful microorganisms in the protection of stored products or harvest, and in the protection of materials.

The term "stored products or harvest" is understood to denote natural substances of plant or animal origin and their processed forms for which long-term protection is desired. Stored products of plant origin, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and alike. Preferably, "stored products" is understood to denote natural substances of plant origin and their processed forms, more preferably fruits and their processed forms, such as pomes, stone fruits, soft fruits and citrus fruits and their processed forms, where application of compounds I and compositions thereof can also prevent disadvantageous effects such as decay, discoloration or mold.

The term "protection of materials" is to be understood to denote the protection of technical and non-living materials, such as adhesives, glues, wood, paper, paperboard, textiles, leather, paint dispersions, plastics, cooling lubricants, fiber, or fabrics against the infestation and destruction by harmful microorganisms, such as fungi and bacteria.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

The compounds I and compositions thereof, respectively, may be used for improving the health of a plant. The invention also relates to a method for improving plant health by treating a plant, its propagation material, and/or the locus where the plant is growing or is to grow with an effective amount of compounds I and compositions thereof, respectively.

The term "plant health" is to be understood to denote a condition of the plant and/or its products which is determined by several indicators alone or in combination with each other, such as yield (e.g. increased biomass and/or increased content of valuable ingredients), plant vigor (e.g. improved plant growth and/or greener leaves ("greening effect")), quality (e.g. improved content or composition of certain ingredients), and tolerance to abiotic and/or biotic stress. The above identified indicators for the health condition of a plant may be interdependent or may result from each other.

The compounds I are employed as such or in form of compositions by treating the fungi, the plants, plant propagation materials, such as seeds; soil, surfaces, materials, or rooms to be protected from fungal attack with a fungicidally effective amount of the active substances. The application can be carried out both before and after the infection of the plants, plant propagation materials, such as seeds; soil, surfaces, materials or rooms by the fungi.

An agrochemical composition comprises a fungicidally effective amount of a compound I. The term "fungicidally effective amount" denotes an amount of the composition or of the compounds I, which is sufficient for controlling harmful fungi on cultivated plants or in the protection of stored products or harvest or of materials and which does not result in a substantial damage to the treated plants, the treated stored products or harvest, or to the treated materials. Such an amount can vary in a broad range and is dependent on various factors, such as the fungal species to be controlled, the treated cultivated plant, stored product, harvest or material, the climatic conditions and the specific compound I used.

Plant propagation materials may be treated with compounds I as such or a composition comprising at least one compound I prophylactically either at or before planting or transplanting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials, such as seeds, e.g. by dusting, coating, or drenching, amounts of active substance of generally from 0.1 to 1000 g, preferably from 1 to 1000 g, more preferably from 1 to 100 g and most preferably from 5 to 100 g, per 100 kg of plant propagation material (preferably seeds) are required.

The user applies the agrochemical composition usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

The compounds I and the compositions containing them may be applied in combination with, or by utilizing smart agricultural technologies, such as precision agriculture, remote and proximate imaging and image recognition, or smart agricultural site management programs. Such technologies typically include models, e.g. computer programs, that support the user by considering information from a variety of sources to increase quality and yield of harvested material, reduce damage by pests including the prediction of pest pressure and smart application of crop protection products, secure environmental protection, support quick and reliable agronomic decision making, reduce usage of fertilizers and crop protection products, reduce product residues in consumables, increase spatial and temporal precision of agronomical measures, automate processes, and enable traceability of measures. Commercially available systems which include agronomic models are e.g. FieldScripts^{™} (The Climate Corporation), Xarvio^{™} (BASF) and AGLogic^{™} (John Deere).

Information input for these models include but is not limited to soil data, information on the plants that are currently growing or that may grow at the area of interest including crops and/or unwanted vegetation, weather information, information on the location of the area and directly derivable information thereof, information on pest pressure, information on beneficial organisms, comprising forecast, present and / or historic information of any of the aforementioned.

The information usable for precision agriculture may be based on input by at least one user, be accessible from external data sources and databases, or be based on sensor data. Data sources typically include proximate-detection systems like soil-borne sensors and remote sensing as may be achieved by imaging with unmanned airborne vehicles like drones, or satellites. Sensors may be included in an Internet-of-Things system and may be directly or indirectly connected to the processing unit, e.g. via a wireless network and/or cloud applications. The information is typically taken into account by at least one processing unit and used to provide recommendations, and to generate control signals.

Typical technologies that are used in smart agricultural technologies include self-steering robots (such as tractors, harvesters, drones), artificial intelligence (e.g. machine learning), imaging technologies (e.g. image segmentation technologies), big data analysis, and model gene¬ra¬tion, cloud computing, and machine-to-machine communication.

Precision agriculture such as precision farming is characterized by spatially and/or temporally resolved, targeted application of active ingredients like pesticides, plant-growth-regulators, ferti¬lizers, and/or water including the variation of application rates over the agronomic site, zone or spot application, and of the spatially and/or temporally resolved, targeted planting or seeding of desired plant propagation material to an agronomic site. Precision farming typically includes the use of geo-positioning technologies like GPS for gaining information on the location and bounda¬ries of the area of interest, the utilized application equipment, sensing equipment and recorded data, and to control the actions of farm vehicles such as spraying. By combining geo-positioning data with (digital) maps, it is possible to (semi)-automate agricultural measures at the site of interest, e.g. by using (semi)-autonomous spraying or seeding equipment.

Precision farming may typically include the application of smart spraying equipment, e.g. spot spraying, and precision spraying at a farm, e.g. by irrigation systems, tractors, robots, helicopters, airplanes, unmanned aerial vehicles, such as drones. Such equipment usually includes input sensors (e.g. a camera) and a processing unit configured to analyze the input data and configured to provide a recommendation or decision based on the analysis of input data to apply the compounds I or compositions comprising them to the agronomic site, e.g. the soil, the crop plants, or to control pests in a specific and precise manner. For example, pests may be detected, identified, and/or classified from imagery acquired by a camera. Such identification and/ classification can make use of image processing algorithms, which may utilize artificial intelligence (e.g. machine learning algorithms), or decision trees. In this manner, the compounds I or com¬po¬sitions described herein can be applied only at the required location, point in time and dose rate.

The compounds I, their N-oxides and salts can be converted into customary types of agrochemical compositions, e.g. solutions, emulsions, suspensions, dusts, powders, pastes, granules, pressings, capsules, and mixtures thereof. Examples for composition types (see also "Catalogue of pesticide formulation types and international coding system", Technical Monograph No. 2, 6th Ed. May 2008, CropLife International) are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials, such as seeds (e.g. GF). The compositions are prepared in a known manner, such as described by Mollet and Grubemann, Formulation technology, Wiley VCH, Weinheim, 2001; or by Knowles, New developments in crop protection product formulation, Agrow Reports DS243, T&F Informa, London, 2005. The invention also relates to agrochemical compositions comprising an auxiliary and at least one compound I.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers, and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e.g. toluene, paraffin, tetrahydronaphthalene, and alkylated naphthalenes; alcohols, e.g. ethanol, propanol, butanol, benzyl alcohol, cyclohexanol, glycols; DMSO; ketones, e.g. cyclohexanone; esters, e.g. lactates, carbonates, fatty acid esters, gamma-butyrolactone; fatty acids; phosphonates; amines; amides, e.g. *N*-methyl pyrrolidone, fatty acid dimethyl amides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (Int. Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and of alkyl naphthalenes, sulfosuccinates, or sulfosuccinamates. Examples of sulfates are sulfates of fatty acids, of oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, *N*-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of *N*-substituted fatty acid amides are fatty acid glucamides or fatty acid alkanolamides. Examples of esters are fatty acid esters, glycerol esters, or monoglycerides. Examples of sugar-based surfactants are sorbitans, ethoxylated sorbitans, sucrose and glucose esters, or alkylpolyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide, and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the compound I on the target. Examples are surfactants, mineral or vegetable oils, and other auxiliaries, e.g. as listed by Knowles, Adjuvants and additives, Agrow Reports DS256, T&F Informa UK, 2006, chapter 5. Suitable thickeners are polysaccharides (e.g. xanthan gum, carboxymethyl cellulose), inorganic clays (organically modified or unmodified), polycarboxylates, and silicates. Suitable bactericides are bronopol and isothiazolinone derivatives, such as alkylisothiazolinones and benzisothiazolinones. Suitable anti-freezing agents are ethylene glycol, propylene glycol, urea, and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants). Suitable tackifiers or binders are polyvinyl pyrrolidones, polyvinyl acetates, polyvinyl alcohols, polyacrylates, biological or synthetic waxes, and cellulose ethers.

The agrochemical compositions generally comprise between 0.01 and 95 %, preferably between 0.1 and 90 %, more preferably between 1 and 70 %, and in particular between 10 and 60 %, by weight of active substances (e.g. at least one compound I). The agrochemical compositions generally comprise between 5 and 99.9 %, preferably between 10 and 99.9 %, more preferably between 30 and 99 %, and in particular between 40 and 90 %, by weight of at least one auxiliary. The active substances (e.g. compounds I) are employed in a purity of from 90 % to 100 %, preferably from 95-% to 100 % (according to NMR spectrum).

For the purposes of treatment of plant propagation materials, particularly seeds, solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60 % by weight, preferably from 0.1 to 40 %, in the ready-to-use preparations. Application can be carried out before or during sowing. Methods for applying compound I and compositions thereof, respectively, onto plant propagation material, especially seeds, include dressing, coating, pelleting, dusting, soaking, as well as in-furrow application methods. Preferably, compound I or the compositions thereof, respectively, are applied on to the plant propagation material by a method such that germination is not induced, e.g. by seed dressing, pelleting, coating, and dusting.

Various types of oils, wetters, adjuvants, fertilizers, or micronutrients, and further pesticides (e.g. fungicides, growth regulators, herbicides, insecticides, safeners) may be added to the compounds I or the compositions thereof as premix, or, not until immediately prior to use (tank mix). These agents can be admixed with the compositions according to the invention in a weight ratio of 1:100 to 100:1, preferably 1:10 to 10:1.

A pesticide is generally a chemical or biological agent (such as pestidal active ingredient, compound, composition, virus, bacterium, antimicrobial, or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term "pesticide" includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology e.g. to increase plant growth, biomass, yield or any other quality parameter of the harvestable goods of a crop plant.

Biopesticides have been defined as a form of pesticides based on microorganisms (bacteria, fungi, viruses, nematodes, etc.) or natural products (compounds, such as metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances that control pests or provide other crop protection uses as defined below, but are relatively non-toxic to mammals.

Mixing the compounds I or the compositions comprising them in the use form as fungicides with other fungicides results in many cases in an expansion of the fungicidal spectrum of activity or in a prevention of fungicide resistance development. Furthermore, in many cases, synergistic effects are obtained (synergistic mixtures).

The following list of pesticides II, in conjunction with which the compounds I can be used, is intended to illustrate the possible combinations but does not limit them:
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxy-strobin (A.1.17), 2-(2-(3-(2,6-dichlorophenyl)-1-methyl-allylideneaminooxymethyl)-phenyl)-2-methoxyimino-*N*-methyl-acetamide (A.1.18), pyribencarb (A.1.19), triclopyricarb/chloro-dincarb (A.1.20), famoxadone (A.1.21), fenamidone (A.1.21), methyl-*N*-[2-[(1,4-dimethyl-5-phenyl-pyrazol-3-yl)oxylmethyl]phenyl]-*N*-methoxy-carbamate (A.1.22), metyltetraprole (A.1.25), (*Z*,2*E*)-5-[1-(2,4-dichlorophenyl)pyrazol-3-yl]-oxy-2-methoxyimino-*N*,3-dimethyl-pent-3-enamide (A.1.34), (Z,2E)-5-[1-(4-chlorophenyl)pyrazol-3-yl]oxy-2-methoxyimino-*N*,3-dimethyl-pent-3-enamide (A.1.35), pyriminostrobin (A.1.36), bifujunzhi (A.1.37), 2-(ortho-((2,5-dimethylphenyl-oxymethylen)phenyl)-3-methoxy-acrylic acid methylester (A.1.38);
   - inhibitors of complex III at Qᵢ site: cyazofamid (A.2.1), amisulbrom (A.2.2), [(6S,7R,8R)-8-benzyl-3-[(3-hydroxy-4-methoxy-pyridine-2-carbonyl)amino]-6-methyl-4,9-dioxo-1,5-dioxonan-7-yl] 2-methylpropanoate (A.2.3), fenpicoxamid (A.2.4), florylpicoxamid (A.2.5), metarylpicoxamid (A.2.6);
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28), N-[2-[2-chloro-4-(trifluoromethyl)phenoxy]phenyl]-3-(difluoromethyl)-5-fluoro-1-methyl-pyrazole-4-carboxamide (A.3.29), methyl (E)-2-[2-[(5-cyano-2-methyl-phenoxy)methyl]phenyl]-3-methoxy-prop-2-enoate (A.3.30), isoflucypram (A.3.31), 2-(difluoromethyl)-*N*-(1,1,3-trimethyl-indan-4-yl)-pyridine-3-carboxamide (A.3.32), 2-(difluoromethyl)-*N*-[(3*R*)-1,1,3-trimethylindan-4-yl]-pyridine-3-carboxamide (A.3.33), 2-(difluoromethyl)-*N*-(3-ethyl-1,1-dimethyl-indan-4-yl)-pyridine-3-carboxamide (A.3.34), 2-(difluoromethyl)-*N*-[(3*R*)-3-ethyl-1,1-dimethyl-indan-4-yl]-pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3*R*)-1,1-dimethyl-3-propyl-indan-4-yl]-pyridine-3-carboxamide (A.3.37), 2-(difluoromethyl)-*N*-(3-isobutyl-1,1-dimethyl-indan-4-yl)-pyridine-3-carboxamide (A.3.38), 2-(difluoromethyl)-*N*-[(3*R*)-3-isobutyl-1,1-dimethyl-indan-4-yl]pyridine-3-carboxamide (A.3.39) cyclobutrifluram (A.3.24);
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e.g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(trifluoromethoxy)phenyl]-2-pyridyl]propan-2-ol (B.1.32), fluoxytioconazole (B.1.33), ipfentrifluconazole (B.1.37), mefentrifluconazole (B.1.38), (2R)-2-[4-(4-chlorophenoxy)-2-(trifluoromethyl)phenyl]-1-(1,2,4-triazol-1-yl)propan-2-ol, (2S)-2-[4-(4-chlorophenoxy)-2-(trifluoromethyl)phenyl]-1-(1,2,4-triazol-1-yl)propan-2-ol, 2-(chloromethyl)-2-methyl-5-(p-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52), 4-[[6-[2-(2,4-difluorophenyl)-1,1-difluoro-2-hydroxy-3-(1,2,4-triazol-1-yl)propyl]-3-pyridyl]oxy]benzonitrile (B.1.53), 2-[6-(4-bromo-phenoxy)-2-(trifluoromethyl)-3-pyridyl]-1-(1,2,4-triazol-1-yl)propan-2-ol (B.1.54), 2-[6-(4-chlorophenoxy)-2-(trifluoromethyl)-3-pyridyl]-1-(1,2,4-triazol-1-yl)propan-2-ol (B.1.55), methyl 2-[2-chloro-4-(4-chlorophenoxy)phenyl]-2-hydroxy-3-(1,2,4-triazol-1-yl)propanoate (B.1.56), methyl 2-[2-chloro-4-(4-chlorophenoxy)phenyl]-2-hydroxy-3-(1,2,4-triazol-1-yl)propanoic acid (B.1.57);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), *N*-ethyl-2-[(3-ethynyl-8-methyl-6-quinolyl)oxy]butanamide (D.1.8), *N*-ethyl-2-[(3-ethynyl-8-methyl-6-quinolyl)oxy]-2-methy1-sulfanyl-acetamide (D.1.9), 2-[(3-ethynyl-8-methyl-6-quinolyl)oxy]-*N*-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-*N*-(2-fluoroethyl)-2-methylsulfanyl-acetamide (D.1.15), 4-(2-bromo-4-fluorophenyl)-*N*-(2-chloro-6-fluoro-phenyl)-2,5-dimethyl-pyrazol-3-amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7), phenamacril (D.2.8);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7), zinc thiazole (G.2.8);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), fluoxapiprolin (G.5.3), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyr-azol-1-yl]acetyl]-4-piperidyl]-*N*-tetralin-1-yl-pyridine-2-carboxamide (G.5.6), 4-[1-[2-[5-cyclopropyl-3-(difluoromethyl)pyrazol-1-yl]acetyl]-4-piperidyl]-*N*-tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifluoromethyl)pyrazol-1-yl]acetyl]-4-piperidyl]-*N*-tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1*H*,5*H*-[1,4]dithiino[2,3-c:5,6-c']dipyrrole-1,3,5,7(2*H*,6*H*)-tetraone (H.4.10);
I) Cell wall synthesis inhibitors
   - inhibitors of glucan synthesis: validamycin (1.1.1), polyoxin B (1.1.2);
   - melanin synthesis inhibitors: pyroquilon (1.2.1), tricyclazole (1.2.2), carpropamid (1.2.3), dicyclomet (1.2.4), fenoxanil (1.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4-cyclopropyl-*N*-(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flumetylsulforim (K.1.60), flusulfamide (K.1.15), flutianil (K.1.16), harpin (K.1.17), methasulfocarb (K.1.18), nitrapyrin (K.1.19), nitrothal-isopropyl (K.1.20), tolprocarb (K.1.21), oxin-copper (K.1.22), proquinazid (K.1.23), seboctylamine (K.1.61), tebufloquin (K.1.24), tecloftalam (K.1.25), triazoxide (K.1.26), *N*'-(4-(4-chloro-3-trifluoromethyl-phenoxy)-2,5-dimethyl-phenyl)-*N*-ethyl-*N*-methyl formamidine (K.1.27), *N*'-(4-(4-fluoro-3-trifluoromethyl-phenoxy)-2,5-dimethyl-phenyl)-*N*-ethyl-*N*-methyl formamidine (K.1.28), *N*'-[4-[[3-[(4-chlorophenyl)methyl]-1,2,4-thiadiazol-5-yl]oxy]-2,5-dimethyl-phenyl]-*N-*ethyl-*N*-methyl-formamidine (K.1.29), *N*'-(5-bromo-6-indan-2-yloxy-2-methyl-3-pyridyl)-*N-*ethyl-*N*-methyl-formamidine (K.1.30), *N*'-[5-bromo-6-[1-(3,5-difluorophenyl)ethoxy]-2-methyl-3-pyridyl]-*N*-ethyl-*N*-methyl-formamidine (K.1.31), *N*'-[5-bromo-6-(4-isopropylcyclohexoxy)-2-methyl-3-pyridyl]-*N*-ethyl-*N*-methyl-formamidine (K.1.32), *N*'-[5-bromo-2-methyl-6-(1-phenylethoxy)-3-pyridyl]-*N*-ethyl-*N*-methyl-formamidine (K.1.33), *N*'-(2-methyl-5-trifluoromethyl-4-(3-trimethylsilanyl-propoxy)-phenyl)-*N*-ethyl-*N*-methyl formamidine (K.1.34), *N*'-(5-difluoromethyl-2-methyl-4-(3-trimethylsilanyl-propoxy)-phenyl)-*N*-ethyl-*N*-methyl formamidine (K.1.35), 2-(4-chloro-phenyl)-*N*-[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3-yl]-pyridine (K.1.38), 5-chloro-1-(4,6-dimethoxy-pyrimidin-2-yl)-2-methyl-1*H*-benzoimidazole (K.1.39), ethyl (Z)-3-amino-2-cyano-3-phenyl-prop-2-enoate (K.1.40), picarbutrazox (K.1.41), pentyl *N*-[6-[[(*Z*)-[(1-methyltetrazol-5-yl)-phenyl-methylene]amino]oxymethyl]-2-pyridyl]carbamate (K.1.42), but-3-ynyl *N*-[6-[[(*Z*)-[(1-methyltetrazol-5-yl)-phenyl-methylene]amino]oxymethyl]-2-pyridyl]carbamate (K.1.43), ipflufenoquin (K.1.44), quinofumelin (K.1.47), benziothiazolinone (K.1.48), bromothalonil (K.1.49), 2-(6-benzyl-2-pyridyl)quinazoline (K.1.50), 2-[6-(3-fluoro-4-methoxy-phenyl)-5-methyl-2-pyridyl]quinazoline (K.1.51), dichlobentiazox (K.1.52), *N*'-(2,5-dimethyl-4-phenoxy-phenyl)-*N*-ethyl-*N*-methyl-formamidine (K.1.53), aminopyrifen (K.1.54), fluopimomide (K.1.55), *N*'-[5-bromo-2-methyl-6-(1-methyl-2-propoxy-ethoxy)-3-pyridyl]-*N-*ethyl-*N*-methyl-formamidine (K.1.56), *N*'-[4-(4,5-dichlorothiazol-2-yl)oxy-2,5-dimethylphenyl]-*N*-ethyl-*N*-methyl-formamidine (K.1.57), flufenoxadiazam (K.1.58), *N*-methyl-4-[5-(trifluoromethyl)-1,2,4-oxadiazol-3-yl]benzenecarbothioamide (K.1.59), *N*-methoxy-*N*-[[4-[5-(trifluoromethyl)-1,2,4-oxadiazol-3-yl]phenyl]methyl]cyclopropanecarboxamide (K.1.60; WO2018/177894, WO 2020/212513), *N*-((4-[5-(trifluoromethyl)-1,2,4-oxadiazol-3-yl]phenyl)methyl)propanamide (K.1.62), 3,3,3-trifluoro-*N*-[[3-fluoro-4-[5-(trifluoromethyl)-1,2,4-oxadiazol-3-yl]phenyl]methyl]propanamide (K.1.63), 3,3,3-trifluoro-*N*-[[2-fluoro-4-[5-(trifluoromethyl)-1,2,4-oxadiazol-3-yl]phenyl]methyl]propanamide (K.1.64), *N*-[2,3-difluoro-4-[5-(trifluoromethyl)-1,2,4-oxadiazol-3-yl]benzyl]butanamide (K.1.65), *N*-[[2,3-difluoro-4-[5-(trifluoromethyl)-1,2,4-oxadiazol-3-yl]phenyl]methyl]-3,3,3-trifluoro-propanamide (K.1.66), 1-methoxy-1-methyl-3-[[4-[5-(trifluoromethyl)-1,2,4-oxadiazol-3-yl]phenyl]methyl]-urea (K.1.67), 1,1-diethyl-3-[[4-[5-[trifluoromethyl]-1,2,4-oxadiazol-3-yl]phenyl]methyl]urea (K.1.68), *N*,2-dimethoxy-*N*-[[4-[5-(trifluoromethyl)-1,2,4-oxadiazol-3-yl]phenyl]methyl]propanamide (K.1.69), *N*-ethyl-2-methyl-*N*-[[4-[5-(trifluoromethyl)-1,2,4-oxadiazol-3-yl]phenyl]methyl]propanamide (K.1.70), 1-methoxy-3-methyl-1-[[4-[5-(trifluoromethyl)-1,2,4-oxadiazol-3-yl]-phenyl]methyl]urea (K.1.71), 1-[[4-[5-(trifluoromethyl)-1,2,4-oxadiazol-3-yl]phenyl]methyl]pyrrolidin-2-one (K.1.72), 1-[[4-[5-(trifluoromethyl)-1,2,4-oxadiazol-3-yl]phenyl]methyl]piperidin-2-one (K.1.73), 4-[[4-[5-(trifluoromethyl)-1,2,4-oxadiazol-3-yl]phenyl]methyl]morpholin-3-one (K.1.74), 4,4-dimethyl-2-[[4-[5-(trifluoromethyl)-1,2,4-oxadiazol-3-yl]phenyl]methyl]isoxazolidin-3-one (K.1.75), 2-[[4-[5-(trifluoromethyl)-1,2,4-oxadiazol-3-yl]phenyl]methyl]isoxazolidin-3-one (K.1.76), 5,5-dimethyl-2-[[4-[5-(trifluoromethyl)-1,2,4-oxadiazol-3-yl]phenyl]methyl]-isoxazolidin-3-one (K.1.77), 3,3-dimethyl-1-[[4-[5-(trifluoromethyl)-1,2,4-oxadiazol-3-yl]phenyl]methyl]piperidin-2-one (K.1.78), 2-[[4-[5-(trifluoromethyl)-1,2,4-oxadiazol-3-yl]phenyl]methyl]oxazinan-3-one (K.1.79), 1-[[3-fluoro-4-[5-(trifluoromethyl)-1,2,4-oxadiazol-3-yl]phenyl]-methyl]azepan-2-one (K.1.80), 4,4-dimethyl-1-[[4-[5-(trifluoromethyl)-1,2,4-oxadiazol-3-yl]-phenyl]methyl]pyrrolidin-2-one (K.1.81), 5-methyl-1-[[4-[5-(trifluoromethyl)-1,2,4-oxadiazol-3-yl]phenyl]methyl]pyrrolidin-2-one (K.1.82), ethyl 1-[[4-[5-(trifluoromethyl)-1,2,4-oxadiazol-3-yl]phenyl]methyl]pyrazole-4-carboxylate (K.1.83), *N*-methyl-1-[[4-[5-(trifluoromethyl)-1,2,4-oxadiazol-3-yl]phenyl]methyl]pyrazole-4-carboxamide (K.1.84), *N*,*N*-dimethyl-1-[4-[5-(trifluoromethyl)-1,2,4-oxadiazol-3-yl]benzyl]-1H-1,2,4-triazol-3-amine (K.1.85), *N*-methoxy-*N*-methyl-1-[[4-[5-(trifluoromethyl)-1,2,4-oxadiazol-3-yl]phenyl]methyl]pyrazole-4-carboxamide (K.1.86), propyl-1-[[4-[5-(trifluoromethyl)-1,2,4-oxadiazol-3-yl]phenyl]methyl]-pyrazole-4-carboxamide (K.1.87), *N*-methoxy-1-[[4-[5-(trifluoromethyl)-1,2,4-oxadiazol-3-yl]phenyl]methyl]pyrazole-4-carboxamide (K.1.88), *N*-allyl-*N*-[[4-[5-(trifluoromethyl)-1,2,4-oxadiazol-3-yl]phenyl]methyl]propanamide (K.1.89), 3-ethyl-1-methoxy-1-[[4-[5-(trifluoromethyl)-1,2,4-oxadiazol-3-yl]phenyl]methyl]urea (K.1.90), 1,3-dimethoxy-1-[[4-[5-(trifluoromethyl)-1,2,4-oxadiazol-3-yl]phenyl]methyl]urea (K.1.91), *N*-allyl-*N*-[[4-[5-(trifluoromethyl)-1,2,4-oxadiazol-3-yl]phenyl]methyl]acetamide (K.1.92), *N*-[4-[5-(trifluoromethyl)-1,2,4-oxadiazol-3-yl]benzyl]cyclopropanecarboxamide (K.1.93), 1-methyl-3-[[4-[5-(trifluoromethyl)-1,2,4-oxadiazol-3-yl]phenyl]methyl]urea (K.1.94), *N*'-[2-chloro-4-(2-fluorophenoxy)-5-methyl-phenyl]-*N*-ethyl-*N*-methyl-formamidine (K.1.95), *N*'-[2-chloro-4-[(4-methoxyphenyl)methyl]-5-methyl-phenyl]-*N*-ethyl-*N*-methyl-formamidine (K.1.96), *N*'-[2-chloro-4-[(4-cyano-phenyl)methyl]-5-methyl-phenyl]-*N*-ethyl-*N*-methyl-formamidine (K.1.97), *N*'-[2,5-dimethyl-4-(*o*-tolylmethyl)phenyl]-*N*-ethyl-*N*-methyl-formamidine (K.1.98), 6-chloro-3-(3-cyclopropyl-2-fluoro-phenoxy)-*N*-[2-(2,4-dimethylphenyl)-2,2-difluoro-ethyl]-5-methyl-pyridazine-4-carboxamide (K.1.99), 3-(3-bromo-2-fluoro-phenoxy)-6-chloro-*N*-[2-(2-chloro-4-methyl-phenyl)-2,2-difluoro-ethyl]-5-methyl-pyridazine-4-carboxamide (K.1.100), 6-chloro-*N*-[2-(2-chloro-4-methyl-phenyl)-2,2-difluoro-ethyl]-3-(3-cyclopropyl-2-fluoro-phenoxy)-5-methyl-pyridazine-4-carboxamide (K.1.101), 6-chloro-3-(3-cyclopropyl-2-fluoro-phenoxy)-*N*-[2-(3,4-dimethylphenyl)-2,2-difluoro-ethyl]-5-methyl-pyridazine-4-carboxamide (K.1.102), 6-chloro-3-(3-chloro-2-fluoro-phenoxy)-*N*-[2-(2,4-dimethylphenyl)-2,2-difluoro-ethyl]-5-methyl-pyridazine-4-carboxamide (K.1.103), *N*-[2-(2-bromo-4-methyl-phenyl)-2,2-difluoro-ethyl]-6-chloro-3-(3-cyclopropyl-2-fluoro-phenoxy)-5-methyl-pyridazine-4-carboxamide (K.1.104);
L) Biopesticides
   L1) Microbial pesticides with fungicidal, bactericidal, viricidal and/or plant defense activator activity: *Ampelomyces quisqualis, Aspergillus flavus, Aureobasidium pullulans, Bacillus altitudinis, B. amyloliquefaciens, B. amyloliquefaciens* ssp. *plantarum* (also referred to as *B*. *velezensis*)*, B. megaterium, B. mojavensis, B. mycoides, B. pumilus, B. simplex, B. solisalsi, B. subtilis, B. subtilis* var. *amyloliquefaciens, B. velezensis, Candida oleophila,* C. *saitoana, Clavibacter michiganensis* (bacteriophages), *Coniothyrium minitans, Cryphonectria parasitica, Cryptococcus albidus, Dilophosphora alopecuri, Fusarium oxysporum, Clonostachys* rosea f. *catenulate* (also named *Gliocladium catenulatum*)*, Gliocladium roseum, Lysobacter antibioticus, L. enzymogenes, Metschnikowia fructicola, Microdochium dimerum, Microsphaeropsis ochracea, Muscodor albus, Paenibacillus alvei, Paenibacillus epiphyticus, P. polymyxa, Pantoea vagans, Penicillium bilaiae, Phlebiopsis gigantea, Pseudomonas* sp., *Pseudomonas chloraphis, Pseudozyma flocculosa, Pichia anomala, Pythium oligandrum, Sphaerodes mycoparasitica, Streptomyces griseoviridis, S. lydicus, S. violaceusniger, Talaromyces flavus, Trichoderma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia,* zucchini yellow mosaic virus (avirulent strain);
   L2) Biochemical pesticides with fungicidal, bactericidal, viricidal and/or plant defense activator activity: harpin protein, *Reynoutria sachalinensis* extract;
   L3) Microbial pesticides with insecticidal, acaricidal, molluscidal and/or nematicidal activity: *Agrobacterium radiobacter, Bacillus cereus, B. firmus, B. thuringiensis, B. thuringiensis* ssp. *aizawai, B. t.* ssp. *israelensis, B. t.* ssp. *galleriae, B. t.* ssp. *kurstaki, B. t.* ssp. *tenebrionis, Beauveria bassiana, B. brongniartii, Burkholderia* spp., *Chromobacterium subtsugae, Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpa zea* nucleopolyhedrovirus (HzNPV), *Helicoverpa zea* single capsid nucleopolyhedrovirus (HzSNPV), *Heterorhabditis bacteriophora, Isaria fumoso*rosea, *Lecanicillium longisporum, L. muscarium, Metarhizium anisopliae, M. anisopliae* var. *anisopliae, M. anisopliae* var. *acridum, Nomuraea rileyi, Paecilomyces fumosoroseus, P. lilacinus, Paenibacillus popilliae, Pasteuria* spp., *P. nishizawae, P. penetrans, P*. *ramosa, P. thornea, P. usgae, Pseudomonas fluorescens, Spodoptera littoralis* nucleopolyhedrovirus (SpliNPV), *Steinernema carpocapsae, S. feltiae, S. kraussei, Streptomyces galbus, S. microflavus;*
   L4) Biochemical pesticides with insecticidal, acaricidal, molluscidal, pheromone and/or nematicidal activity: L-carvone, citral, (*E*,*Z*)-7,9-dodecadien-1-yl acetate, ethyl formate, (*E*,*Z*)-2,4-ethyl decadienoate (pear ester), (*Z*,*Z*,*E*)-7,11,13-hexadecatrienal, heptyl butyrate, isopropyl myristate, lavanulyl senecioate, cis-jasmone, 2-methyl 1-butanol, methyl eugenol, methyl jasmonate, (*E*,*Z*)-2,13-octadecadien-1-ol, (*E*,*Z*)-2,13-octadecadien-1-ol acetate, (*E*,*Z*)-3,13-octadecadien-1-ol, (*R*)-1-octen-3-ol, pentatermanone, (*E*,*Z*,*Z*)-3,8,11-tetradecatrienyl acetate, (*Z*,*E*)-9,12-tetradecadien-1-yl acetate, (Z)-7-tetradecen-2-one, (Z)-9-tetradecen-1-yl acetate, (Z)-11-tetradecenal, (*Z*)-11-tetradecen-1-ol, extract of *Chenopodium ambrosiodes,* Neem oil, Quillay extract;
   L5) Microbial pesticides with plant stress reducing, plant growth regulator, plant growth promoting and/or yield enhancing activity: *Azospirillum amazonense, A. brasilense, A. lipoferum, A. irakense, A. halopraeferens, Bradyrhizobium* spp., *B. elkanii, B. japonicum, B. liaoningense, B. lupini, Delftia acidovorans, Glomus intraradices, Mesorhizobium* spp., *Rhizobium leguminosarum* bv. *phaseoli, R. I*. bv. *trifolii, R. I*. bv. *viciae, R. tropici, Sinorhizobium meliloti;*
O) Insecticides from classes O.1 to O.29
   O.1 Acetylcholine esterase (AChE) inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, triazamate; acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
   O.2 GABA-gated chloride channel antagonists: endosulfan, chlordane; ethiprole, fipronil, flufiprole, pyrafluprole, pyriprole;
   O.3 Sodium channel modulators: acrinathrin, allethrin, d-cis-trans allethrin, d-trans allethrin, bifenthrin, kappa-bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin, metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, transfluthrin; DDT, methoxychlor;
   O.4 Nicotinic acetylcholine receptor (nAChR) agonists: acetamiprid, clothianidin, cycloxaprid, dinotefuran, imidacloprid, nitenpyram, thiacloprid, thiamethoxam; 4,5-dihydro-*N*-nitro-1-(2-oxiranylmethyl)-1*H*-imidazol-2-amine, (2*E*)-1-[(6-chloropyridin-3-yl)methyl]-*N'*-nitro-2-pentylidenehydrazinecarboximidamide; 1-[(6-chloropyridin-3-yl)methyl]-7-methyl-8-nitro-5-propoxy-1,2,3,5,6,7-hexahydroimidazo[1,2-a]pyridine; nicotine; sulfoxaflor, flupyradifurone, triflumezopyrim, fenmezoditiaz;
   O.5 Nicotinic acetylcholine receptor allosteric activators: spinosad, spinetoram;
   O.6 Chloride channel activators: abamectin, emamectin benzoate, ivermectin, lepimectin, milbemectin;
   O.7 Juvenile hormone mimics: hydroprene, kinoprene, methoprene; fenoxycarb, pyriproxyfen;
   O.8 miscellaneous non-specific (multi-site) inhibitors: methyl bromide and other alkyl halides; chloropicrin, sulfuryl fluoride, borax, tartar emetic;
   O.9 Chordotonal organ TRPV channel modulators: pymetrozine, pyrifluquinazon;
   O.10 Mite growth inhibitors: clofentezine, hexythiazox, diflovidazin; etoxazole;
   O.11 Microbial disruptors of insect midgut membranes: *Bacillus thuringiensis, B. sphaericus* and the insecticdal proteins they produce: *Bacillus thuringiensis* subsp. *israelensis, B. sphaericus, B. thuringiensis* subsp. *aizawai, B. thuringiensis* subsp. *kurstaki, B. thuringiensis* subsp. *tenebrionis,* the Bt crop proteins: Cry1Ab, Cry1Ac, Cry1Fa, Cry2Ab, mCry3A, Cry3Ab, Cry3Bb, Cry34/35Ab1;
   O.12 Inhibitors of mitochondrial ATP synthase: diafenthiuron; azocyclotin, cyhexatin, fenbutatin oxide, propargite, tetradifon;
   O.13 Uncouplers of oxidative phosphorylation via disruption of the proton gradient: chlorfenapyr, DNOC, sulfluramid;
   O.14 Nicotinic acetylcholine receptor (nAChR) channel blockers: bensultap, cartap hydrochloride, thiocyclam, thiosultap sodium;
   O.15 Inhibitors of the chitin biosynthesis type 0: bistrifluron, chlorfluazuron, diflubenzuron, flucycloxuron, flufenoxuron, hexaflumuron, lufenuron, novaluron, noviflumuron, teflubenzuron, triflumuron;
   O.16 Inhibitors of the chitin biosynthesis type 1: buprofezin;
   O.17 Moulting disruptors: cyromazine;
   O.18 Ecdyson receptor agonists: methoxyfenozide, tebufenozide, halofenozide, fufenozide, chromafenozide;
   O.19 Octopamin receptor agonists: amitraz;
   O.20 Mitochondrial complex III electron transport inhibitors: hydramethylnon, acequinocyl, fluacrypyrim, bifenazate;
   O.21 Mitochondrial complex I electron transport inhibitors: fenazaquin, fenpyroximate, pyrimidifen, pyridaben, tebufenpyrad, tolfenpyrad; rotenone;
   O.22 Voltage-dependent sodium channel blockers: indoxacarb, metaflumizone, 2-[2-(4-cyanophenyl)-1-[3-(trifluoromethyl)phenyl]ethylidene]-*N*-[4-(difluoromethoxy)phenyl]-hydrazine-carboxamide, *N*-(3-chloro-2-methylphenyl)-2-[(4-chlorophenyl)-[4-[methyl(methylsulfonyl)-amino]phenyl]methylene]-hydrazinecarboxamide, *N*-[4-chloro-2-[[(1,1-dimethylethyl)amino]carbonyl]-6-methylphenyl]-1-(3-chloro-2-pyridinyl)-3-(fluoromethoxy)-1*H*-pyrazole-5-carboxamide, 2-[2-(4-cyanophenyl)-1-[3-(trifluoromethyl)phenyl]ethylidene]-*N*-[4-(difluoromethoxy)phenyl]-hydrazinecarboxamide;
   O.23 Inhibitors of the of acetyl CoA carboxylase: spirodiclofen, spiromesifen, spirotetramat, spiropidion, spirobudiclofen, 11-(4-chloro-2,6-dimethylphenyl)-12-hydroxy-1,4-dioxa-9-azadispiro[4.2.4.2]tetradec-11-en-10-one, spidoxamat;
   O.24 Mitochondrial complex IV electron transport inhibitors: aluminium phosphide, calcium phosphide, phosphine, zinc phosphide, cyanide;
   O.25 Mitochondrial complex II electron transport inhibitors: cyenopyrafen, cyflumetofen, cyetpyrafen;
   O.26 Ryanodine receptor-modulators: chlorantraniliprole, cyantraniliprole, cyclaniliprole, flubendiamide, fluchlodiniliprole, (*R*)-3-chloro-*N*¹-{2-methyl-4-[1,2,2,2 -tetrafluoro-1-(trifluoromethyl)ethyl]phenyl}-*N*²-(1-methyl-2-methylsulfonylethyl)phthalamide, (S)-3-chloro-*N*¹-{2-methyl-4-[1,2,2,2-tetrafluoro-1-(trifluoromethyl)ethyl]phenyl}-*N*²-(1-methyl-2-methylsulfonylethyl)phthalamide, methyl-2-[3,5-dibromo-2-({[3-bromo-1-(3-chloropyridin-2-yl)-1*H*-pyrazol-5-yl]carbonyl}amino)benzoyl]-1,2-dimethylhydrazinecarboxylate; *N*-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide; *N*-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; *N*-[2-(5-amino-1,3,4-thiadiazol-2-yl)-4-chloro-6-methylphenyl]-3-bromo-1-(3-chloro-2-pyridinyl)-1*H*-pyrazole-5-carboxamide; 3-chloro-1-(3-chloro-2-pyridinyl)-*N*-[2,4-dichloro-6-[[(1-cyano-1-methylethyl)amino]carbonyl]phenyl]-1*H*-pyrazole-5-carboxamide; tetrachlorantraniliprole; tetraniliprole; thiotraniliprole; *N*-[4-chloro-2-[[(1,1-dimethylethyl)-amino]carbonyl]-6-methylphenyl]-1-(3-chloro-2-pyridinyl)-3-(fluoromethoxy)-1*H*-pyrazole-5-carboxamide; cyhalodiamide; *N*-[2-(5-amino-1,3,4-thiadiazol-2-yl)-4-chloro-6-methylphenyl]-3-bromo-1-(3-chloro-2-pyridinyl)-1*H*-pyrazole-5-carboxamide;
   O.27: Chordotonal organ modulators: flonicamid;
   O.28. insecticidal compounds of unknown or uncertain mode of action: afido¬pyro¬pen, afoxolaner, azadirachtin, amidoflumet, ben-zoximate, bromopropylate, chino¬methionat, cryolite, cyproflanilid, dicloromezotiaz, dicofol, dimpropyridaz, flufenerim, flometoquin, fluensulfone, fluhexafon, fluopyram, fluralaner, metaldehyde, metoxadiazone, piperonyl butoxide, pyflubumide, pyridalyl, tioxazafen, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 4-cyano-*N*-[2-cyano-5-[[[2,6-dibromo-4-[1,2,2,3,3,3-hexafluoro-1-(trifluoromethyl)propyl]phenyl]amino]carbonyl]phenyl]-2-methyl-benzamide, 4-cyano-3-[(4-cyano-2-methyl-benzoyl)amino]-*N*-[2,6-dichloro-4-[1,2,2,3,3,3-hexafluoro-1-(trifluoromethyl)propyl]phenyl]-2-fluoro-benzamide, *N*-[5-[[[2-chloro-6-cyano-4-[1,2,2,3,3,3-hexafluoro-1-(trifluoromethyl)propyl]phenyl]amino]carbonyl]-2-cyano-phenyl]-4-cyano-2-methyl-benzamide, *N*-[5-[[[2-bromo-6-chloro-4-[2,2,2-trifluoro-1-hydroxy-1-(trifluoromethyl)ethyl]phenyl]amino]carbonyl]-2-cyano-phenyl]-4-cyano-2-methyl-benzamide, *N*-[5-[[[2-bromo-6-chloro-4-[1,2,2,3,3,3-hexafluoro-1-(trifluoromethyl)propyl]phenyl]amino]-carbonyl]-2-cyano-phenyl]-4-cyano-2-methyl-benzamide, 4-cyano-*N*-[2-cyano-5-[[[2,6-dichloro-4-[1,2,2,3,3,3-hexafluoro-1-(trifluoromethyl)propyl]phenyl]amino]carbonyl]phenyl]-2-methyl-benzamide, 1-[2-fluoro-4-methyl-5-[(2,2,2-trifluoroethyl)sulfinyl]phenyl]-3-(trifluoromethyl)-1H-1 ,2,4-triazole-5-amine, *N*-[5-[[[2-bromo-6-chloro-4-[1,2,2,2-tetrafluoro-1-(trifluoromethyl)ethyl]phenyl]amino]carbonyl]-2-cyano-phenyl]-4-cyano-2-methyl-benzamide, 4-cyano-*N*-[2-cyano-5-[[[2,6-dichloro-4-[1,2,2,2-tetrafluoro-1-(trifluoromethyl)ethyl]phenyl]amino]-carbonyl]phenyl]-2-methyl-benzamide, actives on basis of *Bacillus firmus* (Votivo, I-1582); flupyrimin; fluazaindolizine; 5-[3-[2,6-dichloro-4-(3,3-dichloroallyloxy)phenoxy]propoxy]-1*H*-pyrazole; *N*-[5-[[2-bromo-6-chloro-4-[1,2,2,3,3,3-hexafluoro-1-(trifluoromethyl)-propyl]-phenyl]carbamoyl]-2-cyano-phenyl]-4-cyano-2-methyl-benzamide; 4-cyano-N-[2-cyano-5-[[2,6-dichloro-4-[1,2,2,3,3,3-hexafluoro-1-(trifluoromethyl)-propyl]phenyl]carbamoyl]phenyl]-2-methyl-benzamide; 4-cyano-*N*-[2-cyano-5-[[2,6-dichloro-4-[1,2,2,2-tetrafluoro-1-(trifluoromethyl)ethyl]phenyl]carbamoyl]-phenyl]-2-methyl-benzamide; *N*-[5-[[2-bromo-6-chloro-4-[1,2,2,2-tetrafluoro-1-(trifluoromethyl)ethyl]phenyl]carba¬moyl]-2-cyano-phenyl]-4-cyano-2-methyl-benzamide; 2-(1,3-dioxan-2-yl)-6-[2-(3-pyridinyl)-5-thiazolyl]-pyridine; 2-[6-[2-(5-fluoro-3-pyridinyl)-5-thiazo¬lyl]-2-pyridinyl]-pyrimidine; 2-[6-[2-(3-pyridinyl)-5-thiazolyl]-2-pyridinyl]-pyrimidine; *N*-methylsul¬fonyl-6-[2-(3-pyridyl)thiazol-5-yl]pyridine-2-carboxamide; *N*-methylsulfonyl-6-[2-(3-pyridyl)thiazol-5-yl]pyridine-2-carboxamide; 1-[(6-chloro-3-pyridinyl)-methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; *N*-(3-chloro-2-methylphenyl)-2-[(4-chlorophenyl)[4-[methyl(methylsulfonyl)amino]phenyl]-methylene]-hydrazinecarboxamide; 1-[(6-chloro-3-pyridinyl)methyl]-1,2,3,5,6,7-hexahydro-5-methoxy-7-methyl-8-nitro-imidazo[1,2-a]pyridine; 2-(3-pyridinyl)-*N*-(2-pyrimidinylmethyl)-2H-indazole-5-carboxamide; tyclopyrazoflor; sarolaner, lotilaner; *N*-[4-chloro-3-[[(phenylmethyl)amino]carbonyl]phenyl]-1-methyl-3-(1,1,2,2,2-pentafluoroethyl)-4-(trifluoromethyl)-1*H*-pyrazole-5-carboxamide; *N*-[4-chloro-3-[[(phenylmethyl)amino]carbonyl]phenyl]-1-methyl-3-(1,1,2,2,2-pentafluoroethyl)-4-(trifluoromethyl)-1*H*-pyrazole-5-carboxamide; 2-(3-ethylsulfonyl-2-pyridyl)-3-methyl-6-(tri-fluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine; isocycloseram; *N*-[4-chloro-3-(cyclopropylcarbamoyl)phenyl]-2-methyl-5-(1,1,2,2,2-pentafluoroethyl)-4-(tri-fluoromethyl)pyrazole-3-carboxamide, *N*-[4-chloro-3-[(1-cyanocyclopropyl)carbamoyl]-phenyl]-2-methyl-5-(1,1,2,2,2-pentafluoroethyl)-4-(trifluoromethyl)pyrazole-3-carboxamide; acynonapyr; benzpyrimoxan; tigolaner; oxazosulfyl; [(2S,3R,4R,5S,6S)-3,5-dimethoxy-6-methyl-4-propoxy-tetrahydropyran-2-yl]-*N*-[4-[1-[4-(trifluoromethoxy)phenyl]-1,2,4-triazol-3-yl]phenyl]carbamate; [(2*S*,3*R*,4*R*,5*S*,6*S*)-3,4,5-trimethoxy-6-methyl-tetrahydropyran-2-yl]-*N*-[4-[1-[4-(trifluoromethoxy)phenyl]-1,2,4-triazol-3-yl]phenyl]carbamate; [(2S,3R,4R,5S,6S)-3,5-dimethoxy-6-methyl-4-propoxy-tetrahydropyran-2-yl]-*N*-[4-[1-[4-(1,1,2,2,2-pentafluoro-ethoxy)phenyl]-1,2,4-triazol-3-yl]phenyl]carbamate; [(2S,3R,4R,5S,6S)-3,4,5-trimethoxy-6-methyl-tetrahydropyran-2-yl]-*N*-[4-[1-[4-(1,1,2,2,2-pentafluoroethoxy)phenyl]-1,2,4-triazol-3-yl]phenyl]carbamate; (2Z)-3-(2-isopropylphenyl)-2-[(E)-[4-[1-[4-(trifluoromethoxy)phenyl]-1,2,4-triazol-3-yl]phenyl]methylenehydrazono]thiazolidin-4-one, (2Z)-3-(2-isopropylphenyl)-2-[(E)-[4-[1-[4-(1,1,2,2,2-pentafluoroethoxy)phenyl]-1,2,4-triazol-3-yl]phenyl]methylene-hydrazono]thiazolidin-4-one, (2*Z*)-3-(2-isopro¬pyl¬phenyl)-2-[(*E*)-[4-[1-[4-(1,1,2,2,2-pentafluoroethoxy)phenyl]-1,2,4-triazol-3-yl]phenyl]methylenehydrazono]thiazolidin-4-one; 2-(6-chloro-3-ethylsulfonyl-imidazo[1,2-a]pyridin-2-yl)-3-methyl-6-(trifluoromethyl)imidazo[4,5-b]pyridine, 2-(6-bromo-3-ethylsulfonyl-imidazo[1,2-a]pyridin-2-yl)-3-methyl-6-(trifluoromethyl)imidazo[4,5-b]pyridine, 2-(3-ethylsulfonyl-6-iodo-imidazo[1,2-a]pyridin-2-yl)-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(3-ethylsulfonyl-7-iodo-imidazo-[1,2-a]pyridin-2-yl)-3-methyl-6-(trifluoromethyl)imidazo[4,5-b]pyridine, 3-ethylsulfonyl-6-iodo-2-[3-methyl-6-(trifluoromethyl)imidazo[4,5-b]pyridin-2-yl]imidazo[1,2-a]pyridine-8-carbonitrile, 2-[3-ethylsulfonyl-8-fluoro-6-(trifluoromethyl)imidazo[1,2-a]pyridin-2-yl]-3-methyl-6-(trifluoromethyl)imidazo[4,5-b]pyridine, 2-[3-ethylsulfonyl-7-(trifluoromethyl)imidazo[1,2-a]pyridin-2-yl]-3-methyl-6-(trifluoromethylsulfinyl)imidazo[4,5-b]pyridine, 2-[3-ethylsulfonyl-7-(trifluoromethyl)imidazo[1,2-a]pyridin-2-yl]-3-methyl-6-(trifluoromethyl)imidazo[4,5-c]pyridine, 2-(6-bromo-3-ethylsulfonyl-imidazo[1,2-a]pyridin-2-yl)-6-(trifluoromethyl)pyrazolo[4,3-c]pyridine; *N*-[[2-fluoro-4-[(2S,3S)-2-hydroxy-3-(3,4,5-trichlorophenyl)-3-(trifluoromethyl)pyrrolidin-1-yl]phenyl]methyl]cyclopropanecarboxamide; 2-[2-fluoro-4-methyl-5-(2,2,2-trifluoroethylsulfinyl)phenyl]imino-3-(2,2,2-trifluoroethyl)thiazolidin-4-one; flupentiofenox, *N*-[3-chloro-1-(3-pyridyl)pyrazol-4-yl]-2-methylsulfonyl-propanamide, cyclobutrifluram; 2-chloro-5-[1-[2-chloro-4-[1,2,2,2-tetrafluoro-1-(trifluoromethyl)ethyl]-6-(trifluoromethyl)phenyl]pyrazol-4-yl]-*N*-cyclopropyl-pyridine-3-carboxamide; 2-chloro-5-[1-[2-chloro-4-[1,2,2,2-tetrafluoro-1-(trifluoromethyl)ethyl]-6-(trifluoromethoxy)phenyl]pyrazol-4-yl]-N-cyclopropyl-pyridine-3-carboxamide; 5-[1-[2-bromo-4-[1,2,2,2-tetrafluoro-1-(trifluoromethyl)ethyl]-6-(trifluoromethoxy)phenyl]pyrazol-4-yl]-2-chloro-*N*-cyclopropyl-pyridine-3-carboxamide; *N*-[4-chloro-3-[(1-cyanocyclopropyl)carbamoyl]phenyl]-2-methyl-4-methylsulfonyl-5-(1,1,2,2,2-pentafluoroethyl)pyrazole-3-carboxamide, cyproflanilide, nicofluprole; 1,4-dimethyl-2-[2-(pyridin-3-yl)-2*H*-indazol-5-yl]-1,2,4-triazolidine-3,5-dione, 2-[2-fluoro-4-methyl-5-(2,2,2-trifluoroethylsulfanyl)phenyl]imino-3-(2,2,2-trifluoroethyl)thiazolidin-4-one, indazapyroxamet, *N*-[4-chloro-2-(3-pyridyl)thiazol-5-yl]-*N*-ethyl-3-methylsulfonyl-propanamide, *N*-cyclopropyl-5-[(5S)-5-(3,5-dichloro-4-fluoro-phenyl)-5-(trifluoromethyl)-4*H*-isoxazol-3-yl]isoquinoline-8-carboxamide, 5-[(5S)-5-(3,5-dichloro-4-fluoro-phenyl)-5-(trifluoromethyl)-4*H*-isoxazol-3-yl]-*N*-(pyrimidin-2-yl-methyl)isoquinoline-8-carboxamide, *N*-[1-(2,6-difluorophenyl)pyrazol-3-yl]-2-(trifluoromethyl)-benzamide;
   O.29: broflanilide, fluxametamide.

The active substances referred to as component 2, their preparation and their activity e.g. against harmful fungi is known (cf.: https://pesticidecompendium.bcpc.org/); these substances are commercially available. The compounds described by IUPAC nomenclature, their preparation and their pesticidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441, WO 16/156241, WO 16/162265). Some compounds are identified by their CAS Registry Number which is separated by hyphens into three parts, the first consisting from two up to seven digits, the second consisting of two digits, and the third consisting of a single digit.

According to the invention, the solid material (dry matter) of the biopesticides (with the exception of oils such as Neem oil) are considered as active components (e.g. to be obtained after drying or evaporation of the extraction or suspension medium in case of liquid formulations of the microbial pesticides). The weight ratios and percentages used for a biological extract such as Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1 × 10¹⁰ CFU equals one gram of total weight of the respective active component. Colony forming unit is measure of viable microbial cells. In addition, CFU may also be understood as the number of (juvenile) individual nematodes in case of nematode biopesticides, such as *Steinernema feltiae.*

In the binary mixtures the weight ratio of the component 1) and the component 2) generally depends from the properties of the components used, usually it is in the range of from 1:10,000 to 10,000:1, often from 1:100 to 100:1, regularly from 1:50 to 50:1, preferably from 1:20 to 20:1, more preferably from 1:10 to 10:1, even more preferably from 1:4 to 4:1 and in particular from 1:2 to 2:1. According to further embodiments, the weight ratio of the component 1) and the component 2) usually is in the range of from 1000:1 to 1:1, often from 100: 1 to 1:1, regularly from 50:1 to 1:1, preferably from 20:1 to 1:1, more preferably from 10:1 to 1:1, even more preferably from 4:1 to 1:1 and in particular from 2:1 to 1:1. According to further embodiments, the weight ratio of the component 1) and the component 2) usually is in the range of from 20,000:1 to 1:10, often from 10,000:1 to 1:1, regularly from 5,000:1 to 5:1, preferably from 5,000:1 to 10:1, more preferably from 2,000:1 to 30:1, even more preferably from 2,000:1 to 100:1 and in particular from 1,000:1 to 100:1. According to further embodiments, the weight ratio of the component 1) and the component 2) usually is in the range of from 1:1 to 1:1000, often from 1:1 to 1:100, regularly from 1:1 to 1:50, preferably from 1:1 to 1:20, more preferably from 1:1 to 1:10, even more preferably from 1:1 to 1:4 and in particular from 1:1 to 1:2. According to further embodiments, the weight ratio of the component 1) and the component 2) usually is in the range of from 10:1 to 1:20,000, often from 1:1 to 1:10,000, regularly from 1:5 to 1:5,000, preferably from 1:10 to 1:5,000, more preferably from 1:30 to 1:2,000, even more preferably from 1:100 to 1:2,000 to and in particular from 1:100 to 1:1,000.

In the ternary mixtures, i.e. compositions comprising the component 1) and component 2) and a compound III (component 3), the weight ratio of component 1) and component 2) depends from the properties of the active substances used, usually it is in the range of from 1:100 to 100:1, regularly from 1:50 to 50:1, preferably from 1:20 to 20:1, more preferably from 1:10 to 10:1 and in particular from 1:4 to 4:1, and the weight ratio of component 1) and component 3) usually it is in the range of from 1:100 to 100:1, regularly from 1:50 to 50:1, preferably from 1:20 to 20:1, more preferably from 1:10 to 10:1 and in particular from 1:4 to 4:1. Any further active components are, if desired, added in a ratio of from 20:1 to 1:20 to the component 1). These ratios are also suitable for mixtures applied by seed treatment.

When mixtures comprising microbial pesticides are employed in crop protection, the application rates range from 1 × 10⁶ to 5 × 10¹⁶ (or more) CFU/ha, preferably from 1 × 10⁸ to 1 × 10¹³ CFU/ha, and even more preferably from 1 × 10⁹ to 5 × 10¹⁵ CFU/ha and in particular from 1 × 10¹² to 5 × 10¹⁴ CFU/ha. In the case of nematodes as microbial pesticides (e.g. *Steinernema feltiae),* the application rates regularly range from 1 × 10⁵ to 1 × 10¹² (or more), preferably from 1 × 10⁸ to 1 × 10¹¹, more preferably from 5 × 10⁸ to 1 × 10¹⁰ individuals (e.g. in the form of eggs, juvenile or any other live stages, preferably in an infetive juvenile stage) per ha.

When mixtures comprising microbial pesticides are employed in seed treatment, the application rates generally range from 1 × 10⁶ to 1 × 10¹² (or more) CFU/seed, preferably from 1 × 10⁶ to 1 × 10⁹ CFU/seed. Furthermore, the application rates with respect to seed treatment generally range from 1 × 10⁷ to 1 × 10¹⁴ (or more) CFU per 100 kg of seed, preferably from 1 × 10⁹ to 1 × 10¹² CFU per 100 kg of seed.

Preference is given to mixtures comprising as component 2) at least one active substance selected from inhibitors of complex III at Qₒ site in group A), more preferably selected from compounds (A.1.1), (A.1.4), (A.1.8), (A.1.9), (A.1.10), (A.1.12), (A.1.13), (A.1.14), (A.1.17), (A.1.21), (A.1.25), (A.1.34) and (A.1.35); particularly selected from (A.1.1), (A.1.4), (A.1.8), (A.1.9), (A.1.13), (A.1.14), (A.1.17), (A.1.25), (A.1.34) and (A.1.35).

Preference is also given to mixtures comprising as component 2) at least one active substance selected from inhibitors of complex III at Qᵢ site in group A), more preferably selected from compounds (A.2.1), (A.2.3), (A.2.4) and (A.2.6); particularly selected from (A.2.3), (A.2.4) and (A.2.6).

Preference is also given to mixtures comprising as component 2) at least one active substance selected from inhibitors of complex II in group A), more preferably selected from compounds (A.3.2), (A.3.3), (A.3.4), (A.3.7), (A.3.9), (A.3.11), (A.3.12), (A.3.15), (A.3.16), (A.3.17), (A.3.18), (A.3.19), (A.3.20), (A.3.21), (A.3.22), (A.3.23), (A.3.24), (A.3.28), (A.3.31), (A.3.32), (A.3.33), (A.3.34), (A.3.35), (A.3.36), (A.3.37), (A.3.38) and (A.3.39); particularly selected from (A.3.2), (A.3.3), (A.3.4), (A.3.7), (A.3.9), (A.3.12), (A.3.15), (A.3.17), (A.3.19), (A.3.22), (A.3.23), (A.3.24), (A.3.31), (A.3.32), (A.3.33), (A.3.34), (A.3.35), (A.3.36), (A.3.37), (A.3.38) and (A.3.39).

Preference is also given to mixtures comprising as component 2) at least one active substance selected from other respiration inhibitors in group A), more preferably selected from compounds (A.4.5) and (A.4.11); in particular (A.4.11).

Preference is also given to mixtures comprising as component 2) at least one active substance selected from C14 demethylase inhibitors in group B), more preferably selected from compounds (B.1.4), (B.1.5), (B.1.8), (B.1.10), (B.1.11), (B.1.12), (B.1.13), (B.1.17), (B.1.18), (B.1.21), (B.1.22), (B.1.23), (B.1.25), (B.1.26), (B.1.29), (B.1.33), (B.1.34), (B.1.37), (B.1.38), (B.1.43), (B.1.46), (B.1.53), (B.1.54) and (B.1.55); particularly selected from (B.1.5), (B.1.8), (B.1.10), (B.1.17), (B.1.22), (B.1.23), (B.1.25), (B.1.33), (B.1.34), (B.1.37), (B.1.38), (B.1.43) and (B.1.46).

Preference is also given to mixtures comprising as component 2) at least one active substance selected from Delta14-reductase inhibitors in group B), more preferably selected from compounds (B.2.4), (B.2.5), (B.2.6) and (B.2.8); in particular (B.2.4).

Preference is also given to mixtures comprising as component 2) at least one active substance selected from phenylamides and acyl amino acid fungicides in group C), more preferably selected from compounds (C.1.1), (C.1.2), (C.1.4) and (C.1.5); particularly selected from (C.1.1) and (C.1.4).

Preference is also given to mixtures comprising as component 2) at least one active substance selected from other nucleic acid synthesis inhibitors in group C), more preferably selected from compounds (C.2.6), (C.2.7) and (C.2.8).

Preference is also given to mixtures comprising as component 2) at least one active substance selected from group D), more preferably selected from compounds (D.1.1), (D.1.2), (D.1.5), (D.2.4) and (D.2.6); particularly selected from (D.1.2), (D.1.5) and (D.2.6).

Preference is also given to mixtures comprising as component 2) at least one active substance selected from group E), more preferably selected from compounds (E.1.1), (E.1.3), (E.2.2) and (E.2.3); in particular (E.1.3).

Preference is also given to mixtures comprising as component 2) at least one active substance selected from group F), more preferably selected from compounds (F.1.2), (F.1.4) and (F.1.5).

Preference is also given to mixtures comprising as component 2) at least one active substance selected from group G), more preferably selected from compounds (G.3.1), (G.3.3), (G.3.6), (G.5.1), (G.5.3), (G.5.4), (G.5.5), G.5.6), G.5.7), (G.5.8), (G.5.9), (G.5.10) and (G.5.11); particularly selected from (G.3.1), (G.5.1) and (G.5.3).

Preference is also given to mixtures comprising as component 2) at least one active substance selected from group H), more preferably selected from compounds (H.2.2), (H.2.3), (H.2.5), (H.2.7), (H.2.8), (H.3.2), (H.3.4), (H.3.5), (H.4.9) and (H.4.10); particularly selected from (H.2.2), (H.2.5), (H.3.2), (H.4.9) and (H.4.10).

Preference is also given to mixtures comprising as component 2) at least one active substance selected from group I), more preferably selected from compounds (1.2.2) and (1.2.5).

Preference is also given to mixtures comprising as component 2) at least one active substance selected from group J), more preferably selected from compounds (J.1.2), (J.1.5), (J.1.8), (J.1.11) and (J.1.12); in particular (J.1.5).

Preference is also given to mixtures comprising as component 2) at least one active substance selected from group K), more preferably selected from compounds (K.1.41), (K.1.42), (K.1.44), (K.1.47), (K.1.57), (K.1.58) and (K.1.59); particularly selected from (K.1.41), (K.1.44), (K.1.47), (K.1.57), (K.1.58) and (K.1.59).

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.

The microbial pesticides, in particular those from groups L1), L3) and L5), embrace not only the isolated, pure cultures of the respective microorganism as defined herein, but also its cellfree extract, its suspension in a whole broth culture and a metabolite-containing culture medium or a purified metabolite obtained from a whole broth culture of the microorganism.

Many of these biopesticides have been deposited under deposition numbers mentioned herein (the prefices such as ATCC or DSM refer to the acronym of the respective culture collection, for details see e.g. here: http://www.wfcc.info/ccinfo/collection/by acronym/), are referred to in literature, registered and/or are commercially available: mixtures of *Aureobasidium pullulans* DSM 14940 and DSM 14941 isolated in 1989 in Konstanz, Germany (e.g. blastospores in BlossomProtect^{®} from bio-ferm GmbH, Austria), *Azospirillum brasilense* Sp245 originally isolated in wheat reagion of South Brazil (Passo Fundo) at least prior to 1980 (BR 11005; e.g. GELFIX^{®} Gramíneas from BASF Agricultural Specialties Ltd., Brazil), *A. brasilense* strains Ab-V5 and Ab-V6 (e.g. in AzoMax from Novozymes BioAg Produtos papra Agricultura Ltda., Quattro Barras, Brazil or Simbiose-Maíz^{®} from Simbiose-Agro, Brazil; Plant Soil 331, 413-425, 2010), *Bacillus amyloliquefaciens* strain AP-188 (NRRL B-50615 and B-50331; US 8,445,255); *B. amyloliquefaciens* ssp. *plantarum* strains formerly also sometimes referred to as *B. subtilis,* recently together with *B*. *methylotrophicus,* and *B*. *velezensis* classified as *B*. *velezensis* (Int. J. Syst. Evol. Microbiol. 66, 1212-1217, 2016): *B*. a. ssp. *plantarum* or *B*. *velezensis* D747 isolated from air in Kikugawa-shi, Japan (US 20130236522 A1; FERM BP-8234; e.g. Double Nickel^{™} 55 WDG from Certis LLC, USA), *B*. a. ssp. *plantarum* or *B*. *velezensis* FZB24 isolated from soil in Brandenburg, Germany (also called SB3615; DSM 96-2; J. Plant Dis. Prot. 105, 181-197, 1998; e.g. Taegro^{®} from Novozyme Biologicals, Inc., USA), *B*. a. ssp. *plantarum* or *B*. *velezensis* FZB42 isolated from soil in Brandenburg, Germany (DSM 23117; J. Plant Dis. Prot. 105, 181-197, 1998; e.g. RhizoVital^{®} 42 from AbiTEP GmbH, Germany), *B*. a. ssp. *plantarum* or *B*. *velezensis* MBI600 isolated from faba bean in Sutton Bonington, Nottinghamshire, U.K. at least before 1988 (also called 1430; NRRL B-50595; US 2012/0149571 A1; e.g. Integral^{®} from BASF Corp., USA), *B*. a. ssp. *plantarum* or *B*. *velezensis* QST-713 isolated from peach orchard in 1995 in California, U.S.A. (NRRL B-21661; e.g. Serenade^{®} MAX from Bayer Crop Science LP, USA), *B*. a. ssp. *plantarum* or *B*. *velezensis* TJ1000 isolated in 1992 in South Dakoda, U.S.A. (also called 1BE; ATCC BAA-390; CA 2471555 A1; e.g. QuickRoots^{™} from TJ Technologies, Watertown, SD, USA); *B. firmus* CNCM I-1582, a variant of parental strain EIP-N1 (CNCM I-1556) isolated from soil of central plain area of Israel (WO 2009/126473, US 6,406,690; e.g. Votivo^{®} from Bayer CropScience LP, USA), *B*. *pumilus* GHA 180 isolated from apple tree rhizosphere in Mexico (IDAC 260707-01; e.g. PRO-MIX^{®} BX from Premier Horticulture, Quebec, Canada), *B. pumilus* INR-7 otherwise referred to as BU-F22 and BU-F33 isolated at least before 1993 from cucumber infested by *Erwinia tracheiphila* (NRRL B-50185, NRRL B-50153; US 8,445,255), *B. pumilus* KFP9F isolated from the rhizosphere of grasses in South Africa at least before 2008 (NRRL B-50754;

WO 2014/029697; e.g. BAC-UP or FUSION-P from BASF Agricultural Specialities (Pty) Ltd., South Africa), *B. pumilus* QST 2808 was isolated from soil collected in Pohnpei, Federated States of Micronesia, in 1998 (NRRL B-30087; e.g. Sonata^{®} or Ballad^{®} Plus from Bayer Crop Science LP, USA), *B. simplex* ABU 288 (NRRL B-50304; US 8,445,255), *B. subtilis* FB17 also called UD 1022 or UD10-22 isolated from red beet roots in North America (ATCC PTA-11857; System. Appl. Microbiol. 27, 372-379, 2004; US 2010/0260735; WO 2011/109395); *B. thuringiensis* ssp. *aizawai* ABTS-1857 isolated from soil taken from a lawn in Ephraim, Wisconsin, U.S.A., in 1987 (also called ABG-6346; ATCC SD-1372; e.g. XenTari^{®} from BioFa AG, Münsingen, Germany), *B*. *t.* ssp. *kurstaki* ABTS-351 identical to HD-1 isolated in 1967 from diseased Pink Bollworm black larvae in Brownsville, Texas, U.S.A. (ATCC SD-1275; e.g. Dipel^{®} DF from Valent BioSciences, IL, USA), *B. t.* ssp. *kurstaki* SB4 isolated from *E. saccharina* larval cadavers (NRRL B-50753; e.g. Beta Pro^{®} from BASF Agricultural Specialities (Pty) Ltd., South Africa), *B*. *t.* ssp. *tenebrionis* NB-176-1, a mutant of strain NB-125, a wild type strain isolated in 1982 from a dead pupa of the beetle Tenebrio molitor (DSM 5480; EP 585 215 B1; e.g. Novodor^{®} from Valent BioSciences, Switzerland), *Beauveria bassiana* GHA (ATCC 74250; e.g. BotaniGard^{®} 22WGP from Laverlam Int. Corp., USA), *B*. *bassiana* JW-1 (ATCC 74040; e.g. Naturalis^{®} from CBC (Europe) S.r.l., Italy), *B*. *bassiana* PPRI 5339 isolated from the larva of the tortoise beetle *Conchyloctenia punctata* (NRRL 50757; e.g. BroadBand^{®} from BASF Agricultural Specialities (Pty) Ltd., South Africa), *Bradyrhizobium elkanii* strains SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e.g. GELFIX 5 from BASF Agricultural Specialties Ltd., Brazil), *B. japonicum* 532c isolated from Wisconsin field in U.S.A. (Nitragin 61A152; Can. J. Plant. Sci. 70, 661-666, 1990; e.g. in Rhizoflo^{®}, Histick^{®}, Hicoat^{®} Super from BASF Agricultural Specialties Ltd., Canada), *B*. *japonicum* E-109 variant of strain USDA 138 (INTA E109, SEMIA 5085; Eur. J. Soil Biol. 45, 28-35, 2009; Biol. Fertil. Soils 47, 81-89, 2011); *B. japonicum* strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e.g. GELFIX 5 or ADHERE 60 from BASF Agricultural Specialties Ltd., Brazil), *B*. *japonicum* SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e.g. GELFIX 5 or ADHERE 60 from BASF Agricultural Specialties Ltd., Brazil); *Burkholderia sp.* A396 isolated from soil in Nikko, Japan, in 2008 (NRRL B-50319; WO 2013/032693; Marrone Bio Innovations, Inc., USA), *Coniothyrium minitans* CON/M/91-08 isolated from oilseed rape (WO 1996/021358; DSM 9660; e.g. Contans^{®} WG, Intercept^{®} WG from Bayer CropScience AG, Germany), harpin (alpha-beta) protein (Science 257, 85-88, 1992; e.g. Messenger^{™} or HARP-N-Tek from Plant Health Care pic, U.K.), *Helicoverpa armigera* nucleopolyhedrovirus (HearNPV) (J. Invertebrate Pathol. 107, 112-126, 2011; e.g. Helicovex^{®} from Adermatt Biocontrol, Switzerland; Diplomata^{®} from Koppert, Brazil; Vivus^{®} Max from AgBiTech Pty Ltd., Queensland, Australia), *Helicoverpa zea* single capsid nucleopolyhedrovirus (HzSNPV) (e.g. Gemstar^{®} from Certis LLC, USA), *Helicoverpa zea* nucleopolyhedrovirus ABA-NPV-U (e.g. Heligen^{®} from AgBiTech Pty Ltd., Queensland, Australia), *Heterorhabditis bacteriophora* (e.g. Nemasys^{®} G from BASF Agricultural Specialities Limited, UK), *Isaria fumosorosea* Apopka-97 isolated from mealy bug on gynura in Apopka, Florida, U.S.A. (ATCC 20874; Biocontrol Science Technol. 22(7), 747-761, 2012; e.g. PFR-97^{™} or PreFeRal^{®} from Certis LLC, USA), *Metarhizium anisopliae* var. *anisopliae* F52 also called 275 or V275 isolated from codling moth in Austria (DSM 3884, ATCC 90448; e.g. Met52^{®} Novozymes Biologicals BioAg Group, Canada), *Metschnikowia fructicola* 277 isolated from grapes in the central part of Israel (US 6,994,849; NRRL Y-30752; e.g. formerly Shemer^{®} from Agrogreen, Israel), *Paecilomyces ilacinus* 251 isolated from infected nematode eggs in the Philippines (AGAL 89/030550; WO1991/02051; Crop Protection 27, 352-361, 2008; e.g. BioAct^{®}from Bayer CropScience AG, Germany and MeloCon^{®} from Certis, USA), *Paenibacillus alvei* NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), *Paenibacillus* strains isolated from soil samples from a variety of European locations including Germany: *P. epiphyticus* Lu17015 (WO 2016/020371; DSM 26971), *P. polymyxa* ssp. *plantarum* Lu16774 (WO 2016/020371; DSM 26969), *P. p.* ssp. *plantarum* strain Lu17007 (WO 2016/020371; DSM 26970); *Pasteuria nishizawae* Pn1 isolated from a soybean field in the mid-2000s in Illinois, U.S.A. (ATCC SD-5833; Federal Register 76(22), 5808, February 2, 2011; e.g. Clariva^{™} PN from Syngenta Crop Protection, LLC, USA), *Penicillium bilaiae* (also called *P*. *bilaii*) strains ATCC 18309 (= ATCC 74319), ATCC 20851 and/or ATCC 22348 (= ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e.g. Jump Start^{®}, Provide^{®} from Novozymes Biologicals BioAg Group, Canada), *Reynoutria sachalinensis* extract (EP 0307510 B1; e.g. Regalia^{®} SC from Marrone Biolnnovations, Davis, CA, USA or Milsana^{®} from BioFa AG, Germany), *Steinernema carpocapsae* (e.g. Millenium^{®} from BASF Agricultural Specialities Limited, UK), S. *feltiae* (e.g. Nemashield^{®} from BioWorks, Inc., USA; Nemasys^{®}from BASF Agricultural Specialities Limited, UK), *Streptomyces microflavus* NRRL B-50550 (WO 2014/124369; Bayer CropScience, Germany), *Trichoderma asperelloides* JM41R isolated in South Africa (NRRL 50759; also referred to as *T. fertile;* e.g. Trichoplus^{®} from BASF Agricultural Specialities (Pty) Ltd., South Africa), *T. harzianum* T-22 also called KRL-AG2 (ATCC 20847; BioControl 57, 687-696, 2012; e.g. Plantshield^{®} from BioWorks Inc., USA or SabrEx^{™} from Advanced Biological Marketing Inc., Van Wert, OH, USA).

According to another embodiment of the mixtures, the at least one pesticide II is selected from the groups L1) to L5):
L1) Microbial pesticides with fungicidal, bactericidal, viricidal and/or plant defense activator activity: *Aureobasidium pullulans* DSM 14940 and DSM 14941 (L1.1), *Bacillus amyloliquefaciens* AP-188 (L.1.2), *B. amyloliquefaciens* ssp. *plantarum* D747 (L.1.3), *B*. *amyloliquefaciens* ssp. *plantarum* FZB24 (L.1.4), *B. amyloliquefaciens* ssp. *plantarum* FZB42 (L.1.5), *B. amyloliquefaciens* ssp. *plantarum* MBI600 (L.1.6), *B*. *amyloliquefaciens* ssp. *plantarum* QST-713 (L.1.7), *B. amyloliquefaciens* ssp. *plantarum* TJ1000 (L.1.8), *B. pumilus* GB34 (L.1.9), *B. pumilus* GHA 180 (L.1.10), *B. pumilus* INR-7 (L.1.11), *B. pumilus* KFP9F (L.1.12), *B. pumilus* QST 2808 (L.1.13), *B. simplex* ABU 288 (L.1.14), *B*. *subtilis* FB17 (L.1.15), *Coniothyrium minitans* CON/M/91-08 (L.1.16), *Metschnikowia fructicola* NRRL Y-30752 (L.1.17), *Paenibacillus alvei* NAS6G6 (L.1.18), *P. epiphyticus* Lu17015 (L.1.25), *P*. *polymyxa* ssp. *plantarum* Lu16774 (L.1.26), *P. p.* ssp. *plantarum* strain Lu17007 (L.1.27), *Penicillium bilaiae* ATCC 22348 (L.1.19), *P. bilaiae* ATCC 20851 (L.1.20), *Penicillium bilaiae* ATCC 18309 (L.1.21), *Streptomyces microflavus* NRRL B-50550 (L.1.22), *Trichoderma asperelloides* JM41R (L.1.23), *T. harzianum* T-22 (L.1.24);
L2) Biochemical pesticides with fungicidal, bactericidal, viricidal and/or plant defense activator activity: harpin protein (L.2.1), *Reynoutria sachalinensis* extract (L.2.2);
L3) Microbial pesticides with insecticidal, acaricidal, molluscidal and/or nematicidal activity: *Bacillus firmus* 1-1582 (L.3.1); *B. thuringiensis* ssp. *aizawai* ABTS-1857 (L.3.2), *B. t.* ssp. *kurstaki* ABTS-351 (L.3.3), *B. t.* ssp. *kurstaki* SB4 (L.3.4), *B. t.* ssp. *tenebrionis* NB-176-1 (L.3.5), *Beauveria bassiana* GHA (L.3.6), *B*. *bassiana* JW-1 (L.3.7), *B*. *bassiana* PPRI 5339 (L.3.8), *Burkholderia* sp. A396 (L.3.9), *Helicoverpa armigera* nucleopolyhedrovirus (HearNPV) (L.3.10), *Helicoverpa zea* nucleopolyhedrovirus (HzNPV) ABA-NPV-U (L.3.11), *Helicoverpa zea* single capsid nucleopolyhedrovirus (HzSNPV) (L.3.12), *Heterohabditis bacteriophora* (L.3.13), *Isaria fumosorosea* Apopka-97 (L.3.14), *Metarhizium anisopliae* var. *anisopliae* F52 (L.3.15), *Paecilomyceslilacinus* 251 (L.3.16), *Pasteuria nishizawae* Pn1 (L.3.17), *Steinernema carpocapsae* (L.3.18), *S*. *feltiae* (L.3.19);
L4) Biochemical pesticides with insecticidal, acaricidal, molluscidal, pheromone and/or nematicidal activity: cis-jasmone (L.4.1), methyl jasmonate (L.4.2), Quillay extract (L.4.3);
L5) Microbial pesticides with plant stress reducing, plant growth regulator, plant growth promoting and/or yield enhancing activity: *Azospirillum brasilense* Ab-V5 and Ab-V6 (L.5.1), *A. brasilense* Sp245 (L.5.2), *Bradyrhizobium elkanii* SEMIA 587 (L.5.3), *B. elkanii* SEMIA 5019 (L.5.4), *B. japonicum* 532c (L.5.5), *B. japonicum* E-109 (L.5.6), *B. japonicum* SEMIA 5079 (L.5.7), *B. japonicum* SEMIA 5080 (L.5.8).

The present invention furthermore relates to agrochemical compositions comprising a mixture of at least one compound I (component 1) and at least one biopesticide selected from the group L) (component 2), in particular at least one biopesticide selected from the groups L1) and L2), as described above, and if desired at least one suitable auxiliary.

The present invention furthermore relates to agrochemical compositions comprising a mixture of of at least one compound I (component 1) and at least one biopesticide selected from the group L) (component 2), in particular at least one biopesticide selected from the groups L3) and L4), as described above, and if desired at least one suitable auxiliary.

Preference is also given to mixtures comprising as pesticide II (component 2) a biopesticide selected from the groups L1), L3) and L5), preferably selected from strains denoted above as (L.1.2), (L.1.3), (L.1.4), (L.1.5), (L.1.6), (L.1.7), (L.1.8), (L.1.10), (L.1.11), (L.1.12), (L.1.13), (L.1.14), (L.1.15), (L.1.17), (L.1.18), (L.1.19), (L.1.20), (L.1.21), (L.1.25), (L.1.26), (L.1.27), (L.3.1); (L.3.9), (L.3.16), (L.3.17), (L.5.1), (L.5.2), (L.5.3), (L.5.4), (L.5.5), (L.5.6), (L.5.7), (L.5.8); (L.4.2), and (L.4.1); even more preferably selected from (L.1.2), (L.1.6), (L.1.7), (L.1.8), (L.1.11), (L.1.12), (L.1.13), (L.1.14), (L.1.15), (L.1.18), (L.1.19), (L.1.20), (L.1.21), (L.3.1); (L.3.9), (L.3.16), (L.3.17), (L.5.1), (L.5.2), (L.5.5), (L.5.6); (L.4.2), and (L.4.1). These mixtures are particularly suitable for treatment of propagation materials, i. e. seed treatment purposes and likewise for soil treatment. These seed treatment mixtures are particularly suitable for crops such as cereals, corn and leguminous plants such as soybean.

Preference is also given to mixtures comprising as pesticide II (component 2) a biopesticide selected from the groups L1), L3) and L5), preferably selected from strains denoted above as (L1.1), (L.1.2), (L.1.3), (L.1.6), (L.1.7), (L.1.9), (L.1.11), (L.1.12), (L.1.13), (L.1.14), (L.1.15), (L.1.17), (L.1.18), (L.1.22), (L.1.23), (L.1.24), (L.1.25), (L.1.26), (L.1.27), (L.2.2); (L.3.2), (L.3.3), (L.3.4), (L.3.5), (L.3.6), (L.3.7), (L.3.8), (L.3.10), (L.3.11), (L.3.12), (L.3.13), (L.3.14), (L.3.15), (L.3.18), (L.3.19); (L.4.2), even more preferably selected from (L.1.2), (L.1.7), (L.1.11), (L.1.13), (L.1.14), (L.1.15), (L.1.18), (L.1.23), (L.3.3), (L.3.4), (L.3.6), (L.3.7), (L.3.8), (L.3.10), (L.3.11), (L.3.12), (L.3.15), and (L.4.2). These mixtures are particularly suitable for foliar treatment of cultivated plants, preferably of vegetables, fruits, vines, cereals, corn, and leguminous crops such as soybeans.

The compositions comprising mixtures of active ingredients can be prepared by usual means, e.g. by the means given for the compositions of compounds I.

When living microorganisms, such as pesticides II from groups L1), L3) and L5), form part of the compositions, such compositions can be prepared by usual means (e.g. H.D. Burges: Formulation of Microbial Biopesticides, Springer, 1998; WO 2008/002371, US 6,955,912, US 5,422,107).

### Examples:

### Synthetic process

Example 1: 1-[2-[[(*E*)-[3-(4-Chlorophenyl)-1-methyl-prop-2-ynylidene]amino]oxymethyl]phenyl]-4-methyl-tetrazol-5-one (examples numbered according to Table S below)

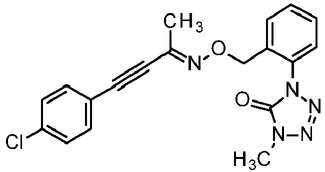

Step 1-1: 4-(4-Chlorophenyl)but-3-yn-2-ol

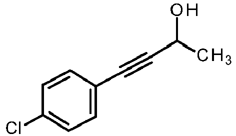

To a solution of 1-chloro-4-iodo-benzene (20 g, 84.03 mmol) in DMSO (200 mL) was added but-3-yn-2-ol (11.8 g, 168.1 mmol), Cul (321 mg, 1.68 mmol), Pd(PPh₃)₂Cl₂ (1.2 g, 1.68 mmol), Et₃N (25 g, 252.10 mmol) at 20 °C. The reaction was stirred at 45 °C for 16 h. TLC (PE: EtOAc = 10: 1) showed the reaction was completed. The reaction was poured into water (500 mL) and the aqueous phase was extracted with EtOAc (100 mL × 3). The combined organic phase was washed with brine (100 mL), dried over Na₂SO₄, concentrated and purified by column chromatography on silica gel (PE~PE: EtOAc=~1:4) to give 4-(4-chlorophenyl)but-3-yn-2-ol (14 g, yield: 93 %) as a yellow solid. ¹H NMR: (400 MHz, CHCl₃-d) δ = 7.31 - 7.18 (m, 4H), 4.68 (q, J = 6.6 Hz, 1H), 1.84 (br s, 1H), 1.48 (d, J = 6.6 Hz, 3H).

Step 1-2: 4-(4-Chlorophenyl)but-3-yn-2-one

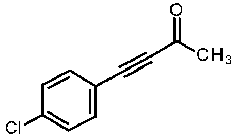

To a solution of 4-(4-chlorophenyl)but-3-yn-2-ol (14 g, 77.35 mmol) in DCM (100 mL) was added MnO₂ (67 g, 773.5 mmol) at 10 °C. The reaction was stirred at 25 °C for 16 h. TLC (PE: EtOAc = 5: 1) showed the reaction was completed. The reaction was filtered and the filtrate was concentrated. The crude product was purified by column chromatography on silica gel (PE~PE: EtOAc = 3: 2) to give 4-(4-chlorophenyl)but-3-yn-2-one (12.5 g, yield: 90 %) as a brown solid. ¹H NMR: (400 MHz, CHCl₃-d) δ = 7.51 (br d, J = 8.1 Hz, 2H), 7.37 (d, J = 8.1 Hz, 2H), 2.46 (s, 3H).

Step 1-3: 4-(4-Chlorophenyl)but-3-yn-2-one oxime

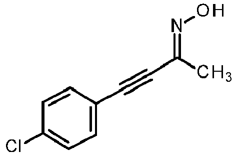

To a solution of 4-(4-chlorophenyl)but-3-yn-2-one (8.4 g, 46.93 mmol) in MeOH (80 mL) was added NaOAc (7.7 g, 93.85 mmol) and NH₂OH · HCl (6.6 g, 93.85 mmol) at 15 °C. The reaction was stirred at 15 °C for 3 h. TLC (PE: EtOAc = 7: 1) showed the reaction was completed. The reaction was poured into water (100 mL), the aqueous phase was extracted with EtOAc (50 mL × 3). The combined organic phase was washed with brine (50 mL), dried over Na₂SO₄, concentrated and purified by column chromatography on silica gel (PE~PE: EtOAc = 9:1) to give 4-(4-chlorophenyl)but-3-yn-2-one oxime (3.2 g, yield: 35 %) as a white solid. ¹H NMR: (400 MHz, CHCl₃-d) δ = 7.51 - 7.42 (m, 2H), 7.36 - 7.29 (m, 2H), 2.16 (d, J = 6.4 Hz, 3H).

Step 2-1: 1-(Methoxymethyl)-2-nitro-benzene 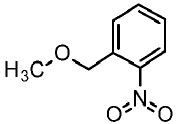 To a solution of 1-bromomethyl-2-nitro-benzene (20 g, 92.59 mmol) in MeOH (100 mL) was added MeONa solution (-30% in MeOH) (20 g, 111.11 mmol) dropwised at 15 °C. The reaction was stirred at 35 °C for 5 h. TLC (PE: EtOAc = 10:1) showed the reaction was completed. The reaction was poured into water (100 mL), the aqueous phase was extracted with EtOAc (50 mL × 3). The combined organic phase was washed with brine (50 mL), dried over Na₂SO₄, concentrated, and purified by column chromatography on silica gel (PE~PE: EtOAc = 4: 6) to give 1-(methoxymethyl)-2-nitro-benzene (15.5 g, yield: 100 %) as a yellow solid. ¹H NMR: (400 MHz, CHCl₃-d) δ = 8.08 (d, J = 8.1 Hz, 1H), 7.79 (d, J = 7.8 Hz, 1H), 7.66 (t, J = 7.6 Hz, 1H), 7.49 - 7.40 (m, 1H), 4.85 (s, 2H), 3.50 (s, 3H).

Step 2-2: 2-(Methoxymethyl)aniline

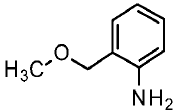

To a mixture of Pd/C (2 g) in MeOH (80 mL) was added 1-(methoxymethyl)-2-nitro-benzene (15.5 g, 92.81 mmol) carefully, the mixture was stirred for 16 h at 50 °C under H₂ (15 PSi). TLC (PE: EtOAc = 10:1) showed the reaction was completed. The reaction mixture was filtered and the filtrate was concentrated and purified by column chromatography on silica gel (PE~PE: EtOAc = 1:1) to give 2-(methoxymethyl)aniline (10.5 g, yield: 83 %) as a yellow oil. ¹H NMR: (400 MHz, CHCl₃-d) δ = 7.14 (dt, J = 1.3, 7.7 Hz, 1H), 7.10 - 7.01 (m, 1H), 6.80 - 6.65 (m, 2H), 4.49 (s, 2H), 3.35 (s, 3H).

Step 2-3: *N*-[2-(Methoxymethyl)phenyl]formamide

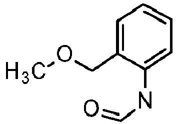

The solution of triphosgene (3.9 g, 13.14 mmol) in DCM (30 mL) was cooled to 0 °C was added a solution of 2-(methoxymethyl)aniline (4 g, 29.20 mmol) in DCM (10 mL) dropwised and stirred at 0 °C for 1h under N₂. Then TEA (7.4 g, 72.99 mmol) was added into the resulting solution at 0 °C and stirred for 3h. TLC (PE: EtOAc = 5:1) showed the reaction was completed. The reaction was filtered and the filtrate was concentrated. The crude product was washed with PE (20 mL), filtered and the filtrate was concentrated to give *N*-[2-(methoxymethyl)phenyl]formamide (6.4 g, crude) as yellow oil and used directly without purification.

Step 2-4: 4-[2-(Methoxymethyl)phenyl]-1*H*-tetrazol-5-one

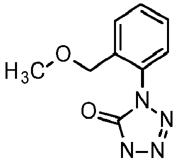

A mixture of *N*-[2-(methoxymethyl)phenyl]formamide (6.4 g, 39.26 mmol) in TMSN3 (13.5 g, 117.8 mmol) was stirred at 100 °C for 16 h under N₂. LCMS showed the reaction was completed. The reaction was poured into water (50 mL), the aqueous phase was extracted with EtOAc (50 mL x 3). The combined organic phase was washed with brine (50 mL), dried over Na₂SO₄, concentrated and triturated by PE: MTBE = 20: 1 to give 4-[2-(methoxymethyl)phenyl]- *H*-tetrazol-5-one (4.7 g, yield: 78 %, two steps) as a white solid. 1H NMR: R (400 MHz, CHC₃-d) δ = 13.22 (br s, 1H), 7.66 - 7.61 (m, 1H), 7.55 (dt, J = 1.7, 7.3 Hz, 1H), 7.52 - 7.44 (m, 2H), 4.55 (s, 2H), 3.33 (s, 3H).

Step 2-5: 1-[2-(Methoxymethyl)phenyl]-4-methyl-tetrazol-5-one

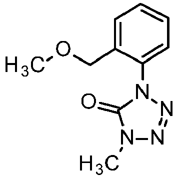

To a solution of 4-[2-(Methoxymethyl)phenyl]-1*H*-tetrazol-5-one (4 g, 19.42 mmol) in acetone (40 mL) was added K₂CO₃ (6.7 g, 48.54 mmol), then the mixture was cooled to 0 °C and Me₂SO₄ (2.9 g, 23.30 mmol) was added. The mixture was stirred at 15 °C for 4 h under N₂. LCMS showed the reaction was completed. The reaction was poured into water (100 mL), the aqueous phase was extracted with EtOAc (50 mL x 3). The combined organic phase was washed with brine (20 mL), dried over Na₂SO₄,concentrated and triturated by PE: MTBE = 10:1 to give 1-[2-(methoxymethyl)phenyl]-4-methyl-tetrazol-5-one (2.2 g, yield: 44 %) as a white solid. ¹H NMR: (400 MHz, CHC₃-d) δ = 7.61 (d, J = 7.5 Hz, 1H), 7.51 (dt, J = 1.8, 7.3 Hz, 1H), 7.48 - 7.40 (m, 2H), 4.52 (s, 2H), 3.73 (s, 3H), 3.32 (s, 3H).

Step 2-6: 1-[2-(Bromomethyl)phenyl]-4-methyl-tetrazol-5-one

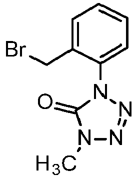

To a solution of 1-[2-(methoxymethyl)phenyl]-4-methyl-tetrazol-5-one (2 g, 9.09 mmol) in DCM (20 mL) was added HBr/AcOH (27 g, 109.10 mmol) at 0 °C, the reaction mixture was stirred at 15 °C for 16 h under N₂. LCMS showed the reaction was completed. The reaction was poured into water (10 mL), the aqueous phase was extracted with DCM (5 mL x 3). The combined organic phase was washed with brine (5 mL), dried over Na₂SO₄,concentrated and purified by column chromatography on silica gel (PE~ EtOAc) to give 1-[2-(bromomethyl)-phenyl]-4-methyl-tetrazol-5-one (1.8 g, yield: 67 %) as a yellow solid. ¹H NMR: (400 MHz, CHCl₃-d) δ = 7.59 - 7.53 (m, 1H), 7.52 - 7.43 (m, 3H), 4.60 (s, 2H), 3.75 (s, 3H).

### Step 3: 1-[2-[[(E)-[3-(4-Chlorophenyl)-1-methyl-prop-2-ynylidene]amino]oxymethyl]phenyl]-4-methyl-tetrazol-5-one (Example 1)

To a solution of 1-[2-(bromomethyl)phenyl]-4-methyl-tetrazol-5-one (0.277 g, 1.03 mmol) in MeCN (4 mL) was added 4-(4-chlorophenyl)but-3-yn-2-one oxime (0.2 g, 1.03 mmol), Cs₂CO₃ (0.672 g, 2.06 mmol) at 15 °C, the reaction mixture was stirred at 15 °C for 16 h under N₂. LCMS showed the reaction was completed. The reaction was poured into water (10 mL), the aqueous phase was extracted with EtOAc (10 mL x 3). The combined organic phase was washed with brine (5 mL), dried over Na₂SO₄,concentrated and purified by Prep-HPLC (NH₄HCO₃) to give the title compound (0.26 g, yield: 66 %) as a yellow solid. ¹H NMR: (400 MHz, MeOH-d₄) δ = 7.63 - 7.52 (m, 3H), 7.51 - 7.43 (m, 3H), 7.41 - 7.35 (m, 2H), 5.26 (s, 2H), 3.68 (s, 3H), 1.95 (s, 3H).

### Example 2: 4-[2-[[(E)-[3-(4-Chlorophenyl)-1-methyl-prop-2-ynylidene]amino]oxymethyl]phenyl]-5-methoxy-2-methyl-1,2,4-triazol-3-one

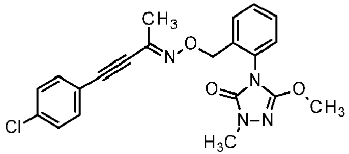

Step 1-1: 1-(Dimethylamino)-3-(o-tolyl)urea

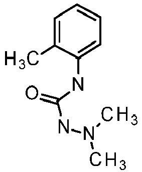

To a mixture of *N*-(o-tolyl)formamide (8 g, 60.10 mmol) in DCM (70 mL) cooled at 0 °C was added TEA (6.1 g, 60.10 mmol) and stirred at 20 °C for 10 min, then a solution of 1,1-dimethyl hydrazine (5.8 g, 60.10 mmol) in DCM (10 mL) was added and the mixture was stirred at 20 °C for 16 h. LCMS showed the reaction was completed. The reaction mixture was quenched with H₂O (10 mL), extracted with DCM (10 mL x 2), washed with brine (10 mL), dried over Na₂SO₄ and concentrated to give the 1-(dimethylamino)-3-(o-tolyl)urea (6 g, yield: 52 %) as a yellow solid which was used directly without further purification. ¹H NMR: (400 MHz, CHC₃-d) δ = 8.12 (br s, 1H), 7.96 (d, J = 8.0 Hz, 1H), 7.25 - 7.11 (m, 2H), 7.04 - 6.95 (m, 1H), 5.35 (br s, 1H), 2.64 (s, 6H), 2.28 (s, 3H).

Step 1-2: 5-Chloro-2-methyl-4-(o-tolyl)-1,2,4-triazol-3-one

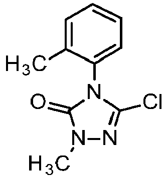

The solution of triphosgene (12.3 g, 41.45 mmol) in EtOAc (30 mL) was heated to 80 °C, then the solution of 1-(dimethylamino)-3-(o-tolyl)urea (4 g, 20.73 mmol) in EtOAc/DCM (6 mL/ 18 mL) was added and the resulting mixture was stirred at 80 °C for 2 h. LCMS showed the reaction was completed. The reaction mixture was quenched with H₂O (50 mL), extracted with EtOAc (50 mL x 2), washed with brine (50 mL), dried over Na₂SO₄and concentrated. The crude product was purified by silica gel column (PE~ EtOAc) to give the 5-chloro-2-methyl-4-(o-tolyl)-1,2,4-triazol-3-one (3.6 g, yield: 78 %) as a white solid. ¹H NMR: (400 MHz, CHCl₃-d) δ = 7.48 - 7.30 (m, 3H), 7.18 (d, J = 7.6 Hz, 1H), 3.55 (s, 3H), 2.23 (s, 3H).

Step 1-3: 5-Methoxy-2-methyl-4-(o-tolyl)-1,2,4-triazol-3-one

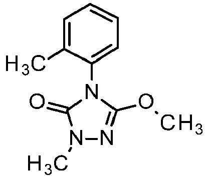

To a solution of 5-chloro-2-methyl-4-(o-tolyl)-1,2,4-triazol-3-one (3.6 g, 16.07 mmol) in MeOH (40 mL) was added NaOMe (11.6 g, 64.28 mmol) and stirred at 60 °C for 16 h. LCMS showed the reaction was completed. The reaction mixture was quenched with H₂O (50 mL), extracted with EtOAc (50 mL x 3), washed with brine (10 mL), dried over Na₂SO₄and concentrated. The crude product was purified by silica gel column (PE~MTBE) to give the 5-methoxy-2-methyl-4-(o-tolyl)-1,2,4-triazol-3-one (2 g, yield: 57 %) as a white solid. ¹H NMR: (400 MHz, CHC₃-d) δ = 7.39 - 7.27 (m, 3H), 7.19 (d, J = 8.1 Hz, 1H), 3.95 (s, 3H), 3.46 (s, 3H), 2.23 (s, 3H).

Step 1-4: 4-[2-(Bromomethyl)phenyl]-5-methoxy-2-methyl-1,2,4-triazol-3-one

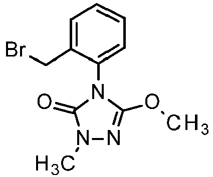

To a solution of 5-methoxy-2-methyl-4-(o-tolyl)-1,2,4-triazol-3-one (1.8 g, 8.22 mmol) in CCl₄ (20 mL) was added NBS (1.6 g, 9.04 mmol), AIBN (135 mg, 0.82 mmol). The mixture was stirred at 80 °C for 5 h. LCMS showed the reaction was completed. The reaction was quenched with H₂O (10 mL), extracted with DCM (10 mL x 2), washed with brine (10 mL), dried over Na₂SO₄and concentrated. The crude product was purified by silica gel column (PE~MTBE) to give the 4-[2-(bromomethyl)phenyl]-5-methoxy-2-methyl-1,2,4-triazol-3-one (1.6 g, yield: 59 %) as a yellow solid. ¹H NMR: (400 MHz, CHC₃-d) δ = 7.60 - 7.49 (m, 1H), 7.43 (dd, J = 2.2, 4.7 Hz, 1H), 7.36 - 7.30 (m, 1H), 7.26 - 7.17 (m, 1H), 4.61 (d, J = 10.8 Hz, 1H), 4.36 (d, J = 10.9 Hz, 1H), 3.96 (s, 3H), 3.49 - 3.47 (m, 3H).

### Step 2: 4-[2-[[(E)-[3-(4-Chlorophenyl)-1-methyl-prop-2-ynylidene]amino]oxymethyl]phenyl]-5-methoxy-2-methyl-1,2,4-triazol-3-one (Example 2)

To a solution of 4-[2-(bromomethyl)phenyl]-5-methoxy-2-methyl-1,2,4-triazol-3-one (0.307 g, 1.03 mmol) in MeCN (4 mL) was added 4-(4-chlorophenyl)but-3-yn-2-one oxime (see Example 1, step 1-3) (0.2 g, 1.03 mmol) and Cs₂CO₃(0.672 g, 2.06 mmol), the mixture was stirred for 16 h at 15 °C under N₂. LCMS showed the reaction was completed. Then reaction mixture was quenched with H₂O (10 mL), extracted with EtOAc (5 mL x 3), washed with brine (5 mL), dried over Na₂SO₄and concentrated. The crude product was purified by preparative HPLC (NH₄HCO₃) to give the the title compound (0.165 g, yield: 39 %) as a light-yellow solid. ¹H NMR: (400 MHz, MeOH-d₄) δ = 7.57 - 7.47 (m, 5H), 7.47 - 7.45 (m, 1H), 7.42 - 7.37 (m, 2H), 5.25 - 5.09 (m, 2H), 3.95 (s, 3H), 3.42 (s, 3H), 1.99 (s, 3H).

### Example 3: 1-[2-[[(E)-[3-(3-Chlorophenyl)-1-methyl-prop-2-ynylidene]amino]oxymethyl]phenyl]-4-methyl-tetrazol-5-one)

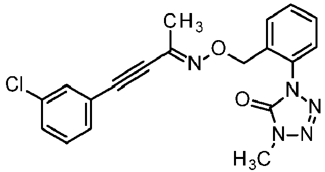

Step 1-1: 4-(3-Chlorophenyl)but-3-yn-2-ol

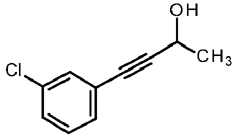

To a solution of 1-chloro-3-iodo-benzene (20 g, 84.03 mmol) in DMSO (200 mL) was added but-3-yn-2-ol (11.8 g, 168.1 mmol), Cul (321 mg, 1.68 mmol), Pd(PPh₃)₂Cl₂ (1.2 g, 1.68 mmol), Et3N (25 g, 252.10 mmol) at 20 °C. The reaction was stirred at 45 °C for 16 h. TLC (PE: EtOAc = 10: 1) showed the reaction was completed. The reaction was poured into water (500 mL), the aqueous phase was extracted with EtOAc (100 mL x 3). The combined organic phase was washed with brine (100 mL), dried over Na₂SO₄,concentrated and purified by column chromatography on silica gel (PE~ PE: EtOAc = 1: 9) to give 4-(3-chlorophenyl)but-3-yn-2-ol (16.4 g, crude) as a brown oil. ¹H NMR: (400 MHz, CHC₃-d) δ = 7.45 (d, J = 1.5 Hz, 1H), 7.37 - 7.24 (m, 3H), 4.79 (q, J = 6.5 Hz, 1H), 2.03 - 1.90 (m, 1H), 1.59 (d, J = 6.6 Hz, 3H).

Step 1-2: 4-(3-Chlorophenyl)but-3-yn-2-one

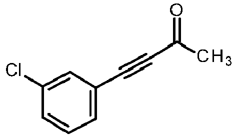

To a solution of 4-(3-chlorophenyl)but-3-yn-2-ol (16.4 g, 90.61 mmol) in DCM (160 mL) was added MnO₂ (78.8 g, 906.1 mmol) at 15 °C and the reaction was stirred at 25 °C for 16 h. TLC (PE: EtOAc = 5: 1) showed the reaction was completed. The reaction was filtered and the filtrate was concentrated. The crude product was purified by column chromatography on silica gel (PE~ PE: EtOAc = 6: 4) to give 4-(3-chlorophenyl)but-3-yn-2-one (13 g, yield: 80 %) as a brown solid. ¹H NMR: (400 MHz, CHC₃-d) δ = 7.56 (s, 1H), 7.50 - 7.41 (m, 2H), 7.38 - 7.29 (m, 1H), 2.46 (s, 3H).

Step 1-3: 4-(3-Chlorophenyl)but-3-yn-2-one oxime

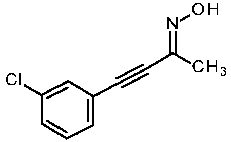

To a solution of 4-(3-chlorophenyl)but-3-yn-2-one (7.3 g, 40.78 mmol) in MeOH (70 mL) was added NaOAc (4.0 g, 48.94 mmol) and the solution of NH₂OH.HCl (3.1 g, 44.86 mmol) in MeOH (70 mL) 0 °C. The reaction was stirred at 15 °C for 16 h. TLC (PE: EtOAc = 7: 1) showed the reaction was completed. The reaction was poured into water (150 mL), the aqueous phase was extracted with EtOAc (50 mL x 3). The combined organic phase was washed with brine (50 mL), dried over Na₂SO₄,concentrated and purified by column chromatography on silica gel (PE~ PE: EtOAc = 85: 15) to give 4-(3-chlorophenyl)but-3-yn-2-one oxime (3 g, yield: 38 %) as a white solid. ¹H NMR: (400 MHz, CHC₃-d) δ = 7.54 (td, J = 1.8, 14.3 Hz, 1H), 7.48 - 7.28 (m, 3H), 2.18 (d, J = 5.4 Hz, 3H).

### Step 3: 1-[2-[[(E)-[3-(3-Chlorophenyl)-1-methyl-prop-2-ynylidene]amino]oxymethyl]phenyl]-4-methyl-tetrazol-5-one (Example 3)

To a solution of 4-(3-chlorophenyl)but-3-yn-2-one oxime (0.2 g, 1.03 mmol) in MeCN (4 mL) was added 1-[2-(bromomethyl)phenyl]-4-methyl-tetrazol-5-one (see step 2-6 of Example 1) (0.277 g, 1.03 mmol) and Cs₂CO₃(0.672 g, 2.06 mmol) at 15 °C, the reaction mixture was stirred at 15 °C for 16 h under N₂. LCMS showed the reaction was completed. The reaction was poured into water (10 mL), the aqueous phase was extracted with EtOAc (10 mL x 3). The combined organic phase was washed with brine (5 mL), dried over Na₂SO₄, concentrated and purified by preparative HPLC (NH₄HCO₃) to give the title compound (0.189 g, yield: 48 %) as a yellow solid. ¹H NMR: (400 MHz, MeOH-d₄) δ = 7.64 - 7.53 (m, 3H), 7.53 - 7.47 (m, 2H), 7.45 - 7.29 (m, 3H), 5.27 (s, 2H), 3.69 (s, 3H), 1.96 (s, 3H).

### Example 4:4-[2-[[(E)-[3-(3-Chlorophenyl)-1-methyl-prop-2-ynylidene]amino]oxymethyl]phenyl]-5-methoxy-2-methyl-1,2,4-triazol-3-one

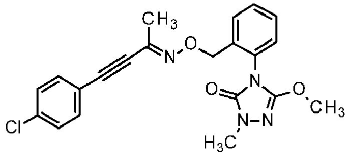

To a solution of 4-(3-chlorophenyl)but-3-yn-2-one oxime (0.2 g, 1.03 mmol) in MeCN (4 mL) was added 4-[2-(bromomethyl)phenyl]-5-methoxy-2-methyl-1,2,4-triazol-3-one (see step 1-4 of Example 2) (0.307 g, 1.03 mmol) and Cs₂CO₃(0.672 g, 2.06 mmol) at 15 °C, the reaction mixture was stirred at 15 °C for 16 h under N₂. LCMS showed the reaction was completed. The reaction was poured into water (10 mL), the aqueous phase was extracted with EtOAc (10 mL x 3). The combined organic phase was washed with brine (5 mL), dried over Na₂SO₄, concentrated and purified by preparative HPLC (NH₄HCO₃) to give the title compound (0.180 g, yield: 42 %) as a yellow oil. ¹H NMR: (400 MHz, MeOH-d₄) δ = 7.59 - 7.47 (m, 4H), 7.45 - 7.40 (m, 2H), 7.39 - 7.29 (m, 2H), 5.27 - 5.08 (m, 2H), 3.96 (s, 3H), 3.42 (s, 3H), 2.00 (s, 3H).

### Example 5: Ethyl N-[2-[[(E)-[3-(4-chlorophenyl)-1-methyl-prop-2-ynylidene]amino]oxymethyl]-phenyl]-N-methoxy-carbamate

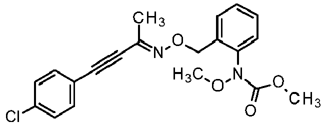

### Step 1: N-(o-Tolyl)hydroxylamine

To a solution of 1-methyl-2-nitro-benzene (10 g, 73 mmol) and NH₄Cl (4.3 g, 80 mmol) in THF (180 mL) and H₂O (90 mL) was added Zn (9.3 g, 146 mmol) at 0 °C and stirred at 0 °C for 3 h. TLC (PE:EtOAc = 5:1) showed the reaction was completed. The reaction mixture was filtered and the filtrate was extracted with EtOAc (300 mL x 3). The organic layer was dried over Na₂SO₄and concentrated to give the product (4.4 g, yield: 49.0 %) as a yellow oil and used directly for next step.

### Step 2: Methyl N-hydroxy-N-(o-tolyl)carbamate

The solution of *N*-(o-tolyl)hydroxylamine (4.4 g, 36 mmol) in MTBE (150 mL) and NaHCO₃ aq. (90 mL) was added methyl chloroformiate (2.9 g, 32.4 mmol) at 0 °C, the mixture was stirred at 0 °C for 3 h. TLC (PE: EtOAc = 3:1) showed the reaction was completed. The reaction mixture was quenched with H₂O (100 mL) and extracted with EtOAc (200 mL x 3), dried over Na₂SO₄,concentrated and the residue was purified by column (PE: EtOAc = 3:1) to give the product (4 g, yield: 67.3%) as a yellow solid. ¹H NMR: (400 MHz, CHC₃-d) δ ppm 7.30 - 7.34 (m, 1 H) 7.27 (m, 3 H) 3.78 (s, 3 H) 2.33 (s, 3 H).

### Step 3: Methyl N-methoxy-N-(o-tolyl)carbamate

To a solution of methyl *N*-hydroxy-*N*-(*o*-tolyl)carbamate (4 g, 24.2 mmol) in DMF (40 mL) was added K₂CO₃ (4 g, 29.1 mmol) and Mel (4.1 g, 29.1 mmol) at 0 °C. The mixture was stirred at 15 °C for 12 h. TLC (PE:EtOAc = 3:1) showed the reaction was completed, the reaction mixture was quenched with H₂O (50 mL) and extracted with EtOAc (50 mL x 3). The organic layer was washed with brine (50 mL x 2), dried over Na₂SO₄, concentrated and purified by column (PE:EtOAc = 3:1) to give the the product (4 g, yield: 92.3 %) as a yellow oil. ¹H NMR: (400 MHz, CHCl₃-d) δ ppm 7.27 (m, 4 H) 3.79 (s, 3 H) 3.74 (s, 3 H) 2.30 (s, 3 H).

### Step 4: Methyl N-[2-(bromomethyl)phenyl]-N-methoxy-carbamate

To a solution of methyl *N*-methoxy-*N*-(*o*-tolyl)carbamate (0.8 g, 4.5 mmol) in CCl₄ (15 mL) was added AIBN (74 mg, 0.45 mmol) and NBS (876 mg, 5.0 mmol), the mixture was stirred at 80°C for 12 h. LCMS showed the reaction was completed. The reaction mixture was filtered and the filtrate was concentrated to give the product (1 g, yield: 81.4 %) as a yellow oil. ¹H NMR: (400 MHz, CHC₃-d) δ ppm 8.04 (dd, J=7.97, 1.32 Hz, 1 H) 7.49 - 7.56 (m, 2 H) 7.38 (d, J=2.13 Hz, 1 H) 4.56 (s, 2 H) 3.81 (s, 3 H) 3.78 (s, 3 H).

### Step 5: Ethyl N-[2-[[(E)-[3-(4-chlorophenyl)-1-methyl-prop-2-ynylidene]amino]oxymethyl]phenyl]-N-methoxy-carbamate

To a solution of methyl *N*-[2-(bromomethyl)phenyl]-*N*-methoxy-carbamate (0.5 g, 1.8 mmol) in MeCN (10 mL) was added Cs₂CO₃(1.2 g, 3.6 mmol) and 4-(4-chlorophenyl)but-3-yn-2-one oxime (353 mg, 1.8 mmol, see example 1, step 1-2), the mixture was stirred at 15 °C for 12 h. LCMS showed the reaction was completed. The reaction mixture was quenched with H₂O (20 mL), extracted with EtOAc (20 mL x 3). The organic layer was dried over Na₂SO₄, concentrated and the residue was purified by Prep-HPLC and freeze-drying to give the product (250 mg, yield: 36.0 %) as a yellow oil.

### Example 6: Ethyl N-[2-[[(E)-[3-(3-chlorophenyl)-1-methyl-prop-2-ynylidene]amino]oxymethyl]-phenyl]-N-methoxy-carbamate

To a solution of methyl *N*-[2-(bromomethyl)phenyl]-*N*-methoxy-carbamate (0.5 g, 1.8 mmol) in MeCN (10 mL) was added Cs₂CO₃(1.2 g, 3.6 mmol) and 4-(3-chlorophenyl)but-3-yn-2-one (353 mg, 1.8 mmol, see example 3, step 1-2), the mixture was stirred at 15 °C for 12 h. LCMS showed the reaction was completed. The reaction mixture was quenched with H₂O (20 mL) and extracted with EtOAc (20 mL x 3). The organic layer was dried over Na₂SO₄,concentrated and purified by Prep-HPLC and freeze-drying to give the product (250 mg, yield: 36.0 %) as a yellow oil.

### Example 7: (E)-4-(4-chlorophenyl)-N-[[2-[(E)-C-(1,4,2-dioxazin-3-yl)-N-methoxy-carbonimidoyl]-phenyl]methoxy]but-3-yn-2-imine

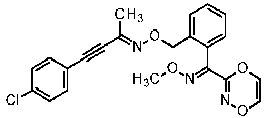

Step 1: Methyl (2E)-2-methoxyimino-2-[2-(methoxymethyl)phenyl]acetate

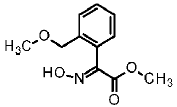

To a solution of methyl 2-[2-(methoxymethyl)phenyl]-2-oxo-acetate (10.0 g, 48 mmol) in Pyridine (50 mL) was added MeONH₂.HCl (12.1 g, 144 mmol) at 15 °C. The mixture was heated to 80 °C and stirred at 80 °C for 16 h. LCMS showed the reaction was completed. The mixture was quenched with ice water (100 mL), adjusted pH = 4 with 1 M HCI aq. and extracted with EtOAc (50 mL x 3). The organic layer was combined and washed with brine (50 mL), dried over Na₂SO₄and concentrated. The residue was purified by column (PE~ EtOAc) to give the product (6.3 g, yield: 55 %) as yellow oil. 1H NMR: (400MHz, CDCl₃) δ = 7.45 - 7.34 (m, 3H), 7.18 (d, J = 7.4 Hz, 1H), 4.34 (s, 2H), 4.04 (s, 3H), 3.87 (s, 3H), 3.29 (s, 3H).

Step 2: (*E*)-1-(1,4,2-Dioxazin-3-yl)-*N*-methoxy-1-[2-(methoxymethyl)phenyl]methanimine

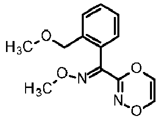

To a solution of NH₂OH.HCl (1.78 g, 25.3 mmol) in MeOH (50 mL) was added KOH (2.8 g, 50.6 mmol) at 15 °C, then methyl (2*E*)-2-methoxyimino-2-[2-(methoxymethyl)phenyl]acetate (3 g, 12.7 mmol) in MeOH (10 mL) was added to the mixture at 15 °C. The mixture was heated to 50 °C and stirred at 50 °C for 30 min. TLC (PE:EtOAc = 1: 1) showed the reaction were completed. Then 1,2-dibromo ethane (10.7 g, 56.9 mmol) and K₂CO₃ (1.75 g, 12.7 mmol) was added to the mixture at 15°C. The mixture was heated to 65 °C and stirred at 65 °C for 16 h. LCMS showed the reaction was completed. The mixture was quenched with NH₄Cl aq. (100 mL) and extracted with EtOAc (50 mL x 3). The organic layer was combined and washed with brine (50 mL), dried over Na₂SO₄and concentrated. The residue was purified by column (PE~PE: EtOAc = 3: 1) to give the product (1.5 g, yield: 45 %) as yellow oil. ¹H NMR:
(400MHz,C0Cl₃) δ = 7.49 - 7.44 (m, 1H), 7.41 - 7.31 (m, 2H), 7.20 - 7.12 (m, 1H), 4.53 - 4.48 (m, 2H), 4.39 (s, 2H), 4.21 - 4.16 (m, 2H), 3.98 (s, 3H), 3.32 (s, 3H).

Step 3: (*E*)-1-[2-(Chloromethyl)phenyl]-1-(1,4,2-dioxazin-3-yl)-*N*-methoxy-methanimine

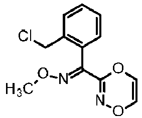

AcCI (590 mg, 7.5 mmol) was added to the suspension of AlCl₃ (998 mg, 7.5 mmol) in DCM (10 mL) dropwise at 15 °C under N₂ and the mixture was stirred at 15 °C for 15 min. Then the solution of (*E*)-1-(1,4,2-dioxazin-3-yl)-*N*-methoxy-1-[2-(methoxymethyl)phenyl]methanimine (800 mg, 3.0 mmol) in DCM (4 mL) was added to the mixture dropwise at 15 °C under N₂ and the mixture was warmed to 30 °C and stirred at 30°C for 3 h. LCMS showed the reaction was completed. The mixture was quenched with ice water (30 mL) and extracted with DCM (15 mL x 3). The organic layer was combined, dried over Na₂SO₄ and concentrated. The residue was purified by column (PE~PE:EtOAc = 3: 1) to give the product (650 mg, yield: 80 %) as yellow solid. ¹H NMR: (400MHz, CDCl₃) δ = 7.52 (d, J = 7.3 Hz, 1H), 7.44 - 7.35 (m, 2H), 7.18 (d, J = 7.2 Hz, 1H), 4.54 - 4.51 (m, 2H), 4.51 - 4.48 (m, 2H), 4.21 - 4.18 (m, 2H), 4.02 - 3.99 (s, 3H).

### Step 4: (E)-4-(4-chlorophenyl)-N-[[2-[(E)-C-(1,4,2-dioxazin-3-yl)-N-methoxy-carbonimidoyl]phenyl]methoxy]but-3-yn-2-imine

The mixture of (*E*)-1-[2-(chloromethyl)phenyl]-1-(1,4,2-dioxazin-3-yl)-*N*-methoxy-methan-imine (300 mg, 1.1 mmol), 4-(4-chlorophenyl)but-3-yn-2-one (210 mg, 1.1 mmol, see example 1, step 1-2) and Cs₂CO₃(720 mg, 2.2 mmol) in MeCN (5 mL) was stirred at 15 °C for 16 h. LCMS showed the reaction was completed. The mixture was poured into ice water (15 mL) and extracted with EtOAc (10 mL x 3), washed with brine (15 mL), dried over Na₂SO₄ and concentrated. he residue was purified by column (PE∼PE:EtOAc = 3: 1) and trituration with (PE:EtOAc = 3: 1) to give the desired product (180 mg, yield: 39 %) as white solid.

### Example 8: (E)-4-(3-Chlorophenyl)-N-[[2-[(E)-C-(1,4,2-dioxazin-3-yl)-N-methoxy-carbonimido-yl]phenyl]methoxy]but-3-yn-2-imine

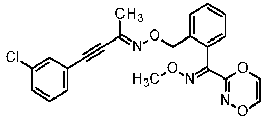

The mixture of (*E*)-1-[2-(chloromethyl)phenyl]-1-(1,4,2-dioxazin-3-yl)-*N*-methoxy-methan-imine (400 mg, 1.5 mmol), 4-(3-chlorophenyl)but-3-yn-2-one (290 mg, 1.5 mmol, see example 3, step 1-2) and Cs₂CO₃ (978 mg, 3.0 mmol) in MeCN (8 mL) was stirred at 15 C for 16 h. LCMS showed the reaction was completed. The mixture was poured into ice water (20 mL) and extracted with EtOAc (15 mL x 3), washed with brine (15 mL), dried over Na₂SO₄ and concentrated. The residue was purified by column (PE∼PE:EtOAc = 3: 1) and trituration with (PE:EtOAc = 3:1) to give the desired product (298 mg, yield: 47 %) as white solid.

The following examples in Table S were synthesized as described above and characterized by LCMS as described in Table LC.

**Table LC: LCMS Methods**

| **Method A** | **Device details** |
|---|---|
| Column: Agilent Eclipse Plus C18 (50 mm x 4.6 mm x 3 µm) | LCMS2020 (Shimadzu) Ionization source: ESI |
| Mobile Phase: | Mass range: 100 - 800 amu |
| A: 10 mM Ammonium formate in water. | Polarity: Dual; Mode: Scan |
| B: 0.1 % Formic acid in acetonitrile. | LC System: Nexera High pressure |
| Gradient: 10 % B to 100 % B in 1.5 min. Hold 1 min | gradient system, Binary pump |
| 100% B. 1 min 10 % B. Run time: 3.50 or 3.75 min. | Detector: PDA |
| Flow: 1.2 ml/min; Column oven: 30°C/40°C | Scan wavelength: 220 nm / max plot |

| **Method B** | **Device details** |
|---|---|
| Column: Xbridge Shield RP18 (2.1 x 50mm x 5 µm) | Agilent 1200 & 6120B |
| Mobile Phase: | Ionization source: ESI |
| A: H₂O+10mM NH₄HCO₃; B: Acetonitrile | Mass range: 100 - 1000 amu |
| Gradient: 5% B in 0.40 min and 5-95% B at 0.40-3.40 min, hold on 95% B for 0.45min, 95-5% B in 0.01 min, Run time: 4.50 min. | Polarity: positive; Mode: Scan LC System: Nexera High pressure gradient system, Binary pump |
| Flow: 0.8 ml/min; Column oven: 40°C | Detector: PDA |
| | Scan wavelength: 220 nm |

| **Method C** | **Device details** |
|---|---|
| Column: Xbridge Shield RP18 (2.1 x 50mm x 5 µm) | Agilent 1200 & 6120B |
| Mobile Phase: | Ionization source: ESI |
| A: H₂O+10mM NH₄HCO₃; B: Acetonitrile. | Mass range: 100 - 1000 amu |
| Gradient: 50%B in 0.40min; 50-100% B at 0.40-3.40 min, hold on 100% B for 0.45 min, 100-50% B in 0.01 min. Run time: 4.50 min. | Polarity: positive; Mode: Scan |
| | LC System: Nexera High pressure gradient system, Binary pump |
| Flow: 0.8 ml/min; Column oven: 40°C | Detector: PDA |
| | Scan wavelength: 220 nm |

| **Method D** | **Device details** |
|---|---|
| Column: Xbridge Shield RP18 (2.1 x 50mm x 5 µm) | Agilent 1200 & 6120B |
| Mobile Phase: | Ionization source: ESI |
| A: H₂O+10mM NH₄HCO₃; B: Acetonitrile. | Mass range: 100 - 1000 amu |
| Gradient: 50%B in 0.40min; 50-100% B at 0.40-3.40 min, hold on 100% B for 0.45 min, 100-50% B in 0.01 min. Run time: 4.50 min. | Polarity: positive; Mode: Scan |
| | LC System: Nexera High pressure |
| | gradient system, Binary pump |
| Flow: 0.8 ml/min; Column oven: 40°C | Detector: PDA |
| | Scan wavelength: 220 nm |

| **Method E** | **Device details** |
|---|---|
| Column: Kinetex -C18 (50 mm x 2.1 mm x 5 µm) | Shimadzu LC-20AD&MS 2020 |
| Mobile Phase: | Ionization source: ESI |
| A: 0.037% Trifluoroacetic acid in water. | Mass range: 100 - 1000 amu |
| B: 0.018% Trifluoroacetic acid in acetonitrile. | Polarity: Positive; Mode: Scan |
| Gradient: 5% B in 0.40 min; 5-95% B at 0.4-3.0 | LC System: Nexera High pressure |
| min, hold on 95% B for 1.00min; 95-5%B in | gradient system, Binary pump |
| 0.01min. | Detector: DAD |
| Flow: 1.0 mL/min; Column oven: 40°C | Scan wavelength: 220 nm / max plot |

| **Method F** | **Device details** |
|---|---|
| Column: Agilent Eclipse Plus C18 (50 mm x 4.6 | LCMS2020 (Shimadzu) |
| mm x 3 µm) | Ionization source: ESI |
| Mobile Phase: | Mass range: 100 - 800 amu |
| A: 10 mM Ammonium formate in water. | Polarity: Dual; Mode: Scan |
| B: Acetonitrile | LC System: Nexera High pressure gradient system, Binary pump |
| Gradient: 10 % B to 100 % B in 5 min. Hold 3 min | |
| 100 % B. 2 min 10 % B. Run time: 10 min. | Detector: PDA |
| Flow: 1.2 ml/min; Column oven: 40°C | Scan wavelength: 220 nm / max plot |

| **Method G** | **Device details** |
|---|---|
| Column: Kinetex -C18 (50 mm x 2.1 mm x 5 µm) | Shimadzu LC-20AD&MS 2020 |
| Mobile Phase: | Ionization source: ESI |
| A: 0.04% Trifluoroacetic acid in water. | Mass range: 100 - 1000 amu |
| B: 0.02% Trifluoroacetic acid in acetonitrile. | Polarity: Positive; Mode: Scan |
| Gradient: 5% B to 95% B in 3 min. 5% B in 0.01 min; 5-95% B (0.01-1.60 min, 95%-100% B (1.60 - 2.50 min), 100%-5% B (2.50 - 2.52. mn with a | LC System: Nexera High pressure gradient system, Binary pump |
| | Detector: DAD |
| holld at 5% B for 0.48 min. | Scan wavelength: 220 nm / max plot |
| Flow: 1.8 mL/min; Column oven: 40°C | |

**Table S:**

| **No.** | **Structure** | **Rₜ [min]** | **Mass** | **Method** |
|---|---|---|---|---|
| 1 | 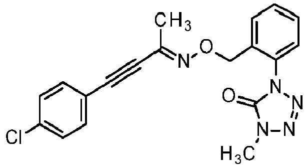 | 1.735 | 382.1 | G |
| 2 | 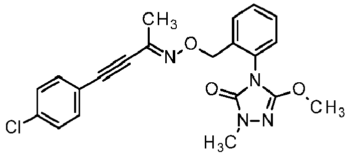 | 1.662 | 411.1 | G |
| 3 | 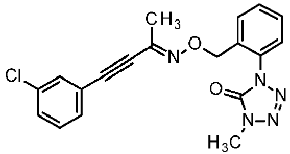 | 1.742 | 382.1 | G |
| 4 | 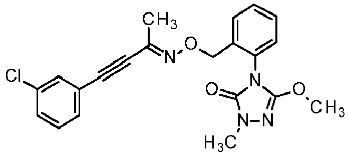 | 1.659 | 411.1 | G |
| 5 | 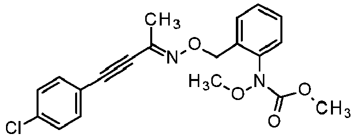 | 1.803 | 387.1 | G |
| 6 | 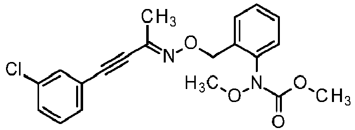 | 1.755 | 269.0 | G |
| 7 | 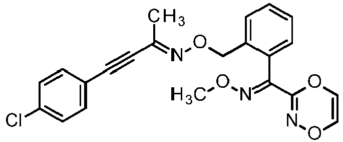 | 1.807 | 387.1 | G |
| 8 | 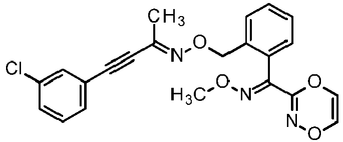 | 1.759 | 269.1 | G |

### Biological studies

### Microtest

For the following 10 fungal pathogens tested in micro tests, the active compounds were formulated separately as a stock solution having a concentration of 10000 ppm in dimethyl sulfoxide. The stock solutions were mixed according to the ratio, pipetted onto a micro titer plate (MTP) and diluted with water to the stated concentrations.

### 1. Activity against late blight pathogen Phytophthora infestans (PHYTIN)

A spore suspension of *Phytophtora infestans* containing a pea juice-based aqueous nutrient medium or DDC medium was then added.

### 2. Activity against Alternaria solani causing early blight in patoto and tomato (ALTESO)

A spore suspension of *Alternaria solani* in an aqueous biomalt or yeast-bactopeptone-glycerine or DOB solution was then added.

### 3. Activity caused resistant isolate of Alternaria solani causing early blight (ALTESO res)

A spore suspension of a Qol (F129L) resistant isolate of *Alternaria solani* in an aqueous biomalt or yeast-bactopeptone-glycerine or DOB solution was then added.

### 4. Activity against Cercospora soiina causing frogeye leaf spot of soybeans (CERCSO)

A spore suspension of *Cercospora sojina* in an aqueous biomalt or yeast-bactopeptone-glycerine or DOB solution was then added.

### 5. Activity against Leptosphaeria nodorum causing what leaf spots (LEPTNO)

A spore suspension of *Leptosphaeria nodorum* in an aqueous biomalt or yeast-bactopeptone-glycerine or DOB solution was then added.

### 6. Activity against resistant isolate of net blotch pathogen Pyrenophora teres (PYRNTE res)

A spore suspension of a Qol (F129L) resistant isolate of *Pyrenophora teres* in an aqueous biomalt or yeast-bactopeptone-glycerine or DOB solution was then added.

### 7. Activity against rice blast pathogen Pyricularia orvzae (PYRIOR)

A spore suspension of *Pyricularia oryzae* in an aqueous biomalt or yeast-bactopeptone-glycerine or DOB solution was then added.

### 8. Activity against Sclerotinia sclerotiorum causing white mold (SCLESC)

*Sclerotinia sclerotiorum* spores (solute with ultrasonic from a filter membrane) in an aqueous biomalt or yeast-bactopeptone-glycerine or DOB solution was then added.

### 9. Activity against wheat leaf blotch pathogen Septoria tritici (SEPTTR)

A spore suspension of *Septoria tritici* in an aqueous biomalt or yeast-bactopeptone-glycerine or DOB solution was then added.

### 10. Activity against resistant isolate of Septoria tritici (SEPTTR res)

A spore suspension of a Qol (G143A) resistant isolate of *Septoria tritici* in an aqueous biomalt or yeast-bactopeptone-glycerine or DOB solution was then added.

### 11. Activity against anthracnose pathogen Colletotrichum orbiculare (COLLLA)

A spore suspension of *Colletotrichum orbiculare* in an aqueous bio malt solution was then added.

The MTPs (for any of the above 10 pathogens) were placed in a water vapor-saturated chamber at a temperature of 18°C. Using an absorption photometer, the MTPs were measured at 405 nm 7 days after the inoculation.

The measured parameters were compared to the growth of the active compound-free control variant (100%) and the fungus-free blank value to determine the relative growth in % of the pathogens in the respective active compounds.

The results of the abovementioned microtest use examples are given in the following Tables.

**Table 1.**

| **Compound No.** | | **1** |
|---|---|---|
| **Structure** | | 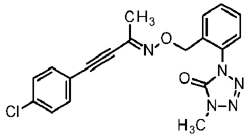 |
| **Fungal pathogen** | | **Growth (%) at 125 ppm** |
| | CERCSO | 0 |
| | PYRIOR | 0 |
| | SCLESC | 11 |
| | SEPTTR | 5 |
| | SEPTTR res (G143A) | 13 |

**Table 2.**

| **Compound No.** | | **2** |
|---|---|---|
| **Structure** | | 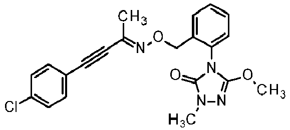 |
| **Fungal pathogen** | | **Growth (%) at 125 ppm** |
| | PYRIOR | 16 |

**Table 3.**

| **Compound No.** | | **4** |
|---|---|---|
| **Structure** | | 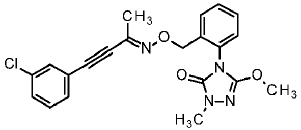 |
| **Fungal pathogen** | | **Growth (%) at 125 ppm** |
| | PHYTIN | 0 |
| | ALTESO | 4 |
| | ALTESO res (F129L) | 16 |
| | LEPTNO | 7 |
| | PYRNTE res (F129L) | 0 |
| | PYRIOR | 0 |
| | SCLESC | 17 |
| | SEPTTR | 0 |
| | SEPTTR res (G143A) | 0 |

**Table 4.**

| **Compound** No. | **5** | **6** | **7** | **8** |
|---|---|---|---|---|
| **Fungal pathogen** | **Growth (%) at 125 ppm** | | | |
| ALTESO | 10 | 0 | 1 | 2 |
| COLLLA | 0 | 0 | 0 | 0 |
| LEPTNO | 0 | 0 | 0 | 2 |
| PYRNTE res (F129L) | 0 | 0 | | |
| PYRIOR | 0 | 0 | 0 | 0 |
| SCLESC | 12 | 17 | 1 | 19 |
| SEPTTR | 0 | 0 | 0 | 0 |

The test results in Tables 1 to 4 show that compounds of formula I control various phytopathogenic fungi including those containing an amino acid substitution F129L or G143A in the mitochondrial cytochrome b protein conferring resistance to Qo inhibitors.

### Green House trials

The compound was dissolved in a mixture of acetone and/or dimethylsulfoxide and the wetting agent/emulsifier Wettol, which is based on ethoxylated alkylphenoles, in a ratio (volume) solvent-emulsifier of 99 to 1 to give a total volume of 5 ml. Subsequently, water was added to total volume of 100 ml. This stock solution was then diluted with the described solvent-emulsifier-water mixture to the final concentration given in the table below.

### Use example 1. Curative control of brown rust on wheat caused by Puccinia recondita (PUCCRT K4)

The first two developed leaves of potted wheat seedling were dusted with spores of *Puccinia recondita.* To ensure the success the artificial inoculation, the plants were transferred to a humid chamber without light and a relative humidity of 95 to 99 % and 20 to 24 °C for 24 h. The next day the plants were cultivated for 3 days in a greenhouse chamber at 20-24 °C and a relative humidity between 65 and 70 %. Then the plants were sprayed to run-off with previously described spray solution, containing the concentration of active ingredient or their mixture as described below. The plants were allowed to air-dry. Then the trial plants were cultivated for 8 days in a greenhouse chamber at 20-24 °C and a relative humidity between 65 and 70 %. The extent of fungal attack on the leaves was visually assessed as % diseased leaf area.

### Use example 2. Preventative control of brown rust on wheat caused by Puccinia recondita (PUCCRT P1)

The first two developed leaves of potted wheat seedling were sprayed to run-off with previously described spray solution, containing the concentration of active ingredient or their mixture as described below. The next day the plants were dusted with spores of *Puccinia recondita.* To ensure the success the artificial inoculation, the plants were transferred to a humid chamber without light and a relative humidity of 95 to 99 % and 20 to 24 °C for 24 h. Then the trial plants were cultivated for 6 days in a greenhouse chamber at 20-24 °C and a relative humidity between 65 and 70 %. The extent of fungal attack on the leaves was visually assessed as % diseased leaf area.

### Use example 3. Protective control of soybean rust on soybeans caused by Phakopsora pachyrhizi (PHAKPA P2)

Leaves of potted soybean seedlings were sprayed to run-off with the previously described spray solution, containing the concentration of active ingredient or their mixture as described below. The plants were allowed to air-dry. The trial plants were cultivated for 2 days in a greenhouse chamber at 23-27 °C and a relative humidity between 60 and 80 %. Then the plants were inoculated with spores of *Phakopsora pachyrhizi.* The strain used contains the amino acid substitution F129L in the mitochondrial cytochrome b protein conferring resistance to Qo inhibitors. To ensure the success the artificial inoculation, the plants were transferred to a humid chamber with a relative humidity of about 95 % and 20 to 24 °C for 24 hr. The trial plants were cultivated for up to 14 days in a greenhouse chamber at 23 to 27 °C and a relative humidity between 60 and 80 %. The extent of fungal attack on the leaves was visually assessed as % diseased leaf area, the disease level of untreated controls was usually higher than 85 %.

### Use example 4. Protective control of soybean rust on soybeans caused by Phakopsora pachyrhizi (PHAKPA P6)

Leaves of potted soybean seedlings were sprayed to run-off with the previously described spray solution, containing the concentration of active ingredient as described below. The plants were allowed to air-dry. The trial plants were cultivated for six days in a greenhouse chamber at 23-27 °C and a relative humidity between 60 and 80 %. Then the plants were inoculated with spores of *Phakopsora pachyrhizi.* The strain used contains the amino acid substitution F129L in the mitochondrial cytochrome b protein conferring resistance to Qo inhibitors. To ensure the success the artificial inoculation, the plants were transferred to a humid chamber with a relative humidity of about 95 % and 23 to 27 °C for 24 hr. The trial plants were cultivated for up to 14 days in a greenhouse chamber at 23 to 27 °C and a relative humidity between 60 and 80 %. The extent of fungal attack on the leaves was visually assessed as % diseased leaf area, the disease level of untreated controls was usually higher than 85 %.

**Table 5:**

| | | **PUCCRT Disease level (%)** | | | | | **PUCCRT Disease level (%)** | |
|---|---|---|---|---|---|---|---|---|
| **No.** | **Structure** | **K4 at 16 ppm** | **P1 at 16 ppm** | | **No.** | **Structure** | **K4 at 16 ppm** | **P1 at 16 ppm** |
| | Untreated | 84 | 86 | | 4 | 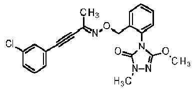 | 31 | 13 |
| 2 | 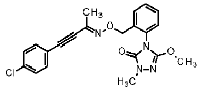 | 30 | 11 | | | | | |

**Table 6.**

| | | **PHAKPA Disease level (%)** | | **PUCCRT Disease level (%)** | |
|---|---|---|---|---|---|
| **No.** | **Structure** | **P2 at 63 ppm** | **P6 at 63 ppm** | **K4 at 63 ppm** | **P1 at 63 ppm** |
| | Untreated | 97 | 74 | 85 | 82 |
| 5 | 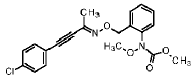 | 43 | 48 | 70 | 10 |
| 6 | 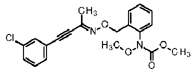 | 11 | 13 | 50 | 7 |
| 7 | 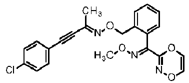 | 33 | 32 | 53 | 13 |
| 8 | 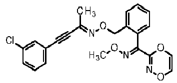 | 7 | 12 | 28 | 0 |

The test results in Tables 5 and 6 show that prophylactic and curative application of compounds I to plants reduces disease development caused by various phytopathogenic fungi including those containing the amino acid substitution F129L in the mitochondrial cytochrome b protein conferring resistance to Qo inhibitors.

## Claims

1. Compounds of formula I 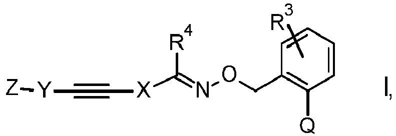 wherein
Q is a group selected from the groups Q1, Q2, Q3 and Q4, wherein * represents the point of attachment to the phenyl ring 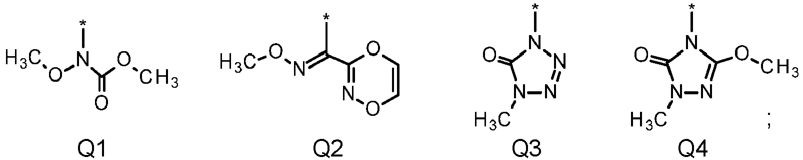
R³ is selected from hydrogen, halogen, CN, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, -O-C₁-C₄-alkyl, -O-C₁C₄-haloalkyl, -O-C₃-C₆-cycloalkyl, -C₁C₂-alkyl-C₃-C₆-cycloalkyl, phenyl, 3- to 6-membered heterocycloalkyl and 5- or 6-membered heteroaryl, wherein said heterocycloalkyl and heteroaryl besides carbon atoms contain 1, 2 or 3 heteroatoms selected from N, O and S provided that such heterocycle cannot contain 2 contiguous atoms selected from O and S, wherein said phenyl, heterocycloalkyl and heteroaryl are bound directly or via an oxygen atom or via a C₁-C₂-alkylene linker, and wherein said phenyl and heteroaryl are unsubstituted or substituted by 1, 2 or 3 identical or different substituents selected from halogen, CN, NH₂, NO₂, C₁-C₄-alkyl, C₁-C₄-haloalkyl, -O-C₁C₄-alkyl and -O-C₁C₄-haloalkyl;
R⁴ is selected from hydrogen, C₁-C₆-alkyl, C₂-C₄-alkenyl, C₂-C₄-alkynyl, C₁-C₆-haloalkyl, C₂-C₄-haloalkenyl, C₂-C₄-haloalkynyl, -(C₁-C₂-alkyl)-O-(C₁-C₂-alkyl), -(C₁-C₂-alkyl)-O-(C₁C₂-haloalkyl) and -C₁-C₄-alkyl-C₃-C₆-cycloalkyl;
X, Y , independently of each other, are a direct bond or the divalent group -CL¹L²-;
L¹, L², independently of each other, are selected from hydrogen, C₁-C₃-alkyl, C₁-C₃-haloalkyl, C₂-C₃-haloalkenyl, C₂-C₃-haloalkynyl, -(C₁-C₂-alkyl)-O-(C₁-C₂-alkyl), -(C₁-C₂-alkyl)-O-(C₁-C₂-haloalkyl), cyclopropyl and -C₁-C₂-alkyl-cyclopropyl; or L¹ and L², together with the interjacent carbon atom, form a cyclopropyl; wherein the cyclic moieties of L¹ and L², independently of each other, are unsubstituted or carry 1 or 2 identical or different groups R^{L}:
R^{L} is selected from halogen, CN, NO₂, C₁-C₄-alkyl, C₁-C₄-haloalkyl, -O-C₁-C₄-alkyl and -O-C₁-C₄-haloalkyl;
Z is selected from hydrogen, C₁-C₆-alkyl, C₂-C₆-alkenyl, -C(=O)-C₁-C₄-alkyl, C₁-C₄-alkyl-O-C₁-C₄-alkyl and SiR^{S1}_{R}^{S2}_{R}^{S3},
R^{S1}, R^{S2}, R^{S3} , independently of each other, are selected from hydrogen, halogen, C₁-C₄-alkyl, -O-C₁C₄-alkyl, phenyl and phenoxy, wherein phenyl and phenoxy are unsubstituted or carry 1, 2 or 3 identical or different groups R^{Sa}: R^{Sa} is selected from halogen, C₁-C₄-alkyl and -O-C₁C₄-alkyl; and wherein the abovementioned non-cyclic groups Z are unsubstituted or carry 1, 2, 3 or up to the maximum number of identical or different groups R^{na}:
R^{na} is selected from halogen, CN, hydroxy, C₁-C₄-alkyl and -O-C₁C₄-alkyl;
or
Z is selected from C₃-C₆-cycloalkyl, phenyl, 3- to 6-membered heterocycloalkyl, 3- to 6-membered heterocycloalkenyl and 5- or 6-membered heteroaryl, wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S, and wherein the abovementioned cyclic groups Z are unsubstituted or carry 1, 2, 3 or up to the maximum number of identical or different groups R^{a}:
R^{a} is selected from halogen, CN, hydroxy, NR^{A}R^{B}, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁C₄-alkyl, -C(=O)-O-C₁-C₄-alkyl, -C(=O)-NH-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl, wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S, wherein said phenyl, heterocycloalkyl, heterocycloalkenyl and heteroaryl are bound directly or via an oxygen atom or via a C₁-C₂-alkylene linker, and/or 2 R^{a} substituents bound to neighboring ring atoms, together with the two interjacent ring atoms, form a saturated, partially unsaturated or aromatic 5- to 6-membered fused carbo- or heterocycle, wherein the heterocycle includes beside carbon atoms 1 or 2 heteroatoms independently selected from N, O and S as ring member atoms, provided that such heterocycle cannot contain 2 contiguous atoms selected from O and S; and wherein the aliphatic and cyclic moieties of R^{a} are unsubstituted or carry 1, 2, 3, 4 or up to the maximum number of identical or different groups R^{b}:
R^{b} is selected from halogen, CN, hydroxy, NO₂, C₁-C₄-alkyl, C₁-C₄-haloalkyl, -O-C₁C₄-alkyl, and -O-C₁C₄-haloalkyl;
R^{A}, R^{B} independently of each other, are selected from hydrogen, C₁-C₄-alkyl and C₁-C₄-haloalkyl;
and in form of stereoisomers and tautomers thereof, and the N-oxides and the agriculturally acceptable salts thereof.

2. The compounds according to claim 1, wherein Q is selected from Q3 and Q4.

3. The compounds according to any one of the claims 1 to 2, wherein R³ is selected from hydrogen, CN, halogen, C₁-C₂-alkyl, C₁-C₂-haloalkyl, C₂-C₄-alkenyl, C₃-C₄-cycloalkyl, -O-C₁C₂-alkyl and -O-C₁C₂-haloalkyl.

4. The compounds according to claim 3, wherein R³ is selected from hydrogen, halogen, C₁-C₂-alkyl, and C₁-C₂-haloalkyl.

5. The compounds according to any one of the claims 1 to 4, wherein R³ is ortho position to the methyl oxime side chain.

6. The compounds according to any one of the claims 1 to 5, wherein R⁴ is selected from halogen, C₁-C₂-alkyl, C₁-C₂-haloalkyl, C₃-C₄-cycloalkyl, -O-C₁C₂-alkyl and -O-C₁C₂-haloalkyl.

7. The compounds according to any one of the claims 1 to 6, wherein X is a direct bond.

8. The compounds according to any one of the claims 1 to 7, wherein Y is a direct bond.

9. The compounds according to any of the claims 1 to 8, wherein Z is selected from is selected from hydrogen, C₁C₄-alkyl, -C(=O)-C₁-C₄-alkyl and SiR^{S1}R^{S2}R^{S3}, wherein R^{S1}, R^{S2}, R^{S3}, independently of each other, are selected from hydrogen, C₁-C₄-alkyl and phenyl, wherein phenyl is unsubstituted or carry 1, 2 or 3 identical or different groups R^{Sa}, whererein R^{Sa} is selected from halogen, C₁-C₄-alkyl and -O-C₁-C₄-alkyl, and wherein Z is unsubstituted or carries 1, 2, 3 or up to the maximum number of identical or different groups R^{na}, wherein R^{na} is selected from halogen and hydroxy.

10. The compounds according to any one of the claims 1 to 8, wherein Z is selected from C₃-C₆-cycloalkyl, phenyl, 5- or 6-membered heterocycloalkyl, 5- or 6-membered heterocycloalkenyl and 5- or 6-membered heteroaryl, wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S, cannot contain 2 contiguous atoms selected from O and S, wherein Z is unsubstituted or carries 1, 2, or 3 identical or different groups R^{a} selected from halogen, CN, C₁-C₂-alkyl, C₁-C₂-haloalkyl, -O-C₁C₂-alkyl, -O-C₁-C₂-haloalkyl, C₃-C₄-cycloalkyl and C₃-C₄-halocycloalkyl and/or 2 R^{a} substituents bound to neighboring ring atoms, together with the two interjacent ring atoms, form a saturated, partially unsaturated or aromatic 5- to 6-membered fused carbo- or heterocycle, wherein the heterocycle includes beside carbon atoms 1 or 2 heteroatoms independently selected from N, O and S as ring member atoms, provided that such heterocycle cannot contain 2 contiguous atoms selected from O and S; and wherein the aliphatic and cyclic moieties of R^{a} are unsubstituted or carry 1, 2, 3, 4 or up to the maximum number of identical or different groups R^{b} selected from halogen, CN, hydroxy, NO₂, C₁-C₄-alkyl, C₁-C₄-haloalkyl, -O-C₁C₄-alkyl, and -O-C₁C₄-haloalkyl.

11. The compounds according to claim 10, wherein Z is phenyl, and wherein Z is unsubstituted or carries 1, 2 or 3 identical or different groups R^{a} selected from halogen, CN, C₁-C₂-alkyl, C₁-C₂-haloalkyl, -O-C₁C₂-alkyl, -O-C₁C₂-haloalkyl, C₃-C₄-cycloalkyl and C₃-C₄-halocycloalkyl.

12. Agrochemical compositions comprising an auxiliary and at least one compound of formula I as defined in any of claims 1 to 11 or in the form of a stereoisomer and tautomer thereof or an agriculturally acceptable salt or N-oxide thereof.

13. Use of the compounds as defined in any of the claims 1 to 11 or a composition as defined in claim 12 for combating phytopathogenic fungi.

14. A method for combating phytopathogenic fungi comprising: treating curatively and/or preventively the plants or the plant propagation material of said plants that are at risk of being diseased from the said phytopathogenic fungi, and/or applying to the said phytopathogenic fungi, at least one compound of formula I as defined in any of the claims 1 to 11 or an agrochemical composition as defined in claim 12.

15. The use according to claim 13 or the method according to claim 14, wherein the phytopathogenic fungi contain an amino acid substitution F129L in the mitochondrial cytochrome b protein conferring resistance to Qo inhibitors.
